# EUROPEAN PATENT APPLICATION

(11) **EP 4 782 453 A1**
(43) Date of publication of application: **29.07.2026**
(21) Application number: 24870655.8
(22) Date of filing: 23.09.2024
(51) Int. Cl.: C07D 487/04, C07D 471/04, A61K 31/537, A61K 31/519, A61P 35/00, A61P 3/10, A61P 37/02

(54) **MENIN-MLL INTERACTION INHIBITOR AND PREPARATION METHOD THEREFOR, AND APPLICATION**

(30) Priority: 27.09.2023 CN 202311262366
(71) Applicant: Beyang Therapeutics Co., Ltd., Suzhou, Jiangsu 215127 (CN)
(72) Inventor: HU, Qiyue, Suzhou, Jiangsu 215127 (CN); HU, Weimin, Suzhou, Jiangsu 215127 (CN); LIU, Suxing, Suzhou, Jiangsu 215127 (CN)
(74) Representative: HGF
(86) International application number: PCT/CN2024/120409
(87) International publication number: WO 2025/067111

(57) **Abstract**

The present invention discloses a Menin-MLL interaction inhibitor, a preparation method therefor and uses thereof. The Menin-MLL interaction inhibitor is expected to be used in the treatment of tumors, diabetes and other diseases mediated by Menin-MLL interaction.

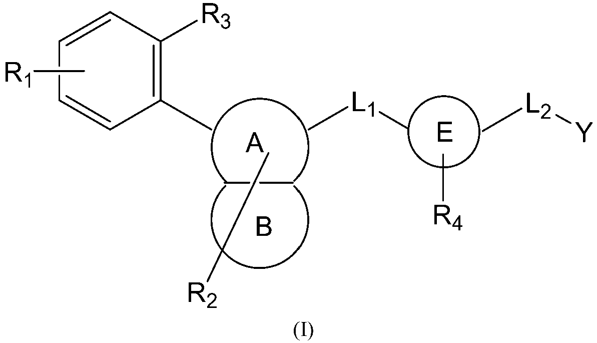

## Description

### TECHNICAL FIELD

The present invention relates to the technical field of chemical medicine, and in particular to a Menin-MLL interaction inhibitor, a preparation method therefor and uses thereof, in particular uses thereof in the treatment of tumors.

### BACKGROUND ART

Mixed lineage leukemia (MLL) proteins are histone methyltransferases, which are crucial for epigenetic regulation of gene transcription. Many acute leukemias, including acute myeloblastic leukemia (AML), acute lymphoblastic leukemia (ALL) and mixed lineage leukemia (MLL), are featured with the presence of a chimeric MLL fusion protein resulting from the chromosomal translocation of a MLL gene located on chromosome 11 band q23 (11q23). The chimeric MLL fusion protein retains approximately 1,400 amino acids of the N-terminus of MLL, but is fused to one of approximately 80 chaperone proteins (for example, AF4, AF9, ENL, AF10, ELL, AF6, AFlp, and GAS7). The MLL fusion protein lacks the primitive histone methyltransferase activity at the C-terminus of MLL, and acquires the ability to regulate the transcription of numerous oncogenes (including HOX and MEIS 1), resulting in increased cell proliferation and reduced cell differentiation, ultimately leading to leukemia.

Menin is a scaffold protein encoded by a multiple endocrine neoplasia type 1 (MEN1) gene. Menin mutations cause hereditary autosomal dominant neoplasia syndrome (MEN1 syndrome), resulting in the formation of tumors in several endocrine organs. The binding of Menin to the N-terminus of the MLL fusion protein is necessary for the observed oncogenic activity of the MLL fusion protein. This binding has been shown to constitutively upregulate the expression of HOX and MEIS1 oncogenes and to impair the proliferation and differentiation of hematopoietic cells, resulting in leukemia. MLL1 acts as a universal transcriptional activator for various gene regulatory pathways. In MLL fusion leukemias featured with distinct chromosomal translocations at 11q23, Menin is critical for maintaining MLL-associated myeloid transformation. In gene knockdown experiments, when the function of Menin decreases, the abnormal gene expression program mediated by MLL fusion is abolished. Furthermore, other studies have described the importance of Menin-MLL interaction in breast cancer, prostate cancer, synovial sarcoma and hepatocellular carcinoma. In these indications, the interactions between Menin and MLL fusion proteins and between Menin and MLL have been described to strictly regulate the expression of disease-related genes. Inhibiting the binding of Menin to MLL has recently become a new therapeutic strategy. Its beneficial therapeutic effects have been assumed in the models of leukemia, prostate cancer, breast cancer, colon cancer, liver cancer and synovial sarcoma. Hence, the interactions between Menin and MLL fusion proteins and between Menin and MLL represent potential small molecule therapeutic targets.

The nucleophosmin 1 (NPM1) gene encodes for a multifunctional protein located primarily in the nucleolus. Mutations in the NPM1 gene lead to its aberrant cytoplasmic localization (NPM1c). The interaction between MLL and Menin in NPM1-mutated AML shares common HOX gene characteristics and dependence with the rearrangement of MLL and Menin. AML cells with NPM1 mutations rely on the interaction between Menin and wild-type MLL to maintain this leukemogenesis, and these cells are sensitive to the blockade of Menin-MLL binding. Both MLL gene rearrangements and NPM1c can be targeted in the same manner, since MLL fusion proteins and wild-type MLL share a common amino-terminal domain.

The rearrangement of MLL genes occur in 5-10% of acute leukemia cases, and are particularly common in infantile acute leukemias (up to 70% of cases). MLL gene-rearranged leukemic subtypes are characterized by invasiveness, treatment resistance and high rates of early recurrence, even after initial complete remission. Leukemia patients, especially infants, with MLL gene chromosomal translocations have a very poor prognosis, with a 5-year survival rate of less than 40%. NPM1 is the most commonly mutant gene (approximately 30%) in adult acute myeloid leukemia (AML). There is an urgent need for a novel therapeutic strategy to treat this leukemia. Therefore, a small molecule inhibitor that blocks the Menin-MLL interaction represents a valuable target for the treatment of diseases involving MLL fusion proteins, exhibiting great importance in providing new options for cancer treatment.

### SUMMARY OF THE INVENTION

In view of the need in the prior art, the present invention provides a Menin-MLL interaction inhibitor, a preparation method therefor and uses thereof.

In a first aspect of the present invention, there is provided a compound or a pharmaceutically acceptable salt, stereoisomer, ester, prodrug, solvate and deuterated compound thereof, wherein the compound has a structure of: 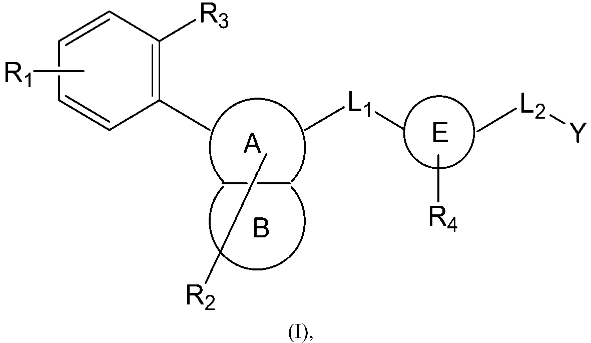 wherein,
Ring A is 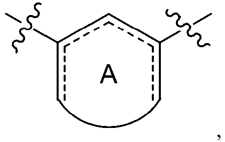 -̅ -̅ -̅ -̅ -̅ -̅ represents a single or double bond, and said Ring A is a five- to seven-membered aromatic or heterocyclic ring;
Ring B is a five- to seven-membered nitrogenous heterocyclic ring;
Ring E is a three- to eight-membered saturated or partially saturated aliphatic ring, an aromatic ring, or a four- to twelve-membered saturated or partially saturated heterocyclic ring;
L₁ and L₂ are independently selected from the group consisting of: a single bond, C₁-C₁₀ alkylene, and -(C₀-C₁₀ alkylene)-Q-(C₀-C₁₀ alkylene)- , with Q selected from the group consisting of: -O-, -S-, -C(O)-, -C(O)O-, -OC(O)-, - C(O)N(R_{La})-, -N(R_{La})C(O)-, -N(R_{La})C(O)O-, -N(R_{La})C(O)N(R_{La})-, -N(R_{La})-, -S(O)₂-, -S(O)₂N(R_{La})-, -N(R_{La})S(O)₂-, -S(O)-, -S(O)N(R_{La})-, -N(R_{La})S(O)-, -C(=NR_{La})-, and -C(=NR_{La})-NR_{La}-; H atom(s) in said alkylene can be optionally substituted with a group selected from the group consisting of: H, C₁-C₁₀ alkyl, C₂-C₁₀ alkenyl, C₂-C₁₀ alkynyl, C₃-C₁₀ cycloalkyl, C₆-C₁₀ aryl, 4-10-membered heterocyclyl, halogen, cyano, nitro, azido, halogenated C₁-C₁₀ alkyl, hydroxyl, C₁-C₁₀ alkoxy, halogenated C₁-C₁₀ alkoxy, amino, and C₁-C₁₀ alkylamino; H in said cycloalkyl, aryl or heterocyclyl may be optionally substituted with a group selected from the group consisting of: H, halogen, C₁-C₁₀ alkyl, and halogenated C₁-C₁₀ alkyl; R_{La} is selected from the group consisting of: H, C₁-C₁₀ alkyl, C₂-C₁₀ alkenyl, C₂-C₁₀ alkynyl, C₃-C₁₀ cycloalkyl, C₃-C₁₀ cycloalkylalkyl, C₆-C₁₀ aryl, C₇-C₁₂ aralkyl, 4-10-membered heterocyclyl, and 4-10-membered heterocyclylalkyl;
R₁ is one or more independent substituents on the benzene ring, and each R₁ is independently selected from the group consisting of: H, halogen, C₁-C₁₀ alkyl, C₂-C₁₀ alkenyl, C₂-C₁₀ alkynyl, cyano, nitro, azido, halogenated C₁-C₁₀ alkyl, halogenated C₁-C₁₀ alkoxy, C₁-C₁₀ cyanoalkyl, -OR₁₀₁, -C(O)R₁₀₁, -C(O)OR₁₀₁, - NR₁₀₂C(O)OR₁₀₁, -OC(O)R₁₀₁, -NR₁₀₂SO₂R₁₀₁, -SO₂NR₁₀₁R₁₀₂, -NR₁₀₂C(O)R₁₀₁, -C(O)NR₁₀₁R₁₀₂, -NR₁₀₁R₁₀₂, -SR₁₀₁, -S(O)R₁₀₁, -S(O)₂R₁₀₁, -SO₃H, -NR₁₀₂(CR₁₀₃R₁₀₄)ₜOR₁₀₁, -(C₀-C₆ alkylene)-(C₆-C₁₀ aryl), -SO₂-(C₀-C₆ alkylene)-(C₆-C₁₀ aryl), -S-(C₀-C₆ alkylene)-(C₆-C₁₀ aryl), -O-(C₀-C₆ alkylene)-(C₆-C₁₀ aryl), -(C₀-C₆ alkylene)-(4-10-membered heterocyclyl), -SO₂-(C₀-C₆ alkylene)-(4-10-membered heterocyclyl), -S-(C₀-C₆ alkylene)-(4-10-membered heterocyclyl), -O-(C₀-C₆ alkylene)-(4-10-membered heterocyclyl), -(C₀-C₆ alkylene)-(C₃-C₁₀ cycloalkyl), -SO₂-(C₀-C₆ alkylene)-(C₃-C₁₀ cycloalkyl), -S-(C₀-C₆ alkylene)-(C₃-C₁₀ cycloalkyl), and -O-(C₀-C₆ alkylene)-(C₃-C₁₀ cycloalkyl), with t being an integer ranging from 0 to 5; wherein said C₁-C₁₀ alkyl, C₂-C₁₀ alkenyl, C₂-C₁₀ alkynyl, C₃-C₁₀ cycloalkyl, C₆-C₁₀ aryl, or 4-10-membered heterocyclyl may be optionally substituted with one or more groups selected from the group consisting of: halogen, cyano, nitro, azido, -OR', -C(O)R', -C(O)OR', -OC(O)R', -NR'C(O)OR", -NR'SO₂R", -SO₂NR'R", - NR'C(O)R", -C(O)NR'R", -NR'R", -SR', -SOR', -SO₂R', -SO₃H, C₁-C₁₀ alkyl, halogenated C₁-C₁₀ alkyl, halogenated C₁-C₁₀ alkoxy, C₁-C₁₀ cyanoalkyl, C₃-C₁₀ cycloalkyl, phenyl, and 4-10-membered heterocyclyl; R₁₀₁ to R₁₀₄ are each independently selected from the group consisting of: H, C₁-C₁₀ alkyl, C₂-C₁₀ alkenyl, C₂-C₁₀ alkynyl, C₃-C₁₀ cycloalkyl, C₃-C₁₀ cycloalkylalkyl, C₆-C₁₀ aryl, C₇-C₁₂ aralkyl, 4-10-membered heterocyclyl, and 4-10-membered heterocyclylalkyl; wherein said C₁-C₁₀ alkyl, C₂-C₁₀ alkenyl, C₂-C₁₀ alkynyl, C₃-C₁₀ cycloalkyl, C₆-C₁₀ aryl, or 4-10-membered heterocyclyl may be optionally substituted with one or more groups selected from the group consisting of: halogen, cyano, nitro, azido, -OR', -C(O)R', -C(O)OR', -OC(O)R', -NR'C(O)OR", -NR'SO₂R", - SO₂NR'R", -NR'C(O)R", -C(O)NR'R", -NR'R", -SR', -SOR', -SO₂R', -SO₃H, C₁-C₁₀ alkyl, halogenated C₁-C₁₀ alkyl, halogenated C₁-C₁₀ alkoxy, C₁-C₁₀ cyanoalkyl, C₃-C₁₀ cycloalkyl, phenyl, and 4-10-membered heterocyclyl;
R₂ is one or more independent substituents on a 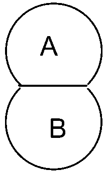 ring, and each R₂ is independently selected from the group consisting of: H, =O, C₁-C₁₀ alkyl, C₂-C₁₀ alkenyl, C₂-C₁₀ alkynyl, halogen, cyano, nitro, azido, halogenated C₁-C₁₀ alkyl, halogenated C₁-C₁₀ alkoxy, C₁-C₁₀ cyanoalkyl, -OR₂₀₁, -C(O)R₂₀₁, -C(O)OR₂₀₁, -NR₂₀₂C(O)OR₂₀₁, - OC(O)R₂₀₁, -NR₂₀₂SO₂R₂₀₁, -SO₂NR₂₀₁R₂₀₂, -NR₂₀₂C(O)R₂₀₁, -C(O)NR₂₀₁R₂₀₂, -NR₂₀₁R₂₀₂, -SR₂₀₁, -S(O)R₂₀₁, - S(O)₂R₂₀₁, -SO₃H, -NR₂₀₂(CR₂₀₃R₂₀₄)ⱼOR₂₀₁, -(C₀-C₆ alkylene)-(C₆-C₁₀ aryl), -SO₂-(C₀-C₆ alkylene)-(C₆-C₁₀ aryl), - S-(C₀-C₆ alkylene)-(C₆-C₁₀ aryl), -O-(C₀-C₆ alkylene)-(C₆-C₁₀ aryl), -(C₀-C₆ alkylene)-(4-10-membered heterocyclyl), -SO₂-(C₀-C₆ alkylene)-(4-10-membered heterocyclyl), -S-(C₀-C₆ alkylene)-(4-10-membered heterocyclyl), -O-(C₀-C₆ alkylene)-(4-10-membered heterocyclyl), -(C₀-C₆ alkylene)-(C₃-C₁₀ cycloalkyl), -SO₂-(C₀-C₆ alkylene)-(C₃-C₁₀ cycloalkyl), -S-(C₀-C₆ alkylene)-(C₃-C₁₀ cycloalkyl), and -O-(C₀-C₆ alkylene)-(C₃-C₁₀ cycloalkyl), with j being an integer ranging from 0 to 5; wherein said C₁-C₁₀ alkyl, C₂-C₁₀ alkenyl, C₂-C₁₀ alkynyl, C₃-C₁₀ cycloalkyl, C₆-C₁₀ aryl, or 4-10-membered heterocyclyl may be optionally substituted with one or more groups selected from the group consisting of: halogen, cyano, nitro, azido, -OR', -C(O)R', -C(O)OR', -OC(O)R', - NR'C(O)OR", -NR'SO₂R", -SO₂NR'R", -NR'C(O)R", -C(O)NR'R", -NR'R", -SR', -SOR', -SO₂R', -SO₃H, C₁-C₁₀ alkyl, halogenated C₁-C₁₀ alkyl, halogenated C₁-C₁₀ alkoxy, C₁-C₁₀ cyanoalkyl, C₃-C₁₀ cycloalkyl, phenyl, and 4-10-membered heterocyclyl;
R₂₀₁ to R₂₀₄ are each independently selected from the group consisting of: H, C₁-C₁₀ alkyl, C₂-C₁₀ alkenyl, C₂-C₁₀ alkynyl, C₃-C₁₀ cycloalkyl, C₃-C₁₀ cycloalkylalkyl, C₆-C₁₀ aryl, C₇-C₁₂ aralkyl, 4-10-membered heterocyclyl, and 4-10-membered heterocyclylalkyl; wherein said C₁-C₁₀ alkyl, C₂-C₁₀ alkenyl, C₂-C₁₀ alkynyl, C₃-C₁₀ cycloalkyl, C₆-C₁₀ aryl, or 4-10-membered heterocyclyl may be optionally substituted with one or more groups selected from the group consisting of: halogen, cyano, nitro, azido, -OR', -C(O)R', -C(O)OR', -OC(O)R', -NR'C(O)OR", -NR'SO₂R", - SO₂NR'R", -NR'C(O)R", -C(O)NR'R", -NR'R", -SR', -SOR', -SO₂R', -SO₃H, C₁-C₁₀ alkyl, halogenated C₁-C₁₀ alkyl, halogenated C₁-C₁₀ alkoxy, C₁-C₁₀ cyanoalkyl, C₃-C₁₀ cycloalkyl, phenyl, and 4-10-membered heterocyclyl; R₃ is selected from the group consisting of: H, halogen, C₁-C₁₀ alkyl, C₂-C₁₀ alkenyl, C₂-C₁₀ alkynyl, cyano, nitro, azido, halogenated C₁-C₁₀ alkyl, halogenated C₁-C₁₀ alkoxy, C₁-C₁₀ cyanoalkyl, -OR₃₀₁, -C(O)R₃₀₁, -C(O)OR₃₀₁, - NR₃₀₂C(O)OR₃₀₁, -OC(O)R₃₀₁, -NR₃₀₂SO₂R₃₀₁, -SO₂NR₃₀₁R₃₀₂, -NR₃₀₂C(O)R₃₀₁, -C(O)NR₃₀₁R₃₀₂, -NR₃₀₁R₃₀₂, - SR₃₀₁, -S(O)R₃₀₁, -S(O)₂R₃₀₁, -SO₃H, -NR₃₀₂(CR₃₀₃R₃₀₄)ᵢOR₃₀₁, -(C₀-C₆ alkylene)-(C₆-C₁₀ aryl), -SO₂-(C₀-C₆ alkylene)-(C₆-C₁₀ aryl), -S-(C₀-C₆ alkylene)-(C₆-C₁₀ aryl), -O-(C₀-C₆ alkylene)-(C₆-C₁₀ aryl), -(C₀-C₆ alkylene)-(4-10-membered heterocyclyl), -SO₂-(C₀-C₆ alkylene)-(4-10-membered heterocyclyl), -S-(C₀-C₆ alkylene)-(4-10-membered heterocyclyl), -O-(C₀-C₆ alkylene)-(4-10-membered heterocyclyl), -(C₀-C₆ alkylene)-(C₃-C₁₀ cycloalkyl), -SO₂-(C₀-C₆ alkylene)-(C₃-C₁₀ cycloalkyl), -S-(C₀-C₆ alkylene)-(C₃-C₁₀ cycloalkyl), and -O-(C₀-C₆ alkylene)-(C₃-C₁₀ cycloalkyl), with i being an integer ranging from 0 to 5; wherein said C₁-C₁₀ alkyl, C₂-C₁₀ alkenyl, C₂-C₁₀ alkynyl, C₃-C₁₀ cycloalkyl, C₆-C₁₀ aryl, or 4-10-membered heterocyclyl may be optionally substituted with one or more groups selected from the group consisting of: halogen, cyano, nitro, azido, -OR', -C(O)R', -C(O)OR', -OC(O)R', - NR'C(O)OR", -NR'SO₂R", -SO₂NR'R", -NR'C(O)R", -C(O)NR'R", -NR'R", -SR', -SOR', -SO₂R', -SO₃H, C₁-C₁₀ alkyl, halogenated C₁-C₁₀ alkyl, halogenated C₁-C₁₀ alkoxy, C₁-C₁₀ cyanoalkyl, C₃-C₁₀ cycloalkyl, phenyl, and 4-10-membered heterocyclyl;
R₃₀₁ to R₃₀₄ are each independently selected from the group consisting of: H, C₁-C₁₀ alkyl, C₂-C₁₀ alkenyl, C₂-C₁₀ alkynyl, C₃-C₁₀ cycloalkyl, C₃-C₁₀ cycloalkylalkyl, C₆-C₁₀ aryl, C₇-C₁₂ aralkyl, 4-10-membered heterocyclyl, and 4-10-membered heterocyclylalkyl; wherein said C₁-C₁₀ alkyl, C₂-C₁₀ alkenyl, C₂-C₁₀ alkynyl, C₃-C₁₀ cycloalkyl, C₆-C₁₀ aryl, or 4-10-membered heterocyclyl may be optionally substituted with one or more groups selected from the group consisting of: halogen, cyano, nitro, azido, -OR', -C(O)R', -C(O)OR', -OC(O)R', -NR'C(O)OR", -NR'SO₂R", - SO₂NR'R", -NR'C(O)R", -C(O)NR'R", -NR'R", -SR', -SOR', -SO₂R', -SO₃H, C₁-C₁₀ alkyl, halogenated C₁-C₁₀ alkyl, halogenated C₁-C₁₀ alkoxy, C₁-C₁₀ cyanoalkyl, C₃-C₁₀ cycloalkyl, phenyl, and 4-10-membered heterocyclyl;
or, R₃₀₁ and R₃₀₂, together with the atom attached thereto, form heterocyclyl, and said heterocyclyl may be optionally substituted with one or more groups selected from the group consisting of: H, =O, C₁-C₁₀ alkyl, C₂-C₁₀ alkenyl, C₂-C₁₀ alkynyl, halogen, cyano, nitro, azido, halogenated C₁-C₁₀ alkyl, halogenated C₁-C₁₀ alkoxy, C₁-C₁₀ cyanoalkyl, - OR₅, -C(O)R₅, -C(O)OR₅, -NR₆C(O)OR₅, -OC(O)R₅, -NR₆SO₂R₅, -SO₂NR₅R₆, -NR₆C(O)R₅, -C(O)NR₅R₆, -NR₅R₆, -SR₅, -S(O)R₅, -S(O)₂R₅, -SO₃H, -(C₀-C₆ alkylene)-(C₆-C₁₀ aryl), -SO₂-(C₀-C₆ alkylene)-(C₆-C₁₀ aryl), -S-(C₀-C₆ alkylene)-(C₆-C₁₀ aryl), -O-(C₀-C₆ alkylene)-(C₆-C₁₀ aryl), -(C₀-C₆ alkylene)-(4-10-membered heterocyclyl), -SO₂-(C₀-C₆ alkylene)-(4-10-membered heterocyclyl), -S-(C₀-C₆ alkylene)-(4-10-membered heterocyclyl), -O-(C₀-C₆ alkylene)-(4-10-membered heterocyclyl), -(C₀-C₆ alkylene)-(C₃-C₁₀ cycloalkyl), -SO₂-(C₀-C₆ alkylene)-(C₃-C₁₀ cycloalkyl), -S-(C₀-C₆ alkylene)-(C₃-C₁₀ cycloalkyl), and -O-(C₀-C₆ alkylene)-(C₃-C₁₀ cycloalkyl); wherein said C₁-C₁₀ alkyl, C₂-C₁₀ alkenyl, C₂-C₁₀ alkynyl, C₃-C₁₀ cycloalkyl, C₆-C₁₀ aryl, or 4-10-membered heterocyclyl may be optionally substituted with one or more groups selected from the group consisting of: halogen, cyano, nitro, azido, - OR', -C(O)R', -C(O)OR', -OC(O)R', -NR'C(O)OR", -NR'SO₂R", -SO₂NR'R", -NR'C(O)R", -C(O)NR'R", -NR'R", - SR', -SOR', -SO₂R', -SO₃H, C₁-C₁₀ alkyl, halogenated C₁-C₁₀ alkyl, halogenated C₁-C₁₀ alkoxy, C₁-C₁₀ cyanoalkyl, C₃-C₁₀ cycloalkyl, phenyl, and 4-10-membered heterocyclyl;
R₅ and R₆ are each independently selected from the group consisting of: H, C₁-C₁₀ alkyl, C₂-C₁₀ alkenyl, C₂-C₁₀ alkynyl, C₃-C₁₀ cycloalkyl, C₃-C₁₀ cycloalkylalkyl, C₆-C₁₀ aryl, C₇-C₁₂ aralkyl, 4-10-membered heterocyclyl, and 4-10-membered heterocyclylalkyl; wherein said C₁-C₁₀ alkyl, C₂-C₁₀ alkenyl, C₂-C₁₀ alkynyl, C₃-C₁₀ cycloalkyl, C₆-C₁₀ aryl, or 4-10-membered heterocyclyl may be optionally substituted with one or more groups selected from the group consisting of: halogen, cyano, nitro, azido, -OR', -C(O)R', -C(O)OR', -OC(O)R', -NR'C(O)OR", -NR'SO₂R", - SO₂NR'R", -NR'C(O)R", -C(O)NR'R", -NR'R", -SR', -SOR', -SO₂R', -SO₃H, C₁-C₁₀ alkyl, halogenated C₁-C₁₀ alkyl, halogenated C₁-C₁₀ alkoxy, C₁-C₁₀ cyanoalkyl, C₃-C₁₀ cycloalkyl, phenyl, and 4-10-membered heterocyclyl;
R₄ is one or more independent substituents on Ring E, and each R₄ is independently selected from the group consisting of: H, halogen, =O, C₁-C₁₀ alkyl, C₂-C₁₀ alkenyl, C₂-C₁₀ alkynyl, cyano, nitro, azido, halogenated C₁-C₁₀ alkyl, halogenated C₁-C₁₀ alkoxy, C₁-C₁₀ cyanoalkyl, -OR₄₀₁, -C(O)R₄₀₁, -C(O)OR₄₀₁, -NR₁₀₂C(O)OR₄₀₁, -OC(O)R₄₀₁, - NR₄₀₂SO₂R₄₀₁, -SO₂NR₄₀₁R₄₀₂, -NR₄₀₂C(O)R₄₀₁, -C(O)NR₄₀₁R₄₀₂, -NR₄₀₁R₄₀₂, -SR₄₀₁, -S(O)R₄₀₁, -S(O)₂R₄₀₁, -SO₃H, -NR₄₀₂(CR₄₀₃R₄₀₄)ₘOR₁₀₄, -(C₀-C₆ alkylene)-(C₆-C₁₀ aryl), -SO₂-(C₀-C₆ alkylene)-(C₆-C₁₀ aryl), -S-(C₀-C₆ alkylene)-(C₆-C₁₀ aryl), -O-(C₀-C₆ alkylene)-(C₆-C₁₀ aryl), -(C₀-C₆ alkylene)-(4-10-membered heterocyclyl), -SO₂-(C₀-C₆ alkylene)-(4-10-membered heterocyclyl), -S-(C₀-C₆ alkylene)-(4-10-membered heterocyclyl), -O-(C₀-C₆ alkylene)-(4-10-membered heterocyclyl), -(C₀-C₆ alkylene)-(C₃-C₁₀ cycloalkyl), -SO₂-(C₀-C₆ alkylene)-(C₃-C₁₀ cycloalkyl), - S-(C₀-C₆ alkylene)-(C₃-C₁₀ cycloalkyl), and -O-(C₀-C₆ alkylene)-(C₃-C₁₀ cycloalkyl), with m being an integer ranging from 0 to 5; wherein said C₁-C₁₀ alkyl, C₂-C₁₀ alkenyl, C₂-C₁₀ alkynyl, C₃-C₁₀ cycloalkyl, C₆-C₁₀ aryl, or 4-10-membered heterocyclyl may be optionally substituted with one or more groups selected from the group consisting of: halogen, cyano, nitro, azido, -OR', -C(O)R', -C(O)OR', -OC(O)R', -NR'C(O)OR", -NR'SO₂R", -SO₂NR'R", - NR'C(O)R", -C(O)NR'R", -NR'R", -SR', -SOR', -SO₂R', -SO₃H, C₁-C₁₀ alkyl, halogenated C₁-C₁₀ alkyl, halogenated C₁-C₁₀ alkoxy, C₁-C₁₀ cyanoalkyl, C₃-C₁₀ cycloalkyl, phenyl, and 4-10-membered heterocyclyl;
R₄₀₁ to R₄₀₄ are each independently selected from the group consisting of: H, C₁-C₁₀ alkyl, C₂-C₁₀ alkenyl, C₂-C₁₀ alkynyl, C₃-C₁₀ cycloalkyl, C₃-C₁₀ cycloalkylalkyl, C₆-C₁₀ aryl, C₇-C₁₂ aralkyl, 4-10-membered heterocyclyl, and 4-10-membered heterocyclylalkyl; wherein said C₁-C₁₀ alkyl, C₂-C₁₀ alkenyl, C₂-C₁₀ alkynyl, C₃-C₁₀ cycloalkyl, C₆-C₁₀ aryl, or 4-10-membered heterocyclyl may be optionally substituted with one or more groups selected from the group consisting of: halogen, cyano, nitro, azido, -OR', -C(O)R', -C(O)OR', -OC(O)R', -NR'C(O)OR", -NR'SO₂R", - SO₂NR'R", -NR'C(O)R", -C(O)NR'R", -NR'R", -SR', -SOR', -SO₂R', -SO₃H, C₁-C₁₀ alkyl, halogenated C₁-C₁₀ alkyl, halogenated C₁-C₁₀ alkoxy, C₁-C₁₀ cyanoalkyl, C₃-C₁₀ cycloalkyl, phenyl, and 4-10-membered heterocyclyl;
Y is selected from the group consisting of: C₁-C₁₀ alkyl, C₂-C₁₀ alkenyl, C₂-C₁₀ alkynyl, three- to eight-membered saturated or partially saturated alcyl, aryl, four- to twelve-membered heterocyclyl, aryl-fused three- to eight-membered alcyl, aryl-fused three- to eight-membered heterocyclyl, three- to eight-membered aliphatic ring-fused aryl, three- to eight-membered aliphatic ring-fused heterocyclyl, three- to eight-membered heterocyclic ring-fused three- to eight-membered aliphatic ring, three- to eight-membered heterocyclic ring-fused aryl, and three- to eight-membered heterocyclic ring-fused five- to ten-membered heterocyclyl, and Y may be optionally substituted with one or more R^{Y} groups, with R^{Y} selected from the group consisting of: H, =O, 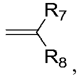 C₁-C₁₀ alkyl, C₂-C₁₀ alkenyl, C₂-C₁₀ alkynyl, halogen, cyano, nitro, azido, halogenated C₁-C₁₀ alkyl, halogenated C₁-C₁₀ alkoxy, C₁-C₁₀ cyanoalkyl, -OR₇, -C(O)R₇, -C(O)OR₇, -NR₈C(O)OR₇, -OC(O)R₇, -NR₈SO₂R₇, -SO₂NR₇R₈, -NR₇C(O)R₈, -C(O)NR₇R₈, -NR₇R₈, -SR₇, -S(O)R₇, -S(O)₂R₇, -SO₃H, -(C₀-C₆ alkylene)-(C₆-C₁₀ aryl), -SO₂-(C₀-C₆ alkylene)-(C₆-C₁₀ aryl), -S-(C₀-C₆ alkylene)-(C₆-C₁₀ aryl), -O-(C₀-C₆ alkylene)-(C₆-C₁₀ aryl), -(C₀-C₆ alkylene)-(4-10-membered heterocyclyl), -SO₂-(C₀-C₆ alkylene)-(4-10-membered heterocyclyl), -S-(C₀-C₆ alkylene)-(4-10-membered heterocyclyl), -O-(C₀-C₆ alkylene)-(4-10-membered heterocyclyl), -(C₀-C₆ alkylene)-(C₃-C₁₀ cycloalkyl), -SO₂-(C₀-C₆ alkylene)-(C₃-C₁₀ cycloalkyl), -S-(C₀-C₆ alkylene)-(C₃-C₁₀ cycloalkyl), and -O-(C₀-C₆ alkylene)-(C₃-C₁₀ cycloalkyl); wherein said C₁-C₁₀ alkyl, C₂-C₁₀ alkenyl, C₂-C₁₀ alkynyl, C₃-C₁₀ cycloalkyl, C₆-C₁₀ aryl, or 4-10-membered heterocyclyl may be optionally substituted with one or more groups selected from the group consisting of: halogen, cyano, nitro, azido, - OR', -C(O)R', -C(O)OR', -OC(O)R', -NR'C(O)OR", -NR'SO₂R", -SO₂NR'R", -NR'C(O)R", -C(O)NR'R", -NR'R", - SR', -SOR', -SO₂R', -SO₃H, C₁-C₁₀ alkyl, halogenated C₁-C₁₀ alkyl, halogenated C₁-C₁₀ alkoxy, C₁-C₁₀ cyanoalkyl, C₃-C₁₀ cycloalkyl, phenyl, and 4-10-membered heterocyclyl;
R₇ and R₈ are each independently selected from the group consisting of: H, C₁-C₁₀ alkyl, C₂-C₁₀ alkenyl, C₂-C₁₀ alkynyl, C₃-C₁₀ cycloalkyl, C₃-C₁₀ cycloalkyl alkyl, C₆-C₁₀ aryl, C₇-C₁₂ aralkyl, 4-10-membered heterocyclyl, and 4-10-membered heterocyclyl alkyl; wherein the C₁-C₁₀ alkyl, C₂-C₁₀ alkenyl, C₂-C₁₀ alkynyl, C₃-C₁₀ cycloalkyl, C₆-C₁₀ aryl, or 4-10-membered heterocyclyl may be optionally substituted with one or more groups selected from the group consisting of: halogen, cyano, nitro, azido, -OR', -C(O)R', -C(O)OR', -OC(O)R', -NR'C(O)OR", -NR'SO₂R", - SO₂NR'R", -NR'C(O)R", -C(O)NR'R", -NR'R", -SR', -SOR', -SO₂R', -SO₃H, C₁-C₁₀ alkyl, halogenated C₁-C₁₀ alkyl, halogenated C₁-C₁₀ alkoxy, C₁-C₁₀ cyanoalkyl, C₃-C₁₀ cycloalkyl, phenyl, and 4-10-membered heterocyclyl; R' and R" are each independently selected from the group consisting of: **H,** C₁-C₁₀ alkyl, C₂-C₁₀ alkenyl, C₂-C₁₀ alkynyl, C₃-C₁₀ cycloalkyl, C₃-C₁₀ cycloalkylalkyl, C₆-C₁₀ aryl, C₇-C₁₂ aralkyl, 4-10-membered heterocyclyl, and 4-10-membered heterocyclylalkyl.

In some embodiments of the present invention, Ring A is a five- or six-membered aromatic or nitrogenous heterocyclic aromatic ring, for example,

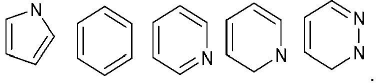

In some embodiments of the present invention, Ring B is a five-membered or six-membered nitrogenous heterocyclic ring, for example, 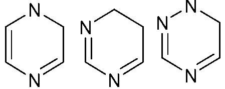 or

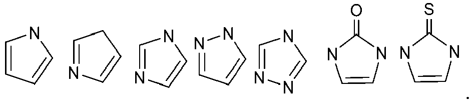

In some embodiments of the present invention, Ring 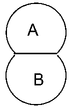 is one of the following structures:

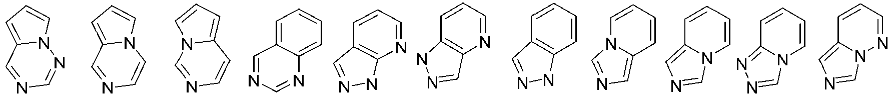

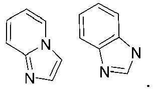

In some embodiments of the present invention, the 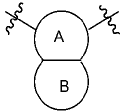 moiety is 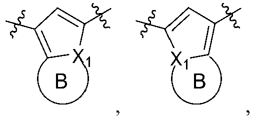 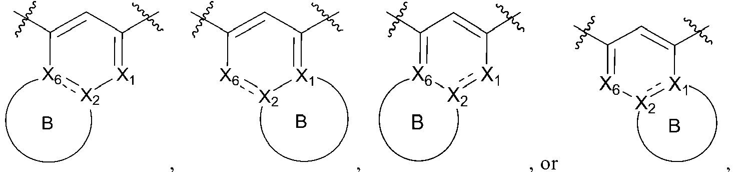 wherein X₁ is N or C, X₂ is N or C, X₆ is N or C, and -̅ -̅ -̅ -̅ -̅ -̅ represents a single or double bond.

In some embodiments of the present invention, the 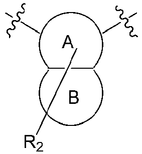 moiety is 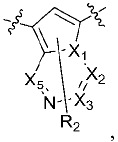 wherein X₁ is N or C, X₂ is N or C, X₃ is N or C, and X₅ is N or C, for example,

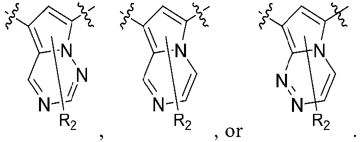

In some embodiments of the present invention, the 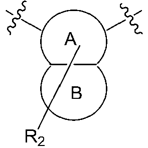 moiety is 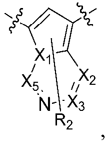 wherein X₁ is N or C, X₂ is N or C, X₃ is N or C, and X₅ is N or C, for example,

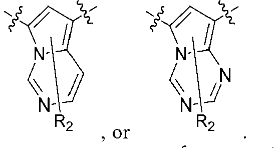

In some embodiments of the present invention, the 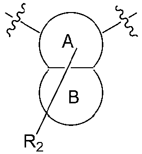 moiety is 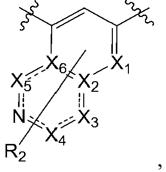 wherein X₁ is N or C, X₂ is N or C, X₃ is N or C, X₄ is N or C or absent (that is, X₃ is directly linked to N), X₅ is N or C or absent (that is, X₆ is directly linked to N), X₆ is N or C, and -̅ -̅ -̅ -̅ -̅ -̅ represents a single or double bond. In some embodiments of the present invention, the compound has a structure as follows:

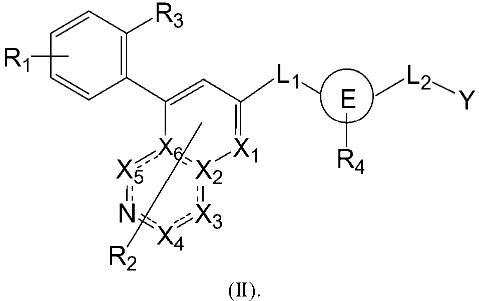

In some embodiments of the present invention, X₂ is N.

In some embodiments of the present invention, X₃ is absent.

In some embodiments of the present invention, X₄ is C.

In some embodiments of the present invention, the 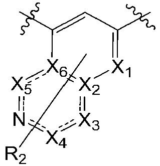 moiety is 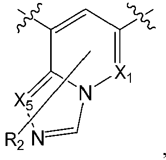 for example,

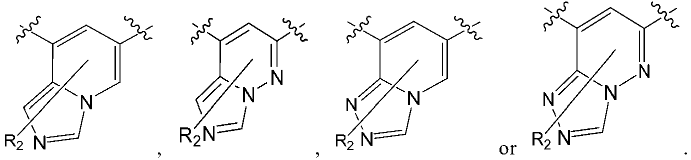

In some embodiments of the present invention, the 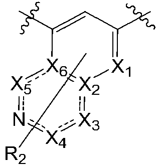 moiety is 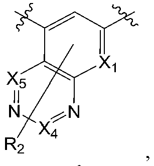 for example, 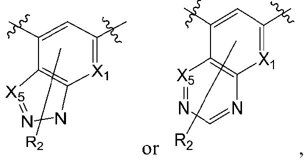 for example,

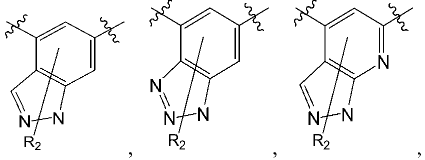

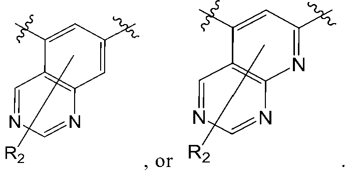

In some embodiments of the present invention, the 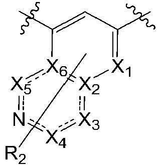 moiety is 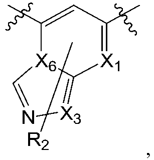 for example,

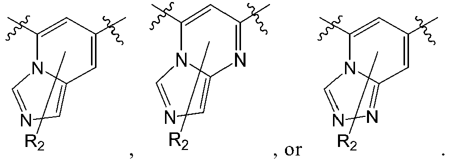

In a preferred embodiment of the present invention, the 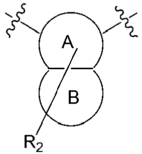 moiety is 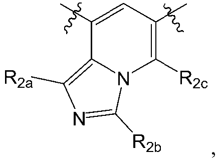 wherein R₂ₐ, R_{2b}, or R_{2c} is defined as those defined for R₂ above.

In a preferred embodiment of the present invention, the 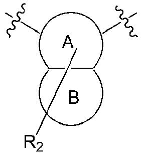 moiety is 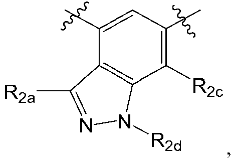 wherein R₂ₐ, R_{2d}, or R_{2c} is defined as those defined for R₂ above.

In some embodiments of the present invention, the 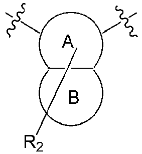 moiety is 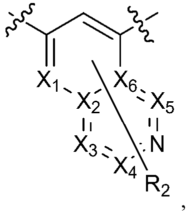 wherein X₁ is N or C, X₂ is N or C, X₃ is N or C, X₄ is N or C or absent (that is, X₃ is directly linked to N), X₅ is N or C or absent (that is, X₆ is directly linked to N), X₆ is N or C, and -̅ -̅ -̅ -̅ -̅ -̅ represents a single or double bond.

In some embodiments of the present invention, the 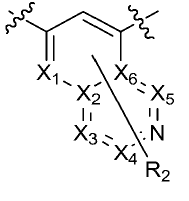 moiety is for example 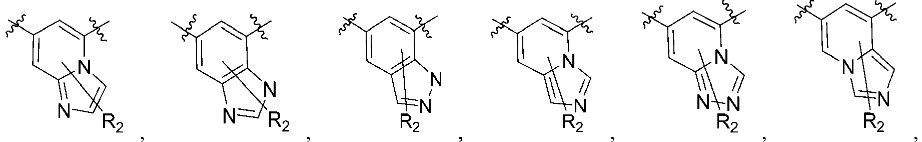 or

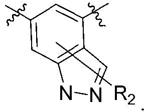

In some embodiments of the present invention, the compound has a structure as follows:

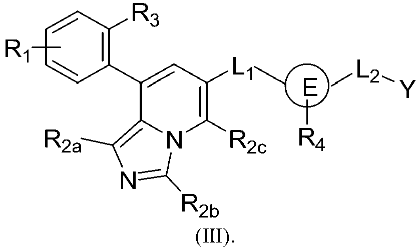

In some embodiments of the present invention, the compound has a structure as follows:

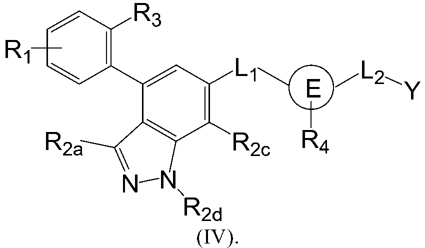

In other embodiments of the present invention, Ring A is 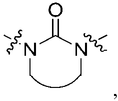 and Ring B is an aromatic ring or a five- or six-membered nitrogenous heterocyclic aromatic ring, for example, the 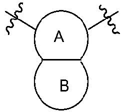 moiety may be 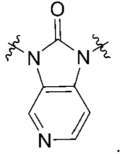 In some embodiments of the present invention, the compound has a structure as follows:

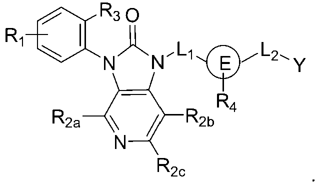

In other embodiments of the present invention, Ring A is 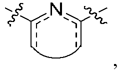 and Ring B is an aromatic ring or a five- or six-membered nitrogenous heterocyclic aromatic ring, for example, the 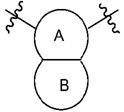 moiety may be 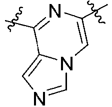 .In some embodiments of the present invention, the compound has a structure as follows:

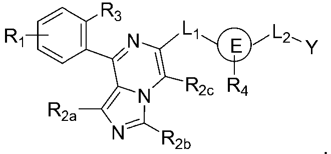

In some embodiments of the present invention, R₂ is selected from the group consisting of: H, C₁-C₆ alkyl (for example, -CH₃, 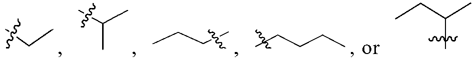 ), halogen (for example, F or Cl), cyano, nitro, azido, halogenated C₁-C₆ alkyl (for example, -CHF₂, -CH₂F, -CF₃, -CH₂-CH₂F, -CH₂-CHF₂, - CH₂-CF₃, -CH₂CH₂-CF₃, or -CH₂CH₂CH₂-CF₃), halogenated C₁-C₆ alkoxy (for example, 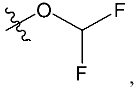 or 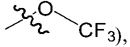 C₁-C₆ cyanoalkyl (for example, 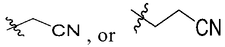 ), -OR₂₀₁, -C(O)R₂₀₁, -C(O)OR₂₀₁, - NR₂₀₂C(O)OR₂₀₁, -OC(O)R₂₀₁, -NR₂₀₂SO₂R₂₀₁, -SO₂NR₂₀₁R₂₀₂, -NR₂₀₂C(O)R₂₀₁, -C(O)NR₂₀₁R₂₀₂, -NR₂₀₁R₂₀₂, -SR₂₀₁, -S(O)₂R₂₀₁, -SO₃H, -(C₀-C₃ alkylene)-(phenyl), -(C₀-C₃ alkylene)-(4-10-membered heterocyclyl), and -(C₀-C₃ alkylene)-(C₃-C₆ cycloalkyl); wherein the phenyl, 4-10-membered heterocyclyl, or C₃-C₁₀ cycloalkyl may be optionally substituted with one or more groups selected from the group consisting of: halogen, cyano, hydroxyl, amino, C₁-C₃ alkyl, and C₁-C₃ halogenated alkyl. Specifically, the 4-10-membered heterocyclic ring is a 4-6-membered heterocyclic ring , for example, 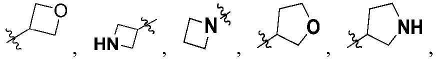 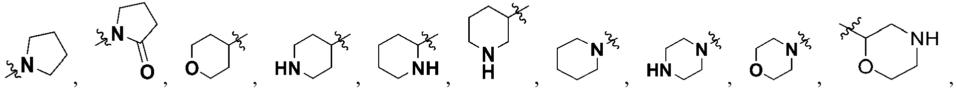 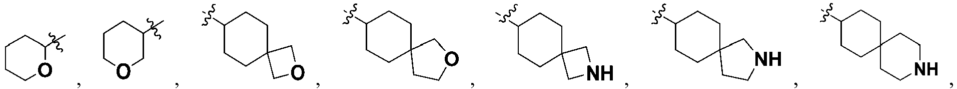 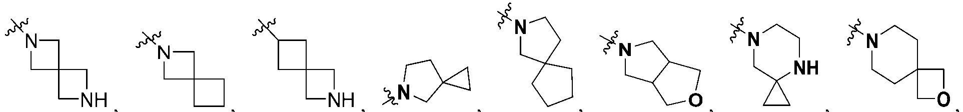 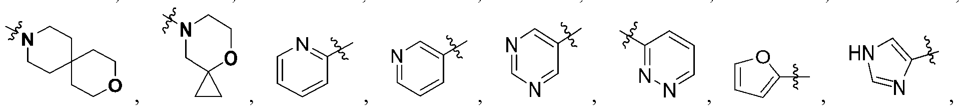 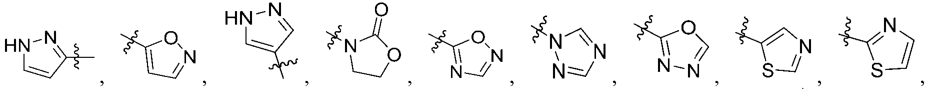 or 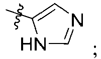 and specifically, the C₃-C₈ cycloalkyl is C₃-C₆ cycloalkyl, for example,

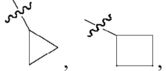

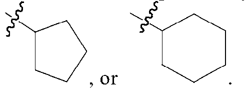

In some embodiments of the present invention, R₂₀₁ is selected from H and C₁₋₆ alkyl.

In some embodiments of the present invention, R₂₀₂ is selected from the group consisting of: H, C₁-C₆ alkyl, C₃-C₆ cycloalkyl, and -(C₁-C₃ alkylene)-(C₃-C₆ cycloalkyl) (for example, 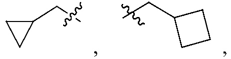 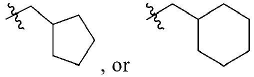 ).

In some embodiments of the present invention, R₂ is selected from the group consisting of: H, C₁-C₆ alkyl, halogen, cyano, halogenated C₁-C₆ alkyl, halogenated C₁-C₆ alkoxy, C₁-C₆ cyanoalkyl, -O(C₀-C₆ alkyl), - C(O)(C₀-C₆ alkyl), and -(C₀-C₃ alkylene)-(C₃-C₆ cycloalkyl).

In some embodiments of the present invention, R₂ₐ is selected from the group consisting of: H and halogen (for example, F or Cl).

In some embodiments of the present invention, R_{2b} is selected from the group consisting of: H, C₁-C₆ alkyl (for example, methyl), halogenated C₁-C₆ alkyl (for example, -CF₃), halogenated C₁-C₆ alkoxy (for example, -OCF₃), -(C₀-C₃ alkylene)-(C₃-C₆ cycloalkyl) (for example, 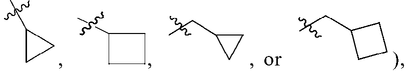 and -C(O)(C₀-C₆ alkyl) (for example,

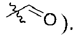

In some embodiments of the present invention, R_{2c} is selected from the group consisting of: H and halogen (for example, F or Cl).

In some embodiments of the present invention, R_{2d} is selected from the group consisting of: H, C₁-C₆ alkyl, halogenated C₁-C₆ alkyl, -(C₀-C₃ alkylene)-(C₃-C₆ cycloalkyl), and -C(O)(C₀-C₆ alkyl), in particular H or C₁-C₃ alkyl (for example, methyl).

In some embodiments of the present invention, the 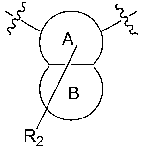 moiety is selected from the group consisting of:

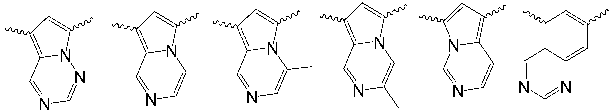

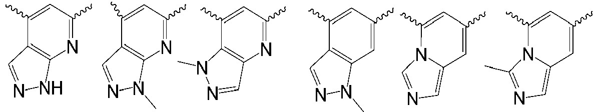

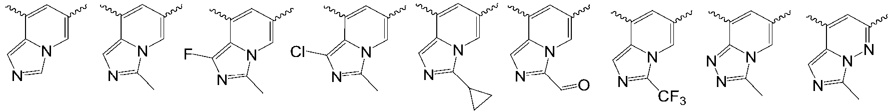

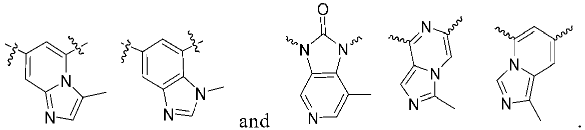

In some embodiments of the present invention, the 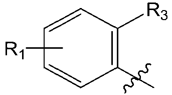 moiety is 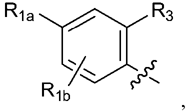 wherein R₁ₐ is H or halogen (for example, F or Cl), and R_{1b} is one or more independent substituents on a benzene ring, and is defined as those defined for R₁ above.

Specifically, R₁ may be independently selected from the group consisting of: H, halogen, C₁-C₆ alkyl, halogen, cyano, nitro, azido, halogenated C₁-C₆ alkyl, -N(C₀-C₆ alkyl)C(O)(C₀-C₆ alkyl), and -C(O)N(C₀-C₆ alkyl)(C₀-C₆ alkyl).

In some embodiments of the present invention, R₁ₐ is H.

In some embodiments of the present invention, R₁ₐ is F.

In some embodiments of the present invention, R₁ₐ is Cl.

In some embodiments of the present invention, R_{1b} is H.

More specifically, the 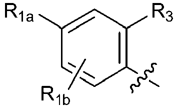 moiety may be 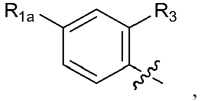 for example,

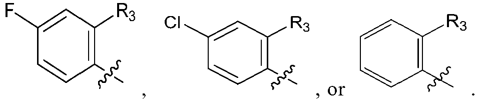

In some embodiments of the present invention, the compound has a structure as follows:

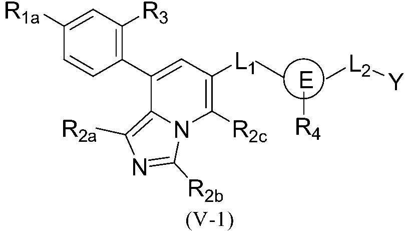

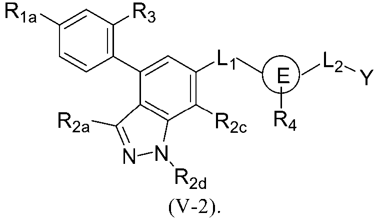

In some embodiments of the present invention, R₃ is -NR₃₀₂C(O)R₃₀₁ or -C(O)NR₃₀₁R₃₀₂.

In some embodiments of the present invention, R₃₀₁ or R₃₀₂ is independently selected from the group consisting of: H, optionally substituted C₁-C₆ alkyl, optionally substituted C₃-C₆ cycloalkyl, optionally substituted C₃-C₁₀ cycloalkylalkyl, optionally substituted 4-10-membered heterocyclyl, optionally substituted 4-10-membered heterocyclylalkyl; or, R₃₀₁ and R₃₀₂, together with the atom attached thereto, form optionally substituted 4-8-membered saturated or partially saturated heterocyclic ring (in particular, saturated heterocyclic ring).

In some embodiments of the present invention, R₃₀₁ or R₃₀₂ is independently selected from the group consisting of: H, C₁-C₆ alkyl, -(C₀-C₆ alkylene)-(C₃-C₆ cycloalkyl), and -(C₀-C₆ alkylene)-(4-10-membered heterocyclyl), wherein the C₁-C₆ alkyl, C₃-C₆ cycloalkyl, or 4-10-membered heterocyclyl is optionally substituted with one or more groups selected from the group consisting of: halogen, cyano, nitro, azido, -OR', -C(O)R', -C(O)OR', -OC(O)R', -NR'C(O)OR", -NR'SO₂R", -SO₂NR'R", -NR'C(O)R", -C(O)NR'R", -NR'R", - SR', -SOR', -SO₂R', -SO₃H, C₁-C₆ alkyl, halogenated C₁-C₆ alkyl, halogenated C₁-C₆ alkoxy, C₁-C₆ cyanoalkyl, C₃-C₆ cycloalkyl, phenyl, and 4-10-membered heterocyclyl, and R' and R" are each independently selected from the group consisting of: H and C₁-C₆ alkyl. In some embodiments of the present invention, R₃₀₁ or R₃₀₂ is independently selected from the group consisting of: H, C₁-C₆ alkyl (in particular, ethyl, or isopropyl), C₃-C₆ cycloalkyl (for example, cyclopropyl, or cyclobutyl), -(C₁-C₃ alkylene)-(C₃-C₆ cycloalkyl), halogenated C₁-C₆ alkyl (for example, -CHF₂, -CF₃, - CH₂-CHF₂, or -CH₂-CF₃), -(C₁-C₃ alkylene)-O-(C₀-C₃ alkyl), and -(C₁-C₃ alkylene)-NH-(C₀-C₃ alkyl).

In some embodiments of the present invention, R₃₀₁ is isopropyl.

In some embodiments of the present invention, R₃₀₂ is selected from the group consisting of: H, C₁-C₆ alkyl (for example, ethyl, or isopropyl), halogenated C₁-C₆ alkyl (for example, -CH₂-CHF₂, or -CH₂-CF₃), C₃-C₆ cycloalkyl (for example, cyclopropyl, or cyclobutyl), -(C₁-C₃ alkylene)-O-(C₀-C₃ alkyl) (for example, 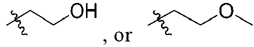 ), and -(C₁-C₃ alkylene)-NH-(C₀-C₃ alkyl) (for example,

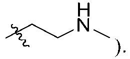

In some embodiments of the present invention, R₃ is

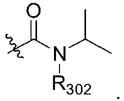

In some embodiments of the present invention, R₃ is selected from the group consisting of: 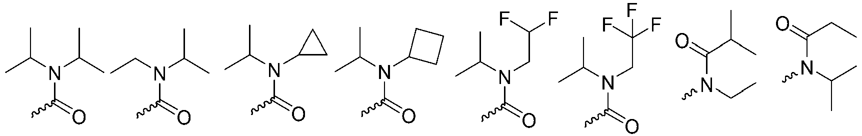 and

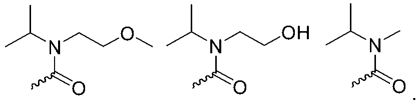

In some embodiments of the present invention, R₃₀₁ and R₃₀₂, together with the atom attached thereto, form a 4-8-membered saturated heterocyclic ring, which may be optionally substituted with one or more R₃₀₅, with R₃₀₅ selected from the group consisting of: H, halogen (for example, F), C₁-C₆ alkyl, C₂-C₆ alkenyl, C₂-C₆ alkynyl, cyano, hydroxyl, amino, halogenated C₁-C₆ alkyl, C₁-C₆ alkoxy, C₁-C₆ alkylamino, halogenated C₁-C₆ alkoxy, C₁-C₆ cyanoalkyl, C₃-C₆ cycloalkyl, and 4-10-membered heterocyclyl.

In some embodiments of the present invention, R₃ is 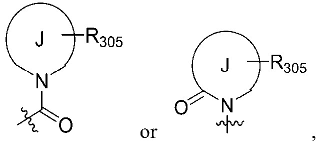 and Ring J is a 4-8-membered saturated heterocyclic ring, which may be a monocyclic or bicyclic ring (including a fused or spiro ring), and optionally, which may include one or more other heteroatoms, and R₃₀₅ is one or more independent substituents on Ring J, and is defined as those defined above.

In some embodiments of the present invention, Ring J is

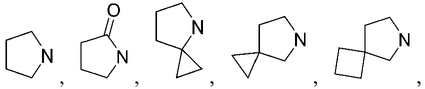

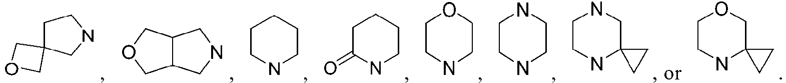

In some embodiments of the present invention, R₃ is selected from the group consisting of:

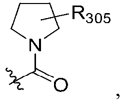

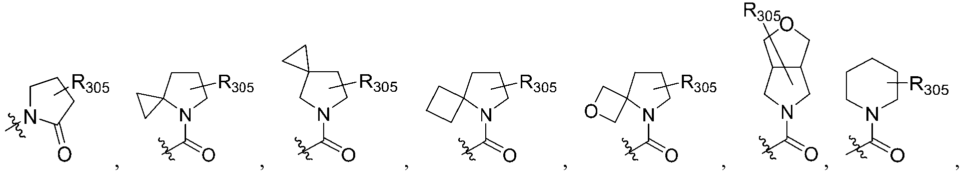

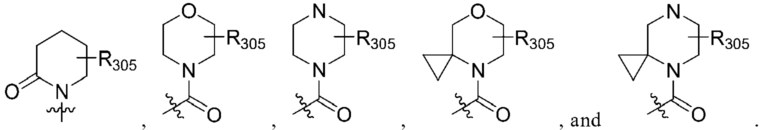

In some embodiments of the present invention, R₃₀₅ is selected from the group consisting of: H, halogen (for example, F), C₁-C₆ alkyl (for example, methyl), halogenated C₁-C₆ alkyl (for example, -CHF₂, -CH₂F, or - CF₃), C₃-C₆ cycloalkyl (for example, 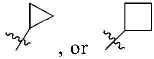 ), and 4-6-membered heterocycloalkyl (for example,

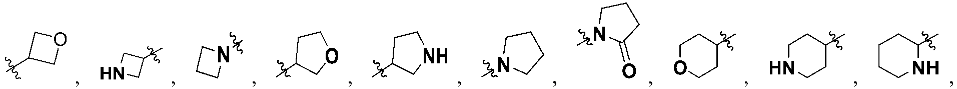

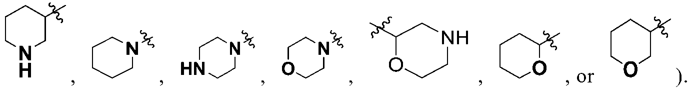

In some embodiments of the present invention, R₃₀₅ is selected from the group consisting of: H, halogen (for example, F), C₁-C₃ alkyl, and C₁-C₃ halogenated alkyl.

In some embodiments of the present invention, R₃₀₅ is selected from the group consisting of: H, F, -CH₃, - CHF₂, -CH₂F, and -CF₃.

In some embodiments of the present invention, R₃ is selected from the group consisting of:

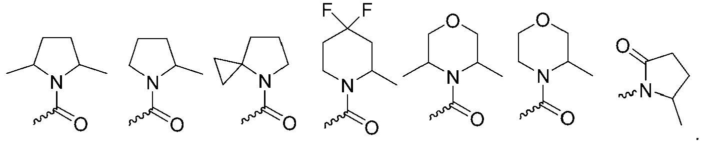

In some embodiments of the present invention, R₃ is -(C₀-C₆ alkylene)-(C₆-C₁₀ aryl), for example, phenyl, wherein the aryl (for example, phenyl) may be optionally substituted with one or more R₃₀₆, and R₃₀₆ is selected from the group consisting of: H, halogen (for example, F), C₁-C₆ alkyl, C₂-C₆ alkenyl, C₂-C₆ alkynyl, cyano, hydroxyl, amino, halogenated C₁-C₆ alkyl, C₁-C₆ alkoxy, C₁-C₆ alkylamino, halogenated C₁-C₆ alkoxy, C₁-C₆ cyanoalkyl, C₃-C₆ cycloalkyl, and 4-10-membered heterocyclyl.

In some embodiments of the present invention, R₃ is 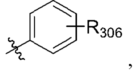 and R₃₀₆ is one or more independent substituents (in particular, 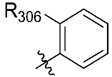 ) on a benzene ring.

In some embodiments of the present invention, R₃₀₆ is selected from the group consisting of: H, halogen (for example, F), C₁-C₆ alkyl (for example, methyl, ethyl, or isopropyl), halogenated C₁-C₆ alkyl (for example, - CHF₂, -CH₂F, or -CF₃), and C₃-C₆ cycloalkyl (for example, 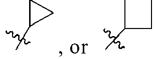 ).

In some embodiments of the present invention, R₃₀₆ is selected from the group consisting of: H, C₁-C₃ alkyl, halogenated C₁-C₃ alkyl, and C₃-C₄ cycloalkyl.

In some embodiments of the present invention, R₃₀₆ is selected from the group consisting of:

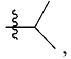

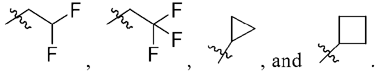

In some embodiments of the present invention, R₃ is selected from the group consisting of:

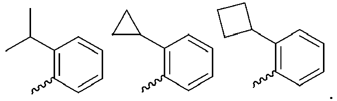

In some embodiments of the present invention, R₃ is -(C₀-C₆ alkylene)-(4-10-membered heterocyclyl), the 4-10-membered heterocyclyl may be optionally substituted with one or more R₃₀₇, and R₃₀₇ is selected from the group consisting of: H, halogen (for example, F), C₁-C₆ alkyl, C₂-C₆ alkenyl, C₂-C₆ alkynyl, cyano, hydroxyl, amino, halogenated C₁-C₆ alkyl, C₁-C₆ alkoxy, C₁-C₆ alkylamino, halogenated C₁-C₆ alkoxy, C₁-C₆ cyanoalkyl, C₃-C₆ cycloalkyl, and 4-10-membered heterocyclyl. More specifically, the 4-10-membered heterocyclic ring may be

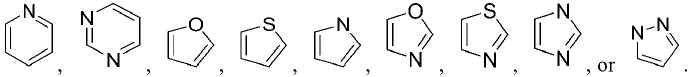

In some embodiments of the present invention, R₃ is selected from the group consisting of:

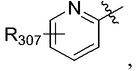

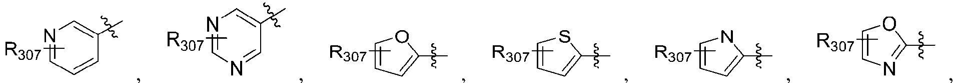

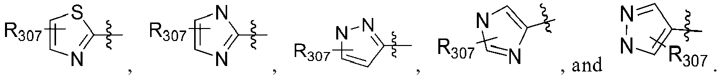

In some embodiments of the present invention, R₃₀₇ is selected from the group consisting of: H, halogen (for example, F), C₁-C₆ alkyl (for example, methyl, ethyl, or isopropyl), halogenated C₁-C₆ alkyl (for example, - CHF₂, -CH₂F, or -CF₃), and C₃-C₆ cycloalkyl (for example, 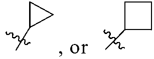 ).

In some embodiments of the present invention, R₃₀₇ is selected from the group consisting of: H, C₁-C₃ alkyl, halogenated C₁-C₃ alkyl, and C₃-C₄ cycloalkyl.

In some embodiments of the present invention, R₃₀₇ is selected from the group consisting of:

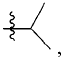

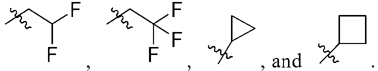

In some embodiments of the present invention, R₃ is selected from the group consisting of:

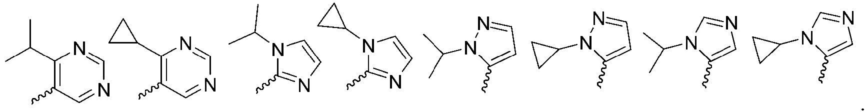

In some embodiments of the present invention, L₁ is a single bond.

In some embodiments of the present invention, L₁ is C₁-C₆ alkylene, in particular, C₁-C₄ alkylene, for example, methylene,

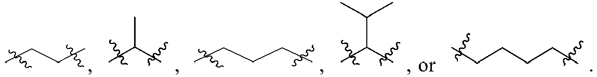

In some embodiments of the present invention, L₁ is -O-, -S- or -N(R_{La})-; more specifically, R_{La} may be selected from the group consisting of: H and C₁-C₆ alkyl (for example, methyl).

In some embodiments of the present invention, Ring E is a 3-8-membered saturated or partially saturated aliphatic ring , for example,

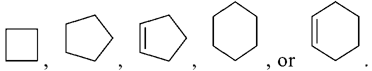

In some embodiments of the present invention, the 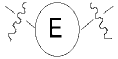 moiety is

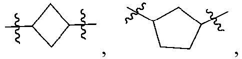

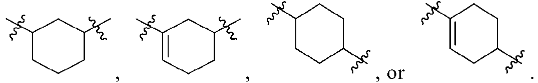

In some embodiments of the present invention, Ring E is a 4-12-membered saturated or partially saturated heterocyclic ring, in particular, a 4-10-membered saturated or partially saturated nitrogenous heterocyclic ring, for example,

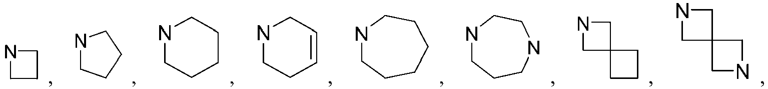

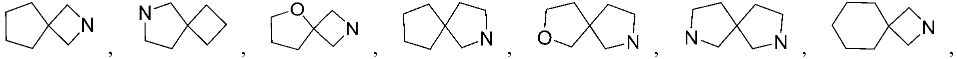

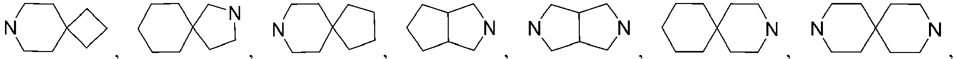

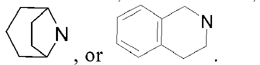

In some embodiments of the present invention, the 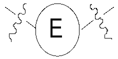 moiety (which has a left terminal linked to L₁ and a right terminal linked to L₂) is 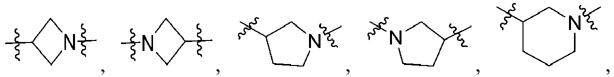 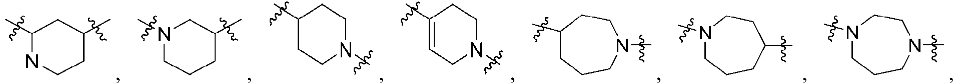 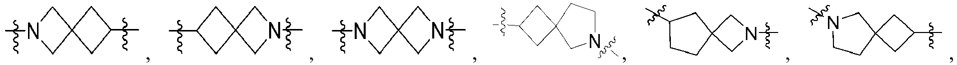 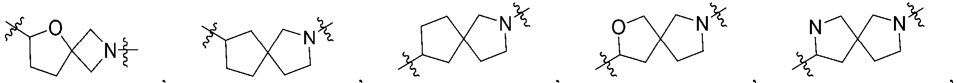 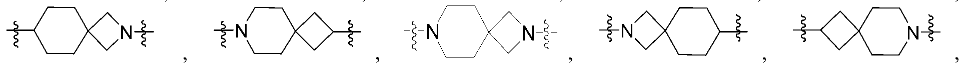 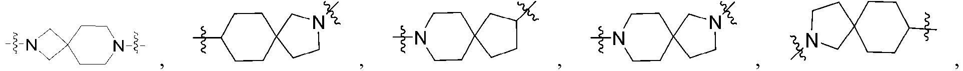 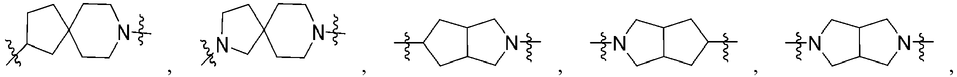 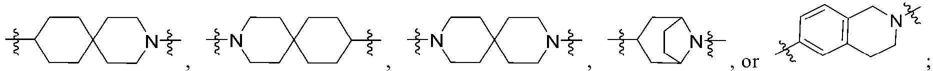 in particular, 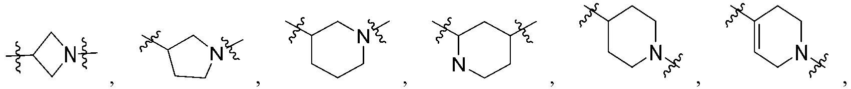 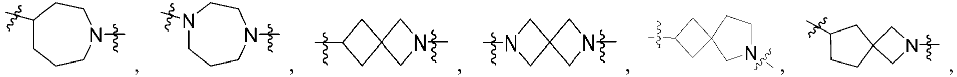 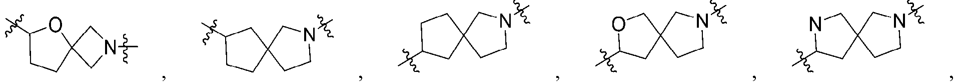 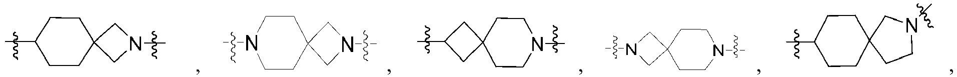 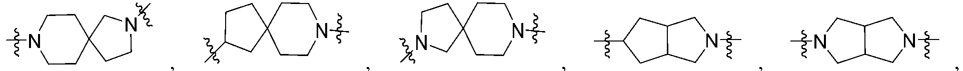 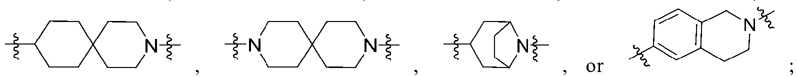 preferably, the 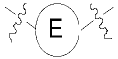 moiety is

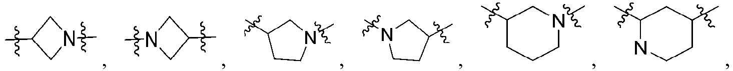

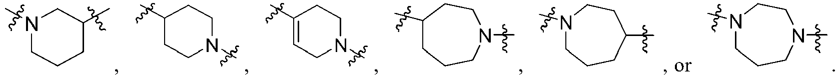

In some embodiments of the present invention, the 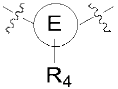 moiety is

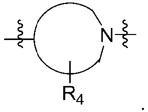

In some embodiments of the present invention, the 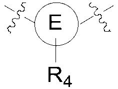 moiety is 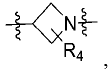 in particular

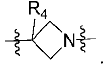

In some embodiments of the present invention, the 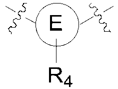 moiety is 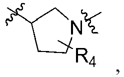 in particular

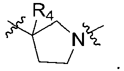

In some embodiments of the present invention, R₄ is selected from the group consisting of: H, halogen (for example, F), =O, cyano, nitro, azido, hydroxyl, C₁-C₆ alkyl, halogenated C₁-C₆ alkyl, C₁-C₆ alkoxy, halogenated C₁-C₆ alkoxy, and C₁-C₆ cyanoalkyl.

In some embodiments of the present invention, R₄ is selected from the group consisting of: H, halogen (for example, F), hydroxyl, C₁-C₃ alkyl, and C₁-C₃ alkoxy.

In some embodiments of the present invention, R₄ is selected from the group consisting of: H, F and hydroxyl.

In some embodiments of the present invention, the 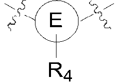 moiety (which has a left terminal linked to L₁ and a right terminal linked to L₂) is selected from the group consisting of:

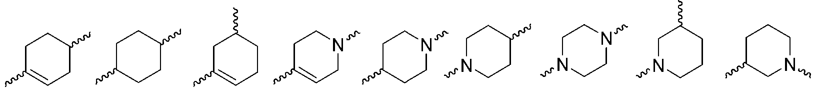

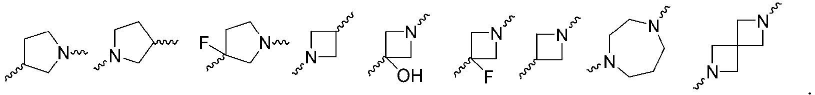

In some embodiments of the present invention, L₂ is a single bond.

In some embodiments of the present invention, L₂ is C₁-C₆ alkylene or -(C₀-C₆ alkylene)-Q-(C₀-C₆ alkylene)-, wherein the H atom in the alkylene may be optionally substituted with a group selected from the group consisting of: H, C₁-C₃ alkyl, halogen, cyano, halogenated C₁-C₃ alkyl, hydroxyl, C₁-C₃ alkoxy, halogenated C₁-C₃ alkoxy, amino, C₁-C₆ alkylamino, C₃-C₆ cycloalkyl, 3-6-membered heterocyclyl (for example, 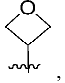 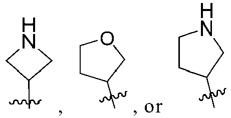 ), C₁-C₃ alkyl-substituted 3-6-membered heterocyclyl (for example, 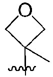 ).

In some embodiments of the present invention, Q is selected from the group consisting of: -O-, -S-, -C(O)-, - C(O)O-, -OC(O)-, -C(O)N(R_{La})-, -N(R_{La})C(O)-, -N(R_{La})C(O)O-, -N(R_{La})C(O)N(R_{La})-, -N(R_{La})-, -S(O)₂-, - S(O)₂N(R_{La})-, -N(R_{La})S(O)₂-, -S(O)-, -S(O)N(R_{La})-, -N(R_{La})S(O)-, -C(=NR_{La})-, and -C(=NR_{La})-NR_{La}-; wherein each R_{La} is independently selected from the group consisting of: H and C₁-C₆ alkyl (for example, methyl, or ethyl).

In some embodiments of the present invention, Q is selected from the group consisting of: -O-, -S-, -C(O)-, - C(O)O-, -OC(O)-, -C(O)N(R_{La})-, -N(R_{La})C(O)-, -N(R_{La})C(O)O-, -N(R_{La})-, -S(O)₂-, and -S(O)-; wherein each R_{La} is independently selected from the group consisting of: H and C₁-C₃ alkyl (for example, methyl, or ethyl). In some embodiments of the present invention, Q is selected from the group consisting of: -O-, -C(O)-, - N(C₀-C₃ alkyl)-, and -N(C₀-C₃ alkyl)C(O)-.

In some embodiments of the present invention, L₂ is selected from the group consisting of: methylene, 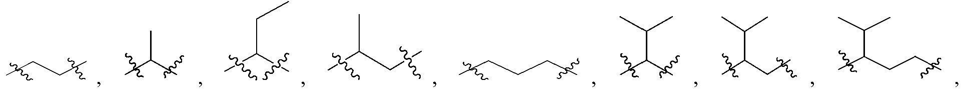 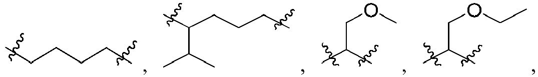 -O-, -C(O)-, -NHC(O)-,

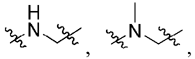

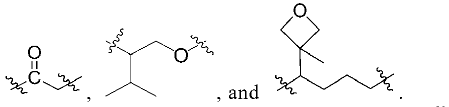

In some embodiments of the present invention, R^{Y} is R^{Y0}, which is selected from the group consisting of: H, =O, C₁-C₆ alkyl, C₂-C₆ alkenyl, C₂-C₆ alkynyl, halogen, cyano, nitro, azido, halogenated C₁-C₆ alkyl, halogenated C₁-C₆ alkoxy, C₁-C₆ cyanoalkyl, -OR₇, -C(O)R₇, -C(O)OR₇, -NR₈C(O)OR₇, -OC(O)R₇, - NR₈SO₂R₇, -SO₂NR₇R₈, -NR₇C(O)R₈, -C(O)NR₇R₈, -NR₇R₈, -SR₇, -S(O)R₇, -S(O)₂R₇, -SO₃H, -(C₀-C₆ alkylene)-(phenyl), -SO₂-(C₀-C₆ alkylene)₋(phenyl), -S-(C₀-C₆ alkylene).(phenyl), -O-(C₀-C₆ alkylene)₋(phenyl), -(C₀-C₆ alkylene)-(4-10-membered heterocyclyl), -SO₂-(C₀-C₆ alkylene)₋(4-10-membered heterocyclyl), -S-(C₀-C₆ alkylene)₋(4-10-membered heterocyclyl), -O-(C₀-C₆ alkylene)-(4-10-membered heterocyclyl), -(C₀-C₆ alkylene)₋(C₃-C₆ cycloalkyl), -SO₂-(C₀-C₆ alkylene)₋(C₃-C₆ cycloalkyl), -S-(C₀-C₆ alkylene)₋(C₃-C₆ cycloalkyl), and -O-(C₀-C₆ alkylene)-(C₃-C₆ cycloalkyl), and the C₁-C₆ alkyl, C₂-C₆ alkenyl, C₂-C₆ alkynyl, C₃-C₆ cycloalkyl, phenyl, or 4-10-membered heterocyclyl may be optionally substituted with one or more groups selected from the group consisting of: halogen, cyano, nitro, azido, -OR', -C(O)R', - C(O)OR', -NR'C(O)R", -C(O)NR'R", -NR'R", C₁-C₆ alkyl, halogenated C₁-C₆ alkyl, halogenated C₁-C₆ alkoxy, C₁-C₆ cyanoalkyl, C₃-C₆ cycloalkyl, phenyl, and 4-10-membered heterocyclyl.

In some embodiments of the present invention, R^{Y} is R^{Y1}, which is selected from the group consisting of: H, -C(O)R₇, -C(O)OR₇, -NR₈SO₂R₇, -NR₈C(O)OR₇, -NR₈C(O)R₇, -C(O)NR₇R₈, -NR₇R₈, -S(O)₂R₇, C₁-C₆ alkyl, -(C₀-C₆ alkylene)₋(phenyl), -(C₀-C₆ alkylene)-(4-10-membered heterocyclyl), and -(C₀-C₆ alkylene)-(C₃-C₆ cycloalkyl); wherein the C₁-C₆ alkyl, C₃-C₆ cycloalkyl, phenyl, or 4-10-membered heterocyclyl may be optionally substituted with one or more groups selected from the group consisting of: halogen, cyano, nitro, azido, -OR', -C(O)R', -C(O)OR', -NR'C(O)R", -C(O)NR'R", -NR'R", C₁-C₆ alkyl, halogenated C₁-C₆ alkyl, halogenated C₁-C₆ alkoxy, C₁-C₆ cyanoalkyl, C₁-C₆ hydroxylalkyl, C₁-C₆ alkoxyalkyl, C₃-C₆ cycloalkyl, phenyl, and 4-10-membered heterocyclyl; R₇ and R₈ are each independently selected from the group consisting of: H, C₁-C₆ alkyl, -(C₀-C₆ alkylene)-(C₃-C₆ cycloalkyl), -(C₀-C₆ alkylene)₋(phenyl), and -(C₀-C₆ alkylene)-(4-10-membered heterocyclyl); wherein the C₁-C₆ alkyl, C₃-C₆ cycloalkyl, phenyl, or 4-10-membered heterocyclyl may be optionally substituted with one or more groups selected from the group consisting of: halogen, cyano, nitro, azido, -OR', -C(O)R', -C(O)OR', -NR'C(O)R", -C(O)NR'R", -NR'R", C₁-C₆ alkyl, halogenated C₁-C₆ alkyl, halogenated C₁-C₆ alkoxy, C₁-C₆ cyanoalkyl, C₃-C₆ cycloalkyl, phenyl, and 4-10-membered heterocyclyl.

In some embodiments of the present invention, R^{Y} is R^{Y2}, which is selected from the group consisting of: H, cyano, C₁-C₆ alkyl, and 4-10-membered heterocyclyl, wherein the C₁-C₆ alkyl or 4-10-membered heterocyclyl may be optionally substituted with one or more groups selected from the group consisting of: =O, halogen, cyano, nitro, amino, 4-10-membered heterocyclyl, C₃-C₈ cycloalkyl, 4-10-membered heterocyclyl, -(C₀-C₄ alkylene)-OH, -(C₁-C₄ alkylene)-O-(C₁-C₄ alkyl), C₁-C₄ alkoxy, halogenated C₁-C₄ alkoxy, -C(O)O(C₀-C₆ alkyl), -C(O)N(C₀-C₆ alkyl)(C₀-C₆ alkyl), -N(C₀-C₆ alkyl)C(O) (C₀-C₆ alkyl), -SO₂(C₀-C₆ alkyl), - SO₂(phenyl), halogenated -SO₂(C₁-C₆ alkyl), -SO₂N(C₀-C₆ alkyl)(C₀-C₆ alkyl), -SO₂NH(phenyl), - NHSO₂(C₀-C₆ alkyl), -NHSO₂(phenyl) and -NHSO₂(C₁-C₆ halogenated alkyl); and in R^{Y2}, the cycloalkyl or heterocyclyl may be further optionally substituted with a substituent selected from the group consisting of: C₁-C₄ alkyl, and halogenated C₁-C₄ alkyl.

In some embodiments of the present invention, R^{Y} is R^{Y3}, which is selected from the group consisting of: H, =O, 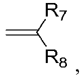 C₁-C₆ alkyl, halogenated C₁-C₆ alkyl, C₁-C₆ alkoxy, halogenated C₁-C₆ alkoxy, -(C₀-C₆ alkylene)-(C₃-C₆ cycloalkyl), -(C₀-C₆ alkylene)-(phenyl), and -(C₀-C₆ alkylene)-(4-10-membered heterocyclyl); wherein the C₃-C₆ cycloalkyl, phenyl, or 4-10-membered heterocyclyl may be optionally substituted with one or more groups selected from the group consisting of: halogen, cyano, nitro, azido, -OR', -C(O)R', -C(O)OR', -NR'C(O)R", -C(O)NR'R", -NR'R", C₁-C₆ alkyl, halogenated C₁-C₆ alkyl, halogenated C₁-C₆ alkoxy, C₁-C₆ cyanoalkyl, C₃-C₆ cycloalkyl, phenyl, and 4-10-membered heterocyclyl.

Specifically, Y is selected from groups in sets Y1, Y2, Y3, Y4, and Y5.

In some embodiments of the present invention, Y1 is 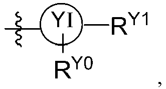 wherein Ring YI is a 3-8-membered saturated or partially saturated aliphatic ring, a benzene ring, or a 4-12-membered saturated or partially saturated heterocyclic ring, and R^{Y0} is one or more independent substituents on a ring, and is defined as above in the present invention.

In some embodiments of the present invention, Ring YI is

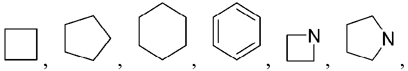

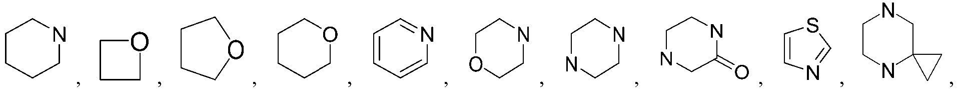

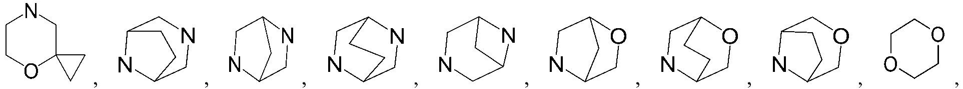

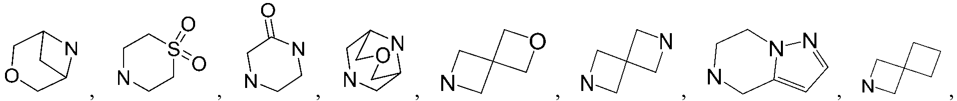

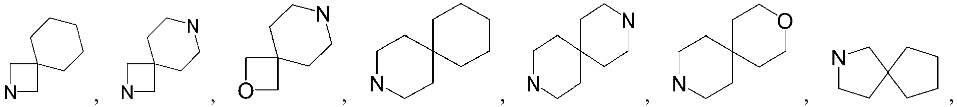

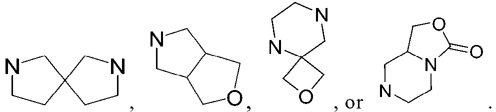

In some embodiments of the present invention, Y1 is 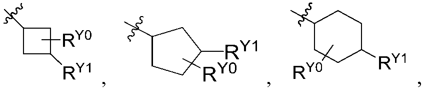 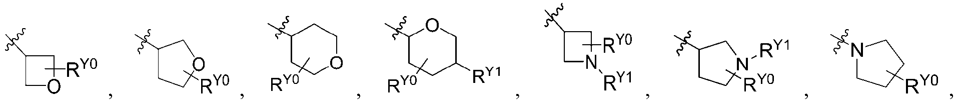 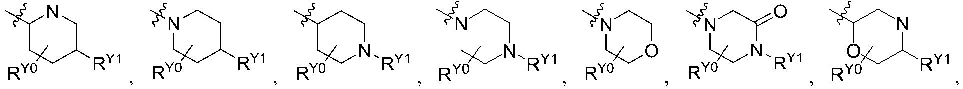 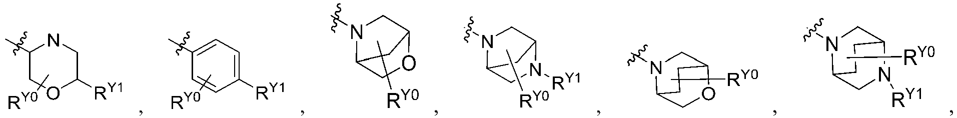 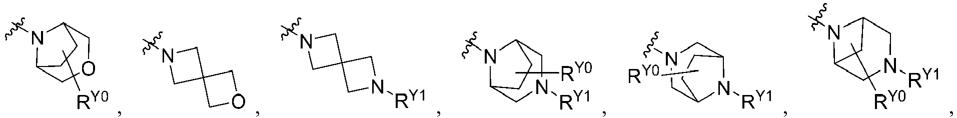 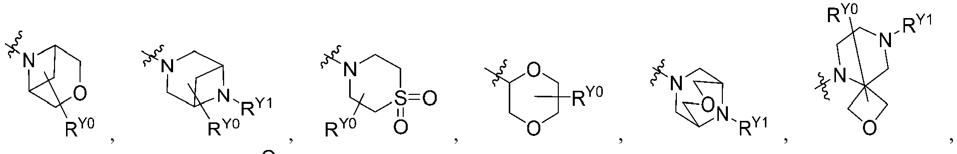 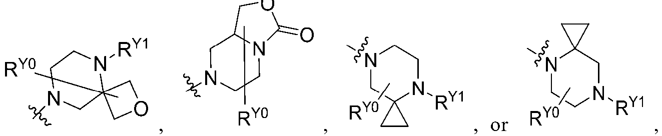 in particular,

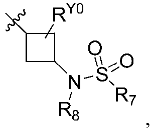

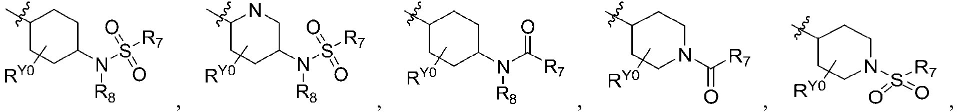

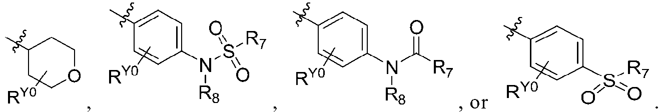

In some embodiments of the present invention, in Y1, R^{Y0} is selected from the group consisting of: H, halogen (for example, F), C₁-C₃ alkyl (for example, methyl), halogenated C₁-C₃ alkyl, hydroxyl, and C₁-C₃ alkoxy.

In some embodiments of the present invention, R^{Y1} is selected from the group consisting of: -NR₈SO₂R₇, - NR₈C(O)OR₇, -C(O)R₇, -C(O)OR₇, -C(O)NR₇R₈, -S(O)₂R₇, C₁-C₆ alkyl, and -(C₀-C₃ alkylene)(4-10-membered heterocyclyl), wherein the C₁-C₆ alkyl or 4-10-membered heterocyclyl may be optionally substituted with one or more groups selected from the group consisting of: halogen, cyano, hydroxyl, amino, C₁-C₆ alkyl, halogenated C₁-C₆ alkyl, C₁-C₆ alkoxy, C₁-C₆ alkylamino, halogenated C₁-C₆ alkoxy, hydroxyl-substituted C₁-C₆ alkyl, -C(O)N(C₀-C₆ alkyl)(C₀-C₆ alkyl), and -N(C₀-C₆ alkyl)C(O)(C₀-C₆ alkyl).

In some embodiments of the present invention, R₇ is selected from the group consisting of: H, C₁-C₆ alkyl (for example, methyl, ethyl, n-propyl, or isopropyl), halogenated C₁-C₆ alkyl, hydroxyl-substituted C₁-C₆ alkyl, -(C₀-C₃ alkylene)-(C₃-C₆ cycloalkyl) (for example, 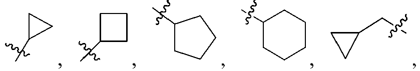 or 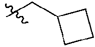 ), and -(C₀-C₃ alkylene)-(4-10-membered heterocyclyl) (the 4-10-membered heterocyclyl is, for example, 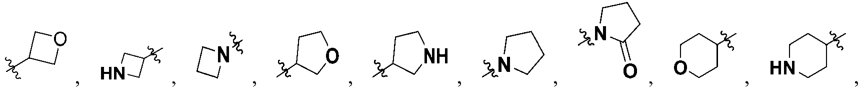 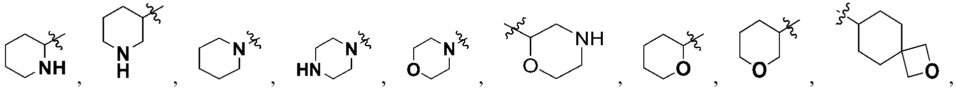 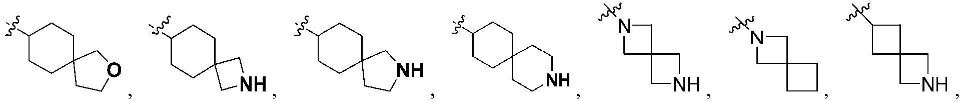 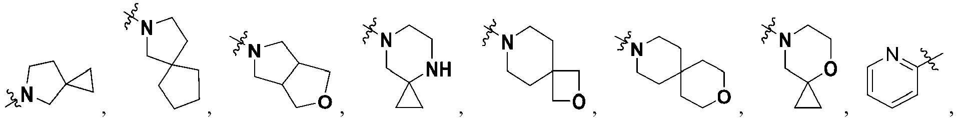 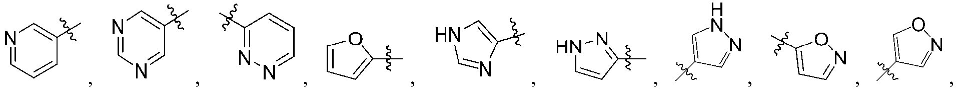 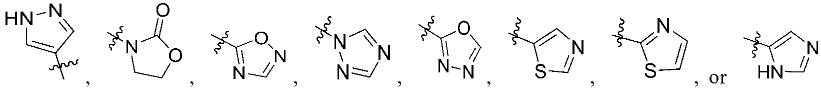 ), wherein the C₁-C₆ alkyl, C₃-C₆ cycloalkyl, or 4-10-membered heterocyclyl may be optionally substituted with one or more groups selected from the group consisting of: halogen, cyano, hydroxyl, C₁-C₆ alkyl, halogenated C₁-C₆ alkyl, C₁-C₆ alkoxy, halogenated C₁-C₆ alkoxy, hydroxyl-substituted C₁-C₆ alkyl, -C(O)N(C₀-C₆ alkyl)( C₀-C₆ alkyl), and -N(C₀-C₆ alkyl)C(O)(C₀-C₆ alkyl).

In some embodiments of the present invention, R₇ is selected from the group consisting of: C₁-C₆ alkyl, C₃-C₆ cycloalkyl, -(C₁-C₃ alkylene)-(C₃-C₆ cycloalkyl), 4-10-membered heterocyclyl, and -(C₁-C₃ alkylene)-(4-10-membered heterocyclyl).

In some embodiments of the present invention, R₈ is selected from H and C₁₋₆ alkyl (for example, methyl). In some embodiments of the present invention, R₈ is H.

In some embodiments of the present invention, R^{Y1} is -NR₈SO₂R₇, wherein R₇ is selected from the group consisting of: H, C₁-C₆ alkyl (for example, methyl, ethyl, n-propyl, or isopropyl), halogenated C₁-C₆ alkyl, hydroxyl-substituted C₁-C₆ alkyl, C₃-C₆ cycloalkyl (for example, 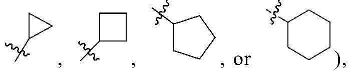 (C₁-C₃ alkylene)-(C₃-C₆ cycloalkyl) (for example, 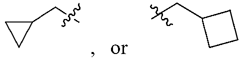 ), and 4-10-membered heterocyclyl (for example, 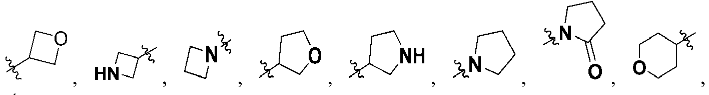 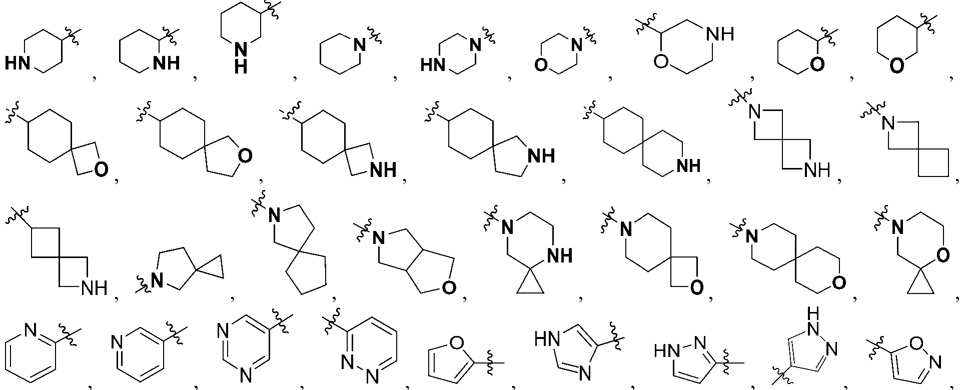 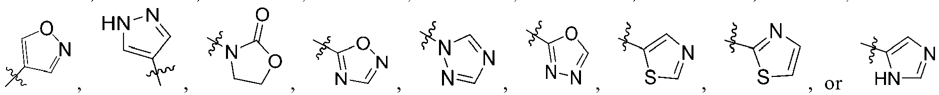 ), wherein the 4-10-membered heterocyclyl may be optionally substituted with one or more groups selected from the group consisting of: halogen, cyano, hydroxyl, amino, C₁-C₆ alkyl, halogenated C₁-C₆ alkyl, C₁-C₆ alkoxy, C₁-C₆ alkylamino, halogenated C₁-C₆ alkoxy, hydroxyl-substituted C₁-C₆ alkyl, -C(O)N(C₀-C₆ alkyl)( C₀-C₆ alkyl), and -N(C₀-C₆ alkyl)C(O)( C₀-C₆ alkyl); and R₈ is selected from the group consisting of: H and C₁-C₆ alkyl (for example, methyl).

In some embodiments of the present invention, R^{Y1} selected from the group consisting of:

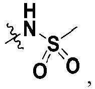

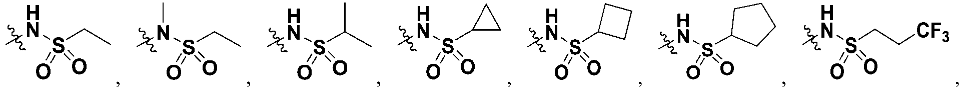

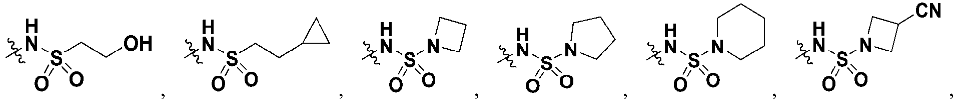

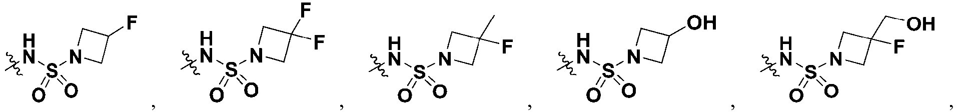

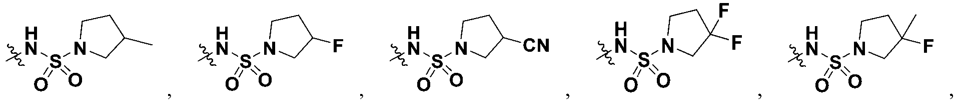

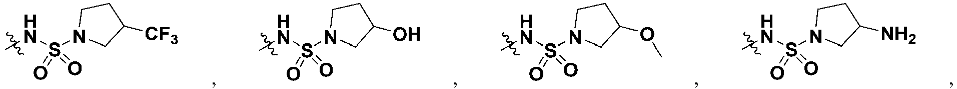

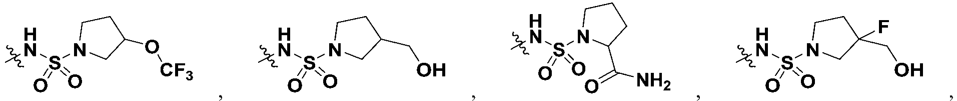

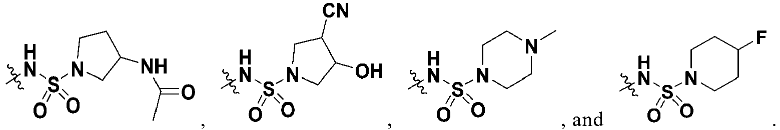

In some embodiments of the present invention, R^{Y1} is -NR₈C(O)OR₇, wherein R₇ is selected from the group consisting of: H and C₁-C₆ alkyl (for example, methyl, ethyl, n-propyl, isopropyl, or tert-butyl), and R₈ is selected from the group consisting of: H and C₁-C₆ alkyl (for example, methyl).

In some embodiments of the present invention, R^{Y1} is selected from the group consisting of:

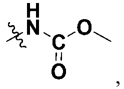

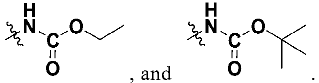

In some embodiments of the present invention, R^{Y1} is -NR₈C(O)R₇, wherein R₇ is selected from the group consisting of: H, C₁-C₆ alkyl (for example, methyl, ethyl, n-propyl, isopropyl, or tert-butyl), and halogenated C₁-C₆ alkyl, and R₈ is selected from the group consisting of: H, C₁-C₆ alkyl (for example, methyl).

In some embodiments of the present invention, R^{Y1} is selected from the group consisting of:

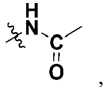

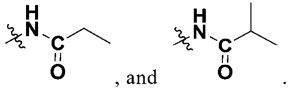

In some embodiments of the present invention, R^{Y1} is -C(O)R₇, wherein R₇ is selected from the group consisting of: H, C₁-C₆ alkyl (for example, methyl, or ethyl), -(C₀-C₃ alkylene)-(C₃-C₆ cycloalkyl) (for example, 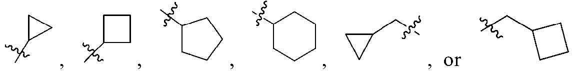 ), and -(C₀-C₃ alkylene)-(4-10-membered heterocyclyl) (the 4-10-membered heterocyclyl is, for example, 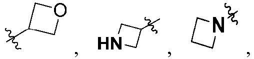 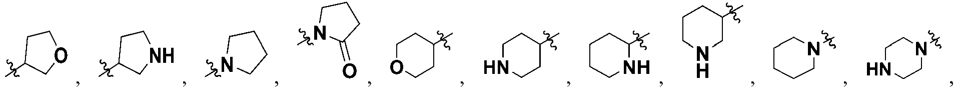 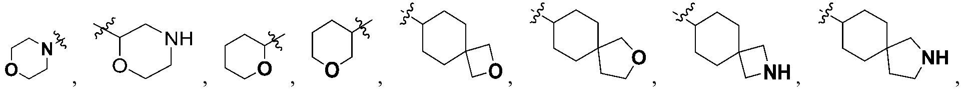 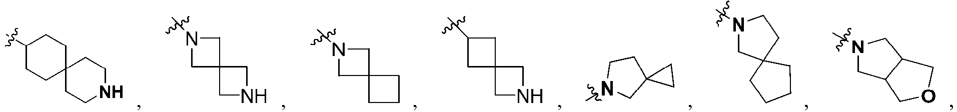 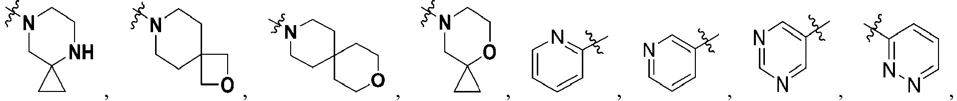 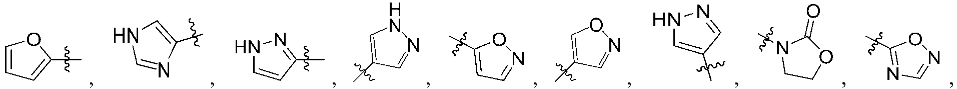 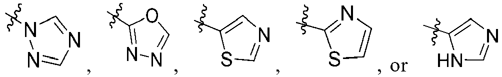 ), wherein the C₁-C₆ alkyl, C₃-C₆ cycloalkyl, or 4-10-membered heterocyclyl may be optionally substituted with one or more groups selected from the group consisting of: halogen, cyano, hydroxyl, C₁-C₆ alkyl, C₁-C₆ alkoxy, amino, and C₁-C₆ alkylamino.

In some embodiments of the present invention, R^{Y1} is selected from the group consisting of:

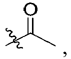

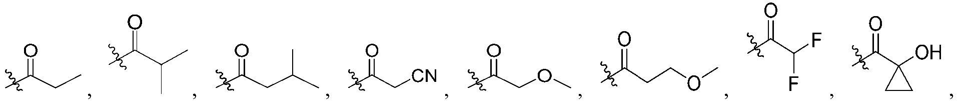

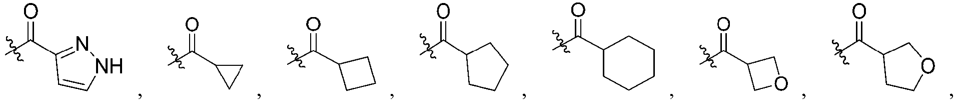

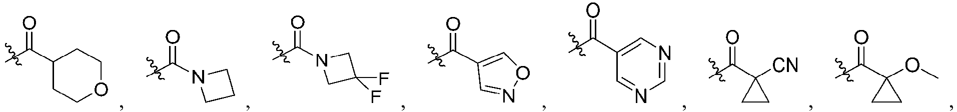

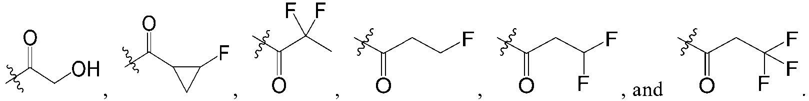

In some embodiments of the present invention, R^{Y1} is -C(O)R₇, wherein R₇ is selected from the group consisting of: H and C₁-C₆ alkyl (for example, methyl, or ethyl).

In some embodiments of the present invention, R^{Y1} is selected from the group consisting of: 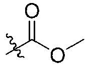 and

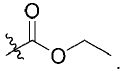

In some embodiments of the present invention, R^{Y1} is -C(O)NR₇R₈, wherein R₇ is selected from the group consisting of: H, C₁-C₆ alkyl (for example, methyl, or ethyl), and C₃-C₆ cycloalkyl (for example, cyclopropyl), and R₈ is selected from H and C₁-C₆ alkyl (for example, methyl).

In some embodiments of the present invention, R^{Y1} is selected from the group consisting of:

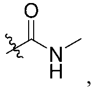

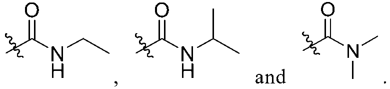

In some embodiments of the present invention, R^{Y1} is -S(O)₂R₇, wherein R₇ is selected from the group consisting of: H, C₁-C₆ alkyl (for example, methyl, or ethyl), and C₃-C₆ cycloalkyl (for example, cyclopropyl).

In some embodiments of the present invention, R^{Y1} is selected from the group consisting of:

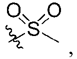

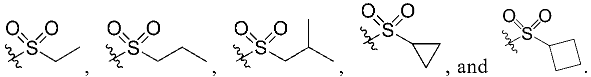

In some embodiments of the present invention, R^{Y1} is -(C₀-C₃ alkylene)(4-10-membered heterocyclyl), and the 4-10-membered heterocyclyl is, for example, , 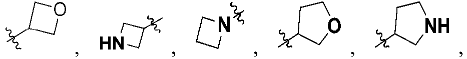 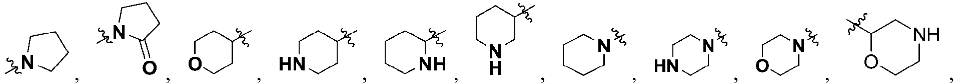 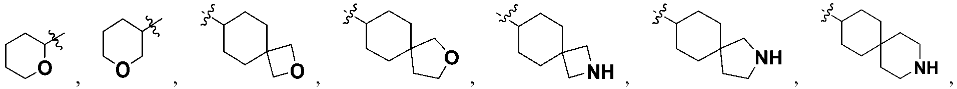 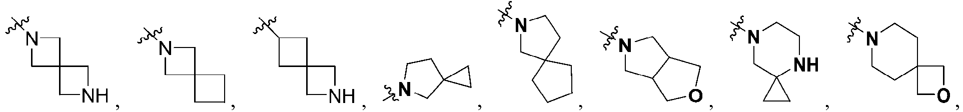 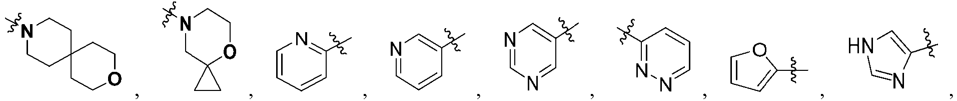 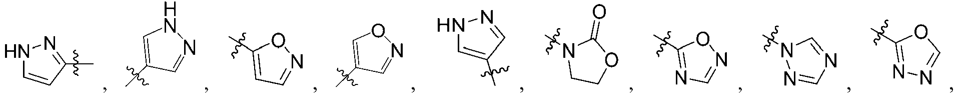 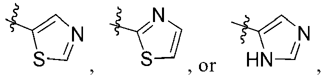 and the 4-10-membered heterocyclyl may be optionally substituted with one or more groups selected from the group consisting of: halogen, cyano, hydroxyl, C₁-C₆ alkyl, C₁-C₆ alkoxy, amino, and C₁-C₆ alkylamino.

In some embodiments of the present invention, R^{Y1} is selected from the group consisting of:

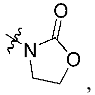

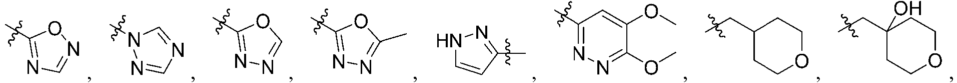

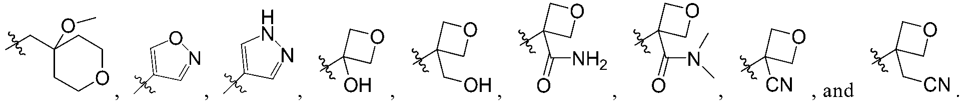

In some embodiments of the present invention, R^{Y1} is selected from the group consisting of: H, C₁-C₆ alkyl, halogenated C₁-C₆ alkyl, hydroxyl-substituted C₁-C₆ alkyl, C₁-C₆ alkoxyalkyl, -O-(hydroxyl-substituted C₁-C₆ alkyl), -O-(C₁-C₆ alkoxyalkyl), and C₁-C₆ cyanoalkyl.

In some embodiments of the present invention, Y1 is selected from the group consisting of:

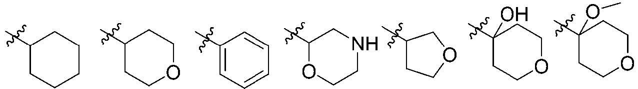

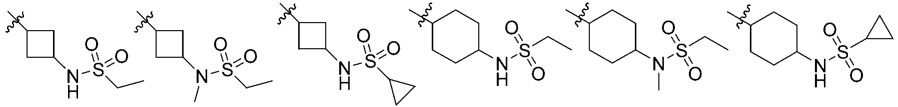

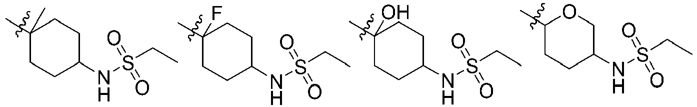

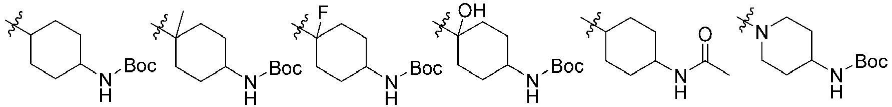

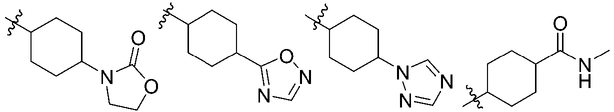

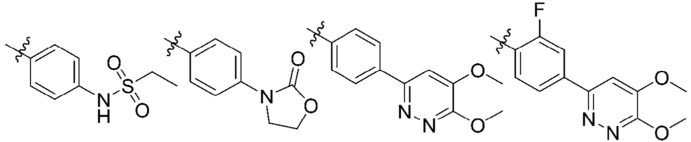

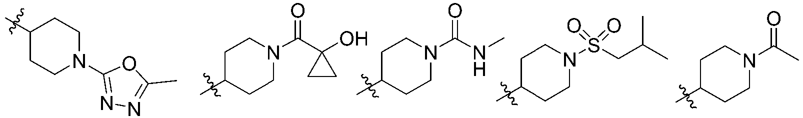

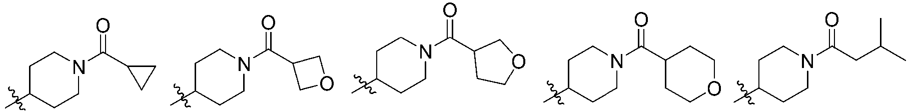

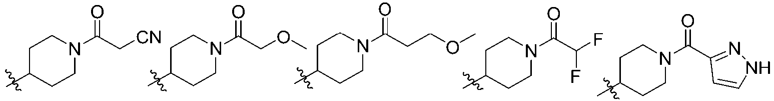

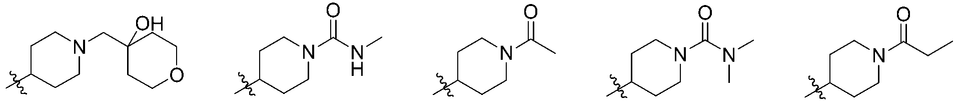

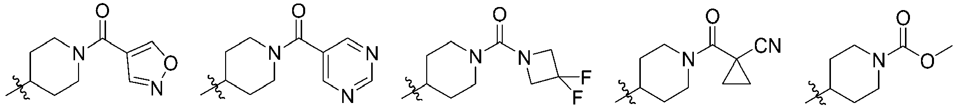

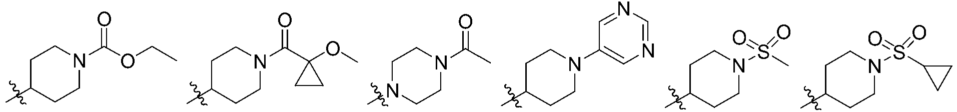

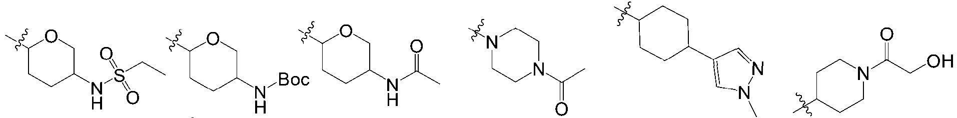

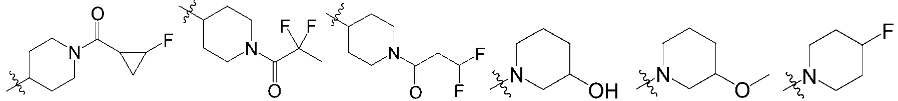

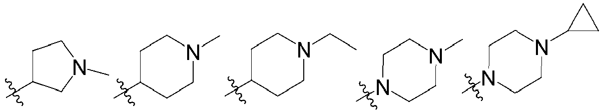

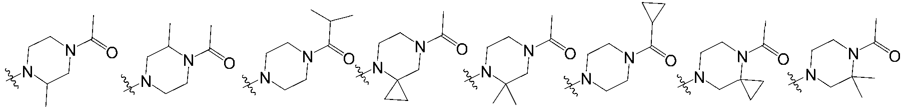

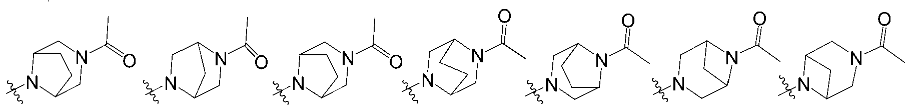

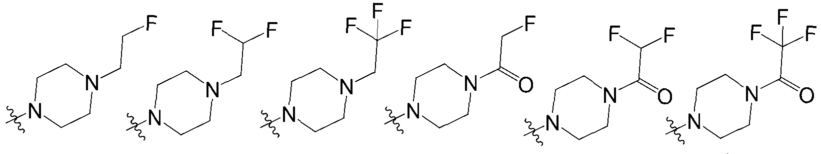

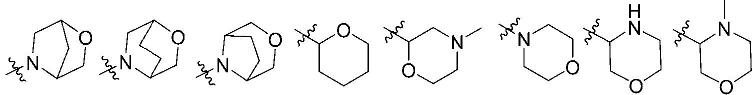

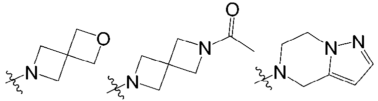

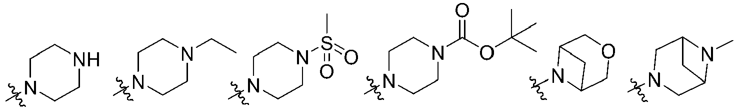

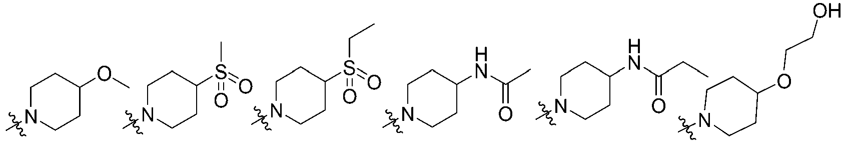

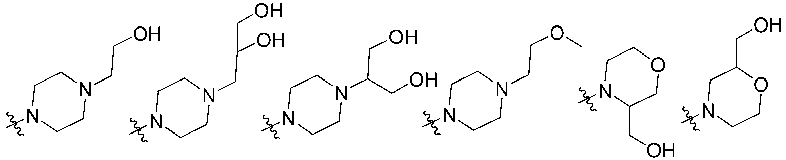

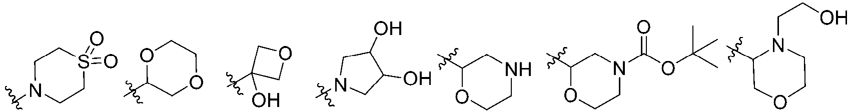

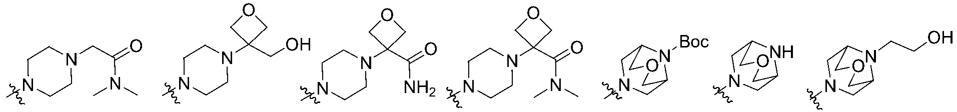

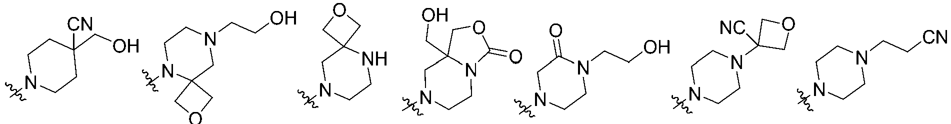

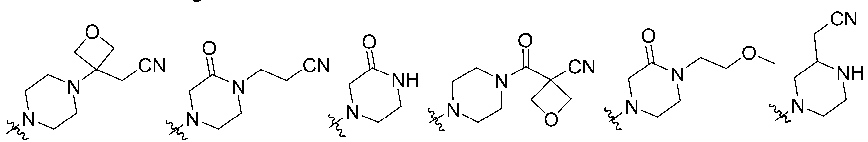

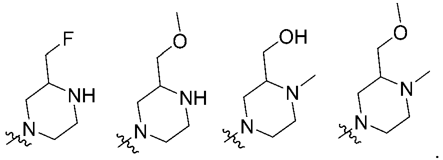

In some embodiments of the present invention, L₂ is C₁-C₆ alkylene (for example, methylene, 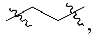 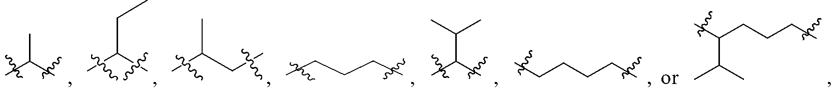 in particular, straight-chain alkylene, for example, methylene, or 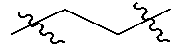 ), alkoxy-substituted C₁-C₆ alkylene (for example, 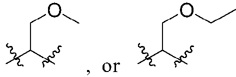 ) or -N(C₀-C₃ alkyl)-(C₀-C₆ alkylene)- (for example, 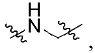 or 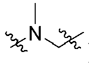 ), or -(C₀-C₆ alkylene)-O- (for example, 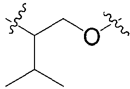 ), and Y is Y1.

In some embodiments of the present invention, the 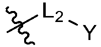 moiety is selected from the group consisting of:

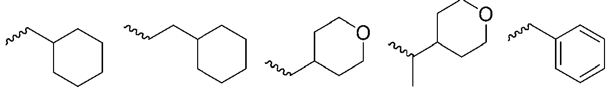

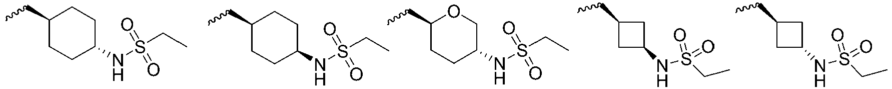

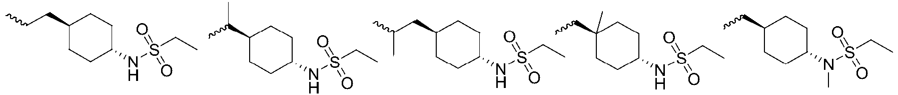

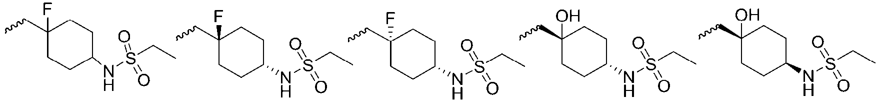

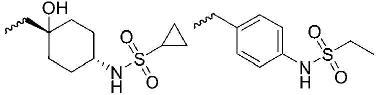

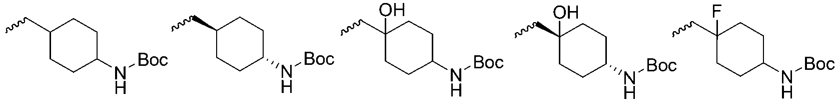

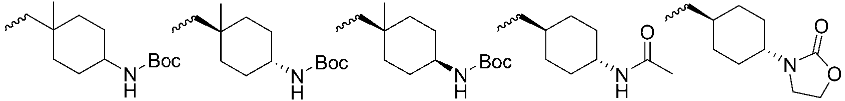

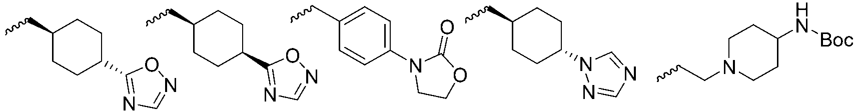

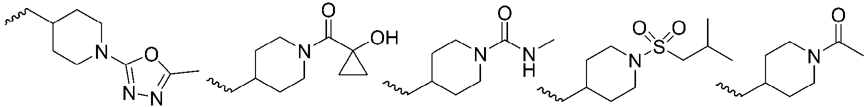

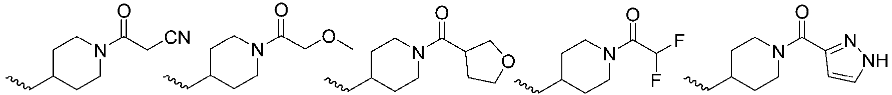

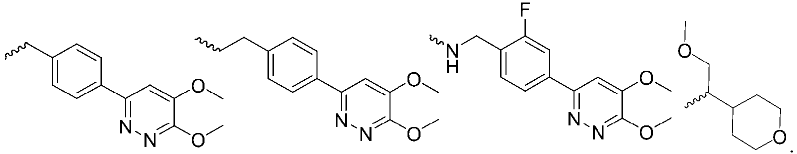

In some embodiments of the present invention, Y2 is 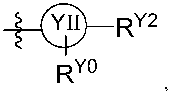 wherein Ring YII is a benzene ring-fused four- to eight-membered aliphatic ring, a benzene ring-fused four- to eight-membered heterocyclic ring, or a bicyclic heterocyclic ring, and R^{Y0} is one or more independent substituents on a ring, and is defined as above in the present invention.

In some embodiments of the present invention, Ring YII is

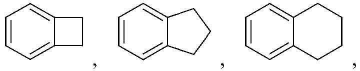

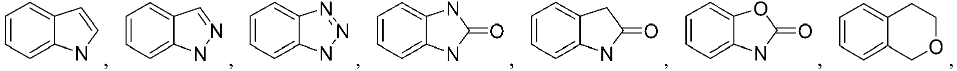

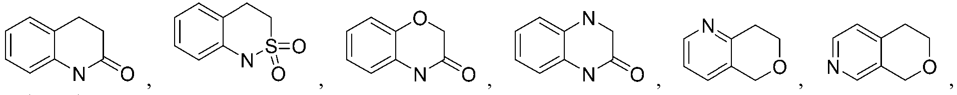

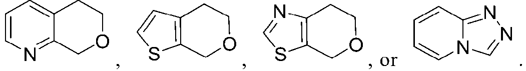

In some embodiments of the present invention, Y2 is

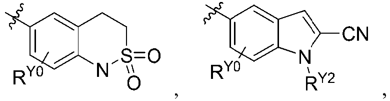

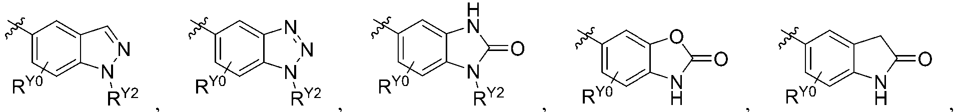

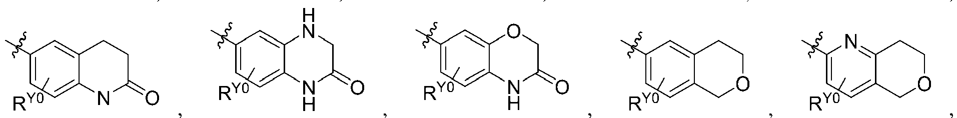

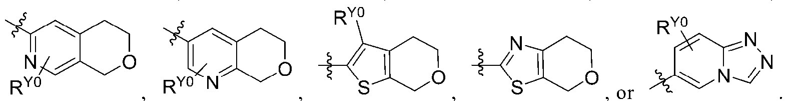

In some embodiments of the present invention, in Y2, R^{Y0} is selected from the group consisting of: H, halogen (for example, F), C₁-C₃ alkyl (for example, methyl), halogenated C₁-C₃ alkyl, hydroxyl, and C₁-C₃ alkoxy, in particular, H or methyl.

In some embodiments of the present invention, R^{Y2} is selected from the group consisting of: H, -(C₁-C₄ alkylene)-OH, -(C₁-C₄ alkylene)-O-(C₁-C₄ alkyl), -(C₀-C₄ alkylene)-(C₃-C₆ cycloalkyl), and -(C₀-C₄ alkylene)-(optionally substituted 4-10-membered heterocyclyl); and the 4-10-membered heterocyclyl is 4-6-membered saturated heterocyclyl, for example,

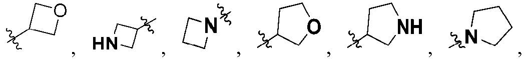

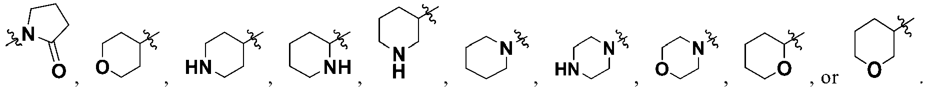

In some embodiments of the present invention, Y2 is selected from the group consisting of:

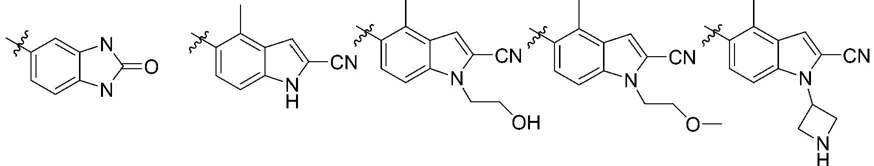

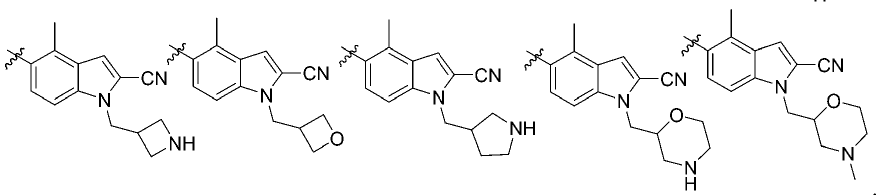

In some embodiments of the present invention, L₂ is C₁-C₆ alkylene (for example, methylene, 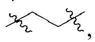 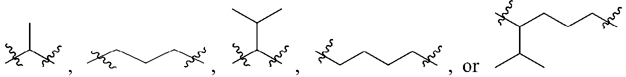 ), alkoxy-substituted C₁-C₆ alkylene (for example, 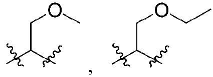 ) or -N(C₀-C₃ alkyl)-(C₀-C₆ alkylene)- (for example, 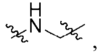 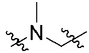 ), and Y is Y2.

In some embodiments of the present invention, Y3 is 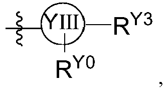 wherein Ring YIII is a four- to eight-membered monocyclic nitrogenous saturated heterocyclic ring, a four- to eight-membered monocyclic nitrogenous saturated heterocyclic ring-fused benzene ring, a four- to eight-membered monocyclic nitrogenous saturated heterocyclic ring-fused four- to eight-membered aliphatic ring, or a six- to tenmembered bridged nitrogenous saturated heterocyclic ring, and R^{Y0} is one or more independent substituents on the ring, and is defined as above in the present invention.

In some embodiments of the present invention, Ring YIII is 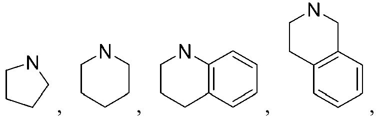 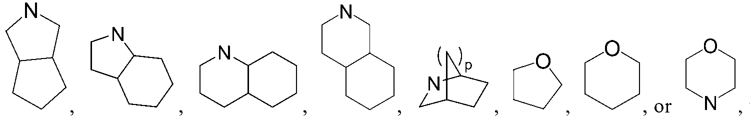 wherein p is 1 or 2.

In some embodiments of the present invention, Y3 is 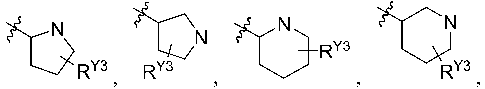 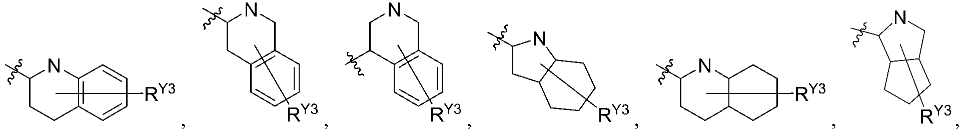 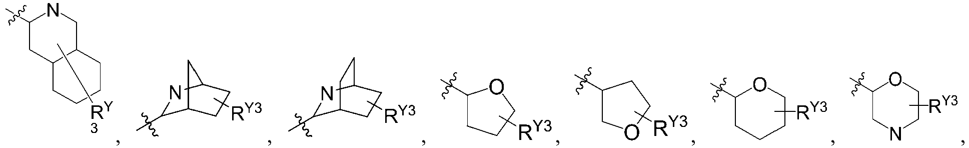 or

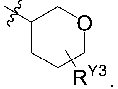

In some embodiments of the present invention, in Y3, R^{Y0} is selected from the group consisting of: H, halogen (for example, F), C₁-C₃ alkyl (for example, methyl), halogenated C₁-C₃ alkyl, hydroxyl, and C₁-C₃ alkoxy, in particular H.

In some embodiments of the present invention, R^{Y3} is selected from the group consisting of: H, 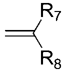 (for example, 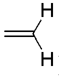 ), halogen (for example, F), C₁-C₆ alkyl (for example, methyl), C₁-C₆ alkoxy (for example, methoxy), phenyl, and halogenated phenyl (for example, 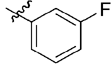 ).

In some embodiments of the present invention, Y3 is selected from the group consisting of:

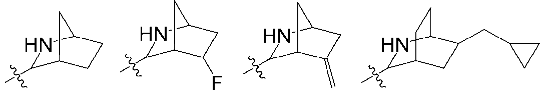

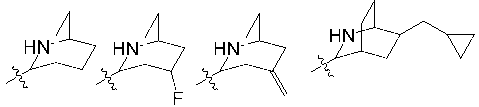

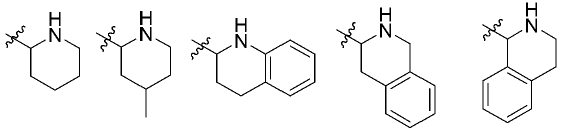

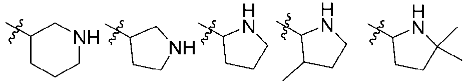

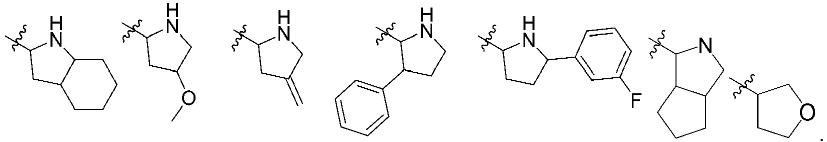

In some embodiments of the present invention, L₂ is C(O), and Y is Y3.

In some embodiments of the present invention, the 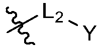 moiety is selected from the group consisting of:

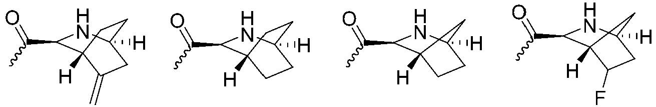

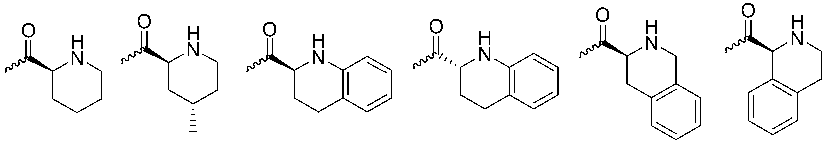

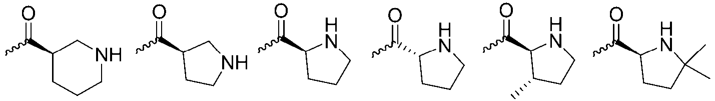

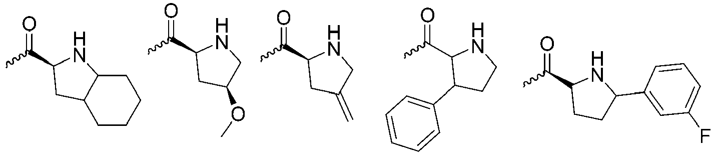

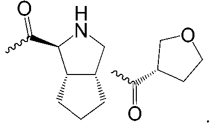

In some embodiments of the present invention, Y4 is 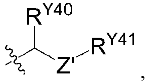 wherein,
R^{Y40} is selected from the group consisting of: C₁-C₆ alkyl, halogenated C₁-C₆ alkyl, C₃-C₆ cycloalkyl, 3-6-membered heterocyclyl, and C₁-C₃ alkyl-substituted 3-6-membered heterocyclyl;
Z' is selected from the group consisting of: C₀-C₆ alkylene, -(C₀-C₃ alkylene)-O-(C₀-C₃ alkylene)-, -(C₀-C₃ alkylene)-S-(C₀-C₃ alkylene)-, -(C₀-C₃ alkylene)-NH-(C₀-C₃ alkylene)-, -(C₀-C₃ alkylene)-C(O)NH-(C₀-C₃ alkylene)-, -(C₀-C₃ alkylene)-NHC(O)-(C₀-C₃ alkylene)-, and C₃-C₆ cycloalkylene; wherein H in said alkylene is optionally substituted with one or more groups selected from the group consisting of: halogen, cyano, hydroxyl, and amino;
R^{Y41} is selected from the group consisting of: H, halogen, cyano, C₁-C₆ alkyl, C₃-C₁₀ cycloalkyl, C₆-C₁₀ aryl, 4-12-membered heterocyclyl, -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -S(C₀₋₁₀ alkyl), -SO(C₀₋₁₀ alkyl), - SO₂(C₀₋₁₀ alkyl), -SO₂N(C₀₋₁₀ alkyl)(C₀₋₁₀ alkyl), -COO(C₀₋₁₀ alkyl), -OCO(C₀₋₁₀ alkyl), -CON(C₀₋₁₀ alkyl)(C₀₋₁₀ alkyl), and -CO(C₀₋₁₀ alkyl); wherein H in the alkyl, cycloalkyl, aryl, or heterocyclyl is optionally substituted with a group selected from the group consisting of: halogen, cyano, C₁-C₆ alkyl, C₃-C₁₀ cycloalkyl, 4-12-membered heterocyclyl, -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -S(C₀₋₁₀ alkyl), -SO(C₀₋₁₀ alkyl), - SO₂(C₀₋₁₀ alkyl), -SO₂N(C₀₋₁₀ alkyl)(C₀₋₁₀ alkyl), -COO(C₀₋₁₀ alkyl), -OCO(C₀₋₁₀ alkyl), -CON(C₀₋₁₀ alkyl)(C₀₋₁₀ alkyl), -CO(C₀₋₁₀ alkyl), -N(C₀₋₁₀ alkyl)(4-10-membered heterocyclyl), -N(C₀₋₁₀ alkyl)CO(4-10-membered heterocyclyl), -N(C₀₋₁₀ alkyl)CON(4-10-membered heterocyclyl), -N(C₀₋₁₀ alkyl)SO₂(4-10-membered heterocyclyl), -O(4-10-membered heterocyclyl), -S(4-10-membered heterocyclyl), -SO(4-10-membered heterocyclyl), -SO₂(4-10-membered heterocyclyl), -SO₂N(C₀₋₁₀ alkyl)( 4-10-membered heterocyclyl), -COO(4-10-membered heterocyclyl), -OCO(4-10-membered heterocyclyl), -CON(C₀₋₁₀ alkyl)( 4-10-membered heterocyclyl), and -CO(4-10-membered heterocyclyl), wherein H in the alkyl or heterocyclyl is optionally substituted with one or more groups selected from the group consisting of: halogen, cyano, C₁-C₆ alkyl, halogenated C₁-C₆ alkyl, C₁-C₆ hydroxyl alkyl, C₁-C₆ alkoxyalkyl, -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), - O(C₀₋₁₀ alkyl), -S(C₀₋₁₀ alkyl), -SO(C₀₋₁₀ alkyl), -SO₂(C₀₋₁₀ alkyl), -SO₂N(C₀₋₁₀ alkyl)(C₀₋₁₀ alkyl), -COO(C₀₋₁₀ alkyl), -OCO(C₀₋₁₀ alkyl), -CON(C₀₋₁₀ alkyl)(C₀₋₁₀ alkyl), and -CO(C₀₋₁₀ alkyl).

In some embodiments of the present invention, R^{Y40} is C₁-C₄ alkyl or halogenated C₁-C₄ alkyl, for example, 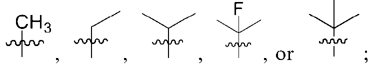 in some embodiments of the present invention, R^{Y40} is optionally substituted with C₁-C₃ alkyl-substituted 3-6-membered heterocyclyl, for example, 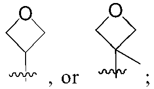 and in some embodiments of the present invention, R^{Y40} is C₃-C₆ cycloalkyl, for example, cyclopropyl, or cyclobutyl. In some embodiments of the present invention, Z' is a single bond or C₁-C₆ alkylene, in particular straight-chain C₁-C₄ alkylene, wherein H in the alkylene is optionally substituted with one or more groups selected from the group consisting of: halogen, cyano, and hydroxyl. In some embodiments of the present invention, Z' is C₃-C₆ cycloalkylene, for example,

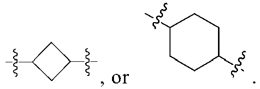

In some embodiments of the present invention, Y4 is 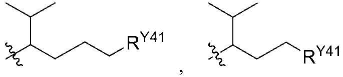 or 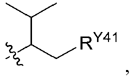 in particular,

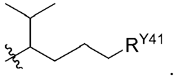

In some embodiments of the present invention, R^{Y41} is selected from the group consisting of: -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -COO(C₀₋₁₀ alkyl), -OCO(C₀₋₁₀ alkyl), and -CON(C₀₋₁₀ alkyl)(C₀₋₁₀ alkyl); wherein H in the alkyl is optionally substituted with one or more groups selected from the group consisting of: halogen, cyano, hydroxyl, C₁-C₆ alkoxy; more specifically, R^{Y41} is selected from the group consisting of: -N(C₀₋₁₀ alkyl)(C₀₋₁₀ alkyl), -O(C₀₋₁₀ alkyl), and -COO(C₀₋₁₀ alkyl); wherein H in the alkyl is optionally substituted with one or more groups selected from the group consisting of: hydroxyl and C₁-C₃ alkoxy. In some embodiments of the present invention, R^{Y41} is selected from the group consisting of: OH,

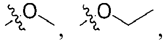

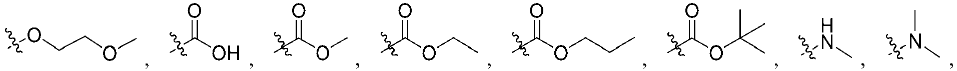

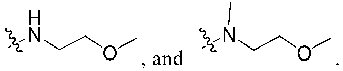

In some embodiments of the present invention, R^{Y41} is 4-12-membered heterocyclyl, H in the heterocyclyl is optionally substituted with one or more R^{Y42}, with each R^{Y42} independently selected from the group consisting of: H, C₁-C₆ alkyl, C₁-C₆ halogenated alkyl, C₁-C₆ hydroxylalkyl, C₁-C₆ alkoxyalkyl, C₃-C₆ cycloalkyl, 4-6-membered saturated heterocyclyl, -N(C₀₋₆ alkyl)(C₀₋₆ alkyl), -N(C₀₋₆ alkyl)CO(C₀₋₆ alkyl), -N(C₀₋₆ alkyl)CON(C₀₋₆ alkyl), -N(C₀₋₆ alkyl)SO₂(C₀₋₆ alkyl), -O(C₀₋₆ alkyl), -S(C₀₋₆ alkyl), -SO(C₀₋₆ alkyl), -SO₂(C₀₋₆ alkyl), -SO₂N(C₀₋₆ alkyl)(C₀₋₆ alkyl), -COO(C₀₋₆ alkyl), -OCO(C₀₋₆ alkyl), -CON(C₀₋₆ alkyl)(C₀₋₆ alkyl), - CO(C₀₋₆ alkyl), -N(C₀₋₆ alkyl)(4-6-membered heterocyclyl), -SO₂(4-6-membered heterocyclyl), -COO(4-6-membered heterocyclyl), -OCO(4-6-membered heterocyclyl), -CON(C₀₋₆ alkyl)( 4-6-membered heterocyclyl), and -CO(4-6-membered heterocyclyl); wherein H in the alkyl or heterocyclyl is optionally substituted with one or more groups selected from the group consisting of: halogen, cyano, hydroxyl, C₁-C₆ alkyl, halogenated C₁-C₆ alkyl, C₁-C₆ hydroxylalkyl, C₁-C₆ alkoxyalkyl, C₁-C₆ cyanoalkyl, -N(C₀₋₆ alkyl)(C₀₋₆ alkyl), -N(C₀₋₆ alkyl)CO(C₀₋₆ alkyl), -N(C₀₋₆ alkyl)CON(C₀₋₆ alkyl), -N(C₀₋₆ alkyl)SO₂(C₀₋₆ alkyl), -O(C₀₋₆ alkyl), -S(C₀₋₆ alkyl), -SO(C₀₋₆ alkyl), -SO₂(C₀₋₆ alkyl), -SO₂N(C₀₋₆ alkyl)(C₀₋₆ alkyl), -COO(C₀₋₆ alkyl), -OCO(C₀₋₆ alkyl), -CON(C₀₋₆ alkyl)(C₀₋₆ alkyl), and -CO(C₀₋₆ alkyl).

Specifically, in the definition of R^{Y41}, the 4-14-membered heterocyclyl is saturated or partially saturated heterocyclyl, for example, 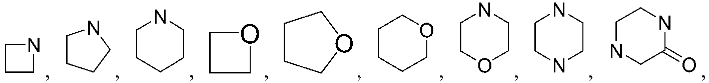 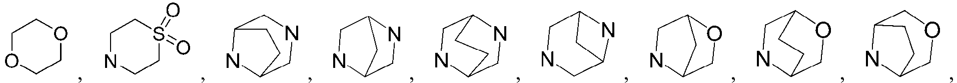 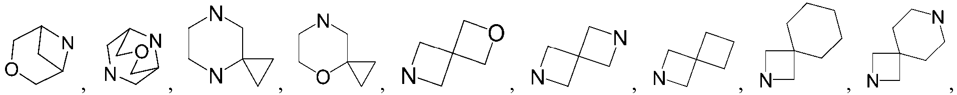 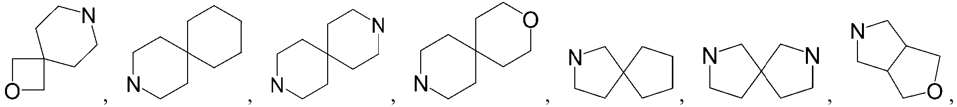 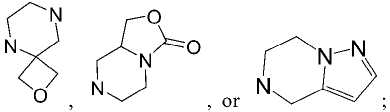 and more specifically, R^{Y41} may be selected from the group consisting of:

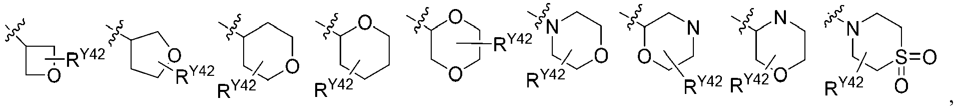

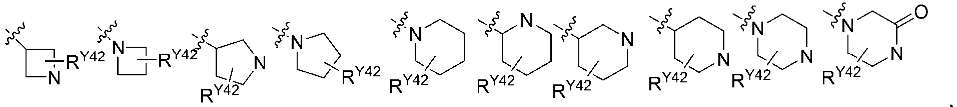

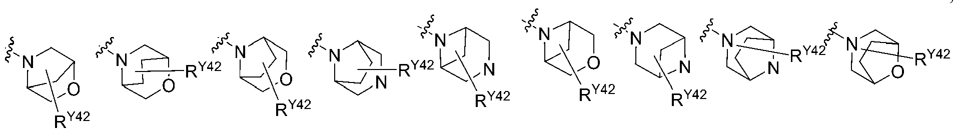

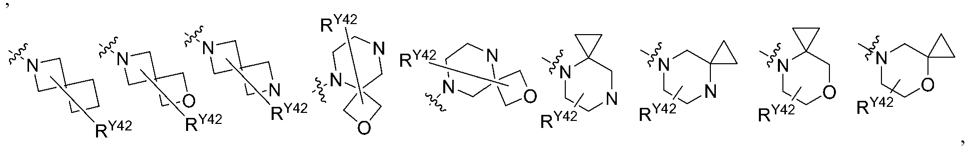

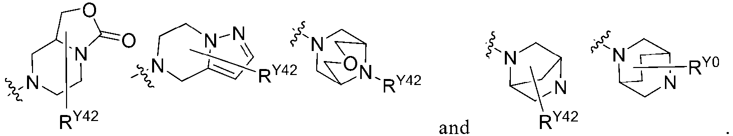

In some embodiments of the present invention, each R^{Y42} is independently selected from the group consisting of: H, halogen, 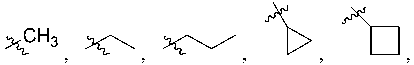 CF₃, CHF₂, CH₂F, 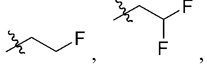 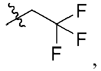 hydroxyl, 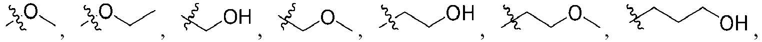 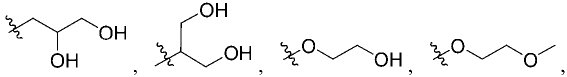 cyano,

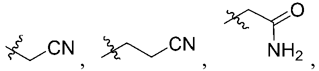

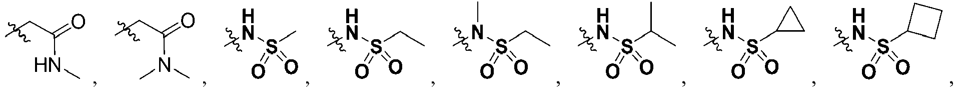

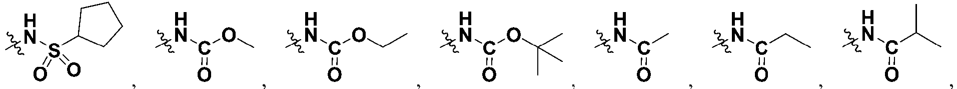

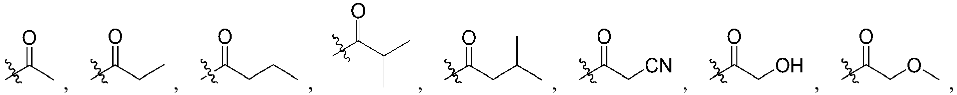

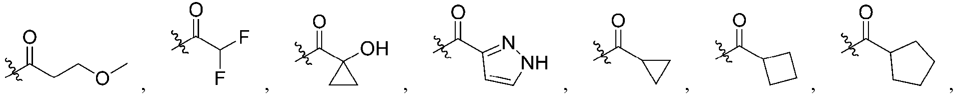

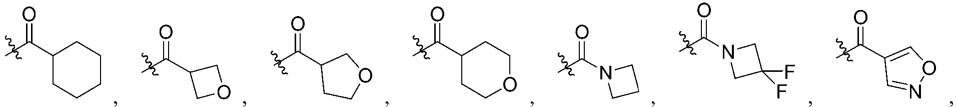

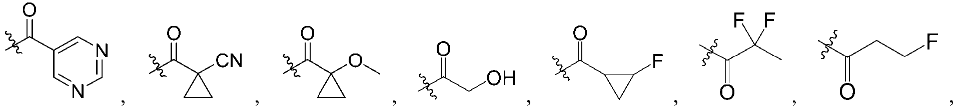

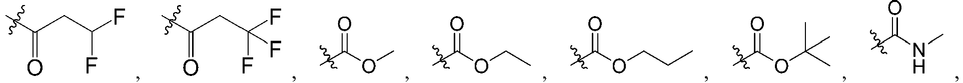

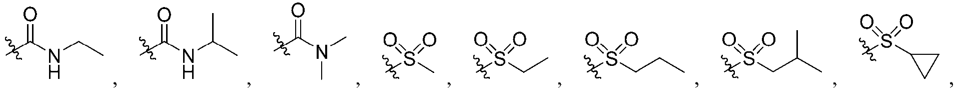

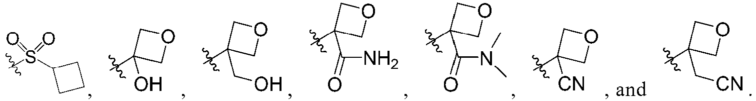

In some embodiments of the present invention, Y4 is 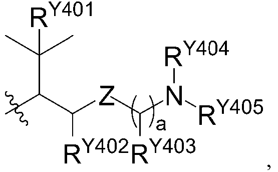 wherein,
R^{Y401} is selected from the group consisting of: H, halogen (for example, F), C₁-C₃ alkyl, and C₁-C₃ halogenated alkyl;
R^{Y402} and R^{Y413} are independently selected from the group consisting of: H, halogen (for example, F), hydroxyl, amino, C₁-C₃ alkyl, halogenated C₁-C₃ alkyl, and -(C₀-C₃ alkylene)-(C₃-C₆ cycloalkyl), wherein the alkyl or cycloalkyl may be optionally substituted with one or more groups selected from the group consisting of: halogen, cyano, hydroxyl, amino, C₁-C₆ alkoxy, and C₁-C₆ alkylamino; or, R^{Y402} and R^{Y403}, together with the carbon atom attached thereto, form an aliphatic ring or a heterocyclic ring, wherein the aliphatic ring or heterocyclic ring may be optionally substituted with one or more groups selected from the group consisting of: halogen, cyano, hydroxyl, amino, C₁-C₆ alkyl, and halogenated C₁-C₆ alkyl;
R^{Y404} and R^{Y405} are independently selected from the group consisting of: H and C₁-C₆ alkyl, wherein the alkyl may be optionally substituted with one or more groups selected from the group consisting of: halogen, cyano, hydroxyl, amino, C₁-C₆ alkoxy, and C₁-C₆ alkylamino; or, R^{Y404} and R^{Y405}, together with the nitrogen atom attached thereto, form a heterocyclic ring, or, R^{Y402} and R^{Y404}, together with the nitrogen or nitrogen atom attached thereto, form a heterocyclic ring, R^{Y403} and R^{Y404}, together with the carbon or nitrogen atom attached thereto, form a heterocyclic ring, wherein the heterocyclic ring may be optionally substituted with one or more groups selected from the group consisting of: halogen, cyano, nitro, azido, C₁-C₁₀ alkyl, C₂-C₁₀ alkenyl, C₂-C₁₀ alkynyl, -(C₀-C₆ alkylene)-(C₃-C₁₀ cycloalkyl), -(C₀-C₆ alkylene)-(phenyl), -(C₀-C₆ alkylene)-(4-10-membered heterocyclyl), halogenated C₁-C₁₀ alkyl, halogenated C₁-C₁₀ alkoxy, -N(C₀₋₁₀ alkyl)(C₀₋₁₀ alkyl), - N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -S(C₀₋₁₀ alkyl), -SO(C₀₋₁₀ alkyl), -SO₂(C₀₋₁₀ alkyl), -SO₂N(C₀₋₁₀ alkyl)(C₀₋₁₀ alkyl), -COO(C₀₋₁₀ alkyl), -OCO(C₀₋₁₀ alkyl), -CON(C₀₋₁₀ alkyl)(C₀₋₁₀ alkyl), -CO(C₀₋₁₀ alkyl), and -CO(C₃-C₁₀ cycloalkyl); wherein H in the C₀₋₁₀ alkyl, C₁-C₁₀ alkyl, C₂-C₁₀ alkenyl, C₂-C₁₀ alkynyl, C₀-C₆ alkylene, C₃-C₁₀ cycloalkyl, phenyl, or 4-10-membered heterocyclyl is optionally substituted with one or more groups selected from the group consisting of: halogen, cyano, nitro, azido, C₁-C₁₀ alkyl, halogenated C₁-C₁₀ alkyl, halogenated C₁-C₁₀ alkoxy, -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -S(C₀₋₁₀ alkyl), -SO(C₀₋₁₀ alkyl), -SO₂(C₀₋₁₀ alkyl), -SO₂N(C₀₋₁₀ alkyl)(C₀₋₁₀ alkyl), -COO(C₀₋₁₀ alkyl), -OCO(C₀₋₁₀ alkyl), -CON(C₀₋₁₀ alkyl)(C₀₋₁₀ alkyl), and -CO(C₀₋₁₀ alkyl); Z is selected from the group consisting of: C₀-C₆ alkylene, -(C₀-C₃ alkylene)-O-(C₀-C₃ alkylene)-, -(C₀-C₃ alkylene)-S-(C₀-C₃ alkylene)-, -(C₀-C₃ alkylene)-NH-(C₀-C₃ alkylene)-, -(C₀-C₃ alkylene)-C(O)NH-(C₀-C₃ alkylene)-, -(C₀-C₃ alkylene)-NHC(O)-(C₀-C₃ alkylene)-; and
a is 0 or 1.

In some embodiments of the present invention, R^{Y401} is selected from the group consisting of: H and halogen (for example, F).

In some embodiments of the present invention, R^{Y401} is H.

In some embodiments of the present invention, R^{Y401} is F.

In some embodiments of the present invention, R^{Y402} and R^{Y403} are independently selected from the group consisting of: H, halogen (for example, F), hydroxyl, amino, C₁-C₃ alkyl, halogenated C₁-C₃ alkyl, hydroxyl-substituted C₁-C₃ alkyl, alkoxy-substituted C₁-C₆ alkyl, amino-substituted C₁-C₃ alkyl, and alkylamino-substituted C₁-C₆ alkyl.

In some embodiments of the present invention, R^{Y402} and R^{Y403} are both H.

In some embodiments of the present invention, R^{Y402} and R^{Y403}, together with the carbon atom attach thereto, form a four- to six-membered saturated aliphatic ring, for example,

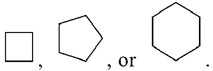

In some embodiments of the present invention, R^{Y404} is selected from the group consisting of: H and C₁-C₆ alkyl (for example, methyl).

In some embodiments of the present invention, R^{Y404} is H.

In some embodiments of the present invention, R^{Y405} is selected from the group consisting of: H, C₁-C₆ alkyl, halogenated C₁-C₆ alkyl, hydroxyl-substituted C₁-C₆ alkyl, alkoxy-substituted C₁-C₆ alkyl, amino-substituted C₁-C₆ alkyl, and alkylamino-substituted C₁-C₆ alkyl.

In some embodiments of the present invention, R^{Y405} is selected from the group consisting of: C₁-C₆ alkyl (for example, methyl), and alkoxy-substituted C₁-C₆ alkyl (for example, 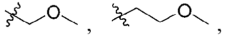 or

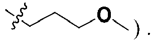

In some embodiments of the present invention, R^{Y404} and R^{Y405}, together with the nitrogen atom attached thereto, form a four- to twelve-membered nitrogenous heterocyclic ring, for example, 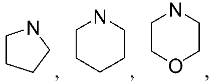 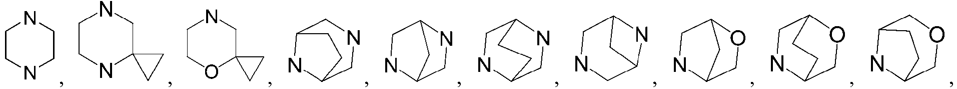 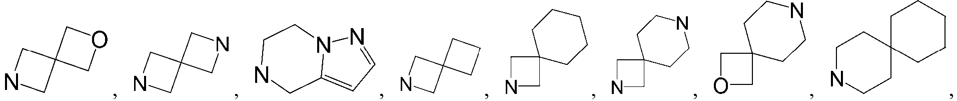 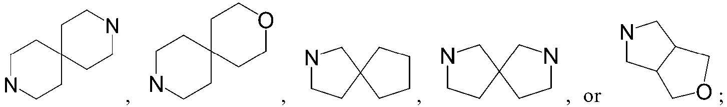 the heterocyclic ring is optionally substituted with one or more R^{Y406} groups;
R^{Y406} is selected from the group consisting of: halogen, cyano, nitro, azido, C₁-C₁₀ alkyl, C₂-C₁₀ alkenyl, C₂-C₁₀ alkynyl, -(C₀-C₆ alkylene)-(C₃-C₁₀ cycloalkyl), -(C₀-C₆ alkylene)-(phenyl), -(C₀-C₆ alkylene)-(4-10-membered heterocyclyl), halogenated C₁-C₁₀ alkyl, halogenated C₁-C₁₀ alkoxy, -N(C₀₋₁₀ alkyl)(C₀₋₁₀ alkyl), - N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -S(C₀₋₁₀ alkyl), -SO(C₀₋₁₀ alkyl), -SO₂(C₀₋₁₀ alkyl), -SO₂N(C₀₋₁₀ alkyl)(C₀₋₁₀ alkyl), -COO(C₀₋₁₀ alkyl), -OCO(C₀₋₁₀ alkyl), -CON(C₀₋₁₀ alkyl)(C₀₋₁₀ alkyl), -CO(C₀₋₁₀ alkyl), and -CO(C₃-C₁₀ cycloalkyl); wherein H in the C₀₋₁₀ alkyl, C₁-C₁₀ alkyl, C₂-C₁₀ alkenyl, C₂-C₁₀ alkynyl, C₀-C₆ alkylene, C₃-C₁₀ cycloalkyl, phenyl, or 4-10-membered heterocyclyl is optionally substituted with one or more groups selected from the group consisting of: halogen, cyano, nitro, azido, C₁-C₁₀ alkyl, halogenated C₁-C₁₀ alkyl, halogenated C₁-C₁₀ alkoxy, -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -S(C₀₋₁₀ alkyl), -SO(C₀₋₁₀ alkyl), -SO₂(C₀₋₁₀ alkyl), -SO₂N(C₀₋₁₀ alkyl)(C₀₋₁₀ alkyl), -COO(C₀₋₁₀ alkyl), -OCO(C₀₋₁₀ alkyl), -CON(C₀₋₁₀ alkyl)(C₀₋₁₀ alkyl), and -CO(C₀₋₁₀ alkyl). In some embodiments of the present invention, R^{Y406} is selected from the group consisting of: halogen, cyano, hydroxyl, amino, C₁-C₆ alkyl, halogenated C₁-C₆ alkyl, C₁-C₆ alkoxy, halogenated C₁-C₆ alkoxy, C₁-C₆ alkoxyalkyl, C₁-C₆ alkylamino, C₁-C₆ alkylaminoalkyl, C₃-C₆ cycloalkyl, -CO(C₀₋₆ alkyl), -CO(C₃-C₆ cycloalkyl), -CO(halogenated C₀₋₆ alkyl), -SO₂(C₀₋₁₀ alkyl), and -SO₂(halogenated C₀₋₁₀ alkyl).

In some embodiments of the present invention, a is 1.

In some embodiments of the present invention, Z is a single bond, methylene or O.

In some embodiments of the present invention, a is 0, and Z is O.

In some embodiments of the present invention, a is 1, and Z is methylene.

In some embodiments of the present invention, Y4 is selected from the group consisting of:

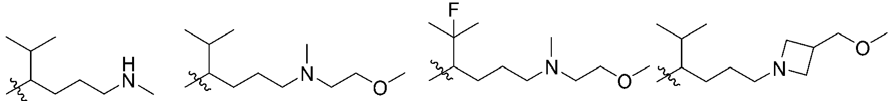

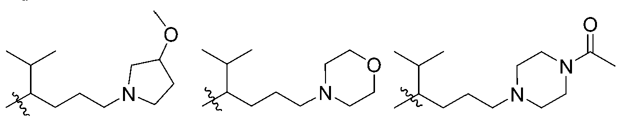

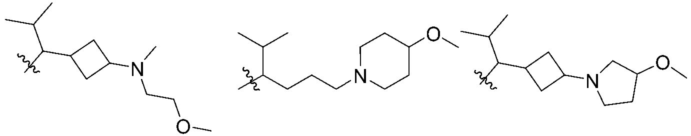

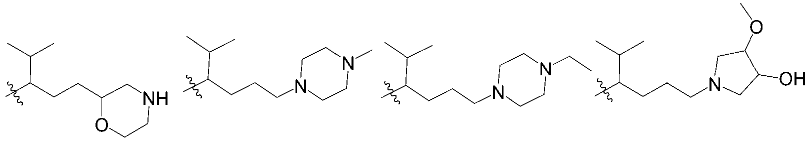

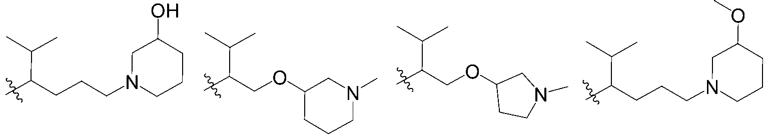

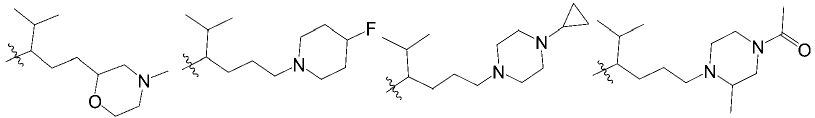

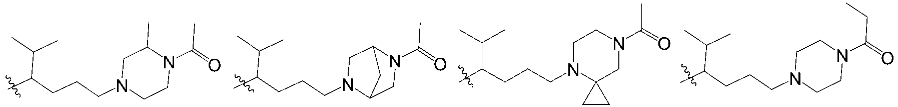

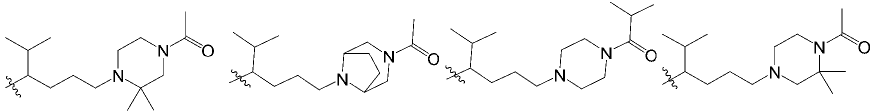

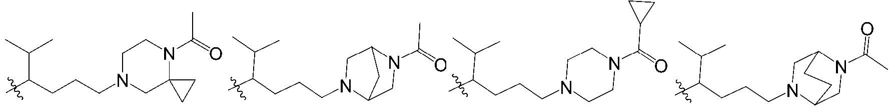

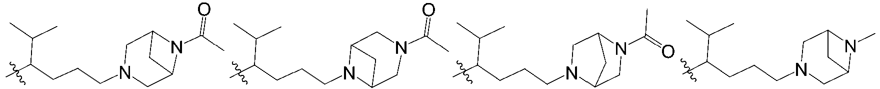

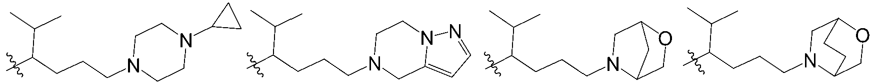

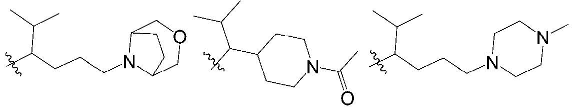

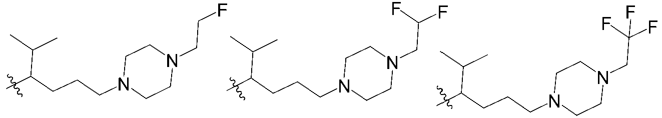

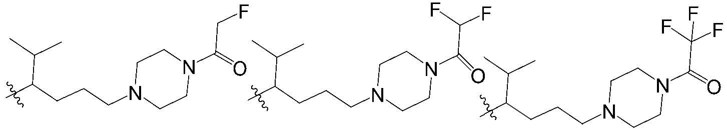

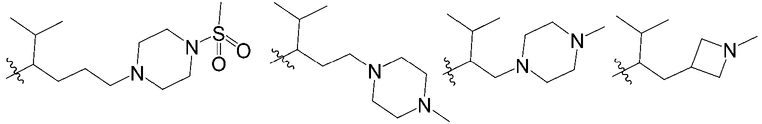

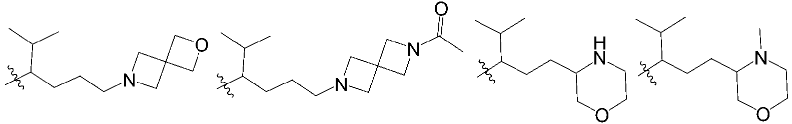

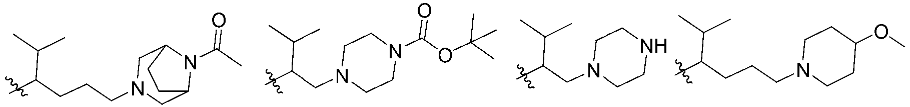

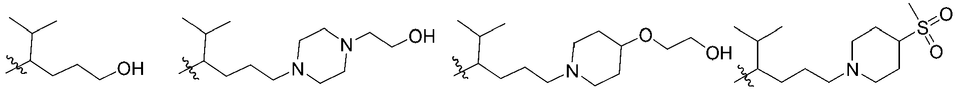

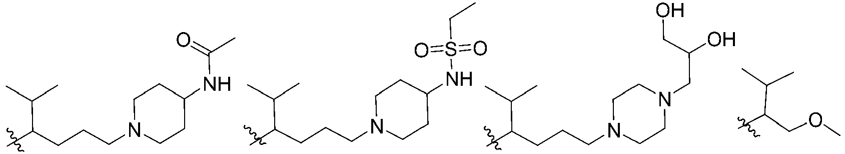

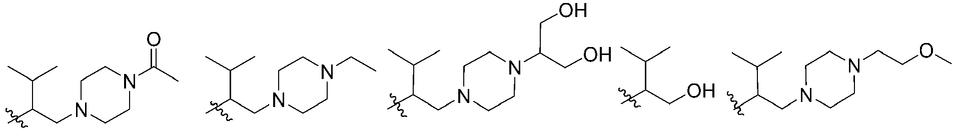

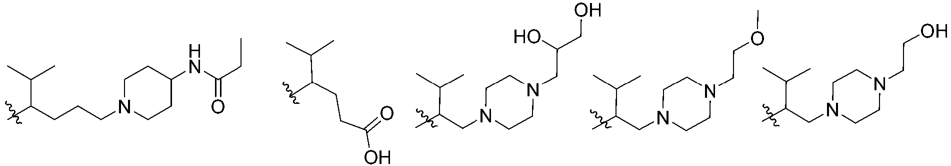

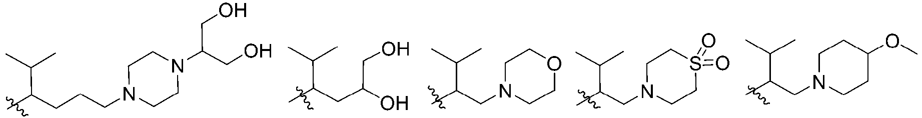

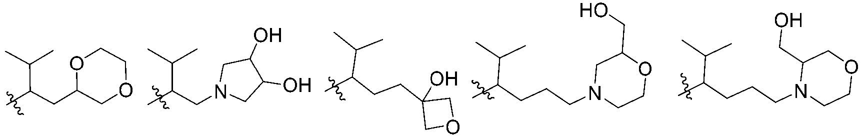

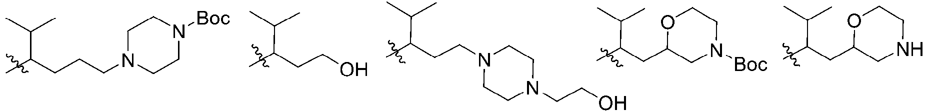

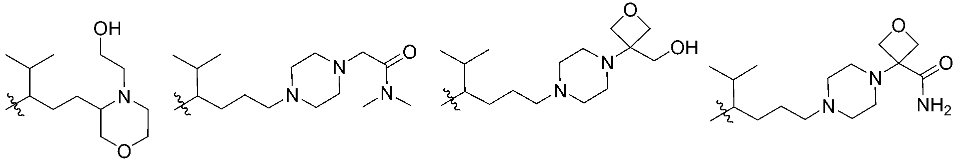

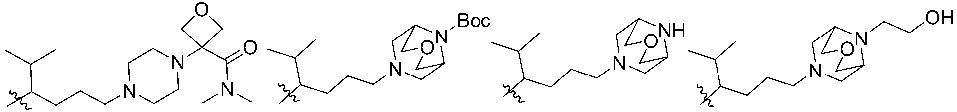

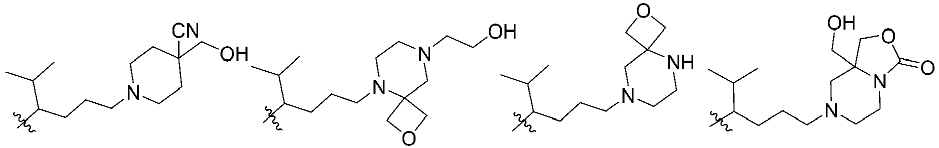

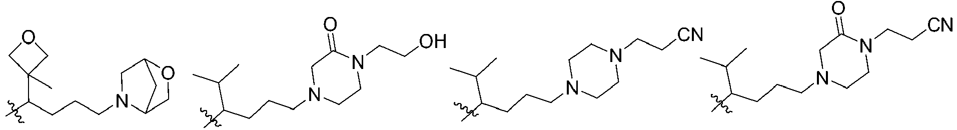

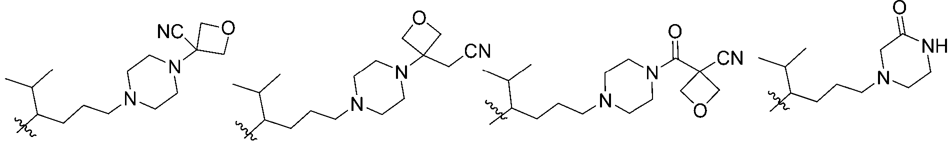

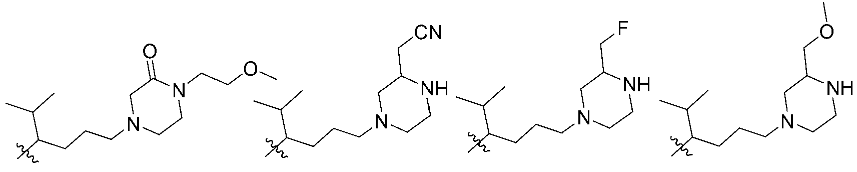

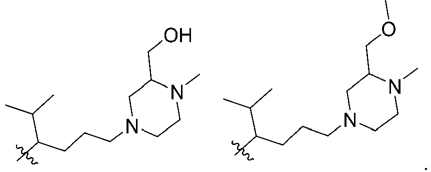

In some embodiments of the present invention, L₂ is a single bond, and Y is Y4.

In some embodiments of the present invention, the 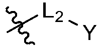 moiety is selected from the group consisting of:

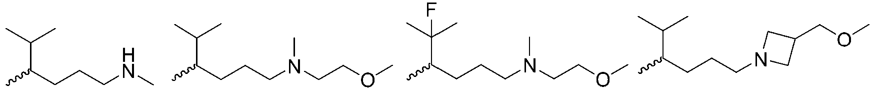

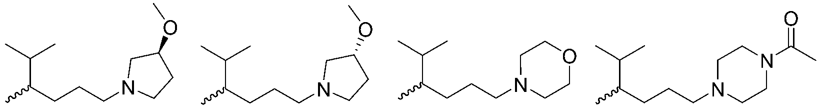

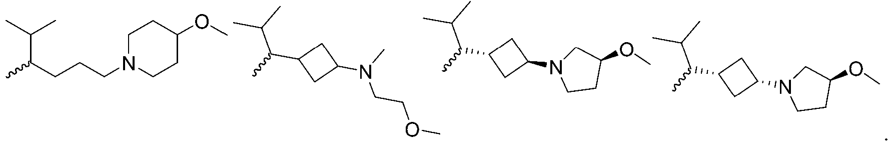

In some embodiments of the present invention, Y5 is 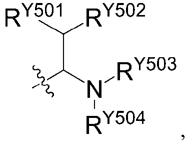 wherein R^{Y501} and R^{Y502} are independently selected from the group consisting of: H, halogen (for example, F), hydroxyl, amino, C₁-C₃ alkyl, halogenated C₁-C₃ alkyl, and -(C₀-C₃ alkylene)-(C₃-C₆ cycloalkyl), wherein the alkyl or cycloalkyl may be optionally substituted with one or more groups selected from the group consisting of: halogen, cyano, hydroxyl, amino, C₁-C₆ alkoxy, and C₁-C₆ alkylamino; or, R^{Y501} and R^{Y502}, together with the carbon atom attached thereto, form an aliphatic ring or a heterocyclic ring, wherein the aliphatic ring or heterocyclic ring may be optionally substituted with one or more groups selected from the group consisting of: halogen, cyano, hydroxyl, amino, C₁-C₆ alkyl, and halogenated C₁-C₆ alkyl;
R^{Y503} and R^{Y504} are independently selected from the group consisting of: H and C₁-C₆ alkyl, wherein said alkyl may be optionally substituted with one or more groups selected from the group consisting of: halogen, cyano, hydroxyl, amino, C₁-C₆ alkoxy, and C₁-C₆ alkylamino; or, R^{Y503} and R^{Y504}, together with the nitrogen atom attached thereto, form a heterocyclic ring, wherein said heterocyclic ring may be optionally substituted with one or more groups selected from the group consisting of: halogen, cyano, nitro, azido, C₁-C₁₀ alkyl, C₂-C₁₀ alkenyl, C₂-C₁₀ alkynyl, -(C₀-C₆ alkylene)-(C₃-C₁₀ cycloalkyl), -(C₀-C₆ alkylene)-(phenyl), -(C₀-C₆ alkylene)-(4-10-membered heterocyclyl), halogenated C₁-C₁₀ alkyl, halogenated C₁-C₁₀ alkoxy, -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -S(C₀₋₁₀ alkyl), -SO(C₀₋₁₀ alkyl), -SO₂(C₀₋₁₀ alkyl), -SO₂N(C₀₋₁₀ alkyl)(C₀₋₁₀ alkyl), - COO(C₀₋₁₀ alkyl), -OCO(C₀₋₁₀ alkyl), -CON(C₀₋₁₀ alkyl)(C₀₋₁₀ alkyl), -CO(C₀₋₁₀ alkyl), and -CO(C₃-C₁₀ cycloalkyl); wherein H in said C₀₋₁₀ alkyl, C₁-C₁₀ alkyl, C₂-C₁₀ alkenyl, C₂-C₁₀ alkynyl, C₀-C₆ alkylene, C₃-C₁₀ cycloalkyl, phenyl, or 4-10-membered heterocyclyl is optionally substituted with one or more groups selected from the group consisting of: halogen, cyano, nitro, azido, C₁-C₁₀ alkyl, halogenated C₁-C₁₀ alkyl, halogenated C₁-C₁₀ alkoxy, -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -S(C₀₋₁₀ alkyl), -SO(C₀₋₁₀ alkyl), - SO₂(C₀₋₁₀ alkyl), -SO₂N(C₀₋₁₀ alkyl)(C₀₋₁₀ alkyl), -COO(C₀₋₁₀ alkyl), -OCO(C₀₋₁₀ alkyl), -CON(C₀₋₁₀ alkyl)(C₀₋₁₀ alkyl), and -CO(C₀₋₁₀ alkyl).

In some embodiments of the present invention, R^{Y501} and R^{Y502} are independently selected from the group consisting of: C₁-C₃ alkyl, and halogenated C₁-C₃ alkyl.

In some embodiments of the present invention, R^{Y501} and R^{Y502}, together with the carbon atom attached thereto, form C₃-C₆ cycloalkyl, and the C₃-C₆ cycloalkyl may be optionally substituted with one or more groups selected from the group consisting of: halogen, cyano, hydroxyl, amino, C₁-C₃ alkoxy, and C₁-C₃ alkylamino. In some embodiments of the present invention, R^{Y503} is H.

In some embodiments of the present invention, R^{Y503} is C₁-C₃ alkyl.

In some embodiments of the present invention, R^{Y503} and R^{Y504}, together with the nitrogen atom attached thereto, form a four- to twelve-membered nitrogenous heterocyclic ring, for example, 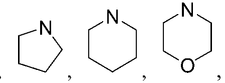 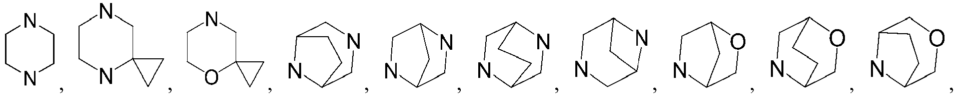 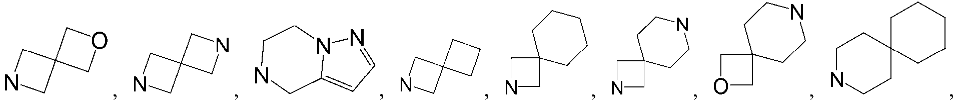 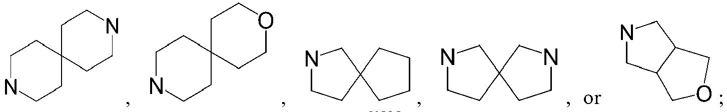 the heterocyclic ring is optionally substituted with one or more R^{Y505} groups;
R^{Y505} is selected from the group consisting of: halogen, cyano, nitro, azido, C₁-C₁₀ alkyl, C₂-C₁₀ alkenyl, C₂-C₁₀ alkynyl, -(C₀-C₆ alkylene)-(C₃-C₁₀ cycloalkyl), -(C₀-C₆ alkylene)-(phenyl), -(C₀-C₆ alkylene)-(4-10-membered heterocyclyl), halogenated C₁-C₁₀ alkyl, halogenated C₁-C₁₀ alkoxy, -N(C₀₋₁₀ alkyl)(C₀₋₁₀ alkyl), - N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -S(C₀₋₁₀ alkyl), -SO(C₀₋₁₀ alkyl), -SO₂(C₀₋₁₀ alkyl), -SO₂N(C₀₋₁₀ alkyl)(C₀₋₁₀ alkyl), -COO(C₀₋₁₀ alkyl), -OCO(C₀₋₁₀ alkyl), -CON(C₀₋₁₀ alkyl)(C₀₋₁₀ alkyl), -CO(C₀₋₁₀ alkyl), and -CO(C₃-C₁₀ cycloalkyl); wherein H in the C₀₋₁₀ alkyl, C₁-C₁₀ alkyl, C₂-C₁₀ alkenyl, C₂-C₁₀ alkynyl, C₀-C₆ alkylene, C₃-C₁₀ cycloalkyl, phenyl, or 4-10-membered heterocyclyl is optionally substituted with one or more groups selected from the group consisting of: halogen, cyano, nitro, azido, C₁-C₁₀ alkyl, halogenated C₁-C₁₀ alkyl, halogenated C₁-C₁₀ alkoxy, -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -S(C₀₋₁₀ alkyl), -SO(C₀₋₁₀ alkyl), -SO₂(C₀₋₁₀ alkyl), -SO₂N(C₀₋₁₀ alkyl)(C₀₋₁₀ alkyl), -COO(C₀₋₁₀ alkyl), -OCO(C₀₋₁₀ alkyl), -CON(C₀₋₁₀ alkyl)(C₀₋₁₀ alkyl), and -CO(C₀₋₁₀ alkyl). In some embodiments of the present invention, R^{Y505} is selected from the group consisting of: halogen, cyano, hydroxyl, amino, C₁-C₆ alkyl, halogenated C₁-C₆ alkyl, C₁-C₆ alkoxy, halogenated C₁-C₆ alkoxy, C₁-C₆ alkoxyalkyl, C₁-C₆ alkylamino, C₁-C₆ alkylaminoalkyl, C₃-C₆ cycloalkyl, -CO(C₀₋₆ alkyl), -CO(C₃-C₆ cycloalkyl), -CO(halogenated C₀₋₆ alkyl), -SO₂(C₀₋₁₀ alkyl), and -SO₂(halogenated C₀₋₁₀ alkyl).

In some embodiments of the present invention, Y5 is selected from the group consisting of:

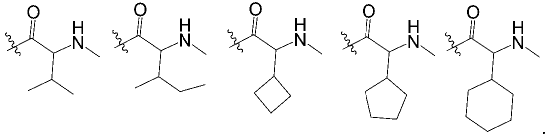

In some embodiments of the present invention, L₂ is C(O), and Y is Y5.

In some embodiments of the present invention, the compound has a structure as follows:

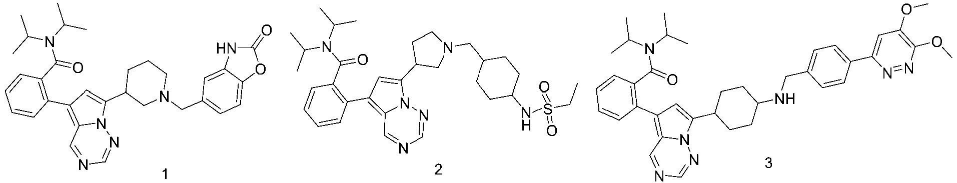

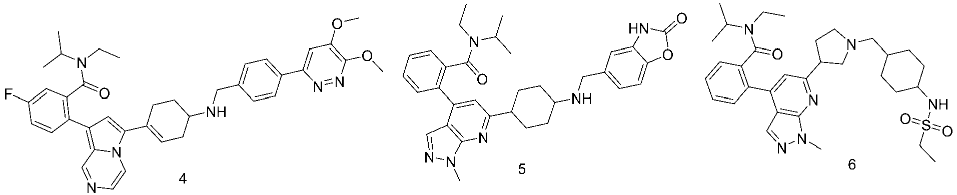

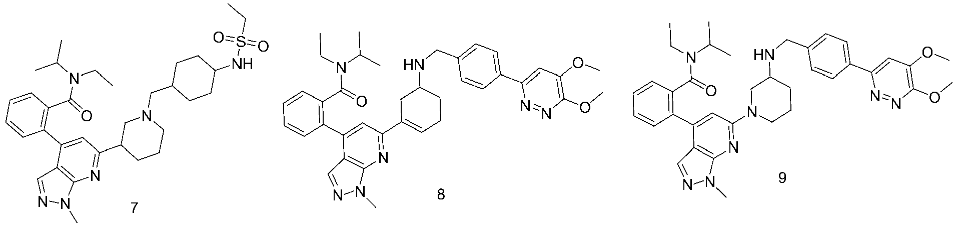

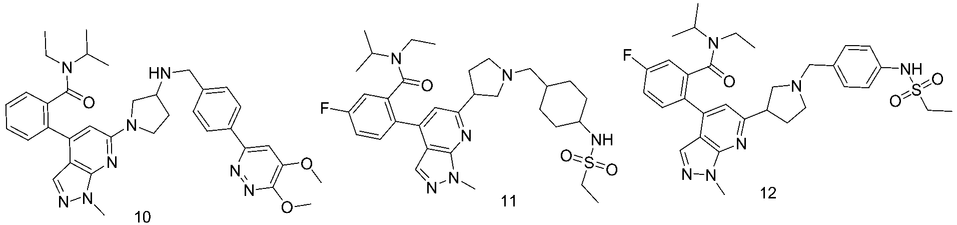

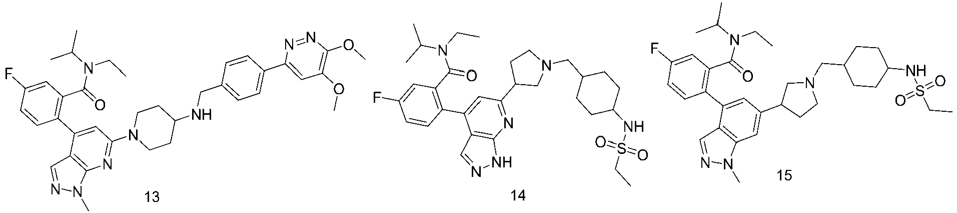

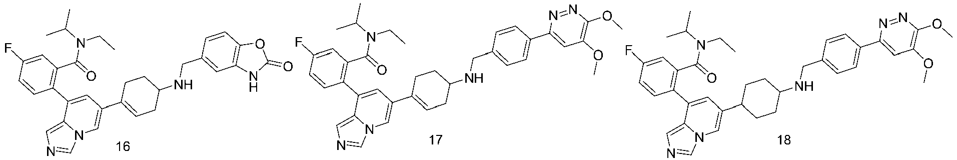

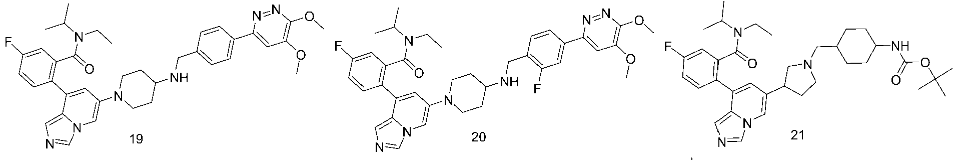

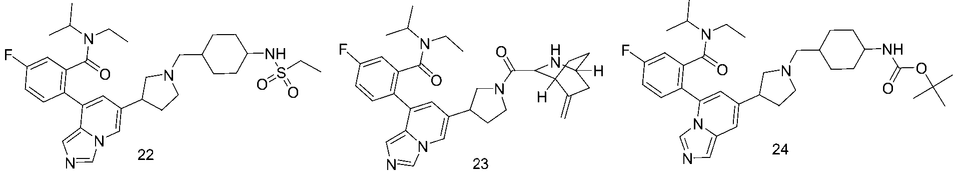

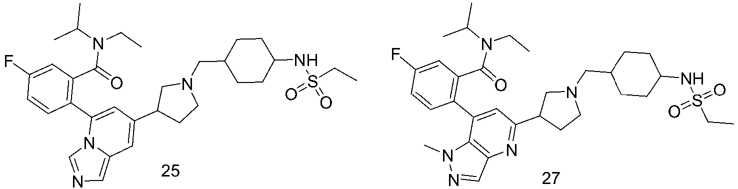

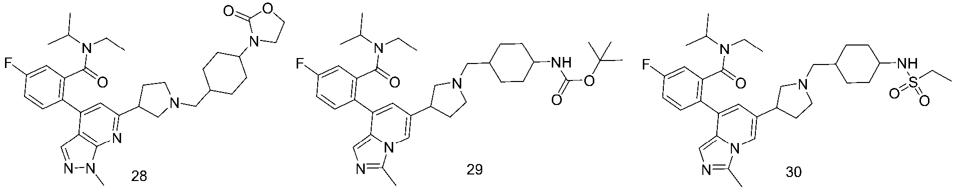

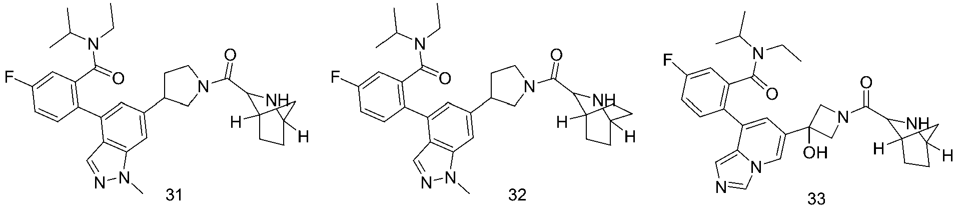

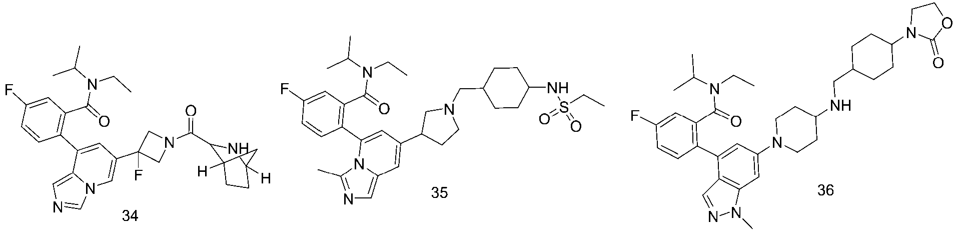

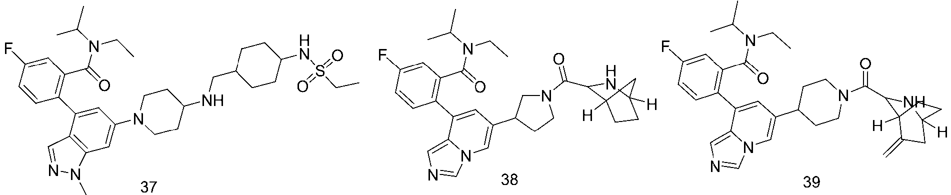

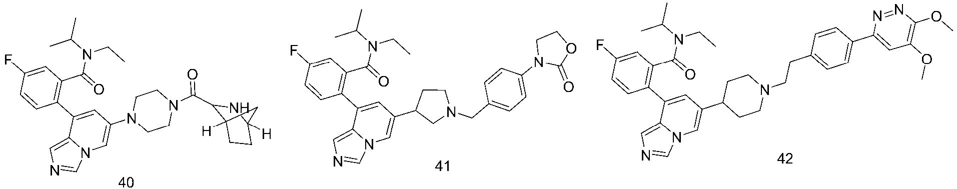

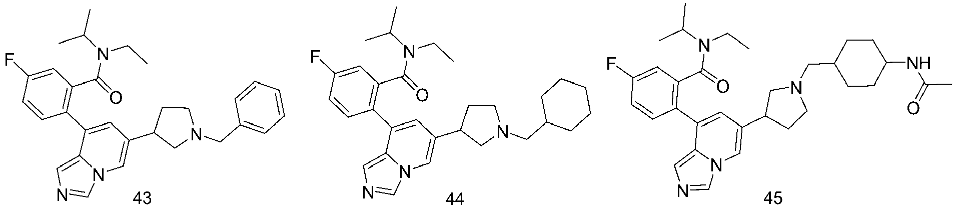

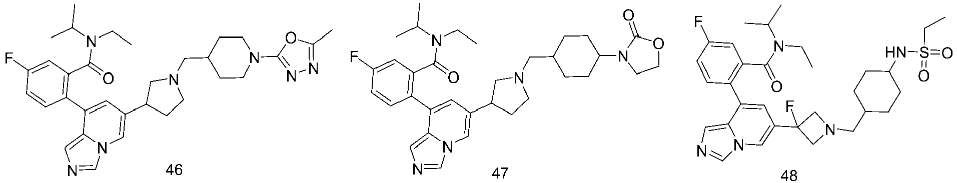

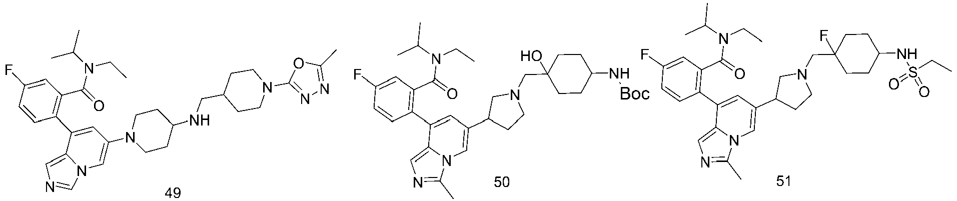

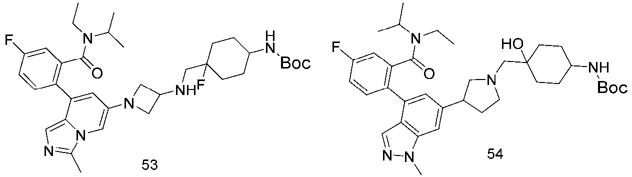

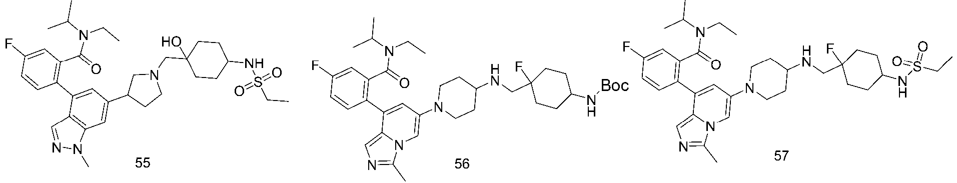

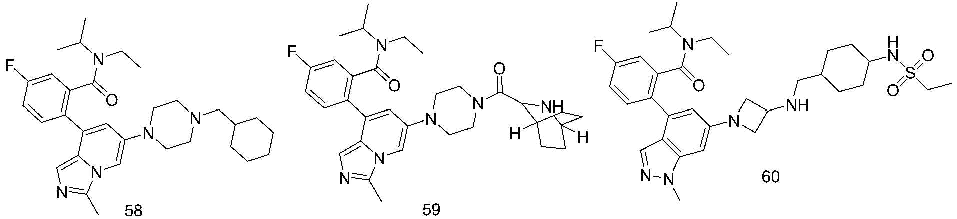

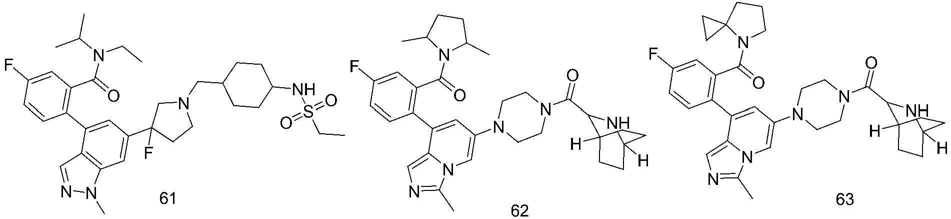

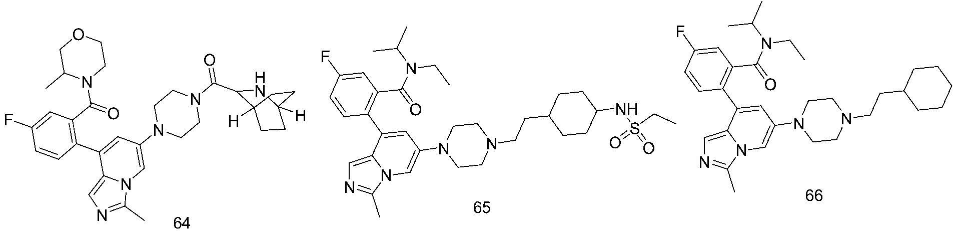

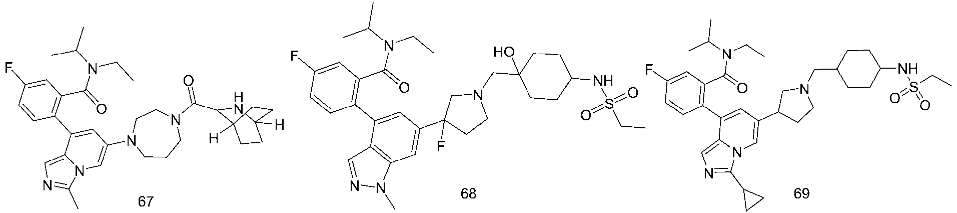

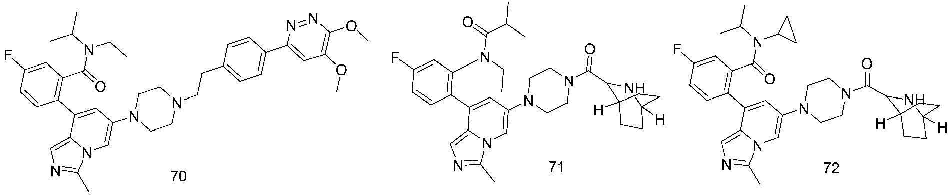

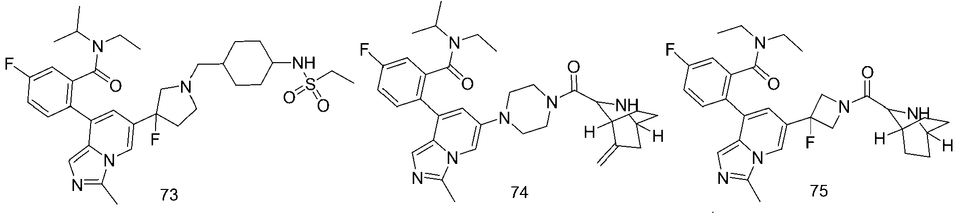

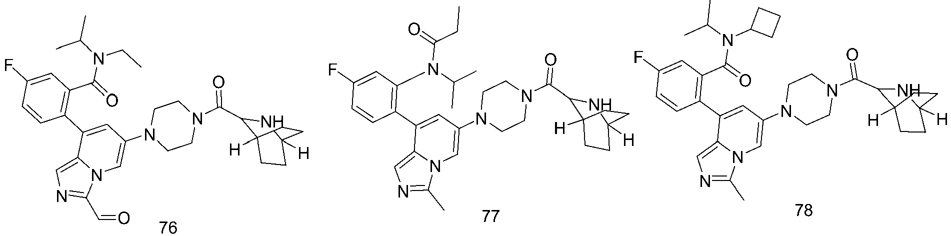

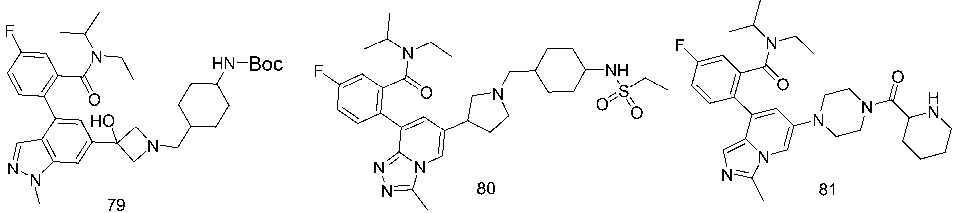

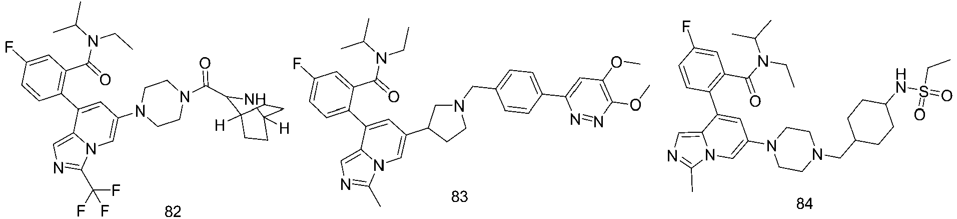

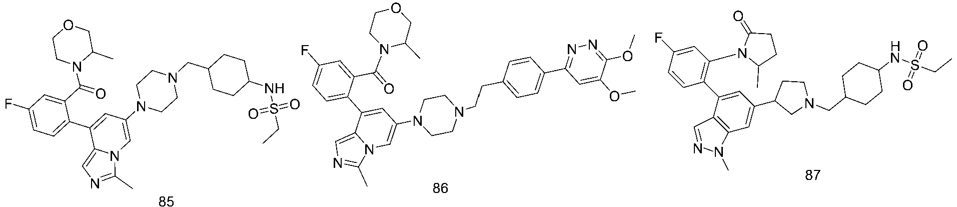

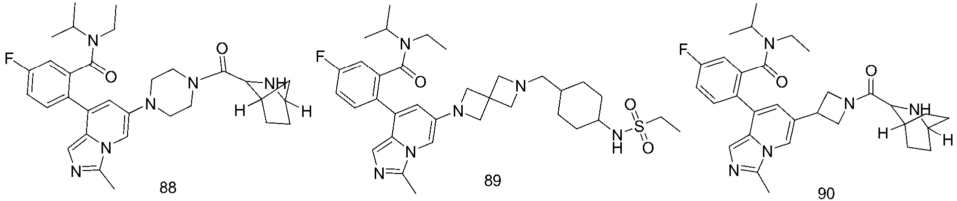

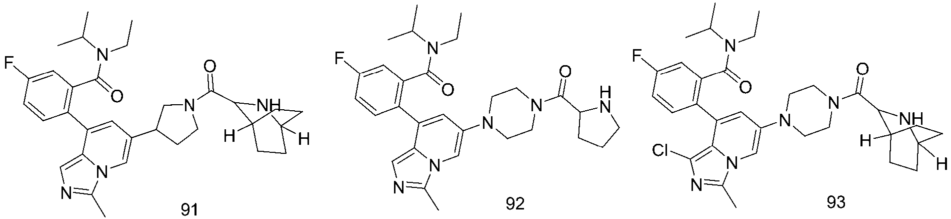

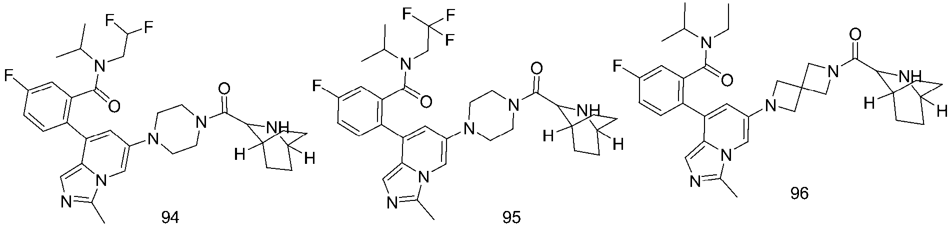

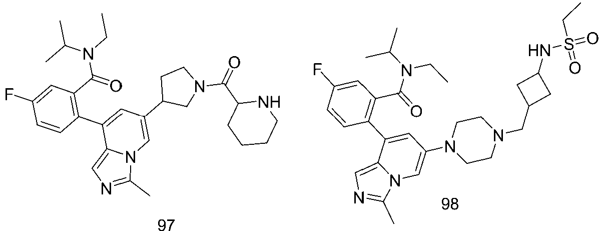

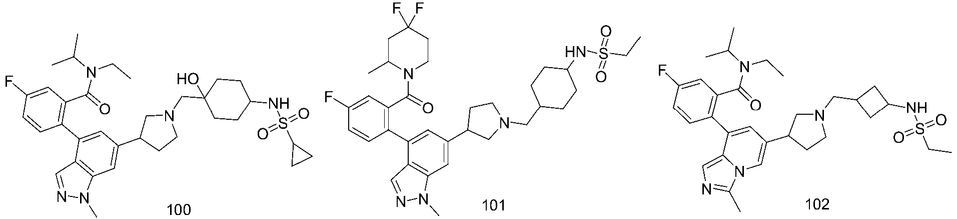

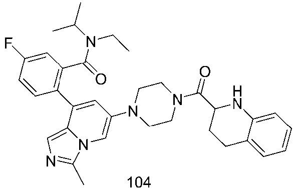

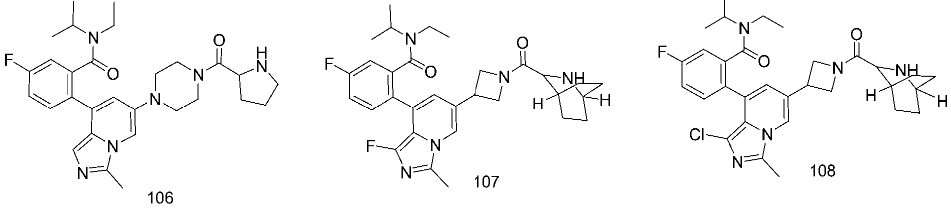

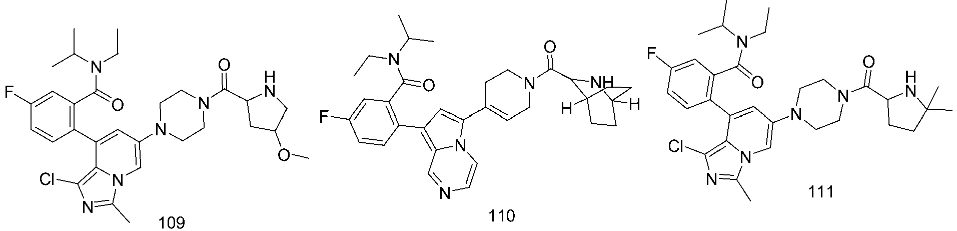

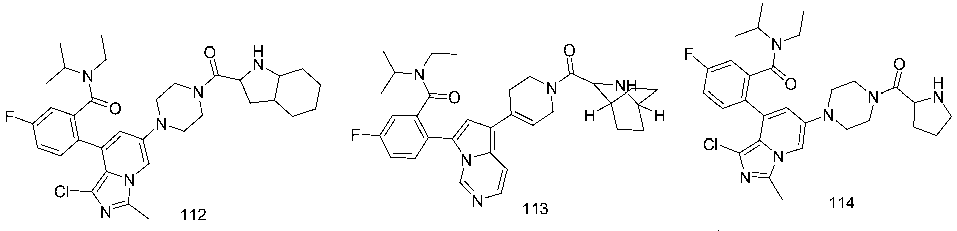

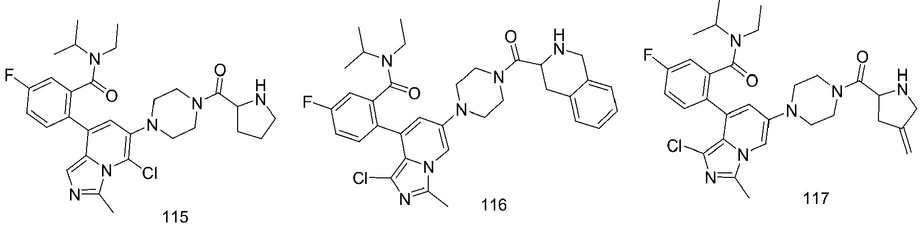

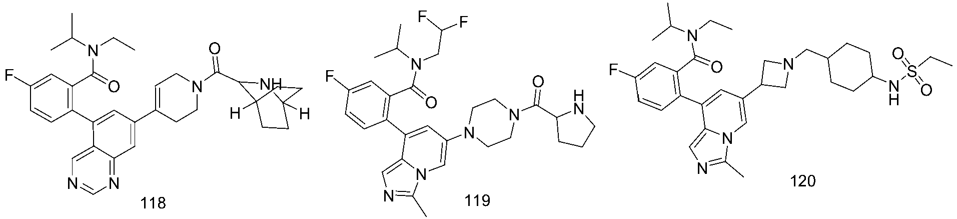

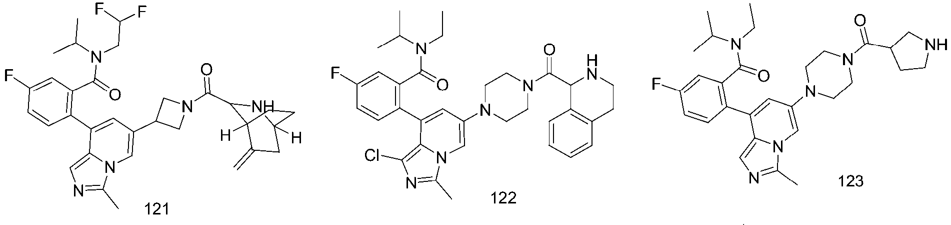

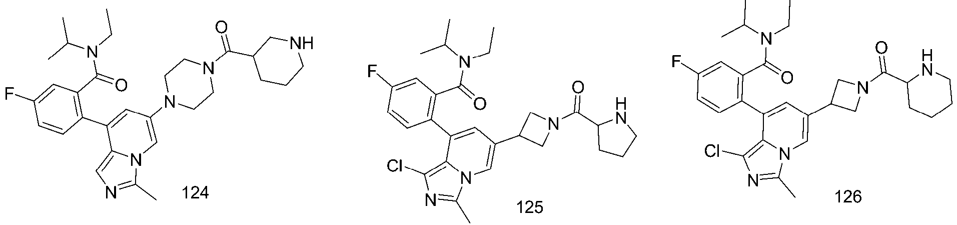

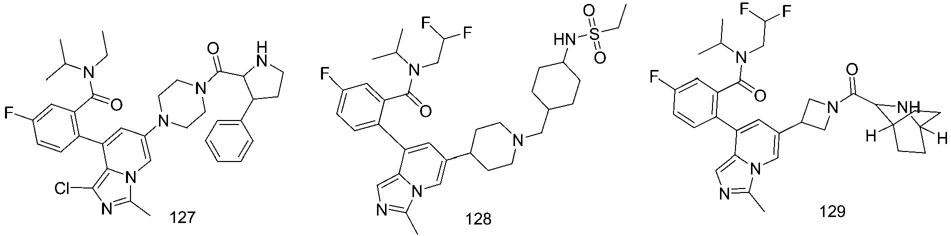

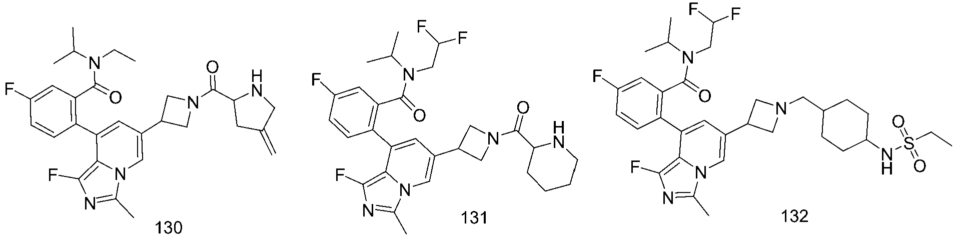

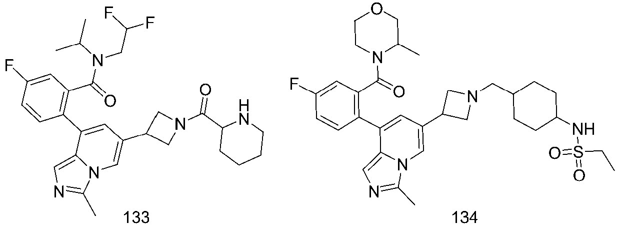

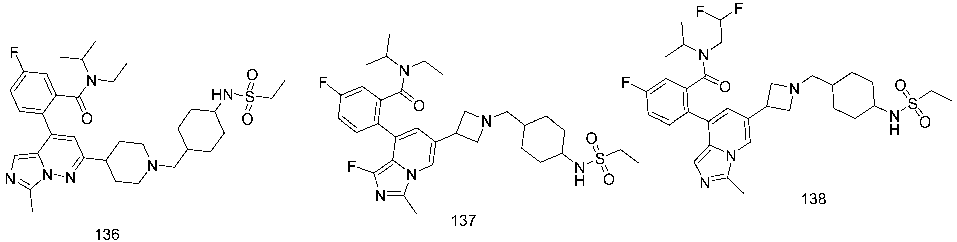

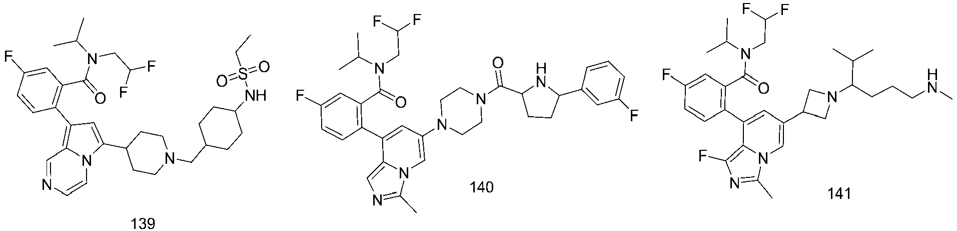

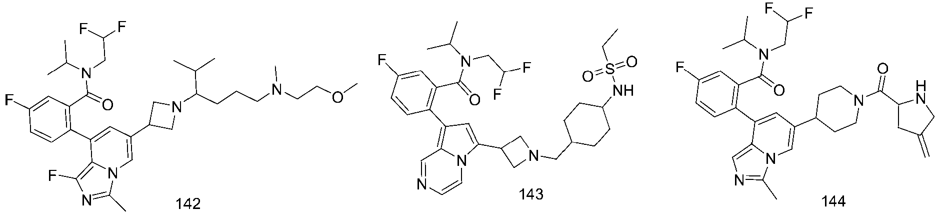

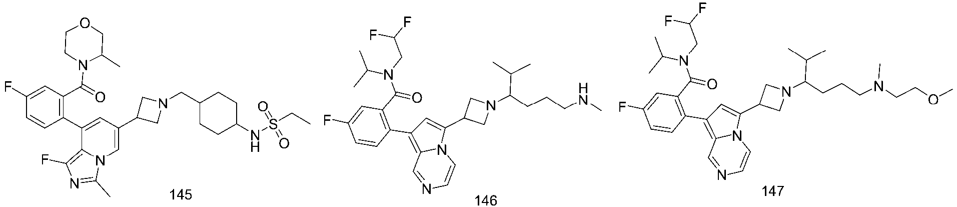

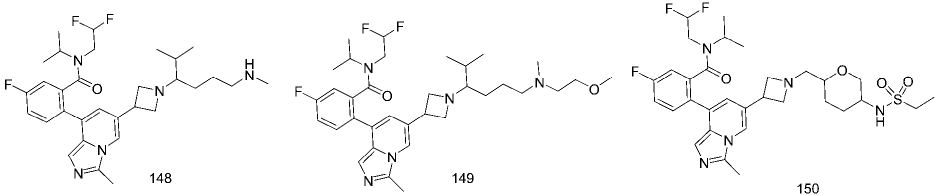

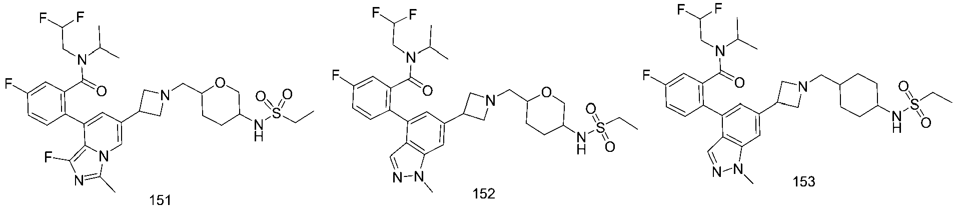

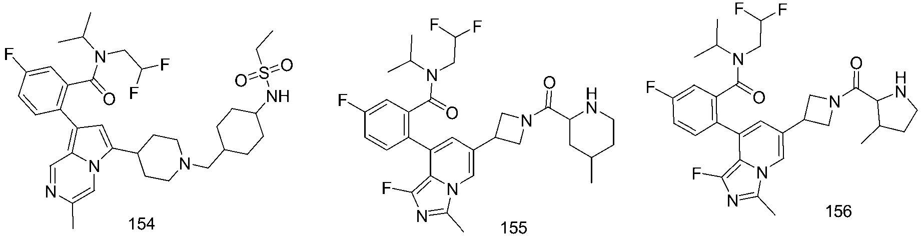

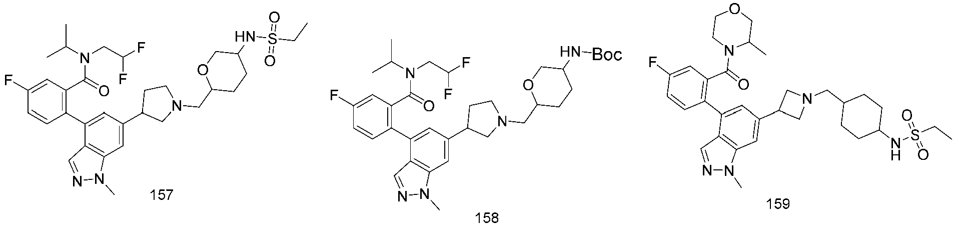

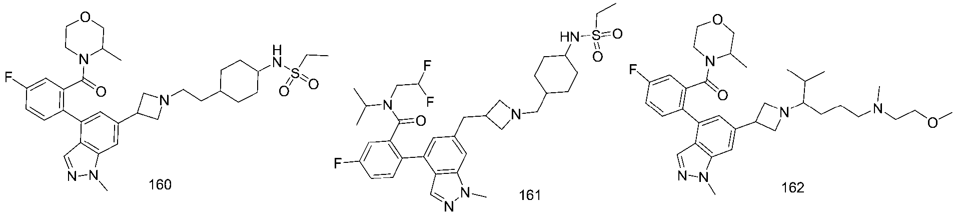

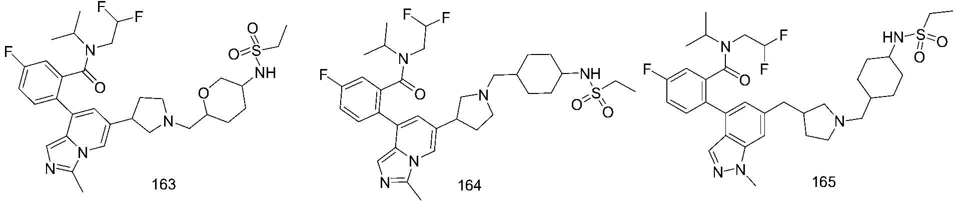

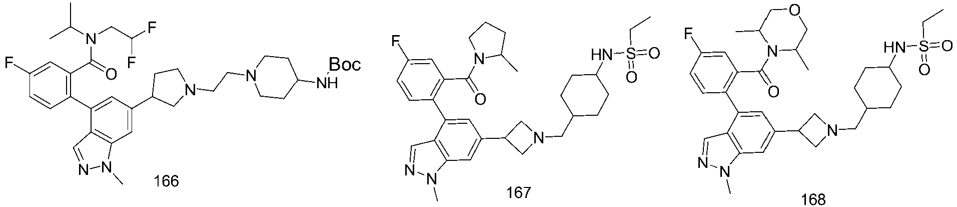

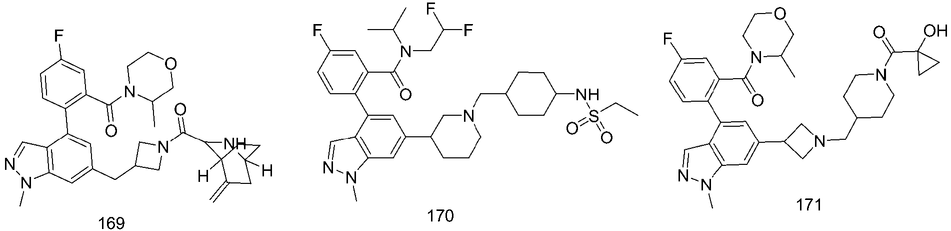

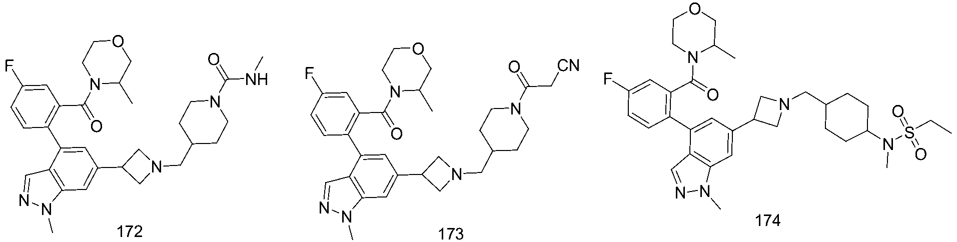

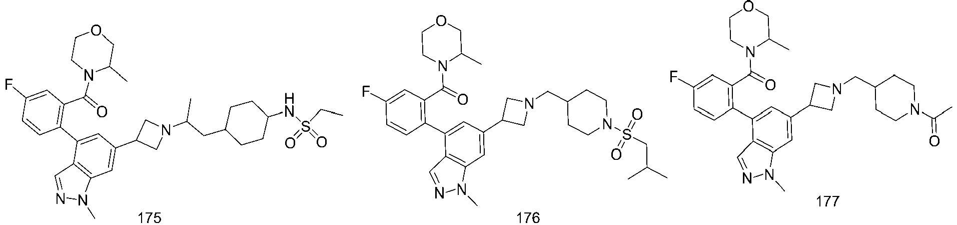

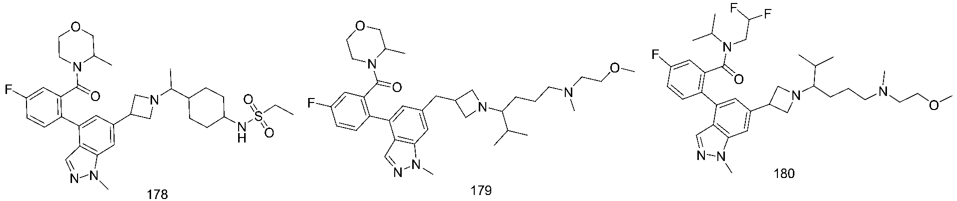

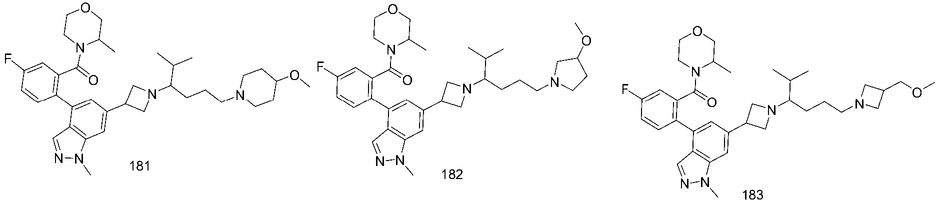

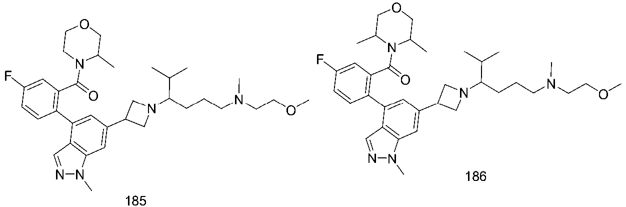

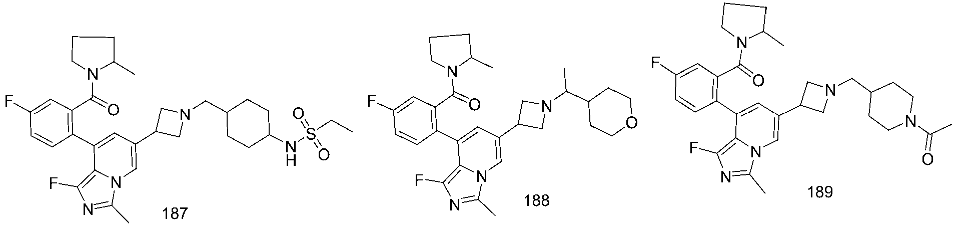

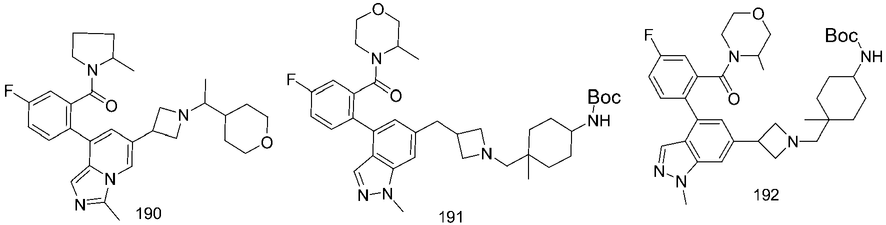

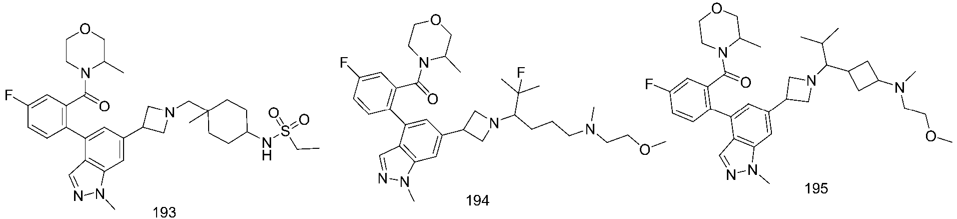

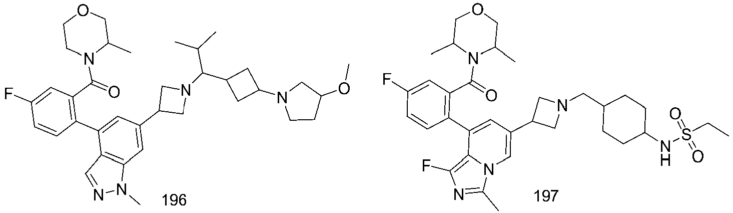

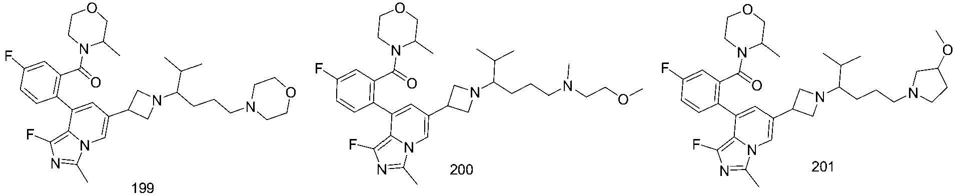

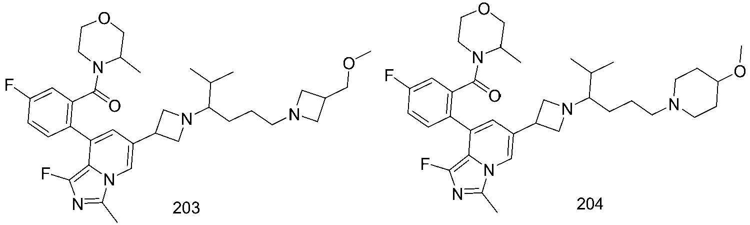

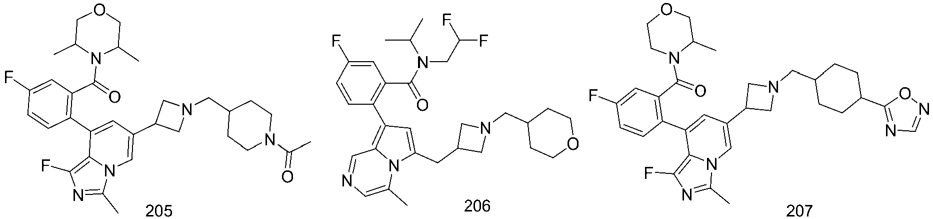

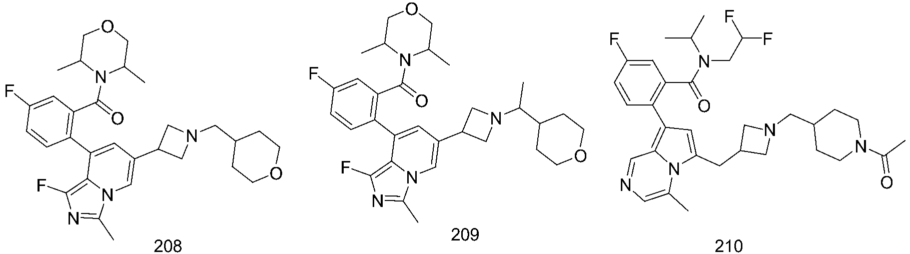

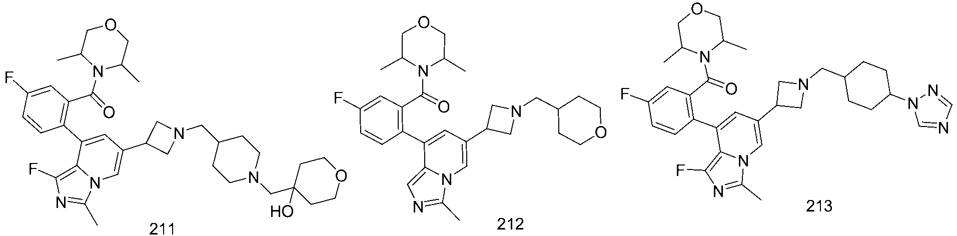

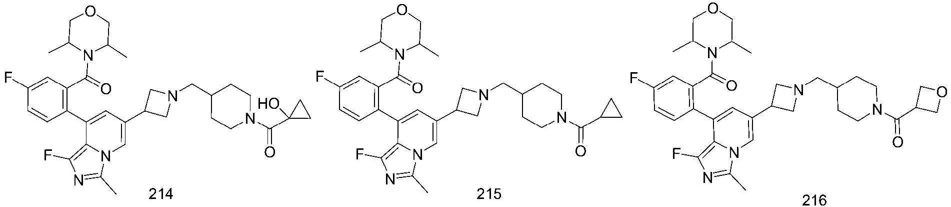

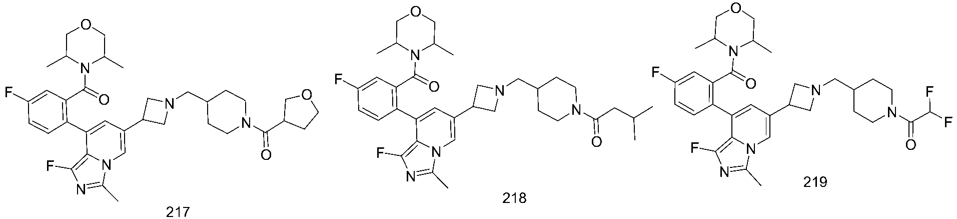

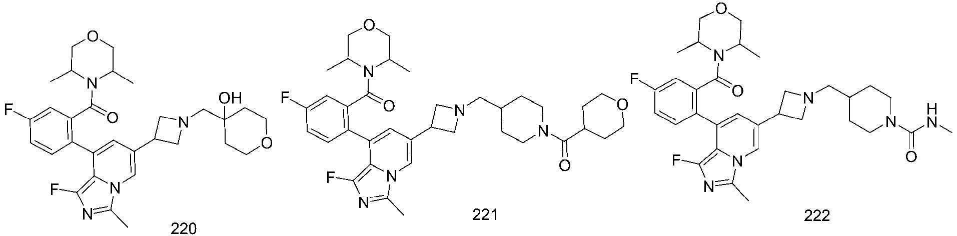

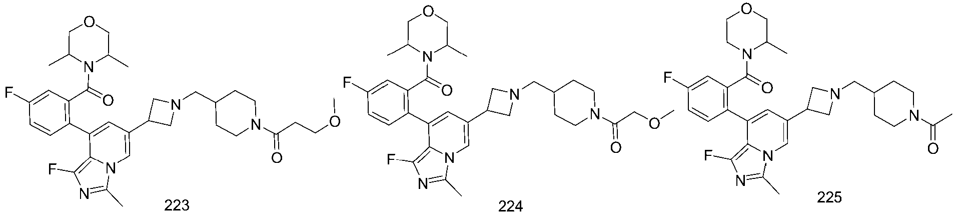

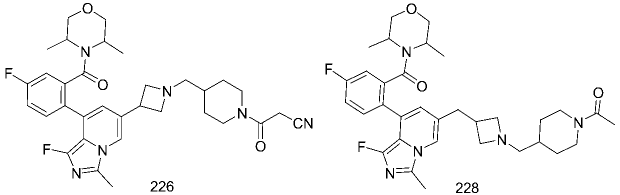

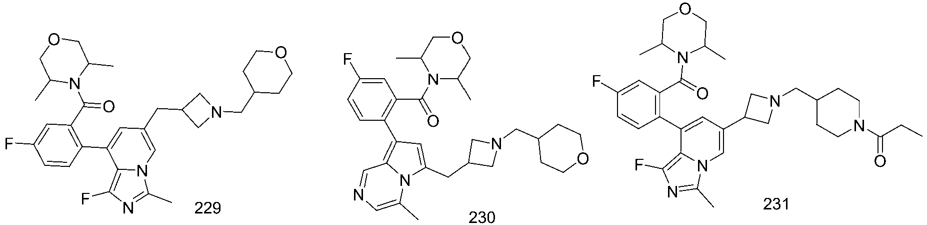

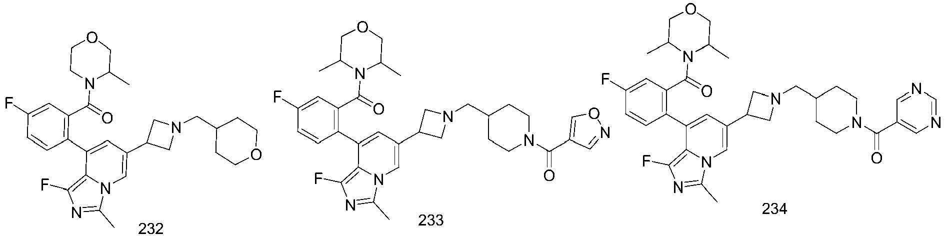

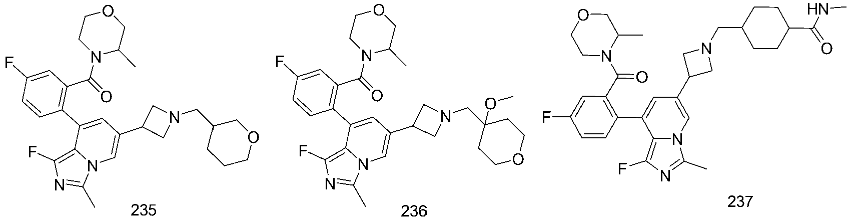

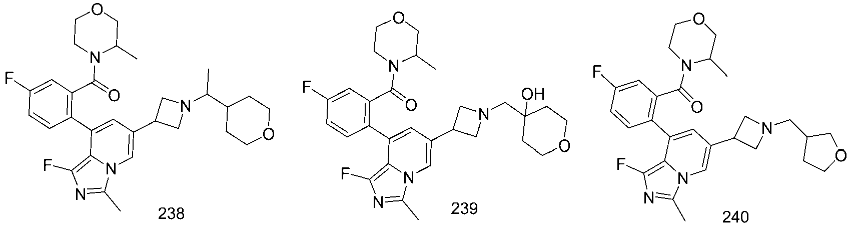

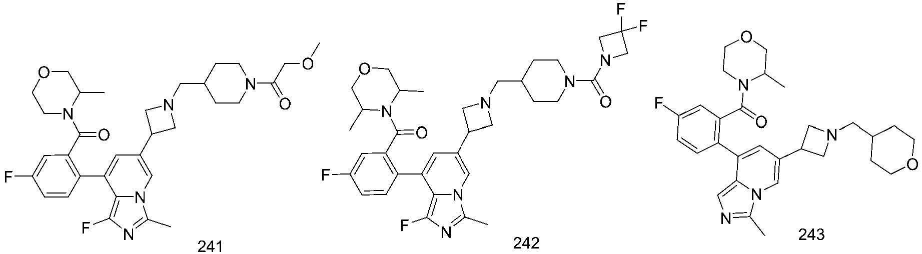

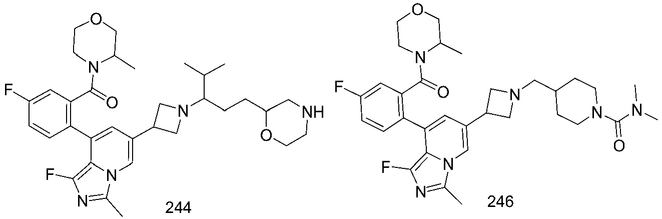

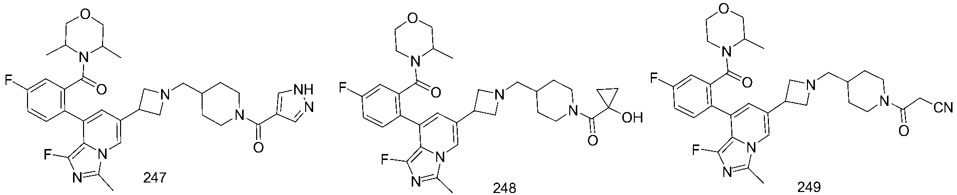

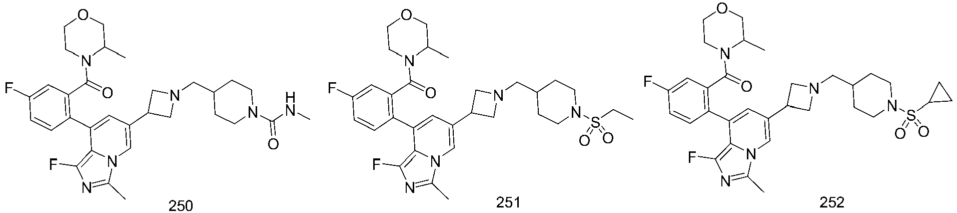

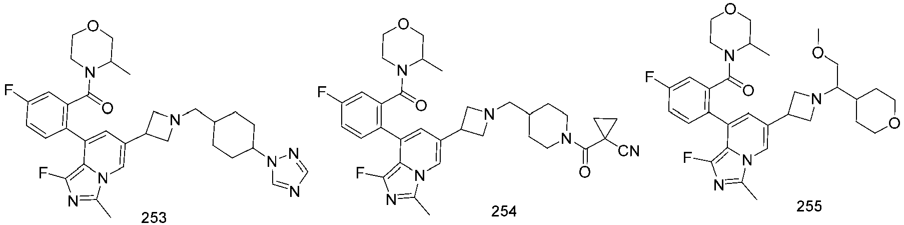

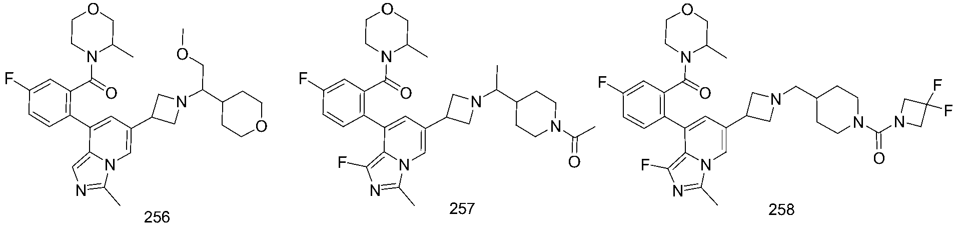

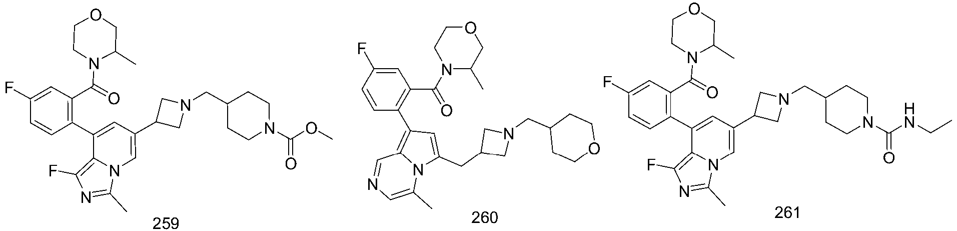

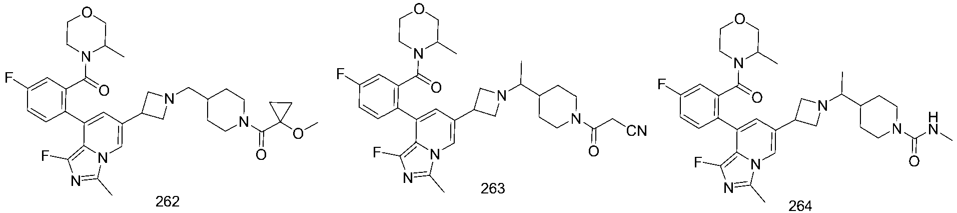

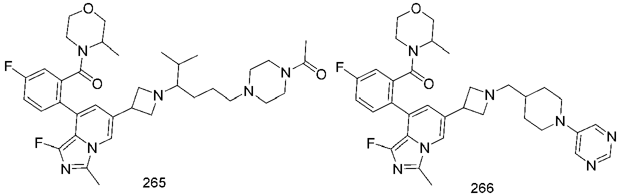

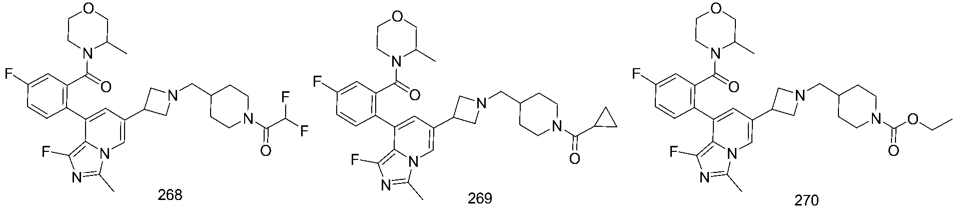

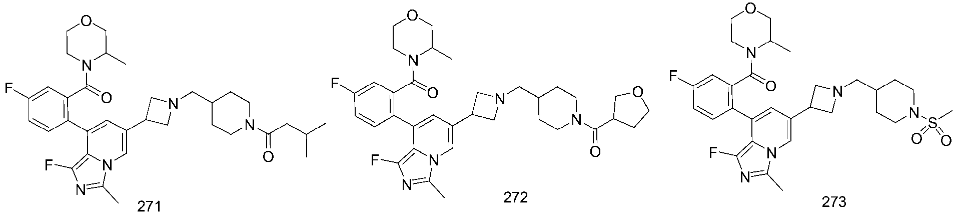

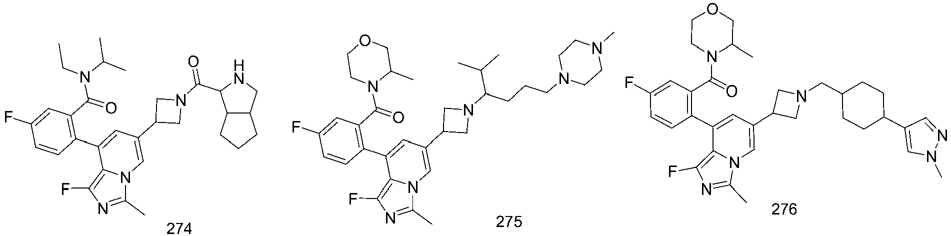

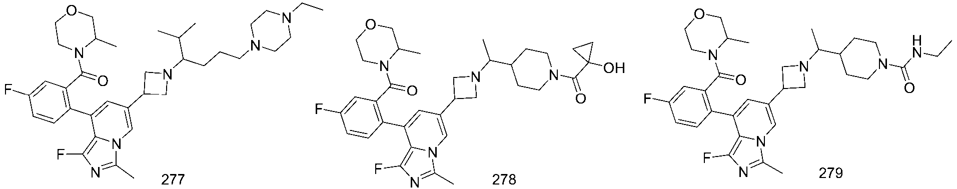

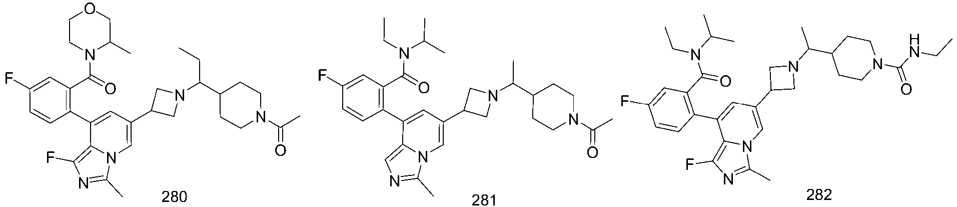

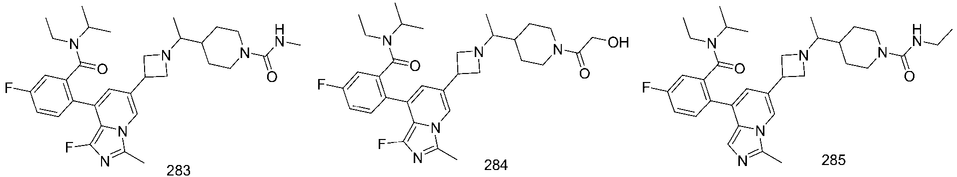

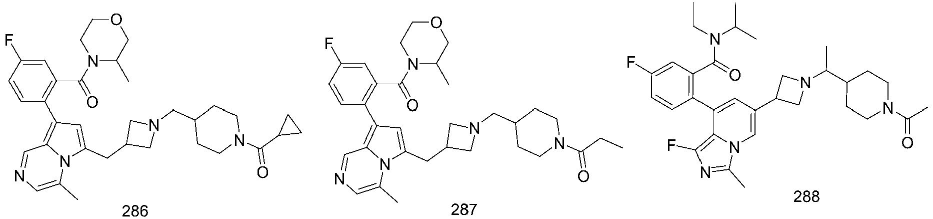

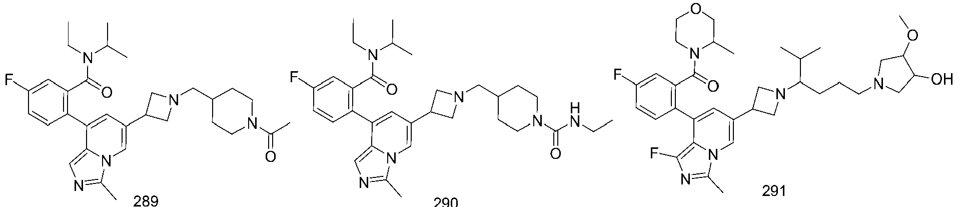

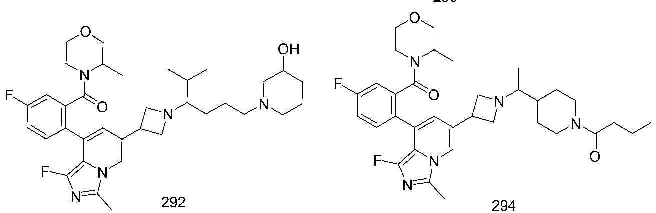

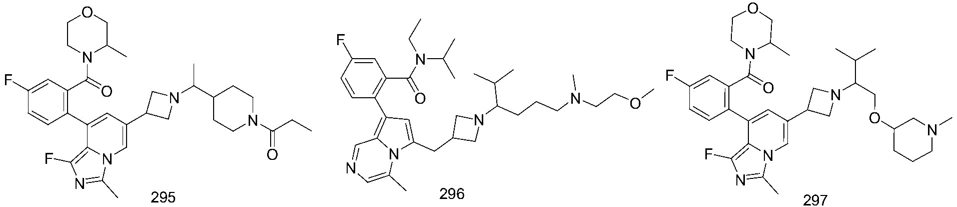

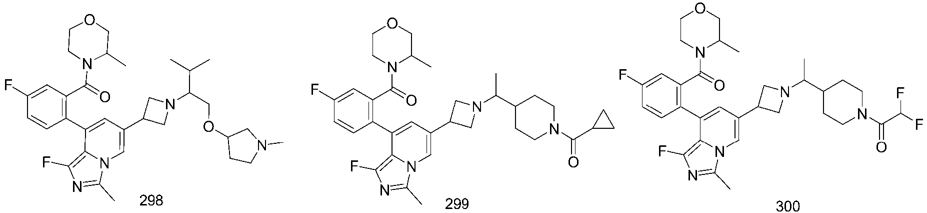

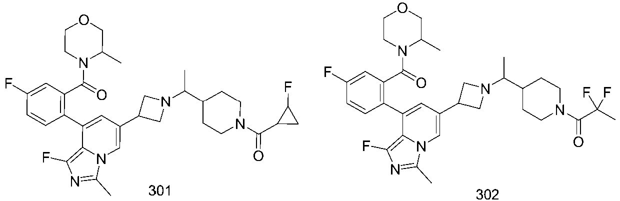

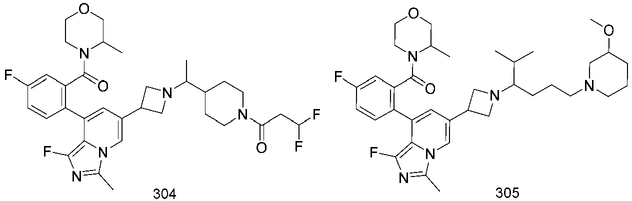

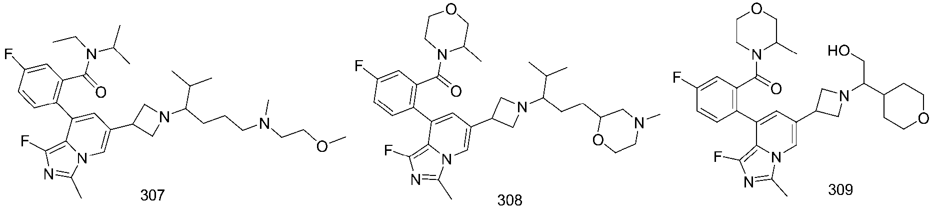

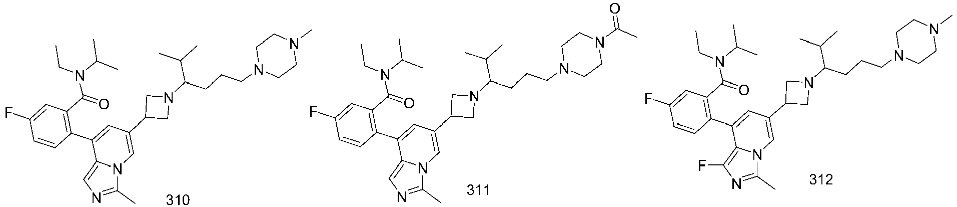

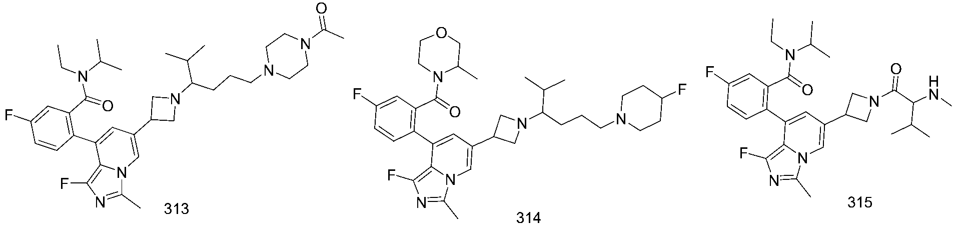

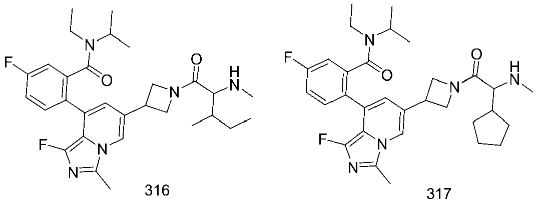

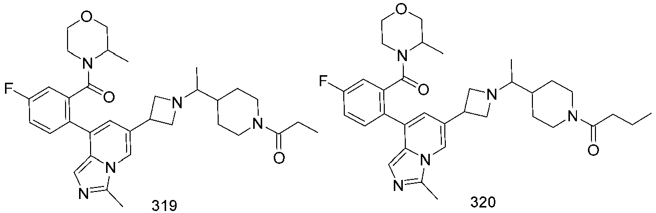

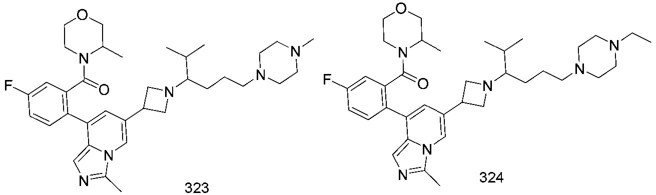

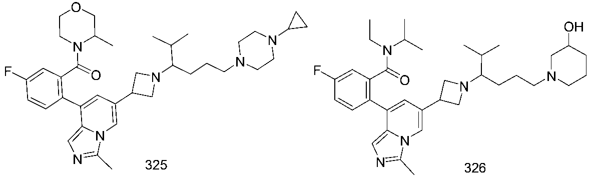

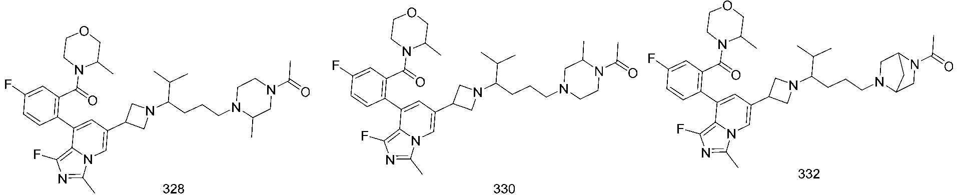

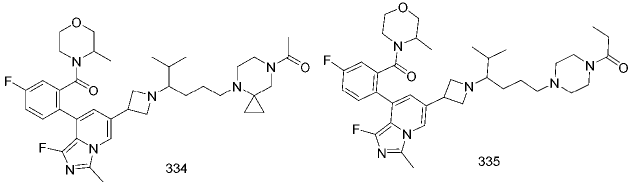

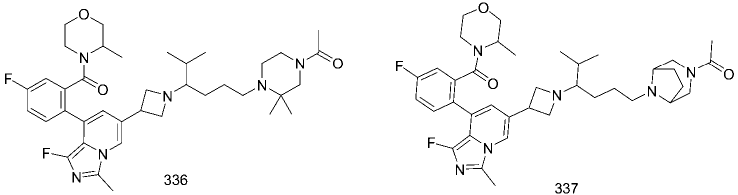

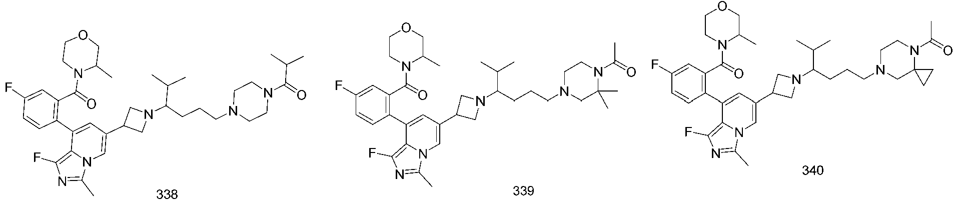

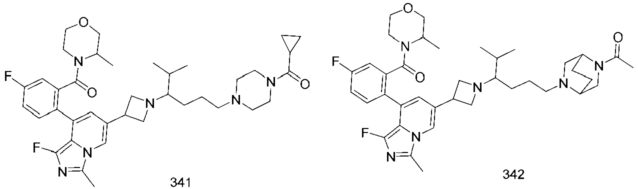

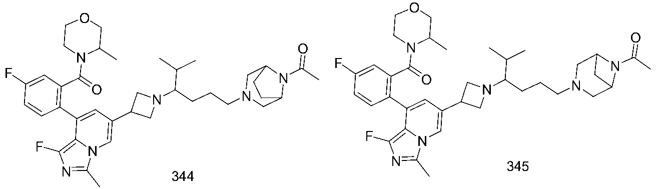

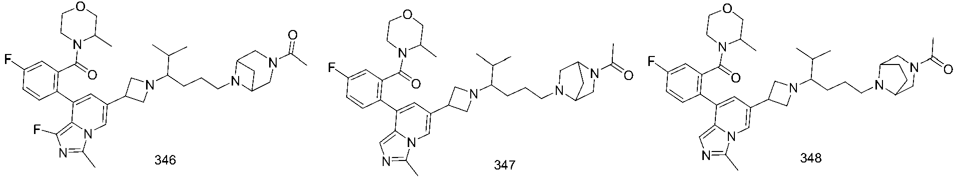

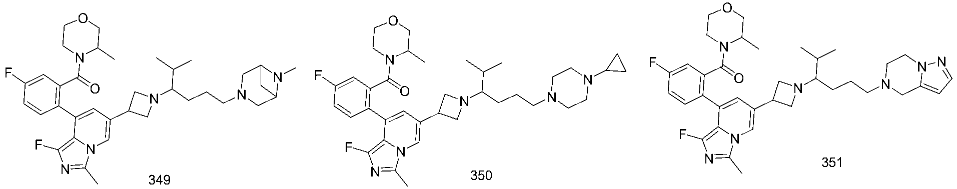

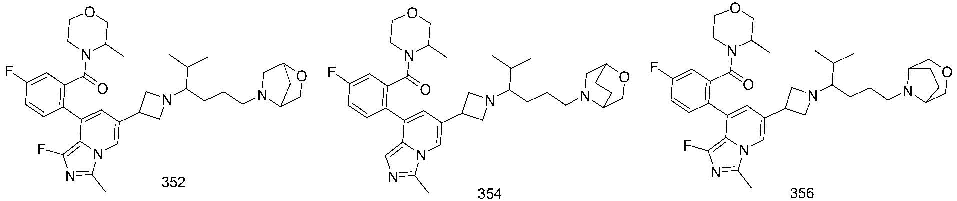

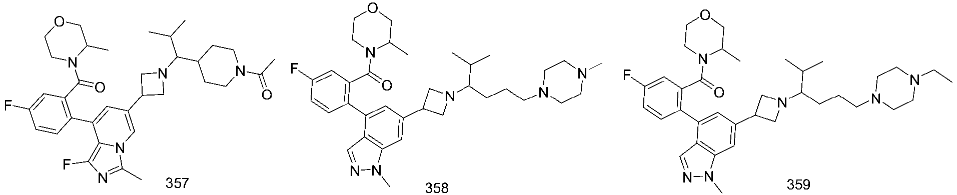

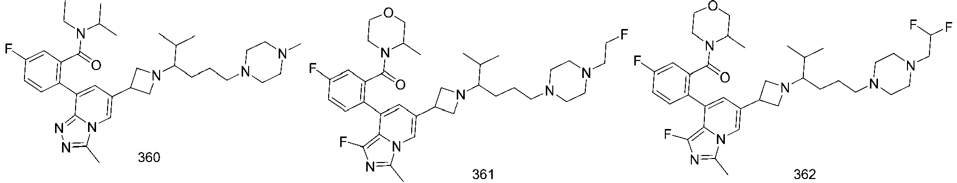

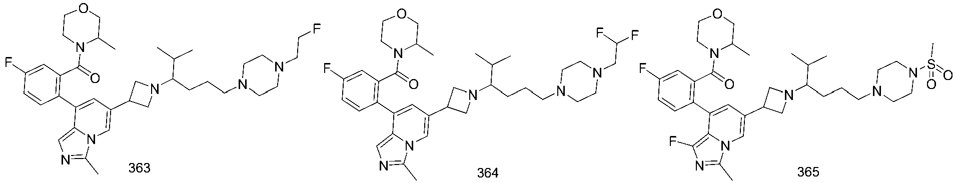

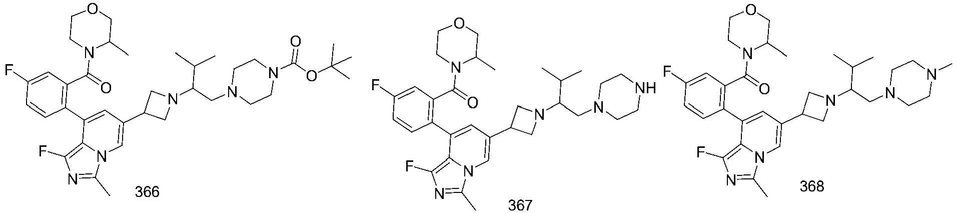

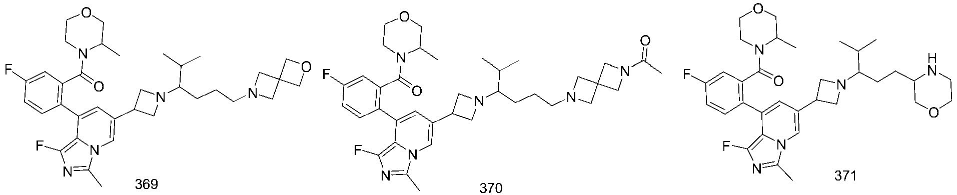

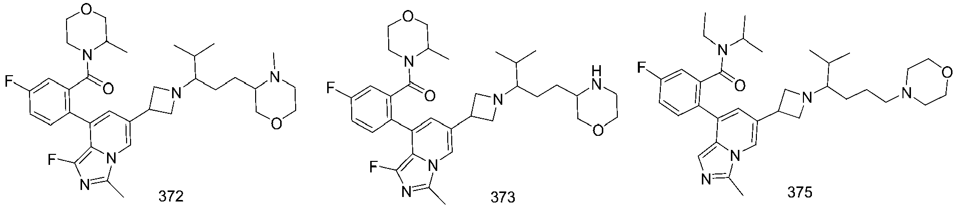

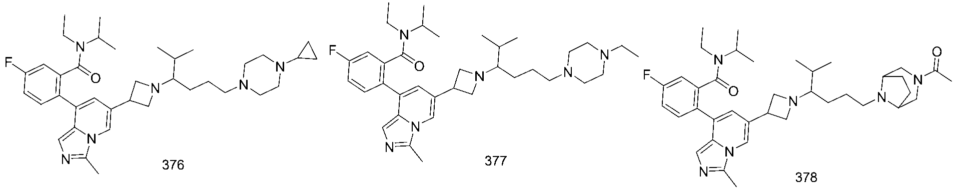

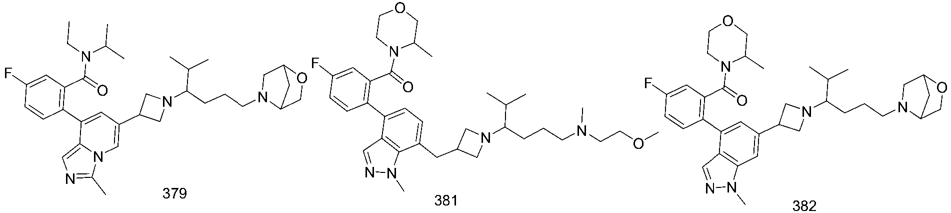

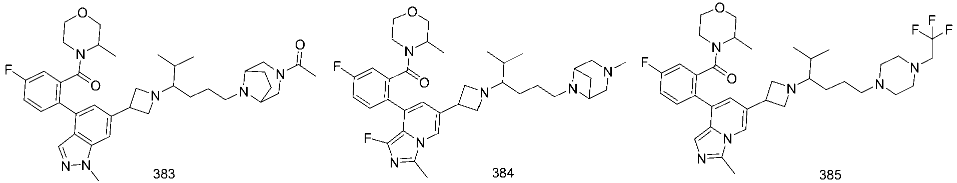

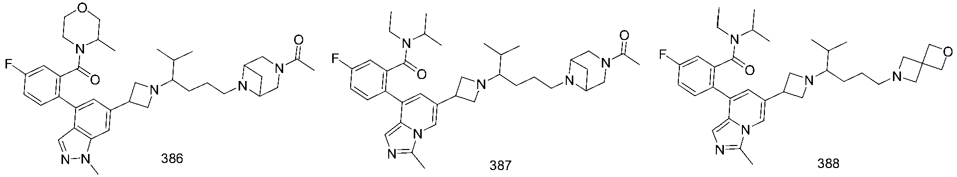

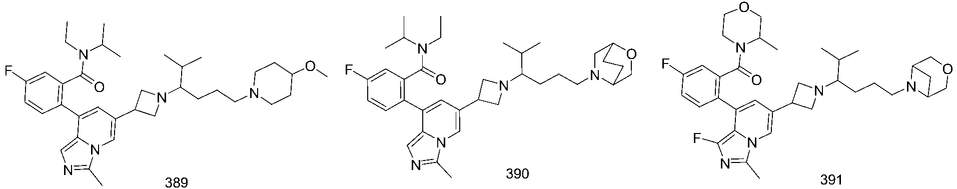

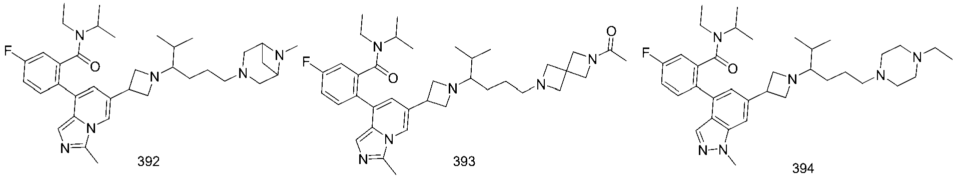

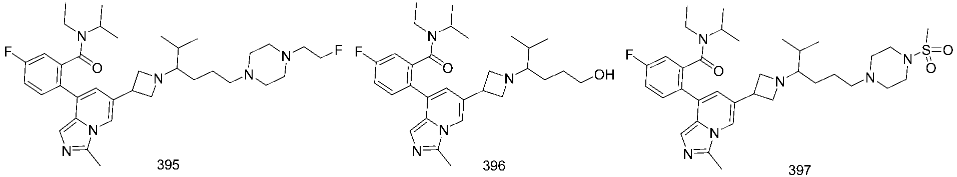

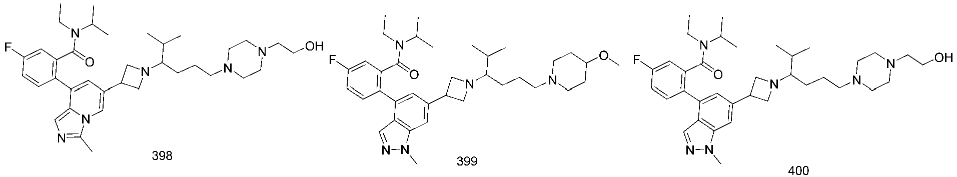

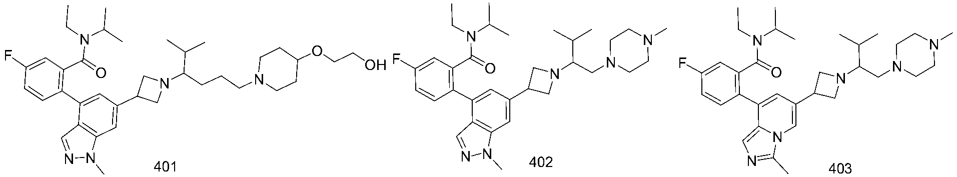

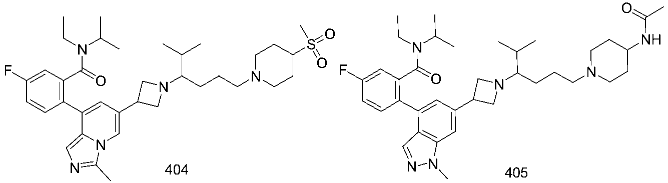

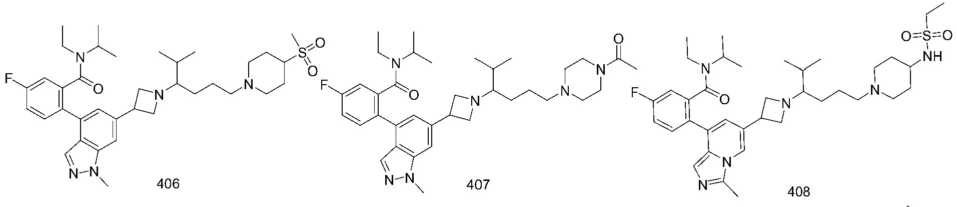

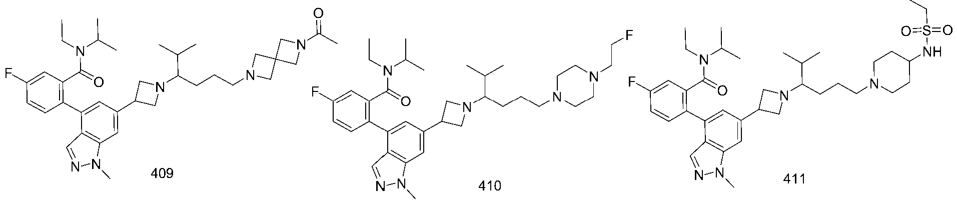

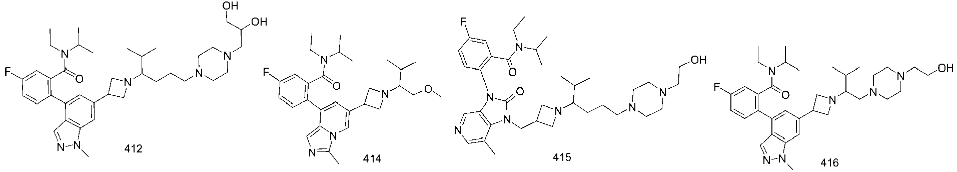

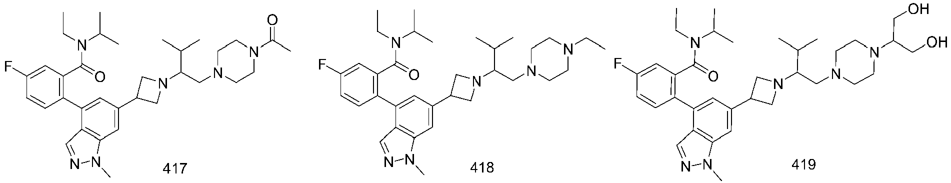

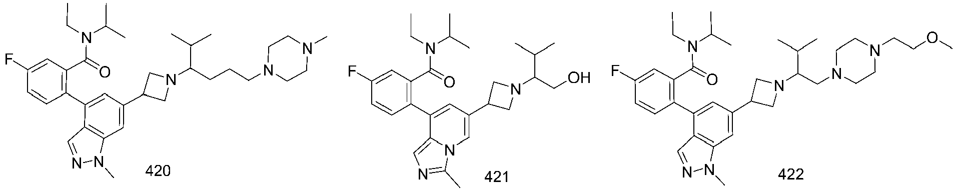

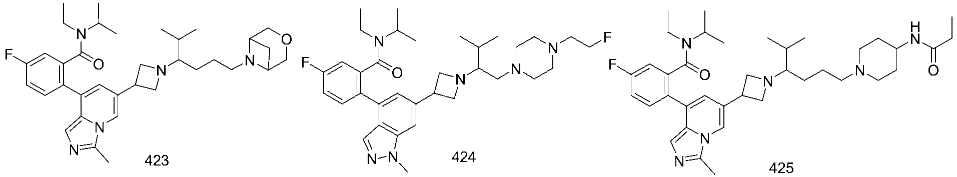

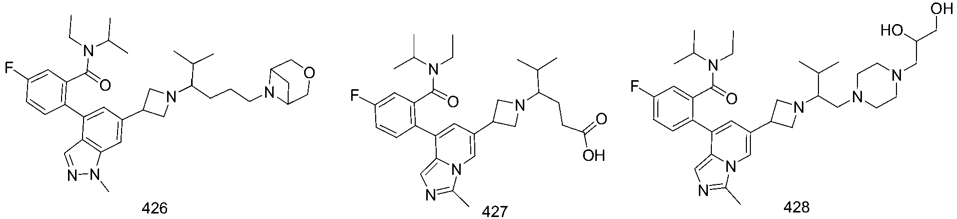

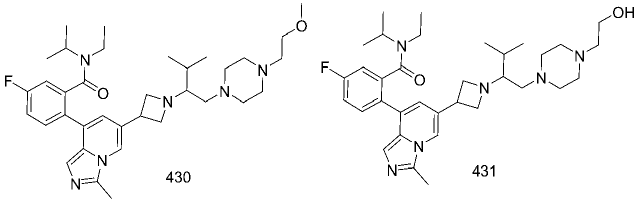

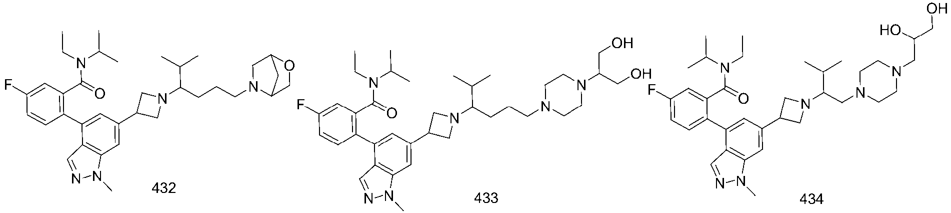

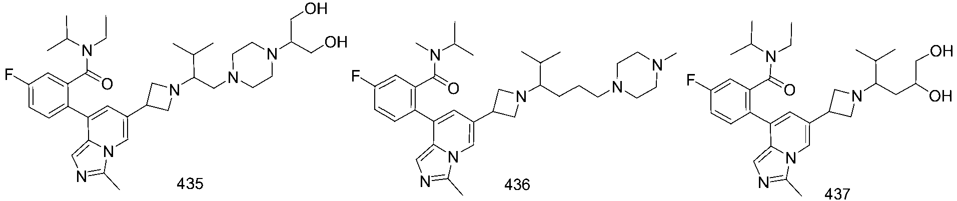

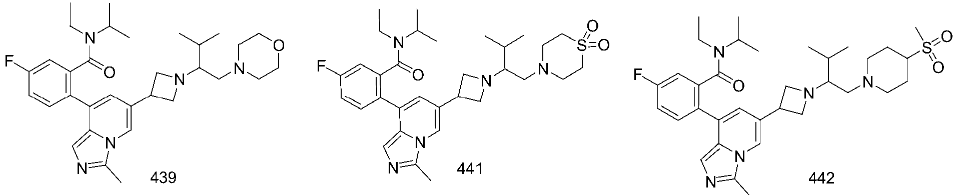

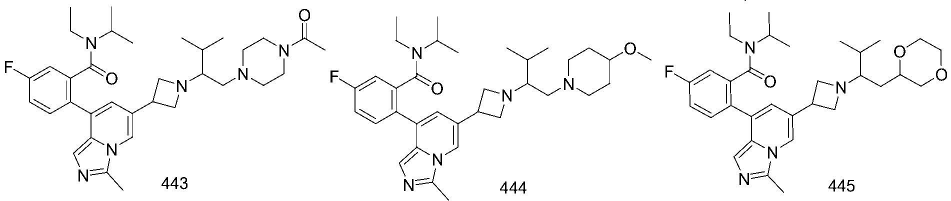

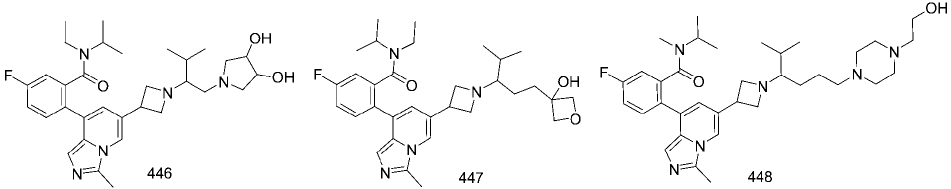

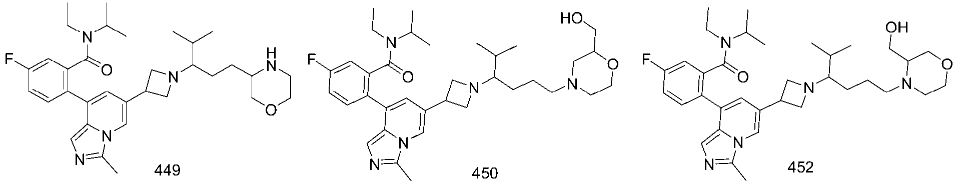

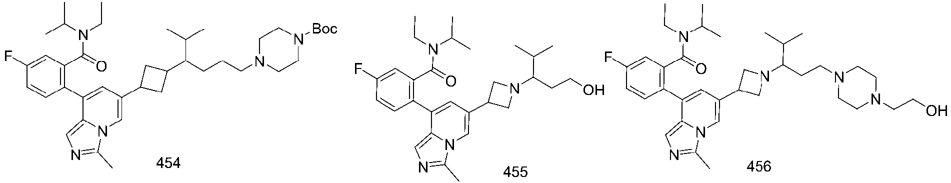

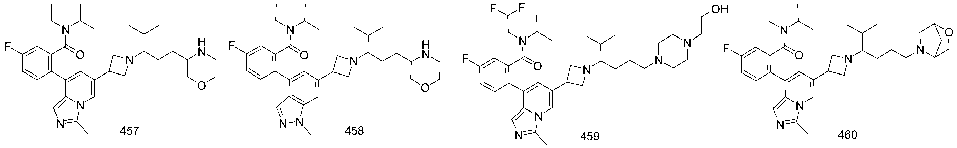

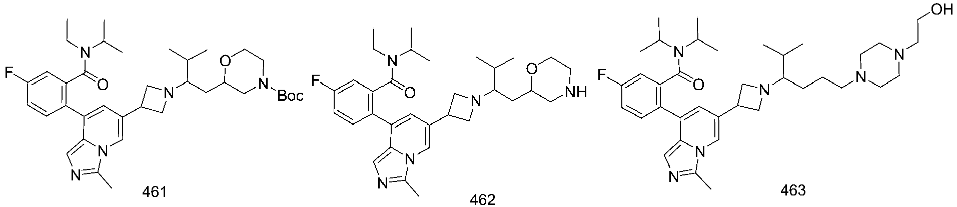

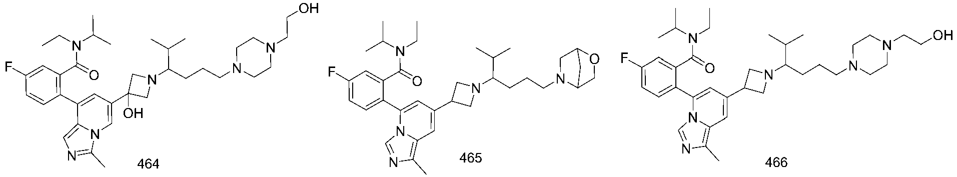

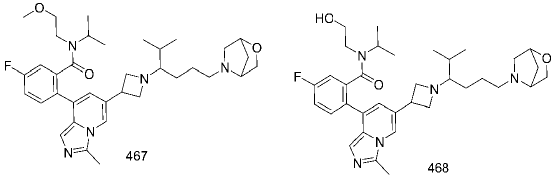

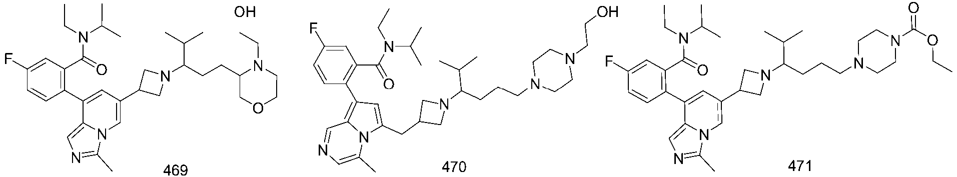

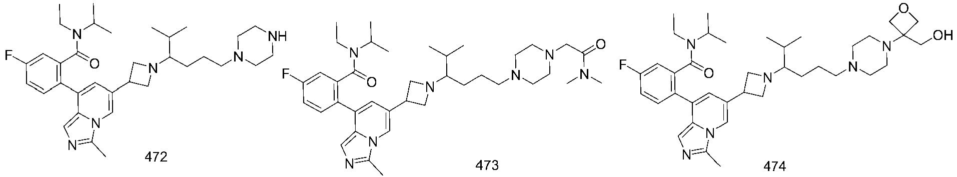

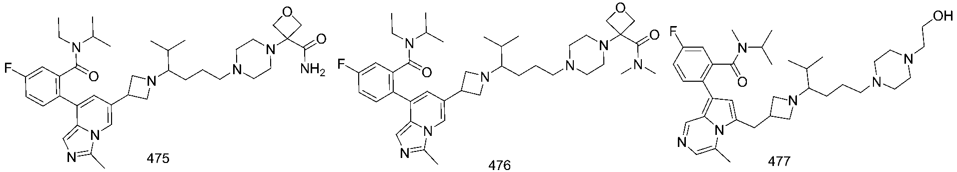

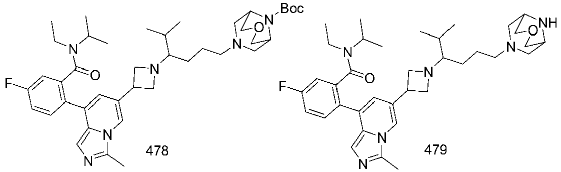

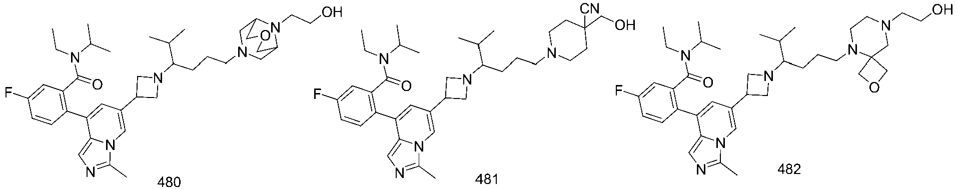

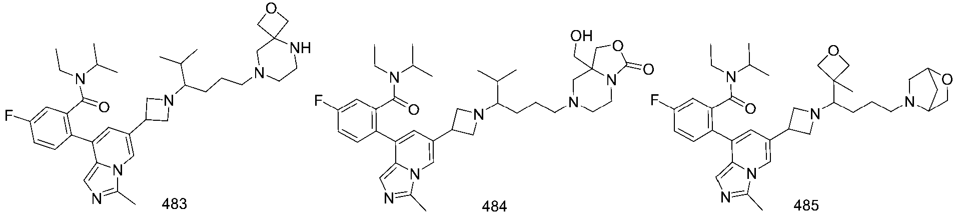

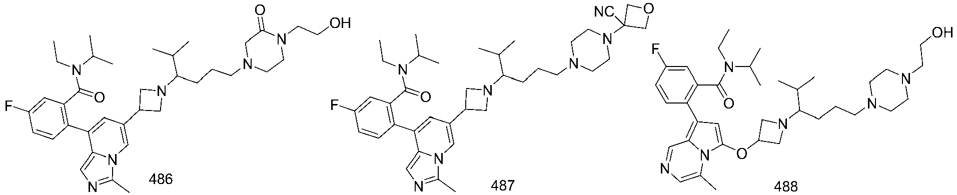

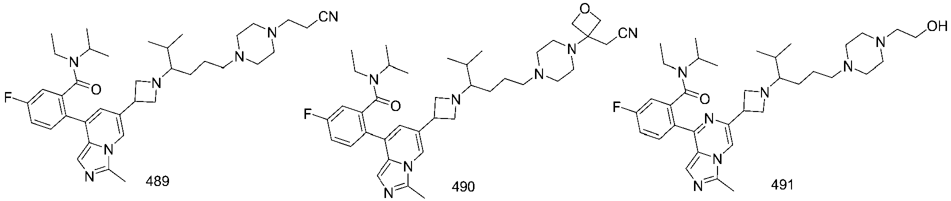

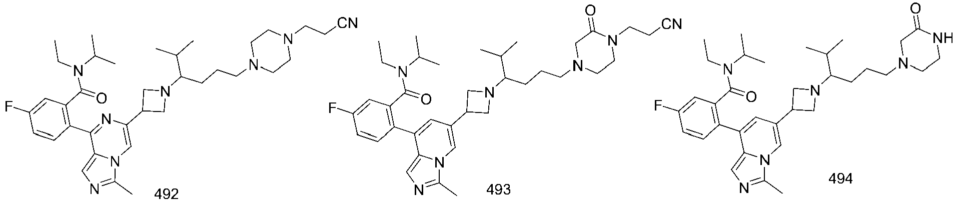

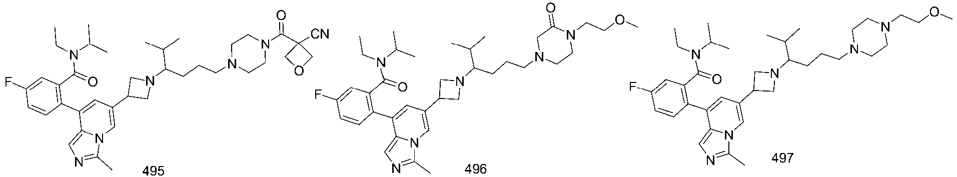

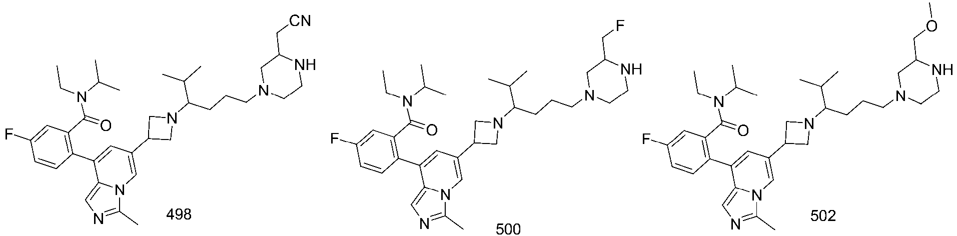

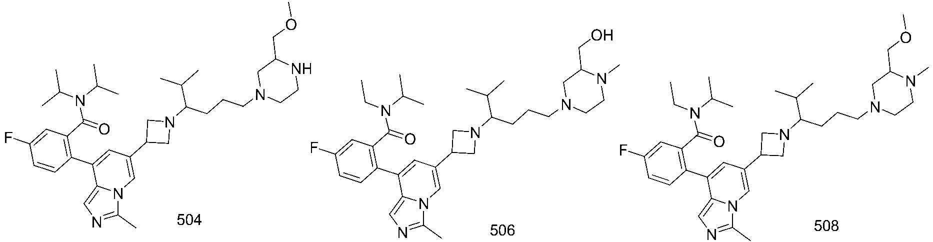

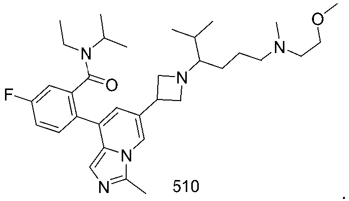

In some embodiments of the present invention, the stereoisomer has a structure of:

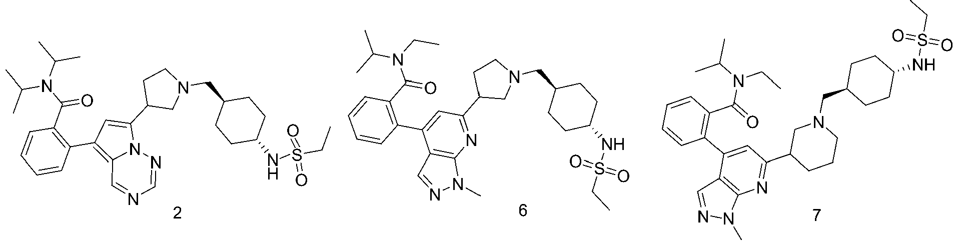

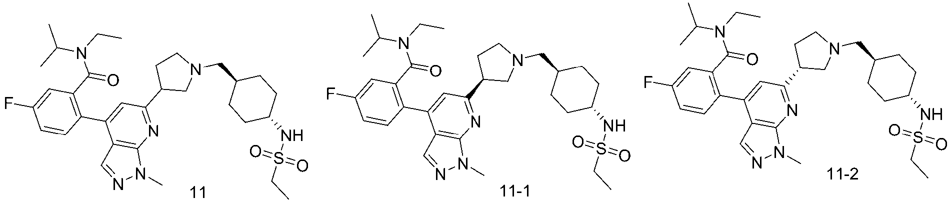

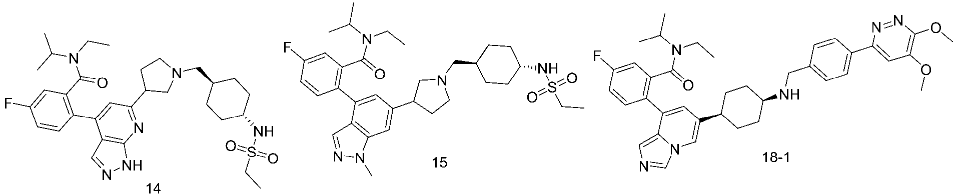

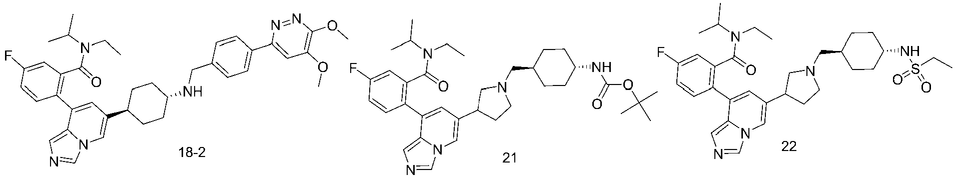

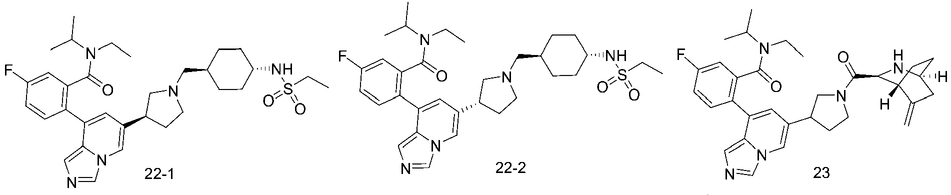

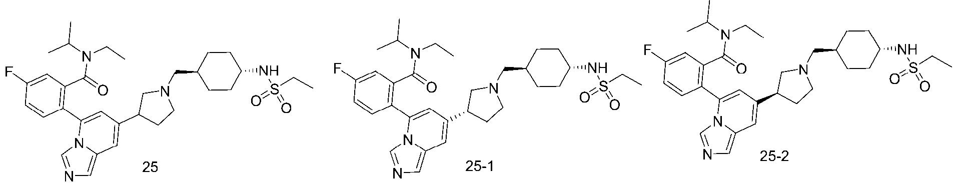

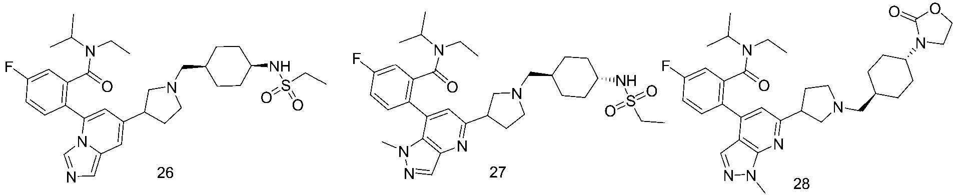

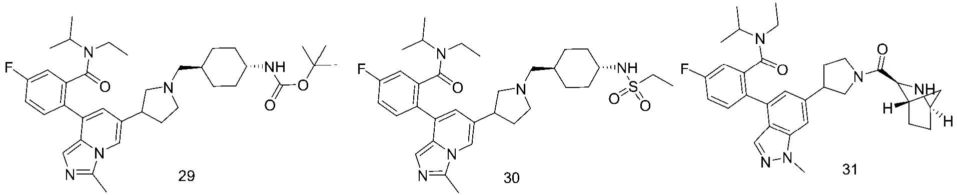

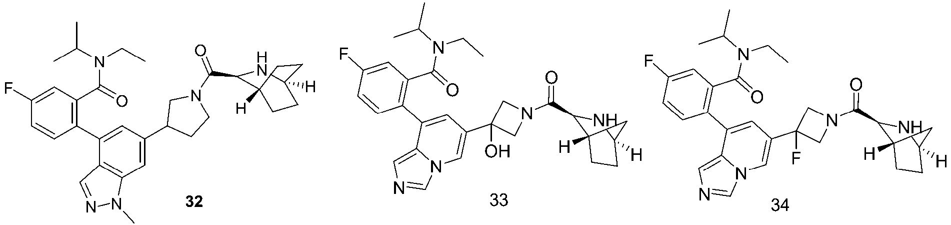

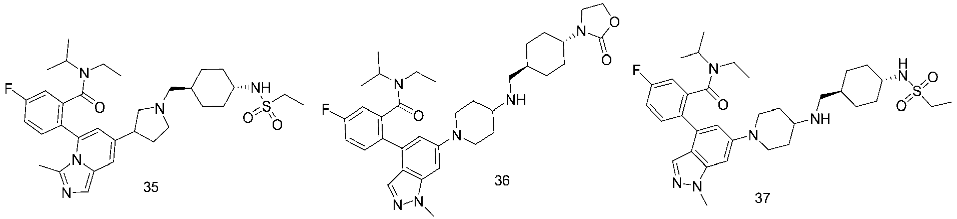

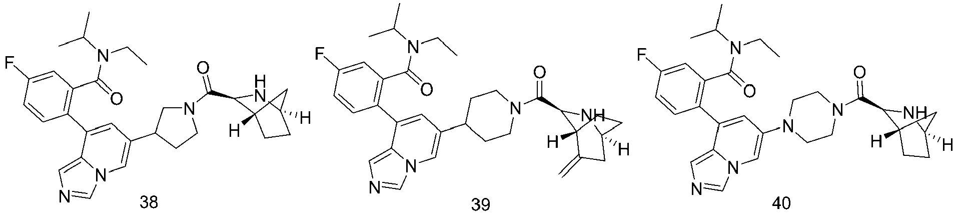

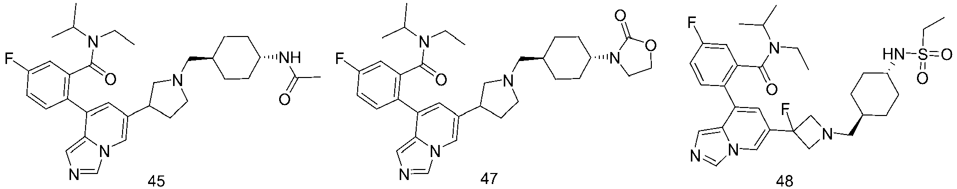

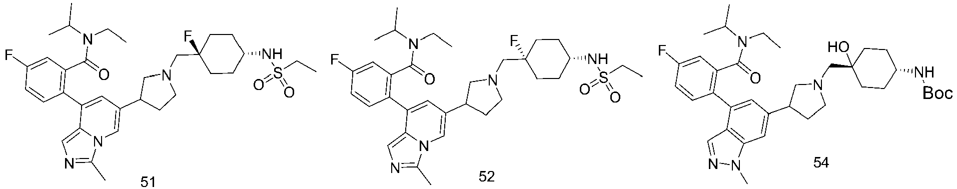

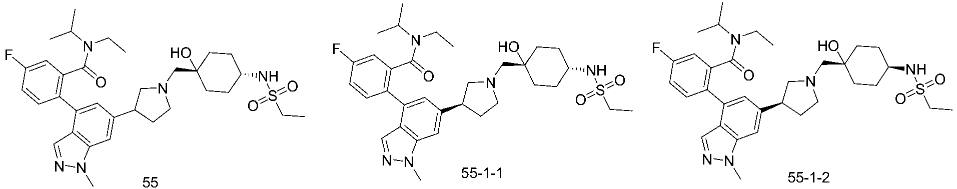

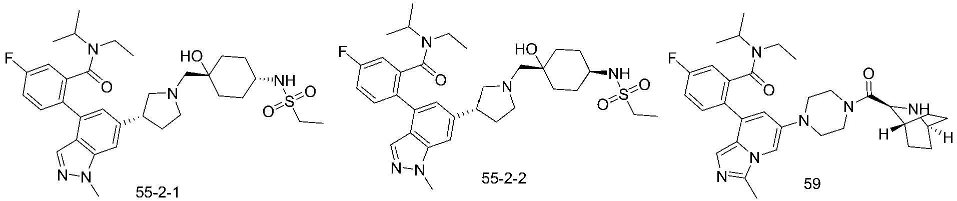

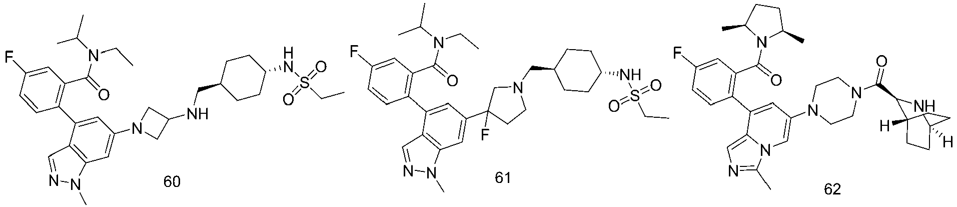

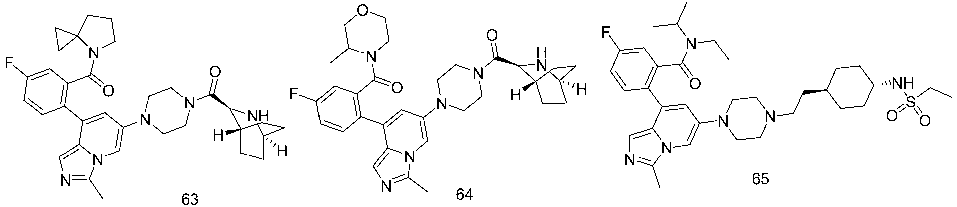

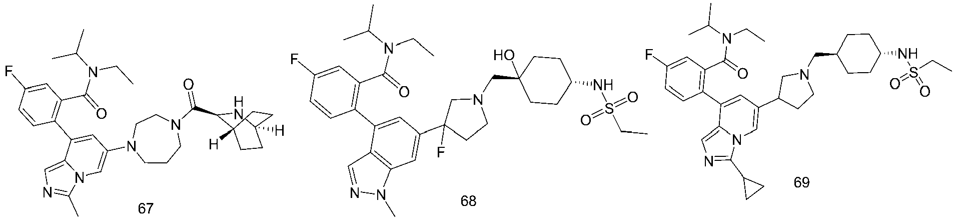

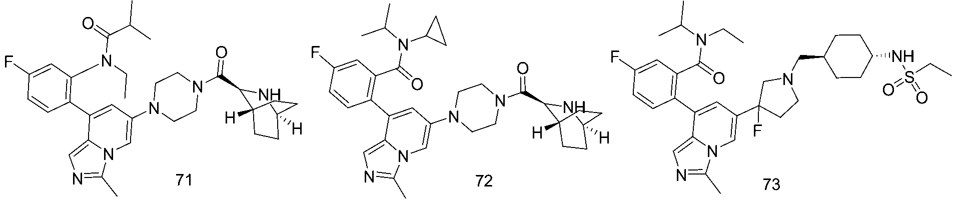

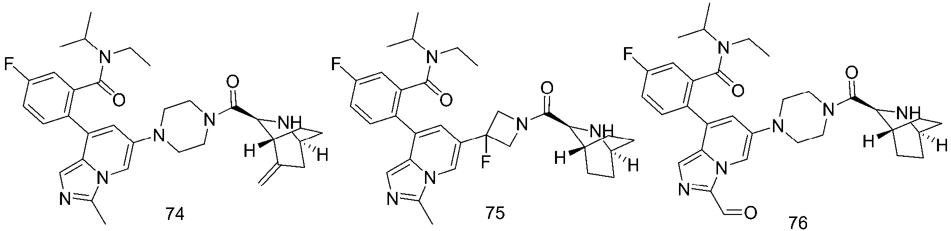

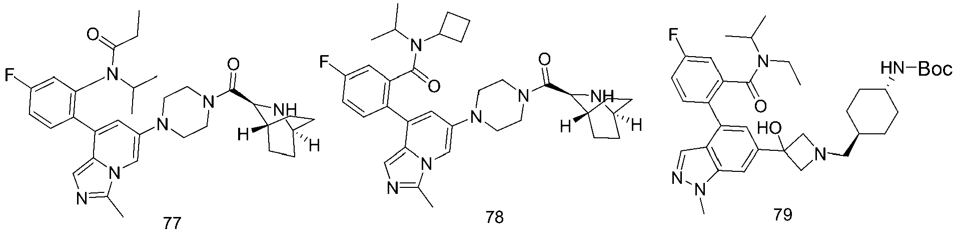

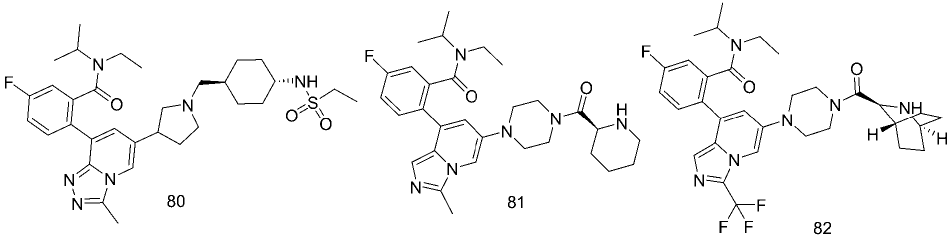

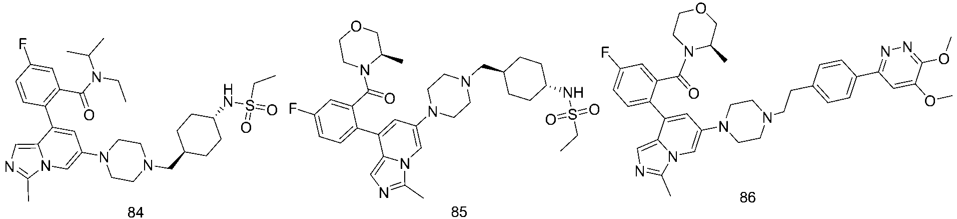

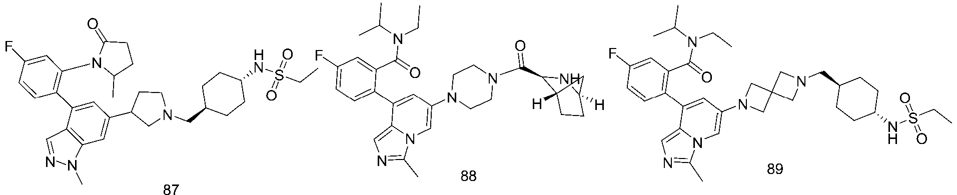

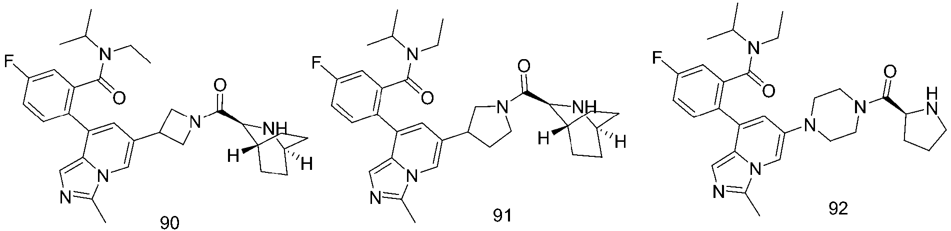

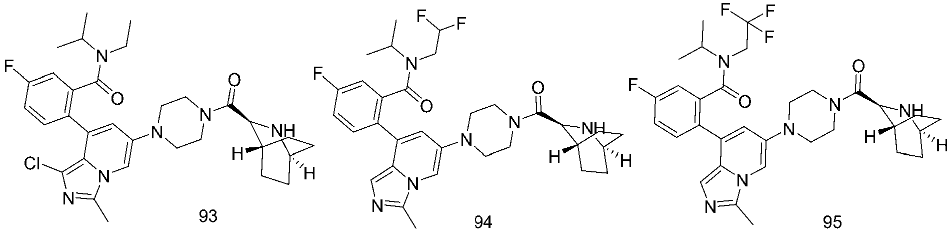

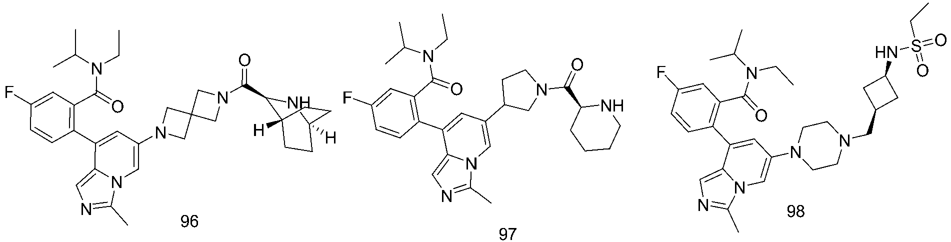

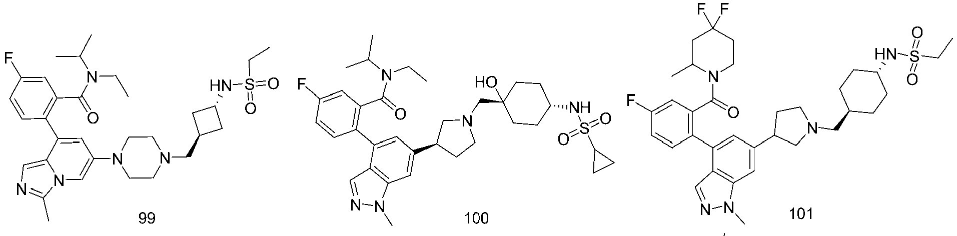

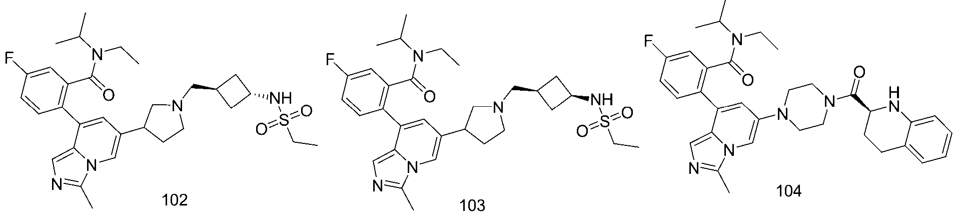

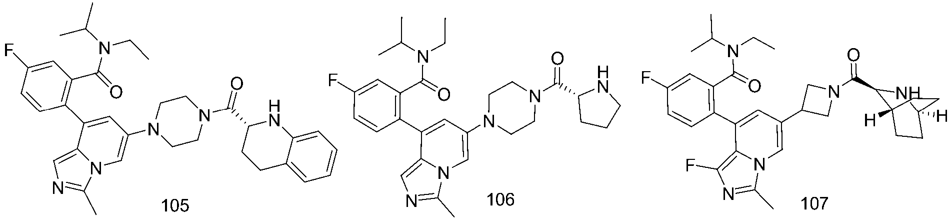

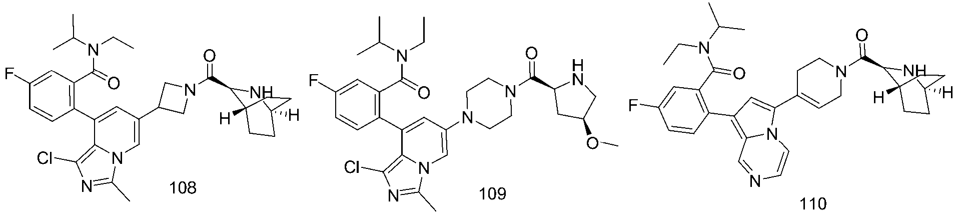

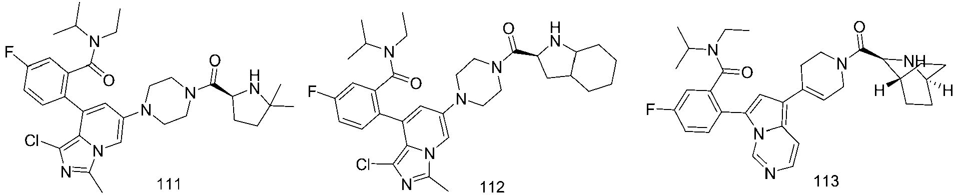

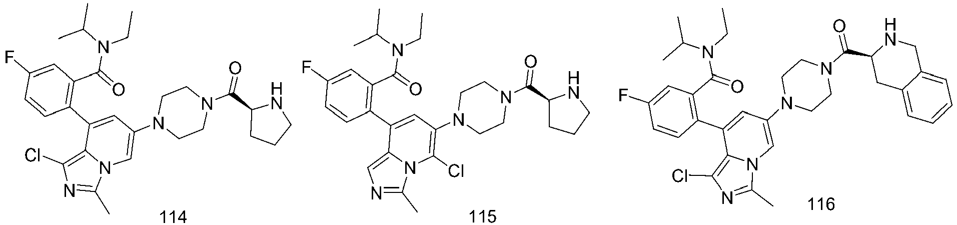

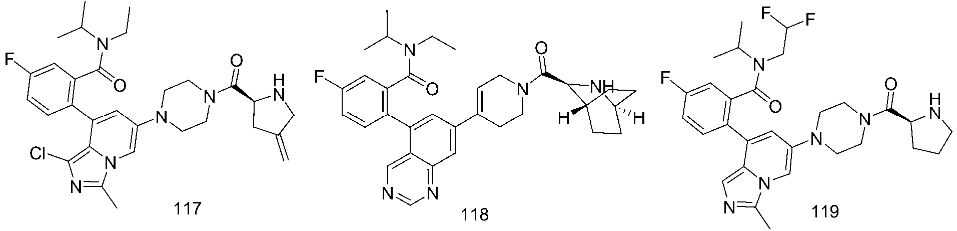

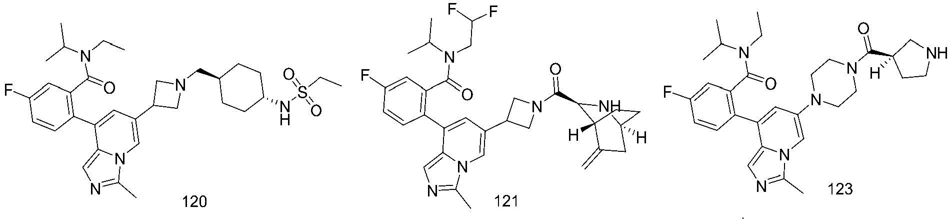

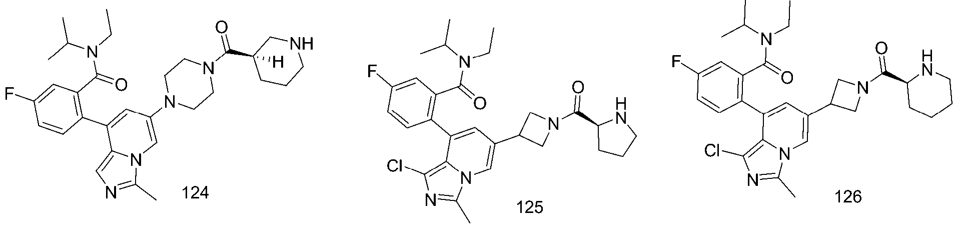

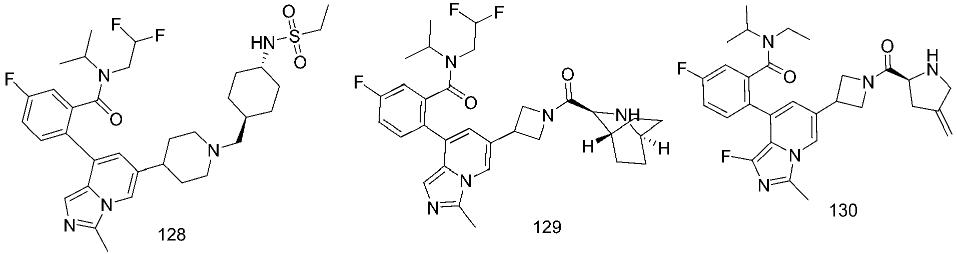

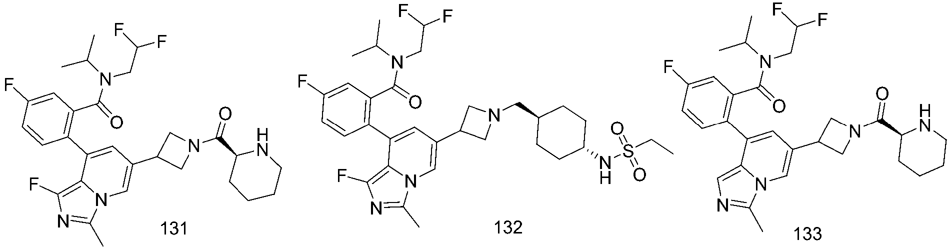

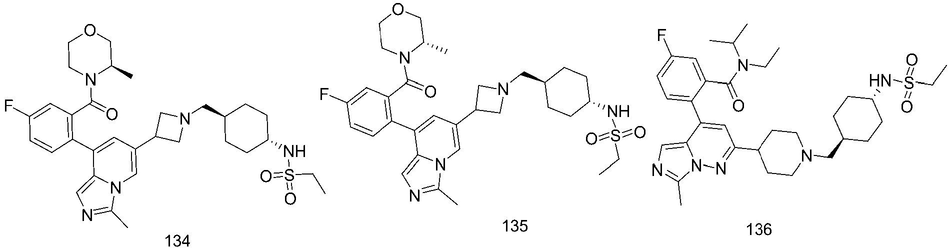

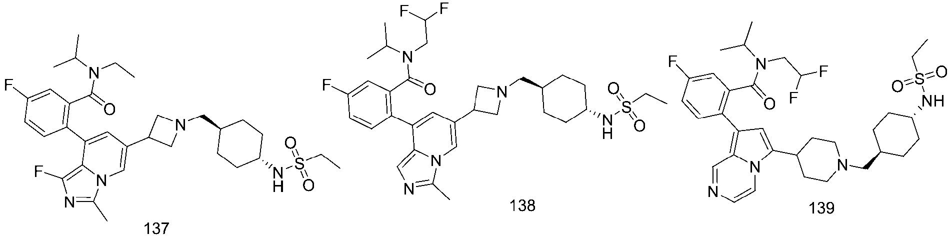

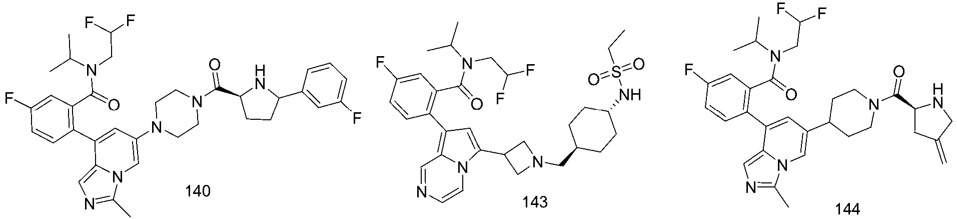

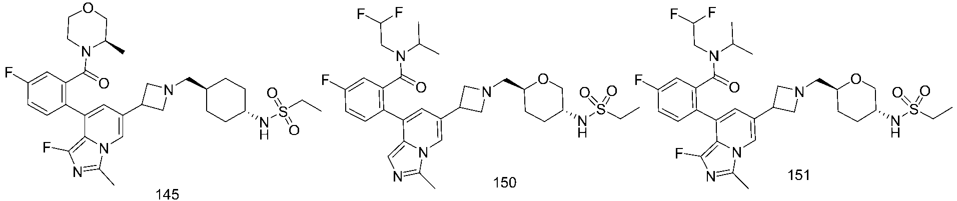

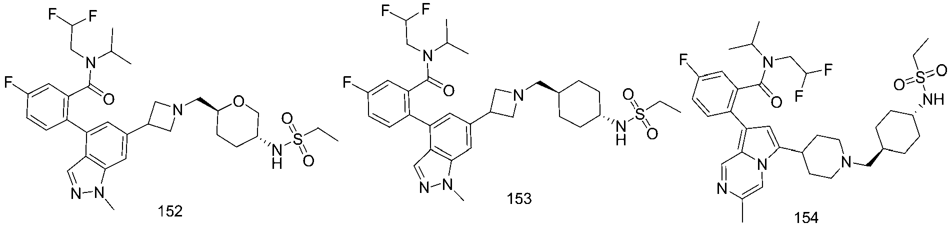

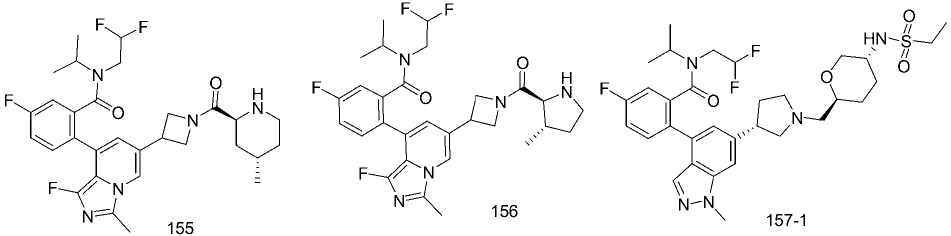

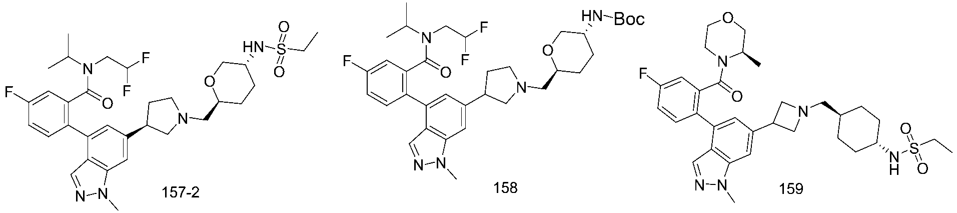

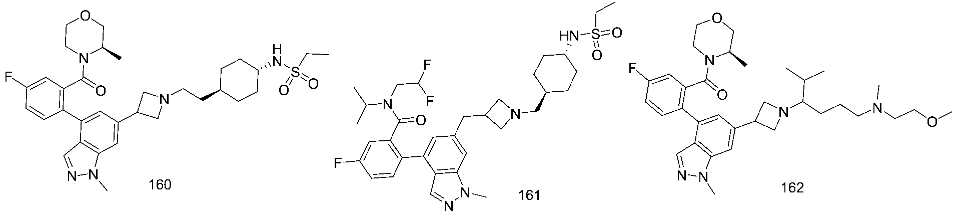

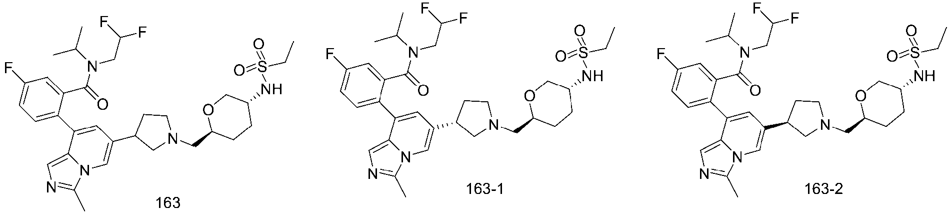

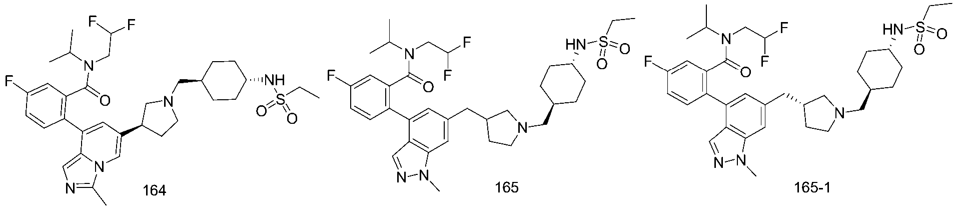

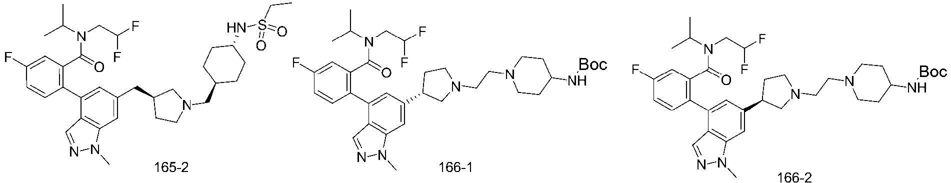

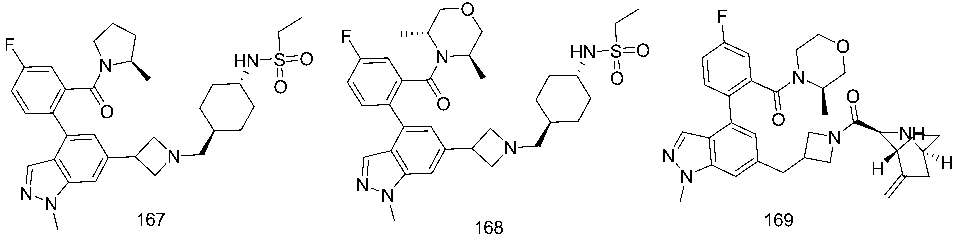

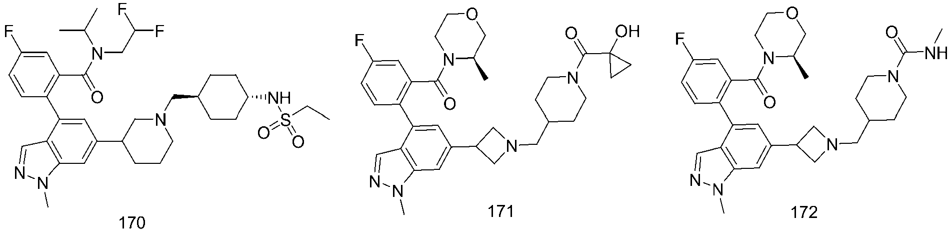

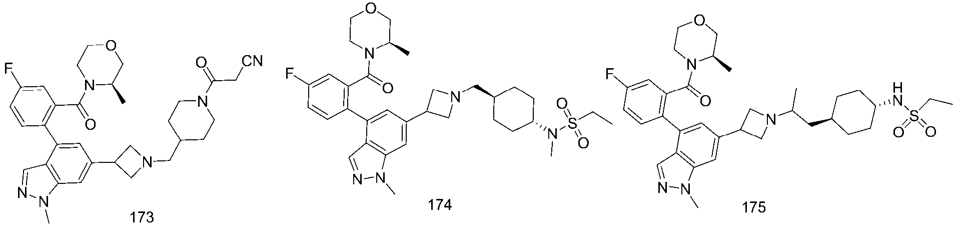

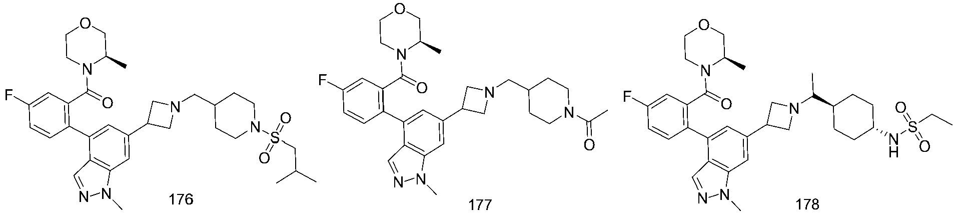

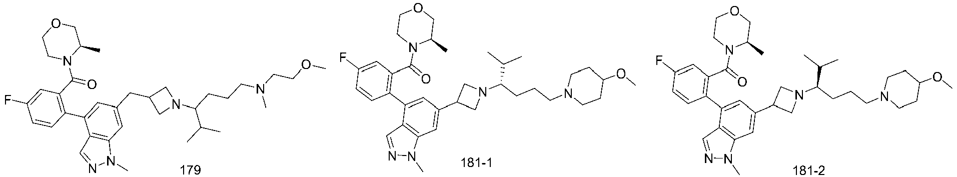

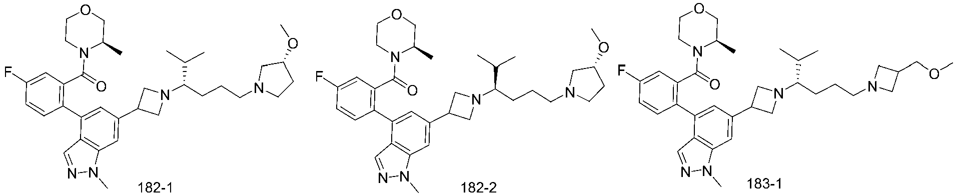

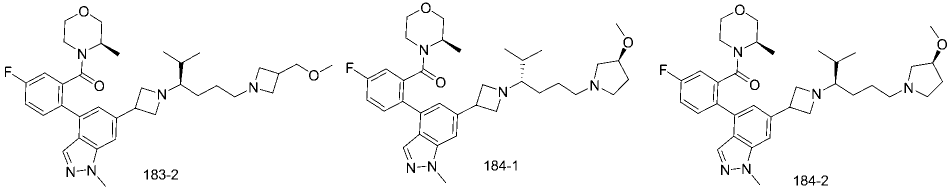

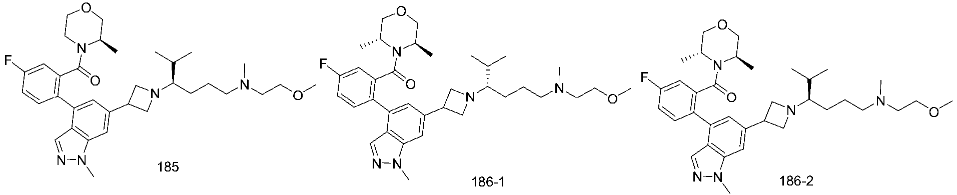

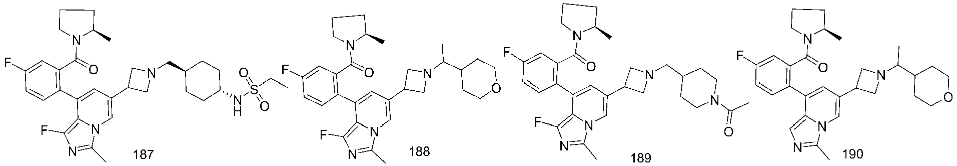

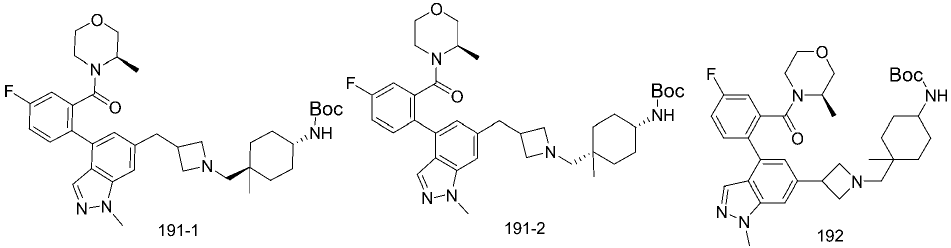

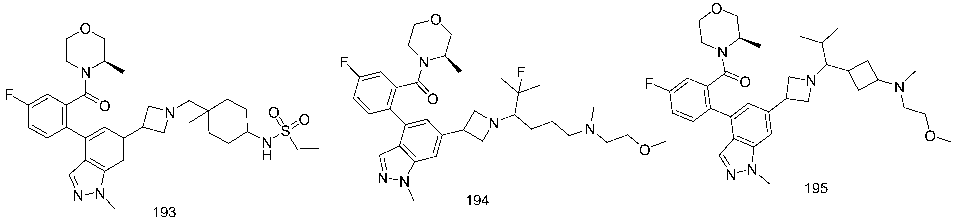

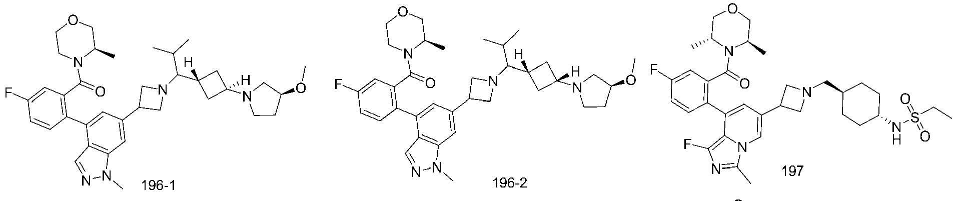

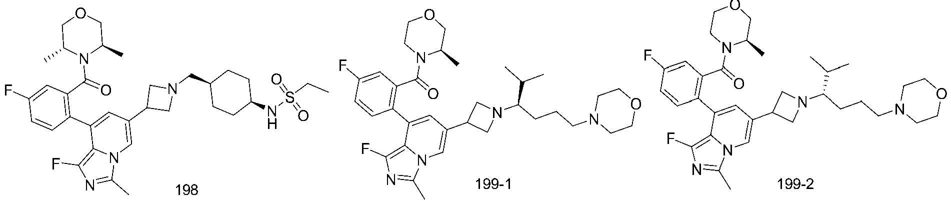

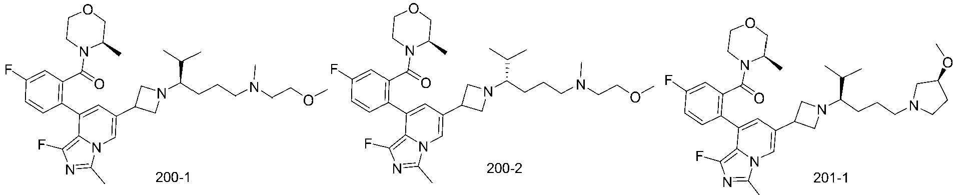

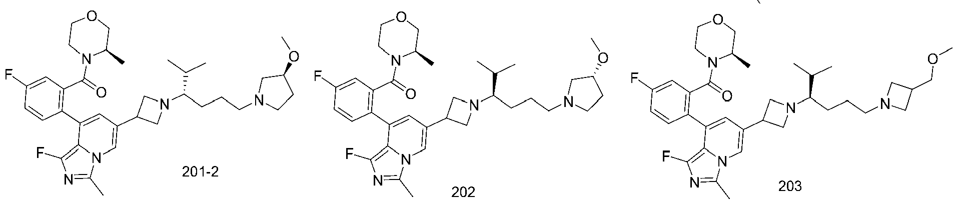

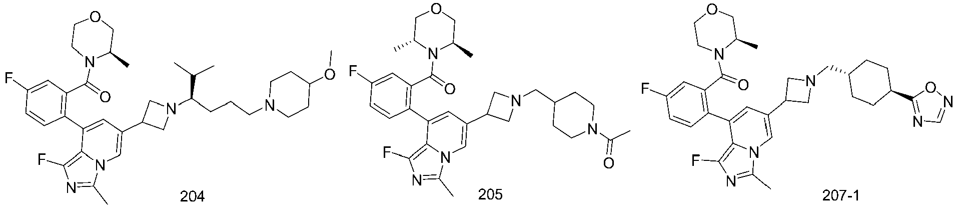

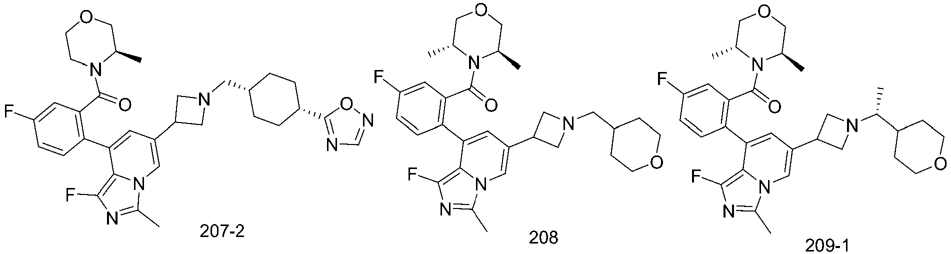

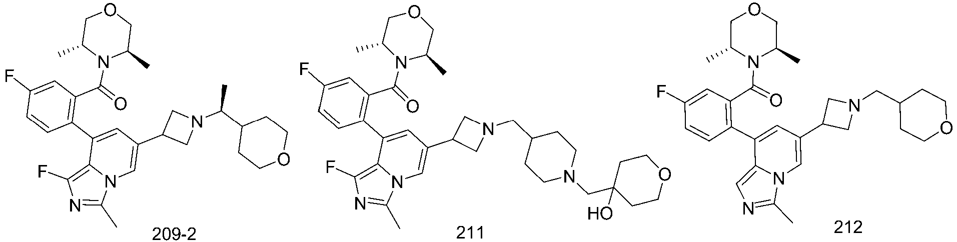

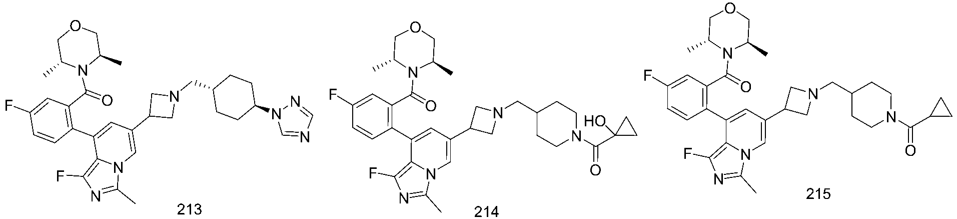

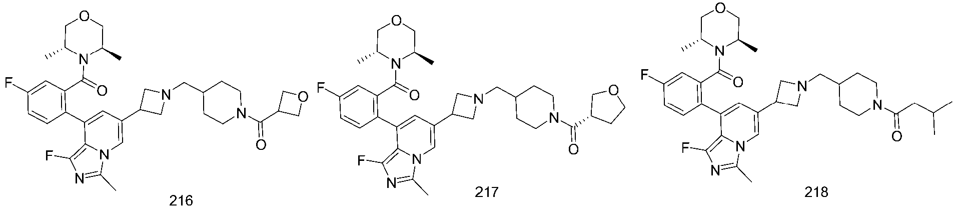

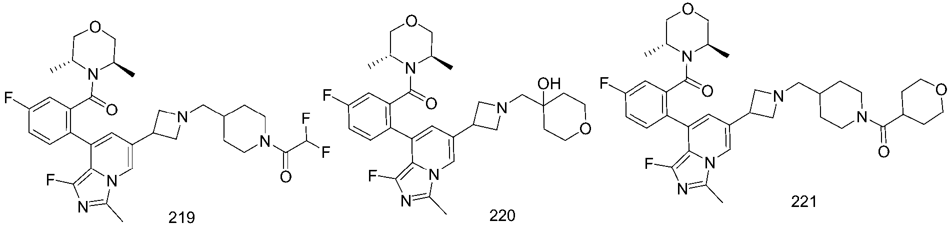

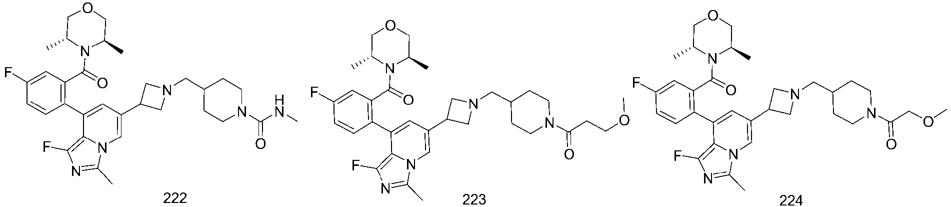

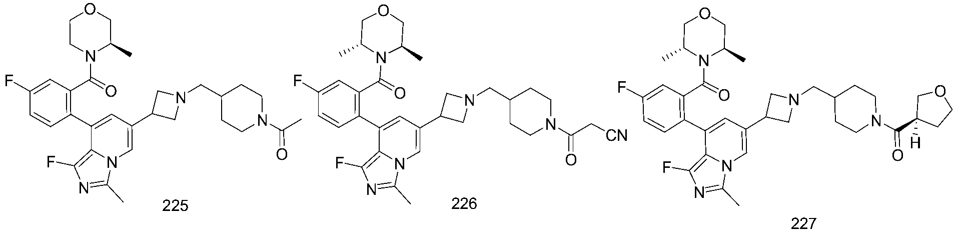

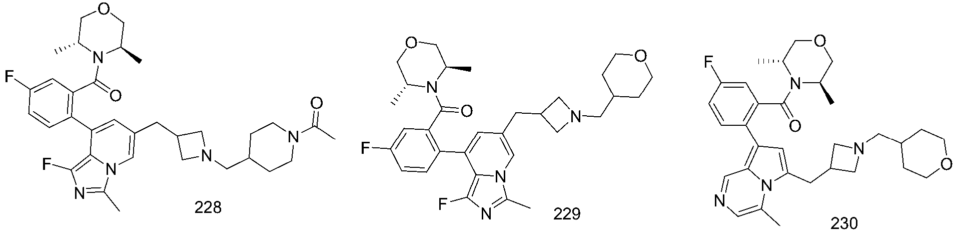

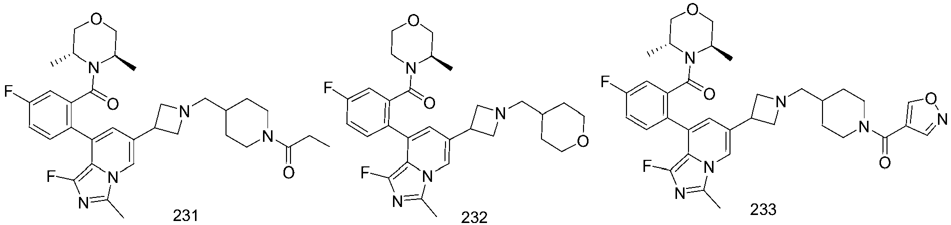

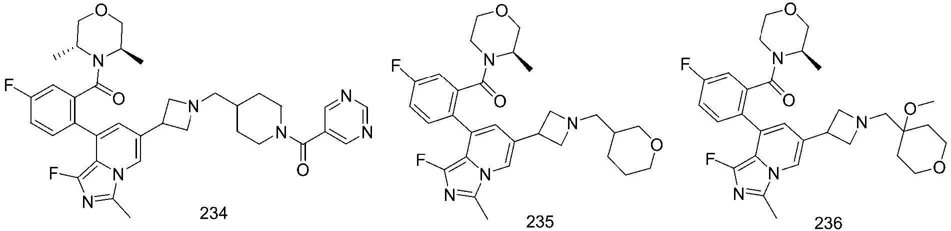

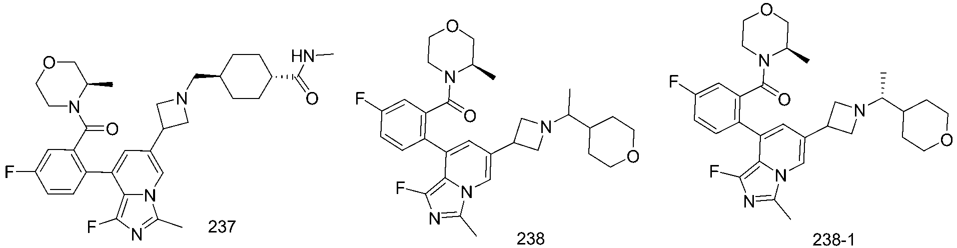

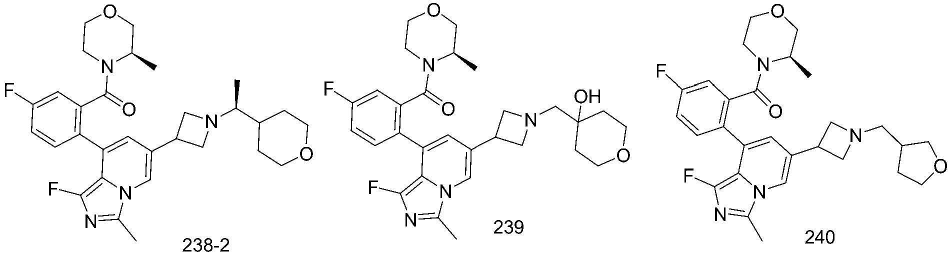

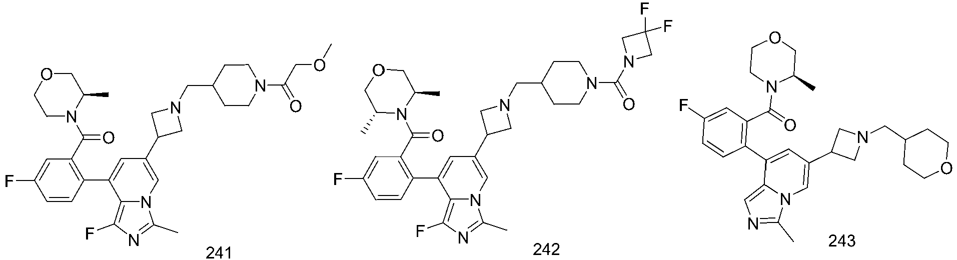

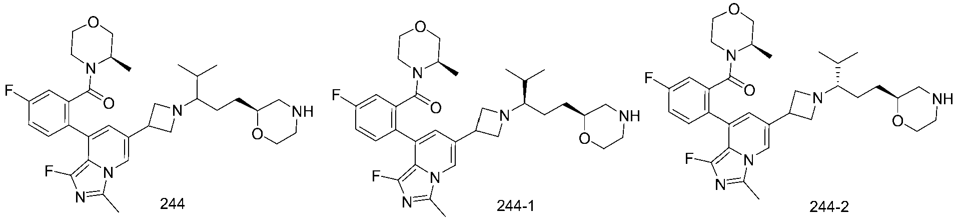

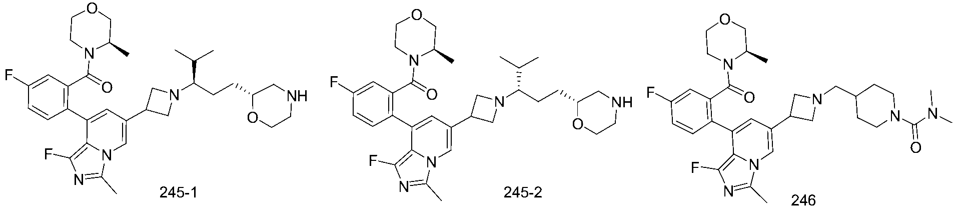

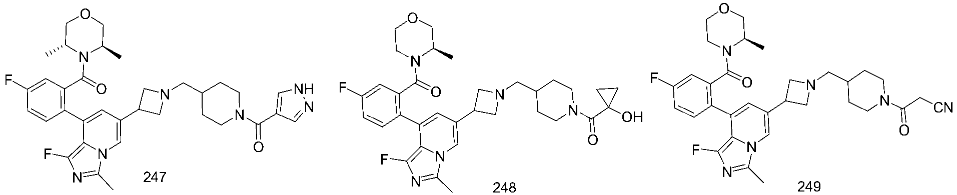

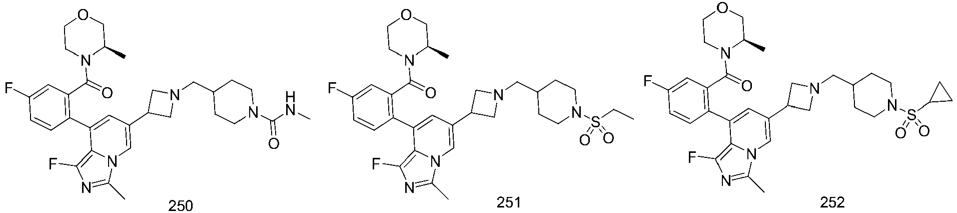

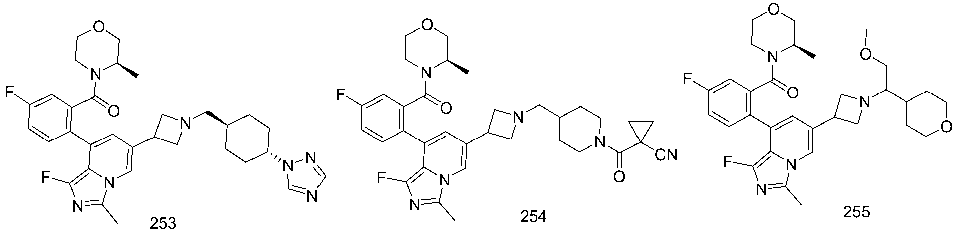

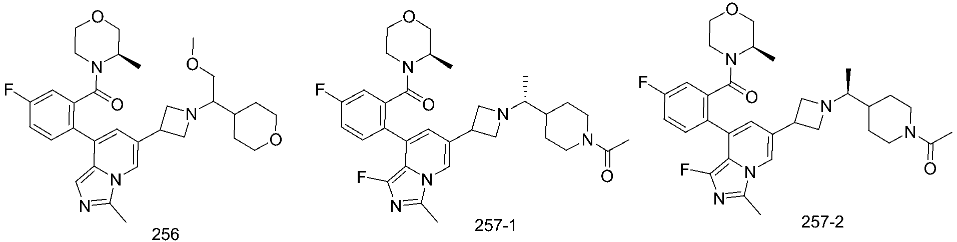

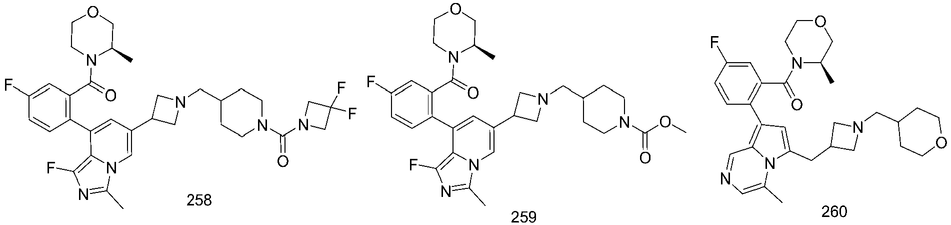

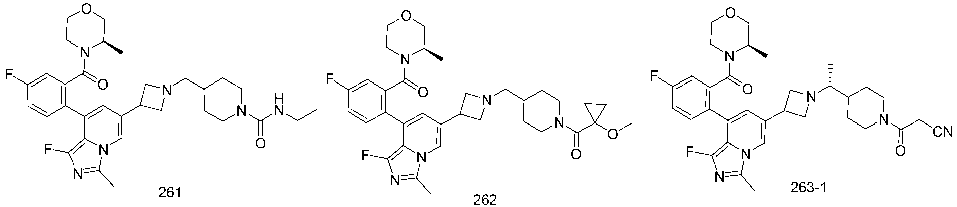

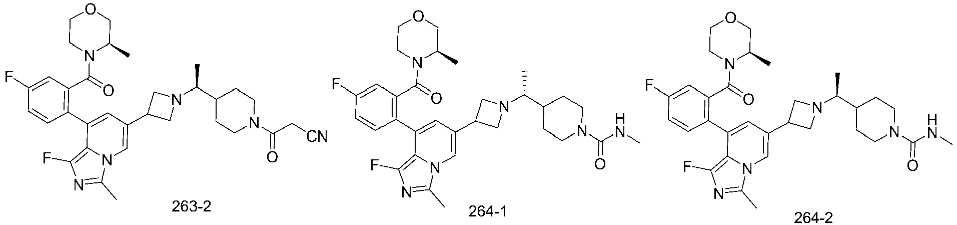

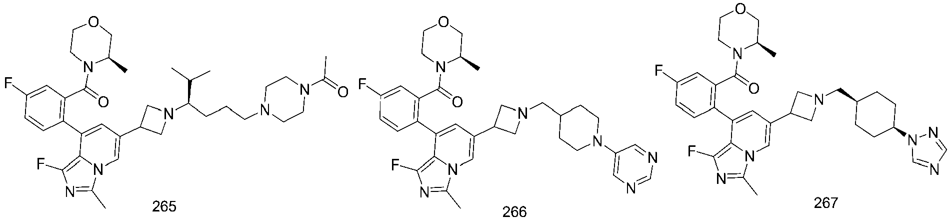

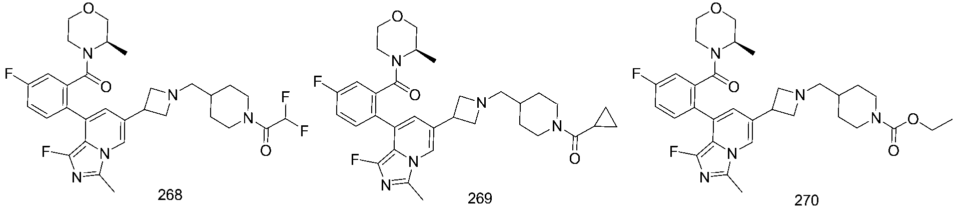

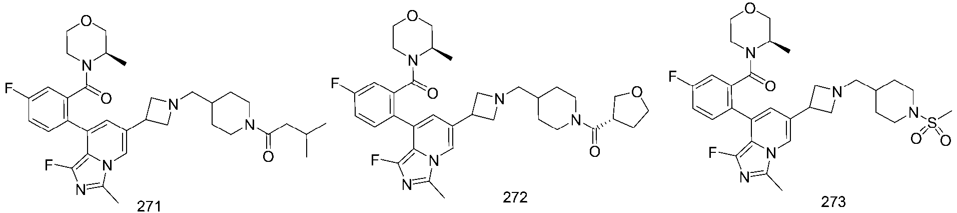

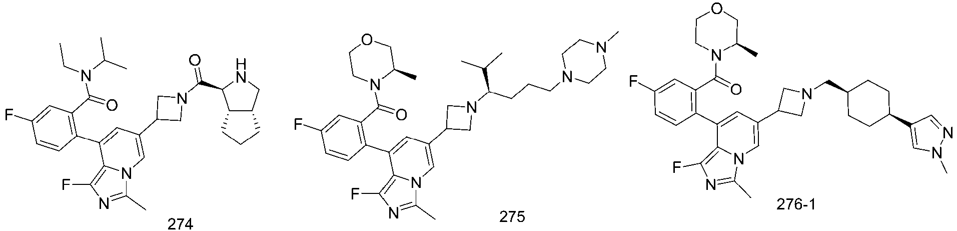

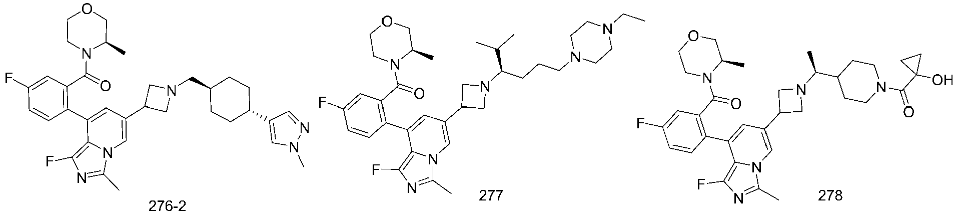

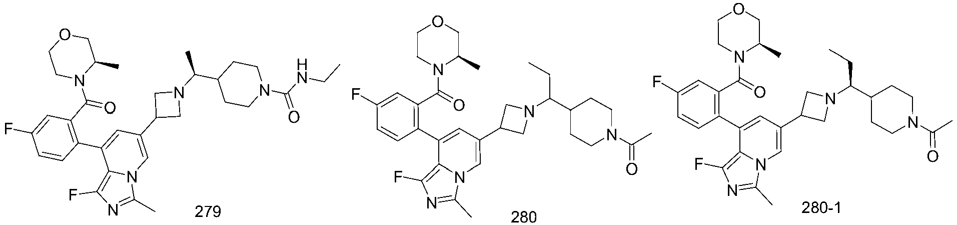

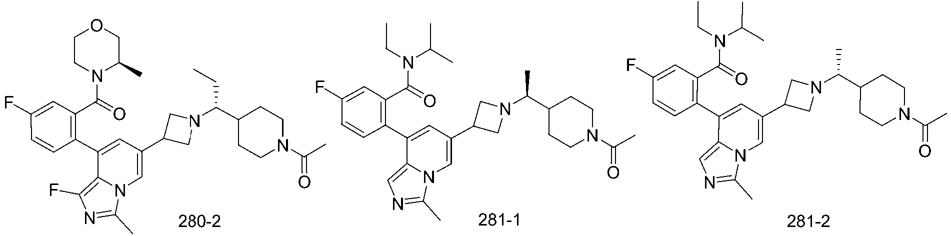

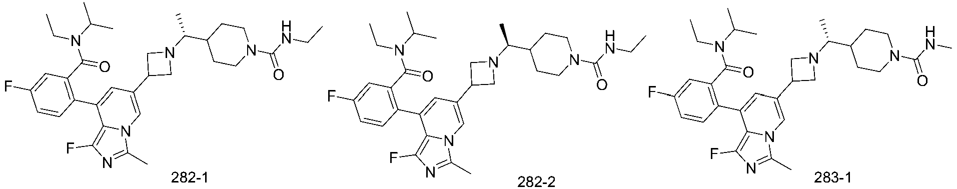

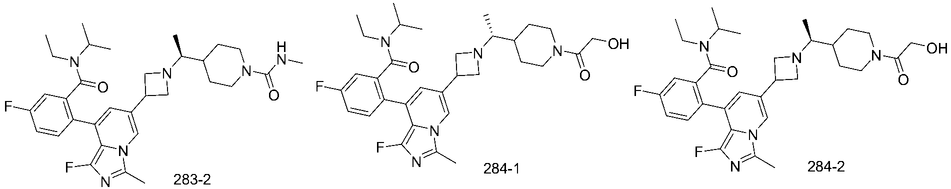

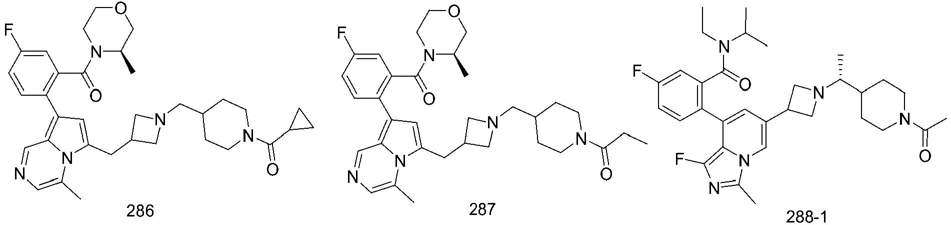

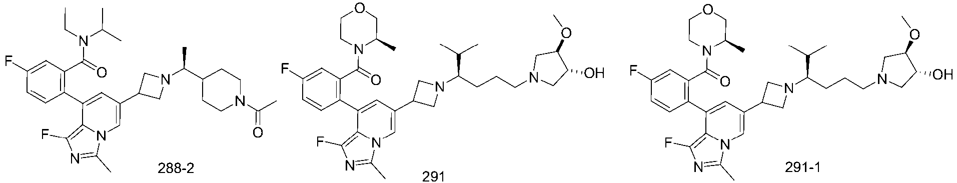

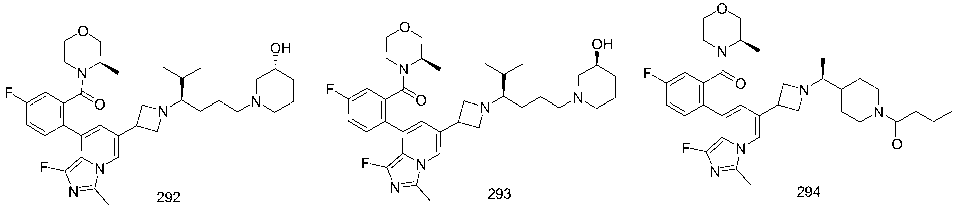

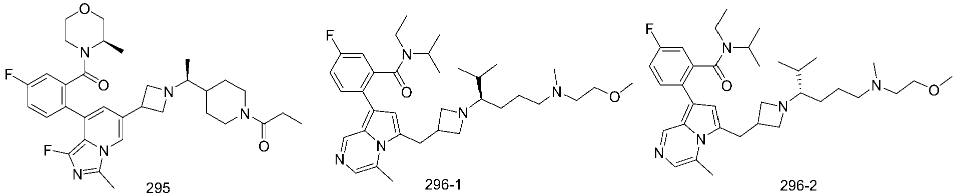

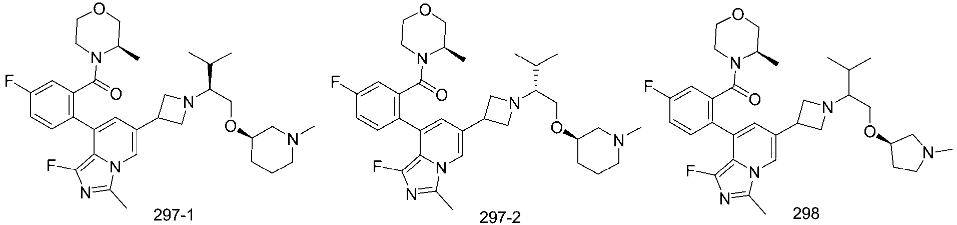

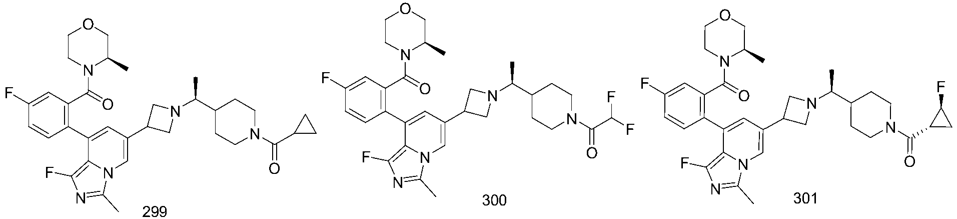

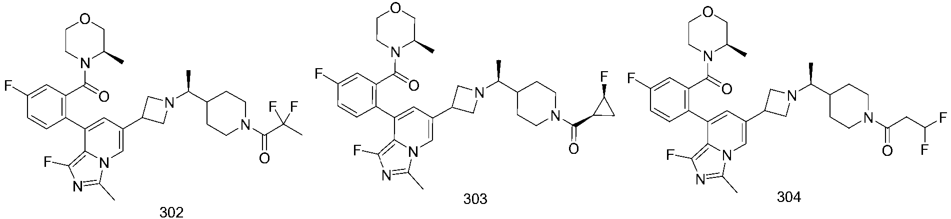

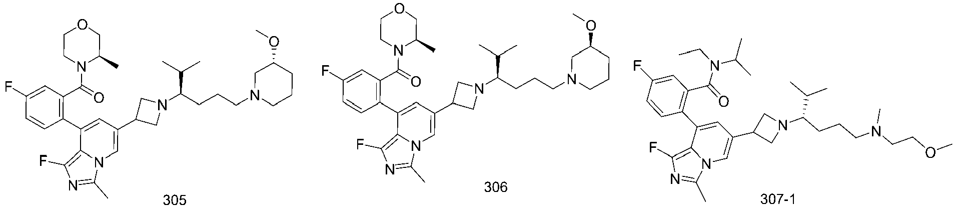

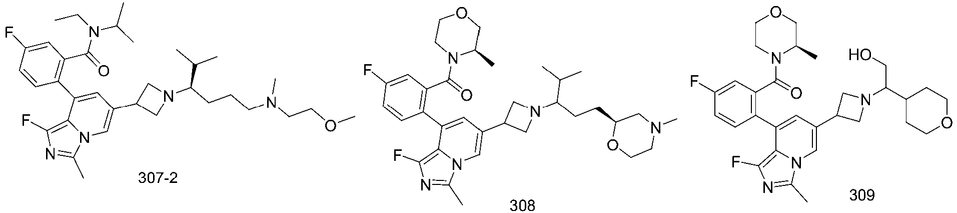

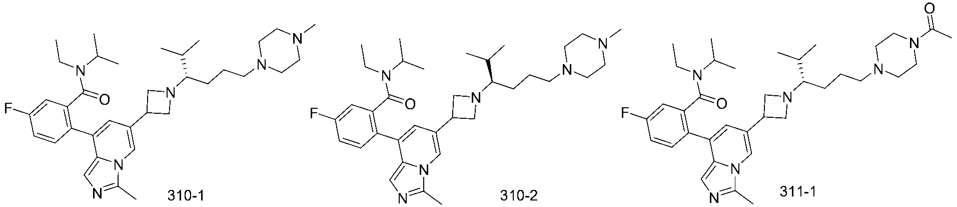

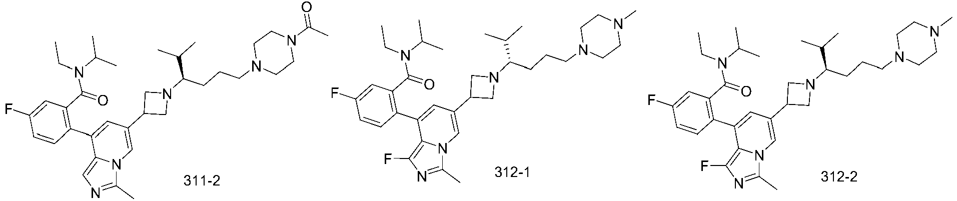

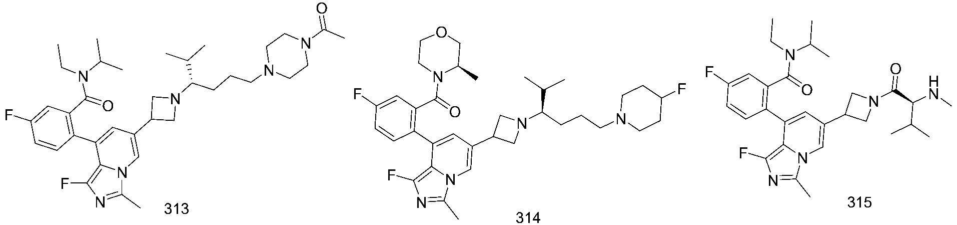

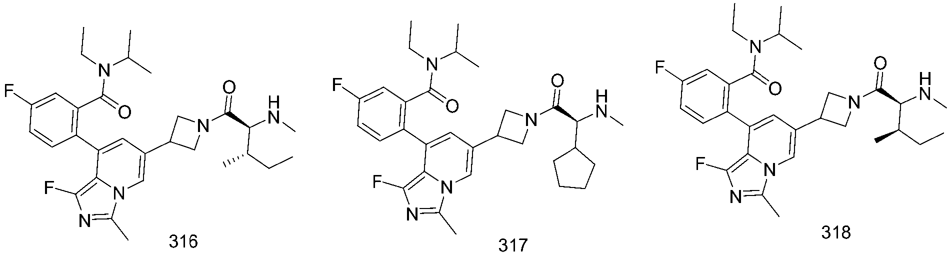

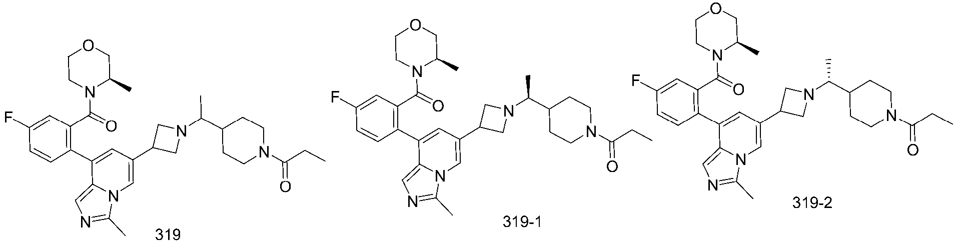

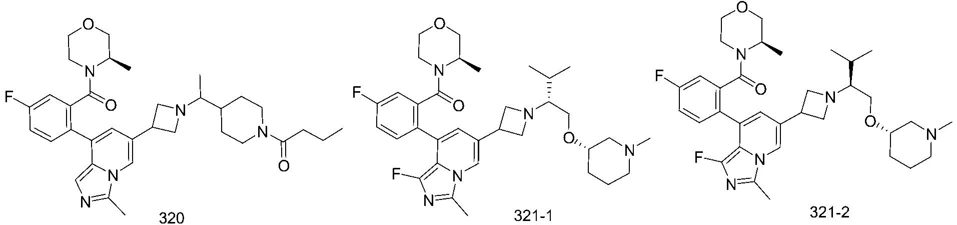

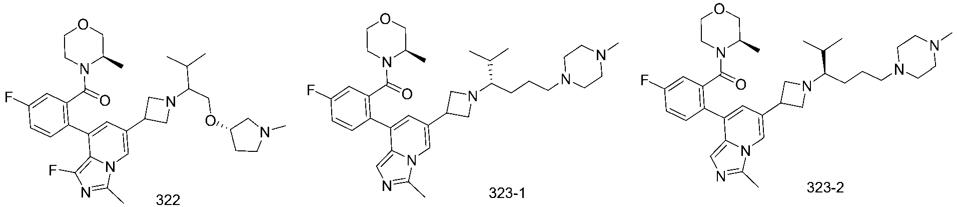

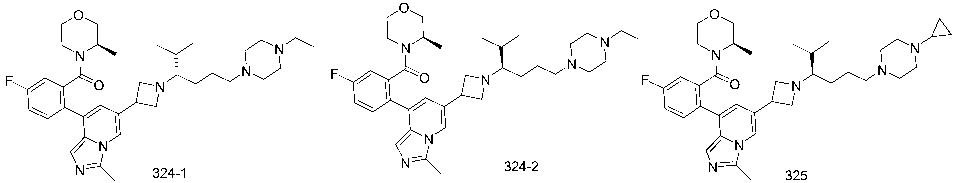

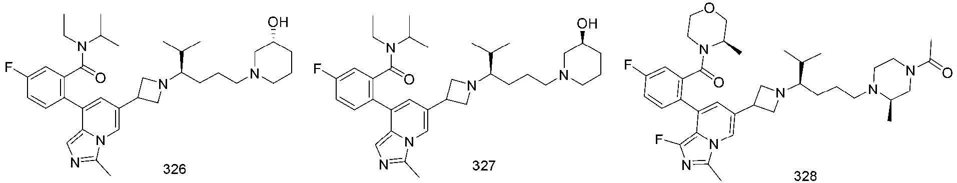

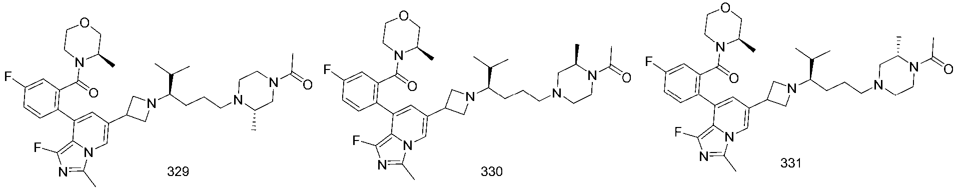

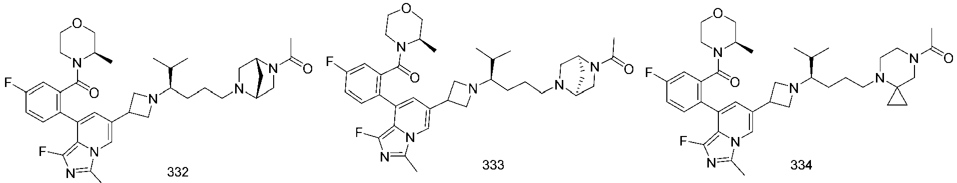

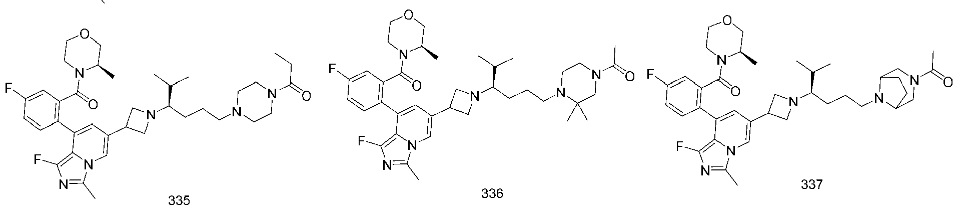

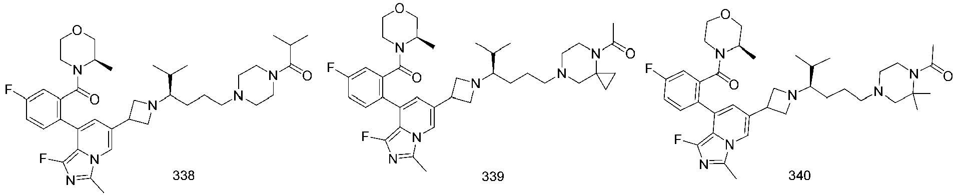

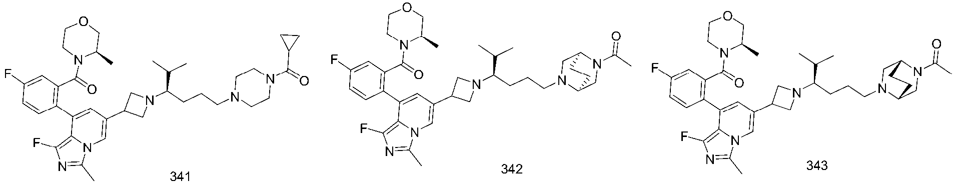

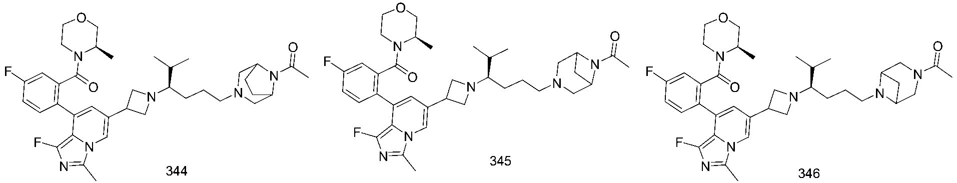

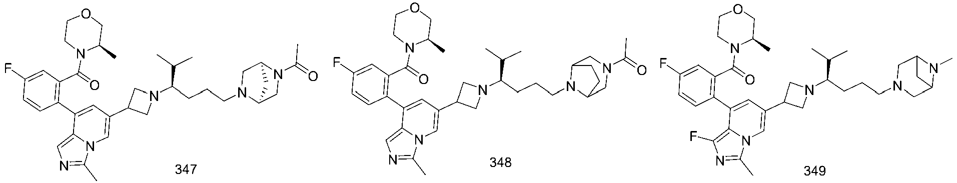

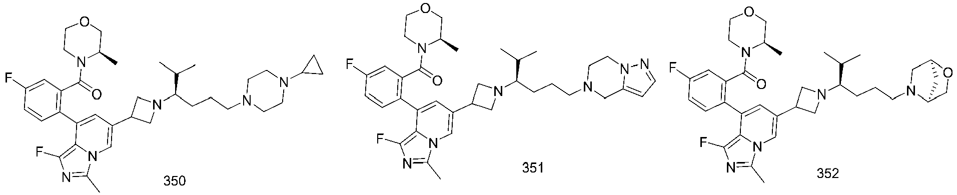

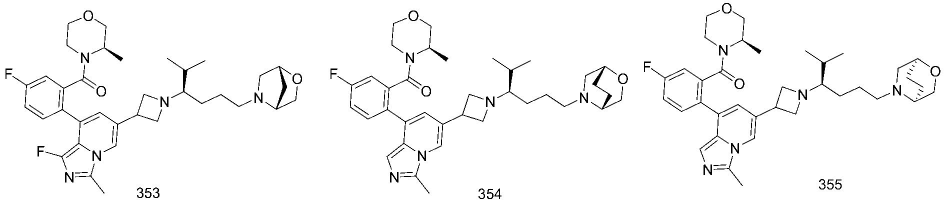

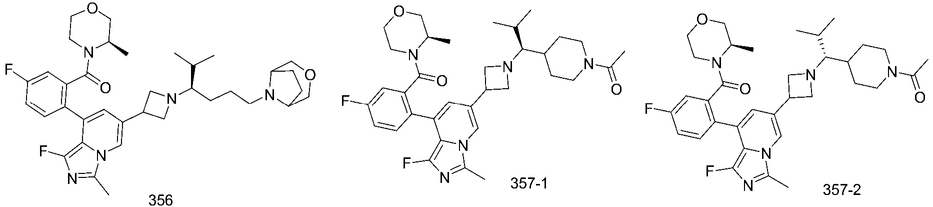

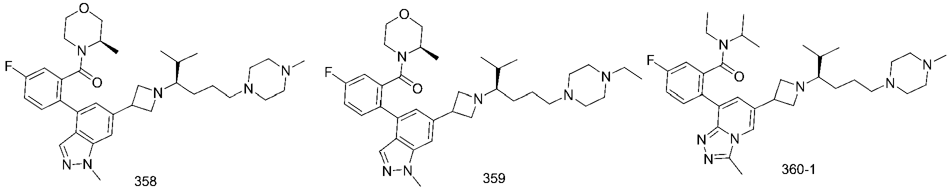

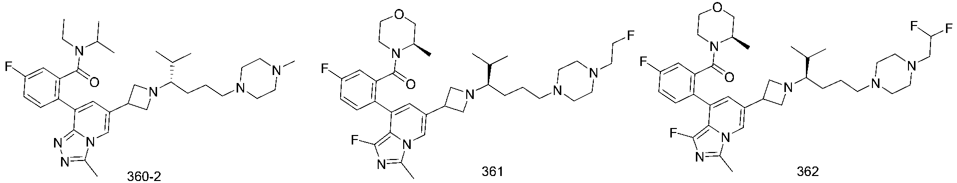

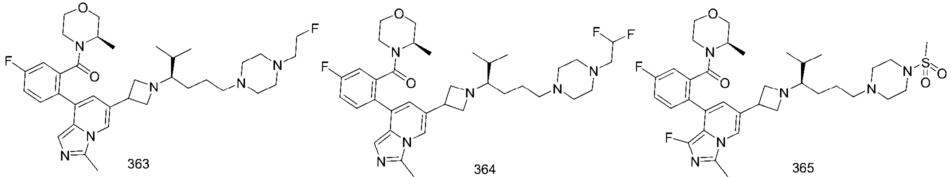

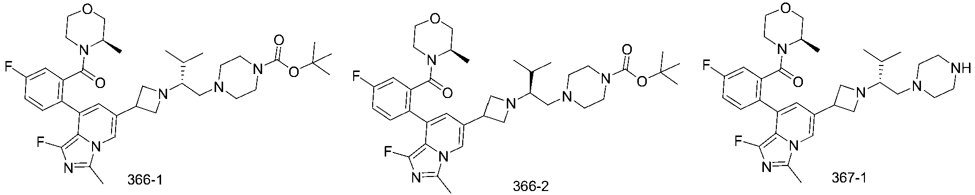

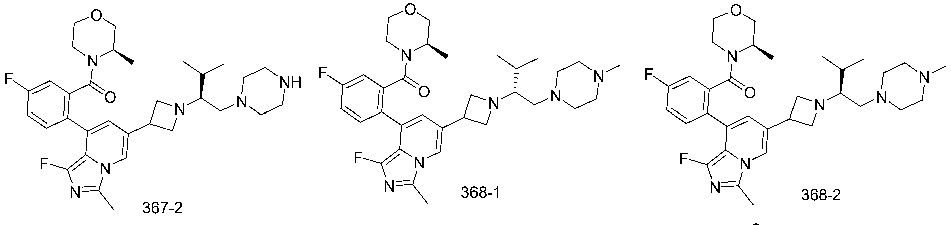

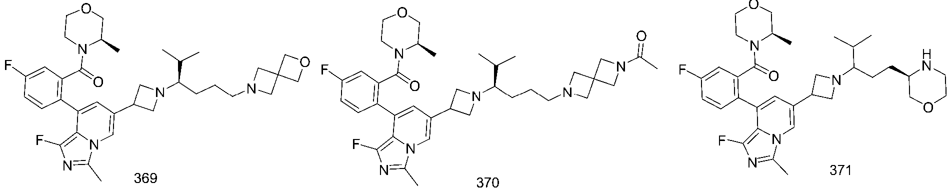

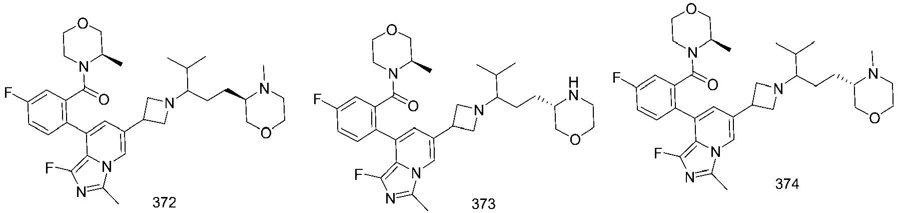

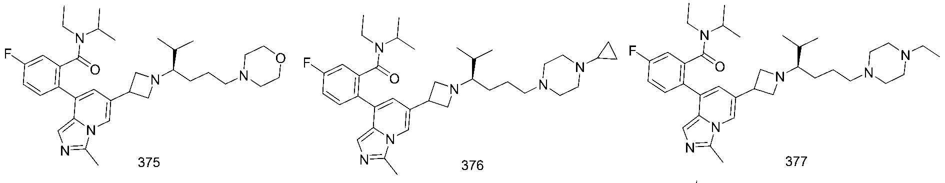

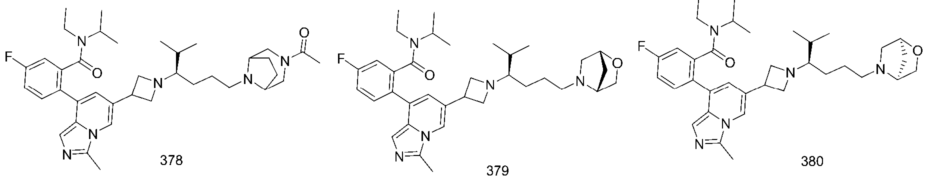

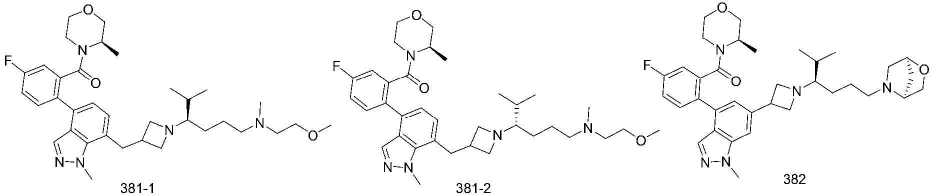

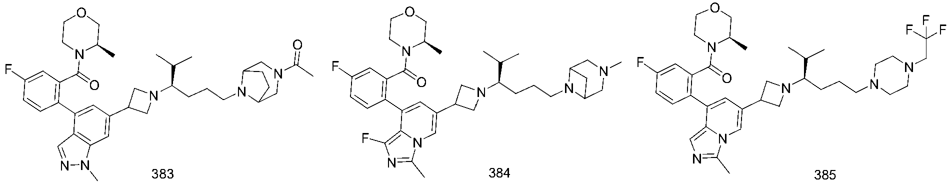

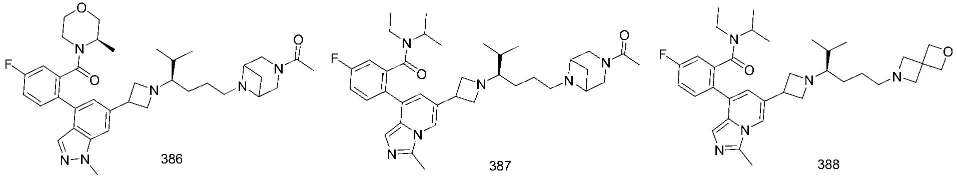

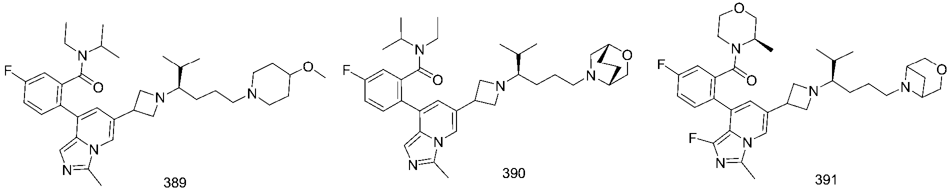

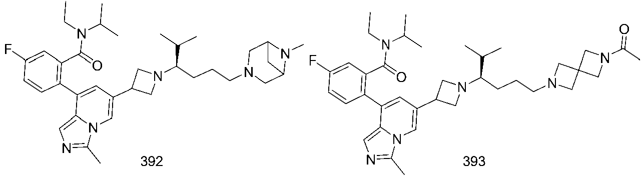

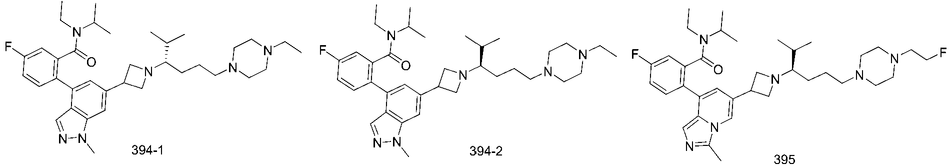

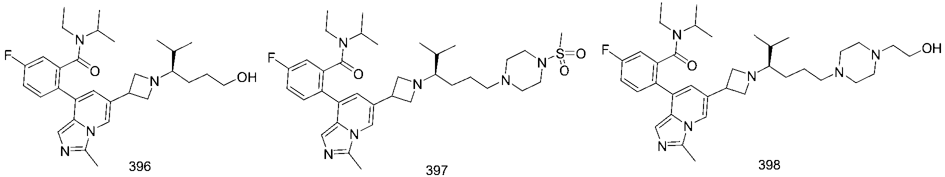

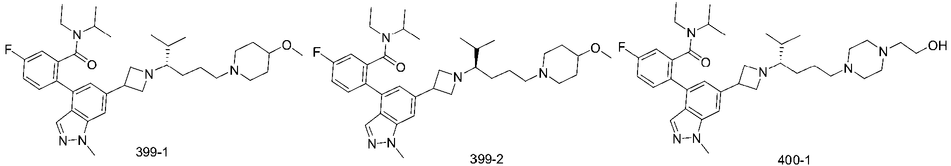

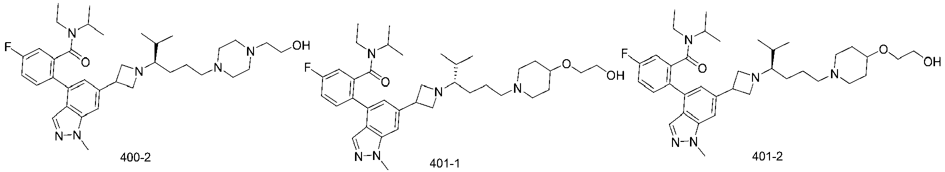

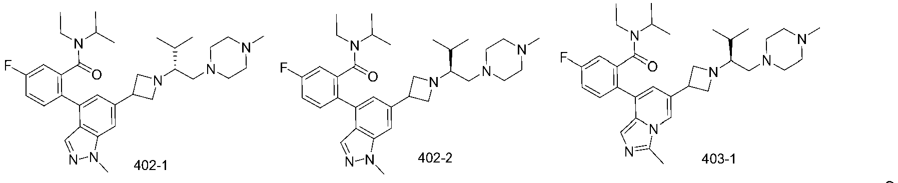

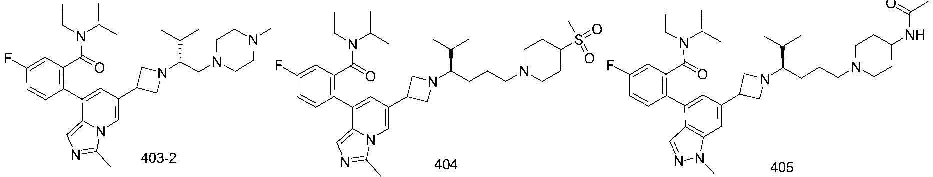

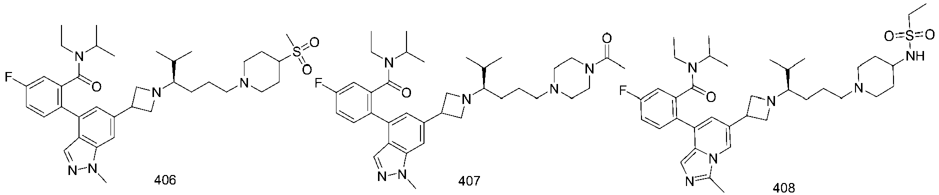

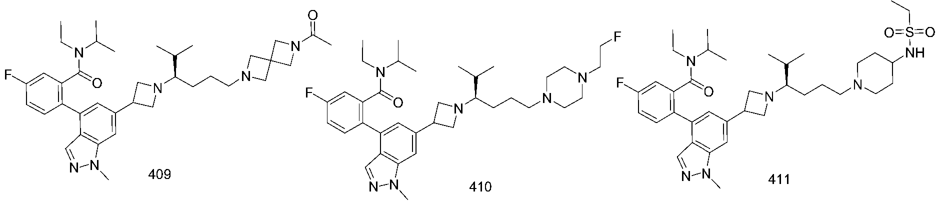

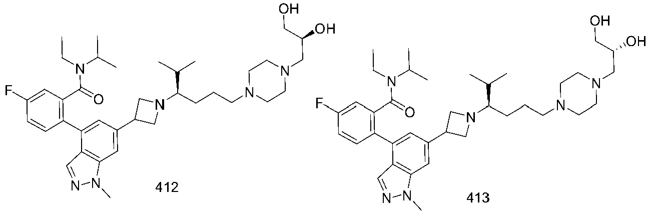

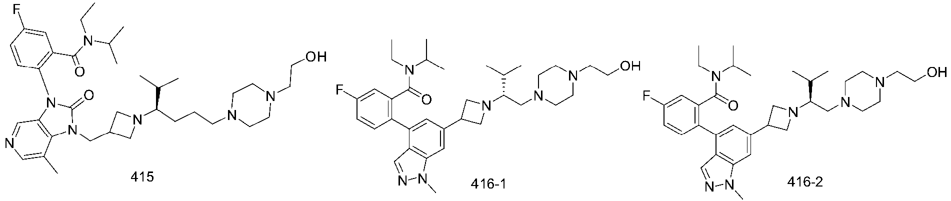

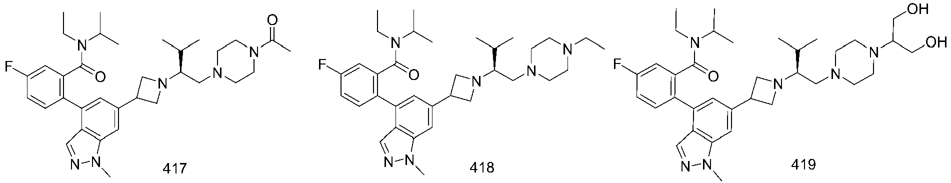

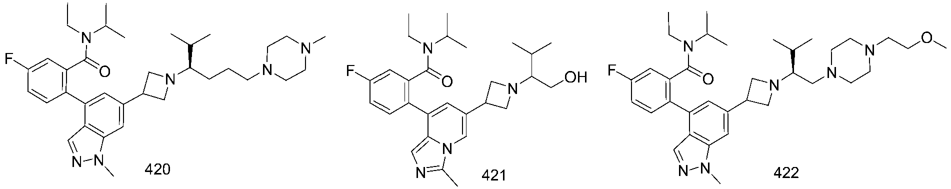

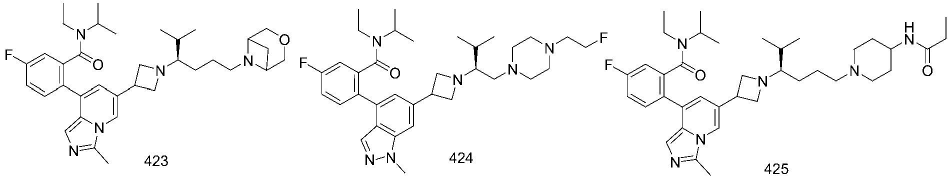

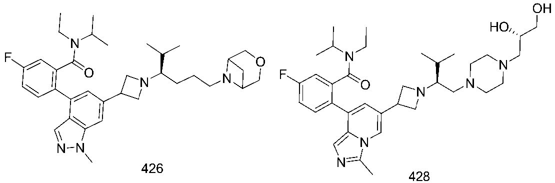

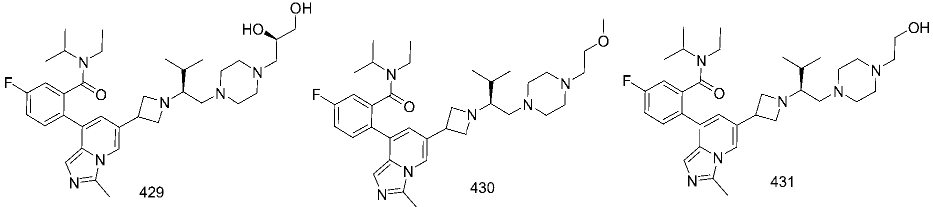

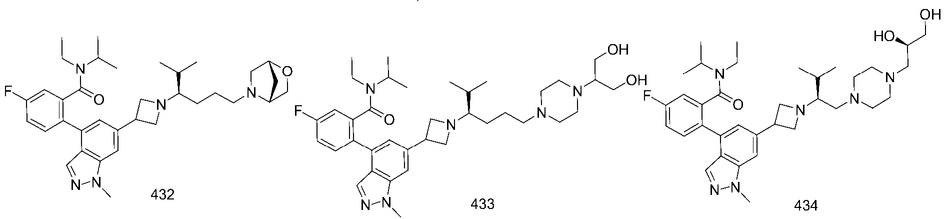

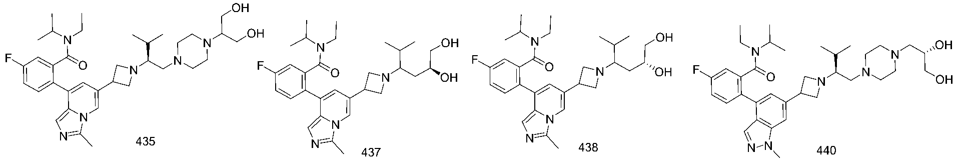

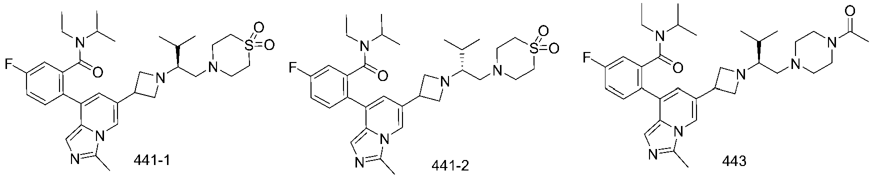

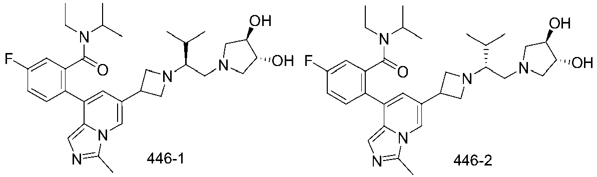

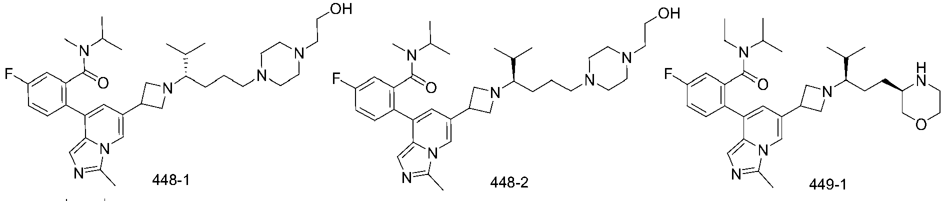

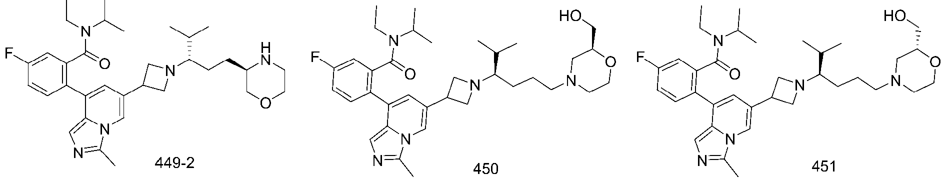

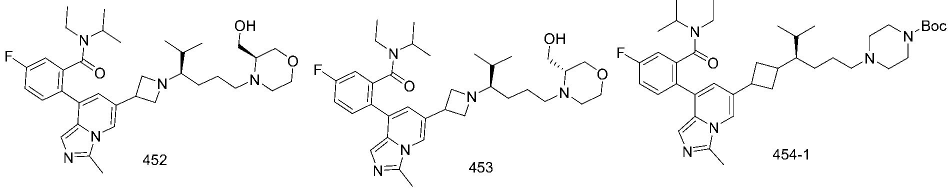

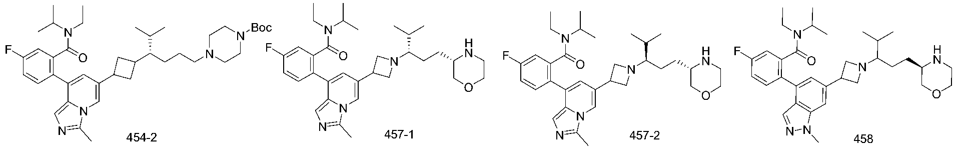

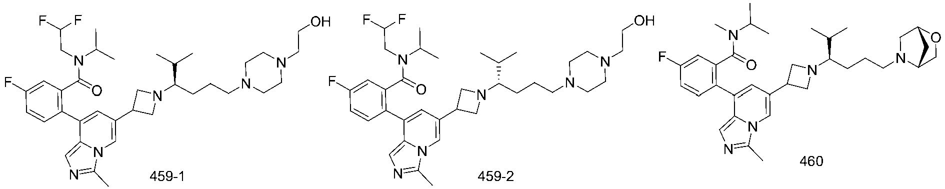

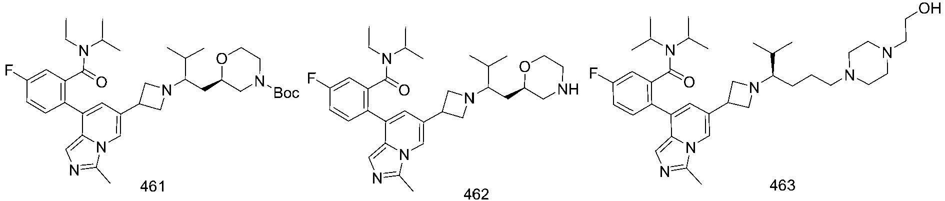

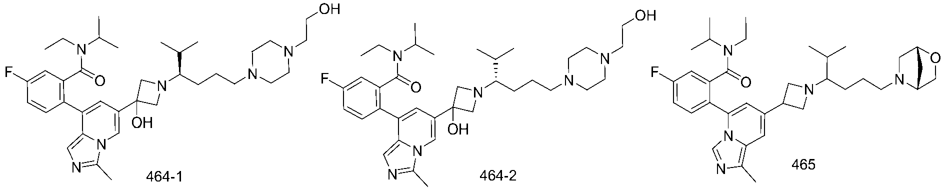

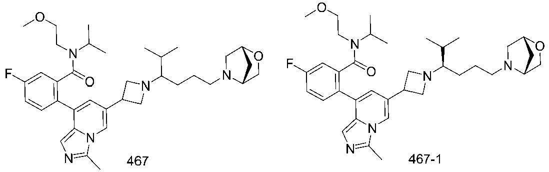

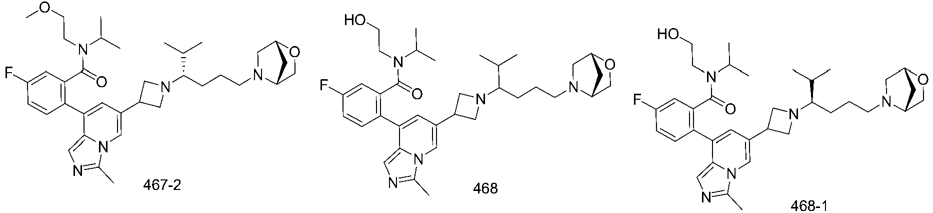

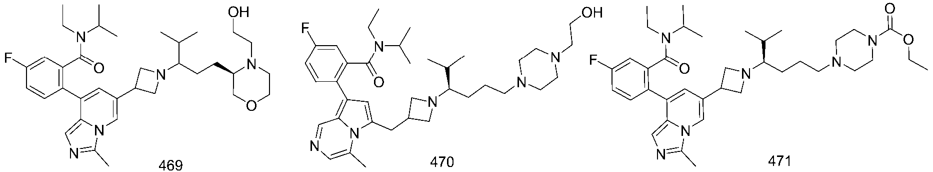

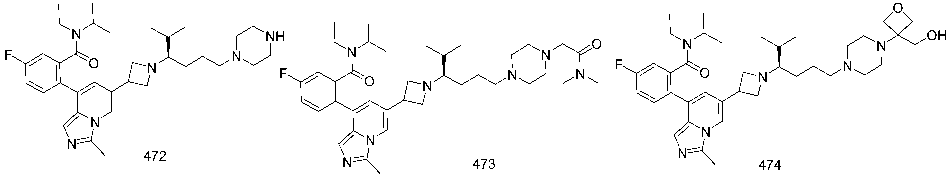

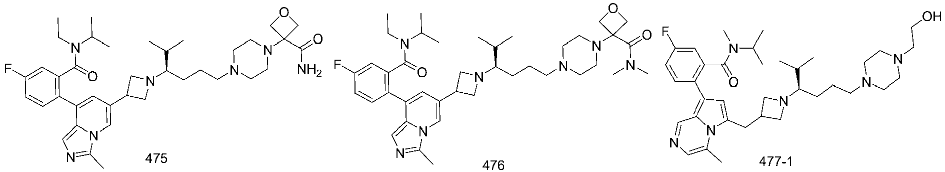

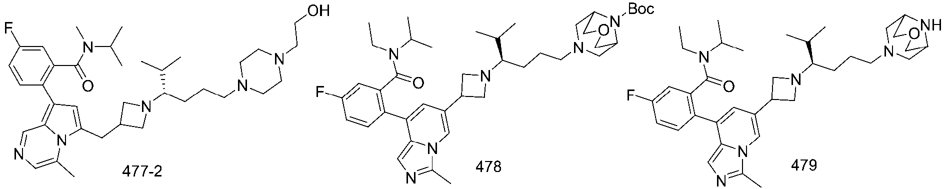

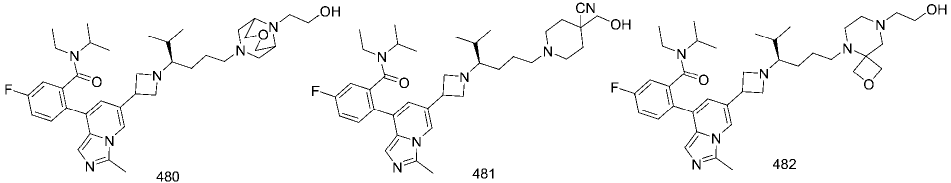

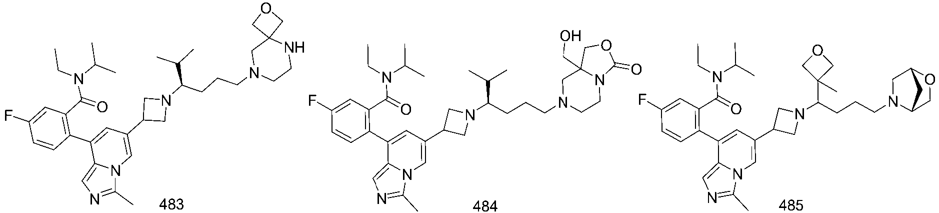

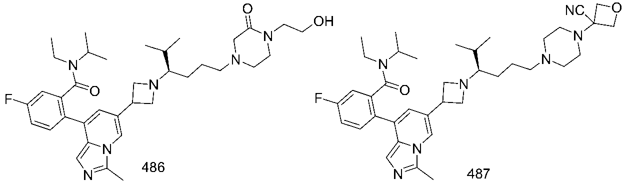

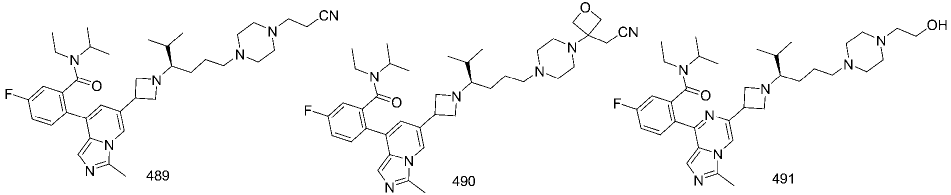

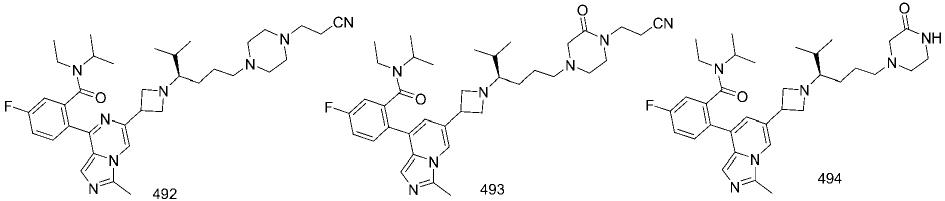

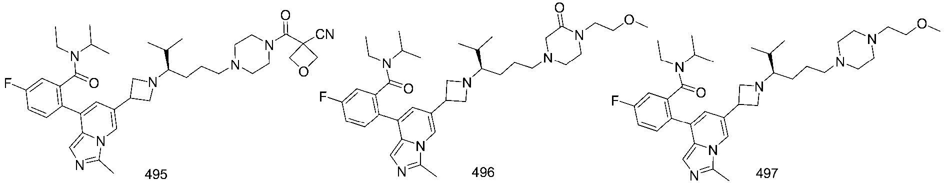

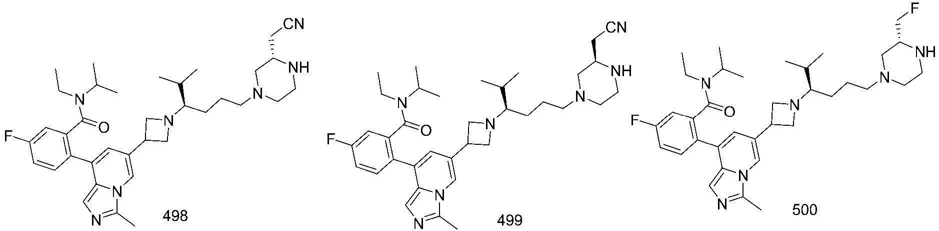

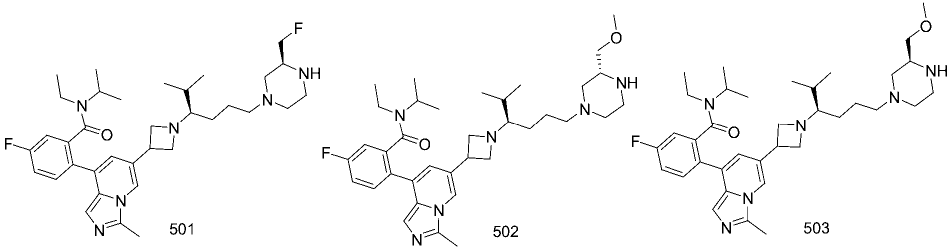

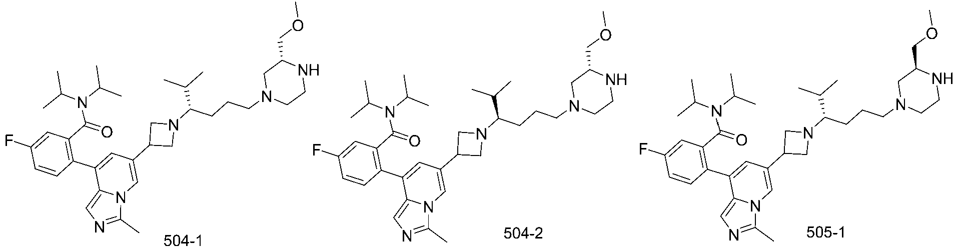

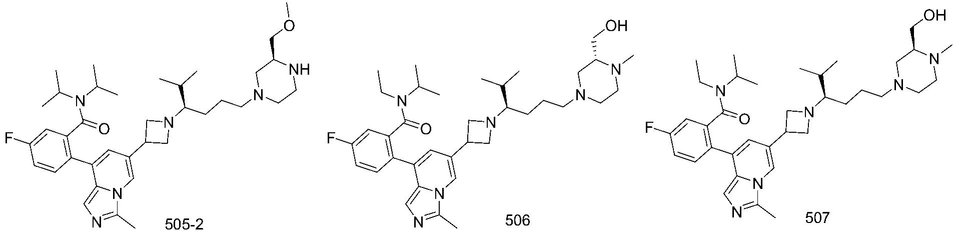

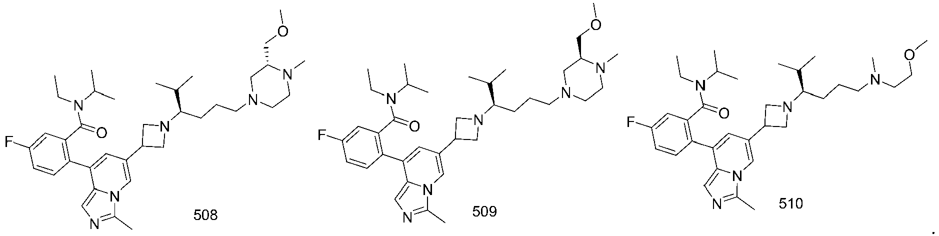

In a second aspect of the present invention, there is provided a pharmaceutical composition, including the compound described in the first aspect or the pharmaceutically acceptable salt, stereoisomer, ester, prodrug, solvate and deuterated compound thereof, and a pharmaceutically acceptable excipient.

Specifically, the pharmaceutically acceptable excipient may be selected from one or more of: disintegrants, binders, lubricants, suspending agents, stabilizers, fillers, absorption promoters, surfactants, flavoring agents, antioxidants, preservatives, etc.

Specifically, in this pharmaceutical composition, the compound described in the first aspect or the pharmaceutically acceptable salt, stereoisomer, ester, prodrug, solvate, and deuterated compound thereof, may be used alone, or in combination with other types of active ingredients.

Specifically, the pharmaceutical composition may be delivered via any appropriate route of administration, for example, gastrointestinal administration routes (for example, oral, sublingual, or rectal administration) or non-gastrointestinal administration (for example, intravenous, intramuscular, intranasal, intraocular, intracranial, vaginal, intraperitoneal, transdermal, subcutaneous, intradermal, or respiratory administration) routes.

Specifically, the pharmaceutical composition may take any suitable dosage form, for example: gastrointestinal dosage forms, for example, including but not limited to, tablets, pills, powders, granules, capsules, troches, syrups, liquids, emulsions, suspensions, etc.; and non-gastrointestinal dosage forms, such as injectable dosage forms (for example, for subcutaneous, intravenous, intramuscular, or intraperitoneal injection), respiratory dosage forms (for example, sprays, aerosols, or powder aerosols), dermal dosage forms (for example, topical solutions, lotions, ointments, plasters, pastes, or patches), mucosal dosage forms (for example, eye drops, ophthalmic ointments, nasal drops, gargles, or sublingual tablets), and cavity dosage forms (for example, suppositories, aerosols, effervescent tablets, drops, or pills) for the rectum, vagina, urethra, nose, ear canal, etc.

Specifically, various dosage forms of the aforementioned pharmaceutical composition may be prepared according to conventional production methods in the field of pharmacy. For example, an active ingredient is mixed with one or more pharmaceutically acceptable excipients to prepare the pharmaceutical composition of a desired dosage form.

Specifically, in the aforementioned pharmaceutical composition, the compound described in the first aspect or the pharmaceutically acceptable salt, stereoisomer, ester, prodrug, solvate and deuterated compound thereof may have a weight percentage of 0.1-99.5%, for example, 0.5%, 1%, 5%, 10%, 20%, 30%, 40%, 50%, 60%, 70%, 80%, 90%, 95%, or 99%, in particular 1-30%.

In a third aspect of the present invention, there is provided use of the compound described in the first aspect or the pharmaceutically acceptable salt, stereoisomer, ester, prodrug, solvate and deuterated compound thereof in the preparation of a medicament for inhibiting Menin-MLL interaction.

In a fourth aspect of the present invention, there is provided use of the compound described in the first aspect or the pharmaceutically acceptable salt, stereoisomer, ester, prodrug, solvate and deuterated compound thereof in the preparation of a medicament for preventing and/or treating a disease mediated by Menin-MLL interaction.

In some embodiments of the present invention, the disease is a tumor, including, but not limited to, a hematological tumor (for example, leukemia or lymphoma), bladder cancer, brain cancer (for example, glioma or diffuse intrinsic pontine glioma (DIPG)), breast cancer (for example, triple-negative breast cancer or estrogen receptor-positive breast cancer (i.e., ER + breast cancer)), intestinal cancer (for example, colon cancer, rectal cancer or colorectal cancer), gastrointestinal stromal tumor (GIST), cervical cancer, gastric cancer, genitourinary cancer, head and neck cancer, liver cancer, lung cancer, melanoma, ovarian cancer, pancreatic cancer, prostate cancer (for example, castration-resistant prostate cancer), kidney cancer (for example, renal cell carcinoma), skin cancer, thyroid cancer (for example, papillary thyroid cancer), testicular cancer, glioblastoma, sarcoma (for example, Ewing's sarcoma), and AIDS-related cancers.

In some embodiments of the present invention, the tumor is a hematological tumor, for example, leukemia, lymphoma, myeloma, etc.

In some embodiments of the present invention, the tumor is a solid tumor, for example, prostate cancer, lung cancer, breast cancer, pancreatic cancer, colon cancer, liver cancer, melanoma, or glioblastoma.

In some embodiments of the present invention, the disease includes, but is not limited to, mixed lineage leukemia (MLL), MLL-related leukemia, MLL-associated leukemia, MLL-positive leukemia, MLL-induced leukemia, rearranged mixed lineage leukemia (MLL-r), leukemia associated with MLL rearrangement or MLL gene rearrangement, acute leukemia, chronic leukemia, lymphoblastic leukemia, lymphocytic leukemia, myelocytic leukemia, myeloid leukemia, childhood leukemia, acute lymphoblastic leukemia (ALL), acute myeloid leukemia (AML), acute myelogenous leukemia, acute non-lymphocytic leukemia, chronic lymphocytic leukemia (CLL), chronic myeloid leukemia (CML), treatment-related leukemia, myelodysplastic syndrome (MDS), myeloproliferative disorder (MPD) , myeloproliferative neoplasia (MPN), plasmacytoma, multiple myeloma, myelodysplasia, cutaneous T-cell lymphoma, lymphoid tumors, hairy cell leukemia, leukemic meningitis, multiple myeloma, Hodgkin lymphoma, and non-Hodgkin lymphoma (malignant lymphoma).

In some embodiments of the present invention, the disease is diabetes, insulin resistance, or hyperglycemia.

In some embodiments of the present invention, the diseases is an autoimmune disease.

In some embodiments of the present invention, the disease is non-alcoholic hepatitis.

In a fifth aspect of the present invention, there is provided a method for inhibiting Menin-MLL interaction, including: administering the compound described in the first aspect or the pharmaceutically acceptable salt, stereoisomer, ester, prodrug, solvate and deuterated compound thereof, or the pharmaceutical composition described in the second aspect.

In some embodiments of the present invention, the method is performed in vitro.

In some embodiments of the present invention, the method is performed in vivo.

In a sixth aspect of the present invention, there is provided a method for preventing and/or treating a disease mediated by Menin-MLL interaction, including: administering the compound described in the first aspect or the pharmaceutically acceptable salt, stereoisomer, ester, prodrug, solvate and deuterated compound thereof, or the pharmaceutical composition described in the second aspect, to a subject in need thereof.

Specifically, the disease is defined as in the fourth aspect of the present invention.

Specifically, the subject is a mammal, in particular a human.

### DETAILED DESCRIPTION OF THE INVENTION

Unless otherwise defined, all scientific and technical terms used in the present invention have the same meanings as those generally understood by those of ordinary skill in the art that is involved in the present invention.

In the present invention, the term "aliphatic group" refers to a linear or branched hydrocarbon chain that is completely saturated or contains one or more unsaturated units, or a cyclic hydrocarbon group (also referred to herein as "carbo atomic ring" or "aliphatic ring") that is completely saturated or contains one or more unsaturated units, and the aliphatic group is attached to the remainder of the molecule via a single bond. Suitable aliphatic groups include, but are not limited to, linear or branched and substituted or unsubstituted alkyl, alkenyl, alkynyl, and their mixtures, for example, (cycloalkyl)alkyl, (cycloalkenyl)alkyl, (cycloalkyl)alkenyl, etc. A typical aliphatic group contains 1 to 10 (for example, 1, 2, 3, 4, 5, 6, 7, 8, 9, or 10) carbon atoms, preferably 1 to 6 carbon atoms.

The term "alkyl" refers to a linear or branched hydrocarbon chain free radical that contains no unsaturated bond, and the hydrocarbon chain free radical is attached to the remainder of the molecule via a single bond. A typical alkyl group contains 1 to 10 (for example, 1, 2, 3, 4, 5, 6, 7, 8, 9, or 10) carbon atoms, preferably 1 to 6 carbon atoms, for example, methyl, ethyl, n-propyl, isopropyl, n-butyl, isobutyl, tert-butyl, n-amyl, isoamyl, neoamyl, tert-amyl, n-hexyl, isohexyl, etc. If alkyl is substituted with cycloalkyl, then "cycloalkylalkyl" is derived correspondingly, for example, cyclopropylmethyl, cyclopropylethyl, cyclobutylmethyl, cyclopentylmethyl, cyclohexylmethyl, etc. Typical cycloalkylalkyl groups include C₃-C₁₀ cycloalkylalkyl, for example, -(C₁-C₃ alkylene)-(C₃-C₆ cycloalkyl). If alkyl is substituted with aryl, then "aralkyl" is derived correspondingly, for example, benzyl, diphenylmethyl, or phenylethyl. If alkyl is substituted with heterocyclyl, then "heterocyclylalkyl" is derived correspondingly. In the present invention, C₀ alkyl refers to H, that is, C₀₋₁₀ alkyl includes H and C₁₋₁₀ alkyl.

The term "alkylene" refers to a hydrocarbon group (divalent alkyl group) derived by removing two hydrogen atoms from an alkane molecule, and it may be either linear or branched and is attached to the remainder of the molecule via a single bond. A typical alkylene group herein contains 1 to 10 (for example, 1, 2, 3, 4, 5, 6, 7, 8, 9, or 10) carbon atoms, preferably 1 to 6 carbon atoms, such as methylene (-CH₂-), ethylene, propylene, butylene, etc. In the present invention, C₀ alkylene refers to a single bond, that is, C₀₋₁₀ alkylene includes a single bond and C₁₋₁₀ alkylene.

The term "cycloalky" refers to an aliphatic cyclic hydrocarbon, including for example 1 to 4 monocyclic and/or fused rings containing 3-18 carbon atoms, preferably 3-10 (for example, 3, 4, 5, 6, 7, 8, 9, or 10) carbon atoms, such as cyclopropyl, cyclobutyl, cyclopentyl, cyclohexyl, or adamantyl.

The term "alkoxy" refers to a substituent group derived from a hydroxyl group by substituting hydrogen with an alkyl group, for example, an alkoxy group containing 1-10 carbon atoms, such as methoxy, ethoxy, propoxy, butoxy, etc.

The term "alkylamino" refers to a substituent group derived from an amino group (-NH2) by substituting one or two hydrogens with an alkyl group(s), for example, an alkylamino group containing 1-10 carbon atoms, such as

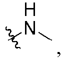

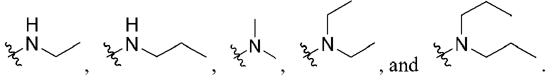

The term "halogen" refers to fluorine, chlorine, bromine or iodine.

The term "halogenated alkyl" refers to a group derived from an alkyl group by substituting one or more hydrogens with a halogen atom(s) (for example, fluorine, chlorine, bromine, or iodine), for example,-CHF₂, -CH₂F, -CF₃, -CH₂-CH₂F, -CH₂-CHF₂, -CH₂-CF₃, -CH₂CH₂-CF₃, or -CH₂CH₂CH₂-CF₃.

The term "aryl" refers to a monocyclic or polycyclic free radical, including a polycyclic free radical containing a single aryl group and/or a fused aryl group, for example, containing 1-3 monocyclic or fused rings and 6-18 (for example, 6, 8, 10, 12, 14, 16, or 18) carboatomic ring atoms. In the present invention, the C₆-C₁₂ aryl refers to an aryl group containing 6-12 carboatomic ring atoms, for example, phenyl, naphthyl, biphenyl, indenyl, etc.

The term "heterocyclyl" refers to a 3-18-membered cyclyl group containing 2 to 17 carbon atoms and 1 to 10 heteroatoms selected from N, O, or S atoms. The heterocyclyl group may be a monocyclic, bicyclic, tricyclic, or tetracyclic system, or an additional polycyclic system, which may include a fused (two rings sharing two ring atoms), spiro (two rings sharing one ring atom), or bridged (two rings sharing more than three ring atoms) ring system (excluding linked rings). The heterocyclyl group may be partially saturated (e.g., heteroaryl) or fully saturated (e.g., heterocycloalkyl). In the compound of the present invention, an appropriate heteroaryl group contains 1, 2 or 3 types of heteroatoms selected from N, O or S atoms, and includes, for example, coumarin (including 8-coumarin), quinolyl (including 8-quinolyl), isoquinolyl, pyridinyl, pyrazinyl, pyrazolyl, pyrimidinyl, furanyl, pyrrolyl, thienyl, thiazolyl, isothiazolyl, triazolyl, tetrazolyl, isoxazolyl, oxazolyl, imidazolyl, indolyl, isoindolyl, indazolyl, indolizinyl, phthalazinyl, pteridinyl, purinyl, oxadiazolyl, thiadiazolyl, furazanyl, pyridazinyl, triazinyl, cinnolinyl, benzimidazolyl, benzofuranyl, benzofurazanyl, thienyl, benzothiazolyl, benzoxazolyl, quinazolinyl, quinoxalinyl, naphthyridinyl and furopyridinyl. In the compound of the present invention, an appropriate heterocycloalkyl group contains 1, 2 or 3 types of heteroatoms selected from N, O or S atoms, and includes, for example, pyrrolidinyl, tetrahydrofuranyl, dihydrofuran, tetrahydrothienyl, tetrahydrothiopyranyl, piperidinyl, morpholinyl, thiomorpholinyl, oxathiacyclohexanyl, piperazinyl, azacyclobutanyl, oxacyclobutanyl, thiacyclobutanyl, homopiperidinyl, oxacyclopropanyl, thiacyclopropanyl, azepinyl, oxazacycloheptyl, diazepinyl, triazepinyl, 1,2,3,6-tetrahydropyridyl, 2-pyrrolinyl, 3-pyrrolinyl, dihydroindolyl, 2H-pyranyl, 4H-pyranyl, dioxacyclohexanyl, 1,3-dioxolanyl, pyrazolinyl, dithanyl, dithiolanyl, dihydropyranyl, dihydrothienyl, pyrazolidinyl, imidazolinyl, imidazolidinyl, 3-azabicyclo[3.1.0]hexyl,3-azabicyclo[4.1.0]heptyl, 3H-indolyl, and quinazinyl. In the present invention, for the optionally substituted heterocyclyl group, any suitable carbon atom or heteroatom may be at the substitution position.

As an example, for 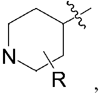 the position of substitution for R may be occupied by any suitable carbon atom or nitrogen atom, which may be, for example,

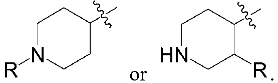

The term "optionally substituted" group may be halogen, -CN, -NO₂, -OR', -NR'R", -S(O)t-R', -S(O)t-NR'R", - COR', -C(O)OR', -C(O)NR'R", -C(O)N(R')OR", -OC(O)R', -OC(O)NR'R", -NR'C(O)R", -N(R')C(O)NR'R', - N(R')C(NR')NR'R', -NR'-S(O)t-R', -NR'-S(O)t-NR'R', -N=S(O)R'R", -S(NR')(O)R", -N(R')CN, -P(O)(R')NR'R", - P(O)(R')OR" or -P(O)R'R", C₁₋₆ alkyl, C₁₋₆ halogenated alkyl, C₁₋₆ alkenyl, C₁₋₆ alkynyl, C₃₋₆ cycloalkyl, C₄₋₁₀ cycloalkylalkyl, C₆₋₁₀ aryl, C₆₋₁₀ arylalkyl, C₃₋₈ heterocyclyl, or C₃₋₈ heterocyclylalkyl; t is 0, 1 or 2; R' and R" are each independently selected from the group consisting of: H, halogen, -CN, -NO₂, alkyl, halogenated alkyl, cycloalkyl, cycloalkylalkyl, aryl, arylalkyl, heterocyclyl, heterocyclylalkyl; or, R' and R", which are linked to the same nitrogen, form a heterocyclic ring together with this nitrogen atom.

The term "pharmaceutically acceptable salt" includes acid addition salts and alkali addition salts.

The term "acid addition salts" includes, but is not limited to, salts derived from inorganic acids (such as hydrochloric acid, nitric acid, phosphoric acid, sulfuric acid, hydrobromic acid, hydroiodic acid, and phosphonic acid), as well as salts derived from organic acids (such as aliphatic monocarboxylic and dicarboxylic acids, phenyl-substituted alkanoic acid, hydroxyalkanoic acid, dialkanoic acid, aromatic acid, and aliphatic and aromatic sulfonic acid). The acid addition salts include, for example, acetate, salicylate, decanoate, stearate, oleate, caproate, malate, glycolate, ethanesulfonate, hydroxyesulfonate, etc. Therefore, these salts include, but are not limited to, sulfate, pyrosulfate, bisulfate, sulfite, bisulfite, nitrate, phosphate, monohydrogen phosphate, dihydrogen phosphate, metaphosphate, pyrophosphate, hydrochloride, hydrobromide, iodate, acetate, propionate, caprylate, isobutyrate, oxalate, malonate, succinate, suberate, sebacate, fumarate, maleate, amygdalate, benzoate, chlorobenzoate, methylbenzoate, dinitrobenzoate, phthalate, benzenesulfonate, tosylate, phenylacetate, citrate, lactate, tartrate and methanesulfonate, and further include salts of amino acids, such as arginine, gluconate, galacturonate, aspartate, glutamate, etc. The acid addition salts may be prepared by bringing free alkali into contact with a sufficient amount of the desired acid in a conventional way. The free alkali may be regenerated by bringing a salt into contact with an alkali, and may be isolated in a conventional way.

The term "alkali addition salts" refer to salts formed with metals or amines (such as hydroxides of alkali metals and alkaline earth metals), or with organic amines. Examples of metals used as cations include, but are not limited to, sodium, potassium, magnesium, and calcium. Examples of suitable amines include, but are not limited to, N,N'-dibenzylethylenediamine, chloroprocaine, choline, diethanolamine, ethylenediamine(ethane-1,2-diamine), N-methylglucosamine, and procaine. The alkali addition salts may be prepared by bringing free acid into contact with a sufficient amount of the desired alkali in a conventional way. The free acid may be regenerated by bringing a salt into contact with an acid, and may be isolated in a conventional way.

The term "stereoisomer" exists as enantiomers, diastereomers, and geometric isomers. Some compounds of the present invention have a cyclic hydrocarbon group, which may be substituted on more than one carbon atom, in which case all their geometric forms (including cis and trans forms), and their mixtures, shall fall within the scope of the present invention.

The term "solvate" refers to the physical binding of the compound of the present invention to one or more solvent molecules. This physical binding includes ionic and covalent bonding to various degrees, including hydrogen bonding. In some cases, the solvate may be isolated, for example, where one or more solvent molecules are incorporated into the crystal lattice of a crystalline solid. The solvate includes solution phases and separable solvates. Representative solvates include alcoholate, methylate, etc.

The term "deuterated compound" refers to a compound in which one or more hydrogen atoms (for example, 1, 2, 3, 4, or 5 hydrogen atoms) are substituted with deuterium atoms (D).

It should be recognized that, depending on the source of the chemical material used for synthesis, the abundance of natural isotopes varies in the synthesized compounds. Therefore, the compound of the present invention will inherently comprise a small amount of deuterated isotopologues. Despite this variation, the concentrations of stable hydrogen and carbon isotopes of this natural abundance are still low and insignificant compared to the degree of stable isotope substitution in the compound of the present invention. A reference can be made to, for example, Wada, E et al., Seikagaku, 1994, 66: 15; and Gannes, LZ et al., Comp Biochem Physiol Mol Integr Physiol, 1998, 119: 725. In the compound of the present invention, any atom that is not specified as deuterium exists in its natural isotope abundance. Unless otherwise indicated, when a position is specifically specified as "H" or "hydrogen", this position shall be understood as having hydrogen with the isotopic composition corresponding to its natural abundance. Similarly, unless otherwise indicated, when a position is specifically specified as "D" or "deuterium", this position shall be understood as having deuterium with the abundance of at least 3000 times greater than the natural abundance (which is 0.015%) of deuterium (i.e., at least 45% of deuterium incorporated).

The term "isotope enrichment coefficient" as used herein refers to the ratio of the isotopic abundance of a particular isotope to its natural abundance.

In other embodiments, the isotope enrichment coefficient of the compound of the present invention for each specified deuterium atom is at least 3500 (with 52.5% of deuterium incorporated at each specified deuterium atom), at least 4000 (with 60% of deuterium incorporated), at least 4500 (with 67.5% of deuterium incorporated), at least 5000 (with 75% of deuterium incorporated), at least 5500 (with 82.5% of deuterium incorporated), at least 6000 (90% of deuterium incorporated), at least 6333.3 (with 95% of deuterium incorporated), at least 6466.7 (with 97% of deuterium incorporated), at least 6600 (with 99% of deuterium incorporated) or at least 6633.3 (with 99.5% of deuterium incorporated).

The term "isotopologue" refers to a substance with a chemical structure differing from the specific compound of the present invention only in its isotopic composition.

The term "prodrug" refers to the compound forms of formula I (including acetal, ester and zwitterionic forms), which are suitable for administration to patients without excessive toxicity, irritation, allergic reactions or the like and are effective for the purpose of their application. The prodrug is transformed in vivo, for example, by hydrolysis in the blood, to obtain a parent compound.

The terms "patient" or "subject" and the like are used interchangeably herein, referring to any animals or their cells that have been treated according to the methods described herein, whether in vitro or in situ. Specifically, the aforementioned animals include mammals, for example, rats, mice, guinea pigs, rabbits, dogs, monkeys, and humans, in particular humans.

The term "treatment" refers to the process of preventing, curing, reversing, attenuating, alleviating, minimizing, inhibiting, restraining and/or stopping one or more clinical symptoms of a disease after its onset.

The term "prevention" refers to the process of treating a disease, before its onset, in order to avoid, minimize, or make it difficult for the disease to occur or progress.

The term "tumor" refers to a neoplasm formed by the proliferation of local tissue cells in the body under the action of various tumorigenic factors. Based on the cell characteristics of the neoplasm and its degree of harm to the body, tumors are further divided into two major categories: benign tumors and malignant tumors. "Malignant tumors" refer to diseases that are characterized by uncontrollable growth and spread of malignant cells, along with tissue infiltration, and are determined through pathological examination to meet the criteria for classification as malignant tumors according to the "Classification of Diseases and Causes of Death" standards published by the Ministry of Health.

In specific embodiments, the compound of the present invention is for use in the treatment of leukemia associated with MLL rearrangement, acute lymphogenous leukemia associated with MLL rearrangement, acute lymphoblastic leukemia associated with MLL rearrangement, acute lymphoid leukemia associated with MLL rearrangement, acute myeloid leukemia associated with MLL rearrangement, acute myelogenous leukemia associated with MLL rearrangement, or acute myeloblastic leukemia associated with MLL rearrangement. As used in the present application, "MLL rearrangement" means the rearrangement of MLL genes.

The term "autoimmune disease" refers to a disease caused by the body's immune response to its own antigens, resulting in damage to its own tissues. It can be divided into organ-specific autoimmune diseases (mainly affecting a single organ) and systemic autoimmune diseases (affecting multiple tissues or organs throughout the body). The American Autoimmune Related Diseases Association has listed a relatively comprehensive list of autoimmune diseases, among which common autoimmune diseases typically include: systemic lupus erythematosus, type 1 diabetes, rheumatoid arthritis, multiple sclerosis, ankylosing spondylitis, psoriasis, celiac disease, ulcerative colitis, Crohn's disease, etc.

Unless otherwise indicated, the range of values expressed in the form "from x to y" or "x-y" shall be understood as including x and y. When several preferred ranges are described in the form of "from x to y" or "x-y" for a specific feature, it should be understood that all ranges combining different endpoints can also be considered.

The disclosures of all the publications, patents and published patent specifications cited herein are incorporated herein by reference in their entireties.

The technical solutions of the present invention will be described clearly and completely below in conjunction with the embodiments of the present invention. Obviously, the embodiments described are only part of rather than all of the embodiments of the present invention. Based on the embodiments in the present invention, all other embodiments obtained by those ordinarily skilled in the art without making inventive efforts shall fall within the protection scope of the present invention.

### Synthesis Examples

The structure of the compound was determined by nuclear magnetic resonance (NMR) or/and mass spectrometry (MS). NMR shifts (δ) were given in units of 10⁻⁶ (ppm). NMR measurements were performed using a Bruker AVANCE-400 NMR spectrometer or a Bruker AVANCE NEO 500M. The measurement solvents included deuterated dimethyl sulfoxide (DMSO-*d₆*), deuterated chloroform (CDCl₃) and deuterated methanol (CD₃OD), with tetramethylsilane (TMS) serving as the internal standard.

MS measurements were performed using an Agilent 1200/1290 DAD-6110/6120 Quadrupole LC-MS instrument (manufacturer: Agilent; MS model: 6110/6120 Quadrupole MS), waters ACQuity UPLC-QD/SQD (manufacturer: waters; MS model: waters ACQuity Qda Detector/waters SQ Detector), and THERMO Ultimate 3000-Q Exactive (manufacturer: THERMO; MS model: THERMO Q Exactive).

High-performance liquid chromatography (HPLC) analysis was performed using Agilent HPLC 1200DAD, Agilent HPLC 1200VWD and Waters HPLC e2695-2489 high-pressure liquid chromatographs.

Chiral HPLC analytic determination was performed using the Agilent 1260 DAD high-performance liquid chromatograph.

High-performance liquid phase preparation was performed using Waters 2767, Waters 2767-SQ Detecor2, Shimadzu LC-20AP and Gilson-281preparative chromatographs.

Chiral preparation was performed using a Shimadzu LC-20A preparative chromatograph.

Combiflash Rf200 (TELEDYNE ISCO) was used as the CombiFlash rapid preparation chromatograph.

Yantai Huanghai HSGF254 or Qingdao GF254 silica gel plates were used for thin-layer chromatography (TLC), with the size of 0.15 mm to 0.2 mm for TLC and the size of 0.4 mm to 0.5 mm for products separated and purified by the TLC.

For silica-gel column chromatography, Yantai Huanghai Silica Gel 200-300-mesh silica gel was generally used as the carrier.

Kinase average inhibition rate and IC₅₀ value were measured using NovoStar microplate reader (BMG, Germany). The known starting materials of the present disclosure were synthesized by methods known in the art, or purchased from ABCR GmbH & Co. KG, Acros Organics, Aldrich Chemical Company, Accela ChemBio Inc, Darui Chemical and other companies.

Unless otherwise specified in the examples, the reactions can be carried out under an argon or nitrogen atmosphere.

The argon or nitrogen atmosphere refers to the condition where a reaction flask is connected to an argon or nitrogen balloon with a volume of about 1 L.

The hydrogen atmosphere refers to the condition where a reaction flask is connected to a hydrogen balloon with a volume of about 1 L.

A hydrogenator or an autoclave was used for the pressurized hydrogenation reaction.

The hydrogenation reaction typically includes evacuating, filling with hydrogen, and repeating 3 times.

A CEM Discover-S 908860 microwave reactor was used for the microwave reaction.

Unless otherwise specified in the examples, the solution refers to an aqueous solution.

Unless otherwise specified in the examples, the reaction temperature refers to room temperature, which is 20°C to 30°C.

The reaction process in the examples was monitored by TLC. The developing solvent used in the reaction, the eluent system for column chromatography used to purify the compounds, and the developing solvent system for TLC included: an n-hexane/ethyl acetate system, a dichloromethane/methanol system, a dichloromethane/ethyl acetate system, etc. The volume ratio of solvents was regulated based on the polarity of the compounds, or small amounts of basic or acidic reagents (such as triethylamine and acetic acid) could also be added for adjustment.

### Example 1

### 2-(7-{1-[(2-oxo-2,3-dihydro-1,3-benzoxazol-5-yl)methyl]piperidin-3-yl}pyrrolo[2,1-f][1,2,4]triazin-5-yl)-N,N-diisopropylbenzamide 1

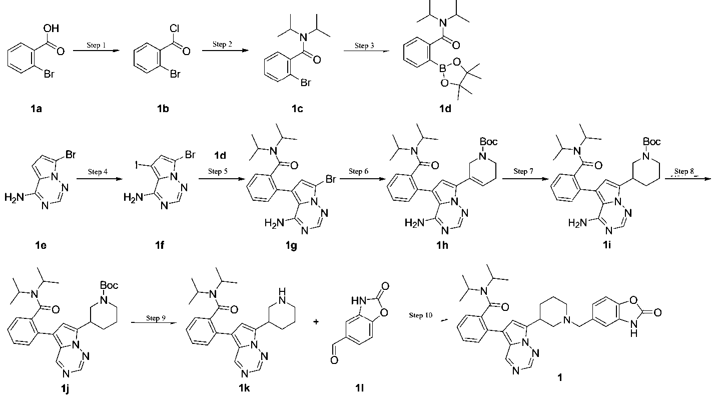

### Step 1

### 2-Bromobenzoyl chloride 1b

2-Bromobenzoic acid **1a** (5.00 g, 24.9 mmol) was dissolved in dichloromethane (35 mL), and cooled to 0°C. Under a nitrogen atmosphere, oxalyl chloride (3.79 g, 29.9 mmol) and *N,N*-dimethylformamide (0.1 mL) were added, and the mixture was stirred at room temperature for 1.0 hour. The dichloromethane solution of title Compound **1b** was then obtained, which was directly used in the next step of reaction without treatment.

### Step 2

### 2-bromo-N,N-diisopropylbenzamide 1c

The dichloromethane solution of Compound **1b** prepared from the previous step was cooled to 0°C, diisopropylamine (46.1 g, 455 mmol) was added, and the mixture was stirred at room temperature for 12 hours. 2N aqueous hydrochloric acid solution was added to regulate the PH value of the reaction mixture to 2-3, and the reaction mixture was diluted with water (10 mL), and extracted with dichloromethane (20 mL × 3). The organic phase was dried over anhydrous sodium sulfate and concentrated under a reduced pressure, and the resulting residues were purified by the silica-gel column chromatography using ethyl acetate/petroleum ether as an elution system, to obtain the title Compound **1c** (6.32 g, 22.2 mmol, two-step yield: 89.2%).
MS m/z (ESI): 283.9 [M+1]
¹H NMR (400 MHz, DMSO-*d*₆) δ 7.65 (d, *J* = 8.0 Hz, 1H), 7.43 (t, *J =* 7.6 Hz, 1H), 7.34 - 7.24 (m, 2H), 3.58 (dt, *J* = 13.6, 6.8 Hz, 1H), 3.45 (dt, *J =* 13.6, 6.8 Hz, 1H), 1.46 (d, *J* = 6.8 Hz, 6H), 1.17 (d, *J =* 6.8 Hz, 3H), 1.03 (d, *J =* 6.8 Hz, 3H).

### Step 3

### N,N-diisopropyl-2-(tetramethyl-1,3,2-dioxaboran-2-yl)benzamide 1d

Compound **1c** (1.50 g, 5.28 mmol), potassium acetate (1.04 g, 10.6 mmol) and bis(pinacolato)diboron (2.01 g, 7.92 mmol) were dissolved in dioxane (20 mL). Under a nitrogen atmosphere, Pd(dppf)Cl₂ (386 mg, 527 µmol) was added, and the mixture was stirred at 100°C to allow for reacting for 12 hours. The reaction mixture was filtered, the filtrate was concentrated under a reduced pressure, and the residues were purified by the silica-gel column chromatography using ethyl acetate/petroleum ether as an elution system, to obtain the crude product of title Compound **1d** (790 mg).
MS m/z (ESI): 332.1 [M+1]
¹H NMR (400 MHz, DMSO-*d*₆) δ 7.66 (d, *J* = 7.6 Hz, 1H), 7.50 - 7.45 (m, 1H), 7.37 - 7.32 (m, 1H), 7.15 (d, *J* = 7.6 Hz, 1H), 3.54 (s, 2H), 1.47 (d, *J =* 6.8 Hz, 6H), 1.25 (s, 12H), 1.10 - 1.05 (m, 6H).

### Step 4

### 7-bromo-5-iodopyrrolo[2,1-f][1,2,4]triazin-4-amine 1f

7-bromopyrrolo[2,1-f][1,2,4]triazin-4-amine **1e** (10.0 g, 46.9 mmol) was dissolved in *N,N*-dimethylformamide (500 mL), N-iodosuccinimide (11.6 g, 51.6 mmol) was added, and the mixture was stirred at room temperature for 12 hours. The reaction mixture was diluted with water (500 mL) and filtered under a reduced pressure to obtain a filter cake, which was the crude product of title Compound **1f** (13.7 g, 40.4 mmol, yield: 86.1%).

¹H NMR (400 MHz, DMSO-*d*₆) δ 7.97 (s, 1H), 7.08 (s, 1H).

### Step 5

### 2-{4-amino-7-bromopyrrolo[2,1-f][1,2,4]triazin-5-yl}-N,N-diisopropylbenzamide 1g

Compound **1f** (8.0 g, 23.6 mmol) was dissolved in dimethyl sulfoxide (650 mL), N,N-dimethylformamide (100 mL) and water (70 mL). Sodium carbonate (5.00 g, 47.2 mmol), Compound **1d** (9.38 g, 28.3 mmol) and tetrakis(triphenylphosphine)palladium (1.36 g, 1.18 mmol) were added, and the mixture was stirred at 60°C under a nitrogen atmosphere to allow for reacting for 12 hours. The reaction mixture was concentrated under a reduced pressure, diluted with ethyl acetate (500 mL), and then washed with water (500 mL×3). The aqueous phase was extracted with ethyl acetate (500 mL×3). The organic phases were combined, dried over anhydrous sodium sulfate, and concentrated under a reduced pressure. The resulting residues were purified by the silica-gel column chromatography using dichloromethane/methanol as an elution system, to obtain the title Compound **1g** (3.32 g, 7.97 mmol, yield: 33.8%).
MS m/z (ESI): 416.0 [M+1]
¹H NMR (400 MHz, DMSO-*d*₆) δ 8.00 (s, 1H), 7.54 - 7.43 (m, 2H), 7.39 (dd, *J* = 5.6, 2.8 Hz, 1H), 7.36 - 7.27 (m, 1H), 6.75 (s, 1H), 3.62 - 3.46 (m, 1H), 3.31 - 3.20 (m, 1H), 1.39 (d, *J* = 6.4 Hz, 3H), 0.99 (d, *J =* 62.0 Hz, 6H), 0.27 (d, *J* = 41.6 Hz, 3H).

### Step 6

### 5-(4-amino-5-{2-[diisopropylaminoformyl]phenyl}pyrrolo[2,1-f][1,2,4]triazin-7-yl)-1,2,3,6-tetrahydropyridin-1-tert-butyl formate 1h

Compound **1g** (450 mg, 1.08 mmol) and 5-(tetramethyl-1,3,2-dioxaboran-2-yl)-1,2,3,6-tetrahydropyridin-1-tert-butyl formate (401 mg, 1.30 mmol) were dissolved in dioxane (5.6 mL) and water (1.4 mL). Sodium carbonate (299 mg, 2.16 mmol) and tetrakis(triphenylphosphine)palladium (125 mg, 108 µmol) were added, and the mixture was stirred at 90°C under a nitrogen atmosphere to allow for reacting for 12 hours. The reaction mixture was filtered, the filtrate was concentrated under a reduced pressure, and the resulting residues were purified by the silica-gel column chromatography using dichloromethane/methanol as an elution system, to obtain the title Compound **1h** (294 mg, 567 µmol, yield: 52.4%).
MS m/z (ESI): 519.3 [M+1]
¹H NMR (400 MHz, DMSO-*d*₆) δ 7.95 (s, 1H), 7.49 (dd, *J =* 9.2*,* 5.6 Hz, 2H), 7.42 - 7.27 (m, 2H), 7.01 (s, 1H), 6.65 (s, 1H), 4.26 (s, 2H), 3.44 (d, *J =* 66.8 Hz, 6H), 2.31 (s, 3H), 1.45 - 1.37 (m, 12H), 1.00 (d, *J* = 73.2 Hz, 7H), 0.25 (s, 2H).

### Step 7

### 3-(4-amino-5-{2-[diisopropylaminoformyl]phenyl}pyrrolo[2,1-f][1,2,4]triazin-7-yl)piperidin-1-tert-butyl formate 1i

Compound **1h**(294 mg, 567 µmol) was dissolved in acetic acid (4 mL), followed by the addition of PtO₂ (51.5 mg, 227 µmol). Under a hydrogen atmosphere, the mixture was stirred at room temperature for 12 hours. The mixture was filtered, the filtrate was concentrated under a reduced pressure, and the residues were purified by the silica-gel column chromatography using dichloromethane/methanol as an elution system, to obtain the title Compound **1i** (207 mg, 398 µmol, yield: 70.1%).
MS m/z (ESI): 521.25 [M+1]

### Step 8

### 3-(5-{2-[diisopropylaminoformyl]phenyllpyrrolo[2,1-f][1,2,4]triazin-7-yl)piperidin-1-tert-butyl formate 1j

Compound **1i** (207 mg, 567 µmol) was dissolved in tetrahydrofuran (5.0 mL) and cooled to 0°C, followed by the addition of tert-butyl nitrite (246 mg, 2.39 mmol), and the mixture was stirred at room temperature to allow for reacting for 12 hours. The reaction mixture was concentrated under a reduced pressure, and the residues were purified by the silica-gel column chromatography using dichloromethane/methanol as an elution system, to obtain the title Compound **1j** (36.0 mg, 71.2 µmol, yield: 17.9%).
MS m/z (ESI): 506.25 [M+1]
¹H NMR (400 MHz, DMSO-*d*₆) δ 9.06 (s, 1H), 8.59 (s, 1H), 7.57 (d, *J =* 7.6 Hz, 1H), 7.52 - 7.41 (m, 2H), 7.30 (dd, *J =* 7.6, 1.2 Hz, 1H), 7.00 (d, *J =* 4.4 Hz, 1H), 5.76 (s, 1H), 4.07 (dd, *J =* 29.6, 6.4 Hz, 2H), 3.17 (d, *J =* 5.2 Hz, 3H), 2.97 - 2.82 (m, 1H), 2.08 (s, 1H), 1.83 - 1.66 (m, 2H), 1.53 (d, *J =* 16.0 Hz, 1H), 1.40 (d, *J =* 6.4 Hz, 12H), 1.08 (dd, *J =* 16.0, 6.4 Hz, 4H), 0.92 - 0.82 (m, 3H), 0.26 (t, *J* = 6.4 Hz, 3H).

### Step 9

### 2-[7-(piperidin-3-yl)pyrrolo[2,1-f][1,2,4]triazin-5-yl]-N,N-diisopropylbenzamide 1k

At 0°C, Compound **1j** (15.0 mg, 29.7 µmol) was dissolved in methane dioxide (1.5 mL) and trifluoroacetic acid (0.5 mL). The mixture was stirred at room temperature to allow for reacting for 3.0 hours. The reaction mixture was concentrated under a reduced pressure, to obtain the crude product of title Compound **1k** (12.0 mg), which was directly used in the next step of reaction without purification.
MS m/z (ESI): 406.1 [M+1]

### Step 10

### 2-(7-{1-[(2-oxo-2,3-dihydro-1,3-benzoxazol-5-yl)methyl]piperidin-3-yl}pyrrolo[2,1-f][1,2,4]triazin-5-yl)-N,N-diisopropylbenzamide 1

The crude product of Compound **1k** (12.0 mg), *N,N*-diisopropylethylamine (3.82 mg, 29.6 µmol) and 2-oxo-3*H*-1,3-benzoxazol-5-formaldehyde **11** (5.79 mg, 35.5 µmol) were dissolved in dichloromethane (3 mL), and stirred at room temperature for 1.5 hours. The mixture was cooled to 0°C, sodium triacetoxyborohydride (31.4 mg, 148 µmol) was added, and the mixture was stirred at room temperature for 12 hours. The reaction mixture was concentrated under a reduced pressure, the resulting residues were purified by the thin-layer chromatography using dichloromethane/methanol as an elution system, and the obtained crude product was purified by the HPLC (mobile phase: acetonitrile, water (0.1% formic acid), elution gradient: 45%-50%), to obtain the title Compound **1** (13.0 mg, 23.5 µmol, yield: 79.5%).
MS m/z (ESI): 553.2 [M+1]
¹H NMR (400 MHz, DMSO-*d*₆) δ 9.03 (d, *J =* 3.6 Hz, 1H), 8.54 (s, 1H), 7.56 (d, *J =* 7.6 Hz, 1H), 7.52 - 7.38 (m, 2H), 7.29 (d, *J* = 7.2 Hz, 1H), 7.17 (s, 1H), 7.05 -6.92 (m, 3H), 3.54 (s, 1H), 3.26 - 3.21 (m, 1H), 3.02 (s, 1H), 2.83 (s, 1H), 2.19 - 1.97 (m, 3H), 1.70 (s, 2H), 1.48 (s, 1H), 1.39 (t, *J =* 5.6 Hz, 3H), 1.23 (s, 1H),1.03 (dd, *J =* 25.6, 6.8 Hz, 3H), 0.91 - 0.78 (m, 3H), 0.22 (dd, *J =* 10.0, 6.8 Hz, 3H).

### Example 2

### N,N-diisopropyl-2-[7-(1-{[(1r,4r)-4-ethanesulfonamidocyclohexyl]methyl}pyrrolidin-3-yl)pyrrolo[2,1-f][1,2,4]triazin-5-yl]benzamide 2

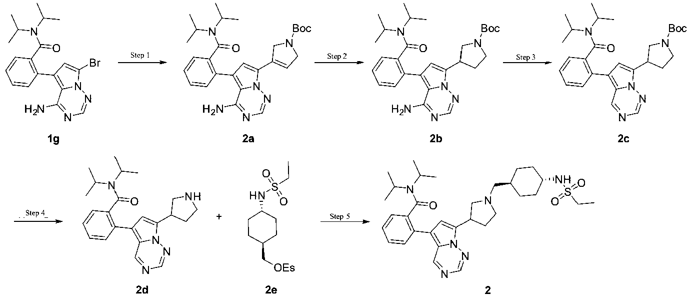

### Step 1

### 3-(4-amino-5-{2-[diisopropylaminoformyl]phenyl}pyrrolo[2,1-f][1,2,4]triazin-7-yl)-2,5-dihydro-1H-pyrrol-1-tert-butyl formate 2a

Compound **1g** (450 mg, 1.08 mmol) and 3-(tetramethyl-1,3,2-dioxaboran-2-yl)-2,5-dihydro-1*H*-pyrrol-1-tert-butyl formate (382 mg, 1.30 mmol) were dissolved in dioxane (4.0 mL) and water (1.0 mL). Potassium carbonate (299 mg, 2.16 mmol) and tetrakis(triphenylphosphine)palladium (124 mg, 108 µmol) were added, and the mixture was stirred at 90°C under a nitrogen atmosphere to allow for reacting for 12 hours. The reaction mixture was filtered, the filtrate was concentrated under a reduced pressure, and the resulting residues were purified by the silica-gel column chromatography using dichloromethane/methanol as an elution system, to obtain the title Compound **2a** (334 mg, 661 µmol, yield: 61.2%).
MS m/z (ESI): 505.3 [M+1]
¹H NMR (400 MHz, DMSO-*d*₆) δ 8.04 (s, 1H), 7.53 - 7.44 (m, 2H), 7.39 (d, *J =* 7.2 Hz, 1H), 7.33 (d, *J =* 8.4 Hz, 1H), 6.86 (d, *J* = 6.8 Hz, 1H), 6.65 (d, *J =* 14.8 Hz, 1H), 4.37 (d, *J =* 74.4 Hz, 4H), 3.58 (s, 1H), 3.28 (s, 1H), 1.49 - 1.33 (m, 12H), 0.99 (d, *J =* 62.4 Hz, 7H), 0.23 (s, 2H).

### Step 2

### 3-(4-amino-5-{2-[diisopropylaminoformyl]phenyl}pyrrolo[2,1-f][1,2,4]triazin-7-yl)pyrrolidin-1-tert-butyl formate 2b

Compound **2a** (334 mg, 661 µmol) was dissolved in acetic acid (5 mL), followed by the addition of PtO₂ (60.1 mg, 264 µmol). Under a hydrogen atmosphere, the mixture was stirred at room temperature for 12 hours. The reaction mixture was filtered, the filtrate was concentrated under a reduced pressure, and the residues were purified by the silica-gel column chromatography using dichloromethane/methanol as an elution system, to obtain the title Compound **2b** (335 mg, 661 µmol, yield: 99.9%).
MS m/z (ESI): 507.3 [M+1]
¹H NMR (400 MHz, DMSO-*d*₆) δ 7.92 (s, 1H), 7.51 - 7.41 (m, 2H), 7.36 (d, *J =* 6.8 Hz, 1H), 7.30 (d, *J =* 6.4 Hz, 1H), 6.53 (s, 1H), 3.82 (s, 2H), 3.44 (dd, *J =* 14.0, 6.8 Hz, 1H), 3.39 - 3.20 (m, 4H), 2.27 (dd, *J =* 21.2, 16.0 Hz, 1H), 2.07 - 1.90 (m, 1H), 1.86 (s, 2H), 1.40 (d, *J =* 12.0 Hz, 12H), 1.17 - 0.76 (m, 7H), 0.24 (s, 2H).

### Step 3

### 3-(5-{2-[diisopropylaminoformyl]phenyl}pyrrolo[2,1-f][1,2,4]triazin-7-yl)pyrrolidin-1-tert-butyl formate 2c

Compound **2b**(230 mg, 453 µmol) and 2-hydroxylbenzoic acid (6.27 mg, 45.4 µmol) were dissolved in tetrahydrofuran (4.6 mL) and cooled to 0°C, followed by the addition of tert-butyl nitrite (280 mg, 2.72 mmol), and the mixture was stirred at room temperature to allow for reacting for 1.0 hour, warmed to 60°C and stirred to allow for reacting for 1.0 hour. The reaction mixture was concentrated under a reduced pressure, and the residues were purified by the preparative thin-layer chromatography using ethyl acetate/petroleum ether as an elution system, to obtain the title Compound 2c (63.0 mg, 128 µmol, yield: 28.2%).
MS m/z (ESI): 492.3 [M+1]

### Step 4

### N,N-diisopropyl-2-[7-(pyrrolidin-3-yl)pyrrolo[2,1-f][1,2,4]triazin-5-yl]benzamide 2d

At 0°C, Compound **2c** (60.0 mg, 122 µmol) was dissolved in methane dioxide (0.75 mL) and trifluoroacetic acid (0.25 mL). The mixture was stirred at room temperature to allow for reacting for 2.0 hours. The mixture was concentrated under a reduced pressure, to obtain the crude product of title Compound **2d,** which was directly used in the next step of reaction without purification.
MS m/z (ESI): 392.2 [M+1]

### Step 5

### N,N-diisopropyl-2-[7-(1-{[(1r,4r)-4-ethanesulfonamidocyclohexyl]methyl}pyrrolidin-3-yl)pyrrolo[2,1-f][1,2,4]triazin-5-yl]benzamide 2

The crude product of Compound **2d** (50.0 mg) and [(1r,4r)-4-ethanesulfonamidocyclohexyl]methylethan-1-sulfonate **2e** (22.5 mg, 72 µmol) were dissolved in *N,N*-dimethylformamide (1 mL), followed by the addition of potassium carbonate (26.5 mg, 192 µmol) and potassium iodide (7.97 mg, 48.0 µmol), and the mixture was stirred at 80°C for 12 hours. The reaction mixture was concentrated under a reduced pressure, the resulting residues were purified by the silica-gel column chromatography using dichloromethane/methanol as an elution system, and the obtained crude product was purified by the HPLC (mobile phase: acetonitrile, water (0.1% formic acid), elution gradient: 35%-45%), to obtain the title Compound **2** (9.7 mg, 16.3 µmol, yield: 33.9 %).
MS m/z (ESI): 595.3 [M+1]
¹H NMR (400 MHz, DMSO-*d*₆) δ 9.04 (d, *J =* 2.4 Hz, 1H), 8.56 (s, 1H), 7.59 (d, *J =* 7.2 Hz, 1H), 7.50 - 7.42 (m, 2H), 7.30 (d, *J* = 7.2 Hz, 1H), 7.04 (d, *J =* 3.2 Hz, 1H), 7.00 (dd, *J =* 7.6, 3.2 Hz, 1H), 3.95 - 3.82 (m, 1H), 3.28 (d, *J* = 7.2 Hz, 2H), 3.06 - 2.89 (m, 4H), 2.66 (q, *J =* 8.0 Hz, 1H), 2.40 - 2.15 (m, 4H), 1.83 (d, *J =* 12.4 Hz, 5H), 1.41 (dd, *J* = 6.4, 1.6 Hz, 3H), 1.36 - 1.07 (m, 10H), 0.87 (d, *J =* 6.8 Hz, 5H), 0.25 (dd, *J =* 6.8, 2.4 Hz, 3H).

### Example 3

### 2-{7-[4-({[4-(5,6-dimethoxypyridazin-3-yl)phenyl]methyl}amino)cyclohexyl]pyrrolo[2,1-f][1,2,4]triazin-5-yl}-N,N-diisopropylbenzamide 3

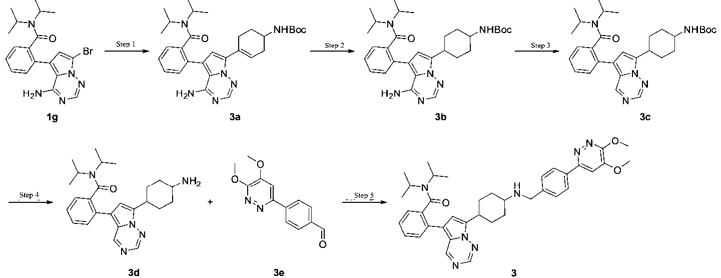

### Step 1

### N-[4-(4-amino-5-{2-[diisopropylaminoformyl]phenyl}pyrrolo[2,1-f][1,2,4]triazin-7-yl)cyclohexyl-3-ene-1-yl]tert-butyl carbamate 3a

Compound **1g** (500 mg, 1.20 mmol) and *N*-[4-(tetramethyl-1,3,2-dioxaboran-2-yl)cyclohexyl-3-ene-1-yl]tert-butyl carbamate (466 mg, 1.44 mmol) were dissolved in dioxane (8.0 mL) and water (2.0 mL). Potassium carbonate (332 mg, 2.40 mmol) and tetrakis(triphenylphosphine)palladium (138.8 mg, 120.1 µmol) were added, and the mixture was stirred at 90°C under a nitrogen atmosphere to allow for reacting for 12 hours. The reaction mixture was filtered, the filtrate was concentrated under a reduced pressure, the residues were purified by the silica-gel column chromatography using dichloromethane/methanol as an elution system, and the resulting crude product is triturated with ethyl acetate (20 mL) for purification, to obtain the title Compound **3a** (490 mg, 920 µmol, yield: 76.5%).
MS m/z (ESI): 533.3 [M+1]
¹H NMR (400 MHz, DMSO-*d*₆) δ 7.93 (s, 1H), 7.51 - 7.43 (m, 2H), 7.37 (d, *J=* 8.0 Hz, 1H), 7.31 (d, *J=* 8.4 Hz, 1H), 6.94 - 6.79 (m, 2H), 6.64 (s, 1H), 3.52 (s, 2H), 3.26 (s, 1H), 2.64 - 2.53 (m, 1H), 2.42 (s, 1H), 2.20 - 2.06 (m, 1H), 1.90 (d, *J* = 8.4 Hz, 1H), 1.62 - 1.48 (m, 1H), 1.40 (s, 13H), 1.21 - 0.10 (m, 8H).

### Step 2

### N-[4-(4-amino-5-{2-[bdiisopropylaminoformyl]phenyl}pyrrolo[2,1-f][1,2,4]triazin-7-yl)cyclohexyl]tert-butyl carbamate 3b

Compound **3a** (440 mg, 826 µmol) was dissolved in acetic acid (10 mL), followed by the addition of PtO₂ (75.0 mg, 330 µmol). Under a hydrogen atmosphere (1.5 Mpa), the mixture was stirred at room temperature for 12 hours. The reaction mixture was filtered, the filtrate was concentrated under a reduced pressure, and the residues were purified by the silica-gel column chromatography using dichloromethane/methanol as an elution system, to obtain the title Compound **3b** (433mg, 810 µmol, yield: 98.0%).
MS m/z (ESI): 535.4 [M+1]
¹H NMR (400 MHz, DMSO-*d*₆) δ 7.88 (d, *J=* 3.6 Hz, 1H), 7.51 - 7.39 (m, 2H), 7.38 - 7.33 (m, 1H), 7.32 - 7.27 (m, 1H), 6.92 - 6.75 (m, 1H), 6.65 - 6.40 (m, 1H), 3.64 - 3.49 (m, 1.43H), 3.31 - 3.21 (m, 2H), 3.04 - 2.96 (m, 0.56H), 2.09 - 1.52 (m, 7H), 1.38 (s, 12H), 1.34 - 1.29 (m, 1H), 1.19 - 0.77 (m, 7H), 0.24 (s, 2H).

### Step 3

### N-[4-(5-{2-[diisopropylaminoformyl]phenyl}pyrrolo[2,1-f][1,2,4]triazin-7-yl)cyclohexyl]tert-butyl carbamate 3c

Compound **3b** (353 mg, 660 µmol) and 2-hydroxylbenzoic acid (9.12 mg, 66.0 µmol) were dissolved in tetrahydrofuran (10.0 mL) and cooled to 0°C, followed by the addition of tert-butyl nitrite (408 mg, 3.96 mmol). The mixture was warmed to 70°C and stirred to allow for reacting for 1.0 hour. The reaction mixture was concentrated under a reduced pressure, and the residues were purified by the silica-gel column chromatography using ethyl acetate/petroleum ether as an elution system, to obtain the title Compound **3c** (140 mg, 269 µmol, yield: 40.8%). MS m/z (ESI): 520.3 [M+1]

### Step 4

### 2-[7-(4-aminocyclohexyl)pyrrolo[2,1-f][1,2,4]triazin-5-yl]-N,N-diisopropyl)benzamide 3d

At 0°C, Compound **3c** (120 mg, 231 µmol) was dissolved in methane dioxide (4.0 mL) and trifluoroacetic acid (1.0 mL). The mixture was stirred at room temperature to allow for reacting for 2.0 hours. The mixture was concentrated under a reduced pressure, to obtain the crude product of title Compound **3d,** which was directly used in the next step of reaction without purification.
MS m/z (ESI): 420.2 [M+1]

### Step 5

### 2-{7-[4-({[4-(5,6-dimethoxypyridazin-3-yl)phenyl]methyl}amino)cyclohexyl]pyrrolo[2,1-f][1,2,4]triazin-5-yl}-N,N-diisopropylbenzamide 3

Compound **3d** (49.0 mg, 117 µmol) and 4-(5,6-dimethoxypyridazin-3-yl)benzaldehyde 3e(28.5 mg, 117 µmol) were dissolved in dichloromethane (5 mL), and the mixture was stirred at 0°C for 0.5 hours. Sodium triacetoxyborohydride (124 mg, 584 µmol) was added, and the mixture was stirred at room temperature for 12 hours. The reaction mixture was concentrated under a reduced pressure, the resulting residues were purified by the silica-gel column chromatography using dichloromethane/methanol as an elution system, and the obtained crude product was purified by the HPLC (mobile phase: acetonitrile, water (0.05% ammonia water), elution gradient: 45%-90%), to obtain the title Compound 3 (2.51 mg, 3.87 µmol, yield: 3.31%).
MS m/z (ESI): 648.4 [M+1]
¹H NMR (400 MHz, DMSO-*d*₆) δ 9.04 (s, 1H), 8.54 (s, 1H), 8.03 (d, *J=* 8.0 Hz, 2H), 7.63 - 7.55 (m, 2H), 7.54 - 7.41 (m, 4H), 7.30 (d, *J=* 7.2 Hz, 1H), 7.01 (s, 1H), 4.05 (s, 3H), 3.98 (s, 3H), 3.78 (s, 2H), 3.41 - 3.38 (m, 1H), 3.27 - 3.24 (m, 1H), 2.86 (s, 1H), 2.03 - 1.57 (m, 10H), 1.40 (d, *J=* 6.8 Hz, 3H), 1.15 (d, *J =* 6.8 Hz, 3H), 0.87 (d, *J* = 6.4 Hz, 3H), 0.27 (d, *J=* 6.4 Hz, 3H).

### Example 4

### 2-{6-[4-({[4-(5,6-dimethoxypyridazin-3-yl)phenyl]methyl}amino)cyclohexyl-1-ene-1-yl]pyrrolo[1,2-a]pyrazin-8-yl}-N-ethyl-5-fluoro-N-isopropylbenzamide 4

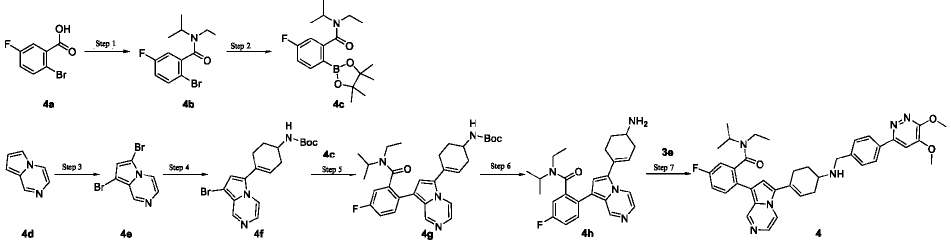

### Step 1

### 2-bromo-N-ethyl-5-fluoro-N-isopropylbenzamide 4b

N-ethylprop-2-amine (12 g, 137.67 mmol) and triethylamine (27.72 g, 273.96 mmol) were added to the solution of 2-bromo-5-fluoro-benzoic acid **4a** (20 g, 91.32 mmol) in dichloromethane (250 mL), followed by the slow addition of HATU (52.08 g, 136.98 mmol). The mixture was stirred at 25°C for 12 hours. The mixture was washed with a saturated aqueous sodium carbonate solution (100 mL×3). The organic layer was dried over anhydrous sodium sulfate, filtered and concentrated under a reduced pressure to obtain a crude product, which was purified by the silica-gel column chromatography (A: petroleum ether, B: ethyl acetate, 0% B to 15% B at A, TLC (petroleum ether :ethyl acetate =1:1, Rf=0.35)), to obtain the title Compound **4b** (24 g, 83.29 mmol, yield91.20%) as white solid.
MS m/z (ESI): 289.8 [M+1]
¹H NMR (400 MHz, DMSO-*d*₆) δ 7.80 - 7.61 (m, 1H), 7.38 - 7.29 (m, 1H), 7.22 (dt, J = 3.0, 8.6 Hz, 1H), 3.57 - 3.38 (m, 2H), 3.26 (qd, J = 7.0, 13.8 Hz, 1H), 1.25 - 1.17 (m, 6H), 1.06 (d, J = 6.6 Hz, 2H), 0.96 (t, J = 7.1 Hz, 1H)

### Step 2

### N-ethyl-5-fluoro-N-isopropyl-2-(tetramethyl-1,3,2-dioxaboran-2-yl)benzamide 4c

Compound **4b** (13 g, 45.11 mmol), potassium acetate (13.3 g, 135.52 mmol) and bis(pinacolato)diboron (35 g, 137.83 mmol) were dissolved in DMSO(200 mL). Under a nitrogen atmosphere, Pd(dppf)Cl₂.DCM (3.30 g, 4.51 mmol) was added, and the mixture was stirred at 110°C to allow for reacting for 12 hours. After the mixture was cooled to room temperature, 100 mL of water was added, and the mixture was extracted with ethyl acetate (150m1 × 3). The organic layer was dried over anhydrous sodium sulfate, filtered and concentrated under a reduced pressure to obtain a crude product, which was purified by the silica-gel column chromatography purified (A: petroleum ether, B:ethyl acetate, 0% B to 20% B at A, TLC (petroleum ether :ethyl acetate =3:1, Rf=0.2)), to obtain the title Compound **4c** (10 g, 29.83 mmol, yield: 66.12%).
MS m/z (ESI): 336.2 [M+1]
¹H NMR (400 MHz, DMSO-*d*₆) δ 7.73 (dd, J = 6.5, 8.1 Hz, 1H), 7.20 (dt, J = 2.4, 8.7 Hz, 1H), 7.09 (br dd, J = 2.4, 9.4 Hz, 1H), 3.54 (td, J = 6.6, 13.2 Hz, 1H), 3.31 - 3.27 (m, 2H), 1.28 - 1.20 (m, 16H), 1.16 (s, 4H)

### Step 3

### 6,8-dibromopyrrolo[1,2-a]pyrazine 4e

Pyrrolo[1,2-a]pyrazine **4d** (2.0 g, 16.93 mmol) was dissolved in dichloromethane (80 mL), followed by the addition of *N*-bromosuccinimide (6.03 g, 33.86 mmol), and the mixture was stirred at 25°C for 2 hours. The reaction mixture was quenched by adding water (20 mL), and extracted with dichloromethane (20 mL × 3). The organic phase was dried over anhydrous sodium sulfate, and concentrated under a reduced pressure to obtain the crude product of title Compound **4e** (4.0 g, 14.5 mmol, yield: 85.63%). The crude product was directly used in the next step of reaction without purification.
MS m/z (ESI): 276.9 [M+1].

### Step 4

### N-(4-{8-bromopyrrolo[1,2-a]pyrazin-6-yl}cyclohexyl-3-ene-1-yl)tert-butyl carbamate 4f

Compound **4e** (1.5 g, 5.44 mmol) and *N*-[4-(tetramethyl-1,3,2-dioxaboran-2-yl)cyclohexyl-3-ene-1-yl]tert-butyl carbamate (1.50 g, 4.64 mmol) were dissolved in dioxane (20 mL) and water (2.0 mL). Sodium carbonate (1.20 g, 11.32 mmol) and a 1,1-bis(diphenylphosphino)ferrocene palladium (II) dichloride dichloromethane complex (225.00 mg, 275.52 µmol) were added, and the mixture was stirred at 90°C under a nitrogen atmosphere to allow for reacting for 12 hours. The reaction mixture was cooled to room temperature, diluted with water (20 mL), and extracted with ethyl acetate (30 mL×3). The organic phases were combined, washed with saturated brine (40 mL), dried over anhydrous sodium sulfate, and concentrated under a reduced pressure. The resulting residues were purified by the silica-gel column chromatography using petroleum ether/ethyl acetate as an elution system, to obtain the title Compound **4f** (900 mg, 2.29 mmol, yield: 42.2%).
MS m/z (ESI): 393.9 [M+1]

### Step 5

### N-[4-(8-12-[ethyl(isopropyl)aminoformyl]-4-fluorophenyllpyrrolo[1,2-a]pyrazin-6-yl)cyclohexyl-3-ene-1-yl]tert-butyl carbamate 4g

Compound **4f** (900 mg, 2.29 mmol) and Compound **4c**(1.15 g, 3.44 mmol) were dissolved in dioxane (15 mL) and water (1.5 mL). Sodium carbonate (730 mg, 6.89 mmol) and a 1,1-bis(diphenylphosphino)ferrocene palladium (II) dichloride dichloromethane complex (150 mg, 230.15 µmol) were added, and the mixture was stirred at 90°C under a nitrogen atmosphere to allow for reacting for 12 hours. The reaction mixture was cooled to room temperature, diluted with water (20 mL), and extracted with ethyl acetate (20 mL×3). The organic phases were combined, washed with saturated brine (30 mL), dried over anhydrous sodium sulfate, and concentrated under a reduced pressure. The resulting residues were purified by the silica-gel column chromatography using petroleum ether/ethyl acetate as an elution system, to obtain the title Compound **4g** (600 mg, 1.15 mmol, yield: 50.23%).
MS m/z (ESI): 521.2 [M+1]

### Step 6

### 2-[6-(4-aminocyclohexyl-1-ene-1-yl)pyrrolo[1,2-a]pyrazin-8-yl]-N-ethyl-5-fluoro-N-isopropylbenzamide 4h

Compound **4g** (170 mg, 326.52 µmol) was dissolved in methane dioxide (4.94 mL), followed by the addition of trifluoroacetic acid (372.3 mg, 3.27 mmol, 0.25 mL). The mixture was stirred at room temperature to allow for reacting for 1.0 hours. The reaction mixture was concentrated under a reduced pressure, to obtain the crude product of title Compound **4h** (220 mg), which was directly used in the next step of reaction without purification.

### Step 7

### 2-{6-[4-({[4-(5,6-dimethoxypyridazin-3-yl)phenyl]methyl}amino)cyclohexyl-1-ene-1-yl]pyrrolo[1,2-a]pyrazin-8-yl}-N-ethyl-5-fluoro-N-isopropylbenzamide 4

The crude product of Compound **4h** (140 mg) was dissolved in dichloromethane (3.0 mL), followed by the addition of triethylamine (265.02 mg, 2.62 mmol) and Compound **3e**(51.18 mg, 209.52 µmol), and the mixture was stirred at room temperature for 0.5 hours. Sodium cyanoborohydride (49.38 mg, 785.72 µmol) was added, and the mixture was stirred at 45°C for 12 hours. The reaction mixture was cooled to room temperature, diluted with a saturated aqueous sodium bicarbonate solution (20 mL), and extracted with dichloromethane (20 mL × 3). The organic phases were combined, dried over anhydrous sodium sulfate, and concentrated under a reduced pressure. The resulting residues were purified by the HPLC (mobile phase: acetonitrile, water (0.1% formic acid), elution gradient: 8%-38%), to obtain the title Compound **4** (79.75 mg, 122.93 µmol, yield: 46.9%).
MS m/z (ESI): 649.2 [M+1]
¹H NMR (400 MHz, DMSO-*d*₆) δ 8.74 (s, 1H), 8.25 (d, J=5.2 Hz, 1H), 8.05 (d, J=8.4 Hz, 2H), 7.63 - 7.57 (m, 2H), 7.55 - 7.47 (m, 3H), 7.37 - 7.21 (m, 2H), 6.85 - 6.80 (m, 1H), 6.07 (s, 1H), 4.05 (s, 3H), 3.99 (s, 3H), 3.87 (s, 2H), 2.92 - 2.77 (m, 2H), 2.58 (s, 1H), 2.45 - 2.22 (m, 4H), 2.12 - 1.98 (m, 2H), 1.64 - 1.48 (m, 1H), 1.26 - 1.01 (m, 1H), 0.89 - 0.85 (m, 2H), 0.82 - 0.74 (m, 3H), 0.73 - 0.65 (m, 1H), 0.28 - 0.16 (m, 3H).

### Example 5

### N-ethyl-2-[1-methyl-6-(4-{[(2-oxo-2,3-dihydro-1,3-benzoxazol-5-yl)methylamino}cyclohexyl)-1H-pyrazolo[3,4-b]pyridin-4-yl]-N-isopropylbenzamide 5

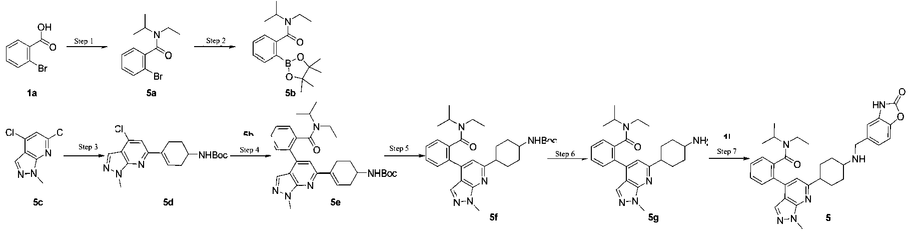

### Step 1

### 2-bromo-N-ethyl-N-isopropylbenzamide 5a

Compound **1a** (5.00 g, 24.8 mmol), isopropylethylamine (2.60 g, 29.8 mmol, 3.61 mL), HOBt (4.03 g, 29.8 mmol) and diisopropylethylamine (6.43 g, 49.7 mmol, 8.67 mL) were dissolved in dichloromethane (50 mL), and cooled to 0°C, followed by the addition of EDCI (5.72 g, 29.8 mmol), and the mixture was stirred at room temperature for 12 hours. The reaction mixture was concentrated under a reduced pressure, and the resulting residues were purified by the silica-gel column chromatography using ethyl acetate/petroleum ether as an elution system, to obtain the title Compound **5a** (6.29 g, 23.2 mmol, yield: 93.6%).
MS m/z (ESI): 269.9 [M+1]
¹H NMR (400 MHz, DMSO-*d*₆) δ 7.69 - 7.63 (m, 1H), 7.48 - 7.42 (m, 1H), 7.37 - 7.29 (m, 2H), 4.57 - 4.46 (m, 0.27H), 3.55 - 3.40 (m, 1.42H), 3.32 - 3.22 (m, 0.72H), 3.19 - 3.09 (m, 0.27H), 2.98 - 2.88 (m, 0.26H), 1.28 - 1.15 (m, 6H), 1.08 - 0.90 (m, 3H).

### Step 2

### N-ethyl-N-isopropyl-2-(tetramethyl-1,3,2-dioxaboran-2-yl)benzamide 5b

Compound **5a** (3.00 g, 11.1 mmol), potassium acetate (2.18 g, 22.2 mmol) and bis(pinacolato)diboron (4.23 g, 16.6 mmol) were dissolved in dioxane (40 mL). Under a nitrogen atmosphere, Pd(dppf)Cl₂(812 mg, 1.11 mmol) was added, and the mixture was stirred at 100°C to allow for reacting for 12 hours. The reaction mixture was cooled to room temperature, diluted with water (120 mL), and extracted with ethyl acetate (120 mL×3). The organic phases were combined, dried over anhydrous sodium sulfate, and concentrated under a reduced pressure. The resulting residues were purified by the silica-gel column chromatography using ethyl acetate/petroleum ether as an elution system, to obtain the crude product of title Compound **5b** (1.51 g). The crude product was directly used in the next step of reaction.

### Step 3

### N-(4-{4-chloro-1-methyl-1H-pyrazolo[3,4-b]pyridin-6-yl}cyclohexyl-3-ene-1-yl)tert-butyl carbamate 5d

4,6-dichloro-1-methyl-1H-pyrazolo[3,4-b]pyridine **5c**(500 mg, 2.47 mmol) and N-[4-(tetramethyl-1,3,2-dioxaboran-2-yl)cyclohexyl-3-ene-1-yl]tert-butyl carbamate (640 g, 1.98 mmol) were dissolved in dioxane (6.0 mL) and water (1.2 mL). Sodium carbonate (524 mg, 4.95 mmol) and 1,1-bis(diphenylphosphino)ferrocene palladium (II) dichloride (181 mg, 247 µmol) were added, and the mixture was stirred at 80°C under a nitrogen atmosphere to allow for reacting for 12 hours. The reaction mixture was cooled to room temperature and filtered, and the filtrate was concentrated under a reduced pressure. The resulting residues were purified by the silica-gel column chromatography using petroleum ether/ethyl acetate as an elution system, to obtain the title Compound **5d** (360 mg, 0.99 mmol, yield: 40.1%).

¹H NMR (400 MHz, DMSO-*d*₆) δ 8.16 (s, 1H), 7.58 (s, 1H), 6.89 (d, J = 7.2 Hz, 1H), 6.82 (s, 1H), 4.05 (s, 3H), 3.57 (s, 1H), 2.83 (d, J = 17.6 Hz, 1H), 2.54 (s, 1H), 2.48 - 2.45 (m, 1H), 2.16 (dd, J = 17.2, 8.8 Hz, 1H), 1.95 (d, J = 11.8 Hz, 1H), 1.58 (tt, J = 10.8, 5.2 Hz, 1H), 1.40 (s, 9H).

### Step 4

### N-[4-(4-{2-[ethyl(isopropyl)aminoformyl]-phenyl} 1-methyl-1H-pyrazolo[3,4-b]pyridin-6-yl)cyclohexyl-3-ene-1-yl]tert-butyl carbamate 5e

Compound **5d** (350 mg, 0.96 mmol) and Compound **5b**(336 mg, 1.06 mmol) were dissolved in dioxane (6.0 mL) and water (1.2 mL). Sodium carbonate (204 mg, 1.93 mmol) and 1,1-bis(diphenylphosphino)ferrocene palladium (II) dichloride (70.6 mg, 96 µmol) were added, and the mixture was stirred at 80°C under a nitrogen atmosphere to allow for reacting for 12 hours. The reaction mixture was cooled to room temperature and filtered, and the filtrate was concentrated under a reduced pressure. The resulting residues were purified by the silica-gel column chromatography using petroleum ether/ethyl acetate as an elution system, to obtain the title Compound **5e** (380 mg, 0.73 mmol, yield: 75.2%).
MS m/z (ESI): 518.3 [M+1]
¹H NMR (400 MHz, DMSO-*d*₆) δ 7.98 (d, J = 8.9 Hz, 1H), 7.68 - 7.65 (m, 1H), 7.63 - 7.56 (m, 2H), 7.48 - 7.42 (m, 1H), 7.38 (d, J = 2.8 Hz, 1H), 6.87 (d, J = 7.8 Hz, 1H), 6.67 (s, 1H), 4.06 (d, J = 2.4 Hz, 3H), 3.55 (s, 1H), 3.36 (dd, J = 12.8, 6.4 Hz, 3H), 2.79 (d, J = 39.2 Hz, 2H), 2.17 (s, 1H), 1.99 - 1.93 (m, 1H), 1.64 - 1.50 (m, 1H), 1.40 (s, 9H), 0.84 (dd, J = 6.4, 4.0 Hz, 6H), 0.67 (d, J = 6.8 Hz, 3H).

### Step 5

### N-[4-(4-{2-[ethyl(isopropyl)aminoformyl]-phenyl} 1-methyl-1H-pyrazolo[3,4-b]pyridin-6-yl)cyclohexyl]tert-butyl carbamate 5f

Compound **5e** (380 mg, 734 µmol) was dissolved in methanol (5 mL), followed by the addition of Pd/C (10 wt.% 50% water, 8.92 mg, 73.4 µmol). Under a hydrogen atmosphere (15 Psi), the mixture was stirred at room temperature for 2 hours. The reaction mixture was filtered, and the filtrate was concentrated under a reduced pressure, to obtain the crude product of title Compound **5f** (300 mg). The crude product was directly used in the next step of reaction without purification.
MS m/z (ESI): 520.4 [M+1]
¹H NMR (400 MHz, DMSO-*d*₆) δ 7.93 (d, J = 11.6 Hz, 1H), 7.65 - 7.55 (m, 3H), 7.44 (d, J = 6.0 Hz, 1H), 7.22 - 7.07 (m, 1H), 6.96 - 6.76 (m, 1H), 4.05 (d, J = 3.2 Hz, 3H), 3.40 (d, J = 17.2 Hz, 1H), 2.88 (dd, J = 21.6, 7.6 Hz, 1H), 2.77 - 2.65 (m, 1H), 2.51 (s, 2H), 1.91 (s, 3H), 1.80 - 1.55 (m, 4H), 1.39 (d, J = 1.2 Hz, 9H), 0.86 (d, J = 6.4 Hz, 3H), 0.79 - 0.65 (m, 6H).

### Step 6

### 2-[6-(4-aminocyclohexyl)-1-methyl-1H-pyrazolo[3,4-b]pyridin-4-yl]-N-ethyl-N-isopropylbenzamide 5g

Compound **5f** (300 mg, 577 µmol) was dissolved in methane dioxide (6.0 mL), followed by the addition of trifluoroacetic acid (0.5 mL) at 0°C. The mixture was stirred at room temperature to allow for reacting for 2.0 hours. The mixture was concentrated under a reduced pressure, to obtain the crude product of title Compound **5g** (245 mg), which was directly used in the next step of reaction without purification.
MS m/z (ESI): 420.3 [M+1]

### Step 7

### N-ethyl-2-[1-methyl-6-(4-{[(2-oxo-2,3-dihydro-1,3-benzoxazol-5-yl)methyl]amino}cyclohexyl)-1H-pyrazolo[3,4-b]pyridin-4-yl]-N-isopropylbenzamide 5

The crude product of Compound **5g** (80 mg, 191 µmol) was dissolved in dichloromethane (1.0 mL), followed by the addition of triethylamine to regulate the PH value of the reaction mixture to 9-10, followed by the addition of Compound 11 (31.1 mg, 191 µmol) and sodium triacetoxyborohydride (121 mg, 572 µmol) at 0°C, and the mixture was stirred at room temperature for 12 hours. The reaction mixture was concentrated under a reduced pressure. The resulting residues were purified by the HPLC (mobile phase: acetonitrile, water (0.1% trifluoroacetic acid), elution gradient: 5%-50%), to obtain the title Compound **5** (62.6 mg, 110 µmol, yield: 58.0 %).
MS m/z (ESI): 567.4 [M+1]
¹H NMR (400 MHz, DMSO-*d*₆) δ 11.88 (d, J = 9.2 Hz, 1H), 8.67 (d, J = 66.0 Hz, 2H), 7.98 (d, J = 4.8 Hz, 1H), 7.65 - 7.42 (m, 4H), 7.41 - 7.10 (m, 4H), 4.23 (d, J = 5.2 Hz, 2H), 4.07 (d, J = 14.8 Hz, 3H), 3.42 - 2.70 (m, 5H), 2.30 - 1.49 (m, 8H), 1.09 - 0.13 (m, 9H).

### Example 6

### N-ethyl-2-[1-methyl-6-(1-{[(1r,4r)-4-ethanesulfonamidocyclohexyl]methyl}pyrrolidin-3-yl)-1H-pyrazolo[3,4-b]pyridin-4-yl]-N-isopropylbenzamide 6

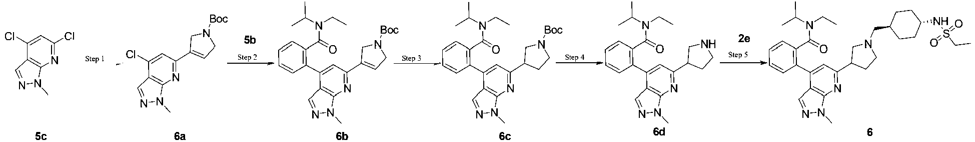

### Step 1

### 3-{4-chloro-1-methyl-1H-pyrazolo[3,4-b]pyridin-6-yl}-2,5-dihydro-1H-pyrrol-1-tert-butyl formate 6a

4,6-Dichloro-1-methyl-1H-pyrazolo[3,4-b]pyridine **5c** (200 mg, 989 µmol) was dissolved in dioxane (3.0 mL) and water (0.6 mL). Sodium carbonate (209 mg, 1.98 mmol), 3-(tetramethyl-1,3,2-dioxaboran-2-yl)-2,5-dihydro-1*H-*pyrrol-1-tert-butyl formate (233 mg, 791.9 µmol) and Pd(dppf)Cl₂(72.4 mg, 98.9 µmol) were added, and the mixture was stirred at 80°C under a nitrogen atmosphere to allow for reacting for 12 hours. The reaction mixture was cooled to room temperature and filtered, and the filtrate was concentrated under a reduced pressure. The resulting residues were purified by the silica-gel column chromatography using petroleum ether/ethyl acetate as an elution system, to obtain the title Compound **6a** (150 mg, 448 µmol, yield: 45.2%).
MS m/z (ESI): 279.1 [M-55]
¹H NMR (400 MHz, DMSO-*d*₆) δ 8.23 (s, 1H), 7.83 (d, *J=* 2.0 Hz, 1H), 6.99 (d, *J=* 12.8 Hz, 1H), 4.54 (s, 2H), 4.32 (s, 2H), 4.06 (d, *J* = 1.2 Hz, 3H), 1.46 (d, *J =* 8.8 Hz, 9H).

### Step 2

### 3-(4-{2-[ethyl(isopropyl)aminoformyl]-phenyl}1-methyl-1H-pyrazolo[3,4-b]pyridin-6-yl)-2,5-dihydro-1H-pyrrol-1-tert-butyl formate 6b

Compound **6a** (130 mg, 388 µmol) and Compound **5b** (184 mg, 582 µmol) were dissolved in dioxane (3.0 mL) and water (0.6 mL). Sodium carbonate (204 mg, 1.93 mmol) and 1,1-bis(diphenylphosphino)ferrocene palladium (II) dichloride (70.6 mg, 96 µmol) were added, and the mixture was stirred at 100°C under a nitrogen atmosphere to allow for reacting for 16 hours. The reaction mixture was cooled to room temperature, and concentrated under a reduced pressure. The resulting residues were purified by the preparative thin-layer chromatography using petroleum ether/ethyl acetate as an elution system, to obtain the title Compound **6b** (60 mg, 122.5 µmol, yield: 31.5%).

### Step 3

### 3-(4-{2-[ethyl(isopropyl)aminoformyl]-phenyl}1-methyl-1H-pyrazolo[3,4-b]pyridin-6-yl)pyrrolidin-1-tert-butyl formate 6c

Compound **6b** (60 mg, 122.5 µmol) was dissolved in methanol (2.0 mL), followed by the addition of Pd/C (6.0 mg). Under a hydrogen atmosphere (15 Psi), the mixture was stirred at room temperature for 2 hours. The reaction mixture was filtered, the filtrate was concentrated under a reduced pressure, and the residues were purified by the silica-gel column chromatography using petroleum ether/ethyl acetate as an elution system, to obtain the title Compound **6c** (53.0 mg, 107 µmol, yield: 87.9%).
MS m/z (ESI): 492.3 [M+1]
¹H NMR (400 MHz, DMSO-*d*₆) δ 7.98 (s, 1H), 7.61 (dd, *J=* 10.4, 6.4 Hz, 3H), 7.46 (dd, *J =* 12.0, 6.4 Hz, 1H), 7.14 (s, 1H), 4.05 (s, 3H), 3.76 - 3.36 (m, 5H), 2.89 (s, 1H), 2.25 (s, 1H), 2.20 - 2.11 (m, 1H), 1.41 (d, *J* = 4.8 Hz, 9H), 0.90 - 0.14 (m, 9H).

### Step 4

### N-ethyl-2-[1-methyl-6-(pyrrolidin-3-yl)-1H-pyrazolo[3,4-b]pyridin-4-yl]-N-isopropylbenzamide 6d

Compound **6c** (47.0 mg, 95.6 µmol) was dissolved in methane dioxide (4.0 mL), followed by the addition of trifluoroacetic acid (1.0 mL) at 0°C. The mixture was stirred at room temperature to allow for reacting for 2.0 hours. The reaction mixture was concentrated under a reduced pressure, to obtain the crude product of title Compound **6d** (38 mg), which was directly used in the next step of reaction without purification.
MS m/z (ESI): 392.3 [M+1]

### Step 5

### N-ethyl-2-[1-methyl-6-(1-{[(1r,4r)-4-ethanesulfonamidocyclohexyl]methyl}pyrrolidin-3-yl)-1H-pyrazolo[3,4-b]pyridin-4-yl]-N-isopropylbenzamide 6

The crude product of Compound **6d** (38.0 mg, 97.0 µmol) and [(1r,4r)-4-ethanesulfonamidocyclohexyl]methylethan-1-sulfonate **2e**(45.8 mg, 97.0 µmol) were dissolved in *N,N-*dimethylformamide (2.0 mL), followed by the addition of potassium carbonate (53.6 mg, 388 µmol) and potassium iodide (16.1 mg, 97.0 µmol), and the mixture was stirred at 80°C for 16 hours. The reaction mixture was concentrated under a reduced pressure, the residues were purified by the preparative thin-layer chromatography, and the obtained crude product was purified by the HPLC (mobile phase: acetonitrile, water (0.1% formic acid), elution gradient: 55%-40%), to obtain the title Compound **6** (13.0 mg, 21.8 µmol, yield: 22.5%) as white solid.
MS m/z (ESI): 595.3 [M+1]
¹H NMR (400 MHz, DMSO-*d*₆) δ 7.91 (s, 1H), 7.62 - 7.54 (m, 3H), 7.42 (d, *J =* 4.4 Hz, 1H), 7.16 (s, 1H), 4.05 (s, 3H), 3.71 - 3.60 (m, 1H), 3.42 (d, *J =* 6.8 Hz, 1H), 3.22 - 2.71 (m, 9H), 2.47 (d, *J=* 12.4 Hz, 1H), 2.29 (s, 2H), 2.18 - 2.07 (m, 1H), 1.94 - 1.79 (m, 4H), 1.42 (d, *J=* 20.8 Hz, 1H), 1.33 - 0.06 (m, 17H).

### Example 8

### 2-{6-[5-({[4-(5,6-dimethoxypyridazin-3-yl)phenyl]methyl}amino)cyclohexyl-1-ene-1-yl]1-methyl-1H-pyrazolo[3,4-b]pyridin-4-yl}-N-ethyl-N-isopropylbenzamide 8

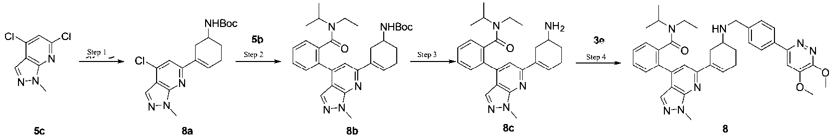

### Step 1

### N-(3-{4-chloro-1-methyl-1H-pyrazolo[3,4-b]pyridin-6-yl}cyclohexyl-3-ene-1-yl)tert-butyl carbamate 8a

4,6-Dichloro-1-methyl-1H-pyrazolo[3,4-b]pyridine **5c** (300 mg, 1.48 mmol) was dissolved in dioxane (12 mL) and water (2.0 mL). Sodium carbonate (315 mg, 2.97 mmol) , *N*-[3-(tetramethyl-1,3,2-dioxaboran-2-yl)cyclohexyl-3-ene-1-yl]tert-butyl carbamate (504 mg, 1.56 mmol) and Pd(dppf)Cl₂ (109 mg, 149 µmol) were added, and the mixture was stirred at 80°C under a nitrogen atmosphere to allow for reacting for 12 hours. The reaction mixture was cooled to room temperature and filtered, and the filtrate was concentrated under a reduced pressure. The resulting residues were purified by the silica-gel column chromatography using petroleum ether/ethyl acetate as an elution system, to obtain the title Compound **8a** (220 mg, 606 µmol, yield: 40.8%) as white solid.
MS m/z (ESI): 363.1 [M+1]
¹H NMR (400 MHz, DMSO-*d*₆) δ 8.16 (s, 1H), 7.57 (s, 1H), 6.95 *(d, J=* 7.6 Hz, 1H), 6.86 (s, 1H), 4.04 (s, 3H), 3.62 (s, 1H), 2.93 (d, *J* = 15.6 Hz, 1H), 2.32 (dd, *J=* 16.8, 10.4 Hz, 3H), 1.83 (d, *J=* 12.4 Hz, 1H), 1.58 - 1.39 (m, 10H).

### Step 2

### N-[3-(4-{2-[ethyl(isopropyl)aminoformyl]phenyl}-1-methyl-1H-pyrazolo[3,4-b]pyridin-6-yl)cyclohexyl-3-ene-1-yl]tert-butyl carbamate 8b

Compound **8a** (250 mg, 689 µmol) and sodium carbonate (146 mg, 1.38 mmol) were dissolved in dioxane (6.0 mL) and water (1.0 mL). Compound **5b** (437 mg, 689 µmol) and 1,1-bis(diphenylphosphino)ferrocene palladium (II) dichloride (50.4 mg, 68.9 µmol) were added, and the mixture was stirred at 100°C under a nitrogen atmosphere to allow for reacting for 12 hours. The reaction mixture was cooled to room temperature and filtered, and the filtrate was concentrated under a reduced pressure. The resulting residues were purified by the silica-gel column chromatography using petroleum ether/ethyl acetate as an elution system, to obtain the title Compound **8b** (227 mg, 439 µmol, yield: 63.7%).
MS m/z (ESI): 518.3 [M+1]

### Step 3

2-[6-(5-aminocyclohexyl-1-ene-1-yl)-1-methyl-1*H*-pyrazolo[3,4-b]pyridin-4-yl]-*N*-ethyl-*N*-isopropylbenzamide **8c** Compound **8b** (90.0 mg, 174 µmol) was dissolved in methane dioxide (2.1 mL), followed by the addition of trifluoroacetic acid (0.7 mL) at 0°C. The mixture was stirred at room temperature to allow for reacting for 1.0 hours. The reaction mixture was concentrated under a reduced pressure, to obtain the crude product of title Compound **8c** (72 mg), which was directly used in the next step of reaction without purification.
MS m/z (ESI): 418.3 [M+1]

### Step 4

### 2-{6-[5-({[4-(5,6-dimethoxypyridazin-3-yl)phenyl]methyl}amino)cyclohexyl-1-ene-1-yl]1-methyl-1H-pyrazolo[3,4-b]pyridin-4-yl}-N-ethyl-N-isopropylbenzamide 8

The crude product of Compound **8c** (72 mg, 172 µmol) was dissolved in dichloromethane (4.0 mL), followed by the addition of Compound **3e** (42 mg, 172 µmol). The mixture was cooled to 0°C, sodium triacetoxyborohydride (183 mg, 862 µmol) was added, and the mixture was stirred at room temperature for 12 hours. The reaction mixture was concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 50%-90%), to obtain the title Compound **8** (20.3 mg, 31.4 µmol, yield: 14.7 %).
MS m/z (ESI): 646.3 [M+1]
¹H NMR (400 MHz, DMSO-*d*₆) δ 8.04 (d, *J =* 8.0 Hz, 2H), 7.97 (d, *J =* 8.8 Hz, 1H), 7.70 - 7.64 (m, 1H), 7.63 - 7.51 (m, 5H), 7.49 - 7.42 (m, 1H), 7.38 (d, *J =* 4.0 Hz, 1H), 6.71 (s, 1H), 4.29 - 4.19 (m, 0.23 H), 4.05 (s, 6H), 3.98 (s, 3H), 3.92 (s, 2H), 3.40 - 3.35 (m, 1H), 3.01 (dd, *J =* 37.2, 16.0 Hz, 1H), 2.89 - 2.69 (m, 2H), 2.44 - 2.18 (m, 4H), 1.97 (d, *J =* 11.2 Hz, 1H), 1.41 (d, *J =* 6.8 Hz, 1H), 1.08 - 0.61 (m, 7H), 0.17 (d, *J =* 6.4 Hz, 2H).

### Example 9

### 2-{6-[3-({[4-(5,6-dimethoxypyridazin-3-yl)phenyl]methyl}amino)piperidin-1-yl]-1-methyl-1H-pyrazolo[3,4-b]pyridin-4-yl}-N-ethyl-N-isopropylbenzamide 9

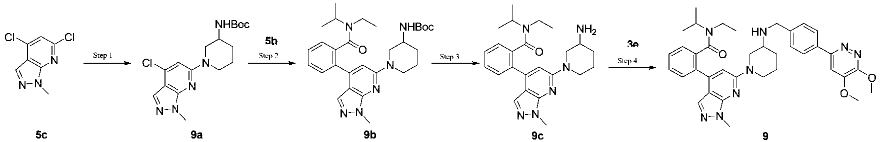

### Step 1

### N-(1-{4-chloro-1-methyl-1H-pyrazolo[3,4-b]pyridin-6-yl}piperidin-3-yl)tert-butyl carbamate 9a

4,6-Dichloro-1-methyl-1H-pyrazolo[3,4-b]pyridine **5c** (300 mg, 1.48 mmol) and N-(piperidin-3-yl)tert-butyl carbamate (297 mg, 1.48 mmol) were dissolved in toluene (6 mL). Pd₂(dba)₃ (85.3 mg, 148 µmol), BINAP (184 mg, 296 µmol) and K₂CO₃ (410 mg, 2.97 mmol) were added, and the mixture was stirred at 80°C under a nitrogen atmosphere to allow for reacting for 16 hours. The reaction mixture was cooled to room temperature and filtered, and the filtrate was concentrated under a reduced pressure. The resulting residues were purified by the silica-gel column chromatography using petroleum ether/ethyl acetate as an elution system, to obtain the title Compound **9a** (60.0 mg, 164 µmol, yield: 11%) as colorless oil.
MS m/z (ESI): 366.1 [M+1]

### Step 2

### N-[1-(4-{2-[ethyl(isopropyl)aminoformyl]phenyl}-1-methyl-1H-pyrazolo[3,4-b]pyridin-6-yl)piperidin-3-yl]tert-butyl carbamate 9b

Compound **9a** (55.0 mg, 150 µmol) and Compound **5b** (95.3 mg, 300 µmol) were dissolved in dioxane (4.0 mL) and water (1.0 mL). Xphos-Pd-G2 (11.8 mg, 15.0 µmol), Xphos (14.3 mg, 30.0 µmol) and Cs₂CO₃ (97.9 mg, 300 µmol) were added, and the mixture was stirred at 80°C under a nitrogen atmosphere to allow for reacting for 16 hours. The reaction mixture was cooled to room temperature, and concentrated under a reduced pressure. The resulting residues were purified by the preparative thin-layer chromatography using petroleum ether/ethyl acetate as an elution system, to obtain the title Compound **9b** (64.0 mg, 122 µmol, yield: 81.7%).
MS m/z (ESI): 521.3 [M+1]

### Step 3

### 2-[6-(3-aminopiperidin-1-yl)-1-methyl-1H-pyrazolo[3,4-b]pyridin-4-yl]-N-ethyl-N-isopropylbenzamide 9c

Compound **9b** (50.0 mg, 96 µmol) was dissolved in methane dioxide (2.0 mL), followed by the addition of trifluoroacetic acid (0.5 mL) at 0°C. The mixture was stirred at room temperature to allow for reacting for 2.0 hours. The reaction mixture was concentrated under a reduced pressure, to obtain the crude product of title Compound **9c** (40 mg), which was directly used in the next step of reaction without purification.
MS m/z (ESI): 421.2 [M+1]

### Step 4

### 2-{6-[3-({[4-(5,6-dimethoxypyridazin-3-yl)phenyl]methyl}amino)piperidin-1-yl]-1-methyl-1H-pyrazolo[3,4-b]pyridin-4-yl}-N-ethyl-N-isopropylbenzamide 9

The crude product of Compound **9c** (40 mg, 95.1 µmol) was dissolved in dichloromethane (2.5 mL), followed by the addition of Compound **3e** (23.2 mg, 95.1 µmol). The mixture was cooled to 0°C, sodium triacetoxyborohydride (141 mg, 665 µmol) was added, and the mixture was stirred at room temperature for 16 hours. The reaction mixture was concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% formic acid), elution gradient: 5%-50%), to obtain the title Compound **9** (26.0 mg, 40 µmol, yield: 42.1 %) as white solid.
MS m/z (ESI): 649.3 [M+1]
¹H NMR (400 MHz, DMSO-*d*₆) δ 8.05 - 7.83 (m, 2H), 7.74 - 7.25 (m, 8H), 6.78 - 6.63 (m, 1H), 4.50 - 4.39 (m, 1H), 4.28 - 4.11 (m, 2H), 4.05 (s, 3H), 3.98 (s, 3H), 3.91 (d, *J =* 8.4 Hz, 2H), 3.86 (s, 3H), 3.13 - 2.62 (m, 5H), 1.98 (s, 1H), 1.74 (s, 1H), 1.39 (s, 2H), 1.14 - 0.55 (m, 7H), 0.31 - 0.14 (m, 2H).

### Example 11

### N-ethyl-5-fluoro-2-[1-methyl-6-(1-{[(1r,4r)-4-ethanesulfonamidocyclohexylmethyl}pyrrolidin-3-yl)-1H-pyrazolo[3,4-b]pyridin-4-yl]-N-(isopropyl)benzamide 11

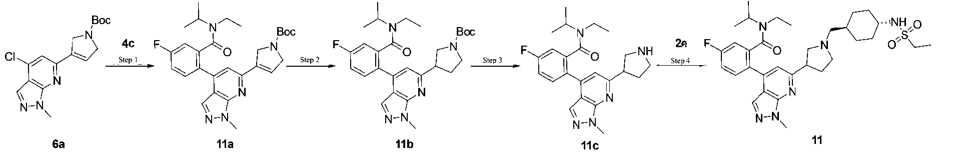

### Step 1

### 3-(4-{2-[ethyl(isopropyl)aminoformyl]-4-fluorophenyl} 1-methyl-1H-pyrazolo[3,4-b]pyridin-6-yl)-2,5-dihydro-1H-pyrrol-1-tert-butyl formate 11a

Compound **6a** (100 mg, 299 µmol) and Compound **4c** (215 mg, 448 µmol) were dissolved in dioxane (8.0 mL) and water (2.0 mL). Sodium carbonate (63.3 mg, 597 µmol) and Pd(dppf)Cl₂ (21.8 mg, 29.8 µmol) were added, and the mixture was stirred at 100°C under a nitrogen atmosphere to allow for reacting for 16 hours. The reaction mixture was cooled to room temperature and filtered, and the filtrate was concentrated under a reduced pressure. The resulting residues were purified by the silica-gel column chromatography using petroleum ether/ethyl acetate as an elution system, to obtain the title Compound **11a** (97.0 mg, 191 µmol, yield: 63.9%) as white solid.
MS m/z (ESI): 508.2 [M+1]

### Step 2

### 3-(4-{2-[ethyl(isopropyl)aminoformyl]-4-fluorophenyl} 1-methyl-1H-pyrazolo[3,4-b]pyridin-6-yl)pyrrolidin-1-tert-butyl formate 11b

Compound **11a** (97.0 mg, 191 µmol) was dissolved in methanol (10 mL), followed by the addition of Pd/C (10 mg). Under a hydrogen atmosphere (15 Psi), the mixture was stirred at room temperature for 2 hours. The reaction mixture was filtered, and the filtrate was concentrated under a reduced pressure, to obtain the crude product of title Compound **11b** (100 mg). The crude product was directly used in the next step of reaction without purification.
MS m/z (ESI): 510.2 [M+1]

### Step 3

### N-ethyl-5-fluoro-2-[1-methyl-6-(pyrrolidin-3-yl)-1H-pyrazolo[3,4-b]pyridin-4-yl]-N-isopropylbenzamide 11c

Compound **11b** (50.0 mg, 98.1 µmol) was dissolved in methane dioxide (2.0 mL), followed by the addition of trifluoroacetic acid (0.4 mL) at 0°C. The mixture was stirred at room temperature to allow for reacting for 2.0 hours. The reaction mixture was concentrated under a reduced pressure, to obtain the crude product of title Compound **11c** (40 mg), which was directly used in the next step of reaction without purification.
MS m/z (ESI): 410.2 [M+1]

### Step 5

### N-ethyl-5-fluoro-2-[1-methyl-6-(1-{[(1r,4r)-4-ethanesulfonamidocyclohexyl]methyl}pyrrolidin-3-yl)-1H-pyrazolo[3,4-b]pyridin-4-yl]-N-(isopropyl)benzamide 11

The crude product of Compound **11c** (40mg) and [(1r,4r)-4-ethanesulfonamidocyclohexyl]methylethan-1-sulfonate **2e** (166 mg, 352 µmol) were dissolved in *N,N*-dimethylformamide (6.0 mL), followed by the addition of potassium carbonate (162 mg, 1.17 mmol) and potassium iodide (48.6 mg, 293 µmol), and the mixture was stirred at 80°C for 16 hours. The reaction mixture was cooled to room temperature and filtered, and the filtrate was concentrated under a reduced pressure. The resulting residues were purified by the silica-gel column chromatography using dichloromethane/methanol as an elution system, and the obtained crude product was purified by the HPLC (mobile phase: acetonitrile, water (0.1% formic acid), elution gradient: 5%-60%), to obtain the title Compound **11** (70.0 mg, 114 µmol, yield: 38.9 %) as white solid.
MS m/z (ESI): 613.3 [M+1]
¹H NMR (400 MHz, DMSO-*d*₆) δ 7.95 (s, 1H), 7.68 (dd, *J=* 8.4, 5.2 Hz, 1H), 7.48 - 7.33 (m, 2H), 7.13 (s, 1H), 7.00 *(d, J=* 7.6 Hz, 1H), 4.04 (s, 3H), 3.63 - 3.56 (m, 2H), 3.08 - 2.62 (m, 10H), 2.40 - 2.01 (m, 4H), 1.84 (s, 4H), 1.54 - 0.06 (m, 18H).

### Examples 11-1 and 11-2

### N-ethyl-5-fluoro-2-{1-methyl-6-[(3R)-1-{[(1r,4r)-4-ethanesulfonamidocyclohexylmethyl}pyrrolidin-3-yl]-1H-pyrazolo[3,4-b]pyridin-4-yl}-N-(isopropyl)benzamide 11-1

### N-ethyl-5-fluoro-2-{1-methyl-6-[(3S)-1-{[(1r,4r)-4-ethanesulfonamidocyclohexyl]methyl}pyrrolidin-3-yl]-1H-pyrazolo[3,4-b]pyridin-4-yl}-N-(isopropyl)benzamide 11-2

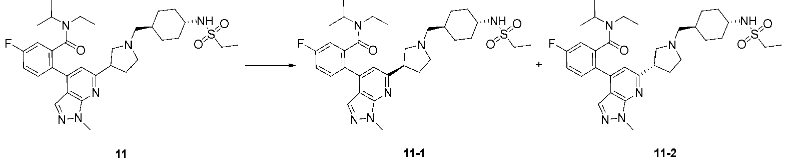

Racemic Compound **11** (68 mg, 111 µmol) was purified by the chiral preparative SFC (chiral preparative column: CHIRALCEL AD (250 mm * 30 mm, 10 um); mobile phase: n-hexane/isopropanol/diethylamine = 70/30/0.1 (V/V/V)), and the corresponding components were collected and concentrated under a reduced pressure. The obtained respective components were respectively purified by the HPLC (mobile phase: acetonitrile, water (0.1% formic acid), elution gradient: 10%-35%), to obtain the title Compounds **11-1** (12 mg, 0.02 mmol, yield: 17.6%) and **11-2** (7.0 mg, 0.01 mmol, yield: 10.2%).

### Single-configuration Compound 11-1:

MS m/z (ESI):613.2 [M+1]
Chiral SFC analysis: retention time: 2.310 min; chromatographic column: DAICEL AD-H 4.6 mm I.D.*250 mm L 5 µm; mobile phase: CO₂/IPA[0.1% NH₃ (7M methanol solution)] = 60/40.

1H NMR (400 MHz, DMSO-*d*₆) δ 7.95 (d, *J=* 2.8 Hz, 1H), 7.67 (dt, *J* = 9.2, 4.8 Hz, 1H), 7.50 - 7.33 (m, 2H), 7.13 (s, 1H), 7.00 (d, *J=* 7.6 Hz, 1H), 4.25 - 4.13(m, 0.25H), 4.04 (d, *J* = 2.0 Hz, 3H), 3.62 - 3.49 (m, 1.78H), 3.05 - 2.56 (m, 9H), 2.36 - 2.16 (m, 3H), 2.08 (s, 1H), 1.83 (t, *J* = 17.2 Hz, 4H), 1.42 - 1.28 (m,1H), 1.27 - 1.13 (m, 5H), 1.06 - 0.65 (m, 9H), 0.21 (t, *J =* 6.0 Hz, 2H).

### Single-configuration Compound 11-2:

MS m/z (ESI):613.2 [M+1]
Chiral SFC analysis: retention time: 2.425 min; chromatographic column: DAICEL AD-H 4.6 mm I.D.*250 mm L 5 µm; mobile phase: CO₂/IPA[0.1% NH₃ (7M methanol solution)] = 60/40.

¹H NMR (400 MHz, DMSO-*d*₆) δ 7.74 (s, 1H), 7.46 (s, 1H), 7.31 - 7.10 (m, 2H), 6.92 (s, 1H), 6.80 (d, *J =* 6.4 Hz, 1H), 4.03 - 3.94 (m, 0.47H), 3.83 (s, 3H), 3.48 -3.37 (m, 1.62H), 2.85 - 2.67 (m, 5H), 2.60 - 2.38 (m, 4H), 2.19 - 1.48 (m, 9H), 1.23 - 0.40 (m, 16H).

### Example 13

### 2-{6-[4-({[4-(5,6-dimethoxypyridazin-3-yl)phenyl]methyl}amino)piperidin-1-yl]-1-methyl-1H-pyrazolo[3,4-b]pyridin-4-yl}-N-ethyl-5-fluoro-N-isopropylbenzamide 13

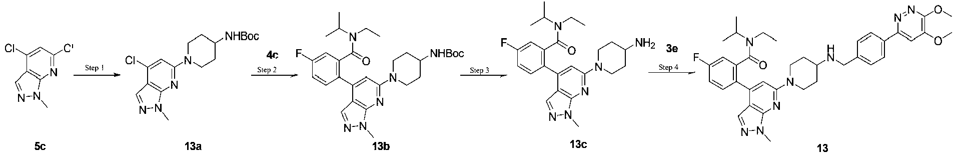

### Step 1

### N-(1-{4-chloro-1-methyl-1H-pyrazolo[3,4-b]pyridin-6-yl}piperidin-4-yl)tert-butyl carbamate 13a

4,6-Dichloro-1-methyl-1H-pyrazolo[3,4-b]pyridine **5c** (450 mg, 2.23 mmol) and *N*-(piperidin-4-yl)tert-butyl carbamate (446 mg, 2.23 mmol) were dissolved in toluene (8 mL). Pd₂(dba)₃ (256 mg, 445 µmol), BINAP (554 mg, 890 µmol) and K₂CO₃ (615 mg, 4.45 mmol) were added, and the mixture was stirred at 80°C under a nitrogen atmosphere to allow for reacting for 12 hours. The reaction mixture was cooled to room temperature, and filtered through diatomite, and the filtrate was concentrated under a reduced pressure. The resulting residues were purified by the preparative thin-layer chromatography using petroleum ether/ethyl acetate as an elution system, to obtain the title Compound **13a** (78.0 mg, 213 µmol, yield: 9.57%) as yellow solid.
MS m/z (ESI): 366.2 [M+1]
¹H NMR (400 MHz, DMSO-*d*₆) δ 7.83 (s, 1H), 6.95 (s, 1H), 6.85 *(d, J=* 7.6 Hz, 1H), 4.37 (d, *J =* 13.2 Hz, 2H), 3.85 (s, 3H), 3.53 (s, 1H), 3.02 (dd, *J =* 18.4, 6.8 Hz, 2H), 1.80 (d, *J=* 10.4 Hz, 2H), 1.39 (s, 9H), 1.35 (dd, *J* = 7.6, 4.4 Hz, 2H).

### Step 2

### N-[1-(4-{2-[ethyl(isopropyl)aminoformyl]-4-fluorophenyl}-1-methyl-1H-pyrazolo[3,4-b]pyridin-6-yl)piperidin-4-yl]tert-butyl carbamate 13b

Compound **13a** (73.0 mg, 199 µmol) and Compound **4c** (133 mg, 199 µmol) were dissolved in dioxane (7.3 mL) and water (1.5 mL). Xphos-Pd-G2 (15.7 mg, 19.9 µmol), Xphos (19.0 mg, 39.9 µmol) and Cs₂CO₃ (130 mg, 399 µmol) were added, and the mixture was stirred at 80°C under a nitrogen atmosphere to allow for reacting for 12 hours. The reaction mixture was cooled to room temperature, and concentrated under a reduced pressure. The residues were diluted with water (20 mL), and extracted with ethyl acetate (20.0 mL×3). The organic phase was dried over anhydrous sodium sulfate, and concentrated under a reduced pressure. The resulting residues were purified by the preparative thin-layer chromatography using petroleum ether/ethyl acetate as an elution system, and the obtained crude product was purified by the high-performance liquid chromatography (mobile phase: acetonitrile, water (0.1% formic acid), elution gradient: 55%-65%), to obtain the title product **13b** (20.0 mg, 37.1 µmol, yield: 18.6%).
MS m/z (ESI): 539.3 [M+1]
¹H NMR (400 MHz, DMSO-*d*₆) δ 7.66 (s, 2H), 7.45 - 7.31 (m, 2H), 6.88 (d, *J =* 7.6 Hz, 1H), 6.69 (d, *J=* 4.4 Hz, 1H), 4.39 - 4.21 (m, 2H), 3.87 (d, *J =* 2.8 Hz, 3H), 3.52 (s, 1H), 3.40 - 3.34 (m, 2H), 3.01 (dd, *J =* 23.6, 11.6 Hz, 2H), 2.89 (dd, *J=* 13.6, 7.2 Hz, 1H), 1.81 (d, *J=* 11.2 Hz, 2H), 1.39 (s, 11H), 0.77 (s, 7H), 0.26 (d, *J =* 6.8 Hz, 2H).

### Step 3

### 2-[6-(4-aminopiperidin-1-yl)-1-methyl-1H-pyrazolo[3,4-b]pyridin-4-yl]-N-ethyl-5-fluoro-N-isopropylbenzamide 13c

Compound **13b** (15.0 mg, 27.8 µmol) was dissolved in methane dioxide (0.3 mL), followed by the addition of trifluoroacetic acid (0.1 mL) at 0°C. The mixture was stirred at room temperature to allow for reacting for 1.0 hours. The reaction mixture was concentrated under a reduced pressure, to obtain the crude product of title Compound **13c** (12 mg), which was directly used in the next step of reaction without purification.
MS m/z (ESI): 439.2 [M+1]

### Step 4

### 2-{6-[4-({[4-(5,6-dimethoxypyridazin-3-yl)phenyl]methyl}amino)piperidin-1-yl]-1-methyl-1H-pyrazolo[3,4-b]pyridin-4-yl}-N-ethyl-5-fluoro-N-isopropylbenzamide 13

The crude product of Compound 13c (12.0 mg, 37.3 µmol) was dissolved in dichloromethane (1.5 mL), followed by the addition of Compound 3e (6.68 mg, 27.3 µmol), and the mixture was stirred at room temperature for 1.5 hours. The mixture was cooled to 0°C, sodium triacetoxyborohydride (29.0 mg, 136 µmol) was added, and the mixture was stirred at room temperature for 12 hours. The reaction mixture was concentrated under a reduced pressure. The resulting residues were purified by the preparative thin-layer chromatography using dichloromethane/methanol as an elution system, to obtain the title Compound 13 (12.0 mg, 18 µmol, yield: 65.7%) as white solid.
MS m/z (ESI): 667.3 [M+1]
¹H NMR (400 MHz, DMSO-*d*₆) δ 8.03 (d, *J=* 8.0 Hz, 2H), 7.66 (dd, *J =* 12.4, 4.4 Hz, 2H), 7.59 (s, 1H), 7.50 (d, *J=* 8.0 Hz, 2H), 7.38 (ddd, *J* = 19.2, 10.0, 4.0 Hz, 2H), 6.70 (d, *J=* 6.0 Hz, 1H), 4.26 (s, 2H), 4.05 (s, 3H), 3.98 (s, 3H), 3.87 (d, *J=* 3.2 Hz, 3H), 3.83 (s, 2H), 3.03 (d, *J=* 9.2 Hz, 2H), 2.89 (dd, *J=* 13.2, 6.8 Hz, 1H), 2.70 (s, 1H), 1.94 (d, *J= 11.6* Hz, 2H), 1.30 *(d, J=* 9.6 Hz, 2H), 1.11 - 0.63 (m, 7H), 0.26 *(d, J=* 6.8 Hz, 2H).

### Example 14

### N-ethyl-5-fluoro-N-isopropyl-2-[6-(1-{[(1r,4r)-4-ethanesulfonamidocyclohexyl]methyl}pyrrolidin-3-yl)-1H-pyrazolo[3,4-b]pyridin-4-yl]benzamide 14

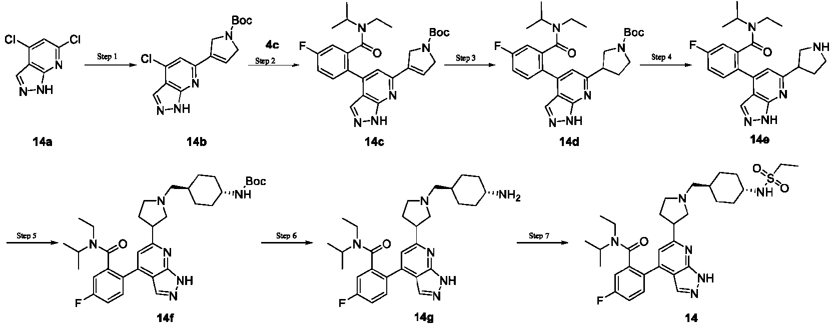

### Step 1

### 3-{4-chloro-1H-pyrazolo[3,4-b]pyridin-6-yl}-2,5-dihydro-1H-pyrrol-1-tert-butyl formate 14b

4,6-Dichloro-1H-pyrazolo[3,4-b]pyridine **14a** (2.0 g, 10.64 mmol) and 3-(tetramethyl-1,3,2-dioxaboran-2-yl)-2,5-dihydro-1*H*-pyrrol-1-tert-butyl formate (3.14 g, 10.64 mmol) were dissolved in dioxane (30 mL) and water (5 mL). Sodium carbonate (3.38 g, 31.9 mmol) and Pd (dppf)Cl₂.DCM (779 mg, 1.06 mmol) were added. Under a nitrogen atmosphere, the mixture was stirred at 90°Cto allow for reacting for 12 hours. The reaction mixture was cooled to room temperature, water (40 mL) was added, and the mixture was extracted with ethyl acetate (50 mL x 3). The organic phases were combined, washed with saturated brine (50 mL), dried over anhydrous sodium sulfate, and concentrated under a reduced pressure. The resulting residues were purified by the silica-gel column chromatography using petroleum ether/ethyl acetate as an elution system, to obtain the title Compound **14b** (600 mg, 1.87 mmol, yield: 17.6%).
MS m/z (ESI): 321.1 [M+1]
¹H NMR (400 MHz, CDCl₃) δ 8.17 (s, 1H), 7.43 - 7.28 (m, 1H), 6.94 - 6.74 (m, 1H), 4.65 (br s, 2H), 4.45 (br d, *J=* 12.9 Hz, 2H), 1.54 (br s, 9H).

### Step 2

### 3-(4-{2-[ethyl(isopropyl)aminoformyl]-4-fluorophenyl}-1H-pyrazolo[3,4-b]pyridin-6-yl)-2,5-dihydro-1H-pyrrol-1-tert-butyl formate 14c

Compound **14b** (600 mg, 1.87 mmol) and Compound **4c** (800 mg, 2.39 mmol) were dissolved in dioxane (8.0 mL) and water (1.5 mL). Cesium carbonate (1.83 g, 1.93 mmol) and Pd(dppf)Cl₂.DCM (122 mg, 187.2 µmol) were added, and the mixture was stirred at 90 °C under a nitrogen atmosphere to allow for reacting for 12 hours. The reaction mixture was cooled to room temperature, water (15 mL) was added, and the mixture was extracted with ethyl acetate (20 mL x 3). The organic phases were combined, washed with saturated brine (20 mL), dried over anhydrous sodium sulfate, and concentrated under a reduced pressure. The resulting residues were purified by the silica-gel column chromatography using petroleum ether/ethyl acetate as an elution system, to obtain the title Compound **14c** (170 mg, 344.4 µmol, yield: 18.4%) as brown solid.
MS m/z (ESI): 494.2 [M+1]
¹H NMR (400 MHz, DMSO-*d*₆) δ 13.92 - 13.69 (m, 1H), 8.04 - 7.91 (m, 1H), 7.73 (dd, *J=* 5.5, 8.5 Hz, 1H), 7.55 - 7.35 (m, 3H), 6.69 (br d, *J =* 12.4 Hz, 1H), 4.61 - 4.46 (m, 2H), 4.29 (br d, *J* = 3.4 Hz, 2H), 3.41 - 3.32 (m, 2H), 2.79 (br dd, *J=* 6.7, 13.6 Hz, 1H), 1.46 *(d, J=* 7.3 Hz, 9H), 0.93 - 0.54 (m, 7H), 0.16 (br t, *J=* 6.0 Hz, 2H)

### Step 3

### 3-(4-{2-[ethyl(isopropyl)aminoformyl]-4-fluorophenyl}-1H-pyrazolo[3,4-b]pyridin-6-yl)pyrrolidin-1-tert-butyl formate 14d

Compound 14c (100 mg, 202.6 µmol) was dissolved in methanol (5.0 mL), followed by the addition of Pd/C (21.6 mg, 202.6 µmol). Under a hydrogen atmosphere (15 Psi), the mixture was stirred at room temperature for 12 hours. The reaction mixture was filtered, and the filtrate was concentrated under a reduced pressure, to obtain the crude product of title Compound **14d** (100 mg, 201.8 µmol, yield: 99.6%). The crude product was directly used in the next step of reaction without purification.
MS m/z (ESI): 496.1 [M+1]

### Step 4

### N-ethyl-5-fluoro-N-isopropyl-2-[6-(pyrrolidin-3-yl)-1H-pyrazolo[3,4-b]pyridin-4-yl]benzamide 14e

Compound **14d** (100 mg, 201.8 µmol) was dissolved in methane dioxide (2.0 mL), followed by the addition of trifluoroacetic acid (0.5 mL) at 0°C. The mixture was stirred at room temperature to allow for reacting for 1.0 hours. The reaction mixture was concentrated under a reduced pressure, to obtain the crude product of title Compound **14e** (100 mg). The crude product was directly used in the next step of reaction without purification.
MS m/z (ESI): 396.3 [M+1]

### Step 5

### N-[(1r,4r)-4-{[3-(4-{2-[ethyl(isopropyl)aminoformyl]-4-fluorophenyl}-1H-pyrazolo[3,4-b]pyridin-6-yl)pyrrolidin-1-yl]methyl}cyclohexyl]tert-butyl carbamate 14f

The crude product of Compound **14e** (100 mg), N-[(1r,4r)-4-formylcyclohexyl]tert-butyl carbamate (49.07 mg, 215.90 µmol) and triethylamine (59.58 mg, 588.82 µmol) were dissolved in methanol (2.0 mL), and stirred at room temperature for 1.0 hour. Sodium cyanoborohydride (37.00 mg, 588.82 µmol) was added, and the mixture was stirred at room temperature for 12 hours. The reaction mixture was diluted with a saturated aqueous sodium bicarbonate solution (10 mL), and extracted with dichloromethane (20 mL × 3). The organic phases were combined, washed with saturated brine (20 mL), dried over anhydrous sodium sulfate, and concentrated under a reduced pressure. The resulting residues were purified by the HPLC (mobile phase: acetonitrile, water (0.1% NH₄HCO₃), elution gradient: 40%-70%), to obtain the title Compound **14f** (70 mg, 115.36 µmol, yield: 58.8%) as white solid.
MS m/z (ESI): 607.5 [M+1]
¹H NMR (400 MHz, DMSO-*d*₆) δ 13.68 - 13.51 (m, 1H), 7.93 (s, 1H), 7.74 - 7.63 (m, 1H), 7.51 - 7.31 (m, 2H), 7.08 (s, 1H), 6.67 (br d, *J=* 8.0 Hz, 1H), 3.60 - 3.46 (m, 1H), 3.22 - 3.06 (m, 1H), 2.98 (br *t, J* = 8.1 Hz, 1H), 2.89 - 2.72 (m, 2H), 2.56 (br d, *J =* 8.3 Hz, 1H), 2.32 - 2.01 (m, 4H), 1.89 - 1.67 (m, 4H), 1.36 (s, 9H), 1.17 - 1.03 (m, 2H), 0.94 - 0.84 (m, 4H), 0.79 - 0.61 (m, 4H), 0.19 (br dd, *J =* 4.4, 6.0 Hz, 2H)

### Step 6

### N-ethyl-5-fluoro-N-isopropyl-2-[6-(1-{[(1r,4r)-4-aminocyclohexyl]methyl}pyrrolidin-3-yl)-1H-pyrazolo[3,4-b]pyridin-4-yl]benzamide 14g

Compound **14f** (60 mg, 98.88 µmol) was dissolved in methane dioxide (3.0 mL), followed by the addition of trifluoroacetic acid (1.5 mL) at 0°C. The mixture was stirred at room temperature to allow for reacting for 1.0 hours. The reaction mixture was concentrated under a reduced pressure, to obtain the crude product of title Compound **14g** (65 mg). The crude product was directly used in the next step of reaction without purification.
MS m/z (ESI): 507.4 [M+1]

### Step 7

### N-ethyl-5-fluoro-N-isopropyl-2-[6-(1-{[(1r,4r)-4-ethanesulfonamidocyclohexyl]methyl}pyrrolidin-3-yl)-1H-pyrazolo[3,4-b]pyridin-4-yl]benzamide 14

The crude product of Compound **14g** (62 mg) was dissolved in dichloromethane (3.0 mL), followed by the addition of ethanesulfonyl chloride (26 mg, 202.2 µmol) and triethylamine (51 mg, 504 µmol), and the mixture was stirred at room temperature for 12 hours. The reaction mixture was concentrated under a reduced pressure, and purified by the HPLC (mobile phase: acetonitrile, water (0.1% NH₄HCO₃), elution gradient: 34%-64%), to obtain the title Compound **14** (4.86 mg, 8.12 µmol, yield: 8.13 %) as white solid.
MS m/z (ESI): 599.4 [M+1]
¹H NMR (400 MHz, DMSO-*d*₆) δ 13.58 (s, 1H), 7.93 (s, 1H), 7.72 - 7.64 (m, 1H), 7.47 - 7.33 (m, 2H), 7.08 (s, 1H), 7.04 - 6.92 (m, 1H), 3.58 - 3.47 (m, 2H), 3.03 - 2.91 (m, 4H), 2.89 - 2.81 (m, 1H), 2.78 - 2.71 (m, 1H), 2.60 - 2.54 (m, 1H), 2.30 - 2.16 (m, 3H), 2.13 - 2.02 (m, 1H), 1.91 - 1.72 (m, 4H), 1.43 - 1.13 (m, 7H), 1.09 - 0.81 (m, 6H), 0.81 - 0.59 (m, 4H), 0.25 - 0.13 (m, 2H).

### Example 15

### N-ethyl-5-fluoro-2-[1-methyl-6-(1-{[(1r,4r)-4-ethanesulfonamidocyclohexyl]methyl}pyrrolidin-3-yl)-1H-indazol-4-yl]-N-isopropylbenzamide 15

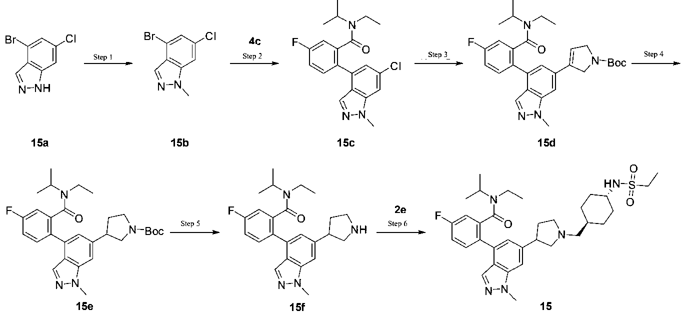

### Step 1

### 4-bromo-6-chloro-1-methyl-1H-indazole 15b

4-Bromo-6-chloro-1*H*-indazole **15a** (2.0 g, 8.64 mmol) and cesium carbonate (5.63 g, 17.2 mmol) were dissolved in N,N-dimethylformamide (20 mL), followed by the addition of iodomethane (1.35 g, 9.50 mmol). The mixture was stirred at room temperature to allow for reacting for 1.0 hour. The reaction mixture was concentrated under a reduced pressure, followed by adding water (50 mL) and extracting with ethyl acetate (50 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **15b** (1.30 g, 5.30 mmol, yield: 61.2%) as white solid.
MS m/z (ESI): 244.95 [M+1]
¹H NMR (400 MHz, DMSO-*d*₆) δ 8.04 (d, *J =* 0.8 Hz, 1H), 7.95 - 7.89 (m, 1H), 7.44 (d, *J =* 1.6 Hz, 1H), 4.06 (s, 3H).

### Step 2

### 2-(6-chloro-1-methyl-1H-indazol-4-yl)-N-ethyl-5-fluoro-N-isopropylbenzamide 15c

Compound **15b** (500 mg, 2.04 mmol) and Compound **4c** (1.50 g, 2.24 mmol) were dissolved in dioxane (20 mL) and water (4.0 mL). Sodium carbonate (431 mg, 4.07 mmol) and Pd(dppf)Cl₂ (149 mg, 203 µmol) were added, and the mixture was stirred at 70 °C under a nitrogen atmosphere to allow for reacting for 12 hours. The reaction mixture was cooled to room temperature and filtered, and the filtrate was concentrated under a reduced pressure. The resulting residues were purified by the silica-gel column chromatography using petroleum ether/ethyl acetate as an elution system, to obtain the title Compound **15c** (519 mg, 1.39 mmol, yield: 68.1%) as white solid.
MS m/z (ESI): 374.1 [M+1]
¹H NMR (400 MHz, DMSO-*d*₆) δ 7.95 - 7.87 (m, 2H), 7.66 (dd, *J=* 8.4, 5.6 Hz, 1H), 7.45 - 7.32 (m, 2H), 7.07 (d, *J= 1.6* Hz, 1H), 4.18 (dd, *J=* 13.6, 6.8 Hz, 0.18H), 4.06 (d, *J=* 1.6 Hz, 3H), 3.37 (s, 2H), 2.81 (dq, *J=* 13.6, 6.8 Hz, 0.89H), 1.07 (s, 7H), 0.17 (d, *J =* 6.8 Hz, 2H).

### Step 3

### 3-(4-{2-[ethyl(isopropyl)aminoformyl]-4-fluorophenyl}-1-methyl-1H-indazol-6-yl)-2,5-dihydro-1H-pyrrol-1-tert-butyl formate 15d

Compound **15c** (886 mg, 1.42 mmol, 60% purity) and 3-(tetramethyl-1,3,2-dioxaboran-2-yl)-2,5-dihydro-1*H*-pyrrol-1-tert-butyl formate (419 mg, 1.42 mmol) were dissolved in dioxane (20 mL) and water (4 mL). Sodium carbonate (301 mg, 2.84 mmol) and Pd (dppf)Cl₂ (104 mg, 142 µmol) were added. Under a nitrogen atmosphere, the mixture was stirred at 100°Cto allow for reacting for 12 hours. The reaction mixture was cooled to room temperature, and filtered through diatomite, and the filtrate was concentrated under a reduced pressure. Water (50 mL) was added to the residues, and the mixture was extracted with ethyl acetate (50 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **15d** (260 mg, 513 µmol, yield: 36.0%) as white solid.
MS m/z (ESI): 507.2 [M+1]

### Step 4

### 3-(4-{2-[ethyl(isopropyl)aminoformyl]-4-fluorophenyl}-1-methyl-1H-indazol-6-yl)pyrrolidin-1-tert-butyl formate 15e

Compound **15d** (260 mg, 513 µmol) was dissolved in methanol (10 mL), followed by the addition of Pd/C (26 mg). Under a hydrogen atmosphere (15 Psi), the mixture was stirred at room temperature for 12 hours. The reaction mixture was filtered, and the filtrate was concentrated under a reduced pressure. The residues were purified by the silica-gel column chromatography using petroleum ether/ethyl acetate as an elution system, to obtain the title Compound **15e** (235 mg, 462 µmol, yield: 90%) as white solid.
MS m/z (ESI): 509.2 [M+1]

### Step 5

### N-ethyl-5-fluoro-2-[1-methyl-6-(pyrrolidin-3-yl)-1H-indazol-4-yl]-N-(isopropyl)benzamide 15f

Compound **15e** (30 mg, 58.9 µmol) was dissolved in methane dioxide (0.9 mL), followed by the addition of trifluoroacetic acid (0.3 mL) at 0°C. The mixture was stirred at room temperature to allow for reacting for 1.0 hours. The reaction mixture was concentrated under a reduced pressure, to obtain the crude product of title Compound **15f** (24 mg). The crude product was directly used in the next step of reaction without purification.
MS m/z (ESI): 409.3 [M+1]

### Step 6

### N-ethyl-5-fluoro-2-[1-methyl-6-(1-{[(1r,4r)-4-ethanesulfonamidocyclohexyl]methyl}pyrrolidin-3-yl)-1H-indazol-4-yl]-N-isopropylbenzamide 15

The crude product of Compound **15f** (24 mg) was dissolved in *N,N*-dimethylformamide (1.0 mL), followed by the addition of potassium carbonate (24.3 mg, 176 µmol), and the mixture was stirred at room temperature for 0.5 hours. Potassium iodide (9.75 mg, 58.7 µmol) and Compound **2e** (55.5 mg, 117µmol) were added, and the mixture was stirred at 80°C to allow for reacting for 12 hours. The reaction mixture was filtered, and the filtrate was concentrated under a reduced pressure. The resulting residues were purified by the preparative thin-layer chromatography using dichloromethane/methanol as an elution system, to obtain the title Compound **15** (7.0 mg, 11.4 µmol, yield: 19.4 %) as white solid.
MS m/z (ESI): 612.2 [M+1]
¹H NMR (400 MHz, DMSO-*d*₆) δ 7.79 (s, 1H), 7.57 (dt, *J=* 10.0, 5.2 Hz, 1H), 7.52 (s, 1H), 7.38 (t, *J=* 8.8 Hz, 1H), 7.30 (s, 1H), 7.04 (d, *J* = 2.0 Hz, 1H), 7.01 (d, *J=* 7.6 Hz, 1H), 4.25 - 4.16 (m, 0.3H), 4.02 (d, *J=* 3.2 Hz, 3H), 3.39 (d, *J* = 6.8 Hz, 2H), 3.25 (d, *J =* 6.8 Hz, 1H), 2.96 (dd, *J =* 7.2, 3.2 Hz, 2H), 2.86 (ddd, *J =* 20.4, 13.6, 7.8 Hz, 3H), 2.71 - 2.59 (m, 2H), 2.45 (d, *J =* 8.8 Hz, 1H), 2.24 *(d, J=* 6.8 Hz, 3H), 1.94 - 1.75 (m, 5H), 1.10 (ddd, *J* = 128, 66.4, 4.4 Hz, 12H), 0.67 (t, *J=* 6.8 Hz, 3H), 0.13 - 0.06 (m, 2H).

### Example 16

### N-ethyl-5-fluoro-2-[6-(4-{[(2-oxo-2,3-dihydro-l,3-benzoxazol-5-yl)methyl]amino}cyclohexyl-1-ene-1-yl)imidazo[1,5-a]pyridin-8-yl]-N-isopropylbenzamide 16

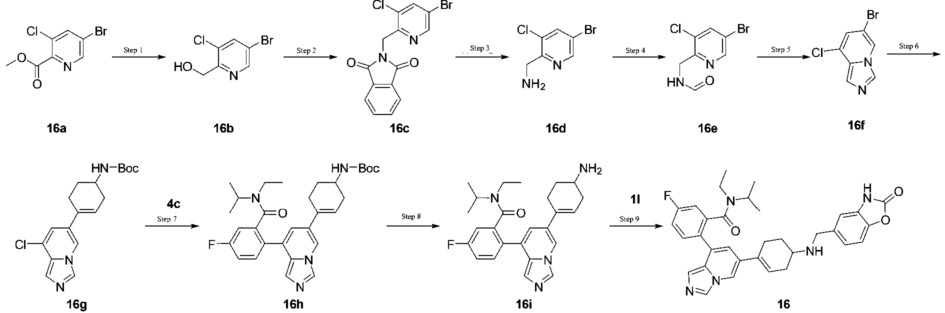

### Step 1

### (5-bromo-3-chloropyridin-2-yl)methanol 16b

5-Bromo-3-chloropyridin-2-methyl carboxylate **16a**(25 g, 99.8 mmol) was dissolved in methanol (250 mL) and tetrahydrofuran (250 mL), cooled to 0°C, followed by the addition of sodium borohydride (20 g, 528.7 mmol). The mixture was stirred at room temperature to allow for reacting for 8.0 hour. The reaction mixture was cooled to 0°C, quenched by adding water (40 mL), and extracted with ethyl acetate (50 mL x 3). The organic phases were combined, dried over anhydrous sodium sulfate, and concentrated under a reduced pressure, to obtain the crude product of title Compound **16b** (18 g, 80.9 mmol, yield: 81.1%) as white solid. The crude product was directly used in the next step of reaction without purification.
MS m/z (ESI): 223.8 [M+1]

### Step 2

### 2-[(5-bromo-3-chloropyridin-2-yl)methyl]-2,3-dihydro-1H-isoindol-1,3-dione 16c

Compound **16b** (13 g, 58.4 mmol), 2,3-dihydro-1H-isoindol-1,3-dione (9.5 g, 64.6 mmol) and triphenylphosphine (23 g, 87.69 mmol) were dissolved in tetrahydrofuran (100 mL), and cooled to 0°C under a nitrogen atmosphere, followed by the addition of DIAD (17.7 g, 87.53 mmol). The mixture was stirred at room temperature to allow for reacting for 12 hours, with white solid precipitated. The reaction mixture was filtered under a reduced pressure, and the filter cake was washed with ethyl acetate and then dried under a reduced pressure, to obtain the crude product of title Compound **16c** (14 g, 39.8 mmol, yield: 68.1%) as yellow solid. The crude product was directly used in the next step of reaction without purification.
MS m/z (ESI): 352.8 [M+1]

### Step 3

### (5-bromo-3-chloropyridin-2-yl)methylamine 16d

Compound **16c** (19.3 g, 54.9 mmol) was dissolved in the solution of methylamine in ethanol (170.49 g, 1.65 mol, 189.64 mL, 30% purity). The mixture was stirred at 65°C to allow for reacting for 12 hours. The reaction mixture was cooled to room temperature and concentrated under a reduced pressure, followed by adding water (50 mL) and extracting with ethyl acetate (50 mL x 3). The organic phases were combined, dried over anhydrous sodium sulfate, and concentrated under a reduced pressure. The crude product of title Compound **16d** (30 g, 135 mmol, yield: 247%) was obtained as blue solid. The crude product was directly used in the next step of reaction without purification.
MS m/z (ESI): 222.9 [M+1]

### Step 4

### N-[(5-bromo-3-chloropyridin-2-yl)methyl]formamide 16e

Compound **16d** (6.0 g, 27.1 mmol) was dissolved in ethyl formate (60 mL), followed by the addition of triethylamine (4.36 g, 43.1 mmol). The mixture was stirred at 70°C to allow for reacting for 12 hours. The reaction mixture was cooled under reduced pressure to room temperature, and concentrated under a reduced pressure, to obtain the crude product of title Compound **16e** (6.0 g, 24.05 mmol, yield: 88.8%). The crude product was directly used in the next step of reaction without purification.
MS m/z (ESI): 251.0 [M+1]

### Step 5

### 6-bromo-8-chloroimidazo[1,5-a]pyridine 16f

Compound **16e(24.0** g, 96.2 mmol) was dissolved in toluene (200 mL), followed by the addition of phosphorus oxychloride (73.75 g, 480.98 mmol). The mixture was stirred at 100°C to allow for reacting for 2.0 hours. The reaction mixture was cooled to room temperature, and concentrated under a reduced pressure. The residues were dissolved in dichloromethane (40 mL), followed by the addition of an aqueous sodium hydroxide solution (3 M, 60 mL). The mixture was extracted with dichloromethane (100 mL x 3), and the organic phases were combined, dried over anhydrous sodium sulfate, concentrated under a reduced pressure . The resulting residues were purified by the silica-gel column chromatography using petroleum ether/ethyl acetate as an elution system, to obtain the title Compound **16f** (4.0 g, 17.28 mmol, yield: 17.96%) as yellow oil.
MS m/z (ESI): 233.0 [M+1]

### Step 6

### N-(4-{8-chloroimidazo[1,5-a]pyridin-6-yl}cyclohexyl-3-ene-1-yl)tert-butyl carbamate 16g

Compound **16f** (760 mg, 3.28 mmol), *N*-[4-(tetramethyl-1,3,2-dioxaboran-2-yl)cyclohexyl-3-ene-1-yl]tert-butyl carbamate (950 mg, 2.94 mmol) and sodium carbonate (1.04 g, 9.85 mmol) were dissolved in dioxane (15 mL) and water (1.5 mL). Under a nitrogen atmosphere, Pd(dppf)Cl₂.DCM (270 mg, 330 µmol) was added, and the mixture was stirred at 90°C to allow for reacting for 8.0 hours. The reaction mixture was cooled to room temperature, followed by adding water (30 mL) and extracting with ethyl acetate (30 mL x 3). The organic phases were combined, dried over anhydrous sodium sulfate and filtered, and the filtrate was concentrated under a reduced pressure. The resulting residues were purified by the silica-gel column chromatography using petroleum ether/ethyl acetate as an elution system, to obtain the title Compound **16g** (750 mg, 2.16 mmol, yield: 65.67%).
MS m/z (ESI): 348.0 [M+1]

### Step 7

### N-[4-(8-{2-[ethyl(isopropyl)aminoformyl]-4-fluorophenyl}imidazo[1,5-a]pyridin-6-yl}cyclohexyl-3-ene-1-yl)tert-butyl carbamate 16h

Compound **16g** (750 mg, 2.16 mmol), Compound **4c** (1.08 g, 3.23 mmol) and cesium carbonate (2.11 g, 6.47 mmol) were dissolved in dioxane (10 mL) and water (1.0 mL). Under a nitrogen atmosphere, Pd(dppf)Cl₂ (140.5 mg, 215.6 µmol) was added, and the mixture was stirred at 100°C to allow for reacting for 8.0 hours. The reaction mixture was cooled to room temperature, followed by adding water (30 mL) and extracting with ethyl acetate (40 mL x 3). The organic phases were combined, dried over anhydrous sodium sulfate and filtered, and the filtrate was concentrated under a reduced pressure. The resulting residues were purified by the silica-gel column chromatography using petroleum ether/ethyl acetate as an elution system, to obtain the title Compound **16h** (730 mg, 1.40 mmol, yield: 65.0%).
MS m/z (ESI): 521.2 [M+1]

### Step 8

### 2-[6-(4-aminocyclohex-1-ene-1-yl)imidazo[1,5-a]pyridin-8-yl]-N-ethyl-5-fluoro-N-isopropylbenzamide 16i

Compound **16h** (90 mg, 172.9 µmol) was dissolved in methane dioxide (2.0 mL), followed by the addition of trifluoroacetic acid (197.10 mg, 1.73 mmol, 133.18 µL). The mixture was stirred at room temperature to allow for reacting for 1.0 hours. The reaction mixture was concentrated under a reduced pressure, to obtain the crude product of title Compound **16i** (70 mg, 166.46 µmol, 96.29% yield). The crude product was directly used in the next step of reaction without purification.
MS m/z (ESI): 421.2 [M+1]

### Step 9

### N-ethyl-5-fluoro-2-[6-(4-{[(2-oxo-2,3-dihydro-l,3-benzoxazol-5-yl)methyl]amino}cyclohexyl-1-ene-1-yl)imidazo[1,5-a]pyridin-8-yl]-N-isopropylbenzamide 16

Compound **16i** (50 mg, 93.54 µmol, trifluoroacetate) was dissolved in dichloromethane (2.0 mL), followed by the addition of triethylamine (94.65 mg, 935.38 µmol) and Compound **11** (30.52 mg, 187.08 µmol), and the mixture was stirred at room temperature for 1.0 hour. Sodium cyanoborohydride (25 mg, 397.82 µmol) was added, and the mixture was stirred at room temperature to allow for reacting for 12 hours. An aqueous sodium bicarbonate solution (20 mL) was added to the reaction mixture, which was then extracted with dichloromethane (20 mL x 3). The organic phases were combined, dried over anhydrous sodium sulfate and filtered, and the filtrate was concentrated under a reduced pressure. The resulting residues were purified by the HPLC (mobile phase: acetonitrile, water (0.1% formic acid), elution gradient: 8%-38%), to obtain the title Compound **16** (22.2 mg, 39.1 µmol, yield: 41.8%)
MS m/z (ESI): 568.1 [M+1]
¹H NMR (400 MHz, DMSO-*d*₆) δ 8.59 - 8.24 (m, 3H), 7.73 (dd, *J* = 5.2, 8.4 Hz, 1H), 7.38 - 7.31 (m, 1H), 7.29 - 7.22 (m, 4H), 7.03 (s, 1H), 6.20 (s, 1H), 4.28 - 4.14 (m, 2H), 3.61 - 3.33 (m, 2H), 3.04 - 2.90 (m, 1H), 2.84 - 2.23 (m, 6H), 1.93 - 1.70 (m, 1H), 1.36 - 0.73 (m, 7H), 0.29 (d, *J* = 6.4 Hz, 2H).

### Examples 18-1 and 18-2

### N-ethyl-5-fluoro-N-(isopropyl)-2-{6-[(1s,4s)-4-({[4-(5,6-dimethoxypyridazin-3-yl)phenyl]methyl}amino)cyclohexyl]imidazo[1,5-a]pyridin-8-yl}benzamide 18-1 N-ethyl-5-fluoro-N-(isopropyl)-2-{6-[(1r,4r)-4-({[4-(5,6-dimethoxypyridazin-3-yl)phenyl]methyl}amino)cyclohexyl]imidazo[1,5-a]pyridin-8-yl}benzamide 18-2

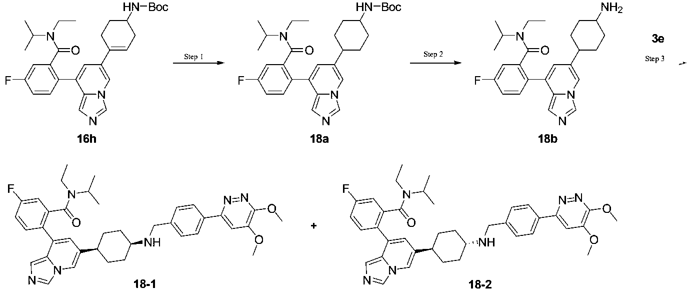

### Step 1

### N-[4-(8-{2-[ethyl(isopropyl)aminoformyl]-4-fluorophenyl}imidazo[1,5-a]pyridin-6-yl)cyclohexyl]tert-butyl carbamate 18a

Compound **16h** (300 mg, 576.2 µmol) was dissolved in methanol (25 mL), followed by the addition of Pd/C (250 mg, 2.06 mmol). Under a hydrogen atmosphere (15 Psi), the mixture was stirred at room temperature for 12 hours. The reaction mixture was filtered, and the filtrate was concentrated under a reduced pressure. The residues were dissolved in methanol (25 mL), followed by the addition of Pd/C (250 mg, 2.06 mmol). Under a hydrogen atmosphere (15 Psi), the mixture was further stirred at room temperature for 12 hours. The reaction mixture was filtered, and the filtrate was concentrated under a reduced pressure. The resulting residues were purified by the HPLC (mobile phase: acetonitrile, water (0.1% ammonia water), elution gradient:40%-70%), to obtain the title Compound **18a** (170 mg, 325.3 µmol, yield: 56.5%) as yellow solid.
MS m/z (ESI): 523.2 [M+1]

### Step 2

### 2-[6-(4-aminocyclohexyl)imidazo[1,5-a]pyridin-8-yl]-N-ethyl-5-fluoro-N-isopropylbenzamide 18b

Compound **18a(170** mg, 325.3 µmol) was dissolved in methane dioxide (10 mL), followed by the addition of trifluoroacetic acid (370.88 mg, 3.25 mmol, 250.99 µL). The mixture was stirred at room temperature to allow for reacting for 1.0 hours. The reaction mixture was concentrated under a reduced pressure, to obtain the crude product of title Compound **18b** (130 mg, 307.67 µmol, 94.59% yield). The crude product was directly used in the next step of reaction without purification.
MS m/z (ESI): 423.2 [M+1]

### Step 3

### N-ethyl-5-fluoro-N-(isopropyl)-2-[6-[(1s,4s)-4-({[4-(5,6-dimethoxypyridazin-3-yl)phenyl]methyl}amino)cyclohexyl]imidazo[1,5-a]pyridin-8-yl}benzamide 18-1 N-ethyl-5-fluoro-N-(isopropyl)-2-{6-[(1r,4r)-4-({[4-(5,6-dimethoxypyridazin-3-yl)phenyl]methyl)amino)cyclohexyl]imidazo[1,5-a]pyridin-8-yl}benzamide 18-2

Compound **18b** (200 mg, 372.74 µmol, trifluoroacetate) was dissolved in dichloromethane (3.0 mL), followed by the addition of triethylamine (113.15 mg, 1.12 mmol) and Compound 3e(72.83 mg, 298.20 µmol), and the mixture was stirred at room temperature for 0.5 hours. Sodium cyanoborohydride (70.27 mg, 1.12 mmol) was added, and the mixture was stirred at 45°C to allow for reacting for 12 hours. An aqueous sodium bicarbonate solution (20 mL) was added to the reaction mixture, which was then extracted with dichloromethane (20 mL x 3). The organic phases were combined, dried over anhydrous sodium sulfate and filtered, and the filtrate was concentrated under a reduced pressure. The resulting residues were purified by the HPLC (mobile phase: acetonitrile, water (0.1% ammonia water), elution gradient:43%-73%), to obtain the title Compounds **18-1** (12.46 mg, 19.15 µmol, yield: 5.14%) and **18-2** (19.67 mg, 30.23 µmol, yield: 8.11%)

### Single-configuration Compound 18-1:

MS m/z (ESI):651.1 [M+1]
HPLC analysis: retention time: 1.307 min, 10-80AB _4min.1cm
SFC analysis: retention time: 1.604 min; chromatographic column: Cellulose-2 50×4.6 mm I.D., 3 um; mobile phase: CO2/[MeOH+ACN (0.05% DEA)] = 40/60.

1H NMR (400 MHz, DMSO-*d*₆) δ 8.32 (s, 1H), 8.17 (s, 1H), 8.03 (d, J=8.0 Hz, 2H), 7.67 (dd, J=6.0, 8.4 Hz, 1H), 7.59 (s, 1H), 7.49 (d, J=7.6 Hz, 2H), 7.40 - 7.27 (m, 2H), 7.10 (s, 1H), 6.63 - 6.58 (m, 1H), 4.05 (s, 3H), 3.99 (s, 3H), 3.83 (s, 2H), 3.47 - 3.38 (m, 1H), 3.30 - 3.22 (m, 1H), 3.00 - 2.83 (m, 1H), 2.47 - 2.33 (m, 2H), 2.17 - 1.96 (m, 3H), 1.83 (d, J=12.0 Hz, 2H), 1.47 - 1.32 (m, 2H), 1.26 - 1.13 (m, 2H), 1.08 - 0.84 (m, 4H), 0.80 - 0.69 (m, 3H), 0.24 (d, J=6.4 Hz, 2H).

### Single-configuration Compound 18-2:

MS m/z (ESI):651.2 [M+1]
HPLC analysis: retention time: 1.379 min, 10-80AB_4min.lcm
SFC analysis: retention time: 0.911 min; chromatographic column: Cellulose-2 50×4.6 mm I.D., 3 um; mobile phase: CO2/[MeOH+ACN (0.05% DEA)] = 40/60.

¹H NMR (400 MHz, DMSO-*d*₆) δ8.36 (s, 1H), 8.20 (s, 1H), 8.03 (d, J=8.0 Hz, 2H), 7.68 (dd, J=5.6, 8.4 Hz, 1H), 7.57 (s, 1H), 7.51 (d, J=8.0 Hz, 2H), 7.41 - 7.27 (m, 2H), 7.10 (s, 1H), 6.69 - 6.63 (m, 1H), 4.05 (s, 3H), 3.98 (s, 3H), 3.77 (s, 2H), 3.43 (td, J=6.4, 13.2 Hz, 1H), 3.30 - 3.25 (m, 1H), 2.96 - 2.76 (m, 2H), 2.47 - 2.37 (m, 1H), 2.21 - 2.07 (m, 1H), 1.95 - 1.77 (m, 4H), 1.54 (d, J=11.6 Hz, 4H), 0.94 - 0.68 (m, 7H), 0.26 (d, J=6.4 Hz, 2H).

### Example 19

### 2-(6-[4-({[4-(5,6-dimethoxypyridazin-3-yl)phenyl]methyl}amino)piperidin-1-yl]imidazo[1,5-a]pyridin-8-yl)-N-ethyl-5-fluoro-N-isopropylbenzamide 19

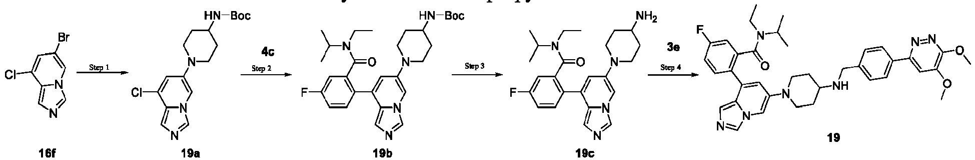

### Step 1

### N-(1-{8-chloroimidazo[1,5-a]pyridin-6-yl}piperidin-4-yl)tert-butyl carbamate 19a

Compound **16f** (1.0 g, 4.32 mmol), N-(piperidin-4-yl)tert-butyl carbamate (870 mg, 4.34 mmol) and cesium carbonate (4.22 g, 12.96 mmol) were dissolved in dioxane (15 mL). Ruphos (202 mg, 432.9 µmol) and Pd₂ (dba)₃ (400 mg, 436.38 µmol) were added. Under a nitrogen atmosphere, the mixture was stirred at 90°Cto allow for reacting for 12 hours. The reaction mixture was cooled to room temperature, water (20 mL) was added, and the mixture was extracted with ethyl acetate (35 mL x 3). The organic phases were combined, washed with saturated brine, dried over anhydrous sodium sulfate, and concentrated under a reduced pressure. The resulting residues were purified by the silica-gel column chromatography using petroleum ether/ethyl acetate as an elution system, to obtain the title Compound **19a** (315 mg, 897.8 µmol, yield: 20.78%).
MS m/z (ESI): 351.1 [M+1]

### Step 2

### N-[1-(8-{2-[ethyl(isopropyl)aminoformyl]-4-fluorophenyl}imidazo[1,5-a]pyridin-6-yl)piperidin-4-yl]tert-butyl carbamate 19b

Compound **19a** (315 mg, 897.8 µmol) and Compound **4c** (362 mg, 1.08 mmol) were dissolved in dioxane (4 mL) and water (0.8 mL). Potassium phosphate (572 mg, 2.69 mmol) and Pd(dtbpf)Cl₂ (59 mg, 90.5 µmol) were added, and the mixture was stirred at 80 °C under a nitrogen atmosphere to allow for reacting for 12 hours. The reaction mixture was cooled to room temperature, water (15 mL) was added, and the mixture was extracted with ethyl acetate (25 mL x 3). The organic phases were combined, washed with saturated brine, dried over anhydrous sodium sulfate, and concentrated under a reduced pressure. The resulting residues were purified by the silica-gel column chromatography using petroleum ether/ethyl acetate as an elution system, to obtain the title Compound **19b** (300 mg, 572.9 µmol, yield: 63.8%).
MS m/z (ESI): 524.3 [M+1]

### Step 3

### 2-[6-(4-aminopiperidin-1-yl)imidazo[1,5-a]pyridin-8-yl]-N-ethyl-5-fluoro-N-isopropylbenzamide 19c

Compound **19b** (300 mg, 572.9 µmol) was dissolved in methane dioxide (5 mL), followed by the addition of trifluoroacetic acid (2.96 g, 25.96 mmol, 2 mL) at 0°C. The mixture was stirred at room temperature to allow for reacting for 1.0 hours. The reaction mixture was concentrated under a reduced pressure, to obtain the crude product of title Compound 19c (300 mg), which was directly used in the next step of reaction without purification.
MS m/z (ESI): 424.2 [M+1]

### Step 4

### 2-{6-[4-({[4-(5,6-dimethoxypyridazin-3-yl)phenyl]methyl}amino)piperidin-1-yl]imidazo[1,5-a]pyridin-8-yl}-N-ethyl-5-fluoro-N-isopropylbenzamide 19

The crude product of Compound **19c** (150 mg, 279 µmol, trifluoroacetate), Compound **3e**(69 mg, 282.5 µmol) and triethylamine (57 mg, 563 µmol) were dissolved in methanol (2.0 mL), and stirred at room temperature for 1.0 hour. Sodium cyanoborohydride (53 mg, 843.4 µmol) was added, and the mixture was stirred at room temperature for 11 hours. A saturated aqueous sodium bicarbonate solution (10 mL) was added, and the mixture was extracted with dichloromethane (20 mL x 3). The organic phases were combined, washed with saturated brine, dried over anhydrous sodium sulfate and filtered, and the filtrate was concentrated under a reduced pressure. The resulting residues were purified by the HPLC (mobile phase: acetonitrile, water (0.1% ammonium bicarbonate), elution gradient: 30%-60%), to obtain the title Compound **19** (13.12 mg, 20.13 µmol, yield: 7.21%) as white solid.
MS m/z (ESI): 652.5 [M+1]
¹H NMR (400 MHz, DMSO-*d*₆) δ 8.25 (s, 1H), 8.10 - 7.97 (m, 2H), 7.78 (s, 1H), 7.72 - 7.64 (m, 1H), 7.59 (s, 1H), 7.54 - 7.45 (m, 2H), 7.42 - 7.27 (m, 2H), 7.08 - 7.01 (m, 1H), 6.69 - 6.59 (m, 1H), 4.05 (s, 3H), 3.99 (s, 3H), 3.85 (s, 2H), 3.50 - 3.39 (m, 3H), 2.99 - 2.86 (m, 1H), 2.84 - 2.54 (m, 4H), 2.02 - 1.88 (m, 2H), 1.52 - 1.37 (m, 2H), 0.96 - 0.70 (m, 7H), 0.34 - 0.28 (m, 2H).

### Example 21

### N-[(1r,4r)-4-{[3-(8-{2-[ethyl(isopropyl)aminoformyl]-4-fluorophenyl}imidazo[1,5-a]pyridin-6-yl)pyrrolidin-1-yl]methyl}cyclohexyl]tert-butyl carbamate 21

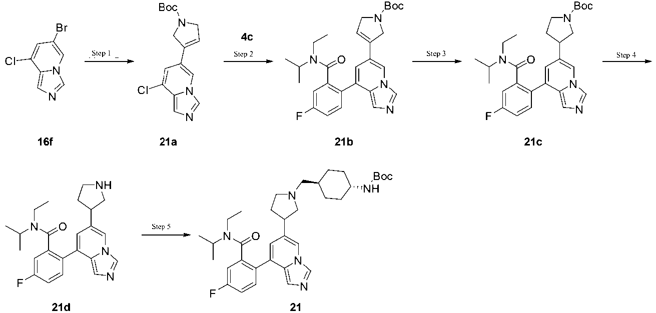

### Step 1

### 3-{8-chloroimidazo[1,5-a]pyridin-6-yl}-2,5-dihydro-1H-pyrrol-1-tert-butyl formate 21a

Compound **16f** (2.0 g, 6.91 mmol), 3-(tetramethyl-1, 3,2-dioxaboran-2-yl)-2,5-dihydro-1*H*-pyrrol-1-tert-butyl formate (1.8 g, 6.10 mmol) and sodium carbonate (2.18 g, 20.6 mmol) were dissolved in dioxane (30 mL) and water (3 mL). Pd (dppf)Cl₂.DCM (282.24 mg, 345.6 µmol) was added. Under a nitrogen atmosphere, the mixture was stirred at 95°Cto allow for reacting for 12 hours. The reaction mixture was cooled to room temperature, followed by adding water (20 mL) and extracting with ethyl acetate (20 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **21a** (1.2 g, 3.75 mmol, yield: 54.29%).
MS m/z (ESI): 320.1 [M+1]

### Step 2

### 3-(8-{2-[ethyl(isopropyl)aminoformyl]-4-fluorophenyl}imidazo[1,5-a]pyridin-6-yl)-2,5-dihydro-1H-pyrrol-1-tert-butyl formate 21b

Compound **21a** (1.2 g, 3.75 mmol) and Compound **4c** (2.62 g, 7.81 mmol) were dissolved in dioxane (24 mL) and water (2.5 mL). Cesium carbonate (3.67 g, 11.26 mmol) and Pd(dtbpf)Cl₂ (244.57 mg, 375.2 µmol) were added, and the mixture was stirred at 100 °C under a nitrogen atmosphere to allow for reacting for 8.0 hours. The reaction mixture was cooled to room temperature, followed by adding water (20 mL) and extracting with ethyl acetate (20 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **21b** (1.43 g, 2.9 mmol, yield: 77.4%).
MS m/z (ESI): 493.2 [M+1]

### Step 3

### 3-(8-{2-[ethyl(isopropyl)aminoformyl]-4-fluorophenyl}imidazo[1,5-a]pyridin-6-yl)pyrrolidin-1-tert-butyl formate 21c

Compound **21b** (430 mg, 873 µmol) was dissolved in methanol (20 mL), followed by the addition of Pd/C (103.4 mg, 873 µmol). Under a hydrogen atmosphere (15 Psi), the mixture was stirred at room temperature for 12 hours. The reaction mixture was filtered, and the filtrate was concentrated under a reduced pressure, to obtain the crude product of title Compound **21c** (400 mg, 808.7 µmol, yield: 92.6%). The crude product was directly used in the next step of reaction without purification.
MS m/z (ESI): 495.3 [M+1]

### Step 4

### N-ethyl-5-fluoro-N-isopropyl-2-[6-(pyrrolidin-3-yl)imidazo[1,5-a]pyridin-8-yl]benzamide 21d

Compound **21c** (400 mg, 808.7 µmol) was dissolved in dichloromethane (10 mL), followed by the addition of trifluoroacetic acid (922.14 mg, 8.09 mmol, 623.07 µL) at 0°C. The mixture was stirred at room temperature to allow for reacting for 1.0 hours. The reaction mixture was concentrated under a reduced pressure, to obtain the crude product of title Compound **21d** (300 mg). The crude product was directly used in the next step of reaction without purification.
MS m/z (ESI): 395.3 [M+1]

### Step 5

### N-[(1r,4r)-4-{[3-(8-{2-[ethyl(isopropyl)aminoformyl]-4-fluorophenyl}imidazo[1,5-a]pyridin-6-yl)pyrrolidin-1-yl]methyl}cyclohexyl]tert-butyl carbamate 21

The crude product of Compound **21d** (300 mg, 590 µmol, trifluoroacetate), *N*-[(1r,4r)-4-formylcyclohexyl]tert-butyl carbamate (201.15 mg, 884.94 µmol) and triethylamine (298.49 mg, 2.95 mmol) were dissolved in methanol (10 mL), and stirred at room temperature for 0.5 hours.Sodium cyanoborohydride (74.15 mg, 1.18 mmol) was added, and the reaction mixture was stirred at room temperature for 1.0 hour. The reaction mixture was filtered, and the filtrate was concentrated under a reduced pressure. The resulting residues were purified by the HPLC (mobile phase: acetonitrile, water (0.1% ammonia water), elution gradient: 58%-78%), to obtain the title Compound **21** (142 mg, 234.4 µmol, yield: 39.1%) as white solid.
MS m/z (ESI): 606.3 [M+1]
¹H NMR (400 MHz, DMSO-*d*₆) δ 8.36 - 8.32 (m, 1H), 8.23 - 8.19 (m, 1H), 7.69 - 7.62 (m, 1H), 7.40 - 7.27 (m, 2H), 7.10 (s, 1H), 6.71 - 6.63 (m, 2H), 3.49 - 3.38 (m, 1H), 3.29 - 3.06 (m, 3H), 2.99 - 2.77 (m, 2H), 2.68 - 2.54 (m, 2H), 2.47 - 2.32 (m, 1H), 2.29 - 2.10 (m, 3H), 1.87 - 1.65 (m, 5H), 1.39 - 1.34 (m, 9H), 1.33 - 1.25 (m, 1H), 1.18 - 1.00 (m, 3H), 0.94 - 0.81 (m, 5H), 0.79 - 0.71 (m, 3H), 0.28 (dd, *J =* 6.8, 12.4 Hz, 2H).

### Example 22

### N-ethyl-5-fluoro-N-isopropyl-2-[6-(1-{[(1r,4r)-4-ethanesulfonamidocyclohexylmethyl}pyrrolidin-3-yl)imidazo[1,5-a]pyridin-8-yl]benzamide 22

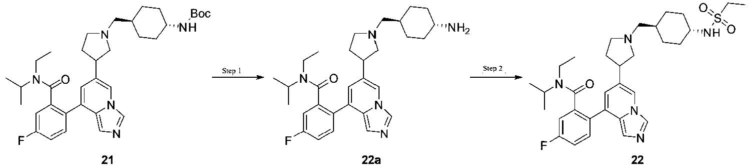

### Step 1

### N-ethyl-5-fluoro-N-isopropyl-2-[6-(1-{[(1r,4r)-4-aminocyclohexyl]methyl}pyrrolidin-3-yl)imidazo[1,5-a]pyridin-8-yl]benzamide 22a

Compound **21** (100 mg, 165.08 µmol) was dissolved in dichloromethane (5.0 mL), followed by the addition of trifluoroacetic acid (740.00 mg, 6.49 mmol, 0.5 mL) at 0°C. The mixture was stirred at room temperature to allow for reacting for 1.0 hours. The reaction mixture was concentrated under a reduced pressure, to obtain the crude product of title Compound **22a** (80 mg). The crude product was directly used in the next step of reaction without purification.
MS m/z (ESI): 506.5 [M+1]

### Step 2

### N-ethyl-5-fluoro-N-isopropyl-2-[6-(1-{[(1r,4r)-4-ethanesulfonamidocyclohexyl]methyl}pyrrolidin-3-yl)imidazo[1,5-a]pyridin-8-yl]benzamide 22

The crude product of Compound **22a** (80 mg) was dissolved in dichloromethane (3.0 mL), followed by the addition of ethanesulfonyl chloride (40.7 mg, 316.4 µmol) and triethylamine (80.04 mg, 791 µmol), and the mixture was stirred at room temperature for 12 hours. The reaction mixture was diluted with dichloromethane (20 mL), followed by the addition of a saturated aqueous sodium carbonate solution (20 mL). The mixture was extracted with dichloromethane (20 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 35%-65%), to obtain the title Compound **22** (1.92 mg, 3.21 µmol, yield: 2.03 %) as white solid.
MS m/z (ESI): 598.3 [M+1]
¹H NMR (400 MHz, CD₃OD) δ 8.57 - 8.16 (m, 3H), 7.71 (dd, *J =* 5.2, 8.4 Hz, 1H), 7.35 (dt, *J =* 2.8, 8.4 Hz, 1H), 7.29 - 7.21 (m, 2H), 6.86 - 6.80 (m, 1H), 3.56 (td, *J =* 6.4, 12.0 Hz, 1H), 3.45 - 3.38 (m, 2H), 3.20 - 2.91 (m, 5H), 2.81 - 2.58 (m, 3H), 2.43 - 2.30 (m, 1H), 2.03 (d, *J* = 4.8 Hz, 3H), 1.96 - 1.87 (m, 2H), 1.56 (s, 1H), 1.38 - 1.25 (m, 6H), 1.18 - 1.03 (m, 3H), 1.01 - 0.88 (m, 6H), 0.82 (t, *J=* 7.2 Hz, 1H), 0.32 (dd, *J* = 4.4, 6.4 Hz, 2H).

### Examples 22-1 and 22-2

### N-ethyl-5-fluoro-N-isopropyl-2-{6-[(3R)-1-{[(1r,4r)-4-ethanesulfonamidocyclohexyl]methyl}pyrrolidin-3-yl]imidazo[1,5-a]pyridin-8-yl}benzamide 22-1

### N-ethyl-5-fluoro-N-isopropyl-2-{6-[(3S)-1-{[(1r,4r)-4-ethanesulfonamidocyclohexyl]methyl}pyrrolidin-3-yl]imidazo[1,5-a]pyridin-5-yl}benzamide 22-2

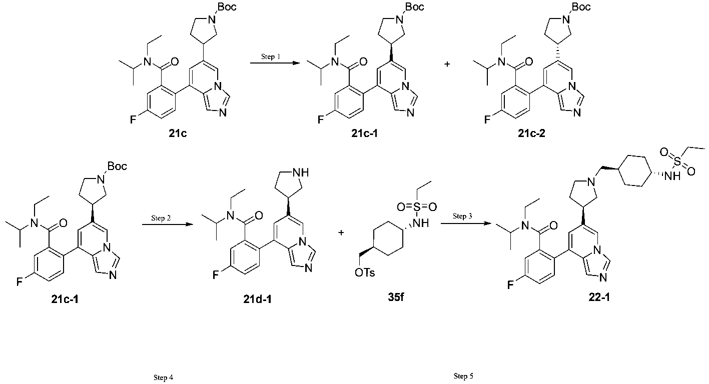

### Step 1

### (3R)-3-(8-{2-[ethyl(isopropyl)aminoformyl]-4-fluorophenyl}imidazo[1,5-a]pyridin-6-yl)pyrrolidin-1-tert-butyl formate 21c-1

### (3S)-3-(8-{2-[ethyl(isopropyl)aminoformyl]-4-fluorophenyl}imidazo[1,5-a]pyridin-6-yl)pyrrolidin-1-tert-butyl formate 21c-2

Compound **21c** (1.38 g, 2.78 mmol) was separated and purified by the chiral preparative SFC (preparative column: DAICEL CHIRALPAK OD (250 mm * 30 mm,10 um), mobile phase: 35%-35% (v/v) carbon dioxide-ethanol (0.1% ammonia water)), to obtain the title Compounds **21c-1** (490 mg, 990.7 µmol, yield: 35.5%) and **21c-2** (524 mg, 1.06 mmol, yield: 38.0%).

### Single-configuration Compound 21c-1:

MS m/z (ESI):495.6 [M+1]
SFC analysis: retention time: 1.42 min; chromatographic column: Chiralcel OD-3 50×4.6 mm I.D., 3 um; mobile phase: 5%-40% (v/v) EtOH (0.05% DEA)/CO₂.

### Single-configuration Compound 21c-2:

MS m/z (ESI):495.4 [M+1]
SFC analysis: retention time: 0.817 min; chromatographic column: Chiralcel OD-3 50×4.6 mm I.D., 3 um; mobile phase: 5%-40% (v/v) EtOH (0.05% DEA)/CO₂.

### Steps 2 and 4

### N-ethyl-5-fluoro-N-isopropyl-2-{6-[(3R)-pyrrolidin-3-yl]imidazo[1,5-a]pyridin-8-yl]benzamide 21d-1

### N-ethyl-5-fluoro-N-isopropyl-2-{6-[(3S)-pyrrolidin-3-yl]imidazo[1,5-a]pyridin-8-yl]benzamide 21d-2

Compound **21c-1/21c-2** (220 mg/150 mg, 244.8 µmol/303.3 µmol) was dissolved in methane dioxide (4.5 mL/3.0 mL), followed by the addition of trifluoroacetic acid (1.5 mL/1.0 mL). The mixture was stirred at room temperature to allow for reacting for 1.0/0.5 hours. The reaction mixture was concentrated under a reduced pressure, to obtain the crude product of title Compound **21d-1/21d-2** (168 mg/155 mg). The crude product was directly used in the next step of reaction without purification.

### Single-configuration Compound 21d-1:

MS m/z (ESI): 395.3 [M+1]

### Single-configuration Compound 21d-2:

MS m/z (ESI): 395.1 [M+1]

### Steps 3 and 5

### N-ethyl-5-fluoro-N-isopropyl-2-{6-[(3R)-1-{[(1r,4r)-4-ethanesulfonamidocyclohexyl]methyl}pyrrolidin-3-yl]imidazo[1,5-a]pyridin-8-yl}benzamide 22-1

### N-ethyl-5-fluoro-N-isopropyl-2-{6-[(3S)-1-{[(1r,4r)-4-ethanesulfonamidocyclohexyl]methyl}pyrrolidin-3-yl]imidazo[1,5-a]pyridin-5-yl}benzamide 22-2

The crude product of Compound **21c-1/21c-2** (160 mg/154 mg) was dissolved in DMF (3.0 mL/3.0 mL), followed by the addition of potassium carbonate (217 mg/209 mg, 1.57 mmol/1.52 mmol), TBAI(11.6 mg/11.2 mg, 31.5 µmol/30.3 µmol) and Compound **35f(142** mg/228 mg, 378 µmol/606 µmol), and the mixture was stirred at 90°C for (12.0/12.0) hours. An aqueous sodium bicarbonate solution (10 mL/10 mL) was added to the reaction mixture, which was then extracted with dichloromethane (20 mL x 3/20 mL x 3). The organic phases were combined, washed with saturated brine, dried over anhydrous sodium sulfate and filtered, and the filtrate was concentrated under a reduced pressure. The resulting residues were purified by the HPLC (mobile phase: acetonitrile, water (0.1% ammonia water)/acetonitrile, water (0.1% ammonium bicarbonate), elution gradient: 34%-64%/24%-54%), to obtain the title Compound **22-1/22-2** (7.68 mg/15.58 mg, 12.85 µmol/26.06 µmol, yield: 4.08%/8.60%)

### Single-configuration Compound 22-1:

MS m/z (ESI):598.2 [M+1]
Chiral HPLC: retention time: 1.653 min; chromatographic column: Chiralcel OD-3 50 X 4.6 mm I.D., 3 um; mobile phase: 5%-40% (v/v) MeOH (0.05% DEA)/CO₂.

¹H NMR (400 MHz, DMSO-*d*₆) δ 8.35 (s, 1H), 8.24 - 8.18 (m, 1H), 7.70 - 7.62 (m, 1H), 7.41 - 7.26 (m, 2H), 7.10 (s, 1H), 7.01 - 6.96 (m, 1H), 6.72 - 6.64 (m, 1H), 3.51 - 3.37 (m, 1H), 3.24 - 3.18 (m, 1H), 3.02 - 2.90 (m, 4H), 2.85 - 2.76 (m, 1H), 2.64 - 2.53 (m, 2H), 2.47 - 2.32 (m, 1H), 2.29 - 2.12 (m, 3H), 1.88 - 1.73 (m, 5H), 1.39 - 1.13 (m, 7H), 0.97 - 0.71 (m, 9H), 0.31 - 0.24 (m, 2H).

### Single-configuration Compound 22-2:

MS m/z (ESI):598.2 [M+1]
Chiral HPLC: retention time: 1.717 min; chromatographic column: Chiralcel OD-3 50 X 4.6 mm I.D., 3 um; mobile phase: 5%-40% (v/v) MeOH (0.05% DEA)/CO₂.

¹H NMR (400 MHz, DMSO-*d*₆) δ 8.36 (br d, J = 7.9 Hz, 1H), 8.28 - 8.18 (m, 1H), 7.74 - 7.63 (m, 1H), 7.42 - 7.27 (m, 2H), 7.16 - 7.08 (m, 1H), 6.68 - 6.57 (m, 1H), 8.35 (s, 1H), 8.26 - 8.16 (m, 1H), 7.74 - 7.59 (m, 1H), 7.45 - 7.25 (m, 2H), 7.20 - 6.82 (m, 2H), 6.73 - 6.63 (m, 1H), 3.48 - 3.39 (m, 1H), 3.24 - 3.18 (m, 1H), 3.06 - 2.87 (m, 4H), 2.86 - 2.76 (m, 1H), 2.69 - 2.54 (m, 2H), 2.48 - 2.36 (m, 1H), 2.33 - 2.11 (m, 3H), 1.89 - 1.73 (m, 4H), 1.64 - 1.48 (m, 1H), 1.39 - 1.12 (m, 7H), 0.97 - 0.72 (m, 9H), 0.32 - 0.23 (m, 2H).

### Example 23

### N-ethyl-5-fluoro-2-(6-{1-[(1S,3S,4R)-5-methylene-2-azabicyclo[2.2.2]octan-3-carbonyl]pyrrolidin-3-yl}imidazo[1,5-a]pyridin-8-yl)-N-(isopropyl)benzamide 23

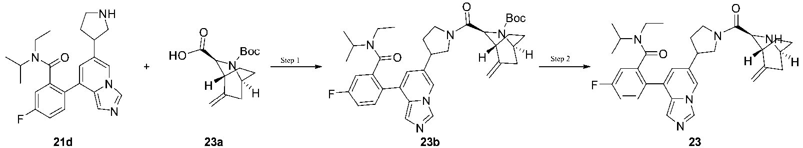

### Step 1

### (1S,3S,4R)-3-[3-(8-{2-[ethyl(isopropyl)aminoformyl]- 4-fluorophenyl}imidazo[1,5-a]pyridin-6-yl)pyrrolidin-1-carbonyl]-5-methylene-2-azabicyclo[2. 2.2]octan-2-tert-butyl carboxylate 23b

Compound **21d** (90.0 mg, 177 µmol, trifluoroacetate), (1*S*,3*S*,4*R*)-2-tert-butoxycarbonyl-5-methylene-2-azabicyclo[2.2.2]octan-3-carboxylic acid **23a** (45.0 mg, 265.5 µmol, prepared by referring to the method disclosed in Reference Example 13 on Page 93 in the Patent Application No. WO2020032105A1) and *N,N-*diisopropylethylamine (89.6 mg, 693 µmol) were dissolved in dichloromethane (3 mL), and cooled to 0°C. EDCI (50.89 mg, 265.48 µmol) and HOBt (40.66 mg, 265.48 µmol) were added, and the mixture was stirred at room temperature for 12 hours. Water (20 mL) was added to the reaction mixture, which was then extracted with dichloromethane (20 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 41%-71%), to obtain the title Compound **23b** (30 mg, 46.6 µmol, yield: 26.3%) as white solid.
MS m/z (ESI): 644.3 [M+1]

### Step 2

### N-ethyl-5-fluoro-2-(6-{1-[(1S,3S,4R)-5-methylene-2-azabicyclo[2.2.2]octan-3-carbonyl ]pyrrolidin-3-yl}imidazo[1,5-a]pyridin-8-yl)-N-(isopropyl)benzamide 23

Compound **23b** (25.0 mg, 38.8 µmol) was dissolved in methane dioxide (4.8 mL) and trifluoroacetic acid (0.2 mL). The mixture was stirred at room temperature to allow for reacting for 0.5 hours. The reaction mixture was concentrated under a reduced pressure, and the resulting residues were purified by the HPLC (mobile phase: acetonitrile, water (0.1% ammonia water), elution gradient: 36%-66%), to obtain the title Compound **23** (8.84 mg, 16.26 µmol, yield: 41.87%).
MS m/z (ESI): 544.4 [M+1]
¹H NMR (400 MHz, CD₃OD) δ 8.40 - 8.34 (m, 1H), 8.28 - 8.21 (m, 1H), 7.71 (dd, *J =* 5.2, 8.4 Hz, 1H), 7.40 - 7.31 (m, 1H), 7.29 - 7.20 (m, 2H), 6.87 - 6.77 (m, 1H), 5.11 - 4.95 (m, 1H), 4.14 - 3.75 (m, 3H), 3.69 - 3.47 (m, 3H), 3.46 - 3.35 (m, 2H), 3.17 - 3.12 (m, 1H), 3.10 - 2.94 (m, 1H), 2.77 - 2.53 (m, 3H), 2.33 - 1.99 (m, 2H), 1.96 - 1.70 (m, 2H), 1.65 - 1.51 (m, 2H), 1.17 - 1.05 (m, 1H), 0.97 (d, *J* = 5.6 Hz, 3H), 0.93 - 0.78 (m, 4H), 0.36 - 0.29 (m, 2H).

### Example 24

### N-[4-{[3-(5-{2-[ethyl(isopropyl)aminoformyl]-4-fluorophenyl}imidazol[1,5-a]pyridin-7-yl}pyrrolidin-1-yl)methyl)cyclohexyl)tert-butyl carbamate 24

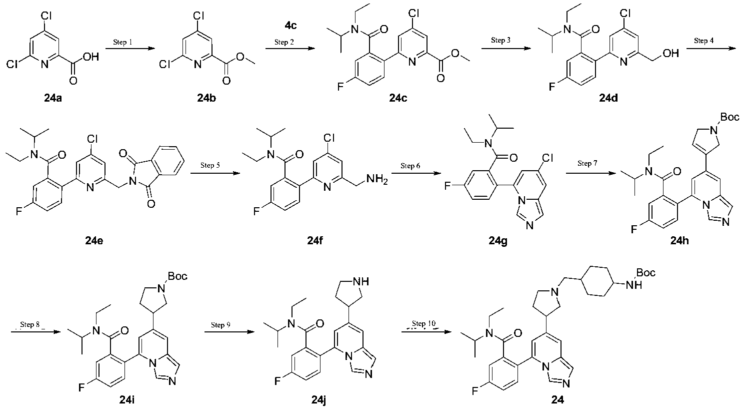

### Step 1

### 4,6-dichloropyridin-2-methyl carboxylate 24b

4,6-Dichloropyridin-2-carboxylic acid **24a** (15 g, 78.13 mmol) was dissolved in methanol (150 mL), and cooled to 0°C, followed by the slow addition of SOCl₂ (18.59 g, 156.25 mmol). The mixture was stirred at 65°C to allow for reacting for 12 hours. The reaction mixture was concentrated under a reduced pressure, and dichloromethane (150 mL) was added to dissolve the residues. The organic phase was washed with saturated aqueous sodium bicarbonate solution (50 mL x 3), dried over anhydrous sodium sulfate, and concentrated under a reduced pressure, to obtain the crude product of title Compound **24b** (15 g, 72.8 mmol, yield: 93.2%). The crude product was directly used in the next step of reaction without purification.
MS m/z (ESI): 206.0 [M+1]
¹H NMR (400 MHz, CDCl₃) δ 8.05 (d, J *=* 1.6 Hz, 1H), 7.55 (d, J = 1.6 Hz, 1H), 4.00 (s, 3H).

### Step 2

### 4-chloro-6-{2-[ethyl(isopropyl)aminoformyl]-4-fluorophenyl}pyridin-2-methyl carboxylate 24c

Compound **24b** (3.93 g, 19.09 mmol), Compound **4c** (6.4 g, 19.09 mmol) and sodium carbonate (6.07 g, 57.28 mmol) were dissolved in dioxane (35 mL) and water (5.0 mL). Under a nitrogen atmosphere, Pd(dppf)Cl₂ (1.40 g, 1.91 mmol) was added, and the mixture was stirred at 75°C to allow for reacting for 1.0 hour. The reaction mixture was cooled to room temperature, followed by adding water (30 mL) and extracting with ethyl acetate (50 mL x 3). The organic phases were combined, washed with saturated brine, dried over anhydrous sodium sulfate and filtered, and the filtrate was concentrated under a reduced pressure. The resulting residues were purified by the silica-gel column chromatography using petroleum ether/ethyl acetate as an elution system, to obtain the title Compound **24c** (6.0 g, 15.84 mmol, yield: 82.96%).
MS m/z (ESI): 379.1 [M+1]
¹H NMR (400 MHz, DMSO-*d*₆) δ 8.08 - 8.04 (m, 1H), 7.95 - 7.90 (m, 1H), 7.88 - 7.81 (m, 1H), 7.46 - 7.39 (m, 1H), 7.32 - 7.26 (m, 1H), 3.90 - 3.88 (m, 3H), 3.57 (td, *J =* 6.6, 13.3 Hz, 1H), 3.43 - 3.36 (m, 1H), 3.21 - 3.02 (m, 2H), 1.07 - 0.97 (m, 6H), 0.76 (br d, *J=* 6.5 Hz, 3H).

### Step 3

### 2-[4-chloro-6-(hydroxymethyl)pyridin-2-yl]-N-ethyl-5-fluoro-N-(isopropyl)benzamide 24d

Compound **24c** (6.0 g, 15.84 mmol) was dissolved in tetrahydrofuran (60 mL) and methanol (60 mL). The reaction mixture was cooled to 0°C, followed by adding NaBH₄ (2.5 g, 66.1 mmol). The mixture was stirred at room temperature to allow for reacting for 8 hour. Water (50 mL) was added to the reaction mixture, which was then extracted with ethyl acetate (50 mL x 3). The organic phases were combined, washed with saturated brine, dried over anhydrous sodium sulfate, and concentrated under a reduced pressure. The crude product of title Compound **24d** (4.7 g, 13.4 mmol, yield: 84.6%) was obtained. The crude product was directly used in the next step of reaction without purification.
MS m/z (ESI): 351.0 [M+1]

### Step 4

### 2-{4-chloro-6-[(1,3-dioxo-2,3-dihydro-1H-isoindol-2-yl)methyl]pyridin-2-yl}-N-ethyl-5-fluoro-N-(isopropyl)benzamide 24e

Compound **24d** (4.7 g, 13.4 mmol), 2,3-dihydro-1*H*-isoindol-1,3-dione (2.37 g, 16.1 mmol) and triphenylphosphine (4.22 g, 16.1 mmol) were dissolved in tetrahydrofuran (60 mL), and cooled to 0°C under a nitrogen atmosphere, followed by the dropwise addition of DIAD(4.06 g, 20.1 mmol). The mixture was stirred at room temperature to allow for reacting for 12 hours, and the reaction mixture was concentrated under a reduced pressure. The resulting residues were purified by the silica-gel column chromatography using petroleum ether/ethyl acetate as an elution system, to obtain the title Compound **24e** (3.5 g, 7.29 mmol, yield: 54.4%).
MS m/z (ESI): 480.2 [M+1]

### Step 5

### 2-[6-(aminomethyl)-4-chloropyridin-2-yl]-N-ethyl-5-fluoro-N-(isopropyl)benzamide 24f

Compound **24e** (3.5 g, 7.29 mmol) was dissolved in the solution of methylamine in ethanol ((45 g, 434.68 mmol, 50.06 mL, 30% purity). The mixture was stirred at 50°C to allow for reacting for 1.0 hours. The reaction mixture was cooled to room temperature, and concentrated under a reduced pressure. The crude product of title Compound **24f** (3.5 g, 10.0 mmol, yield: 137.2 %) was obtained. The crude product was directly used in the next step of reaction without purification.
MS m/z (ESI): 350.1 [M+1]

### Step 6

### 2-{7-chloroimidazo[1,5-a]pyridin-5-yl}-N-ethyl-5-fluoro-N-(isopropyl)benzamide 24g

Compound **24f** (1.75 g, 5.0 mmol) was dissolved in ethyl formate (20 mL), followed by the addition of triethylamine (3.04 g, 30.1 mmol). The mixture was stirred at 70°C to allow for reacting for 12 hours. The reaction mixture was cooled to room temperature under a reduced pressure, and concentrated under a reduced pressure, and the obtained crude product (1.5 g, 5.03 mmol) was dissolved in toluene (15 mL), followed by the addition of phosphorus oxychloride (3.65 g, 23.82 mmol). The mixture was stirred at 100°C to allow for reacting for 2.0 hours. The reaction mixture was cooled to room temperature, and concentrated under a reduced pressure. The residues were dissolved in dichloromethane (20 mL), and a saturated aqueous sodium bicarbonate solution was added to regulate PH to 8. The mixture was extracted with dichloromethane (35 mL x 3), and the organic phases were combined, washed with saturated brine, dried over anhydrous sodium sulfate, and concentrated under a reduced pressure. The resulting residues were purified by the silica-gel column chromatography using petroleum ether/ethyl acetate as an elution system, to obtain the title Compound **24g** (810 mg, 2.25 mmol, yield: 56.7%).
MS m/z (ESI): 360.0 [M+1]
¹H NMR (400 MHz, DMSO-*d*₆) δ 8.29 - 7.83 (m, 2H), 7.56 - 7.40 (m, 4H), 6.59 - 6.43 (m, 1H), 3.79 - 3.43 (m, 2H), 3.00 - 2.80 (m, 1H), 1.03 - 0.63 (m, 9H).

### Step 7

### 3-(5-{2-[ethyl(isopropyl)aminoformyl]-4-fluorophenyl}imidazo[1,5-a]pyridin-7-yl)-2,5-dihydro-1H-pyrrol-1-tert-butyl formate 24h

Compound **24g** (810 mg, 2.25 mmol), 3-(tetramethyl-1,3,2-dioxaboran-2-yl)-2,5-dihydro-1*H*-pyrrol-1-tert-butyl formate (697.71 mg, 2.36 mmol) and potassium phosphate (1.19 g, 5.63 mmol) were dissolved in dioxane (10 mL) and water (1.5 mL). Pd (dppf)Cl₂ (150 mg, 230.15 µmol) was added. Under a nitrogen atmosphere, the mixture was stirred at 80°Cto allow for reacting for 12 hours. The reaction mixture was cooled to room temperature, water (20 mL) was added, and the mixture was extracted with ethyl acetate (30 mL x 3). The organic phases were combined, washed with saturated brine, dried over anhydrous sodium sulfate, and concentrated under a reduced pressure. The resulting residues were purified by the silica-gel column chromatography using petroleum ether/ethyl acetate as an elution system, to obtain the title Compound **24h** (780 mg, 1.58 mmol, yield: 70.34%).
MS m/z (ESI): 493.2 [M+1]
¹H NMR (400 MHz, DMSO-*d*₆) δ 8.24 - 7.63 (m, 2H), 7.58 - 7.39 (m, 4H), 7.00 - 6.65 (m, 1H), 6.45 - 6.13 (m, 1H), 4.40 (br d, J = 3.0 Hz, 2H), 4.29 - 4.14 (m, 2H), 3.75 - 3.39 (m, 2H), 3.04 - 2.73 (m, 1H), 1.45 (d, *J =* 8.8 Hz, 9H), 1.03 - 0.38 (m, 9H).

### Step 8

### 3-(5-{2-[ethyl(isopropyl)aminoformyl]-4-fluorophenyl}imidazo[1,5-a]pyridin-7-yl)pyrrolidin-1-tert-butyl formate 24i

Compound **24h** (320 mg, 649.6 µmol) was dissolved in methanol (10 mL), followed by the addition of Pd/C (200 mg, 1.88 mmol). Under a hydrogen atmosphere (15 Psi), the mixture was stirred at room temperature for 12 hours. The reaction mixture was filtered, and the filtrate was concentrated under a reduced pressure, to obtain the crude product of title Compound **24i** (300 mg, 606.55 µmol, yield: 93.4%). The crude product was directly used in the next step of reaction without purification.
MS m/z (ESI): 495.3 [M+1]

### Step 9

### N-ethyl-5-fluoro-N-isopropyl-2-[7-(pyrrolidin-3-yl)imidazo[1,5-a]pyridin-5-yl]benzamide 24j

Compound **24i** (387 mg, 782.45 µmol) was dissolved in methane dioxide (5.0 mL), followed by the addition of trifluoroacetic acid (3.70 g, 32.45 mmol, 2.5 mL) at 0°C. The mixture was stirred at room temperature to allow for reacting for 1.0 hours. The reaction mixture was concentrated under a reduced pressure, to obtain the crude product of title Compound **24j** (400 mg). The crude product was directly used in the next step of reaction without purification. MS m/z (ESI): 395.2 [M+1]

### Step 10

### N-(4-{[3-(5-{2-[ethyl(isopropyl)aminoformyl]-4-fluorophenyl}imidazo[1,5-a]pyridin-7-yl)pyrrolidin-1-yl]methyl}cyclohexyl)tert-butyl carbamate 24

The crude product of Compound **24j** (400 mg, 786.62 µmol, trifluoroacetate), *N*-[(1r,4r)-4-formylcyclohexyl]tert-butyl carbamate (215 mg, 945.89 µmol) and triethylamine (160 mg, 1.58 mmol) were dissolved in methanol (5.0 mL), and stirred at room temperature for 1.0 hour. Sodium cyanoborohydride (150 mg, 2.39 mmol) was added, and the reaction mixture was stirred at room temperature for 11 hour. A saturated aqueous sodium bicarbonate solution (10 mL) was added to the reaction mixture, which was then extracted with dichloromethane (20 mL x 3). The organic phases were combined, washed with saturated brine (20 mL), dried over anhydrous sodium sulfate, and concentrated under a reduced pressure. The resulting residues were purified by the silica-gel column chromatography using dichloromethane/methanol as an elution system, to obtain the title Compound **24** (340 mg, 561.26 µmol, yield: 71.35%).
MS m/z (ESI): 606.6 [M+1]
¹H NMR (400 MHz, DMSO-*d*₆) δ 8.12 - 7.64 (m, 2H), 7.50 - 7.27 (m, 4H), 6.74 - 6.61 (m, 1H), 6.57 - 6.45 (m, 1H), 3.64 - 3.41 (m, 1H), 3.28 - 3.20 (m, 1H), 3.19 - 3.06 (m, 1H), 2.97 - 2.73 (m, 2H), 2.67 - 2.54 (m, 2H), 2.47 - 2.31 (m, 1H), 2.29 - 2.09 (m, 3H), 1.97 - 1.60 (m, 5H), 1.57 - 0.51 (m, 24H).

### Examples 25 and 26

### N-ethyl-5-fluoro-N-isopropyl-2-[7-(1-{[(1r,4r)-4-ethanesulfonamidocyclohexyl]methyl}pyrrolidin-3-yl)imidazo[1,5-a]pyridin-5-yl]benzamide 25

### N-ethyl-5-fluoro-N-isopropyl-2-[7-(1-{[(1s,4s)-4-ethanesulfonamidocyclohexyl]methyl}pyrrolidin-3-yl)imidazo[1,5-a]pyridin-5-yl]benzamide 26

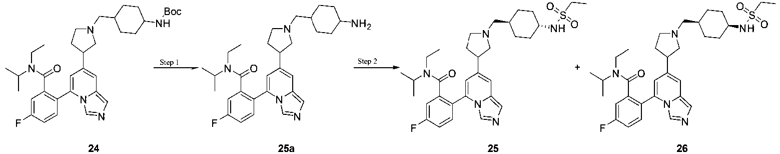

### Step 1

### 2-(7-{1-[(4-aminocyclohexyl)methyl]pyrrolidin-3-yl}imidazo[1,5-a]pyridin-5-yl)-N-ethyl-5-fluoro-N-(isopropyl)benzamide 25a

Compound **24** (340 mg, 561.26 µmol) was dissolved in dichloromethane (5.0 mL), followed by the addition of trifluoroacetic acid (2.96 g, 25.96 mmol, 2 mL) at 0°C. The mixture was stirred at room temperature to allow for reacting for 1.0 hours. The reaction mixture was concentrated under a reduced pressure, to obtain the crude product of title Compound **25a** (350 mg). The crude product was directly used in the next step of reaction without purification. MS m/z (ESI): 506.5 [M+1]

### Step 2

### N-ethyl-5-fluoro-N-isopropyl-2-[7-(1-{[(1r,4r)-4-ethanesulfonamidocyclohexyl]methyl}pyrrolidin-3-yl)imidazo[1,5-a]pyridin-5-yl]benzamide 25

### N-ethyl-5-fluoro-N-isopropyl-2-[7-(1-{[(1s,4s)-4-ethanesulfonamidocyclohexyl]methyl}pyrrolidin-3-yl)imidazo[1,5-a]pyridin-5-yl]benzamide 26

The crude product of Compound **25a** (347 mg) was dissolved in dichloromethane (5.0 mL), followed by the addition of ethanesulfonyl chloride (144.01 mg, 1.12 mmol) and triethylamine (283.33 mg, 2.80 mmol), and the mixture was stirred at room temperature for 12 hours. A saturated aqueous sodium carbonate solution (10 mL) was added to the reaction mixture. The mixture was extracted with dichloromethane (20 mL x 3). The organic phases were combined, washed with saturated brine, dried over anhydrous sodium sulfate, and concentrated under a reduced pressure. The resulting residues were purified by the HPLC (mobile phase: acetonitrile, water (0.1% ammonium bicarbonate), elution gradient: 50%-80%) to obtain a mixture (100 mg), which was separated and purified by the preparative SFC (preparative column: DAICEL CHIRALPAK IF (250 mm * 50 mm,10 um), mobile phase: 25% n-hexane-ethanol (0.1% ammonia water)), to obtain the title Compounds **25** (1.69 mg, 2.83 µmol, yield: 1.69%) and **26** (1.95 mg, 3.26 µmol, yield: 1.95%).

### Compound 25:

MS m/z (ESI): 598.5 [M+1]
SFC analysis: retention time: 4.613 min; chromatographic column: Chiralpak IF-3 50 x 4.6 mm I.D., 3 um; mobile phase: n-hexane (0.05% isopropylamine)/[ethanol +acetonitrile (0.05% isopropylamine)] = 80/20.

¹H NMR (400 MHz, DMSO-*d*₆) δ 8.12 - 7.62 (m, 2H), 7.52 - 7.29 (m, 4H), 7.04 - 6.93 (m, 1H), 6.59 - 6.43 (m, 1H), 3.30 - 3.17 (m, 3H), 3.07 - 2.76 (m, 6H), 2.74 - 2.56 (m, 2H), 2.36 - 2.12 (m, 3H), 1.92 - 1.67 (m, 6H), 1.28 - 1.14 (m, 7H), 1.00 - 0.63 (m, 9H).

### Compound 26:

MS m/z (ESI): 598.4 [M+1]
SFC analysis: retention time: 3.614 min; chromatographic column: Chiralpak IF-3 50 x 4.6 mm I.D., 3 um; mobile phase: n-hexane (0.05% isopropylamine)/[ethanol +acetonitrile (0.05% isopropylamine)] = 80/20.

¹H NMR (400 MHz, DMSO-*d*₆) δ 8.13 - 7.58 (m, 2H), 7.51 - 7.28 (m, 4H), 6.98 - 6.87 (m, 1H), 6.57 - 6.44 (m, 1H), 3.28 - 3.21 (m, 2H), 3.07 - 2.72 (m, 5H), 2.71 - 2.55 (m, 2H), 2.43 - 2.09 (m, 4H), 1.63 - 1.33 (m, 10H), 1.29 - 1.13 (m, 6H), 1.00 - 0.65 (m, 7H).

### Examples 25-1 and 25-2

### N-ethyl-5-fluoro-N-isopropyl-2-{7-[(3S)-1-{[(1r,4r)-4-ethanesulfonamidocyclohexyl]methyl}pyrrolidin-3-yl]imidazo[1,5-a]pyridin-5-yl}benzamide 25-1

### N-ethyl-5-fluoro-N-isopropyl-2-{7-[(3R)-1-{[(1r,4r)-4-ethanesulfonamidocyclohexyl]methyl}pyrrolidin-3-yl]imidazo[1,5-a]pyridin-5-yl}benzamide 25-2

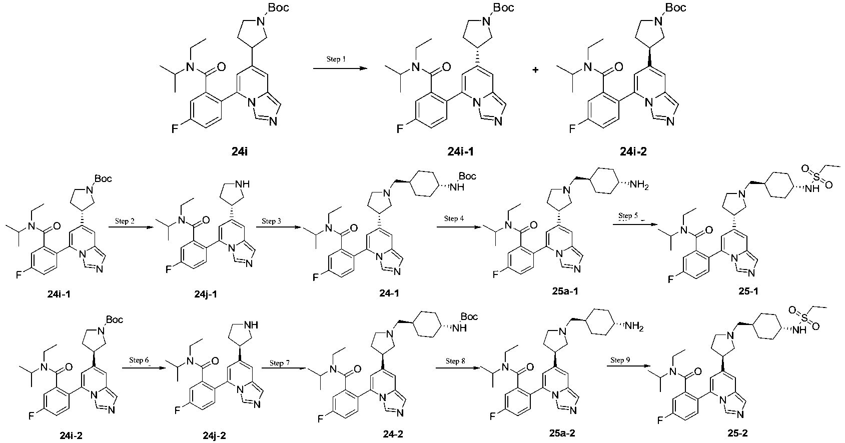

### Step 1

### (3S)-3-(5-{2-[ethyl(isopropyl)aminoformyl]-4-fluorophenyl}imidazo[1,5-a]pyridin-7-yl)pyrrolidin-1-tert-butyl formate 24i-1

### (3R)-3-(5-{2-[ethyl(isopropyl)aminoformyl]-4-fluorophenyl}imidazo[1,5-a]pyridin-7-yl)pyrrolidin-1-tert-butyl formate 24i-2

Compound **24i** (300 mg, 606.6 µmol) was separated and purified by the chiral preparative SFC (preparative column: DAICEL CHIRALPAK IC (250 mm * 30 mm,10 um), mobile phase: 0% to 50% (v/v) carbon dioxide-ethanol (0.1% ammonia water)), to obtain the title Compounds **24i-1** (100 mg, 202.2 µmol, yield: 33.3%) and **24i-2** (100 mg, 202.2 µmol, yield: 33.3%).

### Single-configuration Compound 24i-1:

MS m/z (ESI):495.2 [M+1]
SFC analysis: retention time: 1.50 min; chromatographic column: Chiralpak IC-3 50 x 4.6 mm I.D., 3 um; mobile phase: CO₂/EtOH (0.05% DEA) = 60/40.

### Single-configuration Compound 24i-2:

MS m/z (ESI):495.2 [M+1]
SFC analysis: retention time: 2.0 min; chromatographic column: Chiralpak IC-3 50 x 4.6 mm I.D., 3 um; mobile phase: CO₂/EtOH (0.05% DEA) = 60/40.

### Steps 2 and 6

### N-ethyl-5-fluoro-N-isopropyl-2-{7-[(3S)-pyrrolidin-3-yl]imidazo[1,5-a]pyridin-5-yl]benzamide 24j-1

### N-ethyl-5-fluoro-N-isopropyl-2-{7-[(3R)-pyrrolidin-3-yl]imidazo[1,5-a]pyridin-5-yl]benzamide 24j-2

Compound **24i-1/24i-2** (100 mg/100 mg, 202.2 µmol/202.2 µmol) was dissolved in methane dioxide (3.0 mL/3.0 mL), followed by the addition of trifluoroacetic acid (1.0 mL/1.0 mL). The mixture was stirred at room temperature to allow for reacting for 1.0 hours. The reaction mixture was concentrated under a reduced pressure, to obtain the crude product of title Compound **24j-1/24j-2** (100 mg/100 mg). The crude product was directly used in the next step of reaction without purification.

### Single-configuration Compound 24j-1:

MS m/z (ESI): 395.2 [M+1]

### Single-configuration Compound 24j-2:

MS m/z (ESI): 395.2 [M+1]

### Steps 3 and 7

### N-[(1r,4r)-4-{[(3S)-3-(5-12-[ethyl(isopropyl)aminofonnyl]-4-fluorophenyl}imidazo[1,5-a]pyridin-7-yl)pyrrolidin-1-yl]methyl}cyclohexyl]tert-butyl carbamate 24-1

### N-[(1r,4r)-4-{[(3R)-3-(5-{2-[ethyl(isopropyl)aminoformyl]-4-fluorophenyl}imidazo[1,5-a]pyridin-7-yl)pyrrolidin-1-yl]methyl}cyclohexyl]tert-butyl carbamate 24-2

The crude product of Compound **24j-1/24j-2** (100 mg/100 mg) was dissolved in methanol (2.0 mL/3.0 mL), followed by the addition of triethylamine (40 mg/40 mg, 395.3 µmol/395.3 µmol) and *N*-[(1*r*,4*r*)-4-formylcyclohexyl]tert-butyl carbamate (53.64 mg/53.75 mg, 235.98 µmol/236.47 µmol), and the mixture was stirred at room temperature for (1.0/1.0) hour. Sodium cyanoborohydride (37.5 mg / 37.5 mg, 596.74 µmol/596.74 µmol) was added, and the mixture was stirred at room temperature to allow for reacting for (11/11) hours. An aqueous sodium bicarbonate solution (10 mL/10 mL) was added to the reaction mixture, which was then extracted with dichloromethane (20 mL x 3/20 mL x 3). The organic phases were combined, washed with saturated brine, dried over anhydrous sodium sulfate and filtered, and the filtrate was concentrated under a reduced pressure. The resulting residues were purified by the silica-gel column chromatography using dichloromethane/methanol as an elution system, to obtain the title Compound **24-1/24-2** (100 mg/100 mg, 165.08 µmol/165.08 µmol, yield: 83.94%/83.94%)

### Single-configuration Compound 24-1:

MS m/z (ESI):606.5 [M+1]
SFC analysis: retention time: 1.16 min; chromatographic column: Chiralpak IC-3 50×4.6 mm I.D., 3 um; mobile phase: CO2/[IPA+ACN (0.05% DEA)] = 50/50.

### Single-configuration Compound 24-2:

MS m/z (ESI):606.4 [M+1]
SFC analysis: retention time: 0.87 min; chromatographic column: Chiralpak IC-3 50×4.6 mm I.D., 3 um; mobile phase: CO2/[IPA+ACN (0.05% DEA)] = 50/50.

### Steps 4 and 8

### N-ethyl-5-fluoro-N-isopropyl-2-{7-[(3S)-1-{[(1r,4r)-4-aminocyclohexyl]methyl}pyrrolidin-3-yl]imidazo[1,5-a]pyridin-5-yl}benzamide 25a-1

### N-ethyl-5-fluoro-N-isopropyl-2-{7-[(3R)-1-{[(1r,4r)-4-aminocyclohexyl]methyl}pyrrolidin-3-yl]imidazo[1,5-a]pyridin-5-yl}benzamide 25a-2

Compound **24-1/24-2** (100 mg/100 mg, 165.08 µmol/165.08 µmol) was dissolved in methane dioxide (2.0 mL/2.0 mL), followed by the addition of trifluoroacetic acid (1.0 mL/1.0 mL). The mixture was stirred at room temperature to allow for reacting for 1.0 hours. The reaction mixture was concentrated under a reduced pressure, to obtain the crude product of title Compound **25a-1/25a-2** (100 mg/100 mg). The crude product was directly used in the next step of reaction without purification.

### Single-configuration Compound 25a-1:

MS m/z (ESI): 506.2 [M+1]

### Single-configuration Compound 25a-2:

MS m/z (ESI): 506.2 [M+1]

### Steps 5 and 9

### N-ethyl-5-fluoro-N-isopropyl-2-{7-[(3S)-1-{[(1r,4r)-4-ethanesulfonamidocyclohexyl]methyl}pyrrolidin-3-yl]imidazo[1,5-a]pyridin-5-yl}benzamide 25-1

### N-ethyl-5-fluoro-N-isopropyl-2-{7-[(3R)-1-{[(1r,4r)-4-ethanesulfonamidocyclohexyl]methyl}pyrrolidin-3-yl]imidazo[1,5-a]pyridin-5-yl}benzamide 25-2

The crude product of Compound **25a-1/25^{a}-2** (100 mg/100 mg) was dissolved in dichloromethane (3.0 mL), followed by the addition of ethanesulfonyl chloride (32 mg/31.82 mg, 248.8 µmol/247.5 µmol) and triethylamine (85 mg/83.48, 840 µmol/825.0 µmol), and the mixture was stirred at room temperature for 2 hours. The reaction mixture was diluted with dichloromethane (20 mL), followed by the addition of a saturated aqueous sodium bicarbonate solution (10 mL). The mixture was extracted with dichloromethane (20 mL x 3). The organic phases were combined, washed with saturated brine, dried over anhydrous sodium sulfate, and concentrated under a reduced pressure. The resulting residues were purified by the HPLC (mobile phase: acetonitrile, water (0.1% formic acid), elution gradient: 0%-30%), to obtain the title Compound **25-1/25-2** (2.57 mg/2.99 mg, 4.30 µmol/5.0 µmol, yield: 2.66%/3.03%).

### Single-configuration Compound 25-1:

MS m/z (ESI):598.4 [M+1]
Chiral HPLC: retention time: 5.45 min; chromatographic column: Chiralpak IA-3 50 x 4.6 mm I.D., 3 um; mobile phase: hexane (0.05% IPAm)/IPA+ACN (0.05% IPAm) = 85/15.

¹H NMR (400 MHz, DMSO-*d*₆) δ 8.20 (s, 1H), 8.09 - 7.61 (m, 2H), 7.52 - 7.27 (m, 4H), 6.99 (d, J = 7.6 Hz, 1H), 6.58 - 6.43 (m, 1H), 3.40 - 3.20 (m, 3H), 3.12 - 2.76 (m, 6H), 2.75 - 2.59 (m, 2H), 2.38 - 2.11 (m, 3H), 1.92 - 1.70 (m, 5H), 1.62 - 0.41 (m, 17H).

### Single-configuration Compound 25-2:

MS m/z (ESI):598.4 [M+1]
Chiral HPLC: retention time: 4.85 min; chromatographic column: Chiralpak IA-3 50 x 4.6 mm I.D., 3 um; mobile phase: hexane (0.05% IPAm)/IPA+ACN (0.05% IPAm) = 85/15.

¹H NMR (400 MHz, DMSO-*d*₆) δ 8.20 (s, 1H), 8.09 - 7.60 (m, 2H), 7.53 - 7.28 (m, 4H), 6.99 (d, *J =* 7.6 Hz, 1H), 6.58 - 6.44 (m, 1H), 3.46 - 3.19 (m, 3H), 3.18 - 2.78 (m, 6H), 2.76 - 2.61 (m, 2H), 2.40 - 2.09 (m, 3H), 1.94 - 1.70 (m, 5H), 1.68 - 0.31 (m, 17H)

### Example 27

### N-ethyl-5-fluoro-2-[1-methyl-5-(1-{[(1r,4r)-4-ethanesulfonamidocyclohexyl]methyl}pyrrolidin-3-yl)-1H-pyrazolo[4,3-b]pyridin-7-yl]-N-(isopropyl)benzamide 27

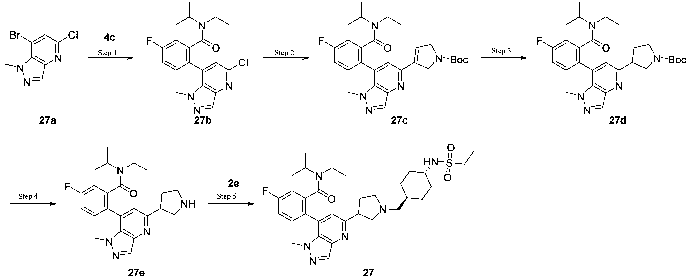

### Step 1

### 2-{5-chloro-1-methyl-1H-pyrazolo[4,3-b]pyridin-7-yl}-N-ethyl-5-fluoro-N-(isopropyl)benzamide 27b

7-Bromo-5-chloro-1-methyl-1*H*-pyrazolo[4,3-*b*]pyridine **27a**(450 mg, 1.83 mmol) and Compound **4c**(1.47 g, 2.19 mmol) were dissolved in dioxane (12 mL) and water (2.0 mL). Sodium carbonate (387 mg, 3.65 mmol) and Pd(dppf)Cl₂ (66.8 mg, 91.3 µmol) were added, and the mixture was stirred at 70°C under a nitrogen atmosphere to allow for reacting for 16 hours. The reaction mixture was cooled to room temperature and filtered, and the filtrate was concentrated under a reduced pressure. The resulting residues were purified by the column chromatography (C18; mobile phase: acetonitrile, water (0.1% trifluoroacetic acid), elution gradient: 40%-60%), to obtain the title Compound **27b** (425 mg, 1.13 mmol, yield: 62.1%).
MS m/z (ESI): 375.1 [M+1]
¹H NMR (400 MHz, DMSO-*d*₆) δ 8.28 (s, 1H), 7.69 (dd, *J=* 22.4, 15.2 Hz, 1H), 7.46 (t, *J=* 7.6 Hz, 2H), 7.32-7.19 (m, 1H), 3.85 - 3.51 (m, 4H), 3.27 - 2.62 (m, 2H), 1.14 - 0.37 (m, 9H).

### Step 2

### 3-(7-{2-[ethyl(isopropyl)aminoformyl]-4-fluorophenyl}1-methyl-1H-pyrazolo[4,3-b]pyridin-5-yl)-2,5-dihydro-1H-pyrrol-1-tert-butyl formate 27c

Compound **27b**(300 mg, 533 µmol) and 3-(tetramethyl-1,3,2-dioxaboran-2-yl)-2,5-dihydro-1*H*-pyrrol-1-tert-butyl formate (189 mg, 640 µmol) were dissolved in dioxane (4.0 mL) and water (0.7 mL). Potassium carbonate (221 mg, 1.60 mmol) and Pd(dppf)Cl₂ (39.0 mg, 53.4 µmol) were added, and the mixture was stirred at 100°C under a nitrogen atmosphere to allow for reacting for 16 hours. The reaction mixture was concentrated under a reduced pressure, and the resulting residues were purified by the preparative thin-layer chromatography using petroleum ether/ethyl acetate as an elution system, to obtain the title Compound **27c** (167 mg, 329 µmol, yield: 61.7%).
MS m/z (ESI): 508.25 [M+1]

### Step 3

### 3-(7-{2-[ethyl(isopropyl)aminoformyl]-4-fluorophenyl} 1-methyl-1H-pyrazolo[4,3-b]pyridin-5-yl)pyrrolidin-1-tert-butyl formate 27d

Compound **27c** (167 mg, 329 µmol) was dissolved in methanol (3.0 mL), followed by the addition of Pd/C (16.08 mg). Under a hydrogen atmosphere (15 Psi), the mixture was stirred at room temperature for 5 hours. The reaction mixture was filtered, and the filtrate was concentrated under a reduced pressure, to obtain the crude product of title Compound **27d** (110 mg, 215 µmol, yield: 65.6%). The crude product was directly used in the next step of reaction without purification.
MS m/z (ESI): 510.2 [M+1]

### Step 4

### N-ethyl-5-fluoro-2-[1-methyl-5-(yridinene-3-yl)-1H-pyrazolo[4,3-b]yridine-7-yl]-N-(propan-2-yl)benzamide

### N-ethyl-5-fluoro-2-[1-methyl-5-(pyrrolidin-3-yl)-1H-pyrazolo[4,3-b]pyridin-7-yl]-N-isopropylbenzamide 27e

Compound **27d** (110 mg, 216 µmol) was dissolved in methane dioxide (2.0 mL), followed by the addition of trifluoroacetic acid (0.6 mL) at 0°C. The mixture was stirred at room temperature to allow for reacting for 2.0 hours. The reaction mixture was concentrated under a reduced pressure, to obtain the crude product of title Compound **27e** (134 mg), which was directly used in the next step of reaction without purification.
MS m/z (ESI): 410.2 [M+1]

### Step 5

### N-ethyl-5-fluoro-2-[1-methyl-5-(1-{[(1r,4r)-4-ethanesulfonamidocyclohexyl]methyl}pyrrolidin-3-yl)-1H-pyrazolo[4,3-b]pyridin-7-yl]-N-(isopropyl)benzamide 27

The crude product of Compound **27e** (134 mg) and Compound **2e**(103 mg, 327 µmol) were dissolved in *N,N-*dimethylacetamide (2.0 mL), followed by the addition of potassium carbonate (135 mg, 0.98 mmol) and potassium iodide (54.3 mg, 327 µmol), and the mixture was stirred at 80°C for 16 hours. The reaction mixture was cooled to room temperature, and concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% formic acid), elution gradient: 25%-50%), to obtain the title Compound **27** (25.3 mg, 41.29 µmol, yield: 19.03 %) as white solid.
MS m/z (ESI): 613.3 [M+1]
¹H NMR (400 MHz, DMSO-*d*₆) δ 8.15 (s, 1H), 8.11 (s, 0.49H), 7.57 (s, 1H), 7.39 (td, *J=* 8.8, 2.8 Hz, 1H), 7.33 (d, *J=* 8.8 Hz, 1H), 7.16 (s, 1H), 6.71 (d, *J =* 7.6 Hz, 1H), 3.92 (s, 0.3H), 3.73 - 3.55 (m, 4.7H), 3.11 - 2.98 (m, 6H), 2.95 (dd, *J* = 14.8, 7.2 Hz, 4H), 2.64 (s, 2H), 2.31 (s, 1H), 2.13 (s, 1H), 1.85 (dd, *J* = 30.8, 12.0 Hz, 4H), 1.47 (s, 1H), 1.31 - 1.18 (m, 5H), 1.06 - 0.72 (m, 8H), 0.56 (s, 2H).

### Example 29

### N-[(1r,4r)-4-{[3-(8-{2-[ethyl(isopropyl)aminoformyl]-4-fluorophenyl}-3-methylimidazo[1,5-a]pyridin-6-yl)pyrrolidin-1-yl]methyl}cyclohexyl]tert-butyl carbamate 29

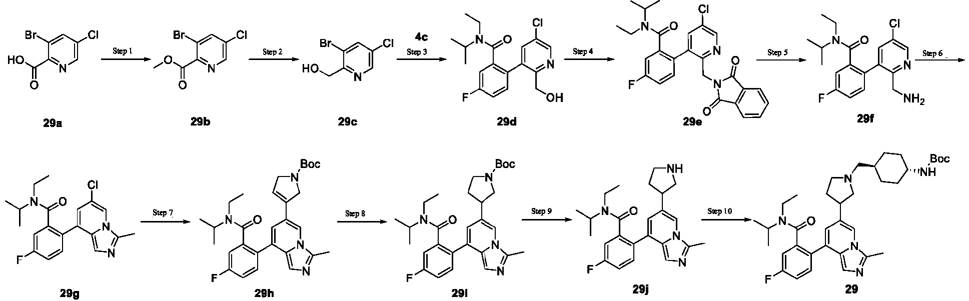

### Step 1

### 3-bromo-5-chloropyridin-2-methyl carboxylate 29b

3-Bromo-5-chloropyridin-2-carboxylic acid **29a** (20.0 g, 84.58 mmol) was dissolved in methanol (200 mL), and cooled to 0°C, followed by the addition of thionyl chloride (20.13 g, 169.17 mmol). The mixture was stirred at 0°C to allow for reacting for 5 min, warmed to 65°C and further stirred to allow for reacting for 12 hours. The reaction mixture was concentrated under a reduced pressure, and the residues was dissolved by adding dichloromethane (60 mL), and washed with a saturated aqueous sodium carbonate solution (40 mL x 2). The organic phases were combined, dried over anhydrous sodium sulfate, and concentrated under a reduced pressure, to obtain the crude product of title Compound **29b** (19.5 g, 77.85 mmol, yield: 92.04%) as white solid. The crude product was directly used in the next step of reaction without purification.
MS m/z (ESI): 252.0 [M+1]

### Step 2

### (3-bromo-5-chloropyridin-2-yl)methanol 29c

Compound **29b** (19.5 g, 77.85 mmol) was dissolved in methanol (200 mL) and tetrahydrofuran (200 mL), and cooled to 0°C, followed by the addition of sodium borohydride (15.0 g, 396.49 mmol). The mixture was stirred at room temperature to allow for reacting for 12.0 hour. The reaction mixture was cooled to 0°C, quenched by adding water (100 mL), and extracted with ethyl acetate (200 mL x 3). The organic phases were combined, dried over anhydrous sodium sulfate, and concentrated under a reduced pressure, to obtain the crude product of title Compound **29c** (16 g, 71.92 mmol, yield: 92.38%) as white solid. The crude product was directly used in the next step of reaction without purification.
MS m/z (ESI): 224.0 [M+1]

### Step 3

### 2-[5-chloro-2-(hydroxymethyl)pyridin-3-yl]-N-ethyl-5-fluoro-N-(isopropyl)benzamide 29d

Compound **29c** (6 g, 26.97 mmol), Compound **4c**(8.14 g, 24.27 mmol) and sodium carbonate (8.58 g, 80.91 mmol) were dissolved in dioxane (36 mL) and water (24 mL). Pd (dppf)Cl₂ (1.97 g, 2.70 mmol) was added. Under a nitrogen atmosphere, the mixture was stirred at 95°Cto allow for reacting for 20 hours. The reaction mixture was cooled to room temperature, followed by adding water (20 mL) and extracting with ethyl acetate (20 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **29d** (4.6 g, 13.11 mmol, yield: 48.62%).
MS m/z (ESI): 351.1 [M+1]

### Step 4

### 2-{5-chloro-2-[(1,3-dioxo-2,3-dihydro-1H-isoindol-2-yl)methyl]pyridin-3-yl}-Nethyl-5-fluoro-N-(isopropyl)benzamide 29e

Compound **29d** (4.6 g, 13.11 mmol), 2,3-dihydro-1*H*-isoindol-1,3-dione (2.32 g, 15.73 mmol) and triphenylphosphine (5.16 g, 19.67 mmol) were dissolved in tetrahydrofuran (37 mL), under a nitrogen atmosphere, and cooled to 0°C, followed by the addition of DIAD (3.98 g, 19.67 mmol). The mixture was stirred at room temperature to allow for reacting for 12 hour. Water (20 mL) was added to the reaction mixture, which was then extracted with ethyl acetate (20 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **29e** (4 g, 8.33 mmol, yield: 63.6%) as yellow solid.
MS m/z (ESI): 480.1 [M+1]

### Step 5

### 2-[2-(aminomethyl)-5-chloropyridin-3-yl]-N-ethyl-5-fluoro-N-(isopropyl)benzamide 29f

Compound **29e** (4 g, 8.33 mmol) was dissolved in the solution of methylamine in ethanol (71.92 g, 694.72 mmol, 80.00 mL, 30%). The mixture was stirred at 65°C to allow for reacting for 12 hours. The reaction mixture was cooled to room temperature, and concentrated under a reduced pressure, to obtain the crude product of title Compound **29f** (4.0 g) as yellow solid. The crude product was directly used in the next step of reaction without purification.
MS m/z (ESI): 350.3 [M+1]

### Step 6

### 2-{6-chloro-3-methylimidazo[1,5-a]pyridin-8-yl}-N-ethyl-5-fluoro-N-(isopropyl)benzamide 29g

The crude product of Compound **29f** (4.0 g) was dissolved in acetic anhydride (40 mL), followed by the addition of p-toluenesulfonic acid (1.60 g, 9.29 mmol). The mixture was stirred at 100°C to allow for reacting for 2.0 hours. The reaction mixture was cooled to room temperature, poured into water (60 mL), regulated to be alkaline with a 10% aqueous sodium hydroxide solution, and extracted with dichloromethane (100 mL x 3). The organic phases were combined, washed with saturated aqueous sodium carbonate solution (100 mL x 2), dried over anhydrous sodium sulfate, and concentrated under a reduced pressure. The resulting residues were purified by the silica-gel column chromatography using petroleum ether/ethyl acetate as an elution system, to obtain the title Compound **29g** (1.5 g, 4.01 mmol, yield: 35.1%).
MS m/z (ESI): 374.1 [M+1]

### Step 7

### 3-(8-{2-[ethyl(isopropyl)aminoformyl]-4-fluorophenyl}-3-methylimidazo[1,5-a]pyridin-6-yl)-2,5-dihydro-1H-pyrrol-1-tert-butyl formate 29h

Compound **29g** (1.5 g, 4.01 mmol), 3-(tetramethyl-1,3,2-dioxaboran-2-yl)-2,5-dihydro-1*H*-pyrrol-1-tert-butyl formate (1.77 g, 5.99 mmol) and cesium carbonate (4.00 g, 12.28 mmol) were dissolved in dioxane (15 mL) and water (5 mL). Pd(dtbpf)Cl₂ (261.50 mg, 401.23 µmol) was added. Under a nitrogen atmosphere, the mixture was stirred at 95°Cto allow for reacting for 12 hours. The reaction mixture was cooled to room temperature, followed by adding water (20 mL) and extracting with ethyl acetate (20 mL x 3). The organic phases were combined, washed with saturated brine (30 mL), dried over anhydrous sodium sulfate, and concentrated under a reduced pressure. The resulting residues were purified by the silica-gel column chromatography using petroleum ether/ethyl acetate as an elution system, to obtain the title Compound **29h** (1.8 g, 3.55 mmol, yield: 88.6%).
MS m/z (ESI): 507.3 [M+1]

### Step 8

### 3-(8-{2-[ethyl(isopropyl)aminoformyl]-4-fluorophenyl}-3-methylimidazo[1,5-a]pyridin-6-yl)pyrrolidin-1-tert-butyl formate 29i

Compound **29h** (600 mg, 1.18 mmol) was dissolved in methanol (10 mL), followed by the addition of Pd/C (143.8 mg, 1.18 mmol). Under a hydrogen atmosphere (15 Psi), the mixture was stirred at room temperature for 12 hours. The reaction mixture was filtered, and the filtrate was concentrated under a reduced pressure, to obtain the crude product of title Compound **29i** (400 mg, 786.4 µmol, yield: 66.4%). The crude product was directly used in the next step of reaction without purification.
MS m/z (ESI): 509.2 [M+1]

### Step 9

### N-ethyl-5-fluoro-2-[3-methyl-6-(pyrrolidin-3-yl)imidazo[1,5-a]pyridin-8-yl]-N-(isopropyl)benzamide 29j

Compound **29i** (400 mg, 786.4 µmol) was dissolved in dichloromethane (6 mL), followed by the addition of trifluoroacetic acid (2.37 g, 20.77 mmol, 1.60 mL) at 0°C. The mixture was stirred at room temperature to allow for reacting for 1.0 hours. The reaction mixture was concentrated under a reduced pressure, to obtain the crude product of title Compound **29j** (400 mg). The crude product was directly used in the next step of reaction without purification. MS m/z (ESI): 409.6 [M+1]

### Step 10

### N-[(1r,4r)-4-{[3-(8-{2-[ethyl(isopropyl)aminoformyl]-4-fluorophenyl}-3-methylimidazo[1,5-a]pyridin-6-yl)pyrrolidin-1-yl]methyl}cyclohexyl]tert-butyl carbamate 29

The crude product of Compound **29j** (70 mg, 134 µmol, trifluoroacetate), *N*-[(1*r*,4*r*)-4-formylcyclohexyl]tert-butyl carbamate (60.90 mg, 267.93 µmol) and triethylamine (67.78 mg, 669.81 µmol) were dissolved in methanol (3.0 mL), and stirred at room temperature for 0.5 hours. Sodium cyanoborohydride (12.63 mg, 200.94 µmol) was added, and the mixture was stirred at room temperature for 2.0 hours. The reaction mixture was diluted by adding dichloromethane (30 mL), followed by the addition of a saturated aqueous sodium carbonate solution (20 mL), and the mixture was then extracted with dichloromethane (20 mL x 3). The organic phases were combined, dried over anhydrous sodium sulfate, and concentrated under a reduced pressure. The resulting residues were purified by the preparative thin-layer chromatography using dichloromethane/methanol as an elution system, to obtain the title Compound **29** (12.1 mg, 19.52 µmol, yield: 14.57 %) as white solid.
MS m/z (ESI): 620.3 [M+1]
¹H NMR (400 MHz, DMSO-*d*₆) δ 8.09 - 7.96 (m, 1H), 7.72 - 7.59 (m, 1H), 7.47 - 7.22 (m, 2H), 7.05 (s, 1H), 6.82 - 6.59 (m, 2H), 3.13 - 2.80 (m, 7H), 2.65 - 2.57 (m, 2H), 2.36 - 2.24 (m, 1H), 2.17 - 2.00 (m, 1H), 1.85 - 1.72 (m, 3H), 1.65 - 1.48 (m, 2H), 1.37 (s, 9H), 1.27 - 1.10 (m, 7H), 1.08 - 0.97 (m, 3H), 0.92 - 0.66 (m, 4H), 0.58 - 0.48 (m, 1H), 0.37 - 0.17 (m, 3H).

### Example 30

### N-ethyl-5-fluoro-2-[3-methyl-6-(1-{[(1r,4r)-4-ethanesulfonamidocyclohexyl]methyl}pyrrolidin-3-yl)imidazo[1,5-a]pyridin-8-yl]-N-(isopropyl)benzamide 30

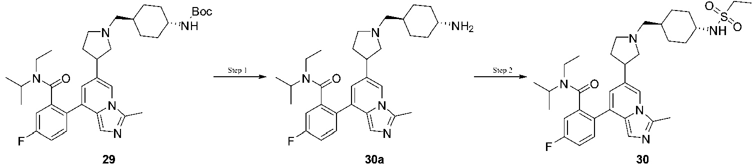

### Step 1

### N-ethyl-5-fuoro-2-[3-methyl-6-(1-{[(1r,4r)-4-aminocyclohexyl]methyl}pyrrolidin-3-yl)imidazo[1,5-a]pyridin-8-yl]-N-(isopropyl)benzamide 30a

Compound **29** (150 mg, 242 µmol) was dissolved in dichloromethane (3.0 mL), followed by the addition of trifluoroacetic acid (275.95 mg, 2.42 mmol, 186.45 µL) at 0°C. The mixture was stirred at room temperature to allow for reacting for 1.0 hours. The reaction mixture was concentrated under a reduced pressure, to obtain the crude product of title Compound **30a** (150 mg). The crude product was directly used in the next step of reaction without purification.
MS m/z (ESI): 520.5 [M+1]

### Step 2

### N-ethyl-5-fluoro-2-[3-methyl-6-(1-{[(1r,4r)-4-ethanesulfonamidocyclohexyl]methyl}pyrrolidin-3-yl)imidazo[1,5-a]pyridin-8-yl]-N-(isopropyl)benzamide 30

The crude product of Compound **30a** (150 mg) was dissolved in dichloromethane (3.0 mL), and cooled to 0°C, followed by the addition of ethanesulfonyl chloride (60.9 mg, 473.40 µmol) and triethylamine (119.76 mg, 1.18 mmol, 164.96 µL). The mixture was stirred at room temperature for 12 hours. Water (20 mL) was added to the reaction mixture. The mixture was extracted with ethyl acetate (20 mL x 2). The organic phases were combined, washed with saturated brine (20 mL), dried over anhydrous sodium sulfate, and concentrated under a reduced pressure. The resulting residues were purified by the HPLC (mobile phase: acetonitrile, water (0.1% ammonia water), elution gradient: 35%-65%), to obtain the title Compound **30** (23.96 mg, 39.16 µmol, yield: 16.55 %) as white solid.
MS m/z (ESI): 612.5 [M+1]
¹H NMR (400 MHz, DMSO-*d*₆) δ 7.92 (s, 1H), 7.69 - 7.60 (m, 1H), 7.40 - 7.26 (m, 2H), 7.03 - 6.96 (m, 2H), 6.68 - 6.61 (m, 1H), 3.44 - 3.39 (m, 1H), 3.30 - 3.23 (m, 2H), 3.10 - 2.71 (m, 6H), 2.66 - 2.55 (m, 5H), 2.42 - 2.32 (m, 1H), 2.29 - 2.12 (m, 3H), 1.92 - 1.68 (m, 5H), 1.38 - 1.01 (m, 7H), 0.98 - 0.70 (m, 7H), 0.34 - 0.25 (m, 2H).

### Example 31

### 2-(6-{1-[(1R,3S,4S)-2-azabicyclo[2.2.1]heptan-3-carbonyl]pyrrolidin-3-yl}-1-methyl-1H-indazol-4-yl)-N-ethyl-5-fluoro-N-(isopropyl)benzamide 31

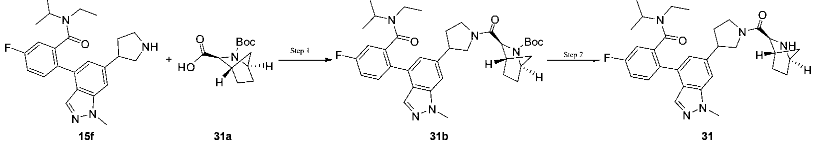

### Step 1

### (1R,3S,4S)-3-[3-(4-{2-[ethyl(isopropyl)aminoformyl]-4-fluorophenyl}-1-methyl-1H-indazol-6-yl)pyrrolidin-1-carbonyl]-2-azabicyclo[2.2.1]heptan-2-tert-butyl carboxylate 31b

Compound **15f** (31.9 mg, 78.1 µmol), (1*R*, 3*S*,4*S*)-2-[(tert-butoxy)carbonyl]-2-azabicyclo[2.2.1]heptan-3-carboxylic acid **31a(18.8** mg, 78.1 µmol) and *N,N-*diisopropylethylamine (89.6 mg, 693 µmol) were dissolved in DMF (1 mL), and cooled to 0°C. EDCI (19.4 mg, 101 µmol) and HOBt (12.6 mg, 93.7 µmol) were added, and the mixture was stirred at room temperature for 12 hours. Water (10 mL) was added to the reaction mixture, which was then extracted with ethyl acetate (10 mL x 3). The organic phases were combined, dried over anhydrous sodium sulfate, and concentrated under a reduced pressure. The resulting residues were purified by the preparative thin-layer chromatography using dichloromethane/methanol as an elution system, to obtain the title Compound **31b** (35 mg, 55.4 µmol, yield: 70.8%) as white solid.
MS m/z (ESI): 632.25 [M+1]

### Step 2

### 2-(6-{1-[(1R,3S,4S)-2-azabicyclo[2.2.1]heptan-3-carbonyl]pyrrolidin-3-yl}-1-methyl-1H-indazol-4-yl)-N-ethyl-5-fluoro-N-(isopropyl)benzamide 31

Compound **31b** (31.0 mg, 49.0 µmol) was dissolved in methane dioxide (0.9 mL) and trifluoroacetic acid (0.3 mL). The mixture was stirred at room temperature to allow for reacting for 1.0 hours. The reaction mixture was concentrated under a reduced pressure, and the resulting residues were purified by the HPLC (mobile phase: acetonitrile, water (0.1% formic acid), elution gradient: 40%-55%), to obtain the title Compound **31** (10.0 mg, 18.8 µmol, yield: 76.6%) as white solid.
MS m/z (ESI): 532.3 [M+1]
¹H NMR (400 MHz, DMSO-*d*₆) δ 7.84 (d, *J=* 4.0 Hz, 1H), 7.67 - 7.56 (m, 2H), 7.44 - 7.27 (m, 2H), 7.13 - 7.03 (m, 1H), 4.29 - 4.10 (m, 0.46H), 4.05 (d, *J=* 2.8 Hz, 4H), 3.93 (dt, *J=* 21.2, 12.8 Hz, 1H), 3.84 - 3.54 (m, 4.61H), 3.33 (ddd, *J* = 42.4, 12.8, 7.6 Hz, 3H), 2.89 - 2.69 (m, 2H), 2.33 (dd, *J =* 19.6, 17.6 Hz, 1H), 2.14 (ddd, *J=* 27.6, 18.8, 8.4 Hz, 1H), 1.67 - 1.32 (m, 6H), 1.09 - 0.52 (m, 7H), 0.08 (dd, *J =* 14.8, 6.8 Hz, 2H).

### Example 33

### 2-(6-{1-[(1R,3S,4S)-2-azabicyclo[2.2.1]heptan-3-carbonyl]-3-hydroxylazacyclobutan-3-yl}imidazo[1,5-a]pyridin-8-yl)-N-ethyl-5-fluoro-N-(isopropyl)benzamide 33

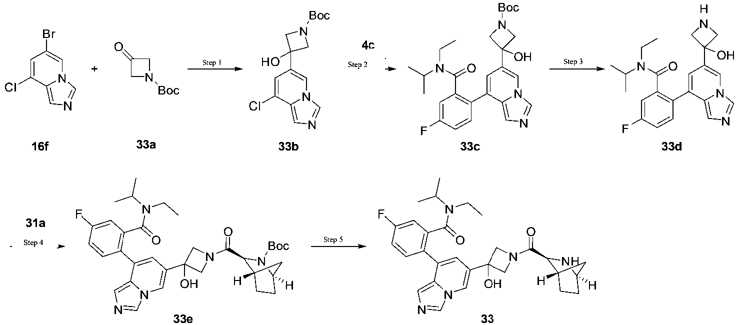

### Step 1

### 3-{8-chloroimidazo[1,5-a]pyridin-6-yl}-3-hydroxylazacyclobutan-1-tert-butyl carboxylate 33b

Compound **16f** (1.7 g, 7.34 mmol) was dissolved in tetrahydrofuran (20 mL), and cooled to 0°C, followed by the addition of the solution of *i*-PrMgCl.LiCl in tetrahydrofuran (1.3 M, 8.47 mL). The mixture was stirred at 0°C to allow for reacting for 10 min, and then, 3-oxoazacyclobutan-1-tert-butyl carboxylate (1.51 g, 8.81 mmol) was added. The mixture was stirred at room temperature to allow for reacting for 2.0 hour. The reaction mixture was quenched by adding a saturated aqueous ammonium chloride solution (30 mL), and extracted with ethyl acetate (50 mL x 3). The organic phases were combined, washed with saturated brine (50 mL), dried over anhydrous sodium sulfate, and concentrated under a reduced pressure. The resulting residues were purified by the silica-gel column chromatography using petroleum ether/ethyl acetate as an elution system, to obtain the title Compound **33b** (950 mg, 2.93 mmol, yield: 39.95%).
MS m/z (ESI): 324.0 [M+1]
¹H NMR (400 MHz, DMSO-*d*₆) δ 8.52 - 8.46 (m, 2H), 7.44 (s, 1H), 7.00 (d, J = 1.0 Hz, 1H), 6.60 (s, 1H), 4.16 - 3.96 (m, 4H), 1.41 (s, 9H).

### Step 2

### 3-(8-{2-[ethyl(isopropyl)aminoformyl]-4-fluorophenyl}imidazo[1,5-a]pyridin-6-yl)-3-hydroxylazacyclobutan-1-tert-butyl carboxylate 33c

Compound **33b** (300 mg, 926.57 µmol) and Compound **4c**(465.91 mg, 1.39 mmol) were dissolved in dioxane (5.0 mL) and water (1.0 mL). Potassium phosphate (590.04 mg, 2.78 mmol) and Pd(dtbpf)Cl₂ (60.39 mg, 92.66 µmol) were added, and the mixture was stirred at 90 °C under a nitrogen atmosphere to allow for reacting for 12.0 hours. The reaction mixture was cooled to room temperature, water (15 mL) was added, and the mixture was extracted with ethyl acetate (25 mL x 3). The organic phases were combined, washed with saturated brine (20 mL), dried over anhydrous sodium sulfate, and concentrated under a reduced pressure. The resulting residues were purified by the silica-gel column chromatography using petroleum ether/ethyl acetate as an elution system, to obtain the title Compound **33c** (230 mg, 463.18 µmol, yield: 49.99%).
MS m/z (ESI): 497.2 [M+1]
¹H NMR (400 MHz, DMSO-*d*₆) δ 8.50 (br d, J = 3.1 Hz, 2H), 7.70 (dd, J = 5.6, 8.6 Hz, 1H), 7.44 - 7.28 (m, 2H), 7.15 (s, 1H), 6.82 - 6.71 (m, 1H), 6.55 (s, 1H), 4.09 - 3.91 (m, 4H), 3.48 (td, J = 6.5, 13.2 Hz, 1H), 3.28 - 3.22 (m, 1H), 2.98 (br dd, J = 6.9, 13.4 Hz, 1H), 1.40 (s, 9H), 0.97 - 0.74 (m, 7H), 0.33 (br d, J = 6.4 Hz, 2H).

### Step 3

### N-ethyl-5-fluoro-2-[6-(3-hydroxylazacyclobutan-3-yl)imidazo[1,5-a]pyridin-8-yl]-N-(isopropyl) benzamide 33d

Compound **33c**(110 mg, 221.52 µmol) was dissolved in dichloromethane (3.0 mL), followed by the addition of trifluoroacetic acid (1.48 g, 12.98 mmol, 1 mL) at 0°C. The mixture was stirred at room temperature to allow for reacting for 0.5 hours. The reaction mixture was concentrated under a reduced pressure, to obtain the crude product of title Compound **33d** (110 mg). The crude product was directly used in the next step of reaction without purification. MS m/z (ESI): 397.2 [M+1]

### Step 4

### (1R,3S,4S)-3-[3-(8-{2-[ethyl(isopropyl)aminoformyl]- 4-fluorophenyl}imidazo[1,5-a]pyridin-6-yl)-3-hydroxylazacyclobutan-1-carbonyl]-2-azabicyclo[2.2.1]heptan-2-tert-butyl carboxylate 33e

Compound **31a** (57 mg, 236.24 µmol), *N,N*-diisopropylethylamine (90 mg, 696.36 µmol), EDCI(62 mg, 323.42 µmol) and HOBt(44 mg, 325.63 µmol) were dissolved in dichloromethane (2 mL), followed by the addition of the crude product of Compound **33d** (110 mg), and the mixture was stirred at room temperature for 12 hours. A saturated aqueous sodium bicarbonate solution (10 mL) was added to the reaction mixture, which was then extracted with dichloromethane (20 mL x 3). The organic phases were combined, washed with saturated brine (20 mL), dried over anhydrous sodium sulfate, and concentrated under a reduced pressure. The resulting residues were purified by the HPLC (mobile phase: acetonitrile, water (0.1% formic acid), elution gradient:22%-52%), to obtain the title Compound **33e** (100 mg, 161.4 µmol, yield: 75.6%) as white solid.
MS m/z (ESI): 620.3 [M+1]

### Step 5

### 2-(6-{1-[(1R,3S,4S)-2-azabicyclo[2.2.1]heptan-3-carbonyl]-3-hydroxylazacyclobutan-3-yl}imidazo[1,5-a]pyridin-8-yl)-N-ethyl-5-fluoro-N-(isopropyl)benzamide 33

Compound **33e** (100 mg, 161.36 µmol) was dissolved in methane dioxide (3.0 mL) and trifluoroacetic acid (1.0 mL). The mixture was stirred at room temperature to allow for reacting for 1.0 hours. The reaction mixture was concentrated under a reduced pressure, and the resulting residues were purified by the HPLC (mobile phase: acetonitrile, water (0.1%ammonium bicarbonate), elution gradient: 15%-45%), to obtain the title Compound **33** (32.76 mg, 63.05 µmol, yield: 39.07 %).
MS m/z (ESI): 520.0 [M+1]
¹H NMR (400 MHz, DMSO-*d*₆) δ 8.54 - 8.42 (m, 2H), 7.76 - 7.65 (m, 1H), 7.44 - 7.28 (m, 2H), 7.21 - 7.11 (m, 1H), 6.86 - 6.57 (m, 2H), 4.64 - 3.89 (m, 5H), 3.50 - 3.43 (m, 1H), 3.28 - 3.16 (m, 2H), 3.10 - 2.88 (m, 1H), 1.76 - 0.63 (m, 14H), 0.35 - 0.25 (m, 2H).

### Example 34

### 2-(6-{ 1-[(1R,3S,4S)-2-azabicyclo[2.2.1]heptan-3-carbonyl]-3-fluoroazacyclobutan-3-yl}imidazo[1,5-a]pyridin-8-yl)-N-ethyl-5-fluoro-N-(isopropyl)benzamide 34

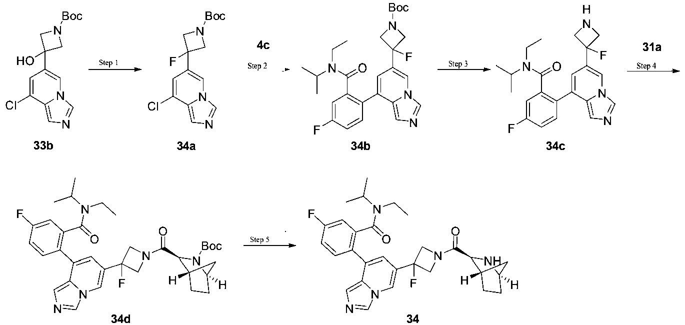

### Step 1

### 3-{8-chloroimidazo[1,5-a]pyridin-6-yl}-3-fluoroazacyclobutan-1-tert-butyl carboxylate 34a

Compound **33b** (300 mg, 926.57 µmol) was dissolved in dichloromethane (5.0 mL), and cooled to -60°C, followed by the dropwise addition of DAST(298.71 mg, 1.85 mmol, 0.5 mL). The mixture was stirred -60°C to allow for reacting for 1.0 hour, and then slowly warmed to room temperature, at which the mixture was stirred to allow for reacting for 1.0 hour. The reaction mixture was quenched by adding a saturated aqueous sodium bicarbonate solution (20 mL), and extracted with dichloromethane (30 mL x 3). The organic phases were combined, washed with saturated brine (30 mL), dried over anhydrous sodium sulfate, and concentrated under a reduced pressure. The resulting residues were purified by the silica-gel column chromatography using petroleum ether/ethyl acetate as an elution system, to obtain the title Compound **34a** (235 mg, 721.4 µmol, yield: 77.9%) as brown solid.
MS m/z (ESI): 326.0 [M+1]

### Step 2

### 3-(8-{2-[ethyl(isopropyl)aminoformyl]-4-fluorophenyl}imidazo[1,5-a]pyridin-6-yl)-3-fluoroazacyclobutan-1-tert-butyl carboxylate 34b

Compound **34a** (235 mg, 721.38 µmol) and Compound **4c**(485 mg, 1.45 mmol) were dissolved in dioxane (3.0 mL) and water (0.5 mL). Potassium phosphate (460 mg, 2.17 mmol) and Pd(dtbpf)Cl₂ (48 mg, 73.65 µmol) were added, and the mixture was stirred at 90 °C under a nitrogen atmosphere to allow for reacting for 12.0 hours. The reaction mixture was cooled to room temperature, water (15 mL) was added, and the mixture was extracted with ethyl acetate (25 mL x 3). The organic phases were combined, washed with saturated brine (20 mL), dried over anhydrous sodium sulfate, and concentrated under a reduced pressure. The resulting residues were purified by the silica-gel column chromatography using petroleum ether/ethyl acetate as an elution system, to obtain the title Compound **34b** (240 mg, 481.38 µmol, yield: 66.7 %).
MS m/z (ESI): 499.3 [M+1]
¹H NMR (400 MHz, DMSO-*d*₆) δ 8.68 (s, 1H), 8.46 (s, 1H), 7.70 (dd, J = 5.5, 8.6 Hz, 1H), 7.45 - 7.31 (m, 2H), 7.24 - 7.19 (m, 1H), 6.74 - 6.67 (m, 1H), 4.41 - 4.22 (m, 4H), 3.51 - 3.44 (m, 1H), 3.34 - 3.26 (m, 1H), 2.91 (br dd, J = 6.9, 13.6 Hz, 1H), 1.40 (s, 9H), 1.00 - 0.68 (m, 7H), 0.33 (br d, J = 6.6 Hz, 2H).

### Step 3

### N-ethyl-5-fluoro-2-[6-(3-fluoroazacyclobutan-3-yl)imidazo[1,5-a]pyridin-8-yl]-N-(isopropyl)benzamide 34c

Compound **34b** (230 mg, 461.33 µmol) was dissolved in dichloromethane (3.0 mL), followed by the addition of trifluoroacetic acid (2.22 g, 19.47 mmol, 1.5 mL) at 0°C. The mixture was stirred at room temperature to allow for reacting for 1.0 hours. The reaction mixture was concentrated under a reduced pressure, to obtain the crude product of title Compound **34c** (200 mg). The crude product was directly used in the next step of reaction without purification. MS m/z (ESI): 399.1 [M+1]

### Step 4

### (1R,3S,4S)-3-[3-(8- {2-[ethyl(isopropyl)aminoformyl]- 4-fluorophenyl}imidazo[1,5-a]pyridin-6-yl)-3-fluoroazacyclobutan-1-carbonyl]-2-azabicyclo[2.2.1]heptan-2-tert-butyl carboxylate 34d

Compound **31a** (57 mg, 236.24 µmol), *N,N*-diisopropylethylamine (85 mg, 657.69 µmol), EDCI(62 mg, 323.42 µmol) and HOBt(41 mg, 303.43 µmol) were dissolved in dichloromethane (3 mL), followed by the addition of the crude product of Compound **34c** (100 mg), and the mixture was stirred at room temperature for 12 hours. A saturated aqueous sodium bicarbonate solution (10 mL) was added to the reaction mixture, which was then extracted with dichloromethane (20 mL x 3). The organic phases were combined, washed with saturated brine (20 mL), dried over anhydrous sodium sulfate, and concentrated under a reduced pressure. The resulting residues were purified by the preparative thin-layer chromatography using dichloromethane/methanol as an elution system, to obtain the title Compound **34d** (100 mg, 160.85 µmol, yield: 82.43%) as white solid.
MS m/z (ESI): 622.4 [M+1]

### Step 5

### 2-(6-{1-[(1R,3S,4S)-2-azabicyclo[2.2.1]heptan-3-carbonyl]-3-fluoroazacyclobutan-3-yl}imidazo[1,5-a]pyridin-8-yl)-N-ethyl-5-fluoro-N-(isopropyl)benzamide 34

Compound **34d** (100 mg, 160.85 µmol) was dissolved in methane dioxide (3.0 mL) and trifluoroacetic acid (1.5 mL). The mixture was stirred at room temperature to allow for reacting for 1.0 hours. The reaction mixture was concentrated under a reduced pressure, and the resulting residues were purified by the HPLC (mobile phase: acetonitrile, water (0.1% ammonia water), elution gradient: 25%-55%), to obtain the title Compound **34** (45.77 mg, 87.75 µmol, yield: 54.56 %).
MS m/z (ESI): 522.2 [M+1]
¹H NMR (400 MHz, DMSO-*d*₆) δ 8.76 - 8.64 (m, 1H), 8.46 (s, 1H), 7.77 - 7.65 (m, 1H), 7.46 - 7.30 (m, 2H), 7.28 - 7.18 (m, 1H), 6.79 - 6.70 (m, 1H), 4.87 - 4.54 (m, 2H), 4.47 - 4.16 (m, 2H), 3.51 - 3.39 (m, 2H), 3.25 - 3.22 (m, 1H), 3.02 - 2.62 (m, 2H), 1.57 - 0.65 (m, 14H), 0.36 - 0.30 (m, 2H).

### Example 35

### N-ethyl-5-fluoro-2-[3-methyl-7-(1-{[(1r,4r)-4-ethanesulfonamidocyclohexyl]methyl}pyrrolidin-3-yl)imidazo[1,5-a]pyridin-5-yl]-N-(isopropyl)benzamide 35

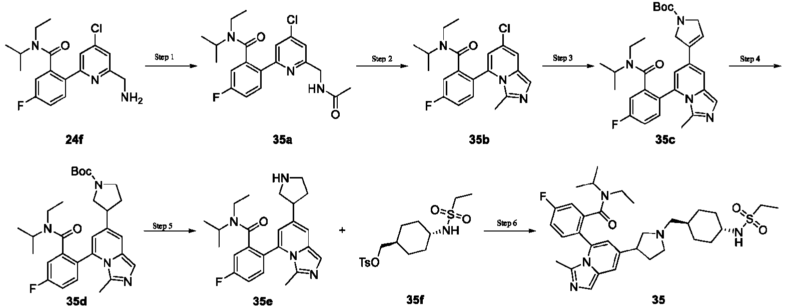

### Step 1

### 2-[4-chloro-6-(acetamidomethyl)pyridin-2-yl]-N-ethyl-5-fluoro-N-(isopropyl)benzamide 35a

Compound **24f** (4.1 g, 11.72 mmol) and triethylamine (3.56 g, 35.16 mmol) were dissolved in dichloromethane (50 mL), followed by the dropwise addition of acetyl chloride (1.38 g, 17.58 mmol, 1.07 mL). The mixture was stirred at room temperature to allow for reacting for 1.0 hour. Water (50 mL) was added to the reaction mixture, which was then extracted with ethyl acetate (40 mL x 3). The organic phases were combined, washed with saturated brine (30 mL x 2), dried over anhydrous sodium sulfate, and concentrated under a reduced pressure, to obtain the crude product of title Compound **35a** (5.4 g). The crude product was directly used in the next step of reaction without purification. MS m/z (ESI): 392.2 [M+1]

### Step 2

### 2-{7-chloro-3-methylimidazo[1,5-a]pyridin-5-yl}-N-ethyl-5-fluoro-N-(isopropyl)benzamide 35b

The crude product of Compound **35a** (5.5 g) was dissolved in toluene (40 mL), followed by the addition of phosphorus oxychloride (10.76 g, 70.18 mmol, 8 mL). The mixture was stirred at 100°C to allow for reacting for 1.0 hours. The reaction mixture was cooled to room temperature, and concentrated under a reduced pressure. A saturated aqueous sodium bicarbonate solution (100 mL) was added to the reaction mixture, which was then extracted with dichloromethane (80 mL x 3). The organic phases were combined, washed with saturated brine (50 mL x 2), dried over anhydrous sodium sulfate, and concentrated under a reduced pressure. The resulting residues were purified by the silica-gel column chromatography using petroleum ether/ethyl acetate as an elution system, to obtain the title Compound **35b** (1.4 g, 3.74 mmol, yield: 26.7%) as yellow solid.
MS m/z (ESI): 374.1[M+1]
¹H NMR (400 MHz, DMSO-*d*₆) δ 7.77 - 7.64 (m, 2H), 7.47 - 7.37 (m, 2H), 7.27 (br d, J = 2.4 Hz, 1H), 6.44 - 6.36 (m, 1H), 5.75 (s, 1H), 3.81 - 3.69 (m, 1H), 3.04 - 2.90 (m, 1H), 1.99 - 1.92 (m, 3H), 1.13 - 1.03 (m, 5H), 0.91 (d, J = 6.8 Hz, 1H), 0.67 (br t, J = 6.8 Hz, 3H).

### Step 3

### 3-(5-{2-[ethyl(isopropyl)aminoformyl]-4-fluorophenyl}-3-methylimidazo[1,5-a]pyridin-7-yl)-2,5-dihydro-1H-pyrrol-1-tert-butyl formate 35c

Compound **35b** (900 mg, 2.41 mmol), 3-(tetramethyl-1,3,2-dioxaboran-2-yl)-2,5-dihydro-1*H*-pyrrol-1-tert-butyl formate (852.74 mg, 2.89 mmol) and potassium phosphate (1.28 g, 6.04 mmol) were dissolved in dioxane (15 mL) and water (1.8 mL). Pd(dtbpf)Cl₂ (161.61 mg, 247.96 µmol) was added. Under a nitrogen atmosphere, the mixture was stirred at 80°Cto allow for reacting for 12 hours. The reaction mixture was cooled to room temperature, water (30 mL) was added, and the mixture was extracted with ethyl acetate (20 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **35c** (740 mg, 1.46 mmol, yield: 60.7%).
MS m/z (ESI): 507.3 [M+1]
¹H NMR (400 MHz, DMSO-*d*₆) δ 7.67 (br d, J = 6.1 Hz, 1H), 7.47 - 7.28 (m, 4H), 6.75 - 6.65 (m, 1H), 6.33 (br s, 1H), 4.38 (br d, J = 4.5 Hz, 2H), 4.18 (br d, J = 4.4 Hz, 2H), 3.78 (br d, J = 1.3 Hz, 1H), 3.20 (br s, 1H), 2.99 - 2.85 (m, 1H), 2.02 - 1.93 (m, 3H), 1.44 (br d, J = 9.4 Hz, 9H), 1.11 - 0.95 (m, 6H), 0.86 - 0.79 (m, 1H), 0.60 (br t, J = 6.6 Hz, 2H).

### Step 4

### 3-(5-{2-[ethyl(isopropyl)aminoformyl]-4-fluorophenyl}-3-methylimidazo[1,5-a]pyridin-7-yl)pyrrolidin-1-tert-butyl formate 35d

Compound **35c** (140 mg, 276.35 µmol) was dissolved in methanol (10 mL), followed by the addition of 10% Pd/C (29.41 mg, 276.35 µmol). Under a hydrogen atmosphere (15 Psi), the mixture was stirred at room temperature for 12 hours. The reaction mixture was filtered, and the filtrate was concentrated under a reduced pressure, to obtain the crude product of title Compound **35d** (130 mg, 255.6 µmol, yield: 92.5%). The crude product was directly used in the next step of reaction without purification.
MS m/z (ESI): 509.2 [M+1]

### Step 5

### N-ethyl-5-fluoro-2-[3-methyl-7-(pyrrolidin-3-yl)imidazo[1,5-a]pyridin-5-yl]-N-(isopropyl)benzamide 35e

Compound **35d** (130 mg, 255.6 µmol) was dissolved in methane dioxide (3.0 mL), followed by the addition of trifluoroacetic acid (1.48 g, 12.98 mmol, 1 mL) at 0°C. The mixture was stirred at room temperature to allow for reacting for 1.0 hours. The reaction mixture was concentrated under a reduced pressure, to obtain the crude product of title Compound **35e** (110 mg). The crude product was directly used in the next step of reaction without purification. MS m/z (ESI): 409.3 [M+1]

### Step 6

### N-ethyl-5-fluoro-2-[3-methyl-7-(1-{[(1r,4r)-4-ethanesulfonamidocyclohexylmethyl}pyrrolidin-3-yl)imidazo[1,5-a]pyridin-5-yl]-N-(isopropyl)benzamide 35

The crude product of Compound **35e** (50.0 mg, 95.69 µmol, trifluoroacetate) was dissolved in *N,N-*dimethylformamide (1 mL), followed by the addition of [(1r,4r)-4-ethanesulfonamidocyclohexyl]methyl 4-methylphenyl-1-sulfonate **35**f(50 mg, 133.15 µmol), potassium carbonate (26.45 mg, 191.38 µmol) and TBAI(3.53 mg, 9.57 µmol), and the mixture was stirred at 90°C for 12 hours. A saturated aqueous sodium bicarbonate solution (10 mL) was added to the reaction mixture, which was then extracted with ethyl acetate (20 mL x 3). The organic phases were combined, washed with saturated brine (20 mL), dried over anhydrous sodium sulfate, and concentrated under a reduced pressure, and the resulting residues were purified by the preparative thin-layer chromatography using dichloromethane/methanol as an elution system, to obtain the title Compound **35** (2.88 mg, 4.71 µmol, yield: 4.92%). MS m/z (ESI): 612.3 [M+1]
¹H NMR (400 MHz, CD₃OD) δ 7.70 - 7.51 (m, 1H), 7.45 - 7.14 (m, 4H), 6.67 - 6.34 (m, 1H), 4.05 - 3.62 (m, 1H), 3.27 - 3.21 (m, 1H), 3.15 - 2.99 (m, 4H), 2.93 - 2.57 (m, 3H), 2.49 - 2.24 (m, 3H), 2.18 - 2.07 (m, 2H), 2.05 - 1.97 (m, 2H), 1.95 - 1.82 (m, 3H), 1.74 - 1.60 (m, 1H), 1.48 - 1.25 (m, 8H), 1.22 - 0.98 (m, 9H), 0.90 - 0.81 (m, 2H).

### Example 36

### N-ethyl-5-fluoro-2-{1-methyl-6-[4-({[(1r,4r)-4-(2-oxo-1,3-oxazolidin-3-yl)cyclohexyl]methyl}amino)piperidin-1-yl]-1H-indazol-4-yl}-N-(isopropyl)benzamide 36

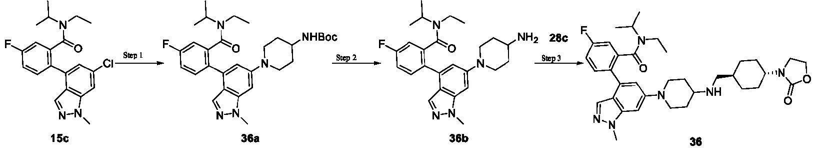

### Step 1

### N-[1-(4-{2-[ethyl(isopropyl)aminoformyl]-4-fluorophenyl}-1-methyl-1H-indazol-6-yl)piperidin-4-yl]tert-butyl carbamate 36a

Compound **15c** (500 mg, 936 µmol) and *N*-(piperidin-4-yl)tert-butyl carbamate (187 mg, 936 µmol) were dissolved in toluene (10.5 mL) and tertiary butanol (3.5 mL). Pd(Oac)₂ (21.0 mg, 93.6 µmol), Xphos(89.3 mg, 187 µmol) and sodium tert-butoxide (89.3 mg, 187 µmol) were added, and the mixture was stirred at 120°C under a nitrogen atmosphere to allow for reacting for 8.0 hours. The reaction mixture was cooled to room temperature, and concentrated under a reduced pressure. The resulting residues were purified by the preparative thin-layer chromatography using petroleum ether/ethyl acetate as an elution system, to obtain the title Compound **36a** (454 mg, 844.4 µmol, yield: 90.2%) as white solid.
MS m/z (ESI): 538.2 [M+1]
¹H NMR (400 MHz, DMSO-*d*₆) δ 7.66 (d, J = 5.2 Hz, 1H), 7.59 (ddd, J = 8.4, 5.6, 2.8 Hz, 1H), 7.36 (td, J = 8.4, 2.8 Hz, 1H), 7.28 (td, J = 9.6, 2.8 Hz, 1H), 6.94 - 6.80 (m, 3H), 3.95 (d, J = 3.6 Hz, 3H), 3.71 (d, J = 3.2 Hz, 2H), 3.38 (dd, J = 13.2, 6.4 Hz, 2H), 3.27 (dt, J = 13.6, 6.8 Hz, 1H), 2.83 (dt, J = 24.8, 9.2 Hz, 3H), 1.83 (d, J = 11.2 Hz, 2H), 1.57 - 1.44 (m, 2H), 1.39 (s, 9H), 0.85 (d, J = 6.4 Hz, 3H), 0.70 (dt, J = 33.2, 7.2 Hz, 4H), 0.13 (d, J = 6.4 Hz, 2H).

### Step 2

### 2-[6-(4-aminopiperidin-1-yl)-1-methyl-1H-indazol-4-yl]-N-ethyl-5-fluoro-N-(isopropyl)benzamide 36b

Compound **36a** (200 mg, 371.98 µmol) was dissolved in methane dioxide (2.0 mL), followed by the addition of trifluoroacetic acid (0.8 mL) at 0°C. The mixture was stirred at room temperature to allow for reacting for 1.0 hours. The reaction mixture was concentrated under a reduced pressure, to obtain the crude product of title Compound **36b** (430 mg), which was directly used in the next step of reaction without purification.
MS m/z (ESI): 438.1 [M+1]

### Step 3

### N-ethyl-5-fluoro-2-{1-methyl-6-[4-({[(1r,4r)-4-(2-oxo-1,3-oxazolidin-3-yl)cyclohexyl]methyl}amino)piperidin-1-yl]-1H-indazol-4-yl}-N-(isopropyl)benzamide 36

The crude product of Compound **36b** (150 mg) was dissolved in dichloromethane (2.0 mL), followed by the addition of Compound **28c** (25.2 mg, 128 µmol), and the mixture was stirred at 0°C for 0.5 hours. Sodium triacetoxyborohydride (81.3 mg, 383 µmol) was added, and the mixture was stirred at room temperature for 16 hours. The reaction mixture was concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% formic acid), elution gradient: 30%-45%), to obtain the title Compound **36** (19.5 mg, 31.5 µmol, yield: 45.4 %) as white solid.
MS m/z (ESI): 619.25 [M+1]
¹H NMR (400 MHz, DMSO-*d*₆) δ 8.29 (s, 1H), 7.69 - 7.64 (m, 1H), 7.62 - 7.56 (m, 1H), 7.37 (td, J = 8.8, 2.8 Hz, 1H), 7.32 - 7.24 (m, 1H), 6.92 (s, 1H), 6.87 - 6.83 (m, 1H), 4.27 - 4.20 (m, 2H), 3.97 - 3.94 (m, 3H), 3.73 (s, 2H), 3.51 - 3.42 (m, 3H), 3.41 - 3.34 (m, 1H), 3.28 (dd, J = 13.6, 6.8 Hz, 1H), 2.88 - 2.70 (m, 4H), 2.55 (d, J = 6.4 Hz, 2H), 1.92 (dd, J = 40.0, 11.6 Hz, 4H), 1.68 (d, J = 10.4 Hz, 2H), 1.43 (dd, J = 22.0, 12.0 Hz, 5H), 1.09 - 0.63 (m, 9H), 0.12 (d, J = 6.8 Hz, 2H).

### Example 38

### 2-(6-{1-[(1R,3S,4S)-2-azabicyclo[2.2.1]heptan-3-carbonyl]pyrrolidin-3-yl}imidazo[1,5-a]pyridin-8-yl)-N-ethyl-5-fluoro-N-(isopropyl)benzamide 38

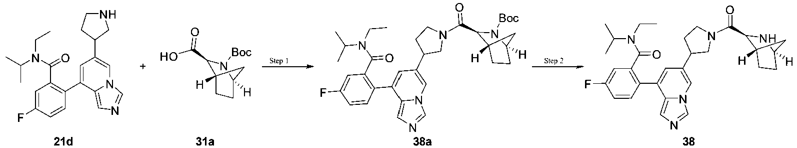

### Step 1

### (1R,3S,4S)-3-[3-(8-{2-[ethyl(isopropyl)aminoformyl]-4-fluorophenyl}imidazo[1,5-a]pyridin-6-yl)pyrrolidin-1-carbonyl]-2-azabicyclo[2.2.1]heptan-2-tert-butyl carboxylate 38a

Compound **31a** (54 mg, 223.80 µmol) and *N,N*-diisopropylethylamine (80 mg, 619 µmol) were dissolved in dichloromethane (2 mL), and cooled to 0°C, followed by the addition of EDCI (58 mg, 302.55 µmol) and HOBt (41 mg, 303.4 µmol). Compound **21d** (102 mg, 200.6 µmol, trifluoroacetate) was then added, and the mixture was stirred at room temperature for 12 hours. A saturated aqueous sodium bicarbonate solution (10 mL) was added to the reaction mixture, which was then extracted with dichloromethane (20 mL x 3). The organic phases were combined, washed with saturated brine (20 mL), dried over anhydrous sodium sulfate, and concentrated under a reduced pressure. The residues were purified by the preparative thin-layer chromatography using dichloromethane/methanol as an elution system, to obtain the title Compound **38a** (80 mg, 129.5 µmol, yield: 64.6%).
MS m/z (ESI): 618.4 [M+1]

### Step 2

### 2-(6-{1-[(1R,3S,4S)-2-azabicyclo[2.2.1]heptan-3-carbonyl]pyrrolidin-3-yl}imidazo[1,5-a]pyridin-8-yl)-N-ethyl-5-fluoro-N-(isopropyl)benzamide 38

Compound **38a** (80 mg, 129.5 µmol) was dissolved in methane dioxide (3.0 mL) and trifluoroacetic acid (1.0 mL). The mixture was stirred at room temperature to allow for reacting for 1.0 hours. The reaction mixture was concentrated under a reduced pressure, and the resulting residues were purified by the HPLC (mobile phase: acetonitrile, water (0.1% ammonia water), elution gradient: 20%-50%), to obtain the title Compound **38** (17.7 mg, 34.2 µmol, yield: 26.4 %).
MS m/z (ESI): 518.3 [M+1]
¹H NMR (400 MHz, DMSO-*d*₆) δ 8.45 - 8.35 (m, 1H), 8.34 - 8.26 (m, 1H), 7.74 - 7.64 (m, 1H), 7.44 - 7.28 (m, 2H), 7.17 - 7.09 (m, 1H), 6.77 - 6.62 (m, 1H), 4.29 - 3.55 (m, 3H), 3.54 - 3.40 (m, 2H), 3.28 - 3.18 (m, 3H), 3.01 - 2.66 (m, 2H), 2.42 - 1.77 (m, 3H), 1.56 - 0.68 (m, 14H), 0.30 - 0.22 (m, 2H).

### Example 39

### N-ethyl-5-fluoro-2-(6-{1-[(1S,3S,4R)-5-methylene-2-azabicyclo[2.2.2]octan-3-carbonyl]piperidin-4-yl}imidazo[1,5-a]pyridin-8-yl)-N-(isopropyl)benzamide 39

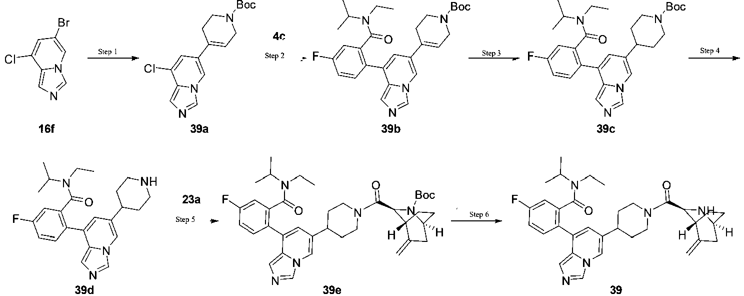

### Step 1

### 4-{8-chloroimidazo[1,5-a]pyridin-6-yl}-1,2,3,6-tetrahydropyridin-1-tert-butyl carboxylate 39a

Compound **16f** (500 mg, 2.16 mmol), 4-(tetramethyl-1,3,2-dioxaboran-2-yl)- 1,2,3,6-tetrahydropyridin-1-tert-butyl carboxylate (560 mg, 1.81 mmol) and sodium carbonate (686.8 mg, 6.48 mmol) were dissolved in dioxane (10 mL) and water (2 mL). Pd (dppf)Cl₂ (158 mg, 216 µmol) was added. Under a nitrogen atmosphere, the mixture was stirred at 90°Cto allow for reacting for 12 hours. The reaction mixture was cooled to room temperature, followed by adding water (20 mL) and extracting with ethyl acetate (20 mL x 3). The organic phases were combined, washed with saturated brine (40 mL), dried over anhydrous sodium sulfate, and concentrated under a reduced pressure. The resulting residues were purified by the silica-gel column chromatography using petroleum ether/ethyl acetate as an elution system, to obtain the title Compound **39a** (400 mg, 1.20 mmol, yield: 55.5%).
MS m/z (ESI): 334.1 [M+1]

### Step 2

### 4-(8-{2-[ethyl(isopropyl)aminoformyl]-4-fluorophenyl}imidazo[1,5-a]pyridin-6-yl)-1,2,3,6-tetrahydropyridin-1-tert-butyl formate 39b

Compound **39a** (400 mg, 1.20 mmol) and Compound **4c** (600 mg, 1.79 mmol) were dissolved in dioxane (12 mL) and water (4.0 mL). Cesium carbonate (1.17 g, 3.59 mmol) and Pd(dtbpf)Cl₂ (78.1 mg, 119.8 µmol) were added, and the mixture was stirred at 95 °C under a nitrogen atmosphere to allow for reacting for 12 hours. The reaction mixture was cooled to room temperature, water (20 mL) was added, and the mixture was extracted with ethyl acetate (30 mL x 3). The organic phases were combined, washed with saturated brine (30 mL), dried over anhydrous sodium sulfate, and concentrated under a reduced pressure. The resulting residues were purified by the silica-gel column chromatography using petroleum ether/ethyl acetate as an elution system, to obtain the title Compound **39b** (400 mg, 789.6 µmol, yield: 65.9%).
MS m/z (ESI): 507.3 [M+1]

### Step 3

### 4-(8-{2-[ethyl(isopropyl)aminoformyl]-4-fluorophenyllimidazo[1,5-a]pyridin-6-yl)piperidin-1-tert-butyl formate 39c

Compound **39b** (400 mg, 789.6 µmol) was dissolved in methanol (20 mL), followed by the addition of Pd/C (96 mg, 790 µmol). Under a hydrogen atmosphere (15 Psi), the mixture was stirred at room temperature for 12 hours. The reaction mixture was filtered, and the filtrate was concentrated under a reduced pressure, to obtain the crude product of title Compound **39c** (300 mg). The crude product was directly used in the next step of reaction without purification. MS m/z (ESI): 509.5 [M+1]

### Step 4

### N-ethyl-5-fluoro-2-[6-(piperidin-4-yl)imidazo[1,5-a]pyridin-8-yl]-N-(isopropyl)benzamide 39d

Compound **39c** (300 mg, 589.8 µmol) was dissolved in dichloromethane (4 mL), followed by the addition of trifluoroacetic acid (134.50 mg, 1.18 mmol, 90.88 µL) at 0°C. The mixture was stirred at room temperature to allow for reacting for 12 hours. The reaction mixture was concentrated under a reduced pressure, to obtain the crude product of title Compound **39d** (230 mg, 563.02 µmol, yield: 95.5%). The crude product was directly used in the next step of reaction without purification.
MS m/z (ESI): 409.2 [M+1]

### Step 5

### (1S,3S,4R)-3-[4-(8-{2-[ethyl(isopropyl)aminoformyl]-4-fluorophenyl}imidazo[1,5-a]pyridin-6-yl)piperidin-1-carbonyl]-5-methylene-2-azabicyclo[2.2.2]octan-2-tert-butyl carboxylate 39e

Compound **39d** (100.0 mg, 244.8 µmol), (1*S*,3*S*,4*R*)-2-tert-butoxycarbonyl-5-methylene-2-azabicyclo[2.2.2]octan-3-carboxylic acid **23a** (40.00 mg, 149.63 µmol) and *N,N*-diisopropylethylamine (126.55 mg, 979.17 µmol) were dissolved in dichloromethane (4 mL), and cooled to 0°C. EDCI (70.39 mg, 367.19 µmol) and HOBt (56.23 mg, 367.19 µmol) were added, and the mixture was stirred at room temperature for 12 hours. Water (10 mL) was added to the reaction mixture, which was then extracted with ethyl acetate (20 mL x 2). The organic phases were combined, dried over anhydrous sodium sulfate, and concentrated under a reduced pressure. The resulting residues were purified by the preparative thin-layer chromatography using dichloromethane/methanol as an elution system, to obtain the title Compound **39e** (50 mg, 76.01 µmol, yield: 31.05 %) as white solid.
MS m/z (ESI): 658.4 [M+1]

### Step 6

### N-ethyl-5-fluoro-2-(6-{1-[(1S,3S,4R)-5-methylene-2-azabicyclo[2.2.2]octan-3-carbonyl]piperidin-4-yl}imidazo[1,5-a]pyridin-8-yl)-N-(isopropyl)benzamide 39

Compound **39e** (50 mg, 76.01 µmol) was dissolved in methane dioxide (2.0 mL) and trifluoroacetic acid (8.67 mg, 76.01 µmol, 5.86 µL). The mixture was stirred at room temperature to allow for reacting for 0.5 hours. The reaction mixture was concentrated under a reduced pressure, and the resulting residues were purified by the HPLC (mobile phase: acetonitrile, water (0.1% ammonia water), elution gradient: 32%-62%), to obtain the title Compound **39** (11.53 mg, 20.67 µmol, yield: 27.2 %).
MS m/z (ESI): 558.3 [M+1]
¹H NMR (400 MHz, DMSO-*d*₆) δ 8.36 (br d, J = 7.9 Hz, 1H), 8.28 - 8.18 (m, 1H), 7.74 - 7.63 (m, 1H), 7.42 - 7.27 (m, 2H), 7.16 - 7.08 (m, 1H), 6.68 - 6.57 (m, 1H), 4.92 (br d, J = 13.6 Hz, 1H), 4.73 (s, 1H), 4.65 - 4.53 (m, 1H), 3.94 - 3.72 (m, 2H), 3.53 - 3.40 (m, 2H), 3.24 - 3.00 (m, 3H), 2.73 (br d, J = 8.3 Hz, 2H), 2.40 - 2.22 (m, 2H), 2.01 - 1.74 (m, 2H), 1.72 - 1.35 (m, 7H), 0.97 - 0.65 (m, 7H), 0.32 - 0.13 (m, 3H).

### Example 40

### 2-(6-{4-[(1R,3S,4S)-2-azabicyclo[2.2.1] heptan-3-carbonyl]piperazin-1-yl}imidazo[1,5-a]pyridin-8-yl)-N-ethyl-5-fluoro-N-(isopropyl)benzamide 40

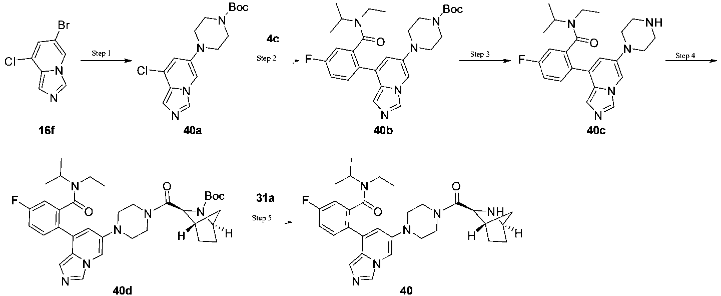

### Step 1

### 4-{8-chloroimidazo[1,5-a]pyridin-6-yl}-piperazin-1-tert-butyl carboxylate 40a

Compound **16f** (500 mg, 2.16 mmol), piperazin-1-tert-butyl carboxylate (403 mg, 2.16 mmol), Puphos(101 mg, 216.44 µmol) and cesium carbonate (2.11 g, 6.48 mmol) were dissolved in dioxane (10 mL). Pd₂ (dba)₃ (158 mg, 216 µmol) was added. Under a nitrogen atmosphere, the mixture was stirred at 90°Cto allow for reacting for 12 hours. The reaction mixture was cooled to room temperature, water (20 mL) was added, and the mixture was extracted with ethyl acetate (35 mL x 3). The organic phases were combined, washed with saturated brine (30 mL), dried over anhydrous sodium sulfate, and concentrated under a reduced pressure. The resulting residues were purified by the silica-gel column chromatography using petroleum ether/ethyl acetate as an elution system, to obtain the title Compound **40a** (200 mg, 593.80 µmol, yield: 27.5%).
MS m/z (ESI): 337.1 [M+1]

### Step 2

### 4-(8-{2-[ethyl(isopropyl)aminoformyl]-4-fluorophenyl}imidazo[1,5-a]pyridin-6-yl)-piperazin-1-tert-butyl formate 40b

Compound **40a** (150 mg, 445.35 µmol) and Compound **4c** (300 mg, 894.93 µmol) were dissolved in dioxane (3.0 mL) and water (0.5 mL). Potassium phosphate (284 mg, 1.34 mmol) and Pd(dtbpf)Cl₂ (30 mg, 46.03 µmol) were added, and the mixture was stirred at 90 °C under a nitrogen atmosphere to allow for reacting for 12 hours. The reaction mixture was cooled to room temperature, water (20 mL) was added, and the mixture was extracted with ethyl acetate (35 mL x 3). The organic phases were combined, washed with saturated brine (30 mL), dried over anhydrous sodium sulfate, and concentrated under a reduced pressure. The resulting residues were purified by the silica-gel column chromatography using petroleum ether/ethyl acetate as an elution system, to obtain the title Compound **40b** (100 mg, 196.23 µmol, yield: 44.1%).
MS m/z (ESI): 510.2 [M+1]

### Step 3

### N-ethyl-5-fluoro-2-[6-(piperazin-1-yl)imidazo[1,5-a]pyridin-8-yl]-N-(isopropyl)benzamide 40c

Compound **40b** (100 mg, 196.23 µmol) was dissolved in dichloromethane (3 mL), followed by the addition of trifluoroacetic acid (1.48 g, 12.98 mmol, 1 mL) at 0°C. The mixture was stirred at room temperature to allow for reacting for 0.5 hours. The reaction mixture was concentrated under a reduced pressure, to obtain the crude product of title Compound **40c** (100 mg). The crude product was directly used in the next step of reaction without purification. MS m/z (ESI): 410.1 [M+1]

### Step 4

### (1R,3S,4S)-3-[4-(8-{2-[ethyl(isopropyl)aminoformyl]-4-fluorophenyl}imidazo[1,5-a]pyridin-6-yl)piperazin-1-carbonyl]-2-azabicyclo[2.2.1]heptan-2-tert-butyl carboxylate 40d

Compound **31a** (52.23 mg, 216.45 µmol) and *N,N*-diisopropylethylamine (176.42 mg, 1.37 mmol, 237.76 µL) were dissolved in dichloromethane (3 mL), and cooled to 0°C, followed by the addition of EDCI (56.45 mg, 294.45 µmol) and HOBt(40.31 mg, 298.35 µmol). The crude product of Compound **40c** (100 mg) was then added, and the mixture was stirred at room temperature for 12 hours. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 20%-50%), to obtain the title Compound **40d** (80 mg, 126.43 µmol, yield: 64.4%).
MS m/z (ESI): 633.4 [M+1]

### Step 5

### 2-(6-{4-[(1R,3S,4S)-2-azabicyclo[2.2.1]heptan-3-carbonyl]piperazin-1-yl}imidazo[1,5-a]pyridin-8-yl)-N-ethyl-5-fluoro-N-(isopropyl)benzamide 40

Compound **40d** (80 mg, 126.4 µmol) was dissolved in methane dioxide (1.5 mL) and trifluoroacetic acid (0.5 mL). The mixture was stirred at room temperature to allow for reacting for 1.0 hours. The reaction mixture was concentrated under a reduced pressure, and the resulting residues were purified by the HPLC (mobile phase: acetonitrile, water (0.1% ammonia water), elution gradient: 25%-55%), to obtain the title Compound **40** (53.1 mg, 93.3 µmol, yield: 46.2 %).
MS m/z (ESI): 533.4 [M+1]
¹H NMR (400 MHz, DMSO-*d*₆) δ 8.27 (s, 1H), 7.83 (s, 1H), 7.72 - 7.63 (m, 1H), 7.44 - 7.28 (m, 2H), 7.11 - 7.02 (m, 1H), 6.71 - 6.62 (m, 1H), 3.76 - 3.38 (m, 8H), 3.07 - 2.86 (m, 5H), 2.42 (s, 1H), 1.54 - 1.30 (m, 4H), 1.26 - 0.98 (m, 3H), 0.98 - 0.67 (m, 7H), 0.34 - 0.26 (m, 2H).

### Example 42

### 2-[6-(1-{2-[4-(5,6-dimethoxypyridazin-3-yl)phenyl]ethyl}piperidin-4-yl) imidazo[1,5-a]pyridin-8-yl]-N-ethyl-5-fluoro-N-(isopropyl)benzamide 42

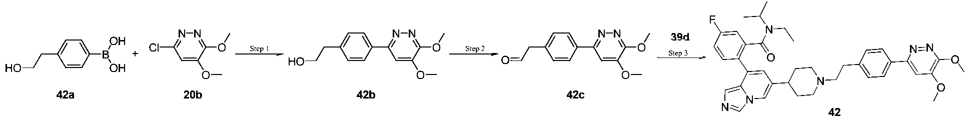

### Step 1

### 2-[4-(5,6-dimethoxypyridazin-3-yl)phenyl]ethyl-1-ol 42b

[4-(2-hydroxyethyl)phenyl]boric acid **42a** (855.65 mg, 5.16 mmol) and 6-chloro-3,4-dimethoxypyridazine **20b** (900 mg, 5.16 mmol) were dissolved in dioxane (10 mL) and water (2.0 mL). Sodium carbonate (1.09 g, 10.31 mmol) and tetrakis(triphenylphosphine)palladium (595.70 mg, 515.51 µmol) were added, and the mixture was stirred at 100°C under a nitrogen atmosphere to allow for reacting for 3.0 hours. The reaction mixture was filtered, and the filtrate was concentrated under a reduced pressure. The resulting residues were purified by the silica-gel column chromatography using dichloromethane/methanol as an elution system, to obtain the title Compound **42b** (1.23 g, 4.73 mmol, yield: 91.7%) as white solid.
MS m/z (ESI): 261.1 [M+1]
¹H NMR (400 MHz, DMSO-*d*₆) δ 7.99 (d, J = 8.4 Hz, 2H), 7.56 (s, 1H), 7.36 (d, J = 8.4 Hz, 1H), 4.68 (t, J = 5.2 Hz, 1H), 4.05 (s, 2H), 3.98 (s, 2H), 3.65 (td, J = 7.2, 5.6 Hz, 1H), 2.79 (t, J = 6.8 Hz, 1H).

### Step 2

### 2-[4-(5,6-dimethoxypyridazin-3-yl)phenyl]acetaldehyde 42c

Compound **42b** (40 mg, 153.68 µmol) was dissolved in dichloromethane (2.0 mL). The mixture was cooled to 0°C, and a Dess-Martin oxidizing agent (84.73 mg, 199.78 µmol) was added. The mixture was stirred at room temperature to allow for reacting for 2.0 hours. The reaction mixture was filtered, and the filtrate was concentrated under a reduced pressure. The resulting residues were purified by the preparative thin-layer chromatography using ethyl acetate/petroleum ether as an elution system, to obtain the title Compound **42c** (26.27 mg, 101.71 µmol, yield: 66.2%) as yellow solid.

¹H NMR (400 MHz, DMSO-*d*₆) δ 9.74 (t, J= 1.6 Hz, 1H), 8.07 (d, J= 8.0 Hz, 2H), 7.61 (s, 1H), 7.39 (d, J= 8.0 Hz, 2H), 4.06 (s, 3H), 3.99 (s, 3H), 3.87 (d, J= 1.6 Hz, 2H).

### Step 3

### 2-[6-(1-{2-[4-(5,6-dimethoxypyridazin-3-yl)phenyl]ethyl}piperidin-4-yl) imidazo[1,5-a]pyridin-8-yl]-N-ethyl-5-fluoro-N-(isopropyl)benzamide 42

Compound **39d** (71.18 mg, 174.23 µmol), triethylamine (58.77 mg, 580.78 µmol, 80.73 µL) and Compound **42c** (50 mg, 193.59 µmol) were dissolved in methanol (0.9 mL), and the mixture was stirred at room temperature for 0.5 hours. Sodium cyanoborohydride (24.33 mg, 387.19 µmol) was added, and the mixture was stirred at room temperature for 0.5 hours. An aqueous sodium bicarbonate solution (5.0 mL) was added to the reaction mixture, which was then extracted with ethyl acetate (5 mL x 3). The organic phases were combined, dried over anhydrous sodium sulfate, and concentrated under a reduced pressure. The resulting residues were purified by the HPLC (mobile phase: acetonitrile, water (0.1%ammonium bicarbonate), elution gradient: 34%-64%), to obtain the title Compound **42** (5.70 mg, 8.76 µmol, yield: 4.52%) as offwhite solid.
MS m/z (ESI): 651.5 [M+1]
¹H NMR (400 MHz, CDCl₃) δ 8.13 (s, 1H), 7.94 (d, J = 8.4 Hz, 2H), 7.76 (s, 1H), 7.69 - 7.60 (m, 1H), 7.36 (d, J = 8.0 Hz, 2H), 7.25 (s, 1H), 7.23 - 7.11 (m, 3H), 6.82 - 6.78 (m, 1H), 4.24 (s, 3H), 4.02 (s, 3H), 3.63 - 3.37 (m, 2H), 3.30 - 3.17 (m, 2H), 3.02 - 2.93 (m, 3H), 2.82 - 2.71 (m, 2H), 2.54 - 2.44 (m, 1H), 2.32 - 2.17 (m, 2H), 2.01 - 1.83 (m, 4H), 0.79 (m, J = 9.52 Hz, 7H), 0.24 (d, J = 6.8 Hz, 2H).

### Example 48

### N-ethyl-5-fluoro-2-[6-(3-fluoro-1-{[(1r,4r)-4-ethanesulfonamidocyclohexyl]methyl}azacyclobutan-3-yl)imidazo[1,5-a]pyridin-8-yl]-N-(isopropyl)benzamide 48

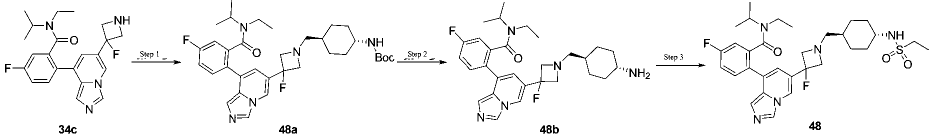

### Step 1

### N-[(1r,4r)-4-{[3-(8-{2-[ethyl(isopropyl)aminoformyl]-4-fluorophenyl}imidazo[1,5-a]pyridin-6-yl)-3-fluoroazacyclobutan-1-yl]methyl}cyclohexyl]tert-butyl carbamate 48a

Compound **34c** (122.99 mg, 240 µmol, trifluoroacetate), *N*-[(1*r*,4*r*)-4-formylcyclohexyl]tert-butyl carbamate (65.46 mg, 288.00 µmol) and triethylamine (72.86 mg, 720.00 µmol) were dissolved in methanol (3.0 mL), and stirred at room temperature for 1.0 hour. Sodium cyanoborohydride (45.70 mg, 727.20 µmol) was added, and the mixture was stirred at room temperature for 11 hours. A saturated aqueous sodium bicarbonate solution (10 mL) was added to the reaction mixture, which was then extracted with dichloromethane (15 mL x 2). The organic phases were combined, dried over anhydrous sodium sulfate, and concentrated under a reduced pressure. The resulting residues were purified by the preparative thin-layer chromatography using dichloromethane/methanol as an elution system, to obtain the title Compound **48a** (105 mg, 172.2 µmol, yield: 71.75%) as white solid.
MS m/z (ESI): 610.4 [M+1]

### Step 2

### N-ethyl-5-fluoro-2-[6-(3-fluoro-1-{[(1r,4r)-4-aminocyclohexyl]methyl}azacyclobutan-3-yl)imidazo[1,5-a]pyridin-8-yl]-N-(isopropyl)benzamide 48b

Compound **48a** (105 mg, 172.2 µmol) was dissolved in dichloromethane (3.0 mL), followed by the addition of trifluoroacetic acid (1.48 g, 12.98 mmol, 1 mL) at 0°C. The mixture was stirred at room temperature to allow for reacting for 1.0 hours. The reaction mixture was concentrated under a reduced pressure, to obtain the crude product of title Compound **48b** (105 mg). The crude product was directly used in the next step of reaction without purification. MS m/z (ESI): 510.4 [M+1]

### Step 3

### N-ethyl-5-fluoro-2-[6-(3-fluoro-1-{[(1r,4r)-4-ethanesulfonamidocyclohexyl]methyl}azacyclobutan-3-yl)imidazo[1,5-a]pyridin-8-yl]-N-(isopropyl)benzamide 48

The crude product of Compound **48b** (88 mg) was dissolved in dichloromethane (2.0 mL), followed by the addition of diisopropylethylamine (90.83 mg, 702.77 µmol), cooling to 0°C and the slow dropwise addition of ethanesulfonyl chloride (45.18 mg, 351.38 µmol), and the mixture was stirred at room temperature for 12 hours. The reaction mixture was diluted with water (10 mL), and extracted with dichloromethane (10 mL x 3). The organic phases were combined, washed with saturated brine, dried over anhydrous sodium sulfate, and concentrated under a reduced pressure. The resulting residues were purified by the preparative thin-layer chromatography using dichloromethane/methanol as an elution system, to obtain the title Compound **48** (7.06 mg, 11.73 µmol, yield: 8.35%) as offwhite solid.
MS m/z (ESI): 602.4 [M+1]
¹H NMR (400 MHz, DMSO-*d*₆) δ 8.63 - 8.57 (m, 1H), 8.47 (s, 1H), 7.74 - 7.66 (m, 1H), 7.44 - 7.28 (m, 2H), 7.19 (s, 1H), 7.01 - 6.96 (m, 1H), 6.86 - 6.78 (m, 1H), 3.72 - 3.62 (m, 2H), 3.49 - 3.39 (m, 3H), 3.31 - 3.22 (m, 1H), 3.02 - 2.87 (m, 4H), 2.39 - 2.32 (m, 2H), 1.90 - 1.67 (m, 4H), 1.23 - 1.13 (m, 6H), 1.02 - 0.69 (m, 9H), 0.34 - 0.28 (m, 2H).

### Example 50

### N-(4-{[3-(8-{2-[ethyl(isopropyl)aminoformyl]-4-fluorophenyl}-3-methylimidazo[1,5-a]pyridin-6-yl)pyrrolidin-1-yl]methyl}-4-hydroxylcyclohexyl)tert-butyl carbamate 50

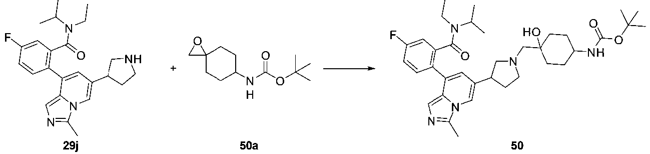

Compound **29j**(200 mg, 382.75 µmol, trifluoroacetate), *N*-{1-oxaspiro[2.5]octan-6-yl}tert-butyl carbamate **50a**(95.70 mg, 421.03 µmol) and triethylamine (193.65 mg, 1.91 mmol) were dissolved in ethanol (1.5 mL), and the mixture was stirred at 70°C for 12 hours. The reaction mixture was concentrated under a reduced pressure. The resulting residues were purified by the HPLC (mobile phase: acetonitrile, water (0.1% formic acid), elution gradient: 7%-37%), to obtain the title Compound **50** (80 mg, 125.8 µmol, yield: 32.9%) as yellow solid.
MS m/z (ESI): 636.4 [M+1]
¹H NMR (400 MHz, DMSO-*d*₆) δ 8.14 (s, 0.16H), 8.03 (s, 1H), 7.70 - 7.60 (m, 1H), 7.44 - 7.28 (m, 2H), 7.04 (s, 1H), 6.83 - 6.62 (m, 2H), 5.21 - 4.53 (m, 1H), 3.67 - 3.41 (m, 4H), 3.27 - 3.08 (m, 4H), 2.96 - 2.82 (m, 1H), 2.60 (s, 3H), 2.36 - 2.21 (m, 1H), 2.17 - 2.01 (m, 1H), 1.77 - 1.44 (m, 7H), 1.43 - 1.29 (m, 12H), 0.99 - 0.65 (m, 7H), 0.32 - 0.20 (m, 2H).

### Examples 51 and 52

### N-ethyl-5-fluoro-2-[3-methyl-6-(1-{[(1r,4r)-4-ethanesulfonamido-1-ffuorocyclohexyl]methyl}pyrrolidin-3-yl) imidazo[1,5-a]pyridin-8-yl]-N-(isopropyl)benzamide 51

### N-ethyl-5-fluoro-2-[3-methyl-6-(1-{[(1s,4s)-4-ethanesulfonamido-1-fluorocyclohexyl]methyl}pyrrolidin-3-yl) imidazo[1,5-a]pyridin-8-yl]-N-(isopropyl)benzamide 52

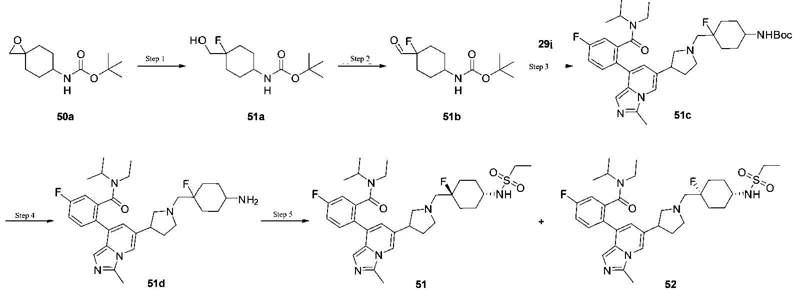

### Step 1

### N-[4-fluoro-4-(hydroxymethyl)cyclohexyl]tert-butyl carbamate 51a

Compound **50a** (1.0 g, 4.40 mmol) was dissolved in dichloromethane (15.0 mL), and cooled to -78°C under the protection of nitrogen. A hydrogen fluoride pyridine solution (622.88 mg, 4.40 mmol, 5 mL, hydrogen fluoride 70% purity) was added dropwise slowly, and the mixture was stirred at -78°C for 2.0 hours. A saturated aqueous sodium bicarbonate solution (30 mL) was added to quench the reaction, and the mixture was concentrated under a reduced pressure. The crude product were dissolved in methanol (40 mL), followed by the addition of Boc₂O (4.80 g, 22.00 mmol, 5.05 mL) and DMAP (53.75 mg, 439.95 µmol), and the mixture was stirred at room temperature for 12 hours. Water (30 mL) was added to the mixture, which was then extracted with dichloromethane (30 mL × 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **51a** (450 mg, 1.82 mmol, yield: 41.36%) as white solid.
MS m/z (ESI): 270.1 [M+23]
¹H NMR (400 MHz, CDCl₃) δ 4.46 (br d, J = 1.3 Hz, 1H), 3.69 - 3.54 (m, 2H), 3.53 - 3.40 (m, 1H), 2.07 - 1.88 (m, 4H), 1.49 - 1.41 (m, 13H).

### Step 2

### N-(4-fluoro-4-formylcyclohexyl)tert-butyl carbamate 51b

Oxalyl chloride (462 mg, 3.64 mmol, 317.53 µL) was dissolved in dichloromethane (5 mL), and the mixture was cooled to -78°C, followed by the dropwise addition of the dichloromethane solution (5 mL) of DMSO (427 mg, 5.47 mmol, 387.83 µL). The mixture was stirred at -78°C for 10 min, followed by the dropwise addition of the solution of Compound **51a**(450 mg, 1.82 mmol) in dichloromethane (5.0 mL). The mixture was stirred at -78°C to allow for reacting for 0.5 hours, triethylamine (1.84 g, 18.20 mmol) was then added, and the mixture was warmed to room temperature and further stirred to allow for reacting for 1 hour. A saturated aqueous ammonium chloride solution (15 mL) was added to quench the reaction, and the reaction mixture was extracted with dichloromethane (20 mL × 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **51b** (300 mg, 1.22 mmol, yield: 67.2%) as white solid.
MS m/z (ESI): 286.1 [M+41]
¹H NMR (400 MHz, CDCl₃) δ 9.84 - 9.69 (m, 1H), 4.56 (br s, 1H), 3.60 - 3.44 (m, 1H), 2.02 - 1.90 (m, 4H), 1.80 - 1.67 (m, 2H), 1.45 (s, 11H).

### Step 3

### N-(4-{[3-(8-{2-[ethyl(isopropyl)aminoformyl]-4-fluorophenyl}-3-methylimidazo[1,5-a]pyridin-6-yl)pyrrolidin-1-yl]methyl}-4-fluorocyclohexyl)tert-butyl carbamate 51c

Compound 29j(100 mg, 244.79 µmol), Compound **51b**(72 mg, 293.53 µmol) and triethylamine (25 mg, 247.06 µmol) were dissolved in methanol (3.0 mL), and stirred at room temperature for 1.0 hour. Sodium cyanoborohydride (47 mg, 747.91 µmol) was added, and the mixture was stirred at room temperature for 11 hours. A saturated aqueous sodium bicarbonate solution (10 mL) was added to the reaction mixture, which was then extracted with dichloromethane (20 mL × 3). The organic phases were combined, washed with saturated brine (40 mL), dried over anhydrous sodium sulfate, and concentrated under a reduced pressure. The resulting residues were purified by the preparative thin-layer chromatography using dichloromethane/methanol as an elution system, to obtain the title Compound **51c** (105 mg, 164.63 µmol, yield: 67.25%).
MS m/z (ESI): 638.3 [M+1]

### Step 4

### 2-(6-{1-[(4-amino-1-fluorocyclohexyl)methyl]pyrrolidin-3-yl}-3-methylimidazo[1,5-a]pyridin-8-yl)-N-ethyl-5-fluoro-N-(isopropyl) benzamide 51d

Compound 51c(100 mg, 156.79 µmol) was dissolved in methane dioxide (1.5 mL), followed by the addition of trifluoroacetic acid (740.00 mg, 6.49 mmol, 0.5 mL) at 0°C. The mixture was stirred at room temperature to allow for reacting for 0.5 hours. The reaction mixture was concentrated under a reduced pressure, to obtain the crude product of title Compound **51d** (100 mg), which was directly used in the next step of reaction without purification. MS m/z (ESI): 538.2 [M+1]

### Step 5

### N-ethyl-5-fluoro-2-[3-methyl-6-(1-{[(1r,4r)-4-ethanesulfonamido-1-fluorocyclohexyl]methyl}pyrrolidin-3-yl) imidazo[1,5-a]pyridin-8-yl]-N-(isopropyl)benzamide 51

### N-ethyl-5-fluoro-2-[3-methyl-6-(1-{[(1s,4s)-4-ethanesulfonamido-1-fluorocyclohexyl]methyl}pyrrolidin-3-yl) imidazo[1,5-a]pyridin-8-yl]-N-(isopropyl)benzamide 52

The crude product of Compound **51d** (100 mg) and triethylamine (77.63 mg, 767.21 µmol) were dissolved in dichloromethane (3.0 mL), followed by the addition of ethanesulfonyl chloride (39.46 mg, 306.89 µmol), and the mixture was stirred at room temperature for 12 hours. A saturated aqueous sodium bicarbonate solution (10 mL) was added to the reaction mixture, which was then extracted with dichloromethane (20 mL × 3). The organic phases were combined, washed with saturated brine (40 mL), dried over anhydrous sodium sulfate, and concentrated under a reduced pressure. The resulting residues were purified by the HPLC (C18; mobile phase: acetonitrile, water (0.1% ammonium bicarbonate), elution gradient: 33%-63%), to obtain the title Compounds **51** (1.21 mg, 1.92 µmol, yield: 1.25%) and **52** (4.01 mg, 6.37 µmol, yield: 4.15%) both as white solid.

### Compound 51:

MS m/z (ESI):630.2 [M+1]
HPLC: retention time: 1.101 min.

¹H NMR (400 MHz, DMSO-*d*₆) δ 7.93 (s, 1H), 7.70 - 7.60 (m, 1H), 7.42 - 7.25 (m, 2H), 7.04 - 6.96 (m, 2H), 6.73 - 6.61 (m, 1H), 3.46 - 3.42 (m, 1H), 3.28 - 3.24 (m, 2H), 3.03 - 2.89 (m, 4H), 2.78 - 2.61 (m, 5H), 2.57 (s, 3H), 2.21 - 2.11 (m, 1H), 1.91 - 1.62 (m, 7H), 1.51 - 1.39 (m, 2H), 1.22 - 1.13 (m, 3H), 1.09 - 1.01 (m, 2H), 0.93 - 0.75 (m, 6H), 0.34 - 0.25 (m, 2H).

### Compound 52:

MS m/z (ESI):630.1 [M+1]
Chiral HPLC: retention time: 0.974 min.

¹H NMR (400 MHz, DMSO-*d*₆) δ 7.92 (s, 1H), 7.70 - 7.60 (m, 1H), 7.41 - 7.26 (m, 2H), 7.12 - 6.96 (m, 2H), 6.73 - 6.59 (m, 1H), 3.47 - 3.41 (m, 1H), 3.29 - 3.22 (m, 2H), 3.16 - 3.05 (m, 1H), 3.02 - 2.84 (m, 4H), 2.78 - 2.68 (m, 2H), 2.67 - 2.55 (m, 5H), 2.22 - 2.07 (m, 1H), 1.98 - 1.64 (m, 5H), 1.59 - 1.35 (m, 4H), 1.27 - 1.13 (m, 3H), 1.10 - 0.70 (m, 8H), 0.33 - 0.24 (m, 2H).

### Example 53

### N-[4-({[1-(8-{2-[ethyl(isopropyl)aminoformyl]-4-fluorophenyl}-3-methylimidazo[1,5-a]pyridin-6-yl)azacyclobutan-3-yl]amino}methyl)-4-fluorocyclohexyl]tert-butyl carbamate 53

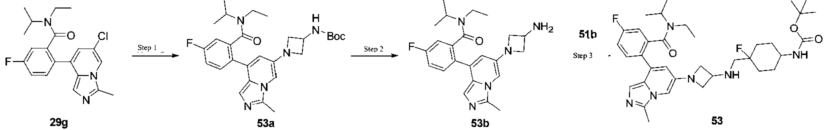

### Step 1

### N-[1-(8-{2-[ethyl(isopropyl)aminoformyl]-4-fluorophenyl}-3-methylimidazo[1,5-a]pyridin-6-yl)azacyclobutan-3-yl] tert-butyl carbamate 53a

Compound **29g** (500 mg, 1.34 mmol), *N*-(azacyclobutan-3-yl)tert-butyl carbamate (276.41 mg, 1.60 mmol) and cesium carbonate (1.31 g, 4.01 mmol) were dissolved in *t*-AmOH (10 mL). Ruphos (124.82 mg, 267.49 µmol) and Pd₂ (dba)₃ (122.47 mg, 133.74 µmol) were added. Under a nitrogen atmosphere, the mixture was stirred at 90°Cto allow for reacting for 12 hours. The reaction mixture was cooled to room temperature, water (25 mL) was added, and the mixture was extracted with ethyl acetate (30 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **53a** (420 mg, 824.15 µmol, yield: 61.6%) as brown solid.
MS m/z (ESI): 510.1 [M+1]

### Step 2

### 2-[6-(3-aminoazacyclobutan-1-yl)-3-methylimidazo[1,5-a]pyridin-8-yl]-N-ethyl-5-fluoro-N-(isopropyl)benzamide 53b

Compound **53a** (200 mg, 392.45 µmol) was dissolved in dichloromethane (3 mL), followed by the addition of trifluoroacetic acid (1.48 g, 12.98 mmol, 1 mL) at 0°C. The mixture was stirred at room temperature to allow for reacting for 1.0 hours. The reaction mixture was concentrated under a reduced pressure, to obtain the crude product of title Compound **53b** (180 mg). The crude product was directly used in the next step of reaction without purification. MS m/z (ESI): 410.1 [M+1]

### Step 3

### N-[4-({[1-(8-{2-[ethyl(isopropyl)aminoformyl]-4-fluorophenyl}-3-methylimidazo[1,5-a]pyridin-6-yl)azacyclobutan-3-yl]amino}methyl)-4-fluorocyclohexyl]tert-butyl carbamate 53

The crude product of Compound **53b** (160 mg), Compound **51b** (110 mg, 448.45 µmol) and triethylamine (119.00 mg, 1.18 mmol) were dissolved in methanol (3.5 mL), and stirred at room temperature for 1.0 hour. Sodium cyanoborohydride (73.90 mg, 1.18 mmol) was added, and the reaction mixture was stirred at room temperature for 11 hour. A saturated aqueous sodium bicarbonate solution (15 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 5%-35%), to obtain the title Compound **53** (80 mg, 125.24 µmol, yield: 31.95%) as yellow solid.
MS m/z (ESI): 639.7 [M+1]
¹H NMR (400 MHz, DMSO-*d*₆) δ 8.17 (br s, 1H), 7.70 - 7.61 (m, 1H), 7.38 - 7.28 (m, 2H), 6.99 - 6.94 (m, 2H), 6.79 - 6.72 (m, 1H), 6.28 - 6.22 (m, 1H), 3.99 - 3.91 (m, 3H), 3.71 - 3.63 (m, 4H), 3.36 - 3.29 (m, 4H), 2.97 - 2.90 (m, 1H), 2.71 - 2.57 (m, 3H), 1.92 - 1.77 (m, 3H), 1.37 (s, 14H), 0.92 - 0.88 (m, 3H), 0.87 - 0.82 (m, 3H), 0.78 - 0.72 (m, 1H), 0.33 - 0.30 (m, 2H).

### Examples 54 and 55

### N-[(1s,4s)-4-{[3-(4-{2-[ethyl(isopropyl)aminoformyl]-4-fluorophenyl}-1-methyl-1H-indazol-6-yl) pyrrolidin-1-yl]methyl}-4-hydroxylcyclohexyl]tert-butyl carbamate 54

### N-ethyl-5-fluoro-2-[1-methyl-6-(1-{[(1s,4s)-4-ethanesulfonamido-1-hydroxylcyclohexyl]methyl}pyrrolidin-3-yl)-1H-indazol-4-yl]-N-(isopropyl)benzamide 55

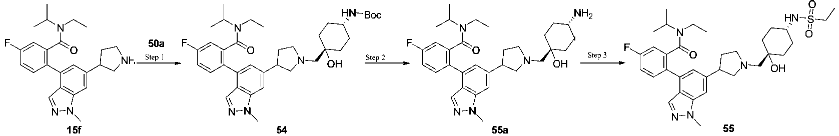

### Step 1

### N-[(1s,4s)-4-{[3-(4-{2-[ethyl(isopropyl)aminoformyl]-4-fluorophenyl}-1-methyl-1H-indazol-6-yl) pyrrolidin-1-yl]methyl}-4-hydroxylcyclohexyl]tert-butyl carbamate 54

Compound **15f** (100 mg, 85.7 µmol), *N*-{1-oxaspiro[2.5]octan-6-yl} tert-butyl carbamate **50a** (19.5 mg, 85.7 µmol) and DIPEA(22.2 mg, 171 µmol) were dissolved in ethanol (1.0 mL), and the mixture was stirred at 60°C for 12 hours. The reaction mixture was concentrated under a reduced pressure. The resulting residues were purified by the preparative thin-layer chromatography using dichloromethane/methanol as an elution system, to obtain the title Compound **54** (30 mg, 47.2 µmol, yield: 55.0 %) as white solid.
MS m/z (ESI): 636.25 [M+1]
¹H NMR (400 MHz, DMSO-*d*₆) δ 7.79 (s, 1H), 7.62 - 7.50 (m, 2H), 7.42 - 7.34 (m, 1H), 7.33 - 7.24 (m, 1H), 7.07 (d, J = 16.0 Hz, 1H), 6.69 (s, 1H), 4.26 - 4.17 (m, 0.19H), 4.03 (d, J = 3.6 Hz, 3H), 3.83 (s, 0.85H), 3.42 - 3.36 (m, 1H), 3.28 - 3.22 (m, 1H), 3.19 - 2.96 (m, 2H), 2.89 - 2.70 (m, 3H), 2.45 - 2.35 (m, 3H), 2.25 - 2.14 (m, 1H), 1.91 - 1.75 (m, 1H), 1.64 - 1.43 (m, 6H), 1.37 (s, 9H), 1.33 - 1.26 (m, 2H), 1.04 - 0.59 (m, 7H), 0.09 (t, J = 6.4 Hz, 2H).

### Step 2

### N-ethyl-5-fluoro-2-[1-methyl-6-(1-{[(1s,4s)-4-amino-1-hydroxylcyclohexyl]methyl}pyrrolidin-3-yl)-1H-indazol-4-yl]-N-(isopropyl)benzamide 55a

Compound **54** (15.0 mg, 23.6 µmol) was dissolved in methane dioxide (0.3 mL), followed by the addition of trifluoroacetic acid (0.1 mL) at 0°C. The mixture was stirred at 0°C to allow for reacting for 1.0 hour. The reaction mixture was concentrated under a reduced pressure, to obtain the crude product of title Compound **55a** (30 mg), which was directly used in the next step of reaction without purification.
MS m/z (ESI): 536.25 [M+1]

### Step 3

### N-ethyl-5-fluoro-2-[1-methyl-6-(1-{[(1s,4s)-4-ethanesulfonamido-1-hydroxylcyclohexyl]methyl}pyrrolidin-3-yl)-1H-indazol-4-yl]-N-(isopropyl)benzamide 55

The crude product of Compound **55a** (30 mg) and DIPEA(12.2 mg, 94.1 µmol) was dissolved in DMF(0.5 mL), and cooled to 0°C, followed by the addition of ethanesulfonyl chloride (3.02 mg, 23.5 µmol), and the mixture was stirred at room temperature for 2.0 hours. The reaction mixture was concentrated under a reduced pressure. The resulting residues were purified by the HPLC (mobile phase: acetonitrile, water (0.1% formic acid), elution gradient: 10%-35%), to obtain the title Compound **55** (3.00 mg, 4.78 µmol, yield: 20.3 %) as white solid.
MS m/z (ESI):628.25 [M+1]
¹H NMR (400 MHz, DMSO-*d*₆) δ 7.71 (s, 1H), 7.55 - 7.41 (m, 2H), 7.35 - 7.25 (m, 1H), 7.24 - 7.15 (m, 1H), 6.98 (d, J = 15.6 Hz, 1H), 6.93 - 6.82 (m, 1H), 4.18 - 4.08 (m, 0.27H), 3.94 (s, 3H), 3.01 - 2.56 (m, 9H), 2.37 - 2.24 (m, 3H), 2.19 - 2.06 (m, 1H), 1.79 - 1.70 (m, 1H), 1.67 - 1.40 (m, 6H), 1.32 - 1.21 (m, 2H), 1.16 - 1.05 (m, 3H), 0.94 - 0.48 (m, 7H), 0.08 (t, J = 6.0 Hz, 2H).

### Examples 55-1-1, 55-1-2, 55-2-1 and 55-2-2

### N-ethyl-5-fluoro-2-[1-methyl-6-[(3R)-1-{[(1r,4s)-4-ethanesulfonamido-1-hydroxylcyclohexyl]methyl}pyrrolidin-3-yl]-1H-indazol-4-yl]-N-(isopropyl)benzamide 55-1-1

### N-ethyl-5-fluoro-2-[1-methyl-6-[(3R)-1-{[(1r,4r)-4-ethanesulfonamido-1-hydroxylcyclohexyl]methyl}pyrrolidin-3-yl]-1H-indazol-4-yl]-N-(isopropyl)benzamide 55-1-2

### N-ethyl-5-fluoro-2-[1-methyl-6-[(3S)-1-{[(1s,4s)-4-ethanesulfonamido-1-hydroxylcyclohexyl]methyl}pyrrolidin-3-yl]-1H-indazol-4-yl]-N-(isopropyl)benzamide 55-2-1

### N-ethyl-5-fluoro-2-[1-methyl-6-[(3S)-1-{[(1r,4r)-4-ethanesulfonamido-1-hydroxylcyclohexyl]methyl}pyrrolidin-3-yl]-1H-indazol-4-yl]-N-(isopropyl)benzamide 55-2-2

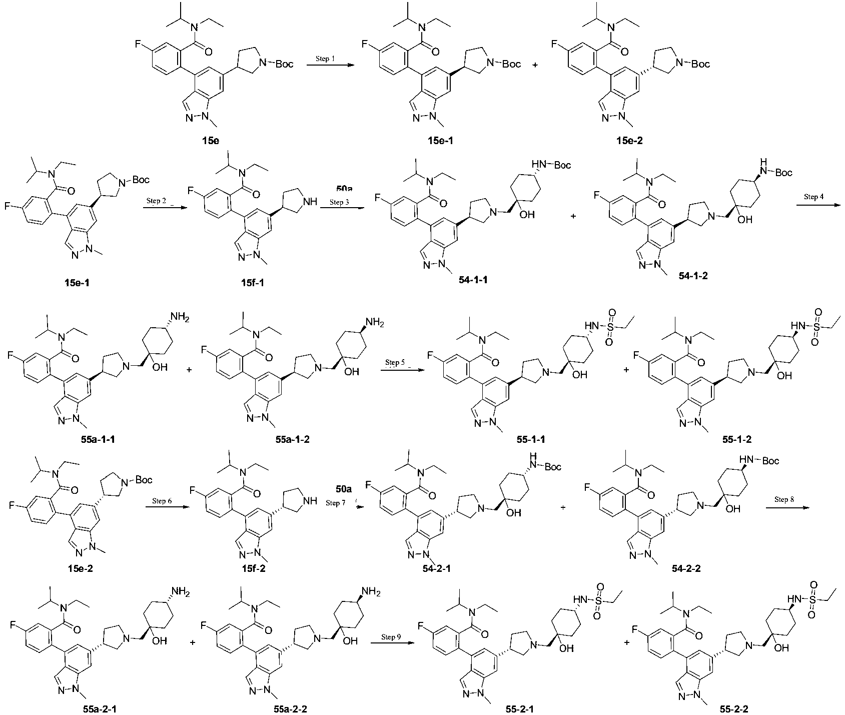

### Step 1

### (3R)-3-(4-{2-[ethyl(isopropyl)aminoformyl]-4-fluorophenyl}-1-methyl-1H-indazol-6-yl)pyrrolidin-1-tert-butyl formate 15e-1

### (3S)-3-(4-{2-[ethyl(isopropyl)aminoformyl]-4-fluorophenyl}-1-methyl-1H-indazol-6-yl)pyrrolidin-1-tert-butyl formate 15e-2

Compound **15e** (930 mg, 1.83 mmol) was separated and purified by the chiral preparative SFC (preparative column: DAICEL IG (250 mm * 30 mm,10 um), mobile phase: 70% (v/v) carbon dioxide-ethanol (0.1% NH₃)), to obtain the title Compounds **15e-1** (378 mg, 743.2 µmol, yield: 40.65%) and **15e-2** (400 mg, 786.4 µmol, yield: 43.01%).

### Single-configuration Compound 15e-1:

MS m/z (ESI):509.2 [M+1]
SFC analysis: retention time: 5.306 min; chromatographic column: DAICEL IG 4.6 mm*250 mm L5 µm; mobile phase: CO₂/MeOH[0.1% NH₃ (7M methanol solution)] = 70/30.

¹H NMR (400 MHz, DMSO-*d*₆) δ 7.82 (s, 1H), 7.59 (q, J = 5.6 Hz, 2H), 7.43 - 7.26 (m, 2H), 7.03 (s, 1H), 4.21 (dd, J = 13.6, 7.6 Hz, 0.26H), 4.14 - 3.98 (m, 3H), 3.74 (dd, J = 10.0, 8.0 Hz, 1H), 3.57 - 3.34 (m, 3H), 3.31 - 3.19 (m, 2H), 2.91 - 2.72 (m, 1H), 2.23 (d, J = 5.6 Hz, 1H), 2.12 - 1.97 (m, 1H), 1.41 (d, J = 6.4 Hz, 9H), 1.04 - 0.60 (m, 7H), 0.07 (d, J = 5.6 Hz, 2H).

### Single-configuration Compound 15e-2:

MS m/z (ESI):509.2 [M+1]
SFC analysis: retention time: 4.702 min; chromatographic column: DAICEL IG 4.6 mm*250 mm L5 µm; mobile phase: CO₂/MeOH[0.1% NH₃ (7M methanol solution)] = 70/30.

¹H NMR (400 MHz, DMSO-*d*₆) δ 7.82 (s, 1H), 7.59 (t, J = 7.2 Hz, 2H), 7.43 - 7.26 (m, 2H), 7.02 (s, 1H), 4.27 - 3.99 (m, 4H), 3.74 (dd, J = 10.0, 8.0 Hz, 1H), 3.44 (dd, J = 54.0, 7.2 Hz, 3H), 3.31 - 3.22 (m, 2H), 2.90 - 2.71 (m, 1H), 2.22 (s, 1H), 2.03 (d, J = 6.8 Hz, 1H), 1.41 (d, J = 6.4 Hz, 9H), 1.05 - 0.57 (m, 7H), 0.07 (d, J = 5.6 Hz, 2H).

### Steps 2 and 6

### N-ethyl-5-fluoro-2-[1-methyl-6-[(3R)-pyrrolidin-3-yl]-1H-indazol-4-yl]-N-(isopropyl)benzamide 15f-1

### N-ethyl-5-fluoro-2-[1-methyl-6-[(3S)-pyrrolidin-3-yl]-1H-indazol-4-yl]-N-(isopropyl)benzamide 15f-2

At 0°C, Compound **15e-1/15e-2** (110 mg/116 mg, 202.2 µmol/294 µmol) was dissolved in methane dioxide (1.0 mL/4.0 mL), followed by the addition of trifluoroacetic acid (0.3 mL/1.0 mL). The mixture was stirred at room temperature to allow for reacting for 1.0 hours. The reaction mixture was concentrated under a reduced pressure, to obtain the crude product of title Compound **15f-1/15f-2** (200 mg/166 mg). The crude product was directly used in the next step of reaction without purification.

### Single-configuration Compound 15f-1:

MS m/z (ESI): 409.25 [M+1]

### Single-configuration Compound 15f-1:

MS m/z (ESI): 409.25 [M+1]

### Steps 3 and 7

### N-[(1s,4s)-4-{[(3R)-3-(4-{2-[ethyl(isopropyl)aminoformyl]-4-fluorophenyl}-1-methyl-1H-indazol-6-yl)pyrrolidin-1-yl]methyl}-4-hydroxylcyclohexyl]tert-butyl carbamate 54-1-1

### N-[(1r,4r)-4-{[(3R)-3-(4-{2-[ethyl(isopropyl)aminoformyl]-4-fluorophenyl}-1-methyl-1H-indazol-6-yl)pyrrolidin-1-yl]methyl}-4-hydroxylcyclohexyl]tert-butyl carbamate 54-1-2

### N-[(1s,4s)-4-{[(3S)-3-(4-{2-[ethyl(isopropyl)aminoformyl]-4-fluorophenyl}-1-methyl-1H-indazol-6-yl)pyrrolidin-1-yl]methyl}-4-hydroxylcyclohexyl]tert-butyl carbamate 54-2-1

### N-[(1r,4r)-4-{[(3S)-3-(4-{2-[ethyl(isopropyl)aminoformyl]-4-fluorophenyl}-1-methyl-1H-indazol-6-yl)pyrrolidin-1-yl]methyl}-4-hydroxylcyclohexyl]tert-butyl carbamate 54-2-2

The crude product of Compound **15f-1/15f-2** (200 mg/166 mg) and DIPEA(111 mg/114 mg, 862 µmol/884 µmol) were dissolved in ethanol (4.0 mL/15.0 mL), followed by the addition of Compound 50a(49 mg/67 mg, 215 µmol/294 µmol), and the mixture was stirred at 60°C for 12 hours. The reaction mixture was concentrated under a reduced pressure. The resulting residues were purified by the silica-gel column chromatography using dichloromethane/methanol as an elution system, to obtain the mixture of title Compounds **54-1-1 and 54-1-2/54-2-1 and 54-2-2** (173 mg/350 mg).

### Mixture of 54-1-1 and 54-1-2:

MS m/z (ESI):636.35 [M+1]

### Mixture of 54-2-1 and 54-2-2:

MS m/z (ESI):636.45 [M+1]

### Steps 4 and 8

### N-ethyl-5-fluoro-2-{1-methyl-6-[(3R)-1-{[(1s,4s)-4-amino-1-hydroxylcyclohexyl]methyl}pyrrolidin-3-yl]-1H-indazol-4-yl}-N-(isopropyl)benzamide 55a-1-1

### N-ethyl-5-fluoro-2-{1-methyl-6-[(3R)-1-{[(1r,4r)-4-amino-1-hydroxylcyclohexyl]methyl}pyrrolidin-3-yl]-1H-indazol-4-yl}-N-(isopropyl)benzamide 55a-1-2

### N-ethyl-5-fluoro-2-{1-methyl-6-[(3S)-1-{[(1s,4s)-4-amino-1-hydroxylcyclohexyl]methyl}pyrrolidin-3-yl]-1H-indazol-4-yl}-N-(isopropyl)benzamide 55a-2-1

### N-ethyl-5-fluoro-2-{1-methyl-6-[(3S)-1-{[(1r,4r)-4-amino-1-hydroxylcyclohexyl]methyl}pyrrolidin-3-yl]-1H-indazol-4-yl}-N-(isopropyl)benzamide 55a-2-2

At 0°C, the mixture of Compounds **54-1-1 and 54-1-2/the** mixture of Compounds **54-2-1 and 54-2-2** (293 mg/187 mg, 322 µmol/294 µmol) was dissolved in methane dioxide (3.0 mL/4.0 mL), followed by the addition of trifluoroacetic acid (0.6 mL/1.0 mL). The mixture was stirred at room temperature to allow for reacting for 1.0 hours. The reaction mixture was concentrated under a reduced pressure, to obtain the crude mixture of title Compounds **55a-1-1 and 55a-1-2** (210 mg), which was directly used in the next step of reaction without purification. The crude product of the mixture of 55a-2-1 and 55a-2-2 was purified by the HPLC (mobile phase: acetonitrile, water (0.1% trifluoroacetic acid), elution gradient: 25%-40%), to obtain the mixture of title Compounds **55a-2-1 and 55a-2-2** (201 mg).

### Mixture of 55a-1-1 and 55a-1-2:

MS m/z (ESI): 536.25 [M+1]

### Mixture of 55a-2-1 and 55a-2-2:

MS m/z (ESI): 536.10 [M+1]

### Steps 5 and 9

### N-ethyl-5-fluoro-2-[1-methyl-6-[(3R)-1-{[(1s,4s)-4-ethanesulfonamido-1-hydroxylcyclohexyl]methyl}pyrrolidin-3-yl]-1H-indazol-4-yl]-N-(isopropyl)benzamide 55-1-1

### N-ethyl-5-fluoro-2-[1-methyl-6-[(3R)-1-{[(1s,4s)-4-ethanesulfonamido-1-hydroxylcyclohexyl]methyl}pyrrolidin-3-yl]-1H-indazol-4-yl]-N-(isopropyl)benzamide 55-1-2

### N-ethyl-5-fluoro-2-[1-methyl-6-[(3R)-1-{[(1s,4s)-4-ethanesulfonamido-1-hydroxylcyclohexyl]methyl}pyrrolidin-3-yl]-1H-indazol-4-yl]-N-(isopropyl)benzamide 55-2-1

### N-ethyl-5-fluoro-2-[1-methyl-6-[(3S)-1-{[(1r,4r)-4-ethanesulfonamido-1-hydroxylcyclohexyl]methyl}pyrrolidin-3-yl]-1H-indazol-4-yl]-N-(isopropyl)benzamide 55-1-2

The crude product of the mixture of Compounds **55a-1-1 and 55a-1-2/the mixture of Compounds 55^{a}-2-1 and 55a-2-2** (200 mg/160 mg) was dissolved in DMF (2.0 mL/10 mL), followed by the addition of ethanesulfonyl chloride (39.6 mg/38.4 mg, 308 µmol/299 µmol) and DIPEA(159 mg/193, 1.22 mmol/1.49 mmol), and the mixture was stirred at room temperature for 2 hours. The reaction mixture was concentrated under a reduced pressure. The resulting residues were purified by the HPLC (mobile phase: acetonitrile, water (0.1% formic acid), elution gradient: 25%-40%), to obtain the mixture of 55-1-1 and 55-1-2/the mixture of 55-2-1 and 55-2-2 (110 mg/125 mg). The resulting mixture was purified by the preparative SFC (chromatographic column: chiralpak-AD-H, mobile phase: CO₂-MeOH (0.1%NH₃)), to obtain the title Compound **55-1-1/55-1-2** (60.0 mg/4.0 mg, 95.6 µmol/6.37 µmol) and the title Compound **55-2-1/55-2-2** (88.5 mg/7.52 mg, 140 µmol/11 µmol)

### Single-configuration Compound 55-1-1:

MS m/z (ESI):628.2 [M+1]
HPLC: retention time: 2.865 min; chromatographic column: Shim-pack Velox C18, 2.7um, 3.0*50 mm; mobile phase: water (0.1% trifluoroacetic acid)/acetonitrile (0.1% trifluoroacetic acid); elution gradient: 5%-95%.

Chiral SFC: retention time: 7.233 min; chromatographic column: DAICEL AD-H 4.6 mm I.D.*250 mm L 5 µm; mobile phase: CO₂/MeOH[0.1% NH₃ (7M methanol solution)] = 60/40.

¹H NMR (400 MHz, DMSO-*d*₆) δ 7.79 (d, J = 1.2 Hz, 1H), 7.62 - 7.50 (m, 2H), 7.40 - 7.35 (m, 1H), 7.32 - 7.25 (m, 1H), 7.07 (d, J = 16.0 Hz, 1H), 6.98 (dd, J = 7.6, 3.2 Hz, 1H), 4.27 - 4.17 (m, 0.21H), 4.08 - 3.96 (m, 3H), 3.89 (s, 1H), 3.42 - 3.33 (m, 2H), 3.27 - 3.21 (m, 1H), 3.05 - 2.91 (m, 4H), 2.90 - 2.69 (m, 4H), 2.44 - 2.36 (m, 2H), 2.27 - 2.14 (m, 1H), 1.89 - 1.77 (m, 1H), 1.68 - 1.50 (m, 6H), 1.43 - 1.30 (m, 2H), 1.22 - 1.14 (m, 3H), 1.05 - 0.59 (m, 7H), 0.08 (t, J = 6.0 Hz, 2H).

### Single-configuration Compound 25-1-2:

MS m/z (ESI):628.3 [M+1]
HPLC: retention time: 2.483 min; chromatographic column: Shim-pack Velox C18, 2.7um, 3.0*50 mm; mobile phase: water (0.1% trifluoroacetic acid)/acetonitrile (0.1% trifluoroacetic acid); elution gradient: 5%-95%.

¹H NMR (400 MHz, DMSO-*d*₆) δ 8.29 (s, 0.97H), 7.79 (s, 1H), 7.62 - 7.47 (m, 2H), 7.43 - 7.33 (m, 1H), 7.29 (t, J = 10.4 Hz, 1H), 7.07 (d, J = 16.0 Hz, 1H), 6.92 (d, J = 7.2 Hz, 1H), 4.25 - 4.17 (m, 0.38H), 4.02 (s, 3H), 3.22 (s, 3H), 3.06 - 2.91 (m, 4H), 2.89 - 2.65 (m, 5H), 2.46 (s, 1H), 2.23 (s, 1H), 1.95 - 1.66 (m, 5H), 1.64 - 1.21 (m, 5H), 1.17 (t, J = 7.2 Hz, 3H), 1.02 - 0.53 (m, 7H), 0.08 (dd, J = 9.6, 6.8 Hz, 2H).

### Single-configuration Compound 55-2-1:

MS m/z (ESI):628.25 [M+1]
HPLC: retention time: 2.877 min; chromatographic column: Shim-pack Velox C18, 2.7um, 3.0*50 mm; mobile phase: water (0.1% trifluoroacetic acid)/acetonitrile (0.1% trifluoroacetic acid); elution gradient: 5%-95%.

Chiral SFC: retention time: 6.242 min; chromatographic column: DAICEL AD-H 4.6 mm I.D.*250 mm L 5 µm; mobile phase: CO₂/MeOH[0.1% NH₃ (7M methanol solution)] = 60/40.

¹H NMR (400 MHz, DMSO-*d*₆) δ 7.79 (s, 1H), 7.61 - 7.49 (m, 2H), 7.38 (td, J = 8.4, 2.4 Hz, 1H), 7.32 - 7.25 (m, 1H), 7.11 - 7.03 (m, 1H), 6.98 (dd, J = 7.6, 3.6 Hz, 1H), 4.26 - 4.15 (m, 0.21H), 4.08 - 3.95 (m, 3H), 3.90 (s, 1H), 3.43 - 3.33 (m, 2H), 3.30 - 3.20 (m, 1H), 3.07 - 2.90 (m, 4H), 2.89 - 2.63 (m, 4H), 2.47 - 2.35 (m, 2H), 2.28 - 2.14 (m, 1H), 1.91 - 1.75 (m, 1H), 1.68 - 1.48 (m, 6H), 1.45 - 1.28 (m, 2H), 1.18 (td, J = 7.2, 2.4 Hz, 3H), 1.06 - 0.56 (m, 7H), 0.08 (t, J = 6.0 Hz, 2H).

### Single-configuration Compound 55-2-2:

MS m/z (ESI):628.25 [M+1]
HPLC: retention time: 2.874 min; chromatographic column: Shim-pack Velox C18, 2.7um, 3.0*50 mm; mobile phase: water (0.1% trifluoroacetic acid)/acetonitrile (0.1% trifluoroacetic acid); elution gradient: 5%-95%.

Chiral SFC: retention time: 1.985 min; chromatographic column: DAICEL AD-H 4.6 mm I.D.*250 mm L 5 µm; mobile phase: CO₂/MeOH[0.1% NH₃ (7M methanol solution)] = 60/40.

'H NMR (400 MHz, DMSO-*d*₆) δ 7.83 (s, 1H), 7.64 - 7.55 (m, 2H), 7.40 (td, J = 8.4, 2.4 Hz, 1H), 7.36 - 7.27 (m, 1H), 7.13 - 7.04 (m, 1H), 6.95 (d, J = 6.8 Hz, 1H), 4.72 (brs, 1H), 4.19 (dt, J = 14.0, 7.2 Hz, 0.28H), 4.11 - 3.98 (m, 3H), 3.77 - 3.34 (m, 4H), 3.30 - 3.21 (m, 3H), 3.19 - 2.72 (m, 6H), 2.37 - 2.29 (m, 1H), 2.14 - 1.94 (m, 1H), 1.88 - 1.65 (m, 4H), 1.53 - 1.37 (m, 4H), 1.18 (t, J = 7.2 Hz, 3H), 1.01 - 0.57 (m, 7H), 0.08 (t, J = 6.0 Hz, 2H).

### Examples 56 and 57

### N-[4-({[1-(8-{2-[ethyl(isopropyl)aminoformyl]-4-fluorophenyll-3-methylimidazo[1,5-a]pyridin-6-yl)piperidin-4-yllamino } methyl)-4-fluorocyclohexyl]tert-butyl carbamate 56

### 2-[6-(4-{[(4-ethanesulfonamido-1-fluorocyclohexyl)methyl]amino}piperidin-1-yl)-3-methylimidazo[1,5-a]pyridin-8-yl]-N-ethyl-5-fluoro-N-(isopropyl)benzamide 57

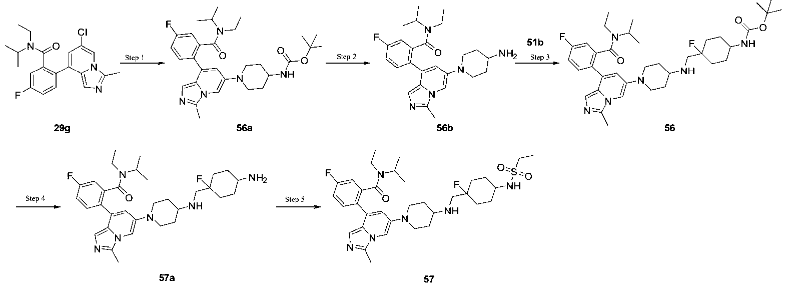

### Step 1

### N-[1-(8-{2-[ethyl(isopropyl)aminoformyl]-4-fluorophenyl}-3-methylimidazo[1,5-a]pyridin-6-yl)piperidin-4-yl]tert-butyl carbamate 56a

Compound **29g** (300 mg, 802.46 µmol), *N*-(piperidin-4-yl)tert-butyl carbamate (321.43 mg, 1.60 mmol) and cesium carbonate (784.37 mg, 2.41 mmol) were dissolved in tert-amyl alcohol (5 mL). Ruphos (74.89 mg, 160.49 µmol) and Pd₂ (dba)₃ (73.48 mg, 80.25 µmol) were added. Under a nitrogen atmosphere, the mixture was stirred at 90°Cto allow for reacting for 12 hours. The reaction mixture was cooled to room temperature, water (15 mL) was added, and the mixture was extracted with ethyl acetate (20 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **56a** (400 mg, 743.95 µmol, yield: 92.71%).
MS m/z (ESI): 538.2 [M+1]

### Step 2

### 2-[6-(4-aminopiperidin-1-yl)-3-methylimidazo[1,5-a]pyridin-8-yl]-N-ethyl-5-fluoro-N-(isopropyl)benzamide 56b

Compound **56a** (250.00 mg, 325.48 µmol) was dissolved in methane dioxide (3.0 mL), followed by the addition of trifluoroacetic acid (1.48 g, 12.98 mmol, 1 mL) at 0°C. The mixture was stirred at room temperature to allow for reacting for 1.0 hours. The reaction mixture was concentrated under a reduced pressure, to obtain the crude product of title Compound **56b** (200 mg), which was directly used in the next step of reaction without purification.
MS m/z (ESI): 438.1 [M+1]

### Step 3

### N-[4-({[1-(8-{2-[ethyl(isopropyl)aminoformyl]-4-fluorophenyl}-3-methylimidazo[1,5-a]pyridin-6-yl)piperidin-4-yl]amino}methyl)-4-fluorocyclohexyl]tert-butyl carbamate 56

The crude product of Compound **56b** (135 mg, 244.75 µmol, trifluoroacetate), Compound 51b(68 mg, 277.22 µmol) and triethylamine (74 mg, 731.30 µmol) were dissolved in methanol (3.0 mL), and stirred at room temperature for 1.0 hour. Sodium cyanoborohydride (47 mg, 747.91 µmol) was added, and the mixture was stirred at room temperature for 2.0 hours. A saturated aqueous sodium bicarbonate solution (10 mL) was added, and the mixture was extracted with dichloromethane (20 mL x 3). The organic phases were combined, washed with saturated brine, dried over anhydrous sodium sulfate and filtered, and the filtrate was concentrated under a reduced pressure. The resulting residues were purified by the HPLC (mobile phase: acetonitrile, water (0.1% ammonium bicarbonate), elution gradient: 41%-71%), to obtain the title Compound **56** (6.19 mg, 9.28 µmol, yield: 3.79%).
MS m/z (ESI): 667.2 [M+1]
¹H NMR (400 MHz, DMSO-*d*₆) δ 7.72 - 7.63 (m, 1H), 7.41 - 7.22 (m, 3H), 7.00 - 6.93 (m, 1H), 6.81 - 6.70 (m, 1H), 6.64 - 6.56 (m, 1H), 3.51 - 3.40 (m, 3H), 3.29 - 3.23 (m, 1H), 3.01 - 2.58 (m, 6H), 2.54 (s, 3H), 1.96 - 1.83 (m, 4H), 1.72 - 1.55 (m, 3H), 1.45 - 1.32 (m, 15H), 0.98 - 0.68 (m, 7H), 0.36 - 0.29 (m, 2H).

### Step 4

### 2-[6-(4-{[(4-amino-1-ffuorocyclohexyl)methyl]amino}piperidin-1-yl)-3-methylimidazo[1,5-a]pyridin-8-yl]-N-ethyl-5-fluoro-N-(isopropyl) benzamide 57a

Compound **56** (150.00 mg, 224.94 µmol) was dissolved in methane dioxide (3.0 mL), followed by the addition of trifluoroacetic acid (1.48 g, 12.98 mmol, 1 mL) at 0°C. The mixture was stirred at room temperature to allow for reacting for 0.5 hours. The reaction mixture was concentrated under a reduced pressure, to obtain the crude product of title Compound **57a** (130 mg), which was directly used in the next step of reaction without purification.
MS m/z (ESI): 567.2 [M+1]

### Step 5

### 2-[6-(4-{[(4-ethanesulfonamido-1-fluorocyclohexyl)methyl]amino}piperidin-1-yl)-3-methylimidazo[1,5-a]pyridin-8-yl]-N-ethyl-5-fluoro-N-(isopropyl)benzamide 57

The crude product of Compound **57a** (120 mg, 211.74 µmol, trifluoroacetate) and triethylamine (108 mg, 1.07 mmol) were dissolved in dichloromethane (2.0 mL), followed by the addition of ethanesulfonyl chloride (33 mg, 256.65 µmol), and the mixture was stirred at room temperature for 0.25 hours. A saturated aqueous sodium bicarbonate solution (10 mL) was added to the reaction mixture, which was then extracted with dichloromethane (20 mL × 3). The organic phases were combined, washed with saturated brine (40 mL), dried over anhydrous sodium sulfate, and concentrated under a reduced pressure. The resulting residues were purified by the HPLC (C18; mobile phase: acetonitrile, water (0.1% ammonium bicarbonate), elution gradient: 26%-56%), to obtain the title Compound **57** (13.21 mg, 20.05 µmol, yield: 9.47 %) as yellow solid.
MS m/z (ESI):659.3 [M+1]
¹H NMR (400 MHz, DMSO-*d*₆) δ 7.71 - 7.63 (m, 1H), 7.40 - 7.22 (m, 3H), 7.08 (d, J = 8.0 Hz, 1H), 6.99 - 6.93 (m, 1H), 6.63 - 6.56 (m, 1H), 3.50 - 3.37 (m, 3H), 3.32 - 3.27 (m, 1H), 3.15 - 3.04 (m, 1H), 3.03 - 2.87 (m, 3H), 2.86 - 2.57 (m, 5H), 2.54 (s, 3H), 1.95 - 1.81 (m, 4H), 1.78 - 1.67 (m, 2H), 1.58 - 1.45 (m, 4H), 1.42 - 1.31 (m, 2H), 1.19 (t, J = 7.2 Hz, 3H), 1.11 - 0.71 (m, 7H), 0.35 - 0.29 (m, 2H).

### Example 58

### 2-{6-[4-(cyclohexylmethyl)piperazin-1-yl]-3-methylimidazo[1,5-a]pyridin-8-yl}-N-ethyl-5-fluoro-N-(isopropyl)benzamide 58

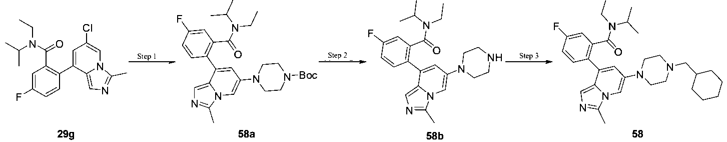

### Step 1

### 4-(8-{2-[ethyl(isopropyl)aminoformyl]-4-fluorophenyl}-3-methylimidazo[1,5-a]pyridin-6-yl)piperazin-1-tert-butyl carboxylate 58a

Compound **29g** (500 mg, 1.34 mmol), piperazin-1-tert-butyl carboxylate (498.20 mg, 2.67 mmol) and cesium carbonate (1.31 g, 4.01 mmol) were dissolved in tert-amyl alcohol (10 mL). Ruphos (124.82 mg, 267.49 µmol) and Pd₂ (dba)₃ (122.47 mg, 133.74 µmol) were added. Under a nitrogen atmosphere, the mixture was stirred at 95°Cto allow for reacting for 12 hours. The reaction mixture was cooled to room temperature, water (25 mL) was added, and the mixture was extracted with ethyl acetate (30 mL x 3). The organic phases were combined, washed with saturated brine (25 mL), dried over anhydrous sodium sulfate, and concentrated under a reduced pressure. The resulting residues were purified by the silica-gel column chromatography using petroleum ether/ethyl acetate as an elution system, to obtain the title Compound **58a** (660 mg, 1.26 mmol, yield: 94.24%).
MS m/z (ESI): 524.3 [M+1]
¹H NMR (400 MHz, DMSO-*d*₆) δ 7.72 - 7.61 (m, 1H), 7.43 - 7.27 (m, 3H), 7.01 - 6.94 (m, 1H), 6.60 (s, 1H), 3.49 - 3.37 (m, 5H), 3.31 - 3.28 (m, 1H), 2.92 (s, 5H), 2.55 (s, 3H), 1.42 (s, 9H), 1.11 - 0.72 (m, 7H), 0.36 - 0.27 (m, 2H).

### Step 2

### N-ethyl-5-fluoro-2-[3-methyl-6-(piperazin-1-yl)imidazo[1,5-a]pyridin-8-yl]-N-(isopropyl)benzamide 58b

Compound **58a** (50.00 mg, 95.49 µmol) was dissolved in methane dioxide (1.5 mL), followed by the addition of trifluoroacetic acid (740.00 mg, 6.49 mmol, 0.5 mL) at 0°C. The mixture was stirred at room temperature to allow for reacting for 1.0 hours. The reaction mixture was concentrated under a reduced pressure, and the resulting residues were purified by the HPLC (mobile phase: acetonitrile, water (0.1% ammonia water), elution gradient: 18%-48%), to obtain the title Compound **58b** (1.36 mg, 3.21 µmol, yield: 3.36%).
MS m/z (ESI): 424.3 [M+1]
¹H NMR (400 MHz, DMSO-*d*₆) δ 7.71 - 7.62 (m, 1H), 7.41 - 7.19 (m, 3H), 7.00 - 6.93 (m, 1H), 6.63 - 6.56 (m, 1H), 3.48 - 3.43 (m, 2H), 3.35 - 3.29 (m, 2H), 2.99 - 2.91 (m, 4H), 2.83 (br s, 4H), 2.58 - 2.52 (m, 3H), 1.09 - 0.72 (m, 7H), 0.34 - 0.28 (m, 2H).

### Step 3

### 2-{6-[4-(cyclohexylmethyl)piperazin-1-yl]-3-methylimidazo[1,5-a]pyridin-8-yl}-N-ethyl-5-fluoro-N-(isopropyl)benzamide 58

The crude product of Compound **58b** (129.01 mg, 240 µmol, trifluoroacetate), cyclohexane formaldehyde (33 mg, 294.20 µmol) and triethylamine (50 mg, 494.12 µmol) were dissolved in methanol (2.0 mL), and stirred at room temperature for 1.0 hour. Sodium cyanoborohydride (46 mg, 732 µmol) was added, and the mixture was stirred at room temperature for 11 hours. A saturated aqueous sodium bicarbonate solution (10 mL) was added, and the mixture was extracted with dichloromethane (20 mL x 3). The organic phases were combined, dried over anhydrous sodium sulfate and filtered, and the filtrate was concentrated under a reduced pressure. The resulting residues were purified by the HPLC (mobile phase: acetonitrile, water (0.1% ammonia water), elution gradient: 60%-90%), to obtain the title Compound **58** (48.8 mg, 93.9 µmol, yield: 39.13 %).
MS m/z (ESI): 520.2 [M+1]
¹H NMR (400 MHz, DMSO-*d*₆) δ 7.71 - 7.64 (m, 1H), 7.41 - 7.24 (m, 3H), 7.00 - 6.93 (m, 1H), 6.64 - 6.57 (m, 1H), 3.48 - 3.40 (m, 1H), 3.31 - 3.26 (m, 1H), 3.09 - 2.87 (m, 5H), 2.54 (s, 3H), 2.48 - 2.40 (m, 4H), 2.16 - 2.10 (m, 2H), 1.78 - 1.62 (m, 5H), 1.57 - 1.45 (m, 1H), 1.25 - 1.08 (m, 4H), 0.91 - 0.72 (m, 8H), 0.35 - 0.28 (m, 2H).

### Example 59

### N-ethyl-5-fluoro-2-(3-methyl-6-{4-[(1R,3S,4S)-2-azabicyclo[2.2.2]octan-3-carbonyl]piperazin-1-yl}imidazo[1,5-a]pyridin-8-yl)-N-(isopropyl)benzamide 59

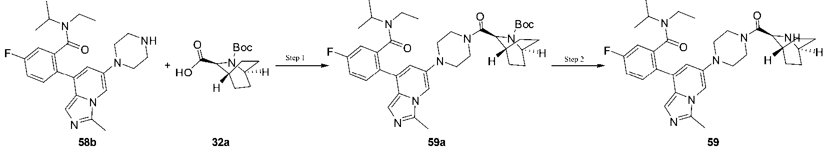

### Step 1

### (1R,3S,4S)-3-[4-(8-{2-[ethyl(isopropyl)aminoformyl]-4-fluorophenyl}-3-methylimidazo[1,5-a]pyridin-6-yl)piperazin-1-carbonyl]-2-azabicyclo[2.2.2]octan-2-tert-butyl carboxylate 59a

(1*R*,3*S*,4*S*)-2-[(tert-butoxy)carbonyl]-2-azabicyclo[2.2.2]octan-3-carboxylic acid **32a**(68 mg, 266.34 µmol) and *N,N*-diisopropylethylamine (157 mg, 1.21 mmol, 211.59 µL) were dissolved in DCM (2.0 mL). The mixture was cooled to 0°C, followed by the addition of EDCI (62 mg, 323.42 µmol), HOBT (50 mg, 370.03 µmol) and Compound **58b** (130 mg, 241.84 µmol, trifluoroacetate ), and the mixture was stirred at room temperature for 12 hours. A saturated aqueous sodium bicarbonate solution (10 mL) was added to the reaction mixture, which was then extracted with dichloromethane (20 mL x 3). The organic phases were combined, dried over anhydrous sodium sulfate, and concentrated under a reduced pressure. The resulting residues were purified by the preparative thin-layer chromatography using dichloromethane/methanol as an elution system, to obtain the title Compound **59a** (100 mg, 151.3 µmol, yield: 62.6%).
MS m/z (ESI): 661.2 [M+1]

### Step 2

### N-ethyl-5-fluoro-2-(3-methyl-6-{4-[(1R,3S,4S)-2-azabicyclo[2.2.2]octan-3-carbonyl]piperazin-1-yl}imidazo[1,5-a]pyridin-8-yl)-N-(isopropyl)benzamide 59

Compound **59a** (100 mg, 151.33 µmol) was dissolved in methane dioxide (3.0 mL) and trifluoroacetic acid (1.0 mL). The mixture was stirred at room temperature to allow for reacting for 1.0 hours. The reaction mixture was concentrated under a reduced pressure, and purified by the HPLC (mobile phase: acetonitrile, water (0.1% ammonia water), elution gradient: 30%-56%), to obtain the title Compound **59** (37.9 mg, 67.6 µmol, yield: 44.7%) as yellow solid.
MS m/z (ESI): 561.6 [M+1]
¹H NMR (400 MHz, DMSO-*d*₆) δ 7.70 - 7.63 (m, 1H), 7.41 - 7.29 (m, 3H), 7.00 - 6.95 (m, 1H), 6.64 - 6.58 (m, 1H), 3.91 (s, 1H), 3.75 - 3.49 (m, 4H), 3.48 - 3.40 (m, 1H), 3.09 - 2.74 (m, 7H), 2.55 (s, 3H), 1.84 - 1.40 (m, 9H), 1.12 - 0.73 (m, 7H), 0.34 - 0.27 (m, 2H).

### Example 60

### N-ethyl-5-fluoro-2-{1-methyl-6-[3-({[(1r,4r)-4-ethanesulfonamidocyclohexyl]methyl}amino)azacyclobutan-1-yl]-1H-indazol-4-yl}-N-(isopropyl)benzamide 60

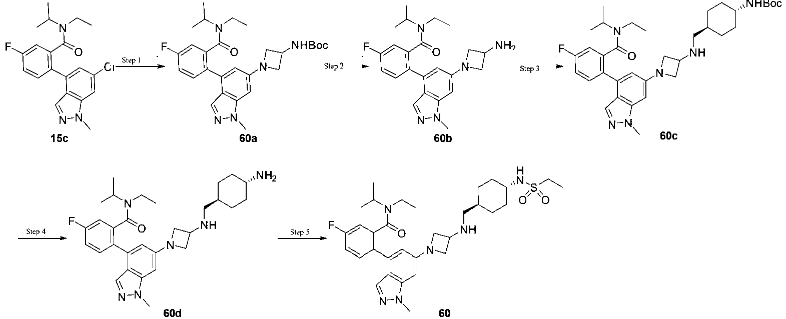

### Step 1

### N-[1-(4-{2-[ethyl(isopropyl)aminoformyl]-4-fluorophenyl}-1-methyl-1H-indazol-6-yl)azacyclobutan-3-yl]tert-butyl carbamate 60a

Compound **15c** (143 mg, 267 µmol) and *N*-(azacyclobutan-3-yl)tert-butyl carbamate (46.1 mg, 267 µmol) were dissolved in toluene (0.9 mL) and tertiary butanol (0.3 mL). Pd(Oac)₂ (6.01 mg, 26.7 µmol), Xphos(25.5 mg, 53.5 µmol) and sodium tert-butoxide (38.6 mg, 401 µmol) were added, and the mixture was stirred at 100°C under a nitrogen atmosphere to allow for reacting for 8.0 hours. The reaction mixture was cooled to room temperature, and concentrated under a reduced pressure. The resulting residues were purified by the preparative thin-layer chromatography using petroleum ether/ethyl acetate as an elution system, to obtain the title Compound **60a** (90 mg, 177 µmol, yield: 66.0%) as white solid.
MS m/z (ESI): 510.25 [M+1]
¹H NMR (400 MHz, DMSO-*d*₆) δ 7.66 - 7.51 (m, 3H), 7.37 (td, J = 8.8, 2.8 Hz, 1H), 7.26 (dd, J = 9.2, 2.8 Hz, 1H), 6.41 (s, 1H), 6.33 (d, J = 1.6 Hz, 1H), 4.44 - 4.19 (m, 1H), 4.14 - 4.06 (m, 2H), 3.91 (d, J = 3.6 Hz, 3H), 3.64 (t, J = 6.4 Hz, 2H), 3.42 - 3.34 (m, 1H), 3.29 - 3.21 (m, 2H), 1.40 (s, 9H), 0.94 - 0.63 (m, 7H), 0.16 (d, J = 6.4 Hz, 2H).

### Step 2

### 2-[6-(3-aminoazacyclobutan-1-yl)-1-methyl-1H-indazol-4-yl]-N-ethyl-5-fluoro-N-(isopropyl)benzamide 60b

Compound **60a** (85.0 mg, 167 µmol) was dissolved in methane dioxide (1.0 mL), followed by the addition of trifluoroacetic acid (0.2 mL) at 0°C. The mixture was stirred at 0°C to allow for reacting for 1.0 hour. The reaction mixture was concentrated under a reduced pressure, to obtain the crude product of title Compound **60b** (110 mg), which was directly used in the next step of reaction without purification.
MS m/z (ESI): 410.2 [M+1]

### Step 3

### N-[(1r,4r)-4-({[1-(4-{2-[ethyl(isopropyl)aminoformyl]-4-fluorophenyl}-1-methyl-1H-indazol-6-yl)azacyclobutan-3-yl]amino}methyl)cyclohexyl]tert-butyl carbamate 60c

The crude product of Compound **60a** (110 mg) and *N*-[(1*r*,4*r*)-4-formylcyclohexyl]tert-butyl carbamate (37.9 mg, 167 µmol) were dissolved in dichloromethane (1.0 mL). Sodium triacetoxyborohydride (106 mg, 500 µmol) was added, and the mixture was stirred at room temperature for 12 hours. The reaction mixture was concentrated under a reduced pressure. The resulting residues were purified by the preparative thin-layer chromatography using petroleum ether/ethyl acetate as an elution system, to obtain the title Compound **60c** (30.0 mg, 103 µmol, yield: 28.9%) as yellow solid.
MS m/z (ESI): 621.30 [M+1]

### Step 4

### N-ethyl-5-fluoro-2-{1-methyl-6-[3-({[(1r,4r)-4-aminocyclohexyl]methyl}amino)azacyclobutan-1-yl]-1H-indazol-4-yl}-N-(isopropyl)benzamide 60d

Compound **60c** (25.0 mg, 40.3 µmol) was dissolved in methane dioxide (0.2 mL), followed by the addition of trifluoroacetic acid (0.05 mL) at 0°C. The mixture was stirred at 0°C to allow for reacting for 1.0 hour. The reaction mixture was concentrated under a reduced pressure, to obtain the crude product of title Compound **60d** (50 mg), which was directly used in the next step of reaction without purification.
MS m/z (ESI): 521.25 [M+1]

### Step 5

### N-ethyl-5-fluoro-2-{1-methyl-6-[3-({[(1r,4r)-4-aminocyclohexyl]methyl}amino)azacyclobutan-1-yl]-1H-indazol-4-yl}-N-(isopropyl)benzamide 60

The crude product of Compound **60d** (50 mg) and diisopropylethylamine ((20.8 mg, 161 µmol) were dissolved in DMF(0.5 mL), followed by the addition of the solution of ethanesulfonyl chloride (5.19 mg, 40.3 µmol) in DMF(0.2 mL), and the mixture was stirred at room temperature for 2 hours. The reaction mixture was concentrated under a reduced pressure, and the resulting residues were purified by the HPLC (C18; mobile phase: acetonitrile, water (0.1% trifluoroacetic acid), elution gradient: 10%-35%), to obtain the title Compound 60 (3.0 mg, 4.90 µmol, yield: 12.1%) as white solid.
MS m/z (ESI): 613.25 [M+1]
¹H NMR (400 MHz, DMSO-*d*₆) δ 7.50 - 7.37 (m, 2H), 7.22 (td, J = 8.8, 2.8 Hz, 1H), 7.13 (td, J = 8.8, 2.8 Hz, 1H), 6.83 (d, J = 7.6 Hz, 1H), 6.30 - 6.12 (m, 2H), 4.14 - 4.06 (m, 0.27H), 3.89 (q, J = 6.8 Hz, 2H), 3.76 (d, J = 3.6 Hz, 3H), 3.51 (dq, J = 12.8, 6.4 Hz, 1H), 3.35 (d, J = 6.4 Hz, 2H), 2.86 - 2.78 (m, 3H), 2.75 - 2.66 (m, 1H), 2.17 (d, J = 6.8 Hz, 2H), 1.72 (d, J = 10.4 Hz, 2H), 1.63 (d, J = 15.2 Hz, 2H), 1.21 - 0.99 (m, 7H), 0.96 - 0.86 (m, 1H), 0.84 - 0.68 (m, 5H), 0.65 - 0.51 (m, 3.72H), 0.01 (d, J = 6.4 Hz, 2.33H).

### Example 61

### N-ethyl-5-fluoro-2-[6-(3-fluoro-1-{[(1r,4r)-4-ethanesulfonamidocyclohexyl]methyl}pyrrolidin-3-yl)-1-methyl-1H-indazol-4-yl]-N-(isopropyl) benzamide 61

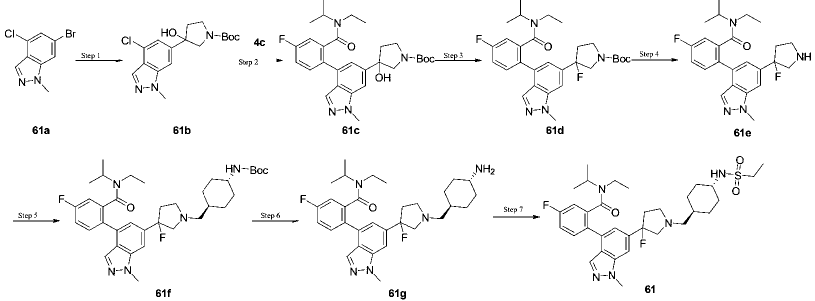

### Step 1

### 3-(4-chloro-1-methyl-1H-indazol-6-yl)-3-hydroxylpyrrolidin-1-tert-butyl carboxylate 61b

6-Bromo-4-chloro-1-methylindazole **61a** (1.80 g, 7.33 mmol) was dissolved in THF(36 mL), to which isopropylmagnesium chloride (2M, 7.80 mL) was dropwise added at 0°C, followed by reacting for 2 hours at 50°C. The above mixture was cooled to 0°C, followed by the addition of the solution of 3-oxopyrrolidin-1-tert-butyl carboxylate (4.08 g, 22.02 mmol) in THF(4.0 mL), and the resulting mixture was allowed to react at 22°C for 12 hours. The reaction mixture was quenched with water (100 mL), extracted with ethyl acetate (100.0 mL × 3), dried over anhydrous sodium sulfate, and filtered, and the filtrate was concentrated under a reduced pressure. The resulting residues were purified by the silica-gel column chromatography using petroleum ether/ethyl acetate as an elution system. to obtain the crude product of title Compound **61b** (3.15 g, 8.95 mmol, yield: 122%) as yellow solid.
MS m/z (ESI): 352.2 [M+1]

### Step 2

### 3-(4-{2-[ethyl(isopropyl)aminoformyl]-4-fluorophenyl}-1-methyl-1H-indazol-6-yl)-3-hydroxylpyrrolidin-1-tert-butyl carboxylate 61c

Compound **61b** (1.6 g, 3.18 mmol) and Compound **4c**(1.68 g, 3.50 mmol) were dissolved in dioxane (60 mL) and water (10 mL). Sodium carbonate (675 mg, 6.37 mmol) and Pd(dppf)Cl₂ (233 mg, 318 µmol) were added, and the mixture was stirred at 100 °C under a nitrogen atmosphere to allow for reacting for 6.0 hours. The reaction mixture was cooled to room temperature and filtered, and the filtrate was concentrated under a reduced pressure. The resulting residues were purified by the silica-gel column chromatography using dichloromethane/methanol as an elution system, to obtain the title Compound **61c** (470 mg, 896 µmol, yield: 28.1%) as yellow solid.
MS m/z (ESI): 525.3 [M+1]
¹H NMR (400 MHz, DMSO-*d*₆) δ 7.86 - 7.73 (m, 2H), 7.59 (m, 1H), 7.43 - 7.26 (m, 2H), 7.24 - 7.17 (m, 1H), 5.57 (d, J = 10.4 Hz, 1H), 4.27 - 4.18 (m, 0.19H), 4.06 (s, 3H), 3.65 - 3.34 (m, 5H), 3.26 - 3.12 (m, 1H), 2.85 (m, 1H), 2.39 - 2.23 (m, 1H), 2.08 - 2.00 (m, 1H), 1.41 (dd, J = 13.2, 4.8 Hz, 10H), 1.03 - 0.80 (m, 4H), 0.67 (m, 3H), 0.11 (d, J = 6.0 Hz, 2H).

### Step 3

### 3-(4-{2-[ethyl(isopropyl)aminoformyl]-4-fluorophenyl}-1-methyl-1H-indazol-6-yl)-3-fluoropyrrolidin-1-tert-butyl carboxylate 61d

Compound **61c** (200 mg, 381.2 µmol) was dissolved in dichloromethane (4.0 mL), and cooled to -78°C, followed by the dropwise addition of the solution of DAST (73.74 mg, 457.47 µmol) in dichloromethane (1 mL). The mixture was stirred at -45°C to allow for reacting for 2.0 hour. The reaction mixture was quenched by adding water (10 mL), and extracted with ethyl acetate (20 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **61d** (199 mg, 378 µmol, yield: 99%) as yellow solid.
MS m/z (ESI): 527.2 [M+1]

### Step 4

### N-ethyl-5-fluoro-2-[6-(3-fluoropyrrolidin-3-yl)-1-methyl-1H-indazol-4-yl]-N-(isopropyl)benzamide 61e

Compound **61d** (110 mg, 208 µmol) was dissolved in methane dioxide (4.0 mL), followed by the addition of trifluoroacetic acid (1.0 mL) at 0°C. The mixture was stirred at room temperature to allow for reacting for 1.0 hours. The reaction mixture was concentrated under a reduced pressure, to obtain the crude product of title Compound **61e** (135 mg). The crude product was directly used in the next step of reaction without purification.
MS m/z (ESI): 427.2 [M+1]

### Step 5

### N-[(1r,4r)-4-{[3-(4-{2-[ethyl(isopropyl)aminoformyl]-4-fluorophenyl}-1-methyl-1H-indazol-6-yl)-3-fluoropyrrolidin-1-yl]methyl} cyclohexyl]tert-butyl carbamate 61f

The crude product of Compound **61e** (135 mg) and *N*-[(1*r*,4*r*)-4-formylcyclohexyl]tert-butyl carbamate (47.4 mg, 208 µmol) were dissolved in dichloromethane (10.0 mL), and stirred at room temperature for 0.5 hours. The reaction mixture was cooled to 0°C, followed by adding sodium triacetoxyborohydride (221 mg, 1.04 mmol) and stirring at room temperature for 12 hours. The reaction mixture was concentrated under a reduced pressure, and the residues were purified by the silica-gel column chromatography using dichloromethane/methanol as an elution system, to obtain the title Compound **61f** (90 mg, 141 µmol, yield: 67.5%) as yellow solid.
MS m/z (ESI): 638.2 [M+1]

### Step 6

### N-ethyl-5-fluoro-2-[6-(3-fluoro-1-{[(1r,4r)-4-aminocyclohexyl]methyl}pyrrolidin-3-yl)-1-methyl-1H-indazol-4-yl]-N-(isopropyl)benzamide 61g

Compound **61f** (90 mg, 141 µmol) was dissolved in methane dioxide (4.0 mL), followed by the addition of trifluoroacetic acid (1.0 mL) at 0°C. The mixture was stirred at room temperature to allow for reacting for 1.0 hours. The reaction mixture was concentrated under a reduced pressure, to obtain the crude product of title Compound **61g** (102 mg). The crude product was directly used in the next step of reaction without purification.
MS m/z (ESI): 538.1 [M+1]

### Step 7

### N-ethyl-5-fluoro-2-[6-(3-fluoro-1-{[(1r,4r)-4-ethanesulfonamidocyclohexyl]methyl}pyrrolidin-3-yl)-1-methyl-1H-indazol-4-yl]-N-(isopropyl)benzamide 61

The crude product of Compound **61g** (102 mg) and DIPEA (182 mg, 1.41 mmol) were dissolved in DMF(5.0 mL), and cooled to 0°C. Ethanesulfonyl chloride (18.1 mg, 141 µmol) was added, and the mixture was stirred at room temperature for 1.0 hour. The reaction mixture was concentrated under a reduced pressure, the resulting residues were purified by the silica-gel column chromatography using dichloromethane/methanol as an elution system, and the obtained crude product was purified by the HPLC (mobile phase: acetonitrile, water (0.1% formic acid), elution gradient: 25%-40%), to obtain the title Compound **61** (39.8 mg, 63.1 µmol, yield: 43.2 %) as white solid.
MS m/z (ESI):630.1 [M+1]
¹H NMR (400 MHz, DMSO-*d*₆) δ 7.87 (d, J = 5.2 Hz, 1H), 7.70 (s, 1H), 7.64 - 7.56 (m, 1H), 7.40 (td, J = 8.4, 2.4 Hz, 1H), 7.36 - 7.28 (m, 1H), 7.13 (s, 1H), 7.05 - 6.94 (m, 1H), 4.26 - 4.18 (m, 0.20H), 4.08 (d, J = 4.0 Hz, 3H), 3.43 - 3.37 (m, 1H), 3.26 - 3.19 (m, 1H), 3.14 - 2.79 (m, 7H), 2.73 - 2.69 (m, 0.80H), 2.40 - 2.36 (m, 1H), 2.34 - 2.25 (m, 3H), 1.90 - 1.83 (m, 2H), 1.82 - 1.76 (m, 1H), 1.60 - 1.12 (m, 7H), 1.07 - 0.59 (m, 9H), 0.13 (t, J = 6.4 Hz, 2H).

### Example 62

### (1R,3S,4S)- 3-[4-(8-{2-[(2R,5S)-2,5-dimethylpyrrolidin-1-carbonyl]-4-fluorophenyl}-3-methylimidazo[1,5-a]pyridin-6-yl)piperazin-1-carbyl]-2-azabicyclo[2.2.2]octane 62

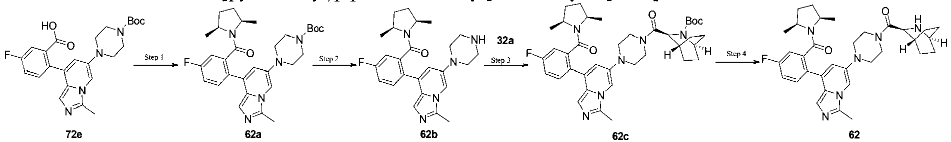

### Step 1

### 4-(8-{2-[(2R,5S)-2,5-dimethylpyrrolidin-1-carbonyl]-4-fluorophenyl}-3-methylimidazo[1,5-a]pyridin-6-yl)piperazin-1-tert-butyl carboxylate 62a

(2*R*,5*S*)-2,5-dimethylpyrrolidine (39.39 mg, 290.43 µmol, hydrochloride) and Compound **72e** (120 mg, 264.03 µmol) were dissolved in DMF (2.0 mL). DIPEA (102.37 mg, 792.09 µmol), EDCI (75.92 mg, 396.05 µmol) and HOBt (53.51 mg, 396.05 µmol) were added, and the mixture was stirred at room temperature to allow for reacting for 12.0 hours. The reaction mixture was concentrated under a reduced pressure. The resulting residues were purified by the preparative thin-layer chromatography using dichloromethane/methanol as an elution system, to obtain the title Compound **62a** (110 mg, 205.36 µmol, yield: 77.8%).
MS m/z (ESI): 536.4 [M+1]

### Step 2

### 1-(8-{2-[(2R,5S)-2,5-dimethylpyrrolidin-1-carbonyl]-4-fluorophenyl}-3-methylimidazo[1,5-a]pyridin-6-yl)piperazine 62b

Compound **62a** (110 mg, 205.36 µmol) was dissolved in methane dioxide (3.0 mL), followed by the addition of trifluoroacetic acid (1.48 g, 12.98 mmol, 1 mL). The mixture was stirred at room temperature to allow for reacting for 1.0 hours. The reaction mixture was concentrated under a reduced pressure, to obtain the crude product of title Compound **62b** (100 mg). The crude product was directly used in the next step of reaction without purification.
MS m/z (ESI): 436.2 [M+1]

### Step 3

### (1R,3S,4S)-3-[4-(8-{2-[(2R,5S)-2,5-dimethylpyrrolidin-1-carbonyl]-4-fluorophenyl}-3-methylimidazo[1,5-a]pyridin-6-yl)piperazin-1-carbonyl]-2-azabicyclo[2.2.2]octan-2-tert-butyl carboxylate 62c

Compound **32a(44** mg, 172.34 µmol) and N,N-diisopropylethylamine (64 mg, 495.19 µmol) were dissolved in DCM (2.0 mL). The mixture was cooled to 0°C, followed by the addition of EDCI (48 mg, 250.39 µmol), HOBT (33 mg, 244.22 µmol) and Compound **62b** (90 mg, 163.77 µmol, trifluoroacetate ), and the mixture was stirred at room temperature for 12 hours. The reaction mixture was concentrated under a reduced pressure. The resulting residues were purified by the preparative thin-layer chromatography using dichloromethane/methanol as an elution system, to obtain the title Compound **62c** (80 mg, 118.90 µmol, yield: 72.60%).
MS m/z (ESI): 673.3 [M+1]

### Step 4

### (1R,3S,4S)-3-[4-(8-{2-[(2R,5S)-2,5-dimethylpyrrolidin-1-carbonyl]-4-fluorophenyl}-3-methylimidazo[1,5-a]pyridin-6-yl)piperazin-1-carbyl]-2-azabicyclo[2.2.2]octane 62

Compound **62c** (80 mg, 118.9 µmol) was dissolved in methane dioxide (3.0 mL), followed by the addition of trifluoroacetic acid (13.56 mg, 118.90 µmol). The mixture was stirred at room temperature to allow for reacting for 1.0 hours. The reaction mixture was concentrated under a reduced pressure, and the resulting residues were purified by the preparative thin-layer chromatography using dichloromethane/methanol as an elution system, to obtain the title Compound **62** (4.01 mg, 7.00 µmol, yield: 5.89%).
MS m/z (ESI): 573.4 [M+1]
¹H NMR (400 MHz, CD₃OD) δ 7.80 - 7.74 (m, 1H), 7.37 - 7.34 (m, 2H), 7.32 - 7.27 (m, 1H), 7.18 (s, 1H), 6.87 - 6.81 (m, 1H), 4.56 - 4.54 (m, 1H), 3.72 - 3.65 (m, 3H), 3.57 - 3.46 (m, 3H), 3.19 - 3.08 (m, 4H), 2.66 (s, 3H), 2.23 - 2.18 (m, 2H), 2.06 - 2.03 (m, 2H), 1.88 - 1.84 (m, 2H), 1.63 - 1.56 (m, 4H), 1.37 - 1.32 (m, 7H), 0.92 - 0.88 (m, 3H).

### Example 65

### N-ethyl-5-fluoro-2-[3-methyl-6-(4-{2-[(1r,4r) -4-ethanesulfonamidocyclohexyl]ethyl}piperazine -1-yl)imidazo[1,5-a]pyridin-8-yl]-N-(isopropyl)benzamide 65

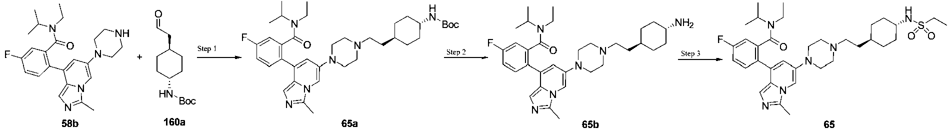

### Step 1

### N-[(1r,4r)-4-{2-[4-(8-{2-[ethyl(isopropyl)aminoformyl]-4-fluorophenyl}-3-methylimidazo[1,5-a]pyridin-6-yl)piperazin-1-yl]ethyl}cyclohexyl]tert-butyl carbamate 65^{a}

Compound **58b** (102.13 mg, 190 µmol, trifluoroacetate), *N*-[(1*r*,4*r*)-4-(2-oxoethyl)cyclohexyl]tert-butyl carbamate **160a** (55.02 mg, 228.00 µmol) and triethylamine (19.23 mg, 190.00 µmol) were dissolved in methanol (2.0 mL), and stirred at room temperature for 1.0 hour. Sodium cyanoborohydride (35.82 mg, 570.00 µmol) was added, and the mixture was stirred at room temperature for 11 hours. A saturated aqueous sodium bicarbonate solution (10 mL) was added to the reaction mixture, which was then extracted with dichloromethane (20 mL x 3). The organic phases were combined, washed with saturated brine (20 mL), dried over anhydrous sodium sulfate, and concentrated under a reduced pressure. The resulting residues were purified by the preparative thin-layer chromatography using dichloromethane/methanol as an elution system, to obtain the title Compound **65a** (70 mg, 107.88 µmol, yield: 56.78%) as brown oil.
MS m/z (ESI): 649.2 [M+1]

### Step 2

### N-ethyl-5-fluoro-2-[3-methyl-6-(4-{2-[(1r,4r)-4-aminocyclohexyl]ethyl}piperazine-1-yl)imidazo[1,5-a]pyridin-8-yl]-N-(isopropyl)benzamide 65b

Compound **65a** (75 mg, 115.59 µmol) was dissolved in dichloromethane (1.5 mL), followed by the addition of trifluoroacetic acid (740.00 mg, 6.49 mmol, 0.5 mL) at 25°C. The mixture was stirred at room temperature to allow for reacting for 30 min. The reaction mixture was concentrated under a reduced pressure, to obtain the crude product of title Compound **65b** (70 mg). The crude product was directly used in the next step of reaction without purification. MS m/z (ESI): 549.2 [M+1]

### Step 3

### N-ethyl-5-fluoro-2-[3-methyl-6-(4-{2-[(1r,4r)-4-ethanesulfonamidocyclohexyl]ethyl}piperazine-1-yl)imidazo[1,5-a]pyridin-8-yl]-N-(isopropyl)benzamide 65

The crude product of Compound **65b** (70 mg) was dissolved in dichloromethane (2.0 mL), followed by the addition of triethylamine (58.48 mg, 577.95 µmol), cooling to 0°C and the slow dropwise addition of ethanesulfonyl chloride (29.72 mg, 231.18 µmol), and the mixture was stirred at room temperature for 30 min. Water (20 mL) was added to the reaction mixture, which was then extracted with ethyl acetate (20 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **65** (13.48 mg, 21.03 µmol, yield: 18.20%).
MS m/z (ESI): 641.5 [M+1]
¹H NMR (400 MHz, DMSO-*d₆*) δ 7.72 - 7.63 (m, 1H), 7.41 - 7.20 (m, 3H), 7.03 - 6.94 (m, 2H), 6.64 - 6.58 (m, 1H), 3.51 - 3.39 (m, 1H), 3.30 - 3.23 (m, 1H), 3.06 - 2.87 (m, 8H), 2.55 (s, 3H), 2.48 (s, 4H), 2.33 (t, J=7.3 Hz, 2H), 1.91 - 1.80 (m, 2H), 1.79 - 1.65 (m, 2H), 1.40 - 1.30 (m, 2H), 1.22 - 1.15 (m, 5H), 1.04 - 0.70 (m, 10H), 0.32 (d, J=6.6 Hz, 2H).

### Example 67

### N-ethyl-5-fluoro-2-(3-methyl-6-{4-[(1R,3S,4S)-2-azabicyclo[2.2.2]octan-3-carbonyl]-1,4-diazacycloheptan-1-yl}imidazo[1,5-a]pyridin-8-yl)-N-(isopropyl)benzamide 67

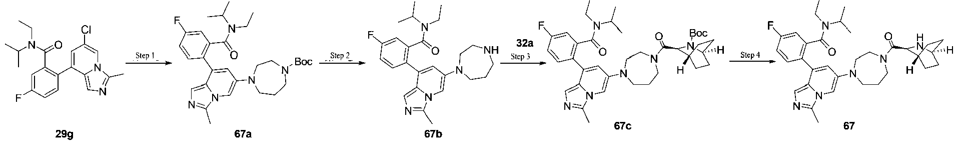

### Step 1

### 4-(8-{2-[ethyl(isopropyl)aminoformyl]-4-fluorophenyl}-3-methylimidazo[1,5-a]pyridin-6-yl)-1,4-diazacycloheptan-1-tert-butyl carboxylate 67a

Compound **29g** (300 mg, 802.5 µmol), 1,4-diazacycloheptan-1-tert-butyl carboxylate (321.43 mg, 1.60 mmol) and cesium carbonate (784.37 mg, 2.41 mmol) were dissolved in tert-amyl alcohol (8.0 mL). Ruphos (74.89 mg, 160.49 µmol) and Pd₂ (dba)₃ (73.48 mg, 80.25 µmol) were added. Under a nitrogen atmosphere, the mixture was stirred at 90°Cto allow for reacting for 12 hours. The reaction mixture was cooled to room temperature, water (25 mL) was added, and the mixture was extracted with ethyl acetate (20 mL x 3). The organic phases were combined, washed with saturated brine (20 mL), dried over anhydrous sodium sulfate, and concentrated under a reduced pressure. The resulting residues were purified by the silica-gel column chromatography using petroleum ether/ethyl acetate as an elution system, to obtain the title Compound **67a** (379 mg, 704.90 µmol, yield: 87.84%).
MS m/z (ESI): 538.3 [M+1]

### Step 2

### 2-[6-(1,4-diazacycloheptan-1-yl)-3-methylimidazo[1,5-a]pyridin-8-yl]-N-ethyl-5-fluoro-N-(isopropyl)benzamide 67b

Compound **67a** (120.00 mg, 223.19 µmol) was dissolved in methane dioxide (3.0 mL), followed by the addition of trifluoroacetic acid (1.48 g, 12.98 mmol, 1.0 mL) at 0°C. The mixture was stirred at room temperature to allow for reacting for 1.0 hours. The reaction mixture was concentrated under a reduced pressure, to obtain the crude product of title Compound **67b** (101 mg).
MS m/z (ESI): 438.2 [M+1]

### Step 3

### (1R,3S,4S)-3-[4-(8-{2-[ethyl(isopropyl)aminoformyl]-4-fluorophenyl}-3-methylimidazo[1,5-a]pyridin-6-yl)-1,4-diazacycloheptan-1-carbonyl]-2-azabicyclo[2.2.2]octan-2-tert-butyl carboxylate 67c

Compound **32a**(49.72 mg, 194.76 µmol) and *N,N*-diisopropylethylamine (160.17 mg, 1.24 mmol, 215.87 µL) were dissolved in DCM (2.0 mL). The mixture was cooled to 0°C, followed by the addition of EDCI (51.25 mg, 267.35 µmol), HOBT (36.60 mg, 270.89 µmol) and Compound **67b** (97.66 mg, 177.05 µmol, trifluoroacetate ), and the mixture was stirred at room temperature for 12 hours. Water (10 mL) was added to the reaction mixture, which was then extracted with dichloromethane (15 mL x 3). The organic phases were combined, dried over anhydrous sodium sulfate, and concentrated under a reduced pressure. The resulting residues were purified by the HPLC (mobile phase: acetonitrile, water (0.5% ammonia water), elution gradient: 37%-67%), to obtain the title Compound **67c** (79 mg, 117.06 µmol, yield: 66.12 %).
MS m/z (ESI): 675.3 [M+1]

### Step 4

### N-ethyl-5-fluoro-2-(3-methyl-6-{4-[(1R,3S,4S)-2-azabicyclo[2.2.2]octan-3-carbonyl]-1,4-diazacycloheptan-1-yl}imidazo[1,5-a]pyridin-8-yl)-N-(isopropyl)benzamide 67

Compound **67c** (55 mg, 81.5 µmol) was dissolved in methane dioxide (1.5 mL) and trifluoroacetic acid (0.5 mL). The mixture was stirred at room temperature to allow for reacting for 1.0 hours. The reaction mixture was concentrated under a reduced pressure, and purified by the HPLC (mobile phase: acetonitrile, water (0.1% ammonia water), elution gradient: 30%-60%), to obtain the title Compound **67** (0.34 mg, yield: 0.7%).
MS m/z (ESI): 575.5 [M+1]
¹H NMR (400 MHz, CD₃OD) δ 7.72 - 7.58 (m, 1H), 7.21 - 6.97 (m, 4H), 6.76 - 6.63 (m, 1H), 4.01 - 3.76 (m, 3H), 3.60 - 3.32 (m, 10H), 3.00 - 2.86 (m, 3H), 2.83 - 2.71 (m, 1H), 2.33 - 2.26 (m, 1H), 1.93 - 1.72 (m, 5H), 1.53 - 1.49 (m, 1H), 1.24 - 1.18 (m, 2H), 0.90 - 0.79 (m, 7H), 0.25 - 0.16 (m, 2H).

### Example 68

### N-ethyl-5-fluoro-2-[6-(3-fluoro-1-{[(1s,4s)-4-ethanesulfonamido-1-hydroxylcyclohexyl]methyl}pyrrolidin-3-yl)-1-methyl-1H-indazol-4-yl]-N-(isopropyl)benzamide 68

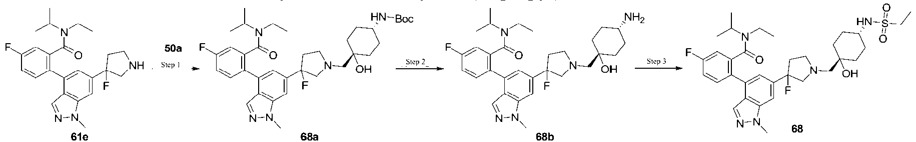

### Step 1

### N-[(1s,4s)-4-{[3-(4-{2-[ethyl(isopropyl)aminoformyl]-4-fluorophenyl}-1-methyl-1H-indazol-6-yl)-3-fluoropyrrolidin-1-yl]methyl}-4-hydroxylcyclohexyl]tert-buty1 carbamate 68a

Compound **61e** (60.0 mg, 141 µmol), *N*-{1-oxaspiro[2.5]octan-6-yl}tert-buty1 carbamate **50a**(44.8 mg, 197 µmol) and DIPEA(36.4 mg, 281 µmol, 49.0 µL) were dissolved in ethanol (3.0 mL), and the mixture was stirred at 60°C for 6.0 hours. Compound **50a** (44.8 mg, 197 µmol) was supplemented, and the mixture was stirred at 60°C for 12 hours. The reaction mixture was concentrated under a reduced pressure. The resulting residues were purified by the preparative thin-layer chromatography using petroleum ether/ethyl acetate as an elution system, to obtain the title Compound **68a** (56.7 mg, 86.7 µmol, yield: 61.6%) as yellow solid.
MS m/z (ESI): 654.3 [M+1]
¹H NMR (400 MHz, DMSO-*d₆*) δ 7.86 (d, J = 3.6 Hz, 1H), 7.70 (s, 1H), 7.60 (m, 1H), 7.43 - 7.25 (m, 2H), 7.15 (s, 1H), 6.69 (s, 1H), 4.22 (s, 0.26H), 4.08 (d, J = 4.0 Hz, 3H), 3.86 (s, 1H), 3.44 - 3.34 (m, 1H), 3.25 - 3.10 (m, 3H), 3.02 (s, 1H), 2.97 - 2.78 (m, 2H), 2.48 - 2.35 (m, 3H), 2.26 (s, 1H), 1.64 - 1.43 (m, 6H), 1.42 - 1.30 (m, 11H), 1.01 (d, J = 14.4 Hz, 1H), 0.87 (d, J = 6.8 Hz, 3H), 0.67 (m, 3H), 0.17 - 0.09 (m, 2H).

### Step 2

### N-ethyl-5-fluoro-2-[6-(3-fluoro-1-{[(1s,4s)-4-amino-1-hydroxylcyclohexylmethyl}pyrrolidin-3-yl)-1-methyl-1H-indazol-4-yl]-N-(isopropyl)benzamide 68b

Compound **68a** (50.0 mg, 76.5 µmol) was dissolved in methane dioxide (2.0 mL), followed by the addition of trifluoroacetic acid (0.4 mL) at 0°C. The mixture was stirred at room temperature to allow for reacting for 1.0 hour. The reaction mixture was concentrated under a reduced pressure, to obtain the crude product of title Compound **68b** (40 mg), which was directly used in the next step of reaction without purification.
MS m/z (ESI): 554.1 [M+1]

### Step 3

### N-ethyl-5-fluoro-2-[6-(3-fluoro-1-{[(1s,4s)-4-ethanesulfonamido-1-hydroxylcyclohexyl]methyl}pyrrolidin-3-yl)-1-methyl-1H-indazol-4-yl]-N-(isopropyl)benzamide 68

The crude product of Compound **68b** (40 mg) and DIPEA(37.4 mg, 289 µmol, 50.3 µL) were dissolved in DMF(2.0 mL), and cooled to 0°C, followed by the addition of ethanesulfonyl chloride (11.2 mg, 86.7 µmol), and the mixture was stirred at room temperature for 1.0 hour. The reaction mixture was concentrated under a reduced pressure. The resulting residues were purified by the silica-gel column chromatography using dichloromethane/methanol as an elution system, and the obtained crude product was purified by the HPLC (mobile phase: acetonitrile, water (0.1% formic acid), elution gradient: 5%-30%), to obtain the title Compound **68** (13.2 mg, 20.4 µmol, yield: 26.4%) as white solid.
MS m/z (ESI):646.2 [M+1]
¹H NMR (400 MHz, DMSO-*d₆*) δ 7.86 (d, J = 4.0 Hz, 1H), 7.69 (s, 1H), 7.60 (dt, J = 10.4, 5.2 Hz, 1H), 7.44 - 7.27 (m, 2H), 7.14 (s, 1H), 6.99 (dd, J = 7.6, 4.0 Hz, 1H), 4.22 (s, 0.22H), 4.08 (d, J = 4.0 Hz, 3H), 3.93 (s, 1H), 3.39 (dd, J = 11.6, 5.2 Hz, 1H), 3.27 - 3.12 (m, 3H), 3.07 - 2.79 (m, 6H), 2.45 (d, J = 8.4 Hz, 2H), 2.27 (d, J = 7.6 Hz, 1H), 1.59 (s, 6H), 1.34 (d, J = 7.2 Hz, 2H), 1.18 (d, J = 2.8 Hz, 3H), 1.00 (s, 0.65H), 0.87 (d, J = 6.8 Hz, 2.67H), 0.74 - 0.61 (m, 4H), 0.15 - 0.08 (m, 2H).

### Example 69

### 2-[3-cyclopropyl-6-(1-{[(1r,4r)-4-ethanesulfonamidocyclohexyl]methyl}pyrrolidin-3-yl)imidazo[1,5-a]pyridin-8-yl]-N-ethyl-5-fluoro-N-(isopropyl)benzamide 69

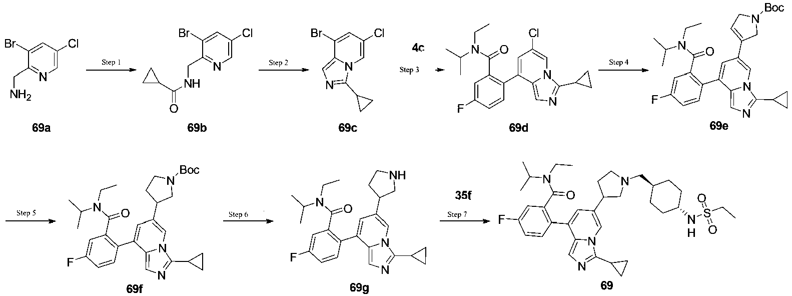

### Step 1

### N-[(3-bromo-5-chloropyridin-2-yl)methyl]cyclopropane formamide 69b

(3-Bromo-5-chloropyridin-2-yl)methylamine **69a** (1 g, 3.88 mmol, hydrochloride) was dissolved in dichloromethane (7.0 mL), and cooled to 0°C, followed by the addition of cyclopropanecarboxylic acid (267.00 mg, 3.10 mmol), triethylamine (1.18 g, 11.63 mmol, 1.62 mL) and HATU (1.77 g, 4.65 mmol). The mixture was stirred at room temperature to allow for reacting for 12.0 hour. Water (30 mL) was added to the reaction mixture, which was then extracted with ethyl acetate (30 mL x 2). The organic phases were combined, dried over anhydrous sodium sulfate, and concentrated under a reduced pressure, to obtain the crude product of title Compound **69b** (1.0 g, 3.45 mmol, yield: 89.1%). The crude product was directly used in the next step of reaction without purification.
MS m/z (ESI): 291.0 [M+1]

### Step 2

### 8-bromo-6-chloro-3-cyclopropylimidazo[1,5-a]pyridine 69c

Compound **69b** (1 g, 3.45 mmol) was dissolved in toluene (58 mL), followed by the addition of POCl₃ (5.30 g, 34.54 mmol, 3.22 mL). The mixture was stirred at 100°C to allow for reacting for 2.0 hours. The reaction mixture was cooled to room temperature, and concentrated under a reduced pressure. A 10% aqueous sodium hydroxide solution (100 mL) was added to regulate the mixture to alkalinity, and the mixture was then extracted with dichloromethane (50 mL x 2). The organic phases were combined, washed with water (50 mL) and saturated brine (50 mL), dried over anhydrous sodium sulfate, and concentrated under a reduced pressure. The resulting residues were purified by the silica-gel column chromatography using petroleum ether/ethyl acetate as an elution system, to obtain the title Compound **69c** (498 mg, 1.83 mmol, yield: 53.1%).
MS m/z (ESI): 273.0 [M+1]

### Step 3

### 2-{6-chloro-3-cyclopropylimidazo[1,5-a]pyridin-8-yl}-N-ethyl-5-fluoro-N-(isopropyl)benzamide 69d

Compound **69c** (200 mg, 736.54 µmol), Compound **4c**(370.35 mg, 1.10 mmol) and sodium carbonate (234.19 mg, 2.21 mmol) were dissolved in dioxane (3.0 mL) and water (1.0 mL). Pd (dppf)Cl₂ (53.89 mg, 73.65 µmol) was added. Under a nitrogen atmosphere, the mixture was stirred at 90°Cto allow for reacting for 12 hours. The reaction mixture was cooled to room temperature, water (50 mL) was added, and the mixture was extracted with ethyl acetate (50 mL x 2). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **69d** (254 mg, 635.18 µmol, yield: 86.24%).
MS m/z (ESI): 400.2 [M+1]

### Step 4

### 3-(3-cyclopropyl-8-{2-[ethyl(isopropyl)aminoformyl]-4-ffuorophenyl}imidazo[1,5-a]pyridin-6-yl)-2,5-dihydro-1H-pyrrol-1-tert-butyl formate 69e

Compound **69d** (250 mg, 625.18 µmol), 3-(tetramethyl-1,3,2-dioxaboran-2-yl)-2,5-dihydro-1*H*-pyrrol-1-tert-butyl formate (276.81 mg, 937.76 µmol) and cesium carbonate (611.08 mg, 1.88 mmol) were dissolved in dioxane (6.0 mL) and water (1.0 mL). Pd(dtbpf)Cl₂ (40.75 mg, 62.52 µmol) was added. Under a nitrogen atmosphere, the mixture was stirred at 90°Cto allow for reacting for 12 hours. The reaction mixture was cooled to room temperature, water (100 mL) was added, and the mixture was extracted with ethyl acetate (100 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **69e** (198 mg, 371.73 µmol, yield: 59.46%).

### Step 5

### 3-(3-cyclopropyl-8-{2-[ethyl(isopropyl)aminoformyl]-4-fluorophenyl}imidazo[1,5-a]pyridin-6-yl)pyrrolidin-1-tert-butyl formate 69f

Compound **69e** (198 mg, 371.73 µmol) was dissolved in tetrahydrofuran (5.0 mL), followed by the addition of Pd/C (45.15 mg, 371.73 µmol). Under a hydrogen atmosphere (15 Psi), the mixture was stirred at room temperature for 3.0 hours. The reaction mixture was filtered, and the filtrate was concentrated under a reduced pressure, to obtain the crude product of title Compound **69f** (198 mg, 370.33 µmol, yield: 99.6%). The crude product was directly used in the next step of reaction without purification.
MS m/z (ESI): 535.4 [M+1]

### Step 6

### 2-[3-cyclopropyl-6-(pyrrolidin-3-yl)imidazo[1,5-a]pyridin-8-yl]-N-ethyl-5-fluoro-N-(isopropyl)benzamide 69g

The crude product of Compound **69f** (198.00 mg, 370.33 µmol) was dissolved in dichloromethane (3.0 mL), followed by the addition of trifluoroacetic acid (42.23 mg, 370.33 µmol, 28.53 µL) at 0°C. The mixture was stirred at room temperature to allow for reacting for 0.5 hours. The reaction mixture was concentrated under a reduced pressure, to obtain the crude product of title Compound **69g** (168 mg). The crude product was directly used in the next step of reaction without purification.
MS m/z (ESI): 435.3 [M+1]

### Step 7

### 2-[3-cyclopropyl-6-(1-{[(1r,4r)-4-ethanesulfonamidocyclohexyl]methyl}pyrrolidin-3-yl)imidazo[1,5-a]pyridin-8-yl]-N-ethyl-5-fluoro-N-(isopropyl)benzamide 69

The crude product of Compound **69g** (158 mg, 363.60 µmol) and Compound **35f**(273.06 mg, 727.19 µmol) were dissolved in acetonitrile (10.0 mL), followed by the addition of potassium carbonate (251.26 mg, 1.82 mmol), and the mixture was stirred at room temperature for 0.5 hours. TBAI (13.43 mg, 36.36 µmol) was added, and the mixture was further stirred at room temperature for 1.0 hour. Water (20 mL) was added to the reaction mixture, which was then extracted with dichloromethane (20 mL x 3). The organic phases were combined, dried over anhydrous sodium sulfate, and concentrated under a reduced pressure. The resulting residues were purified by the HPLC (mobile phase: acetonitrile, water (0.1% trifluoroacetic acid), elution gradient: 6%-36%), to obtain the title Compound **69** (92 mg, 144.23 µmol, yield: 39.67 %).
MS m/z (ESI): 638.2 [M+1]
¹H NMR (400 MHz, DMSO-*d₆*) δ 10.04 - 9.90 (m, 1H), 8.51 - 8.43 (m, 1H), 7.67 - 7.60 (m, 1H), 7.58 - 7.51 (m, 1H), 7.47 - 7.37 (m, 2H), 7.08 - 6.98 (m, 2H), 3.82 - 3.71 (m, 2H), 3.59 - 3.52 (m, 1H), 3.48 - 3.40 (m, 1H), 3.37 - 3.29 (m, 1H), 3.24 - 3.17 (m, 1H), 3.15 - 3.07 (m, 2H), 3.01 - 2.94 (m, 3H), 2.61 - 2.53 (m, 1H), 2.43 - 2.32 (m, 1H), 1.94 - 1.72 (m, 5H), 1.68 - 1.54 (m, 1H), 1.31 - 1.15 (m, 9H), 1.14 - 1.01 (m, 5H), 0.96 - 0.89 (m, 3H), 0.84 - 0.77 (m, 1H), 0.72 - 0.63 (m, 2H), 0.44 - 0.27 (m, 2H).

### Example 70

### 2-[6-(4-{2-[4-(5,6-dimethoxypyridazin-3-yl)phenyl]ethyl}piperazin-1-yl)-3-methylimidazo[1,5-a]pyridin-8-yl]-N-ethyl-5-fluoro-N-(isopropyl)benzamide 70

Compound **58b** (49.09 mg, 104.54 µmol, trifluoroacetate) and triethylamine (35.26 mg, 348.47 µmol) were dissolved in methanol (0.5 mL), and the mixture was stirred at room temperature for 0.25 hours. The methanol solution (0.1 mL) of Compound **42c** (30 mg, 116.16 µmol) was added, and the mixture was stirred at room temperature for 1.0 hour. Sodium cyanoborohydride (14.60 mg, 232.31 µmol) was added, and the mixture was stirred at room temperature for 0.5 hours. Water (10 mL) was added to the reaction mixture, which was then extracted with ethyl acetate (10 mL x 3). The organic phases were combined, dried over anhydrous sodium sulfate, and concentrated under a reduced pressure. The resulting residues were purified by the HPLC (mobile phase: acetonitrile, water (0.1%ammonium bicarbonate), elution gradient: 34%-64%), to obtain the title Compound **70** (6.25 mg, 9.39 µmol, yield: 8.08%) as yellow solid.
MS m/z (ESI): 666.4 [M+1]
¹H NMR (400 MHz, CDCl₃) δ 7.99 - 7.91 (m, 2H), 7.65 (t, J = 5.2 Hz, 1H), 7.36 (d, J = 8.0 Hz, 2H), 7.22 - 7.10 (m, 4H), 6.97 - 6.92 (m, 1H), 6.79 (s, 1H), 4.24 (s, 3H), 4.02 (s, 3H), 3.58 - 3.37 (m, 2H), 3.25 - 3.15 (m, 2H), 3.15 - 3.04 (m, 2H), 3.03 - 2.96 (m, 1H), 2.95 - 2.89 (m, 2H), 2.76 - 2.71 (m, 4H), 2.66 - 2.61 (m, 3H), 1.38 - 1.22 (m, 1H), 1.20 - 1.11 (m, 1H), 1.01 (t, J = 7.0 Hz, 3H), 0.95 - 0.87 (m, J = 6.8 Hz, 3H), 0.79 (t, J = 7.0 Hz, 1H), 0.28 (d, J = 6.4 Hz, 2H).

### Example 71

### N-ethyl-N-[5-fluoro-2-(3-methyl-6-{4-[(1R,3S,4S)-2-azabicyclo[2.2.2]octan-3-carbonyl]piperazin-1-yl}imidazo[1,5-a]pyridin-8-yl)phenyl]-2-methylpropionamide 71

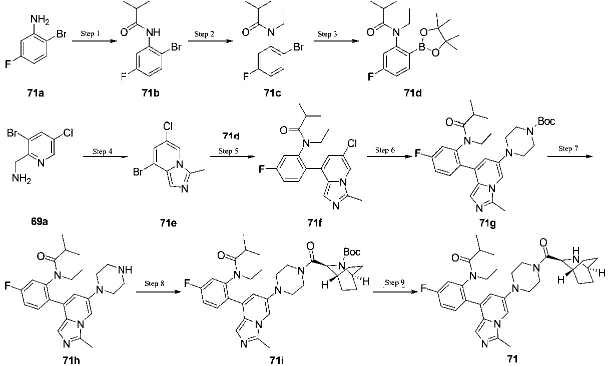

### Step 1

### N-(2-bromo-5-fluorophenyl)-2-methylpropionamide 71b

2-Bromo-5-fluoroaniline **71a** (5 g, 26.31 mmol) and triethylamine (5.33 g, 52.63 mmol) were dissolved in dichloromethane (50 mL), and cooled to 0°C, followed by the dropwise addition of 2-methylpropionyl chloride (3.5 g, 32.85 mmol, 3.44 mL). The mixture was stirred at room temperature to allow for reacting for 12.0 hour. The reaction mixture was washed with water (35 mL x 3), and then washed with a 1N aqueous HCl solution (35 mL). The organic phase was dried over anhydrous sodium sulfate, and concentrated under a reduced pressure, to obtain the crude product of title Compound **71b** (5.0 g, 19.22 mmol, yield: 73.05%) as yellow solid. The crude product was directly used in the next step of reaction without purification.
MS m/z (ESI): 262.2 [M+1]
¹H NMR (400 MHz, CDCl₃) δ 8.29 (dd, J = 2.9, 11.1 Hz, 1H), 7.75 (br s, 1H), 7.48 (dd, J = 5.8, 8.9 Hz, 1H), 6.72 (ddd, J = 3.1, 7.6, 8.8 Hz, 1H), 2.61 (td, J = 7.0, 13.9 Hz, 1H), 1.30 (d, J = 6.9 Hz, 6H).

### Step 2

### N-(2-bromo-5-fluorophenyl)-N-ethyl-2-methylpropionamide 71c

Compound **71b** (2 g, 7.69 mmol) was dissolved in tetrahydrofuran (20 mL), and cooled to 0°C under a nitrogen atmosphere, followed by the addition of NaH (615.08 mg, 15.38 mmol, 60% purity), and the mixture was stirred to allow for reacting for 0.5 hours. At 0°C, iodoethane (2.40 g, 15.38 mmol, 1.24 mL) was added dropwise, and the mixture was slowly warmed to room temperature and stirred to allow for reacting for 12 hours. The reaction mixture was quenched with a saturated aqueous ammonium chloride solution (20 mL), and extracted with ethyl acetate (30 mL x 3). The organic phase was dried over anhydrous sodium sulfate, and concentrated under a reduced pressure. The resulting residues were purified by the silica-gel column chromatography using petroleum ether/ethyl acetate as an elution system, to obtain the title Compound **71c** (1.8 g, 6.25 mmol, yield: 81.24%) as white solid.
MS m/z (ESI): 289.8 [M+1]
¹H NMR (400 MHz, CDCl₃) δ 7.66 (dd, J = 5.8, 8.7 Hz, 1H), 7.09 - 6.95 (m, 2H), 4.06 (qd, J = 7.1, 13.9 Hz, 1H), 3.33 (qd, J = 7.1, 13.9 Hz, 1H), 2.20 (td, J = 6.7, 13.4 Hz, 1H), 1.16 - 1.07 (m, 6H), 1.01 (d, J = 6.8 Hz, 3H).

### Step 3

### N-ethyl-N-[5-fluoro-2-(tetramethyl-1,3,2-dioxaboran-2-yl)phenyl]-2-methylpropionamide 71d

Compound **71c** (1.6 g, 5.55 mmol), potassium acetate (2.72 g, 27.76 mmol) and bis(pinacolato)diboron (2.82 g, 11.11 mmol) were dissolved in DMAc (20 mL). Under a nitrogen atmosphere, Pd(dppf)Cl₂ (609.43 mg, 832.88 µmol) was added, and the mixture was stirred at 90°C to allow for reacting for 12 hours. After the mixture was cooled to room temperature, 20 mL of water was added, and the mixture was extracted with ethyl acetate (30ml × 3). The organic layer was dried over anhydrous sodium sulfate, filtered and concentrated under a reduced pressure. The residues were purified by the silica-gel column chromatography using petroleum ether/ethyl acetate as an elution system, to obtain the title Compound **71d** (1.2 g, 3.58 mmol, yield: 64.47%) as yellow solid.
MS m/z (ESI): 336.0 [M+1]

### Step 4

### 8-bromo-6-chloro-3-methylimidazo[1,5-a]pyridine 71e

Compound **69a** (5.0 g, 19.38 mmol, hydrochloride) was dissolved in acetic anhydride (50 mL), followed by the addition of p-toluenesulfonic acid (3.34 g, 19.38 mmol). The mixture was stirred at 100°C to allow for reacting for 2.0 hours. The reaction mixture was cooled to room temperature, followed by adding a 10% aqueous sodium hydroxide solution (150 mL). The mixture was filtered, and the filter cake was dried under vacuum to obtain the crude product of title Compound **71e** (5.35 g, 21.79 mmol, yield: 112.4%).
MS m/z (ESI): 246.8 [M+1]

### Step 5

### N-(2-{6-chloro-3-methylimidazo[1,5-a]pyridin-8-yl}-5-fluorophenyl)-N-ethyl-2-methylpropionamide 71f

Compound **71e** (400 mg, 1.63 mmol), Compound **71d** (546.18 mg, 1.63 mmol) and sodium carbonate (518.07 mg, 4.89 mmol) were dissolved in dioxane (5 mL) and water (1.0 mL). Under a nitrogen atmosphere, Pd(dppf)Cl₂ (119.22 mg, 162.93 µmol) was added, and the mixture was stirred at 90°C to allow for reacting for 12.0 hours. The reaction mixture was cooled to room temperature, followed by adding water (20 mL) and extracting with ethyl acetate (25 mL x 3). The organic phases were combined, dried over anhydrous sodium sulfate and filtered, and the filtrate was concentrated under a reduced pressure. The resulting residues were purified by the silica-gel column chromatography using petroleum ether/ethyl acetate as an elution system, to obtain the title Compound **71f** (280 mg, 748.96 µmol, yield: 45.97%).
MS m/z (ESI): 374.2 [M+1]

### Step 6

### 4-{8-[2-(N-ethyl-2-methylpropionamido)-4-fluorophenyl]-3-methylimidazo[1,5-a]pyridin-6-yl}piperazin-1-tert-butyl carboxylate 71g

Compound **71f** (280.00 mg, 748.96 µmol), piperazin-1-tert-butyl carboxylate (280 mg, 1.50 mmol) and cesium carbonate (733 mg, 2.25 mmol) were dissolved in tert-amyl alcohol (3 mL). Ruphos (70 mg, 150.01 µmol) and Pd₂ (dba)₃ (70 mg, 76.44 µmol) were added. Under a nitrogen atmosphere, the mixture was stirred at 90°Cto allow for reacting for 12 hours. The reaction mixture was cooled to room temperature, and filtered through diatomite, and the filtrate was concentrated under a reduced pressure. The resulting residues were purified by the silica-gel column chromatography using dichloromethane/methanol as an elution system, to obtain the title Compound **71g** (400 mg, 763.88 µmol, yield: 101.99%) as yellow solid.
MS m/z (ESI): 524.2 [M+1]

### Step 7

*N*-ethyl-*N*-{5-fluoro-2-[3-methyl-6-(piperazin-1-yl)imidazo[1,5-a]pyridin-8-yl]phenyl}-2-methylpropionamide **71h** Compound **71g** (150 mg, 286.46 µmol) was dissolved in methane dioxide (3.0 mL), followed by the addition of trifluoroacetic acid (1.48 g, 12.98 mmol, 1 mL). The mixture was stirred at room temperature to allow for reacting for 1.0 hours. The reaction mixture was concentrated under a reduced pressure, to obtain the crude product of title Compound **71h** (120 mg). The crude product was directly used in the next step of reaction without purification.
MS m/z (ESI): 424.2 [M+1]

### Step 8

### (1R,3S,4S)-3-(4-{8-[2-(N-ethyl-2-methylpropionamido)-4-fluorophenyl]-3-methylimidazo[1,5-a]pyridin-6-yl}piperazin-1-carbonyl)-2-azabicyclo[2.2.2]octan-2-tert-butyl carboxylate 71i

Compound **32a** (61 mg, 238.93 µmol) and *N,N*-diisopropylethylamine (87 mg, 673.15 µmol, 117.25 µL) were dissolved in DCM (2.0 mL). The mixture was cooled to 0°C, followed by the addition of EDCI (65 mg, 339.07 µmol), HOBT (45 mg, 333.03 µmol) and Compound **71h** (120 mg, 223.24 µmol, trifluoroacetate ), and the mixture was stirred at room temperature for 12 hours. The reaction mixture was concentrated under a reduced pressure. The resulting residues were purified by the silica-gel column chromatography using dichloromethane/methanol as an elution system, to obtain the title Compound **71i** (80 mg, 121.06 µmol, yield: 54.23%).
MS m/z (ESI): 661.6 [M+1]

### Step 9

### N-ethyl-N-[5-fluoro-2-(3-methyl-6-{4-[(1R,3S,4S)-2-azabicyclo[2.2.2]octan-3-carbonyl]piperazin-1-yl}imidazo[1,5-a]pyridin-8-yl)phenyl]-2-methylpropionamide 71

Compound **71i** (80 mg, 121.06 µmol) was dissolved in methane dioxide (3.0 mL) and trifluoroacetic acid (1.0 mL). The mixture was stirred at room temperature to allow for reacting for 0.5 hours. The reaction mixture was concentrated under a reduced pressure, and purified by the HPLC (mobile phase: acetonitrile, water (0.1% ammonium bicarbonate), elution gradient: 20%-50%), to obtain the title Compound **71** (24.22 mg, 43.2 µmol, yield: 35.68 %) as yellow solid.
MS m/z (ESI): 561.3 [M+1]
¹H NMR (400 MHz, DMSO-*d₆*) δ 7.67 - 7.52 (m, 1H), 7.48 - 7.13 (m, 3H), 6.83 (s, 1H), 6.56 - 6.47 (m, 1H), 3.94 (s, 1H), 3.89 - 3.77 (m, 1H), 3.71 - 3.52 (m, 4H), 3.12 - 2.93 (m, 4H), 2.91 - 2.76 (m, 2H), 2.55 (s, 3H), 2.42 - 2.28 (m, 1H), 1.86 - 1.37 (m, 9H), 1.22 - 0.68 (m, 7H), 0.59 - 0.53 (m, 2H).

### Example 72

### N-cyclopropyl-5-fluoro-2-(3-methyl-6-{4-[(1R,3S,4S)-2-azabicyclo[2.2.2]octan-3-carbonyl]piperazin-1-yl}imidazo[1,5-a]pyridin-8-yl)-N-(isopropyl)benzamide 72

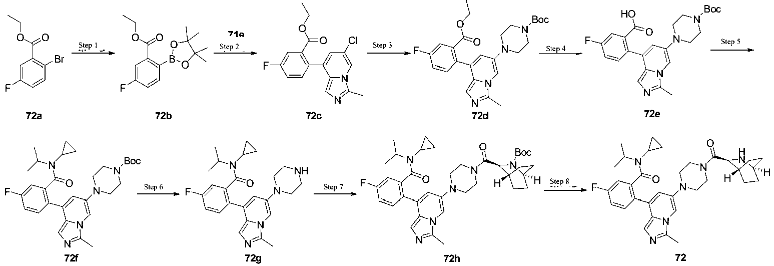

### Step 1

### 5-fluoro-2-(tetramethyl-1,3,2-dioxaboran-2-yl)ethyl benzoate 72b

2-Bromo-5-ethyl fluorobenzoate **72a** (20 g, 80.95 mmol), potassium acetate (12.0 g, 122.47 mmol) and bis(pinacolato)diboron (31.0 g, 122.1 mmol) were dissolved in dioxane (200 mL). Under a nitrogen atmosphere, Pd(dppf)Cl₂ (6.00 g, 8.20 mmol) was added, and the mixture was stirred at 100°C to allow for reacting for 12 hours. The reaction mixture was cooled to room temperature, and then filtered, and the filtrate was concentrated under a reduced pressure . The resulting residues were purified by the silica-gel column chromatography using petroleum ether/ethyl acetate as an elution system, to obtain the crude product of title Compound **72b** (32.4 g, 110.16 mmol, yield: 136.1%) as yellow oil. The crude product was directly used in the next step of reaction.

¹H NMR (400 MHz, CDCl₃) δ 7.64 - 7.59 (m, 1H), 7.51 - 7.45 (m, 1H), 7.25 - 7.19 (m, 1H), 4.44 - 4.35 (m, 2H), 1.42 (s, 12H), 1.41 - 1.37 (m, 3H).

### Step 2

### 2-{6-chloro-3-methylimidazo[1,5-a]pyridin-8-yl}-5-ethyl fluorobenzoate 72c

Compound **71e** (2 g, 8.15 mmol), Compound **72b**(2.93 g, 8.48 mmol) and sodium carbonate (2.59 g, 24.44 mmol) were dissolved in dioxane (18 mL) and water (3.0 mL). Under a nitrogen atmosphere, Pd(dppf)Cl₂ (600 mg, 820.00 µmol) was added, and the mixture was stirred at 90°C to allow for reacting for 12.0 hours. The reaction mixture was cooled to room temperature, followed by adding water (30 mL) and extracting with ethyl acetate (30 mL x 3). The organic phases were combined, dried over anhydrous sodium sulfate and filtered, and the filtrate was concentrated under a reduced pressure. The resulting residues were purified by the silica-gel column chromatography using petroleum ether/ethyl acetate as an elution system, to obtain the title Compound **72c** (1.23 g, 3.70 mmol, yield: 45.37%).
MS m/z (ESI): 333.2 [M+1]

### Step 3

### 4-{8-[2-(ethoxycarbonyl)-4-fluorophenyl]-3-methylimidazo[1,5-a]pyridin-6-yl}piperazin-1-tert-butyl carboxylate 72d

Compound **72c** (1.23 g, 3.70 mmol), piperazin-1-tert-butyl carboxylate (1.38 g, 7.39 mmol) and cesium carbonate (3.61 g, 11.09 mmol) were dissolved in tert-amyl alcohol (15 mL). Ruphos (345 mg, 739.34 µmol) and Pd₂ (dba)₃ (340 mg, 371.29 µmol) were added. Under a nitrogen atmosphere, the mixture was stirred at 90°Cto allow for reacting for 12 hours. The reaction mixture was cooled to room temperature, and filtered through diatomite, and the filtrate was concentrated under a reduced pressure. The resulting residues were purified by the silica-gel column chromatography using petroleum ether/ethyl acetate as an elution system, to obtain the title Compound **72d** (1.0 g, 2.07 mmol, yield: 56.1%).
MS m/z (ESI): 483.4 [M+1]

### Step 4

### 2-(6-{4-[(tert-butoxy)carbonyl]piperazin-1-yl}-3-methylimidazo[1,5-a]pyridin-8-yl)-5-fluorobenzoic acid 72e

Compound **72d** (1.0 g, 2.07 mmol) was dissolved in methanol (2.0 mL), tetrahydrofuran (10 mL) and water (2.0 mL), followed by the addition of LiOH.H₂O (260.89 mg, 6.22 mmol), and the mixture was stirred at 40°C to allow for reacting for 12 hours. The reaction mixture was cooled to room temperature, and regulated to pH = 7-8 with 1N aq. HCl, and the organic solvent was removed under a reduced pressure. The aqueous phase was further regulated to pH = 1 with 1N aq. HCl, and extracted with ethyl acetate (30 mL×3). The organic phase was dried over anhydrous sodium sulfate, filtered and concentrated under a reduced pressure. The title Compound **72e** (650 mg, 1.43 mmol, yield: 69.0%) was obtained.
MS m/z (ESI): 455.0 [M+1]

### Step 5

### 4-(8-{2-[cyclopropyl(isopropyl)aminoformyl]-4-fluorophenyl}-3-methylimidazo[1,5-a]pyridin-6-yl)piperazin-1-tert-butyl carboxylate 72f

Isopropylcyclopropylamine (50 mg, 368.64 µmol, hydrochloride) and Compound **72e**(120 mg, 264.03 µmol) were dissolved in DMAc (2.0 mL). DIPEA (170 mg, 1.32 mmol, 229.11 µL) and HATU (151 mg, 397.13 µmol) were added, and the mixture was stirred at 60°C allow for reacting for 2.0 hours. The reaction mixture was concentrated under a reduced pressure. The resulting residues were purified by the silica-gel column chromatography using dichloromethane/methanol as an elution system, to obtain the title Compound **72f** (110 mg, 205.36 µmol, yield: 77.8%).
MS m/z (ESI): 536.4 [M+1]

### Step 6

### N-cyclopropyl-5-fluoro-2-[3-methyl-6-(piperazin-1-yl)imidazo[1,5-a]pyridin-8-yl]-N-(isopropyl)benzamide 72g

Compound **72f** (110 mg, 205.36 µmol) was dissolved in methane dioxide (3.0 mL), followed by the addition of trifluoroacetic acid (1.48 g, 12.98 mmol, 1 mL). The mixture was stirred at room temperature to allow for reacting for 1.0 hours. The reaction mixture was concentrated under a reduced pressure, to obtain the crude product of title Compound **72g** (120 mg). The crude product was directly used in the next step of reaction without purification.
MS m/z (ESI): 436.2 [M+1]

### Step 7

### (1R,3S,4S)-3-[4-(8-{2-[cyclopropyl(isopropyl)aminoformyl]-4-fluorophenyl}-3-methylimidazo[1,5-a]pyridin-6-yl)piperazin-1-carbonyl]-2-azabicyclo[2.2.2]octan-2-tert-butyl carboxylate 72h

Compound **32a(61** mg, 238.93 µmol) and *N,N*-diisopropylethylamine (142 mg, 1.10 mmol, 191.37 µL) were dissolved in DCM (2.0 mL). The mixture was cooled to 0°C, followed by the addition of EDCI (63 mg, 328.64 µmol), HOBT (45 mg, 333.03 µmol) and Compound **72g** (120 mg, 218.36 µmol, trifluoroacetate ), and the mixture was stirred at room temperature for 12 hours. The reaction mixture was concentrated under a reduced pressure. The resulting residues were purified by the silica-gel column chromatography using dichloromethane/methanol as an elution system, to obtain the title Compound **72h** (80 mg, 118.90 µmol, yield: 54.45 %).
MS m/z (ESI): 673.5 [M+1]

### Step 8

### N-cyclopropyl-5-fluoro-2-(3-methyl-6-{4-[(1R,3S,4S)-2-azabicyclo[2.2.2]octan-3-carbonyl]piperazin-1-yl}imidazo[1,5-a]pyridin-8-yl)-N-(isopropyl)benzamide 72

Compound **72h** (80 mg, 118.9 µmol) was dissolved in methane dioxide (3.0 mL) and trifluoroacetic acid (1.0 mL). The mixture was stirred at room temperature to allow for reacting for 0.5 hours. The reaction mixture was concentrated under a reduced pressure, and purified by the HPLC (mobile phase: acetonitrile, water (0.1% ammonium bicarbonate), elution gradient: 21%-51%), to obtain the title Compound **72** (8.69 mg, 15.17 µmol, yield: 12.76 %) as yellow solid.
MS m/z (ESI): 573.4 [M+1]
¹H NMR (400 MHz, DMSO-*d₆*) δ 7.73 - 7.60 (m, 1H), 7.42 - 7.24 (m, 3H), 7.03 (s, 1H), 6.55 (s, 1H), 4.25 - 4.09 (m, 1H), 3.96 (s, 1H), 3.80 - 3.51 (m, 5H), 3.14 - 2.93 (m, 4H), 2.86 (s, 1H), 2.53 (s, 3H), 1.86 - 1.39 (m, 10H), 1.11 - 0.83 (m, 5H), 0.62 - 0.13 (m, 4H).

### Example 73

### N-ethyl-5-fluoro-2-[6-(3-fluoro-1-{[(1r,4r)-4-ethanesulfonamidocyclohexyl]methyl}pyrrolidin-3-yl)-3-methylimidazo[1,5-a]pyridin-8-yl]-N-(isopropyl) benzamide 73

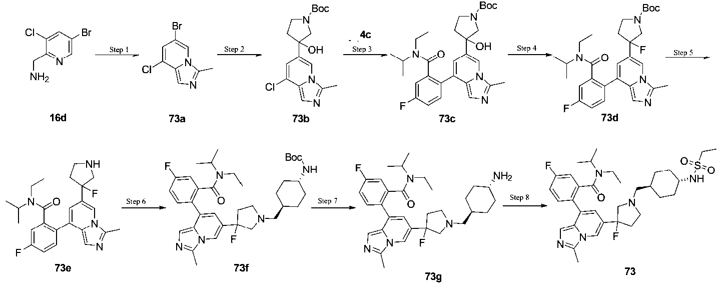

### Step 1

### 6-bromo-8-chloro-3-methylimidazo[1,5-a]pyridine 73a

Compound 16d (10.0 g, 45.15 mmol) was dissolved in acetic anhydride (100 mL), followed by the addition of p-toluenesulfonic acid (4.0 g, 23.23 mmol). The mixture was stirred at 100°C to allow for reacting for 12.0 hours. The reaction mixture was cooled to room temperature, and concentrated under a reduced pressure. Dichloromethane (40 mL) was added for dilution, and the mixture was regulated to PH = 8 with a saturated aqueous sodium carbonate solution. The mixture was extracted with dichloromethane (60 mL x 3), and the organic phases were combined, dried over anhydrous sodium sulfate, and concentrated under a reduced pressure . The resulting residues were purified by the silica-gel column chromatography using petroleum ether/ethyl acetate as an elution system, to obtain the title Compound **73a** (8.0 g, 32.6 mmol, yield: 72.2%).
MS m/z (ESI): 246.7 [M+1]

### Step 2

### 3-{8-chloro-3-methylimidazo[1,5-a]pyridin-6-yl}-3-hydroxylpyrrolidin-1-tert-butyl carboxylate 73b

Compound **73a** (1.5 g, 6.11 mmol) was dissolved in tetrahydrofuran (15 mL), and cooled to 0°C, followed by the addition of the solution of *i*-PrMgCl.LiCl in tetrahydrofuran (1.3 M, 7.05 mL). The mixture was stirred at 0°C to allow for reacting for 10 min, and 3-oxopyrrolidin-1-tert-butyl carboxylate (1.51 g, 8.81 mmol) was then added. The mixture was stirred at room temperature to allow for reacting for 2.0 hour. The reaction mixture was quenched by adding a saturated aqueous ammonium chloride solution (30 mL), and extracted with ethyl acetate (50 mL x 3). The organic phases were combined, washed with saturated brine (50 mL), dried over anhydrous sodium sulfate, and concentrated under a reduced pressure. The resulting residues were purified by the silica-gel column chromatography using petroleum ether/ethyl acetate as an elution system, to obtain the title Compound **73b** (650 mg, 1.85 mmol, yield: 30.24%) as brown solid.
MS m/z (ESI): 352.2 [M+1]

### Step 3

### 3-(8-{2-[ethyl(isopropyl)aminoformyl]-4-fluorophenyl}-3-methylimidazo[1,5-a]pyridin-6-yl)-3-hydroxylpyrrolidin-1-tert-butyl carboxylate 73c

Compound **73b** (650 mg, 1.85 mmol) and Compound **4c**(1 g, 2.98 mmol) were dissolved in dioxane (9.0 mL) and water (1.5 mL). Potassium phosphate (984.33 mg, 4.64 mmol) and Pd (dtbpf)Cl₂ (124.02 mg, 190.29 µmol) were added. Under a nitrogen atmosphere, the mixture was stirred at 95°C to allow for reacting for 12.0 hours. The reaction mixture was cooled to room temperature, water (20 mL) was added, and the mixture was extracted with ethyl acetate (30 mL x 3). The organic phases were combined, washed with saturated brine (30 mL), dried over anhydrous sodium sulfate, and concentrated under a reduced pressure. The resulting residues were purified by the silica-gel column chromatography using petroleum ether/ethyl acetate as an elution system, to obtain the title Compound **73c** (388 mg, 739.58 µmol, yield: 40.0 %).
MS m/z (ESI): 525.3 [M+1]

### Step 4

### 3-(8-{2-[ethyl(isopropyl)aminoformyl]-4-fluorophenyl}-3-methylimidazo[1,5-a]pyridin-6-yl)-3-fluoropyrrolidin-1-tert-butyl carboxylate 73d

Compound **73c** (330 mg, 629.02 µmol) was dissolved in dichloromethane (4.0 mL), and cooled to -60°C, followed by the dropwise addition of DAST(202.78 mg, 1.26 mmol). The mixture was stirred -60°C to allow for reacting for 1.0 hour, and then slowly warmed to room temperature, at which the mixture was stirred to allow for reacting for 1.0 hour. The reaction mixture was quenched by adding a saturated aqueous sodium bicarbonate solution (20 mL), and extracted with dichloromethane (15 mL x 3). The organic phases were combined, dried over anhydrous sodium sulfate, and concentrated under a reduced pressure. The crude product of title Compound **73d** (305 mg, 579.17 µmol, yield: 92.07%) was obtained.
MS m/z (ESI): 527.1 [M+1]

### Step 5

### N-ethyl-5-fluoro-2-[6-(3-fluoropyrrolidin-3-yl)-3-methylimidazo[1,5-a]pyridin-8-yl]-N-(isopropyl)benzamide 73e

Compound **73d** (100 mg, 189.89 µmol) was dissolved in dichloromethane (2.0 mL), followed by the addition of trifluoroacetic acid (740.00 mg, 6.49 mmol, 0.5 mL) at 0°C. The mixture was stirred at room temperature to allow for reacting for 1.0 hours. The reaction mixture was concentrated under a reduced pressure, to obtain the crude product of title Compound **73e** (90 mg). The crude product was directly used in the next step of reaction without purification.
MS m/z (ESI): 427.3 [M+1]

### Step 6

### N-[(1r,4r)-4-{[3-(8-{2-[ethyl(isopropyl)aminoformyl]-4-fluorophenyl}-3-methylimidazo[1,5-a]pyridin-6-yl)-3-fluoropyrrolidin-1-yl]methyl} cyclohexyl]tert-butyl carbamate 73f

The crude product of Compound **73e** (90 mg), *N*-[(1*r*,4*r*)-4-formylcyclohexyl]tert-butyl carbamate (45.42 mg, 199.81 µmol) and triethylamine (50.55 mg, 499.52 µmol) were dissolved in methanol (2.0 mL), and stirred at room temperature for 1.0 hour. Sodium cyanoborohydride (31.70 mg, 504.51 µmol) was added, and the mixture was stirred at room temperature for 11 hours. A saturated aqueous sodium bicarbonate solution (15 mL) was added to the reaction mixture, which was then extracted with dichloromethane (20 mL x 3). The organic phases were combined, dried over anhydrous sodium sulfate, and concentrated under a reduced pressure. The residues were purified by the preparative thin-layer chromatography using dichloromethane/methanol as an elution system, to obtain the title Compound **73f** (108 mg, 169.33 µmol, yield: 101.7%).
MS m/z (ESI): 638.3 [M+1]

### Step 7

### N-ethyl-5-fluoro-2-[6-(3-fluoro-1-{[(1r,4r)-4-aminocyclohexylmethyl}pyrrolidin-3-yl)-3-methylimidazo[1,5-a]pyridin-8-yl]-N-(isopropyl)benzamide 73g

Compound **73f** (106 mg, 166.5 µmol) was dissolved in dichloromethane (1.9 mL), followed by the addition of trifluoroacetic acid (740.00 mg, 6.49 mmol, 0.5 mL) at 0°C. The mixture was stirred at room temperature to allow for reacting for 1.0 hours. The reaction mixture was concentrated under a reduced pressure, to obtain the crude product of title Compound **73g** (95 mg). The crude product was directly used in the next step of reaction without purification.
MS m/z (ESI): 538.3 [M+1]

### Step 8

### N-ethyl-5-fluoro-2-[6-(3-fluoro-1-{[(1r,4r)-4-ethanesulfonamidocyclohexyl]methyl}pyrrolidin-3-yl)-3-methylimidazo[1,5-a]pyridin-8-yl]-N-(isopropyl) benzamide 73

The crude product of Compound **73g** (89.53 mg, 137.38 µmol, TF) and DIPEA (88.77 mg, 686.89 µmol, 119.64 µL) was dissolved in dichloromethane (2.0 mL), and cooled to 0°C, followed by the addition of ethanesulfonyl chloride (44.16 mg, 343.44 µmol), and the mixture was stirred at room temperature for 12 hours. Water (10 mL) was added to the reaction mixture. The mixture was extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 3%-33%), to obtain the title Compound **73** (8.91 mg, 14.15 µmol, yield: 10.3%) as yellow solid.
MS m/z (ESI): 630.4 [M+1]
¹H NMR (400 MHz, DMSO-*d₆*) δ 8.16 (s, 1H), 8.05 - 7.99 (m, 1H), 7.72 - 7.65 (m, 1H), 7.43 - 7.30 (m, 2H), 7.11 - 6.97 (m, 2H), 6.67 - 6.61 (m, 1H), 3.50 - 3.46 (m, 1H), 3.31 - 3.26 (m, 1H), 3.03 - 2.89 (m, 6H), 2.70 - 2.62 (m, 4H), 2.36 - 2.26 (m, 3H), 1.93 - 1.76 (m, 4H), 1.41 - 0.72 (m, 16H), 0.37 - 0.29 (m, 2H).

### Example 75

### N-ethyl-5-fluoro-2-(6-{3-fluoro-1-[(1R,3S,4S)-2-azabicyclo[2.2.2]octan-3-carbonyl]azacyclobutan-3-yl}-3-methylimidazo[1,5-a]pyridin-8-yl)-N-(isopropyl) benzamide 75

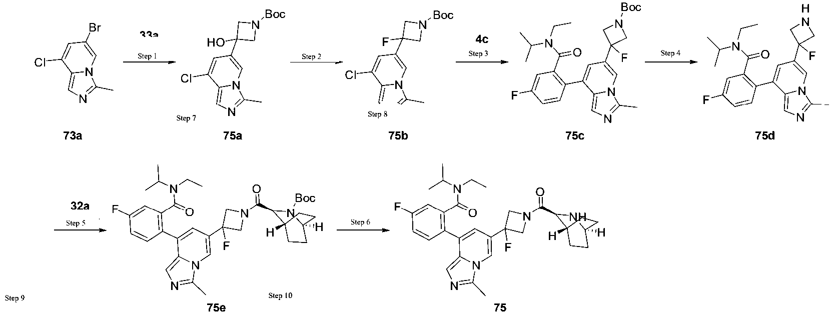

### Step 1

### 3-{8-chloro-3-methylimidazo[1,5-a]pyridin-6-yl}-3-hydroxyazacyclobutan-1-tert-butyl carboxylate 75a

Compound **73a** (1 g, 4.07 mmol) was dissolved in tetrahydrofuran (20 mL), and cooled to 0°C, followed by the addition of the solution of *i*-PrMgCl.LiCl in tetrahydrofuran (1.3 M, 4.70 mL). The mixture was stirred at 0°C to allow for reacting for 20 min, followed by the dropwise addition of the solution of 3-oxoazacyclobutan-1-tert-butyl carboxylate **33a** (836.78 mg, 4.89 mmol) in tetrahydrofuran (2.0 mL). The mixture was stirred at 0°C to allow for reacting for 2.0 hours. The reaction mixture was quenched by adding water (50 mL), and extracted with ethyl acetate (50 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **75a** (650 mg, 1.92 mmol, yield: 47.24%).
MS m/z (ESI): 338.0 [M+1]

### Step 2

### 3-{8-chloro-3-methylimidazo[1,5-a]pyridin-6-yl}-3-fluoroazacyclobutan-1-tert-butyl carboxylate 75b

Compound **75a** (100 mg, 296.03 µmol) was dissolved in dichloromethane (3.0 mL), and cooled to 0°C, followed by the dropwise addition of DAST(95.43 mg, 592.07 µmol, 78.22 µL). The mixture was stirred at room temperature to allow for reacting for 12 hour. The reaction mixture was quenched by adding water (50 mL), and extracted with ethyl acetate (50 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the crude product of title Compound **75b** (150 mg, 441.45 µmol, yield: 149.12%).
MS m/z (ESI): 339.9 [M+1]

### Step 3

### 3-(8-{2-[ethyl(isopropyl)aminoformyl]-4-ffuorophenyl}-3-methylimidazo[1,5-a]pyridin-6-yl)-3-fluoroazacyclobutan-1-tert-butyl carboxylate 75c

The crude product of Compound **75b** (150 mg, 441.45 µmol), Compound **4c**(221.97 mg, 662.17 µmol) and cesium carbonate (431.50 mg, 1.32 mmol) were dissolved in dioxane (3.0 mL) and water (1.0 mL). Pd(dtbpf)Cl₂ (28.77 mg, 44.14 µmol) was added, and the mixture was stirred at 100 °C under a nitrogen atmosphere to allow for reacting for 12.0 hours. The reaction mixture was cooled to room temperature, and concentrated under a reduced pressure. The resulting residues were purified by the silica-gel column chromatography using petroleum ether/ethyl acetate as an elution system, to obtain the title Compound **75c** (170 mg, 331.65 µmol, yield: 75.1 %).
MS m/z (ESI): 513.3 [M+1]

### Step 4

### N-ethyl-5-fluoro-2-[6-(3-fluoroazacyclobutan-3-yl)-3-methylimidazo[1,5-a]pyridin-8-yl]-N-(isopropyl)benzamide 75d

Compound **75c** (170 mg, 331.65 µmol) was dissolved in dichloromethane (3.0 mL), followed by the addition of trifluoroacetic acid (1.48 g, 12.98 mmol, 1 mL) at 0°C. The mixture was stirred at room temperature to allow for reacting for 1.0 hours. The reaction mixture was concentrated under a reduced pressure, to obtain the crude product of title Compound **75d** (170 mg). The crude product was directly used in the next step of reaction without purification. MS m/z (ESI): 413.2 [M+1]

### Step 5

### (1R,3S,4S)-3-[3-(8-{2-[ethyl(isopropyl)aminoformyl]-4-fluorophenyl}-3-methylimidazo[1,5-a]pyridin-6-yl)-3-fluoroazacyclobutan-1-carbonyl]-2-azabicyclo[2.2.2]octan-2-tert-butyl carboxylate 75e

Compound **32a** (105.22 mg, 412.15 µmol), *N,N*-diisopropylethylamine (159.80 mg, 1.24 mmol, 215.36 µL), EDCI(118.51 mg, 618.22 µmol) and HOBt (83.53 mg, 618.22 µmol) were dissolved in dichloromethane (3 mL). The mixture was stirred at room temperature for 0.5 hours, followed by the addition of the crude product of Compound **75d** (170 mg), and the mixture was then stirred at room temperature for 12 hours. A saturated aqueous sodium bicarbonate solution (10 mL) was added to the reaction mixture, which was then extracted with dichloromethane (20 mL x 3). The organic phases were combined, dried over anhydrous sodium sulfate, and concentrated under a reduced pressure. The resulting residues were purified by the preparative thin-layer chromatography using petroleum ether/ethyl acetate as an elution system, to obtain the title Compound **75e** (100 mg, 153.9 µmol, yield: 37.3%).
MS m/z (ESI): 650.4 [M+1]

### Step 6

### N-ethyl-5-fluoro-2-(6-{3-fluoro-1-[(1R,3S,4S)-2-azabicyclo[2.2.2]octan-3-carbonyl]azacyclobutan-3-yl}-3-methylimidazo[1,5-a]pyridin-8-yl)-N-(isopropyl) benzamide 75

Compound **75e** (100 mg, 153.9 µmol) was dissolved in methane dioxide (3.5 mL), followed by the addition of trifluoroacetic acid (17.55 mg, 153.90 µmol, 11.86 µL). The mixture was stirred at room temperature to allow for reacting for 1.0 hours. The reaction mixture was concentrated under a reduced pressure, and the resulting residues were purified by the HPLC (mobile phase: acetonitrile, water (0.1% ammonium bicarbonate), elution gradient: 25%-55%), to obtain the title Compound **75** (50 mg, 90.97 µmol, yield: 59.1 %) as white solid.
MS m/z (ESI): 550.2 [M+1]
¹H NMR (400 MHz, DMSO-*d₆*) δ 8.30 (s, 1H), 7.74 - 7.64 (m, 1H), 7.46 - 7.30 (m, 2H), 7.16 - 7.06 (m, 1H), 6.71 - 6.59 (m, 1H), 4.99 - 4.44 (m, 3H), 4.41 - 4.15 (m, 1H), 3.74 - 3.66 (m, 1H), 3.53 - 3.44 (m, 1H), 3.00 - 2.85 (m, 1H), 2.82 - 2.74 (m, 1H), 2.66 (s, 3H), 1.92 - 1.30 (m, 10H), 0.93 (d, J = 6.8 Hz, 3H), 0.81 - 0.69 (m, 3H), 0.42 - 0.31 (m, 2H).

### Example 76

### N-ethyl-5-fluoro-2-(3-formyl-6-{4-[(1R,3S,4S)-2-azabicyclo[2.2.2]octan-3-carbonyl]piperazin-1-yl}imidazo[1,5-a]pyridin-8-yl)-N-(isopropyl)benzamide 76

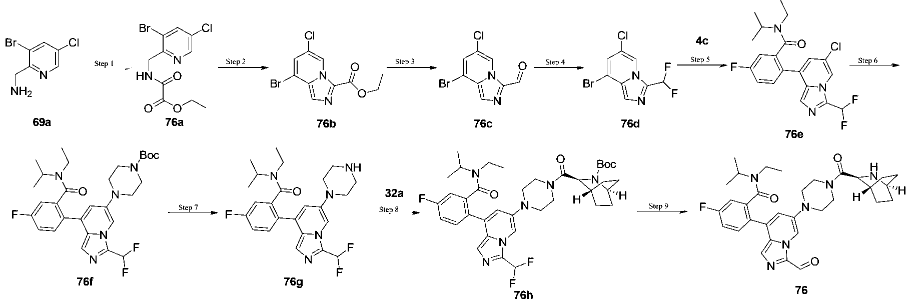

### Step 1

### {[(3-bromo-5-chloropyridin-2-yl)methyl]aminoformyl}ethyl formate 76a

Compound **69a** (10.94 g, 49.39 mmol) and triethylamine (10.00 g, 98.79 mmol) were dissolved in dichloromethane (60 mL), and cooled to 0°C, followed by the slow dropwise addition of 2-chloro-2-oxoethyl acetate (6.74 g, 49.39 mmol, 5.52 mL). The mixture was stirred at room temperature to allow for reacting for 2.0 hour. The reaction mixture was concentrated under a reduced pressure, to obtain the crude product of title Compound 76a (21.05 g, 65.47 mmol, yield: 132.5%) as white solid. The crude product was directly used in the next step of reaction without purification. MS m/z (ESI): 322.8 [M+1]

### Step 2

### 8-bromo-6-chloroimidazo[1,5-a]pyridin-3-ethyl carboxylate 76b

The crude product of Compound **76a** (21.05 g, 65.47 mmol) was dissolved in toluene (100 mL), followed by the addition of phosphorus oxychloride (70.26 g, 458.26 mmol, 41.95 mL). The mixture was stirred at 110°C to allow for reacting for 12.0 hours. The reaction mixture was cooled to room temperature, and concentrated under a reduced pressure. A saturated aqueous sodium bicarbonate solution (100 mL) was added, and the reaction mixture was then extracted with ethyl acetate (60 mL x 3). The organic phases were combined, washed with saturated brine (50 mL), dried over anhydrous sodium sulfate, and concentrated under a reduced pressure. The resulting residues were purified by the silica-gel column chromatography using petroleum ether/ethyl acetate as an elution system, to obtain the title Compound **76b** (3.6 g, 11.86 mmol, yield: 18.12%) as yellow solid.
MS m/z (ESI): 304.9[M+1]
¹H NMR (400 MHz, CDCl₃) δ 9.41 - 9.27 (m, 1H), 7.66 (d, J = 0.7 Hz, 1H), 7.21 (d, J = 1.4 Hz, 1H), 4.45 (q, J = 7.1 Hz, 2H), 1.42 (t, J = 7.2 Hz, 3H).

### Step 3

### 8-bromo-6-chloroimidazo[1,5-a]pyridin-3-formaldehyde 76c

Compound **76b** (1.2 g, 3.95 mmol) was dissolved in dichloromethane (20 mL). Under a nitrogen atmosphere, the mixture was cooled to -78°C, followed by the slow dropwise addition of DIBALH(1.0M, 7.91 mL), and the mixture was stirred at -78°C to allow for reacting for 2.0 hours. The reaction mixture was diluted by adding dichloromethane (50 mL), and the quenched by adding water (50 ml). The organic layer was washed with water (50 mL x 2), dried over anhydrous sodium sulfate, filtered and concentrated under a reduced pressure. The resulting residues were purified by the silica-gel column chromatography using petroleum ether/ethyl acetate as an elution system, to obtain the title Compound **76c** (640 mg, 2.47 mmol, yield: 62.39%).
MS m/z (ESI): 260.8 [M+1]

### Step 4

### 8-bromo-6-chloro-3-(difluoromethyl)imidazo[1,5-a]pyridine 76d

Compound **76c** (640 mg, 2.47 mmol) was dissolved in dichloromethane (10 mL), and cooled to 0°C, followed by the slow dropwise addition of DAST (1.19 g, 7.40 mmol, 977.60 µL). The mixture was stirred at room temperature to allow for reacting for 12.0 hour. A saturated aqueous sodium bicarbonate solution (100 mL) was added to the reaction mixture, which was then extracted with dichloromethane (50 mL x 3). The organic phases were combined, washed with saturated brine (50 mL), dried over anhydrous sodium sulfate, and concentrated under a reduced pressure. The resulting residues were purified by the silica-gel column chromatography using petroleum ether/ethyl acetate as an elution system, to obtain the title Compound **76d** (480 mg, 1.71 mmol, yield: 69.14%).
MS m/z (ESI): 282.8 [M+1]

### Step 5

### 2-[6-chloro-3-(difluoromethyl)imidazo[1,5-a]pyridin-8-yl]-N-ethyl-5-fluoro-N-(isopropyl)benzamide 76e

Compound **76d** (630 mg, 2.24 mmol), Compound **4c** (1.13 g, 3.36 mmol) and cesium carbonate (2.19 g, 6.71 mmol) were dissolved in dioxane (9 mL) and water (3.0 mL). Under a nitrogen atmosphere, Pd(dppf)Cl₂ (163.77 mg, 223.81 µmol) was added, and the mixture was stirred at 100°C to allow for reacting for 12.0 hours. The reaction mixture was concentrated under a reduced pressure. The resulting residues were purified by the silica-gel column chromatography using petroleum ether/ethyl acetate as an elution system, to obtain the crude product of title Compound **76e** (1.0 g, 2.44 mmol, yield: 109%).
MS m/z (ESI): 410.0 [M+1]

### Step 6

### 4-[3-(difluoromethyl)-8-{2-[ethyl(isopropyl)aminoformyl]-4-fluorophenyl}imidazo[1,5-a]pyridin-6-yl]piperazin-1-tert-butyl carboxylate 76f

Compound **76e** (200.00 mg, 488.01 µmol), piperazin-1-tert-butyl carboxylate (181.78 mg, 976.01 µmol) and cesium carbonate (477 mg, 1.46 mmol) were dissolved in tert-amyl alcohol (6 mL). Ruphos (45.54 mg, 97.60 µmol) and Pd₂ (dba)₃ (44.69 mg, 48.80 µmol) were added. Under a nitrogen atmosphere, the mixture was stirred at 100°Cto allow for reacting for 12 hours. The reaction mixture was cooled to room temperature, and concentrated under a reduced pressure. The resulting residues were purified by the silica-gel column chromatography using petroleum ether/ethyl acetate as an elution system, to obtain the title Compound **76f** (180 mg, 321.65 µmol, yield: 65.9%).
MS m/z (ESI): 560.2 [M+1]

### Step 7

2-[3-(difluoromethyl)-6-(piperazin-1-yl)imidazo[1,5-a]pyridin-8-yl]-*N*-ethyl-5-fluoro-*N*-(isopropyl)benzamide **76g** Compound **76f** (160 mg, 285.9 µmol) was dissolved in methane dioxide (4.0 mL), followed by the addition of trifluoroacetic acid (2.96 g, 25.96 mmol, 2 mL). The mixture was stirred at room temperature to allow for reacting for 1.0 hours. The reaction mixture was concentrated under a reduced pressure, to obtain the crude product of title Compound **76g** (160 mg). The crude product was directly used in the next step of reaction without purification.
MS m/z (ESI): 460.2 [M+1]

### Step 8

### (1R,3S,4S)-3-{4-[3-(difluoromethyl)-8-{2-[ethyl(isopropyl)aminoformyl]-4-fluorophenyl}imidazo[1,5-a]pyridin-6-yl]piperazin-1-carbonyl}-2-azabicyclo[2.2.2]octan-2-tert-butyl carboxylate 76h

Compound **32a**(88.90 mg, 348.20 µmol) and *N,N*-diisopropylethylamine (135.01 mg, 1.04 mmol, 181.95 µL) were dissolved in DCM (3.0 mL). The mixture was cooled to 0°C, followed by the addition of EDCI (100.13 mg, 522.30 µmol) and HOBT (70.57 mg, 522.30 µmol). The mixture was stirred at room temperature for 0.5 hours, followed by the addition of the crude product of Compound **76g** (160 mg), and the mixture was further stirred at room temperature for 12 hours. The reaction mixture was diluted with dichloromethane (20 mL), and washed with water (20 mL x 3). The organic phase was dried over anhydrous sodium sulfate, filtered and concentrated under a reduced pressure. The resulting residues were purified by the preparative thin-layer chromatography using petroleum ether/ethyl acetate as an elution system, to obtain the title Compound **76h** (70 mg, 100.46 µmol, yield: 28.85%).
MS m/z (ESI): 697.3 [M+1]

### Step 9

### N-ethyl-5-fluoro-2-(3-formyl-6- {4-[(1R,3S,4S)-2-azabicyclo[2.2.2]octan-3-carbonyl]piperazin-1-yl} imidazo[1,5-a]pyridin-8-yl)-N-(isopropyl)benzamide 76

Compound **76h** (60 mg, 86.11 µmol) was dissolved in methane dioxide (2.0 mL), followed by the addition of trifluoroacetic acid (740.00 mg, 6.49 mmol, 0.5 mL). The mixture was stirred at room temperature to allow for reacting for 0.5 hours. The reaction mixture was concentrated under a reduced pressure, and purified by the HPLC (mobile phase: acetonitrile, water (0.1% ammonium bicarbonate), elution gradient: 25%-55%), to obtain the title Compound **76** (15 mg, 26.10 µmol, yield: 30.3%) as yellow solid.
MS m/z (ESI): 575.2 [M+1]
¹H NMR (400 MHz, DMSO-*d₆*) δ 9.84 (s, 1H), 8.97 - 8.88 (m, 1H), 7.74 - 7.67 (m, 1H), 7.59 - 7.38 (m, 3H), 7.31 (s, 1H), 4.04 - 3.94 (m, 1H), 3.87 - 3.53 (m, 5H), 3.17 (br s, 5H), 2.93 - 2.81 (m, 2H), 1.86 - 1.70 (m, 3H), 1.68 - 1.53 (m, 3H), 1.51 - 1.39 (m, 3H), 0.92 (br d, J = 6.3 Hz, 3H), 0.86 - 0.76 (m, 1H), 0.74 - 0.58 (m, 3H), 0.39 - 0.17 (m, 3H).

### Example 77

### N-[5-fluoro-2-(3-methyl-6-{4-[(1R,3S,4S)-2-azabicyclo[2.2.2]octan-3-carbonyl]piperazin-1-yl}imidazo[1,5-a]pyridin-8-yl)phenyl]-N-(isopropyl)propionamide 77

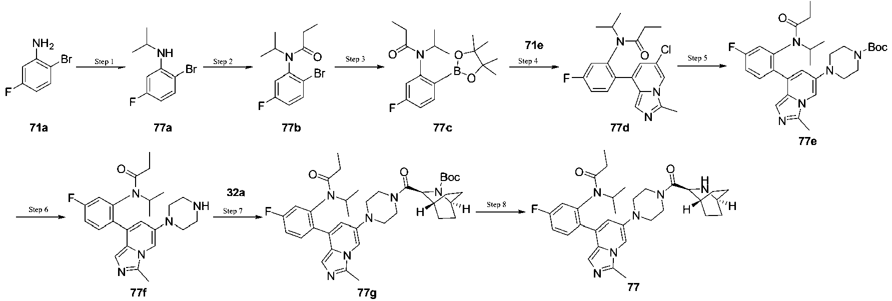

### Step 1

### 2-bromo-5-fluoro-N-(isopropyl)aniline 77a

2-Bromo-5-fluoroaniline **71a**(3 g, 15.79 mmol) was dissolved in methanol (40 mL), followed by the addition of acetone (1.83 g, 31.58 mmol) and acetic acid (2.5 g, 41.63 mmol), and the mixture was stirred at 50°C to allow for reacting for 2.0 hours. Sodium cyanoborohydride (2.98 g, 47.37 mmol) was added, and the mixture was stirred at50°C to allow for reacting for 12.0 hours. The reaction mixture was quenched by adding a saturated aqueous sodium bicarbonate solution (50), concentrated under a reduced pressure to remove the organic solvent, and then extracted with dichloromethane (50 mL x 3). The organic phase was dried over anhydrous sodium sulfate, and concentrated under a reduced pressure. The resulting residues were purified by the silica-gel column chromatography using petroleum ether/ethyl acetate as an elution system, to obtain the title Compound **77a** (550 mg, 2.37 mmol, yield: 15.01%) as colorless oil.
MS m/z (ESI): 232.2 [M+1]
¹H NMR (400 MHz, CDCl₃) δ 8.29 (dd, J = 2.9, 11.1 Hz, 1H), 7.75 (br s, 1H), 7.48 (dd, J = 5.8, 8.9 Hz, 1H), 6.72 (ddd, J = 3.1, 7.6, 8.8 Hz, 1H), 2.61 (td, J = 7.0, 13.9 Hz, 1H), 1.30 (d, J = 6.9 Hz, 6H).

### Step 2

### N-(2-bromo-5-fluorophenyl)-N-(isopropyl)propionamide 77b

Compound **77a** (550 mg, 2.37 mmol) was dissolved in toluene (10 mL), followed by the addition of propionyl chloride (438.52 mg, 4.74 mmol) and DMAP(57.90 mg, 473.95 µmol), and the mixture was stirred at 80°C to allow for reacting for 2.0 hours. The reaction mixture was concentrated under a reduced pressure. The resulting residues were purified by the silica-gel column chromatography using petroleum ether/ethyl acetate as an elution system, to obtain the title Compound **77b** (450 mg, 1.56 mmol, yield: 65.9%) as white solid.
MS m/z (ESI): 289.9 [M+1]
¹H NMR (400 MHz, DMSO-*d₆*) δ 7.85 (dd, J = 6.0, 8.9 Hz, 1H), 7.40 (dd, J = 3.0, 9.3 Hz, 1H), 7.30 (dt, J = 3.0, 8.5 Hz, 1H), 4.64 (td, J = 6.8, 13.5 Hz, 1H), 1.81 (dq, J = 1.5, 7.4 Hz, 2H), 1.17 (d, J = 6.6 Hz, 3H), 0.96 - 0.88 (m, 6H).

### Step 3

### N-[5-fluoro-2-(tetramethyl-1,3,2-dioxaboran-2-yl)phenyl]-N-(isopropyl)propionamide 77c

Compound **77b** (550 mg, 1.91 mmol), potassium acetate (936.62 mg, 9.54 mmol) and bis(pinacolato)diboron (969.38 mg, 3.82 mmol) were dissolved in DMAc (10 mL). Under a nitrogen atmosphere, Pd(dppf)Cl₂ (209.49 mg, 286.30 µmol) was added, and the mixture was stirred at 90°C to allow for reacting for 12 hours. After the mixture was cooled to room temperature, 20 mL of water was added, and the mixture was extracted with ethyl acetate (30ml × 3). The organic layer was dried over anhydrous sodium sulfate, filtered and concentrated under a reduced pressure. The resulting residues were purified by the silica-gel column chromatography using petroleum ether/ethyl acetate as an elution system, to obtain the title Compound **77c** (550 mg, 1.64 mmol, yield: 85.96%) as colorless oil.
MS m/z (ESI): 336.0 [M+1]

### Step 4

### N-(2-16-chloro-3-methylimidazo[1,5-a]pyridin-8-yl}-5-fluorophenyl)-N-(isopropyl)propionamide 77d

Compound **71e** (400 mg, 1.63 mmol), Compound **77c**(545 mg, 1.63 mmol) and sodium carbonate (519 mg, 4.90 mmol) were dissolved in dioxane (5 mL) and water (1.0 mL). Under a nitrogen atmosphere, Pd(dppf)Cl₂ (119 mg, 162.63 µmol) was added, and the mixture was stirred at 90°C to allow for reacting for 12.0 hours. The reaction mixture was cooled to room temperature, followed by adding water (20 mL) and extracting with ethyl acetate (25 mL x 3). The organic phases were combined, dried over anhydrous sodium sulfate and filtered, and the filtrate was concentrated under a reduced pressure. The resulting residues were purified by the silica-gel column chromatography using petroleum ether/ethyl acetate as an elution system, to obtain the title Compound **77d** (150 mg, 401.23 µmol, yield: 24.63%).
MS m/z (ESI): 374.2 [M+1]

### Step 5

### 4-(8-{4-fluoro-2-[N-(isopropyl)propionamido]phenyl}-3-methylimidazo[1,5-a]pyridin-6-yl}piperazin-1-tert-butyl carboxylate 77e

Compound **77d** (150 mg, 401.23 µmol), piperazin-1-tert-butyl carboxylate (150 mg, 805.37 µmol) and cesium carbonate (393 mg, 1.21 mmol) were dissolved in tert-amyl alcohol (2 mL). Ruphos (38 mg, 81.43 µmol) and Pd₂ (dba)₃ (37 mg, 40.41 µmol) were added. Under a nitrogen atmosphere, the mixture was stirred at 90°Cto allow for reacting for 12 hours. The reaction mixture was cooled to room temperature, and filtered through diatomite, and the filtrate was concentrated under a reduced pressure. The resulting residues were purified by the silica-gel column chromatography using dichloromethane/methanol as an elution system, to obtain the title Compound **77e** (200 mg, 381.94 µmol, yield: 95.19%) as yellow solid.
MS m/z (ESI): 524.2 [M+1]

### Step 6

### N-{5-fluoro-2-[3-methyl-6-(piperazin-1-yl)imidazo[1,5-a]pyridin-8-yl]phenyl}-N-(isopropyl)propionamide 77f

Compound **77e** (100 mg, 190.97 µmol) was dissolved in methane dioxide (3.0 mL), followed by the addition of trifluoroacetic acid (1.48 g, 12.98 mmol, 1 mL). The mixture was stirred at room temperature to allow for reacting for 1.0 hours. The reaction mixture was concentrated under a reduced pressure, to obtain the crude product of title Compound **77f** (120 mg). The crude product was directly used in the next step of reaction without purification.
MS m/z (ESI): 424.4 [M+1]

### Step 7

### (1R,3S,4S)-3-[4-(8-{4-fluoro-2-[N-(isopropyl)propionamido]phenyl}-3-methylimidazo[1,5-a]pyridin-6-yl)piperazin-1-carbonyl]-2-azabicyclo[2.2.2]octan-2-tert-butyl carboxylate 77g

Compound **32a** (61 mg, 238.93 µmol) and *N,N*-diisopropylethylamine (145 mg, 1.12 mmol) were dissolved in DCM (2.0 mL). The mixture was cooled to 0°C, followed by the addition of EDCI (65 mg, 339.07 µmol), HOBT (46 mg, 340.43 µmol) and the crude product of Compound **77f** (120 mg), and the mixture was then stirred at room temperature for 12 hours. The reaction mixture was concentrated under a reduced pressure. The resulting residues were purified by the silica-gel column chromatography using dichloromethane/methanol as an elution system, to obtain the title Compound **77g** (50 mg, 75.66 µmol, yield: 33.89%).
MS m/z (ESI): 661.5 [M+1]

### Step 8

### N-[5-fluoro-2-(3-methyl-6-{4-[(1R,3S,4S)-2-azabicyclo[2.2.2]octan-3-carbonyl]piperazin-1-yl}imidazo[1,5-a]pyridin-8-yl)phenyl]-N-(isopropyl)propionamide 77

Compound **77g** (50 mg, 75.66 µmol) was dissolved in methane dioxide (3.0 mL) and trifluoroacetic acid (1.0 mL). The mixture was stirred at room temperature to allow for reacting for 0.5 hours. The reaction mixture was concentrated under a reduced pressure, and purified by the HPLC (mobile phase: acetonitrile, water (0.1% ammonium bicarbonate), elution gradient: 20%-50%), to obtain the title Compound **77** (7.35 mg, 13.11 µmol, yield: 17.32 %) as yellow solid.
MS m/z (ESI): 561.3 [M+1]
¹H NMR (400 MHz, DMSO-*d₆*) δ 7.74 - 7.60 (m, 1H), 7.47 - 7.11 (m, 3H), 6.96 - 6.85 (m, 1H), 6.68 - 6.35 (m, 1H), 4.18 - 3.93 (m, 2H), 3.73 - 3.55 (m, 4H), 3.10 - 2.84 (m, 5H), 2.56 (s, 3H), 2.24 - 2.08 (m, 1H), 2.06 - 1.94 (m, 1H), 1.86 - 1.40 (m, 9H), 1.11 - 0.82 (m, 6H), 0.71 - 0.37 (m, 3H).

### Example 79

### N-[(1r,4r)-4-{[3-(4-{2-[ethyl(isopropyl)aminoformyl]-4-fluorophenyl}-1-methyl-1H-indazol-6-yl)-3-hydroxylazacyclobutan-1-yl]methyl}cyclohexyl]tert-butyl carbamate 79

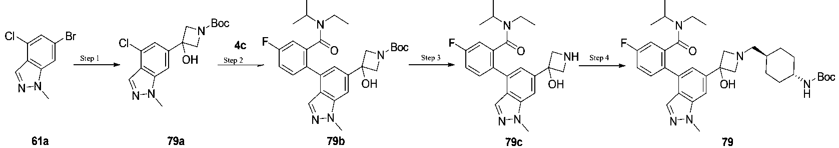

### Step 1

### 3-(4-chloro-1-methyl-1H-indazol-6-yl)-3-hydroxylazacyclobutan-1-tert-butyl carboxylate 79a

6-Bromo-4-chloro-1-methylindazole **61a** (300 mg, 1.22 mmol) was dissolved in THF(6 mL), to which isopropylmagnesium chloride (2M, 1.53 mL) was dropwise added at 0°C, followed by reacting for 2 hours at 50°C. The above mixture was cooled to 22°C, followed by the addition of 3-oxoazacyclobutan-1-tert-butyl carboxylate (732 mg, 4.28 mmol), and the reaction mixture was allowed to react at 22°C for 2 hours. The reaction mixture was quenched with water (50 mL), extracted with ethyl acetate (50.0 mL × 3), dried over anhydrous sodium sulfate, and filtered, and the filtrate was concentrated under a reduced pressure. The resulting residues were purified by the silica-gel column chromatography using petroleum ether/ethyl acetate as an elution system. to obtain the crude product of title Compound **79a** (480 mg) as white solid.
MS m/z (ESI): 338.1 [M+1]
¹H NMR (400 MHz, DMSO-*d₆*) δ 8.08 (s, 1H), 7.74 (s, 1H), 7.29 (s, 1H), 6.59 (s, 1H), 4.17 (d, *J* = 8.8 Hz, 2H), 4.08 (d, *J* = 5.2 Hz, 4H), 4.05 (d, *J* = 4.8 Hz, 1H), 1.42 (s, 9H).

### Step 2

### 3-(4-{2-[ethyl(isopropyl)aminoformyl]-4-fluorophenyl}-1-methyl-1H-indazol-6-yl)-3-hydroxylazacyclobutan-1-tert-butyl carboxylate 79b

Compound **79a** (250 mg, 592 µmol) and Compound **4c**(311 mg, 651µmol) were dissolved in dioxane (20 mL) and water (6 mL). Sodium carbonate (125 mg, 1.18 mmol) and Pd(dppf)Cl₂ (43.3 mg, 59.2 µmol) were added, and the mixture was stirred at 100 °C under a nitrogen atmosphere to allow for reacting for 7.0 hours. The reaction mixture was cooled to room temperature and filtered, and the filtrate was concentrated under a reduced pressure. The resulting residues were purified by the silica-gel column chromatography using petroleum ether/ethyl acetate as an elution system, to obtain the title Compound **79b** (117 mg, 229 µmol, yield: 38.7%) as white solid.
MS m/z (ESI): 510.9 [M+1]
¹H NMR (400 MHz, DMSO-*d₆*) δ 7.84 (s, 1H), 7.73 (s, 1H), 7.61 (dd, J = 8.4, 5.6 Hz, 1H), 7.39 (td, J = 8.8, 2.8 Hz, 1H), 7.31 (ddd, J = 11.6, 9.2, 2.8 Hz, 1H), 7.22 (s, 1H), 6.48 (s, 1H), 4.22 (d, J = 8.4 Hz, 1H), 4.11 - 3.99 (m, 6H), 3.42 (dt, J = 13.2, 6.4 Hz, 1H), 3.21 (dd, J = 13.6, 6.8 Hz, 1H), 2.95 - 2.73 (m, 1H), 1.41 (s, 9H), 0.92 (t, J = 25.6 Hz, 3H), 0.68 (dd, J = 11.6, 5.2 Hz, 3.67H), 0.14 (d, J = 6.4 Hz, 2.35H).

### Step 3

### N-ethyl-5-fluoro-2-[6-(3-hydroxylazacyclobutan-3-yl)-1-methyl-1H-indazol-4-yl]-N-(isopropyl)benzamide 79c

Compound **79b** (75 mg, 146 µmol) was dissolved in methane dioxide (2.0 mL), followed by the addition of trifluoroacetic acid (0.5 mL) at 0°C. The mixture was stirred at room temperature to allow for reacting for 1.0 hours. The reaction mixture was concentrated under a reduced pressure, to obtain the crude product of title Compound **79c** (60 mg). The crude product was directly used in the next step of reaction without purification.
MS m/z (ESI): 411.2 [M+1]

### Step 4

### N-[(1r,4r)-4-{[3-(4-{2-[ethyl(isopropyl)aminoformyl]-4-fluorophenyl}-1-methyl-1H-indazol-6-yl)-3-hydroxylazacyclobutan-1-yl]methyl}cyclohexyl]tert-butyl carbamate 79

The crude product of Compound **79c** (60 mg) and N-[(1*r*,4*r*)-4-formylcyclohexyl]tert-butyl carbamate (29.9 mg, 131 µmol) were dissolved in dichloromethane (0.2 mL). The reaction mixture was cooled to 0°C, followed by adding sodium triacetoxyborohydride (83.6 mg, 394 µmol) and stirring at room temperature for 12 hours. The reaction mixture was concentrated under a reduced pressure, the resulting residues were purified by the silica-gel column chromatography using dichloromethane/methanol as an elution system, and the obtained crude product was purified by the HPLC (mobile phase: acetonitrile, water (0.1% formic acid), elution gradient: 30%-50%), to obtain the title Compound **79** (35 mg, 56.2 µmol, yield: 42.7 %) as white solid.
MS m/z (ESI): 622.3 [M+1]
¹H NMR (400 MHz, DMSO-*d₆*) δ 8.20 (s, 0.51H), 7.80 (d, J = 4.8 Hz, 1H), 7.75 (d, J = 8.8 Hz, 1H), 7.57 (dd, J = 8.4, 5.6 Hz, 1H), 7.45 (d, J = 6.4 Hz, 1H), 7.39 (td, J = 8.8, 2.8 Hz, 1H), 7.29 (ddd, J = 11.6, 9.2, 2.8 Hz, 1H), 6.66 (d, J = 8.0 Hz, 1H), 5.95 (s, 1H), 4.19 (dd, J = 13.6, 6.8 Hz, 0.21H), 4.05 (d, J = 2.4 Hz, 3H), 3.69 (d, J = 7.2 Hz, 1H), 3.54 (d, J = 6.8 Hz, 1H), 3.42 (d, J = 6.4 Hz, 0.85H), 3.26 (d, J = 7.2 Hz, 2H), 3.23 - 3.17 (m, 1H), 3.14 (s, 1H), 2.84 (dt, J = 13.6, 7.2 Hz, 1H), 2.35 (d, J = 6.8 Hz, 2H), 1.87 - 1.66 (m, 4H), 1.36 (s, 9H), 1.25 - 1.06 (m, 3H), 1.05 - 0.89 (m, 2H), 0.89 - 0.58 (m, 7H), 0.12 (d, J = 6.8 Hz, 2H).

### Example 80

### 2-(6-{1-[(4-ethanesulfonamidocyclohexyl)methyl]pyrrolidin-3-yl}-3-methyl-[1,2,4]triazolo[4,3-a]pyridin-8-yl)-N-ethyl-5-fluoro-N-(isopropyl)benzamide 80

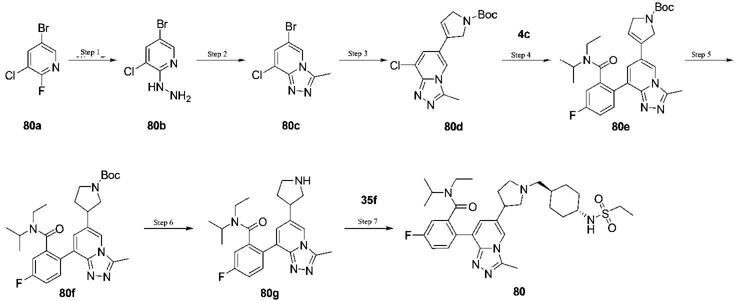

### Step 1

### 5-bromo-3-chloro-2-hydrazinopyridine 80b

5-Bromo-3-chloro-2-fluoropyridine **80a** (2.0 g, 9.5 mmol) was dissolved in ethanol (20 mL), followed by the addition of hydrazine hydrate (4.76 g, 95.04 mmol). The mixture was stirred at 85°C to allow for reacting for 12 hours. The reaction mixture was cooled to room temperature for solid precipitation, and then filtered, and the filter cake was washed with ethanol (20 mL x 3). The filtrate was concentrated under a reduced pressure, to obtain the crude product of title Compound **80b** (1.9 g, 8.54 mmol, yield: 89.96%) as white solid. The crude product was directly used in the next step of reaction without purification.
MS m/z (ESI): 223.9 [M+1]

### Step 2

### 6-bromo-8-chloro-3-methyl-[1,2,4]triazolo[4,3-a]pyridine 80c

Compound **80b** (1.4 g, 6.29 mmol) was dissolved in DME(10 mL), followed by the addition of trimethyl orthoformate (6.05 g, 50.34 mmol). The mixture was stirred at 90°C to allow for reacting for 3.0 hours. The reaction mixture was concentrated under a reduced pressure, and the resulting residues were purified by the silica-gel column chromatography using petroleum ether/ethyl acetate as an elution system, to obtain the title Compound **80c** (1.3 g, 5.27 mmol, yield: 83.81%) as white solid.
MS m/z (ESI): 247.8 [M+1]

### Step 3

### 3-{8-chloro-3-methyl-[1,2,4]triazolo[4,3-a]pyridin-6-yl}-2,5-dihydro-1H-pyrrol-1-tert-butyl formate 80d

Compound **80c** (800 mg, 3.25 mmol), 3-(tetramethyl-1,3,2-dioxaboran-2-yl)-2,5-dihydro-1*H*-pyrrol-1-tert-butyl formate (958 mg, 3.25 mmol) and cesium carbonate (3.17 g, 9.74 mmol) were dissolved in dioxane (9.0 mL) and water (3.0 mL). Pd (dppf)Cl₂ (237.48 mg, 324.55 µmol) was added. Under a nitrogen atmosphere, the mixture was stirred at 100°C to allow for reacting for 12 hours. The reaction mixture was cooled to room temperature, water (100 mL) was added, and the mixture was extracted with ethyl acetate (100 mL x 3). The organic phases were combined, washed with saturated brine (100 mL), dried over anhydrous sodium sulfate, and concentrated under a reduced pressure. The resulting residues were purified by the silica-gel column chromatography using petroleum ether/ethyl acetate as an elution system, to obtain the title Compound **80d** (650 mg, 1.94 mmol, yield: 59.82%).
MS m/z (ESI): 335.0 [M+1]

### Step 4

### 3-(8-{2-[ethyl(isopropyl)aminoformyl]-4-fluorophenyl}-3-methyl-[1,2,4]triazolo[4,3-a]pyridin-6-yl)-2,5-dihydro-1H-pyrrol-1-tert-butyl formate 80e

Compound **80d** (640 mg, 1.91 mmol), Compound **4c**(961.21 mg, 2.87 mmol) and cesium carbonate (1.87 g, 5.73 mmol) were dissolved in dioxane (9.0 mL) and water (3.0 mL). Pd(dtbpf)Cl₂ (124.59 mg, 191.16 µmol) was added. Under a nitrogen atmosphere, the mixture was stirred at 100°Cto allow for reacting for 12.0 hours. The reaction mixture was cooled to room temperature, followed by adding water (20 mL) and extracting with ethyl acetate (20 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **80e** (970 mg, 1.91 mmol, yield: 99.97%). MS m/z (ESI): 508.2 [M+1]

### Step 5

### 3-(8-{2-[ethyl(isopropyl)aminoformyl]-4-fluorophenyl}-3-methyl-[1,2,4]triazolo[4,3-a]pyridin-6-yl)pyrrolidin-1-tert-butyl formate 80f

Compound **80e** (200 mg, 394.01 µmol) was dissolved in THF (5.0 mL), followed by the addition of Pd/C (47.85 mg, 394.01 µmol). Under a hydrogen atmosphere (15 Psi), the mixture was stirred at room temperature for 12 hours. The reaction mixture was filtered, and the filtrate was concentrated under a reduced pressure, to obtain the crude product of title Compound **80f** (160 mg, 313.96 µmol, yield: 79.68%). The crude product was directly used in the next step of reaction without purification.
MS m/z (ESI): 510.3 [M+1]

### Step 6

### N-ethyl-5-fluoro-2-[3-methyl-6-(pyrrolidin-3-yl)-[1,2,4]triazolo[4,3-a]pyridin-8-yl]-N-(isopropyl)benzamide 80g

Compound **80f** (140 mg, 274.72 µmol) was dissolved in dichloromethane (3 mL), followed by the addition of trifluoroacetic acid (1.48 g, 12.98 mmol, 1 mL) at 0°C. The mixture was stirred at room temperature to allow for reacting for 0.5 hours. The reaction mixture was concentrated under a reduced pressure, to obtain the crude product of title Compound **80g** (110 mg). The crude product was directly used in the next step of reaction without purification. MS m/z (ESI): 410.2 [M+1]

### Step 7

### 2-(6-{1-[(4-ethanesulfonamidocyclohexyl)methyl]pyrrolidin-3-yl}-3-methyl-[1,2,4]triazolo[4,3-a]pyridin-8-yl)-N-ethyl-5-fluoro-N-(isopropyl)benzamide 80

The crude product of Compound **80g** (110 mg), [(1r,4r)-4-ethanesulfonamidocyclohexyl]methyl 4-methylphenyl-1-sulfonate **35f**(201.74 mg, 537.24 µmol), potassium carbonate (185.62 mg, 1.34 mmol) and TBAI(9.92 mg, 26.86 µmol) were dissolved in acetonitrile (2 mL), and the mixture was stirred at 80°C for 12 hours. was added to the reaction mixture, which was then extracted with ethyl acetate (50 mL x 3). The organic phases were combined, dried over anhydrous sodium sulfate, and concentrated under a reduced pressure. The resulting residues were purified by the HPLC (mobile phase: acetonitrile, water (0.1% ammonium bicarbonate), elution gradient: 21%-51%), to obtain the title Compound **80** (80 mg, 130.55 µmol, yield: 48.6%).
MS m/z (ESI): 613.2 [M+1]
¹H NMR (400 MHz, DMSO-*d₆*) δ 7.36 (m, 1H), 7.35 - 7.19 (m, 2H), 6.99 (d, J = 7.6 Hz, 1H), 3.54 - 3.38 (m, 2H), 3.30 - 3.19 (m, 2H), 3.05 - 2.91 (m, 4H), 2.87 - 2.79 (m, 1H), 2.68 (s, 3H), 2.66 - 2.59 (m, 2H), 2.48 - 2.39 (m, 1H), 2.33 - 2.13 (m, 3H), 1.91-1.73 (m, 5H), 1.39 - 1.28 (m, 1H), 1.26 - 1.14 (m, 5H), 0.95 - 0.81 (m, 7H), 0.78 - 0.71 (m, 1H), 0.32 - 0.19 (m, 2H).

### Example 82

### N-ethyl-5-fluoro-N-(isopropyl)-2-(6-{4-[(1R,3S,4S)-2-azabicyclo[2.2.2]octan-3-carbonyl]piperazin-1-yl}-3-(trifluoromethyl)imidazo[1,5-a]pyridin-8-yl)benzamide 82

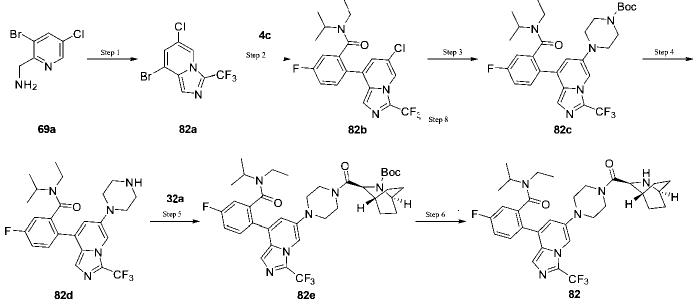

### Step 1

### 8-bromo-6-chloro-3-(trifluoromethyl)imidazo[1,5-a]pyridine 82a

At 0°C, Compound **69a** (500 mg, 1.94 mmol, hydrochloride) was dissolved in trifluoroacetic anhydride (8.0 mL), followed by the addition of p-toluenesulfonic acid (333.80 mg, 1.94 mmol). The mixture was stirred at 100°C to allow for reacting for 2.0 hours. The reaction mixture was cooled to 0°C, followed by dropwise adding ice water (50 mL) and further stirring for 0.5 hours . The mixture was filtered, the filter cake was dissolved with ethyl acetate (50 mL), and the organic phase was washed with saturated aqueous sodium bicarbonate solution (50 mL) and saturated brine (50 mL), dried over anhydrous sodium sulfate, and concentrated under a reduced pressure. The resulting residues were purified by the silica-gel column chromatography using petroleum ether/ethyl acetate as an elution system, to obtain the title Compound **82a** (300 mg, 1.00 mmol, yield: 51.68%).
MS m/z (ESI): 300.8 [M+1]

### Step 2

### 2-[6-chloro-3-(trifluoromethyl)imidazo[1,5-a]pyridin-8-yl]-N-ethyl-5-fluoro-N-(isopropyl)benzamide 82b

Compound **82a** (300 mg, 1.00 mmol), Compound **4c(**335.81 mg, 1.00 mmol) and sodium carbonate (318.52 mg, 3.01 mmol) were dissolved in dioxane (10 mL) and water (2.0 mL). Under a nitrogen atmosphere, Pd(dppf)Cl₂ (73.30 mg, 100.18 µmol) was added, and the mixture was stirred at 90°C to allow for reacting for 2.0 hours. The reaction mixture was cooled to room temperature, and stirred while slowly adding water (50 mL) to quench the reaction, and the aqueous phase was extracted with ethyl acetate (50 mL * 3). The organic phases were combined, washed with saturated brine (50 mL), dried over anhydrous sodium sulfate, and concentrated under a reduced pressure. The resulting residues were purified by the silica-gel column chromatography using petroleum ether/ethyl acetate as an elution system, to obtain the title Compound **82b** (244 mg, 570.33 µmol, yield: 56.93%).
MS m/z (ESI): 428.1 [M+1]

### Step 3

### 4-(8-{2-[ethyl(isopropyl)aminoformyl]-4-fluorophenyl}-3-(trifluoromethyl)imidazo[1,5-a]pyridin-6-yl)piperazin-1-tert-butyl carboxylate 82c

Compound **82b** (100 mg, 233.74 µmol), piperazin-1-tert-butyl carboxylate (43.53 mg, 233.74 µmol) and cesium carbonate (228.47 mg, 701.23 µmol) were dissolved in tert-amyl alcohol (5 mL). Ruphos (10.91 mg, 23.37 µmol) and Pd₂ (dba)₃ (21.40 mg, 23.37 µmol) were added. Under a nitrogen atmosphere, the mixture was stirred at 120°Cto allow for reacting for 12 hours. The reaction mixture was cooled to room temperature, and stirred while slowly adding water (20 mL) to quench the reaction, and the aqueous phase was extracted with ethyl acetate (20 mL * 3). The organic phases were combined, washed with saturated brine (30 mL), dried over anhydrous sodium sulfate, and concentrated under a reduced pressure. The resulting residues were purified by the silica-gel column chromatography using petroleum ether/ethyl acetate as an elution system, to obtain the title Compound **82c** (70 mg, 121.19 µmol, yield: 51.85%).
MS m/z (ESI): 578.3 [M+1]

### Step 4

*N*-ethyl-5-fluoro-2-[6-(piperazin-1-yl)-3-(trifluoromethyl)imidazo[1,5-a]pyridin-8-yl]-*N*-(isopropyl)benzamide **82d** Compound **82c** (70 mg, 121.19 µmol) was dissolved in methane dioxide (2.0 mL), followed by the addition of trifluoroacetic acid (0.2 g, 1.75 mmol, 135.14 µL) at 0°C. The mixture was stirred at room temperature to allow for reacting for 4.0 hours. The reaction mixture was concentrated under a reduced pressure, to obtain the crude product of title Compound **82d** (57.87 mg, 121.19 µmol, yield: 100%). The crude product was directly used in the next step of reaction without purification.
MS m/z (ESI): 478.1 [M+1]

### Step 5

### (1R,3S,4S)-3-[4-(8-{2-[ethyl(isopropyl)aminoformyl]-4-fluorophenyl}-3-(trifluoromethyl)imidazo[1,5-a]pyridin-6-yl)piperazin-1-carbonyl]-2-azabicyclo[2.2.2]octan-2-tert-butyl carboxylate 82e

Compound **32a** (27.11 mg, 106.18 µmol) and N,N-diisopropylethylamine (62.38 mg, 482.63 µmol, 84.07 µL) were dissolved in DCM (5.0 mL). The mixture was cooled to 0°C, followed by the addition of EDCI (27.76 mg, 144.79 µmol) and HOBt (19.56 mg, 144.79 µmol). The mixture was stirred at room temperature for 0.5 hours, followed by the addition of the crude product of Compound **82d** (57.87 mg, 121.19 µmol), and the mixture was further stirred at room temperature for 12 hours. The reaction mixture was quenched by adding water (20 mL), and extracted with ethyl acetate (10 mL x 3). The organic phase was washed with saturated brine (20 mL), dried over anhydrous sodium sulfate, and concentrated under a reduced pressure. The resulting residues were purified by the column chromatography using petroleum ether/ethyl acetate as an elution system, to obtain the title Compound **82e** (56 mg, 78.34 µmol, yield: 64.6%).
MS m/z (ESI): 715.3 [M+1]

### Step 6

### N-ethyl-5-fluoro-N-(isopropyl)-2-(6-{4-[(1R,3S,4S)-2-azabicyclo[2.2.2]octan-3-carbonyl]piperazin-1-yl}-3-(trifluoromethyl)imidazo[1,5-a]pyridin-8-yl)benzamide 82

Compound **82e** (56 mg, 78.34 µmol) was dissolved in methane dioxide (2.0 mL), and cooled to 0°C, followed by the addition of trifluoroacetic acid (0.2 g, 1.75 mmol, 135.14 µL). The mixture was stirred at room temperature to allow for reacting for 2.0 hours. The reaction mixture was concentrated under a reduced pressure. The residues were dissolved with dichloromethane (10 mL). The organic phase was washed with a 10% aqueous sodium bicarbonate solution (10 mL) and water (10 mL), and concentrated under a reduced pressure. The resulting residues were purified by the HPLC (mobile phase: acetonitrile, water (0.1% ammonium bicarbonate), elution gradient: 44%-64%), to obtain the title Compound **82** (14.57 mg, 23.70 µmol, yield: 30.26 %) as yellow solid.
MS m/z (ESI): 615.2 [M+1]
¹H NMR (400 MHz, DMSO-*d₆*) δ 7.72 - 7.66 (m, 1H), 7.50 (s, 1H), 7.46 - 7.36 (m, 2H), 7.29 (s, 1H), 7.03 (s, 1H), 3.91 (s, 1H), 3.75 - 3.38 (m, 6H), 3.10 (br s, 5H), 2.89 - 2.78 (m, 2H), 1.80 - 1.39 (m, 9H), 0.96 - 0.62 (m, 7H), 0.36 - 0.29 (m, 2H).

### Example 87

### N-[(1r,4r)-4-[(3-{4-[4-fluoro-2-(2-methyl-5-oxopyrrolidinyl-1-yl)phenyl]-1-methyl-1H-indazol-6-yl}pyrrolidin-1-yl)methyl]cyclohexyl]ethan-1-sulfonamide 87

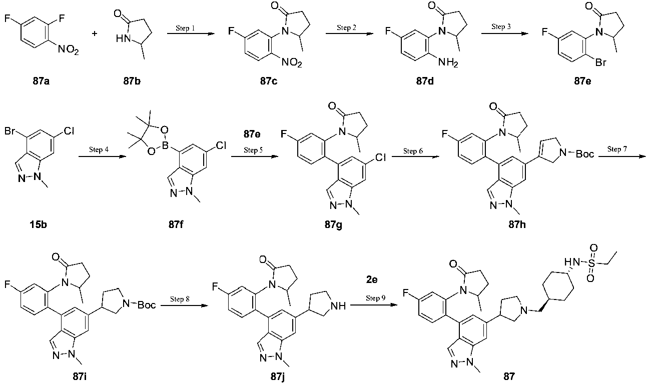

### Step 1

### 1-(5-fluoro-2-nitrophenyl)-5-methylpyrrolidin-2-one 87c

5-Methylpyrrolidin-2-one **87b** (1.87 g, 18.86 mmol) was dissolved in DMF(30 mL), and cooled to 0°C, followed by the addition of sodium hydride (528.00 mg, 19.80 mmol, 90% purity). The reaction mixture was stirred at room temperature for 1.0 hour and cooled to 0°C, followed by the dropwise addition of the solution of 2,4-difluoro-1-nitrobenzene **87a**(3.0 g, 18.86 mmol) in DMF(30 mL). The mixture was stirred at 0°C to allow for reacting for 2.0 hours. Water (50 mL) was dropwise added to the reaction mixture, which was then extracted with ethyl acetate (200 mL x 2). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **87c** (2.20 g, 9.24 mmol, yield: 48.98%) as yellow solid.
MS m/z (ESI): 239.1 [M+1]
¹H NMR (400 MHz, DMSO-*d₆*) δ 8.08 (dt, J = 8.8, 6.4 Hz, 1H), 7.55 (dd, J = 10.0, 2.8 Hz, 1H), 7.40 - 7.35 (m, 1H), 4.48 - 4.43 (m, 1H), 2.46 - 2.31 (m, 3H), 1.77 - 1.68 (m, 1H), 1.21 (d, J = 6.4 Hz, 3H).

### Step 2

### 1-(2-amino-5-fluorophenyl)-5-methylpyrrolidin-2-one 87d

Compound **87c** (2.20 g, 9.24 mmol) was dissolved in methanol (30 mL), followed by the addition of Pd/C (560.82 mg, 4.62 mmol). Under a hydrogen atmosphere (15 Psi), the mixture was stirred at room temperature to allow for reacting for 1.0 hour. The reaction mixture was filtered, and the filtrate was concentrated under a reduced pressure, to obtain the title Compound **87d** (1.67 g, 8.01 mmol, yield: 86.68%) as yellow solid.
MS m/z (ESI): 209.1 [M+1]
¹H NMR (400 MHz, DMSO-*d₆*) δ 7.77 (dd, J = 8.8, 6.0 Hz, 1H), 7.34 (dd, J = 9.2, 3.2 Hz, 1H), 7.23 (ddd, J = 8.8, 8.4, 3.2 Hz, 1H), 4.16 (dd, J = 13.2, 6.4 Hz, 1H), 2.47 - 2.30 (m, 3H), 1.78 - 1.66 (m, 1H), 1.06 (d, J = 6.4 Hz, 3H).

### Step 3

### 1-(2-bromo-5-fluorophenyl)-5-methylpyrrolidin-2-one 87e

Compound **87d** (1.51 g, 7.25 mmol) and copper bromide (1.94 g, 8.70 mmol) were dissolved in acetonitrile (70 mL), and cooled to 0°C, followed by the addition of tert-butyl nitrate (1.50 g, 14.50 mmol), and the mixture was stirred at room temperature for 2.0 hours. The reaction mixture was concentrated under a reduced pressure. The resulting residues were purified by the silica-gel column chromatography using petroleum ether/ethyl acetate as an elution system, to obtain the title Compound **87e** (1.47 g, 5.41 mmol, yield: 74.6%) as yellow oil.
MS m/z (ESI): 272.0 [M+1]
¹H NMR (400 MHz, DMSO-*d₆*) δ 7.77 (dd, J = 8.8, 6.0 Hz, 1H), 7.34 (dd, J = 9.2, 3.2 Hz, 1H), 7.23 (ddd, J = 8.8, 8.4, 3.2 Hz, 1H), 4.16 (dd, J = 13.2, 6.4 Hz, 1H), 2.47 - 2.30 (m, 3H), 1.78 - 1.66 (m, 1H), 1.06 (d, J = 6.4 Hz, 3H).

### Step 4

### 6-chloro-1-methyl-4-(tetramethyl-1,3,2-dioxaboran-2-yl)-1H-indazole 87f

Compound **15b** (1.00 g, 4.07 mmol), potassium acetate (800 mg, 8.15 mmol) and bis(pinacolato)diboron (1.03 g, 4.07 mmol) were dissolved in dioxane (20 mL). Under a nitrogen atmosphere, Pd(dppf)Cl₂ (298 mg, 407 µmol) was added, and the mixture was stirred at 80°C to allow for reacting for 12 hours. The mixture was cooled to room temperature, followed by the addition of water (50 mL) and extraction with ethyl acetate (30 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **87f** (920 mg, 3.14 mmol, yield: 77.2%) as white solid.
MS m/z (ESI): 393.1 [M+1]
¹H NMR (400 MHz, DMSO-*d₆*) δ 8.14 (d, J = 0.8 Hz, 1H), 8.00 (dd, J = 1.6, 1.0 Hz, 1H), 7.38 (d, J = 1.6 Hz, 1H), 4.04 (d, J = 6.0 Hz, 3H), 1.35 (s, 12H).

### Step 5

### 1-[2-(6-chloro-1-methyl-1H-indazol-4-yl)-5-fluorophenyl]-5-methylpyrrolidin-2-one 87g

Compound **87f** (500 mg, 1.71 mmol) and Compound **87e** (465 mg, 1.71 mmol) were dissolved in dioxane (28 mL) and water (4.0 mL). Sodium carbonate (362 mg, 3.42 mmol) and Pd(dppf)Cl₂ (125 mg, 170 µmol) were added, and the mixture was stirred at 80 °C under a nitrogen atmosphere to allow for reacting for 6.0 hours. The reaction mixture was cooled to room temperature, followed by adding water (50 mL) and extracting with ethyl acetate (50 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **87g** (460 mg, 1.29 mmol, yield: 75.2%) as white solid.

¹H NMR (400 MHz, DMSO-*d₆*) δ 7.90 (s, 1H), 7.73 (s, 1H), 7.57 (s, 1H), 7.37 (s, 1H), 7.29 (dd, J = 9.6, 2.8 Hz, 1H), 7.03 (d, J = 1.2 Hz, 1H), 4.07 (s, 3H), 3.08 (s, 1H), 2.32 (ddd, J = 16.8, 9.2, 4.4 Hz, 1H), 2.13 - 2.01 (m, 1H), 1.79 (s, 1H), 1.46 - 1.35 (m, 1H), 0.77 (d, J = 6.4 Hz, 3H).

### Step 6

### 3-{4-[4-fluoro-2-(2-methyl-5-oxopyrrolidinyl-1-yl)phenyl]-1-methyl-1H-indazol-6-yl}-2,5-dihydro-1H-pyrrol-1-tert-butyl formate 87h

Compound **87g** (340 mg, 950 µmol) and 3-(tetramethyl-1,3,2-dioxaboran-2-yl)-2,5-dihydro-1*H*-pyrrol-1-tert-butyl formate (308 mg, 1.05 mmol) were dissolved in dioxane (4.0 mL) and water (0.8 mL). Sodium carbonate (201 mg, 1.90 mmol) and Pd (dppf)Cl₂ (69.5 mg, 95.02 µmol) were added. Under a nitrogen atmosphere, the mixture was stirred at 100°Cto allow for reacting for 12 hours. The reaction mixture was cooled to room temperature, followed by adding water (20 mL) and extracting with ethyl acetate (20 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **87h** (300 mg, 611 µmol, yield: 64.3%) as yellow solid.

¹H NMR (400 MHz, DMSO-*d₆*) δ 7.72 - 7.65 (m, 2H), 7.62 - 7.54 (m, 1H), 7.36 (td, J = 8.4, 2.8 Hz, 1H), 7.30 - 7.22 (m, 2H), 6.44 (d, J = 9.2 Hz, 1H), 4.53 (s, 2H), 4.25 (s, 2H), 4.10 (d, J = 2.0 Hz, 3H), 3.04 (s, 1H), 2.31 (ddd, J = 16.4, 8.8, 4.4 Hz, 1H), 2.05 (dd, J = 17.2, 8.4 Hz, 1H), 1.74 (s, 1H), 1.47 (d, J = 9.2 Hz, 9H), 1.37 (dd, J = 18.4, 13.6 Hz, 1H), 0.78 - 0.70 (m, 3H).

### Step 7

### 3-{4-[4-fluoro-2-(2-methyl-5-oxopyrrolidinyl-1-yl)phenyl]-1-methyl-1H-indazol-6-yl}pyrrolidin-1-tert-butyl formate 87i

Compound **87h** (250 mg, 509 µmol) was dissolved in ethanol (10 mL), followed by the addition of Pd/C (80.0 mg, 752 µmol). Under a hydrogen atmosphere (15 Psi), the mixture was stirred at room temperature for 12 hours. The reaction mixture was filtered, and the filtrate was concentrated under a reduced pressure. The resulting residues were purified by the preparative thin-layer chromatography using petroleum ether/ethyl acetate as an elution system, to obtain the title Compound **87i** (186 mg, 377 µmol, yield: 74.1%) as yellow solid.
MS m/z (ESI): 493.2 [M+1]
¹H NMR (400 MHz, DMSO-*d₆*) δ 7.69 (d, J = 4.8 Hz, 1H), 7.60 - 7.53 (m, 2H), 7.35 (td, J = 8.4, 2.4 Hz, 1H), 7.26 (dd, J = 10.0, 2.4 Hz, 1H), 6.95 (s, 1H), 4.06 (s, 3H), 3.77 (dd, J = 10.0, 8.0 Hz, 1H), 3.52 (d, J = 8.0 Hz, 2H), 3.33 (s, 1H), 3.28 - 3.15 (m, 1H), 3.03 (s, 1H), 2.29 (dd, J = 16.8, 7.6 Hz, 2H), 2.18 - 2.00 (m, 2H), 1.78 (s, 1H), 1.41 (d, J = 3.6 Hz, 10H), 0.71 (dd, J = 6.0, 3.2 Hz, 3H).

### Step 8

### 1-{5-fluoro-2-[1-methyl-6-(pyrrolidin-3-yl)-1H-indazol-4-yl]phenyl}-5-methylpyrrolidin-2-one 87j

Compound **87i** (80.0 mg, 162.41 µmol) was dissolved in methane dioxide (2.0 mL), followed by the addition of trifluoroacetic acid (0.5 mL) at 0°C. The mixture was stirred at room temperature to allow for reacting for 1.0 hours. The reaction mixture was concentrated under a reduced pressure, to obtain the crude product of title Compound **87j** (60 mg). The crude product was directly used in the next step of reaction without purification.
MS m/z (ESI): 393.2 [M+1]

### Step 9

### N-[(1r,4r)-4-[(3-{4-[4-fluoro-2-(2-methyl-5-oxopyrrolidinyl-1-yl)phenyl]-1-methyl-1H-indazol-6-yl}pyrrolidin-1-yl)methyl]cyclohexyl]ethan-1-sulfonamide 87

The crude product of Compound **87j** (60 mg), potassium iodide (25.4 mg, 153 µmol) and potassium carbonate (42.3 mg, 306 µmol) were dissolved in DMAc (3.0 mL), followed by the addition of Compound **2e** (79.4 mg, 168 µmol), and the mixture was stirred at 80°C to allow for reacting for 12 hours. Water (10 mL) was added to the reaction mixture, which was then extracted with ethyl acetate (20 mL x 3). The organic phases were combined, dried over anhydrous sodium sulfate, and concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% formic acid), elution gradient: 30%-40%), to obtain the title Compound **87** (3.40 mg, 5.71 µmol, yield: 22.7%) as white solid.
MS m/z (ESI): 596.3 [M+1]
¹H NMR (400 MHz, DMSO-*d₆*) δ 8.36 (s, 1H), 7.64 (d, J = 2.4 Hz, 1H), 7.53 (ddd, J = 8.6, 6.4, 4.4 Hz, 2H), 7.33 (td, J = 8.4, 2.8 Hz, 1H), 7.28 - 7.22 (m, 1H), 6.99 (dd, J = 12.0, 5.2 Hz, 2H), 4.03 (s, 3H), 3.46 (s, 2H), 3.02 - 2.88 (m, 5H), 2.67 (s, 2H), 2.44 (d, J = 8.8 Hz, 1H), 2.27 (d, J = 7.2 Hz, 4H), 2.12 - 2.02 (m, 1H), 1.83 (d, J = 13.2 Hz, 6H), 1.36 (dd, J = 18.8, 10.4 Hz, 2H), 1.18 (td, J = 7.2, 4.4 Hz, 4H), 0.89 (m, 2H), 0.72 (dd, J = 6.0, 3.2 Hz, 3H).

### Example 89

### N-ethyl-5-ffuoro-2-[3-methyl-6-(6-{[(1r,4r)-4-ethanesulfonamidocyclohexyl]methyl}-2,6-diazaspirocyclo[3.3]heptan-2-yl)imidazo[1,5-a]pyridin-8-yl]-N-(isopropyl)benzamide 89

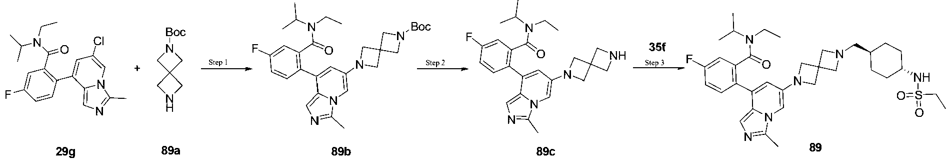

### Step 1

### 6-(8-{2-[ethyl(isopropyl)aminoformyl]-4-fluorophenyl}-3-methylimidazo[1,5-a]pyridin-6-yl)-2,6-diazaspirocyclo[3.3]heptan-2-tert-butyl carboxylate 89b

Compound **29g** (200 mg, 534.97 µmol), 2,6-diazaspirocyclo[3.3]heptan-2-tert-butyl carboxylate (106.06 mg, 534.97 µmol) and cesium carbonate (889 mg, 2.73 mmol) were dissolved in tert-amyl alcohol (3.0 mL). Ruphos (49.93 mg, 106.99 µmol) and Pd₂ (dba)₃ (48.99 mg, 53.50 µmol) were added. Under a nitrogen atmosphere, the mixture was stirred at 110°Cto allow for reacting for 12 hours. The reaction mixture was cooled to room temperature, followed by adding water (50 mL) and extracting with ethyl acetate (50 mL x 3). The organic phases were combined, dried over anhydrous sodium sulfate, and concentrated under a reduced pressure. The resulting residues were purified by the reverse phase preparative column chromatography (column: C8; mobile phase: acetonitrile, water, elution gradient: 45%-55%), to obtain the title Compound **89b** (350 mg, 653.41 µmol, yield: 71.85%).
MS m/z (ESI): 536.5[M+1]

### Step 2

### 2-(6-{2,6-diazaspirocyclo[3.3]heptan-2-yl}-3-methylimidazo[1,5-a]pyridin-8-yl)-N-ethyl-5-fluoro-N-(isopropyl)benzamide 89c

Compound **89b** (250 mg, 466.72 µmol) was dissolved in methane dioxide (3.0 mL), followed by the addition of trifluoroacetic acid (1.48 g, 12.98 mmol, 1 mL) at 0°C. The mixture was stirred at room temperature to allow for reacting for 1.0 hours. The reaction mixture was concentrated under a reduced pressure, to obtain the crude product of title Compound **89c** (200 mg). The crude product was directly used in the next step of reaction without purification. MS m/z (ESI): 436.3 [M+1]

### Step 3

### N-ethyl-5-fluoro-2-[3-methyl-6-(6-{[(1r,4r)-4-ethanesulfonamidocyclohexyl]methyl}-2,6-diazaspirocyclo[3.3]heptan-2-yl)imidazo[1,5-a]pyridin-8-yl]-N-(isopropyl)benzamide 89

Compound **89c** (100 mg, 181.96 µmol, trifluoroacetate) was dissolved in acetonitrile (2.0 mL), followed by the addition of [(1r,4r)-4-ethanesulfonamidocyclohexyl]methyl4-methylphenyl-1-sulfonate **35f** (136 mg, 362.18 µmol), potassium carbonate (126 mg, 911.66 µmol) and TBAI(7 mg, 18.95 µmol), and the mixture was stirred at 80°C for 12 hours. The reaction mixture was concentrated under a reduced pressure, and purified by the HPLC (mobile phase: acetonitrile, water (0.1% ammonia water), elution gradient:27%-57%), to obtain the title Compound **89** (4.76 mg, 7.45 µmol, yield: 4.09%) as yellow solid.
MS m/z (ESI): 639.5 [M+1]
¹H NMR (400 MHz, DMSO-*d₆*) δ 7.70 - 7.61 (m, 1H), 7.40 - 7.27 (m, 2H), 6.99 - 6.94 (m, 2H), 6.26 - 6.19 (m, 1H), 3.82 (s, 4H), 3.47 - 3.38 (m, 2H), 3.21 (s, 4H), 3.01 - 2.89 (m, 4H), 2.52 - 2.51 (m, 3H), 2.19 - 2.12 (m, 2H), 1.89 - 1.79 (m, 2H), 1.75 - 1.66 (m, 2H), 1.20 - 1.11 (m, 6H), 0.94 - 0.72 (m, 9H), 0.31 - 0.26 (m, 2H).

### Example 90

### N-ethyl-5-fluoro-2-(3-methyl-6-{1-[(1R,3S,4S)-2-azabicyclo[2.2.2]octan-3-carbonyl]azacyclobutan-3-yl}imidazo[1,5-a]pyridin-8-yl)-N-(isopropyl)benzamide 90

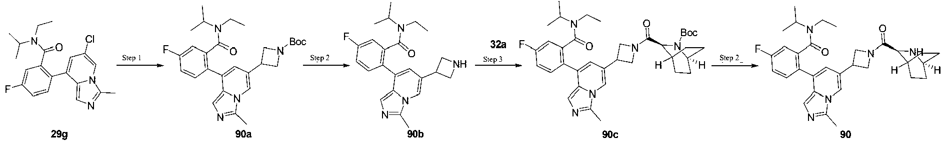

### Step 1

### 3-(8-{2-[ethyl(isopropyl)aminoformyl]-4-fluorophenyl}-3-methylimidazo[1,5-a]pyridin-6-yl)azacyclobutan-1-tert-butyl carboxylate 90a

Compound **29g** (500 mg, 1.34 mmol), potassium (1-(tert-butoxycarbonyl)azacyclobutan-3-yl)trifluoroborate (351.89 mg, 1.34 mmol) and cesium carbonate (1.31 g, 4.01 mmol) were dissolved in water (0.5 mL) and toluene (5.0 mL). Ruphos (70 mg, 150.01 µmol) and Pd₂ (dba)₃ (122.47 mg, 133.74 µmol) were added. Under a nitrogen atmosphere, the mixture was stirred at 100°Cto allow for reacting for 12 hours. The reaction mixture was cooled to room temperature, and filtered through diatomite, and the filtrate was concentrated under a reduced pressure. The resulting residues were purified by the silica-gel column chromatography using petroleum ether/ethyl acetate as an elution system, to obtain the title Compound **90a** (150 mg, 303.28 µmol, yield: 22.68%) as yellow solid.
MS m/z (ESI): 495.4 [M+1]
¹H NMR (400 MHz, DMSO-*d₆*) δ 7.69 (br dd, J = 5.6, 8.4 Hz, 1H), 7.42 - 7.30 (m, 3H), 7.10 - 7.02 (m, 1H), 6.73 - 6.65 (m, 1H), 4.20 (br d, J = 6.6 Hz, 2H), 3.97 - 3.84 (m, 2H), 3.83 - 3.73 (m, 1H), 3.54 - 3.42 (m, 1H), 2.97 (br s, 2H), 2.60 (s, 3H), 1.40 (s, 9H), 0.90 - 0.72 (m, 7H), 0.33 (br d, J = 6.4 Hz, 2H).

### Step 2

### 2-[6-(azacyclobutan-3-yl)-3-methylimidazo[1,5-a]pyridin-8-yl]-N-ethyl-5-fluoro-N-(isopropyl)benzamide 90b

Compound **90a** (150 mg, 303.28 µmol) was dissolved in methane dioxide (3.0 mL), followed by the addition of trifluoroacetic acid (1.48 g, 12.98 mmol, 1 mL) at 0°C. The mixture was stirred at room temperature to allow for reacting for 1.0 hours. The reaction mixture was concentrated under a reduced pressure, to obtain the crude product of title Compound **90b** (100 mg). The crude product was directly used in the next step of reaction without purification. MS m/z (ESI): 395.3 [M+1]

### Step 3

### (1R,3S,4S)-3-[3-(8-{2-[ethyl(isopropyl)aminoformyl]-4-fluorophenyl}-3-methylimidazo[1,5-a]pyridin-6-yl)azacyclobutan-1-carbonyl]-2-azabicyclo[2.2.2]octan-2-tert-butyl carboxylate 90c

(1*R,*3*S*,4*S*)-2-[(tert-butoxy)carbonyl]-2-azabicyclo[2.2.2]octan-3-carboxylic acid **32a**(51 mg, 199.76 µmol) and *N,N*-diisopropylethylamine (80 mg, 618.99 µmol) were dissolved in DCM (2.0 mL). The mixture was cooled to 0°C, followed by the addition of EDCI (57 mg, 297.34 µmol), HOBT (40 mg, 296.03 µmol) and Compound **90b** (100 mg), and the mixture was stirred at room temperature for 12 hours. The reaction mixture was concentrated under a reduced pressure. The resulting residues were purified by the silica-gel column chromatography using petroleum ether/ethyl acetate as an elution system, to obtain the title Compound **90c** (120 mg, 189.94 µmol, yield: 62.6%).
MS m/z (ESI): 632.4 [M+1]

### Step 4

### N-ethyl-5-fluoro-2-(3-methyl-6-{1-[(1R,3S,4S)-2-azabicyclo[2.2.2]octan-3-carbonyl]azacyclobutan-3-yl}imidazo[1,5-a]pyridin-8-yl)-N-(isopropyl)benzamide 90

Compound **90c** (120 mg, 189.94 µmol) was dissolved in methane dioxide (3.0 mL) and trifluoroacetic acid (1.0 mL). The mixture was stirred at room temperature to allow for reacting for 1.0 hours. The reaction mixture was concentrated under a reduced pressure, regulated to PH = 8-9 with ammonia water, and then purified by the HPLC (mobile phase: acetonitrile, water (0.1% ammonium bicarbonate), elution gradient: 14%-44%), to obtain the title Compound **90** (31.39 mg, 59.04 µmol, yield: 31.08%) as yellow solid.
MS m/z (ESI): 532.4 [M+1]
¹H NMR (400 MHz, DMSO-*d₆*) δ 8.10 - 7.99 (m, 1H), 7.74 - 7.63 (m, 1H), 7.43 - 7.27 (m, 2H), 7.12 - 7.01 (m, 1H), 6.78 - 6.61 (m, 1H), 4.69 - 3.78 (m, 6H), 3.65 - 3.57 (m, 1H), 3.50 - 3.41 (m, 1H), 3.02 - 2.86 (m, 1H), 2.80 - 2.73 (m, 1H), 2.60 (s, 3H), 1.83 - 1.34 (m, 9H), 1.12 - 0.67 (m, 7H), 0.34 - 0.25 (m, 2H).

### Example 93

### 2-(1-chloro-3-methyl-6-{4-[(1R,3S,4S)-2-azabicyclo[2.2.2]octan-3-carbonyl]piperazin-1-yl}imidazo[1,5-a]pyridin-8-yl)-N-ethyl-5-fluoro-N-(isopropyl)benzamide 93

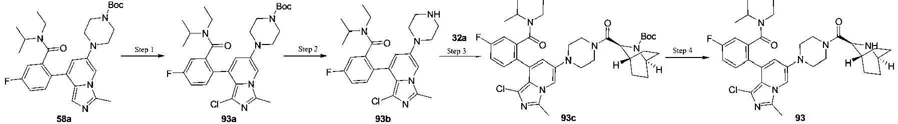

### Step 1

### 4-(1-chloro-8-{2-[ethyl(isopropyl)aminoformyl]-4-fluorophenyl}-3-methylimidazo[1,5-a]pyridin-6-yl)piperazin-1-tert-butyl carboxylate 93a

Compound **58a** (400 mg, 763.88 µmol) and NCS (122.40 mg, 916.66 µmol) were dissolved in acetonitrile (3.0 mL). The mixture was stirred at 80°C to allow for reacting for 12 hours. Ethyl acetate (50 mL) was added to the reaction mixture, which was then washed with water (50 mL x 3). The organic phase was dried over anhydrous sodium sulfate, and concentrated under a reduced pressure. The resulting residues were purified by the silica-gel column chromatography using petroleum ether/ethyl acetate as an elution system, to obtain the title Compound **93a** (200 mg, 358.37 µmol, yield: 46.91%).
MS m/z (ESI): 558.3 [M+1]
¹H NMR (400 MHz, CDCl₃) δ 7.33 - 7.27 (m, 1H), 7.11 - 6.98 (m, 2H), 6.84 - 6.79 (m, 1H), 6.70 - 6.65 (m, 1H), 3.71 - 3.62 (m, 1H), 3.56 - 3.46 (m, 4H), 3.45 - 3.36 (m, 1H), 3.09 - 2.98 (m, 2H), 2.90 - 2.81 (m, 2H), 2.70 - 2.69 (m, 2H), 2.54 - 2.48 (m, 3H), 1.41 (s, 9H), 0.97 - 0.73 (m, 7H), 0.39 - 0.32 (m, 2H).

### Step 2

### 2-[1-chloro-3-methyl-6-(piperazin-1-yl)imidazo[1,5-a]pyridin-8-yl]-N-ethyl-5-fluoro-N-(isopropyl)benzamide 93b

Compound **93a** (60 mg, 107.51 µmol) was dissolved in methane dioxide (3.0 mL), followed by the addition of trifluoroacetic acid (1.48 g, 12.98 mmol, 1 mL) at 0°C. The mixture was stirred at room temperature to allow for reacting for 0.5 hours. The reaction mixture was concentrated under a reduced pressure, to obtain the crude product of title Compound **93b** (60 mg). The crude product was directly used in the next step of reaction without purification. MS m/z (ESI): 458.1 [M+1]

### Step 3

### (1R,3S,4S)-3-[4-(1-chloro-8-{2-[ethyl(isopropyl)aminoformyl]-4-fluorophenyl}-3-methylimidazo[1,5-a]pyridin-6-yl)piperazin-1-carbonyl]-2-azabicyclo[2.2.2]octan-2-tert-butyl carboxylate 93c

(1*R*,3*S*,4*S*)-2-[(tert-butoxy)carbonyl]-2-azabicyclo[2.2.2]octan-3-carboxylic acid **32a**(26.78 mg, 104.90 µmol) and *N,N*-diisopropylethylamine (40.67 mg, 314.69 µmol, 54.81 µL) were dissolved in DCM (3.0 mL). The mixture was cooled to 0°C, followed by the addition of EDCI (30.16 mg, 157.34 µmol), HOBT (21.26 mg, 157.34 µmol) and the crude product of Compound **93b** (60 mg), and the mixture was then stirred at room temperature for 12 hours. Dichloromethane (20 mL) was added to the reaction mixture, and the organic phases were washed with water (20 mL x 3), dried over anhydrous sodium sulfate, and concentrated under a reduced pressure. The resulting residues were purified by the preparative thin-layer chromatography using petroleum ether/ethyl acetate as an elution system, to obtain the title Compound **93c** (40 mg, 57.53 µmol, yield: 54.85 %).
MS m/z (ESI): 695.3 [M+1]

### Step 4

### 2-(1-chloro-3-methyl-6-{4-[(1R,3S,4S)-2-azabicyclo[2.2.2]octan-3-carbonyl]piperazin-1-yl}imidazo[1,5-a]pyridin-8-yl)-N-ethyl-5-fluoro-N-(isopropyl)benzamide 93

Compound **93c** (60 mg, 86.30 µmol) was dissolved in methane dioxide (3.0 mL) and trifluoroacetic acid (1.0 mL). The mixture was stirred at room temperature to allow for reacting for 1.0 hours. The reaction mixture was concentrated under a reduced pressure, and purified by the HPLC (mobile phase: acetonitrile, water (0.1% ammonia water), elution gradient: 28%-58%), to obtain the title Compound **93** (30 mg, 50.41 µmol, yield: 58.41 %) as yellow solid.
MS m/z (ESI): 595.3 [M+1]
¹H NMR (400 MHz, DMSO-*d₆*) δ 7.48 - 7.39 (m, 1H), 7.38 - 7.28 (m, 3H), 6.61 (br s, 1H), 3.99 - 3.90 (m, 1H), 3.81 - 3.43 (m, 6H), 3.15 - 2.90 (m, 5H), 2.85 (br s, 1H), 2.55 (s, 3H), 1.87 - 1.70 (m, 3H), 1.68 - 1.55 (m, 3H), 1.51 - 1.38 (m, 3H), 1.07 - 0.73 (m, 7H), 0.35 - 0.25 (m, 2H).

### Example 94

### N-(2,2-difluoroethyl)-5-fluoro-2-(3-methyl-6- {4-[(1R,3S,4S)-2-azabicyclo[2.2.2]octan-3-carbonyl]piperazin-1-yl}imidazo[1,5-a]pyridin-8-yl)-N-(isopropyl)benzamide 94

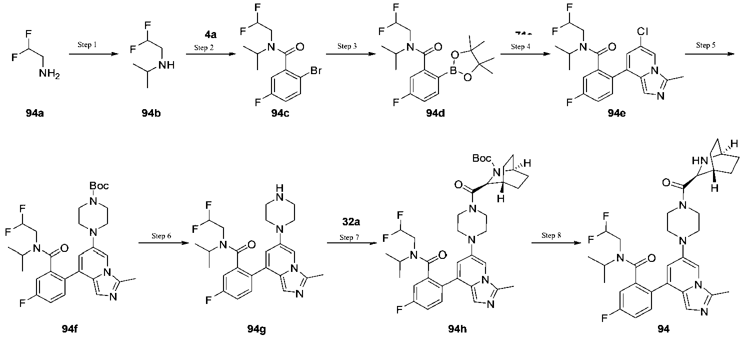

### Step 1

### (2,2-difluoroethyl)(isopropyl)amine 94b

2,2-difluoroethyl-1-amine **94a** (10 g, 123.36 mmol, 8.70 mL) was dissolved in acetone (15 mL), followed by the addition of 10% Pd/C (2.19 g, 20.60 mmol). Under a hydrogen atmosphere (45 PSI), the mixture was heated to 50°C and stirred to allow for reacting for 12.0 hours . The reaction mixture was filtered through diatomite, and washed with methanol. The filtrate was concentrated to 50 mL, and the resulting mixture was regulated to PH = 2 with 2M hydrochloric acid . The mixture was concentrated under a reduced pressure, to obtain the hydrochloride of title Compound **94b** (12.0 g, 97.45 mmol, yield: 78.99%) as white solid.
MS m/z (ESI): 124.2 [M+1]
¹H NMR (400 MHz, DMSO-*d₆*) δ 9.69 - 9.62 (m, 1H), 6.69 - 6.36 (m, 1H), 3.54 - 3.43 (m, 2H), 3.40 - 3.30 (m, 1H), 1.32 - 1.21 (m, 6H).

### Step 2

### 2-bromo-N-(2,2-difluoroethyl)-5-fluoro-N-(isopropyl)benzamide 94c

Compound **4a** (9 g, 41.09 mmol), Compound **94b** (9.84 g, 61.64 mmol, hydrochloride) and DIPEA(15.93 g, 123.28 mmol, 21.47 mL) were dissolved in dichloromethane (120 mL). The mixture was cooled to 0°C, followed by the addition of HATU (23.44 g, 61.64 mmol). The mixture was stirred at 25°C for 12 hours. A saturated aqueous sodium carbonate solution (120 mL) was added to the reaction mixture, which was then extracted with dichloromethane (80ml×3). The organic layer was dried over anhydrous sodium sulfate, filtered and concentrated under a reduced pressure. The resulting residues were purified by the silica-gel column chromatography using petroleum ether/ethyl acetate as an elution system, to obtain the title Compound **94c** (5 g, 15.43 mmol, yield37.54%) as white solid.
MS m/z (ESI): 323.7 [M+1]

### Step 3

### N-(2,2-difluoroethyl)-5-fluoro-N-isopropyl-2-(tetramethyl-1,3,2-dioxaboran-2-yl)benzamide 94d

Compound **94c** (2 g, 6.17 mmol), potassium acetate (1.82 g, 18.51 mmol) and bis(pinacolato)diboron (4.70 g, 18.51 mmol) were dissolved in DMSO(40 mL). Under a nitrogen atmosphere, Pd(dppf)Cl₂ (451.48 mg, 617.02 µmol) was added, and the mixture was stirred at 100°C to allow for reacting for 12 hours. After the mixture was cooled to room temperature, 80 mL of water was added, and the mixture was extracted with ethyl acetate (80ml × 3). The organic layer was dried over anhydrous sodium sulfate, filtered and concentrated under a reduced pressure to obtain residues, i.e., crude products, which were then purified by the silica-gel column chromatography using petroleum ether/ethyl acetate as an elution system, to obtain the title Compound **94d** (2.5 g, 6.73 mmol, yield: 109%).
MS m/z (ESI): 372.2 [M+1]

### Step 4

### 2-{6-chloro-3-methylimidazo[1,5-a]pyridin-8-yl}-N-(2,2-difluoroethyl)-5-fluoro-N-(isopropyl)benzamide 94e

Compound **71e** (450 mg, 1.83 mmol), Compound **94d(1.13** g, 1.83 mmol) and sodium carbonate (582.82 mg, 5.50 mmol) were dissolved in dioxane (12 mL) and water (2.0 mL). Under a nitrogen atmosphere, Pd(dppf)Cl₂ (134.12 mg, 183.30 µmol) was added, and the mixture was stirred at 95°C to allow for reacting for 12.0 hours. The reaction mixture was cooled to room temperature, followed by adding water (30 mL) and extracting with ethyl acetate (50 mL x 3). The organic phases were combined, dried over anhydrous sodium sulfate and filtered, and the filtrate was concentrated under a reduced pressure. The resulting residues were purified by the silica-gel column chromatography using petroleum ether/ethyl acetate as an elution system, to obtain the title Compound **94e** (410 mg, 1.00 mmol, yield: 54.58%).
MS m/z (ESI): 410.2 [M+1]

### Step 5

### 4-(8-{2-[(2,2-difluoroethyl)(isopropyl)aminoformyl]-4-fluorophenyl}-3-methylimidazo[1,5-a]pyridin-6-yl}piperazin-1-tert-butyl carboxylate 94f

Compound **94e** (410 mg, 1.00 mmol), piperazin-1-tert-butyl carboxylate (372.65 mg, 2.00 mmol) and cesium carbonate (977.86 mg, 3.00 mmol) were dissolved in tert-amyl alcohol (7.0 mL). Ruphos (93.36 mg, 200.08 µmol) and Pd₂ (dba)₃ (91.61 mg, 100.04 µmol) were added. Under a nitrogen atmosphere, the mixture was stirred at 95°Cto allow for reacting for 12 hours. The reaction mixture was cooled to room temperature, and filtered through diatomite, and the filtrate was concentrated under a reduced pressure. The resulting residues were purified by the silica-gel column chromatography using petroleum ether/ethyl acetate as an elution system, to obtain the title Compound **94f** (460 mg, 821.98 µmol, yield: 82.16%) as yellow oil.
MS m/z (ESI): 560.3 [M+1]

### Step 6

### N-(2,2-difluoroethyl)-5-fluoro-2-[3-methyl-6-(piperazin-1-yl)imidazo[1,5-a]pyridin-8-yl]-N-(isopropyl)benzamide 94g

Compound **94f** (150 mg, 268.04 µmol) was dissolved in methane dioxide (2.0 mL), followed by the addition of trifluoroacetic acid (740.00 mg, 6.49 mmol, 0.5 mL). The mixture was stirred at room temperature to allow for reacting for 1.0 hours. The reaction mixture was concentrated under a reduced pressure, to obtain the crude product of title Compound **94g** (155 mg). The crude product was directly used in the next step of reaction without purification. MS m/z (ESI): 460.1 [M+1]

### Step 7

### (1R,3S,4S)-3-[4-(8-{2-[(2,2-difluoroethyl)(isopropyl)aminoformyl]-4-fluorophenyl}-3-methylimidazo[1,5-a]pyridin-6-yl)piperazin-1-carbonyl]-2-azabicyclo[2.2.2]octan-2-tert-butyl carboxylate 94h

Compound **32a** (75.27 mg, 294.80 µmol) and *N,N-*diisopropylethylamine (242.46 mg, 1.88 mmol) were dissolved in DCM (3.0 mL). The mixture was cooled to 0°C, followed by the addition of EDCI (77.58 mg, 404.68 µmol), HOBT (55.41 mg, 410.04 µmol) and the crude product of Compound **94g** (155 mg), and the mixture was stirred at room temperature for 12 hours. A saturated aqueous sodium carbonate solution (15 mL) was added to the reaction mixture, which was then extracted with dichloromethane (20 mL x 3). The organic phases were combined, dried over anhydrous sodium sulfate and filtered, and the filtrate was concentrated under a reduced pressure. The resulting residues were purified by the silica-gel column chromatography using petroleum ether/ethyl acetate as an elution system, to obtain the title Compound **94h** (130 mg, 186.57 µmol, yield: 69.61%).
MS m/z (ESI): 697.4 [M+1]

### Step 8

### N-(2,2-difluoroethyl)-5-fluoro-2-(3-methyl-6- {4-[(1R,3S,4S)-2-azabicyclo[2.2.2]octan-3-carbonyl]piperazin-1-yl}imidazo[1,5-a]pyridin-8-yl)-N-(isopropyl)benzamide 94

Compound **94h** (130 mg, 186.57 µmol) was dissolved in methane dioxide (2.0 mL) and trifluoroacetic acid (0.75 mL). The mixture was stirred at room temperature to allow for reacting for 0.5 hours. The reaction mixture was concentrated under a reduced pressure, and purified by the HPLC (mobile phase: acetonitrile, water (0.1% ammonia water), elution gradient: 36%-66%), to obtain the title Compound **94** (32.3 mg, 54.13 µmol, yield: 29.01 %) as yellow solid.
MS m/z (ESI): 597.4 [M+1]
¹H NMR (400 MHz, DMSO-*d₆*) δ 7.73 - 7.61 (m, 1H), 7.49 - 7.25 (m, 3H), 6.95 (s, 1H), 6.65 - 6.52 (m, 1H), 5.95 - 5.52 (m, 1H), 3.94 (s, 1H), 3.82 - 3.43 (m, 7H), 3.15 - 2.74 (m, 6H), 2.71 - 2.53 (m, 3H), 1.90 - 1.21 (m, 10H), 1.03 - 0.84 (m, 3H), 0.33 (s, 2H).

### Example 98

### N-ethyl-5-fluoro-2-[3-methyl-6-(4-{[(1s,3s)-3-ethanesulfonamidocyclobutylmethyl}piperazin-1-yl)imidazo[1,5-a]pyridin-8-yl]-N-(isopropyl)benzamide 98

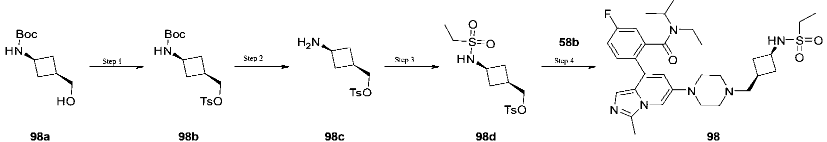

### Step 1

### N-[(1s,3s) -3-{[(4-methylphenylsulfonyl)oxy]methyl}cyclobutyl]tert-butyl carbamate 98b

*N*-[(1*s*,3*s*)-3-(hydroxymethyl)cyclobutyl]tert-butyl carbamate **98a** (200 mg, 993.73 µmol), triethylamine (301.67 mg, 2.98 mmol, 415.52 µL) and DMAP(12.14 mg, 99.37 µmol) were dissolved in dichloromethane (4 mL), and cooled to 0°C, followed by the addition of 4-toluensulfonyl chloride (227.34 mg, 1.19 mmol), and the mixture was stirred at room temperature for 12 hours. Dichloromethane (40 mL) was added to the reaction mixture, which was then washed with water (40 mL×3). The organic phase was dried over anhydrous sodium sulfate, and concentrated under a reduced pressure. The resulting residues were purified by the silica-gel column chromatography using petroleum ether/ethyl acetate as an elution system, to obtain the title Compound **98b** (300 mg, 844.01 µmol, yield: 84.93%) as white solid.
MS m/z (ESI): 378.0 [M+23]

### Step 2

### [(1s,3s)-3-aminocyclobutyl]methyl-4-methylbenzen-1-sulfonate 98c

Compound **98b** (300 mg, 844.01 µmol) was dissolved in dichloromethane (3.0 mL), and cooled to 0°C, followed by the addition of trifluoroacetic acid (1.48 g, 12.98 mmol, 1 mL). The mixture was stirred at room temperature to allow for reacting for 0.5 hours. The reaction mixture was concentrated under a reduced pressure, to obtain the crude product of title Compound **98c** (300 mg) as colorless oil.
MS m/z (ESI): 256.0 [M+1]

### Step 3

### [(1s,3s)-3-ethanesulfonamidocyclobutyl]methyl4-methylbenzen-1-sulfonate 98d

The crude product of Compound **98c** (300 mg) was dissolved in dichloromethane (5 mL), followed by the dropwise addition of triethylamine (246.57 mg, 2.44 mmol, 339.62 µL), and then the slow dropwise addition of ethanesulfonyl chloride (125.32 mg, 974.67 µmol, 92.35 µL). The mixture was stirred at room temperature for 12 hours. The reaction mixture was diluted with dichloromethane (40 mL), and washed with water (40 mL×3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **98d** (200 mg, 575.62 µmol, yield: 70.87%) as white solid.
MS m/z (ESI): 347.9 [M+1]
¹H NMR (400 MHz, CDCl₃) δ 7.84 - 7.77 (m, 2H), 7.38 (d, J = 8.3 Hz, 2H), 4.45 (br d, J = 9.1 Hz, 1H), 4.02 - 3.96 (m, 2H), 3.89 - 3.76 (m, 1H), 3.04 - 2.95 (m, 2H), 2.53 - 2.49 (m, 1H), 2.49 - 2.48 (m, 3H), 2.48 - 2.44 (m, 1H), 2.35 - 2.22 (m, 1H), 1.83 - 1.70 (m, 2H), 1.40 - 1.32 (m, 3H).

### Step 4

### N-ethyl-5-fluoro-2-[3-methyl-6-(4-{[(1s,3s)-3-ethanesulfonamidocyclobutylmethyl}piperazin-1-yl)imidazo[1,5-a]pyridin-8-yl]-N-(isopropyl)benzamide 98

Compound **58b** (90 mg, 167.43 µmol, trifluoroacetate) was dissolved in acetonitrile (2.0 mL), followed by the addition of Compound **98d** (116.34 mg, 334.85 µmol), potassium carbonate (115.70 mg, 837.13 µmol) and TBAI(6.18 mg, 16.74 µmol), and the mixture was stirred at 80°C for 12 hours. Water (30 mL) was added to the reaction mixture, which was then extracted with dichloromethane (50 mL x 3). The organic phases were combined, dried over anhydrous sodium sulfate, and concentrated under a reduced pressure, and the resulting residues were purified by the HPLC (mobile phase: acetonitrile, water (0.1% ammonia water), elution gradient:24%-54%), to obtain the title Compound **98** (40 mg, 66.80 µmol, yield: 39.9 %) as yellow solid.
MS m/z (ESI): 599.4 [M+1]
¹H NMR (400 MHz, DMSO-*d₆*) δ 7.70 - 7.63 (m, 1H), 7.40 - 7.22 (m, 4H), 6.99 - 6.93 (m, 1H), 6.63 - 6.56 (m, 1H), 3.64 - 3.52 (m, 1H), 3.48 - 3.39 (m, 1H), 3.08 - 2.82 (m, 7H), 2.57 - 2.51 (m, 3H), 2.46 (br s, 4H), 2.42 - 2.25 (m, 5H), 2.13 - 2.01 (m, 1H), 1.66 - 1.53 (m, 2H), 1.17 (t, J = 7.3 Hz, 3H), 1.12 - 0.99 (m, 1H), 0.90 (br d, J = 6.6 Hz, 3H), 0.81 (t, J = 6.9 Hz, 2H), 0.76 - 0.68 (m, 1H), 0.34 - 0.25 (m, 2H).

### Example 101

### N-{4-[(3-{4-[2-(4,4-difluoro-2-methylpiperidin-1-carbonyl)-4-fluorophenyl]-1-methyl-1H-indazol-6-yl}pyrrolidin-1-yl)methyl]cyclohexyl}ethan-1-sulfonamide 101

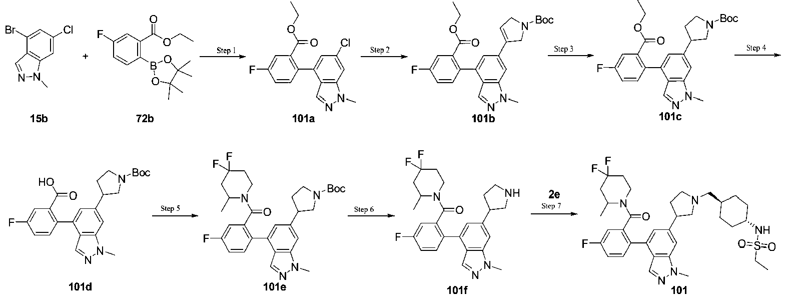

### Step 1

### 2-(6-chloro-1-methyl-1H-indazol-4-yl)-5-ethyl fluorobenzoate 101a

Compound **15b** (1.00 g, 4.07 mmol) and Compound **72b** (1.32 g, 4.48 mmol) were dissolved in dioxane (18 mL) and water (2.0 mL). Sodium carbonate (1.30 g, 12.22 mmol) and Pd(dppf)Cl₂ (298 mg, 407 µmol) were added, and the mixture was stirred at 70 °C under a nitrogen atmosphere to allow for reacting for 16 hours. The reaction mixture was cooled to room temperature, and filtered through diatomite, and the filtrate was concentrated under a reduced pressure. The resulting residues were purified by the silica-gel column chromatography using petroleum ether/ethyl acetate as an elution system, to obtain the title Compound **101a** (1.21 g, 3.64 mmol, yield: 81.2%) as faint yellow solid.
MS m/z (ESI): 333.0 [M+1]
¹H NMR (400 MHz, DMSO-*d₆*) δ 7.88 (s, 1H), 7.72 - 7.65 (m, 2H), 7.63 - 7.53 (m, 2H), 7.01 (d, J = 1.6 Hz, 1H), 4.06 (s, 3H), 3.92 (q, J = 7.2 Hz, 2H), 0.80 (t, J = 7.2 Hz, 3H).

### Step 2

### 3-{4-[2-(ethoxycarbonyl)-4-fluorophenyl]-1-methyl-1H-indazol-6-yl}-2,5-dihydro-1H-pyrrol-1-tert-butyl formate 101b

Compound **101a** (1.10 g, 3.31 mmol) and 3-(tetramethyl-1,3,2-dioxaboran-2-yl)-2,5-dihydro-1*H*-pyrrol-1-tert-butyl formate (1.46 g, 4.96 mmol) were dissolved in dioxane (18 mL) and water (2 mL). Sodium carbonate (1.05 g, 9.92 mmol) and Pd (dppf)Cl₂ (242 mg, 331 µmol) were added. Under a nitrogen atmosphere, the mixture was stirred at 100°Cto allow for reacting for 12 hours. The reaction mixture was cooled to room temperature, and filtered through diatomite, and the filtrate was concentrated under a reduced pressure. The resulting residues were purified by the silica-gel column chromatography using petroleum ether/ethyl acetate as an elution system, to obtain the title Compound **101b** (916 mg, 1.97 mmol, yield: 59.5%) as white solid.
MS m/z (ESI): 466.2 [M+1]
¹H NMR (400 MHz, CDCl₃) δ 7.71 - 7.51 (m, 5H), 7.23 (d, J = 11.6 Hz, 1H), 6.51 (d, J = 12.4 Hz, 1H), 4.54 (s, 2H), 4.25 (s, 2H), 4.10 (d, J = 2.0 Hz, 3H), 3.89 (dd, J = 14.8, 7.6 Hz, 2H), 1.47 (d, J = 9.6 Hz, 9H), 0.76 (t, J = 7.6 Hz, 3H).

### Step 3

### 3-{4-[2-(ethoxycarbonyl)-4-fluorophenyl]-1-methyl-1H-indazol-6-yl}pyrrolidin-1-tert-butyl formate 101c

Compound **101b** (910 mg, 1.95 mmol) was dissolved in ethanol (40 mL), followed by the addition of Pd/C (303 mg). Under a hydrogen atmosphere (15 Psi), the mixture was stirred at room temperature for 4.0 hours. The reaction mixture was filtered, and the filtrate was concentrated under a reduced pressure, to obtain the crude product of title Compound **101c** (909 mg). The crude product was directly used in the next step of reaction without purification.
MS m/z (ESI): 468.2 [M+1]
¹H NMR (400 MHz, DMSO-*d₆*) δ 7.71 - 7.47 (m, 5H), 6.92 (s, 1H), 4.34 (s, 0.29H), 4.10 - 4.00 (m, 3H), 3.94 - 3.84 (m, 2H), 3.76 (dd, J = 10.0, 8.0 Hz, 1H), 3.52 (t, J = 9.2 Hz, 2H), 3.44 (dd, J = 6.8, 4.4 Hz, 1H), 3.30 - 3.24 (m, 0.70H), 2.26 (d, J = 5.2 Hz, 1H), 2.09 (dd, J = 20.4, 10.4 Hz, 1H), 1.41 (d, J = 6.4 Hz, 9H), 0.76 (dd, J = 15.6, 7.2 Hz, 3H).

### Step 4

### 2-(6-{1-[(tert-butoxy)carbonyl]pyrrolidin-3-yl}-1-methyl-1H-indazol-4-yl)-5-fluorobenzoic acid 101d

Compound **101c** (900 mg, 1.93 mmol) was dissolved in methanol (14 mL) and water (6.0 mL), followed by the addition of LiOH.H₂O (230 mg, 9.63 mmol), and the mixture was stirred at 30°C to allow for reacting for 12 hours. The reaction mixture was concentrated under a reduced pressure. The resulting mixture was purified by the reverse phase preparative column chromatography (C18 column; mobile phase: acetonitrile, water, elution gradient: 40%-55%), to obtain the title Compound **101d** (822 mg, 1.87 mmol, yield: 97.1%) as white solid.
MS m/z (ESI): 440.1[M+1]
¹H NMR (400 MHz, DMSO-*d₆*) δ 12.99 (s, 1H), 7.56 (m, 5H), 6.97 (s, 1H), 4.05 (s, 3H), 3.81 - 3.73 (m, 1H), 3.52 (t, J = 9.2 Hz, 2H), 3.34 (s, 1H), 3.27 (s, 1H), 2.26 (s, 1H), 2.14 - 2.01 (m, 1H), 1.41 (d, J = 5.6 Hz, 9H).

### Step 5

### 3-{4-[2-(4,4-difluoro-2-methylpiperidin-1-carbonyl)-4-fluorophenyl]-1-methyl-1H-indazol-6-yl}pyrrolidin-1-tert-butyl formate 101e

Compound **101d** (200 mg, 455 µmol) was dissolved in DMF (4.0 mL), followed by the addition of DIPEA (235 mg, 1.82 mmol) and 4,4-difluoro-2-dimethylpiperidine(67.7 mg, 501 µmol, hydrochloride). The mixture was cooled to 0°C, followed by the addition of HATU (208 mg, 546 µmol). The reaction mixture was stirred at room temperature for 1.0 hour, and concentrated under a reduced pressure. The resulting residues were purified by the silica-gel column chromatography using petroleum ether/ethyl acetate as an elution system, to obtain the title Compound **101e** (160 mg, 287 µmol, yield: 63.1%) as white solid.
MS m/z (ESI): 557.2 [M+1]

### Step 6

### 4-[2-(4,4-difluoro-2-methylpiperidin-1-carbonyl)-4-fluorophenyl]-1-methyl-6-(pyrrolidin-3-yl)-1H-indazole 101f

Compound **101e** (160 mg, 287 µmol) was dissolved in methane dioxide (2.4 mL), followed by the addition of trifluoroacetic acid (0.8 mL) at 0°C. The mixture was stirred at room temperature to allow for reacting for 1.0 hours. The reaction mixture was concentrated under a reduced pressure, to obtain the crude product of title Compound **101f** (130 mg). The crude product was directly used in the next step of reaction without purification.
MS m/z (ESI): 457.2 [M+1]

### Step 7

### N-{4-[(3-{4-[2-(4,4-difluoro-2-methylpiperidin-1-carbonyl)-4-fluorophenyl]-1-methyl-1H-indazol-6-yl}pyrrolidin-1-yl)methyl]cyclohexyl}ethan-1-sulfonamide 101

The crude product of Compound **101f** (130 mg) was dissolved in *N,N*-dimethylformamide (2.0 mL), followed by the addition of potassium carbonate (118 mg, 854 µmol), potassium iodide (47.3 mg, 285 µmol) and Compound **2e** (148 mg, 313 µmol), and the mixture was stirred at 80°C to allow for reacting for 12 hours. The reaction mixture was filtered, and the filtrate was concentrated under a reduced pressure. The resulting residues were purified by the silica-gel column chromatography using dichloromethane/methanol as an elution system, and the obtained crude product was purified by the HPLC (mobile phase: acetonitrile, water (0.1% trifluoroacetic acid), elution gradient: 10%-40%), to obtain the title Compound **101** (68.3 mg, 104 µmol, yield: 36.3%) as white solid.
MS m/z (ESI): 660.3 [M+1]
¹H NMR (400 MHz, DMSO-*d*₆) δ 7.84 (s, 1H), 7.62 (s, 2H), 7.42 (t, *J* = 8.4 Hz, 2H), 7.10 (s, 1H), 6.78 (d, *J* = 7.6 Hz, 1H), 4.50 (m, 1H), 4.07 (s, 4H), 3.76 (m, 3H), 3.43 - 3.35 (m, 1H), 3.32 - 3.02 (m, 5H), 2.98 (q, *J* = 7.2 Hz, 2H), 2.48 - 2.43 (m, 1H), 2.29 - 1.61 (m, 9H), 1.39 - 0.96 (m, 9H), 0.45 (s, 2H).

### Example 107

### N-ethyl-5-fluoro-2-(1-fluoro-3-methyl-6-{1-[(1R,3S,4S)-2-azabicyclo[2.2.2]octan-3-carbonyl]azacyclobutan-3-yl}imidazo[1,5-a]pyridin-8-yl)-N-(isopropyl)benzamide 107

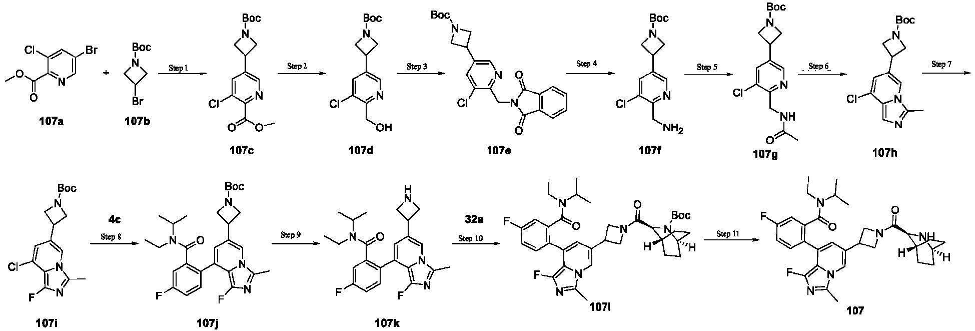

### Step 1

### 5-{1-[(tert-butoxy)carbonyl]azacyclobutan-3-yl}-3-chloropyridin-2-methyl carboxylate 107c

5-Bromo-3-chloropyridin-2-methyl carboxylate **107a** (10 g, 39.92 mmol), 3-bromoazacyclobutan-1-tert-butyl carboxylate **107b** (12.25 g, 51.90 mmol), Ir[dF(CF₃)ppy]₂(dtbpy) (PF₆) (448 mg, 399.24 µmol), NiCl₂.dtbbpy (238 mg, 598.86 µmol), tris(trimethylsilyl)silane (9.95 g, 40.00 mmol) and sodium carbonate (8.46 g, 79.85 mmol) were dissolved in dimethoxyethane (480 mL), and the reaction mixture was placed under a 10W LED bluelight lamp, and stirred to allow for reacting for 3.0 hours. The reaction mixture was concentrated under a reduced pressure, and the residues were dissolved in ethyl acetate (100 mL), and washed with water (400 mL x 3). The organic phase was dried over anhydrous sodium sulfate, and concentrated under a reduced pressure. The resulting residues were purified by the silica-gel column chromatography using petroleum ether/ethyl acetate as an elution system, to obtain the title Compound **107c** (8.7 g, 26.62 mmol, yield: 66.69%) as yellow oil.
MS m/z (ESI): 327.0 [M+1]
¹H NMR (400 MHz, CDCl₃) δ 8.47 (d, *J* = 1.8 Hz, 1H), 7.82 (br d, *J* = 1.5 Hz, 1H), 4.40 (t, *J* = 8.8 Hz, 2H), 4.01 (s, 3H), 3.95 (dd, *J* = 5.6, 8.6 Hz, 2H), 3.83 - 3.74 (m, 1H), 1.50 - 1.44 (m, 9H).

### Step 2

### 3-[5-chloro-6-(hydroxylmethyl)pyridin-3-yl]azacyclobutan-1-tert-butyl carboxylate 107d

Compound **107c** (7.7 g, 23.56 mmol) was dissolved in methanol (77 mL) and tetrahydrofuran (77 mL), and cooled to 0°C, followed by the addition of sodium borohydride (2.77 g, 73.28 mmol). The mixture was stirred at room temperature to allow for reacting for 3.0 hour. The reaction mixture was cooled to 0°C, a saturated aqueous ammonium chloride solution (500 mL) was added to quench the reaction, methanol and tetrahydrofuran were removed under a reduced pressure, and the aqueous phase was extracted with ethyl acetate (500 mL). The organic phase was dried over anhydrous sodium sulfate, and concentrated under a reduced pressure, to obtain the crude product of title Compound **107d** (6 g, 20.08 mmol, yield: 85.23%) as yellow oil. The crude product was directly used in the next step of reaction without purification.
MS m/z (ESI): 299.0 [M+1]

### Step 3

### 3-{5-chloro-6-[(1,3-dioxo-2,3-dihydro-1H-isoindol-2-yl)methyl]pyridin-3-yl}azacyclobutan-1-tert-butyl carboxylate 107e

Compound **107d** (5.5 g, 18.41 mmol), 2,3-dihydro-1*H*-isoindol-1,3-dione (2.99 g, 20.34 mmol) and triphenylphosphine (7.25 g, 27.63 mmol) were dissolved in tetrahydrofuran (70 mL), and cooled to 0°C under a nitrogen atmosphere, followed by the addition of DIAD (5.58 g, 27.58 mmol). The mixture was stirred at room temperature to allow for reacting for 12 hour. The reaction mixture was diluted with ethyl acetate (500 mL), and washed with water (500 mL x 3). The organic phase was dried over anhydrous sodium sulfate, and concentrated under a reduced pressure. The resulting residues were purified by the silica-gel column chromatography using petroleum ether/ethyl acetate as an elution system, to obtain the title Compound **107e** (7 g, 16.36 mmol, yield: 88.87%) as colorless oil.

¹H NMR (400 MHz, DMSO-*d*₆) δ 8.33 - 8.29 (m, 1H), 8.04 (d, *J* = 1.8 Hz, 1H), 7.97 - 7.87 (m, 4H), 5.02 - 4.96 (m, 2H), 4.24 - 4.15 (m, 2H), 3.94 - 3.79 (m, 3H), 1.45 - 1.34 (m, 9H).

### Step 4

### 3-[6-(aminomethyl)-5-chloropyridin-3-yl]azacyclobutan-1-tert-butyl carboxylate 107f

Compound **107e** (7 g, 16.36 mmol) was dissolved in the solution of methylamine in ethanol (29.93 g, 289.09 mmol, 33.29 mL, 30%). The mixture was stirred at 65°C to allow for reacting for 12 hours. The reaction mixture was cooled to room temperature, and concentrated under a reduced pressure, to obtain the crude product of title Compound **107f** (4.87 g, 16.35 mmol, yield: 100%) as light yellow oil. The crude product was directly used in the next step of reaction without purification.
MS m/z (ESI): 298.0 [M+1]

### Step 5

### 3-[5-chloro-6-(acetamidomethyl)pyridin-3-yl]azacyclobutan-1-tert-butyl carboxylate 107g

Compound **107f** (4.87 g, 16.35 mmol) was dissolved in dichloromethane (70 mL), followed by the addition of triethylamine (4.96 g, 49.06 mmol). The reaction mixture was cooled to 0°C, followed by dropwise adding acetyl chloride (1.54 g, 19.63 mmol) slowly and stirring at room temperature to allow for reacting for 1.0 hour. The reaction mixture was diluted with dichloromethane (300 mL), and washed with water (300 mL x 3). The organic phase was dried over anhydrous sodium sulfate, and concentrated under a reduced pressure, to obtain the crude product of title Compound **107g** (6 g, 17.66 mmol, yield: 107.96%). The crude product was directly used in the next step of reaction without purification.
MS m/z (ESI): 340.0 [M+1]

### Step 6

### 3-{8-chloro-3-methylimidazo[1,5-a]pyridin-6-yl}azacyclobutan-1-tert-butyl carboxylate 107h

Compound **107g** (3 g, 8.83 mmol) was dissolved in dichloromethane (40 mL), followed by the addition of a Burgess reagent (2.31 g, 9.71 mmol). The mixture was stirred at 40°C to allow for reacting for 12.0 hours. The reaction mixture was cooled to room temperature, followed by adding dichloromethane (100 mL) and washing with water (100 mL x 3). The organic phase was dried over anhydrous sodium sulfate, and concentrated under a reduced pressure. The resulting residues were purified by the silica-gel column chromatography using dichloromethane/methanol as an elution system, to obtain the title Compound **107h** (2 g, 6.22 mmol, yield: 70.4%).

¹H NMR (400 MHz, CDCl₃) δ 7.48 (d, J = 3.1 Hz, 2H), 6.88 - 6.82 (m, 1H), 4.35 (t, J = 8.8 Hz, 2H), 4.03 - 3.91 (m, 2H), 3.73 - 3.60 (m, 1H), 2.67 (s, 3H), 1.50 (s, 9H).

### Step 7

### 3-{8-chloro-1-fluoro-3-methylimidazo[1,5-a]pyridin-6-yl}azacyclobutan-1-tert-butyl carboxylate 107i

Compound **107h** (200 mg, 621.50 µmol) was dissolved in tetrahydrofuran (3.0 mL), followed by the addition of pyridine (73.74 mg, 932.25 µmol, 75.40 µL). The mixture was cooled to -40°C, a Selectfluor fluridizer (279.62 mg, 789.31 µmol) was added, and the mixture was stirred at -40°C to allow for reacting for 0.5 hours. A saturated aqueous sodium thiosulfate solution (30 mL) was added to the reaction mixture, which was then extracted with ethyl acetate (30 mL x 3). The organic phases were combined, dried over anhydrous sodium sulfate, and concentrated under a reduced pressure. The resulting residues were purified by the silica-gel column chromatography using dichloromethane/ethyl acetate as an elution system, to obtain the title Compound **107i** (60 mg, 176.58 µmol, yield: 28.4%).
MS m/z (ESI): 340.1 [M+1]

### Step 8

### 3-(8-{2-[ethyl(isopropyl)aminoformyl]-4-fluorophenyl}-1-fluoro-3-methylimidazo[1,5-a]pyridin-6-yl)azacyclobutan-1-tert-butyl formate 107j

Compound **107i** (120 mg, 353.16 µmol), Compound **4c**(295.96 mg, 529.74 µmol) and potassium phosphate (224.89 mg, 1.06 mmol) were dissolved in dioxane (2.0 mL) and water (0.4 mL). Pd(dtbpf)Cl₂ (23.02 mg, 35.32 µmol) was added. Under a nitrogen atmosphere, the mixture was stirred at 90°Cto allow for reacting for 12 hours. The reaction mixture was cooled to room temperature, followed by adding water (30 mL) and extracting with ethyl acetate (30 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **107j** (120 mg, 234.11 µmol, yield: 66.3%).
MS m/z (ESI): 513.3 [M+1]
¹H NMR (400 MHz, DMSO-*d*₆) δ 7.94 (s, 1H), 7.53 (dt, J = 2.7, 5.6 Hz, 1H), 7.40 - 7.27 (m, 2H), 6.53 (s, 1H), 4.18 (br s, 2H), 3.74 (br s, 3H), 3.55 - 3.42 (m, 1H), 3.32 (br dd, J = 6.5, 13.1 Hz, 1H), 3.03 - 2.88 (m, 1H), 2.54 (s, 3H), 1.39 (s, 9H), 1.00 - 0.74 (m, 7H), 0.43 (br s, 2H).

### Step 9

### 2-[6-(azacyclobutan-3-yl)-1-fluoro-3-methylimidazo[1,5-a]pyridin-8-yl]-N-ethyl-5-fluoro-N-(isopropyl)benzamide 107k

Compound **107j** (120 mg, 234.11 µmol) was dissolved in dichloromethane (3.0 mL), followed by the addition of trifluoroacetic acid (1.48 g, 12.98 mmol, 1 mL) at 0°C. The mixture was stirred at room temperature to allow for reacting for 1.0 hours. The reaction mixture was concentrated under a reduced pressure, to obtain the crude product of title Compound **107k** (100 mg). The crude product was directly used in the next step of reaction without purification.
MS m/z (ESI): 413.2 [M+1]

### Step 10

### (1R,3S,4S)-3-[3-(8-{2-[ethyl(isopropyl)aminoformyl]-4-fluorophenyl}-1-fluoro-3-methylimidazo[1,5-a]pyridin-6-yl)azacyclobutan-1-carbonyl]-2-azabicyclo[2.2.2]octan-2-tert-butyl carboxylate 1071

(1R,35,45)-2-[(tert-butoxy)carbonyl]-2-azabicyclo[2.2.2]octan-3-carboxylic acid **32a**(61 mg, 238.93 µmol) and *N,N*-diisopropylethylamine (95 mg, 735.07 µmol) were dissolved in DCM (3.0 mL). The mixture was cooled to 0°C, followed by the addition of EDCI (58 mg, 302.56 µmol), HOBT (42 mg, 310.83 µmol) and Compound **107k** (123 mg, 233.62 µmol, trifluoroacetate ), and the mixture was stirred at room temperature for 12 hours. The reaction mixture was concentrated under a reduced pressure. The resulting residues were purified by the silica-gel column chromatography using petroleum ether/ethyl acetate as an elution system, to obtain the title Compound **107l** (70 mg, 107.73 µmol, yield: 46.11%).
MS m/z (ESI): 650.4 [M+1]

### Step 11

### N-ethyl-5-fluoro-2-(1-fluoro-3-methyl-6-{1-[(1R,3S,4S)-2-azabicyclo[2.2.2]octan-3-carbonyl]azacyclobutan-3-yl}imidazo[1,5-a]pyridin-8-yl)-N-(isopropyl)benzamide 107

Compound **107l** (70 mg, 107.73 µmol) was dissolved in methane dioxide (3.0 mL) and trifluoroacetic acid (1.0 mL). The mixture was stirred at room temperature to allow for reacting for 1.0 hours. The reaction mixture was concentrated under a reduced pressure, regulated to PH = 8-9 with ammonia water, and then purified by the HPLC (mobile phase: acetonitrile, water (0.1% ammonium bicarbonate), elution gradient: 24%-54%), to obtain the title Compound **107** (29.9 mg, 54.40 µmol, yield: 50.49%) as yellow solid.
MS m/z (ESI): 550.3[M+1]
¹H NMR (400 MHz, DMSO-*d*₆) δ 88.03 - 7.86 (m, 1H), 7.58 - 7.25 (m, 3H), 6.64 - 6.42 (m, 1H), 4.67 - 3.77 (m, 6H), 3.66 - 3.53 (m, 1H), 3.51 - 3.41 (m, 1H), 3.02 - 2.72 (m, 2H), 2.55 (s, 3H), 1.83 - 1.33 (m, 9H), 1.08 - 0.63 (m, 7H), 0.50 - 0.31 (m, 2H).

### Example 108

### 2-(1-chloro-3-methyl-6-{1-[(1R,3S,4S)-2-azabicyclo[2.2.2]octan-3-carbonyl]azacyclobutan-3-yl}imidazo[1,5-a]pyridin-8-yl)-N-ethyl-5-fluoro-N-(isopropyl)benzamide 108

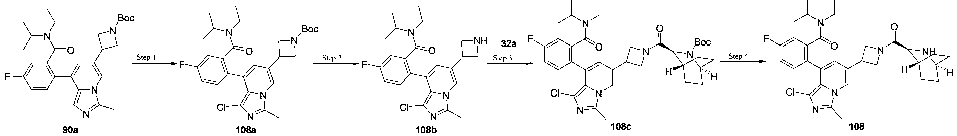

### Step 1

### 3-(1-chloro-8-{2-[ethyl(isopropyl)aminoformyl]-4-fluorophenyl}-3-methylimidazo[1,5-a]pyridin-6-yl)azacyclobutan-1-tert-butyl carboxylate 108a

Compound **90a** (680 mg, 1.37 mmol), acetic acid (180 mg, 3.00 mmol, 171.59 µL) and NCS (238.66 mg, 1.79 mmol) were dissolved in trichloromethane (10 mL). The mixture was stirred at room temperature to allow for reacting for 12 hours. A saturated aqueous sodium bicarbonate solution (20 mL) was added to the reaction mixture, which was then extracted with dichloromethane (30 mL x 3). The organic phase was dried over anhydrous sodium sulfate, and concentrated under a reduced pressure. The resulting residues were purified by the silica-gel column chromatography using petroleum ether/ethyl acetate as an elution system, to obtain the title Compound **108a** (450 mg, 850.59 µmol, yield: 61.87%).
MS m/z (ESI): 529.3 [M+1]
¹H NMR (400 MHz, DMSO-*d₆*) δ 8.07 (br s, 1H), 7.51 - 7.40 (m, 1H), 7.39 - 7.27 (m, 2H), 6.69 (s, 1H), 4.20 (br d, J = 5.0 Hz, 2H), 3.99 - 3.74 (m, 3H), 3.65 - 3.50 (m, 1H), 3.41 - 3.33 (m, 1H), 2.87 (br dd, J = 6.6, 13.2 Hz, 1H), 2.60 (s, 3H), 1.40 (s, 10H), 1.06 - 0.70 (m, 7H), 0.27 (br d, J = 6.3 Hz, 2H).

### Step 2

### 2-[6-(azacyclobutan-3-yl)-1-chloro-3-methylimidazo[1,5-a]pyridin-8-yl]-N-ethyl-5-fluoro-N-(isopropyl)benzamide 108b

Compound **108a** (110 mg, 207.92 µmol) was dissolved in methane dioxide (3.0 mL), followed by the addition of trifluoroacetic acid (1.48 g, 12.98 mmol, 1 mL) at 0°C. The mixture was stirred at room temperature to allow for reacting for 0.5 hours. The reaction mixture was concentrated under a reduced pressure, to obtain the crude product of title Compound **108b** (110 mg). The crude product was directly used in the next step of reaction without purification.
MS m/z (ESI): 429.2 [M+1]

### Step 3

### (1R,3S,4S)-3-[3-(1-chloro-8-{2-[ethyl(isopropyl)aminoformyl]-4-fluorophenyl}-3-methylimidazo[1,5-a]pyridin-6-yl)azacyclobutan-1-carbonyl]-2-azabicyclo[2.2.2]octan-2-tert-butyl carboxylate 108c

(1R,3S,4S)-2-[(tert-butoxy)carbonyl]-2-azabicyclo[2.2.2]octan-3-carboxylic acid **32a**(53.94 mg, 211.28 µmol) and *N,N*-diisopropylethylamine (84.61 mg, 654.70 µmol,114.03 µL) were dissolved in DCM (2.0 mL). The mixture was cooled to 0°C, followed by the addition of EDCI (60.29 mg, 314.49 µmol), HOBT (42.31 mg, 313.11 µmol) and the crude product of Compound **108b** (110 mg), and the mixture was then stirred at room temperature for 12 hours. The reaction mixture was concentrated under a reduced pressure. The resulting residues were purified by the silica-gel column chromatography using petroleum ether/ethyl acetate as an elution system, to obtain the title Compound **108c** (92.1 mg, 138.24 µmol, yield: 66.5 %).
MS m/z (ESI): 666.4 [M+1]

### Step 4

### 2-(1-chloro-3-methyl-6-{1-[(1R,3S,4S)-2-azabicyclo[2.2.2]octan-3-carbonyl]azacyclobutan-3-yl}imidazo[1,5-a]pyridin-8-yl)-N-ethyl-5-fluoro-N-(isopropyl)benzamide 108

Compound **108c** (92.1 mg, 138.24 µmol) was dissolved in methane dioxide (3.0 mL) and trifluoroacetic acid (1.0 mL). The mixture was stirred at room temperature to allow for reacting for 1.0 hours. The reaction mixture was concentrated under a reduced pressure, and purified by the HPLC (mobile phase: acetonitrile, water (0.1% ammonia water), elution gradient: 37%-67%), to obtain the title Compound **108** (35.11 mg, 62.02 µmol, yield: 44.86 %) as yellow solid.
MS m/z (ESI): 566.3 [M+1]
¹H NMR (400 MHz, DMSO-*d*₆) δ 8.14 - 8.03 (m, 1H), 7.50 - 7.26 (m, 3H), 6.76 - 6.58 (m, 1H), 4.67 - 3.79 (m, 6H), 3.65 - 3.51 (m, 2H), 2.94 - 2.71 (m, 2H), 2.59 (s, 3H), 1.82 - 1.37 (m, 9H), 1.06 - 0.66 (m, 7H), 0.32 - 0.20 (m, 2H).

### Example 110

### N-ethyl-5-fluoro-N-(isopropyl)-2-(6-{1-[(1R,3S,4S)-2-azabicyclo[2.2.2]octan-3-carbonyl]-1,2,3,6-tetrahydropyridin-4-yl}pyrrolo[1,2-a]pyrazin-8-yl)benzamide 110

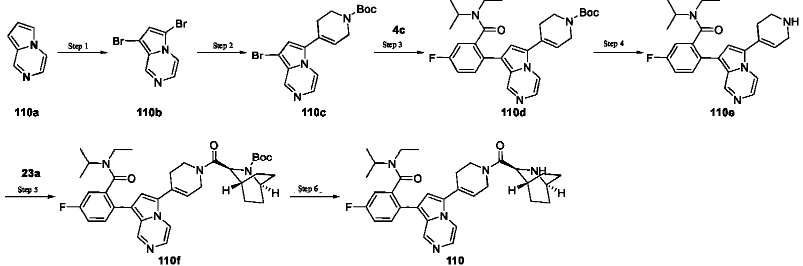

### Step 1

### 6,8-dibromopyrrolo[1,2-a]pyrazine 110b

Pyrrolo[1,2-a]pyrazine **110a** (2 g, 16.93 mmol) was dissolved in DCM (80 mL), followed by the addition of NBS (6.06 g, 34.03 mmol). The mixture was stirred at room temperature for 1.0 hour. A saturated aqueous sodium bicarbonate solution (70 mL) was added to the reaction mixture, which was then extracted with dichloromethane (50 mL x 3). The organic phases were combined, dried over anhydrous sodium sulfate, and concentrated under a reduced pressure. The crude product of title Compound **110b** (4.58 g, 16.60 mmol, yield: 98.04%) was obtained. The crude product was directly used in the next step of reaction without purification.
MS m/z (ESI): 276.9[M+1]
¹H NMR (400 MHz, DMSO-*d₆*) δ 8.73 (s, 1H), 8.12 (dd, J = 1.1, 4.9 Hz, 1H), 7.72 (d, J = 5.0 Hz, 1H), 7.31 (s, 1H).

### Step 2

### 4-{8-bromopyrrolo[1,2-a]pyrazin-6-yl}-1,2,3,6-tetrahydropyridin-1-tert-butyl carboxylate 110c

Compound **110b** (1 g, 3.62 mmol), 4-(tetramethyl-1,3,2-dioxaboran-2-yl)- 1,2,3,6-tetrahydropyridin-1-tert-butyl carboxylate (1.01 g, 3.26 mmol) and sodium carbonate (1.15 g, 10.87 mmol) were dissolved in dioxane (10 mL) and water (2 mL). Pd (dppf)Cl₂ (265.18 mg, 362.41 µmol) was added. Under a nitrogen atmosphere, the mixture was stirred at 90°Cto allow for reacting for 3.0 hours. The reaction mixture was cooled to room temperature, followed by adding water (30 mL) and extracting with ethyl acetate (30 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **110c** (497 mg, 1.31 mmol, yield: 36.25%).
MS m/z (ESI): 380.2 [M+1]

### Step 3

### 4-(8-{2-[ethyl(isopropyl)aminoformyl]-4-fluorophenyl}pyrrolo[1,2-a]pyrazin-6-yl)-1,2,3,6-tetrahydropyridin-1-tert-butyl carboxylate 110d

Compound **110c** (497 mg, 1.31 mmol) and Compound **4c**(1 g, 1.49 mmol) were dissolved in dioxane (8 mL) and water (2.0 mL). Sodium carbonate (417.78 mg, 3.94 mmol) and Pd(dppf)Cl₂ (96.14 mg, 131.39 µmol) were added, and the mixture was stirred at 90 °C under a nitrogen atmosphere to allow for reacting for 12 hours. The reaction mixture was cooled to room temperature, followed by adding water (30 mL) and extracting with ethyl acetate (30 mL x 3). The organic phases were combined, dried over anhydrous sodium sulfate, and concentrated under a reduced pressure. The resulting residues were purified by the silica-gel column chromatography using dichloromethane/ethyl acetate as an elution system, to obtain the title Compound **110d** (540 mg, 1.07 mmol, yield: 81.13%).
MS m/z (ESI): 507.3 [M+1]
¹H NMR (400 MHz, DMSO-*d₆*) δ 8.77 (s, 1H), 8.36 (br d, J = 4.4 Hz, 1H), 7.25 (m, 4H), 6.95 - 6.85 (m, 1H), 6.23 - 6.04 (m, 1H), 4.30 - 3.96 (m, 3H), 3.57 (br d, J = 5.6 Hz, 2H), 3.41 - 3.34 (m, 1H), 3.29 (br s, 2H), 2.97 - 2.74 (m, 1H), 1.44 (s, 9H), 1.08 - 0.66 (m, 7H), 0.23 (br d, J = 6.4 Hz, 2H).

### Step 4

### N-ethyl-5-fluoro-N-(isopropyl)-2-[6-(1,2,3,6-tetrahydropyridin-4-yl)pyrrolo[1,2-a]pyrazin-8-yl]benzamide 110e

Compound **110d** (200 mg, 335.56 µmol) was dissolved in dichloromethane (3 mL), followed by the addition of trifluoroacetic acid (1.48 g, 12.98 mmol, 1 mL) at 0°C. The mixture was stirred at room temperature to allow for reacting for 1.0 hours. The reaction mixture was concentrated under a reduced pressure, to obtain the crude product of title Compound **110e** (150 mg, 369.01 µmol, yield: 110%). The crude product was directly used in the next step of reaction without purification.
MS m/z (ESI): 407.1 [M+1]

### Step 5

### (1R,3S,4S)-3-[4-(8-{2-[ethyl(isopropyl)aminoformyl]-4-fluorophenyl}pyrrolo[1,2-a]pyrazin-6-y1)-1,2,3,6-tetrahydropyridin-1-carbonyl]-2-azabicyclo[2.2.2]octan-2-tert-butyl carboxylate 110f

Compound **32a** (67.00 mg, 262.42 µmol) and *N,N*-diisopropylethylamine (161.39 mg, 1.25 mmol, 217.51 µL) were dissolved in dichloromethane (3 mL), and cooled to 0°C. EDCI (71.82 mg, 374.63 µmol), HOBT (50.62 mg, 374.63 µmol) and Compound **110e** (130 mg, 249.75 µmol, trifluoroacetic acid) were added, and the mixture was stirred at room temperature for 12 hours. The reaction mixture was concentrated under a reduced pressure. The resulting residues were purified by the silica-gel column chromatography using dichloromethane/methanol as an elution system, to obtain the title Compound **110f** (100 mg, 155.33 µmol, yield: 62.19%).
MS m/z (ESI): 644.4 [M+1]

### Step 6

### N-ethyl-5-fluoro-N-(isopropyl)-2-(6-{1-[(1R,3S,4S)-2-azabicyclo[2.2.2]octan-3-carbonyl]-1,2,3,6-tetrahydropyridin-4-yl}pyrrolo[1,2-a]pyrazin-8-yl)benzamide 110

Compound **110f** (100 mg, 155.33 µmol) was dissolved in methane dioxide (3.0 mL) and trifluoroacetic acid (1.48 g, 12.98 mmol, 1 mL). The mixture was stirred at room temperature to allow for reacting for 1.0 hours. The reaction mixture was concentrated under a reduced pressure, and the resulting residues were purified by the HPLC (mobile phase: acetonitrile, water (0.1% ammonium bicarbonate), elution gradient: 22%-52%), and the obtained isomer mixture was purified by the preparative SFC (separation column: DAICEL CHIRALPAK IA (250 mm * 30 mm, 10 um); mobile phase: 25-25% (v/v) Heptane-EtOH:CAN=4:1 (0.1%IPA)), to obtain the title Compound **110** (29.69 mg, 54.61 µmol, yield: 37.11%).
MS m/z (ESI): 544.3 [M+1]
¹H NMR (400 MHz, DMSO-*d₆*) δ 8.77 (s, 1H), 8.37 (br s, 1H), 7.61 (m, 1H), 7.57 - 7.51 (m, 1H), 7.39 - 7.31 (m, 1H), 7.29 - 7.23 (m, 1H), 6.94 - 6.89 (m, 1H), 6.26 - 6.11 (m, 1H), 4.39 - 4.07 (m, 2H), 4.02 - 3.86 (m, 1H), 3.73 - 3.58 (m, 1H), 3.40 - 3.31 (m, 2H), 2.95 - 2.71 (m, 2H), 2.58 - 2.52 (m, 1H), 2.46 (m, 1H), 1.89 - 1.23 (m, 10H), 1.09 - 0.68 (m, 7H), 0.23 (m, 2H).

### Example 113

### N-ethyl-5-fluoro-N-(isopropyl)-2-(5-{1-[(1R,3S,4S)-2-azabicyclo[2.2.2]octan-3-carbonyl]-1,2,3,6-tetrahydropyridin-4-yl}pyrrolo[1,2-c]pyrimidin-7-yl)benzamide 113

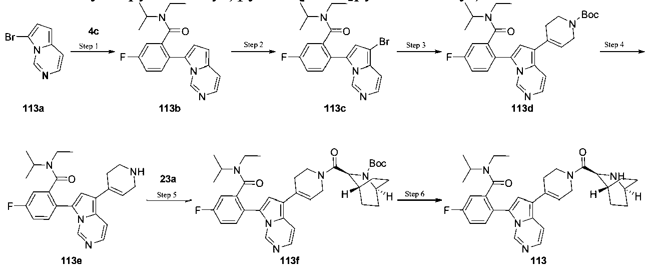

### Step 1

### N-ethyl-5-fluoro-N-(isopropyl)-2-{pyrrolo[1,2-c]pyrimidin-7-yl}benzamide 113b

7-Bromopyrrolo[1,2-c]pyrimidine **113a** (500 mg, 2.54 mmol) and Compound **4c** (2.55 g, 3.81 mmol) were dissolved in dioxane (3 mL) and water (1.0 mL). Cesium carbonate (2.48 g, 7.61 mmol) and Pd(dppf)Cl₂ (165.39 mg, 253.77 µmol) were added, and the mixture was stirred at 100 °C under a nitrogen atmosphere to allow for reacting for 12 hours. The reaction mixture was cooled to room temperature, and concentrated under a reduced pressure. The resulting residues were purified by the silica-gel column chromatography using petroleum ether/ethyl acetate as an elution system, to obtain the title Compound **113b** (900 mg, 2.77 mmol, yield: 109%).
MS m/z (ESI): 326.0 [M+1]

### Step 2

### 2-{5-bromopyrrolo[1,2-c]pyrimidin-7-yl}-N-ethyl-5-fluoro-N-(isopropyl)benzamide 113c

Compound **113b** (800 mg, 2.46 mmol) was dissolved in DCM (24 mL), followed by the addition of NBS(437.60 mg, 2.46 mmol). The mixture was stirred at room temperature for 12 hours. Dichloromethane (100 mL) was added to the reaction mixture, which was then washed with water (100 mL x 3). The organic phase was dried over anhydrous sodium sulfate, and concentrated under a reduced pressure. The resulting residues were purified by the silica-gel column chromatography using petroleum ether/ethyl acetate as an elution system, to obtain the title Compound **113c** (620 mg, 1.53 mmol, yield: 62.4%).
MS m/z (ESI): 404.0[M+1]

### Step 3

### 4-(7-{2-[ethyl(isopropyl)aminoformyl]-4-fluorophenyl}pyrrolo[1,2-c]pyrimidin-8-yl)-1,2,3,6-tetrahydropyridin-1-tert-butyl carboxylate 113d

Compound **113c** (300 mg, 742.07 µmol), 4-(tetramethyl-1,3,2-dioxaboran-2-yl)- 1,2,3,6-tetrahydropyridin-1-tert-butyl carboxylate (344.18 mg, 1.11 mmol) and cesium carbonate (725.34 mg, 2.23 mmol) were dissolved in dioxane (3 mL) and water (1 mL). Pd (dppf)Cl₂ (54.30 mg, 74.21 µmol) was added. Under a nitrogen atmosphere, the mixture was stirred at 100°Cto allow for reacting for 12.0 hours. The reaction mixture was cooled to room temperature, and concentrated under a reduced pressure. The resulting residues were purified by the silica-gel column chromatography using petroleum ether/ethyl acetate as an elution system, to obtain the title Compound **113d** (290 mg, 572.43 µmol, yield: 77.14%).
MS m/z (ESI): 507.2 [M+1]

### Step 4

### N-ethyl-5-fluoro-N-(isopropyl)-2-[5-(1,2,3,6-tetrahydropyridin-4-yl)pyrrolo[1,2-c]pyrimidin-7-yl]benzamide 113e

Compound **113d** (290 mg, 572.43 µmol) was dissolved in dichloromethane (3 mL), followed by the addition of trifluoroacetic acid (1.48 g, 12.98 mmol, 1 mL) at 0°C. The mixture was stirred at room temperature to allow for reacting for 0.5 hours. The reaction mixture was concentrated under a reduced pressure, to obtain the crude product of title Compound **113e** (290 mg). The crude product was directly used in the next step of reaction without purification. MS m/z (ESI): 407.1 [M+1]

### Step 5

### (1R,3S,4S)-3-[4-(7-{2-[ethyl(isopropyl)aminoformyl]-4-fluorophenyl}pyrrolo[1,2-c]pyrimidin-5-yl)-1,2,3,6-tetrahydropyridin-1-carbonyl]-2-azabicyclo[2.2.2]octan-2-tert-butyl carboxylate 113f

Compound **32a** (142.24 mg, 557.14 µmol) and *N,N*-diisopropylethylamine (216.02 mg, 1.67 mmol, 291.13 µL) were dissolved in dichloromethane (3 mL), and cooled to 0°C. EDCI (160.21 mg, 835.71 µmol) and HOBT (112.92 mg, 835.71 µmol) were added, and the mixture was stirred at room temperature for 0.5 hours. The crude product of Compound **113e** (290 mg) was added, and the mixture was stirred at room temperature for 12 hours. Dichloromethane (50 mL) was added to the reaction mixture, which was then washed with water (50 mL x 3). The organic phase was dried over anhydrous sodium sulfate, and concentrated under a reduced pressure. The resulting residues were purified by the silica-gel column chromatography using petroleum ether/ethyl acetate as an elution system, to obtain the title Compound **113f** (150 mg, 233.00 µmol, yield: 41.82 %).
MS m/z (ESI): 644.3 [M+1]

### Step 6

### N-ethyl-5-fluoro-N-(isopropyl)-2-(5-{1-[(1R,3S,4S)-2-azabicyclo[2.2.2]octan-3-carbonyl]-1,2,3,6-tetrahydropyridin-4-yl}pyrrolo[1,2-c]pyrimidin-7-yl)benzamide 113

Compound **113f** (150 mg, 233.00 µmol) was dissolved in methane dioxide (3.0 mL) and trifluoroacetic acid (1.48 g, 12.98 mmol, 1 mL). The mixture was stirred at room temperature to allow for reacting for 0.5 hours. The reaction mixture was concentrated under a reduced pressure, and the resulting residues were purified by the HPLC (mobile phase: acetonitrile, water (0.1% ammonia water), elution gradient: 42%-72%), to obtain the title Compound **113** (50 mg, 91.97 µmol, yield: 39.47%).
MS m/z (ESI): 544.4 [M+1]
¹H NMR (400 MHz, DMSO-*d₆*) δ 8.79 - 8.67 (m, 1H), 7.76 - 7.65 (m, 1H), 7.61 (br d, J = 6.4 Hz, 1H), 7.45 - 7.31 (m, 3H), 6.94 - 6.85 (m, 1H), 6.06 - 5.91 (m, 1H), 4.29 - 4.01 (m, 2H), 3.97 - 3.83 (m, 1H), 3.72 - 3.51 (m, 2H), 3.43 (td, J = 6.5, 13.1 Hz, 1H), 3.31 (br dd, J = 6.6, 13.1 Hz, 2H), 2.89 - 2.74 (m, 2H), 2.55 (br s, 1H), 1.87 - 1.23 (m, 10H), 1.00 - 0.57 (m, 7H), 0.32 (br d, J = 4.5 Hz, 2H).

### Example 115

### 2-(5-chloro-3-methyl-6-{4-[(2S)-pyrrolidin-2-carbonyl]piperazin-1-yl}imidazo[1,5-a]pyridin-8-yl)-N-ethyl-5-fluoro-N-(isopropyl)benzamide 115

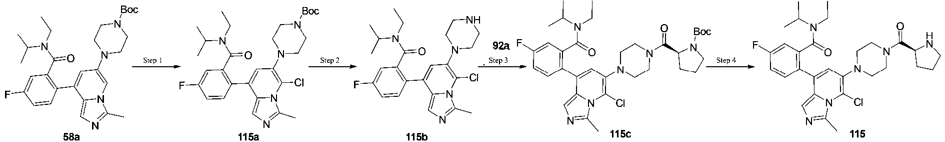

### Step 1

### 4-(5-chloro-8-{2-[ethyl(isopropyl)aminoformyl]-4-fluorophenyl}-3-methylimidazo[1,5-a]pyridin-6-yl)piperazin-1-tert-butyl carboxylate 115a

Compound **58a** (1 g, 1.72 mmol) and NCS(222.62 mg, 1.67 mmol) was dissolved in dichloromethane (10 mL). Under a nitrogen atmosphere, the mixture was stirred at 20°Cto allow for reacting for 1.0 hours. A saturated aqueous sodium bicarbonate solution (10 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/ethyl acetate as an elution system, to obtain the title Compound **115a** (710 mg, 1.27 mmol, yield: 74.02%).
MS m/z (ESI): 558.1 [M+1]
¹H NMR (400 MHz, DMSO-*d₆*) δ 7.68 - 7.59 (m, 1H), 7.43 - 7.30 (m, 2H), 7.09 (s, 1H), 6.73 - 6.62 (m, 1H), 3.54 - 3.38 (m, 5H), 3.30 - 3.21 (m, 1H), 2.99 - 2.94 (m, 3H), 2.88 - 2.79 (m, 4H), 2.58 - 2.54 (m, 1H), 1.46 - 1.37 (m, 9H), 1.20 - 1.04 (m, 1H), 0.98 - 0.88 (m, 3H), 0.84 - 0.74 (m, 3H), 0.39 - 0.32 (m, 2H).

### Step 2

### 2-[5-chloro-3-methyl-6-(piperazin-1-yl)imidazo[1,5-a]pyridin-8-yl]-N-ethyl-5-fluoro-N-(isopropyl)benzamide 115b

Compound **115a** (150 mg, 268.78 µmol) was dissolved in methane dioxide (2.0 mL), followed by the addition of trifluoroacetic acid (740.00 mg, 6.49 mmol, 0.5 mL) at 0°C. The mixture was stirred at room temperature to allow for reacting for 0.5 hours. The reaction mixture was concentrated under a reduced pressure, to obtain the crude product of title Compound **115b** (123 mg). The crude product was directly used in the next step of reaction without purification.
MS m/z (ESI): 458.3 [M+1]

### Step 3

### (2S)-2-[4-(5-chloro-8-{2-[ethyl(isopropyl)aminoformyl]-4-fluorophenyl}-3-methylimidazo[1,5-a]pyridin-6-yl)piperazin-1-carbonyl]-pyrrolidin-1-tert-butyl carboxylate 115c

(2*S*)-1-[(tert-butoxy)carbonyl]pyrrolidin-2-carboxylic acid **92a** (50.95 mg, 236.72 µmol) and *N,N-*diisopropylethylamine (194.69 mg, 1.51 mmol) were dissolved in DCM (3.0 mL). The mixture was cooled to 0°C, followed by the addition of EDCI (62.29 mg, 324.95 µmol), HOBT (44.49 mg, 329.26 µmol) and the crude product of Compound **115b** (123 mg), and the mixture was then stirred at room temperature for 12 hours. A saturated aqueous sodium carbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the preparative thin-layer chromatography using petroleum ether/ethyl acetate as an elution system, to obtain the title Compound **115c** (120 mg, 183.15 µmol, yield: 68.2%).
MS m/z (ESI): 655.2 [M+1]

### Step 4

### 2-(5-chloro-3-methyl-6-{4-[(2S)-pyrrolidin-2-carbonyl]piperazin-1-yl}imidazo[1,5-a]pyridin-8-yl)-N-ethyl-5-fluoro-N-(isopropyl)benzamide 115

Compound **115c** (120 mg, 183.15 µmol) was dissolved in methane dioxide (2.0 mL) and trifluoroacetic acid (0.5 mL). The mixture was stirred at room temperature to allow for reacting for 0.5 hours. The reaction mixture was concentrated under a reduced pressure, and purified by the HPLC (mobile phase: acetonitrile, water (0.1% ammonia water), elution gradient: 29%-59%), to obtain the title Compound **115** (3.06 mg, 5.51 µmol, yield: 3.01%) as green solid.
MS m/z (ESI): 555.2 [M+1]
¹H NMR (400 MHz, DMSO-*d*₆) δ 7.67 - 7.61 (m, 1H), 7.42 - 7.31 (m, 2H), 7.08 (s, 1H), 6.71 - 6.67 (m, 1H), 3.90 - 3.83 (m, 1H), 3.63 (s, 4H), 3.48 - 3.39 (m, 2H), 2.97 (s, 4H), 2.93 - 2.82 (m, 5H), 2.68 - 2.60 (m, 1H), 2.04 - 1.94 (m, 1H), 1.70 - 1.53 (m, 3H), 1.23 (s, 1H), 0.95 - 0.89 (m, 3H), 0.85 - 0.74 (m, 4H), 0.38 - 0.33 (m, 2H).

### Example 118

### N-ethyl-5-fluoro-N-(isopropyl)-2-(7-{1-[(1R,3S,4S)-2-azabicyclo[2.2.2]octan-3-carbonyl]-1,2,3,6-tetrahydropyridin-4-yl}quinazolin-5-yl)benzamide 118

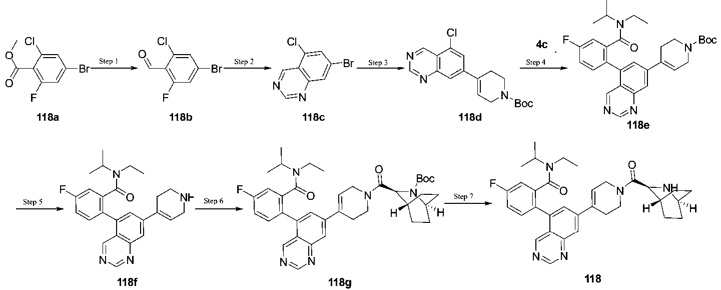

### Step 1

### 4-bromo-2-chloro-6-fluorobenzaldehyde 118b

Methyl 4-bromo-2-chloro-6-fluorobenzoate **118a**(2 g, 7.48 mmol) was dissolved in dichloromethane (56.36 mL), cooled to -78°C, followed by the slow dropwise addition of DIBAL-H (3.19 g, 22.43 mmol). The reaction mixture was stirred at -65°C to allow for reacting for 2.0 hours . Methanol (5.0 mL) was dropwise added to the reaction mixture, which was further stirred for 0.5 hours. Methanol (2 mL), a 10% aqueous sodium hydroxide solution (2 mL) and water (2 mL) were added to the reaction mixture, which was then warmed to room temperature, followed by the addition of anhydrous sodium sulfate (20 g) and further stirring for 0.5 hours. The mixture was filtered, and the filtrate was dried over anhydrous sodium sulfate, and concentrated under a reduced pressure, to obtain the title Compound **118b** (1.67 g, 7.03 mmol, yield: 94.1%; purity: 66%).
MS m/z (ESI): 238.9 [M+1]
¹H NMR (400 MHz, DMSO-*d₆*) δ 10.17 (s, 1H), 7.72 (s, 2H).

### Step 2

### 7-bromo-5-chloroquinazoline 118c

Compound **118b** (200 mg, 842.27 µmol) and formamidine acetate (263.07 mg, 2.53 mmol) were dissolved in acetonitrile (10 mL). Potassium carbonate (582.05 mg, 4.21 mmol) was added, and the mixture was stirred at 110°C at for 12 hours. The reaction mixture was cooled to room temperature, quenched by adding water (50 mL), and extracted with ethyl acetate (50 mL x 3). The organic phase was washed with saturated brine, dried over anhydrous sodium sulfate, and concentrated under a reduced pressure. The resulting residues were purified by the silica-gel column chromatography using petroleum ether/ethyl acetate as an elution system, to obtain the title Compound **118c** (102 mg, 418.91 µmol, yield: 49.7%).
MS m/z (ESI): 243.0[M+1]

### Step 3

### 4-(5-chloroquinazolin-7-yl)-1,2,3,6-tetrahydropyridin-1-tert-butyl carboxylate 118d

Compound **118c** (102 mg, 418.91 µmol), 4-(tetramethyl-1,3,2-dioxaboran-2-yl)- 1,2,3,6-tetrahydropyridin-1-tert-butyl carboxylate (129.53 mg, 418.91 µmol) and sodium carbonate (133.20 mg, 1.26 mmol) were dissolved in dioxane (5 mL) and water (1 mL). Pd (dppf)Cl₂ (30.65 mg, 41.89 µmol) was added. Under a nitrogen atmosphere, the mixture was stirred at 100°Cto allow for reacting for 4.0 hours. The reaction mixture was cooled to room temperature, quenched by adding water (50 mL), and extracted with ethyl acetate (50 mL x 3). The organic phase was washed with saturated brine, dried over anhydrous sodium sulfate, and concentrated under a reduced pressure. The resulting residues were purified by the preparative thin-layer chromatography using petroleum ether/ethyl acetate as an elution system, to obtain the title Compound **118d** (61 mg, 176.39 µmol, yield: 42.1%).
MS m/z (ESI): 346.1 [M+1]
¹H NMR (400 MHz, CDCl₃) δ 9.68 (s, 1H), 9.34 (s, 1H), 7.85 (s, 1H), 7.78 (s, 1H), 6.37 (br s, 1H), 4.17 (br d, J = 2.1 Hz, 2H), 3.69 (t, J = 5.6 Hz, 2H), 2.63 (br s, 2H), 1.50 (s, 9H).

### Step 4

### 4-(5-{2-[ethyl(isopropyl)aminoformyl]-4-fluorophenyl}quinazolin-7-yl)-1,2,3,6-tetrahydropyridin-1-tert-butyl carboxylate 118e

Compound **118d** (61 mg, 176.39 µmol) and Compound **4c** (73.91 mg, 176.39 µmol) were dissolved in dioxane (5 mL) and water (1.0 mL). Sodium carbonate (56.09 mg, 529.17 µmol) and Pd(dppf)Cl₂ (12.91 mg, 17.64 µmol) were added, and the mixture was stirred at 100 °C under a nitrogen atmosphere to allow for reacting for 2.0 hours. The reaction mixture was cooled to room temperature, quenched by adding water (50 mL), and extracted with ethyl acetate (50 mL x 3). The organic phase was washed with saturated brine, dried over anhydrous sodium sulfate, and concentrated under a reduced pressure. The resulting residues were purified by the silica-gel column chromatography using petroleum ether/ethyl acetate as an elution system, to obtain the title Compound **118e** (51 mg, 98.34 µmol, yield: 55.75%).
MS m/z (ESI): 519.3 [M+1]

### Step 5

### N-ethyl-5-fluoro-N-(isopropyl)-2-[7-(1,2,3,6-tetrahydropyridin-4-yl)quinazolin-5-yl]benzamide 118f

Compound **118e** (150 mg, 289.23 µmol) was dissolved in dichloromethane (5 mL), followed by the addition of trifluoroacetic acid (1.48 g, 12.98 mmol, 1 mL) at 0°C. The mixture was stirred at room temperature to allow for reacting for 1.0 hours. The reaction mixture was quenched by adding a saturated aqueous sodium bicarbonate solution (10 mL), and extracted with dichloromethane (10 mL x 3). The organic phase was dried over anhydrous sodium sulfate, and concentrated under a reduced pressure. The crude product of title Compound **118f** (120 mg, 286.73 µmol, yield: 99.14%) was obtained. The crude product was directly used in the next step of reaction without purification. MS m/z (ESI): 419.3 [M+1]

### Step 6

### (1R,3S,4S)-3-[4-(5-{2-[ethyl(isopropyl)aminoformyl]-4-fluorophenyl}quinazolin-7-yl)-1,2,3,6-tetrahydropyridin-1-carbonyl]-2-azabicyclo[2.2.2]octan-2-tert-butyl carboxylate 118g

Compound **32a** (57.53 mg, 225.34 µmol) and *N,N*-diisopropylethylamine (145.61 mg, 1.13 mmol) were dissolved in dichloromethane (5.1 mL), and cooled to 0°C. EDCI (64.80 mg, 338.01 µmol) and HOBT (64.80 mg, 338.01 µmol) were added, and the mixture was stirred at room temperature for 1.0 hours. The crude product of Compound **118f** (120 mg, 286.73 µmol) was added, and the mixture was stirred at room temperature for 12 hours. Water (20 mL) was added to the reaction mixture, which was then extracted with ethyl acetate (10 mL x 3). The organic phase was dried over anhydrous sodium sulfate, and concentrated under a reduced pressure. The resulting residues were purified by the silica-gel column chromatography using petroleum ether/ethyl acetate as an elution system, to obtain the title Compound **118g** (102 mg, 155.54 µmol, yield: 54.2%).
MS m/z (ESI): 656.4 [M+1]

### Step 7

### N-ethyl-5-fluoro-N-(isopropyl)-2-(7-{1-[(1R,3S,4S)-2-azabicyclo[2.2.2]octan-3-carbonyl]-1,2,3,6-tetrahydropyridin-4-yl}quinazolin-5-yl)benzamide 118

Compound **118g** (102 mg, 155.54 µmol) was dissolved in methane dioxide (5.0 mL) and trifluoroacetic acid (1.48 g, 12.98 mmol, 1 mL). The mixture was stirred at room temperature to allow for reacting for 0.5 hours. The reaction mixture was concentrated under a reduced pressure, followed by the addition of a saturated aqueous sodium carbonate solution (10 mL), and the reaction mixture was extracted with ethyl acetate (5 mL x 3). The organic phase was dried over anhydrous sodium sulfate, and concentrated under a reduced pressure. The resulting residues were purified by the preparative thin-layer chromatography using dichloromethane/methanol as an elution system, to obtain the title Compound **118** (14.34 mg, 25.81 µmol, yield: 16.59%).
MS m/z (ESI): 556.2 [M+1]
¹H NMR (400 MHz, CD₃OD) δ 9.42 - 9.09 (m, 2H), 8.10 - 7.80 (m, 2H), 7.69 - 7.54 (m, 1H), 7.46 - 7.24 (m, 2H), 6.66 - 6.50 (m, 1H), 4.59 - 4.42 (m, 2H), 4.40 - 4.28 (m, 1H), 4.26 - 4.04 (m, 1H), 3.83 - 3.77 (m, 1H), 3.49 (s, 1H), 2.85 - 2.74 (m, 2H), 2.24 - 2.13 (m, 1H), 2.08 - 1.98 (m, 3H), 1.91 - 1.83 (m, 2H), 1.75 - 1.69 (m, 1H), 1.67 - 1.55 (m, 2H), 1.29 (s, 3H), 1.04 - 0.86 (m, 5H), 0.73 - 0.63 (m, 2H), 0.31 - 0.23 (m, 1H), 0.13 - -0.03 (m, 2H).

### Example 120

### N-ethyl-5-fluoro-2-[3-methyl-6-(1-{[(1r,4r)-4-ethanesulfonamidocyclohexyl]methyl}-azacyclobutan-3-yl)imidazo[1,5-a]pyridin-8-yl]-N-(isopropyl)benzamide 120

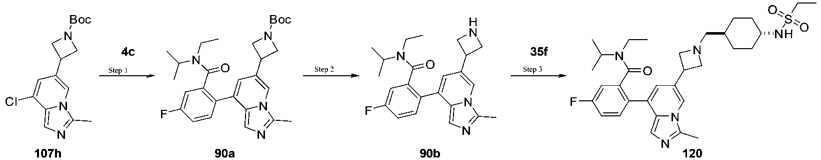

### Step 1

### 3-(8-{2-[ethyl(isopropyl)aminoformyl]-4-fluorophenyl}-3-methylimidazo[1,5-a]pyridin-6-yl)azacyclobutan-1-tert-butyl carboxylate 90a

Compound **107h** (400 mg, 1.24 mmol), Compound **4c**(909.12 mg, 1.49 mmol) and potassium phosphate (791.54 mg, 3.73 mmol) were dissolved in dioxane (8.0 mL) and water (2.0 mL). Pd(dtbpf)Cl₂ (81.01 mg, 124.30 µmol) was added. Under a nitrogen atmosphere, the mixture was stirred at 90°Cto allow for reacting for 12 hours. The reaction mixture was cooled to room temperature, followed by adding water (30 mL) and extracting with ethyl acetate (30 mL x 3). The organic phases were combined, dried over anhydrous sodium sulfate, and concentrated under a reduced pressure. The resulting residues were purified by the preparative column chromatography using petroleum ether/ethyl acetate as an elution system, to obtain the title Compound **90a** (584 mg, 1.18 mmol, yield: 94.99%).
MS m/z (ESI): 495.2[M+1]
¹H NMR (400 MHz, DMSO-*d*₆) δ 8.03 (br s, 1H), 7.77 - 7.59 (m, 1H), 7.46 - 7.25 (m, 2H), 7.05 (s, 1H), 6.67 (br s, 1H), 4.20 (br d, J = 5.4 Hz, 2H), 3.98 - 3.72 (m, 3H), 3.48 (br s, 1H), 3.29 (br d, J = 7.1 Hz, 1H), 3.07 - 2.86 (m, 1H), 2.59 (s, 3H), 1.40 (s, 9H), 1.21 - 1.11 (m, 1H), 0.97 - 0.70 (m, 6H), 0.32 (br d, J = 6.1 Hz, 2H).

### Step 2

### 2-[6-(azacyclobutan-3-yl)-3-methylimidazo[1,5-a]pyridin-8-yl]-N-ethyl-5-fluoro-N-(isopropyl)benzamide 90b

Compound **90a** (150 mg, 303.28 µmol) was dissolved in methane dioxide (3.0 mL), followed by the addition of trifluoroacetic acid (1.48 g, 12.98 mmol, 1 mL) at 0°C. The mixture was stirred at room temperature to allow for reacting for 1.0 hours. The reaction mixture was concentrated under a reduced pressure, to obtain the crude product of title Compound **90b** (154 mg). The crude product was directly used in the next step of reaction without purification. MS m/z (ESI): 395.2 [M+1]

### Step 3

### N-ethyl-5-fluoro-2-[3-methyl-6-(1-{[(1r,4r)-4-ethanesulfonamidocyclohexyl]methyl}-azacyclobutan-3-yl)imidazo[1,5-a]pyridin-8-yl]-N-(isopropyl)benzamide 120

Compound **90b** (154 mg, 302.85 µmol, trifluoroacetate) was dissolved in DMSO(2.0 mL), followed by the addition of [(1r,4r)-4-ethanesulfonamidocyclohexyl]methyl4-methylphenyl-1-sulfonate **35f** (227.55 mg, 605.69 µmol), potassium carbonate (300 mg, 2.17 mmol) and TBAI(11.19 mg, 30.28 µmol), and the mixture was stirred at 100°C for 4.0 hours. The reaction mixture was filtered, concentrated under a reduced pressure, and the resulting residues were purified by the HPLC (mobile phase: acetonitrile, water (0.1% formic acid), elution gradient:5%-35%), to obtain the title Compound **120** (7.6 mg, 12.71 µmol, yield: 4.20%) as yellow solid.
MS m/z (ESI): 598.2 [M+1]
¹H NMR (400 MHz, DMSO-*d*₆) δ 8.20 (s, 1H), 8.00 - 7.90 (m, 1H), 7.73 - 7.61 (m, 1H), 7.43 - 7.27 (m, 2H), 7.07 - 6.94 (m, 2H), 6.84 - 6.74 (m, 1H), 3.73 - 3.54 (m, 3H), 3.51 - 3.38 (m, 1H), 3.36 - 3.12 (m, 3H), 3.03 - 2.84 (m, 4H), 2.59 (s, 3H), 2.40 - 2.30 (m, 2H), 1.91 - 1.67 (m, 4H), 1.27 - 0.70 (m, 15H), 0.33 - 0.22 (m, 2H).

### Example 121

### N-(2,2-difluoroethyl)-5-fluoro-2-(3-methyl-6-{1-[(1S,3S,4R)-5-methylene-2-azabicyclo[2.2.2]octan-3-carbonyl]azacyclobutan-3-yl}imidazo[1,5-a]pyridin-8-yl)-N-(isopropyl)benzamide 121

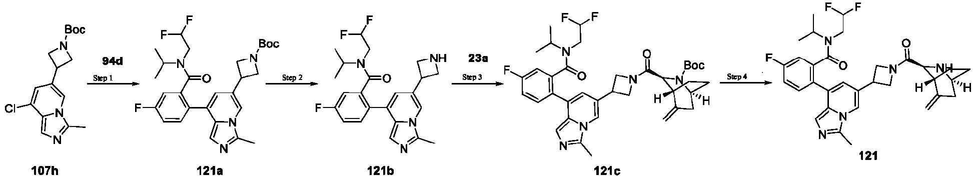

### Step 1

### 3-(8-{2-[(2,2-difluoroethyl)(isopropyl)aminoformyl]-4-fluorophenyl}-3-methylimidazo[1,5-a]pyridin-6-yl)azacyclobutan-1-tert-butyl carboxylate 121a

Compound **107h** (500 mg, 1.55 mmol), Compound **94d**(961.26 mg, 1.55 mmol) and cesium carbonate (1.52 g, 4.66 mmol) were dissolved in dioxane (15 mL) and water (5.0 mL). Pd(dtbpf)Cl₂ (101.27 mg, 155.38 µmol) was added. Under a nitrogen atmosphere, the mixture was stirred at 100°Cto allow for reacting for 12 hours. The reaction mixture was cooled to room temperature, followed by adding water (30 mL) and extracting with ethyl acetate (30 mL x 3). The organic phases were combined, dried over anhydrous sodium sulfate, and concentrated under a reduced pressure. The resulting residues were purified by the preparative column chromatography using petroleum ether/ethyl acetate as an elution system, and the resulting crude product was purified by the preparative thin-layer chromatography using dichloromethane/methanol as an elution system, to obtain the title Compound **121a** (200 mg, 376.95 µmol, yield: 24.3%).
MS m/z (ESI): 531.2[M+1]

### Step 2

### 2-[6-(azacyclobutan-3-yl)-3-methylimidazo[1,5-a]pyridin-8-yl]-N-(2,2-difluoroethyl)-5-fluoro-N-(isopropyl)benzamide 121b

Compound **121a** (70 mg, 131.93 µmol) was dissolved in methane dioxide (2.0 mL), followed by the addition of trifluoroacetic acid (740.00 mg, 6.49 mmol, 0.5 mL) at 0°C. The mixture was stirred at room temperature to allow for reacting for 1.0 hours. The reaction mixture was concentrated under a reduced pressure, to obtain the crude product of title Compound **121b** (72 mg). The crude product was directly used in the next step of reaction without purification.
MS m/z (ESI): 431.2 [M+1]

### Step 3

### (1S,3S,4R)-3-[3-(8-{2-[(2,2-difluoroethyl)(isopropyl)aminoformyl]-4-fluorophenyl}-3-methylimidazo[1,5-a]pyridin-6-yl)azacyclobutan-1-carbonyl]-5-methylene-2-azabicyclo[2.2.2]octan-2-tert-butyl carboxylate 121c

Compound **23a**(43.47 mg, 162.61 µmol) and *N,N*-diisopropylethylamine (63.05 mg, 487.84 µmol) were dissolved in DCM (3.0 mL). The mixture was cooled to 0°C, followed by the addition of EDCI (46.76 mg, 243.92 µmol), HOBT (32.96 mg, 243.92 µmol) and Compound **121b** (70 mg, 162.61 µmol), and the mixture was stirred at room temperature for 12 hours. The reaction mixture was diluted with dichloromethane (50 mL), and washed with water (50 mL x 3), and the organic phase was concentrated under a reduced pressure. The resulting residues were purified by the silica-gel column chromatography using dichloromethane/methanol as an elution system, to obtain the title Compound **121c** (80 mg, 117.69 µmol, yield: 72.4 %).
MS m/z (ESI): 680.2 [M+1]

### Step 4

### N-(2,2-difluoroethyl)-5-fluoro-2-(3-methyl-6-{1-[(1S,3S,4R)-5-methylene-2-azabicyclo[2.2.2]octan-3-carbonyl]azacyclobutan-3-yl}imidazo[1,5-a]pyridin-8-yl)-N-(isopropyl)benzamide 121

Compound **121c** (80 mg, 117.69 µmol) was dissolved in methane dioxide (3.0 mL) and trifluoroacetic acid (1.0 mL). The mixture was stirred at room temperature to allow for reacting for 0.5 hours. The reaction mixture was concentrated under a reduced pressure, and purified by the HPLC (mobile phase: acetonitrile, water (0.1% ammonia water), elution gradient: 29%-59%), to obtain the title Compound **121** (17.25 mg, 29.76 µmol, yield: 21.56%) as white solid.
MS m/z (ESI): 580.4 [M+1]
¹H NMR (400 MHz, DMSO-*d*₆) δ 8.12 - 8.03 (m, 1H), 7.76 - 7.66 (m, 1H), 7.50 - 7.36 (m, 2H), 7.09 - 6.98 (m, 1H), 6.74 - 6.60 (m, 1H), 6.01 - 5.43 (m, 1H), 4.99 - 4.84 (m, 1H), 4.79 - 4.09 (m, 5H), 4.05 - 3.85 (m, 2H), 3.71 - 3.48 (m, 3H), 2.98 (s, 1H), 2.60 (s, 3H), 2.44 - 2.22 (m, 3H), 1.76 - 1.62 (m, 2H), 1.49 - 1.37 (m, 2H), 0.96 - 0.85 (m, 3H), 0.41 - 0.28 (m, 3H).

### Example 128

### N-(2,2-difluoroethyl)-5-fluoro-2-[3-methyl-6-(1-{[(1r,4r)-4-ethanesulfonamidocyclohexyl]piperidin-4-yl}imidazo[1,5-a]pyridin-8-yl)-N-(isopropyl)benzamide 128

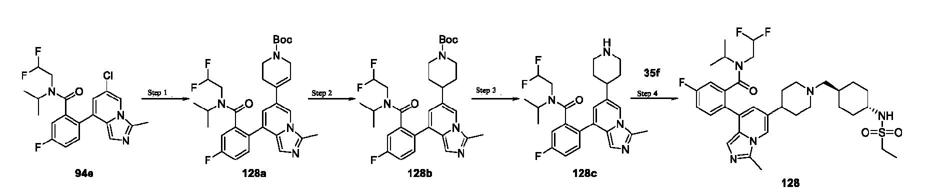

### Step 1

### 4-(8-{2-[(2,2-difluoroethyl)(isopropyl)aminoformyl]-4-fluorophenyl}-3-methylimidazo[1,5-a]pyridin-6-yl)-1,2,3,6-tetrahydropyridin-1-tert-butyl formate 128a

Compound **94e** (365 mg, 890.61 µmol), 4-(tetramethyl-1,3,2-dioxaboran-2-yl)- 1,2,3,6-tetrahydropyridin-1-tert-butyl carboxylate (413.07 mg, 1.34 mmol) and potassium phosphate (567.14 mg, 2.67 mmol) were dissolved in dioxane (8.0 mL) and water (1.0 mL). Pd(dtbpf)Cl₂ (58.04 mg, 89.06 µmol) was added. Under a nitrogen atmosphere, the mixture was stirred at 90°Cto allow for reacting for 12 hours. The reaction mixture was cooled to room temperature, followed by adding water (20 mL) and extracting with ethyl acetate (20 mL x 3). The organic phases were combined, washed with saturated brine (40 mL), dried over anhydrous sodium sulfate, and concentrated under a reduced pressure. The resulting residues were purified by the silica-gel column chromatography using petroleum ether/ethyl acetate as an elution system, to obtain the title Compound **128a** (450 mg, 808.45 µmol, yield: 90.78%).
MS m/z (ESI): 557.2 [M+1]

### Step 2

### 4-(8-{2-[(2,2-difluoroethyl)(isopropyl)aminoformyl]-4-fluorophenyl}-3-methylimidazo[1,5-a]pyridin-6-yl)piperidin-1-tert-butyl formate 128b

Compound **128a** (450 mg, 808.45 µmol) was dissolved in tetrahydrofuran (10 mL), followed by the addition of Pd/C (86.04 mg, 808.45 µmol). Under a hydrogen atmosphere (15 Psi), the mixture was stirred at room temperature for 6 hours. The reaction mixture was filtered, and the filtrate was concentrated under a reduced pressure, to obtain the crude product of title Compound **128b** (300 mg, 537.02 µmol, yield: 66.43%). The crude product was directly used in the next step of reaction without purification.
MS m/z (ESI): 559.4 [M+1]

### Step 3

### N-(2,2-difluoroethyl)-5-fluoro-2-[3-methyl-6-(piperidin-4-yl)imidazo[1,5-a]pyridin-8-yl]-N-(isopropyl)benzamide 128c

Compound **128b** (50 mg, 89.50 µmol) was dissolved in dichloromethane (2.5 mL) and trifluoroacetic acid (740.00 mg, 6.49 mmol, 0.5 mL). The mixture was stirred at room temperature to allow for reacting for 1.0 hours. A saturated aqueous sodium carbonate solution (20 ml) was added to the reaction mixture, which was then extracted with ethyl acetate (10 ml×3). The organic phases were combined, dried over anhydrous sodium sulfate, and concentrated under a reduced pressure to obtain the title Compound **128c** (41 mg, 89.42 µmol, yield: 99.9%).
MS m/z (ESI): 459.3 [M+1]

### Step 4

### N-(2,2-difluoroethyl)-5-fluoro-2-[3-methyl-6-(1-{[(1r,4r)-4-ethanesulfonamidocyclohexyl]piperidin-4-yl}imidazo[1,5-a]pyridin-8-yl)-N-(isopropyl)benzamide 128

Compound **128c** (51.24 mg, 89.5 µmol, trifluoroacetate) was dissolved in acetonitrile (2.0 mL), followed by the addition of [(1r,4r)-4-ethanesulfonamidocyclohexyl]methyl4-methylphenyl-1-sulfonate **35f** (67.21 mg, 179.00 µmol), potassium carbonate (61.85 mg, 447.50 µmol) and TBAI(3.31 mg, 8.95 µmol), and the mixture was stirred at 80°C for 12 hours. Water (20 mL) was added to the reaction mixture, which was then extracted with dichloromethane (20 mL x 3). The organic phases were combined, dried over anhydrous sodium sulfate, and concentrated under a reduced pressure, the resulting residues were purified by the preparative thin-layer chromatography using petroleum ether/ethyl acetate as an elution system, and the obtained crude product was purified by the HPLC (mobile phase: acetonitrile, water (0.1% ammonia water), elution gradient:39%-69%), to obtain the title Compound **128** (27.51 mg, 41.57 µmol, yield: 46.44%) as white solid.
MS m/z (ESI): 662.4 [M+1]
¹H NMR (400 MHz, DMSO-*d*₆) δ 7.88 (s, 1H), 7.73 - 7.61 (m, 1H), 7.47 - 7.35 (m, 2H), 7.04 - 6.96 (m, 2H), 6.53 (s, 1H), 5.90 - 5.56 (m, 1H), 4.15 - 3.44 (m, 2H), 3.03 - 2.88 (m, 5H), 2.68 - 2.55 (m, 3H), 2.46 - 2.31 (m, 2H), 2.11 - 2.05 (m, 2H), 1.99 (s, 4H), 1.79 - 1.63 (m, 6H), 1.44 - 1.36 (m, 1H), 1.27 - 1.15 (m, 5H), 1.02 - 0.80 (m, 6H), 0.32 - 0.29 (m, 2H).

### Example 131

### N-(2,2-difluoroethyl)-5-fluoro-2-(1-fluoro-3-methyl-6-{1-[(2S)-piperidin-2-carbonyl]azacyclobutan-3-yl}imidazo[1,5-a]pyridin-8-yl)-N- (isopropyl)benzamide 131

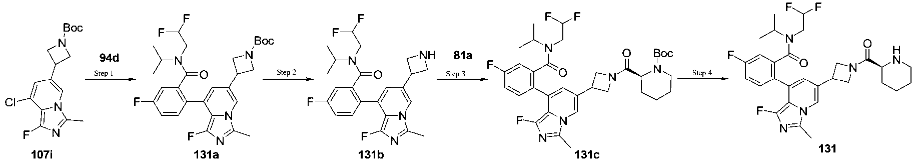

### Step 1

### 3-(8-12-[(2,2-difluoroethyl)(isopropyl)aminoformyl]-4-fluorophenyl}-1-fluoro-3-methylimidazo[1,5-a]pyridin-6-yl)azacyclobutan-1-tert-butyl carboxylate 131a

Compound **107i** (380 mg, 1.12 mmol), Compound **94d**(1.47 g, 2.57 mmol) and potassium phosphate (712.15 mg, 3.35 mmol) were dissolved in dioxane (8.0 mL) and water (2.0 mL). Pd(dtbpf)Cl₂ (72.89 mg, 111.83 µmol) was added. Under a nitrogen atmosphere, the mixture was stirred at 90°Cto allow for reacting for 12 hours. The reaction mixture was cooled to room temperature, followed by adding water (30 mL) and extracting with ethyl acetate (30 mL x 3). The organic phases were combined, dried over anhydrous sodium sulfate, and concentrated under a reduced pressure. The resulting residues were purified by the preparative column chromatography using petroleum ether/ethyl acetate as an elution system, to obtain the title Compound **131a** (530 mg, 966.15 µmol, yield: 86.4%) as brown solid.
MS m/z (ESI): 549.3[M+1]

### Step 2

### 2-[6-(azacyclobutan-3-yl)-1-fluoro-3-methylimidazo[1,5-a]pyridin-8-yl]-N-(2,2-difluoroethyl)-5-fluoro-N-(isopropyl)benzamide 131b

Compound **131a** (130 mg, 236.98 µmol) was dissolved in methane dioxide (3.0 mL), followed by the addition of trifluoroacetic acid (1.48 g, 12.98 mmol, 1 mL) at 0°C. The mixture was stirred at room temperature to allow for reacting for 1.0 hours. The reaction mixture was concentrated under a reduced pressure, to obtain the crude product of title Compound **131b** (129.4 mg). The crude product was directly used in the next step of reaction without purification.
MS m/z (ESI): 449.2 [M+1]

### Step 3

### (2S)-2-[3-(8-{2-[(2,2-difluoroethyl)(isopropyl)aminoformyl]-4-fluorophenyl}-1-fluoro-3-methylimidazo[1,5-a]pyridin-6-yl)azacyclobutan-1-carbonyl]piperidin-1-tert-butyl carboxylate 131c

Compound **81a** (52.24 mg, 227.86 µmol) and DIPEA (88.35 mg, 683.58 µmol) were dissolved in DCM (2 mL). The mixture was cooled to 0°C, followed by the addition of EDCI (52.42 mg, 273.43 µmol) and HOBT (36.95 mg, 273.43 µmol), and the mixture was stirred at room temperature for 0.5 hours. Compound **131b** (102.19 mg, 227.86 µmol) was added, and the mixture was stirred at room temperature for 12 hours. A saturated aqueous sodium bicarbonate solution (20 mL) was added to the reaction mixture, which was then extracted with dichloromethane (20 mL x 3). The organic phases were combined, dried over anhydrous sodium sulfate, and concentrated under a reduced pressure. The resulting residues were purified by the preparative thin-layer chromatography using ethyl acetate/ethanol as an elution system, to obtain the title Compound **131c** (50 mg, 75.79 µmol, yield: 33.26%) as yellow solid.
MS m/z (ESI): 660.3 [M+1]

### Step 4

### N-(2,2-difluoroethyl)-5-fluoro-2-(1-fluoro-3-methyl-6-{1-[(2S)-piperidin-2-carbonyl]azacyclobutan-3-yl}imidazo[1,5-a]pyridin-8-yl)-N-(isopropyl)benzamide 131

Compound **131c** (50 mg, 75.79 µmol) was dissolved in methane dioxide (3 mL), followed by the addition of trifluoroacetic acid (1.48 g, 12.98 mmol, 1 mL). The mixture was stirred at room temperature to allow for reacting for 1.0 hours. The reaction mixture was concentrated under a reduced pressure. The resulting residues were purified by the HPLC (mobile phase: acetonitrile, water (0.1% ammonia water), elution gradient: 31%-61%), to obtain the title Compound **131** (12.75 mg, 22.78 µmol, yield: 60.1 %) as yellow solid.
MS m/z (ESI): 560.3 [M+1]
¹H NMR (400 MHz, DMSO-*d₆*) δ 7.97 (s, 1H), 7.62 - 7.51 (m, 1H), 7.47 - 7.26 (m, 2H), 6.57 - 6.42 (m, 1H), 6.15 - 5.67 (m, 1H), 4.68 - 4.42 (m, 1H), 4.31 - 4.06 (m, 2H), 3.93 - 3.39 (m, 5H), 3.27 - 3.12 (m, 2H), 3.00 - 2.88 (m, 1H), 2.55 (s, 3H), 1.84 - 1.57 (m, 2H), 1.50 - 1.20 (m, 4H), 1.05 - 0.87 (m, 3H), 0.71 - 0.12 (m, 3H).

### Example 132

### N-(2,2-difluoroethyl)-5-fluoro-2-[1-fluoro-3-methyl-6-(1-{[(1r,4r)-4-ethanesulfonamidocyclohexyl]methyl}-azacyclobutan-3-yl)imidazo[1,5-a]pyridin-8-yl]-N-(isopropyl)benzamide 132

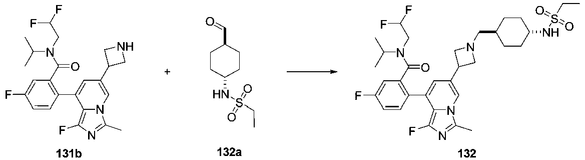

Compound **131b** (129 mg, 229.34 µmol, trifluoroacetate), *N*-[(1*r*,4*r*)-4-formylcyclohexyl]ethan-1-sulfonamide **132a** (75.44 mg, 344.01 µmol) and triethylamine (69.62 mg, 688.03 µmol) were dissolved in methanol (2.0 mL), and stirred at room temperature for 0.5 hours. Sodium cyanoborohydride (43.24 mg, 688.03 µmol) was added, and the mixture was stirred at room temperature for 1.0 hours. A saturated aqueous odium hydrogen carbonate solution (20 mL) was added to the reaction mixture, which was then extracted with dichloromethane (20 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 39%-69%), to obtain the title Compound **132** (8.73 mg, 13.39 µmol, yield: 5.84 %) as yellow solid.
MS m/z (ESI): 652.3 [M+1]
¹H NMR (400 MHz, DMSO-*d*₆) δ 7.83 (s, 1H), 7.60 - 7.52 (m, 1H), 7.45 - 7.35 (m, 2H), 7.00 - 6.94 (m, 1H), 6.61 (s, 1H), 6.07 - 5.71 (m, 1H), 3.72 - 3.48 (m, 5H), 3.44 - 3.36 (m, 1H), 3.07 - 2.92 (m, 5H), 2.53 (s, 3H), 2.27 - 2.20 (m, 2H), 1.91 - 1.68 (m, 4H), 1.23 - 1.12 (m, 6H), 0.99 - 0.84 (m, 5H), 0.64 - 0.19 (m, 3H).

### Example 134

### N-[(1r,4r)-4-{[3-(8-{4-fluoro-2-[(3R)-3-methylmorpholin-4-carbonyl]phenyl}-3-methylimidazo[1,5-a]pyridin-6-yl)azacyclobutan-1-yl]methyl}cyclohexyl]ethan-1-sulfonamide 134

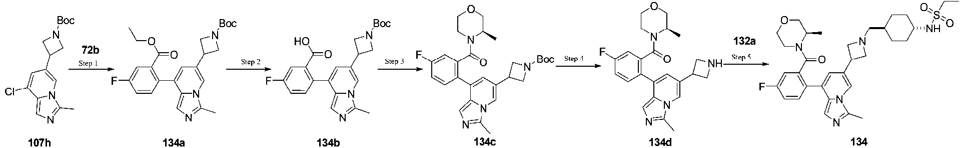

### Step 1

### 3-{8-[2-(ethoxycarbonyl)-4-fluorophenyl]-3-methylimidazo[1,5-a]pyridin-6-yl}azacyclobutan-1-tert-butyl carboxylate 134a

Compound **107h** (300 mg, 932.25 µmol), Compound **72b**(548.40 mg, 1.86 mmol) and cesium carbonate (911.24 mg, 2.80 mmol) were dissolved in dioxane (6.0 mL) and water (1.0 mL). Pd(dtbpf)Cl₂ (60.76 mg, 93.23 µmol) was added. Under a nitrogen atmosphere, the mixture was stirred at 90°Cto allow for reacting for 12 hours. The reaction mixture was cooled to room temperature, water (20 mL) was added, and the mixture was extracted with ethyl acetate (10 mL x 3). The organic phases were combined, dried over anhydrous sodium sulfate, and concentrated under a reduced pressure. The resulting residues were purified by the preparative column chromatography using petroleum ether/ethyl acetate as an elution system, to obtain the title Compound **134a** (415 mg, 915.10 µmol, yield: 98.16%) as yellow oil. MS m/z (ESI): 454.3[M+1]

### Step 2

### 2-(6-{1-[(tert-butoxy)carbonyl]azacyclobutan-3-yl}-3-methylimidazo[1,5-a]pyridin-8-yl)-5-fluorobenzoic acid 134b

Compound **134a** (375 mg, 826.89 µmol) was dissolved in ethanol (3.0 mL), tetrahydrofuran (3.0 mL) and water (1.5 mL), followed by the addition of LiOH.H₂O (59.41 mg, 2.48 mmol), and stirred at 60°C to allow for reacting for 6.0 hours. The reaction mixture was cooled to room temperature, followed by the addition of a saturated aqueous ammonium chloride solution (10 mL), and the reaction mixture was extracted with ethyl acetate (10 mL×3). The organic phase was dried over anhydrous sodium sulfate, filtered and concentrated under a reduced pressure. The crude product of title Compound **134b** (350 mg, 822.65 µmol, yield: 99.5%) was obtained as yellow oil. The crude product was directly used in the next step of reaction without purification.
MS m/z (ESI): 426.3 [M+1]
¹H NMR (400 MHz, DMSO-*d₆*) δ 8.00 (s, 1H), 7.70 - 7.42 (m, 3H), 6.86 (s, 1H), 6.63 (s, 1H), 4.24 (s, 2H), 4.00 - 3.90 (m, 2H), 3.88 - 3.74 (m, 1H), 2.61 (s, 3H), 1.40 (s, 9H).

### Step 3

### 3-(8-{4-fluoro-2-[(3R)-3-methylmorpholin-4-carbonyl]phenyl}-3-methylimidazo[1,5-a]pyridin-6-yl)azacyclobutan-1-tert-butyl carboxylate 134c

Compound **134b** (240 mg, 564.11 µmol), (3*R*)-3-methylmorpholine (57.06 mg, 564.11 µmol) and triethylamine (285.41 mg, 2.82 mmol) were dissolved in DMAc (5.0 mL), followed by the addition of HATU (428.98 mg, 1.13 mmol). Under a nitrogen atmosphere, the reaction mixture was stirred at 65°C for 4.0 hours. Water (50 mL) was added to the reaction mixture, which was then extracted with ethyl acetate (50 mL x 3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **134c** (240 mg, 471.90 µmol, yield: 83.65%) as yellow oil.
MS m/z (ESI): 509.4 [M+1]
¹H NMR (400 MHz, CDCl₃) δ 7.72 - 7.62 (m, 1H), 7.62 (s, 1H), 7.26 - 7.20 (m, 2H), 7.19 - 7.11 (m, 1H), 6.91 - 6.70 (m, 1H), 4.13 (q, J = 7.1 Hz, 2H), 4.01 - 3.92 (m, 2H), 3.79 - 3.69 (m, 2H), 2.72 (s, 3H), 2.09 (s, 1H), 2.05 (s, 3H), 1.47 (s, 12H).

### Step 4

### (3R)-4-{2-[6-(azacyclobutan-3-yl)- 3-methylimidazo[1,5-a]pyridin-8-yl]-5-fluorobenzoyl}-3-methylmorpholine 134d

Compound **134c** (130 mg, 255.61 µmol) was dissolved in methane dioxide (5.0 mL), followed by the addition of trifluoroacetic acid (1.48 g, 12.98 mmol, 1 mL) at 0°C. The mixture was stirred at room temperature to allow for reacting for 1.0 hours. A saturated aqueous sodium carbonate solution (20 mL) was added to the reaction mixture, which was then extracted with ethyl acetate (10 mL×3). The organic phases were combined, dried over anhydrous sodium sulfate, filtered and concentrated under a reduced pressure, to obtain the crude product of title Compound **134d** (104 mg, 254.61 µmol, yield: 99.6%). The crude product was directly used in the next step of reaction without purification.
MS m/z (ESI): 409.4 [M+1]

### Step 5

### N-[(1r,4r)-4-{[3-(8-{4-fluoro-2-[(3R)-3-methylmorpholin-4-carbonyl]phenyl}-3-methylimidazo[1,5-a]pyridin-6-yl)azacyclobutan-1-yl]methyl}cyclohexyl]ethan-1-sulfonamide 134

Compound **134d** (104 mg, 254.61 µmol) and triethylamine (62.08 mg, 613.47 µmol) were dissolved in methanol (1.0 mL), and stirred at room temperature for 10 min. The methanol solution (1.0 mL) of *N*-[ (1*r*,4*r*)-4-formylcyclohexyl]ethan-1-sulfonamide **132a** (67.27 mg, 306.73 µmol) was added, and the mixture was stirred at room temperature for 1.0 hour. Sodium cyanoborohydride (25.70 mg, 408.98 µmol) was added, and the mixture was stirred at room temperature for 1.0 hours. The reaction mixture was concentrated under a reduced pressure. The resulting residues were purified by the HPLC (mobile phase: acetonitrile, water (0.1%ammonium bicarbonate), elution gradient: 19%-49%), to obtain the title Compound **134** (33.98 mg, 55.54 µmol, yield: 27.2%) as faint yellow solid.
MS m/z (ESI): 612.4 [M+1]
¹H NMR (400 MHz, DMSO-*d*₆) δ 8.02 - 7.88 (m, 1H), 7.81 - 7.60 (m, 1H), 7.48 - 7.26 (m, 2H), 7.11 - 6.92 (m, 2H), 6.89 - 6.72 (m, 1H), 4.39 - 4.10 (m, 1H), 3.94 - 3.63 (m, 1H), 3.62 - 3.45 (m, 4H), 3.23 - 3.15 (m, 1H), 3.14 - 3.03 (m, 2H), 2.95 (q, J = 7.0 Hz, 4H), 2.88 - 2.66 (m, 1H), 2.60 (s, 3H), 2.25 (d, J = 6.4 Hz, 2H), 1.85 (d, J = 11.2 Hz, 2H), 1.81 - 1.66 (m, 2H), 1.24 - 1.09 (m, 7H), 1.03 (d, J = 6.4 Hz, 1H), 0.98 - 0.83 (m, J = 11.6 Hz, 2H), 0.74 - 0.30 (m, 1H).

### Example 136

### N-ethyl-5-fluoro-2-[7-methyl-2-(1-{[(1r,4r)-4-ethanesulfonamidocyclohexyl]methyl}piperidin-4-yl)imidazo[1,5-b]pyridazin-4-yl]-N-(isopropyl)benzamide 136

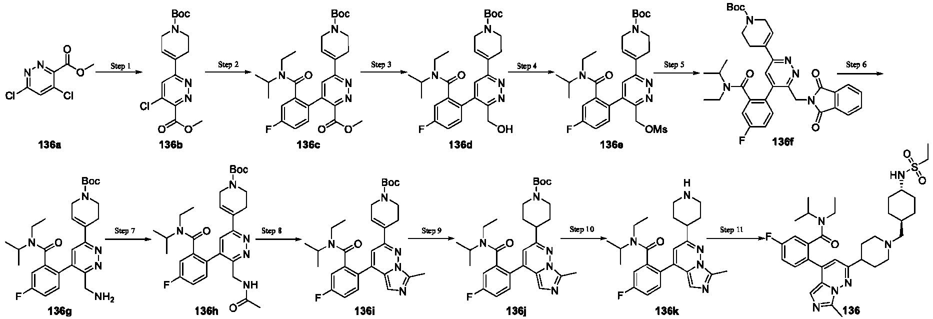

### Step 1

### 6-{1-[(tert-butoxy)carbonyl]-1,2,3,6-tetrahydropyridin-4-yl}-4-chloropyridazin-3-methyl carboxylate 136b

4,6-Dichloropyridazin-3-methyl carboxylate **136^{a}** (2 g, 9.66 mmol), 4-(tetramethyl-1,3,2-dioxaboran-2-yl)-1,2,3,6-tetrahydropyridin-1-tert-butyl carboxylate (2.99 g, 9.66 mmol) and sodium carbonate (3.07 g, 28.98 mmol) were dissolved in dioxane (40 mL) and water (8 mL). Pd (dppf)Cl₂ (706.91 mg, 966.12 µmol) was added. Under a nitrogen atmosphere, the mixture was stirred at 90°Cto allow for reacting for 4.0 hours. The reaction mixture was cooled to room temperature, water (100 mL) was added, and the mixture was extracted with ethyl acetate (100 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **136b** (2.52 g, 7.12 mmol, yield: 73.72%) as yellow oil.
MS m/z (ESI): 354.0 [M+1]
¹H NMR (400 MHz, DMSO-*d₆*) δ 8.33 (br s, 1H), 8.06 (s, 1H), 7.02 (br s, 1H), 6.00 (br s, 1H), 4.13 (br s, 2H), 3.98 (s, 4H), 3.92 (s, 4H), 3.57 (br s, 2H), 3.49 (br s, 1H), 2.67 (br s, 2H), 2.34 (br s, 2H), 1.43 (br s, 17H), 1.06 (s, 8H)

### Step 2

### 6-{l-[(tert-butoxy)carbonyl]-1,2,3,6-tetrahydropyridin-4-yl} -4-{2-[ethyl(isopropyl)aminoformyl]-4-fluorophenyl }pyridazin-3-methyl carboxylate 136c

Compound **136b** (2.5 g, 7.07 mmol), Compound **4c** (3.55 g, 8.48 mmol) and cesium carbonate (6.91 g, 21.20 mmol) were dissolved in dioxane (20 mL) and water (4.0 mL). Under a nitrogen atmosphere, Pd(dtbpf)Cl₂ (460.53 mg, 706.61 µmol) was added, and the mixture was stirred at 90°C to allow for reacting for 4.0 hours. The reaction mixture was cooled to room temperature, followed by adding water (100 mL) and extracting with ethyl acetate (100 mL x 3). The organic phases were combined, washed with saturated brine, dried over anhydrous sodium sulfate and filtered, and the filtrate was concentrated under a reduced pressure. The resulting residues were purified by the silica-gel column chromatography using petroleum ether/ethyl acetate as an elution system, to obtain the title Compound **136c** (1.38 g, 2.62 mmol, yield: 37.09%).
MS m/z (ESI): 527.3 [M+1]
¹H NMR (400 MHz, CDCl₃) δ 7.72 - 7.66 (m, 1H), 7.50 (s, 1H), 7.46 - 7.36 (m, 2H), 7.29 (s, 1H), 7.03 (s, 1H), 3.91 (s, 1H), 3.75 - 3.38 (m, 6H), 3.10 (br s, 5H), 2.89 - 2.78 (m, 2H), 1.80 - 1.39 (m, 9H), 0.96 - 0.62 (m, 7H), 0.36 - 0.29 (m, 2H).

### Step 3

### 4-(5-{2-[ethyl(isopropyl)aminoformyl]-4-fluorophenyl}-6-(hydroxymethyl)pyridazin-3-yl)-1,2,3,6-tetrahydropyridin-1-tert-butyl carboxylate 136d

Compound **136c** (1.3 g, 2.47 mmol) was dissolved in tetrahydrofuran (20 mL), and the reaction mixture was cooled to 0°C, followed by the addition of NaBH₄ (466.98 mg, 12.34 mmol). The mixture was stirred at room temperature to allow for reacting for 8 hour. The reaction mixture was cooled to 0°C, followed by adding a saturated aqueous ammonium chloride solution (50 mL) and further stirring for 0.5 hours. The reaction mixture was extracted with ethyl acetate (50 mL x 3). The organic phases were combined, washed with saturated brine, dried over anhydrous sodium sulfate, and concentrated under a reduced pressure. The resulting residues were purified by the silica-gel column chromatography using petroleum ether/ethyl acetate as an elution system, to obtain the title Compound **136d** (545 mg, 1.09 mmol, yield: 44.28%) as reddish orange oil.
MS m/z (ESI): 499.1 [M+1]

### Step 4

### 4-(5-{2-[ethyl(isopropyl)aminoformyl]-4-fluorophenyl}-6-[(methylsulfonyloxy)methyl]pyridazin-3-yl)-1,2,3,6-tetrahydropyridin-1-tert-butyl carboxylate 136e

Compound **136d** (545 mg, 1.09 mmol) was dissolved in dichloromethane (10 mL), followed by the addition of triethylamine (221.22 mg, 2.19 mmol) and methanesulfonic anhydride (228.49 mg, 1.31 mmol). The mixture was stirred at room temperature to allow for reacting for 4.0 hour. The reaction mixture was cooled to 0°C, followed by the addition of a saturated aqueous ammonium chloride solution (20 mL), and the reaction mixture was extracted with dichloromethane (20 mL x 3). The organic phases were combined, dried over anhydrous sodium sulfate, and concentrated under a reduced pressure. The crude product of title Compound **136e** (629 mg, 1.09 mmol, yield: 99.78%) was obtained. The crude product was directly used in the next step of reaction without purification.
MS m/z (ESI): 577.2 [M+1]

### Step 5

### 4-{6-[(1,3-dioxo-2,3-dihydro-1H-isoindol-2-yl)methyl]-5-{2-[ethyl(isopropyl)aminoformyl]-4-fluorophenyl}pyridazin-3-yl}-1,2,3,6-tetrahydropyridin-1-tert-butyl carboxylate 136f

The crude product of Compound **136e** (629 mg, 1.09 mmol), 2,3-dihydro-1H-isoindol-1,3-dione (192.58 mg, 1.31 mmol) and cesium carbonate (710.76 mg, 2.18 mmol) were dissolved in tetrahydrofuran (15 mL). The reaction mixture was stirred at 65°C to allow for reacting for 12 hours. Water (50 mL) was added to the reaction mixture, which was then extracted with ethyl acetate (50 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **136f** (350 mg, 557.59 µmol, yield: 51.12%) as yellow oil.
MS m/z (ESI): 628.3 [M+1]

### Step 6

### 4-[6-(aminomethyl)-5-{2-[ethyl(isopropyl)aminoformyl]-4-fluorophenyl}pyridazin-3-yl]-1,2,3,6-tetrahydropyridin-1-tert-butyl carboxylate 136g

Compound **136f** (350 mg, 557.59 µmol) was dissolved in ethanol (10.00 mL), followed by the dropwise addition of hydrazine hydrate (360 mg, 5.75 mmol, 80% purity) while stirring, and the mixture was stirred at 70°C to allow for reacting for 2 hours. The reaction mixture was cooled to 0°C, quenched by adding 1 M aqueous hydrochloric acid solution (20 mL), and stirred for 0.5 hours. The mixture was filtered, and the filter cake was rinsed with a 40% aqueous sodium hydroxide solution. The aqueous phase was extracted with dichloromethane (30 mL x 3), and the organic phases were combined, washed once with saturated aqueous sodium carbonate solution and once with saturated brine, and concentrated under a reduced pressure, to obtain the crude product of title Compound **136g** (236 mg, 474.27 µmol, yield: 85.1%) as yellow oil. The crude product was directly used in the next step of reaction without purification.
MS m/z (ESI): 498.3 [M+1]

### Step 7

### 4-[6-(acetamidomethyl)-5-{2-[ethyl(isopropyl)aminoformyl]-4-fluorophenyl}pyridazin-3-yl]-1,2,3,6-tetrahydropyridin-1-tert-butyl carboxylate 136h

The crude product of Compound **136g** (236 mg, 474.27 µmol) and triethylamine (95.98 mg, 948.55 µmol) were dissolved in dichloromethane (10 mL), followed by the dropwise addition of the solution of acetyl chloride (48.40 mg, 616.56 µmol) in dichloromethane (10 mL). The mixture was stirred at room temperature to allow for reacting for 2.0 hour. Water (50 mL) was added to the reaction mixture, which was then extracted with dichloromethane (50 mL x 3). The organic phases were combined, washed with saturated brine (50 mL), dried over anhydrous sodium sulfate, and concentrated under a reduced pressure, to obtain the crude product of title Compound **136h** (300 mg, 555.93 µmol, yield: 117.22%) as light yellow oil. The crude product was directly used in the next step of reaction without purification.
MS m/z (ESI): 540.2 [M+1]

### Step 8

### 4-(4-{2-[ethyl(isopropyl)aminoformyl]-4-fluorophenyl}-7-methylimidazo[1,5-b]pyridazin-2-yl)-1,2,3,6-tetrahydropyridin-1-tert-butyl carboxylate 136i

The crude product of Compound **136h** (300 mg, 555.93 µmol) was dissolved in dichloromethane (10 mL), followed by the addition of a Burgess reagent (145.73 mg, 611.52 µmol). The mixture was stirred at 40°C to allow for reacting for 4.0 hours. The reaction mixture was cooled to room temperature, followed by adding water (50 mL) and extracting with ethyl acetate (50 mL x 3). The organic phases were combined, washed with saturated brine (50 mL), dried over anhydrous sodium sulfate, and concentrated under a reduced pressure. The resulting residues were purified by the silica-gel column chromatography using petroleum ether/ethyl acetate as an elution system, to obtain the title Compound **136i** (167 mg, 320.15 µmol, yield: 57.59%) as yellow solid.
MS m/z (ESI): 522.5[M+1]
¹H NMR (400 MHz, DMSO-*d*₆) δ 7.78 (dd, J = 5.4, 8.5 Hz, 1H), 7.48 - 7.40 (m, 2H), 7.24 (br s, 1H), 6.93 (s, 1H), 6.64 (br s, 1H), 4.09 (br s, 2H), 3.61 - 3.49 (m, 2H), 3.47 - 3.37 (m, 2H), 2.94 - 2.84 (m, 1H), 2.63 (s, 3H), 1.43 (s, 9H), 1.12 - 0.66 (m, 9H), 0.35 (br d, J = 6.3 Hz, 2H).

### Step 9

### Tert-butyl 4-(4-{2-[ethyl(propan-2-yl)carbamoyl]-4-fluorophenyl}-7-methylimidazo[1,5-b]pyridazin-2-yl)piperidine-1-carboxylate

### 4-(4-{2-[ethyl(isopropyl)aminoformyl]-4-fluorophenyl}-7-methylimidazo[1,5-b]pyridazin-2-yl)piperidin-1-tert-butyl formate 136j

Compound **136i** (80 mg, 153.37 µmol) was dissolved in tetrahydrofuran (10 mL), followed by the addition of 50% Pd/C (7.45 mg, 30.67 µmol). Under a hydrogen atmosphere (15 Psi), the mixture was stirred at room temperature for 12 hours. The reaction mixture was filtered through diatomite, and the filtrate was concentrated under a reduced pressure, to obtain the title Compound **136j** crude product of (80 mg, 152.78 µmol, yield: 99.6%). The crude product was directly used in the next step of reaction without purification.
MS m/z (ESI): 524.2 [M+1]

### Step 10

### N-ethyl-5-fluoro-2-[7-methyl-2-(piperidin-4-yl)imidazo[1,5-b]pyridazine-4-yl]-N-(propan-2-yl)benzamide N-ethyl-5-fluoro-2-[7-methyl-2-(piperidin-4-yl)imidazo[1,5-b]pyridazin-4-yl]-N-(isopropyl)benzamide 136k

The crude product of Compound **136j** (80 mg, 152.78 µmol) was dissolved in methane dioxide (3.0 mL), followed by the addition of trifluoroacetic acid (1 g, 8.77 mmol, 675.68 µL) at 0°C. The mixture was stirred at room temperature to allow for reacting for 0.5 hours. The reaction mixture was concentrated under a reduced pressure, to obtain the crude product of title Compound **136k** (64 mg). The crude product was directly used in the next step of reaction without purification.
MS m/z (ESI): 424.3 [M+1]

### Step 11

### N-ethyl-5-fluoro-2-[7-methyl-2-(1-{[(1r,4r)-4-ethanesulfonamidocyclohexyl]methyl}piperidin-4-yl)imidazo[1,5-b]pyridazin-4-yl]-N-(propan-2-yl)benzamide

### N-ethyl-5-fluoro-2-[7-methyl-2-(1-{[(1r,4r)-4-ethanesulfonamidocyclohexyl]methyl}piperidin-4-yl)imidazo[1,5-b]pyridazin-4-yl]-N-(isopropyl)benzamide 136

The crude product of Compound **136k** (64 mg), *N*-[(1*r*,4*r*)-4-formylcyclohexyl]ethan-1-sulfonamide **132a**(41.42 mg, 151.11 µmol) and triethylamine (30.58 mg, 302.23 µmol) were dissolved in methanol (5.0 mL), and stirred at room temperature for 0.5 hours. Sodium cyanoborohydride (28.49 mg, 453.34 µmol) was added, and the mixture was stirred at room temperature for 12 hours. A saturated aqueous ammonium chloride solution (50 mL) was added to the reaction mixture, which was then extracted with ethyl acetate (50 mL x 3). The organic phases were combined, dried over anhydrous sodium sulfate, and concentrated under a reduced pressure. The resulting residues were purified by the preparative thin-layer chromatography using dichloromethane/methanol as an elution system, and the resulting crude product was purified by the preparative thin-layer chromatography using petroleum ether/ethyl acetate as an elution system, to obtain the title Compound **136** (18.92 mg, 30.18 µmol, yield: 19.97%) as yellow solid.
MS m/z (ESI): 627.4 [M+1]
¹H NMR (400 MHz, DMSO-*d*₆) δ 7.75 (dd, J = 5.6, 8.4 Hz, 1H), 7.47 - 7.36 (m, 2H), 7.21 (s, 1H), 7.00 (d, J = 7.6 Hz, 1H), 6.58 - 6.52 (m, 1H), 3.48 - 3.38 (m, 1H), 3.03 - 2.92 (m, 5H), 2.85 (m, 1H), 2.69 - 2.62 (m, 1H), 2.61 (s, 3H), 2.15 - 2.04 (m, 2H), 2.02 - 1.92 (m, 2H), 1.91 - 1.81 (m, 4H), 1.80 - 1.70 (m, 4H), 1.43 - 1.34 (m, 1H), 1.23 (s, 3H), 1.21 - 1.15 (m, 4H), 0.95 - 0.91 (m, 3H), 0.91 - 0.84 (m, 2H), 0.84 - 0.77 (m, 3H), 0.40 - 0.35 (m, 2H).

### Example 139

### N-(2,2-difluoroethyl)-5-fluoro-N-(isopropyl)-2-[6-(1-{[(1r,4r)-4-ethanesulfonamidocyclohexyl]methyl}piperidin-4-yl)pyrrolo[1,2-a]pyrazin-8-yl]benzamide 139

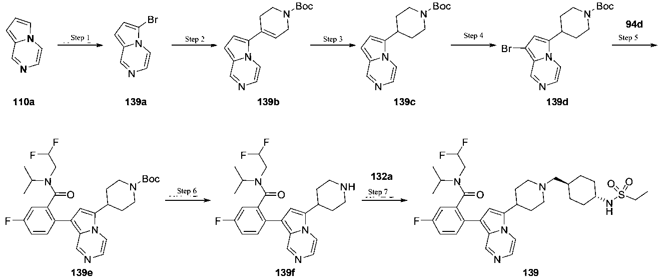

### Step 1

### 6-bromopyrrolo[1,2-a]pyrazine 139a

Pyrrolo[1,2-a]pyrazine **110a** (1.0 g, 8.46 mmol) was dissolved in trichloromethane (25 mL), and cooled to -10 °C, followed by the addition of NBS (1.35g, 7.59 mmol). The reaction mixture was stirred at-10 °C for 1.0 hour. A saturated aqueous sodium bicarbonate solution (20 mL) was added to the reaction mixture, which was then extracted with dichloromethane (20 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **139a** (1.3 g, 6.60 mmol, yield: 77.94%).
MS m/z (ESI): 198.9[M+1]

### Step 2

### 4-{pyrrolo[1,2-a]pyrazin-6-yl}-1,2,3,6-tetrahydropyridin-1-tert-butyl carboxylate 139b

Compound **139a** (500 mg, 2.54 mmol), 4-(tetramethyl-1,3,2-dioxaboran-2-yl)- 1,2,3,6-tetrahydropyridin-1-tert-butyl carboxylate (941.60 mg, 3.05 mmol) and sodium carbonate (806.89 mg, 7.61 mmol) were dissolved in dioxane (8 mL) and water (2 mL). Pd (dppf)Cl₂ (185.68 mg, 253.77 µmol) was added. Under a nitrogen atmosphere, the mixture was stirred at 90°Cto allow for reacting for 12 hours. The reaction mixture was cooled to room temperature, followed by adding water (30 mL) and extracting with ethyl acetate (30 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **139b** (750 mg, 2.51 mmol, yield: 98.72%).
MS m/z (ESI): 300.1 [M+1]

### Step 3

### 4-{pyrrolo[1,2-a]pyrazin-6-yl}piperidin-1-tert-butyl carboxylate 139c

Compound **139b**(750 mg, 2.51 mmol) was dissolved in tetrahydrofuran (20 mL), followed by the addition of Pd/C(300.00 mg, 2.82 mmol) under a nitrogen atmosphere. The reaction atmosphere was substituted with a nitrogen atmosphere (15 psi), and the mixture was stirred at 40°C for 2.0 hours. The reaction mixture was filtered, and the filtrate was concentrated under a reduced pressure. The crude product of title Compound **139c** (700 mg, 2.32 mmol, yield: 92.7%) was obtained.
MS m/z (ESI): 302.2[M+1]

### Step 4

### 4-{8-bromopyrrolo[1,2-a]pyrazin-6-yl}piperidin-1-tert-butyl carboxylate 139d

Compound **139c** (580 mg, 1.92 mmol) was dissolved in dichloromethane (6.0 mL), followed by the addition of NBS (411.03 mg, 2.31 mmol), and the mixture was stirred at room temperature for 12 hours. NBS (411.03 mg, 2.31 mmol) was supplemented, and the mixture was further stirred for 0.5 hours. Water (10 mL) was added to the reaction mixture, which was then extracted with dichloromethane (10 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **139d** (272 mg, 715.27 µmol, yield: 37.2%).
MS m/z (ESI): 382.2[M+1]
¹H NMR (400 MHz, DMSO-*d₆*) δ 8.68 (s, 1H), 8.27 (d, J = 4.9 Hz, 1H), 7.55 (d, J = 5.0 Hz, 1H), 6.95 (s, 1H), 4.15 - 3.96 (m, 3H), 2.89 (s, 3H), 1.90 (d, J = 12.4 Hz, 3H), 1.41 (s, 9H).

### Step 5

### 4-(8-{2-[(2,2-difluoroethyl)(isopropyl)aminoformyl]-4-fluorophenyl}pyrrolo[1,2-a]pyrazin-6-yl)-piperidin-1-tert-butyl carboxylate 139e

Compound **139d** (272 mg, 715.27 µmol) and Compound **94d**(885.02 mg, 1.43 mmol) were dissolved in dioxane (8 mL) and water (2.0 mL). Potassium phosphate (455.48 mg, 2.15 mmol) and Pd(dtbpf)Cl₂ (46.62 mg, 71.53 µmol) were added, and the mixture was stirred at 90 °C under a nitrogen atmosphere to allow for reacting for 12 hours. The reaction mixture was cooled to room temperature, followed by adding water (10 mL) and extracting with dichloromethane (10 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **139e** (125 mg, 229.52 µmol, yield: 32.1%).
MS m/z (ESI): 545.4 [M+1]

### Step 6

### N-(2,2-difluoroethyl)-5-fluoro-2-[6-(piperidin-4-yl)pyrrolo[1,2-a]pyrazin-8-yl]-N-(isopropyl)benzamide 139f

Compound **139e** (125 mg, 229.52 µmol) was dissolved in dichloromethane (4 mL), followed by the addition of trifluoroacetic acid (1.78 g, 15.58 mmol, 1.2 mL) at 0°C. The mixture was stirred at room temperature to allow for reacting for 1.0 hours. The reaction mixture was concentrated under a reduced pressure, to obtain the crude product of title Compound **139f** (105 mg, 236.22 µmol, yield: 103%). The crude product was directly used in the next step of reaction without purification.
MS m/z (ESI): 445.4 [M+1]

### Step 7

### N-(2,2-difluoroethyl)-5-fluoro-N-(isopropyl)-2-[6-(1-{[(1r,4r)-4-ethanesulfonamidocyclohexyl]methyl}piperidin-4-yl)pyrrolo[1,2-a]pyrazin-8-yl]benzamide 139

Compound **139f** (168 mg, 377.96 µmol), *N*-[(1*r*,4*r*)-4-formylcyclohexyl]ethan-1-sulfonamide **132a**(99.46 mg, 453.55 µmol) and triethylamine (114.74 mg, 1.13 mmol) were dissolved in methanol (2.0 mL), and stirred at room temperature for 1.0 hour. Sodium cyanoborohydride (73.63 mg, 1.17 mmol) was added, and the reaction mixture was stirred at room temperature for 11 hour. Water (5.0 mL) was added to the reaction mixture, which was then extracted with dichloromethane (5 mL x 3). The organic phases were combined, dried over anhydrous sodium sulfate, and concentrated under a reduced pressure. The resulting residues were purified by the preparative thin-layer chromatography using dichloromethane/ethyl acetate as an elution system, and the obtained crude product was purified by the HPLC (mobile phase: acetonitrile, water (0.1% ammonia water), elution gradient: 42%-72%), to obtain the title Compound **139** (9.49 mg, 14.65 µmol, yield: 3.88 %) as white solid.
MS m/z (ESI): 648.3 [M+1]
¹H NMR (400 MHz, DMSO-*d*₆) δ 8.70 (s, 1H), 8.18 (d, J = 4.4 Hz, 1H), 7.65 - 7.49 (m, 2H), 7.42 - 7.29 (m, 2H), 6.99 (d, J = 7.6 Hz, 1H), 6.78 - 6.63 (m, 1H), 6.06 - 5.65 (m, 1H), 3.69 - 3.52 (m, 1H), 3.47 - 3.38 (m, 1H), 3.05 - 2.87 (m, 6H), 2.14 - 2.01 (m, 4H), 1.93 - 1.74 (m, 6H), 1.70 - 1.54 (m, 2H), 1.48 - 1.34 (m, 1H), 1.28 - 1.16 (m, 5H), 1.09 - 0.78 (m, 6H), 0.24 (d, J = 6.8 Hz, 3H).

### Example 140

### N-(2,2-difluoroethyl)-5-fluoro-2-(6-{4-[(2S)-5-(3-fluorophenyl)pyrrolidin-2-carbonyl]piperazin-1-yl}-3-methylimidazo[1,5-a]pyridin-8-yl)-N-(isopropyl)benzamide 140

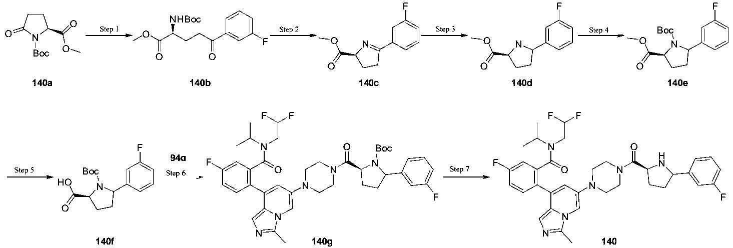

### Step 1

### (2S)-2-{[(tert-butoxy)carbonyl]amino}-5-(3-fluorophenyl)-5-methyl oxopentanoate 104b

1-Bromo-3-fluorobenzene (4.32 g, 24.69 mmol) was dissolved in THF (42.39 mL), and cooled to 0°C, followed by the dropwise addition of *i*-PrMgCl·LiCl(1.3 M, 18.99 mL), and the reaction mixture was stirred for 1 hour. At -40°C, the above reaction mixture was added to the solution of 2-methyl(2S)-5-oxopyrrolidin-1,2-dicarboxylic acid 1-tert-butyl ester (5 g, 20.57 mmol) in THF (42.39 mL), and the reaction mixture was stirred for 4.0 hours. In an ice bath, a saturated aqueous ammonium chloride solution (50 mL) was added to the reaction mixture, which was then extracted with ethyl acetate (50 mL×3). The organic phases were combined, dried over anhydrous sodium sulfate, and concentrated under a reduced pressure, to obtain the crude product of title Compound **140b** (2.57 g, 7.57 mmol, yield: 36.8%) as oil.
MS m/z (ESI): 240.2 [M-100+1]

### Step 2

### (2S)-5-(3-fluorophenyl)-3,4-dihydro-2H-pyrrol-2-methyl carboxylate 140c

Compound **140b** (300mg, 884.02 µmol) was dissolved in dichloromethane (6.0 mL), and cooled to 0°C, followed by the addition of trifluoroacetic acid (2.96 g, 25.96 mmol, 2 mL). The reaction mixture was stirred at room temperature for 2.0 hours. The reaction mixture was concentrated under a reduced pressure. The crude product of title Compound **140c** (200 mg, 904.05 µmol, yield102.27%) as yellow oil was obtained.
MS m/z (ESI): 222.1 [M+1]

### Step 3

### (2S)-5-(3-fluorophenyl)pyrrolidin-2-methyl carboxylate 140d

Compound **140c** (200 mg, 904.05 µmol) was dissolved in methanol (2.0 mL), followed by the addition of sodium cyanoborohydride (170.44 mg, 2.71 mmol), and the mixture was stirred at room temperature to allow for reacting for 0.5 hours. The solvent was removed from the reaction mixture under a reduced pressure, followed by the addition of water (10 mL), and the reaction mixture was extracted with ethyl acetate (10ml×3). The organic layer was dried over anhydrous sodium sulfate, filtered and concentrated under a reduced pressure, to obtain the crude product of title Compound **140d** (200 mg, 895.89 µmol, yield: 99.1%).
MS m/z (ESI): 224.2 [M+1]

### Step 4

### 2-methyl(2S)-5-(3-fluorophenyl)pyrrolidin-1,2-dicarboxylic acid 1-tert-butyl ester 140e

Compound **140d** (200 mg, 3.76 mmol) was dissolved in dichloromethane (2.5 mL), followed by the addition of triethylamine (142.78 mg, 1.41 mmol), DMAP(12 mg, 94.07 µmol) and (Boc)₂°(307.95 mg, 1.41 mmol), and the mixture was stirred at room temperature to allow for reacting for 2.0 hours. Water (10 mL) was added to the reaction mixture, which was then extracted with ethyl acetate (10 mL x 3). The organic phases were combined, dried over anhydrous sodium sulfate and filtered, and the filtrate was concentrated under a reduced pressure. The resulting residues were purified by the preparative thin-layer chromatography using petroleum ether/ethyl acetate as an elution system, to obtain the title Compound **104e** (55 mg, 170.09 µmol, yield: 9.04%) as oil.
MS m/z (ESI): 268.2 [M-55]

### Step 5

### (2S)-1-[(tert-butoxy)carbonyl]-5-(3-fluorophenyl)pyrrolidin-2-carboxylic acid 140f

Compound **140e** (55 mg, 170.09 µmol) was dissolved in methanol (0.2 mL), tetrahydrofuran (0.8 mL) and water (0.2 mL), followed by the addition of LiOH.H₂O (21.41 mg, 510.27 µmol), and the mixture was stirred at room temperature to allow for reacting for 12 hours. The organic solvent was removed from the reaction mixture under a reduced pressure, followed by the addition of 1M aq. HCl (8.0 mL) until the precipitation of white solid, and the reaction mixture was transferred using 1M HCl and filtered. The filter cake was transferred and dried to subsequently obtain the title Compound **140f** (40 mg, 129.31 µmol, yield: 76.03%).
MS m/z (ESI): 254.0 [M-55]
¹H NMR (400 MHz, DMSO-*d₆*) δ 12.93 - 12.31 (m, 1H), 7.56 - 7.25 (m, 3H), 7.02 (d, J = 7.8 Hz, 1H), 4.97 - 4.64 (m, 1H), 4.35 - 4.17 (m, 1H), 2.39 - 2.12 (m, 2H), 1.98 - 1.66 (m, 2H), 1.44 - 0.98 (m, 9H).

### Step 6

### (2S)-2-[4-(8-{2-[(2,2-difluoroethyl)(isopropyl)aminoformyl]-4-fluorophenyl}-3-methylimidazo[1,5-a]pyridin-6-yl)piperazin-1-carbonyl]-5-(3-fluorophenyl)pyrrolidin-1-tert-butyl carboxylate 140g

Compound **140f** (40 mg, 129.31 µmol) and DIPEA (116.99 mg, 905.18 µmol) were dissolved in DMF (2.0 mL). The mixture was cooled to 0°C, followed by the addition of EDCI (37.43 mg, 195.26 µmol), HOBT (26.73 mg, 197.85 µmol) and Compound **94g** (59.42 mg, 129.31 µmol), and the mixture was stirred at room temperature for 12 hours. Water (5 mL) was added to the reaction mixture, which was then extracted with dichloromethane (5 mL x 3). The organic phases were combined, dried over anhydrous sodium sulfate, and concentrated under a reduced pressure. The resulting residues were purified by the preparative thin-layer chromatography using ethyl acetate as an elution system, to obtain the title Compound **140g** (17.5 mg, 23.31 µmol, yield: 18.02%).
MS m/z (ESI): 751.4 [M+1]

### Step 7

### N-(2,2-difluoroethyl)-5-fluoro-2-(6-{4-[(2S)-5-(3-fluorophenyl)pyrrolidin-2-carbonyl]piperazin-1-yl}-3-methylimidazo[1,5-a]pyridin-8-yl)-N-(isopropyl)benzamide 140

Compound **140g** (17.5 mg, 23.31 µmol) was dissolved in methane dioxide (1.0 mL), and cooled to 5°C, followed by the addition of trifluoroacetic acid (444.00 mg, 3.89 mmol, 0.3 mL). The mixture was stirred at room temperature to allow for reacting for 1.0 hours. The reaction mixture was concentrated under a reduced pressure. The resulting residues were purified by the HPLC (mobile phase: acetonitrile, water (0.1% ammonia water), elution gradient: 39%-69%), to obtain the title Compound **140** (4.22 mg, 6.49 µmol, yield: 27.8 %) as yellow solid.
MS m/z (ESI): 651.4 [M+1]
¹H NMR (400 MHz, DMSO-*d₆*) δ 7.73 - 7.64 (m, 1H), 7.48 - 7.31 (m, 4H), 7.29 - 7.20 (m, 2H), 7.12 - 6.92 (m, 2H), 6.62 (s, 1H), 5.96 - 5.55 (m, 1H), 4.17 - 4.06 (m, 2H), 3.74 - 3.46 (m, 6H), 3.17 - 2.94 (m, 5H), 2.56 - 2.54 (m, 3H), 2.21 - 2.03 (m, 2H), 2.00 - 1.90 (m, 1H), 1.59 - 1.41 (m, 1H), 0.96 - 0.85 (m, 3H), 0.44 - 0.26 (m, 3H).

### Examples 141 and 142

### N-(2,2-difluoroethyl)-5-fluoro-2-(1-fluoro-3-methyl-6-{1-[2-methyl-6-(methylamino)hexan-3-yl]azacyclobutan-3-yl}imidazo[1,5-a]pyridin-8-yl)-N-(isopropyl)benzamide 141

### N-(2,2-difluoroethyl)-5-fluoro-2-[1-fluoro-6-(1-{6-[(2-methoxyethyl)(methyl)amino]-2-methylhexan-3-yl}azacyclobutan-3-yl)-3-methylimidazo[1,5-a]pyridin-8-yl]-N-(isopropyl)benzamide 142

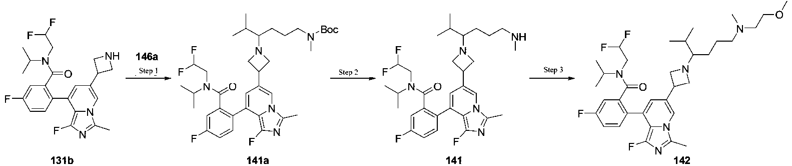

### Step 1

### N-{4-[3-(8-{2-[(2,2-difluoroethyl)(isopropyl)aminoformyl]-4-fluorophenyl}-1-fluoro-3-methylimidazo[1,5-a]pyridin-6-yl)azacyclobutan-1-yl]-5-methylhexyl}-N-methyltert-butyl carbamate 141a

Compound **131b** (81.75 mg, 182.29 µmol) and *N*-methyl-*N*-(5-methyl-4-oxohexyl)tert-butyl carbamate **146a** (66.54 mg, 273.44 µmol) were dissolved in methanol (2.0 mL), followed by the addition of ZnCl₂ (49.69 mg, 364.58 µmol), and the mixture was stirred at 60°C for 1.0 hour. The reaction mixture was cooled to room temperature, followed by adding sodium cyanoborohydride (34.37 mg, 546.87 µmol) and stirring at 60°C for 2.0 hours. A saturated aqueous sodium bicarbonate solution (20 mL) was added to the reaction mixture, which was then extracted with dichloromethane (20 mL x 3). The organic phases were combined, dried over anhydrous sodium sulfate, and concentrated under a reduced pressure. The resulting residues were purified by the preparative thin-layer chromatography using petroleum ether/ethyl acetate/ethanol as an elution system, to obtain the title Compound **141a** (50 mg, 73.99 µmol, yield: 40.59%) as yellow solid.
MS m/z (ESI): 676.5 [M+1]

### Step 2

### N-(2,2-difluoroethyl)-5-fluoro-2-(1-fluoro-3-methyl-6-{1-[2-methyl-6-(methylamino)hexan-3-yl]azacyclobutan-3-yl}imidazo[1,5-a]pyridin-8-yl)-N-(isopropyl)benzamide 141

Compound **141a** (20 mg, 29.59 µmol) was dissolved in dichloromethane (3.0 mL), followed by the addition of trifluoroacetic acid (1.48 g, 12.98 mmol, 1 mL) at 0°C. The mixture was stirred at room temperature to allow for reacting for 1.0 hours. The reaction mixture was concentrated under a reduced pressure to obtain a crude product . The resulting residues were purified by the HPLC (mobile phase: acetonitrile, water (0.1% ammonium bicarbonate), elution gradient: 34%-64%), to obtain the title Compound **141** (4.55 mg, 7.90 µmol, yield: 26.71%).
MS m/z (ESI): 576.4 [M+1]
¹H NMR (400 MHz, CD₃OD) δ 7.78 (s, 1H), 7.62 - 7.52 (m, 1H), 7.39 - 7.20 (m, 2H), 6.74 - 6.63 (m, 1H), 6.13 - 5.73 (m, 1H), 3.84 - 3.55 (m, 5H), 3.43 - 3.34 (m, 1H), 3.27 - 3.18 (m, 2H), 2.67 - 2.54 (m, 5H), 2.42 (s, 3H), 2.28 - 2.16 (m, 1H), 1.93 - 1.79 (m, 1H), 1.69 - 1.54 (m, 2H), 1.48 - 1.26 (m, 2H), 1.05 - 0.88 (m, 9H), 0.45 (s, 3H).

### Step 3

### N-(2,2-difluoroethyl)-5-fluoro-2-[1-fluoro-6-(1-{6-[(2-methoxyethyl)(methyl)amino]-2-methylhexan-3-yl}azacyclobutan-3-yl)-3-methylimidazo[1,5-a]pyridin-8-yl-N-(isopropyl)benzamide 142

Compound **141** (51.04 mg, 74 µmol, trifluoroacetate), 1-bromo-2methoxyethane (20.57 mg, 147.98 µmol), sodium iodide (33.28 mg, 222.00 µmol) and potassium carbonate (61.36 mg, 444.00 µmol) were dissolved in DMF(2.0 mL), and stirred at 50°C to allow for reacting for 12 hours. The reaction mixture was cooled to room temperature, followed by adding water (30 mL) and extracting with dichloromethane (20 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 45%-75%), to obtain the title Compound **142** (11.12 mg, 17.55 µmol, yield: 23.71%).
MS m/z (ESI): 634.4 [M+1]
¹H NMR (400 MHz, CD₃OD) δ 7.78 (s, 1H), 7.61 - 7.54 (m, 1H), 7.37 - 7.25 (m, 2H), 6.71 (s, 1H), 6.17 - 5.67 (m, 1H), 3.82 - 3.57 (m, 5H), 3.54 - 3.47 (m, 2H), 3.34 - 3.31 (m, 4H), 3.27 - 3.19 (m, 2H), 2.66 - 2.55 (m, 5H), 2.49 - 2.38 (m, 2H), 2.32 - 2.19 (m, 4H), 1.93 - 1.80 (m, 1H), 1.66 - 1.49 (m, 2H), 1.44 - 1.33 (m, 1H), 1.32 - 1.21 (m, 1H), 1.03 - 0.89 (m, 9H), 0.45 (s, 3H).

### Example 143

### N-(2,2-difluoroethyl)-2-(6-{1-[(4-ethanesulfonamidocyclohexyl)methyl]azacyclobutan-3-yl}pyrrolo[1,2-a]pyrazin-8-yl)-5-fluoro-N-(isopropyl)benzamide 143

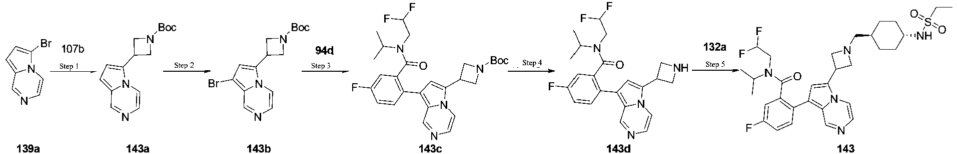

### Step 1

### 4-{pyrrolo[1,2-a]pyrazin-6-yl}-azacyclobutan-1-tert-butyl carboxylate 143a

Compound **139a** (500 mg, 2.54 mmol), 3-bromoazacyclobutan-1-tert-butyl carboxylate **107b** (778.90 mg, 3.30 mmol), Ir[dF(CF₃)ppy]₂(dtbpy) (PF₆) (28.47 mg, 25.38 µmol), NiCl₂.dtbbpy (15.15 mg, 38.06 µmol), tris(trimethylsilyl)silane (631.01 mg, 2.54 mmol) and sodium carbonate (537.93 mg, 5.08 mmol) were dissolved in dimethoxyethane (10 mL), and the reaction mixture was placed under a 10W LED bluelight lamp, and stirred at 25°C to allow for reacting for 14 hours. The reaction mixture was concentrated under a reduced pressure, and the residues were dissolved in ethyl acetate (30 mL), and washed with water (10 mL x 3). The organic phase was dried over anhydrous sodium sulfate, and concentrated under a reduced pressure. The resulting residues were purified by the silica-gel column chromatography using petroleum ether/ethyl acetate as an elution system, to obtain the title Compound **143a** (299 mg, 1.09 mmol, yield: 43.11%) as yellow oil.
MS m/z (ESI): 274.0 [M+1]
¹H NMR (400 MHz, CD₃OD) δ 8.74 (s, 1H), 7.89 (d, J= 5.2 Hz, 1H), 7.45 - 7.39 (m, 1H), 7.03 (d, J = 4.4 Hz, 1H), 6.98 (d, J = 4.4 Hz, 1H), 4.47 (t, J = 8.4 Hz, 2H), 4.29 - 4.18 (m, 1H), 4.14 - 4.02 (m, 2H), 1.46 (s, 9H).

### Step 2

### 4-{8-bromopyrrolo[1,2-a]pyrazin-6-yl}azacyclobutan-1-tert-butyl carboxylate 143b

Compound **143a** (199 mg, 728.06 µmol) was dissolved in dichloromethane (5.0 mL), followed by the addition of NBS (129.58 mg, 728.06 µmol), and the mixture was stirred at room temperature for 3.0 hours. Water (20 mL) was added to the reaction mixture, which was then extracted with dichloromethane (10 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **143b** (251 mg, 712.61 µmol, yield: 97.88%).
MS m/z (ESI): 354.1[M+1]

### Step 3

### 3-(8-{2-[(2,2-difluoroethyl)(isopropyl)aminoformyl]-4-fluorophenyl}pyrrolo[1,2-a]pyrazin-6-yl)-azacyclobutan-1-tert-butyl carboxylate 143c

Compound **143b** (251 mg, 712.61 µmol) and Compound **94d**(603.93 mg, 1.07 mmol) were dissolved in dioxane (6.0 mL) and water (1.0 mL). Sodium carbonate (226.59 mg, 2.14 mmol) and Pd(dppf)Cl₂ (52.14 mg, 71.26 µmol) were added, and the mixture was stirred at 90 °C under a nitrogen atmosphere to allow for reacting for 12 hours. The reaction mixture was cooled to room temperature, followed by adding water (30 mL) and extracting with dichloromethane (20 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **143c** (249 mg, 482.04 µmol, yield: 67.6%).
MS m/z (ESI): 517.3 [M+1]
¹H NMR (400 MHz, CD₃OD) δ 8.79 - 8.71 (m, 1H), 7.97 - 7.86 (m, 1H), 7.67 - 7.59 (m, 1H), 7.56 - 7.50 (m, 1H), 7.39 - 7.31 (m, 1H), 7.28 - 7.22 (m, 1H), 7.03 (s, 1H), 6.12 - 5.73 (m, 1H), 4.53 - 4.44 (m, 2H), 4.11 - 4.00 (m, 2H), 3.81 - 3.67 (m, 1H), 3.58 (q, J = 8.8 Hz, 1H), 1.46 (s, 9H), 1.32 - 1.19 (m, 6H), 0.34 (d, J = 6.8 Hz, 2H).

### Step 4

### 2-[6-(azacyclobutan-3-yl)pyrrolo[1,2-a]pyrazin-8-yl]-N-(2,2-difluoroethyl)-5-fluoro- N-(isopropyl)benzamide 143d

Compound **143c** (50 mg, 96.80 µmol) was dissolved in dichloromethane (5 mL), followed by the addition of trifluoroacetic acid (1.48 g, 12.98 mmol, 1 mL) at 0°C. The mixture was stirred at room temperature to allow for reacting for 1.0 hours. The reaction mixture was concentrated under a reduced pressure, to obtain the crude product of title Compound **143d** (40 mg). The crude product was directly used in the next step of reaction without purification. MS m/z (ESI): 417.2 [M+1]

### Step 5

### N-(2,2-difluoroethyl)-2-(6-{1-[(4-ethanesulfonamidocyclohexyl)methyl]azacyclobutan-3-yl}pyrrolo[1,2-a]pyrazin-8-yl)-5-fluoro-N-(isopropyl)benzamide 143

The crude product of Compound **143d** (40 mg), *N*-[(1*r*,4*r*)-4-formylcyclohexyl]ethan-1-sulfonamide **132a**(14.63 mg, 66.73 µmol) and triethylamine (13.51 mg, 133.47 µmol) were dissolved in methanol (2.0 mL), and stirred at room temperature for 1.0 hours. Sodium cyanoborohydride (5.59 mg, 88.98 µmol) was added, and the mixture was stirred at room temperature for 11 hours. Water (20 mL) was added to the reaction mixture, which was then extracted with dichloromethane (10 mL x 3). The organic phases were combined, dried over anhydrous sodium sulfate, and concentrated under a reduced pressure. The resulting residues were purified by the preparative thin-layer chromatography using dichloromethane/ethyl acetate as an elution system, to obtain the title Compound **143** (17.36 mg, 28.01 µmol, yield: 63.96%) as yellow solid.
MS m/z (ESI): 620.3 [M+1]
¹H NMR (400 MHz, CD₃OD) δ 8.72 (s, 1H), 8.03 - 7.93 (m, 1H), 7.68 - 7.57 (m, 1H), 7.54 - 7.44 (m, 1H), 7.40 - 7.31 (m, 1H), 7.25 (d, J = 8.8 Hz, 1H), 6.95 (s, 1H), 6.14 - 5.76 (m, 1H), 4.12 - 4.02 (m, 1H), 3.93 (s, 2H), 3.83 - 3.67 (m, 1H), 3.64 - 3.52 (m, 1H), 3.15 - 3.08 (m, 1H), 3.06 - 2.98 (m, 2H), 2.46 - 2.40 (m, 2H), 2.01 (d, J = 11.6 Hz, 2H), 1.85 (d, J = 11.6 Hz, 2H), 1.36 - 1.28 (m, 8H), 1.13 - 1.01 (m, 3H), 0.98 - 0.94 (m, 3H), 0.91 - 0.83 (m, 1H), 0.39 - 0.28 (m, 3H).

### Example 145

### N-[(1r,4r)-4-{[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]methyl}cyclohexyl]ethan-1-sulfonamide 145

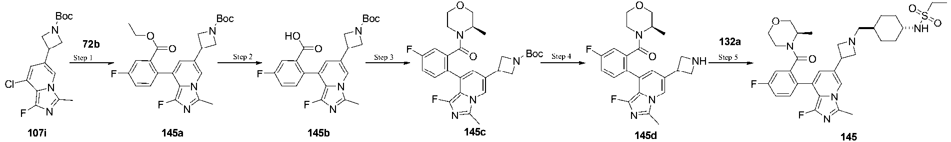

### Step 1

### 3-{8-[2-(ethoxycarbonyl)-4-fluorophenyl]-1-fluoro-3-methylimidazo[1,5-a]pyridin-6-yl}azacyclobutan-1-tert-butyl carboxylate 145a

Compound **107i** (260 mg, 765.17 µmol), Compound **72b**(450.11 mg, 1.53 mmol) and cesium carbonate (747.93 mg, 2.30 mmol) were dissolved in dioxane (12.0 mL) and water (2.0 mL). Pd(dtbpf)Cl₂ (49.87 mg, 76.52 µmol) was added. Under a nitrogen atmosphere, the mixture was stirred at 90°Cto allow for reacting for 12 hours. The reaction mixture was cooled to room temperature, followed by adding water (30 mL) and extracting with ethyl acetate (30 mL x 3). The organic phases were combined, dried over anhydrous sodium sulfate, and concentrated under a reduced pressure. The resulting residues were purified by the preparative column chromatography using petroleum ether/ethyl acetate as an elution system, to obtain the title Compound **145a** (251 mg, 532.35 µmol, yield: 69.6%) as yellow oil. MS m/z (ESI): 472.3[M+1]

### Step 2

### 2-(6-{1-[(tert-butoxy)carbonyl]azacyclobutan-3-yl}-1-fluoro-3-methylimidazo[1,5-a]pyridin-8-yl)-5-fluorobenzoic acid 145b

Compound **145a** (251 mg, 532.35 µmol) was dissolved in ethanol (3.0 mL), tetrahydrofuran (3.0 mL) and water (1.5 mL), followed by the addition of LiOH.H₂O (38.25 mg, 1.60 mmol), and stirred at 60°C to allow for reacting for 4.0 hours. The reaction mixture was cooled to room temperature, followed by the addition of a saturated aqueous ammonium chloride solution (10 mL), the reaction mixture was then extracted with trichloromethane/isopropanol (2: 1; 10 mL×3). The organic phase was concentrated under a reduced pressure. The crude product of title Compound **145b** (236 mg, 532.20 µmol, yield: 99.97%) as yellow solid was obtained. The crude product was directly used in the next step of reaction without purification.
MS m/z (ESI): 444.1 [M+1]

### Step 3

### 3-(1-fluoro-8-{4-fluoro-2-[(3R)-3-methylmorpholin-4-carbonyl]phenyl}-3-methylimidazo[1,5-a]pyridin-6-yl)azacyclobutan-1-tert-butyl carboxylate 145c

Compound **145b** (236 mg, 478.98 µmol), (3*R*)-3-methylmorpholine (53.29 mg, 526.88 µmol) and triethylamine (145.40 mg, 1.44 mmol) were dissolved in DMAc (10 mL), followed by the addition of HATU (364.25 mg, 957.96 µmol). Under a nitrogen atmosphere, the reaction mixture was stirred at 60°C for 6.0 hours. Water (20 mL) was added to the reaction mixture, which was then extracted with ethyl acetate (20 mL x 3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **145c** (250 mg, 474.77 µmol, yield: 99.12%) as yellowish green solid.
MS m/z (ESI): 527.2 [M+1]

### Step 4

### (3R)-4-{2-[6-(azacyclobutan-3-yl)-1-fluoro3-methylimidazo[1,5-a]pyridin-8-yl]-5-fluorobenzoyl}-3-methylmorpholine 145d

Compound **145c** (100 mg, 189.91 µmol) was dissolved in methane dioxide (5.0 mL), followed by the addition of trifluoroacetic acid (1.48 g, 12.98 mmol, 1 mL) at 0°C. The mixture was stirred at room temperature to allow for reacting for 1.0 hours. The reaction mixture was concentrated under a reduced pressure, to obtain the crude product of title Compound **145d** (90 mg). The crude product was directly used in the next step of reaction without purification. MS m/z (ESI): 427.2 [M+1]

### Step 5

### N-[(1r,4r)-4-{[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]methyl}cyclohexyl]ethan-1-sulfonamide 145

The crude product of Compound **145d** (90 mg) and triethylamine (57.65 mg, 569.74 µmol) were dissolved in methanol (5.0 mL). *N*-[ (1*r*,4*r*)-4-formylcyclohexyl]ethan-1-sulfonamide **132a** (45.81 mg, 208.90 µmol) was added, and the mixture was stirred at room temperature for 1.0 hour. Sodium cyanoborohydride (23.87 mg, 379.83 µmol) was added, and the mixture was stirred at room temperature for 12 hours. A saturated aqueous ammonium chloride solution (20 mL) was added, and the mixture was extracted with ethyl acetate (10 mL×3). The organic phases were combined, dried over anhydrous sodium sulfate, and concentrated under a reduced pressure. The resulting residues were purified by the silica-gel column chromatography using petroleum ether/ethyl acetate as an elution system, and the obtained crude product was purified by the HPLC (mobile phase: acetonitrile, water (0.1% ammonia water), elution gradient: 27%-57%), to obtain the title Compound **145** (21.6 mg, 34.30 µmol, yield: 18.1 %) as yellow solid. MS m/z (ESI): 630.3[M+1]
¹H NMR (400 MHz, CD₃OD) δ 7.80 (d, J = 10.8 Hz, 1H), 7.64 - 7.50 (m, 1H), 7.39 - 7.13 (m, 2H), 6.85 - 6.50 (m, 1H), 4.63 - 4.35 (m, 4H), 3.77 - 3.68 (m, 3H), 3.65 - 3.54 (m, 1H), 3.25 - 3.17 (m, 2H), 3.14 - 3.07 (m, 1H), 3.07 - 2.97 (m, 3H), 2.60 (s, 3H), 2.43 (d, J = 6.8 Hz, 2H), 2.04 (s, 1H), 2.02 - 1.96 (m, 2H), 1.89 - 1.80 (m, 2H), 1.37 - 1.26 (m, 7H), 1.23 - 1.13 (m, 1H), 1.11 - 0.98 (m, 2H), 0.86 - 0.71 (m, 1H).

### Examples 146 and 147

### N-(2,2-difluoroethyl)-5-fluoro-2-(6-{1-[2-methyl-6-(methylamino)hexan-3-yl]azacyclobutan-3-yl}pyrrolo[1,2-a]pyrazin-8-yl)-N-(isopropyl)benzamide 146

### N-(2,2-difluoroethyl)-5-fluoro-2-[6-(1-{6-[(2-methoxyethyl)(methyl)amino]-2-methylhexan-3-yl}azacyclobutan-3-yl)pyrrolo[1,2-a]pyrazin-8-yl]-N-(isopropyl)benzamide 147

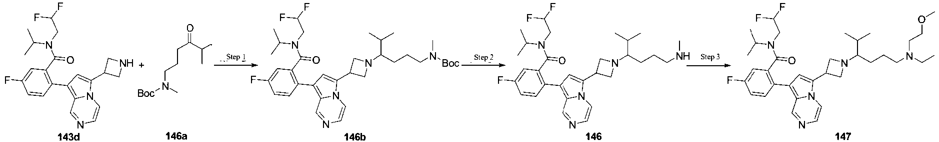

### Step 1

### N-{4-[3-(8-{2-[(2,2-difluoroethyl)(isopropyl)aminoformyl]-4-fluorophenyl}pyrrolo[1,2-a]pyrazin-6-yl)azacyclobutan-1-yl]-5-methylhexyl}-N-methyltert-butyl carbamate 146b

Compound **143d** (101 mg, 242.53 µmol) and N-methyl-N-(5-methyl-4-oxohexyl)tert-butyl carbamate **146a** (64.92 mg, 266.79 µmol) were dissolved in methanol (5.0 mL), followed by the addition of ZnCl₂ (66.11 mg, 485.07 µmol), and the mixture was stirred at 60°C for 1.0 hour. The reaction mixture was cooled to room temperature, followed by adding sodium cyanoborohydride (45.72 mg, 727.60 µmol) and stirring at 60°C for 13 hours. A saturated aqueous sodium bicarbonate solution (20 mL) was added to the reaction mixture, which was then extracted with dichloromethane (20 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **146b** (80 mg, 124.27 µmol, yield: 51.24%) as yellow solid.
MS m/z (ESI): 644.3 [M+1]

### Step 2

### N-(2,2-difluoroethyl)-5-fluoro-2-(6-{1-[2-methyl-6-(methylamino)hexan-3-yl]azacyclobutan-3-yl}pyrrolo[1,2-a]pyrazin-8-yl)-N-(isopropyl)benzamide 146

Compound **146b** (30 mg, 46.60 µmol) was dissolved in dichloromethane (5.4 mL), followed by the addition of trifluoroacetic acid (888.00 mg, 7.79 mmol, 600.00 µL) at 0°C. The mixture was stirred at room temperature to allow for reacting for 1.0 hours. Ammonia water (5.0 mL) was added to the reaction mixture, which was then concentrated under a reduced pressure. The resulting residues were purified by the HPLC (mobile phase: acetonitrile, water (0.1% ammonia water), elution gradient: 28%-58%), to obtain the title Compound **146** (2.48 mg, 4.56 µmol, yield: 9.79%). MS m/z (ESI): 544.4 [M+1]
¹H NMR (400 MHz, CD₃OD) δ 8.63 (d, J = 6.4 Hz, 1H), 7.92 (dd, J = 0.8, 1.2 Hz, 1H), 7.56 - 7.48 (m, 1H), 7.40 (d, J = 5.2 Hz, 1H), 7.25 (q, J = 8.4 Hz, 1H), 7.15 (t, J = 2.8 Hz, 1H), 6.83 (d, J = 3.2 Hz, 1H), 6.06 - 5.69 (m, 1H), 3.91 - 3.80 (m, 3H), 3.73 - 3.58 (m, 1H), 3.53 - 3.43 (m, 1H), 2.72 - 2.56 (m, 2H), 2.42 (d, J = 2.8 Hz, 3H), 2.11 - 2.03 (m, 1H), 1.82 - 1.70 (m, 1H), 1.59 - 1.49 (m, 2H), 1.38 - 1.17 (m, 6H), 0.88 - 0.82 (m, 9H), 0.29 - 0.16 (m, 3H).

### Step 3

### N-(difluoroethyl)-5-fluoro-2-[6-(1-{6-[(2-methoxyethyl)(methyl)amino]-2-methylhexan-3-yl}azacyclobutan-3-yl)pyrrolo[1,2-a]pyrazin-8-yl]-N-(isopropyl)benzamide 147

Compound **146** (16 mg, 29.43 µmol), 1-bromo-2methoxyethane (8.18 mg, 58.86 µmol), sodium iodide (13.23 mg, 88.29 µmol) and potassium carbonate (24.40 mg, 176.58 µmol) were dissolved in DMF(1.0 mL), and stirred at 50 °C to allow for reacting for 12 hours. The reaction mixture was cooled to room temperature, followed by adding water (30 mL) and extracting with dichloromethane (20 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 40%-70%), to obtain the title Compound **147** (8.18 mg, 13.59 µmol, yield: 46.2%).
MS m/z (ESI): 602.4 [M+1]
¹H NMR (400 MHz, CD₃OD) δ 8.54 (s, 1H), 7.90 - 7.76 (m, 1H), 7.46 - 7.39 (m, 1H), 7.35 - 7.28 (m, 1H), 7.16 (q, J = 8.4 Hz, 1H), 7.10 - 7.00 (m, 1H), 6.75 (d, J = 6.0 Hz, 1H), 5.95 - 5.62 (m, 1H), 3.84 - 3.69 (m, 3H), 3.64 - 3.48 (m, 1H), 3.40 (q, J = 8.8 Hz, 1H), 3.32 (d, J = 11.2 Hz, 2H), 3.14 (s, 3H), 3.12 - 3.01 (m, 3H), 2.42 (d, J = 11.2 Hz, 2H), 2.28 - 2.19 (m, 2H), 2.10 (d, J = 1.2 Hz, 3H), 2.02 - 1.94 (m, 1H), 1.76 - 1.62 (m, 1H), 1.45 - 1.33 (m, 2H), 1.27 - 1.05 (m, 2H), 0.80 - 0.72 (m, 9H), 0.22 - 0.06 (m, 3H).

### Example 150

### N-(2,2-difluoroethyl)-2-[6-(1-{[(2S,5R)-5-ethanesulfonamidooxacyclohexan-2-yl]methyl}azacyclobutan-3-yl)-3-methylimidazo[1,5-a]pyridin-8-yl]-5-fluoro-N-(isopropyl)benzamide 150

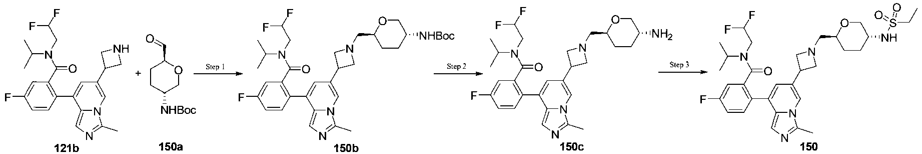

### Step 1

### N-[(3R,6S)-6-{[3-(8-{2-[(2,2-difluoroethyl)(isopropyl)aminoformyl]-4-fluorophenyl}-3-methylimidazo[1,5-a]pyridin-6-yl)azacyclobutan-1-yl]methyl}oxacyclohexan-3-yl]tert-butyl carbamate 150b

Compound **121b** (97.36 mg, 226.17 µmol), *N*-[(3*R*,6*S*)-6-formyloxacyclohexan-3-yl]tert-butyl carbamate **150a** (57.04 mg, 248.79 µmol) and triethylamine (45.77 mg, 452.34 µmol) were dissolved in methanol (2.0 mL), and stirred at room temperature for 20 min. Sodium cyanoborohydride (42.64 mg, 678.51 µmol) was added, and the mixture was stirred at room temperature for 0.5 hours. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the preparative thin-layer chromatography using petroleum ether/ethyl acetate/ethanol as an elution system, to obtain the title Compound **150b** (120 mg, 186.41 µmol, yield: 82.4%) as yellow oil.
MS m/z (ESI): 644.4 [M+1]

### Step 2

### 2-[6-(1-{[(2S,5R)-5-aminooxacyclohexan-2-yl]methyl}azacyclobutan-3-yl)-3-methylimidazo[1,5-a]pyridin-8-yl]-N-(2,2-difluoroethyl)-5-fluoro-(isopropyl)benzamide 150c

Compound **150b** (120 mg, 186.41 µmol) was dissolved in dichloromethane (3.0 mL), followed by the addition of trifluoroacetic acid (1.48 g, 12.98 mmol, 1 mL) at 0°C. The mixture was stirred at room temperature to allow for reacting for 1.0 hours. The reaction mixture was concentrated under a reduced pressure, to obtain the crude product of title Compound **150c** (105 mg). The crude product was directly used in the next step of reaction without purification. MS m/z (ESI): 544.3 [M+1]

### Step 3

### N-(2,2-difluoroethyl)-2-[6-(1-{[(2S,5R)-5-ethanesulfonamidooxacyclohexan-2-yl]methyl}azacyclobutan-3-yl)-3-methylimidazo[1,5-a]pyridin-8-yl]-5-fluoro-N-(isopropyl)benzamide 150

The crude product of Compound **150c** (100 mg) was dissolved in dichloromethane (2.5 mL), followed by the addition of triethylamine (51.48 mg, 508.78 µmol), cooling to 0°C, and the slow dropwise addition of ethanesulfonyl chloride (32.71 mg, 254.39 µmol), and the mixture was stirred at room temperature for 1.0 hour. A saturated aqueous sodium bicarbonate solution (10 mL) was added to the reaction mixture, which was then extracted with ethyl acetate (10 mL x 3). The organic phases were combined, dried over anhydrous sodium sulfate, and concentrated under a reduced pressure. The resulting residues were purified by the HPLC (mobile phase: acetonitrile, water (0. 1% ammonia water), elution gradient: 26%-56%), to obtain the title Compound **150** (16.99 mg, 26.72 µmol, yield: 15.76 %) as white solid. MS m/z (ESI): 636.2 [M+1]
¹H NMR (400 MHz, DMSO-*d₆*) δ 7.92 (s, 1H), 7.74 - 7.63 (m, 1H), 7.49 - 7.34 (m, 2H), 7.13 - 6.97 (m, 2H), 6.75 (s, 1H), 5.96 - 5.56 (m, 1H), 3.82 (s, 1H), 3.66 - 3.49 (m, 5H), 3.22 - 3.09 (m, 4H), 3.05 - 2.95 (m, 3H), 2.60 - 2.56 (m, 3H), 2.48 - 2.39 (m, 2H), 1.94 (s, 1H), 1.75 - 1.63 (m, 1H), 1.45 - 1.14 (m, 6H), 0.90 (s, 3H), 0.32 (s, 3H).

### Example 152

### N-(2,2-difluoroethyl)-2-[6-(1-{[(2S,5R)-5-ethanesulfonamidooxacyclohexan-2-yl]methyl}azacyclobutan-3-yl)-1-methyl-1H-indazol-4-yl]-5-fluoro-N-(isopropyl)benzamide 152

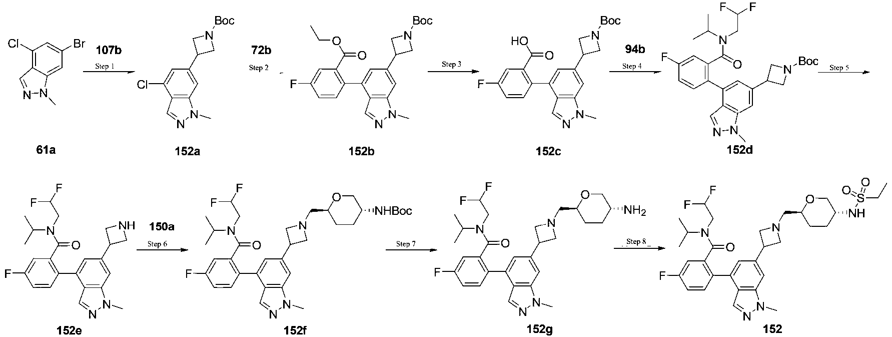

### Step 1

### 3-(4-chloro-1-methyl-1H-indazol-6-yl) azacyclobutan-1-tert-butyl carboxylate 152a

Compound **61a** (4.5 g, 18.33 mmol), 3-bromoazacyclobutan-1-tert-butyl carboxylate **107b**(5.63 g, 23.83 mmol), Ir[dF(CF₃)ppy]₂(dtbpy) (PF₆)(206 mg, 184 µmol), NiCl₂.dtbbpy (109.5 mg, 275 µmol), tris(trimethylsilyl)silane (4.56 g, 18.33 mmol) and sodium carbonate (3.89 g, 36.66 mmol) were dissolved in dimethoxyethane (320 mL), and the reaction mixture was placed under a 10W LED bluelight lamp, and stirred at 25°C to allow for reacting for 3.0 hours. The reaction mixture was filtered, the filter cake was washed with ethyl acetate, the filtrate was concentrated under a reduced pressure, and the resulting residues were purified by the silica-gel column chromatography using petroleum ether/ethyl acetate as an elution system, to obtain the title Compound **152a** (3.75 g, 11.65 mmol, yield: 63.6%) as brown oil.
MS m/z (ESI): 321.9 [M+1]

### Step 2

3-{4-[2-(ethoxycarbonyl)-4-fluorophenyl]-1-methyl-1*H*-indazol-6-yl}azacyclobutan-1-tert-butyl carboxylate **152b** Compound **152a** (2 g, 4.47 mmol), Compound **72b**(1.97 g, 6.71 mmol) and potassium phosphate (2.85 g, 13.42 mmol) were dissolved in dioxane (24.0 mL) and water (3.0 mL). Pd(dtbpf)Cl₂ (291.64 mg, 447.48 µmol) was added. Under a nitrogen atmosphere, the mixture was stirred at 90°Cto allow for reacting for 2 hours. The reaction mixture was cooled to room temperature, followed by adding water (50 mL) and extracting with ethyl acetate (20 mL x 3). The organic phases were combined, dried over anhydrous sodium sulfate, and concentrated under a reduced pressure. The resulting residues were purified by the preparative column chromatography using petroleum ether/ethyl acetate as an elution system, to obtain the title Compound **152b** (1.90 g, 4.19 mmol, yield: 93.6%) as yellow oil.
MS m/z (ESI): 454.1[M+1]
¹H NMR (400 MHz, CDCl₃) δ 7.71 - 7.64 (m, 2H), 7.45 (dd, J = 5.6, 8.3 Hz, 1H), 7.34 - 7.28 (m, 2H), 7.01 - 6.97 (m, 1H), 4.41 (t, J = 8.6 Hz, 2H), 4.16 - 4.11 (m, 1H), 4.10 (s, 3H), 4.07 (br d, J = 8.4 Hz, 2H), 3.96 - 3.87 (m, 1H), 3.40 (d, J = 15.2 Hz, 1H), 1.48 (s, 9H), 1.25 (s, 3H).

### Step 3

### 2-(6-{1-[(tert-butoxy)carbonyl]azacyclobutan-3-yl}-1-methyl-1H-indazol-4-yl)-5-fluorobenzoic acid 152c

Compound **152b** (1.9 g, 4.19 mmol) was dissolved in ethanol (12 mL), tetrahydrofuran (12 mL) and water (6.0 mL), followed by the addition of LiOH.H₂O (301.00 mg, 12.57 mmol), and the mixture was stirred at 50°Cto allow for reacting for 5.0 hours. The mixture was cooled to room temperature, followed by removing the organic solvent under a reduced pressure. The mixture was regulated to PH = 7 by adding 1M HCl, and extracted with dichloromethane (20 mL × 3). The organic phases were combined, dried over anhydrous sodium sulfate, and concentrated under a reduced pressure. The crude product of title Compound **152c** (1.62 g, 3.81 mmol, yield: 90.89%) as tan solid was obtained. The crude product was directly used in the next step of reaction without purification.
MS m/z (ESI): 426.1 [M+1]

### Step 4

### 3-(4-{2-[(2,2-difluoroethyl)(isopropyl)aminoformyl]-4-fluorophenyl}-1-methyl-1H-indazol-6-yl)azacyclobutan-1-tert-butyl carboxylate 152d

Compound **152c** (300 mg, 705.13 µmol), Compound **94b** (168.81 mg, 1.06 mmol, hydrochloride) and 2-chloro-1-methylpyridinium iodide (234.19 mg, 916.67 µmol) were dissolved in DMF(3.0 mL), followed by the addition of DIPEA(273.4 mg, 2.12 mmol). The reaction mixture was stirred at room temperature for 12 hours. Ethyl acetate (20 mL) was added to the reaction mixture, which was then washed with water (10 mL×3). The organic phase was dried over anhydrous sodium sulfate, filtered and concentrated under a reduced pressure. The resulting residues were purified by the preparative thin-layer chromatography using petroleum ether/ethyl acetate as an elution system, to obtain the title Compound **152d** (317 mg, 597.46 µmol, yield: 84.7%) as yellow solid.
MS m/z (ESI): 531.2 [M+1]

### Step 5

### 2-[6-(azacyclobutan-3-yl)-1-methyl-1H-indazol-4-yl]-N-(2,2-difluoroethyl)-5-fluoro-N-(isopropyl)benzamide 152e

Compound **152d** (110 mg, 207.32 µmol) was dissolved in dichloromethane (5.0 mL), followed by the addition of trifluoroacetic acid (1.48 g, 12.98 mmol, 1 mL) at 25°C. The mixture was stirred at room temperature to allow for reacting for 0.5 hours. The reaction mixture was concentrated under a reduced pressure, to obtain the crude product of title Compound **152e** (89 mg). The crude product was directly used in the next step of reaction without purification. MS m/z (ESI): 431.2 [M+1]

### Step 6

### N-[(3R,6S)-6-{[3-(4-{2-[(2,2-difluoroethyl)(isopropyl)aminoformyl]-4-fluorophenyl}-1-methyl-1H-indazol-6-yl)azacyclobutan-1-yl]methyl}oxacyclohexan-3-yl]tert-butyl carbamate 152f

The crude product of Compound **152e** (89 mg), *N*-[(3*R*,6*S*)-6-formyloxacyclohexan-3-yl]tert-butyl carbamate **150a** (56.88 mg, 248.10 µmol) and triethylamine (62.76 mg, 620.26 µmol) were dissolved in methanol (2.0 mL), and stirred at room temperature for 1.0 hour. Sodium cyanoborohydride (25.99 mg, 413.51 µmol) was added, and the mixture was stirred at room temperature for 1.0 hours. Water (20 mL) was added to the reaction mixture, which was then extracted with ethyl acetate (10 mL x 3). The organic phases were combined, dried over anhydrous sodium sulfate, and concentrated under a reduced pressure. The resulting residues were purified by the preparative thin-layer chromatography using dichloromethane/methanol as an elution system, to obtain the title Compound **152f** (80 mg, 124.27 µmol, yield: 60.1%) as yellow oil.
MS m/z (ESI): 644.4 [M+1]

### Step 7

### 2-[6-(1-{[(2S,5R)-5-aminooxacyclohexan-2-yl]methyl}azacyclobutan-3-yl)-1-methyl-1H-indazol-4-yl]-N-(2,2-difluoroethyl)-5-fluoro-(isopropyl)benzamide 152g

Compound **152f** (100 mg, 155.34 µmol) was dissolved in dichloromethane (5.0 mL), followed by the addition of trifluoroacetic acid (1.48 g, 12.98 mmol, 1 mL) at 25°C. The mixture was stirred at room temperature to allow for reacting for 1.0 hours. The reaction mixture was concentrated under a reduced pressure, to obtain the crude product of title Compound **152g** (84 mg). The crude product was directly used in the next step of reaction without purification. MS m/z (ESI): 544.2 [M+1]

### Step 8

### N-(2,2-difluoroethyl)-2-[6-(1-{[(2S,5R)-5-ethanesulfonamidooxacyclohexan-2-yl]methyl}azacyclobutan-3-yl)-1-methyl-1H-indazol-4-yl]-5-fluoro-N-(isopropyl)benzamide 152

The crude product of Compound **152g** (84 mg) was dissolved in dichloromethane (5.0 mL), followed by the addition of triethylamine (46.91 mg, 463.56 µmol), cooling to 0°C, and the slow dropwise addition of ethanesulfonyl chloride (23.84 mg, 185.42 µmol), and the mixture was stirred at room temperature for 13 hours. Water (20 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 33%-63%), to obtain the title Compound **152** (20.32 mg, 31.96 µmol, yield: 20.69 %) as white solid.
MS m/z (ESI): 636.4 [M+1]
¹H NMR (400 MHz, CD₃OD) δ 7.86 (s, 1H), 7.70 - 7.59 (m, 1H), 7.52 (s, 1H), 7.42 - 7.33 (m, 1H), 7.30 - 7.21 (m, 1H), 7.09 (d, J = 6.0 Hz, 1H), 5.91 - 5.54 (m, 1H), 4.09 (s, 3H), 4.04 - 3.97 (m, 1H), 3.96 - 3.84 (m, 3H), 3.70 - 3.50 (m, 2H), 3.40 - 3.33 (m, 3H), 3.29 - 3.16 (m, 2H), 3.16 - 3.09 (m, 1H), 3.05 (q, J = 7.4 Hz, 2H), 2.67 - 2.58 (m, 2H), 2.15 - 2.01 (m, 1H), 1.75 - 1.66 (m, 1H), 1.56 - 1.34 (m, 2H), 1.31 (t, J = 7.4 Hz, 3H), 0.90 (d, J = 6.4 Hz, 3H), 0.13 (d, J = 6.8 Hz, 3H).

### Example 153

### N-(2,2-difluoroethyl)-5-fluoro-2-[1-methyl-6-(1-{[(1r,4r)-4-ethanesulfonamidocyclohexyl]methyl}-azacyclobutan-3-yl)-1H-indazol-4-yl]-N-(isopropyl)benzamide 153

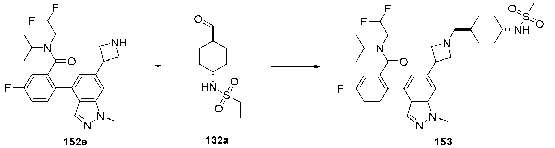

Compound **152e** (56.79 mg, 104.30 µmol, trifluoroacetate), *N*-[(1*r*,4*r*)-4-formylcyclohexyl]ethan-1-sulfonamide **132a** (25.16 mg, 114.73 µmol) and triethylamine (31.66 mg, 312.90 µmol) were dissolved in methanol (2.0 mL), and stirred at room temperature for 1.0 hours. Sodium cyanoborohydride (13.11 mg, 208.60 µmol) was added, and the mixture was stirred at room temperature for 1.0 hours. Water (50 mL) was added to the reaction mixture, which was then extracted with dichloromethane (50 mL x 3). The organic phases were combined, dried over anhydrous sodium sulfate, and concentrated under a reduced pressure. The resulting residues were purified by the preparative thin-layer chromatography using dichloromethane/tetrahydrofuran as an elution system, and the obtained crude product was purified by the HPLC (mobile phase: acetonitrile, water (0.1% ammonia water), elution gradient: 38%-68%), to obtain the title Compound **153** (9.62 mg, 15.18 µmol, yield: 14.55 %) as white solid.
MS m/z (ESI): 634.3 [M+1]
¹H NMR (400 MHz, CD₃OD) δ 7.74 (d, J = 0.8 Hz, 1H), 7.56 - 7.49 (m, 1H), 7.39 (s, 1H), 7.30 - 7.22 (m, 1H), 7.16 (dd, J = 2.6, 8.8 Hz, 1H), 6.97 (d, J = 0.8 Hz, 1H), 5.83 - 5.40 (m, 1H), 3.97 (s, 3H), 3.86 - 3.76 (m, 1H), 3.76 - 3.68 (m, 2H), 3.61 - 3.30 (m, 2H), 3.16 - 2.95 (m, 4H), 2.95 - 2.86 (m, 2H), 2.32 (d, J = 6.8 Hz, 2H), 1.94 - 1.85 (m, 2H), 1.81 - 1.70 (m, 2H), 1.34 - 1.13 (m, 7H), 1.01 - 0.86 (m, 3H), 0.78 (d, J = 6.4 Hz, 3H), 0.01 (d, J = 6.4 Hz, 2H).

### Example 154

### N-(2,2-difluoroethyl)-5-fluoro-2-[3-methyl-6-(1-{[(1r,4r)-4-ethanesulfonamidocyclohexyl]methyl}piperidin-4-yl)pyrrolo[1,2-a]pyrazin-8-yl]-N-(isopropyl)benzamide 154

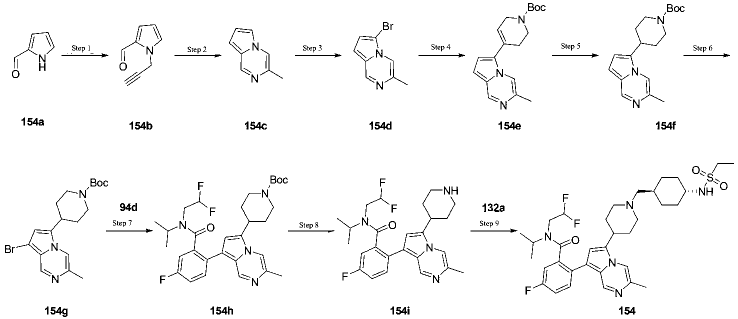

### Step 1

### 1-(prop-2-yn-1-yl)-1H-pyrrol-2-formaldehyde 154b

1*H*-pyrrol-2-formaldehyde **154a** (5.0 g, 52.58 mmol) was dissolved in DMF (40 mL), and cooled to 0 °C, followed by the slow addition of sodium hydride (2.52 g, 63.09 mmol, 60%purity), and the mixture was stirred for 0.5 hours. 3-Bromoprop-1-yne (9.38 g, 63.09 mmol) was dissolved in DMF (30 mL), and then slowly dropwise added to the above reaction mixture. The resulting reaction mixture was slowly warmed to room temperature, and stirred to allow for reacting for 12 hours. In an ice-water bath, a saturated aqueous ammonium chloride solution (50 mL) was added to the reaction mixture, which was then extracted with ethyl acetate (50 mL x 3). The organic phases were combined, washed with water (50 mL x 3), dried over anhydrous sodium sulfate, and concentrated under a reduced pressure. The resulting residues were purified by the silica-gel column chromatography using petroleum ether/ethyl acetate as an elution system, to obtain the title Compound **154b** (3.3 g, 24.78 mmol, yield: 47.14%).
MS m/z (ESI): 134.3[M+1]
¹H NMR (400 MHz, CDCl₃) δ 9.56 (d, *J* = 0.9 Hz, 1H), 7.27 (s, 1H), 6.97 (dd, *J =* 1.7, 4.1 Hz, 1H), 6.29 (dd, *J* = 2.6, 4.0 Hz, 1H), 5.21 (d, *J =* 2.5 Hz, 2H), 2.47 (t, *J =* 2.6 Hz, 1H).

### Step 2

### 3-methylpyrrolo[1,2-a]pyrazine 154c

Compound **154b** (3 g, 22.53 mmol) was dissolved in DMF (30 mL), followed by the addition of ammonium acetate (2.08 g, 27.05 mmol) and DBU (3.44 g, 22.56 mmol). The reaction mixture was stirred at80 °C for 12 hour. Water (30 mL) was added to the reaction mixture, which was then extracted with ethyl acetate (35 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **154c** (1.3 g, 9.84 mmol, yield: 43.66%) as brown solid.
MS m/z (ESI): 133.1[M+1]

### Step 3

### 6-bromo-3-methylpyrrolo[1,2-a]pyrazine 154d

Compound **154c** (1.0 g, 7.57 mmol) was dissolved in trichloromethane (25 mL), and cooled to -10°C, followed by the addition of NBS (1.22 g, 6.85 mmol). The reaction mixture was stirred at-10 °C for 1.0 hour. A saturated aqueous sodium bicarbonate solution (30 mL) was added to the reaction mixture, which was then extracted with dichloromethane (20 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **154d** (700 mg, 3.32 mmol, yield: 43.83%) as brown solid.
MS m/z (ESI): 213.0[M+1]

### Step 4

### 4-{3-methylpyrrolo[1,2-a]pyrazin-6-yl}-1,2,3,6-tetrahydropyridin-1-tert-butyl carboxylate 154e

Compound **154d** (500 mg, 2.37 mmol), 4-(tetramethyl-1,3,2-dioxaboran-2-yl)- 1,2,3,6-tetrahydropyridin-1-tert-butyl carboxylate (879.02 mg, 2.84 mmol) and sodium carbonate (753.27 mg, 7.11 mmol) were dissolved in dioxane (10 mL) and water (2 mL). Pd (dppf)Cl₂ (173.34 mg, 236.90 µmol) was added. Under a nitrogen atmosphere, the mixture was stirred at 90°Cto allow for reacting for 12 hours. The reaction mixture was cooled to room temperature, followed by adding water (30 mL) and extracting with ethyl acetate (30 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **154e** (600 mg, 1.91 mmol, yield: 80.82%).
MS m/z (ESI): 314.2 [M+1]

### Step 5

### 4-{3-methylpyrrolo[1,2-a]pyrazin-6-yl}piperidin-1-tert-butyl carboxylate 154f

Compound **154e** (600 mg, 1.91 mmol) was dissolved in tetrahydrofuran (20 mL), followed by the addition of 10% Pd/C (257.14 mg, 241.63 µmol) under a nitrogen atmosphere. The reaction atmosphere was substituted with a nitrogen atmosphere (15 psi), and the mixture was stirred at room temperature for 12.0 hours. The reaction mixture was filtered, and the filtrate was concentrated under a reduced pressure. The crude product of title Compound **154f** (590 mg, 1.87 mmol, yield: 97.7%) was obtained. The crude product was directly used in the next step of reaction without purification.
MS m/z (ESI): 316.0[M+1]

### Step 6

### 4-{8-bromo-3-methylpyrrolo[1,2-a]pyrazin-6-yl}piperidin-1-tert-butyl carboxylate 154g

Compound **154f** (590 mg, 1.87 mmol) was dissolved in dichloromethane (20 mL), followed by the addition of NBS (400 mg, 2.25 mmol), and the mixture was stirred at room temperature for 1.0 hours. A saturated aqueous sodium bicarbonate solution (30 mL) was added to the reaction mixture, which was then extracted with dichloromethane (30 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **154g** (425 mg, 1.08 mmol, yield: 57.6%) as brown solid. MS m/z (ESI): 394.1[M+1]

### Step 7

### 4-(8-{2-[(2,2-difluoroethyl)(isopropyl)aminoformyl]-4-fluorophenyl}-3-methylpyrrolo[1,2-a]pyrazin-6-yl)-piperidin-1-tert-butyl carboxylate 154h

Compound **154g** (300 mg, 760.83 µmol) and Compound **94d** (282.42 mg, 760.83 µmol) were dissolved in dioxane (5 mL) and water (1.0 mL). Potassium phosphate (484.50 mg, 2.28 mmol) and Pd(dtbpf)Cl₂ (49.59 mg, 76.08 µmol) were added, and the mixture was stirred at 95 °C under a nitrogen atmosphere to allow for reacting for 3.0 hours. The reaction mixture was cooled to room temperature, followed by adding water (20 mL) and extracting with ethyl acetate (20 mL x 3). The organic phases were combined, dried over anhydrous sodium sulfate, and concentrated under a reduced pressure. The resulting residues were purified by the silica-gel column chromatography using dichloromethane/ethyl acetate as an elution system, to obtain the title Compound **154h** (150 mg, 268.51 µmol, yield: 35.3%).
MS m/z (ESI): 559.3 [M+1]

### Step 8

### N-(2,2-difluoroethyl)-5-fluoro-2-[3-methyl-6-(piperidin-4-yl)pyrrolo[1,2-a]pyrazin-8-yl]-N-(isopropyl)benzamide 154i

Compound **154h** (130 mg, 232.71 µmol) was dissolved in dichloromethane (3 mL), followed by the addition of trifluoroacetic acid (1.48 g, 12.98 mmol, 1 mL) at 0°C. The mixture was stirred at room temperature to allow for reacting for 1.0 hours. The reaction mixture was concentrated under a reduced pressure, to obtain the crude product of title Compound **154i** (110 mg). The crude product was directly used in the next step of reaction without purification. MS m/z (ESI): 459.3 [M+1]

### Step 9

### N-(2,2-difluoroethyl)-5-fluoro-2-[3-methyl-6-(1-{[(1r,4r)-4-ethanesulfonamidocyclohexyl]methyl}piperidin-4-yl)pyrrolo[1,2-a]pyrazin-8-yl]-N-(isopropyl)benzamide 154

The crude product of Compound **154i** (110 mg), *N*-[(1*r*,4*r*)-4-formylcyclohexyl]ethan-1-sulfonamide **132a**(50.56 mg, 230.55 µmol) and triethylamine (38.88 mg, 384.25 µmol) were dissolved in methanol (2.0 mL), and stirred at room temperature for 0.5 hours. Sodium cyanoborohydride (36.22 mg, 576.38 µmol) was added, and the mixture was stirred at room temperature for 2.0 hours. A saturated aqueous sodium bicarbonate solution (30 mL) was added to the reaction mixture, which was then extracted with dichloromethane (20 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 42%-72%), to obtain the title Compound **154** (16.56 mg, 25.02 µmol, yield: 13.02 %) as white solid.
MS m/z (ESI): 662.3 [M+1]
¹H NMR (400 MHz, DMSO-*d*₆) δ 8.70 - 8.57 (m, 1H), 8.02 (s, 1H), 7.65 - 7.51 (m, 1H), 7.43 - 7.17 (m, 2H), 6.99 (d, J = 8.0 Hz, 1H), 6.65 (s, 1H), 6.12 - 5.70 (m, 1H), 3.69 - 3.50 (m, 1H), 3.48 - 3.32 (m, 2H), 3.20 - 2.85 (m, 6H), 2.34 (s, 3H), 2.18 - 1.98 (m, 4H), 1.97 - 1.71 (m, 6H), 1.69 - 1.50 (m, 2H), 1.48 - 1.33 (m, 1H), 1.30 - 0.17 (m, 13H).

### Examples 157-1 and 157-2

### N-(2,2-difluoroethyl)-2-{6-[(3S)-1-{[(2S,5R)-5-ethanesulfonamidooxacyclohexan-2-yl]methyl}pyrrolidin-3-yl]-1-methyl-1H-indazol-4-yl} -5-fluoro-N-(isopropyl)benzamide 157-1

### N-(2,2-difluoroethyl)-2-{6-[(3R)-1-{[(2S,5R)-5-ethanesulfonamidooxacyclohexan-2-yl]methyl}pyrrolidin-3-yl]-1-methyl-1H-indazol-4-yl}-5-fluoro-N-(isopropyl)benzamide 157-2

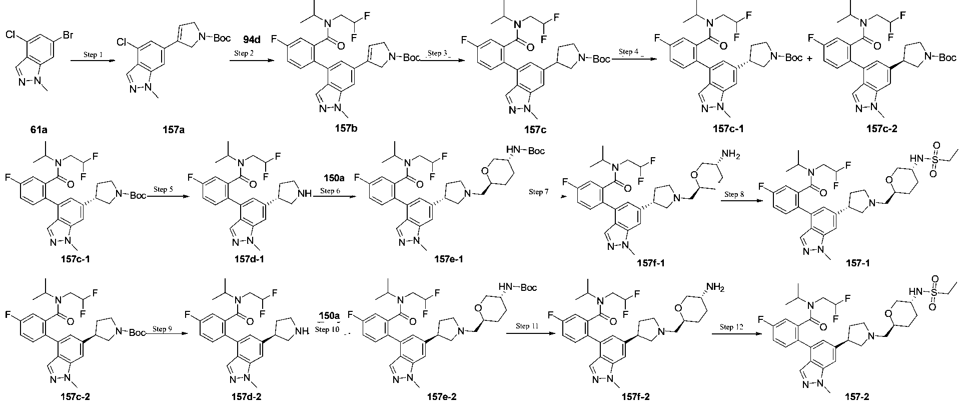

### Step 1

### 3-{4-chloro-1-methyl-1H-indazol-6-yl}-2,5-dihydro-1H-pyrrol-1-tert-butyl formate 157a

Compound **61a** (1 g, 4.07 mmol) was dissolved in dioxane (6.0 mL) and water (1.0 mL). Sodium carbonate (1.30 g, 12.22 mmol), 3-(tetramethyl-1,3,2-dioxaboran-2-yl)-2,5-dihydro-1*H*-pyrrol-1-tert-butyl formate (1.32 g, 4.48 mmol) and Pd(dppf)Cl₂(298.04 mg, 407.33 µmol) were added, and the mixture was stirred at 90°C under a nitrogen atmosphere to allow for reacting for 3.0 hours. The reaction mixture was cooled to room temperature, followed by adding water (100 mL) and extracting with ethyl acetate (50 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **157a** (1.35 g, 4.04 mmol, yield: 99.29%) as yellow solid.
MS m/z (ESI): 334.1 [M+1]

### Step 2

### 3-(4-{2-[(2,2-difluoroethyl)(isopropyl)aminoformyl]-4-fluorophenyl}-1-methyl-1H-indazol-6-yl)-2,5-dihydro-1H-pyrrol-1-tert-butyl formate 157b

Compound **157a** (500 mg, 1.50 mmol) and Compound **94d** (1.11 g, 3.00 mmol) were dissolved in dioxane (8.0 mL) and water (1.0 mL). Potassium phosphate (881.35 mg, 3.74 mmol) and Pd(dtbpf)Cl₂ (97.62 mg, 149.78 µmol) were added, and the mixture was stirred at 90°C under a nitrogen atmosphere to allow for reacting for 2.0 hours. The reaction mixture was cooled to room temperature, followed by adding water (50 mL) and extracting with ethyl acetate (20 mL x 3). The organic phases were combined, dried over anhydrous sodium sulfate, and concentrated under a reduced pressure. The resulting residues were purified by the preparative thin-layer chromatography using petroleum ether/ethyl acetate as an elution system, to obtain the title Compound **157b** (800 mg, 1.47 mmol, yield: 98.4%) as yellow oil.
MS m/z (ESI): 543.3 [M+1]

### Step 3

### 3-(4-{2-[(2,2-difluoroethyl)(isopropyl)aminoformyl]-4-fluorophenyl}-1-methyl-1H-indazol-6-yl)pyrrolidin-1-tert-butyl formate 157c

Compound **157b** (800 mg, 1.47 mmol) was dissolved in methanol (15.0 mL), followed by the addition of Pd/C (17.46 mg, 147.44 µmol). Under a hydrogen atmosphere (15 Psi), the mixture was stirred at room temperature for 26 hours. The reaction mixture was filtered, the filtrate was concentrated under a reduced pressure, and the residues were purified by the silica-gel column chromatography using petroleum ether/ethyl acetate as an elution system, to obtain the title Compound **157c** (800 mg, 1.47 mmol, yield: 99.6%).
MS m/z (ESI): 545.3 [M+1]

### Step 4

### (3S)-3-(4-{2-[(2,2-difluoroethyl)(isopropyl)aminoformyl]-4-fluorophenyl}-1-methyl-1H-indazol-6-yl)pyrrolidin-1-tert-butyl formate 157c-1

### (3R)-3-(4-{2-[(2,2-difluoroethyl)(isopropyl)aminoformyl]-4-fluorophenyl}-1-methyl-1H-indazol-6-yl)pyrrolidin-1-tert-butyl formate 157c-2

Compound **157c** (800 mg, 1.47 mmol) was separated and purified by the chiral preparative SFC (preparative column: DAICEL CHIRALPAK IK (250 mm * 30 mm, 10 um), mobile phase: 20% - 20% (v/v) carbon dioxide-methanol (0.1% ammonia water)), to obtain the title Compounds **157c-1** (110 mg, 201.98 µmol, yield: 13.75%) and **157c-2** (156 mg, 286.45 µmol, yield: 19.5%).

### Single-configuration Compound 157c-1:

MS m/z (ESI):545.3 [M+1]
SFC analysis: retention time: 1.318 min; chromatographic column: Chiralpak IK-3 50×4.6 mm I.D., 3 um; mobile phase: CO₂/MeOH (0.05% DEA): elution gradient: 5%-40%.

### Single-configuration Compound 157c-2:

MS m/z (ESI):545.3 [M+1]
SFC analysis: retention time: 1.235 min; chromatographic column: Chiralpak IK-3 50×4.6 mm I.D., 3 um; mobile phase: CO₂/MeOH (0.05% DEA): elution gradient: 5%-40%.

### Steps 5 and 9

### N-(2,2-difluoroethyl)-5-fluoro-2-{1-methyl-6-[(3S)-pyrrolidin-3-yl]-1H-indazol-5-yl}-N-(isopropyl)benzamide 157d-1

### N-(2,2-difluoroethyl)-5-fluoro-2-{1-methyl-6-[(3R)-pyrrolidin-3-yl]-1H-indazol-4-yl}-N-(isopropyl)benzamide 157d-2

Compound **157c-1/157c-2** (50 mg/50 mg, 91.81 µmol/91.81 µmol) was dissolved in methane dioxide (3.0 mL/5.0 mL), followed by the addition of trifluoroacetic acid (1.0 mL/1.0 mL). The mixture was stirred at room temperature to allow for reacting for 1.0 hours. The reaction mixture was concentrated under a reduced pressure, to obtain the crude product of title Compound **157d-1/157d-2** (40.81 mg/40.81 mg). The crude product was directly used in the next step of reaction without purification.

### Single-configuration Compound 157d-1:

MS m/z (ESI): 445.1 [M+1]

### Single-configuration Compound 157d-2:

MS m/z (ESI): 445.1 [M+1]

### Steps 6 and 10

### N-[(3R,6S)-6-{[(3S)-3-(4-{2-[(2,2-difluoroethyl)(isopropyl)aminoformyl]-4-fluorophenyl}-1-methyl-1H-indazol-6-yl)pyrrolidin-1-yl]methyl}oxacyclohexan-3-yl]tert-butyl carbamate 157e-1

### N-[(3R,6S)-6-{[(3R)-3-(4-{2-[(2,2-difluoroethyl)(isopropyl)aminoformyl]-4-fluorophenyl}-1-methyl-1H-indazol-6-yl)pyrrolidin-1-yl]methyl}oxacyclohexan-3-yl]tert-butyl carbamate 157e-2

The crude product of Compound **157d-1/157d-2** (40.81 mg/40.81 mg) was dissolved in methanol (2.0 mL /2.0 mL), followed by the addition of triethylamine (27.87 mg/27.87 mg, 275.44 µmol/275.44 µmol) and Compound **150a** (25.26 mg/25.26 mg, 110.18 µmol/110.18 µmol), and the mixture was stirred at room temperature for (1.0/1.0) hour. Sodium cyanoborohydride (11.54 mg / 11.54 mg, 183.63 µmol/183.63 µmol) was added, and the mixture was stirred at room temperature to allow for reacting for (13/12) hours. An aqueous sodium bicarbonate solution (20 mL/20 mL) was added to the reaction mixture, which was then extracted with ethyl acetate (10 mL x 3/10 mL x 3). The organic phases were combined, washed with saturated brine, dried over anhydrous sodium sulfate and filtered, and the filtrate was concentrated under a reduced pressure. The resulting residues were purified by the preparative thin-layer chromatography using dichloromethane/methanol as an elution system, to obtain the title Compound **157e-1/157e-2** (30 mg/40 mg, 45.61 µmol/60.81 µmol, yield: 49.7%/66.2%)

### Single-configuration Compound 157e-1:

MS m/z (ESI):658.3 [M+1]

### Single-configuration Compound 157e-2:

MS m/z (ESI):658.3 [M+1]

### Steps 7 and 11

### 2-{6-[(3S)-1-{[(2S,5R)-5-aminooxacyclohexan-2-yl]methyl}pyrrolidin-3-yl]-1-methyl-1H-indazol-4-yl}-N-(2,2-difluoroethyl)-5-fluoro-N-(isopropyl)benzamide 157f-1

### 2-{6-[(3R)-1-{[(2S,5R)-5-aminooxacyclohexan-2-yl]methyl}pyrrolidin-3-yl]-1-methyl-1H-indazol-4-yl}-N-(2,2-difluoroethyl)-5-fluoro-N-(isopropyl)benzamide 157f-2

Compound **157e-1/157e-2** (30 mg/40 mg, 45.61 µmol/60.81 µmol) was dissolved in methane dioxide (5.0 mL/5.0 mL), followed by the addition of trifluoroacetic acid (1.0 mL/1.0 mL). The mixture was stirred at room temperature to allow for reacting for 1.0 hours. The reaction mixture was concentrated under a reduced pressure, to obtain the crude product of title Compound **157f-1/157f-2** (25 mg/33.9 mg). The crude product was directly used in the next step of reaction without purification.

### Single-configuration Compound 157f-1:

MS m/z (ESI): 558.5 [M+1]

### Single-configuration Compound 157f-1:

MS m/z (ESI): 558.5 [M+1]

### Steps 8 and 12

### N-(2,2-difluoroethyl)-2-{6-[(3S)-1-{[(2S,5R)-5-ethanesulfonamidooxacyclohexan-2-yl]methyl}pyrrolidin-3-yl]-1-methyl-1H-indazol-4-yl}-5-fluoro-N-(isopropyl)benzamide 157-1

### N-(2,2-difluoroethyl)-2-{6-[(3R)-1-{[(2S,5R)-5-ethanesulfonamidooxacyclohexan-2-yl]methyl}pyrrolidin-3-yl]-1-methyl-1H-indazol-4-yl}-5-fluoro-N-(isopropyl)benzamide 157-2

The crude product of Compound **157f-1/157f-2** (25 mg/33.9 mg) was dissolved in dichloromethane (5.0 mL/5.0 mL), followed by the addition of ethanesulfonyl chloride (9.6 mg/12.98 mg, 74.5 µmol/100.94 µmol) and triethylamine (11.3 mg/15.32, 111.7 µmol/151.4 µmol), and the mixture was stirred at room temperature for 12 hours. Water (20 mL) was added to the reaction mixture, which was then extracted with dichloromethane (10 mL x 3). The organic phases were combined, washed with saturated brine, dried over anhydrous sodium sulfate, and concentrated under a reduced pressure. The resulting residues were purified by the preparative thin-layer chromatography using dichloromethane/methanol as an elution system/by the HPLC (mobile phase: acetonitrile, water (0.1% ammonia water), elution gradient: 0%-30%), to obtain the title Compound **157-1/157-2** (16.86 mg/11.58 mg, 25.95 µmol/17.82 µmol, yield: 69.71%/35.31%).

### Single-configuration Compound 157-1:

MS m/z (ESI):650.4 [M+1]
Chiral SFC: retention time: 2.948 min; chromatographic column: Chiralcel OX-3 50 x 4.6 mm I.D., 3 um; mobile phase: CO₂/MeOH (0.05% DEA): elution gradient: 30%.

¹H NMR (400 MHz, CD₃OD) δ 7.84 (s, 1H), 7.63 (dd, J = 5.4, 8.6 Hz, 1H), 7.53 (s, 1H), 7.38 (dt, J = 2.6, 8.6 Hz, 1H), 7.27 (dd, J = 2.6, 8.8 Hz, 1H), 7.11 (s, 1H), 5.90 - 5.53 (m, 1H), 4.08 (s, 3H), 4.06 - 3.99 (m, 1H), 3.68 - 3.53 (m, 3H), 3.52 - 3.43 (m, 1H), 3.28 - 3.22 (m, 2H), 3.20 - 3.14 (m, 1H), 3.08 - 2.98 (m, 3H), 2.89 - 2.79 (m, 1H), 2.73 - 2.61 (m, 2H), 2.44 - 2.32 (m, 1H), 2.22 - 2.07 (m, 1H), 2.05 - 1.94 (m, 1H), 1.82 - 1.73 (m, 1H), 1.55 - 1.40 (m, 2H), 1.31 (t, J = 7.2 Hz, 5H), 0.90 (d, J = 6.4 Hz, 3H), 0.14 (d, J = 6.4 Hz, 3H).

### Single-configuration Compound 157-2:

MS m/z (ESI):650.4 [M+1]
Chiral SFC: retention time: 2.154 min; chromatographic column: Chiralcel OX-3 50 x 4.6 mm I.D., 3 um; mobile phase: CO₂/MeOH (0.05% DEA): elution gradient: 30%.

¹H NMR (400 MHz, CD₃OD) δ 7.84 (s, 1H), 7.63 (t, J = 5.2 Hz, 1H), 7.52 (d, J = 3.6 Hz, 1H), 7.37 (td, J = 4.2, 8.2 Hz, 1H), 7.26 (dd, J = 2.6, 8.8 Hz, 1H), 7.11 (d, J = 3.6 Hz, 1H), 5.93 - 5.55 (m, 1H), 4.08 (s, 3H), 4.05 - 3.98 (m, 1H), 3.62 - 3.45 (m, 4H), 3.29 - 3.23 (m, 2H), 3.19 - 3.12 (m, 1H), 3.08 - 2.97 (m, 3H), 2.87 - 2.77 (m, 1H), 2.72 - 2.59 (m, 2H), 2.45 - 2.31 (m, 1H), 2.23 - 2.07 (m, 1H), 2.05 - 1.94 (m, 1H), 1.81 - 1.73 (m, 1H), 1.57 - 1.42 (m, 2H), 1.31 (t, J = 7.4 Hz, 5H), 0.90 (d, J = 6.8 Hz, 3H), 0.19 - 0.06 (m, 3H).

### Example 159

### N-[(1r,4r)-4-{[3-(4-{4-fluoro-2-[(3R)-3-methylmorpholin-4-carbonyl]phenyl}-1-methyl-1H-indazol-6-yl)azacyclobutan-1-yl]methyl}cyclohexyl]ethan-1-sulfonamide 159

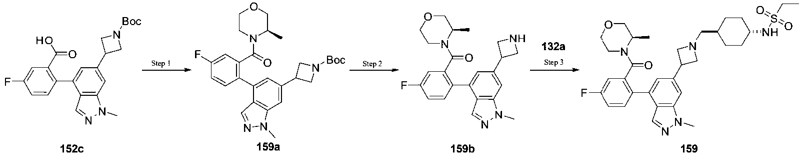

### Step 1

### 3-(4-{4-fluoro-2-[(3R)-3-methylmorpholin-4-carbonyl]phenyl}-1-methyl-1H-indazol-6-yl)azacyclobutan-1-tert-butyl carboxylate 159a

Compound **152c** (100 mg, 235.04 µmol), triethylamine (71.35 mg, 705.13 µmol) and HATU(178.74 mg, 470.09 µmol) were dissolved in dichloromethane (2.0 mL), and stirred at room temperature for 10 min, followed by the addition of (3R)-3-methylmorpholine (26.15 mg, 258.55 µmol). The reaction mixture was stirred at 50°C for 2 hours. A saturated aqueous sodium bicarbonate solution (20 mL) was added to the reaction mixture, which was then extracted with dichloromethane (20 mL × 3). The organic phase was dried over anhydrous sodium sulfate, filtered and concentrated under a reduced pressure. The resulting residues were purified by the preparative thin-layer chromatography using dichloromethane/ethyl acetate as an elution system, to obtain the title Compound **159a** (100 mg, 196.62 µmol, yield: 63.7%).
MS m/z (ESI): 453.2 [M-55]

### Step 2

### 6-(azacyclobutan-3-yl)-4-{4-fluoro-2-[(3R)-3-methylmorpholin-4-carbonyl]phenyl}-1-methyl-1H-indazole 159b

Compound **159a** (60 mg, 117.97 µmol) was dissolved in dichloromethane (3.4 mL), followed by the addition of trifluoroacetic acid (888.00 mg, 7.79 mmol, 600.00 µL) at 25°C. The mixture was stirred at room temperature to allow for reacting for 1.0 hours. The reaction mixture was concentrated under a reduced pressure, to obtain the crude product of title Compound **159b** (55 mg). The crude product was directly used in the next step of reaction without purification.
MS m/z (ESI): 409.3 [M+1]

### Step 3

### N-[(1r,4r)-4-{[3-(4-{4-fluoro-2-[(3R)-3-methylmorpholin-4-carbonylphenyl}-1-methyl-1H-indazol-6-yl)azacyclobutan-1-yl]methyl}cyclohexyl]ethan-1-sulfonamide 159

Compound **159b** (50 mg, 22.41 µmol), *N*-[(1*r*,4*r*)-4-formylcyclohexyl]ethan-1-sulfonamide **132a** (32.21 mg, 146.89 µmol) and triethylamine (37.16 mg, 367.23 µmol) were dissolved in methanol (1.5 mL), and stirred at room temperature for 0.5 hours. Sodium cyanoborohydride (23.85 mg, 379.47 µmol) was added, and the mixture was stirred at room temperature for 2.0 hours. A saturated aqueous sodium bicarbonate solution (5 mL) was added to the reaction mixture, which was then extracted with dichloromethane (5 mL x 3). The organic phases were combined, dried over anhydrous sodium sulfate, and concentrated under a reduced pressure. The resulting residues were purified by the preparative thin-layer chromatography using petroleum ether/ethyl acetate/ethanol as an elution system, and the obtained crude product was purified by the HPLC (mobile phase: acetonitrile, water (0.1% ammonia water), elution gradient: 24%-54%), to obtain the title Compound **159** (9.45 mg, 15.45 µmol, yield: 12.62 %) as white solid. MS m/z (ESI): 612.2 [M+1]
¹H NMR (400 MHz, DMSO-*d*₆) δ 7.88 - 7.74 (m, 1H), 7.71 - 7.54 (m, 2H), 7.50 - 7.25 (m, 2H), 7.17 - 6.93 (m, 2H), 4.34 - 4.02 (m, 4H), 3.87 - 3.35 (m, 6H), 3.19 - 3.04 (m, 3H), 3.01 - 2.91 (m, 3H), 2.89 - 2.72 (m, 1H), 2.46 - 2.17 (m, 3H), 1.91 - 1.70 (m, 4H), 1.25 - 1.11 (m, 7H), 1.03 - 0.86 (m, 3H), 0.18 (s, 1H).

### Example 161

### N-(2,2-difluoroethyl)-5-fluoro-2-{1-methyl-6-[(1-{[(1r,4r)-4-ethanesulfonamidocyclohexyl]methyl}azacyclobutan-3-yl)methyl]-1H-indazol-4-yl}-N-(isopropyl)benzamide 161

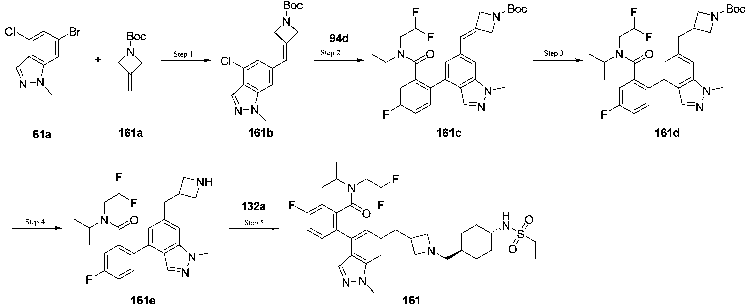

### Step 1

### 3-[(4-chloro-1-methyl-1H-indazol-6-yl)methylene]azacyclobutan-1-tert-butyl formate 161b

Compound **61a** (500 mg, 2.04 mmol), 3-methyleneazacyclobutan-1-tert-butyl formate **161a** (448.03 mg, 2.65 mmol) and tetrabutylammonium acetate (1.23 g, 4.07 mmol) were dissolved in dioxane (9.0 mL). Palladium acetate (91.45 mg, 407.33 µmol) and Xphos (149.02 mg, 203.66 µmol) were added, and the mixture was stirred at 80°C under a nitrogen atmosphere to allow for reacting for 12.0 hours. The reaction mixture was cooled to room temperature, followed by adding water (20 mL) and extracting with ethyl acetate (20 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **161b** (465 mg, 1.39 mmol, yield: 68.40%) as yellow oil.
MS m/z (ESI): 334.1 [M+1]

### Step 2

### 3-[(4-{2-[(2,2-difluoroethyl)(isopropyl)aminoformyl]-4-fluorophenyl}-1-methyl-1H-indazol-6-yl)methylene]azacyclobutan-1-tert-butyl formate 161c

Compound **161b** (250 mg, 748.92 µmol) and Compound **94d** (333.60 mg, 898.71 µmol) were dissolved in dioxane (6.0 mL) and water (1.0 mL). Potassium phosphate (476.91 mg, 2.25 mmol) and Pd(dtbpf)Cl₂ (48.81 mg, 74.89 µmol) were added, and the mixture was stirred at 95°C under a nitrogen atmosphere to allow for reacting for 12.0 hours. The reaction mixture was cooled to room temperature, followed by adding water (20 mL) and extracting with ethyl acetate (20 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **161c** (290 mg, 534.47 µmol, yield: 71.37%) as yellow solid.
MS m/z (ESI): 543.2 [M+1]

### Step 3

### 3-[(4-{2-[(2,2-difluoroethyl)(isopropyl)aminoformyl]-4-fluorophenyl}-1-methyl-1H-indazol-6-yl)methyl]azacyclobutan-1-tert-butyl formate 161d

Compound **161c** (290 mg, 534.47 µmol) was dissolved in tetrahydrofuran (10 mL), followed by the addition of 10% Pd/C (304.87 mg, 286.48 µmol). Under a hydrogen atmosphere (15 Psi), the mixture was stirred at 25°C for 2 hours. The reaction mixture was filtered, and the filtrate was concentrated under a reduced pressure, to obtain the title Compound **161d** (240 mg, 440.68 µmol, yield: 82.45%).
MS m/z (ESI): 545.2 [M+1]

### Step 4

### 2-{6-[(azacyclobutan-3-yl)methyl]-1-methyl-1H-indazol-4-yl}-N-(2,2-difluoroethyl)-5-fluoro-N-(isopropyl)benzamide 161e

Compound **161d** (120 mg, 220.34 µmol) was dissolved in methane dioxide (2.5 mL), followed by the addition of trifluoroacetic acid (25.12 mg, 220.34 µmol, 16.98 µL). The mixture was stirred at room temperature to allow for reacting for 1.0 hours. The reaction mixture was concentrated under a reduced pressure, to obtain the crude product of title Compound **161e** (95 mg, 213.73 µmol). The crude product was directly used in the next step of reaction without purification.
MS m/z (ESI): 445.2 [M+1]

### Step 5

### N-(2,2-difluoroethyl)-5-fluoro-2-{1-methyl-6-[(1-{[(1r,4r)-4-ethanesulfonamidocyclohexyl]methyl}azacyclobutan-3-yl)methyl]-1H-indazol-4-yl}-N-(isopropyl)benzamide 161

The crude product of Compound **165e** (123.06 mg, 220.34 µmol, trifluoroacetate) was dissolved in methanol (2.0 mL), followed by the addition of triethylamine (66.89 mg, 661.02 µmol) and Compound **132a**(57.98 mg, 264.41 µmol), and the mixture was stirred at room temperature for 1.0 hour. Sodium cyanoborohydride (41.96 mg, 667.63 µmol) was added, and the mixture was stirred at room temperature to allow for reacting for 11 hours. A saturated aqueous sodium bicarbonate solution (10 mL) was added to the reaction mixture, which was then extracted with dichloromethane (10 mL x 3). The organic phases were combined, dried over anhydrous sodium sulfate and filtered, and the filtrate was concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% ammonia water), elution gradient: 37%-67%), to obtain the title Compound **161** (21.95 mg, 33.88 µmol, yield: 15.38%) as white solid.
MS m/z (ESI):648.4 [M+1]
¹H NMR (400 MHz, DMSO-*d₆*) δ 7.80 - 7.74 (m, 1H), 7.64 - 7.55 (m, 1H), 7.47 - 7.34 (m, 3H), 6.97 (d, J = 7.6 Hz, 1H), 6.85 (s, 1H), 5.93 - 5.56 (m, 1H), 4.07 - 3.97 (m, 3H), 3.62 - 3.38 (m, 2H), 3.29 - 3.21 (m, 3H), 2.99 - 2.90 (m, 5H), 2.82 - 2.74 (m, 2H), 2.70 - 2.61 (m, 1H), 2.20 - 2.12 (m, 2H), 1.87 - 1.77 (m, 2H), 1.74 - 1.66 (m, 2H), 1.23 - 1.09 (m, 6H), 0.94 - 0.81 (m, 5H), 0.14 - 0.03 (m, 3H).

### Example 163

### N-(2,2-difluoroethyl)-2-[6-(1-{[(2S,5R)-5-ethanesulfonamidooxacyclohexan-2-yl]methyl}pyrrolidin-3-yl)-3-methylimidazo[1,5-a]pyridin-8-yl]-5-fluoro-N-(isopropyl)benzamide 163

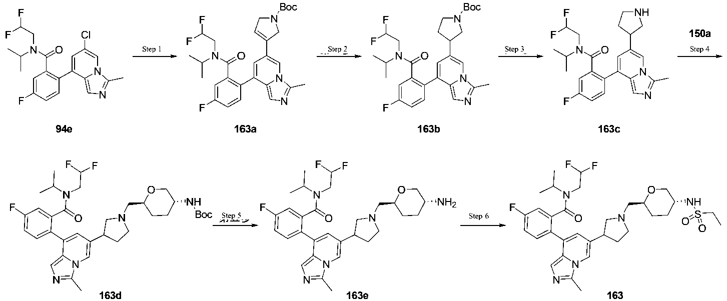

### Step 1

### 3-(8-{2-[(2,2-difluoroethyl)(isopropyl)aminoformyl]-4-fluorophenyl}-3-methylimidazo[1,5-a]pyridin-6-yl)-2,5-dihydro-1H-pyrrol-1-tert-butyl formate 163a

Compound **94e** (500 mg, 1.22 mmol) was dissolved in dioxane (8.0 mL) and water (2.0 mL). Potassium phosphate (766.9 mg, 3.66 mmol), 3-(tetramethyl-1,3,2-dioxaboran-2-yl)-2,5-dihydro-1*H*-pyrrol-1-tert-butyl formate (540.19 mg, 1.83 mmol) and Pd(dtbpf)Cl₂ (79.51 mg, 122.0 µmol) were added, and the mixture was stirred at 95°C under a nitrogen atmosphere to allow for reacting for 12 hours. The reaction mixture was cooled to room temperature, followed by adding water (30 mL) and extracting with ethyl acetate (30 mL x 3). The organic phases were combined, dried over anhydrous sodium sulfate, and concentrated under a reduced pressure. The resulting residues were purified by the silica-gel column chromatography using dichloromethane/ethyl acetate as an elution system, to obtain the title Compound **163a** (400 mg, 737.2 µmol, yield: 60.43%) as brown solid.
MS m/z (ESI): 543.4 [M+1]

### Step 2

### 3-(8-{2-[(2,2-difluoroethyl)(isopropyl)aminoformyl]-4-fluorophenyl}-3-methylimidazo[1,5-a]pyridin-6-yl)pyrrolidin-1-tert-butyl formate 163b

Compound **163a** (400 mg, 737.2 µmol) was dissolved in tetrahydrofuran (10 mL), followed by the addition of 10% Pd/C (100 mg, 939.67 µmol). Under a hydrogen atmosphere (15 Psi), the mixture was stirred at room temperature for 12 hours. The reaction mixture was filtered, the filtrate was concentrated under a reduced pressure, and the residues were purified by the preparative thin-layer chromatography using petroleum ether/ethyl acetate as an elution system, to obtain the title Compound **163b** (100 mg, 183.62 µmol, yield: 29.4%).
MS m/z (ESI): 545.4 [M+1]

### Step 3

### N-(2,2-difluoroethyl)-5-fluoro-2-[3-methyl-6-(pyrrolidin-3-yl)imidazo[1,5-a]pyridin-8-yl]-N-(isopropyl)benzamide 163c

Compound **163b** (100 mg, 183.62 µmol) was dissolved in methane dioxide (3.0 mL), followed by the addition of trifluoroacetic acid (1.0 mL). The mixture was stirred at room temperature to allow for reacting for 1.0 hours. The reaction mixture was concentrated under a reduced pressure, to obtain the crude product of title Compound **163c** (102 mg). The crude product was directly used in the next step of reaction without purification.
MS m/z (ESI): 445.4 [M+1]

### Step 4

### N-[(3R,6S)-6-{[3-(8-{2-[(2,2-difluoroethyl)(isopropyl)aminoformyl]-4-fluorophenyl}-3-methylimidazo[1,5-a]pyridin-6-yl)pyrrolidin-1-yl]methyl}oxacyclohexan-3-yl]tert-butyl carbamate 163d

The crude product of Compound **163c** (102 mg) was dissolved in methanol (2.0 mL), followed by the addition of triethylamine (37.04 mg, 366.00 µmol) and Compound **150a**(50 mg, 218.08 µmol), and the mixture was stirred at room temperature for 0.5 hours. Sodium cyanoborohydride (34.50 mg, 549.00 µmol) was added, and the mixture was stirred at room temperature to allow for reacting for 2.0 hours. A saturated aqueous sodium bicarbonate solution (30 mL) was added to the reaction mixture, which was then extracted with dichloromethane (20 mL x 3). The organic phases were combined, dried over anhydrous sodium sulfate and filtered, and the filtrate was concentrated under a reduced pressure. The resulting residues were purified by the preparative thin-layer chromatography using ethyl acetate/ethanol as an elution system, to obtain the title Compound **163d** (100 mg, 152.03 µmol, yield: 83.08 %) as yellow solid.
MS m/z (ESI):658.4 [M+1]

### Step 5

### 2-[6-(1-{[(2S,5R)-5-aminooxacyclohexan-2-yl]methyl}pyrrolidin-3-yl)-3-methylimidazo[1,5-a]pyridin-8-yl]-N-(2,2-difluoroethyl)-5-fluoro-N-(isopropyl)benzamide 163e

Compound **163d** (100 mg, 152.03 µmol) was dissolved in methane dioxide (3.0 mL), followed by the addition of trifluoroacetic acid (1.0 mL). The mixture was stirred at room temperature to allow for reacting for 1.0 hours. The reaction mixture was concentrated under a reduced pressure, to obtain the crude product of title Compound **163e** (100 mg, 179.32 µmol, yield: 117.95%). The crude product was directly used in the next step of reaction without purification.
MS m/z (ESI): 558.2 [M+1]

### Step 6

### N-(2,2-difluoroethyl)-2-[6-(1-{[(2S,5R)-5-ethanesulfonamidooxacyclohexan-2-yl]methyl}pyrrolidin-3-yl)-3-methylimidazo[1,5-a]pyridin-8-yl]-5-fluoro-N-(isopropyl)benzamide 163

The crude product of Compound **163e** (90 mg, 133.99 µmol, trifluoroacetate) was dissolved in dichloromethane (3.0 mL), followed by the addition of ethanesulfonyl chloride (34.46 mg, 267.99 µmol) and triethylamine (67.79 mg, 669.97 µmol), and the mixture was stirred at room temperature for 1.0 hour. A saturated aqueous sodium bicarbonate solution (30 mL) was added to the reaction mixture, which was then extracted with dichloromethane (20 mL x 3). The organic phases were combined, dried over anhydrous sodium sulfate, and concentrated under a reduced pressure. The resulting residues were purified by the HPLC (mobile phase: acetonitrile, water (0.1% ammonium bicarbonate), elution gradient: 32%-62%), to obtain the title Compound **163** (41.27 mg, 63.52 µmol, yield: 47.4%) as white solid. MS m/z (ESI):650.3 [M+1]
¹H NMR (400 MHz, CD₃OD) δ 7.91 (s, 1H), 7.73 - 7.64 (m, 1H), 7.42 - 7.33 (m, 1H), 7.31 - 7.22 (m, 1H), 7.10 (s, 1H), 6.77 (s, 1H), 6.01 - 5.57 (m, 1H), 4.07 - 3.94 (m, 1H), 3.74 - 3.57 (m, 2H), 3.51 - 3.35 (m, 2H), 3.29 - 3.00 (m, 6H), 2.98 - 2.87 (m, 1H), 2.86 - 2.71 (m, 1H), 2.68 - 2.54 (m, 6H), 2.37 - 2.22 (m, 1H), 2.16 - 2.04 (m, 1H), 1.97 - 1.70 (m, 2H), 1.59 - 1.37 (m, 2H), 1.31 (t, J = 7.2 Hz, 3H), 0.99 - 0.88 (m, 3H), 0.47 - 0.26 (m, 3H).

### Examples 163-1 and 163-2

### N-(2,2-difluoroethyl)-2-{6-[(3S)-1-{[(2S,5R)-5-ethanesulfonamidooxacyclohexan-2-yl]methyl}pyrrolidin-3-yl]- 3-methylimidazo[1,5-a]pyridin-8-yl}-5-fluoro-N-(isopropyl)benzamide 163-1

### N-(2,2-difluoroethyl)-2-{6-[(3R)-1-{[(2S,5R)-5-ethanesulfonamidooxacyclohexan-2-yl]methyl}pyrrolidin-3-yl]- 3-methylimidazo[1,5-a]pyridin-8-yl}-5-fluoro-N-(isopropyl)benzamide 163-2

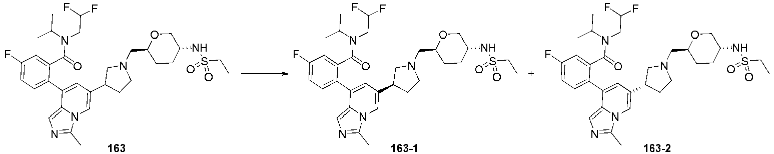

Compound **163** (40 mg, 61.56 µmol) was separated and purified by the chiral preparative SFC (preparative column: DAICEL CHIRALPAK IC (250 mm * 30 mm, 10 um), mobile phase: carbon dioxide/[acetonitrile/isopropanol (0.1% ammonia water)], elution gradient: 55%), to obtain the title Compounds **163-1** (5.62 mg, 8.65 µmol, yield: 14.05%) and **163-2** (5.6 mg, 8.62 µmol, yield: 14.0%).

### Single-configuration Compound 163-1:

MS m/z (ESI):649.5 [M+1]
SFC analysis: retention time: 1.708 min; chromatographic column: Chiralpak IC-3 50×4.6 mm I.D., 3 um; mobile phase: CO₂/[IPA+ACN (0.05% DEA)]: elution gradient: 40%.

¹H NMR (400 MHz, CD₃OD) δ 7.90 (s, 1H), 7.73 - 7.66 (m, 1H), 7.41 - 7.34 (m, 1H), 7.27 (dd, J = 2.4, 8.4 Hz, 1H), 7.10 (s, 1H), 6.76 (s, 1H), 6.02 - 5.61 (m, 1H), 4.07 - 3.97 (m, 1H), 3.72 - 3.58 (m, 2H), 3.52 - 3.37 (m, 2H), 3.28 - 2.99 (m, 6H), 2.98 - 2.89 (m, 1H), 2.84 - 2.71 (m, 1H), 2.68 - 2.55 (m, 6H), 2.37 - 2.25 (m, 1H), 2.16 - 2.06 (m, 1H), 1.96 - 1.85 (m, 1H), 1.80 - 1.72 (m, 1H), 1.54 - 1.46 (m, 1H), 1.33 - 1.30 (m, 4H), 0.95 (d, J = 6.4 Hz, 3H), 0.36 (t, J = 6.8 Hz, 3H).

### Single-configuration Compound 163-2:

MS m/z (ESI):649.5 [M+1]
SFC analysis: retention time: 1.08 min; chromatographic column: Chiralpak IC-3 50×4.6 mm I.D., 3 um; mobile phase: CO₂/[IPA+ACN (0.05% DEA)]: elution gradient: 40%.

¹H NMR (400 MHz, CD₃OD) δ 7.91 (s, 1H), 7.72 - 7.65 (m, 1H), 7.41 - 7.33 (m, 1H), 7.31 - 7.25 (m, 1H), 7.10 (s, 1H), 6.79 - 6.71 (m, 1H), 5.99 - 5.56 (m, 1H), 4.08 - 3.95 (m, 1H), 3.73 - 3.58 (m, 2H), 3.53 - 3.35 (m, 2H), 3.29 - 3.00 (m, 6H), 2.97 - 2.87 (m, 1H), 2.85 - 2.72 (m, 1H), 2.67 - 2.57 (m, 6H), 2.38 - 2.24 (m, 1H), 2.16 - 2.05 (m, 1H), 1.97 - 1.71 (m, 2H), 1.55 - 1.45 (m, 1H), 1.34 - 1.28 (m, 4H), 0.95 (d, J = 6.8 Hz, 3H), 0.48 - 0.26 (m, 3H).

### Example 164

### N-(2,2-difluoroethyl)-5-fluoro-2-{3-methyl-6-[(3S)-1-{[(1r,4r)-4-ethanesulfonamidocyclohexyl]methyl}pyrrolidin-3-yl]imidazo[1,5-a]pyridin-8-yl]-N-(isopropyl)benzamide 164

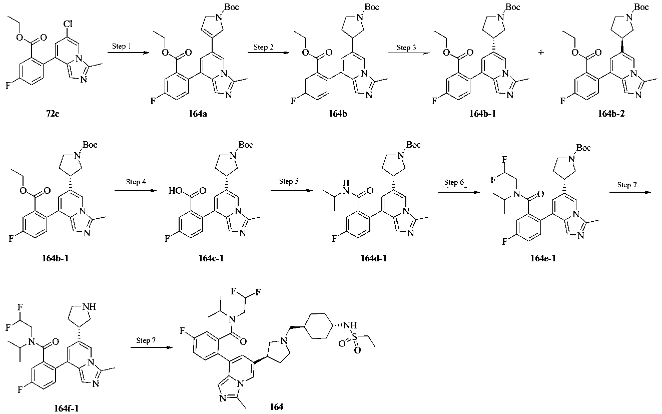

### Step 1

### 3-{8-[2-(ethoxycarbonyl)-4-fluorophenyl]-3-methylimidazo[1,5-a]pyridin-6-yl}-2,5-dihydro-1H-pyrrol-1-tert-butyl formate 164a

Compound **72c** (2.35 g, 7.06 mmol) was dissolved in dioxane (25 mL) and water (5.0 mL). Potassium phosphate (4.50 g, 21.19 mmol), 3-(tetramethyl-1,3,2-dioxaboran-2-yl)-2,5-dihydro-1*H*-pyrrol-1-tert-butyl formate (3.13 g, 10.59 mmol) and Pd(dtbpf)Cl₂ (460.28 mg, 706.22 µmol) were added, and the mixture was stirred at 95°C under a nitrogen atmosphere to allow for reacting for 12 hours. The reaction mixture was cooled to room temperature, followed by adding water (30 mL) and extracting with ethyl acetate (30 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **164a** (2.7 g, 5.80 mmol, yield: 82.13%) as brown solid.
MS m/z (ESI): 466.3 [M+1]

### Step 2

### 3-{8-[2-(ethoxycarbonyl)-4-fluorophenyl]-3-methylimidazo[1,5-a]pyridin-6-yl}-pyrrolidin-1-tert-butyl formate 164b

Compound **164a** (2.7 g, 5.80 mmol) was dissolved in tetrahydrofuran (30.0 mL), followed by the addition of 10% Pd/C (500 mg, 469.84 µmol). Under a hydrogen atmosphere (15 Psi), the mixture was stirred at 40°C to allow for reacting for 8.0 hour. The reaction mixture was filtered, the filtrate was concentrated under a reduced pressure, and the residues were purified by the HPLC (mobile phase: acetonitrile, water (0.1% formic acid), elution gradient: 38%-58%), to obtain the title Compound **164b** (1.0 g, 2.14 mmol, yield: 36.88%).
MS m/z (ESI): 468.2 [M+1]

### Step 3

### (3S)-3-{8-[2-(ethoxycarbonyl)-4-fluorophenyl]-3-methylimidazo[1,5-a]pyridin-6-yl}-pyrrolidin-1-tert-butyl formate 164b-1

### (3R)-3-{8-[2-(ethoxycarbonyl)-4-fluorophenyl]-3-methylimidazo[1,5-a]pyridin-6-yl}-pyrrolidin-1-tert-butyl formate 164b-1

Compound **164b** (1.0 g, 2.14 mmol) was separated and purified by the chiral preparative SFC (preparative column: DAICEL CHIRALPAKAD (250 mm * 30 mm, 10 um), mobile phase: 5%-25% (v/v) carbon dioxide-ethanol (0.05% diethylamide)), to obtain the title Compounds **164b-1** (325 mg, 695.14 µmol, yield: 32.5%) and **164b-2** (233 mg, 498.36 µmol, yield: 23.0%).

### Single-configuration Compound 164b-1:

MS m/z (ESI):468.2 [M+1]
SFC analysis: retention time: 1.409 min; chromatographic column: Chiralpak AD-3 50×4.6 mm I.D., 3 um; mobile phase: CO₂/EtOH (0.05% DEA): elution gradient: 5%-25%.

### Single-configuration Compound 164b-2:

MS m/z (ESI):468.2 [M+1]
SFC analysis: retention time: 1.547 min; chromatographic column: Chiralpak AD-3 50×4.6 mm I.D., 3 um; mobile phase: CO₂/EtOH (0.05% DEA): elution gradient: 5%-25%.

### Step 4

### 2-{6-[(3S)-1-[(tert-butoxy)carbonyl]pyrrolidin-3-yl]-3-methylimidazo[1,5-a]pyridin-8-yl}-5-fluorobenzoic acid 164c-1

Compound **164b-1**(325 mg, 695.14 µmol) was dissolved in ethanol (1.0 mL), tetrahydrofuran (1.0 mL) and water (1.0 mL), followed by the addition of LiOH.H₂O (87.50 mg, 2.09 mmol), and the mixture was stirred at 25°C to allow for reacting for 12 hours. The reaction mixture was regulated to PH = 3 with 1M HCl, and extracted with trichloromethane/isopropanol (5/1; 20 mL×3). The organic phases were combined, dried over anhydrous sodium sulfate, and concentrated under a reduced pressure. The crude product of title Compound **164c-1** (300 mg, 682.63 µmol, yield: 98.2%) was obtained as brown solid. The crude product was directly used in the next step of reaction without purification.
MS m/z (ESI): 440.4 [M+1]

### Step 5

### (3S)-3-(8-{4-fluoro-2-[(isopropyl)aminoformyl]phenyl}-3-methylimidazo[1,5-a]pyridin-6-yl)pyrrolidin-1-tert-butyl formate 164d-1

Compound **164c-1** (250 mg, 568.86 µmol) was dissolved in dichloromethane (4.0 mL), followed by the addition of prop-2-amine (40.35 mg, 682.63 µmol) and triethylamine (172.69 mg, 1.71 mmol). In an ice bath, HATU (259.56 mg, 682.63 µmol) was slowly added, and the mixture was stirred at room temperature for 2.0 hours. A saturated aqueous sodium carbonate solution (20 mL) was added to the reaction mixture, which was then extracted with dichloromethane (20 mL × 3). The organic phase was dried over anhydrous sodium sulfate, filtered and concentrated under a reduced pressure. The resulting residues were purified by the preparative thin-layer chromatography using petroleum ether/ethyl acetate/ethanol as an elution system, to obtain the title Compound **164d-1** (190 mg, 395.36 µmol, yield: 69.50%) as yellow solid.
MS m/z (ESI): 481.4 [M+1]

### Step 6

### (3S)-3-(8-{2-[(2,2-difluoroethyl)(isopropyl)aminoformyl]-4-fluorophenyl}-3-methylimidazo[1,5-a]pyridin-6-yl)pyrrolidin-1-tert-butyl formate 164e-1

Compound **164d-1** (170 mg, 353.74 µmol) was dissolved in DMF(0.5 mL), followed by the addition of sodium hydride (30 mg, 750.07 µmol, 60%purity). The reaction mixture was stirred at 0°C for 0.5 hours, followed by the addition of 2,2-difluoroethyltrifluoromethanesulfonate (98.46 mg, 459.87 µmol), and the mixture was stirred at room temperature to allow for reacting for 12 hours. A saturated aqueous ammonium chloride solution (15 mL) was added to the reaction mixture, which was then extracted with ethyl acetate (20 mL×3). The organic phase was dried over anhydrous sodium sulfate, filtered and concentrated under a reduced pressure. The resulting residues were purified by the preparative thin-layer chromatography using petroleum ether/ethyl acetate/ethanol as an elution system, to obtain the title Compound **164e-1** (35 mg, 64.27 µmol, yield: 18.17%) as yellow solid.
MS m/z (ESI): 545.5 [M+1]

### Step 7

### N-(2,2-difluoroethyl)-5-fluoro-2-{3-methyl-6-[(3S)-pyrrolidin-3-yl]imidazo[1,5-a]pyridin-8-yl}-N-(isopropyl)benzamide 164f-1

Compound **164e-1** (40 mg, 73.45 µmol) was dissolved in dichloromethane (3.0 mL), followed by the addition of trifluoroacetic acid (1.48 g, 12.98 mmol, 1 mL) at 25°C. The mixture was stirred at room temperature to allow for reacting for 1.0 hours. The reaction mixture was concentrated under a reduced pressure, to obtain the crude product of title Compound **164f-1** (40.77 mg). The crude product was directly used in the next step of reaction without purification.
MS m/z (ESI): 445.2 [M+1]

### Step 8

### N-(2,2-difluoroethyl)-5-fluoro-2-{3-methyl-6-[(3S)-1-{[(1r,4r)-4-ethanesulfonamidocyclohexyl]methyl}pyrrolidin-3-yl]imidazo[1,5-a]pyridin-8-yl]-N-(isopropyl)benzamide 164

The crude product of Compound **164f-1** (40.77 mg), *N*-[(1*r*,4*r*)-4-formylcyclohexyl]ethan-1-sulfonamide **132a**(20 mg, 91.20 µmol) and triethylamine (14.77 mg, 146.00 µmol) was dissolved in methanol (1.0 mL), and the mixture was stirred at room temperature for 0.5 hours. Sodium cyanoborohydride (13.76 mg, 219.00 µmol) was added, and the mixture was stirred at room temperature for 2.0 hours. A saturated aqueous sodium bicarbonate solution (10 mL) was added to the reaction mixture, which was then extracted with dichloromethane (15 mL x 3). The organic phases were combined, dried over anhydrous sodium sulfate, and concentrated under a reduced pressure. The resulting residues were purified by the preparative thin-layer chromatography using dichloromethane/methanol as an elution system, to obtain the title Compound **164** (6.46 mg, 9.97 µmol, yield: 13.66 %) as white solid.
MS m/z (ESI): 648.2 [M+1]
¹H NMR (400 MHz, DMSO-*d*₆) δ 8.06 (s, 0.12H), 7.94 (s, 1H), 7.71 - 7.61 (m, 1H), 7.48 - 7.35 (m, 2H), 6.99 (s, 2H), 6.63 (m, 1H), 5.91 - 5.52 (m, 1H), 3.69 - 3.43 (m, 3H), 3.17 - 2.90 (m, 4H), 2.84 - 2.55 (m, 6H), 2.36 - 2.13 (m, 3H), 1.91 - 1.75 (m, 5H), 1.53 - 1.43 (m, 1H), 1.34 (m, 1H), 1.18 (m, 4H), 0.93 - 0.80 (m, 6H), 0.32 (m, 3H).

### Example 165

### N-(2,2-difluoroethyl)-5-fluoro-2-{1-methyl-6-[(1-{[(1r,4r)-4-ethanesulfonamidocyclohexyl]methyl}pyrrolidin-3-yl)methyl]-1H-indazol-4-yl}-N-(isopropyl)benzamide 165

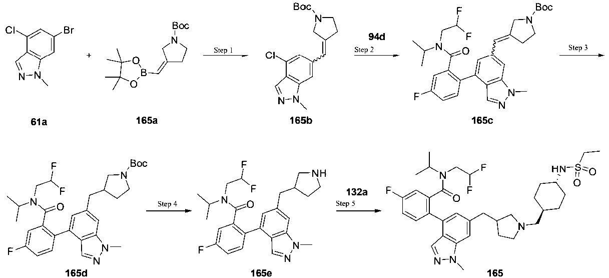

### Step 1

### 3-[(4-chloro-1-methyl-1H-indazol-6-yl)methylene]pyrrolidin-1-tert-butyl formate 165b

Compound **61a** (500 mg, 2.04 mmol) was dissolved in dioxane (6.0 mL) and water (1.0 mL). Sodium carbonate (647.59 mg, 6.11 mmol), 3-[(tetramethyl-1,3,2-dioxaboran-2-yl)methylene]-pyrrolidin-1-tert-butyl formate (692.72 mg, 2.24 mmol) and Pd(dppf)Cl₂ (149.02 mg, 203.66 µmol) were added, and the mixture was stirred at 90°C under a nitrogen atmosphere to allow for reacting for 2.0 hours. The reaction mixture was cooled to room temperature, followed by adding water (100 mL) and extracting with ethyl acetate (50 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **165b** (700 mg, 2.01 mmol, yield: 98.81%) as yellow solid.
MS m/z (ESI): 348.1 [M+1]

### Step 2

### 3-[(4-{2-[(2,2-difluoroethyl)(isopropyl)aminoformyl]-4-fluorophenyl}-1-methyl-1H-indazol-6-yl)methylene]pyrrolidin-1-tert-butyl formate 165c

Compound **165b** (300 mg, 862.47 µmol) and Compound **94d** (384.18 mg, 1.03 mmol) were dissolved in dioxane (8.0 mL) and water (1.0 mL). Potassium phosphate (507.49 mg, 2.39 mmol) and Pd(dtbpf)Cl₂ (56.21 mg, 86.25 µmol) were added, and the mixture was stirred at 90°C under a nitrogen atmosphere to allow for reacting for 2.0 hours. The reaction mixture was cooled to room temperature, followed by adding water (50 mL) and extracting with ethyl acetate (20 mL x 3). The organic phases were combined, dried over anhydrous sodium sulfate, and concentrated under a reduced pressure. The resulting residues were purified by the preparative thin-layer chromatography using petroleum ether/ethyl acetate as an elution system, to obtain the title Compound **165c** (400 mg, 718.63 µmol, yield: 83.32%) as yellow oil.
MS m/z (ESI): 557.5 [M+1]
¹H NMR (400 MHz, CDCl₃) δ 7.74 (s, 1H), 7.47 (td, J = 5.6, 8.6 Hz, 1H), 7.07 - 7.02 (m, 2H), 6.94 - 6.84 (m, 1H), 6.50 - 6.34 (m, 1H), 5.86 - 5.44 (m, 1H), 3.98 (d, J = 5.4 Hz, 3H), 3.60 - 3.45 (m, 2H), 3.40 (dt, J = 6.4, 12.8 Hz, 2H), 2.96 - 2.75 (m, 2H), 2.70 (br t, J = 7.0 Hz, 1H), 1.13 (s, 12H), 0.76 (d, J = 6.4 Hz, 3H), -0.01 (d, J = 6.4 Hz, 3H).

### Step 3

### 3-[(4-{2-[(2,2-difluoroethyl)(isopropyl)aminoformyl]-4-fluorophenyl}-1-methyl-1H-indazol-6-yl)methyl]pyrrolidin-1-tert-butyl formate 165d

Compound **165c** (300 mg, 538.97 µmol) was dissolved in methanol (10 mL), followed by the addition of Pd/C (196.38 mg, 1.62 mmol). Under a hydrogen atmosphere (15 Psi), the mixture was stirred at 50°C for 12 hours. The reaction mixture was filtered, and the filtrate was concentrated under a reduced pressure, to obtain the title Compound **165d** (300 mg, 537.02 µmol, yield: 99.6%).
MS m/z (ESI): 559.4 [M+1]

### Step 4

### N-(2,2-difluoroethyl)-5-fluoro-2-{1-methyl-6-[(pyrrolidin-3-yl)methyl]-1H-indazol-4-yl}-N-(isopropyl)benzamide 165e

Compound **165d** (60 mg, 107.40 µmol) was dissolved in methane dioxide (5.0 mL), followed by the addition of trifluoroacetic acid (12.25 mg, 107.40 µmol, 8.27 µL). The mixture was stirred at room temperature to allow for reacting for 1.0 hours. The reaction mixture was concentrated under a reduced pressure, to obtain the crude product of title Compound **165e** (49.25 mg). The crude product was directly used in the next step of reaction without purification.
MS m/z (ESI): 459.3 [M+1]

### Step 5

### N-(2,2-difluoroethyl)-5-fluoro-2-{1-methyl-6-[(1-{[(1r,4r)-4-ethanesulfonamidocyclohexyl]methyl}pyrrolidin-3-yl)methyl]-1H-indazol-4-yl}-N-(isopropyl)benzamide 165

The crude product of Compound **165e** (49.25 mg) was dissolved in methanol (2.0 mL), followed by the addition of triethylamine (32.61 mg, 322.23 µmol) and Compound **132a**(25.91 mg, 118.15 µmol), and the mixture was stirred at room temperature for 1.0 hours. Sodium cyanoborohydride (13.50 mg, 214.82 µmol) was added, and the mixture was stirred at room temperature to allow for reacting for 12 hours. A saturated aqueous ammonium chloride solution (20 mL) was added to the reaction mixture, which was then extracted with ethyl acetate (10 mL x 3). The organic phases were combined, dried over anhydrous sodium sulfate and filtered, and the filtrate was concentrated under a reduced pressure. The resulting residues were purified by the preparative thin-layer chromatography using dichloromethane/methanol as an elution system, to obtain the title Compound **165** (18.7 mg, 28.26 µmol, yield: 26.3%) MS m/z (ESI):662.4 [M+1]
¹H NMR (400 MHz, CD₃OD) δ 7.84 (s, 1H), 7.66 - 7.60 (m, 1H), 7.42 (s, 1H), 7.37 (dt, J = 2.8, 8.4 Hz, 1H), 7.26 (dd, J = 2.8, 8.8 Hz, 1H), 7.02 (s, 1H), 5.92 - 5.56 (m, 1H), 4.07 (s, 3H), 3.67 - 3.52 (m, 2H), 3.05 - 2.99 (m, 2H), 2.86 (d, J = 7.6 Hz, 2H), 2.78 - 2.69 (m, 2H), 2.62 - 2.51 (m, 2H), 2.36 - 2.24 (m, 3H), 2.07 - 1.94 (m, 4H), 1.90 - 1.81 (m, 2H), 1.66 - 1.53 (m, 2H), 1.30 (br t, J = 7.2 Hz, 6H), 1.06 - 0.97 (m, 2H), 0.91 (d, J = 6.8 Hz, 3H), 0.16 (d, J = 6.8 Hz, 3H).

### Examples 165-1 and 165-2

### N-(2,2-difluoroethyl)-5-fluoro-2-(1-methyl-6-{[(3S)-1-{[(1r,4r)-4-ethanesulfonamidocyclohexyl]methyl}pyrrolidin-3-yl]methyl}-1H-indazol-4-yl)-N-(isopropyl)benzamide 165-1

### N-(2,2-difluoroethyl)-5-fluoro-2-(1-methyl-6-{[(3R)-1-{[(1r,4r)-4-ethanesulfonamidocyclohexyl]methyl}pyrrolidin-3-yl]methyl}-1H-indazol-4-yl)-N-(isopropyl)benzamide 165-2

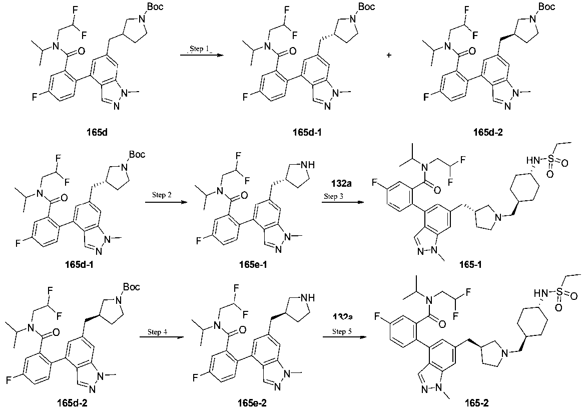

### Step 1

### (3S)-3-[(4-{2-[(2,2-difluoroethyl)(isopropyl)aminoformyl]-4-fluorophenyl}-1-methyl-1H-indazol-6-yl)methyl]pyrrolidin-1-tert-butyl formate 165d-1

### (3R)-3-[(4-{2-[(2,2-difluoroethyl)(isopropyl)aminoformyl]-4-fluorophenyl}-1-methyl-1H-indazol-6-yl)methyl]pyrrolidin-1-tert-butyl formate 165d-2

Compound **165d** (270 mg, 483.32 µmol) was separated and purified by the chiral preparative HPLC (preparative column: DAICEL CHIRALPAK IF (250 mm * 30 mm, 10 um), mobile phase: n-hexane-isopropanol/acetonitrile (0.1%isopropylamine); elution gradient: 15%-15%), to obtain the title Compounds **165d-1** (82 mg, 146.79 µmol, yield: 30.37%) and **165d-2** (90 mg, 161.11 µmol, yield: 33.33%).

### Single-configuration Compound 165d-1:

MS m/z (ESI):559.5 [M+1]
Chiral HPLC analysis: retention time: 3.719 min; chromatographic column: Chiralpak IF-3 50 x 4.6mm I.D., 3 um; mobile phase: n-hexane (0.05% isopropylamine)-isopropanol/acetonitrile (0.05% isopropylamine): elution gradient: 10%-10%.

### Single-configuration Compound 165d-2:

MS m/z (ESI):559.4 [M+1]
Chiral HPLC analysis: retention time: 3.333 min; chromatographic column: Chiralpak IF-3 50 x 4.6mm I.D., 3 um; mobile phase: n-hexane (0.05% isopropylamine)-isopropanol/acetonitrile (0.05% isopropylamine): elution gradient: 10%-10%.

### Steps 2 and 4

### N-(2,2-difluoroethyl)-5-fluoro-2-(1-methyl-6-{[(3S)-pyrrolidin-3-yl]methyl}-1H-indazol-4-yl)-N-(isopropyl)benzamide 165e-1

### N-(2,2-difluoroethyl)-5-fluoro-2-(1-methyl-6-{[(3R)-pyrrolidin-3-yl]methyl}-1H-indazol-4-yl)-N-(isopropyl)benzamide 165e-2

Compound **165d-1/165d-2** (82 mg/90 mg, 146.79 µmol/161.11 µmol) was dissolved in methane dioxide (5.0 mL/5.0 mL), followed by the addition of trifluoroacetic acid (1.0 mL/1.0 mL). The mixture was stirred at room temperature to allow for reacting for 1.0 hours. The reaction mixture was concentrated under a reduced pressure, to obtain the crude product of title Compound **165e-1/165e-2** (67.3 mg/73.87 mg). The crude product was directly used in the next step of reaction without purification.

### Single-configuration Compound 165e-1:

MS m/z (ESI): 459.3 [M+1]

### Single-configuration Compound 165e-2:

MS m/z (ESI): 459.4 [M+1]

### Steps 3 and 5

### N-(2,2-difluoroethyl)-5-fluoro-2-(1-methyl-6-{[(3S)-1-{[(1r,4r)-4-ethanesulfonamidocyclohexyl]methyl}pyrrolidin-3-yl]methyl}-1H-indazol-4-yl)-N-(isopropyl)benzamide 165-1

### N-(2,2-difluoroethyl)-5-fluoro-2-(1-methyl-6-{[(3R)-1-{[(1r,4r)-4-ethanesulfonamidocyclohexyl]methyl}pyrrolidin-3-yl]methyl}-1H-indazol-4-yl)-N-(isopropyl)benzamide 165-2

The crude product of Compound **165e-1/165e-2** (67.3 mg/73.87 mg) was dissolved in methanol (2.0 mL /2.0 mL), followed by the addition of triethylamine (35.68 mg/48.91 mg, 352.64 µmol/483.32 µmol) and Compound **132a**(28.36 mg/38.86 mg, 129.3 µmol/177.22 µmol), and the mixture was stirred at room temperature for (1.0/1.0) hour. Sodium cyanoborohydride (14.77 mg / 20.25 mg, 235.09 µmol/322.21 µmol) was added, and the mixture was stirred at room temperature to allow for reacting for (12/12) hours. An aqueous ammonium chloride solution (20 mL/20 mL) was added to the reaction mixture, which was then extracted with ethyl acetate (10 mL x 3/10 mL x 3). The organic phases were combined, dried over anhydrous sodium sulfate and filtered, and the filtrate was concentrated under a reduced pressure. The resulting residues were purified by the HPLC (mobile phase: acetonitrile, water (0.1%ammonium bicarbonate), elution gradient: 19%-49%/40%-70%), to obtain the title Compound **165-1/165-2** (12.02 mg/32.24 mg, 48.71 µmol/60.81 µmol, yield: 15.45 %/30.24 %)

### Single-configuration Compound 165-1:

MS m/z (ESI):662.4 [M+1]
Chiral SFC: retention time: 1.436 min; chromatographic column: Chiralcel OD-3 50 X 4.6 mm I.D., 3 um; mobile phase: CO2/MeOH (0.05%DEA): elution gradient: 5%-40%.

¹H NMR (400 MHz, CD₃OD) δ 7.70 (s, 1H), 7.51 - 7.44 (m, 1H), 7.27 (s, 1H), 7.22 (dt, J = 2.6, 8.8 Hz, 1H), 7.12 (dd, J = 2.6, 8.8 Hz, 1H), 6.88 (s, 1H), 5.81 - 5.38 (m, 1H), 3.92 (s, 3H), 3.52 - 3.38 (m, 2H), 3.13 - 3.02 (m, 1H), 3.00 - 2.92 (m, 1H), 2.87 (q, J = 7.2 Hz, 2H), 2.72 (d, J = 8.0 Hz, 2H), 2.68 - 2.55 (m, 2H), 2.52 - 2.41 (m, 2H), 2.26 - 2.11 (m, 3H), 1.89 - 1.79 (m, 3H), 1.77 - 1.67 (m, 2H), 1.55 - 1.39 (m, 2H), 1.15 (t, J = 7.2 Hz, 5H), 0.86 (d, J = 12.0 Hz, 2H), 0.76 (d, J = 6.4 Hz, 3H), 0.01 (d, J = 6.4 Hz, 3H).

### Single-configuration Compound 165-2:

MS m/z (ESI):662.5 [M+1]
Chiral SFC: retention time: 1.476 min; chromatographic column: Chiralcel OD-3 50 X 4.6 mm I.D., 3 um; mobile phase: CO₂/MeOH (0.05% DEA): elution gradient: 5%-40%.

¹H NMR (400 MHz, CD₃OD) δ 7.84 (s, 1H), 7.68 - 7.59 (m, 1H), 7.45 - 7.33 (m, 2H), 7.26 (dd, J = 2.6, 8.8 Hz, 1H), 7.02 (s, 1H), 5.92 - 5.54 (m, 1H), 4.07 (s, 3H), 3.65 - 3.51 (m, 2H), 3.27 - 3.17 (m, 1H), 3.15 - 3.07 (m, 1H), 3.02 (q, J = 7.4 Hz, 2H), 2.87 (d, J = 7.2 Hz, 2H), 2.81 - 2.67 (m, 2H), 2.64 - 2.52 (m, 2H), 2.40 - 2.22 (m, 3H), 2.05 - 1.93 (m, 3H), 1.92 - 1.81 (m, 2H), 1.68 - 1.53 (m, 2H), 1.30 (br t, J = 7.3 Hz, 5H), 1.08 - 0.96 (m, 2H), 0.91 (d, J = 6.8 Hz, 3H), 0.16 (d, J = 6.4 Hz, 3H).

### Example 166

### N-(1-{2-[3-(4-{2-[(2,2-difluoroethyl)(isopropyl)aminoformyl]-4-fluorophenyl}-1-methyl-1H-indazol-6-yl)pyrrolidin-1-yl]ethyl}piperidin-4-yl)tert-butyl carbamate 166

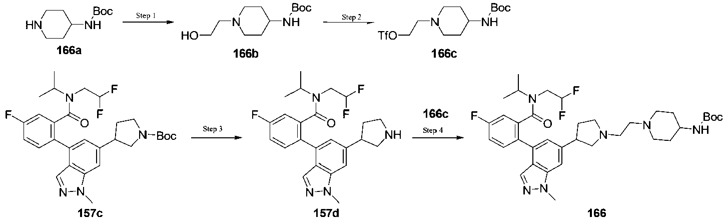

### Step 1

### N-[1-(2-hydroxylethyl) piperidin-4-yl]tert-butyl carbamate 166b

*N*-(piperidin-4-yl)tert-butyl carbamate **166a** (10 g, 49.93 mmol) was dissolved in acetonitrile (50 mL). Potassium carbonate (6.90 g, 49.93 mmol) and 2-bromoethanol (6.24 g, 49.93 mmol) were added, and the mixture was stirred at room temperature to allow for reacting for 12 hours. Water (150 mL) was added to the reaction mixture, which was then extracted with ethyl acetate (50 mL x 3). The organic phases were combined, washed with saturated brine (50 mL), dried over anhydrous sodium sulfate, and concentrated under a reduced pressure. The resulting residues were purified by the silica-gel column chromatography using petroleum ether/ethyl acetate as an elution system, to obtain the title Compound **166b** (8 g, 32.74 mmol, yield: 65.58%).
MS m/z (ESI): 245.2 [M+1]

### Step 2

### N-{1-[2-(trifluoromethylsulfonyloxy)ethyl]piperidin-4-yl}tert-butyl carbamate 166c

Compound **166b** (100 mg, 409.28 µmol) and triethylamine (62.12 mg, 613.92 µmol) were dissolved in dichloromethane (3.0 mL). The mixture was cooled to -78°C, followed by the slow dropwise addition of the dichloromethane solution (3.0 mL) of trifluoromethanesulfonic anhydride (150.12 mg, 532.07 µmol). The reaction mixture was slowly warmed to 0°C, and stirred to allow for reacting for 2.0 hours. The reaction mixture was directly used in the next step of reaction without further treatment.

### Step 3

### N-(2,2-difluoroethyl)-5-fluoro-2-[1-methyl-6-(pyrrolidin-3-yl)-1H-indazol-5-yl]-N-(isopropyl)benzamide 157d

Compound **157c** (50.00 mg, 91.81 µmol) was dissolved in methane dioxide (3.0 mL), followed by the addition of trifluoroacetic acid (999.97 mg, 8.77 mmol, 675.66 µL). The mixture was stirred at room temperature to allow for reacting for 1.0 hours. The reaction mixture was concentrated under a reduced pressure, followed by adding a saturated aqueous sodium bicarbonate solution (20 mL) and extracting with dichloromethane (10 mL x 3). The organic phases were combined, washed with saturated brine (50 mL), dried over anhydrous sodium sulfate, and concentrated under a reduced pressure. The crude product of title Compound **157d** (40.81 mg, 91.81 µmol, yield: 100%) was obtained. The crude product was directly used in the next step of reaction without purification.
MS m/z (ESI): 445.1 [M+1]

### Step 4

### N-(1-{2-[3-(4-{2-[(2,2-difluoroethyl)(isopropyl)aminoformyl]-4-fluorophenyl}-1-methyl-1H-indazol-6-yl)pyrrolidin-1-yl]ethyl}piperidin-4-yl)tert-butyl carbamate 166

The crude product of Compound **157d** (40 mg, 89.99 µmol) was dissolved in dichloromethane (3.0 mL), followed by the addition of triethylamine (9.11 mg, 89.99 µmol). The reaction mixture was cooled to 0°C, followed by the addition of the dichloromethane solution (4.0 mL) of Compound **166c** (101.61 mg, 269.97 µmol ), and the mixture was stirred at room temperature for 12 hours . Water (50 mL) was added to the reaction mixture, which was then extracted with dichloromethane (50 mL x 3). The organic phases were combined, washed with saturated brine, dried over anhydrous sodium sulfate and filtered, and the filtrate was concentrated under a reduced pressure. The resulting residues were purified by the HPLC (mobile phase: acetonitrile, water (0.1%ammonium bicarbonate), elution gradient: 52%-82%), to obtain the title Compound **166** (14.16 mg, 21.11 µmol, yield: 23.46%)
MS m/z (ESI):671.4 [M+1]
¹H NMR (400 MHz, CDCl₃) δ 7.85 - 7.81 (m, 1H), 7.58 - 7.52 (m, 1H), 7.29 (s, 1H), 7.25 - 7.21 (m, 1H), 7.16 (dd, J = 2.4, 8.4 Hz, 1H), 7.07 (s, 1H), 6.04 - 5.69 (m, 1H), 4.49 - 4.37 (m, 1H), 4.08 (s, 3H), 3.64 - 3.54 (m, 3H), 3.53 - 3.46 (m, 2H), 3.14 - 3.01 (m, 2H), 2.90 - 2.86 (m, 2H), 2.79 - 2.69 (m, 2H), 2.67 - 2.61 (m, 1H), 2.59 - 2.52 (m, 2H), 2.44 - 2.31 (m, 1H), 2.24 - 2.12 (m, 2H), 2.05 - 1.99 (m, 1H), 1.95 - 1.91 (m, 2H), 1.45 (s, 9H), 1.39 - 1.34 (m, 2H), 1.28 - 1.26 (m, 2H), 0.90 - 0.87 (m, 3H), 0.12 (d, J = 6.4 Hz, 2H)

### Examples 166-1 and 166-2

### N-(1-{2-[(3S)-3-(4-{2-[(2,2-difluoroethyl)(isopropyl)aminoformyl]-4-fluorophenyl}-1-methyl-1H-indazol-6-yl)pyrrolidin-1-yl]ethyl}piperidin-4-yl)tert-butyl carbamate 166-1

### N-(1-{2-[(3R)-3-(4-{2-[(2,2-difluoroethyl)(isopropyl)aminoformyl]-4-fluorophenyl}-1-methyl-1H-indazol-6-yl)pyrrolidin-1-yl]ethyl}piperidin-4-yl)tert-butyl carbamate 166-2

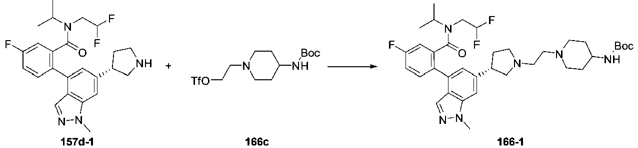

The crude product of Compound **157d-1/157d-2** (40.81 mg/40.81 mg, 91.81 µmol/91.81 µmol) was dissolved in dichloromethane (3.0 mL), followed by the addition of triethylamine (18.58 mg/18.58 mg, 183.63 µmol/183.63 µmol). The reaction mixture was cooled to 0°C, followed by the addition of the dichloromethane solution (2.0 mL/2.0 mL) of Compound **166c**(34.56 mg/34.56 mg, 91.81 µmol/91.81 µmol), and the mixture was stirred at room temperature for 12 hours . Water (50 mL/50 mL) was added to the reaction mixture, which was then extracted with dichloromethane (50 mL x 3/50 mL x 3). The organic phases were combined, washed with saturated brine, dried over anhydrous sodium sulfate and filtered, and the filtrate was concentrated under a reduced pressure. The resulting residues were purified by the HPLC (mobile phase: acetonitrile, water (0.1%ammonium bicarbonate), elution gradient: 53%-83%/53%-83%), to obtain the title Compound **166-1/166-2** (1.23 mg/7.31 mg, 1.83 µmol/10.9 µmol, yield: 2.00%/11.87%).

### Single-configuration Compound 166-1:

MS m/z (ESI):671.3 [M+1]
Chiral SFC: retention time: 1.177 min; chromatographic column: Chiralcel OD-3 50×4.6 mm I.D., 3 um; mobile phase: CO₂/MeOH (0.05% DEA): elution gradient: 5%-40%.

¹H NMR (400 MHz, CDCl₃) δ 7.83 (s, 1H), 7.59 - 7.52 (m, 1H), 7.37 - 7.30 (m, 1H), 7.26 - 7.22 (m, 1H), 7.18 - 7.14 (m, 1H), 7.07 (s, 1H), 6.06 - 5.68 (m, 1H), 4.61 - 4.37 (m, 1H), 4.09 (s, 3H), 3.65 - 3.46 (m, 4H), 3.26 - 2.99 (m, 3H), 2.98 - 2.88 (m, 3H), 2.87 - 2.67 (m, 4H), 2.67 - 2.56 (m, 2H), 2.49 - 2.30 (m, 2H), 2.25 - 2.13 (m, 2H), 2.04 - 1.88 (m, 4H), 1.45 (s, 9H), 0.88 (d, J = 6.4 Hz, 3H), 0.12 - 0.09 (m, 2H).

### Single-configuration Compound 166-2:

MS m/z (ESI):671.5 [M+1]
Chiral SFC: retention time: 1.172 min; chromatographic column: Chiralcel OD-3 50×4.6 mm I.D., 3 um; mobile phase: CO₂/MeOH (0.05% DEA): elution gradient: 5%-40%.

¹H NMR (400 MHz, CDCl₃) δ 7.82 (s, 1H), 7.59 - 7.52 (m, 1H), 7.33 - 7.28 (m, 1H), 7.26 - 7.21 (m, 1H), 7.19 - 7.14 (m, 1H), 7.07 (s, 1H), 6.05 - 5.69 (m, 1H), 4.53 - 4.39 (m, 1H), 4.09 (s, 3H), 3.63 - 3.45 (m, 4H), 3.20 - 3.00 (m, 3H), 2.94 - 2.86 (m, 3H), 2.82 - 2.73 (m, 2H), 2.71 - 2.62 (m, 2H), 2.61 - 2.53 (m, 2H), 2.46 - 2.32 (m, 2H), 2.21 - 2.12 (m, 2H), 2.01 - 1.88 (m, 4H), 1.45 (s, 9H), 0.91 - 0.86 (m, 3H), 0.13 - 0.09 (m, 2H).

### Example 169

### 4-{4-fluoro-2-[(3R)-3-methylmorpholin-4-carbonyl]phenyl}-1-methyl-6-({1-[(1S,3S,4R)-5-methylene-2-azabicyclo[2.2.2]octan-3-carbonyl]azacyclobutan-3-yl}methyl)-1H-indazole 169

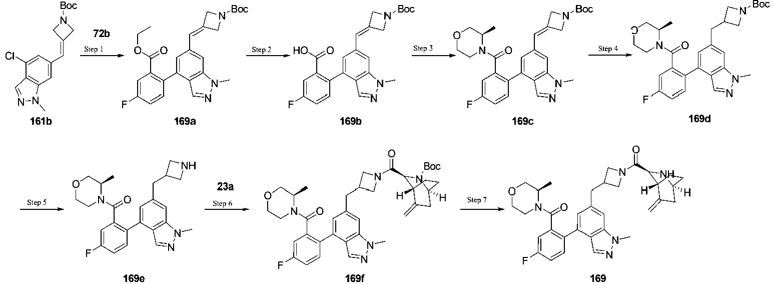

### Step 1

### 3-({4-[2-(ethoxycarbonyl)-4-fluorophenyl]-1-methyl-1H-indazol-6-yl}methylene)azacyclobutan-1-tert-butyl formate 169a

Compound **161b** (665 mg, 1.99 mmol) and Compound **72b** (878.91 mg, 2.39 mmol) were dissolved in dioxane (9.0 mL) and water (1.5 mL). Potassium phosphate (1.27 g, 5.98 mmol) and Pd(dtbpf)Cl₂ (129.84 mg, 199.21 µmol) were added, and the mixture was stirred at 95°C under a nitrogen atmosphere to allow for reacting for 12.0 hours. The reaction mixture was cooled to room temperature, followed by adding water (20 mL) and extracting with ethyl acetate (20 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **169a** (487 mg, 1.05 mmol, yield: 52.51%) as yellow oil.
MS m/z (ESI): 466.2 [M+1]

### Step 2

### 2-[6-({1-[(tert-butoxy)carbonyl]azacyclobutan-3-ylidene}methyl)-1-methyl-1H-indazol-4-yl]-5-fluorobenzoic acid 169b

Compound **169a** (487 mg, 1.05 mmol) was dissolved in ethanol (5 mL) and water (2.5 mL), followed by the addition of LiOH.H₂O (439.00 mg, 10.46 mmol), and the mixture was stirred at 50°C to allow for reacting for 12 hours. The mixture was cooled to room temperature, followed by removing the organic solvent under a reduced pressure. The mixture was regulated to PH < 7 by adding 1M HCl, and extracted with dichloromethane (20 mL × 3). The organic phases were combined, dried over anhydrous sodium sulfate, and concentrated under a reduced pressure. The crude product of title Compound **169b** (450 mg, 1.03 mmol, yield: 98.33%) was obtained as yellow oil. The crude product was directly used in the next step of reaction without purification.
MS m/z (ESI): 438.1 [M+1]

### Step 3

### 3-[(4-{4-fluoro-2-[(3R)-3-methylmorpholin-4-carbonyl]phenyl}-1-methyl-1H-indazol-6-yl)methylene]azacyclobutan-1-tert-butyl formate 169c

Compound **169b** (200 mg, 457.18 µmol), triethylamine (138.79 mg, 1.37 mmol) and HATU (208.60 mg, 548.62 µmol) were dissolved in dichloromethane (3.0 mL), and stirred at room temperature for 10 min, followed by the addition of (3*R*)-3-methylmorpholine (50.87 mg, 502.90 µmol). The reaction mixture was stirred at room temperature for 12 hours. A saturated aqueous sodium bicarbonate solution (15 mL) was added to the reaction mixture, which was then extracted with dichloromethane (15 mL × 3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **169c** (195 mg, 374.57 µmol, yield: 81.93%).
MS m/z (ESI): 521.2 [M+1]

### Step 4

### 3-[(4-{4-fluoro-2-[(3R)-3-methylmorpholin-4-carbonyl]phenyl}-1-methyl-1H-indazol-6-yl)methyl]azacyclobutan-1-tert-butyl formate 169d

Compound **169c** (195 mg, 374.57 µmol) was dissolved in tetrahydrofuran (3.0 mL), followed by the addition of 10% Pd/C (224.14 mg, 2.11 mmol). Under a hydrogen atmosphere (15 Psi), the mixture was stirred at 25°C for 3.0 hours. The reaction mixture was filtered, and the filtrate was concentrated under a reduced pressure, to obtain the title Compound **169d** (180 mg, 344.43 µmol, yield: 91.95%).
MS m/z (ESI): 523.2 [M+1]

### Step 5

### 6-[(azacyclobutan-3-yl)methyl]-4-{4-fluoro-2-[(3R)-3-methylmorpholin-4-carbonyl]phenyl}-1-methyl-1H-indazole 169e

Compound **169d** (90 mg, 172.21 µmol) was dissolved in methane dioxide (2.0 mL), followed by the addition of trifluoroacetic acid (740.00 mg, 6.49 mmol, 0.5 mL). The mixture was stirred at room temperature to allow for reacting for 1.0 hours. The reaction mixture was concentrated under a reduced pressure, to obtain the crude product of title Compound **169e** (70 mg). The crude product was directly used in the next step of reaction without purification. MS m/z (ESI): 423.1 [M+1]

### Step 6

### (1S,3S,4R)-3-{3-[(4-{4-fluoro-2-[(3R)-3-methylmorpholin-4-carbonyl]phenyl}-1-methyl-1H-indazol-6-yl)methyl]azacyclobutan-1-carbonyl}-5-methylene-2-azabicyclo[2.2.2]octan-2-tert-butyl carboxylate 169f

Compound **23a**(38.37 mg, 143.52 µmol) and *N,N*-diisopropylethylamine (118.04 mg, 913.30 µmol) were dissolved in DCM (3.0 mL). The mixture was cooled to 0°C, followed by the addition of EDCI (37.77 mg, 197.01 µmol), HOBT (26.97 mg, 199.62 µmol) and the crude product of Compound **169e** (70 mg), and the mixture was then stirred at room temperature for 12 hours. The reaction mixture was diluted with a saturated aqueous sodium bicarbonate solution (10 mL), and extracted with dichloromethane (15 mL x 3), and the organic phase was dried over anhydrous sodium sulfate, and concentrated under a reduced pressure. The resulting residues were purified by the preparative thin-layer chromatography using petroleum ether/ethyl acetate as an elution system, to obtain the title Compound **169e** (70 mg, 104.20 µmol, yield: 79.86%) as colorless oil.
MS m/z (ESI): 672.6 [M+1]

### Step 7

### 4-{4-fluoro-2-[(3R)-3-methylmorpholin-4-carbonyl]phenyl}-1-methyl-6-({1-[(1S,3S,4R)-5-methylene-2-azabicyclo[2.2.2]octan-3-carbonyl]azacyclobutan-3-yl}methyl)-1H-indazole 169

Compound **169e** (70 mg, 104.20 µmol) was dissolved in methane dioxide (2.0 mL) and trifluoroacetic acid (0.5 mL). The mixture was stirred at room temperature to allow for reacting for 1.0 hours. The reaction mixture was concentrated under a reduced pressure, and purified by the HPLC (mobile phase: acetonitrile, water (0.1% ammonia water), elution gradient: 28%-58%), to obtain the title Compound **169** (36.18 mg, 63.29 µmol, yield: 60.74 %) as white solid.
MS m/z (ESI): 572.5 [M+1]
¹H NMR (400 MHz, DMSO-*d*₆) δ 7.88 - 7.73 (m, 1H), 7.66 - 7.51 (m, 2H), 7.47 - 7.26 (m, 2H), 6.99 - 6.82 (m, 1H), 4.96 - 4.85 (m, 1H), 4.68 (s, 1H), 4.34 - 4.14 (m, 2H), 4.09 - 4.02 (m, 3H), 4.02 - 3.96 (m, 1H), 3.81 (s, 1H), 3.76 - 3.36 (m, 4H), 3.06 (s, 2H), 3.02 - 2.65 (m, 5H), 2.47 - 2.34 (m, 3H), 2.29 - 2.06 (m, 2H), 1.69 - 1.60 (m, 2H), 1.44 - 1.36 (m, 2H), 1.25 - 1.09 (m, 1H), 1.01 - 0.94 (m, 1H), 0.47 - 0.10 (m, 1H).

### Example 170

### N-(2,2-difluoroethyl)-5-fluoro-2-[1-methyl-6-(1-{[(1r,4r)-4-ethanesulfonamidocyclohexyl]methyl}piperidin-3-yl)-1H-indazol-4-yl]-N-(isopropyl)benzamide 170

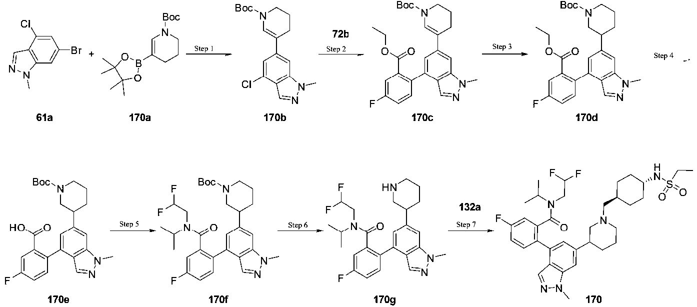

### Step 1

### 5-(4-chloro-1-methyl-1H-indazol-6-yl)-1,2,3,4-tetrahydropyridin-1-tert-butyl formate 170b

Compound **61a** (500 mg, 2.04 mmol) was dissolved in dioxane (6.0 mL) and water (1.0 mL). Sodium carbonate (647.59 mg, 6.11 mmol), 5-(tetramethyl-1,3,2-dioxaboran-2-yl)- 1,2,3,4-tetrahydropyridin-1-tert-butyl formate **170a** (692.72 mg, 2.24 mmol) and Pd(dppf)Cl₂ (149.02 mg, 203.66 µmol) were added, and the mixture was stirred at 90°C under a nitrogen atmosphere to allow for reacting for 2.0 hours. The reaction mixture was cooled to room temperature, followed by adding water (100 mL) and extracting with ethyl acetate (50 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **170b** (700 mg, 2.01 mmol, yield: 98.81%) as yellow solid.
MS m/z (ESI): 348.1 [M+1]

### Step 2

### 5-{4-[2-(ethoxycarbonyl)-4-fluorophenyl]-1-methyl-1H-indazol-6-yl}-1,2,3,4-tetrahydropyridin-1-tert-butyl formate 170c

Compound **170b** (400 mg, 1.15 mmol) and Compound **72b** (676.46 mg, 2.30 mmol) were dissolved in dioxane (12 mL) and water (2.0 mL). Cesium carbonate (1.08 g, 3.45 mmol) and Pd(dtbpf)Cl₂ (74.95 mg, 115.00 µmol) were added, and the mixture was stirred at 90°C under a nitrogen atmosphere to allow for reacting for 3.0 hours. The reaction mixture was cooled to room temperature, followed by adding water (50 mL) and extracting with ethyl acetate (20 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **170c** (490 mg, 1.02 mmol, yield: 88.86%) as yellow oil.
MS m/z (ESI): 480.3 [M+1]

### Step 3

### 3-{4-[2-(ethoxycarbonyl)-4-fluorophenyl]-1-methyl-1H-indazol-6-yl}piperidin-1-tert-butyl formate 170d

Compound **170c** (490 mg, 1.02 mmol) were dissolved in methanol (10.0 mL), followed by the addition of Pd/C (620.52 mg, 5.11 mmol). Under a hydrogen atmosphere (15 Psi), the mixture was stirred at 50°C for 12 hours. The reaction mixture was filtered through diatomite, and the filtrate was concentrated under a reduced pressure, to obtain the title Compound **170d** crude product of (477 mg, 990.54 µmol, yield: 96.94%). The crude product was directly used in the next step of reaction without purification.
MS m/z (ESI): 482.3 [M+1]
¹H NMR (400 MHz, CD₃OD) δ 7.69 - 7.62 (m, 2H), 7.45 (dd, J = 5.4, 8.4 Hz, 1H), 7.33 - 7.28 (m, 1H), 7.23 (s, 1H), 6.92 (s, 1H), 4.08 (s, 3H), 3.50 (s, 2H), 2.87 (d, J = 5.2 Hz, 3H), 2.12 (br d, J = 13.0 Hz, 1H), 1.83 - 1.74 (m, 3H), 1.65 - 1.62 (m, 2H), 1.48 (s, 9H), 0.88 - 0.79 (m, 3H).

### Step 4

### 2-(6-{1-[(tert-butoxy)carbonyl]piperidin-3-yl}-1-methyl-1H-indazol-4-yl)-5-fluorobenzoic acid 170e

Compound **170d** (477 mg, 990.54 µmol) was dissolved in ethanol (10 mL), tetrahydrofuran (10 mL) and water (5.0 mL), followed by the addition of LiOH.H₂O (71.16 mg, 2.97 mmol), and the mixture was stirred at 50°Cto allow for reacting for 12 hours. The mixture was cooled to room temperature, followed by removing the organic solvent under a reduced pressure. The mixture was regulated to PH ≤ 7 by adding 1M HCl, and extracted with dichloromethane (10 mL × 3). The organic phases were combined, dried over anhydrous sodium sulfate, and concentrated under a reduced pressure. The crude product of title Compound **170e** (449 mg, 990.07 µmol, yield: 99.95%) as yellow solid was obtained. The crude product was directly used in the next step of reaction without purification.
MS m/z (ESI): 454.2 [M+1]

### Step 5

### 3-(4-{2-[(2,2-difluoroethyl)(isopropyl)aminoformyl]-4-fluorophenyl}-1-methyl-1H-indazol-6-yl)piperidin-1-tert-butyl carboxylate 170f

Compound **170e** (150 mg, 330.76 µmol), Compound **94b** (79.19 mg, 496.14 µmol, hydrochloride) and 2-chloro-1-methylpyridinium iodide (109.85 mg, 429.98 µmol) were dissolved in DMF(1.88 mL), followed by the addition of DIPEA (128.24 mg, 992.27 µmol). The reaction mixture was stirred at room temperature for 1.0 hours. Ethyl acetate (20 mL) was added to the reaction mixture, which was then washed with water (10 mL×3). The organic phase was dried over anhydrous sodium sulfate, filtered and concentrated under a reduced pressure. The resulting residues were purified by the preparative thin-layer chromatography using petroleum ether/ethyl acetate as an elution system, to obtain the title Compound **170f** (87.6 mg, 156.81 µmol, yield: 47.4%) as yellow solid.
MS m/z (ESI): 559.3 [M+1]

### Step 6

### N-(2,2-difluoroethyl)-5-fluoro-2-[1-methyl-6-(piperidin-3-yl)-1H-indazol-4-yl]-N-(isopropyl)benzamide 170g

Compound **170f** (35 mg, 62.65 µmol) was dissolved in dichloromethane (5.0 mL), followed by the addition of trifluoroacetic acid (1.48 g, 12.98 mmol, 1 mL) at 25°C. The mixture was stirred at room temperature to allow for reacting for 1.0 hours. The reaction mixture was concentrated under a reduced pressure, to obtain the crude product of title Compound **170g** (28.73 mg). The crude product was directly used in the next step of reaction without purification.
MS m/z (ESI): 459.2 [M+1]

### Step 7

### N-(2,2-difluoroethyl)-5-fluoro-2-[1-methyl-6-(1-{[(1r,4r)-4-ethanesulfonamidocyclohexyl]methyl}piperidin-3-yl)-1H-indazol-4-yl]-N-(isopropyl)benzamide 170

The crude product of Compound **170g** (28.73 mg), *N*-[(1*r*,4*r*)-4-formylcyclohexyl]ethan-1-sulfonamide **132a**(12.10 mg, 55.20 µmol) and triethylamine (15.23 mg, 150.54 µmol) were dissolved in methanol (2.0 mL), and the mixture was stirred at room temperature for 1.0 hour. Sodium cyanoborohydride (6.31 mg, 100.36 µmol) was added, and the mixture was stirred at room temperature for 1.0 hours. Water (50 mL) was added to the reaction mixture, which was then extracted with dichloromethane (50 mL x 3). The organic phases were combined, dried over anhydrous sodium sulfate, and concentrated under a reduced pressure. The resulting residues were purified by the preparative thin-layer chromatography using dichloromethane/tetrahydrofuran as an elution system, to obtain the title Compound 170 (9.69 mg, 14.64 µmol, yield: 29.18%) as white solid.
MS m/z (ESI): 662.4 [M+1]
¹H NMR (400 MHz, CD₃OD) δ 7.71 (d, J = 0.8 Hz, 1H), 7.49 (ddd, J = 1.0, 5.4, 8.6 Hz, 1H), 7.34 (s, 1H), 7.24 (dt, J = 2.6, 8.4 Hz, 1H), 7.13 (td, J = 2.2, 8.6 Hz, 1H), 6.93 (s, 1H), 5.79 - 5.44 (m, 1H), 4.44 (s, 1H), 3.94 (s, 3H), 3.55 - 3.38 (m, 2H), 3.14 - 2.94 (m, 2H), 2.93 - 2.83 (m, 4H), 2.14 (t, J = 6.6 Hz, 2H), 2.08 - 1.94 (m, 2H), 1.90 - 1.80 (m, 3H), 1.78 - 1.65 (m, 3H), 1.55 - 1.34 (m, 3H), 1.19 - 1.13 (m, 6H), 0.95 - 0.84 (m, 2H), 0.77 (d, J = 6.4 Hz, 3H), -0.01 (d, J= 6.8 Hz, 2H).

### Example 171

### 1-(4-{[3-(4-{4-fluoro-2-[(3R)-3-methylmorpholin-4-carbonyl]phenyl}-1-methyl-1H-indazol-6-yl)azacyclobutan-1-yl]methyl}piperidin-1-carbonyl)cyclopropan-1-ol 171

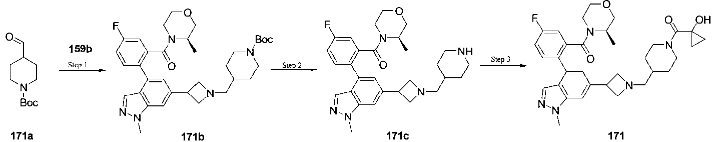

### Step 1

### 4-{[3-(4-{4-fluoro-2-[(3R)-3-methylmorpholin-4-carbonyl]phenyl}-1-methyl-1H-indazol-6-yl)azacyclobutan-1-yl]methyl}piperidin-1-tert-butyl formate 171b

The crude product of Compound **159b** (226.01 mg, 432.57 µmol, trifluoroacetate), 4-formylpiperidin-1-tert-butyl formate **171a** (110.71 mg, 519.08 µmol) and triethylamine (87.54 mg, 865.14 µmol) were dissolved in methanol (2.0 mL), and stirred at room temperature for 30 min. Sodium cyanoborohydride (81.55 mg, 1.30 mmol) was added, and the reaction mixture was stirred at room temperature for 12 hour. Acetic acid (50 mg) was added to the reaction mixture, which was then warmed to 50°C and further stirred for 3.0 hours. A saturated aqueous sodium bicarbonate solution (20 mL) was added to the reaction mixture, which was then extracted with dichloromethane (20 mL x 3). The organic phases were combined, dried over anhydrous sodium sulfate, and concentrated under a reduced pressure. The resulting residues were purified by the preparative thin-layer chromatography using petroleum ether/ethyl acetate/ethanol as an elution system, to obtain the title Compound **171b** (200 mg, 330.17 µmol, yield: 76.33%) as faint yellow solid.
MS m/z (ESI): 606.3 [M+1]

### Step 2

### 4-{4-fluoro-2-[(3R)-3-methylmorpholin-4-carbonyl]phenyl}-1-methyl-6-{1-[(piperidin-4-yl)methyl]azacyclobutan-3-yl}-1H-indazole 171c

Compound **171b** (65 mg, 107.31 µmol) was dissolved in dichloromethane (3.0 mL), followed by the addition of trifluoroacetic acid (1 g, 8.77 mmol, 675.68 µL) at 25°C. The mixture was stirred at room temperature to allow for reacting for 0.5 hours. The reaction mixture was concentrated under a reduced pressure, to obtain the crude product of title Compound **171c** (54.3 mg). The crude product was directly used in the next step of reaction without purification.
MS m/z (ESI): 506.3 [M+1]

### Step 3

### 1-(4-{[3-(4-{4-fluoro-2-[(3R)-3-methylmorpholin-4-carbonyl]phenyl}-1-methyl-1H-indazol-6-yl)azacyclobutan-1-yl]methyl}piperidin-1-carbonyl)cyclopropan-1-ol 171

1-Hydroxylcyclopropan-1-carboxylic acid (12.05 mg, 118.04 µmol), triethylamine (32.58 mg, 321.94 µmol) and HATU(81.61 mg, 214.63 µmol) were dissolved in dichloromethane (5.0 mL), and stirred at room temperature for 30 min, followed by the addition of the crude product of Compound **171c** (54.3 mg). The reaction mixture was stirred at room temperature for 2 hours. 1-Hydroxylcyclopropan-1-carboxylic acid (12.05 mg, 118.04 µmol) and HATU(81.61 mg, 214.63 µmol) were supplemented, and the mixture was further stirred for 4.0 hours. Water (50 mL) was added to the reaction mixture, which was then extracted with dichloromethane (50 mL×3). The organic phase was washed with saturated brine, dried over anhydrous sodium sulfate, filtered and concentrated under a reduced pressure. The resulting residues were purified by the HPLC (mobile phase: acetonitrile, water (0.1% ammonium bicarbonate), elution gradient: 23%-53%), to obtain the title Compound **171** (8.88 mg, 15.06 µmol, yield: 14.03 %) as white solid.
MS m/z (ESI): 590.3 [M+1]
¹H NMR (400 MHz, CDCl₃) δ 7.91 - 7.81 (m, 1H), 7.64 - 7.51 (m, 1H), 7.41 - 7.28 (m, 2H), 7.26 - 7.09 (m, 2H), 4.61 - 4.28 (m, J = 13.1 Hz, 3H), 4.12 (s, 3H), 3.92 - 3.82 (m, 3H), 3.68 - 3.38 (m, 2H), 3.37 - 3.14 (m, 3H), 3.13 - 2.95 (m, 1H), 2.88 - 2.79 (m, 2H), 2.63 - 2.35 (m, 3H), 1.86 - 1.79 (m, 2H), 1.72 - 1.39 (m, 2H), 1.34 - 1.19 (m, 4H), 1.13 - 1.04 (m, 3H), 0.99 - 0.91 (m, 2H), 0.62 - 0.14 (m, 1H).

### Example 174

### N-methyl-N-[(1r,4r)-4-{[3-(4-{4-fluoro-2-[(3R)-3-methylmorpholin-4-carbonyl]phenyl}-1-methyl-1H-indazol-6-yl)azacyclobutan-1-yl]methyl}cyclohexyl]ethan-1-sulfonamide 174

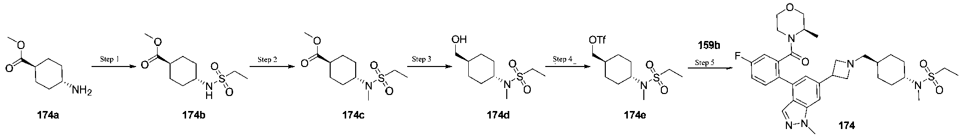

### Step 1

### (1r,4r)-4-ethanesulfonamidocyclohexan-1-methyl carboxylate 174b

(1*r*,4*r*)-4-aminocyclohexan-1-methyl carboxylate **174a** (1.86 g, 9.63 mmol, hydrochloride) was dissolved in dichloromethane (20 mL), and the mixture was cooled to 5°C in an ice-water bath. Triethylamine (1.95 g, 19.26 mmol) was added, followed by the slow dropwise addition of ethylsulfonyl chloride (1.24 g, 9.63 mmol). The reaction mixture was stirred at room temperature for 2 hours. Water (50 mL) was added to the reaction mixture, which was then extracted with ethyl acetate (50 mL x 3). The organic phase was washed with saturated brine, dried over anhydrous sodium sulfate, filtered and concentrated under a reduced pressure. The resulting residues were purified by the silica-gel column chromatography using petroleum ether/ethyl acetate as an elution system, to obtain the title Compound **174b** (1.23 g, 4.93 mmol, yield: 51.23%) as white solid.

¹H NMR (400 MHz, CDCl₃) δ 4.13 (br d, J = 8.0 Hz, 1H), 3.68 (s, 3H), 3.25 (m, 1H), 3.05 (q, J = 7.4 Hz, 2H), 2.29 - 2.20 (m, 1H), 2.17 - 2.10 (m, 2H), 2.08 - 2.00 (m, 2H), 1.61 - 1.48 (m, 2H), 1.38 (t, J = 7.4 Hz, 3H), 1.34 - 1.21 (m, 2H).

### Step 2

### (1r,4r)-4-(N-methylethanesulfonamido)cyclohexan-1-methyl carboxylate 174c

Compound **174b** (500 mg, 1.90 mmol) was dissolved in DMF(20 mL), followed by the addition of potassium carbonate (787.19 mg, 5.70 mmol) and iodomethane (1.08 g, 7.59 mmol) at 25°C. The reaction mixture was stirred at 50°C to allow for reacting for 12 hours. Water (50 mL) was added to the reaction mixture, which was then extracted with ethyl acetate (50 mL x 3). The organic phase was washed with saturated brine, dried over anhydrous sodium sulfate, filtered and concentrated under a reduced pressure. The resulting residues were purified by the silica-gel column chromatography using petroleum ether/ethyl acetate as an elution system, to obtain the title Compound **174c** (260 mg, 987.27 µmol, yield: 52%) as colorless oil.

¹H NMR (400 MHz, CDCl₃) δ 3.60 (m, 4H), 2.90 (q, J = 7.4 Hz, 2H), 2.72 (s, 3H), 2.18 - 2.08 (m, 1H), 2.02 (br d, J = 7.4 Hz, 2H), 1.81 - 1.73 (m, 2H), 1.55 - 1.45 (m, 4H), 1.27 (t, J = 7.4 Hz, 3H).

### Step 3

### N-methyl-N-[(1r,4r)-4-(hydroxymethyl)cyclohexyl]ethan-1-sulfonamide 174d

Compound **174c** (260 mg, 987.27 µmol) was dissolved in dichloromethane (7.7 mL), and cooled to 0°C, followed by the addition of DIBAL-H(I M, 2.47 mL). The mixture was stirred at 0°C to allow for reacting for 1.0 hour, sodium sulfate decahydrate (5.0 g) was added, and the reaction mixture was further stirred for 15 min, then warmed to room temperature, and stirred for 0.5 hours. The mixture was filtered, and the organic phase was collected and concentrated under a reduced pressure . The resulting residues were purified by the silica-gel column chromatography using petroleum ether/ethyl acetate as an elution system, to obtain the title Compound **174d** (128 mg, 543.89 µmol, yield: 55.1%) as colorless oil.

¹H NMR (400 MHz, CDCl₃) δ 3.72 - 3.62 (m, 1H), 3.47 (d, J = 6.4 Hz, 2H), 2.97 (q, J = 7.4 Hz, 2H), 2.81 (s, 3H), 1.93 - 1.80 (m, 4H), 1.63 - 1.50 (m, 4H), 1.43 (m, 2H), 1.35 (t, J = 7.4 Hz, 3H).

### Step 4

### [(1r,4r)-4-(N-methylethanesulfonamido)cyclohexyl]methyl trifluoromethanesulfonate

Compound **174d** (128 mg, 543.89 µmol) was dissolved in dichloromethane (5.0 mL), followed by the addition of triethylamine (110.07 mg, 1.09 mmol). The reaction mixture was cooled to -78°C, followed by dropwise adding the solution of trifluoromethanesulfonic anhydride (199.49 mg, 707.05 µmol) in dichloromethane (3.0 mL) slowly. The reaction mixture was stirred at 0°C for 2 hours. The reaction mixture was directly used in the next step of reaction without treatment.

### Step 5

### N-methyl-N-[(1r,4r)-4-{[3-(4-{4-fluoro-2-[(3R)-3-methylmorpholin-4-carbonyl]phenyl}-1-methyl-1H-indazol-6-yl)azacyclobutan-1-yl]methyl}cyclohexyl]ethan-1-sulfonamide 174

Compound **159b** (120 mg, 293.78 µmol) was dissolved in dichloromethane (10 mL), and cooled to 0°C, followed by the slow dropwise addition of the mixture from the previous step of reaction. After drowpise addition, the mixture was stirred at room temperature for 12 hours. Water (30 mL) was added to the reaction mixture, which was then extracted with ethyl acetate (10 mL x 3). The organic phases were combined, dried over anhydrous sodium sulfate, and concentrated under a reduced pressure. The resulting residues were purified by the preparative thin-layer chromatography using dichloromethane/methanol as an elution system, to obtain the title Compound **174** (41.5 mg, 66.32 µmol, yield: 22.57%) as yellow solid.
MS m/z (ESI): 626.4 [M+1]
¹H NMR (400 MHz, CD₃OD) δ 7.93 - 7.87 (m, 1H), 7.69 (td, J = 5.2, 8.8 Hz, 1H), 7.63 - 7.58 (m, 1H), 7.43 - 7.28 (m, 2H), 7.26 - 7.10 (m, 1H), 4.27 - 4.15 (m, 3H), 4.13 - 4.10 (m, 3H), 3.99 - 3.70 (m, 3H), 3.68 - 3.54 (m, 2H), 3.54 - 3.35 (m, 2H), 3.25 - 3.09 (m, 2H), 3.04 (q, J = 7.4 Hz, 3H), 2.79 (s, 3H), 1.96 - 1.88 (m, 2H), 1.83 - 1.76 (m, 2H), 1.66 (dq, J = 3.6, 12.8 Hz, 3H), 1.32 - 1.27 (m, 4H), 1.26 - 1.11 (m, 4H), 1.08 (d, J = 6.8 Hz, 1H), 0.48 (d, J = 6.8 Hz, 1H).

### Example 175

### N-[(1r,4r)-4-{2-[3-(4-{4-fluoro-2-[(3R)-3-methylmorpholin-4-carbonyl]phenyl}-1-methyl-1H-indazol-6-yl)azacyclobutan-1-yl]propyl}cyclohexyl]ethan-1-sulfonamide 175

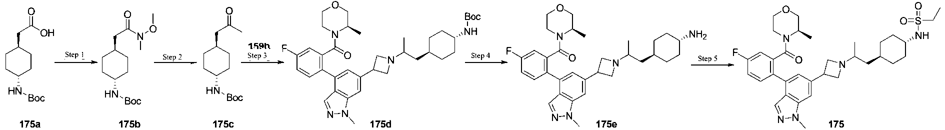

### Step 1

### N-[(1r,4r)-4-{[methoxy(methyl)aminoformyl]methyl}cyclohexyl]tert-butyl carbamate 175b

2-[(1*r*,4*r*)-4-{[(tert-butoxy)carbonyl]amino}cyclohexyl]acetic acid **175a** (500 mg, 1.94 mmol) and triethylamine (589.85 mg, 5.83 mmol) were dissolved in dichloromethane (8.0 mL), followed by the addition of EDCI (744.98 mg, 3.89 mmol) and HOBt (525.11 mg, 3.89 mmol), and the mixture was stirred at room temperature for 30 min, followed by the addition of N-methoxymethylamine (227.44 mg, 2.33 mmol, hydrochloride). The reaction mixture was stirred at room temperature for 12 hours. Water (50 mL) was added to the reaction mixture, which was then extracted with ethyl acetate (50 mL x 3). The organic phase was washed with saturated brine, dried over anhydrous sodium sulfate, filtered and concentrated under a reduced pressure. The resulting residues were purified by the silica-gel column chromatography using petroleum ether/ethyl acetate as an elution system, to obtain the title Compound **175b** (525 mg, 1.75 mmol, yield: 89.95%) as white solid.

¹H NMR (400 MHz, CDCl₃) δ 4.39 (s, 1H), 3.67 (s, 3H), 3.46 - 3.30 (m, 1H), 3.18 (s, 3H), 2.31 (m, 2H), 1.99 (m, 2H), 1.81 (m, 3H), 1.44 (s, 9H), 1.21 - 1.00 (m, 4H).

### Step 2

### N-[(1r,4r)-4-(2-oxopropyl)cyclohexyl]tert-butyl carbamate

Compound **175b** (525 mg, 1.75 mmol) was dissolved in tetrahydrofuran (19.94 mL), and cooled to 0°C, followed by the addition of a methylmagnesium bromide tetrahydrofuran solution (3 M, 640.83 µL). The reaction mixture was stirred at room temperature for 12 hours. Water (50 mL) was added to the reaction mixture, which was then extracted with ethyl acetate (50 mL x 3). The organic phase was washed with saturated brine, dried over anhydrous sodium sulfate, filtered and concentrated under a reduced pressure, to obtain the crude product of title Compound **175c** (82 mg, 321.13 µmol, yield: 18.37%) as white solid.

¹H NMR (400 MHz, CDCl₃) δ 4.42 - 4.32 (m, 1H), 3.44 - 3.30 (m, 1H), 2.32 (d, J = 6.4 Hz, 2H), 2.13 (s, 3H), 2.03 - 1.95 (m, 2H), 1.79 - 1.71 (m, 3H), 1.44 (s, 9H), 1.19 - 0.95 (m, 4H).

### Step 3

### N-[(1r,4r)-4-{2-[3-(4-{4-fluoro-2-[(3R)-3-methylmorpholin-4-carbonyl]phenyl}-1-methyl-1H-indazol-6-yl)azacyclobutan-1-yl]propyl}cyclohexyl]tert-butyl carbamate 175d

Compound **159b** (100 mg, 244.82 µmol) and Compound **175c** (68.77 mg, 269.30 µmol) were dissolved in methanol (10 mL), followed by the addition of zinc dichloride (100.10 mg, 734.45 µmol), and the mixture was stirred at 60°C for 1.0 hour. The reaction mixture was cooled to room temperature, followed by adding sodium cyanoborohydride (46.15 mg, 734.45 µmol) and stirring at 60°C for 4.0 hours. A saturated aqueous ammonium chloride solution (20 mL) was added to the reaction mixture, which was then stirred for 0.5 hours and extracted with ethyl acetate (50 mL x 3). The organic phases were combined, washed with saturated brine, dried over anhydrous sodium sulfate, and concentrated under a reduced pressure. The resulting residues were purified by the preparative thin-layer chromatography using dichloromethane/methanol as an elution system, to obtain the title Compound 175d (90 mg, 138.93 µmol, yield: 56.75%) as white solid.
MS m/z (ESI): 648.6 [M+1]
¹H NMR (400 MHz, CDCl₃) δ 7.98 - 7.84 (m, 1H), 7.74 - 7.54 (m, 1H), 7.44 - 7.28 (m, 2H), 7.27 - 7.13 (m, 2H), 4.56 - 4.00 (m, 8H), 3.92 - 3.34 (m, 5H), 3.25 - 2.74 (m, 4H), 2.58 - 2.19 (m, 2H), 2.09 - 1.76 (m, 4H), 1.45 (s, 9H), 1.38 - 1.26 (m, 4H), 1.22 - 1.17 (m, 2H), 1.14 - 0.96 (m, 4H), 0.58 - 0.05 (m, 2H).

### Step 4

### (1r,4r)-4-{2-[3-(4-{4-fluoro-2-[(3R)-3-methylmorpholin-4-carbonyl]phenyl}-1-methyl-1H-indazol-6-yl)azacyclobutan-1-yl]propyl}cyclohexan-1-amine 175e

Compound **175d** (90 mg, 138.93 µmol) was dissolved in dichloromethane (3.0 mL), followed by the addition of trifluoroacetic acid (1 g, 8.77 mmol, 675.68 µL) at 25°C. The mixture was stirred at room temperature to allow for reacting for 0.5 hours. The reaction mixture was concentrated under a reduced pressure, followed by adding a saturated aqueous sodium bicarbonate solution (20 mL), stirring for 0.5 hours and extracting with dichloromethane (10 mL x 3). The organic phases were combined, washed with saturated brine, dried over anhydrous sodium sulfate, and concentrated under a reduced pressure. The crude product of title Compound **175e** (65 mg, 118.68 µmol, yield: 85.42%) was obtained as white solid.
MS m/z (ESI): 548.4 [M+1]
¹H NMR (400 MHz, CDCl₃) δ 8.12 - 7.84 (m, 1H), 7.68 - 7.28 (m, 3H), 7.23 - 6.95 (m, 2H), 4.80 - 4.22 (m, 4H), 4.13 (s, 3H), 3.95 - 3.39 (m, 5H), 3.14 - 2.78 (m, 4H), 2.55 - 2.34 (m, 2H), 2.29 - 2.08 (m, 4H), 2.04 - 1.99 (m, 2H), 1.35 - 1.29 (m, 8H), 1.22 - 1.19 (m, 2H), 0.89 (br s, 2H).

### Step 5

### N-[(1r,4r)-4-{2-[3-(4-{4-fluoro-2-[(3R)-3-methylmorpholin-4-carbonyl]phenyl}-1-methyl-1H-indazol-6-yl)azacyclobutan-1-yl]propyl}cyclohexyl]ethan-1-sulfonamide 175

The crude product of Compound **175e** (65 mg, 118.68 µmol) was dissolved in dichloromethane (3.0 mL), followed by the addition of triethylamine (24.02 mg, 237.35 µmol), cooling to 0°C, and the slow dropwise addition of the dichloromethane solution (1 mL) of ethanesulfonyl chloride (19.84 mg, 154.28 µmol), and the mixture was stirred at room temperature for 12 hours. Water (30 mL) was added to the reaction mixture, which was then extracted with ethyl acetate (50 mL x 3). The organic phases were combined, washed with saturated brine, dried over anhydrous sodium sulfate, and concentrated under a reduced pressure. The resulting residues were purified by the preparative thin-layer chromatography using dichloromethane/methanol as an elution system, to obtain the title Compound 175 (44.33 mg, 69.28 µmol, yield: 58.38 %) as white solid.
MS m/z (ESI): 640.4[M+1]
¹H NMR (400 MHz, CDCl₃) δ 7.93 - 7.81 (m, 1H), 7.69 - 7.52 (m, 1H), 7.40 - 7.28 (m, 1H), 7.27 - 7.01 (m, 3H), 4.60 - 4.23 (m, 1H), 4.12 (s, 3H), 4.11 - 4.05 (m, 1H), 3.99 - 3.86 (m, 3H), 3.67 - 3.10 (m, 6H), 3.09 - 2.95 (m, 3H), 2.88 - 2.75 (m, 1H), 2.64 - 2.32 (m, 3H), 2.13 - 2.00 (m, 2H), 1.97 - 1.73 (m, 2H), 1.48 - 1.33 (m, 4H), 1.32 - 1.24 (m, 4H), 1.17 - 1.05 (m, 3H), 1.03 - 0.92 (m, 3H), 0.62 - 0.14 (m, 1H).

### Example 178

### N-[(1r,4r)-4-{1-[3-(4-{4-fluoro-2-[(3R)-3-methylmorpholin-4-carbonyl]phenyl}-1-methyl-1H-indazol-6-yl)azacyclobutan-1-yl]ethyl}cyclohexyl]ethan-1-sulfonamide 178

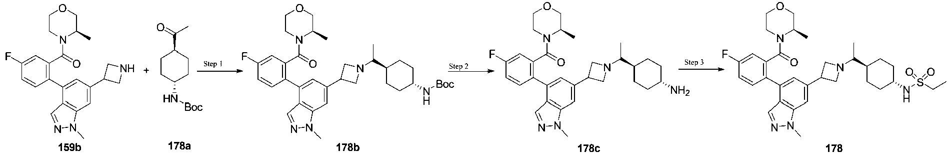

### Step 1

### N-[(1r,4r)-4-{1-[3-(4-{4-fluoro-2-[(3R)-3-methylmorpholin-4-carbonyl]phenyl}-1-methyl-1H-indazol-6-yl)azacyclobutan-1-yl]ethyl}cyclohexyl]tert-butyl carbamate 178b

Compound **159b** (100 mg, 244.82 µmol), (4-acetylcyclohexyl)tert-butyl carbamate **178a** (88.62 mg, 367.23 µmol) and zinc chloride (66.74 mg, 489.63 µmol) were dissolved in methanol (2.0 mL), and stirred at 50°C for 1.0 hour. Sodium cyanoborohydride (46.15 mg, 734.45 µmol) was added, and the mixture was further stirred at 50°C for 3.0 hours. The reaction mixture was cooled to room temperature, followed by adding a saturated aqueous sodium bicarbonate solution (10 mL) and extracting with dichloromethane (10 mL x 3). The organic phases were combined, dried over anhydrous sodium sulfate, and concentrated under a reduced pressure. The resulting residues were purified by the preparative thin-layer chromatography using petroleum ether/ethyl acetate/ethanol as an elution system, to obtain the title Compound **178b** (138 mg, 217.74 µmol, yield: 88.94 %) as faint yellow solid.
MS m/z (ESI): 634.4 [M+1]
¹H NMR (400 MHz, CD₃OD) δ 7.88 (s, 1H), 7.77 - 7.48 (m, 2H), 7.42 - 7.05 (m, 3H), 4.63 - 4.20 (m, 1H), 4.16 - 4.06 (m, 3H), 3.97 - 3.34 (m, 7H), 3.27 - 2.81 (m, 4H), 2.54 - 2.30 (m, 2H), 2.22 - 1.48 (m, 6H), 1.45 - 1.39 (m, 9H), 1.29 - 1.21 (m, 3H), 1.19 - **1.11** (m, 2H), 1.09 - 1.04 (m, 1H), 0.98 - 0.90 (m, 3H), 0.51 - 0.18 (m, 1H).

### Step 2

### (1r,4r)-4-{1-[3-(4-{4-fluoro-2-[(3R)-3-methylmorpholin-4-carbonyl]phenyl}-1-methyl-1H-indazol-6-yl)azacyclobutan-1-yl]ethyl}cyclohexan-1-amine 178c

Compound **178b** (120 mg, 189.34 µmol) was dissolved in dichloromethane (3.0 mL), followed by the addition of trifluoroacetic acid (1.48 g, 12.98 mmol, 1 mL) at 25°C. The mixture was stirred at room temperature to allow for reacting for 0.5 hours. A saturated aqueous sodium bicarbonate solution (20 mL) was added to the reaction mixture, which was then extracted with dichloromethane (20 mL x 3). The organic phases were combined, dried over anhydrous sodium sulfate, concentrated under a reduced pressure and lyophilized, to obtain the crude product of title Compound **178c** (122.64 mg, 229.80 µmol, yield: 121.37%). The crude product was directly used in the next step of reaction without purification.
MS m/z (ESI): 534.3 [M+1]
¹H NMR (400 MHz, CD₃OD) δ 7.78 (s, 1H), 7.63 - 7.47 (m, 1H), 7.46 - 7.41 (m, 1H), 7.31 - 7.24 (m, 1H), 7.21 - 6.96 (m, 2H), 4.39 - 4.12 (m, 1H), 4.03 - 3.99 (m, 3H), 3.79 - 3.70 (m, 3H), 3.50 - 3.29 (m, 2H), 3.18 - 3.10 (m, 2H), 2.88 - 2.74 (m, 1H), 2.71 - 2.59 (m, 1H), 2.43 - 2.23 (m, 2H), 1.94 - 1.86 (m, 2H), 1.72 - 1.33 (m, 6H), 1.15 - 1.06 (m, 4H), 0.99 - 0.94 (m, 1H), 0.81 - 0.79 (m, 3H), 0.42 - 0.07 (m, 1H).

### Step 3

### N-[(1r,4r)-4-{1-[3-(4-{4-fluoro-2-[(3R)-3-methylmorpholin-4-carbonyl]phenyl}-1-methyl-1H-indazol-6-yl)azacyclobutan-1-yl]ethyl}cyclohexyl]ethan-1-sulfonamide 178

The crude product of Compound **178c** (102.19 mg, 157.78 µmol) was dissolved in dichloromethane (2.0 mL), followed by the addition of triethylamine (47.90 mg, 473.34 µmol), cooling to 0°C, and the addition of ethanesulfonyl chloride (40.57 mg, 315.56 µmol), and the mixture was stirred at room temperature for 12 hours. A saturated aqueous sodium bicarbonate solution (10 mL) was added to the reaction mixture, which was then extracted with dichloromethane (10 mL x 3). The organic phases were combined, washed with saturated brine, dried over anhydrous sodium sulfate, and concentrated under a reduced pressure. The resulting residues were purified by the HPLC (mobile phase: acetonitrile, water (0.1% ammonia water), elution gradient: 5%-35%), to obtain the title Compound **178** (9.8 mg, 15.66 µmol, yield: 9.93%) as pink solid.
MS m/z (ESI): 626.3 [M+1]
¹H NMR (400 MHz, DMSO-*d₆*) δ 7.81 (s, 1H), 7.71 - 7.51 (m, 2H), 7.48 - 7.26 (m, 2H), 7.20 - 6.90 (m, 2H), 4.34 - 3.99 (m, 4H), 3.78 (br s, 5H), 3.28 - 2.65 (m, 9H), 2.24 - 2.13 (m, 1H), 1.89 - 1.25 (m, 6H), 1.20 - 1.15 (m, 4H), 1.14 - 0.95 (m, 4H), 0.87 - 0.69 (m, 3H), 0.50 - 0.16 (m, 1H).

### Examples 181-1 and 181-2

### 4-{4-fluoro-2-[(3R)-3-methylmorpholin-4-carbonyl]phenyl}-6-{1-[(3S)-6-(4-methoxypiperidin-1-yl)-2-methylhexan-3-yl]azacyclobutan-3-yl}-1-methyl-1H-indazole 181-1

### 4-{4-fluoro-2-[(3R)-3-methylmorpholin-4-carbonyl]phenyl}-6-{1-[(3R)-6-(4-methoxypiperidin-1-yl)-2-methylhexan-3-yl]azacyclobutan-3-yl}-1-methyl-1H-indazole 181-2

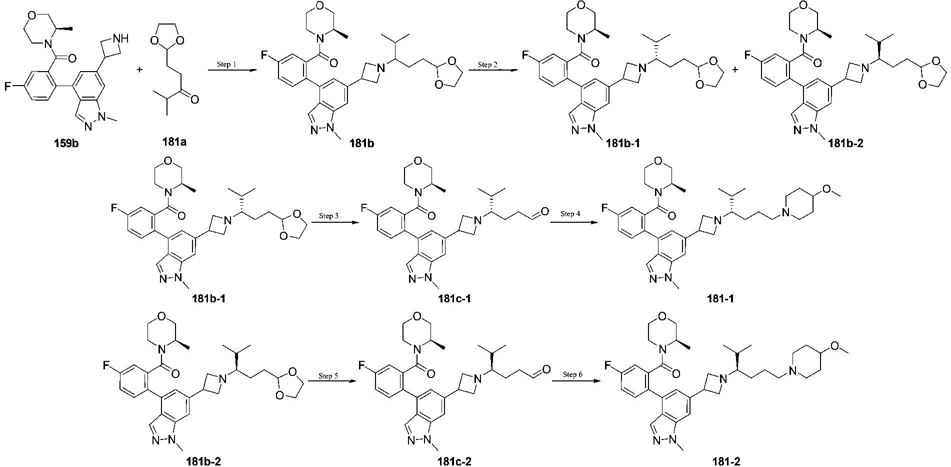

### Step 1

### 6-{1-[1-(1,3-dioxolan-2-yl)-4-methylpentan-3-yl]azacyclobutan-3-yl}-4-{4-fluoro-2-[(3R)-3-methylmorpholin-4-carbonyl]phenyl}-1-methyl-lH-indazole 181b

Compound **159b** (513 mg, 1.26 mmol) and 1-(1,3-dioxolan-2-yl)-4-methylpentan-3-one **181a** (237.92 mg, 1.38 mmol) were dissolved in methanol (10 mL), followed by the addition of ZnCl₂ (513.53 mg, 3.77 mmol), and the mixture was stirred at 50°C for 1.0 hour. The mixture was cooled to room temperature, followed by adding sodium cyanoborohydride (157.85 mg, 2.51 mmol) and stirring at 50°C for 1.0 hour. Water (30 mL) was added to the reaction mixture, which was then extracted with dichloromethane (20 mL x 3). The organic phases were combined, dried over anhydrous sodium sulfate, and concentrated under a reduced pressure. The resulting residues were purified by the silica-gel column chromatography using petroleum ether/ethyl acetate as an elution system, and the obtained crude product was purified by the reverse-phase C18 column chromatography (mobile phase: acetonitrile, water (0.1% ammonia water), elution gradient: 0%-50%), to obtain the title Compound **181b** (300 mg, 531.27 µmol, yield: 63.78%).
MS m/z (ESI): 565.3 [M+1]

### Step 2

### 6-{1-[(3S)-1-(1,3-dioxolan-2-yl)-4-methylpentan-3-yl]azacyclobutan-3-yl}-4-{4-fluoro-2-[(3R)-3-methylmorpholin-4-carbonyl]phenyl}-1-methyl-1H-indazole 181b-1

### 6-{1-[(3R)-1-(1,3-dioxolan-2-yl)-4-methylpentan-3-yl]azacyclobutan-3-yl}-4-{4-fluoro-2-[(3R)-3-methylmorpholin-4-carbonyl]phenyl}-1-methyl-1H-indazole 181b-2

Compound **181b** (300 mg, 531.27 µmol) was separated and purified by the chiral preparative SFC (preparative column: DAICEL DAICEL CHIRALCEL OD (250 mm * 30 mm,10 um), mobile phase: carbon dioxide-isopropanol; elution gradient: 40%), to obtain the title Compounds **181b-1** (106 mg, 187.71 µmol, yield: 35.33%) and **181b-2** (92 mg, 162.92 µmol, yield: 30.67%).

### Single-configuration Compound 181b-1:

MS m/z (ESI):565.3 [M+1]
Chiral HPLC analysis: retention time: 1.456 min; chromatographic column: Chiralcel OD-3 50 x 4.6 mm I.D., 3 um; mobile phase: carbon dioxide-isopropanol (0.05% diethylamide ): elution gradient: 5%-40%.

### Single-configuration Compound 181b-2:

MS m/z (ESI):565.3 [M+1]
Chiral HPLC analysis: retention time: 1.670 min; chromatographic column: Chiralcel OD-3 50 x 4.6 mm I.D., 3 um; mobile phase: carbon dioxide-isopropanol (0.05% diethylamide ): elution gradient: 5%-40%.

### Step 3

### (4S)-4-[3-(4-14-fluoro-2-[(3R)-3-methylmorpholin-4-carbonyl]phenyl]-1-methyl-1H-indazol-6-yl)azacyclobutan-1-yl]-5-methylhexanal 181c-1

Compound **181b-1** (190 mg, 336.47 µmol) was dissolved in acetonitrile (2.0 mL), followed by the addition of a hydrochloric acid solution (1.0 M; 2.0 mL). The mixture was stirred at 50°C to allow for reacting for 1.0 hours. The reaction mixture was concentrated under a reduced pressure, regulated to pH = 12 with a 10 % aqueous NaOH solution, and extracted with dichloromethane (10 mL x 3). The organic phases were combined, dried over anhydrous sodium sulfate, and concentrated under a reduced pressure, to obtain the crude product of title Compound **181c-1** (180 mg). The crude product was directly used in the next step of reaction without purification.

### Single-configuration Compound 181c-1:

MS m/z (ESI): 521.2 [M+1]

### Step 4

### 4-{4-fluoro-2-[(3R)-3-methylmorpholin-4-carbonyl]phenyl}-6-{1-[(3S)-6-(4-methoxypiperidin-1-yl)-2-methylhexan-3-yl]azacyclobutan-3-yl}-1-methyl-1H-indazole 181-1

The crude product of Compound **181c-1** (45 mg, 86.43 µmol) and 4-methoxypiperidine (10.95 mg, 95.08 µmol) were dissolved in methanol (5 mL), and stirred at room temperature for 0.5 hours under the protection of nitrogen. Sodium cyanoborohydride (10.86 mg, 172.87 µmol) was added, allowing for reacting at room temperature for 4.0 hours under the protection of nitrogen. A saturated aqueous ammonium chloride solution (20 mL) was added to the reaction mixture, which was then stirred for 0.5 hours. Water (50 mL) was added, and the mixture was extracted with ethyl acetate (50 mL x 3). The organic phases were combined, washed with saturated brine (50 mL), dried over anhydrous sodium sulfate, and concentrated under a reduced pressure. The resulting residues were purified by the preparative thin-layer chromatography using dichloromethane/methanol as an elution system. The title Compound **181-1** (19.86 mg, 32.04 µmol, yield: 37.07%) was obtained.

### Single-configuration Compound 181-1:

MS m/z (ESI):620.4 [M+1]
Chiral SFC: retention time: 1.875 min; chromatographic column: Chiralpak IC-3 50 x 4.6 mm I.D., 3 um; mobile phase: CO₂/MeOH+ACN (0.05% DEA): elution gradient: 40%.

¹H NMR (400 MHz, CDCl₃) δ 7.93 - 7.80 (m, 1H), 7.67 - 7.51 (m, 1H), 7.37 - 7.28 (m, 1H), 7.26 - 7.05 (m, 3H), 4.73 - 4.33 (m, 1H), 4.13 (s, 3H), 4.08 - 3.91 (m, 1H), 3.89 - 3.79 (m, 2H), 3.69 - 3.39 (m, 2H), 3.34 (s, 3H), 3.30 - 2.96 (m, 4H), 2.89 - 2.77 (m, 3H), 2.50 - 2.34 (m, 5H), 2.25 - 2.14 (m, 1H), 2.06 - 1.91 (m, 2H), 1.88 - 1.78 (m, 1H), 1.72 - 1.63 (m, 3H), 1.42 - 1.32 (m, 2H), 1.29 - 1.22 (m, 2H), 1.10 - 1.02 (m, 1H), 0.96 - 0.89 (m, 6H), 0.63 - 0.04 (m, 2H).

### Step 5

### (4R)-4-[3-(4-{4-fluoro-2-[(3R)-3-methylmorpholin-4-carbonyl]phenyl}-1-methyl-1H-indazol-6-yl)azacyclobutan-1-yl]-5-methylhexanal 181c-2

Compound 181b-2 (180 mg, 318.76 µmol) was dissolved in acetonitrile (2.0 mL), followed by the addition of a hydrochloric acid solution (1.0 M; 2.0 mL). The mixture was stirred at 50°C to allow for reacting for 1.0 hours. The reaction mixture was concentrated under a reduced pressure, regulated to pH = 12 with a 10 % aqueous NaOH solution, and extracted with dichloromethane (10 mL x 3). The organic phases were combined, dried over anhydrous sodium sulfate, and concentrated under a reduced pressure, to obtain the crude product of title Compound 181c-2 (180 mg). The crude product was directly used in the next step of reaction without purification.

### Single-configuration Compound 181c-2:

MS m/z (ESI): 521.3 [M+1]

### Step 6

### 4-{4-fluoro-2-[(3R)-3-methylmorpholin-4-carbonyl]phenyl}-6-{1-[(3R)-6-(4-methoxypiperidin-1-yl)-2-methylhexan-3-yl]azacyclobutan-3-yl}-1-methyl-1H-indazole 181-2

The crude product of Compound **181c-2** (45 mg, 86.43 µmol) and 4-methoxypiperidine (11.95 mg, 103.72 µmol) were dissolved in methanol (1.5 mL), followed by the addition of acetic acid (7.79 mg, 129.65 µmol), and the mixture was stirred at room temperature for 0.5 hours. Sodium cyanoborohydride (16.3 mg, 259.3 µmol) was added, allowing for reacting at room temperature for 11.0 hours under the protection of nitrogen. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonia water), elution **gradient: 41%-71%)** .The title Compound **181-2** (23.72 mg, 38.27 µmol, yield: 44.28%) was obtained.

### Single-configuration Compound 181-2:

MS m/z (ESI):620.5 [M+1]
Chiral SFC: retention time: 1.170 min; chromatographic column: Chiralcel OD-3 50×4.6 mm I.D., 3 um; mobile phase: CO₂/MeOH (0.05% DEA): elution gradient: 5%-40%.

¹H NMR (400 MHz, CD₃OD) δ 7.78 - 7.67 (m, 1H), 7.55 - 7.40 (m, 1H), 7.39 - 7.28 (m, 1H), 7.25 - 7.09 (m, 2H), 7.08 - 6.91 (m, 1H), 4.31 - 4.07 (m, 1H), 3.96 - 3.89 (m, 3H), 3.83 - 3.61 (m, 4H), 3.48 - 3.33 (m, 1H), 3.27 - 3.13 (m, 5H), 3.07 - 2.68 (m, 3H), 2.66 - 2.55 (m, 2H), 2.39 - 2.22 (m, 1H), 2.22 - 2.14 (m, 2H), 2.12 - 1.99 (m, 3H), 1.79 - 1.68 (m, 3H), 1.33 (s, 7H), 1.09 - 1.05 (m, 1H), 0.92 - 0.88 (m, 1H), 0.82 - 0.73 (m, 6H), 0.37 - -0.01 (m, 1H).

### Examples 186-1 and 186-2

### [(4S)-4-[3-(4-{2-[(3R,5R)-3,5-dimethylmorpholin-4-carbonyl]-4-fluorophenyl}-1-methyl-1H-indazol-6-yl)azacyclobutan-1-yl]-5-methylhexyl](2-methoxyethyl)methylamine 186-1

### [(4R)-4-[3-(4-{2-[(3R,5R)-3,5-dimethylmorpholin-4-carbonyl]-4-fluorophenyl}-1-methyl-1H-indazol-6-yl)azacyclobutan-1-yl]-5-methylhexyl](2-methoxyethyl)methylamine 186-2

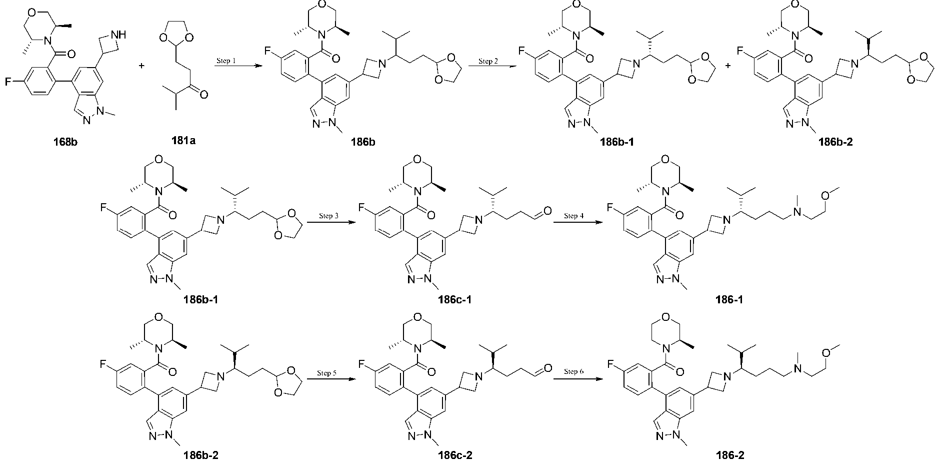

### Step 1

### 4-{2-[(3R,5R)-3,5-dimethylmorpholin-4-carbonyl]-4-fluorophenyl}-6-{1-[1-(dioxolan-2-yl)-4-methylpentan-3-yl]methylpentan-3-yl}-1-methyl-1H-indazole 186b

Compound 168b (3.49 g, 8.26 mmol) and 1-(1,3-dioxolan-2-yl)-4-methylpentan-3-one 181a (1.56 g, 9.09 mmol) were dissolved in methanol (20 mL), followed by the addition of ZnCl₂ (3.38 g, 24.78 mmol), and the mixture was stirred at 50°C for 1.0 hour. The mixture was cooled to room temperature, followed by adding sodium cyanoborohydride (1.04 g, 16.52 mmol) and stirring at 50°C for 13 hour. A saturated aqueous sodium carbonate solution (50 mL) was added to the reaction mixture, which was then extracted with dichloromethane (30 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **186b** (3.89 g, 6.72 mmol, yield: 81.37%) as yellow solid.
MS m/z (ESI): 579.3 [M+1]

### Step 2

### 4-{2-[(3R,5R)-3,5-dimethylmorpholin-4-carbonyl]-4-fluorophenyl}-6-{1-[(3S)-1-(dioxolan-2-yl)-4-methylpentan-3-yl]methylpentan-3-yl}-1-methyl-1H-indazole 186b-1

### 4-{2-[(3R,5R)-3,5-dimethylmorpholin-4-carbonyl]-4-fluorophenyl}-6-{1-[(3R)-1-(dioxolan-2-yl)-4-methylpentan-3-yl]methylpentan-3-yl}-1-methyl-1H-indazole 186b-2

Compound **186b** (3.89 g, 6.72 mmol) was separated and purified by the chiral preparative SFC (preparative column: DAICEL CHIRALPAK IG (250 mm * 30 mm,10 um), mobile phase: carbon dioxide-ethanol (0.1% ammonia water); elution gradient: 40%), to obtain the title Compounds **186b-1** (1.45 g, 2.51 mmol, yield: 37.28%) and **186b-2** (1.89 g, 3.27 mmol, yield: 48.59%).

### Single-configuration Compound 186b-1:

MS m/z (ESI):579.1 [M+1]
Chiral HPLC analysis: retention time: 1.168 min; chromatographic column: Chiralpak IG-3 50 x 4.6 mm I.D., 3 um; mobile phase: carbon dioxide-ethanol (0.05% diethylamide ): elution gradient: 40%.

### Single-configuration Compound 186b-2:

MS m/z (ESI):579.2 [M+1]
Chiral HPLC analysis: retention time: 0.738 min; chromatographic column: Chiralpak IG-3 50 x 4.6 mm I.D., 3 um; mobile phase: carbon dioxide-ethanol (0.05% diethylamide ): elution gradient: 40%.

¹H NMR (400 MHz, CDCl₃) δ 7.79 (s, 1H), 7.50 (dd, J = 5.7, 7.9 Hz, 1H), 7.34 (s, 1H), 7.27 - 7.21 (m, 2H), 7.11 - 6.97 (m, 1H), 4.84 (t, J = 4.5 Hz, 1H), 4.13 (s, 3H), 3.97 - 3.94 (m, 2H), 3.86 - 3.82 (m, 2H), 3.80 - 3.62 (m, 2H), 3.51 - 3.32 (m, 2H), 3.09 - 2.96 (m, 1H), 2.70 - 2.54 (m, 1H), 2.44 - 2.15 (m, 2H), 2.02 - 1.83 (m, 2H), 1.80 - 1.71 (m, 3H), 1.67 - 1.40 (m, 4H), 1.04 (s, 3H), 0.98 (d, J = 6.6 Hz, 6H), 0.87 (s, 3H).

### Step 3

### (4S)-4-[3-(4-{2-[(3R,5R)-3,5-dimethylmorpholin-4-carbonyl]-4-fluorophenyl}-1-methyl-1H-indazol-6-yl)azacyclobutan-1-yl]-5-methylhexanal 186c-1

Compound **186b-1** (153 mg, 264.38 µmol) was dissolved in acetonitrile (2.0 mL), followed by the addition of a hydrochloric acid solution (1.0 M; 2.0 mL). The mixture was stirred at 50°C to allow for reacting for 1.0 hours. The reaction mixture was concentrated under a reduced pressure, followed by adding a saturated aqueous sodium carbonate solution (10 mL) and extracting with dichloromethane (10 mL x 3). The organic phases were combined, dried over anhydrous sodium sulfate, and concentrated under a reduced pressure, to obtain the crude product of title Compound **186c-1** (140 mg, 261.85 µmol, yield: 99.04%). The crude product was directly used in the next step of reaction without purification.

### Single-configuration Compound 186c-1:

MS m/z (ESI): 535.3 [M+1]

### Step 4

### [(4S)-4-[3-(4-{2-[(3R,5R)-3,5-dimethylmorpholin-4-carbonyl]-4-fluorophenyl}-1-methyl-1H-indazol-6-yl)azacyclobutan-1-yl]-5-methylhexyl](2-methoxyethyl)methylamine 186-1

The crude product of Compound **186c-1** (140 mg, 261.85 µmol) and 2-methoxy-N-methyl-ethylamine (35.01 mg, 392.77 µmol) was dissolved in methanol (2 mL), and stirred at room temperature for 1.0 hour under the protection of nitrogen. Acetic acid (15.72 mg, 261.85 µmol) and sodium cyanoborohydride (49.36 mg, 785.54 µmol) were added, allowing for reacting at room temperature for 12 hours under the protection of nitrogen. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% formic acid), elution gradient: 0%-30%), to obtain the title Compound **186-1** (73.28 mg, 120.57 µmol, yield: 46.04%).

### Single-configuration Compound 186-1:

MS m/z (ESI):608.4 [M+1]
Chiral SFC: retention time: 1.101 min; chromatographic column: Chiralcel OD-3 50×4.6 mm I.D., 3um,; mobile phase: CO₂/MeOH (0.05% DEA): elution gradient: 5%-40%.

¹H NMR (400 MHz, DMSO-*d₆*) δ 7.79 - 7.29 (m, 5H), 7.12 - 6.90 (m, 1H), 4.08 - 4.04 (m, 3H), 3.80 - 3.71 (m, 3H), 3.61 - 3.48 (m, 2H), 3.46 - 3.39 (m, 2H), 3.32 - 2.99 (m, 7H), 2.62 - 2.54 (m, 2H), 2.53 - 2.47 (m, 3H), 2.44 - 2.37 (m, 2H), 2.26 - 2.15 (m, 4H), 1.84 - 1.71 (m, 1H), 1.54 - 1.41 (m, 2H), 1.34 - 1.15 (m, 2H), 1.05 - 0.55 (m, 11H).

### Step 5

### (4R)-4-[3-(4-{2-[(3R,5R)-3,5-dimethylmorpholin-4-carbonyl]-4-fluorophenyl}-1-methyl-1H-indazol-6-yl)azacyclobutan-1-yl]-5-methylhexanal 186c-2

Compound 186b-2 (1.89 g, 3.27 mmol) was dissolved in acetonitrile (20 mL), followed by the addition of a hydrochloric acid solution (1.0 M; 20 mL). The mixture was stirred at 50°C to allow for reacting for 1.0 hours. The reaction mixture was concentrated under a reduced pressure, regulated to pH = 12 with a 10 % aqueous NaOH solution, and extracted with dichloromethane (10 mL x 3). The organic phases were combined, dried over anhydrous sodium sulfate, and concentrated under a reduced pressure, to obtain the crude product of title Compound 186c-2 (1.34 g, 2.51 mmol, yield: 76.74%). The crude product was directly used in the next step of reaction without purification.

### Single-configuration Compound 186c-2:

MS m/z (ESI): 535.2 [M+1]

### Step 6

### [(4R)-4-[3-(4-{2-[(3R,5R)-3,5-dimethylmorpholin-4-carbonyl]-4-fluorophenyl}-1-methyl-1H-indazol-6-yl)azacyclobutan-1-yl]-5-methylhexyl](2-methoxyethyl)methylamine 186-2

Compound 186c-2 (150 mg, 280.55 µmol) and 2-methoxy-N-methyl-ethylamine (37.51 mg, 420.83 µmol) were dissolved in methanol (2.0 mL), followed by the addition of acetic acid (16.85 mg, 280.55 µmol), and the mixture was stirred at room temperature for 1.0 hour. Sodium cyanoborohydride (52.89 mg, 841.65 µmol) was added, allowing for reacting at room temperature for 12.0 hours under the protection of nitrogen. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0. 1% ammonia water), elution gradient: 46%-76%). The title Compound **186-2** (100 mg, 164.53 µmol, yield: 58.64%) was obtained.

### Single-configuration Compound 186-2:

**MS m/z** (ESI):608.5 [M+1]
Chiral SFC: retention time: 0.959 min; chromatographic column: Chiralpak AD-3 50×4.6 mm I.D., 3 um; mobile phase: CO₂/MeOH (0.05% DEA): elution gradient: 5%-40%.

¹H NMR (400 MHz, CD₃OD) δ 7.80 (s, 1H), 7.64 - 7.51 (m, 2H), 7.43 - 7.28 (m, 2H), 7.09 (s, 1H), 4.13 (s, 3H), 3.98 - 3.79 (m, 4H), 3.77 - 3.63 (m, 2H), 3.52 (t, J = 5.6 Hz, 2H), 3.37 - 3.33 (m, 5H), 3.12 (d, J = 10.0 Hz, 1H), 2.61 (t, J = 5.6 Hz, 3H), 2.43 (t, J = 7.8 Hz, 2H), 2.35 (d, J = 9.2 Hz, 1H), 2.29 (s, 3H), 2.25 (q, J = 4.4 Hz, 1H), 1.97 - 1.86 (m, 1H), 1.66 - 1.54 (m, 2H), 1.47 - 1.37 (m, 1H), 1.36 - 1.26 (m, 1H), 1.08 (d, J = 4.8 Hz, 3H), 0.98 (d, J = 7.2 Hz, 3H), 0.94 (d, J = 7.2 Hz, 3H), 0.74 (s, 3H).

### Example 187

### N-[(1r,4r)-4-{[3-(1-fluoro-8-{4-fluoro-2-[(2R)-2-methylpyrrolidin-1-carbonyl]phenyl}-3-methylimidazo[1,5-a]yridin-6-yl)azacyclobutan-1-yl]methyl}cyclohexyl]ethan-1-sulfonamide 187

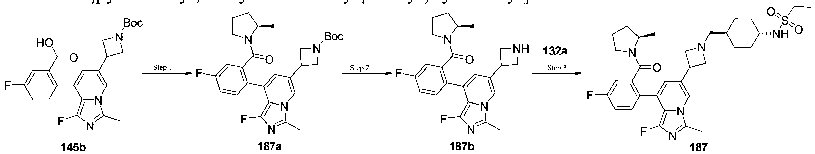

### Step 1

### 3-(1-fluoro-8-{4-fluoro-2-[(2R)-2-methylpyrrolidin-1-carbonyl]phenyl}-3-methylimidazo[1,5-a]pyridin-6-yl)azacyclobutan-1-tert-butyl carboxylate 187a

Compound **145b** (318 mg, 717.12 µmol), triethylamine (217.69 mg, 2.15 mmol) and HATU(327.20 mg, 860.54 µmol) were dissolved in dichloromethane (5.0 mL), and stirred at room temperature for 10 min, followed by the addition of (2R)-2-methylpyrrolidine (73.27 mg, 860.54 µmol). The reaction mixture was stirred at 25°C for 12 hours. A saturated aqueous sodium bicarbonate solution (20 mL) was added to the reaction mixture, which was then extracted with dichloromethane (20 mL × 3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **187a** (340 mg, 665.92 µmol, yield: 92.86%) as yellow solid.
MS m/z (ESI): 511.2 [M+1]

### Step 2

### (2R)-1-{2-[6-(azacyclobutan-3-yl)-1-fluoro-3-methylimidazo[1,5-a]pyridin-8-yl]-5-fluorobenzoyl}-2-methylpyrrolidine 187b

Compound **187a** (210 mg, 411.30 µmol) was dissolved in dichloromethane (3.0 mL), followed by the addition of trifluoroacetic acid (1.48 g, 12.98 mmol, 1 mL) at 25°C. The mixture was stirred at room temperature to allow for reacting for 1.0 hours. The reaction mixture was concentrated under a reduced pressure, diluted with dichloromethane (10 mL), and regulated to PH = 11 with 10% aqueous NaOH solution, the mixture was extracted with dichloromethane (15 mL×3), and the organic phase was dried over anhydrous sodium sulfate, filtered and concentrated under a reduced pressure, to obtain the crude product of title Compound **187b** (200 mg, 487.26 µmol, yield: 118.47%). The crude product was directly used in the next step of reaction without purification.
MS m/z (ESI): 411.2 [M+1]

### Step 3

### N-[(1r,4r)-4-{[3-(1-fluoro-8-{4-fluoro-2-[(2R)-2-methylpyrrolidin-1-carbonyl]phenyl}-3-methylimidazo[1,5-a]pyridin-6-yl)azacyclobutan-1-yl]methyl}cyclohexyl]ethan-1-sulfonamide 187

The crude product of Compound **187b** (96.46 mg, 183.91 µmol), *N*-[(1*r*,4*r*)-4-formylcyclohexyl]ethan-1-sulfonamide **132a**(48.40 mg, 220.69 µmol) and triethylamine (55.83 mg, 551.73 µmol) were dissolved in methanol (2.0 mL), and stirred at room temperature for 1.0 hour. Sodium cyanoborohydride (26.79 mg, 426.33 µmol) was added, and the mixture was stirred at room temperature for 11 hours. A saturated aqueous sodium bicarbonate solution (15 mL) was added to the reaction mixture, which was then extracted with dichloromethane (15 mL x 3). The organic phases were combined, dried over anhydrous sodium sulfate, and concentrated under a reduced pressure. The resulting residues were purified by the HPLC (mobile phase: acetonitrile, water (0.1% formic acid), elution gradient: 14%-44%), to obtain the title Compound **187** (39.33 mg, 64.08 µmol, yield: 34.84 %) as yellow solid.
MS m/z (ESI): 614.2 [M+1]
¹H NMR (400 MHz, DMSO-*d*₆) δ 8.17 (s, 1H), 8.21 - 8.11 (m, 1H), 7.83 (s, 1H), 7.57 - 7.48 (m, 1H), 7.42 - 7.29 (m, 2H), 7.02 - 6.95 (m, 1H), 6.73 - 6.57 (m, 1H), 3.98 - 3.89 (m, 1H), 3.61 - 3.51 (m, 3H), 3.18 - 3.04 (m, 3H), 3.00 - 2.92 (m, 3H), 2.54 (s, 3H), 2.34 - 2.26 (m, 2H), 1.93 - 1.69 (m, 5H), 1.66 - 1.47 (m, 2H), 1.42 - 1.30 (m, 1H), 1.24 - 1.11 (m, 6H), 0.99 - 0.75 (m, 5H).

### Examples 191-1 and 191-2

### N-[(4r)-4-({3-[(4-{4-fluoro-2-[(3R)-3-methylmorpholin-4-carbonyl]phenyl}-1-methyl-1H-indazol-6-yl)methyl]azacyclobutan-1-yl}methyl)-4-methylcyclohexyl]tert-butyl carbamate 191-1

### N-[(4s)-4-({3-[(4-{4-fluoro-2-[(3R)-3-methylmorpholin-4-carbonyl]phenyl}-1-methyl-1H-indazol-6-yl)methyl]azacyclobutan-1-yl}methyl)-4-methylcyclohexyl]tert-butyl carbamate 191-2

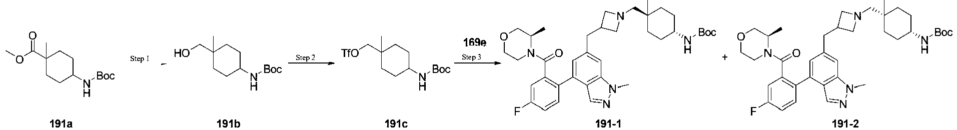

### Step 1

### N-[4-(hydroxymethyl)-4-methylcyclohexyl]tert-butyl carbamate 191b

4-{[(Tert-butoxy)carbonyl]amino}-1-methylcyclohexan-1-methyl carboxylate **191a** (770 mg, 2.84 mmol) was dissolved in THF (8.3 mL), and cooled to 0°C under a nitrogen atmosphere, followed by the slow addition of LiAlH₄ (161.55 mg, 4.26 mmol), and the mixture was stirred at room temperature for 12 hours. Ethyl acetate was added to quench the reaction, the reaction mixture was filtered, and the filtrate was concentrated under a reduced pressure. The resulting residues were purified by the silica-gel column chromatography using petroleum ether/ethyl acetate as an elution system, to obtain the title Compound **191b** (230 mg, 945.17 µmol, yield: 33.31%) as colorless oil.

¹H NMR (400 MHz, DMSO-*d₆*) δ 7.88 (s, 1H), 7.77 - 7.48 (m, 2H), 7.42 - 7.05 (m, 3H), 4.63 - 4.20 (m, 1H), 4.16 - 4.06 (m, 3H), 3.97 - 3.34 (m, 7H), 3.27 - 2.81 (m, 4H), 2.54 - 2.30 (m, 2H), 2.22 - 1.48 (m, 6H), 1.45 - 1.39 (m, 9H), 1.29 - 1.21 (m, 3H), 1.19 - 1.11 (m, 2H), 1.09 - 1.04 (m, 1H), 0.98 - 0.90 (m, 3H), 0.51 - 0.18 (m, 1H).

### Step 2

### N-{4-methyl-4-[(trifluoromethanesulfonyloxy)methyl]cyclohexyl}tert-butyl carbamate 191c

Compound **191b** (230 mg, 945.17 µmol) was dissolved in dichloromethane (4.0 mL), followed by the addition of triethylamine (143.46 mg, 1.42 mmol) at 25°C. The reaction mixture was cooled to -78°C, followed by dropwise adding the solution of trifluoromethanesulfonic anhydride (346.67 mg, 1.23 mmol) in dichloromethane (3 mL) slowly. After dropwise addition, the reaction mixture was warmed to 0°C, and stirred to allow for reacting for 1.0 hour. The reaction mixture was directly used in the next step of reaction without treatment.

### Step 3

### N-[(4r)-4-({3-[(4-{4-fluoro-2-[(3R)-3-methylmorpholin-4-carbonyl]phenyl}-1-methyl-1H-indazol-6-yl)methyl]azacyclobutan-1-yl}methyl)-4-methylcyclohexyl]tert-butyl carbamate 191-1

### N-[(4s)-4-({3-[(4-{4-fluoro-2-[(3R)-3-methylmorpholin-4-carbonyl]phenyl}-1-methyl-1H-indazol-6-yl)methyl]azacyclobutan-1-yl}methyl)-4-methylcyclohexyl]tert-butyl carbamate 191-2

The solution (2 mL) of Compound **191c** resulting from the previous step of reaction was cooled to 0°C, followed by the dropwise addition of triethylamine (19.16 mg, 189.35 µmol) and the dichloromethane solution (1.0 mL) of Compound **169e**(80 mg, 189.35 µmol). The reaction mixture was stirred at room temperature for 12 hours. Water (10 mL) was added to the reaction mixture, which was then extracted with dichloromethane (10 mL x 3). The organic phases were combined, washed with saturated brine, dried over anhydrous sodium sulfate, and concentrated under a reduced pressure. The resulting residues were purified by the HPLC (mobile phase: acetonitrile, water (0.1% formic acid), elution gradient: 24%-44%), to obtain the title Compounds **191-1** (1.01 mg, 1.56 µmol,yield: 0.8%) and **191-2** (1.6 mg, 2.47 µmol, yield: 1.3%).

### Single-configuration Compound 191-1:

MS m/z (ESI):648.3 [M+1]
HPLC: retention time: 3.232 min.

¹H NMR (400 MHz, DMSO-*d₆*) δ 8.18 (s, 0.54H), 7.87 - 7.73 (m, 1H), 7.64 - 7.29 (m, 4H), 6.95 - 6.77 (m, 1H), 4.34 - 3.99 (m, 4H), 3.59 - 3.48 (m, 1H), 3.04 - 2.84 (m, 7H), 2.70 - 2.66 (m, 1H), 2.63 (s, 3H), 2.36 - 2.26 (m, 4H), 2.07 (s, 2H), 1.80 - 1.73 (m, 2H), 1.59 - 1.48 (m, 2H), 1.46 - 1.41 (m, 1H), 1.38 (s, 9H), 1.26 - 1.17 (m, 2H), 1.15 - 1.10 (m, 1H), 1.02 - 0.78 (m, 4H), 0.46 - 0.13 (m, 1H).

### Single-configuration Compound 191-2:

MS m/z (ESI):648.3 [M+1]
HPLC: retention time: 3.116 min.

¹H NMR (400 MHz, DMSO-*d₆*) δ 8.25 - 8.22 (m, 0.5H), 7.85 - 7.71 (m, 1H), 7.64 - 7.26 (m, 4H), 6.94 - 6.60 (m, 2H), 4.31 - 4.12 (m, 1H), 4.08 - 4.01 (m, 3H), 3.86 - 3.76 (m, 1H), 3.26 - 2.83 (m, 10H), 2.80 - 2.60 (m, 2H), 2.46 - 2.31 (m, 1H), 2.19 (s, 2H), 1.51 (s, 2H), 1.36 (s, 9H), 1.31 - 1.21 (m, 4H), 1.20 - 1.07 (m, 3H), 1.04 - 0.92 (m, 1H), 0.81 (s, 3H), 0.49 - 0.13 (m, 1H).

### Example 194

### {5-fluoro-4-[3-(4-{4-fluoro-2-[(3R)-3-methylmorpholin-4-carbonyl]phenyl}-1-methyl-1H-indazol-6-yl)azacyclobutan-1-yl]-5-methylhexyl}(2-methoxyethyl)methylamine 194

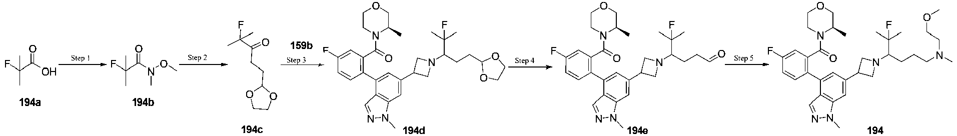

### Step 1

### 2-fluoro-N-methoxy-N,2-dimethylpropionamide 194b

2-Fluoro-2-methylpropionic acid **194a** (10 g, 94.25 mmol) and triethylamine (36.40 g, 359.72 mmol) were dissolved in dichloromethane (200 mL), followed by the addition of EDCI (23.49 g, 122.53 mmol) and HOBt (16.56 g, 122.53 mmol), and the mixture was stirred at room temperature for 5.0 min, followed by the addition of N-methoxymethylamine (13.79 g, 141.38 mmol, hydrochloride). The reaction mixture was stirred at room temperature for 12 hours. Water (300 mL) was added to the reaction mixture, which was then extracted with dichloromethane (100 mL×3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **194b** (7.1 g, 47.60 mmol, yield: 50.5%) as white solid.
¹H NMR (400 MHz, CDCl₃) δ 3.72 (s, 3H), 3.24 (s, 3H),1.64 (s, 3H), 1.58 (s, 3H).

### Step 2

### 1-(1,3-dioxolan-2-yl)-4-fluoro-4-methylpentan-3-one 194c

Under the protection of nitrogen, Mg(1.61 g, 66.29 mmol) was suspended in THF(20 mL), followed by the addition of I₂(50 mg, 197.00 µmol). 2-(2-Bromoethyl)-1,3-dioxolane (10 g, 55.24 mmol) was dissolved in tetrahydrofuran (80 mL), and placed in a dropping funnel. A bromide solution (about 8.0 mL) was dropwise added to the suspension of Mg, and the reaction mixture was heated to 50°C to initiate the reaction, followed by the slow dropwise addition of the residual bromide solution. After dropwise addition, the mixture as stirred at room temperature for 1 hour. At 0°C, the prepared alkyl magnesium bromide tetrahydrofuran solution was slowly dropwise added to the solution of Compound **194b** (3.6 g, 24.13 mmol) in THF(30 mL). The reaction mixture was stirred at room temperature for 12 hours. A saturated aqueous ammonium chloride solution (100 mL) was added to the reaction mixture, which was then extracted with ethyl acetate (70 mL×3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **194c** (500 mg, 2.63 mmol, yield: 10.89%) as yellow liquid.

¹H NMR (400 MHz, CDCl₃) δ 4.95 (t, J = 4.2 Hz, 1H), 4.01 - 3.80 (m, 4H), 2.81 (dt, J = 3.0, 7.4 Hz, 2H), 1.99 (dt, J = 4.3, 7.3 Hz, 2H), 1.50 - 1.42 (m, 6H).

### Step 3

### 6-{1-[1-(1,3-dioxolan-2-yl)-4-fluoro-4-methylpentan-3-yl]azacyclobutan-3-yl}-4-{4-fluoro-2-[(3R)-3-methylmorpholin-4-carbonyl]phenyl}-1-methyl-1H-indazole 194d

Compound **159b** (215 mg, 526.36 µmol) and Compound **194c** (120.14 mg, 631.63 µmol) were dissolved in methanol (3.0 mL), followed by the addition of ZnCl₂ (143.48 mg, 1.05 mmol), and the mixture was stirred at 50°C for 1.0 hour. The mixture was cooled to room temperature, followed by adding sodium cyanoborohydride (99.23 mg, 1.58 mmol) and stirring at 50°C for 12 hour. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the preparative thin-layer chromatography using dichloromethane/methanol as an elution system, to obtain the title Compound **194d** (56 mg, 96.11 µmol, yield: 18.26%).
MS m/z (ESI): 583.5 [M+1]

### Step 4

### 5-fluoro-4-[3-(4-{4-fluoro-2-[(3R)-3-methylmorpholin-4-carbonyl]phenyl}-1-methyl-1H-indazol-6-yl)azacyclobutan-1-yl]-5-methylhexanal 194e

Compound **194d** (56 mg, 96.11 µmol) was dissolved in acetonitrile (2.0 mL), followed by the addition of a hydrochloric acid solution (1.0 M; 2.0 mL). The mixture was stirred at 50°C to allow for reacting for 1.0 hours. The reaction mixture was concentrated under a reduced pressure, diluted with dichloromethane (20 mL), and then washed with a saturated aqueous sodium carbonate solution (20 mL). The organic phases were combined, dried over anhydrous sodium sulfate, and concentrated under a reduced pressure, to obtain the crude product of title Compound **194e** (50 mg, 92.83 µmol, yield: 96.59%) as yellow solid. The crude product was directly used in the next step of reaction without purification.
MS m/z (ESI): 539.3 [M+1]

### Step 5

### {5-fluoro-4-[3-(4-{4-fluoro-2-[(3R)-3-methylmorpholin-4-carbonyl]phenyl}-1-methyl-1H-indazol-6-yl)azacyclobutan-1-yl]-5-methylhexyl}(2-methoxyethyl)methylamine 194

The crude product of Compound **194e** (50 mg, 92.83 µmol) and 2-methoxy-N-methyl-ethylamine (9.93 mg, 111.39 µmol) were dissolved in methanol (1.0 mL), followed by the addition of acetic acid (5.57 mg, 92.83 µmol), and the mixture was stirred at room temperature for 0.5 hours. Sodium cyanoborohydride (17.50 mg, 278.49 µmol) was added, allowing for reacting at room temperature for 11.0 hours under the protection of nitrogen. A saturated aqueous sodium bicarbonate solution (10 mL) was added to the reaction mixture, which was then extracted with dichloromethane (20 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 0%-30%), to obtain the title Compound **194** (14.39 mg, 23.52 µmol, yield: 25.34%).
MS m/z (ESI):612.6 [M+1]
¹H NMR (400 MHz, CD₃OD) δ 8.49 (s, 1.18H), 8.01 - 7.82 (m, 1H), 7.80 - 7.58 (m, 1H), 7.57 - 7.16 (m, 4H), 4.49 - 3.77 (m, 7H), 3.72 - 3.31 (m, 10H), 3.31 - 2.96 (m, 5H), 2.95 - 2.63 (m, 5H), 2.56 - 2.32 (m, 1H), 1.99 - 1.83 (m, 2H), 1.53 - 1.33 (m, 8H), 1.27 - 1.04 (m, 2H), 0.55 - 0.09 (m, 1H).

### Example 195

### 3-{1-[3-(4-{4-fluoro-2-[(3R)-3-methylmorpholin-4-carbonyl]phenyl}-1-methyl-1H-indazol-6-yl)azacyclobutan-1-yl]-2-methylpropyl}-N-(2-methoxyethyl)-N-methylcyclobutan-1-amine 195

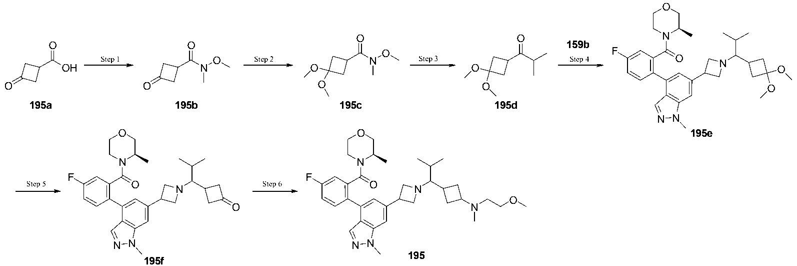

### Step 1

### N-methoxy-N-methyl-3-oxocyclobutan-1-formamide 195b

3-Oxocyclobutan-1-carboxylic acid **195a** (10 g, 87.64 mmol) and triethylamine (26.61 g, 262.93 mmol) were dissolved in dichloromethane (200 mL), followed by the addition of EDCI (20.16 g, 105.17 mmol) and HOBt(14.21 g, 105.17 mmol), and the mixture was stirred at room temperature for 5.0 min, followed by the addition of N-methoxymethylamine (10.26 g, 105.17 mmol, hydrochloride). The reaction mixture was stirred at room temperature for 12 hours. Water (300 mL) was added to the reaction mixture, which was then extracted with dichloromethane (100 mL×3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **195b** (8.5 g, 54.08 mmol, yield: 61.71%) as yellow liquid. ¹H NMR (400 MHz, CDCl₃) δ 3.73 (s, 3H), 3.63 - 3.53 (m, 1H), 3.52 - 3.41 (m, 2H), 3.29 - 3.14 (m, 5H).

### Step 2

### N,3,3-trimethoxy-N-methylcyclobutan-1-formamide 195c

Compound **195b** (4.0 g, 25.45 mmol) was dissolved in methanol (50 mL), followed by the addition of trimethyl orthoformate (5.40 g, 50.90 mmol) and 4-toluenesulfonic acid monohydrate (484.12 mg, 2.55 mmol), and the mixture was stirred at 50°C for 12 hours. The reaction mixture was concentrated under a reduced pressure, and the residues were dissolved with dichloromethane (60 mL), and washed with a saturated aqueous sodium bicarbonate solution (30 mL×3) and saturated brine (30 mL). The organic phase was dried over anhydrous sodium sulfate, filtered and concentrated under a reduced pressure, to obtain the title Compound **195c** (4.5 g, 22.14 mmol, yield: 87%) as colorless liquid.

¹H NMR (400 MHz, CDCl₃) δ 3.66 (s, 3H), 3.20 - 3.14 (m, 10H), 2.39 (br d, J = 8.9 Hz, 4H).

### Step 3

### 1-(3,3-dimethoxycyclobutyl)-2-methylpropan-1-one 195d

Under the protection of nitrogen, Compound **195c** (2.0 g, 9.84 mmol) was dissolved in tetrahydrofuran (30 mL), and cooled to 0°C. An isopropylmagnesium chloride solution (2 M, 14.76 mL) was added dropwise to the reaction mixture, after which the reaction mixture was slowly warmed to room temperature and stirred for 2.0 hours . A saturated aqueous ammonium chloride solution (20 mL) was added to the reaction mixture, which was then extracted with ethyl acetate (30 mL×3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **195d** (1.1 g, 5.91 mmol, yield: 60.02%) as colorless oil.

¹H NMR (400 MHz, CDCl₃) δ 3.19 (s, 3H), 3.16 - 3.09 (m, 4H), 2.65 (td, J = 6.9, 13.9 Hz, 1H), 2.39 - 2.27 (m, 4H), 1.10 (d, J = 6.9 Hz, 6H).

### Step 4

### 6-{1-[1-(3,3-dimethoxycyclobutyl)-2-methylpropyl]azacyclobutan-3-yl}-4-{4-fluoro-2-[(3R)-3-methylmorpholin-4-carbonyl]phenyl}-1-methyl-1H-indazole 195e

Compound **159b** (200 mg, 489.63 µmol) and Compound **195d** (136.79 mg, 734.45 µmol) were dissolved in methanol (2.0 mL), followed by the addition of ZnCl₂ (133.47 mg, 979.27 µmol), and the mixture was stirred at 50°C for 1.0 hour. The mixture was cooled to room temperature, followed by adding sodium cyanoborohydride (92.31 mg, 1.47 mmol) and stirring at 50°C for 10 hour. A saturated aqueous sodium bicarbonate solution (30 mL) was added to the reaction mixture, which was then extracted with dichloromethane (20 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **195e** (250 mg, 431.99 µmol, yield: 88.23%).
MS m/z (ESI): 579.4 [M+1]
¹H NMR (400 MHz, CDCl₃) δ 7.97 - 7.80 (m, 1H), 7.77 - 7.48 (m, 2H), 7.47 - 7.06 (m, 3H), 4.48 - 4.06 (m, 4H), 3.91 - 3.78 (m, 3H), 3.65 - 3.34 (m, 4H), 3.19 - 3.09 (m, 6H), 3.07 - 2.82 (m, 2H), 2.58 - 2.25 (m, 4H), 2.16 - 2.02 (m, 2H), 1.93 - 1.80 (m, 2H), 1.67 - 1.40 (m, 1H), 1.24 (d, J = 7.2 Hz, 1H), 1.07 (d, J = 6.8 Hz, 1H), 0.98 - 0.89 (m, 6H), 0.56 - 0.15 (m, 1H).

### Step 5

### 3-{1-[3-(4-{4-fluoro-2-[(3R)-3-methylmorpholin-4-carbonyl]phenyl}-1-methyl-1H-indazol-6-yl)azacyclobutan-1-yl]-2-methylpropyl}cyclobutan-1-one 195f

Compound **195e** (200 mg, 345.59 µmol) was dissolved in dichloromethane (6.0 mL), followed by the addition of trifluoroacetic acid (2.0 mL). The mixture was stirred at room temperature to allow for reacting for 2.0 hour. The reaction mixture was concentrated under a reduced pressure, diluted with a saturated aqueous sodium carbonate solution (20 mL), and extracted with dichloromethane (20 mL x 3). The organic phases were combined, dried over anhydrous sodium sulfate, and concentrated under a reduced pressure, to obtain the title Compound **195f** (180 mg, 337.933 µmol, yield: 97.78%) as yellow solid.
MS m/z (ESI): 533.3 [M+1]

### Step 6

### 3-{1-[3-(4-{4-fluoro-2-[(3R)-3-methylmorpholin-4-carbonyl]phenyl}-1-methyl-1H-indazol-6-yl)azacyclobutan-1-yl]-2-methylpropyl}-N-(2-methoxyethyl)-N-methylcyclobutan-1-amine 195

Compound **195f** (90 mg, 168.97 µmol) and 2-methoxy-N-methyl-ethylamine (18.07 mg, 202.76 µmol) were dissolved in methanol (2.0 mL), followed by the addition of ZnCl₂ (46.06 mg, 337.93 µmol), and the mixture was stirred at 50°C for 1.0 hour. The mixture was cooled to room temperature, followed by the addition of sodium cyanoborohydride (31.85 mg, 506.90 µmol), allowing for reacting at 50°C for 12.0 hours under the protection of nitrogen. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 36%-66%), to obtain the title Compound **195** (39.42 mg, 65.07 µmol, yield: 38.51%).
MS m/z (ESI):606.3 [M+1]
¹H NMR (400 MHz, CD₃OD) δ 7.96 - 7.02 (m, 6H), 4.47 - 3.78 (m, 7H), 3.67 - 3.32 (m, 8H), 3.28 - 2.81 (m, 3H), 2.76 - 2.62 (m, 1H), 2.60 - 2.33 (m, 3H), 2.33 - 2.10 (m, 6H), 2.08 - 1.03 (m, 7H), 1.03 - 0.88 (m, 6H), 0.55 - 0.16 (m, 1H).

### Examples 196-1 and 196-2

### 4- {4-fluoro-2-[(3R)-3-methylmorpholin-4-carbonyl]phenyl}-1-methyl-6-(1-{2-methyl-1-[(1r,3r)-3-[(3S)-3-methoxypyrrolidin-1-yl]cyclobutyl]propyl}azacyclobutan-3-yl)-1H-indazole 196-1

### 4-{4-fluoro-2-[(3R)-3-methylmorpholin-4-carbonyl]phenyl}-1-methyl-6-(1-{2-methyl-1-[(1s,3s)-3-[(3S)-3-methoxypyrrolidin-1-yl]cyclobutyl]propyl}azacyclobutan-3-yl)-1H-indazole 196-2

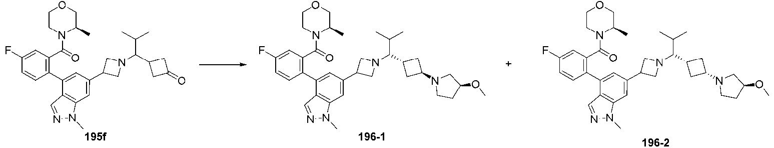

Compound **195f** (180 mg, 337.93 µmol) and (3S)-3-methoxypyrrolidine (41.02 mg, 405.52 µmol) were dissolved in methanol (3.0 mL), followed by the addition of ZnCl₂ (92.12 mg, 675.87 µmol), and the mixture was stirred at 50°C for 1.0 hour. The mixture was cooled to room temperature, followed by the addition of sodium cyanoborohydride (63.71 mg, 1.01 mmol), allowing for reacting at 50°C for 12.0 hours under the protection of nitrogen. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 36%-66%), to obtain the title Compounds **196-1** (4.7 mg, 7.61 µmol, yield: 2.25%) and **196-2** (11.86 mg, 19.20 µmol, yield: 5.68%).

### Compound 196-1:

MS m/z (ESI):618.3 [M+1]
HPLC: retention time: 0.755 min;
¹H NMR (400 MHz, CD₃OD) δ 7.96 - 7.04 (m, 6H), 4.63 - 3.67 (m, 8H), 3.66 - 3.32 (m, 4H), 3.29 - 2.19 (m, 15H), 2.16 - 1.53 (m, 6H), 1.52 - 1.02 (m, 3H), 1.00 - 0.87 (m, 5H), 0.54 - 0.16 (m, 1H).

### Compound 196-2:

MS m/z (ESI):618.4 [M+1]
HPLC: retention time: 1.580 min.
¹H NMR (400 MHz, CD₃OD) δ 7.93 - 7.05 (m, 6H), 4.47 - 4.23 (m, 1H), 4.14 - 4.06 (m, 3H), 3.99 - 3.81 (m, 4H), 3.66 - 3.32 (m, 4H), 3.28 - 3.24 (m, 3H), 3.17 - 2.73 (m, 3H), 2.71 - 2.37 (m, 5H), 2.31 - 1.99 (m, 5H), 1.96 - 1.69 (m, 4H), 1.55 - 1.17 (m, 2H), 1.10 - 1.02 (m, 1H), 1.00 - 0.91 (m, 6H), 0.57 - 0.15 (m, 1H).

### Examples 197 and 198

### N-[(1r,4r)-4-{[3-(8-{2-[(3R,5R)-3,5-dimethylmorpholin-4-carbonyl]-4-fluorophenyl}-1-fluoro-3-methylimidazo[1,5-a]pyridin-6-yl)azacyclobutan-1-yl]methyl}cyclohexyl]ethan-1-sulfonamide 197

### N-[(1s,4s)-4-{[3-(8-{2-[(3R,5R)-3,5-dimethylmorpholin-4-carbonyl]-4-fluorophenyl}-1-fluoro-3-methylimidazo[1,5-a]pyridin-6-yl)azacyclobutan-1-yl]methyl}cyclohexyl]ethan-1-sulfonamide 198

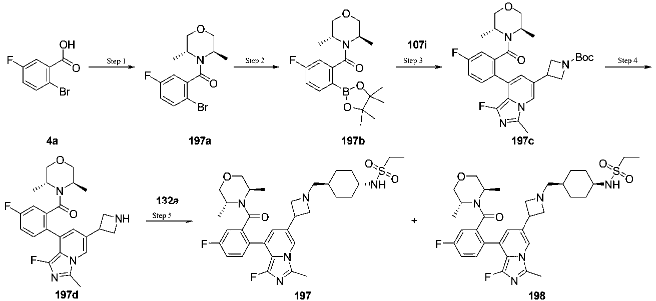

### Step 1

### (3R,5R)-4-(2-bromo-5-fluorobenzoyl)-3,5-dimethylmorpholine 197b

2-Bromo-5-fluoro-benzoic acid **4a** (6.57 g, 29.98 mmol) was dissolved in dichloroethane (150 mL), followed by the addition of triethylamine (9.10 g, 89.93 mmol) and HATU (13.68 g, 35.97 mmol). The reaction mixture was stirred for 5 min, followed by the addition of (3R,5R)-3,5-dimethylmorpholine (5 g, 32.97 mmol, hydrochloride). The mixture was stirred at 50°C for 12 hours. The mixture was washed with a saturated aqueous sodium carbonate solution (100 mL×2). The organic layer was dried over anhydrous sodium sulfate, filtered and concentrated under a reduced pressure to obtain residues, i.e., crude products, which were then purified by the silica-gel column chromatography using petroleum ether/ethyl acetate as an elution system, to obtain the title Compound **197a** (8 g, 25.30 mmol, yield84.41%) as white solid.
MS m/z (ESI): 316.0 [M+1]

### Step 2

### (3R,5R)-4-[5-fluoro-2-(tetramethyl-1,3,2-dioxaboran-2-yl)benzoyl]-3,5-dimethylmorpholine 197b

Compound **197a** (8 g, 25.30 mmol), potassium acetate (7.45 g, 75.91 mmol) and bis(pinacolato)diboron (16.06 g, 63.26 mmol) were dissolved in DMSO (100 mL). Under a nitrogen atmosphere, Pd(dppf)Cl₂.DCM (1.85 g, 2.53 mmol) was added, and the mixture was stirred at 110°C to allow for reacting for 5.0 hours. After the mixture was cooled to room temperature, 200 mL of water was added, and the mixture was extracted with ethyl acetate (100 ml × 3). The organic phases were combined, washed with water (100 ml×3), dried over anhydrous sodium sulfate, filtered and concentrated under a reduced pressure to obtain a crude product. The residues were purified by the silica-gel column chromatography using petroleum ether/ethyl acetate as an elution system, to obtain the title Compound **197b** (5.45 g, 15.00 mmol, yield: 59.3%) as white solid.
MS m/z (ESI): 364.0 [M+1]
¹H NMR (400 MHz, DMSO-*d*₆) δ 7.71 (m, 1H), 7.26-7.20 (m, 2H), 3.78-3.63 (m, 4H), 3.48 - 3.44 (m, 2H), 1.26 (s, 12H), 1.16 (s, 3H), 1.11 (s, 3H)

### Step 3

### 3-(8-{2-[(3R,5R)-3,5-dimethylmorpholin-4-carbonyl]-4-fluorophenyl}-1-fluoro-3-methylimidazol[1,5-a]pyridin-6-yl)azacyclobutan-1-tert-butyl carboxylate 197c

Compound **107i** (2.16 g, 6.36 mmol), Compound **197b**(2.54 g, 6.99 mmol) and potassium phosphate (3.37 g, 15.89 mmol) were dissolved in dioxane (25 mL) and water (5.0 mL). Pd(dtbpf)Cl₂ (414.30 mg, 635.68 µmol) was added. Under a nitrogen atmosphere, the mixture was stirred at 90°Cto allow for reacting for 2.0 hours. The reaction mixture was cooled to room temperature, followed by adding water (30 mL) and extracting with ethyl acetate (30 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **197c** (2.38 g, 4.40 mmol, yield: 69.3%).
MS m/z (ESI): 541.4 [M+1]
¹H NMR (400 MHz, CDCl₆) δ 7.50 - 7.35 (m, 2H), 7.25 - 7.17 (m, 2H), 6.54 (br s, 1H), 4.29 (br t, J = 8.6 Hz, 2H), 4.06 - 3.57 (m, 6H), 3.49 - 3.11 (m, 2H), 2.99 - 2.74 (m, 1H), 2.63 (s, 3H), 1.46 (s, 9H), 1.18 - 0.95 (m, 6H).

### Step 4

### (3R,5R)-4-{2-[6-(azacyclobutan-3-yl)-1-fluoro3-methylimidazo[1,5-a]pyridin-8-yl]-5-fluorobenzoyl}-3,5-dimethylmorpholine 197d

Compound **197c** (1 g, 1.85 mmol) was dissolved in methane dioxide (15.0 mL), followed by the addition of trifluoroacetic acid (5 mL) at 0°C. The mixture was stirred at room temperature to allow for reacting for 1.0 hours. The reaction mixture was concentrated under a reduced pressure, and residues were dissolved by adding dichloromethane (10.0 mL), regulated to PH = 11.0 with a 10% aqueous sodium hydroxide solution, and extracted with dichloromethane (30 mL x 3). The organic phases were combined, dried over anhydrous sodium sulfate, and concentrated under a reduced pressure, to obtain the crude product of title Compound **197d** (870 mg, 1.98 mmol, yield: 106.8%). The crude product was directly used in the next step of reaction without purification. MS m/z (ESI): 441.1 [M+1]

### Step 5

### N-[(1r,4r)-4-{[3-(8-{2-[3R,5R)-3,5-dimethylmorpholin-4-carbonyl]-4-fluorophenyl}-1-fluoro-3-methylimidazo[1,5-a]pyridin-6-yl)azacyclobutan-1-yl]methyl}cyclohexyl]ethan-1-sulfonamide 197

### N-[(1s,4s)-4-{[3-(8-{2-[(3R,5R)-3,5-dimethylmorpholin-4-carbonyl]-4-fluorophenyl}-1-fluoro-3-methylimidazo[1,5-a]pyridin-6-yl)azacyclobutan-1-yl]methyl}cyclohexyl]ethan-1-sulfonamide 198

The crude product of Compound **197d** (870 mg, 1.98 mmol) and*N*-[(1*r*,4*r*)-4-formylcyclohexyl]ethan-1-sulfonamide **132a**(476.45 mg, 2.17 mmol) were dissolved in methanol (15.0 mL). Acetic acid (142.33 mg, 2.37 mmol) was added, and the mixture was stirred at room temperature for 0.5 hours. Sodium cyanoborohydride (372.36 mg, 5.93 mmol) was added, and the reaction mixture was stirred at room temperature for 2.0 hour. A saturated aqueous sodium bicarbonate solution (10 mL) was added, and the mixture was then extracted with dichloromethane (20 mL ×3). The organic phases were combined, dried over anhydrous sodium sulfate, and concentrated under a reduced pressure. The resulting residues were purified by the silica-gel column chromatography using petroleum ether/ethyl acetate as an elution system, and the obtained crude product was purified by the HPLC (mobile phase: acetonitrile, water (0.1% ammonia water), elution gradient: 35%-65%), and then further separated by the preparative SFC(preparative column: DAICEL CHIRALPAK AD(250mm*30mm,10um); mobile phase: CO₂-EtOH(0.1% ammonia water); elution gradient: 30%), to obtain the title Compounds **197** (339.71 mg, 527.68 µmol, yield: 26.72%) and **198** (73.48 mg, 114.14 µmol, yield: 5.78%).

### Compound 197:

MS m/z (ESI): 644.2[M+1]
SFC: retention time: 1.773 min; Chiralpak AD-3 50 x 4.6 mm I.D., 3 um; mobile phase: CO₂-EtOH (0.05% DEA); elution gradient: 5%-40%.

¹H NMR (400 MHz, DMSO-*d*₆) δ 7.88 (s, 1H), 7.57 - 7.30 (m, 3H), 6.97 (d, J = 7.6 Hz, 1H), 6.59 (s, 1H), 3.82 - 3.33 (m, 6H), 3.28 - 2.90 (m, 7H), 2.78 - 2.52 (m, 4H), 2.23 (d, J = 6.8 Hz, 2H), 1.89 - 1.67 (m, 4H), 1.27 - 0.78 (m, 14H).

### Compound 198:

MS m/z (ESI): 644.2[M+1]
SFC: retention time: 1.584 min; ChiralpakAD-3 50 x 4.6 mm I.D., 3 um; mobile phase: CO₂-EtOH (0.05% DEA); elution gradient: 5%-40%.

¹H NMR (400 MHz, DMSO-*d*₆) δ 7.88 (s, 1H), 7.60 - 7.28 (m, 3H), 7.08 - 6.77 (m, 1H), 6.70 - 6.46 (m, 1H), 3.86 - 3.38 (m, 6H), 3.28 - 2.82 (m, 7H), 2.76 - 2.53 (m, 4H), 2.37 - 2.24 (m, 2H), 1.62 - 1.29 (m, 9H), 1.26 - 0.73 (m, 9H).

### Examples 199-1 and 199-2

### (3R)-4-[5-fluoro-2-(1-fluoro-3-methyl-6-{1-[(3R)-2-methyl-6-(morpholin-4-yl)hexan-3-yl]azacyclobutan-3-yl}imidazo[1,5-a]pyridin-8-yl)benzoyl]-3-methylmorpholine 199-1

### (3R)-4-[5-fluoro-2-(1-fluoro-3-methyl-6-{1-[(3S)-2-methyl-6-(morpholin-4-yl)hexan-3-yl]azacyclobutan-3-yl}imidazo[1,5-a]pyridin-8-yl)benzoyl]-3-methylmorpholine 199-2

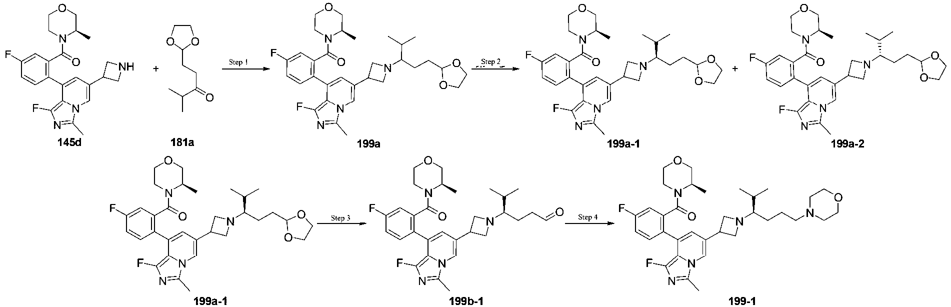

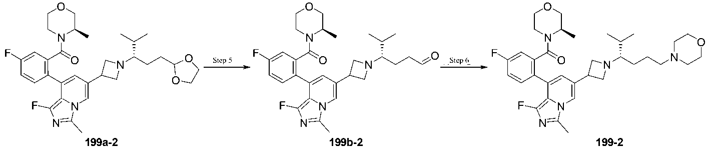

### Step 1

### (3R)-4-[2-(6-{1-[1-(1,3-dioxolan-2-yl)-4-methylpentan-3-yl]azacyclobutan-3-yl}-1-fluoro-3-methylimidazo[1,5-a]pyridin-8-yl)-5-fluorobenzoyl]-3-methylmorpholine 199a

Compound **145d** (422 mg, 989.55 µmol) and 1-(1,3-dioxolan-2-yl)-4-methylpentan-3-one **181a** (187.46 mg, 1.09 mmol) were dissolved in methanol (20 mL), followed by the addition of ZnCl₂ (404.61 mg, 2.97 mmol), and the mixture was stirred at 60°C for 1.0 hour. The mixture was cooled to room temperature, followed by adding sodium cyanoborohydride (186.55 mg, 2.97 mmol) and stirring at 60°C for 4.0 hour. A saturated aqueous ammonium chloride solution (20 mL) was added to the reaction mixture, which was then stirred for 30 min and then diluted with water (50 mL). The mixture was extracted with ethyl acetate (50 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the crude product of title Compound **199a** (500 mg). The crude product was directly used in the next step of reaction without purification.
MS m/z (ESI): 583.6 [M+1]

### Step 2

### (3R)-4-[2-(6-{1-[(3R)-1-(1,3-dioxolan-2-yl)-4-methylpentan-3-yl]azacyclobutan-3-yl}-1-fluoro-3-methylimidazo[1,5-a]pyridin-8-yl)-5-fluorobenzoyl]-3-methylmorpholine 199a-1

### (3R)-4-[2-(6-{1-[(3S)-1-(1,3-dioxolan-2-yl)-4-methylpentan-3-yl]azacyclobutan-3-yl}-1-fluoro-3-methylimidazo[1,5-a]pyridin-8-yl)-5-fluorobenzoyl]-3-methylmorpholine 199a-2

The crude product of Compound **199a** (500 mg) was separated and purified by the chiral preparative SFC (preparative column: DAICEL CHIRALCEL OD (250 mm * 30 mm, 10 um), mobile phase: carbon dioxide-isopropanol (0.1% ammonia water); elution gradient: 45%), to obtain the crude products of title Compounds **199a-1** (151 mg, 259.15 µmol, yield: 26.19%) and **199a-2** (151 mg, 259.15 µmol, yield: 26.19%).

### Single-configuration Compound 199a-1:

MS m/z (ESI):583.3 [M+1]
Chiral HPLC analysis: retention time: 1.845 min; chromatographic column: Chiralcel OD-3 50 x 4.6 mm I.D., 3 um; mobile phase: carbon dioxide-isopropanol (0.05% diethylamide ): elution gradient: 5%-40%.

### Single-configuration Compound 199a-2:

MS m/z (ESI):583.3 [M+1]
Chiral HPLC analysis: retention time: 1.462 min; chromatographic column: Chiralcel OD-3 50 x 4.6 mm I.D., 3 um; chromatographic column: Chiralcel OD-3 50 x 4.6 mm I.D., 3 um; mobile phase: carbon dioxide-isopropanol (0.05% diethylamide): elution gradient: 5%-40%.

### Step 3

### (4R)-4-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-5-methylhexanal 199b-1

Compound **199a-1** (151.00 mg, 259.15 µmol) was dissolved in acetonitrile (2.0 mL), followed by the addition of a hydrochloric acid solution (1.0 M; 2.0 mL). The mixture was stirred at 50°C to allow for reacting for 1.0 hours. The reaction mixture was concentrated under a reduced pressure, regulated to pH = 12 with a 10 % aqueous NaOH solution, and extracted with dichloromethane (10 mL x 3). The organic phases were combined, dried over anhydrous sodium sulfate, and concentrated under a reduced pressure, to obtain the crude product of title Compound **199b-1** (116 mg, 215.36 µmol,yield: 83.1%). The crude product was directly used in the next step of reaction without purification.

### Single-configuration Compound 199b-1:

MS m/z (ESI): 539.2 [M+1]

### Step 4

### (3R)-4-[5-fluoro-2-(1-fluoro-3-methyl-6-{1-[(3R)-2-methyl-6-(morpholin-4-yl)hexan-3-yl]azacyclobutan-3-yl}imidazo[1,5-a]pyridin-8-yl)benzoyl]-3-methylmorpholine 199-1

The crude product of Compound **199b-1** (45 mg, 83.55 µmol) and morpholine (8.73 mg, 100.25 µmol) were dissolved in methanol (3 mL). Sodium cyanoborohydride (10.50 mg, 167.09 µmol) and triethylamine (25.36 mg, 250.64 µmol) were added, allowing for reacting at 30°C for 2.0 hours under the protection of nitrogen. Sodium cyanoborohydride (5.25 mg, 83.55 µmol) was supplemented, and the mixture was further stirred for 0.5 hours. Water was added to the reaction mixture, which was then extracted with ethyl acetate (10 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 33%-63%). The title Compound **199-1** (0.85 mg, 1.39 µmol, yield: 1.67%) was obtained.

### Single-configuration Compound 199-1:

MS m/z (ESI):610.2 [M+1]
Chiral SFC: retention time: 1.177 min; chromatographic column: Chiralcel OD-3 50×4.6 mm I.D., 3 um; mobile phase: CO₂/MeOH (0.05% DEA): elution gradient: 5%-40%.

¹H NMR (400 MHz, CDCl₃) δ 7.57 - 7.43 (m, 2H), 7.26 - 7.10 (m, 2H), 6.85 - 6.61 (m, 1H), 4.71 - 3.83 (m, 4H), 3.81 - 3.55 (m, 8H), 3.42 - 3.21 (m, 3H), 3.20 - 3.04 (m, 1H), 3.03 - 2.89 (m, 1H), 2.82 - 2.70 (m, 1H), 2.63 (s, 3H), 2.54 - 2.29 (m, 8H), 1.37 - 1.10 (m, 4H), 0.98 (s, 6H), 0.86 - 0.73 (m, 1H).

### Step 5

### (4S)-4-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-5-methylhexanal 199b-2

Compound **199a-2** (151.00 mg, 259.15 µmol) was dissolved in acetonitrile (2.0 mL), followed by the addition of a hydrochloric acid solution (1.0 M; 2.0 mL). The mixture was stirred at 50°C to allow for reacting for 1.0 hours. The reaction mixture was concentrated under a reduced pressure, regulated to pH = 12 with a 10 % aqueous NaOH solution, and extracted with dichloromethane (10 mL x 3). The organic phases were combined, dried over anhydrous sodium sulfate, and concentrated under a reduced pressure, to obtain the crude product of title Compound **199b-2** (96 mg, 178.23 µmol, yield: 68.78%). The crude product was directly used in the next step of reaction without purification.

### Single-configuration Compound 199b-2:

MS m/z (ESI): 539.2 [M+1]

### Step 6

### (3R)-4-[5-fluoro-2-(1-fluoro-3-methyl-6-{1-[(3S)-2-methyl-6-(morpholin-4-yl)hexan-3-yl]azacyclobutan-3-yl}imidazo[1,5-a]pyridin-8-yl)benzoyl]-3-methylmorpholine 199-2

The crude product of Compound **199b-2** (32 mg, 59.41 µmol) and morpholine (5.69 mg, 65.35 µmol) were dissolved in methanol (3.0 mL), and stirred at 30°C for 0.5 hours. Sodium cyanoborohydride (11.20 mg, 178.23 µmol) was added, allowing for reacting at 30°C for 11.0 hours under the protection of nitrogen. A saturated aqueous ammonium chloride solution (10 mL) and water (10 mL) were added to the reaction mixture, which was then extracted with ethyl acetate (50 mL x 3). The organic phases were combined, dried over anhydrous sodium sulfate, and concentrated under a reduced pressure. The resulting residues were purified by the HPLC (mobile phase: acetonitrile, water (0.1% ammonium bicarbonate), elution gradient: 30%-60%). The title Compound **199-2** (5.94 mg, 9.74 µmol, yield: 16.40%) was obtained as yellow solid.

### Single-configuration Compound 199-2:

MS m/z (ESI):610.5 [M+1]
Chiral SFC: retention time: 1.172 min; chromatographic column: Chiralcel OD-3 50×4.6 mm I.D., 3 um; mobile phase: CO₂/MeOH (0.05% DEA): elution gradient: 5%-40%.

¹H NMR (400 MHz, CDCl₃) δ 7.48 - 7.30 (m, 2H), 7.17 - 6.98 (m, 2H), 6.75 - 6.47 (m, 1H), 5.29 - 4.02 (m,1H), 3.75 - 3.66 (m, 1H), 3.63 (br s, 3H), 3.56 - 3.45 (m, 2H), 3.39 - 3.20 (m, 1H), 3.16 - 3.05 (m, 2H), 3.01 - 2.62 (m, 2H), 2.53 (s, 3H), 2.37 (br s, 4H), 2.29 - 2.19 (m, 2H), 2.14 - 1.87 (m, 2H), 1.81 - 1.63 (m, 4H), 1.54 - 1.44 (m, 2H), 1.31 - 1.18(m, 3H), 1.12 - 1.00 (m, 1H), 0.90 - 0.79 (m, 6H), 0.75 - 0.03 (m, 1H).

### Example 204

### (3R)-4-[5-fluoro-2-(1-fluoro-6-{1-[(3R)-6-(4-methoxypiperidin-1-yl)-2-methylhexan-3-yl]azacyclobutan-3-yl}-3-methylimidazo[1,5-a]pyridin-8-yl)benzoyl]-3-methylmorpholine 204

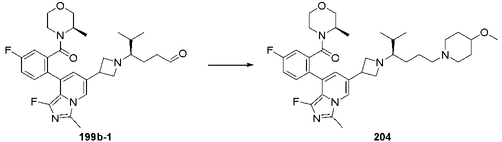

The crude product of Compound **199b-1** (100 mg, 185.66 µmol) and 4-methoxypiperidine (23.52 mg, 204.22 µmol) were dissolved in methanol (1.5 mL), followed by the addition of acetic acid (16.72 mg, 278.49 µmol), and the mixture was stirred at room temperature for 1.0 hour under the protection of nitrogen. Sodium cyanoborohydride (35.00 mg, 556.97 µmol) was added, allowing for reacting at room temperature for 11.0 hours under the protection of nitrogen. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 43%-73%), to obtain the title product **204** (42.8 mg, 67.11 µmol, yield: 36.14%) as yellow solid.
MS m/z (ESI):638.4 [M+1]
Chiral SFC: retention time: 1.164 min; chromatographic column: Chiralcel OD-3 50×4.6 mm I.D., 3 um; mobile phase: CO₂/MeOH (0.05% DEA): elution gradient: 5%-40%.

¹H NMR (400 MHz, DMSO-*d₆*) δ 7.94 - 7.78 (m, 1H), 7.65 - 7.21 (m, 3H), 6.90 - 6.39 (m, 1H), 4.41 - 3.88 (m, 1H), 3.76 - 3.44 (m, 5H), 3.20 (s, 3H), 3.12 (s, 3H), 2.75 - 2.61 (m, 2H), 2.55 (s, 3H), 2.35 - 2.18 (m, 2H), 2.11 - 1.99 (m, 2H), 1.83 - 1.67 (m, 3H), 1.51 - 1.34 (m, 4H), 1.29 - 1.03 (m, 7H), 0.89 - 0.70 (m, 8H).

### Example 206

### N-(2,2-difluoroethyl)-5-fluoro-2-[4-methyl-6-({1-[(oxacyclohexan-4-yl)methyl]azacyclobutan-3-yl}methyl)pyrrolo[1,2-a]pyrazin-8-yl]-N-(isopropyl)benzamide 206

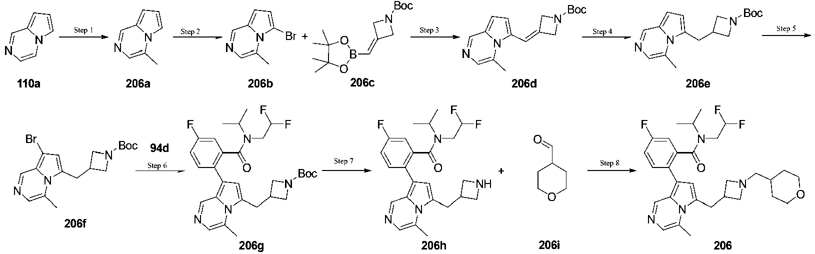

### Step 1

### 4-methylpyrrolo[1,2-a]pyrazine 206a

Pyrrolo[1,2-a]pyrazine **110a** (1.0 g, 8.46 mmol) was dissolved in THF (40 mL), and cooled to -75 °C, followed by the slow dropwise addition of the THF solution (2 M, 9.50 mL) of LDA. The reaction mixture was stirred at -75 °C for 0.5 hours, followed by the addition of iodomethane (3.12 g, 22.01 mmol). The reaction mixture was slowly warmed to room temperature and stirred for 2.0 hours. A saturated aqueous ammonium chloride solution (50 mL) was added to the reaction mixture, which was then extracted with ethyl acetate (30 mL x 3). The organic phases were combined, dried over anhydrous sodium sulfate, and concentrated under a reduced pressure, to obtain the crude product of title Compound **206a** (2.2 g, 16.65 mmol, yield: 98.33%). The crude product was directly used in the next step of reaction without purification.
MS m/z (ESI): 133.2[M+1]
¹H NMR (400 MHz, CDCl₃) δ 8.77 (s, 1H), 7.40 (s, 1H), 7.35 - 7.31 (m, 1H), 6.96 (dd, J = 2.6, 4.0 Hz, 1H), 6.86 (dd, J = 1.0, 4.1 Hz, 1H), 2.50 (s, 3H).

### Step 2

### 6-bromo-4-methylpyrrolo[1,2-a]pyrazine 206b

Compound **206a** (1.2 g, 9.08 mmol) was dissolved in trichloromethane (25 mL), and cooled to -10 °C, followed by the addition of NBS(1.54 g, 8.63 mmol). The reaction mixture was stirred at room temperature for 1.0 hour. A saturated aqueous sodium bicarbonate solution (40 mL) was added to the reaction mixture, which was then extracted with dichloromethane (30 mL x 3). The organic phases were combined, dried over anhydrous sodium sulfate, and concentrated under a reduced pressure. The resulting residues were purified by the silica-gel column chromatography using dichloromethane/ethyl acetate as an elution system, to obtain the title Compound **206b** (750 mg, 3.55 mmol, yield: 39.14%).
MS m/z (ESI): 213.0[M+1]
¹H NMR (400 MHz, CDCl₃) δ 8.77 (s, 1H), 7.40 (s, 1H), 7.35 - 7.31 (m, 1H), 6.96 (dd, J = 2.6, 4.0 Hz, 1H), 6.86 (dd, J = 1.0, 4.1 Hz, 1H), 2.50 (s, 3H).

### Step 3

### 3-({4-methylpyrrolo[1,2-a]pyrazin-6-yl}methylene)azacyclobutan-1-tert-butyl carboxylate 206d

Compound **206b** (100 mg, 473.80 µmol), 3-[(tetramethyl-1,3,2-dioxaboran-2-yl)methylene]azacyclobutan-1-tert-butyl carboxylate **206c** (205.12 mg, 521.18 µmol) and sodium carbonate (150.65 mg, 1.42 mmol) were dissolved in dioxane (6 mL) and water (1 mL). Pd(dtbpf)Cl₂ (30.88 mg, 47.38 µmol) was added. Under a nitrogen atmosphere, the mixture was stirred at 90°Cto allow for reacting for 2.0 hours. The reaction mixture was cooled to room temperature, water (20 mL) was added, and the mixture was extracted with ethyl acetate (15 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **206d** (123 mg, 410.87 µmol, yield: 86.72%).
MS m/z (ESI): 299.9 [M+1]

### Step 4

### 3-({4-methylpyrrolo[1,2-a]pyrazin-6-yl}methyl)azacyclobutan-1-tert-butyl carboxylate 206e

Compound **206d** (488 mg, 1.63 mmol) was dissolved in methanol (10 mL), followed by the addition of 10% Pd/C (395.97 mg, 326.02 µmol) under a nitrogen atmosphere. The reaction atmosphere was substituted with a nitrogen atmosphere (15 psi), and the mixture was stirred at 50°C for 12.0 hours. The reaction mixture was filtered, and the filtrate was concentrated under a reduced pressure, to obtain the crude product of title Compound **206e** (418 mg, 1.39 mmol, yield: 85.08%).
MS m/z (ESI): 301.9 [M+1]

### Step 5

### 3-({8-bromo-4methylpyrrolo[1,2-a]pyrazin-6-yl}methyl)azacyclobutan-1-tert-butyl carboxylate 206f

Compound **206e** (212 mg, 703.43 µmol) was dissolved in trichloromethane (5.0 mL), and cooled to -10°C, followed by the addition of NBS (118.94 mg, 668.25 µmol), and the mixture was stirred at room temperature for 1.0 hour. A saturated aqueous sodium bicarbonate solution (40 mL) was added to the reaction mixture, which was then extracted with dichloromethane (30 mL x 3). The organic phases were combined, dried over anhydrous sodium sulfate, and concentrated under a reduced pressure. The resulting residues were purified by the silica-gel column chromatography using dichloromethane/ethyl acetate as an elution system, to obtain the title Compound **206f** (170 mg, 447.04 µmol, yield: 63.55%).
MS m/z (ESI): 382.0 [M+1]
¹H NMR (400 MHz, CD₃OD) δ 8.47 (s, 1H), 7.16 (s, 1H), 6.71 (s, 1H), 4.16 - 4.08 (m, 2H), 3.71 - 3.65 (m, 2H), 3.62 (d, J = 8.0 Hz, 2H), 3.08 - 2.98 (m, 1H), 2.80 (s, 3H), 1.43 (s, 9H).

### Step 6

### 3-[(8-{2-[(2,2-difluoroethyl)(isopropyl)aminoformyl]-4-fluorophenyl}-4-methylpyrrolo[1,2-a]pyrazin-6-yl)methyl]azacyclobutan-1-tert-butyl carboxylate 206g

Compound **206f** (200 mg, 525.93 µmol) and Compound **94d** (292.84 mg, 788.90 µmol) were dissolved in dioxane (8 mL) and water (1.0 mL). Potassium phosphate (247.98 mg, 1.58 mmol) and Pd(dtbpf)Cl₂ (34.28 mg, 52.59 µmol) were added, and the mixture was stirred at 90 °C under a nitrogen atmosphere to allow for reacting for 2 hours. The reaction mixture was cooled to room temperature, followed by adding water (50 mL) and extracting with ethyl acetate (20 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **206g** (244 mg, 448.03 µmol, yield: 85.19%).
MS m/z (ESI): 545.2 [M+1]

### Step 7

### 2-{6-[(azacyclobutan-3-yl)methyl]-4-methylpyrrolo[1,2-a]pyrazin-8-yl}-N-(2,2-difluoroethyl)-5-fluoro-N-(isopropyl)benzamide 206h

Compound **206g** (80 mg, 146.89 µmol) was dissolved in dichloromethane (5.47 mL), followed by the addition of trifluoroacetic acid (789.33 mg, 6.92 mmol, 533.33 µL) at 0°C. The mixture was stirred at room temperature to allow for reacting for 1.0 hours. The reaction mixture was concentrated under a reduced pressure, to obtain the crude product of title Compound **206h** (65.3 mg). The crude product was directly used in the next step of reaction without purification.
MS m/z (ESI): 445.1 [M+1]

### Step 8

### N-(2,2-difluoroethyl)-5-fluoro-2-[4-methyl-6-({1-[(oxacyclohexan-4-yl)methyl]azacyclobutan-3-yl}methyl)pyrrolo[1,2-a]pyrazin-8-yl]-N-(isopropyl)benzamide 206

The crude product of Compound **206h** (65.3 mg), oxacyclohexan-4-formaldehyde **206i** (21.80 mg, 190.95 µmol) and triethylamine (44.59 mg, 440.66 µmol) were dissolved in methanol (10 mL), and stirred at room temperature for 1.0 hour. Sodium cyanoborohydride (18.46 mg, 293.77 µmol) was added, and the mixture was stirred at 50°C for 2.0 hours. Water (20 mL) was added to the reaction mixture, which was then extracted with dichloromethane (10 mL x 3). The organic phases were combined, dried over anhydrous sodium sulfate, and concentrated under a reduced pressure. The resulting residues were purified by the silica-gel column chromatography using dichloromethane/ethyl acetate as an elution system, to obtain the title Compound **206** (13.68 mg, 25.21 µmol, yield: 17.16%) as yellow solid.
MS m/z (ESI): 543.2 [M+1]
¹H NMR (400 MHz, CD₃OD) δ 8.51 (s, 1H), 7.55 (dd, J = 5.6, 8.8 Hz, 1H), 7.32 (dt, J = 2.8, 8.8 Hz, 1H), 7.26 - 7.13 (m, 2H), 6.67 (s, 1H), 6.13 - 5.75 (m, 1H), 3.96 - 3.87 (m, 2H), 3.65 - 3.61 (m, 3H), 3.59 - 3.51 (m, 1H), 3.42 - 3.35 (m, 2H), 3.13 - 3.04 (m, 2H), 3.02 - 2.91 (m, 1H), 2.83 (s, 3H), 2.47 (d, J = 6.4 Hz, 2H), 1.70 - 1.59 (m, 3H), 1.39 - 1.19 (m, 6H), 0.95 (d, J = 6.4 Hz, 3H), 0.33 (d, J = 6.8 Hz, 2H).

### Examples 207-1 and 207-2

### (3R)-4-{5-fluoro-2-[1-fluoro-3-methyl-6-(1-{[(1r,4r)-4-(1,2,4-oxadiazol-5-yl)cyclohexyl]methyl}azacyclobutan-3-yl)imidazo[1,5-a]pyridin-8yl]benzoyl}-3-methylmorpholine 207-1

### (3R)-4-{5-fluoro-2-[1-fluoro-3-methyl-6-(1-{[(1s,4s)-4-(1,2,4-oxadiazol-5-yl)cyclohexyl]methyl}azacyclobutan-3-yl)imidazo[1,5-a] pyridin-8yl]benzoyl}-3-methylmorpholine 207-2

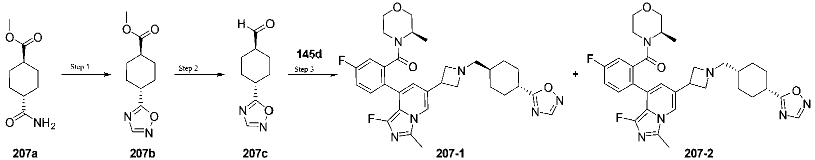

### Step 1

### (1r,4r)-4-(1,2,4-oxadiazol-5-yl)cyclohexan-1-methyl carboxylate 207b

(1*r*,4*r*)-4-aminoformylcyclohexan-1-methyl carboxylate **207a** (1 g, 5.40 mmol) was dissolved in 1,1-dimethoxy-*N*,*N-*dimethyl-methylamine (8.97 g, 75.28 mmol, 10 mL). The reaction mixture was stirred at 100°C for 2.0 hours. The reaction mixture was concentrated under a reduced pressure, and the obtained solid was dissolved in ethanol (30 mL), followed by the addition of hydroxylamine in hydrochloride (750.36 mg, 10.80 mmol). The mixture was stirred at 80°C for 12 hours. The reaction mixture was concentrated under a reduced pressure, and the resulting residues were purified by the silica-gel column chromatography using petroleum ether/ethyl acetate as an elution system, to obtain the title Compound **207b** (750 mg, 3.57 mmol, yield: 66.08%) as white solid.
MS m/z (ESI): 211.1 [M+1]
¹H NMR (400 MHz, CDCl₃) δ 8.33 (s, 1H), 3.70 (s, 3H), 3.08 - 2.93 (m, 1H), 2.44 - 2.35 (m, 1H), 2.30 - 2.22 (m, 2H), 2.20 - 2.11 (m, 2H), 1.73 - 1.59 (m, 4H).

### Step 2

### (1r,4r)-4-(1,2,4-oxadiazol-5-yl)cyclohexan-1-formaldehyde 207c

Compound **207b** (740 mg, 3.52 mmol) was dissolved in DCM(10 mL). Under a nitrogen atmosphere, the mixture was cooled to -75°C, followed by the slow dropwise addition of DIBAL-H (1 M, 3.87 mL), and the mixture was stirred at -75°C to allow for reacting for 2.0 hours. Sodium sulfate decahydrate was added to quench the reaction, the reaction mixture was filtered, and the filtrate was concentrated under a reduced pressure to obtain a crude product. The resulting residues were purified by the silica-gel column chromatography using petroleum ether/ethyl acetate as an elution system, to obtain the title Compound **207c** (180 mg, 998.87 µmol, yield: 28.38%) as colorless oil.
MS m/z (ESI): 181.1 [M+1]
¹H NMR (400 MHz, CDCl₃) δ 9.68 (s, 1H), 8.34 (s, 1H), 2.98 (tt, J = 3.6, 11.9 Hz, 1H), 2.35 - 2.26 (m, 3H), 2.22 - 2.15 (m, 2H), 1.77 - 1.67 (m, 2H), 1.53 - 1.38 (m, 2H).

### Step 3

### (3R)-4-{5-fluoro-2-[1-fluoro-3-methyl-6-(1-{[(1r,4r)-4-(1,2,4-oxadiazol-5-yl)cyclohexyl]methyl}azacyclobutan-3-yl)imidazo[1,5-a]pyridin-8yl]benzoyl}-3-methylmorpholine 207-1

### (3R)-4-{5-fluoro-2-[1-fluoro-3-methyl-6-(1-{[(1s,4s)-4-(1,2,4-oxadiazol-5-yl)cyclohexyl]methyl}azacyclobutan-3-yl)imidazo[1,5-a]pyridin-8yl]benzoyl}-3-methylmorpholine 207-2

The crude product of Compound **145d** (95 mg, 180.41 µmol) and Compound **207c** (48.17 mg, 267.32 µmol) were dissolved in methanol (2.0 mL). Acetic acid (13.38 mg, 222.77 µmol) was added, and the mixture was stirred for 1.0 hour. Sodium cyanoborohydride (42.00 mg, 668.30 µmol) was added, and the mixture was stirred at room temperature for 12.0 hours. A saturated aqueous sodium bicarbonate solution (10 mL) was added, and the mixture was then extracted with dichloromethane (20 mL × 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 36%-66%), to obtain the title Compounds **207-1** (18.67 mg, 31.61 µmol, yield: 14.19%) and **207-2** (17.24 mg, 29.19 µmol,yield: 13.10%).

### Compound 207-1:

MS m/z (ESI): 591.2[M+1]
SFC: retention time: 0.850 min; Chiralpak AS-3 50 x 4.6 mm I.D., 3 um; mobile phase: CO₂-EtOH (0.05% DEA); elution gradient: 5%-40%.

¹H NMR (400 MHz, CD₃OD) δ 8.63 - 8.40 (m, 1H), 7.89 - 7.76 (m, 1H), 7.66 - 7.50 (m, 1H), 7.38 - 7.16 (m, 2H), 6.86 - 6.60 (m, 1H), 4.61 - 4.20 (m, 1H), 4.14 - 3.48 (m, 6H), 3.21 - 2.66 (m, 4H), 2.64 - 2.48 (m, 5H), 2.21 - 1.82 (m, 5H), 1.77 - 0.94 (m, 8H), 0.83 - 0.62 (m, 1H).

### Compound 207-2:

MS m/z (ESI): 591.3[M+1]
SFC: retention time: 2.171 min; Chiralpak OD-3 50 x 4.6 mm I.D., 3 um; mobile phase: CO₂-EtOH (0.05% DEA); elution gradient: 5%-40%.

¹H NMR (400 MHz, CD₃OD) δ 8.60 - 8.18 (m, 1H), 8.04 - 7.87 (m, 1H), 7.80 - 7.44 (m, 1H), 7.43 - 7.08 (m, 2H), 7.03 - 6.42 (m, 1H), 4.52 - 4.15 (m, 5H), 3.64 - 3.55 (m, 1H), 3.43 - 3.39 (m, 1H), 3.23 - 3.16 (m, 2H), 3.04 - 2.95 (m, 1H), 2.71 - 2.55 (m, 3H), 2.21 - 2.10 (m, 2H), 1.98 - 1.78 (m, 5H), 1.70 - 1.39 (m, 5H), 1.30 - 1.05 (m, 4H), 0.82 - 0.64 (m, 1H).

### Examples 209-1 and 209-2

### (3R,5R)-4-[5-fluoro-2-(1-fluoro-3-methyl-6- {1-[(1R)-1-(oxacyclohexan-4-yl)ethyl]azacyclobutan-3-yl}imidazo[1,5-a]pyridin-8-yl)benzoyl]-3,5-dimethylmorpholine 209-1

### (3R,5R)-4-[5-fluoro-2-(1-fluoro-3-methyl-6-{1-[(1S)-1-(oxacyclohexan-4-yl)ethyl]azacyclobutan-3-yl}imidazo[1,5-a]pyridin-8-yl)benzoyl]-3,5-dimethylmorpholine 209-2

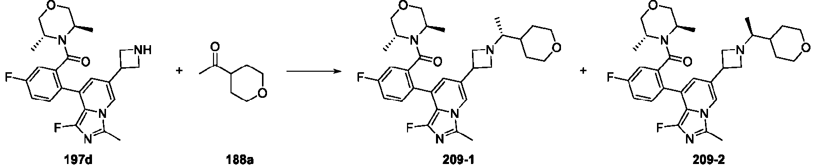

Compound **197d** (159 mg, 360.97 µmol) and Compound **188a** (50.89 mg, 397.06 µmol) were dissolved in methanol (2.0 mL). Zinc chloride (98.40 mg, 721.93 µmol) was added, and the mixture was stirred at 50°C for 1.0 hour. After cooling, sodium cyanoborohydride (68.05 mg, 1.08 mmol) was added, and the mixture was further stirred at 50°C for 11 hours. A saturated aqueous sodium bicarbonate solution (10 mL) was added, and the mixture was then extracted with dichloromethane (10 mL × 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 30%-60%), and the resulting mixture was separated by the chiral preparative HPLC (preparative column: Daicel ChiralPak IG (250 * 30 mm, 10 um); mobile phase: n-hexan-EtOH(0.1% isopropylamine); elution gradient: 20%), to obtain the title Compounds **209-1** (16.80 mg, 30.40 µmol, yield: 8.42%) and **209-2** (27.6 mg, 49.94 µmol, yield: 13.84%).

### Compound 209-1:

MS m/z (ESI): 553.1[M+1]
Chiral HPLC: retention time: 2.590 min; Chiralpak IG-3 50 x 4.6 mm I.D., 3 um; mobile phase: n-hexane (0.05% isopropylamine)-[EtOH+ACN(0.05%DEA)]; elution gradient: 20%;
¹H NMR (400 MHz, DMSO-*d*₆) δ 7.88 (s, 1H), 7.54 - 7.47 (m, 1H), 7.45 - 7.35 (m, 2H), 6.62 (s, 1H), 3.92 - 3.41 (m, 8H), 3.28 - 3.03 (m, 6H), 2.69 - 2.52 (m, 4H), 2.16 (s, 1H), 1.61 - 1.44 (m, 2H), 1.35 - 1.28 (m, 2H), 1.26 - 1.16 (m, 2H), 0.85 (s, 5H), 0.78 - 0.74 (m, 3H).

### Compound 209-2:

MS m/z (ESI): 553.2[M+1]
Chiral HPLC: retention time: 2.583 min; Chiralpak IG-3 50 x 4.6 mm I.D., 3 um; mobile phase: n-hexane (0.05% isopropylamine)-[EtOH+ACN(0.05%DEA)]; elution gradient: 20%;
¹H NMR (400 MHz, DMSO-*d*₆) δ 7.88 (s, 1H), 7.54 - 7.47 (m, 1H), 7.45 - 7.35 (m, 2H), 6.57 (s, 1H), 3.93 - 3.34 (m, 8H), 3.29 - 3.01 (m, 6H), 2.75 - 2.53 (m, 4H), 2.20 - 2.12 (m, 1H), 1.62 - 1.43 (m, 2H), 1.37 - 1.13 (m, 4H), 1.06 - 0.74 (m, 8H).

### Example 211

### 4-[(4-{[3-(8-{2-[(3R,5R)-3,5-dimethylmorpholin-4-carbonyl]-4-fluorophenyl}-1-fluoro-3-methylimidazo[1,5-a]pyridin-6-yl)azacyclobutan-1-yl]methyl}piperidin-1-yl)methyl]oxacyclohexan-4-ol 211

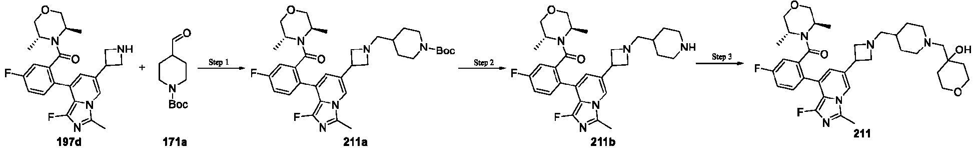

### Step 1

### 4-{[3-(8-{2-[(3R,5R)-3,5-dimethylmorpholin-4-carbonyl]-4-fluorophenyl}-1-fluoro-3-methylimidazo[1,5-a]pyridin-6-yl)azacyclobutan-1-yl]methyl}piperidin-1-tert-butyl carboxylate 211a

Compound **197d** (1.63 g, 3.70 mmol), 4-formylpiperidin-1-tert-butyl formate **171a** (1.03 g, 4.81 mmol) and acetic acid (222.22 mg, 3.70 mmol) were dissolved in methanol (2.0 mL), and stirred at room temperature for 1.0 hour. Sodium cyanoborohydride (697.63 mg, 11.10 mmol) was added, and the reaction mixture was stirred at room temperature for 12 hour. The reaction mixture was concentrated under a reduced pressure, and the residues was added to water (20 mL), and extracted with dichloromethane (20 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **211a** (2.3 g, 3.61 mmol, yield: 97.46%) as yellow oil.
MS m/z (ESI): 638.3 [M+1]
¹H NMR (400 MHz, DMSO-*d₆*) δ 7.99 - 7.85 (m, 1H), 7.55 - 7.47 (m, 1H), 7.46 - 7.36 (m, 2H), 6.61 (br s, 1H), 4.10 (br d, J = 4.4 Hz, 2H), 3.90 (br d, J = 12.6 Hz, 2H), 3.67 - 3.54 (m, 4H), 3.02 (br d, J = 6.8 Hz, 8H), 2.55 (s, 3H), 2.37 - 2.30 (m, 1H), 1.69 - 1.61 (m, 2H), 1.38 (s, 12H), 1.05 - 0.91 (m, 6H).

### Step 2

### (3R,5R)-4-[5-fluoro-2-(1-fluoro-3-methyl-6-{1-[(piperidin-4-yl)methyl]azacyclobutan-3-yl}imidazo[1,5-a]pyridin-8-yl)benzoyl]-3,5-dimethylmorpholine 211b

Compound **211a** (160 mg, 250.88 µmol) was dissolved in dichloromethane (3.0 mL), followed by the addition of trifluoroacetic acid (1 g, 8.77 mmol, 675.68 µL) at 25°C. The mixture was stirred at room temperature to allow for reacting for 0.5 hours. The reaction mixture was concentrated under a reduced pressure, to obtain the crude product of title Compound **211b** (130 mg). The crude product was directly used in the next step of reaction without purification.
MS m/z (ESI): 538.3 [M+1]

### Step 3

### 4-[(4-{[3-(8-{2-[(3R,5R)-3,5-dimethylmorpholin-4-carbonyl]-4-fluorophenyl}-1-fluoro-3-methylimidazo[1,5-a]pyridin-6-yl)azacyclobutan-1-yl]methyl}piperidin-1-yl)methyl]oxacyclohexan-4-ol 211

Compound **211b** (78.20 mg, 120 µmol, trifluoroacetate), triethylamine (60.71 mg, 600.00 µmol) and 1,6-dioxaspiro[2.5]octane (27.39 mg, 240.00 µmol) were dissolved in isopropanol (1.0 mL), and stirred at 60°C for 12 hours and the reaction mixture was concentrated under a reduced pressure. The resulting residues were purified by the HPLC (mobile phase: acetonitrile, water (0.1% ammonia water), elution gradient: 30%-60%), to obtain the title Compound **211** (5.09 mg, 7.81 µmol, yield: 6.51 %) as yellow solid.
MS m/z (ESI): 652.5 [M+1]
¹H NMR (400 MHz, CD₃OD) δ 7.82 (s, 1H), 7.59 - 7.47 (m, 1H), 7.41 - 7.27 (m, 2H), 6.65 (s, 1H), 3.92 - 3.65 (m, 9H), 3.46 - 3.33 (m, 1H), 3.30 - 3.09 (m, 4H), 2.99 - 2.78 (m, 3H), 2.60 (s, 3H), 2.49 - 2.40 (m, 2H), 2.31 (s, 2H), 2.27 - 2.18 (m, 2H), 1.75 - 1.57 (m, 4H), 1.53 - 0.89 (m, 11H).

### Example 212

### (3R,5R)-4-[5-fluoro-2-(3-methyl-6-{1-[(oxacyclohexan-4-yl)methyl]azacyclobutan-3-yl}imidazo[1,5-a]pyridin-8-yl)benzoyl]-3,5-dimethylmorpholine 212

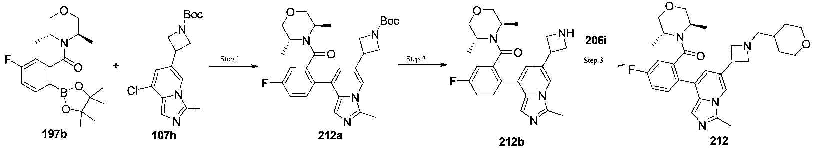

### Step 1

### 3-(8-{2-[(3R,5R)-3,5-dimethylmorpholin-4-carbonyl]-4-fluorophenyl}-3-methylimidazo[1,5-a]pyridin-6-yl)azacyclobutan-1-tert-butyl carboxylate 212a

Compound **107h** (300 mg, 932.25 µmol), Compound **197b**(372.48 mg, 1.03 mmol) and potassium phosphate (495 mg, 2.33 mmol) were dissolved in dioxane (3.0 mL) and water (0.5 mL). Pd(dtbpf)Cl₂ (61 mg, 93.59 µmol) was added. Under a nitrogen atmosphere, the mixture was stirred at 90°Cto allow for reacting for 2.0 hours. The reaction mixture was cooled to room temperature, water (15 mL) was added, and the mixture was extracted with ethyl acetate (20 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **212a** (400 mg, 765.39 µmol, yield: 82.1%).
MS m/z (ESI): 523.2 [M+1]

### Step 2

### (3R,5R)-4-{2-[6-(azacyclobutan-3-yl)-3-methylimidazo[1,5-a]pyridin-8-yl]-5-fluorobenzoyl}-3,5-dimethylmorpholine 212b

Compound **212a** (110 mg, 210.48 µmol) was dissolved in dichloromethane (3.0 mL), followed by the addition of trifluoroacetic acid (1.48 g, 12.98 mmol, 1 mL) at 25°C. The mixture was stirred at room temperature to allow for reacting for 1.0 hours. The reaction mixture was concentrated under a reduced pressure, to obtain the crude product of title Compound **212b** (110 mg). The crude product was directly used in the next step of reaction without purification.
MS m/z (ESI): 423.1 [M+1]

### Step 3

### (3R,5R)-4-[5-fluoro-2-(3-methyl-6-{1-[(oxacyclohexan-4-yl)methyl]azacyclobutan-3-yl}imidazo[1,5-a]pyridin-8-yl)benzoyl]-3,5-dimethylmorpholine 212

The crude product of Compound **212b** (110 mg) and Compound **206i** (28.76 mg, 252.00 µmol) were dissolved in methanol (2.0 mL), followed by the addition of triethylamine (25.50 mg, 252.00 µmol), and the mixture was stirred at 25°C for 0.5 hours. The reaction mixture was cooled to room temperature, followed by adding sodium cyanoborohydride (39.59 mg, 630.00 µmol) and stirring at 25°C for 11.0 hour. A saturated aqueous sodium bicarbonate solution (15 mL) was added to the reaction mixture, which was then extracted with dichloromethane (20 mL x 3). The organic phases were combined, dried over anhydrous sodium sulfate, and concentrated under a reduced pressure. The resulting residues were purified by the HPLC (mobile phase: acetonitrile, water (0.1% formic acid), elution gradient: 0%-26%), to obtain the title Compound **212** (29.98 mg, 57.58 µmol, yield: 27.42 %).
MS m/z (ESI): 521.3 [M+1]
¹H NMR (400 MHz, CD₃OD) δ 8.39 (s, 1.31H), 8.11 (s, 1H), 7.64 (s, 1H), 7.45 - 7.32 (m, 2H), 7.10 (s, 1H), 6.84 (s, 1H), 4.52 - 4.36 (m, 2H), 4.27 - 4.10 (m, 3H), 4.01 - 3.90 (m, 2H), 3.85 - 3.64 (m, 2H), 3.48 - 3.36 (m, 3H), 3.27 - 3.11 (m, 3H), 2.71 (s, 5H), 2.02 - 1.88 (m, 1H), 1.74 - 1.62 (m, 2H), 1.45 - 1.28 (m, 2H), 1.24 - 0.58 (m, 6H).

### Example 213

### (3R,5R)-4-{5-fluoro-2-[1-fluoro-3-methyl-6-(1-{[(1r,4r)-4-(1H-1,2,4-triazol-1-yl)cyclohexyl]methyl}azacyclobutan-3-yl)imidazo[1,5-a]pyridin-8yl]benzoyl}-3,5-dimethylmorpholine 213

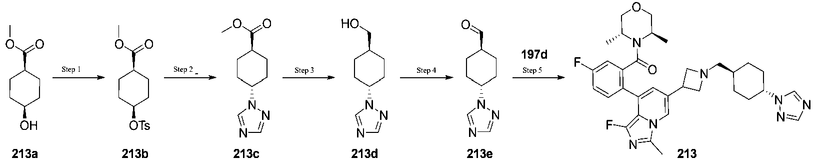

### Step 1

### (1s,4s)-4-[(4-methylphenylsulfonyl)oxy]cyclohexan-1-methyl carboxylate 213b

(1*s*,4*s*)-4-hydroxylcyclohexan-1-methyl carboxylate **213a** (5 g, 31.61 mmol) was dissolved in DCM(70 mL), followed by the addition of DMAP (386.14 mg, 3.16 mmol) and triethylamine (9.59 g, 94.82 mmol). The reaction mixture was cooled to 0°C, followed by adding 4-toluensulfonyl chloride (7.23 g, 37.93 mmol). The reaction mixture was stirred at 25°C for 12.0 hours. Water (50 mL) was added to the reaction mixture, which was then extracted with dichloromethane (50 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **213b** (6.0 g, 19.21 mmol, yield60.77%) as yellow solid.
MS m/z (ESI): 330.2 [M+1]
¹H NMR (400 MHz, DMSO-*d₆*) δ 7.83 - 7.74 (m, 2H), 7.50 - 7.41 (m, 2H), 4.71 - 4.60 (m, 1H), 3.62 - 3.55 (m, 3H), 2.43 - 2.35 (m, 4H), 1.67 - 1.56 (m, 8H).

### Step 2

### (1r,4r)-4-(1H-1,2,4-triazol-1-yl)cyclohexan-1-methyl carboxylate 213c

1H-1,2,4-triazole (1 g, 14.48 mmol) was dissolved in acetonitrile (40 mL), followed by the addition of Compound **213b** (3.62 g, 11.58 mmol), cesium carbonate (9.44 g, 28.96 mmol) and TBAI(534.81 mg, 1.45 mmol). The reaction mixture was heated to 85°C and stirred for 6.0 hours. The reaction mixture was filtered under a reduced pressure, and washed with ethyl acetate (100 mL), and the filtrate was concentrated under a reduced pressure. The resulting residues were purified by the silica-gel column chromatography using petroleum ether/ethyl acetate as an elution system, to obtain the title Compound **213c** (500 mg, 2.39 mmol, yield16.5%) as white solid.

¹H NMR (400 MHz, DMSO-*d₆*) δ 8.52 (s, 1H), 7.99 - 7.89 (m, 1H), 4.34 - 4.23 (m, 1H), 3.62 - 3.60 (m, 3H), 2.45 - 2.36 (m, 1H), 2.11 - 2.00 (m, 4H), 1.83 - 1.73 (m, 2H), 1.58 - 1.48 (m, 2H).

### Step 3

### [(1r,4r)-4-(1H-1,2,4-triazol-1-yl)cyclohexyl]methanol 213d

Compound **213c** (500 mg, 2.39 mmol) was dissolved in DCM(5 mL). Under a nitrogen atmosphere, the mixture was cooled to 0°C, followed by the slow dropwise addition of DIBAL-H (1 M, 4.78 mL), and the mixture was warmed to room temperature and stirred to allow for reacting for 5.0 hours. DIBAL-H (1 M, 2.0 mL) was supplemented, and the mixture was stirred at room temperature to allow for reacting for 12 hours . The reaction mixture was quenched by adding sodium sulfate decahydrate and filtered, and the filtrate was concentrated under a reduced pressure, to obtain the crude product of title Compound **213d** (350 mg, 1.93 mmol, yield: 80.82%) as white solid.

¹H NMR (400 MHz, DMSO-*d₆*) δ 8.53 (s, 1H), 7.96 - 7.89 (m, 1H), 4.50 - 4.45 (m, 1H), 4.27 - 4.13 (m, 1H), 3.28 - 3.22 (m, 2H), 2.09 - 2.02 (m, 2H), 1.89 - 1.81 (m, 2H), 1.77 - 1.64 (m, 2H), 1.50 - 1.35 (m, 1H), 1.14 - 1.01 (m, 2H).

### Step 4

### (1r,4r)-4-(1H-1,2 4-triazol-1-yl)cyclohexan-1-formaldehyde 213e

Compound **213d** (150 mg, 827.66 µmol) was dissolved in DMSO(4 mL). Under a nitrogen atmosphere, the mixture was cooled to 0°C, and a Burgess reagent (256.41 mg, 1.08 mmol) was added slowly. The reaction mixture was stirred at room temperature to allow for reacting for 12 hour. In an ice water bath, water (5 mL) was added to the reaction mixture, which was then extracted with ethyl acetate (15 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **213e** (50 mg, 278.99 µmol, yield: 33.71%) as colorless oil.

¹H NMR (400 MHz, DMSO-*d₆*) δ 9.61 (s, 1H), 8.52 - 8.50 (m, 1H), 7.94 - 7.93 (m, 1H), 4.27 - 4.23 (m, 1H), 2.09 - 2.07 (m, 2H), 1.80 - 1.77 (m, 2H), 1.56 - 1.49 (m, 2H), 1.40 - 1.36 (m, 1H), 1.29 - 1.18 (m, 2H).

### Step 5

### (3R,5R)-4-{5-fluoro-2-[1-fluoro-3-methyl-6-(1-{[(1r,4r)-4-(1H-1,2,4-triazol-1-yl)cyclohexyl]methyl}azacyclobutan-3-yl)imidazo[1,5-a]pyridin-8yl]benzoyl}-3,5-dimethylmorpholine 213

Compound **197d** (40 mg, 90.81 µmol) and Compound **213e** (50.00 mg, 278.99 µmol) were dissolved in methanol (2.0 mL). Acetic acid (5.45 mg, 90.81 µmol) was added, and the mixture was stirred for 1.0 hour. Sodium cyanoborohydride (17.12 mg, 272.43 µmol) was added, and the mixture was stirred at room temperature for 12.0 hours. A saturated aqueous sodium bicarbonate solution (10 mL) was added, and the mixture was then extracted with dichloromethane (20 mL × 3). The organic phases were combined, dried over anhydrous sodium sulfate, and concentrated under a reduced pressure. The resulting residues were purified by the HPLC (mobile phase: acetonitrile, water (0.1% ammonium bicarbonate), elution gradient: 27%-57%), to obtain the title Compound **213** (8.78 mg, 14.54 µmol, yield: 16.02%).
MS m/z (ESI): 604.2[M+1]
¹H NMR (400 MHz, CD₃OD) δ 8.47 (s, 1H), 7.98 - 7.94 (m, 1H), 7.87 - 7.81 (m, 1H), 7.56 - 7.49 (m, 1H), 7.37 - 7.31 (m, 2H), 6.74 - 6.60 (m, 1H), 4.61 - 4.59 (m, 1H), 4.33 - 4.24 (m, 1H), 3.93 - 3.64 (m, 6H), 2.96 - 2.78 (m, 1H), 2.62 - 2.60 (m, 3H), 2.54 - 2.48 (m, 2H), 2.20 - 2.12 (m, 2H), 2.06 - 1.76 (m, 5H), 1.59 - 1.47 (m, 1H), 1.41 - 0.73 (m, 10H).

### Example 220

### 4-{[3-(8-{2-[(3R,5R)-3,5-dimethylmorpholin-4-carbonyl]-4-fluorophenyl}-1-fluoro-3-methylimidazo[1,5-a]pyridin-6-yl)azacyclobutan-1-yl]methyl}oxacyclohexan-4-ol 220

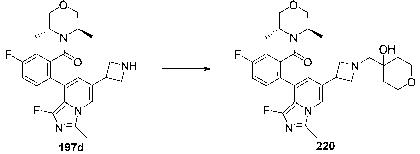

Compound **197d** (88.72 mg, 160 µmol, trifluoroacetate), triethylamine (80.95 mg, 800.00 µmol) and 1,6-dioxaspiro[2.5]octane (36.53 mg, 320.00 µmol) were dissolved in isopropanol (1.0 mL), and stirred at 60°C for 12 hours and the reaction mixture was concentrated under a reduced pressure. The resulting residues were purified by the HPLC (mobile phase: acetonitrile, water (0.1% ammonia water), elution gradient:23%-53%), to obtain the title Compound **220** (31.11 mg, 56.09 µmol, yield: 35.06%) as yellow solid.
MS m/z (ESI): 555.2 [M+1]
¹H NMR (400 MHz, DMSO-*d₆*) δ 7.89 (s, 1H), 7.58 - 7.33 (m, 3H), 6.60 (s, 1H), 4.08 (s, 1H), 3.84 - 3.43 (m, 10H), 3.29 - 3.05 (m, 4H), 2.74 - 2.52 (m, 4H), 2.40 (s, 2H), 1.58 - 1.43 (m, 2H), 1.39 - 1.25 (m, 2H), 1.17 - 0.53 (m, 6H).

### Example 228

### 1-[4-({3-[(8-{2-[(3R,5R)-3,5-dimethylmorpholin-4-carbonyl]-4-fluorophenyl}-3-methylimidazo[1,5-a]pyridin-6-yl)methyl]azacyclobutan-1-yl}methyl)piperidin-1-yl]ethan-1-one 228

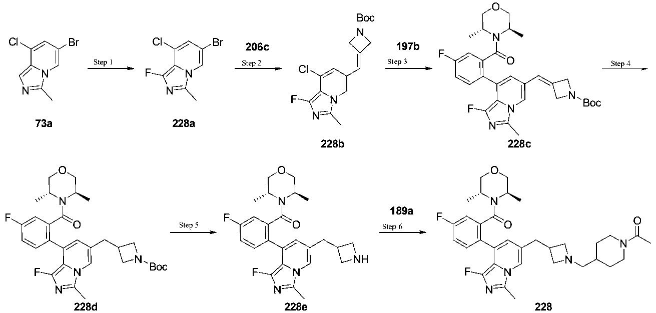

### Step 1

### 6-bromo-8-chloro-1-fluoro-3-methylimidazo[1,5-a]pyridine 228a

Compound **73a** (11.63 g, 47.37 mmol) was dissolved in acetonitrile (500 mL), followed by the addition of pyridine (5.62 g, 71.06 mmol). The mixture was cooled to -10°C, a Selectfluor fluridizer (16.78 g, 47.37 mmol) was added, and the mixture was stirred at -10°C to allow for reacting for 0.5 hours. A saturated aqueous sodium thiosulfate solution (300 mL) was added to the reaction mixture, which was then extracted with ethyl acetate (300 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **228a** (4.8 g, 18.22 mmol, yield: 38.45%).

¹H NMR (400 MHz, CDCl₃) δ 7.60 (s, 1H), 6.65 (s, 1H), 2.56 (s, 3H).

### Step 2

### 3-({8-chloro-3-methylimidazo[1,5-a]pyridin-6-yl}methylene)azacyclobutan-1-tert-butyl carboxylate 228b

Compound **228a** (800 mg, 3.04 mmol), 3-[(tetramethyl-1,3,2-dioxaboran-2-yl)methylene]azacyclobutan-1-tert-butyl carboxylate **206c** (941.02 mg, 3.19 mmol) and sodium carbonate (965.39 mg, 9.11 mmol) were dissolved in dioxane (9 mL) and water (1.8 mL). Pd (dppf)Cl₂ (222.15 mg, 303.61 µmol) was added. Under a nitrogen atmosphere, the mixture was stirred at 95°Cto allow for reacting for 12.0 hours. The reaction mixture was cooled to room temperature, followed by adding water (20 mL) and extracting with ethyl acetate (20 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **228b** (770 mg, 2.19 mmol, yield: 72.09%).
MS m/z (ESI): 352.1 [M+1]

### Step 3

### 3-[(8-{2-[(3R,5R)-3,5-dimethylmorpholin-4-carbonyl]-4-fluorophenyl}-3-methylimidazo[1,5-a]pyridin-6-yl)methylene]azacyclobutan-1-tert-butyl carboxylate 228c

Compound **228b** (200 mg, 568.5 µmol) and Compound **197b** (216.82 mg, 596.93 µmol) were dissolved in dioxane (4 mL) and water (1.0 mL). Potassium phosphate (362.02 mg, 1.71 mmol) and Pd(dtbpf)Cl₂ (37.05 mg, 56.85 µmol) were added, and the mixture was stirred at 90 °C under a nitrogen atmosphere to allow for reacting for 2 hours. The reaction mixture was cooled to room temperature, water (15 mL) was added, and the mixture was extracted with ethyl acetate (15 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **228c** (220 mg, 398.11 µmol, yield: 70.03%).
MS m/z (ESI): 553.2 [M+1]

### Step 4

### 3-[(8-{2-[(3R,5R)-3,5-dimethylmorpholin-4-carbonyl]-4-fluorophenyl}-3-methylimidazo[1,5-a]pyridin-6-yl)methyl]azacyclobutan-1-tert-butyl carboxylate 228d

Compound **228c** (120 mg, 217.15 µmol) was dissolved in methanol (4.0 mL), followed by the addition of 10% Pd/C(100 mg, 939.67 µmol) under a nitrogen atmosphere. The reaction atmosphere was substituted with a nitrogen atmosphere (15 psi), and the mixture was stirred at room temperature for 2.0 hours. The reaction mixture was filtered, and the filtrate was concentrated under a reduced pressure, to obtain the crude product of title Compound **228d** (110 mg, 198.33 µmol, yield: 91.33%) as yellow solid.
MS m/z (ESI): 555.3 [M+1]

### Step 5

### (3R,5R)-4-(2-{6-[(azacyclobutan-3-yl)methyl]-3-methylimidazo[1,5-a]pyridin-8-yl}-5-fluorobenzoyl)-3,5-dimethylmorpholine 228e

Compound **228d** (110 mg, 198.33 µmol) was dissolved in methane dioxide (3.0 mL), followed by the addition of trifluoroacetic acid (1.48 g, 12.98 mmol, 1 mL) at 0°C. The mixture was stirred at room temperature to allow for reacting for 0.5 hours. The reaction mixture was concentrated under a reduced pressure, followed by adding a saturated aqueous sodium bicarbonate solution (10.0 mL) and extracting with dichloromethane (10 mL x 3). The organic phases were combined, dried over anhydrous sodium sulfate, and concentrated under a reduced pressure, to obtain the crude product of title Compound **228e** (90 mg, 198.01 µmol, yield: 99.84%). The crude product was directly used in the next step of reaction without purification.
MS m/z (ESI): 455.2 [M+1]

### Step 6

### 1-[4-({3-[(8-{2-[(3R,5R)-3,5-dimethylmorpholin-4-carbonyl]-4-fluorophenyl}-3-methylimidazo[1,5-a]pyridin-6-yl)methyl]azacyclobutan-1-yl}methyl)piperidin-1-yl]ethan-1-one 228

Compound **228e** (90 mg, 198.01 µmol) and Compound **189a** (46.10 mg, 297.02 µmol) were dissolved in methanol (2.0 mL). Acetic acid (11.89 mg, 198.01 µmol) was added, and the mixture was stirred for 0.5 hour. Sodium cyanoborohydride (37.33 mg, 594.04 µmol) was added, and the mixture was stirred at room temperature for 12.0 hours. A saturated aqueous sodium bicarbonate solution (10 mL) was added, and the mixture was then extracted with dichloromethane (10 mL × 3). The organic phases were combined, dried over anhydrous sodium sulfate, and concentrated under a reduced pressure. The resulting residues were purified by the HPLC (mobile phase: acetonitrile, water (0.1% formic acid), elution gradient: 8%-38%), to obtain the title Compound **228** (33.84 mg, 57.00 µmol, yield: 28.78%)
MS m/z (ESI): 594.2[M+1]
¹H NMR (400 MHz, DMSO-*d₆*) δ 7.86 (s, 1H), 7.54 - 7.33 (m, 3H), 6.42 - 6.19 (m, 1H), 4.36 - 4.27 (m, 1H), 3.80 - 3.70 (m, 2H), 3.42 - 3.16 (m, 5H), 2.98 - 2.90 (m, 3H), 2.76 - 2.67 (m, 3H), 2.59 - 2.53 (m, 3H), 2.36 - 2.30 (m, 2H), 2.09 - 2.07 (m, 2H), 1.98 - 1.94 (m, 3H), 1.69 - 1.60 (m, 2H), 1.56 - 1.45 (m, 1H), 1.19 - 0.56 (m, 9H).

### Example 230

### (3R,5R)-4-{5-fluoro-2-[4-methyl-6-({1-[(oxacyclobutan-4-yl)methyl]azacyclobutan-3-yl}methyl)pyrrolo[1,2-a]pyrazin-8-yl]benzoyl}-3,5-dimethylmorpholine 230

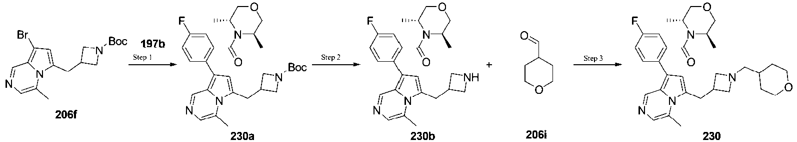

### Step 1

### 3-[(8-{2-[(3R,5R)-3,5-dimethylmorpholin-4-carbonyl]-4-fluorophenyl}-4-methylpyrrolo[1,2-a]pyrazin-6-yl)methyl]azacyclobutan-1-tert-butyl carboxylate 230a

Compound **206f** (170 mg, 447.04 µmol), Compound **197b**(178.62 mg, 491.74 µmol) and potassium phosphate (237.23 mg, 1.12 mmol) were dissolved in dioxane (2 mL) and water (0.3 mL). Pd(dtbpf)Cl₂ (29.14 mg, 44.70 µmol) was added. Under a nitrogen atmosphere, the mixture was stirred at 90°Cto allow for reacting for 2.0 hours. The reaction mixture was cooled to room temperature, followed by adding water (20 mL) and extracting with ethyl acetate (20 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **230a** (220 mg, 409.96 µmol, yield: 91.71%) as yellow solid.
MS m/z (ESI): 537.3 [M+1]

### Step 2

### (3R,5R)-4-(2-{6-[(azacyclobutan-3-yl)methyl]-4-methylpyrrolo[1,2-a]pyrazin-8-yl}-5-fluorobenzoyl)-3,5-dimethylmorpholine 230b

Compound **230a** (110 mg, 204.98 µmol) was dissolved in methane dioxide (3.0 mL), followed by the addition of trifluoroacetic acid (1.48 g, 12.98 mmol, 1 mL) at 0°C. The mixture was stirred at room temperature to allow for reacting for 1.0 hours. The reaction mixture was concentrated under a reduced pressure, to obtain the crude product of title Compound **230b** (115 mg). The crude product was directly used in the next step of reaction without purification.
MS m/z (ESI): 437.1 [M+1]

### Step 3

### (3R,5R)-4-{5-fluoro-2-[4-methyl-6-({1-[(oxacyclobutan-4-yl)methyl]azacyclobutan-3-yl}methyl)pyrrolo[1,2-a]pyrazin-8-yl]benzoyl}-3,5-dimethylmorpholine 230

Compound **230b** (110.11 mg, 200 µmol, trifluoroacetate) and Compound **206i** (34.24 mg, 300.00 µmol) were dissolved in methanol (1.0 mL). Triethylamine (30.36 mg, 300.00 µmol) was added, and the mixture was stirred at room temperature for 1.0 hour. Sodium cyanoborohydride (37.71 mg, 600.00 µmol) was added, and the mixture was stirred at room temperature for 12.0 hours. A saturated aqueous sodium bicarbonate solution (10 mL) was added, and the mixture was then extracted with dichloromethane (20 mL × 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 27%-57%), to obtain the title Compound **230** (27.59 mg, 51.60 µmol, yield: 25.80%)
MS m/z (ESI): 535.3[M+1]
¹H NMR (400 MHz, DMSO-*d*₆) δ 8.59 - 8.24 (m, 1H), 7.59 - 7.17 (m, 4H), 6.75 - 6.48 (m, 1H), 3.83 - 3.75 (m, 2H), 3.73 - 3.47 (m, 4H), 3.43 - 3.37 (m, 2H), 3.26 - 3.11 (m, 4H), 2.89 - 2.65 (m, 7H), 2.25 (d, J = 6.8 Hz, 2H), 1.62 - 0.33 (m, 12H).

### Example 232

### (3R)-4-[5-fluoro-2-(1-fluoro-3-methyl-6-{1-[(oxacyclohexan-4-yl)methyl]azacyclobutan-3-yl}imidazo[1,5-a]pyridin-8-yl)benzoyl]-3-methylmorpholine 232

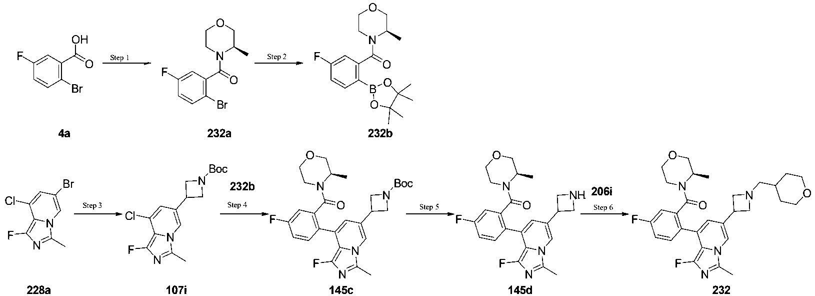

### Step 1

### (3R)-4-(2-bromo-5-fluorobenzoyl)-3-methylmorpholine 232a

2-Bromo-5-fluoro-benzoic acid **4a** (40 g, 182.64 mmol) was dissolved in dichloromethane (500 mL), followed by the addition of DIPEA (70.82 g, 547.93 mmol) and HATU (83.34 g, 219.17 mmol). The reaction mixture was stirred for 5 min, followed by the addition of (3R)-3-methylmorpholine (20.32 g, 200.91 mmol). The mixture was stirred at 50°C for 2 hours. The reaction was quenched with a saturated aqueous sodium carbonate solution (300 mL), and the reaction mixture was extracted with dichloromethane (200 ml × 3). The organic phase was washed with 1M HCl aqueous solution (200 ml×3), dried over anhydrous sodium sulfate, filtered and concentrated under a reduced pressure, to obtain the crude product of title Compound **232a** (55 g, 182.04 mmol, yield99.67%) as yellow oil.
MS m/z (ESI): 301.9 [M+1]

### Step 2

### (3R)-4-[5-fluoro-2-(tetramethyl-1,3,2-dioxaboran-2-yl)benzoyl]-3-methylmorpholine 232b

Compound **232a** (50 g, 165.49 mmol), potassium acetate (48.72 g, 496.46 mmol) and bis(pinacolato)diboron (100 g, 393.80 mmol) were dissolved in DMSO (700 mL). Under a nitrogen atmosphere, Pd(dppf)Cl₂ (10 g, 13.67 mmol) was added, and the mixture was stirred at 110°C to allow for reacting for 6.0 hours. After the mixture was cooled to room temperature, 500 mL of water was added, and the mixture was extracted with ethyl acetate (400 ml ×3). The organic phases were combined, dried over anhydrous sodium sulfate, filtered and concentrated under a reduced pressure to obtain a crude product. The resulting residues were purified by the silica-gel column chromatography using petroleum ether/ethyl acetate as an elution system, to obtain the title Compound **232b** (36 g, 103.09 mmol, yield: 62.3%) as colorless oil.
MS m/z (ESI): 349.9 [M+1]

### Step 3

### 3-{8-chloro-1-fluoro-3-methylimidazo[1,5-a]pyridin-6-yl}azacyclobutan-1-tert-butyl carboxylate 107i

Under the protection of nitrogen, 1,2-dibromoethane (2.59 g, 13.78 mmol) was dissolved in DMAc (250 mL), followed by the addition of zinc powder (8.32 g, 127.16 mmol). The mixture was stirred at 70°C for 10 min, and then cooled to room temperature, followed by the slow dropwise addition of TMSC1 (1.15 g, 10.60 mmol) to the reaction mixture, and after the dropwise addition, the reaction mixture was further stirred at 25° for 20 min. Then, the solution of 3-iodoazacyclobutan-1-tert-butyl carboxylate (30 g, 105.97 mmol) in DMAc (50 mL) was dropwise added to the reaction mixture. The reaction mixture was warmed to 35°C and stirred for 12 hours, until the disappearance of noticeable solid precipitates in the mixture. The reaction mixture was cooled to 25°C, followed by the addition of Compound **228a** (20 g, 75.90 mmol), Pd₂ (dba)₃ (5 g, 8.70 mmol) and tri(2-furyl)phosphine (2.46 g, 10.60 mmol) to the reaction, and the reaction mixture was then subjected to nitrogen substitution three times, warmed to 70°C and stirred to allow for reacting for 5 hours. The reaction mixture was cooled to room temperature, followed by adding water (500 mL) and extracting with ethyl acetate (500 mL x 3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **107i** (12.7 g, 37.38 mmol, yield: 35.27%) as yellow solid.
MS m/z (ESI): 340.1 [M+1]

### Step 4

### 3-(1-fluoro-8-{4-fluoro-2-[(3R)-3-methylmorpholin-4-carbonyl]phenyl}-3-methylimidazo[1,5-a]pyridin-6-yl)azacyclobutan-1-tert-butyl carboxylate 145c

Compound **107i** (12.7 g, 37.38 mmol), Compound **232b**(19.58 g, 56.06 mmol) and potassium phosphate (23.80 g, 112.13 mmol) were dissolved in dioxane (150 mL) and water (30 mL). Pd(dtbpf)Cl₂ (2.44 g, 3.74 mmol) was added. Under a nitrogen atmosphere, the mixture was stirred at 95°Cto allow for reacting for 12.0 hours. The reaction mixture was cooled to room temperature, water (200 mL) was added, and the mixture was extracted with ethyl acetate (150 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **145c** (18.87 g, 35.84 mmol, yield: 95.88%).
MS m/z (ESI): 527.3 [M+1]

### Step 5

### (3R)-4-{2-[6-(azacyclobutan-3-yl)-1-fluoro-3-methylimidazo[1,5-a]pyridin-8-yl]-5-fluorobenzoyl}-3-methylmorpholine 145d

Compound **145c** (150 mg, 284.86 µmol) was dissolved in methane dioxide (5.0 mL), followed by the addition of trifluoroacetic acid (1.48 g, 12.98 mmol, 1 mL) at 0°C. The mixture was stirred at room temperature to allow for reacting for 1.0 hours. The reaction mixture was concentrated under a reduced pressure, followed by the addition of water (5.0 mL), and the reaction mixture was then regulated to PH > 7 with a 10% aqueous sodium hydroxide solution, and extracted with dichloromethane (10 mL x 3). The organic phases were combined, dried over anhydrous sodium sulfate, and concentrated under a reduced pressure, to obtain the crude product of title Compound **145d** (121.48 mg, 284.86 µmol, yield: 100%). The crude product was directly used in the next step of reaction without purification.
MS m/z (ESI): 427.1 [M+1]

### Step 6

### (3R)-4-[5-fluoro-2-(1-fluoro-3-methyl-6-{1-[(oxacyclohexan-4-yl)methyl]azacyclobutan-3-yl}imidazo[1,5-a]pyridin-8-yl)benzoyl]-3-methylmorpholine 232

The crude product of Compound **145d** (121.48 mg, 284.86 µmol) and Compound **206i** (48.77 mg, 427.29 µmol) were dissolved in methanol (3.0 mL). Acetic acid (17.11 mg, 284.86 µmol) was added, and the mixture was stirred for 1.0 hour. Sodium cyanoborohydride (53.70 mg, 854.57 µmol) was added, and the mixture was stirred at room temperature for 12.0 hours. A saturated aqueous sodium bicarbonate solution (10 mL) was added, and the mixture was then extracted with dichloromethane (20 mL × 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 28%-58%), to obtain the title Compound **232** (17.68 mg, 33.70 µmol, yield: 11.83%) as yellow solid.
MS m/z (ESI): 525.3[M+1]
¹H NMR (400 MHz, CD₃OD) δ 7.81 (d, J = 9.2 Hz, 1H), 7.64 - 7.52 (m, 1H), 7.39 - 7.16 (m, 2H), 6.87 - 6.57 (m, 1H), 4.09 - 3.90 (m, 4H), 3.82 - 3.67 (m, 4H), 3.62 - 3.56 (m, 1H), 3.45 - 3.36 (m, 4H), 2.60 (s, 3H), 2.50 (d, J = 6.4 Hz, 2H), 1.73 - 1.62 (m, 4H), 1.50 - 1.39 (m, 1H), 1.36 - 1.23 (m, 4H), 1.20 - 1.12 (m, 1H), 0.81 - 0.80 (m, 1H), 0.80 (d, J = 5.6 Hz, 1H).

### Example 237

### (1r,4r)-4-{[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]methyl}-N-methylcyclohexan-1-formamide 237

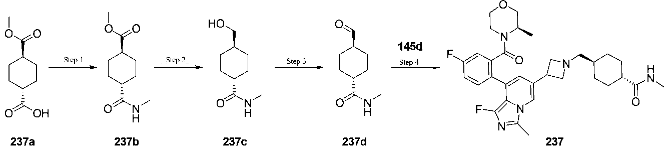

### Step 1

### (1r,4r)-4-(methylaminoformyl)cyclohexan-1-methyl carboxylate 237b

(1*r*,4*r*)-4-(methoxycarbonyl)cyclohexan-1-carboxylic acid **237a** (1 g, 5.37 mmol) was dissolved in DCM (10 mL), followed by the addition of HATU(2.45 g, 6.44 mmol) and triethylamine (1.63 g, 16.11 mmol). The reaction mixture was stirred for 5 min, followed by the addition of methylamine (435.12 mg, 6.44 mmol). The reaction mixture was stirred at 25°C for 12.0 hours. A saturated aqueous sodium carbonate solution (25 mL) was added to the reaction mixture, which was then extracted with dichloromethane (25 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **237b** (920 mg, 4.62 mmol, yield: 85.98%) as white solid.

¹H NMR (400 MHz, DMSO-*d₆*) δ 7.63 (d, J = 4.0 Hz, 1H), 3.58 (s, 3H), 2.54 (d, J = 4.4 Hz, 3H), 2.31 - 2.20 (m, 1H), 2.08 - 1.97 (m, 1H), 1.95 - 1.86 (m, 2H), 1.77 - 1.69 (m, 2H), 1.43 - 1.22 (m, 4H).

### Step 2

### (1r,4r)-4-(hydroxylmethyl)-N-methylcyclohexan-1-formamide 237c

Compound **237b** (920 mg, 4.62 mmol) was dissolved in ethanol (19.88 mL). Under a nitrogen atmosphere, the mixture was cooled to -10°C, sodium borohydride (680 mg, 17.97 mmol) was added slowly, and the mixture was warmed to room temperature and stirred to allow for reacting for 11 hours. Calcium chloride (1.02 g, 9.23 mmol) was supplemented, and the mixture was warmed to 55°C and further stirred to allow for reacting for 2 hours. The reaction mixture was quenched by adding a saturated aqueous ammonium chloride solution (20 mL) and filtered, and the filtrate was extracted with ethyl acetate (50 mL x 3). The organic phase was dried over anhydrous sodium sulfate, and concentrated under a reduced pressure, to obtain the crude product of title Compound **237c** (110 mg, 642.39 µmol, yield: 13.91%) as yellow solid.

¹H NMR (400 MHz, DMSO-*d₆*) δ 7.60 (d, J = 4.0 Hz, 1H), 4.42 - 4.27 (m, 1H), 3.26 - 3.12 (m, 2H), 2.56 - 2.51 (m, 3H), 2.03 - 1.94 (m, 1H), 1.78 - 1.66 (m, 4H), 1.35 - 1.24 (m, 3H), 0.91 - 0.80 (m, 2H).

### Step 3

### (1r,4r)-4-formyl-N-methylcyclohexan-1-formamide 237d

Compound **237c** (100.00 mg, 583.99 µmol) was dissolved in DMSO(1 mL). Under a nitrogen atmosphere, the mixture was cooled to 0°C, and a Burgess reagent (180.92 mg, 759.19 µmol) was added slowly. The reaction mixture was stirred at room temperature to allow for reacting for20 min. In an ice water bath, water (5 mL) was added to the reaction mixture, which was then extracted with ethyl acetate (10 mL x 3). The organic phases were combined, dried over anhydrous sodium sulfate, and concentrated under a reduced pressure, to obtain the crude product of title Compound **237d** (97 mg, 573.22 µmol, yield: 98.16%) as yellow oil. The crude product was directly used in the next step of reaction without purification.

### Step 4

### (1r,4r)-4-{[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]methyl}-N-methylcyclohexan-1-formamide 237

Compound **145d** (81 mg, 189.04 µmol) and Compound **237d** (38.39 mg, 226.85 µmol) were dissolved in methanol (2.0 mL). Triethylamine (57.39 mg, 567.13 µmol) was added, and the mixture was stirred at room temperature for 1.0 hour. Sodium cyanoborohydride (36.00 mg, 572.80 µmol) was added, and the mixture was stirred at room temperature for 11.0 hours. A saturated aqueous sodium bicarbonate solution (10 mL) was added, and the mixture was then extracted with dichloromethane (10 mL × 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 27%-57%) two times, to obtain the title Compound **237** (13 mg, 22.43 µmol, yield: 11.86%) as yellow solid.
MS m/z (ESI): 580.3[M+1]
¹H NMR (400 MHz, CD₃OD) δ 8.68 - 8.43 (m, 1H), 7.92 (s, 1H), 7.67 - 7.50 (m, 1H), 7.44 - 7.16 (m, 2H), 6.89 - 6.53 (m, 1H), 4.66 - 4.25 (m, 3H), 4.15 - 3.86 (m, 3H), 3.84 - 3.35 (m, 3H), 3.23 - 2.83 (m, 4H), 2.70 (s, 3H), 2.62 (s, 3H), 2.31 (s, 6H), 1.70 - 1.58 (m, 1H), 1.57 - 1.40 (m, 2H), 1.38 - 0.96 (m, 4H), 0.76 (s, 1H).

### Example 238

### (3R)-4-[5-fluoro-2-(1-fluoro-3-methyl-6-{1-[1-(oxacyclohexan-4-yl)ethyl]azacyclobutan-3-yl}imidazo[1,5-a]pyridin-8-yl)benzoyl]-3-methylmorpholine 238

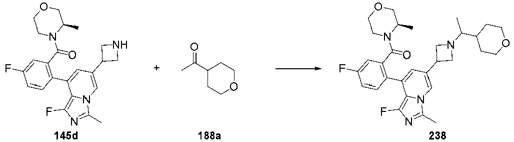

Compound **145d** (81 mg, 189.94 µmol) and Compound **188a** (26.78 mg, 208.93 µmol) were dissolved in methanol (1.0 mL). Zinc chloride (51.78 mg, 379.87 µmol) was added, and the mixture was stirred at 55°C for 1.0 hour. After cooling, sodium cyanoborohydride (36.17 mg, 575.51 µmol) was added, and the mixture was further stirred at 55°C for 11 hours. A saturated aqueous sodium bicarbonate solution (10 mL) was added, and the mixture was then extracted with dichloromethane (10 mL × 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 31%-61%), to obtain the title Compound **238** (50 mg, 92.83 µmol, yield: 48.87 %) as yellow solid.
MS m/z (ESI): 539.4[M+1]
¹H NMR (400 MHz, DMSO-*d*₆) δ 8.04 - 7.69 (m, 1H), 7.68 - 7.16 (m, 3H), 6.93 - 6.35 (m, 1H), 4.50 - 3.37 (m, 9H), 3.28 - 2.63 (m, 7H), 2.54 (s, 3H), 2.26 - 2.06 (m, 1H), 1.68 - 1.42 (m, 2H), 1.38 - 1.00 (m, 5H), 0.91 - 0.56 (m, 4H).

### Examples 238-1 and 238-2

### (3R)-4-[5-fluoro-2-(1-fluoro-3-methyl-6-{1-[(1R)-1-(oxacyclohexan-4-yl)ethyl]azacyclobutan-3-yl}imidazo[1,5-a]pyridin-8-yl)benzoyl]-3-methylmorpholine 238-1

### (3R)-4-[5-fluoro-2-(1-fluoro-3-methyl-6-{1-[(1S)-1-(oxacyclohexan-4-yl)ethyl]azacyclobutan-3-yl}imidazo[1,5-a]pyridin-8-yl)benzoyl]-3-methylmorpholine 238-2

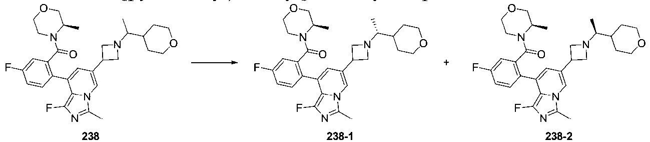

Compound **238** (221 mg, 410 µmol) was purified by the chiral preparative SFC (preparative column: Daicel ChiralPak AD (250*30mm, 10um); mobile phase: CO₂-EtOH (0.1% ammonia water); elution gradient: 15%), to obtain the title Compounds **238-1** (67.24 mg, 124.84 µmol, yield: 30.42 %) and **238-2** (61.24 mg, 113.70 µmol, yield: 27.71 %).

### Compound 238-1:

MS m/z (ESI): 539.3[M+1]
chiral HPLC: retention time: 1.260 min; Chiralpak AD-3 50 x 4.6 mm I.D., 3 um; mobile phase: CO₂-EtOH (0.05%DEA); elution gradient: 5%-40%;
¹H NMR (400 MHz, DMSO-*d*₆) δ 8.01 - 7.73 (m, 1H), 7.68 - 7.18 (m, 3H), 6.92 - 6.46 (m, 1H), 4.41 - 3.41 (m, 9H), 3.29 - 2.84 (m, 7H), 2.55 (s, 3H), 2.26 - 2.06 (m, 1H), 1.63 - 1.42 (m, 2H), 1.35 - 1.06 (m, 5H), 0.84 - 0.68 (m, 4H).

### Compound 238-2:

MS m/z (ESI): 539.4[M+1]
Chiral HPLC: retention time: 1.157 min; Chiralpak AD-3 50 x 4.6 mm I.D., 3 um; mobile phase: CO2-EtOH (0.05%DEA); elution gradient: 5%-40%.
¹H NMR (400 MHz, DMSO-*d*₆) δ 8.10 - 7.76 (m, 1H), 7.67 - 7.18 (m, 3H), 7.02 - 6.30 (m, 1H), 4.72 - 3.35 (m, 9H), 3.29 - 2.74 (m, 7H), 2.54 (s, 3H), 2.21 - 1.99 (m, 1H), 1.62 - 1.43 (m, 2H), 1.35 - 1.26 (m, 2H), 1.24 - 1.10 (m, 3H), 0.87 - 0.59 (m, 4H).

### Example 241

### 1-(4-{[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]methyl}piperidin-1-yl)-2-methoxyethan-1-one 241

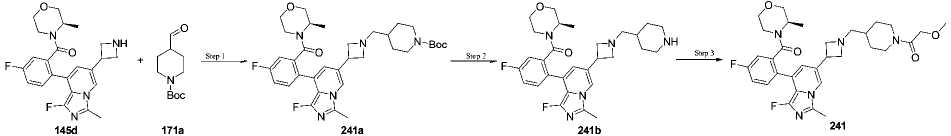

### Step 1

### 4-{[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]methyl}piperidin-1-tert-butyl carboxylate 241a

Compound **145d** (140 mg, 328.29 µmol), 4-formylpiperidin-1-tert-butyl formate **171a** (105.02 mg, 492.43 µmol) and acetic acid (19.71 mg, 328.29 µmol) were dissolved in methanol (2.0 mL), and stirred at room temperature for 1.0 hour. Sodium cyanoborohydride (61.89 mg, 984.86 µmol) was added, and the mixture was stirred at room temperature for 12 hours. A saturated aqueous sodium bicarbonate solution (10 mL) was added to the reaction mixture, which was then extracted with dichloromethane (20 mL x 3). The organic phases were combined, dried over anhydrous sodium sulfate, and concentrated under a reduced pressure. The resulting residues were purified by the preparative thin-layer chromatography using dichloromethane/methanol as an elution system, to obtain the title Compound **241a** (140 mg, 224.46 µmol, yield: 68.37%) as yellow solid.
MS m/z (ESI): 624.3 [M+1]
1.69 - 1.61 (m, 2H), 1.38 (s, 12H), 1.05 - 0.91 (m, 6H).

### Step 2

### (3R)-4-[5-fluoro-2-(1-fluoro-3-methyl-6-{1-[(piperidin-4-yl)methyl]azacyclobutan-3-yl}imidazo[1,5-a]pyridin-8-yl)benzoyl]-3-methylmorpholine 241b

Compound **241a** (140 mg, 224.46 µmol) was dissolved in dichloromethane (3.0 mL), followed by the addition of trifluoroacetic acid (1.48 g, 12.98 mmol, 1 mL) at 25°C. The mixture was stirred at room temperature to allow for reacting for 0.5 hours. The reaction mixture was concentrated under a reduced pressure, to obtain the crude product of title Compound **241b** (100 mg, 190.98 µmol, yield: 85.09%) as yellow solid. The crude product was directly used in the next step of reaction without purification.
MS m/z (ESI): 524.2 [M+1]

### Step 3

### 1-(4-{[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]methyl}piperidin-1-yl)-2-methoxyethan-1-one 241

Triethylamine (47.61 mg, 470.49 µmol) and Compound **241b** (100 mg, 156.83 µmol, trifluoroacetate) were dissolved in dichloromethane (2.0 mL), and stirred at room temperature for 10 min, followed by the addition of 2-methoxyacetyl chloride (20.42 mg, 188.15 µmol). The reaction mixture was stirred at room temperature for 2 hours. A saturated aqueous sodium bicarbonate solution (10 mL) was added to the reaction mixture, which was then extracted with dichloromethane (10 mL × 3). The organic phase was dried over anhydrous sodium sulfate, filtered and concentrated under a reduced pressure. The resulting residues were purified by the HPLC (mobile phase: acetonitrile, water (0.1% ammonia water), elution gradient: 24%-54%), to obtain the title Compound **241** (7.79 mg, 13.08 µmol, yield: 8.34 %) as yellow solid.
MS m/z (ESI): 596.4 [M+1]
¹H NMR (400 MHz, CD₃OD) δ 7.80 (s, 1H), 7.65 - 7.51 (m, 1H), 7.38 - 7.17 (m, 2H), 6.90 - 6.60 (m, 1H), 4.59 - 4.41 (m, 2H), 4.19 - 4.08 (m, 2H), 3.89 - 3.69 (m, 5H), 3.65 - 3.47 (m, 2H), 3.40 - 3.37 (m, 3H), 3.29 - 3.23 (m, 3H), 3.09 - 2.96 (m, 2H), 2.70 - 2.57 (m, 4H), 2.53 - 2.45 (m, 2H), 1.87 - 1.76 (m, 2H), 1.74 - 1.64 (m, 1H), 1.38 - 1.02 (m, 5H), 0.81 - 0.57 (m, 1H).

### Example 243

### (3R)-4-[5-fluoro-2-(3-methyl-6-{1-[(oxacyclohexan-4-yl)methyl]azacyclobutan-3-yl}imidazo[1,5-a]pyridin-8-yl)benzoyl]-3-methylmorpholine 243

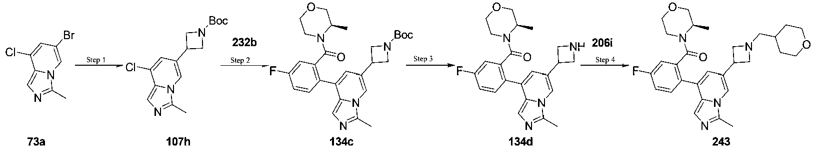

### Step 1

### 3-{8-chloro-3-methylimidazo[1,5-a]pyridin-6-yl}azacyclobutan-1-tert-butyl carboxylate 107h

Under the protection of nitrogen, 1,2-dibromoethane (120.77 mg, 642.87 µmol) was dissolved in DMAc (8.0 mL), followed by the addition of zinc powder (388.04 mg, 5.93 mmol). The mixture was stirred at 70°C for 10 min, and then cooled to room temperature, followed by the slow dropwise addition of TMSCl (53.72 mg, 494.51 µmol) to the reaction mixture, and after the dropwise addition, the reaction mixture was further stirred at 25° for 30 min. Then, the solution of 3-iodoazacyclobutan-1-tert-butyl carboxylate (1.4 g, 4.95 mmol) in DMAc (10 mL) was dropwise added to the reaction mixture. The reaction mixture was warmed to 40°C and stirred for 1 hours, until the disappearance of noticeable solid precipitates in the mixture. The reaction mixture was cooled to 25°C, followed by the addition of Compound **73a** (1.09 g, 4.45 mmol), Pd₂(dba)₃ (284.35 mg, 494.51 µmol) and tri(2-furyl)phosphine (114.81 mg, 494.51 µmol) to the reaction, and the reaction mixture was then subjected to nitrogen substitution three times, warmed to 70°C and stirred to allow for reacting for 12 hours. The reaction mixture was cooled to room temperature, followed by adding water (20 mL) and extracting with ethyl acetate (20 mL x 3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **107h** (560 mg, 1.74 mmol, yield: 35.19%) as brown oil.
MS m/z (ESI): 321.9 [M+1]

### Step 2

### 3-(8-{4-fluoro-2-[(3R)-3-methylmorpholin-4-carbonyl]phenyl}-3-methylimidazo[1,5-a]pyridin-6-yl)azacyclobutan-1-tert-butyl carboxylate 134c

Compound **107h** (500 mg, 1.55 mmol), Compound **232b**(651.09 mg, 1.86 mmol) and potassium phosphate (824.52 mg, 3.88 mmol) were dissolved in dioxane (5.0 mL) and water (1.0 mL). Pd(dtbpf)Cl₂(101.27 mg, 155.38 µmol) was added. Under a nitrogen atmosphere, the mixture was stirred at 90°Cto allow for reacting for 2.0 hours. The reaction mixture was cooled to room temperature, followed by adding water (30 mL) and extracting with ethyl acetate (30 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **134c** (500 mg, 983.12 µmol, yield: 63.27%) as yellow solid.
MS m/z (ESI): 509.2 [M+1]

### Step 3

### (3R)-4-{2-[6-(azacyclobutan-3-yl)-3-methylimidazo[1,5-a]pyridin-8-yl]-5-fluorobenzoyl}-3-methylmorpholine 134d

Compound **134c** (110 mg, 216.29 µmol) was dissolved in methane dioxide (3.0 mL), followed by the addition of trifluoroacetic acid (1.48 g, 12.98 mmol, 1 mL) at 0°C. The mixture was stirred at room temperature to allow for reacting for 1.0 hours. The reaction mixture was concentrated under a reduced pressure to obtain the crude product of title Compound **134d** (100 mg). The crude product was directly used in the next step of reaction without purification.
MS m/z (ESI): 408.9 [M+1]

### Step 4

### (3R)-4-[5-fluoro-2-(3-methyl-6-{1-[(oxacyclohexan-4-yl)methyl]azacyclobutan-3-yl}imidazo[1,5-a]pyridin-8-yl)benzoyl]-3-methylmorpholine 243

The crude product of Compound **134d** (100 mg) and Compound **206i** (36.98 mg, 324.00 µmol) were dissolved in methanol (2.0 mL). Triethylamine (32.79 mg, 324.00 µmol) was added, and the mixture was stirred at room temperature for 0.5 hour. Sodium cyanoborohydride (40.72 mg, 648.00 µmol) was added, and the mixture was stirred at room temperature for 11.0 hours. A saturated aqueous sodium bicarbonate solution (10 mL) was added, and the mixture was then extracted with dichloromethane (20 mL × 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 17%-47%), to obtain the title Compound **243** (63.42 mg, 125.19 µmol, yield: 57.96 %) as yellow solid.
MS m/z (ESI): 507.4[M+1]
¹H NMR (400 MHz, DMSO-*d₆*) δ 8.04 - 7.88 (m, 1H), 7.81 - 7.59 (m, 1H), 7.48 - 7.25 (m, 2H), 7.13 - 6.65 (m, 2H), 4.39 - 3.76 (m, 3H), 3.72 - 3.36 (m, 5H), 3.29 - 3.07 (m, 5H), 3.02 - 2.66 (m, 2H), 2.60 (s, 3H), 2.40 - 2.23 (m, 2H), 1.94 - 0.96 (m, 8H), 0.69 - 0.37 (m, 1H).

### Example 244

### (3R)-4-{5-fluoro-2-[1-fluoro-3-methyl-6-(1-{4-methyl-1-[(2S)-morpholin-2-yl]pentan-3-yl}azacyclobutan-3-yl)imidazo[1,5-a]pyridin-8yl]benzoyl}-3-methylmorpholine 244

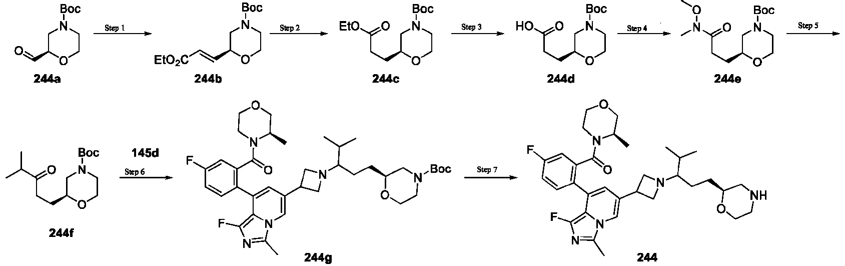

### Step 1

### (2S)-2-[(1E)-3-ethoxy-3-oxopropyl-1-ene-1-yl]morpholin-4-tert-butyl carboxylate 244b

(2*R*)-2-formylmorpholin-4-tert-butyl carboxylate **244a** (18 g, 83.63 mmol) were dissolved in toluene (50 mL), followed by the addition of ethyl(triphenylphosphoranylidene)acetate (29.13 g, 83.63 mmol). The reaction mixture was stirred at 90°C for 16 hours. Water (500 mL) was added to the reaction mixture, which was then extracted with ethyl acetate (300 mL x 3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **244b** (13 g, 45.56 mmol, yield: 54.48%).

¹H NMR (400 MHz, CDCl₃) δ 6.82 (dd, J = 15.8, 4.0 Hz, 1H), 6.11 (d, J = 15.6 Hz, 1H), 4.21 (q, J = 7.0 Hz, 2H), 4.15 - 3.75 (m, 4H), 3.57 (s, 1H), 3.02-2.60 (m, 2H), 1.47 (s, 9H), 1.29 (dd, J = 9.6, 4.6 Hz, 3H).

### Step 2

### (2S)-2-(3-ethoxy-3-oxypropyl)morpholin-4-tert-butyl carboxylate 244c

Compound **244b** (13 g, 45.56 mmol) was dissolved in methanol (40 mL), followed by the addition of 10% Pd/C(5.53 g, 45.56 mmol) under a nitrogen atmosphere. The reaction atmosphere was substituted with a nitrogen atmosphere (20 psi), and the mixture was stirred at 25°C for 16.0 hours. The reaction mixture was filtered, and the filtrate was concentrated under a reduced pressure, to obtain the crude product of title Compound **244c** (13 g, 45.24 mmol, yield: 99.3%).
MS m/z (ESI): 310.0 [M+23]

### Step 3

### 3-[(2S)-4-[(tert-butoxy)carbonyl]morpholin-2-yl]propionic acid 244d

Under the protection of nitrogen, Compound **244c** (13 g, 45.24 mmol) was dissolved in tetrahydrofuran (120 mL) and water (30 mL), followed by the addition of lithium hydroxide (3.25 g, 135.72 mmol), and the mixture was stirred at room temperature for 4.0 hours. The reaction mixture was regulated to PH < 4 by adding a saturated aqueous citric acid solution, and extracted with ethyl acetate (300 mL×3). The organic phase was dried over anhydrous sodium sulfate, filtered and concentrated under a reduced pressure, to obtain the title Compound **244d** (11 g, 42.42 mmol, yield: 93.77%).

¹H NMR (400 MHz, CDCl₃) δ 3.87-3.70 (m, 3H), 3.51-3.27 (m, 2H), 2.97-2.82 (m, 1H), 2.54-2.28 (m, 3H), 1.87-1.71 (m, 2H), 1.44 (s, 9H).

### Step 4

### (2S)-2-{2-[methoxy(methyl)aminoformyl]ethyl}morpholin-4-tert-butyl carboxylate 244e

Compound **244d** (11 g, 42.42 mmol) was dissolved in DMF (130 mL), followed by the addition of HATU (24.20 g, 63.63 mmol) and *N*-methoxymethylamine (6.21 g, 63.63 mmol, hydrochloride). The reaction mixture was stirred at room temperature for 3.0 hours. Water (1000 mL) was added to the reaction mixture, which was then extracted with ethyl acetate (300 mL x 3). The organic phase was washed with saturated brine (500 mL), dried over anhydrous sodium sulfate, filtered and concentrated under a reduced pressure. The resulting residues were purified by the silica-gel column chromatography using petroleum ether/ethyl acetate as an elution system, to obtain the title Compound **244e** (5.0 g, 16.54 mmol, yield: 38.98%).

¹H NMR (400 MHz, CDCl₃) δ 3.90-3.79 (m, 3H), 3.69(s, 3H), 3.52-3.45(m, 1H), 3.42-3.34(m, 1H), 3.18(s, 3H), 2.98-2.86(m, 1H), 2.65-2.51(m, 3H), 1.84-1.76(m, 2H), 1.46(s, 9H).

### Step 5

### (2S)-2-(4-methyl-3-oxopentyl)morpholin-4-tert-butyl carboxylate 244f

Under the protection of nitrogen, Compound **244e** (550 mg, 1.82 mmol) was dissolved in tetrahydrofuran (10 mL), and cooled to -78°C. An isopropyllithium solution (1.6 M, 3.41mL) was added dropwise to the reaction mixture, after which the reaction mixture was stirred at -78°C for 4.0 hours. Water (1.0 mL) and a saturated aqueous ammonium chloride solution (1.0 mL) were added to the reaction mixture, followed by stirring for 0.5 hours and adding water (50 mL), and the reaction mixture was then extracted with ethyl acetate (50 mL x 3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **244f** (165 mg, 578.18 µmol, yield: 31.79%) as colorless oil.
MS m/z (ESI):186.2 [M+1]
¹H NMR (400 MHz, CDCl₃) δ 3.77 (br dd, J = 2.8, 11.1 Hz, 4H), 3.44 - 3.34 (m, 1H), 3.28 - 3.18 (m, 1H), 2.93 - 2.81 (m, 1H), 2.56 - 2.51 (m, 2H), 2.51 - 2.48 (m, 1H), 1.76 - 1.68 (m, 2H), 1.39 (s, 9H), 1.03 (d, J = 7.0 Hz, 6H).

### Step 6

### (2S)-2-{3-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-4-methylpentyl}morpholin-4-tert-butyl carboxylate 244g

Compound **145d** (121.48 mg, 284.86 µmol) and Compound **244f** (89.42 mg, 313.34 µmol) were dissolved in methanol (2.0 mL), followed by the addition of ZnCl₂ (77.65 mg, 569.72 µmol), and the mixture was stirred at 25°C for 1.0 hour. The mixture was cooled to room temperature, followed by the addition of sodium cyanoborohydride (53.70 mg, 854.57 µmol), allowing for reacting at 25°C for 1.0 hours under the protection of nitrogen. A saturated aqueous sodium bicarbonate solution (20 mL) was added to the reaction mixture, which was then extracted with dichloromethane (10 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **244g** (198 mg, 284.55 µmol, yield: 99.89%).
MS m/z (ESI):696.2 [M+1]

### Step 7

### (3R)-4-{5-fluoro-2-[1-fluoro-3-methyl-6-(1-{4-methyl-1-[(2S)-morpholin-2-yl]pentan-3-yl}azacyclobutan-3-yl)imidazo[1,5-a]pyridin-8yl]benzoyl}-3-methylmorpholine 244

Compound **244g** (198.00 mg, 284.55 µmol) was dissolved in dichloromethane (5.0 mL), followed by the addition of trifluoroacetic acid (1.48 g, 12.98 mmol, 1 mL) at 25°C. The mixture was stirred at room temperature to allow for reacting for 1.0 hours. The reaction mixture was concentrated under a reduced pressure, followed by the addition of dichloromethane (5.0 mL) and water (5.0 mL), and the reaction mixture was then regulated to PH >7 with a 10% aqueous NaOH solution, and extracted with dichloromethane (10 mL x 3). The organic phases were combined, dried over anhydrous sodium sulfate, and concentrated under a reduced pressure. The resulting residues were purified by the preparative thin-layer chromatography using dichloromethane/methanol as an elution system, to obtain the title Compound **244** (57.3 mg, 96.19 µmol, yield: 33.8%).
MS m/z (ESI):596.1 [M+1]
¹H NMR (400 MHz, CD₃OD) δ 7.73 (d, J = 10.0 Hz, 1H), 7.54 - 7.41 (m, 1H), 7.27 - 7.06 (m, 2H), 6.75 - 6.49 (m, 1H), 3.85 - 3.63 (m, 4H), 3.61 - 3.40 (m, 4H), 3.29 - 3.23 (m, 2H), 2.98 - 2.75 (m, 4H), 2.58 - 2.51 (m, 1H), 2.50 (s, 3H), 2.25 (s, 1H), 2.08 - 1.92 (m, 1H), 1.82 - 1.71 (m, 1H), 1.60 - 1.34 (m, 4H), 1.30 - 1.14 (m, 3H), 1.06 (d, J = 5.6 Hz, 1H), 0.87 - 0.78 (m, 6H), 0.74 - 0.64 (m, 1H).

### Examples 244-1 and 244-2

### (3R)-4-[5-fluoro-2-(1-fluoro-3-methyl-6-{1-[(3R)-4-methyl-1-[(2S)-morpholin-2-yl]pentan-3-yl]azacyclobutan-3-yl}imidazo[1,5-a]pyridin-8yl)benzoyl]-3-methylmorpholine 244-1

### (3R)-4-[5-fluoro-2-(1-fluoro-3-methyl-6-{1-[(3S)-4-methyl-1-[(2S)-morpholin-2-yl]pentan-3-yl]azacyclobutan-3-yl}imidazo[1,5-a]pyridin-8yl)benzoyl]-3-methylmorpholine 244-2

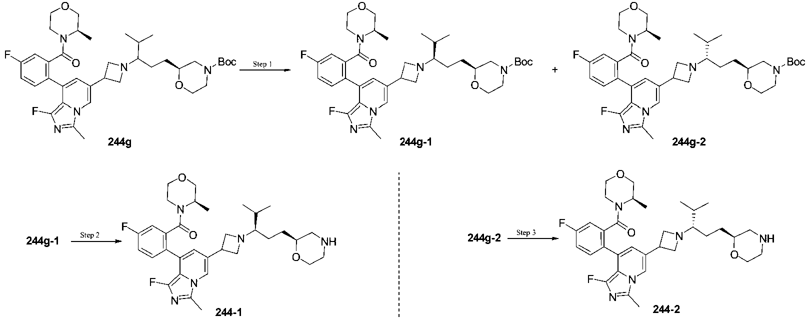

### Step 1

### (2S)-2-[(3R)-3-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-4-methylpentyl]morpholin-4-tert-butyl carboxylate 244g-1

### (2S)-2-[(3S)-3-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-4-methylpentyl]morpholin-4-tert-butyl carboxylate 244g-2

Compound **244g** (100 mg, 143.71 µmol) was separated and purified by the chiral preparative HPLC (preparative column: DAICEL CHIRALPAK IG (250 mm * 30 mm,10 um), mobile phase: n-hexane-isopropanol/acetonitrile (0.1%isopropylamine); elution gradient: 15%), to obtain the title Compounds **244g-1** (25 mg, 35.93 µmol, yield: 25.0%) and **244g-2** (43 mg, 61.80 µmol, yield: 43%).

### Single-configuration Compound 244g-1:

MS m/z (ESI):696.2 [M+1]
Chiral HPLC analysis: retention time: 3.365 min; chromatographic column: Chiralpak IG-3 50 x 4.6 mm I.D., 3 um; mobile phase: n-hexane (0.05% isopropylamine)-ethanol/acetonitrile (0.05% isopropylamine): elution gradient: 10%.

### Single-configuration Compound 244g-2:

MS m/z (ESI):696.5 [M+1]
Chiral HPLC analysis: retention time: 4.023 min; chromatographic column: Chiralpak IG-3 50 x 4.6 mm I.D., 3 um; mobile phase: n-hexane (0.05% isopropylamine)-ethanol/acetonitrile (0.05% isopropylamine): elution gradient: 10%.

### Step 2

### (3R)-4-[5-fluoro-2-(1-fluoro-3-methyl-6-{1-[(3R)-4-methyl-1-[(2S)-morpholin-2-yl]pentan-3-yl]azacyclobutan-3-yl}imidazo[1,5-a]pyridin-8yl)benzoyl]-3-methylmorpholine 244-1

Compound **244g-1** (25.00 mg, 35.93 µmol) was dissolved in dichloromethane (5.0 mL), followed by the addition of trifluoroacetic acid (1.48 g, 12.98 mmol, 1 mL) at 25°C. The mixture was stirred at room temperature to allow for reacting for 1.0 hours. The reaction mixture was concentrated under a reduced pressure, followed by the addition of dichloromethane (5.0 mL) and water (5.0 mL), and the reaction mixture was then regulated to PH >7 with a 10% aqueous NaOH solution, and extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 30%-60%). The title Compound **244-1** (5.45 mg, 9.15 µmol, yield: 25.46%) was obtained as yellow solid.

### Single-configuration Compound 244-1:

MS m/z (ESI):596.4 [M+1]
Chiral SFC: retention time: 1.144 min; chromatographic column: Chiralpak AD-3 50×4.6 mm I.D., 3 um; mobile phase: CO₂/MeOH (0.05% DEA): elution gradient: 5%-40%.

¹H NMR (400 MHz, CD₃OD) δ 7.82 (d, J = 13.0 Hz, 1H), 7.67 - 7.53 (m, 1H), 7.41 - 7.18 (m, 2H), 6.87 - 6.54 (m, 1H), 4.58 - 4.05 (m, 1H), 3.87 - 3.54 (m, 7H), 3.37 (s, 2H), 3.28 - 3.20 (m, 2H), 3.19 - 3.11 (m, 1H), 3.07 - 2.91 (m, 1H), 2.83 (d, J = 12.4 Hz, 1H), 2.76 (d, J = 3.6 Hz, 2H), 2.62 (s, 3H), 2.53 - 2.39 (m, 1H), 2.23 (s, 1H), 1.93 - 1.79 (m, 1H), 1.67 - 1.55 (m, 1H), 1.53 - 1.41 (m, 2H), 1.37 - 1.25 (m, 2H), 1.19 (d, J = 4.2 Hz, 1H), 1.06 - 0.31 (m, 8H).

### Step 3

### (3R)-4-[5-fluoro-2-(1-fluoro-3-methyl-6-{1-[(3S)-4-methyl-1-[(2S)-morpholin-2-yl]pentan-3-yl]azacyclobutan-3-yl}imidazo[1,5-a]pyridin-8yl)benzoyl]-3-methylmorpholine 244-2

Compound **244g-2** (30.00 mg, 43.11 µmol) was dissolved in dichloromethane (3.0 mL), followed by the addition of trifluoroacetic acid (1 g, 8.77 mmol, 675.68 µL) at 25°C. The mixture was stirred at room temperature to allow for reacting for 1.0 hours. The reaction mixture was concentrated under a reduced pressure, followed by the addition of dichloromethane (5.0 mL) and water (5.0 mL), and the reaction mixture was then regulated to PH >7 with a 10% aqueous NaOH solution, and extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonium bicarbonate), elution gradient: 27%-57%). The title Compound **244-2** (8.54 mg, 14.34 µmol, yield: 33.25%) was obtained as yellow solid.

### Single-configuration Compound 244-2:

MS m/z (ESI):596.4 [M+1]
Chiral SFC: retention time: 1.224min; chromatographic column: Chiralpak AD-3 50×4.6 mm I.D., 3 um; mobile phase: CO₂/MeOH (0.05% DEA): elution gradient: 5%-40%.

¹H NMR (400 MHz, CD₃OD) δ 7.89 - 7.77 (m, 1H), 7.70 - 7.52 (m, 1H), 7.44 - 7.18 (m, 2H), 6.89 - 6.61 (m, 1H), 4.64 - 4.08 (m, 2H), 3.87 - 3.56 (m, 7H), 3.50 - 3.37 (m, 2H), 3.25 (d, J = 6.4 Hz, 2H), 3.20 - 3.09 (m, 1H), 3.07 - 2.93 (m, 1H), 2.92 - 2.83 (m, 1H), 2.79 (d, J = 5.6 Hz, 2H), 2.62 (s, 3H), 2.48 (t, J = 11.4 Hz, 1H), 2.24 (s, 1H), 1.93 - 1.81 (m, 1H), 1.52 - 1.41 (m, 3H), 1.36 - 1.26 (m, 1H), 1.24 - 1.13 (m, 1H), 1.00 - 0.50 (m, 8H).

### Examples 245-1 and 245-2

### (3R)-4-[5-fluoro-2-(1-fluoro-3-methyl-6-{1-[(3R)-4-methyl-1-[(2R)-morpholin-2-yl]pentan-3-yl]azacyclobutan-3-yl}imidazo[1,5-a]pyridin-8yl)benzoyl]-3-methylmorpholine 245-1

### (3R)-4-[5-fluoro-2-(1-fluoro-3-methyl-6-{1-[(3S)-4-methyl-1-[(2R)-morpholin-2-yl]pentan-3-yl]azacyclobutan-3-yl}imidazo[1,5-a]pyridin-8yl)benzoyl]-3-methylmorpholine 245-2

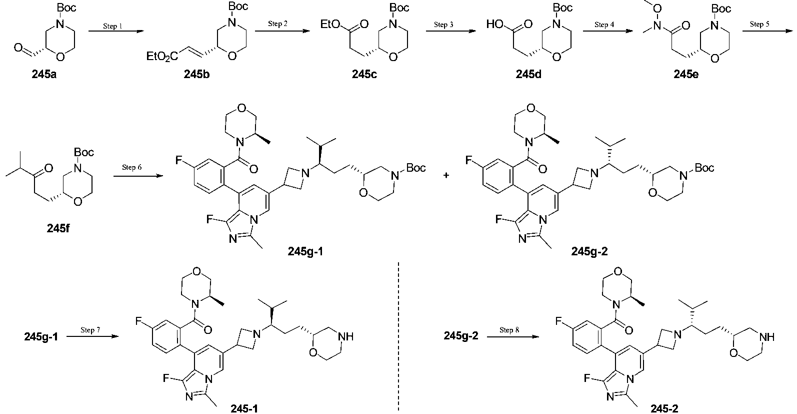

### Step 1

### (2R)-2-[(1E)-3-ethoxy-3-oxopropyl-1-ene-1-yl]morpholin-4-tert-butyl carboxylate 245b

(2*S*)-2-formylmorpholin-4-tert-butyl carboxylate **245a** (20 g, 92.92 mmol) was dissolved in toluene (20 mL), followed by the addition of ethyl(triphenylphosphoranylidene)acetate (32.37 g, 92.92 mmol). The reaction mixture was stirred at 90°C for 16 hours. Water (1000 mL) was added to the reaction mixture, which was then extracted with ethyl acetate (500 mL x 3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **245b** (15 g, 52.57 mmol, yield: 56.58%).

¹H NMR (400 MHz, CDCl₃) δ 6.82 (dd, J = 15.8, 4.0 Hz, 1H), 6.11 (d, J = 15.6 Hz, 1H), 4.21 (q, J = 7.0 Hz, 2H), 4.15 - 3.75 (m, 4H), 3.57 (s, 1H), 3.02-2.60 (m, 2H), 1.47 (s, 9H), 1.29 (dd, J = 9.6, 4.6 Hz, 3H).

### Step 2

### (2R)-2-(3-ethoxy-3-oxypropyl)morpholin-4-tert-butyl carboxylate 245c

Compound **245b** (15.7 g, 55.02 mmol) was dissolved in methanol (160 mL), followed by the addition of 10% Pd/C(6.68 g, 55.02 mmol) under a nitrogen atmosphere. The reaction atmosphere was substituted with a nitrogen atmosphere (20 psi), and the mixture was stirred at 25°C for 16.0 hours. The reaction mixture was filtered, and the filtrate was concentrated under a reduced pressure, to obtain the crude product of title Compound **245c** (13 g, 45.24 mmol, yield: 82.22%).
MS m/z (ESI): 310.1 [M+23]
¹H NMR (400 MHz, CDCl₃) δ 4.13 (q, J = 7.2 Hz, 2H), 3.98-3.72 (m, 3H), 3.50-3.43 (m, 1H), 3.39-3.31 (m, 1H), 2.97-2.84 (m, 1H), 2.69-2.54 (m, 1H), 2.52 - 2.32 (m, 2H), 1.77 (dt, J = 15.2, 7.8 Hz, 2H), 1.46 (s, 9H), 1.25 (t, J = 7.2 Hz, 3H).

### Step 3

### 3-[(2R)-4-[(tert-butoxy)carbonyl]morpholin-2-yl]propionic acid 245d

Under the protection of nitrogen, Compound **245c** (15.7 g, 54.64 mmol) was dissolved in tetrahydrofuran (160 mL) and water (40 mL), followed by the addition of lithium hydroxide (3.93 g, 163.91 mmol), and the mixture was stirred at room temperature for 3.0 hours. The reaction mixture was regulated to PH < 4 by adding a saturated aqueous citric acid solution, and extracted with ethyl acetate (300 mL×3). The organic phase was dried over anhydrous sodium sulfate, filtered and concentrated under a reduced pressure, to obtain the title Compound **245d** (13 g, 50.14 mmol, yield: 91.76%).

¹H NMR (400 MHz, CDCl₃) δ 3.87 - 3.70 (m, 3H), 3.51 - 3.27 (m, 2H), 2.97-2.82 (m, 1H), 2.54 - 2.28 (m, 3H), 1.87-1.71 (m, 2H), 1.44 (s, 9H).

### Step 4

### (2R)-2-{2-[methoxy(methyl)aminoformyl]ethyl}morpholin-4-tert-butyl carboxylate 245e

Compound **245d** (2 g, 7.71 mmol) was dissolved in DCM (20 mL), followed by the addition of HATU (4.40 g, 11.57 mmol), DIPEA(2.99 g, 23.14 mmol) and N-methoxymethylamine (1.13 g, 11.57 mmol, hydrochloride). The reaction mixture was stirred at room temperature for 3.0 hours. Water (100 mL) was added to the reaction mixture, which was then extracted with ethyl acetate (30 mL x 3). The organic phase was washed with saturated brine (50 mL), dried over anhydrous sodium sulfate, filtered and concentrated under a reduced pressure. The resulting residues were purified by the silica-gel column chromatography using petroleum ether/ethyl acetate as an elution system, to obtain the title Compound **245e** (1.0 g, 3.31 mmol, yield: 42.88%).

¹H NMR (400 MHz, CDCl₃) δ 3.90-3.79 (m, 3H), 3.69(s, 3H), 3.52-3.45(m, 1H), 3.42-3.34(m, 1H), 3.18(s, 3H), 2.98-2.86(m, 1H), 2.65-2.51(m, 3H), 1.84-1.76(m, 2H), 1.46(s, 9H).

### Step 5

### (2R)-2-(4-methyl-3-oxopentyl)morpholin-4-tert-butyl carboxylate 245f

Under the protection of nitrogen, Compound **245e** (200.00 mg, 661.45 µmol) was dissolved in tetrahydrofuran (10 mL), and cooled to -65°C. An isopropyllithium solution (1.6 M, 1.24 mL) was added dropwise to the reaction mixture, after which the reaction mixture was stirred at -65°C for 4.0 hours. Water (1.0 mL) and a saturated aqueous ammonium chloride solution (1.0 mL) were added to the reaction mixture, followed by stirring for 0.5 hours and adding water (50 mL), and the reaction mixture was then extracted with ethyl acetate (50 mL x 3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **245f** (130 mg, 455.54 µmol, yield: 68.87%) as colorless oil.

¹H NMR (400 MHz, CDCl₃) δ 3.95 - 3.74 (m, 3H), 3.47 - 3.37 (m, 1H), 3.27 (t, J = 9.6 Hz, 1H), 2.96 - 2.78 (m, 1H), 2.63 - 2.45 (m, 4H), 1.81 - 1.69 (m, 1H), 1.67 - 1.54 (m, 1H), 1.43 (s, 9H), 1.06 (d, J = 7.2 Hz, 6H).

### Step 6

### (2R)-2-[(3R)-3-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-4-methylpentyl]morpholin-4-tert-butyl carboxylate 245g-1

### (2R)-2-[(3S)-3-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-4-methylpentyl]morpholin-4-tert-butyl carboxylate 245g-2

Compound **145d** (178 mg, 417.39 µmol) and Compound **245f**(131.03 mg, 459.13 µmol) were dissolved in methanol (10 mL), followed by the addition of ZnCl₂ (170.67 mg, 1.25 mmol), and the mixture was stirred at 25°C for 1.0 hour. The mixture was cooled to room temperature, followed by the addition of sodium cyanoborohydride (78.69 mg, 1.25 mmol), allowing for reacting at 25°C for 12 hours under the protection of nitrogen. A saturated aqueous ammonium chloride solution (20 mL) was added to the reaction mixture, followed by stirring for 0.5 hours and adding water (50 mL), and the reaction mixture was then extracted with ethyl acetate (50 mL x 3). The organic phases were combined, dried over anhydrous sodium sulfate, and concentrated under a reduced pressure. The resulting residues were purified by the silica-gel column chromatography using petroleum ether/ethyl acetate as an elution system, and the resulting solid was separated and purified by the chiral preparative SFC (preparative column: DAICEL CHIRALCEL OD (250 mm * 30 mm,10 um), mobile phase: CO₂-EtOH (0.1% diethylamide); elution gradient: 25%), to obtain the title Compounds **245g-1** (55 mg, 79.04 µmol, yield: 18.94%) and **245g-2** (52 mg, 74.73 µmol, yield: 17.90%).

### Single-configuration Compound 245g-1:

MS m/z (ESI):696.3 [M+1]
Chiral HPLC analysis: retention time: 1.364 min; chromatographic column: Chiralcel OD-3 50 x 4.6 mm I.D., 3 um; mobile phase: CO₂-EtOH (0.05% DEA): elution gradient: 5%-40%.

### Single-configuration Compound 245g-2:

MS m/z (ESI):696.4 [M+1]
Chiral HPLC analysis: retention time: 1.210 min; chromatographic column: Chiralcel OD-3 50 x 4.6 mm I.D., 3 um; mobile phase: CO₂-EtOH (0.05% DEA): elution gradient: 5%-40%.

### Step 7

### (3R)-4-[5-fluoro-2-(1-fluoro-3-methyl-6-{1-[(3R)-4-methyl-1-[(2R)-morpholin-2-yl]pentan-3-yl]azacyclobutan-3-yl}imidazo[1,5-a]pyridin-8yl)benzoyl]-3-methylmorpholine 245-1

Compound **245g-1** (52.00 mg, 74.73 µmol) was dissolved in dichloromethane (3.0 mL), followed by the addition of trifluoroacetic acid (1 g, 8.77 mmol, 675.68 µL) at 25°C. The mixture was stirred at room temperature to allow for reacting for 1.0 hours. Dichloromethane (10 mL) and a 10% aqueous NaOH solution (20 mL) were added, and the mixture was then extracted with dichloromethane (10 mL x 2). The organic phases were combined, dried over anhydrous sodium sulfate, and concentrated under a reduced pressure. The resulting residues were purified by the HPLC (mobile phase: acetonitrile, water (0.1% ammonia water), elution gradient: 33%-53%). The title Compound 245-1 (25.87 mg, 43.43 µmol, yield: 58.11%) was obtained as yellow oil.

### Single-configuration Compound 245-1:

MS m/z (ESI):596.4 [M+1]
Chiral SFC: retention time: 1.283 min; chromatographic column: Chiralcel OD-3 50×4.6 mm I.D., 3um,; mobile phase: CO₂/MeOH (0.05% DEA): elution gradient: 5%-40%.

¹H NMR (400 MHz, CD₃OD) δ 7.55 - 7.35 (m, 2H), 7.25 - 6.99 (m, 2H), 6.79 - 6.54 (m, 1H), 4.66 - 4.11 (m,1H), 3.90 - 2.72 (m, 18H), 2.61 (s, 3H), 2.57 - 2.43 (m, 1H), 2.09 - 2.03 (m, 1H), 1.79 - 1.75 (m, 1H), 1.51 - 1.40 (m, 3H),1.34 - 1.14 (m, 3H), 0.94 - 0.86 (m, 6H), 0.84 - 0.60 (m, 1H).

### Step 8

### (3R)-4-[5-fluoro-2-(1-fluoro-3-methyl-6-{1-[(3S)-4-methyl-1-[(2R)-morpholin-2-yl]pentan-3-yl]azacyclobutan-3-yl}imidazo[1,5-a]pyridin-8yl)benzoyl]-3-methylmorpholine 245-2

Compound **245g-2** (52 mg, 74.73 µmol) was dissolved in dichloromethane (3.0 mL), followed by the addition of trifluoroacetic acid (1 g, 8.77 mmol, 675.68 µL) at 25°C. The mixture was stirred at room temperature to allow for reacting for 1.0 hours. The reaction mixture was concentrated under a reduced pressure, followed by the addition of dichloromethane (10 mL) and a 10% aqueous NaOH solution (20 mL), and the mixture was then extracted with dichloromethane (10 mL x 2). The organic phases were combined, dried over anhydrous sodium sulfate, and concentrated under a reduced pressure. The resulting residues were purified by the HPLC (mobile phase: acetonitrile, water (0.1% ammonia water), elution gradient: 33%-53%). The title Compound **245-2** (24.67 mg, 41.41 µmol, yield: 55.42%) was obtained as yellow oil.

### Single-configuration Compound 245-2:

MS m/z (ESI):596.4 [M+1]
Chiral SFC: retention time: 1.138 min; chromatographic column: Chiralpak AD-3 50×4.6 mm I.D., 3 um; mobile phase: CO₂/MeOH (0.05% DEA): elution gradient: 5%-40%.

¹H NMR (400 MHz, CDCl₃) δ 7.56 - 7.31 (m, 2H), 7.24 - 7.05 (m, 2H), 6.82 - 6.50 (m, 1H), 4.92 - 4.63 (m, 1H),4.59 - 4.11 (m, 1H), 3.91 - 3.18 (m, 10H), 3.16 - 3.08 (m, 2H), 3.04 - 2.93 (m, 1H), 2.91 - 2.70 (m, 4H), 2.61 (s, 3H), 2.56 -2.42 (m, 1H), 2.10 - 2.03 (m, 1H), 1.82 - 1.77 (m, 1H), 1.52 - 1.26 (m, 5H), 1.20 - 1.04 (m, 1H), 0.90 (t, J = 6.0 Hz, 6H), 0.83- 0.58 (m, 1H).

### Example 255

### (3R)-4-[5-fluoro-2-(1-fluoro-6-{1-[2-methoxy-1-(oxacyclohexan-4-yl)ethyl]azacyclobutan-3-yl}-3-methylimidazo[1,5-a]pyridin-8-yl)benzoyl]-3-methylmorpholine 255

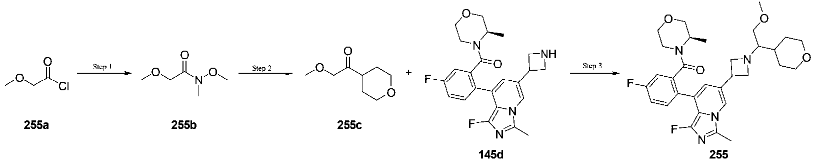

### Step 1

### N,2-dimethoxy-N-methylacetamide 255b

*N*-methoxymethylamine (4.49 g, 46.07 mmol, hydrochloride) and triethylamine (13.99 g, 138.22 mmol) were dissolved in dichloromethane (20 mL), and cooled to 5°C, followed by the slow dropwise addition of the solution of 2-methoxyacetyl chloride **255a** (5 g, 46.07 mmol) in dichloromethane (10 mL). The reaction mixture was stirred at room temperature for 3.0 hours. Water (200 mL) was added to the reaction mixture, which was then extracted with dichloromethane (100 mL×3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **255b** (2.0 g, 15.02 mmol, yield: 32.60%) as colorless oil.

¹H NMR (400 MHz, CDCl₃) δ 4.21 (s, 2H), 3.68 (s, 3H), 3.45 (s, 3H), 3.18 (s, 3H).

### Step 2

### 2-methoxy-1-(oxacyclohexan-4-yl)ethan-1-one 255c

Under the protection of nitrogen, Mg (1.24 g, 51.02 mmol) was suspended in THF (20 mL), followed by the addition of I₂ (100 mg, 394.00 µmol) and then the slow dropwise addition of the solution of 4-bromotetrahydropyran (3.5 g, 21.21 mmol) in tetrahydrofuran (10 mL), and the mixture was stirred at room temperature for 1.0 hour. At 0°C, the prepared alkyl magnesium bromide tetrahydrofuran solution was slowly dropwise added to the solution of Compound **255b** (1.98 g, 14.85 mmol) in THF(30 mL). The reaction mixture was stirred at room temperature for 12 hours. A saturated aqueous ammonium chloride solution (50 mL) was added to the reaction mixture, which was then extracted with methyltert-butyl ether (50 mL×3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **255c** (150 mg, 948.20 µmol, yield: 4.47%) as yellow oil.

¹H NMR (400 MHz, CDCl₃) δ 4.09 (s, 2H), 4.04 - 3.96 (m, 2H), 3.49 - 3.40 (m, 5H), 2.84 - 2.72 (m, 1H), 1.77 - 1.70 (m, 4H).

### Step 3

### (3R)-4-[5-fluoro-2-(1-fluoro-6-{1-[2-methoxy-1-(oxacyclohexan-4-yl)ethyl]azacyclobutan-3-yl}-3-methylimidazo[1,5-a]pyridin-8-yl)benzoyl]-3-methylmorpholine 255

Compound **145d** (66.82 mg, 156.68 µmol) and Compound **255c**(24.79 mg, 156.68 µmol) were dissolved in methanol (5.0 mL). Zinc chloride (64.07 mg, 470.05 µmol) was added, and the mixture was stirred at 50°C for 1.0 hour. The reaction mixture was cooled to room temperature, followed by adding sodium cyanoborohydride (29.54 mg, 470.05 µmol) and stirring at 50°C for 12.0 hours. A saturated aqueous ammonium chloride solution (20 mL) was added, and the reaction mixture was stirred for 0.5 hours. Water (50 mL) was added to the reaction mixture, which was then extracted with ethyl acetate (50 mL x 3). The organic phases were combined, dried over anhydrous sodium sulfate, and concentrated under a reduced pressure. The resulting residues were purified by the HPLC (mobile phase: acetonitrile, water (0.1% ammonium bicarbonate), elution gradient: 24%-54%), to obtain the title Compound 255 (29.54 mg, 51.95 µmol, yield: 33.15%) as yellow solid.
MS m/z (ESI): 569.4[M+1]
¹H NMR (400 MHz, CDCl₃) δ 7.56 - 7.32 (m, 2H), 7.25 - 7.04 (m, 2H), 6.82 - 6.54 (m, 1H), 4.64 - 4.11 (m, 1H),4.05 - 3.92 (m, 2H), 3.84 - 3.49 (m, 5H), 3.48 - 2.73 (m, 13H), 2.61 (s, 3H), 2.47 - 1.98 (m, 2H), 1.80 - 1.68 (m, 1H), 1.58 -1.40 (m, 3H), 1.35 - 1.09 (m, 2H), 0.85 - 0.56 (m, 1H).

### Examples 257-1 and 257-2

### 1-{4-[(1R)-1-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]ethyl]piperidin-1-yl}ethan-1-one 257-1

### 1-{4-[(1S)-1-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]ethyl]piperidin-1-yl}ethan-1-one 257-2

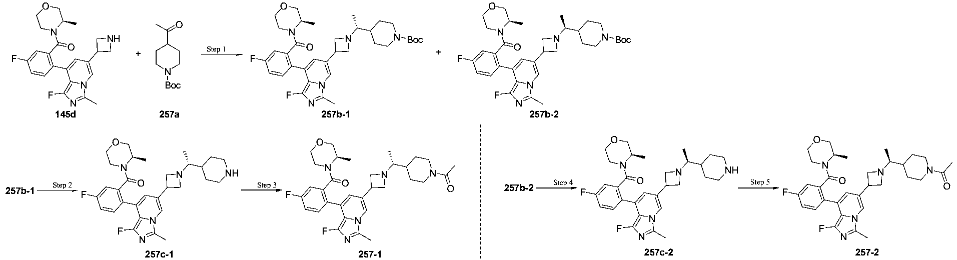

### Step 1

### 4-[(1R)-1-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]ethyl]piperidin-1-tert-butyl carboxylate 257b-1

### 4-[(1S)-1-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]ethyl]piperidin-1-tert-butyl carboxylate 257b-2

Compound **145d** (137 mg, 321.25 µmol) and 4-acetylpiperidin-1-tert-butyl formate **257a** (80.32 mg, 353.38 µmol) were dissolved in methanol (10 mL), followed by the addition of zinc chloride (131.36 mg, 963.75 µmol), and the mixture was stirred at room temperature for 1.0 hour. Sodium cyanoborohydride (60.56 mg, 963.75 µmol) was added, and the mixture was stirred at room temperature for 12 hours. A saturated aqueous ammonium chloride solution (20 mL) was added to the reaction mixture, followed by stirring for 0.5 hours and dilution with water (50 mL), and the reaction mixture was then extracted with ethyl acetate (50 mL x 3). The organic phases were combined, dried over anhydrous sodium sulfate, and concentrated under a reduced pressure. The residues were purified by the silica-gel column chromatography using petroleum ether/ethyl acetate as an elution system, and the obtained crude compound was separated and purified by the chiral preparative SFC (preparative column: DAICEL CHIRALPAK AD (250 mm * 30 mm,10 um), mobile phase: CO₂-ethanol (0.1% ammonia water); elution gradient: 25%), to obtain the title Compounds **257b-1** (95 mg, 148.96 µmol, yield: 46.37%) and **257b-2** (88 mg, 137.98 µmol, yield: 42.95%).

### Single-configuration Compound 257b-1:

MS m/z (ESI):638.2[M+1]
Chiral SFC analysis: retention time: 1.544 min; chromatographic column: Chiralpak AD-3 50 x 4.6 mm I.D., 3 um; mobile phase: CO₂-EtOH (0.05% DEA): elution gradient: 5%-40%.

### Single-configuration Compound 257b-2:

MS m/z (ESI):638.2 [M+1]
Chiral SFC analysis: retention time: 1.168 min; chromatographic column: Chiralpak AD-3 50 x 4.6 mm I.D., 3 um; mobile phase: CO₂-EtOH (0.05% DEA): elution gradient: 5%-40%.

### Step 2

### (3R)-4-[5-fluoro-2-(1-fluoro-3-methyl-6-{1-[(1R)-1-(piperidin-4-yl)ethyl]azacyclobutan-3-yl}imidazo[1,5-a]pyridin-8-yl)benzoyl]-3-methylmorpholine 257c-1

Compound **257b-1** (95 mg, 148.96 µmol) was dissolved in dichloromethane (3.0 mL), followed by the addition of trifluoroacetic acid (1 g, 8.77 mmol, 675.68 µL) at 25°C. The mixture was stirred at room temperature to allow for reacting for 1.0 hours. The reaction mixture was concentrated under a reduced pressure, and then dissolved by adding DCM (10 mL), followed by adding a 10% aqueous sodium hydroxide solution (20 mL), stirring for 0.5 hours and extracting with dichloromethane (10 mL x 3). The organic phases were combined, washed once with saturated brine (50 mL), then dried over anhydrous sodium sulfate, and filtered, and the filtrate was concentrated under a reduced pressure, to obtain the crude product of title Compound **257c-1** (80 mg, 148.80 µmol, yield: 99.89%). The crude product was directly used in the next step of reaction without purification.
MS m/z (ESI): 538.2 [M+1]

### Step 3

### 1-{4-[(1R)-1-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]ethyl]piperidin-1-yl}ethan-1-one 257-1

Triethylamine (45.17 mg, 446.39 µmol) and Compound **257c-1** (80 mg, 148.80 µmol) were dissolved in dichloromethane (5.0 mL), and cooled to 5°C, followed by the addition of acetyl chloride (12.85 mg, 163.68 µmol). The reaction mixture was stirred at room temperature for 2 hours. Water (30 mL) was added to the reaction mixture, which was then extracted with dichloromethane (50 mL×3). The organic phase was dried over anhydrous sodium sulfate, filtered and concentrated under a reduced pressure. The resulting residues were purified by the HPLC (mobile phase: acetonitrile, water (0.1% ammonia water), elution gradient: 22%-52%), to obtain the title Compound **257-1** (56.37 mg, 97.24 µmol, yield: 65.35%) as yellow solid.
MS m/z (ESI): 580.2 [M+1]
Chiral SFC analysis: retention time: 1.316 min; chromatographic column: Chiralcel OD-3 50×4.6 mm I.D., 3 um; mobile phase: CO₂-MeOH(0.05%DEA): elution gradient: 5%-40%.

¹H NMR (400 MHz, CDCl₃) δ 7.56 - 7.33 (m, 2H), 7.25 - 7.04 (m, 2H), 6.88 - 6.61 (m, 1H), 4.76 - 4.08 (m,2H), 3.91 - 3.51 (m, 6H), 3.42 - 2.73 (m, 7H), 2.61 (s, 3H), 2.52 - 2.38 (m, 1H), 2.35 - 2.16 (m, 1H), 2.09 (s, 3H), 1.68 - 1.50 (m, 3H), 1.34 - 1.10 (m, 4H), 0.87 - 0.56 (m, 4H).

### Step 4

### (3R)-4-[5-fluoro-2-(1-fluoro-3-methyl-6-{1-[(1S)-1-(piperidin-4-yl)ethyl]azacyclobutan-3-yl}imidazo[1,5-a]pyridin-8-yl)benzoyl]-3-methylmorpholine 257c-2

Compound **257b-2** (88 mg, 137.98 µmol) was dissolved in dichloromethane (3.0 mL), followed by the addition of trifluoroacetic acid (1 g, 8.77 mmol, 675.68 µL) at 25°C. The mixture was stirred at room temperature to allow for reacting for 1.0 hours. The reaction mixture was concentrated under a reduced pressure, and then dissolved by adding DCM (10 mL), followed by adding a 10% aqueous sodium hydroxide solution (20 mL), stirring for 0.5 hours and extracting with dichloromethane (10 mL x 3). The organic phases were combined, washed once with saturated brine (50 mL), then dried over anhydrous sodium sulfate, and filtered, and the filtrate was concentrated under a reduced pressure, to obtain the crude product of title Compound **257c-2** (74 mg, 137.64 µmol, yield: 99.75%). The crude product was directly used in the next step of reaction without purification.
MS m/z (ESI): 538.2 [M+1]

### Step 5

### 1-{4-[(1S)-1-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]ethyl]piperidin-1-yl}ethan-1-one 257-2

Triethylamine (41.78 mg, 412.91 µmol) and Compound **257c-2** (74 mg, 137.64 µmol) were dissolved in dichloromethane (5.0 mL), and cooled to 5°C, followed by the addition of acetyl chloride (11.88 mg, 151.40 µmol). The reaction mixture was stirred at room temperature for 2 hours. Water (30 mL) was added to the reaction mixture, which was then extracted with dichloromethane (50 mL×3). The organic phase was dried over anhydrous sodium sulfate, filtered and concentrated under a reduced pressure. The resulting residues were purified by the HPLC (mobile phase: acetonitrile, water (0.1% ammonia water), elution gradient: 22%-52%), to obtain the title Compound **257-2** (55.19 mg, 95.21 µmol, yield: 69.17 %) as yellow solid.
MS m/z (ESI): 580.3 [M+1]
Chiral SFC analysis: retention time: 1.304 min; chromatographic column: Chiralcel OD-3 50×4.6 mm I.D., 3 um; mobile phase: CO₂-MeOH(0.05%DEA): elution gradient: 5%-40%.

¹H NMR (400 MHz, CDCl₃) δ 7.57 - 7.35 (m, 2H), 7.26 - 7.07 (m, 2H), 6.88 - 6.59 (m, 1H), 4.78 - 4.10 (m,2H), 3.95 - 3.50 (m, 6H), 3.47 - 2.71 (m, 7H), 2.62 (s, 3H), 2.56 - 2.37 (m, 1H), 2.36 - 2.23 (m, 1H), 2.16 - 1.67 (m, 5H), 1.58- 1.46 (m, 1H), 1.39 - 1.11 (m, 4H), 0.89 - 0.59 (m, 4H).

### Example 260

### (3R)-4-{5-fluoro-2-[4-methyl-6-(1-[(oxacyclobutan-4-yl)methyl]azacyclobutan-3-yl}methyl)pyrrolo[1,2-a]pyrazin-8-yl]benzoyl}-3-methylmorpholine 260

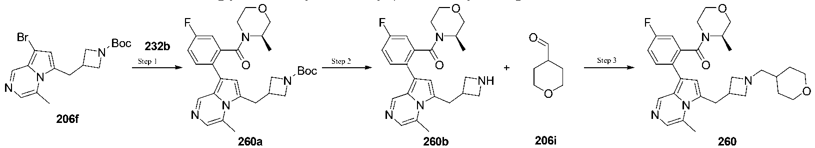

### Step 1

### 3-[(8-{4-fluoro-2-[(3R)-3-methylmorpholin-4-carbonyl]phenyl}-4-methylpyrrolo[1,2-a]pyrazin-6-yl)methyl]azacyclobutan-1-tert-butyl carboxylate 260a

Compound **206f** (200 mg, 525.93 µmol), Compound **232b**(275.49 mg, 788.9 µmol) and potassium phosphate (334.91 mg, 1.58 mmol) were dissolved in dioxane (2 mL) and water (0.5 mL). Pd(dtbpf)Cl₂ (34.28 mg, 52.59 µmol) was added. Under a nitrogen atmosphere, the mixture was stirred at 95°Cto allow for reacting for 2.0 hours. The reaction mixture was cooled to room temperature, water (10 mL) was added, and the mixture was extracted with ethyl acetate (10 mL x 3). The organic phases were combined, dried over anhydrous sodium sulfate, and concentrated under a reduced pressure. The resulting residues were purified by the preparative thin-layer chromatography using dichloromethane/methanol as an elution system, to obtain the title Compound **260a** (200 mg, 382.69 µmol, yield: 72.77%) as yellow solid.
MS m/z (ESI): 523.2 [M+1]

### Step 2

### (3R)-4-(2-{6-[(azacyclobutan-3-yl)methyl]-4-methylpyrrolo[1,2-a]pyrazin-8-yl}-5-fluorobenzoyl)-3-methylmorpholine 260b

Compound **260a** (92 mg, 176.04 µmol) was dissolved in methane dioxide (3.0 mL), followed by the addition of trifluoroacetic acid (1.48 g, 12.98 mmol, 1 mL) at 0°C. The mixture was stirred at room temperature to allow for reacting for 0.5 hours. The reaction mixture was concentrated under a reduced pressure, to obtain the crude product of title Compound **260b** (70 mg). The crude product was directly used in the next step of reaction without purification. MS m/z (ESI): 423.0 [M+1]

### Step 3

### (3R)-4-{5-fluoro-2-[4-methyl-6-({1-[(oxacyclobutan-4-yl)methyl]azacyclobutan-3-yl}methyl)pyrrolo[1,2-a]pyrazin-8-yl]benzoyl}-3-methylmorpholine 260

The crude product of Compound **260b** (70 mg) and Compound **206i** (17.87 mg, 156.57 µmol) were dissolved in methanol (2.0 mL). Triethylamine (56.17 mg, 555.06 µmol) was added, and the mixture was stirred at room temperature for 0.5 hour. Sodium cyanoborohydride (24.60 mg, 391.41 µmol) was added, and the mixture was stirred at room temperature for 12.0 hours. A saturated aqueous sodium bicarbonate solution (10 mL) was added, and the mixture was then extracted with dichloromethane (10 mL × 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 21%-51%), to obtain the title Compound **260** (30.27 mg, 58.14 µmol, yield: 44.56%) as yellow solid.
MS m/z (ESI): 521.3[M+1]
¹H NMR (400 MHz, DMSO-*d*₆) δ 8.62 - 8.43 (m, 1H), 7.62 - 7.45 (m, 1H), 7.39 - 7.19 (m, 3H), 6.62 - 6.51 (m, 1H), 4.38 - 3.90 (m, 1H), 3.84 - 3.75 (m, 2H), 3.70 - 3.36 (m, 6H), 3.27 - 3.19 (m, 3H), 3.13 - 3.02 (m, 1H), 2.85 - 2.71 (m, 7H), 2.29 - 2.21 (m, 2H), 1.77 - 1.41 (m, 4H), 1.25 - 0.99 (m, 4H), 0.64 - 0.28 (m, 1H).

### Example 266

### (3R)-4-{5-fluoro-2-[1-fluoro-3-methyl-6-(1-{[1-(pyrimidin-5-yl)piperidin-4-yl]methyl}azacyclobutan-3-yl)imidazo[1,5-a]pyridin-8yl]benzoyl}-3-methylmorpholine 266

Compound **241b** (83 mg, 158.51 µmol), 5-bromopyrimidine (25.20 mg, 158.51 µmol) and sodium tert-butoxide (45.70 mg, 475.54 µmol) were dissolved in THF (2.0 mL), followed by the addition of Pd-PEPPSI-IpentCl (15.44 mg, 15.85 µmol) under the protection of nitrogen. The reaction mixture was stirred at 60°C under a nitrogen atmosphere for 12 hours. Water (15 mL) was added to the reaction mixture, which was then extracted with ethyl acetate (15 mL x 3). The organic phase was dried over anhydrous sodium sulfate, filtered and concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% ammonium bicarbonate), elution gradient: 22%-52%), to obtain the title Compound **266** (33 mg, 54.85 µmol, yield: 34.60 %) as yellow solid.
MS m/z (ESI): 602.3[M+1]
¹H NMR (400 MHz, DMSO-*d₆*) δ 8.60 - 8.36 (m, 3H), 7.86 (s, 1H), 7.62 - 7.47 (m, 1H), 7.45 - 7.24 (m, 2H), 6.88 - 6.48 (m, 1H), 4.47 - 3.66 (m, 4H), 3.56 (s, 5H), 2.82 (s, 7H), 2.55 (s, 3H), 2.34 (d, J = 6.8 Hz, 2H), 1.90 - 1.69 (m, 2H), 1.57 - 1.39 (m, 1H), 1.31 - 1.00 (m, 4H), 0.88 - 0.48 (m, 1H).

### Example 267

### (3R)-4-{5-fluoro-2-[1-fluoro-3-methyl-6-(1-{[(1s,4s)-4-(1H-1,2,4-triazol-1-yl)cyclohexyl]methyl}azacyclobutan-3-yl)imidazo[1,5-a]pyridin-8yl]benzoyl}-3-methylmorpholine 267

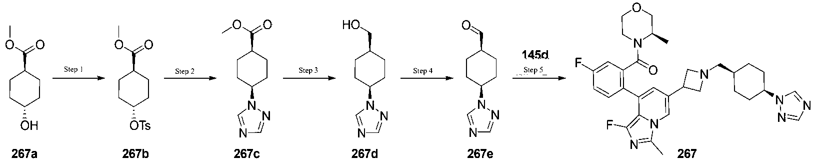

### Step 1

### (1r,4r)-4-[(4-methylphenylsulfonyl)oxy]cyclohexan-1-methyl carboxylate 267b

(1*r*,4*r*)-4-hydroxylcyclohexan-1-methyl carboxylate **267a** (5 g, 31.61 mmol) was dissolved in DCM (70 mL), followed by the addition of DMAP (386.14 mg, 3.16 mmol) and triethylamine (9.59 g, 94.82 mmol). The reaction mixture was cooled to 0°C, followed by adding 4-toluensulfonyl chloride (7.23 g, 37.93 mmol). The reaction mixture was stirred at 25°C for 12.0 hours. Water (50 mL) was added to the reaction mixture, which was then extracted with dichloromethane (50 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **267b** (8.67 g, 27.75 mmol, yield: 87.81%) as yellow solid.

¹H NMR (400 MHz, DMSO-*d₆*) δ 7.82 - 7.77 (m, 2H), 7.48 - 7.43 (m, 2H), 4.52 - 4.37 (m, 1H), 3.58 - 3.54 (m, 3H), 2.43 - 2.38 (m, 3H), 2.35 - 2.26 (m, 1H), 1.88 - 1.75 (m, 4H), 1.51 - 1.38 (m, 4H).

### Step 2

### (1s,4s)-4-(1H-1,2,4-triazol-1-yl)cyclohexan-1-methyl carboxylate 267c

1*H*-1,2,4-triazole (1 g, 14.48 mmol) was dissolved in acetonitrile (40 mL), followed by the addition of Compound **267b** (3.62 g, 11.58 mmol), cesium carbonate (9.44 g, 28.96 mmol) and TBAI(534.81 mg, 1.45 mmol). The reaction mixture was heated to 85°C and stirred for 6.0 hours. The reaction mixture was filtered under a reduced pressure, and washed with ethyl acetate (100 mL), and the filtrate was concentrated under a reduced pressure. The resulting residues were purified by the silica-gel column chromatography using petroleum ether/ethyl acetate as an elution system, to obtain the title Compound **267c** (1.08 g, 5.16 mmol, yield35.65%) as yellow oil.

¹H NMR (400 MHz, DMSO-*d₆*) δ 8.54 (s, 1H), 8.03 - 7.77 (m, 1H), 4.43 - 4.24 (m, 1H), 3.73 - 3.50 (m, 3H), 2.73 - 2.63 (m, 1H), 2.03 - 1.86 (m, 6H), 1.75 - 1.62 (m, 2H).

### Step 3

### [(1s,4s)-4-(1H-1,2,4-triazol-1-yl)cyclohexyl]methanol 267d

Compound **267c** (500 mg, 2.39 mmol) was dissolved in DCM(5 mL). Under a nitrogen atmosphere, the mixture was cooled to 0°C, followed by the slow dropwise addition of DIBAL-H (1 M, 4.78 mL), and the mixture was warmed to room temperature and stirred to allow for reacting for 5.0 hours. DIBAL-H (1 M, 2.0 mL) was supplemented, and the mixture was stirred at room temperature to allow for reacting for 12 hours . The reaction mixture was quenched by adding sodium sulfate decahydrate and filtered, and the filtrate was concentrated under a reduced pressure, to obtain the crude product of title Compound **267d** (400 mg, 2.21 mmol, yield: 92.36%) as white solid. The crude product was directly used in the next step of reaction without purification.

¹H NMR (400 MHz, DMSO-*d₆*) δ 8.57 (s, 1H), 7.94 (s, 1H), 7.33 - 6.72 (m, 1H), 4.49 - 4.29 (m, 2H), 2.11 - 1.96 (m, 2H), 1.85 - 1.75 (m, 2H), 1.67 - 1.36 (m, 6H).

### Step 4

### (1s,4s)-4-(1H-1,2 4-triazol-1-yl)cyclohexan-1-formaldehyde 267e

Compound **267d** (200 mg, 1.10 mmol) was dissolved in DMSO (4 mL). Under a nitrogen atmosphere, the mixture was cooled to 0°C, and a Burgess reagent (341.87 mg, 1.43 mmol) was added slowly. The reaction mixture was stirred at room temperature to allow for reacting for 2 hour. In an ice water bath, water (15 mL) was added to the reaction mixture, which was then extracted with ethyl acetate (15 mL x 3). The organic phases were combined, dried over anhydrous sodium sulfate, and concentrated under a reduced pressure, to obtain the crude product of title Compound **267e** (100 mg, 557.98 µmol, yield: 50.56%) as white solid. The crude product was directly used in the next step of reaction without purification.

### Step 5

### (3R)-4-{5-fluoro-2-[1-fluoro-3-methyl-6-(1-{[(1s,4s)-4-(1H-1,2,4-triazol-1-yl)cyclohexyl]methyl}azacyclobutan-3-yl)imidazo[1,5-a]pyridin-8yl]benzoyl}-3-methylmorpholine 267

Compound **145d** (80 mg, 148.02 µmol, trifluoroacetate) and Compound **267e** (100 mg, 557.98 µmol) were dissolved in methanol (2.0 mL). Triethylamine (56.17 mg, 555.06 µmol) was added, and the mixture was stirred at room temperature for 0.5 hour. Sodium cyanoborohydride (27.90 mg, 444.05 µmol) was added, and the mixture was stirred at room temperature for 12.0 hours. A saturated aqueous sodium bicarbonate solution (10 mL) was added, and the mixture was then extracted with dichloromethane (20 mL × 3). The organic phases were combined, dried over anhydrous sodium sulfate, and concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% ammonium bicarbonate), elution gradient: 23%-53%), to obtain the title Compound **267** (10.72 mg, 18.18 µmol, yield: 12.28%) as yellow solid.
MS m/z (ESI): 590.3[M+1]
¹H NMR (400 MHz, DMSO-*d₆*) δ 8.58 - 8.51 (m, 1H), 8.01 - 7.78 (m, 2H), 7.63 - 7.50 (m, 1H), 7.45 - 7.25 (m, 2H), 6.78 - 6.51 (m, 1H), 4.41 - 4.11 (m, 2H), 4.00 - 3.44 (m, 6H), 3.19 - 2.89 (m, 4H), 2.56 - 2.53 (m, 3H), 2.46 - 2.28 (m, 3H), 2.07 - 1.95 (m, 2H), 1.86 - 1.76 (m, 2H), 1.65 - 1.42 (m, 5H), 1.26 - 1.03 (m, 2H), 0.83 - 0.59 (m, 1H).

### Example 275

### (3R)-4-[5-fluoro-2-(1-fluoro-3-methyl-6-{1-[(3R)-2-methyl-6-(4-methylpiperazin-1-yl)hexan-3-yl]azacyclobutan-3-yl}imidazo[1,5-a]pyridin-8-yl)benzoyl]-3-methylmorpholine 275

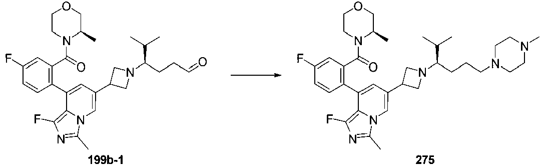

Compound **199b-1** (230 mg, 427.01 µmol) and 1-methylpiperazine (85.54 mg, 854.02 µmol) were dissolved in methanol (2.0 mL), followed by the addition of acetic acid (25.64 mg, 427.01 µmol), and the mixture was stirred at room temperature for 1.0 hour under the protection of nitrogen. Sodium cyanoborohydride (80.50 mg, 1.28 mmol) was added, allowing for reacting at room temperature for 11.0 hours under the protection of nitrogen. A saturated aqueous sodium bicarbonate solution (10 mL) was added to the reaction mixture, which was then extracted with dichloromethane (20 mL x 3). The organic phases were combined, dried over anhydrous sodium sulfate, and concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% ammonia water), elution gradient: 30%-60%), to obtain the title product **275** (95 mg, 152.54 µmol, yield: 35.72%) as yellow solid.
MS m/z (ESI):623.5 [M+1]
¹H NMR (400 MHz, DMSO-*d₆*) δ 7.99 - 7.73 (m, 1H), 7.63 - 7.18 (m, 3H), 6.87 - 6.39 (m, 1H), 4.41 - 3.42 (m, 6H), 3.28 - 2.67 (m, 5H), 2.54 (s, 3H), 2.44 - 1.94 (m, 14H), 1.85 - 0.38 (m, 15H).

### Examples 276-1 and 276-2

### (3R)-4-{5-fluoro-2-[1-fluoro-3-methyl-6-(1-{[(1s,4s)-4-(1-methyl-1H-pyrazol-4-yl)cyclohexyl]methyl}azacyclobutan-3-yl)imidazo[1,5-a]pyridin-8yl]benzoyl}-3-methylmorpholine 276-1

### (3R)-4-{5-fluoro-2-[1-fluoro-3-methyl-6-(1-{[(1r,4r)-4-(1-methyl-1H-pyrazol-4-yl)cyclohexyl]methyl}azacyclobutan-3-yl)imidazo[1,5-a]pyridin-8yl]benzoyl}-3-methylmorpholine 276-2

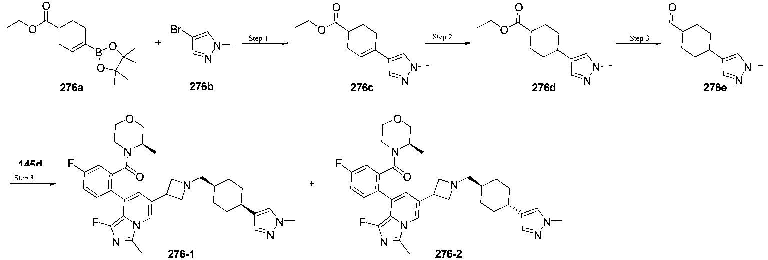

### Step 1

### 4-(1-methyl-1H-pyrazol-4-yl)cyclohex-3-ene-1-ethyl carboxylate 276c

4-(Tetramethyl-1,3,2-dioxaboran-2-yl)cyclohex-3-ene-1-ethyl carboxylate **276a** (2 g, 7.14 mmol) and 4-bromo-1-methyl-1*H*-pyrazole (1.15 g, 7.14 mmol) were dissolved in dioxane (20 mL) and water (4.0 mL). Sodium carbonate (2.27 g, 21.42 mmol) and Pd(dppf)Cl₂ (522.34 mg, 713.86 µmol) were added, and the mixture was stirred at 95°C under a nitrogen atmosphere to allow for reacting for 12 hours. The reaction mixture was cooled to room temperature, followed by adding water (50 mL) and extracting with ethyl acetate (50 mL x 3). The organic phases were combined, dried over anhydrous sodium sulfate, and concentrated under a reduced pressure. The resulting residues were purified by the silica-gel column chromatography using dichloromethane/ethyl acetate as an elution system, to obtain the title Compound **176c** (750 mg, 3.20 mmol, yield: 44.84%) as yellow oil.
MS m/z (ESI): 235.2 [M+1]
¹H NMR (400 MHz, CDCl₃) δ 7.51 (s, 1H), 7.29 (s, 1H), 5.98 - 5.90 (m, 1H), 4.20 - 4.10 (m, 2H), 3.86 (s, 3H), 2.63 - 2.50 (m, 1H), 2.43 - 2.33 (m, 4H), 2.15 - 2.07 (m, 1H), 1.86 - 1.74 (m, 1H), 1.29 - 1.24 (m, 3H).

### Step 2

### 4-(1-methyl-1H-pyrazol-4-yl)cyclohexan-1-ethyl carboxylate 276d

Compound **276c** (500 mg, 2.13 mmol) was dissolved in methanol (10 mL), followed by the addition of Pd/C (51.84 mg, 213.41 µmol). Under a hydrogen atmosphere (15 Psi), the mixture was stirred at room temperature for 12 hours. The reaction mixture was filtered, and the filtrate was concentrated under a reduced pressure, to obtain the crude product of title Compound **276d** (500 mg, 2.12 mmol, yield: 99.15%).
MS m/z (ESI): 237.2 [M+1]

### Step 3

### 4-(1-methyl-1H-pyrazol-4-yl)cyclohexan-1-formaldehyde 276e

Compound **276d** (500 mg, 2.12 mmol) was dissolved in DCM(20 mL). Under a nitrogen atmosphere, the mixture was cooled to -75°C, followed by the slow dropwise addition of DIBAL-H (1 M, 6.35 mL), and the mixture was stirred at -75°C to allow for reacting for 1.0 hours. Methanol (3.0 mL) was added dropwise to the reaction mixture, which was further stirred for 1.0 hour, then warmed to room temperature and stirred for 1.0 hour. Sodium sulfate decahydrate (2 g) was added to the reaction mixture, which was then stirred for 0.5 hours and filtered, and the filtrate was concentrated under a reduced pressure to obtain the crude product of title Compound **276e** (300 mg, 1.56 mmol, yield: 73.75%). The crude product was directly used in the next step of reaction without purification.
MS m/z (ESI): 192.9 [M+1]
¹H NMR (400 MHz, CDCl₃) δ 9.65 (s, 1H), 7.23 (s, 1H), 7.05 (s, 1H), 3.78 (s, 3H), 2.58 - 2.49 (m, 1H), 2.41 - 2.36 (m, 1H), 2.11 - 2.06 (m, 2H), 1.82 - 1.76 (m, 2H), 1.69 - 1.64 (m, 2H), 1.44 - 1.38 (m, 2H).

### Step 4

### (3R)-4-{5-fluoro-2-[1-fluoro-3-methyl-6-(1-{[(1s,4s)-4-(1-methyl-1H-pyrazol-4-yl)cyclohexyl]methyl}azacyclobutan-3-yl)imidazo[1,5-a]pyridin-8yl]benzoyl}-3-methylmorpholine 276-1

### (3R)-4-{5-fluoro-2-[1-fluoro-3-methyl-6-(1-{[(1r,4r)-4-(1-methyl-1H-pyrazol-4-yl)cyclohexyl]methyl}azacyclobutan-3-yl)imidazo[1,5-a]pyridin-8yl]benzoyl}-3-methylmorpholine 276-2

Compound **145d** (160 mg, 375.18 µmol) and Compound **276e**(108.20 mg, 562.78 µmol) were dissolved in methanol (5.0 mL), and stirred at room temperature for 1.0 hour. Sodium cyanoborohydride (70.73 mg, 1.13 mmol) was added, and the reaction mixture was stirred at room temperature for 11.0 hour. A saturated aqueous sodium bicarbonate solution (30 mL) was added, and the mixture was then extracted with dichloromethane (50 mL × 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonium bicarbonate), elution gradient: 36%-66%), and the obtained mixture was purified by the SFC(separation column: DAICEL CHIRALPAK AD (250 mm * 30 mm, 10 um); mobile phase: CO₂-EtOH (0.1% ammonia water), elution gradient: 45%), to obtain the title Compounds **276-1** (9.63 mg, 15.98 µmol, yield: 4.26%) and **276-2** (15.2 mg, 25.22 µmol, yield: 6.72%).

### Compound 276-1:

MS m/z (ESI): 603.5[M+1]
SFC: retention time: 1.726 min; Chiralpak AD-3 50 x 4.6 mm I.D., 3 um; mobile phase: CO₂-EtOH (0.05% DEA); elution gradient: 5%-40%.

¹H NMR (400 MHz, CD₃OD) δ 7.87 - 7.74 (m, 1H), 7.65 - 7.50 (m, 1H), 7.41 (s, 1H), 7.37 - 7.16 (m, 3H), 6.85 - 6.60 (m, 1H), 4.59 - 3.99 (m, 1H), 3.83 (s, 3H), 3.79 - 3.46 (m, 5H), 3.45 - 3.33 (m, 1H), 3.28 - 3.18 (m, 2H), 3.17 - 2.83 (m,2H), 2.77 - 2.69 (m, 1H), 2.59 (s, 3H), 2.57 - 2.42 (m, 2H), 2.36 - 1.86 (m, 1H), 1.82 - 1.71 (m, 4H), 1.70 - 1.55 (m, 3H).

### Compound 276-2:

MS m/z (ESI): 603.4 [M+1]
SFC: retention time: 2.495 min; Chiralpak AD-3 50 x 4.6 mm I.D., 3 um; mobile phase: CO₂-EtOH (0.05% DEA); elution gradient: 5%-40%.

¹H NMR (400 MHz, CD₃OD) δ 7.88 - 7.74 (m, 1H), 7.64 - 7.50 (m, 1H), 7.38 - 7.17 (m, 4H), 6.85 - 6.59 (m, 1H),4.65 - 4.01 (m, 2H), 3.87 - 3.72 (m, 6H), 3.64 - 3.54 (m, 1H), 3.41 - 3.33 (m, 1H), 3.29 - 2.76 (m, 4H), 2.60 (s, 3H), 2.53 -2.36 (m, 3H), 2.01 - 1.83 (m, 4H), 1.52 - 1.41 (m, 1H), 1.39 - 1.23 (m, 4H), 1.21 - 1.02 (m, 3H), 0.80 (br d, J = 4.6 Hz, 1H).

### Example 280

### 1-(4-{1-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]propyl}piperidin-1-yl)ethan-1-one 280

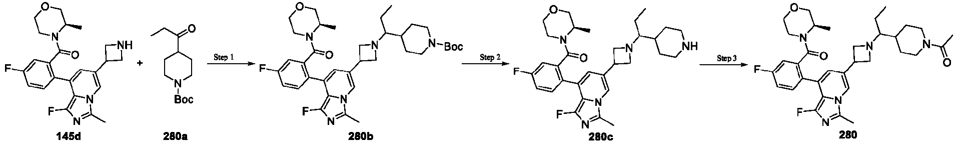

### Step 1

### 4-{1-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]propyl}piperidin-1-tert-butyl carboxylate 280b

Compound **145d** (120 mg, 281.39 µmol) and 4-propionylpiperidin-1-tert-butyl formate **280a** (74.70 mg, 309.53 µmol) were dissolved in methanol (3.0 mL), followed by the addition of zinc chloride (76.70 mg, 562.78 µmol), and the mixture was stirred at 55°C for 1.0 hour. Sodium cyanoborohydride (53.05 mg, 844.16 µmol) was added, and the mixture was stirred at 55°C for 11 hours. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the preparative thin-layer chromatography using dichloromethane/methanol as an elution system, to obtain the title Compound **280b** (180 mg, 276.16 µmol, yield: 98.14%) as yellow oil.
MS m/z (ESI):652.2[M+1]

### Step 2

### (3R)-4-[5-fluoro-2-(1-fluoro-3-methyl-6-{1-[1-(piperidin-4-yl)propyl]azacyclobutan-3-yl}imidazo[1,5-a]pyridin-8-yl)benzoyl]-3-methylmorpholine 280c

Compound **280b** (180 mg, 276.16 µmol) was dissolved in dichloromethane (3.0 mL), followed by the addition of trifluoroacetic acid (1.48 g, 12.98 mmol, 1 mL) at 25°C. The mixture was stirred at room temperature to allow for reacting for 1.0 hours. The reaction mixture was concentrated under a reduced pressure, and then dissolved by adding DCM (10 mL), followed by adding a 10% aqueous sodium hydroxide solution (20 mL), stirring for 0.5 hours and extracting with dichloromethane (10 mL x 3). The organic phases were combined, dried over anhydrous sodium sulfate and filtered, and the filtrate was concentrated under a reduced pressure, to obtain the crude product of title Compound **280c** (150 mg, 271.90 µmol, yield: 98.46%). The crude product was directly used in the next step of reaction without purification.
MS m/z (ESI): 552.3 [M+1]

### Step 3

### 1-(4-{1-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]propyl}piperidin-1-yl)ethan-1-one 280

Triethylamine (82.54 mg, 815.71 µmol) and Compound **280c** (150 mg, 271.90 µmol) were dissolved in dichloromethane (3.0 mL), and cooled to 5°C, followed by the addition of acetyl chloride (25.61 mg, 326.28 µmol). The reaction mixture was stirred at room temperature for 12 hours. Water (10 mL) was added to the reaction mixture, which was then extracted with dichloromethane (10 mL×3). The organic phase was dried over anhydrous sodium sulfate, filtered and concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% ammonium bicarbonate), elution gradient: 25%-55%), to obtain the title Compound **280** (55 mg, 92.64 µmol, yield: 34.07 %) as yellow solid.
MS m/z (ESI): 594.5 [M+1]
¹H NMR (400 MHz, DMSO-*d₆*) δ 7.92 - 7.79 (m, 1H), 7.62 - 7.49 (m, 1H), 7.44 - 7.23 (m, 2H), 6.87 - 6.43 (m, 1H), 4.50 - 3.38 (m, 9H), 3.23 - 3.04 (m, 3H), 3.02 - 2.60 (m, 3H), 2.55 (s, 3H), 2.43 - 2.29 (m, 1H), 2.06 - 1.99 (m, 1H), 1.99 - 1.94 (m, 3H), 1.71 - 1.44 (m, 3H), 1.34 - 1.01 (m, 6H), 0.91 - 0.84 (m, 3H), 0.79 - 0.52 (m, 1H).

### Examples 280-1 and 280-2

### 1-{4-[(1S)-1-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]propyl]piperidin-1-yl}ethan-1-one 280-1

### 1-{4-[(1R)-1-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]propyl]piperidin-1-yl}ethan-1-one 280-2

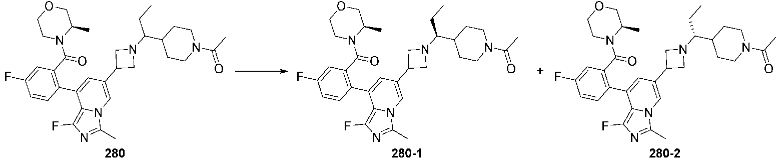

Compound **280** (47.77 mg, 80.46 µmol) was separated and purified by the chiral preparative HPLC (preparative column: DAICEL DAICEL ChiralPak IF, (250*50 mm, 10 um), mobile phase: n-hexane-ethanol (0.1%isopropylamine); elution gradient: 58%), to obtain the title Compounds **280-1** (14.75 mg, 24.84 µmol, yield: 30.88%) and **280-2** (16.31 mg, 27.47 µmol, yield: 34.14%).

### Single-configuration Compound 280-1:

MS m/z (ESI):594.4[M+1]
Chiral HPLC analysis: retention time: 2.226 min; chromatographic column: Chiralpak IF-3 50 x 4.6mm I.D., 3 um; mobile phase: n-hexane (0.05% isopropylamine)-EtOH (0.05% isopropylamine): elution gradient: 35%.

¹H NMR (400 MHz, DMSO-*d₆*) δ 7.86 (d, J = 10.8 Hz, 1H), 7.67 - 7.18 (m, 3H), 6.97 - 6.38 (m, 1H), 4.51 - 3.39 (m, 9H), 3.21 - 3.05 (m, 3H), 3.03 - 2.65 (m, 3H), 2.55 (s, 3H), 2.44 - 2.30 (m, 1H), 2.06 - 1.91 (m, 4H), 1.69 - 1.41 (m, 3H), 1.34 - 1.04 (m, 6H), 0.91 - 0.53 (m, 4H).

### Single-configuration Compound 280-2:

MS m/z (ESI):594.3[M+1]
Chiral HPLC analysis: retention time: 3.682 min; chromatographic column: Chiralpak IF-3 50 x 4.6mm I.D., 3 um; mobile phase: n-hexane (0.05% isopropylamine)-EtOH (0.05% isopropylamine): elution gradient: 35%.

¹H NMR (400 MHz, DMSO-*d₆*) δ 7.97 - 7.74 (m, 1H), 7.66 - 7.16 (m, 3H), 6.89 - 6.51 (m, 1H), 4.53 - 3.38 (m, 9H), 3.22 - 3.08 (m, 3H), 3.05 - 2.65 (m, 3H), 2.55 (s, 3H), 2.42 - 2.29 (m, 1H), 2.08 - 1.89 (m, 4H), 1.70 - 1.44 (m, 3H), 1.34 - 1.05 (m, 6H), 0.91 - 0.54 (m, 4H).

### Example 282

### N-ethyl-4-{1-[3-(8-{2-[ethyl(isopropyl)aminoformyl]-4-fluorophenyl}-1-fluoro-3-methylimidazo[1,5-a]pyridin-6-yl)azacyclobutan-1-yl]ethyl}piperidin-1-formamide 282

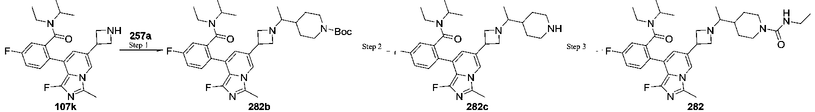

### Step 1

### 4-{1-[3-(8-{2-[ethyl(isopropyl)aminoformyl]-4-fluorophenyl}-1-fluoro-3-methylimidazo[1,5-a]pyridin-6-yl)azacyclobutan-1-yl]ethyl}piperidin-1-tert-butyl carboxylate 282b

Compound **107k** (158 mg, 383.05 µmol) and 4-acetylpiperidin-1-tert-butyl formate **257a** (95.77 mg, 421.36 µmol) were dissolved in methanol (3.0 mL), followed by the addition of zinc chloride (104.42 mg, 766.11 µmol), and the mixture was stirred at 55°C for 1.0 hour. Sodium cyanoborohydride (72.22 mg, 1.15 mmol) was added, and the mixture was stirred at 55°C for 11 hours. A saturated aqueous sodium bicarbonate solution (15 mL) was added to the reaction mixture, which was then extracted with dichloromethane (15 mL x 3). The organic phases were combined, dried over anhydrous sodium sulfate, and concentrated under a reduced pressure. The resulting residues were purified by the preparative thin-layer chromatography using dichloromethane/methanol as an elution system, to obtain the title Compound **282b** (220 mg, 352.69 µmol, yield: 92.07%) as yellow oil.
MS m/z (ESI):624.3 [M+1]

### Step 2

### N-ethyl-5-fluoro-2-(1-fluoro-3-methyl-6-{1-[1-(piperidin-4-yl)ethyl]azacyclobutan-3-yl}imidazo[1,5-a]pyridin-8-yl)-N-(isopropyl)benzamide 282c

Compound **282b** (220 mg, 352.69 µmol) was dissolved in dichloromethane (3.0 mL), followed by the addition of trifluoroacetic acid (1.48 g, 12.98 mmol, 1 mL) at 25°C. The mixture was stirred at room temperature to allow for reacting for 1.0 hours. The reaction mixture was concentrated under a reduced pressure, and then dissolved by adding DCM (10 mL), followed by adding a 10% aqueous sodium hydroxide solution (20 mL), stirring for 0.5 hours and extracting with dichloromethane (10 mL x 3). The organic phases were combined, dried over anhydrous sodium sulfate and filtered, and the filtrate was concentrated under a reduced pressure, to obtain the crude product of title Compound **282c** (180 mg, 343.74 µmol, yield: 97.46%). The crude product was directly used in the next step of reaction without purification.
MS m/z (ESI): 524.3 [M+1]

### Step 3

### N-ethyl-4-{1-[3-(8-{2-[ethyl(isopropyl)aminoformyl]-4-fluorophenyl}-1-fluoro-3-methylimidazo[1,5-a]pyridin-6-yl)azacyclobutan-1-yl]ethyl}piperidin-1-formamide 282

Triethylamine (52.17 mg, 515.60 µmol) and Compound **282c** (90 mg, 171.87 µmol) were dissolved in dichloromethane (2.0 mL), and cooled to 0°C, followed by the addition of isocyanoethane (18.32 mg, 257.80 µmol). The reaction mixture was stirred at room temperature for 2 hours. Water (10 mL) was added to the reaction mixture, which was then extracted with dichloromethane (10 mL×3). The organic phase was dried over anhydrous sodium sulfate, filtered and concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% ammonia water), elution gradient: 28%-58%), to obtain the title Compound 282 (48 mg, 80.71 µmol, yield: 46.96 %) as yellow solid.
MS m/z (ESI): 595.4 [M+1]
¹H NMR (400 MHz, DMSO-*d₆*) δ 7.84 (s, 1H), 7.57 - 7.47 (m, 1H), 7.39 - 7.24 (m, 2H), 6.74 - 6.52 (m, 1H), 6.37 - 6.30 (m, 1H), 4.05 - 3.93 (m, 2H), 3.55 - 3.32 (m, 5H), 3.11 - 2.87 (m, 5H), 2.53 (s, 4H), 2.49 - 2.44 (m, 1H), 2.22 - 2.10 (m, 1H), 1.68 - 1.32 (m, 3H), 1.17 - 0.87 (m, 9H), 0.86 - 0.71 (m, 6H), 0.39 (s, 2H).

### Examples 282-1 and 282-2

### N-ethyl-4-[(1R)-1-[3-(8-{2-[ethyl(isopropyl)aminoformyl]-4-fluorophenyl}-1-fluoro-3-methylimidazo[1,5-a]pyridin-6-yl)azacyclobutan-1-yl]ethyl]piperidin-1-formamide 282-1

### N-ethyl-4-[(1S)-1-[3-(8-{2-[ethyl(isopropyl)aminoformyl]-4-fluorophenyl}-1-fluoro-3-methylimidazo[1,5-a]pyridin-6-yl)azacyclobutan-1-yl]ethyl]piperidin-1-formamide 282-2

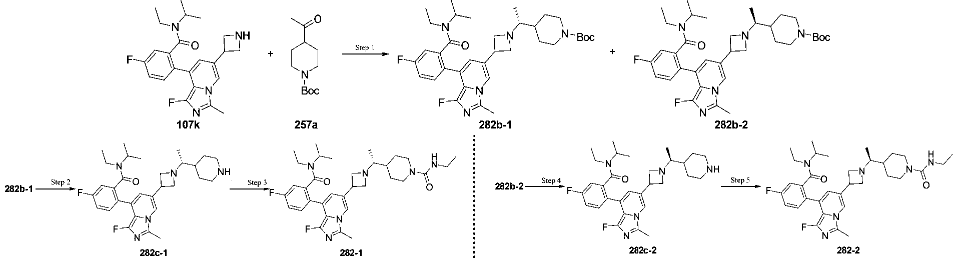

### Step 1

### 4-[(1R)-1-[3-(8-{2-[ethyl(isopropyl)aminoformyl]-4-fluorophenyl}-1-fluoro-3-methylimidazo[1,5-a]pyridin-6-yl)azacyclobutan-1-yl]ethyl]piperidin-1-tert-butyl carboxylate 282b-1

### 4-[(1S)-1-[3-(8-{2-[ethyl(isopropyl)aminoformyl]-4-fluorophenyl}-1-fluoro-3-methylimidazo[1,5-a]pyridin-6-yl)azacyclobutan-1-yl]ethyl]piperidin-1-tert-butyl carboxylate 282b-2

Compound **107k** (800 mg, 1.94 mmol) and 4-acetylpiperidin-1-tert-butyl formate **257a** (529.02 mg, 2.33 mmol) were dissolved in methanol (20 mL), followed by the addition of zinc chloride (793.04 mg, 5.82 mmol), and the mixture was stirred at 50°C for 1.0 hour. Sodium cyanoborohydride (365.65 mg, 5.82 mmol) was added, and the mixture was stirred at 50°C for 11 hours. A saturated aqueous sodium carbonate solution (50 mL) was added to the reaction mixture, which was then extracted with dichloromethane (30 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude compound was separated and purified by the chiral preparative SFC (preparative column: DAICEL CHIRALPAK IG (250 mm * 30 mm,10 um), mobile phase: CO₂-ethanol (0.1% ammonia water); elution gradient: 33%), to obtain the title Compounds **282b-1** (447 mg, 716.61 µmol, yield: 36.95%) and **282b-2** (502 mg, 804.78 µmol, yield: 41.49%).

### Single-configuration Compound 282b-1:

MS m/z (ESI):624.3[M+1]
Chiral SFC analysis: retention time: 2.019 min; chromatographic column: Chiralpak IG-3 50 x 4.6 mm I.D., 3 um; mobile phase: CO₂-EtOH (0.05% DEA): elution gradient: 5%-40%.

### Single-configuration Compound 282b-2:

MS m/z (ESI):624.4 [M+1]
Chiral SFC analysis: retention time: 1.927 min; chromatographic column: Chiralpak IG-3 50 x 4.6 mm I.D., 3 um; mobile phase: CO₂-EtOH (0.05% DEA): elution gradient: 5%-40%.

### Step 2

### N-ethyl-5-fluoro-2-(1-fluoro-3-methyl-6-{1-[(1R)-1-(piperidin-4-yl)ethyl]azacyclobutan-3-yl}imidazo[1,5-a]pyridin-8-yl)-N-(isopropyl)benzamide 282c-1

Compound **282b-1** (447 mg, 716.61 µmol) was dissolved in dichloromethane (3.0 mL), followed by the addition of trifluoroacetic acid (1 g, 8.77 mmol, 675.68 µL) at 25°C. The mixture was stirred at room temperature to allow for reacting for 1.0 hours. The reaction mixture was concentrated under a reduced pressure, and then dissolved by adding DCM (10 mL), followed by adding a 10% aqueous sodium hydroxide solution (20 mL), stirring for 0.5 hours and extracting with dichloromethane (10 mL x 3). The organic phases were combined, washed once with saturated brine (50 mL), then dried over anhydrous sodium sulfate, and filtered, and the filtrate was concentrated under a reduced pressure, to obtain the crude product of title Compound **282c-1** (344 mg, 656.92 µmol, yield: 91.67%). The crude product was directly used in the next step of reaction without purification.
MS m/z (ESI): 524.2 [M+1]

### Step 3

### N-ethyl-4-[(1R)-1-[3-(8-{2-[ethyl(isopropyl)aminoformyl]-4-fluorophenyl}-1-fluoro-3-methylimidazo[1,5-a]pyridin-6-yl)azacyclobutan-1-yl]ethyl]piperidin-1-formamide 282-1

Triethylamine (49.28 mg, 486.96 µmol) and Compound **282c-1** (85 mg, 162.32 µmol) were dissolved in dichloromethane (5.0 mL), and cooled to 5°C, followed by the addition of isocyanoethane (13.84 mg, 194.78 µmol). The reaction mixture was stirred at room temperature for 12 hours. Water (10 mL) was added to the reaction mixture, which was then extracted with dichloromethane (10 mL×3). The organic phase was dried over anhydrous sodium sulfate, filtered and concentrated under a reduced pressure. The resulting residues were purified by the HPLC (mobile phase: acetonitrile, water (0.1% ammonia water), elution gradient: 28%-58%), to obtain the title Compound **282-1** (48.96 mg, 82.32 µmol, yield: 50.72%) as yellow solid.
MS m/z (ESI): 595.4 [M+1]
Chiral SFC analysis: retention time: 1.285 min; chromatographic column: Chiralcel OD-3 50×4.6 mm I.D., 3 um; mobile phase: CO₂-MeOH(0.05%DEA): elution gradient: 5%-40%.

¹H NMR (400 MHz, CD₃OD) δ 7.82 - 7.73 (m, 1H), 7.60 - 7.50 (m, 1H), 7.35 - 7.25 (m, 1H), 7.25 - 7.16 (m, 1H), 6.79 - 6.67 (m, 1H), 4.59 (s, 1H), 4.07 (t, J = 10.6 Hz, 2H), 3.70 (t, J = 6.0 Hz, 2H), 3.66 - 3.54 (m, 2H), 3.52 - 3.37 (m, 1H), 3.28 - 3.13 (m, 4H), 3.10 - 2.97 (m, 1H), 2.78 - 2.63 (m, 2H), 2.58 (s, 3H), 2.43 - 2.31 (m, 1H), 1.71 - 1.60 (m, 2H), 1.52 (t, J = 14.4 Hz, 1H), 1.36 - 1.12 (m, 3H), 1.10 (t, J = 7.2 Hz, 3H), 1.00 (d, J = 6.4 Hz, 2H), 0.96 - 0.83 (m, 6H), 0.43 (d, J = 4.4 Hz, 2H)..

### Step 4

### N-ethyl-5-fluoro-2-(1-fluoro-3-methyl-6-{1-[(1S)-1-(piperidin-4-yl)ethyl]azacyclobutan-3-yl}imidazo[1,5-a]pyridin-8-yl)-N-(isopropyl)benzamide 282c-2

Compound **282b-2** (502 mg, 804.78 µmol) was dissolved in dichloromethane (3.0 mL), followed by the addition of trifluoroacetic acid (1 g, 8.77 mmol, 675.68 µL) at 25°C. The mixture was stirred at room temperature to allow for reacting for 1.0 hours. The reaction mixture was concentrated under a reduced pressure, and then dissolved by adding DCM (10 mL), followed by adding a 10% aqueous sodium hydroxide solution (20 mL), stirring for 0.5 hours and extracting with dichloromethane (10 mL x 3). The organic phases were combined, washed once with saturated brine (50 mL), then dried over anhydrous sodium sulfate, and filtered, and the filtrate was concentrated under a reduced pressure, to obtain the crude product of title Compound **282c-2** (404 mg, 771.49 µmol, yield: 95.86%). The crude product was directly used in the next step of reaction without purification.
MS m/z (ESI): 524.2 [M+1]

### Step 5

### N-ethyl-4-[(1S)-1-[3-(8-{2-[ethyl(isopropyl)aminoformyl]-4-fluorophenyl}-1-fluoro-3-methylimidazo[1,5-a]pyridin-6-yl)azacyclobutan-1-yl]ethyl]piperidin-1-formamide 282-2

Triethylamine (57.97 mg, 572.89 µmol) and Compound **282c-2** (100 mg, 190.96 µmol) were dissolved in dichloromethane (5.0 mL), and cooled to 5°C, followed by the addition of isocyanoethane (11.88 mg, 151.40 µmol). The reaction mixture was stirred at room temperature for 2 hours. Water (30 mL) was added to the reaction mixture, which was then extracted with dichloromethane (50 mL×3). The organic phase was dried over anhydrous sodium sulfate, filtered and concentrated under a reduced pressure. The resulting residues were purified by the HPLC (mobile phase: acetonitrile, water (0.1% ammonia water), elution gradient: 28%-58%), to obtain the title Compound **282-2** (27.31 mg, 45.92 µmol, yield: 24.05 %) as yellow solid.
MS m/z (ESI): 595.3 [M+1]
Chiral SFC analysis: retention time: 1.280 min; chromatographic column: Chiralcel OD-3 50×4.6 mm I.D., 3 um; mobile phase: CO₂-MeOH(0.05%DEA): elution gradient: 5%-40%.

¹H NMR (400 MHz, DMSO-*d₆*) δ 7.90 - 7.79 (m, 1H), 7.58 - 7.48 (m, 1H), 7.41 - 7.25 (m, 2H), 6.75 - 6.56 (m, 1H), 6.35 (t,J = 5.2 Hz, 1H), 4.30 - 3.95 (m, 2H), 3.56 - 3.41 (m, 4H), 3.13 - 2.66 (m, 6H), 2.54 (s, 3H), 2.47 - 2.32 (m, 1H), 2.21 - 2.12(m, 1H), 1.69 - 1.32 (m, 3H), 1.17 - 0.65 (m, 16H), 0.40 (br s, 2H).

### Example 286

### (3R)-4-{2-[6-({1-[(1-cyclopropane carbonylpiperidin-4-yl)methyl]azacyclobutan-3-yl}methyl)-4-methylpyrrolo[1,2-a]pyrazin-8-yl]-5-fluorobenzoyl}-3-methylmorpholine 286

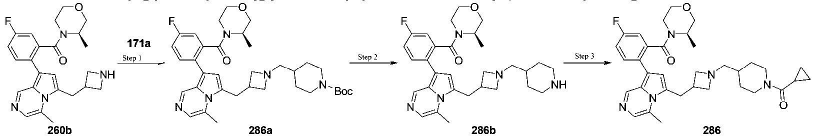

### Step 1

### 4-({3-[(8-{4-fluoro-2-[(3R)-3-methylmorpholin-4-carbonyl]phenyl}-4-methylpyrrolo[1,2-a]pyrazin-6-yl)methyl]azacyclobutan-1-yl}methyl)piperidin-1-tert-butyl carboxylate 286a

Compound **260b** (350 mg, 652.36 µmol, trifluoroacetate) and Compound **171a** (166.96 mg, 782.83 µmol) were dissolved in methanol (10 mL). Triethylamine (132.02 mg, 1.30 mmol) was added, and the mixture was stirred at room temperature for 0.5 hours. Sodium cyanoborohydride (122.99 mg, 1.96 mmol) was added, and the reaction mixture was stirred at room temperature for 12.0 hour. A saturated aqueous sodium bicarbonate solution (20 mL) was added, and the mixture was then extracted with dichloromethane (20 mL × 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **286a** (380 mg, 613.13 µmol, yield: 93.99%) as yellow solid.
MS m/z (ESI): 620.5[M+1]

### Step 2

### (3R)-4-{5-fluoro-2-[4-methyl-6-({1-[(piperidin-4-yl)methyl]azacyclobutan-3-yl}methyl)pyrrolo[1,2-a]pyrazin-8-yl]benzoyl}-3-methylmorpholine 286b

Compound **286a** (120 mg, 193.62 µmol) was dissolved in methane dioxide (3.0 mL), followed by the addition of trifluoroacetic acid (1.48 g, 12.98 mmol, 1 mL) at 0°C. The mixture was stirred at room temperature to allow for reacting for 0.5 hours. The reaction mixture was concentrated under a reduced pressure, to obtain the crude product of title Compound **286b** (100 mg, 192.44 µmol, yield: 99.39). The crude product was directly used in the next step of reaction without purification.
MS m/z (ESI): 520.1 [M+1]

### Step 3

### (3R)-4-{2-[6-({1-[(1-cyclopropane carbonylpiperidin-4-yl)methyl]azacyclobutan-3-yl}methyl)-4-methylpyrrolo[1,2-a]pyrazin-8-yl]-5-fluorobenzoyl}-3-methylmorpholine 286

Cyclopropanecarboxylic acid (20.87 mg, 242.47 µmol), triethylamine (47.91 mg, 473.43 µmol) and HATU(92.19 mg, 242.47 µmol) were dissolved in dichloromethane (2.0 mL), and stirred at room temperature for 10 min, followed by the addition of Compound **286b** (105 mg, 202.06 µmol). The reaction mixture was stirred at room temperature for 12 hours. A saturated aqueous sodium bicarbonate solution (10 mL) was added to the reaction mixture, which was then extracted with dichloromethane (10 mL × 3). The organic phase was washed with saturated brine, dried over anhydrous sodium sulfate, filtered and concentrated under a reduced pressure. The resulting residues were purified by the preparative thin-layer chromatography using dichloromethane/methanol as an elution system, and the obtained crude product was purified by the HPLC (mobile phase: acetonitrile, water (0.1%ammonium bicarbonate), elution gradient: 22%-52%), to obtain the title Compound **286** (51.71 mg, 87.98 µmol, yield: 43.54%) as yellow solid.
MS m/z (ESI): 588.3 [M+1]
¹H NMR (400 MHz, DMSO-*d₆*) δ 8.63 - 8.38 (m, 1H), 7.63 - 7.45 (m, 1H), 7.39 - 7.18 (m, 3H), 6.63 - 6.51 (m, 1H), 4.37 - 3.90 (m, 3H), 3.86 - 3.36 (m, 7H), 3.23 - 2.88 (m, 3H), 2.86 - 2.70 (m, 7H), 2.30 - 2.22 (m, 2H), 1.96 - 1.87 (m, 1H), 1.81 - 1.43 (m, 4H), 1.25 - 0.85 (m, 4H), 0.71 - 0.28 (m, 5H).

### Example 289

### 2-(6-{1-[(1-acetylpiperidin-4-yl)methyl]azacyclobutan-3-yl}-3-methylimidazo[1,5-a]pyridin-8-yl)-N-ethyl-5-fluoro-N-(isopropyl)benzamide 289

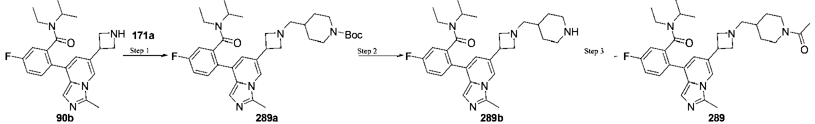

### Step 1

### 4-{[3-(8-{2-[ethyl(isopropyl)aminoformyl]-4-fluorophenyl}-3-methylimidazo[1,5-a]pyridin-6-yl)azacyclobutan-1-yl]methyl}piperidin-1-tert-butyl carboxylate 289a

Compound **90b** (398 mg, 1.01 mmol) and 4-acetylpiperidin-1-tert-butyl formate **171a** (258.21 mg, 1.21 mmol) were dissolved in methanol (10.0 mL), followed by the addition of zinc chloride (109.18 mg, 801.05 µmol), and the mixture was stirred at 25°C for 1.0 hour. Sodium cyanoborohydride (190.21 mg, 3.03 mmol) was added, and the mixture was stirred at 25°C for 11 hours. A saturated aqueous sodium bicarbonate solution (30 mL) was added to the reaction mixture, which was then extracted with dichloromethane (50 mL x 3). The organic phases were combined, dried over anhydrous sodium sulfate, and concentrated under a reduced pressure. The crude product of title Compound **289b** (580 mg, 980.13 µmol, yield: 97.04%) was obtained as yellow oil.
MS m/z (ESI):592.2[M+1]

### Step 2

### N-ethyl-5-fluoro-2-(3-methyl-6-{1-[(piperidin-4-yl)methyl]azacyclobutan-3-yl}imidazo[1,5-a]pyridin-8-yl)-N-(isopropyl)benzamide 289b

Compound **289a** (580 mg, 980.13 µmol) was dissolved in dichloromethane (3.0 mL), followed by the addition of trifluoroacetic acid (1 g, 8.77 mmol, 675.68 µL) at 25°C. The mixture was stirred at room temperature to allow for reacting for 1.0 hours. The reaction mixture was concentrated under a reduced pressure, and then dissolved by adding DCM (10 mL), followed by adding a 10% aqueous sodium hydroxide solution (20 mL), stirring for 0.5 hours and extracting with dichloromethane (10 mL x 3). The organic phases were combined, dried over anhydrous sodium sulfate and filtered, and the filtrate was concentrated under a reduced pressure, to obtain the crude product of title Compound **289b** (470 mg, 955.98 µmol, yield: 97.54%).The crude product was directly used in the next step of reaction without purification.
MS m/z (ESI): 492.2 [M+1]

### Step 3

### 2-(6-{1-[(1-acetylpiperidin-4-yl)methyl]azacyclobutan-3-yl}-3-methylimidazo[1,5-a]pyridin-8-yl)-N-ethyl-5-fluoro-N-(isopropyl)benzamide 289

Triethylamine (49.40 mg, 488.16 µmol) and Compound **289b** (80 mg, 162.72 µmol) were dissolved in dichloromethane (3.0 mL), and cooled to 5°C, followed by the addition of acetyl chloride (15.33 mg, 195.26 µmol). The reaction mixture was stirred at room temperature for 12 hours. Water (30 mL) was added to the reaction mixture, which was then extracted with dichloromethane (50 mL×3). The organic phase was dried over anhydrous sodium sulfate, filtered and concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% ammonium bicarbonate), elution gradient: 22%-52%), to obtain the title Compound **289** (26.35 mg, 49.37 µmol, yield: 30.34 %) as yellow solid.
MS m/z (ESI): 534.4 [M+1]
¹H NMR (400 MHz, CDCl₃) δ 7.70 - 7.40 (m, 2H), 7.23 - 7.01 (m, 3H), 6.96 - 6.74 (m, 1H), 4.65 - 4.39 (m, 1H),3.85 - 3.28 (m, 6H), 3.24 - 2.78 (m, 4H), 2.67 (s, 3H), 2.58 - 2.33 (m, 3H), 2.08 (s, 3H), 1.89 - 1.79 (m, 1H), 1.62 - 1.47 (m,1H), 1.31 - 0.63 (m, 10H), 0.25 (m, 2H).

### Examples 291 and 291-1

### (3r,4r)-1-[(4R)-4-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-5-methylhexyl]-4-methoxypyrrolidin-3-ol 291

### (3R,4R)-1-[(4R)-4-[3-(1-fluoro-8- {4-fluoro-2-[(3R)-3-methylmorpholin-4-carbonyl]phenyl}-3-methylimidazo[1,5-a]pyridin-6-yl)azacyclobutan-1-yl]-5-methylhexyl]-4-methoxypyrrolidin-3-ol 291-1

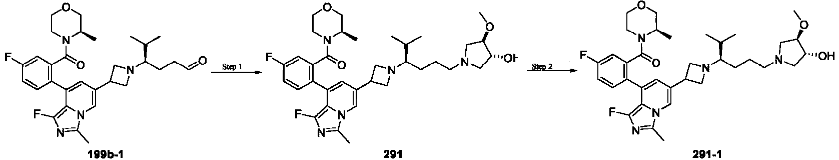

### Step 1

### (3r,4r)-1-[(4R)-4-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-5-methylhexyl]-4-methoxypyrrolidin-3-ol 291

Compound **199b-1** (220 mg, 408.45 µmol) and (3*r*,4*r*)-4-methoxypyrrolidin-3-ol (94.11 mg, 612.67 µmol, hydrochloride) were dissolved in methanol (5.0 mL), and stirred at room temperature for 1.0 hour. Sodium cyanoborohydride (77.00 mg, 1.23 mmol) was added, allowing for reacting at room temperature for 11.0 hours under the protection of nitrogen. A saturated aqueous sodium bicarbonate solution (30 mL) was added to the reaction mixture, which was then extracted with dichloromethane (100 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 26%-56%), to obtain the title product **291** (13.54 mg, 21.16 µmol, yield: 5.18%) as yellow solid.
MS m/z (ESI):640.5 [M+1]
¹H NMR (400 MHz, CDCl₃) δ 7.57 - 7.34 (m, 2H), 7.26 - 7.06 (m, 2H), 6.84 - 6.55 (m, 1H), 4.66 - 4.03 (m, 2H),3.94 - 3.43 (m, 6H), 3.38 (s, 3H), 3.33 - 2.66 (m, 8H), 2.63 (s, 3H), 2.53 - 1.88 (m, 6H), 1.71 - 1.06 (m, 7H), 0.99 - 0.56 (m,7H).

### Step 2

### (3R,4R)-1-[(4R)-4-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-5-methylhexyl]-4-methoxypyrrolidin-3-ol 291-1

Compound **291** (130 mg, 203.20 µmol) was separated and purified by the chiral preparative HPLC (preparative column: DAICEL CHIRALPAK AD (250 mm * 30 mm,10 um), mobile phase: acetonitrile, water (0.1% ammonia water); elution gradient: 30%), and the resulting crude product was purified by the HPLC (mobile phase: acetonitrile, water (0.1% ammonium bicarbonate), elution gradient: 13%-43%), to obtain the title product **291-1** (5.65 mg, 8.83 µmol, yield: 4.35%) as yellow solid.
MS m/z (ESI):640.5 [M+1]
Chiral SFC analysis: retention time: 1.433 min; chromatographic column: Chiralpak AD-3 50 x 4.6 mm I.D., 3 um; mobile phase: CO₂-EtOH (0.05% DEA): elution gradient: 5%-40%.

¹H NMR (400 MHz, CD₃OD) δ 7.86 - 7.74 (m, 1H), 7.64 - 7.50 (m, 1H), 7.41 - 7.15 (m, 2H), 6.89 - 6.56 (m, 1H),4.79 - 4.09 (m, 3H), 4.08 - 3.38 (m, 7H), 3.34 (s, 3H), 3.28 - 2.62 (m, 7H), 2.60 (s, 3H), 2.52 - 2.17 (m, 4H), 1.93 - 0.51 (m,15H).

### Examples 296-1 and 296-2

### N-ethyl-5-fluoro-2-[6-({1-[(3R)-6-[(2-methoxyethyl)(methyl)amino]-2-methylhexan-3-yl]azacyclobutan-3-yl}methyl)-4-methylpyrrolo[1,2-a]pyrazin-8-yl]-N-(isopropyl)benzamide 296-1

### N-ethyl-5-fluoro-2-[6-({1-[(3S)-6-[(2-methoxyethyl)(methyl)amino]-2-methylhexan-3-yl]azacyclobutan-3-yl}methyl)-4-methylpyrrolo[1,2-a]pyrazin-8-yl]-N-(isopropyl)benzamide 296-2

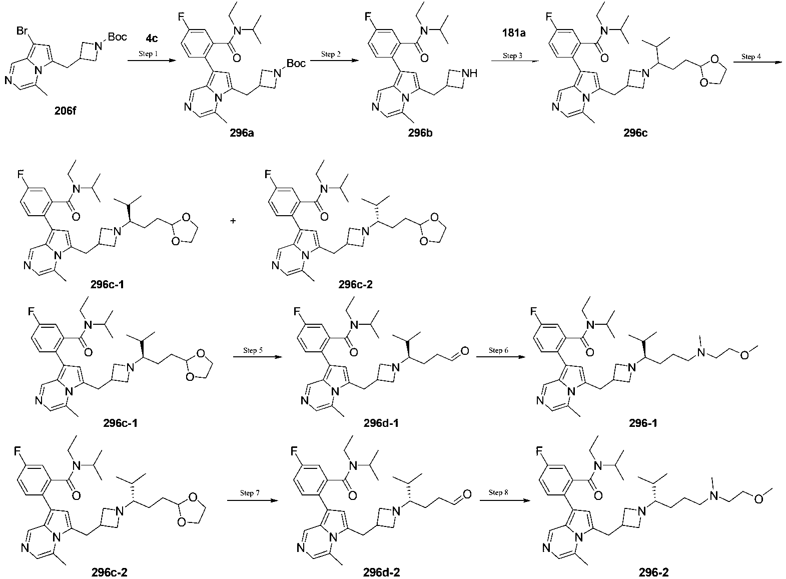

### Step 1

### 3-[(8-{2-[ethyl(isopropyl)aminoformyl]-4-fluorophenyl}-4-methylpyrrolo[1,2-a]pyrazin-6-yl)methyl]azacyclobutan-1-tert-butyl carboxylate 296a

Compound **206f** (300 mg, 788.90 µmol), Compound 4c(396.68 mg, 1.18 mmol) and potassium phosphate (502.37 mg, 2.37 mmol) were dissolved in dioxane (12 mL) and water (3.0 mL). Pd(dtbpf)Cl₂ (51.42 mg, 78.89 µmol) was added. Under a nitrogen atmosphere, the mixture was stirred at 95°Cto allow for reacting for 4.0 hours. The reaction mixture was cooled to room temperature, followed by adding water (20 mL) and extracting with ethyl acetate (20 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **296a** (300 mg, 589.82 µmol, yield: 74.77%) as yellow solid.
MS m/z (ESI): 509.1 [M+1]

### Step 2

### 2-{6-[(azacyclobutan-3-yl)methyl]-4-methylpyrrolo[1,2-a]pyrazin-8-yl}-N-ethyl-5-fluoro-N-(isopropyl)benzamide 296b

Compound **296a** (300 mg, 590.97 µmol) was dissolved in methane dioxide (6.0 mL), followed by the addition of trifluoroacetic acid (2.96 g, 25.96 mmol, 2 mL) at 0°C. The mixture was stirred at room temperature to allow for reacting for 1.0 hours. The reaction mixture was concentrated under a reduced pressure, then dissolved by adding DCM (5.0 mL), followed by adding a 10% aqueous sodium hydroxide solution (20 mL), stirring for 0.5 hours and extracting with dichloromethane (10 mL x 3). The organic phases were combined, dried over anhydrous sodium sulfate and filtered, and the filtrate was concentrated under a reduced pressure, to obtain the crude product of title Compound **296b** (240 mg, 587.50 µmol, yield: 99.41). The crude product was directly used in the next step of reaction without purification.
MS m/z (ESI): 409.2 [M+1]

### Step 3

### 2-[6-({1-[1-(1,3-dioxolan-2-yl)-4-methylpentan-3-yl]azacyclobutan-3-yl}methyl)-4-methylpyrrolo[1,2-a]pyrazin-8-yl]-N-ethyl-5-fluoro-N-(isopropyl)benzamide 296c

Compound **296b** (240 mg, 587.50 µmol) and Compound **181a** (121.42 mg, 705.00 µmol) were dissolved in methanol (4.0 mL). Zinc chloride (240.22 mg, 1.76 mmol) was added, and the mixture was stirred at 50°C for 1.0 hour. The mixture was cooled to room temperature, followed by adding sodium cyanoborohydride (110.76 mg, 1.76 mmol) and stirring at 50°C for 12.0 hour. A saturated aqueous sodium bicarbonate solution (10 mL) was added, and the mixture was then extracted with dichloromethane (10 mL × 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 43%-73%), to obtain the title Compound 296c (220 mg, 389.57 µmol, yield: 66.31%) as yellow solid.
MS m/z (ESI): 565.5[M+1]

### Step 4

### 2-[6-({1-[(3R)-1-(1,3-dioxolan-2-yl)-4-methylpentan-3-yl]azacyclobutan-3-yl}methyl)-4-methylpyrrolo[1,2-a]pyrazin-8-yl]-N-ethyl-5-fluoro-N-(isopropyl)benzamide 296c-1

### 2-[6-({1-[(3S)-1-(1,3-dioxolan-2-yl)-4-methylpentan-3-yl]azacyclobutan-3-yl}methyl)-4-methylpyrrolo[1,2-a]pyrazin-8-yl]-N-ethyl-5-fluoro-N-(isopropyl)benzamide 296c-2

Compound **296c** (220 mg, 389.57 µmol) was separated and purified by the chiral preparative SFC (preparative column: DAICEL DAICEL CHIRALCEL OD (250 mm * 30 mm,10 um), mobile phase: CO₂-MeOH (0.1% ammonia water); elution gradient: 20%), to obtain the title Compounds **296c-1** (83 mg, 146.97 µmol, yield: 37.73%) and **296c-2** (87 mg, 154.06 µmol, yield: 39.55%).

### Single-configuration Compound 296c-1:

MS m/z (ESI):565.5 [M+1]
Chiral HPLC analysis: retention time: 1.975 min; chromatographic column: Chiralcel OD-3 50 x 4.6 mm I.D., 3 um; mobile phase: CO₂-MeOH (0.05% diethylamide): elution gradient: 5%-40%.

### Single-configuration Compound 296c-2:

MS m/z (ESI):565.6 [M+1]
Chiral HPLC analysis: retention time: 1.294 min; chromatographic column: Chiralcel OD-3 50 x 4.6 mm I.D., 3 um; mobile phase: CO₂-MeOH (0.05% diethylamide): elution gradient: 5%-40%.

### Step 5

### N-ethyl-5-fluoro-2-[4-methyl-6-({1-[(3R)-2-methyl-6-oxohexan-3-yl]azacyclobutan-3-yl}methyl)pyrrolo[1,2-a]pyrazin-8yl]-N-(isopropyl)benzamide 296d-1

Compound **296c-1** (83 mg, 146.97 µmol) was dissolved in acetonitrile (2.0 mL), followed by the addition of a hydrochloric acid solution (1.0 M; 2.0 mL). The mixture was stirred at 50°C to allow for reacting for 1.0 hours. The reaction mixture was concentrated under a reduced pressure, diluted with a saturated aqueous sodium carbonate solution (10 mL), and extracted with dichloromethane (10 mL x 3). The organic phases were combined, dried over anhydrous sodium sulfate, and concentrated under a reduced pressure, to obtain the crude product of title Compound **296d-1** (70 mg, 134.44 µmol, yield: 91.47%). The crude product was directly used in the next step of reaction without purification.

### Single-configuration Compound 296d-1:

MS m/z (ESI): 521.2 [M+1]

### Step 6

### N-ethyl-5-fluoro-2-[6-({1-[(3R)-6-[(2-methoxyethyl)(methyl)amino]-2-methylhexan-3-yl]azacyclobutan-3-yl}methyl)-4-methylpyrrolo[1,2-a]pyrazin-8-yl]-N-(isopropyl)benzamide 296-1

The crude product of Compound **296d-1** (70 mg, 134.44 µmol) and 2-methoxy-*N*-methylethylamine (23.97 mg, 268.88 µmol) were dissolved in methanol (2 mL), followed by the addition of acetic acid (8.07 mg, 134.44 µmol), and the mixture was stirred at room temperature for 0.5 hours under the protection of nitrogen. Sodium cyanoborohydride (25.35 mg, 403.32 µmol) was added, allowing for reacting at room temperature for 12.0 hours under the protection of nitrogen. A saturated aqueous sodium bicarbonate solution (20 mL) was added to the reaction mixture, which was then extracted with dichloromethane (20 mL x 3). The organic phases were combined, dried over anhydrous sodium sulfate, and concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% ammonium bicarbonate), elution gradient: 35%-65%). The title Compound **296-1** (38.69 mg, 65.15 µmol, yield: 48.46%) was obtained.

### Single-configuration Compound 296-1:

MS m/z (ESI):594.3 [M+1]
Chiral SFC: retention time: 0.766 min; chromatographic column: Chiralcel OJ-3 50 × 4.6 mm I.D., 3 um; mobile phase: CO₂-MeOH (0.05% DEA): elution gradient: 5%-40%.

¹H NMR (400 MHz, CD₃OD) δ 8.60 - 8.52 (m, 1H), 7.60 - 7.52 (m, 1H), 7.37 - 7.14 (m, 3H), 6.82 - 6.73 (m, 1H), 4.71 - 4.27 (m, 2H), 3.70 - 3.49 (m, 7H), 3.18 - 2.99 (m, 3H), 2.87 - 2.84 (m, 3H), 2.67 - 2.58 (m, 2H), 2.48 - 2.39 (m, 2H), 2.33 - 2.28 (m, 3H), 2.22 - 2.03 (m, 1H), 1.90 - 1.81 (m, 1H), 1.61 - 1.52 (m, 2H), 1.41 - 1.16 (m, 5H), 1.03 - 0.80 (m, 13H), 0.36 - 0.32 (m, 2H).

### Step 7

### N-ethyl-5-fluoro-2-[4-methyl-6-({1-[(3S)-2-methyl-6-oxohexan-3-yl]azacyclobutan-3-yl}methyl)pyrrolo[1,2-a]pyrazin-8yl]-N-(isopropyl)benzamide 296d-2

Compound **296c-2** (87 mg, 154.06 µmol) was dissolved in acetonitrile (2.0 mL), followed by the addition of a hydrochloric acid solution (1.0 M; 2.0 mL). The mixture was stirred at 50°C to allow for reacting for 1.0 hours. The reaction mixture was concentrated under a reduced pressure, diluted with a saturated aqueous sodium carbonate solution (10 mL), and extracted with dichloromethane (10 mL x 3). The organic phases were combined, dried over anhydrous sodium sulfate, and concentrated under a reduced pressure, to obtain the crude product of title Compound **296d-2** (75 mg, 144.04 µmol, yield: 93.50%). The crude product was directly used in the next step of reaction without purification.

### Single-configuration Compound 296d-2:

MS m/z (ESI): 521.2 [M+1]

### Step 8

### N-ethyl-5-fluoro-2-[6-({1-[(3S)-6-[(2-methoxyethyl)(methyl)amino]-2-methylhexan-3-yl]azacyclobutan-3-yl}methyl)-4-methylpyrrolo[1,2-a]pyrazin-8-yl]-N-(isopropyl)benzamide 296-2

The crude product of Compound **296d-2** (75 mg, 144.04 µmol) and 2-methoxy-*N*-methylethylamine (25.68 mg, 288.09 µmol) were dissolved in methanol (2 mL), followed by the addition of acetic acid (8.65 mg, 144.04 µmol), and the mixture was stirred at room temperature for 0.5 hours under the protection of nitrogen. Sodium cyanoborohydride (27.16 mg, 432.13 µmol) was added, allowing for reacting at room temperature for 12.0 hours under the protection of nitrogen. A saturated aqueous sodium bicarbonate solution (20 mL) was added to the reaction mixture, which was then extracted with dichloromethane (20 mL x 3). The organic phases were combined, dried over anhydrous sodium sulfate, and concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% ammonium bicarbonate), elution gradient: 35%-65%). The title Compound **296-2** (33.45 mg, 56.33 µmol, yield: 39.11%) was obtained.

### Single-configuration Compound 296-2:

MS m/z (ESI):594.4 [M+1]
Chiral SFC: retention time: 1.177 min; chromatographic column: Chiralcel OD-3 50×4.6 mm I.D., 3um,; mobile phase: CO₂/MeOH (0.05% DEA): elution gradient: 5%-40%.

¹H NMR (400 MHz, CD₃OD) δ 8.55 - 8.52 (m, 1H), 7.60 - 7.49 (m, 1H), 7.35 - 7.13 (m, 3H), 6.77 - 6.72 (m, 1H), 4.64 - 4.32 (m, 2H), 3.67 - 3.48 (m, 7H), 3.14 - 2.98 (m, 3H), 2.85 - 2.82 (m, 3H), 2.64 - 2.58 (m, 2H), 2.45 - 2.38 (m, 2H), 2.31 - 2.25 (m, 3H), 2.19 - 2.04 (m, 1H), 1.88 - 1.79 (m, 1H), 1.60 - 1.49 (m, 2H), 1.35 - 1.15 (m, 5H), 1.00 - 0.78 (m, 13H), 0.34 - 0.30 (m, 2H).

### Examples 297-1 and 297-2

### (3R)-4-[5-fluoro-2-(1-fluoro-3-methyl-6-{1-[(2S)-3-methyl-1-{[(3R)-1-methylpiperidin-3-yl]oxy}butan-2-yl]azacyclobutan-3-yl}imidazo[1,5-a]pyridin-8yl)benzoyl]-3-methylmorpholine 297-1

### (3R)-4-[5-fluoro-2-(1-fluoro-3-methyl-6-{1-[(2R)-3-methyl-1-{[(3R)-1-methylpiperidin-3-yl]oxy}butan-2-yl]azacyclobutan-3-yl}imidazo[1,5-a]pyridin-8yl)benzoyl]-3-methylmorpholine 297-2

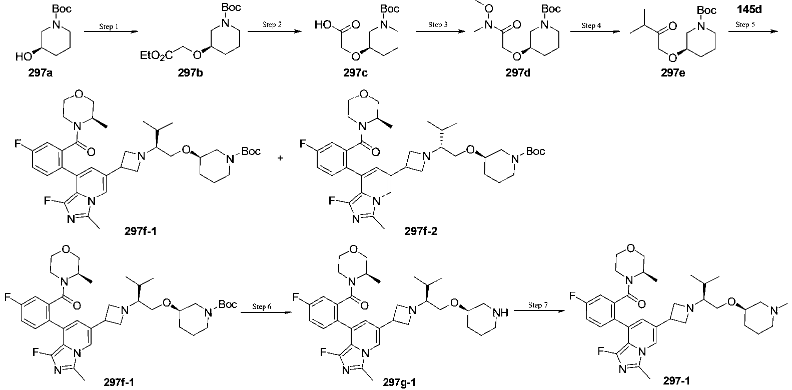

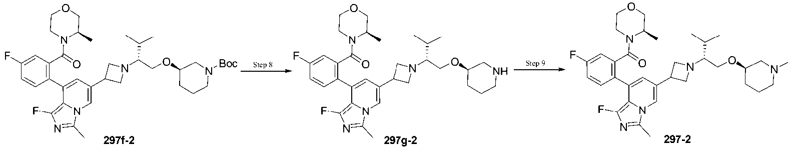

### Step 1

### (3R)-3-(2-ethoxy-2-oxoethoxy)piperidin-1-tert-butyl carboxylate 297b

(3*R*)-3-hydroxylpiperidin-1-tert-butyl carboxylate **297a** (5 g, 24.84 mmol) was dissolved in THF (70 mL), and cooled to 0°C, followed by the addition of sodium hydride (1.19 g, 29.81 mmol, 60% purity), and the mixture was stirred for 0.5 hours. 2-Bromoethyl acetate (4.98 g, 29.81 mmol) was added dropwise, and the mixture was stirred at room temperature for 12 hours. The reaction mixture was cooled to 0°C, followed by the addition of a saturated aqueous ammonium chloride solution (50 mL), and the reaction mixture was extracted with ethyl acetate (50 mL×3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **297b** (2 g, 6.96 mmol, yield: 28.02%).
MS m/z (ESI): 232.1 [M-55]
¹H NMR (400 MHz, CDCl₃) δ 4.29 - 4.04 (m, 4H), 3.82 (s, 1H), 3.65 - 3.56 (m, 1H), 3.41 (tt, J = 3.9, 8.1 Hz, 1H), 3.04 (br s, 2H), 1.99 (br d, J = 5.5 Hz, 1H), 1.81 - 1.72 (m, 1H), 1.64 - 1.53 (m, 1H), 1.49 - 1.44 (m, 10H), 1.26 (br s, 3H).

### Step 2

### 2-{[(3R)-1-[(tert-butoxy)carbonyl]piperidin-3-yl]oxy} acetic acid 297c

Under the protection of nitrogen, Compound **297b** (1.8 g, 6.26 mmol) was dissolved in ethanol (8.0 mL) and water (8.0 mL), followed by the addition of lithium hydroxide (1.31 g, 31.32 mmol), and the reaction mixture was stirred at room temperature for 12 hours. A 1M aqueous hydrochloric acid solution (15 mL) was added to the reaction mixture, which was then extracted with dichloromethane (20 mL × 3). The organic phase was dried over anhydrous sodium sulfate, filtered and concentrated under a reduced pressure, to obtain the title Compound **297c** (1.16 g, 4.47 mmol, yield: 71.42%).
MS m/z (ESI): 204.1 [M-55]
¹H NMR (400 MHz, DMSO-*d₆*) δ 12.55 (br s, 1H), 3.97 (d, J = 10.6 Hz, 2H), 3.75 - 3.49 (m, 1H), 3.37 (tt, J = 3.5, 7.0 Hz, 2H), 1.82 (br d, J = 4.5 Hz, 1H), 1.70 - 1.56 (m, 1H), 1.54 - 1.22 (m, 13H).

### Step 3

### (3R)-3-{[methoxy(methyl)aminoformyl]methoxy}piperidin-1-tert-butyl carboxylate 297d

Compound **297c** (1.16 g, 4.47 mmol) was dissolved in DCM (30 mL), followed by the addition of HATU (2.04 g, 5.37 mmol), triethylamine (1.36 g, 13.42 mmol) and *N*-methoxymethylamine (523.65 mg, 5.37 mmol, hydrochloride). The reaction mixture was stirred at room temperature for 12 hours. A saturated aqueous sodium carbonate solution (30 mL) was added to the reaction mixture, which was then extracted with dichloromethane (30 mL × 3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **297d** (1 g, 3.31 mmol, yield: 73.93%).
MS m/z (ESI): 303.1 [M+1]
¹H NMR (400 MHz, CDCl₃) δ 4.46 - 4.22 (m, 2H), 3.99 - 3.56 (m, 5H), 3.45 (tt, J = 3.8, 8.2 Hz, 1H), 3.25 - 2.94 (m, 5H), 2.03 (br d, J = 12.0 Hz, 1H), 1.67 - 1.54 (m, 1H), 1.45 (s, 11H).

### Step 4

### (3R)-3-(3-methyl-2-oxobutoxy)piperidin-1-tert-butyl carboxylate 297e

Under the protection of nitrogen, Compound **297d** (500 mg, 1.65 mmol) was dissolved in tetrahydrofuran (10 mL), and cooled to -70°C. An isopropyllithium solution (1.0 M, 5.60 mL) was added dropwise to the reaction mixture, after which the reaction mixture was stirred at -70°C for 3.0 hours. The reaction mixture was quenched by adding a saturated aqueous ammonium chloride solution (15 mL) at low temperature, and extracted with ethyl acetate (20 mL × 3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **297e** (160 mg, 560.66 µmol, yield: 33.90%) as colorless oil.

¹H NMR (400 MHz, CDCl₃) δ 4.37 - 4.09 (m, 2H), 3.72 (br s, 1H), 3.58 - 3.47 (m, 1H), 3.35 (br dd, J = 3.5, 7.0 Hz, 1H), 3.17 (br d, J = 1.3 Hz, 2H), 2.82 (br s, 1H), 2.02 - 1.86 (m, 1H), 1.78 (ddd, J = 3.0, 6.6, 10.0 Hz, 1H), 1.68 - 1.62 (m, 1H), 1.46 (s, 10H), 1.11 (d, J = 6.8 Hz, 6H).

### Step 5

### (3R)-3-[(2S)-2-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-3-methylbutoxy]piperidin-1-tert-butyl carboxylate 297f-1

### (3R)-3-[(2R)-2-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-3-methylbutoxy]piperidin-1-tert-butyl carboxylate 297f-2

Compound **145d** (125 mg, 293.11 µmol) and Compound **297e** (92.01 mg, 322.42 µmol) were dissolved in methanol (3.0 mL), followed by the addition of ZnCl₂ (79.90 mg, 586.22 µmol), and the mixture was stirred at 50°C for 1.0 hour. The mixture was cooled to room temperature, followed by the addition of sodium cyanoborohydride (55.26 mg, 879.34 µmol), allowing for reacting at 50°C for 10 hours under the protection of nitrogen. A saturated aqueous sodium bicarbonate solution (10 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system, and the resulting solid was separated and purified by the chiral preparative SFC (preparative column: DAICEL CHIRALPAK IG (250 mm * 30 mm,10 um), mobile phase: CO₂-[ACN/EtOH (0.1% ammonia water)]; elution gradient: 25%), to obtain the title Compounds **297f-1** (40 mg, 57.48 µmol, yield: 19.6%) and **297f-2** (40 mg, 57.48 µmol, yield: 19.6%).

### Single-configuration Compound 297f-1:

MS m/z (ESI):696.1 [M+1]
Chiral HPLC analysis: retention time: 1.643 min; chromatographic column: Chiralpak IG-3 50 x 4.6 mm I.D., 3 um; mobile phase: CO₂-EtOH (0.05% DEA): elution gradient: 5%-40%.

### Single-configuration Compound 297f-2:

MS m/z (ESI):696.2 [M+1]
Chiral HPLC analysis: retention time: 1.789 min; chromatographic column: Chiralpak IG-3 50 x 4.6 mm I.D., 3 um; mobile phase: CO₂-EtOH (0.05% DEA): elution gradient: 5%-40%.

### Step 6

### (3R)-4-[5-fluoro-2-(1-fluoro-3-methyl-6-{1-[(2S)-3-methyl-1-[(3R)-piperidin-3-yloxy]butan-2-yl]azacyclobutan-3-yl}imidazo[1,5-a]pyridin-8yl)benzoyl]-3-methylmorpholine 297g-1

Compound **297f-1** (40.00 mg, 57.48 µmol) was dissolved in dichloromethane (1.5 mL), followed by the addition of trifluoroacetic acid (740.00 mg, 6.49 mmol, 0.5 mL) at 25°C. The mixture was stirred at room temperature to allow for reacting for 1.0 hours. The reaction mixture was concentrated under a reduced pressure. The crude product of title Compound **297g-1** (35 mg, 58.75 µmol, yield: 102.21%) was obtained as yellow solid. The crude product was directly used in the next step of reaction without treatment.

### Step 7

### (3R)-4-[5-fluoro-2-(1-fluoro-3-methyl-6-{1-[(2S)-3-methyl-1-{[(3R)-1-methylpiperidin-3-yl]oxy}butan-2-yl]azacyclobutan-3-yl}imidazo[1,5-a]pyridin-8yl)benzoyl]-3-methylmorpholine 297-1

Compound **297g-1** (40.00 mg, 56.36 µmol, trifluoroacetate) was dissolved in methanol (2.0 mL), followed by the addition of triethylamine (8.55 mg, 84.54 µmol) and formaldehyde (23.50 mg, 281.79 µmol), and the mixture was stirred at 25°C for 1.0 hour. Sodium cyanoborohydride (10.62 mg, 169.07 µmol) was added, allowing for reacting at 25°C for 1.0 hours under the protection of nitrogen. A saturated aqueous sodium bicarbonate solution (10 mL) was added to the reaction mixture, which was then extracted with dichloromethane (20 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 40%-70%), to obtain the title Compound **297-1** (12.56 mg, 20.60 µmol, yield: 36.55%).

### Single-configuration Compound 297-1:

MS m/z (ESI):610.4 [M+1]
Chiral SFC analysis: retention time: 1.123 min; chromatographic column: Chiralcel OD-3 50×4.6 mm I.D., 3 um; mobile phase: CO₂-MeOH(0.05%DEA): elution gradient: 5%-40%.

¹H NMR (400 MHz, DMSO-*d₆*) δ 7.96 - 7.73 (m, 1H), 7.63 - 7.23 (m, 3H), 6.83 - 6.44 (m, 1H), 4.44 - 3.34 (m, 8H), 3.30 - 2.53 (m, 12H), 2.28 - 2.18 (m, 1H), 2.11 (s, 3H), 1.90 - 1.53 (m, 5H), 1.43 - 1.30 (m, 1H), 1.22 - 0.70 (m, 10H).

### Step 8

### (3R)-4-[5-fluoro-2-(1-fluoro-3-methyl-6-{1-[(2R)-3-methyl-1-[(3R)-piperidin-3-yloxy]butan-2-yl]azacyclobutan-3-yl}imidazo[1,5-a]pyridin-8yl)benzoyl]-3-methylmorpholine 297g-2

Compound **297f-2** (40 mg, 57.48 µmol) was dissolved in dichloromethane (1.5 mL), followed by the addition of trifluoroacetic acid (740.00 mg, 6.49 mmol, 0.5 mL) at 25°C. The mixture was stirred at room temperature to allow for reacting for 1.0 hours. The reaction mixture was concentrated under a reduced pressure. The crude product of title Compound **297g-2** (35 mg, 58.75 µmol, yield: 102.21%) was obtained as yellow solid. The crude product was directly used in the next step of reaction without treatment.

### Step 9

### (3R)-4-[5-fluoro-2-(1-fluoro-3-methyl-6-{1-[(2R)-3-methyl-1-{[(3R)-1-methylpiperidin-3-yl]oxy}butan-2-yl]azacyclobutan-3-yl}imidazo[1,5-a]pyridin-8yl)benzoyl]-3-methylmorpholine 297-2

Compound **297g-2** (40.00 mg, 56.36 µmol, trifluoroacetate) was dissolved in methanol (2.0 mL), followed by the addition of triethylamine (8.55 mg, 84.54 µmol) and formaldehyde (23.50 mg, 281.79 µmol), and the mixture was stirred at 25°C for 1.0 hour. Sodium cyanoborohydride (10.62 mg, 169.07 µmol) was added, allowing for reacting at 25°C for 1.0 hours under the protection of nitrogen. A saturated aqueous sodium bicarbonate solution (10 mL) was added to the reaction mixture, which was then extracted with dichloromethane (20 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 40%-70%), to obtain the title Compound **297-2** (8.93 mg, 14.65 µmol, yield: 25.99%).

### Single-configuration Compound 297-2:

MS m/z (ESI):610.4 [M+1]
Chiral SFC: retention time: 1.135 min; chromatographic column: Chiralcel OD-3 50×4.6 mm I.D., 3 um; mobile phase: CO₂/MeOH (0.05% DEA): elution gradient: 5%-40%.

¹H NMR (400 MHz,DMSO-*d*₆) δ 7.94 - 7.76 (m, 1H), 7.63 - 7.22 (m, 3H), 6.82 - 6.42 (m, 1H), 4.43 - 3.41 (m, 8H), 3.24 - 2.53 (m, 12H), 2.28 - 2.21 (m, 1H), 2.12 (s, 3H), 1.87 - 1.55 (m, 5H), 1.43 - 1.31 (m, 1H), 1.21 - 0.74 (m, 10H).

### Example 298

### (3R)-4-[5-fluoro-2-(1-fluoro-3-methyl-6-{1-[(2S)-3-methyl-1-{[(3R)-1-methylpyrrolidin-3-yl]oxy}butan-2-yl]azacyclobutan-3-yl}imidazo[1,5-a]pyridin-8yl)benzoyl]-3-methylmorpholine 298

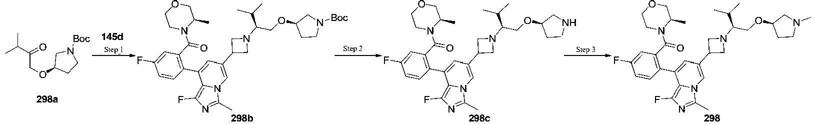

### Step 1

### (3R)-3-[(2S)-2-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-3-methylbutoxy]pyrrolidin-1-tert-butyl carboxylate 298b

Compound **145d** (125 mg, 293.11 µmol) and (3R)-3-(3-methyl-2-oxobutoxy)pyrrolidin-1-tert-butyl carboxylate **298a** (79.54 mg, 293.11 µmol, prepared according to the synthesis route of Compound **297e** by using (3R)-3-hydroxylpyrrolidin-1-tert-butyl carboxylate to replace (3R)-3-hydroxylpiperidin-1-tert-butyl carboxylate in the first step) were dissolved in methanol (3.0 mL), followed by the addition of ZnCl₂ (79.90 mg, 586.22 µmol), and the mixture was stirred at 50°C for 1.0 hour. The mixture was cooled to room temperature, followed by the addition of sodium cyanoborohydride (55.26 mg, 879.34 µmol), allowing for reacting at 50°C for 10 hours under the protection of nitrogen. A saturated aqueous sodium bicarbonate solution (10 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **298b** (110 mg, 161.34 µmol, yield: 55.04%) as colorless oil.
MS m/z (ESI):682.3 [M+1]

### Step 2

### (3R)-4-[5-fluoro-2-(1-fluoro-3-methyl-6-{1-[(2S)-3-methyl-1-[(3R)-pyrrolidin-3-yloxy]butan-2-yl]azacyclobutan-3-yl}imidazo[1,5-a]pyridin-8yl)benzoyl]-3-methylmorpholine 298c

Compound **298b** (110 mg, 161.34 µmol) was dissolved in dichloromethane (3.0 mL), followed by the addition of trifluoroacetic acid (1.48 g, 12.98 mmol, 1 mL) at 25°C. The mixture was stirred at room temperature to allow for reacting for 1.0 hours. The reaction mixture was concentrated under a reduced pressure. The crude product of title Compound **298c** (110 mg) was obtained. The crude product was directly used in the next step of reaction without treatment.
MS m/z (ESI):582.3 [M+1]

### Step 3

### (3R)-4-[5-fluoro-2-(1-fluoro-3-methyl-6-{1-[(2S)-3-methyl-1-{[(3R)-1-methylpyrrolidin-3-yl]oxy}butan-2-yl]azacyclobutan-3-yl}imidazo[1,5-a]pyridin-8yl)benzoyl]-3-methylmorpholine 298

The crude product of Compound **298c** (110 mg) was dissolved in methanol (3.0 mL), followed by the addition of triethylamine (24.00 mg, 237.17 µmol) and formaldehyde (64.15 mg, 790.55 µmol, 37%purity), and the mixture was stirred at 25°C for 1.0 hour. Sodium cyanoborohydride (29.81 mg, 474.33 µmol) was added, allowing for reacting at 25°C for 1.0 hours under the protection of nitrogen. A saturated aqueous sodium bicarbonate solution (10 mL) was added to the reaction mixture, which was then extracted with dichloromethane (20 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 44%-74%), to obtain the title Compound **298** (21.26 mg, 35.69 µmol, yield: 22.57%).
MS m/z (ESI):596.4 [M+1]
¹H NMR (400 MHz, CD₃OD) δ 7.88 - 7.70 (m, 1H), 7.66 - 7.51 (m, 1H), 7.40 - 7.16 (m, 2H), 6.89 - 6.57 (m, 1H), 4.59 - 3.97 (m, 2H), 3.81 - 3.34 (m, 8H), 3.29 - 2.40 (m, 12H), 2.33 (s, 3H), 2.15 - 2.04 (m, 1H), 1.93 - 1.75 (m, 2H), 1.45 - 0.39 (m, 10H).

### Examples 307-1 and 307-2

### N-ethyl-5-fluoro-2-(1-fluoro-6-{1-[(3S)-6-(2-methoxyethyl)(methyl)amino]-2-methylhexan-3-yl]azacyclobutan-3-yl}-3-methylimidazo[1,5-a]pyridin-8-yl)-N-(isopropyl)benzamide 307-1

### N-ethyl-5-ffuoro-2-(1-fluoro-6-{1-[(3R)-6-[(2-methoxyethyl)(methyl)amino]-2-methylhexan-3-yl]azacyclobutan-3-yl}-3-methylimidazo[1,5-a]pyridin-8-yl)-N-(isopropyl)benzamide 307-2

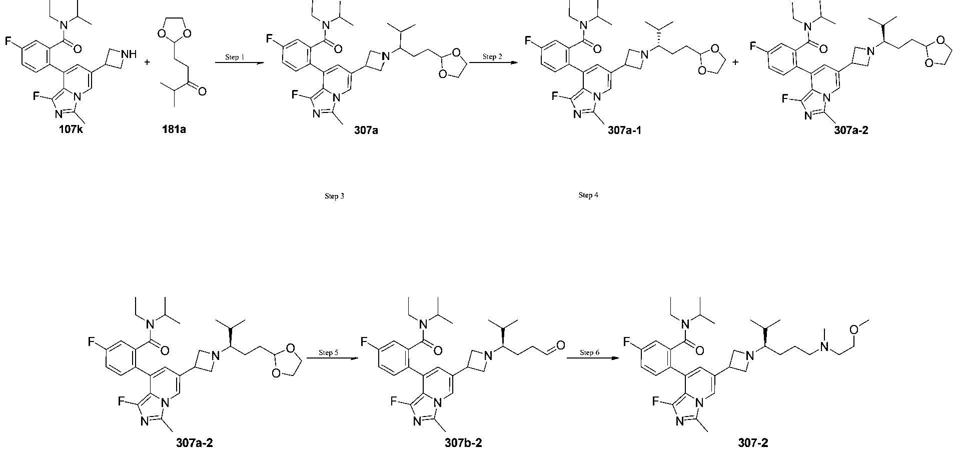

### Step 1

### 2-(6-{1-[1-(1,3-dioxolan-2-yl)-4-methylpentan-3-yl]azacyclobutan-3-yl}-1-fluoro-3-methylimidazo[1,5-a]pyridin-8-yl)-N-ethyl-5-fluoro-N-(isopropyl)benzamide 307a

Compound 107k (281.64 mg, 682.81 µmol) and 1-(1,3-dioxolan-2-yl)-4-methylpentan-3-one **181a** (141.11 mg, 819.37 µmol) were dissolved in methanol (25 mL), followed by the addition of ZnCl₂ (279.19 mg, 2.05 mmol), and the mixture was stirred at 25°C for 1.0 hour. The mixture was cooled to room temperature, followed by adding sodium cyanoborohydride (128.73 mg, 2.05 mmol) and stirring at 25°C for 12 hour. A saturated aqueous sodium carbonate solution (50 mL) was added to the reaction mixture, which was then stirred for 30 min and extracted with dichloromethane (30 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **307a** (350 mg, 615.44 µmol, yield: 90.13%)
MS m/z (ESI): 569.4 [M+1]

### Step 2

### 2-(6-{1-[(3S)-1-(1,3-dioxolan-2-yl)-4-methylpentan-3-yl]azacyclobutan-3-yl}-1-fluoro-3-methylimidazo[1,5-a]pyridin-8-yl)-N-ethyl-5-fluoro-N-(isopropyl)benzamide 307a-1

### 2-(6-{1-[(3R)-1-(1,3-dioxolan-2-yl)-4-methylpentan-3-yl]azacyclobutan-3-yl}-1-fluoro-3-methylimidazo[1,5-a]pyridin-8-yl)-N-ethyl-5-fluoro-N-(isopropyl)benzamide 307a-2

Compound **307a** (1.1 g, 1.93 mmol) was separated and purified by the chiral preparative SFC (preparative column: DAICEL CHIRALCEL OD (250 mm * 50 mm,10 um), mobile phase: n-hexane-isopropanol (0.1%isopropylamine); elution gradient: 10%), to obtain the title Compounds **307a-1** (550 mg, 967.12 µmol, yield: 50.0%) and **307a-2** (500 mg, 879.20 µmol, yield: 45.45%).

### Single-configuration Compound 307a-1:

MS m/z (ESI):569.3 [M+1]
Chiral HPLC analysis: retention time: 2.051 min; chromatographic column: Chiralcel OD-3 50 x 4.6 mm I.D., 3 um; mobile phase: n-hexane (0.05% isopropylamine)-isopropanol (0.05% isopropylamine): elution gradient: 8%.

### Single-configuration Compound 307a-2:

MS m/z (ESI):569.3 [M+1]
Chiral HPLC analysis: retention time: 5.181 min; chromatographic column: Chiralcel OD-3 50 x 4.6 mm I.D., 3 um; mobile phase: n-hexane (0.05% isopropylamine)-isopropanol (0.05% isopropylamine): elution gradient: 8%.

### Step 3

### N-ethyl-5-fluoro-2-(1-fluoro-3-methyl-6-{1-[(3S)-2-methyl-6-oxohexan-3-yl]azacyclobutan-3-yl}imidazo[1,5-a]pyridin-8-yl)-N-(isopropyl)benzamide 307b-1

Compound **307a-1** (300.00 mg, 527.52 µmol) was dissolved in acetonitrile (10.0 mL), followed by the addition of a hydrochloric acid solution (1.0 M; 10.0 mL). The mixture was stirred at 50°C to allow for reacting for 3.0 hours. The reaction mixture was concentrated under a reduced pressure, followed by adding dichloromethane (5 mL) and water (5 mL) and regulating to pH ≥ 7 with a 10 % aqueous NaOH solution, and the reaction mixture was extracted with dichloromethane (10 mL x 3). The organic phases were combined, dried over anhydrous sodium sulfate, and concentrated under a reduced pressure, to obtain the crude product of title Compound **307b-1** (276 mg, 526.07 µmol,yield: 99.72%). The crude product was directly used in the next step of reaction without purification.

### Single-configuration Compound 307b-1:

MS m/z (ESI): 525.4 [M+1]

### Step 4

### N-ethyl-5-fluoro-2-(1-fluoro-6-{1-[(3S)-6-[(2-methoxyethyl)(methyl)amino]-2-methylhexan-3-yl]azacyclobutan-3-yl}-3-methylimidazo[1,5-a]pyridin-8-yl)-N-(isopropyl)benzamide 307-1

The crude product of Compound **307b-1** (110.00 mg, 209.67 µmol) and 2-methoxy-N-methyl-ethylamine (28.03 mg, 314.50 µmol) were dissolved in methanol (5.0 mL), followed by the addition of acetic acid (12.59 mg, 209.67 µmol), and the mixture was stirred at room temperature for 1.0 hour. Sodium cyanoborohydride (39.53 mg, 629.00 µmol) was added, allowing for reacting at 25°C for 12.0 hours under the protection of nitrogen. Water (10 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 56%-86%). The title Compound **307-1** (11.96 mg, 20.01 µmol, yield: 9.54%) was obtained.

### Single-configuration Compound 307-1:

MS m/z (ESI):598.5 [M+1]
Chiral SFC: retention time: 1.177 min; chromatographic column: Chiralcel OD-3 50×4.6 mm I.D., 3 um; mobile phase: CO₂/MeOH (0.05% DEA): elution gradient: 5%-40%.

¹H NMR (400 MHz, CD₃OD) δ 7.78 (s, 1H), 7.59 - 7.51 (m, 1H), 7.30 (dt, J = 2.8, 8.8 Hz, 1H), 7.24 - 7.16 (m, 1H), 6.79 - 6.70 (m, 1H), 3.80 - 3.72 (m, 1H), 3.71 - 3.65 (m, 1H), 3.65 - 3.53 (m, 2H), 3.53 - 3.41 (m, 3H), 3.34 - 3.31 (m, 2H), 3.31 - 3.29 (m, 2H), 3.29 - 3.18 (m, 2H), 3.09 - 2.98 (m, 1H), 2.63 - 2.54 (m, 5H), 2.41 (q, J = 7.2 Hz, 2H), 2.30 - 2.20 (m, 4H), 1.92 - 1.78 (m, 1H), 1.64 - 1.48 (m, 2H), 1.41 - 1.21 (m, 2H), 1.00 (br d, J = 6.4 Hz, 2H), 0.97 - 0.81 (m, 10H), 0.43 (d, J = 4.4 Hz, 2H).

### Step 5

### N-ethyl-5-fluoro-2-(1-fluoro-3-methyl-6-{1-[(3R)-2-methyl-6-oxohexan-3-yl]azacyclobutan-3-yl}imidazo[1,5-a]pyridin-8-yl)-N-(isopropyl)benzamide 307b-2

Compound **307a-2** (300.00 mg, 527.52 µmol) was dissolved in acetonitrile (10.0 mL), followed by the addition of a hydrochloric acid solution (1.0 M; 10.0 mL). The mixture was stirred at 50°C to allow for reacting for 12 hours. The reaction mixture was concentrated under a reduced pressure, followed by adding dichloromethane (5 mL) and water (5 mL) and regulating to pH ≥ 7 with a 10 % aqueous NaOH solution, and the reaction mixture was extracted with dichloromethane (10 mL x 3). The organic phases were combined, dried over anhydrous sodium sulfate, and concentrated under a reduced pressure, to obtain the crude product of title Compound **307b-2** (276 mg, 526.07 µmol, yield: 99.72%). The crude product was directly used in the next step of reaction without purification.

### Single-configuration Compound 307b-2:

MS m/z (ESI): 525.4 [M+1]

### Step 6

### N-ethyl-5-fluoro-2-(1-fluoro-6-{1-[(3R)-6-[(2-methoxyethyl)(methyl)amino]-2-methylhexan-3-yl]azacyclobutan-3-yl}-3-methylimidazo[1,5-a]pyridin-8-yl)-N-(isopropyl)benzamide 307-2

The crude product of Compound **307b-2** (110.00 mg, 209.67 µmol) and 2-methoxy-N-methyl-ethylamine (28.03 mg, 314.50 µmol) were dissolved in methanol (5.0 mL), followed by the addition of acetic acid (12.59 mg, 209.67 µmol), and the mixture was stirred at room temperature for 1.0 hour. Sodium cyanoborohydride (39.53 mg, 629.00 µmol) was added, allowing for reacting at 25°C for 12.0 hours under the protection of nitrogen. Water (10 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonium bicarbonate), elution gradient: 44%-74%). The title Compound **307-2** (3.91 mg, 6.54 µmol, yield: 3.12%) was obtained.

### Single-configuration Compound 307-2:

MS m/z (ESI):598.4 [M+1]
Chiral SFC: retention time: 0.771 min; chromatographic column: Chiralcel AS-3 50×4.6 mm I.D., 3 um; mobile phase: CO₂/MeOH (0.05% DEA): elution gradient: 5%-40%.

¹H NMR (400 MHz, CD₃OD) δ 7.81 - 7.72 (m, 1H), 7.60 - 7.51 (m, 1H), 7.30 (dt, J = 2.4, 8.4 Hz, 1H), 7.25 - 7.17 (m, 1H), 6.82 - 6.65 (m, 1H), 3.79 - 3.72 (m, 1H), 3.72 - 3.66 (m, 1H), 3.65 - 3.54 (m, 2H), 3.54 - 3.38 (m, 3H), 3.32 (d, J = 2.4 Hz, 2H), 3.31 - 3.29 (m, 2H), 3.29 - 3.17 (m, 2H), 3.04 (qd, J = 6.8, 14.0 Hz, 1H), 2.67 - 2.52 (m, 5H), 2.41 (q, J = 7.2 Hz, 2H), 2.31 - 2.16 (m, 4H), 1.92 - 1.79 (m, 1H), 1.65 - 1.50 (m, 2H), 1.44 - 1.33 (m, 1H), 1.31 - 1.21 (m, 1H), 1.00 (d, J = 6.4 Hz, 2H), 0.97 - 0.79 (m, 10H), 0.43 (d, J = 4.8 Hz, 2H).

### Example 308

### (3R)-4-{5-fluoro-2-[1-fluoro-3-methyl-6-(1-{4-methyl-1-[(2S)-4-methylmorpholin-2-yl]pentan-3-yl}azacyclobutan-3-yl)imidazo[1,5-a]pyridin-8yl]benzoyl}-3-methylmorpholine 308

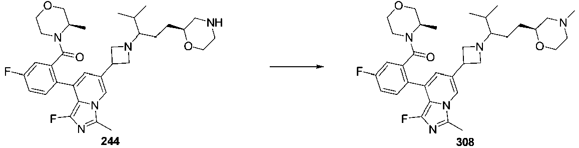

Compound **244** (90 mg, 151.08 µmol) was dissolved in methanol (5.0 mL), followed by the addition of triethylamine (30.57 mg, 302.15 µmol) and formaldehyde (14.71 mg, 181.29 µmol), and the mixture was stirred at 25°C for 10 min. Sodium cyanoborohydride (28.48 mg, 453.23 µmol) was added, allowing for reacting at 25°C for 12.0 hours under the protection of nitrogen. Water (20 mL) was added to the reaction mixture, which was then extracted with dichloromethane (10 mL x 3). The organic phases were combined, dried over anhydrous sodium sulfate, and concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% ammonia water), elution gradient: 35%-65%), to obtain the title Compound 308 (5.28 mg, 8.66 µmol, yield: 5.73%).
MS m/z (ESI):610.5 [M+1]
¹H NMR (400 MHz, CD₃OD) δ 7.88 - 7.73 (m, 1H), 7.66 - 7.50 (m, 1H), 7.39 - 7.12 (m, 2H), 6.87 - 6.53 (m, 1H), 4.59 (s, 6H), 3.84 (d, J = 10.0 Hz, 1H), 3.79 - 3.74 (m, 1H), 3.73 - 3.68 (m, 1H), 3.67 - 3.55 (m, 3H), 3.46 - 3.38 (m, 1H), 3.27 - 3.20 (m, 2H), 2.79 - 2.72 (m, 1H), 2.68 (d, J = 11.6 Hz, 1H), 2.60 (s, 3H), 2.27 (s, 3H), 2.24 - 2.19 (m, 1H), 2.10 (dt, J = 2.0, 11.2 Hz, 1H), 1.88 - 1.77 (m, 2H), 1.62 - 1.44 (m, 3H), 1.36 - 1.23 (m, 2H), 1.17 (d, J = 4.4 Hz, 1H), 0.92 (dd, J = 5.6, 15.2 Hz, 6H), 0.85 - 0.73 (m, 1H).

### Example 309

### 2-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-2-(oxacyclohexan-4-yl)ethan-1-ol 309

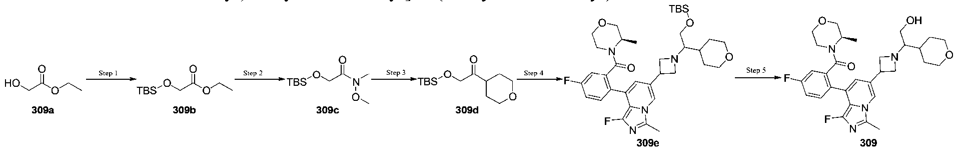

### Step 1

### 2-[(tert-butyldimethylsilicyl)oxy]ethyl acetate 309b

2-Hydroxylethyl acetate (10 g, 96.06 mmol) and imidazole (6.54 g, 96.06 mmol) were dissolved in dichloromethane (50 mL), and cooled to 0°C, followed by the slow addition of TBSCl (7.91 g, 96.06 mmol). The reaction mixture was stirred at room temperature for 2.0 hours. Dichloromethane (50 mL) was added to the reaction mixture, which was then washed with water (100 mL×3). The organic phase was dried over anhydrous sodium sulfate, filtered and concentrated under a reduced pressure, to obtain the title Compound **309b** (13.5 g, 61.82 mmol, yield: 64.36%) as colorless oil.

¹H NMR (400 MHz, DMSO-*d₆*) δ 4.21 (s, 2H), 3.68 (s, 3H), 3.45 (s, 3H), 3.18 (s, 3H).

### Step 2

### 2-[(tert-butyldimethylsilicyl)oxy]-N-methoxy-N-methylacetamide 309c

N-methoxymethylamine (6.25 g, 64.11 mmol, hydrochloride) and Compound **309b** (7 g, 32.06 mmol) were dissolved in THF (150 mL), and cooled to 0°C, followed by the slow dropwise addition of isopropylmagnesium chloride solution (2 M, 32.06 mL). The reaction mixture was stirred at room temperature for 12 hours. The reaction mixture was cooled to 0°C, followed by dropwise adding a saturated aqueous ammonium chloride solution (50 mL) and extracting with ethyl acetate (50 mL×3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **309c** (2.1 g, 9.00 mmol, yield: 28.07%) as colorless oil.

¹H NMR (400 MHz, CDCl₃) δ 4.12 (s, 2H), 4.11 - 3.99 (m, 2H), 1.21 - 1.12 (m, 3H), 0.84 - 0.80 (m, 9H), 0.03 - 0.03 (m, 6H).

### Step 3

### 2-[(tert-butyldimethylsilyl)oxy]-1-(oxacyclohexan-4-yl)ethan-1-one 309d

Under the protection of nitrogen, Mg (1.47 g, 60.47 mmol) was suspended in THF (20 mL), followed by the addition of I₂ (195.31 mg, 1.53 mmol) and then the slow dropwise addition of the solution of 4-bromotetrahydropyran (5 g, 30.30 mmol) in tetrahydrofuran (5.0 mL), and the mixture was stirred at room temperature for 1.0 hour. At 0°C, the prepared alkyl magnesium bromide tetrahydrofuran solution was slowly dropwise added to the solution of Compound **309c** (2.1 g, 9.00 mmol) in THF(25 mL). The reaction mixture was stirred at room temperature for 12 hours. A saturated aqueous ammonium chloride solution (50 mL) was added to the reaction mixture, which was then extracted with methyltert-butyl ether (50 mL×3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **309d** (2.2 g, 8.51 mmol, yield: 28.10%) as yellow oil.

¹H NMR (400 MHz, CDCl₃) δ 4.25 (s, 2H), 3.95 - 3.83 (m, 1H), 3.54 - 3.31 (m, 4H), 2.98 - 2.86 (m, 1H), 1.77 - 1.73 (m, 2H), 1.35 - 1.23 (m, 2H), 0.93 (s, 9H), 0.10 (s, 6H).

### Step 4

### (3R)-4-{2-[6-(1-{2-[(tert-butyldimethylsilyl)oxy]-1-(oxacyclohexan-4-yl)ethyl}azacyclobutan-3-yl)-1-fluoro-3-methylimidazo[1,5-a]pyridin-8-yl]-5-fluorobenzoyl}-3-methylmorpholine 309e

Compound **145d** (64 mg, 150.07 µmol) and Compound **309d**(77.57 mg, 300.15 µmol) were dissolved in methanol (10.0 mL). Zinc chloride (40.91 mg, 300.15 µmol) was added, and the mixture was stirred at 50°C for 1.0 hour. The reaction mixture was cooled to room temperature, followed by adding sodium cyanoborohydride (28.29 mg, 450.22 µmol) and stirring at 50°C for 11.0 hours. A saturated aqueous sodium bicarbonate solution (50 mL) was added, and the mixture was then extracted with dichloromethane (30 mL × 3). The organic phases were combined, dried over anhydrous sodium sulfate, and concentrated under a reduced pressure. The resulting residues were purified by the preparative thin-layer chromatography using dichloromethane/methanol as an elution system, to obtain the title Compound **309e** (40 mg, 59.80 µmol, yield: 39.85%).
MS m/z (ESI): 669.2[M+1]

### Step 5

### 2-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-2-(oxacyclohexan-4-yl)ethan-1-ol 309

Compound **309e** (40 mg, 59.80 µmol) was dissolved in dichloromethane (1.0 mL), the reaction mixture was cooled to 0°C, followed by the addition of trifluoroacetic acid (3 g, 26.31 mmol, 2.03 mL), and the mixture was stirred at 25°C for 48.0 hours. The reaction mixture was concentrated under a reduced pressure, followed by the addition of dichloromethane (10 mL) and a 10% aqueous sodium hydroxide solution (20 mL), and the reaction mixture was then stirred for 0.5 hours. The mixture was extracted with dichloromethane (10 mL×3). The organic phases were combined, dried over anhydrous sodium sulfate, and concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% ammonium bicarbonate), elution gradient: 20%-50%), to obtain the title Compound **309** (8.57 mg, 15.45 µmol, yield: 25.84 %) as yellow solid.
MS m/z (ESI): 555.3[M+1]
¹H NMR (400 MHz, CD₃OD) δ 7.91 - 7.73 (m, 1H), 7.65 - 7.51 (m, 1H), 7.38 - 7.16 (m, 2H), 6.90 - 6.64 (m, 1H),4.64 - 4.04 (m, 2H), 4.01 - 3.92 (m, 2H), 3.84 - 3.52 (m, 7H), 3.49 - 3.34 (m, 5H), 3.17 - 2.71 (m, 2H), 2.60 (s, 3H), 2.39 -2.29 (m, 1H), 1.90 - 1.78 (m, 1H), 1.69 - 1.41 (m, 4H), 1.36 - 1.12 (m, 2H), 0.88 - 0.54 (m, 1H).

### Examples 310-1 and 310-2

### N-ethyl-5-fluoro-2-(3-methyl-6-{1-[(3S)-2-methyl-6-(4-methylpiperazin-1-yl)hexan-3-yl]azacyclobutan-3-yl}imidazo[1,5-a]pyridin-8-yl)-N-(isopropyl)benzamide 310-1

### N-ethyl-5-fluoro-2-(3-methyl-6-{1-[(3R)-2-methyl-6-(4-methylpiperazin-1-yl)hexan-3-yl]azacyclobutan-3-yl}imidazo[1,5-a]pyridin-8-yl)-N-(isopropyl)benzamide 310-2

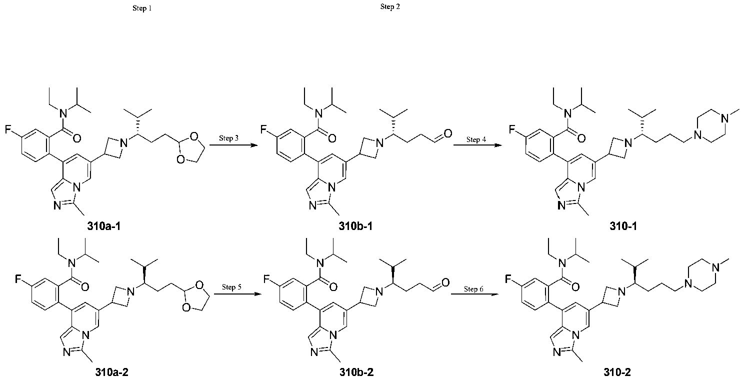

### Step 1

### 2-(6-{1-[1-(1,3-dioxolan-2-yl)-4-methylpentan-3-yl]azacyclobutan-3-yl}-3-methylimidazo[1,5-a]pyridin-8-yl)-N-ethyl-5-fluoro-N-(isopropyl)benzamide 310a

Compound **90b** (400 mg, 1.01 mmol) and 1-(1,3-dioxolan-2-yl)-4-methylpentan-3-one **181a** (261.94 mg, 1.52 mmol) were dissolved in methanol (8.0 mL), followed by the addition of ZnCl₂ (276.40 mg, 2.03 mmol), and the mixture was stirred at 55°C for 1.0 hour. The mixture was cooled to room temperature, followed by adding sodium cyanoborohydride (191.16 mg, 3.04 mmol) and stirring at 55°C for 11 hour. A saturated aqueous sodium carbonate solution (20 mL) was added to the reaction mixture, which was then stirred for 30 min and extracted with dichloromethane (20 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **310a** (500 mg, 907.93 µmol, yield: 89.54%)
MS m/z (ESI): 551.3 [M+1]

### Step 2

### 2-(6-{1-[(3S)-1-(1,3-dioxolan-2-yl)-4-methylpentan-3-yl]azacyclobutan-3-yl}-3-methylimidazo[1,5-a]pyridin-8-yl)-N-ethyl-5-fluoro-N-(isopropyl)benzamide 310a-1

### 2-(6-{1-[(3R)-1-(1,3-dioxolan-2-yl)-4-methylpentan-3-yl]azacyclobutan-3-yl}-3-methylimidazo[1,5-a]pyridin-8-yl)-N-ethyl-5-fluoro-N-(isopropyl)benzamide 310a-2

Compound **310a** (500 mg, 907.93 µmol) was separated and purified by the chiral preparative SFC (preparative column: Daicel Chiralcel OD (250*30 mm, 10 um), mobile phase: carbon dioxide-isopropanol (0.1% ammonia water); elution gradient: 35%), to obtain the title Compounds **310a-1** (165 mg, 299.62 µmol, yield: 33%) and **310a-**2 (155 mg, 281.46 µmol, yield: 31%).

### Single-configuration Compound 310a-1:

MS m/z (ESI):551.2 [M+1]
Chiral HPLC analysis: retention time: 1.456 min; chromatographic column: Chiralcel OD-3 50 x 4.6 mm I.D., 3 um; mobile phase: carbon dioxide-isopropanol (0.05% diethylamide ): elution gradient: 5%-40%.

### Single-configuration Compound 310a-2:

MS m/z (ESI):551.2 [M+1]
Chiral HPLC analysis: retention time: 1.710 min; chromatographic column: Chiralcel OD-3 50 x 4.6 mm I.D., 3 um; mobile phase: carbon dioxide-isopropanol (0.05% diethylamide ): elution gradient: 5%-40%.

### Step 3

### N-ethyl-5-fluoro-2-(3-methyl-6-{1-[(3S)-2-methyl-6-oxohexan-3-yl]azacyclobutan-3-yl}imidazo[1,5-a]pyridin-8-yl)-N-(isopropyl)benzamide 310b-1

Compound **310a-1** (165 mg, 299.62 µmol) was dissolved in acetonitrile (2.0 mL), followed by the addition of a hydrochloric acid solution (1.0 M; 0.3 mL). The mixture was stirred at 55°C to allow for reacting for 1.0 hours. The reaction mixture was concentrated under a reduced pressure, followed by adding dichloromethane (10 mL) and water (5 mL) and regulating to pH ≥ 7 with a 10 % aqueous NaOH solution, and the reaction mixture was extracted with dichloromethane (20 mL x 3). The organic phases were combined, dried over anhydrous sodium sulfate, and concentrated under a reduced pressure, to obtain the crude product of title Compound **310b-1** (150 mg, 296.06 µmol,yield: 98.81%). The crude product was directly used in the next step of reaction without purification.

### Single-configuration Compound 310b-1:

MS m/z (ESI): 507.2 [M+1]

### Step 4

### N-ethyl-5-fluoro-2-(3-methyl-6-{1-[(3S)-2-methyl-6-(4-methylpiperazin-1-yl)hexan-3-yl]azacyclobutan-3-yl}imidazo[1,5-a]pyridin-8-yl)-N-(isopropyl)benzamide 310-1

The crude product of Compound **310b-1** (75.00 mg, 148.03 µmol) and 1-methylpiperazine (17.79 mg, 177.64 µmol) were dissolved in methanol (2.0 mL), followed by the addition of acetic acid (8.89 mg, 148.03 µmol), and the mixture was stirred at room temperature for 1.0 hour. Sodium cyanoborohydride (27.91 mg, 444.09 µmol) was added, allowing for reacting at 25°C for 11.0 hours under the protection of nitrogen. A saturated aqueous sodium bicarbonate solution (10 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 36%-66%). The title Compound **310-1** (12 mg, 20.31 µmol, yield: 13.72%) was obtained.

### Single-configuration Compound 310-1:

MS m/z (ESI):591.6 [M+1]
Chiral SFC: retention time: 1.117 min; chromatographic column: Chiralcel OD-3 50×4.6 mm I.D., 3um,; mobile phase: CO₂/MeOH (0.05% DEA): elution gradient: 5%-40%.

¹H NMR (400 MHz, DMSO-*d₆*) δ 7.92 (s, 1H), 7.72 - 7.62 (m, 1H), 7.42 - 7.24 (m, 2H), 7.07 - 7.00 (m, 1H), 6.75 (d, J = 17.6 Hz, 1H), 3.59 - 3.41 (m, 4H), 3.30 - 3.25 (m, 1H), 3.17 - 3.02 (m, 2H), 2.99 - 2.86 (m, 1H), 2.58 (s, 3H), 2.41 - 2.17 (m, 9H), 2.14 - 2.08 (m, 3H), 2.02 (s, 1H), 1.77 - 1.64 (m, 1H), 1.50 - 1.35 (m, 2H), 1.31 - 1.03 (m, 3H), 0.96 - 0.68 (m, 13H), 0.28 (d, J = 6.4 Hz, 2H).

### Step 5

### N-ethyl-5-fluoro-2-(3-methyl-6-{1-[(3R)-2-methyl-6-oxohexan-3-yl]azacyclobutan-3-yl}imidazo[1,5-a]pyridin-8-yl)-N-(isopropyl)benzamide 310b-2

Compound **310a-2** (155.00 mg, 281.46 µmol) was dissolved in acetonitrile (3.0 mL), followed by the addition of a hydrochloric acid solution (1.0 M; 1.0 mL). The mixture was stirred at 50°C to allow for reacting for 2 hours. The reaction mixture was concentrated under a reduced pressure, followed by adding dichloromethane (5 mL) and water (5 mL) and regulating to pH ≥ 7 with a 10 % aqueous NaOH solution, and the reaction mixture was extracted with dichloromethane (10 mL x 3). The organic phases were combined, dried over anhydrous sodium sulfate, and concentrated under a reduced pressure, to obtain the crude product of title Compound **310b-2** (142 mg, 280.27 µmol, yield: 99.58%). The crude product was directly used in the next step of reaction without purification.

### Single-configuration Compound 310b-2:

MS m/z (ESI): 507.3 [M+1]

### Step 6

### N-ethyl-5-fluoro-2-(3-methyl-6-{1-[(3R)-2-methyl-6-(4-methylpiperazin-1-yl)hexan-3-yl]azacyclobutan-3-yl}imidazo[1,5-a]pyridin-8-yl)-N-(isopropyl)benzamide 310-2

The crude product of Compound **310b-2** (71.00 mg, 140.14 µmol) and 1-methylpiperazine (16.84 mg, 168.16 µmol) were dissolved in methanol (5.0 mL), and stirred at room temperature for 1.0 hour. Sodium cyanoborohydride (26.42 mg, 420.41 µmol) was added, allowing for reacting at 25°C for 11.0 hours under the protection of nitrogen. A saturated aqueous ammonium chloride solution (50 mL) was added to the reaction mixture, which was then extracted with ethyl acetate (50 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 35%-65%). The title Compound **310-2** (6.86 mg, 11.61 µmol, yield: 8.29%) was obtained.

### Single-configuration Compound 310-2:

MS m/z (ESI):591.7 [M+1]
Chiral SFC: retention time: 0.827 min; chromatographic column: Chiralcel AS-3 50×4.6 mm I.D., 3 um; mobile phase: CO₂/MeOH (0.05% DEA): elution gradient: 5%-40%.

¹H NMR (400 MHz, CD₃OD) δ 7.95 - 7.88 (m, 1H), 7.75 - 7.67 (m, 1H), 7.43 - 7.10 (m, 3H), 6.97 - 6.83 (m, 1H),4.81 - 4.24 (m, 2H), 3.87 - 3.33 (m, 5H), 3.30 - 2.69 (m, 4H), 2.66 (s, 3H), 2.61 - 2.17 (m, 11H), 1.93 - 1.81 (m, 1H), 1.68 -1.51 (m, 2H), 1.47 - 0.74 (m, 15H), 0.30 (br d, J = 6.8 Hz, 2H).

### Examples 311-1 and 311-2

### 2-(6-{1-[(3S)-6-(4-acetylpiperazin-1-yl)-2-methylhexan-3-yl]azacyclobutan-3-yl}-3-methylimidazo[1,5-a]pyridin-8-yl)-N-ethyl-5-fluoro-N-(isopropyl)benzamide 311-1

### 2-(6-{1-[(3R)-6-(4-acetylpiperazin-1-yl)-2-methylhexan-3-yl]azacyclobutan-3-yl}-3-methylimidazo[1,5-a]pyridin-8-yl)-N-ethyl-5-fluoro-N-(isopropyl)benzamide 311-2

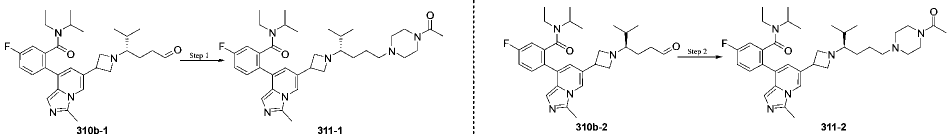

### Step 1

### 2-(6-{1-[(3S)-6-(4-acetylpiperazin-1-yl)-2-methylhexan-3-yl]azacyclobutan-3-yl}-3-methylimidazo[1,5-a]pyridin-8-yl)-N-ethyl-5-fluoro-N-(isopropyl)benzamide 311-1

The crude product of Compound **310b-1** (75.00 mg, 148.03 µmol) and 1-(piperazin-1-yl)ethan-1-one (22.77 mg, 177.64 µmol) were dissolved in methanol (2.0 mL), followed by the addition of acetic acid (8.89 mg, 148.03 µmol), and the mixture was stirred at room temperature for 1.0 hour.Sodium cyanoborohydride (27.91 mg, 444.09 µmol) was added, allowing for reacting at 25°C for 11.0 hours under the protection of nitrogen. A saturated aqueous sodium bicarbonate solution (10 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 28%-58%). The title Compound **311-1** (13 mg, 21.01 µmol, yield: 14.19%) was obtained as yellow solid.

### Single-configuration Compound 311-1:

MS m/z (ESI):619.5 [M+1]
Chiral SFC: retention time: 1.268 min; chromatographic column: Chiralcel OD-3 50×4.6 mm I.D., 3um,; mobile phase: CO₂/MeOH (0.05% DEA): elution gradient: 5%-40%.

¹H NMR (400 MHz, DMSO-*d₆*) δ 7.93 (s, 1H), 7.72 - 7.64 (m, 1H), 7.43 - 7.27 (m, 2H), 7.07 - 6.99 (m, 1H), 6.84 - 6.72 (m, 1H), 3.58 - 3.37 (m, 7H), 3.30 - 3.27 (m, 1H), 3.19 - 3.01 (m, 2H), 2.99 - 2.86 (m, 1H), 2.58 (s, 3H), 2.37 - 2.21 (m, 6H), 2.04 (s, 1H), 1.96 (s, 3H), 1.78 - 1.65 (m, 1H), 1.54 - 1.38 (m, 2H), 1.31 - 1.01 (m, 3H), 0.98 - 0.68 (m, 13H), 0.27 (d, J = 6.4 Hz, 2H).

### Step 2

### 2-(6-{1-[(3R)-6-(4-acetylpiperazin-1-yl)-2-methylhexan-3-yl]azacyclobutan-3-yl}-3-methylimidazo[1,5-a]pyridin-8-yl)-N-ethyl-5-fluoro-N-(isopropyl)benzamide 311-2

The crude product of Compound **310b-2** (71.00 mg, 140.14 µmol) and 1-(piperazin-1-yl)ethan-1-one (21.55 mg, 168.16 µmol) were dissolved in methanol (5.0 mL), and stirred at room temperature for 1.0 hour. Sodium cyanoborohydride (26.42 mg, 420.41 µmol) was added, allowing for reacting at 25°C for 11.0 hours under the protection of nitrogen. A saturated aqueous ammonium chloride solution (50 mL) was added to the reaction mixture, which was then extracted with ethyl acetate (50 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 28%-58%). The title Compound **311-2** (7.75 mg, 12.52 µmol, yield: 8.94%) was obtained.

### Single-configuration Compound 311-2:

MS m/z (ESI):619.6 [M+1]
Chiral SFC: retention time: 1.089 min; chromatographic column: Chiralcel AS-3 50×4.6 mm I.D., 3 um; mobile phase: CO₂/MeOH (0.05% DEA): elution gradient: 5%-40%.

¹H NMR (400 MHz, CD₃OD) δ 7.94 - 7.89 (m, 1H), 7.76 - 7.67 (m, 1H), 7.39 - 7.11 (m, 3H), 6.97 - 6.85 (m, 1H),4.78 - 4.26 (m, 2H), 3.86 - 3.37 (m, 9H), 3.29 - 2.97 (m, 2H), 2.66 (s, 3H), 2.52 - 2.37 (m, 5H), 2.28 (br s, 1H), 2.08 (d, J =3.6 Hz, 3H), 1.95 - 1.82 (m, 1H), 1.69 - 1.52 (m, 2H), 1.50 - 1.10 (m, 3H), 0.98 - 0.76 (m, 12H), 0.30 (br d, J = 6.8 Hz, 2H).

### Example 319

### 1-(4-{1-[3-(8-{4-fluoro-2-[(3R)-3-methylmorpholin-4-carbonyl]phenyl}-3-methylimidazo[1,5-a]pyridin-6-yl)azacyclobutan-1-yl]ethyl}piperidin-1-yl)propan-1-one 319

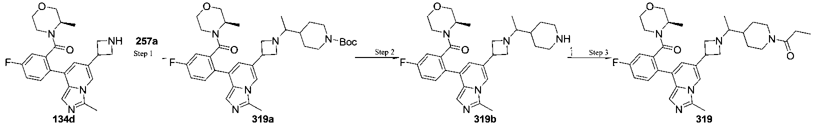

### Step 1

### 4-{1-[3-(8-{4-fluoro-2-[(3R)-3-methylmorpholin-4-carbonyl]phenyl}-3-methylimidazo[1,5-a]pyridin-6-yl)azacyclobutan-1-yl]ethyl}piperidin-1-tert-butyl carboxylate 319a

Compound **134d** (700 mg, 1.71 mmol) and Compound **257a** (467.43 mg, 2.06 mmol) were dissolved in methanol (5.0 mL), followed by the addition of zinc chloride (700.72 mg, 5.14 mmol), and the mixture was stirred at 50°C for 1.0 hour. Sodium cyanoborohydride (323.08 mg, 5.14 mmol) was added, and the mixture was stirred at 50°C for 12 hours. A saturated aqueous sodium bicarbonate solution (30 mL) was added to the reaction mixture, which was then extracted with dichloromethane (30 mL x 3). The organic phases were combined, dried over anhydrous sodium sulfate, and concentrated under a reduced pressure. The residues were purified by the silica-gel column chromatography using dichloromethane/methanol as an elution system, to obtain the title Compound **319a** (618 mg, 997.15 µmol, yield: 58.19%) as yellow solid.
MS m/z (ESI):620.4[M+1]

### Step 2

### (3R)-4-[5-fluoro-2-(3-methyl-6-{1-[1-(piperidin-4-yl)ethyl]azacyclobutan-3-yl}imidazo[1,5-a]pyridin-8-yl)benzoyl]-3-methylmorpholine 319b

Compound **319a** (150 mg, 242.03 µmol) was dissolved in dichloromethane (3.0 mL), followed by the addition of trifluoroacetic acid (1.48 g, 12.98 mmol, 1 mL) at 25°C. The mixture was stirred at room temperature to allow for reacting for 0.5 hours. The reaction mixture was concentrated under a reduced pressure, to obtain the crude product of title Compound **319b** (120 mg). The crude product was directly used in the next step of reaction without purification.
MS m/z (ESI): 520.2 [M+1]

### Step 3

### 1-(4-{1-[3-(8-{4-fluoro-2-[(3R)-3-methylmorpholin-4-carbonyl]phenyl}-3-methylimidazo[1,5-a]pyridin-6-yl)azacyclobutan-1-yl]ethyl}piperidin-1-yl)propan-1-one 319

Triethylamine (57.49 mg, 568.11 µmol) and propionic acid (16.83 mg, 227.25 µmol) were dissolved in dichloromethane (2.0 mL), and cooled to 5°C, followed by the addition of HATU (86.41 mg, 227.25 µmol), and the mixture was stirred for 10 min. The crude product of Compound **319b** (120 mg) was added, and the reaction mixture was stirred at room temperature for 12 hours. A saturated aqueous sodium bicarbonate solution (10 mL) was added to the reaction mixture, which was then extracted with dichloromethane (10 mL × 3). The organic phase was dried over anhydrous sodium sulfate, filtered and concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% ammonia water), elution gradient: 20%-50%), to obtain the title Compound **319** (59.89 mg, 104.03 µmol, yield: 43 %) as yellow solid.
MS m/z (ESI): 576.3 [M+1]
¹H NMR (400 MHz,CD₃OD) δ 8.00 - 7.89 (m, 1H), 7.84 - 7.63 (m, 1H), 7.42 - 7.06 (m, 3H), 7.05 - 6.77 (m, 1H), 4.67 - 4.30 (m, 2H), 4.08 - 3.64 (m, 5H), 3.60 - 3.49 (m, 1H), 3.27 - 2.92 (m, 5H), 2.83 - 2.49 (m, 5H), 2.45 - 2.35 (m, 3H), 1.95 - 1.52 (m, 4H), 1.36 - 1.17 (m, 3H), 1.15 - 1.06 (m, 4H), 0.92 - 0.83 (m, 3H), 0.75 - 0.40 (m, 1H).

### Examples 319-1 and 319-2

### 1-{4-[(1S)-1-[3-(8-{4-fluoro-2-[(3R)-3-methylmorpholin-4-carbonyl]phenyl}-3-methylimidazo[1,5-a]pyridin-6-yl)azacyclobutan-1-yl]ethyl]piperidin-1-yl}propan-1-one 319-1

### 1-{4-[(1R)-1-[3-(8-{4-fluoro-2-[(3R)-3-methylmorpholin-4-carbonyl]phenyl}-3-methylimidazo[1,5-a]pyridin-6-yl)azacyclobutan-1-yl]ethyl]piperidin-1-yl}propan-1-one 319-2

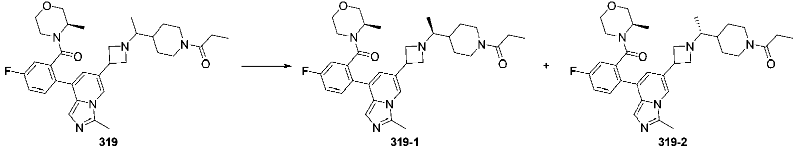

Compound **319** (167.49 mg, 290.93 µmol) was separated and purified by the chiral preparative SFC (preparative column: DAICEL CHIRALPAK AD (250 mm * 30 mm,10 um), mobile phase: CO₂-EtOH (0.1% ammonia water); elution gradient: 25%), to obtain the title Compounds **319-1** (38.96 mg, 67.67 µmol, yield: 23.26%) and **319-2** (40.33 mg, 70.05 µmol, yield: 24.08%).

### Single-configuration Compound 319-1:

MS m/z (ESI):576.3[M+1]
Chiral HPLC analysis: retention time: 1.415 min; chromatographic column: Chiralpak AD-3 50 x 4.6 mm I.D., 3 um; mobile phase: CO₂-EtOH (0.05% diethylamide): elution gradient: 5%-40%.

¹H NMR (400 MHz, DMSO-*d₆*) δ 8.03 - 7.90 (m, 1H), 7.80 - 7.59 (m, 1H), 7.48 - 7.28 (m, 2H), 7.13 - 6.58 (m, 2H), 4.53 - 3.45 (m, 8H), 3.21 - 2.71 (m, 6H), 2.64 - 2.55 (m, 3H), 2.44 - 2.25 (m, 3H), 2.24 - 2.06 (m, 1H), 1.93 - 1.37 (m, 4H), 1.26 - 0.91 (m, 7H), 0.78 - 0.34 (m, 4H).

### Single-configuration Compound 319-2:

MS m/z (ESI):576.3[M+1]
Chiral HPLC analysis: retention time: 1.708 min; chromatographic column: Chiralpak AD-3 50 x 4.6 mm I.D., 3 um; mobile phase: CO₂-EtOH (0.05% diethylamide): elution gradient: 5%-40%.

¹H NMR (400 MHz, DMSO-*d₆*) δ 8.02 - 7.89 (m, 1H), 7.81 - 7.59 (m, 1H), 7.49 - 7.25 (m, 2H), 7.14 - 6.68 (m, 2H), 4.54 - 4.35 (m, 2H), 3.94 - 3.85 (m, 2H), 3.54 - 3.48 (m, 4H), 3.14 - 2.74 (m, 6H), 2.65 - 2.56 (m, 3H), 2.44 - 2.24 (m, 3H), 2.24 - 2.05 (m, 1H), 1.86 - 1.40 (m, 4H), 1.25 - 0.92 (m, 7H), 0.83 - 0.32 (m, 4H).

### Examples 321-1 and 321-2

### (3R)-4-[5-fluoro-2-(1-fluoro-3-methyl-6-{1-[(2R)-3-methyl-1-{[(3S)-1-methylpiperidin-3-yl]oxylbutan-2-yl]azacyclobutan-3-yl}imidazo[1,5-a]pyridin-8yl)benzoyl]-3-methylmorpholine 321-1

### (3R)-4-[5-fluoro-2-(1-fluoro-3-methyl-6-{1-[(2S)-3-methyl-1-{[(3S)-1-methylpiperidin-3-yl]oxy}butan-2-yl]azacyclobutan-3-yl}imidazo[1,5-a]pyridin-8yl)benzoyl]-3-methylmorpholine 321-2

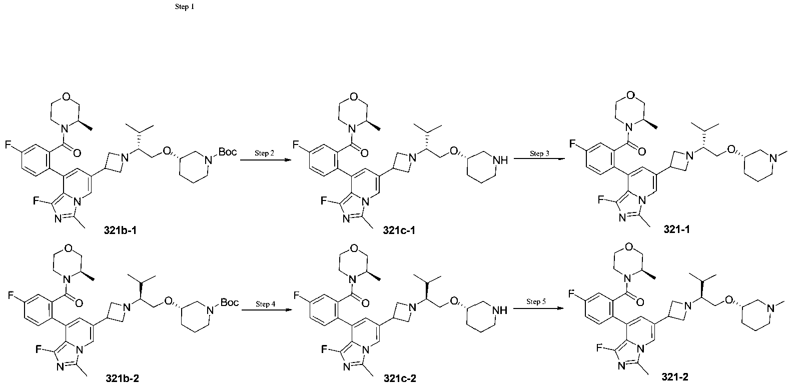

### Step 1

### (3S)-3-[(2R)-2-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-3-methylbutoxy]piperidin-1-tert-butyl carboxylate 321b-1

### (3S)-3-[(2S)-2-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-3-methylbutoxy]piperidin-1-tert-butyl carboxylate 321b-2

Compound **145d** (160 mg, 375.18 µmol) and (3S)-3-(3-methyl-2-oxobutoxy)piperidin-1-tert-butyl carboxylate **321a** (117.78 mg, 412.70 µmol, prepared according to the synthesis route of Compound **297e** by using (3*S*)-3-hydroxylpiperidin-1-tert-butyl carboxylate to replace (3*R*)-3-hydroxylpiperidin-1-tert-butyl carboxylate in the first step) were dissolved in methanol (10 mL), followed by the addition of ZnCl₂ (102.27 mg, 750.37 µmol), and the mixture was stirred at 50°C for 1.0 hour. The mixture was cooled to room temperature, followed by the addition of sodium cyanoborohydride (70.73 mg, 1.13 mmol), allowing for reacting at 50°C for 11 hours under the protection of nitrogen. A saturated aqueous ammonium chloride solution (50 mL) was added to the reaction mixture, which was then extracted with ethyl acetate (15 mL x 3). The organic phases were combined, dried over anhydrous sodium sulfate, and concentrated under a reduced pressure. The resulting residues were purified by the silica-gel column chromatography using dichloromethane/methanol as an elution system, and the resulting solid was separated and purified by the chiral preparative SFC (preparative column: Phenomenex-Cellulose-2 (250 mm * 30 mm,10 um), mobile phase: CO₂-MeOH (0.1% ammonia water); elution gradient: 25%), to obtain the title Compounds **321b-1** (55 mg, 79.04 µmol, yield: 21.07%) and **321b-2** (45 mg, 64.67 µmol, yield: 17.24%).

### Single-configuration Compound 321b-1:

MS m/z (ESI):696.3 [M+1]
Chiral HPLC analysis: retention time: 1.833 min; chromatographic column: Cellulose-2 50 x 4.6 mm I.D., 3 um; mobile phase: CO₂-MeOH (0.05% DEA): elution gradient: 5%-40%.

### Single-configuration Compound 321b-2:

MS m/z (ESI):696.2 [M+1]
Chiral HPLC analysis: retention time: 2.004 min; chromatographic column: Cellulose-2 50 x 4.6 mm I.D., 3 um; mobile phase: CO₂-MeOH (0.05% DEA): elution gradient: 5%-40%.

### Step 2

### (3R)-4-[5-fluoro-2-(1-fluoro-3-methyl-6-{1-[(2R)-3-methyl-1-[(3S)-piperidin-3-yloxy]butan-2-yl]azacyclobutan-3-yl}imidazo[1,5-a]pyridin-8yl)benzoyl]-3-methylmorpholine 321c-1

Compound **321b-1** (55 mg, 79.04 µmol) was dissolved in dichloromethane (3.0 mL), followed by the addition of trifluoroacetic acid (999.97 mg, 8.77 mmol, 675.66 µL) at 25°C. The mixture was stirred at room temperature to allow for reacting for 1.0 hours. The reaction mixture was concentrated under a reduced pressure, and then dissolved by adding DCM (10 mL), followed by adding a 10% aqueous sodium hydroxide solution (20 mL), stirring for 0.5 hours and extracting with dichloromethane (10 mL x 3). The organic phases were combined, dried over anhydrous sodium sulfate and filtered, and the filtrate was concentrated under a reduced pressure, to obtain the crude product of title Compound **321c-1** (47 mg, 78.90 µmol, yield: 99.82%) as yellow solid. The crude product was directly used in the next step of reaction without treatment.
MS m/z (ESI):596.2 [M+1]

### Step 3

### (3R)-4-[5-fluoro-2-(1-fluoro-3-methyl-6-{1-[(2R)-3-methyl-1-{[(3S)-1-methylpiperidin-3-yl]oxy}butan-2-yl]azacyclobutan-3-yl}imidazo[1,5-a]pyridin-8yl)benzoyl]-3-methylmorpholine 321-1

Compound **321c-1** (47.00 mg, 78.90 µmol) was dissolved in methanol (5.0 mL), followed by the addition of formaldehyde (64.02 mg, 788.96 µmol), and the mixture was stirred at 25°C for 1.0 hour. Sodium cyanoborohydride (14.87 mg, 236.69 µmol) was added, allowing for reacting at 25°C for 11.0 hours under the protection of nitrogen. A saturated aqueous sodium bicarbonate solution (20 mL) was added to the reaction mixture, which was then extracted with dichloromethane (10 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 46%-76%), to obtain the title Compound **321-1** (14.91 mg, 24.45 µmol, yield: 30.99%).

### Single-configuration Compound 321-1:

MS m/z (ESI):610.5 [M+1]
Chiral SFC analysis: retention time: 1.152 min; chromatographic column: Chiralcel OD-3 50×4.6 mm I.D., 3 um; mobile phase: CO₂-MeOH(0.05%DEA): elution gradient: 5%-40%.

¹H NMR (400 MHz, CDCl₃) δ 7.55 - 7.35 (m, 2H), 7.24 - 6.99 (m, 2H), 6.79 - 6.51 (m, 1H), 4.65 - 4.11 (m, 1H), 3.80 - 3.68 (m, 2H), 3.64 - 3.50 (m, 3H), 3.41 - 2.71 (m, 9H), 2.61 (s, 3H), 2.60 - 2.29 (m, 2H), 2.27 (s, 3H), 2.17 - 1.71 (m, 6H), 1.58 - 1.08 (m, 4H), 0.96 (br d, J = 6.8 Hz, 3H), 0.90 - 0.53 (m, 4H).

### Step 4

### (3R)-4-[5-fluoro-2-(1-fluoro-3-methyl-6-{1-[(2S)-3-methyl-1-[(3S)-piperidin-3-yloxy]butan-2-yl]azacyclobutan-3-yl}imidazo[1,5-a]pyridin-8yl)benzoyl]-3-methylmorpholine 321c-2

Compound **321b-2** (45.00 mg, 64.67 µmol) was dissolved in dichloromethane (3.0 mL), followed by the addition of trifluoroacetic acid (978.26 mg, 8.58 mmol, 660.99 µL) at 25°C. The mixture was stirred at room temperature to allow for reacting for 1.0 hours. The reaction mixture was concentrated under a reduced pressure, and then dissolved by adding DCM (10 mL), followed by adding a 10% aqueous sodium hydroxide solution (20 mL), stirring for 0.5 hours and extracting with dichloromethane (10 mL x 3). The organic phases were combined, dried over anhydrous sodium sulfate and filtered, and the filtrate was concentrated under a reduced pressure, to obtain the crude product of title Compound **321c-2** (38 mg, 63.79 µmol, yield: 98.64%) as yellow oil. The crude product was directly used in the next step of reaction without treatment.
MS m/z (ESI):596.3 [M+1]

### Step 5

### (3R)-4-[5-fluoro-2-(1-fluoro-3-methyl-6-{1-[(2S)-3-methyl-1-{[(3S)-1-methylpiperidin-3-yl]oxy}butan-2-yl]azacyclobutan-3-yl}imidazo[1,5-a]pyridin-8yl)benzoyl]-3-methylmorpholine 321-2

Compound **321c-2** (39.00 mg, 65.47 µmol) was dissolved in methanol (5.0 mL), followed by the addition of formaldehyde (53.13 mg, 654.67 µmol), and the mixture was stirred at 25°C for 1.0 hour. Sodium cyanoborohydride (12.34 mg, 196.40 µmol) was added, allowing for reacting at 25°C for 11.0 hours under the protection of nitrogen. A saturated aqueous ammonium chloride solution (20 mL) was added to the reaction mixture, which was then extracted with dichloromethane (10 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 46%-76%), to obtain the title Compound **321-2** (8.21 mg, 13.46 µmol, yield: 20.57%).

### Single-configuration Compound 321-2:

MS m/z (ESI):610.4 [M+1]
Chiral SFC: retention time: 1.139 min; chromatographic column: Chiralcel OD-3 50×4.6 mm I.D., 3 um; mobile phase: CO₂/MeOH (0.05% DEA): elution gradient: 5%-40%.

¹H NMR (400 MHz,CDCl₃) δ 7.55 - 7.34 (m, 2H), 7.25 - 6.99 (m, 2H), 6.80 - 6.51 (m, 1H), 4.64 - 4.09 (m, 1H), 3.78 - 3.69 (m, 2H), 3.64 - 3.50 (m, 3H), 3.45 - 2.70 (m, 9H), 2.66 - 2.30 (m, 5H), 2.27 (s, 3H), 2.16 - 1.70 (m, 6H), 1.58 -1.10 (m, 4H), 0.96 (br d, J = 6.8 Hz, 3H), 0.90 - 0.57 (m, 4H).

### Example 322

### (3R)-4-[5-fluoro-2-(1-fluoro-3-methyl-6-{1-[(2S)-3-methyl-1-{[(3S)-1-methylpyrrolidin-3-yl]oxy}butan-2-yl]azacyclobutan-3-yl}imidazo[1,5-a]pyridin-8yl)benzoyl]-3-methylmorpholine 322

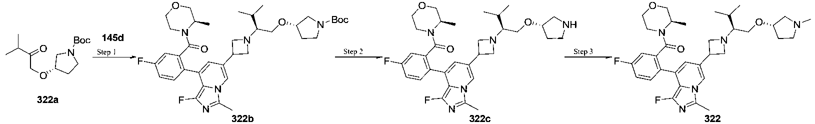

### Step 1

### (3S)-3-[(2S)-2-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-3-methylbutoxy]pyrrolidin-1-tert-butyl carboxylate 322b

Compound **145d** (80 mg, 187.59 µmol) and (3*S*)-3-(3-methyl-2-oxobutoxy)pyrrolidin-1-tert-butyl carboxylate **322a** (76.35 mg, 281.39 µmol, prepared according to the synthesis route of Compound **297e** by using (3S)-3-hydroxylpyrrolidin-1-tert-butyl carboxylate to replace (3*R*)-3-hydroxylpiperidin-1-tert-butyl carboxylate in the first step) were dissolved in methanol (2.0 mL), followed by the addition of ZnCl₂(51.14 mg, 375.18 µmol), and the mixture was stirred at 55°C for 1.0 hour. The mixture was cooled to room temperature, followed by the addition of sodium cyanoborohydride (35.37 mg, 562.78 µmol), allowing for reacting at 55°C for 11 hours under the protection of nitrogen. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **322b** (60 mg, 88.00 µmol, yield: 46.91%) as yellow oil.
MS m/z (ESI):682.2 [M+1]

### Step 2

### (3R)-4-[5-fluoro-2-(1-fluoro-3-methyl-6-{1-[(2S)-3-methyl-1-[(3S)-pyrrolidin-3-yloxy]butan-2-yl]azacyclobutan-3-yl}imidazo[1,5-a]pyridin-8yl)benzoyl]-3-methylmorpholine 322c

Compound **322b** (60 mg, 88.00 µmol) was dissolved in dichloromethane (1.5 mL), followed by the addition of trifluoroacetic acid (740.00 mg, 6.49 mmol, 0.5 mL) at 25°C. The mixture was stirred at room temperature to allow for reacting for 1.0 hours. The reaction mixture was concentrated under a reduced pressure. The crude product of title Compound **322c** (51 mg) was obtained. The crude product was directly used in the next step of reaction without treatment.
MS m/z (ESI):582.3 [M+1]

### Step 3

### (3R)-4-[5-fluoro-2-(1-fluoro-3-methyl-6-{1-[(2S)-3-methyl-1-{[(3S)-1-methylpyrrolidin-3-yl]oxy}butan-2-yl]azacyclobutan-3-yl}imidazo[1,5-a]pyridin-8yl)benzoyl]-3-methylmorpholine 322

The crude product of Compound **322c** (51 mg) was dissolved in methanol (2.0 mL), followed by the addition of triethylamine (13.31 mg, 131.51 µmol) and formaldehyde (36.56 mg, 438.37 µmol, 37%purity), and the mixture was stirred at 25°C for 1.0 hour. Sodium cyanoborohydride (16.53 mg, 263.02 µmol) was added, allowing for reacting at 25°C for 11 hours under the protection of nitrogen. A saturated aqueous sodium bicarbonate solution (10 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 40%-70%), to obtain the title Compound **322** (7 mg, 11.75 µmol, yield: 13.40%).
MS m/z (ESI):596.3 [M+1]
¹H NMR (400 MHz, DMSO-*d₆*) δ 7.95 - 7.79 (m, 1H), 7.63 - 7.23 (m, 3H), 6.81 - 6.49 (m, 1H), 4.46 - 3.41 (m, 8H), 3.24 - 2.66 (m, 6H), 2.63 - 2.57 (m, 1H), 2.55 (s, 3H), 2.48 - 2.21 (m, 5H), 2.17 (s, 3H), 2.03 - 1.88 (m, 1H), 1.78 - 1.54 (m, 2H), 1.26 - 1.04 (m, 2H), 0.89 (d, J = 6.8 Hz, 3H), 0.85 - 0.70 (m, 4H).

### Examples 323-1 and 323-2

### (3R)-4-[5-fluoro-2-(3-methyl-6-{1-[(3S)-2-methyl-6-(4-methylpiperazin-1-yl)hexan-3-yl]azacyclobutan-3-yl}imidazo[1,5-a]pyridin-8-yl)benzoyl]-3-methylmorpholine 323-1

### (3R)-4-[5-fluoro-2-(3-methyl-6-{1-[(3R)-2-methyl-6-(4-methylpiperazin-1-yl)hexan-3-yl]azacyclobutan-3-yl}imidazo[1,5-a]pyridin-8-yl)benzoyl]-3-methylmorpholine 323-2

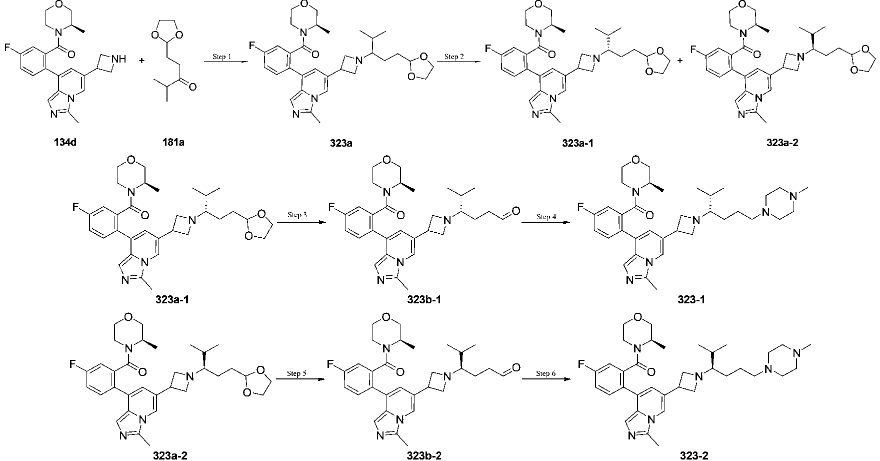

### Step 1

### (3R)-4-[2-(6-{1-[1-(1,3-dioxolan-2-yl)-4-methylpentan-3-yl]azacyclobutan-3-yl}-3-methylimidazo[1,5-a]pyridin-8-yl)-5-fluorobenzoyl]-3-methylmorpholine 323a

Compound **134d** (1.37 g, 3.35 mmol) and 1-(1,3-dioxolan-2-yl)-4-methylpentan-3-one **181a** (866.44 mg, 5.03 mmol) were dissolved in methanol (15 mL), followed by the addition of ZnCl₂ (914.27 mg, 6.71 mmol), and the mixture was stirred at 50°C for 1.0 hour. The mixture was cooled to room temperature, followed by adding sodium cyanoborohydride (632.32 mg, 10.06 mmol) and stirring at 50°C for 10 hour. A saturated aqueous sodium bicarbonate solution (10 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **323a** (1.5 g, 2.66 mmol, yield: 79.2%).
MS m/z (ESI): 565.3[M+1]

### Step 2

### (3R)-4-[2-(6-{1-[(3S)-1-(1,3-dioxolan-2-yl)-4-methylpentan-3-yl]azacyclobutan-3-yl}-3-methylimidazo[1,5-a]pyridin-8-yl)-5-fluorobenzoyl]-3-methylmorpholine 323a-1

### (3R)-4-[2-(6-{1-[(3R)-1-(1,3-dioxolan-2-yl)-4-methylpentan-3-yl]azacyclobutan-3-yl}-3-methylimidazo[1,5-a]pyridin-8-yl)-5-fluorobenzoyl]-3-methylmorpholine 323a-2

Compound **323a** (1.5 g, 2.66 mmol) was separated and purified by the chiral preparative SFC (preparative column: DAICEL CHIRALCEL OD (250 mm * 30 mm,10 um), mobile phase: carbon dioxide-[acetonitrile/ethanol (0.1% ammonia water)]; elution gradient: 20%), to obtain the title Compounds **323a-1** (700 mg, 1.24 mmol, yield: 46.67%) and **323a-2** (700 mg, 1.24 mmol, yield: 46.67%).

### Single-configuration Compound 323a-1:

MS m/z (ESI):565.3 [M+1]
Chiral HPLC analysis: retention time: 1.292 min; chromatographic column: Chiralcel OD-3 50 x 4.6mm I.D., 3 um; mobile phase: CO₂-EtOH (0.05% diethylamide): elution gradient: 5%-40%.

### Single-configuration Compound 323a-2:

MS m/z (ESI):565.3 [M+1]
Chiral HPLC analysis: retention time: 1.542 min; chromatographic column: Chiralcel OD-3 50 x 4.6mm I.D., 3 um; mobile phase: CO₂-EtOH (0.05% diethylamide): elution gradient: 5%-40%.

### Step 3

### (4S)-4-[3-(8-{4-fluoro-2-[(3R)-3-methylmorpholin-4-carbonyl]phenyl}-3-methylimidazo[1,5-a]pyridin-6-yl)azacyclobutan-1-yl]-5-methylhexanal 323b-1

Compound **323a-1** (420.00 mg, 743.77 µmol) was dissolved in acetonitrile (4.0 mL), followed by the addition of a hydrochloric acid solution (1.0 M; 4.0 mL). The mixture was stirred at 50°C to allow for reacting for 1.0 hours. The reaction mixture was concentrated under a reduced pressure, regulated to pH = 11 with a 10 % aqueous NaOH solution, and extracted with dichloromethane (20 mL x 3). The organic phases were combined, dried over anhydrous sodium sulfate, and concentrated under a reduced pressure, to obtain the crude product of title Compound **323b-1** (380 mg, 729.88 µmol, yield: 98.13%). The crude product was directly used in the next step of reaction without purification.

### Single-configuration Compound 323b-1:

MS m/z (ESI): 521.3 [M+1]

### Step 4

### (3R)-4-[5-fluoro-2-(3-methyl-6-{1-[(3S)-2-methyl-6-(4-methylpiperazin-1-yl)hexan-3-yl]azacyclobutan-3-yl}imidazo[1,5-a]pyridin-8-yl)benzoyl]-3-methylmorpholine 323-1

Compound **323b-1(190.00** mg, 364.94 µmol) and 1-methylpiperazine (73.11 mg, 729.88 µmol) were dissolved in methanol (3.0 mL), followed by the addition of acetic acid (21.92 mg, 364.94 µmol), and the mixture was stirred at 25°C for 0.5 hours. Sodium cyanoborohydride (68.80 mg, 1.09 mmol) was added, allowing for reacting 25°C for 12 hours under the protection of nitrogen. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 26%-56%), to obtain the title Compound **323-1** (54.88 mg, 90.74 µmol, yield: 24.86%).

### Single-configuration Compound 323-1:

MS m/z (ESI):605.4 [M+1]
Chiral SFC: retention time: 1.174 min; chromatographic column: Chiralcel OD-3 50×4.6 mm I.D., 3um,; mobile phase: CO₂/MeOH (0.05% DEA): elution gradient: 5%-40%.

¹H NMR (400 MHz, DMSO-*d₆*) δ 8.04 - 7.87 (m, 1H), 7.81 - 7.59 (m, 1H), 7.49 - 7.22 (m, 2H), 7.19 - 6.92 (m, 1H), 6.91 - 6.66 (m, 1H), 4.39 - 3.84 (m, 1H), 3.80 - 3.40 (m, 5H), 3.28 - 2.67 (m, 6H), 2.64 - 2.56 (m, 3H), 2.41 - 2.14 (m, 9H), 2.13 - 2.07 (m, 3H), 2.06 - 1.99 (m, 1H), 1.96 - 1.59 (m, 2H), 1.51 - 1.37 (m, 2H), 1.30 - 1.00 (m, 4H), 0.88 - 0.77 (m, 6H), 0.70 - 0.35 (m, 1H).

### Step 5

### (4R)-4-[3-(8-{4-fluoro-2-[(3R)-3-methylmorpholin-4-carbonyl]phenyl}-3-methylimidazo[1,5-a]pyridin-6-yl)azacyclobutan-1-yl]-5-methylhexanal 323b-2

Compound **323a-2** (400.00 mg, 708.35 µmol) was dissolved in acetonitrile (4.0 mL), followed by the addition of a hydrochloric acid solution (1.0 M; 4.0 mL). The mixture was stirred at 50°C to allow for reacting for 1.0 hours. The reaction mixture was concentrated under a reduced pressure, regulated to pH = 11 with a 10 % aqueous NaOH solution, and extracted with dichloromethane (20 mL x 3). The organic phases were combined, dried over anhydrous sodium sulfate, and concentrated under a reduced pressure, to obtain the crude product of title Compound **323b-2** (360 mg, 691.46 µmol, yield: 97.62%). The crude product was directly used in the next step of reaction without purification.

### Single-configuration Compound 323b-2:

MS m/z (ESI): 521.3 [M+1]

### Step 6

### (3R)-4-[5-fluoro-2-(3-methyl-6-{1-[(3R)-2-methyl-6-(4-methylpiperazin-1-yl)hexan-3-yl]azacyclobutan-3-yl}imidazo[1,5-a]pyridin-8-yl)benzoyl]-3-methylmorpholine 323-1

The crude product of Compound **323b-2** (180.00 mg, 345.73 µmol) and 1-methylpiperazine (69.26 mg, 691.46 µmol) were dissolved in methanol (2.0 mL), followed by the addition of acetic acid (20.76 mg, 345.73 µmol), and the mixture was stirred at 25°C for 1.0 hour. Sodium cyanoborohydride (65.18 mg, 1.04 mmol) was added, allowing for reacting 25°C for 9.0 hours under the protection of nitrogen. A saturated aqueous sodium bicarbonate solution (10 mL) was added to the reaction mixture, which was then extracted with dichloromethane (20 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 26%-56%), to obtain the title Compound **323-2** (75 mg, 124.01 µmol, yield: 35.87%) as yellow solid.

### Single-configuration Compound 323-2:

MS m/z (ESI):605.6 [M+1]
Chiral SFC: retention time: 1.178 min; chromatographic column: Chiralcel OD-3 50×4.6 mm I.D., 3 um; mobile phase: CO₂/MeOH (0.05% DEA): elution gradient: 5%-40%.

¹H NMR (400 MHz, DMSO-*d₆*) δ 8.10 - 7.83 (m, 1H), 7.82 - 7.58 (m, 1H), 7.54 - 7.20 (m, 2H), 7.18 - 6.57 (m, 2H), 4.39 - 3.38 (m, 6H), 3.29 - 2.54 (m, 9H), 2.40 - 2.01 (m, 13H), 1.90 - 0.99 (m, 8H), 0.93 - 0.77 (m, 6H), 0.70 - 0.38 (m, 1H).

### Example 330

### 1-{(2R)-4-[(4R)-4-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-5-methylhexyl]-2-methylpiperazin-1-yl}ethan-1-one 330

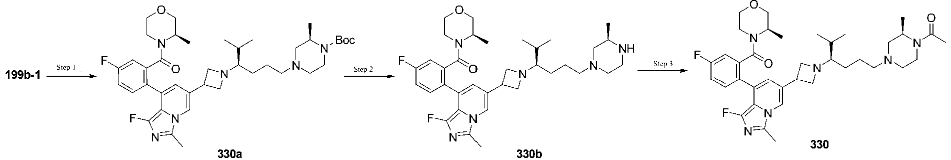

### Step 1

### (2R)-4-[(4R)-4-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-5-methylhexyl]-2-methylpiperazin-1-tert-butyl carboxylate 330a

Compound **199b-1** (150 mg, 278.49 µmol) and (2R)-2-methylpiperazin-1-tert-butyl carboxylate (66.93 mg, 334.18 µmol) were dissolved in methanol (5.0 mL), followed by the addition of acetic acid (1.67 mg, 27.85 µmol), and the mixture was stirred at room temperature for 1.0 hour under the protection of nitrogen. Sodium cyanoborohydride (52.50 mg, 835.46 µmol) was added, allowing for reacting at room temperature for 11 hours under the protection of nitrogen. A saturated aqueous ammonium chloride solution (50 mL) was added to the reaction mixture, which was then extracted with ethyl acetate (50 mL x 3). The organic phases were combined, dried over anhydrous sodium sulfate, and concentrated under a reduced pressure. The resulting residues were purified by the preparative thin-layer chromatography using dichloromethane/methanol as an elution system, to obtain the title product **330a** (156 mg, 215.80 µmol, yield: 77.49%) as yellow oil.
MS m/z (ESI):723.4 [M+1]

### Step 2

### (3R)-4-[5-fluoro-2-(1-fluoro-3-methyl-6-{1-[(3R)-2-methyl-6-[(3R)-3-methylpiperazin-1-yl]hexan-3-yl]azacyclobutan-3-yl}imidazo[1,5-a]pyridin-8-yl)benzoyl]-3-methylmorpholine 330b

Compound **330a** (156 mg, 215.80 µmol) was dissolved in dichloromethane (3.0 mL), followed by the addition of trifluoroacetic acid (1.48 g, 12.98 mmol, 1 mL) at 25°C. The mixture was stirred at room temperature to allow for reacting for 3.0 hours. The reaction mixture was concentrated under a reduced pressure, and then dissolved by adding DCM (10 mL), followed by adding a 10% aqueous sodium hydroxide solution (20 mL), stirring for 0.5 hours and extracting with dichloromethane (10 mL x 3). The organic phases were combined and washed once with saturated brine (50 mL), the organic phase was dried over anhydrous sodium sulfate and filtered, and the filtrate was concentrated under a reduced pressure, to obtain the crude product of title Compound **330b** (134 mg, 215.16 µmol, yield: 99.71%). The crude product was directly used in the next step of reaction without purification.
MS m/z (ESI): 623.3 [M+1]

### Step 3

### 1-{(2R)-4-[(4R)-4-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-5-methylhexyl]-2-methylpiperazin-1-yl}ethan-1-one 330

Triethylamine (65.32 mg, 645.48 µmol) and Compound **330b** (134 mg, 215.16 µmol) were dissolved in dichloromethane (5.0 mL), and cooled to 5°C, followed by the addition of acetic anhydride (26.36 mg, 258.19 µmol). The reaction mixture was stirred at room temperature for 12 hours. Water (30 mL) was added to the reaction mixture, which was then extracted with dichloromethane (50 mL×3). The organic phase was dried over anhydrous sodium sulfate, filtered and concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% ammonia water), elution gradient:34%-64%). The title Compound 330 (64.96 mg, 97.71 µmol, yield: 45.41%) was obtained as yellow solid.
MS m/z (ESI): 665.4 [M+1]
¹H NMR (400 MHz, CDCl₃) δ 7.56 - 7.35 (m, 2H), 7.25 - 7.06 (m, 2H), 6.83 - 6.56 (m, 1H), 4.79 - 4.10 (m, 2H), 3.99 - 3.22 (m, 8H), 3.13 (br s, 2H), 3.02 - 2.65 (m, 4H), 2.61 (s, 3H), 2.38 - 2.17 (m, 2H), 2.11 - 2.04 (m, 4H), 2.03 - 1.77 (m, 3H), 1.71 (br s, 2H), 1.56 - 1.38 (m, 3H), 1.34 - 1.22 (m, 4H), 1.20 - 1.06 (m, 1H), 0.97 - 0.87 (m, 6H), 0.84 - 0.57 (m, 1H).

### Example 332

### 1-[(1R,4R)-5-[(4R)-4-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-5-methylhexyl]-2,5-diazabicyclo[2.2.1]heptan-2-yl]ethan-1-one 332

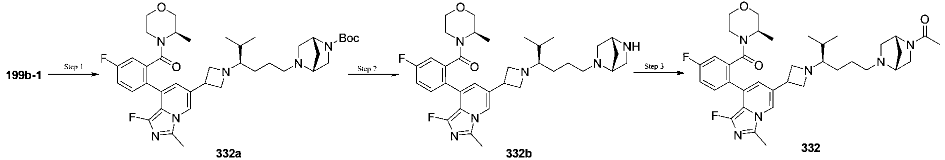

### Step 1

### (1R,4R)-5-[(4R)-4-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-5-methylhexyl]-2,5-diazadicyclo[2.2.1]heptan-2-tert-butyl carboxylate 332a

Compound **199b-1** (180 mg, 334.18 µmol) and (1R,4R)-2,5-diazabicyclo[2.2.1]heptan-2-tert-butyl carboxylate (79.51 mg, 401.02 µmol) were dissolved in methanol (4.0 mL), followed by the addition of acetic acid (20.07 mg, 334.18 µmol), and the mixture was stirred at room temperature for 0.5 hours under the protection of nitrogen. Sodium cyanoborohydride (63.00 mg, 1.00 mmol) was added, allowing for reacting at room temperature for 12 hours under the protection of nitrogen. A saturated aqueous sodium bicarbonate solution (15 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title product **332a** (150 mg, 208.08 µmol, yield: 62.26%) as yellow solid.
MS m/z (ESI):721.5 [M+1]

### Step 2

### (1R,4R)-2-[(4R)-4-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-5-methylhexyl]-2,5-diazabicyclo[2.2.1]heptane 332b

Compound **332a** (150 mg, 208.08 µmol) was dissolved in dichloromethane (3.0 mL), followed by the addition of trifluoroacetic acid (1.48 g, 12.98 mmol, 1 mL) at 25°C. The mixture was stirred at room temperature to allow for reacting for 0.5 hours. The reaction mixture was concentrated under a reduced pressure, and then dissolved by adding DCM (10 mL), followed by adding a 10% aqueous sodium hydroxide solution (20 mL), stirring for 0.5 hours and extracting with dichloromethane (10 mL x 3). The organic phases were combined, dried over anhydrous sodium sulfate and filtered, and the filtrate was concentrated under a reduced pressure, to obtain the crude product of title Compound **332b** (120 mg, 193.31 µmol, yield: 92.90%) as yellow solid. The crude product was directly used in the next step of reaction without purification.
MS m/z (ESI): 621.4 [M+1]

### Step 3

### 1-[(1R,4R)-5-[(4R)-4-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-5-methylhexyl]-2,5-diazabicyclo[2.2.1]heptan-2-yl]ethan-1-one 332

Triethylamine (58.68 mg, 579.92 µmol) and Compound **332b** (120 mg, 193.31 µmol) were dissolved in dichloromethane (2.0 mL), and cooled to 5°C, followed by the addition of acetic anhydride (39.47 mg, 386.61 µmol). The reaction mixture was stirred at room temperature for 1.0 hours. A saturated aqueous sodium bicarbonate solution (15 mL) was added to the reaction mixture, which was then extracted with dichloromethane (15 mL × 3). The organic phase was dried over anhydrous sodium sulfate, filtered and concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% ammonia water), elution gradient:27%-57%). The title Compound **332** (27.93 mg, 42.14 µmol, yield: 21.80%) was obtained as yellow solid.
MS m/z (ESI): 663.4 [M+1]
¹H NMR (400 MHz, DMSO-*d₆*) δ 7.93 - 7.74 (m, 1H), 7.70 - 7.11 (m, 3H), 6.90 - 6.34 (m, 1H), 4.45 - 3.88 (m, 2H), 3.77 - 3.38 (m, 7H), 3.25 - 2.65 (m, 8H), 2.59 - 2.52 (m, 3H), 2.45 - 2.31 (m, 3H), 2.05 - 1.80 (m, 4H), 1.76 - 1.49 (m, 3H), 1.44 - 1.05 (m, 6H), 0.90 - 0.52 (m, 7H).

### Example 348

### 1-{8-[(4R)-4-[3-(8-{4-fluoro-2-[(3R)-3-methylmorpholin-4-carbonyl]phenyl}-3-methylimidazo[1,5-a]pyridin-6-yl)azacyclobutan-1-yl]-5-methylhexyl]-3,8-diazabicyclo[3.2.1]oct-3-yl}ethan-1-one 348

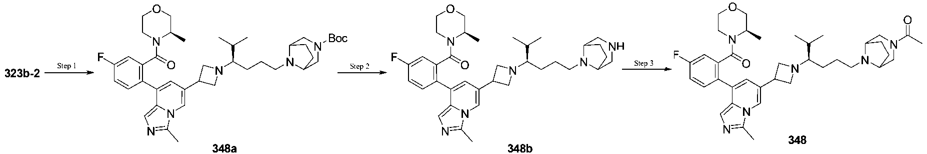

### Step 1

### 8-[(4R)-4-[3-(8-{4-fluoro-2-[(3R)-3-methylmorpholin-4-carbonyl]phenyl}-3-methylimidazo[1,5-a]pyridin-6-yl)azacyclobutan-1-yl]-5-methylhexyl]-3,8-diazabicyclo[3.2.1]octan-3-tert-butyl carboxylate 348a

Compound **323b-2** (130 mg, 249.69 µmol) and 3,8-diazabicyclo[3.2.1]octan-3-tert-butyl carboxylate (106.01 mg, 499.39 µmol) were dissolved in methanol (3.0 mL), followed by the addition of acetic acid (14.99 mg, 249.69 µmol), and the mixture was stirred at room temperature for 0.5 hours under the protection of nitrogen. Sodium cyanoborohydride (47.07 mg, 749.08 µmol) was added, allowing for reacting at room temperature for 12 hours under the protection of nitrogen. A saturated aqueous sodium bicarbonate solution (15 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title product **348a** (150 mg, 209.23 µmol, yield: 83.79%) as yellow solid.
MS m/z (ESI):717.2 [M+1]

### Step 2

### 8-[(4R)-4-[3-(8-{4-fluoro-2-[(3R)-3-methylmorpholin-4-carbonyl]phenyl}-3-methylimidazo[1,5-a]pyridin-6-yl)azacyclobutan-1-yl]-5-methylhexyl]-3,8-diazabicyclo[3.2.1]octane 348b

Compound **348a** (150 mg, 209.23 µmol) was dissolved in dichloromethane (3.0 mL), followed by the addition of trifluoroacetic acid (1.48 g, 12.98 mmol, 1 mL) at 25°C. The mixture was stirred at room temperature to allow for reacting for 0.5 hours. The reaction mixture was concentrated under a reduced pressure, and then dissolved by adding DCM (10 mL), followed by adding a 10% aqueous sodium hydroxide solution (15 mL), stirring for 0.5 hours and extracting with dichloromethane (10 mL x 3). The organic phases were combined, dried over anhydrous sodium sulfate and filtered, and the filtrate was concentrated under a reduced pressure, to obtain the crude product of title Compound **348b** (120 mg, 194.55 µmol, yield: 92.98%). The crude product was directly used in the next step of reaction without purification.
MS m/z (ESI): 523.1 [M+1]

### Step 3

### 1-{8-[(4R)-4-[3-(8-{4-fluoro-2-[(3R)-3-methylmorpholin-4-carbonyl]phenyl}-3-methylimidazo[1,5-a]pyridin-6-yl)azacyclobutan-1-yl]-5-methylhexyl]-3,8-diazabicyclo[3.2.1]oct-3-yl}ethan-1-one 348

Triethylamine (59.06 mg, 583.65 µmol) and Compound **348b** (120 mg, 194.55 µmol) were dissolved in dichloromethane (3.0 mL), and cooled to 5°C, followed by the addition of acetic anhydride (39.72 mg, 389.10 µmol). The reaction mixture was stirred at room temperature for 1.0 hours. A saturated aqueous sodium bicarbonate solution (10 mL) was added to the reaction mixture, which was then extracted with dichloromethane (10 mL × 3). The organic phase was dried over anhydrous sodium sulfate, filtered and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient:26%-56%). The title Compound **348** (46.78 mg, 71.00 µmol, yield: 36.50%) was obtained as yellow solid.
MS m/z (ESI): 659.4 [M+1]
¹H NMR (400 MHz, DMSO-*d₆*) δ 8.08 - 7.83 (m, 1H), 7.81 - 7.60 (m, 1H), 7.49 - 7.26 (m, 2H), 7.12 - 6.93 (m, 1H), 6.91 - 6.60 (m, 1H), 4.37 - 3.83 (m, 2H), 3.73 - 3.35 (m, 6H), 3.24 - 2.62 (m, 10H), 2.62 - 2.58 (m, 3H), 2.29 - 2.18 (m, 2H), 2.10 - 2.01 (m, 1H), 1.96 - 1.88 (m, 3H), 1.82 - 1.65 (m, 3H), 1.52 - 1.38 (m, 3H), 1.35 - 1.20 (m, 3H), 1.19 - 1.12 (m, 1H), 1.08 - 0.98 (m, 1H), 0.90 - 0.79 (m, 6H), 0.70 - 0.37 (m, 1H).

### Example 351

### (3R)-4-[5-fluoro-2-(1-fluoro-3-methyl-6-{1-[(3R)-2-methyl-6-{4H,5H,6H,7H-pyrazolo[1,5-a]pyrazin-5-yl}hexan-3-yl]azacyclobutan-3-yl}imidazo[1,5-a]pyridin-8-yl)benzoyl]-3-methylmorpholine 351

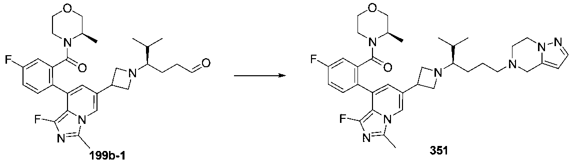

Compound **199b-1** (100 mg, 185.66 µmol) and 4*H*,5*H*,6*H*,7*H*-pyrazolo[1,5-a]pyrazine (59.27 mg, 371.31 µmol, hydrochloride) were dissolved in methanol (2.0 mL), followed by the addition of triethylamine (70 mg, 691.77 µmol), and the mixture was stirred at room temperature for 1.0 hour under the protection of nitrogen. Sodium cyanoborohydride (35.00 mg, 556.97 µmol) was added, allowing for reacting at room temperature for 8.0 hours under the protection of nitrogen. A saturated aqueous sodium bicarbonate solution (10 mL) was added to the reaction mixture, which was then extracted with dichloromethane (15 mL x 3). The organic phases were combined, dried over anhydrous sodium sulfate, and concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% ammonia water), elution gradient:36%-66%). The title Compound **351** (21.18 mg, 32.80 µmol, yield: 17.67%) was obtained as yellow solid.
MS m/z (ESI): 646.4 [M+1]
¹H NMR (400 MHz, DMSO-*d₆*) δ 8.01 - 7.75 (m, 1H), 7.71 - 6.33 (m, 5H), 5.97 (s, 1H), 4.41 - 3.36 (m, 11H), 3.27 - 2.52 (m, 10H), 2.49 - 2.42 (m, 2H), 2.11 - 2.01 (m, 1H), 1.78 - 1.04 (m, 7H), 0.91 - 0.55 (m, 7H).

### Example 353

### (1R,4R)-5-[(4R)-4-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-5-methylhexyl]-2-oxa-5-azabicyclo[2.2.1]heptane 353

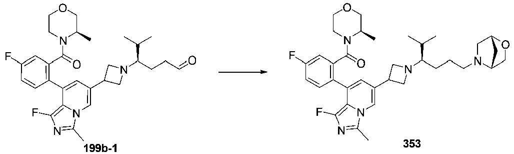

Compound **199b-1** (110 mg, 204.22 µmol) and (1*R*,4*R*)-2-oxa-5-azabicyclo[2.2.1]heptane (55.38 mg, 408.45 µmol, hydrochloride) were dissolved in methanol (3.0 mL), followed by the addition of triethylamine (62.00 mg, 612.67 µmol), and the mixture was stirred at room temperature for 1.0 hour under the protection of nitrogen. Sodium cyanoborohydride (38.50 mg, 612.67 µmol) was added, allowing for reacting at room temperature for 11 hours under the protection of nitrogen. A saturated aqueous sodium bicarbonate solution (15 mL) was added to the reaction mixture, which was then extracted with dichloromethane (15 mL x 3). The organic phases were combined, dried over anhydrous sodium sulfate, and concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% ammonia water), elution gradient:35%-65%). The title Compound **353** (52.61 mg, 84.61 µmol, yield: 41.43%) was obtained as yellow solid.
MS m/z (ESI): 622.4 [M+1]
¹H NMR (400 MHz, DMSO-*d₆*) δ 7.97 - 7.77 (m, 1H), 7.64 - 7.21 (m, 3H), 6.84 - 6.43 (m, 1H), 4.44 - 3.33 (m, 11H), 3.27 - 2.60 (m, 6H), 2.55 (s, 3H), 2.48 - 2.29 (m, 3H), 2.06 - 1.99 (m, 1H), 1.75 - 1.63 (m, 2H), 1.55 - 1.47 (m, 1H), 1.45 - 1.04 (m, 6H), 0.88 - 0.57 (m, 7H).

### Examples 357-1 and 357-2

### 1-{4-[(1S)-1-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-2-methylpropyl]piperidin-1-yl}ethan-1-one 357-1

### 1-{4-[(1R)-1-[3-(1-ffuoro-8-{4-fluoro-2-[(3R)-3-methylmorpholine-4-carbonyl]phenyl}-3-methylimidazo[1,5-a]pyridin-6-yl)azetidin-1-yl]-2-methylpropyl]piperidin-1-yl} ethan-1-one

### 1-{4-[(1R)-1-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-2-methylpropyl]piperidin-1-yl}ethan-1-one 357-2

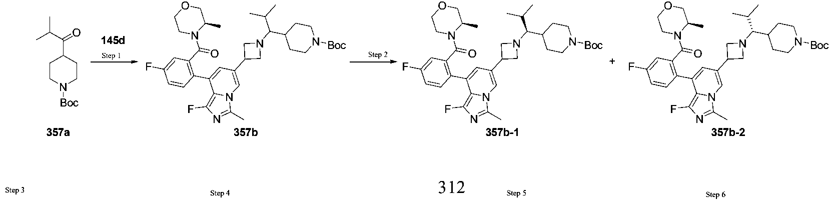

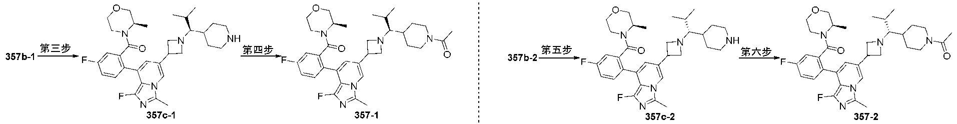

### Step 1

### 4-{1-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-2-methylpropyl}piperidin-1-tert-butyl carboxylate 357b

Compound **145d** (526 mg, 1.23 mmol) and 4-(2-methylpropionyl)piperidin-1-tert-butyl formate **357a** (450 mg, 1.76 mmol) were dissolved in methanol (5.0 mL), followed by the addition of zinc chloride (336.22 mg, 2.47 mmol), and the mixture was stirred at 50°C for 1.0 hour. Sodium cyanoborohydride (232.53 mg, 3.70 mmol) was added, and the mixture was stirred at 50°C for 12 hours. In view of a large amount of leftover raw materials, 4- (2-methylpropionyl)piperidin-1-tert-butyl formate **357a** (450 mg, 1.76 mmol) was supplemented and dissolved in methanol (5.0 mL), zinc chloride (336.22 mg, 2.47 mmol) was added, and the mixture was stirred at 50°C for 1.0 hour. Sodium cyanoborohydride (232.53 mg, 3.70 mmol) was added, and the mixture was stirred at 50°C for 12 hours. A saturated aqueous sodium bicarbonate solution (15 mL) was added to the reaction mixture, which was then extracted with dichloromethane (15 mL x 3). The organic phases were combined, dried over anhydrous sodium sulfate, and concentrated under a reduced pressure. The resulting residues were purified by the preparative thin-layer chromatography using dichloromethane/methanol as an elution system, to obtain the title Compound **357b** (500 mg, 750.96 µmol, yield: 60.88%) as yellow oil.
MS m/z (ESI):666.4[M+1]

### Step 2

### 4-[(1S)-1-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-2-methylpropyl]piperidin-1-tert-butyl carboxylate 357b-1

### 4-[(1R)-1-[3-(1-ffuoro-8-{4-fluoro-2-[(3R)-3-methylmorpholin-4-carbonyl]phenyl}-3-methylimidazo[1,5-a]pyridin-6-yl)azacyclobutan-1-yl]-2-methylpropyl]piperidin-1-tert-butyl carboxylate 357b-2

Compound **357b** (500 mg, 750.96 µmol) was separated and purified by the chiral preparative SFC (preparative column: DAICEL DAICEL ChiralPak AD, (250*30 mm, 10 um), mobile phase: carbon dioxide-ethanol (0.1% ammonia water); elution gradient: 25%), to obtain the title Compounds **357b-1** (200 mg, 300.39 µmol, yield: 40%) and **357b-2** (190 mg, 285.37 µmol, yield: 38.0%).

### Single-configuration Compound 357b-1:

MS m/z (ESI):666.4[M+1]
Chiral HPLC analysis: retention time: 1.086 min; chromatographic column: Chiralpak AD-3 50 x 4.6 mm I.D., 3 um; mobile phase: carbon dioxide-EtOH (0.05% isopropylamine): elution gradient: 5%-40%.

### Single-configuration Compound 357b-2:

MS m/z (ESI):666.5[M+1]
Chiral HPLC analysis: retention time: 1.446 min; chromatographic column: Chiralpak AD-3 50 x 4.6 mm I.D., 3 um; mobile phase: carbon dioxide-EtOH (0.05% isopropylamine): elution gradient: 5%-40%.

### Step 3

### (3R)-4-[5-fluoro-2-(1-fluoro-3-methyl-6-{1-[(1S)-2-methyl-1-(piperidin-4-yl)propyl]azacyclobutan-3-yl}imidazo[1,5-a]pyridin-8-yl)benzoyl]-3-methylmorpholine 357c-1

Compound **357b-1** (200 mg, 300.39 µmol) was dissolved in dichloromethane (3.0 mL), followed by the addition of trifluoroacetic acid (1.48 g, 12.98 mmol, 1 mL) at 25°C. The mixture was stirred at room temperature to allow for reacting for 0.5 hours. The reaction mixture was concentrated under a reduced pressure, dissolved by adding DCM (3.0 mL), regulated to PH = 10 by adding a 10% aqueous sodium hydroxide solution, and extracted with dichloromethane (15 mL x 3). The organic phases were combined, dried over anhydrous sodium sulfate and filtered, and the filtrate was concentrated under a reduced pressure, to obtain the crude product of title Compound **357c-1** (160 mg, 282.84 µmol, yield: 94.16%) as yellow solid. The crude product was directly used in the next step of reaction without purification.
MS m/z (ESI): 566.3 [M+1]

### Step 4

### 1-{4-[(1S)-1-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-2-methylpropyl]piperidin-1-yl}ethan-1-one 357-1

Triethylamine (85.86 mg, 848.51 µmol) and Compound **357c-1** (160 mg, 282.84 µmol) were dissolved in dichloromethane (3.0 mL), and cooled to 5°C, followed by the addition of acetic anhydride (57.75 mg, 565.68 µmol). The reaction mixture was stirred at room temperature for 1.0 hours. A saturated aqueous sodium bicarbonate solution (10 mL) was added to the reaction mixture, which was then extracted with dichloromethane (10 mL × 3). The organic phase was dried over anhydrous sodium sulfate, filtered and concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% ammonia water), elution gradient: 32%-62%), to obtain the title Compound **357-1** (55.00 mg, 90.50 µmol, yield: 32.00%) as yellow solid.
MS m/z (ESI): 608.3 [M+1]
Chiral SFC analysis: retention time: 1.275 min; chromatographic column: Chiralpak OD-3 50 x 4.6mm I.D., 3 um; mobile phase: carbon dioxide-methanol (0.05% isopropylamine): elution gradient: 5%-40%.

¹H NMR (400 MHz, DMSO-*d₆*) δ 8.11 - 7.65 (m, 1H), 7.62 - 7.23 (m, 3H), 6.96 - 6.32 (m, 1H), 4.49 - 3.89 (m, 2H), 3.85 - 3.41 (m, 6H), 3.21 - 2.65 (m, 6H), 2.59 - 2.51 (m, 3H), 2.46 - 2.31 (m, 2H), 2.02 - 1.87 (m, 4H), 1.81 - 1.69 (m, 1H), 1.68 - 1.48 (m, 3H), 1.37 - 1.02 (m, 4H), 0.94 - 0.60 (m, 7H).

### Step 5

### (3R)-4-[5-fluoro-2-(1-fluoro-3-methyl-6-{1-[(1R)-2-methyl-1-(piperidin-4-yl)propyl]azacyclobutan-3-yl}imidazo[1,5-a]pyridin-8-yl)benzoyl]-3-methylmorpholine 357c-2

Compound **357b-2** (190 mg, 285.37 µmol) was dissolved in dichloromethane (3.0 mL), followed by the addition of trifluoroacetic acid (1.48 g, 12.98 mmol, 1 mL) at 25°C. The mixture was stirred at room temperature to allow for reacting for 1.0 hours. The reaction mixture was concentrated under a reduced pressure, dissolved by adding DCM (10.0 mL), regulated to PH = 10 by adding a 10% aqueous sodium hydroxide solution, and extracted with dichloromethane (10 mL x 3). The organic phases were combined, dried over anhydrous sodium sulfate and filtered, and the filtrate was concentrated under a reduced pressure, to obtain the crude product of title Compound **357c-2** (160 mg, 282.84 µmol, yield: 99.11%) as yellow oil. The crude product was directly used in the next step of reaction without purification.
MS m/z (ESI): 566.3 [M+1]

### Step 6

### 1-{4-[(1R)-1-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-2-methylpropyl]piperidin-1-yl}ethan-1-one 357-2

Triethylamine (143.10 mg, 1.41 mmol) and Compound **357c-2**(160 mg, 282.84 µmol) were dissolved in dichloromethane (3.0 mL), and cooled to 5°C, followed by the addition of acetic anhydride (57.75 mg, 565.68 µmol). The reaction mixture was stirred at room temperature for 12 hours. A saturated aqueous sodium bicarbonate solution (15 mL) was added to the reaction mixture, which was then extracted with dichloromethane (15 mL × 3). The organic phase was dried over anhydrous sodium sulfate, filtered and concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% ammonia water), elution gradient: 32%-62%), to obtain the title Compound **357-2** (39.59 mg, 65.14 µmol, yield: 23.03 %) as yellow solid.
MS m/z (ESI): 608.4 [M+1]
Chiral SFC analysis: retention time: 1.273 min; chromatographic column: Chiralpak OD-3 50 x 4.6mm I.D., 3 um; mobile phase: carbon dioxide-methanol (0.05% isopropylamine): elution gradient: 5%-40%.

¹H NMR (400 MHz, DMSO-*d₆*) δ 7.98 - 7.75 (m, 1H), 7.61 - 7.25 (m, 3H), 6.85 - 6.52 (m, 1H), 4.47 - 3.46 (m, 8H), 3.11 (s, 3H), 3.01 - 2.77 (m, 3H), 2.55 (s, 3H), 2.45 - 2.31 (m, 2H), 2.01 - 1.89 (m, 4H), 1.82 - 1.71 (m, 1H), 1.68 - 1.50 (m, 3H), 1.42 - 1.06 (m, 4H), 0.92 - 0.71 (m, 7H).

### Examples 360-1 and 360-2

### N-ethyl-5-fluoro-2-(3-methyl-6-{1-[(3R)-2-methyl-6-(4-methylpiperazin-1-yl)hexan-3-yl]azacyclobutan-3-yl}-[1,2,4]triazolo[4,3-a]pyridin-8-yl)-N-(isopropyl)benzamide 360-1

### N-ethyl-5-fluoro-2-(3-methyl-6-{1-[(3S)-2-methyl-6-(4-methylpiperazin-1-yl)hexan-3-yl]azacyclobutan-3-yl}-[1,2,4]triazolo[4,3-a]pyridin-8-yl)-N-(isopropyl)benzamide 360-2

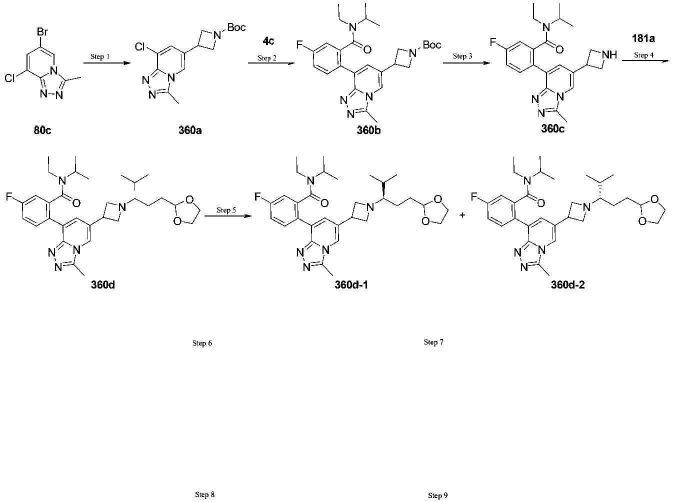

### Step 1

### 3-{8-chloro-3-methyl-[1,2,4]triazolo[4,3-a]pyridin-6-yl}azacyclobutan-1-tert-butyl carboxylate 360a

Under the protection of nitrogen, 1,2-dibromoethane (184.47 mg, 981.97 µmol) was dissolved in DMAc (20 mL), followed by the addition of zinc powder (600.53 mg, 9.18 mmol). The mixture was stirred at 70°C for 10 min, and then cooled to room temperature, followed by the slow dropwise addition of TMSCl (76.75 mg, 706.45 µmol) to the reaction mixture, and after the dropwise addition, the reaction mixture was further stirred at 25° for 30 min. Then, the solution of 3-iodoazacyclobutan-1-tert-butyl carboxylate (2 g, 7.06 mmol) in DMAc (10 mL) was dropwise added to the reaction mixture. The reaction mixture was warmed to 40°C and stirred for 1.0 hours, until the disappearance of noticeable solid precipitates in the mixture. The reaction mixture was cooled to 25°C, followed by the addition of Compound **80c** (1.39 g, 5.62 mmol), Pd₂(dba)₃ (406.21 mg, 706.45 µmol) and tri(2-furyl)phosphine (164.02 mg, 706.45 µmol) to the reaction, and the reaction mixture was then subjected to nitrogen substitution three times, warmed to 70°C and stirred to allow for reacting for 12 hours. The reaction mixture was cooled to room temperature, followed by adding water (150 mL) and extracting with ethyl acetate (100 mL x 3). The organic phase was washed with water (100 mL × 2) and saturated brine (100 mL), dried over anhydrous sodium sulfate, filtered and concentrated under a reduced pressure. The resulting residues were purified by the silica-gel column chromatography using petroleum ether/ethyl acetate as an elution system, to obtain the title Compound **360a** (760 mg, 2.35 mmol, yield: 33.33%) as white solid.
MS m/z (ESI): 323.4 [M+1]
¹H NMR (400 MHz, CDCl₃) δ 7.66 (s, 1H), 7.41 (s, 1H), 4.40 (t, J = 8.8 Hz, 2H), 3.96 (dd, J = 5.2, 8.4 Hz, 2H), 3.77 - 3.68 (m, 1H), 2.77 (s, 3H), 1.49 (s, 9H).

### Step 2

### 3-(8-{2-[ethyl(isopropyl)aminoformyl]-4-fluorophenyl}-3-methyl-[1,2,4]triazolo[4,3-a]pyridin-6-yl)azacyclobutan-1-tert-butyl carboxylate 360b

Compound **360a** (760 mg, 2.35 mmol), Compound **4c** (1.46 g, 2.83 mmol) and potassium phosphate (1.50 g, 7.06 mmol) were dissolved in dioxane (20 mL) and water (4.0 mL). Pd(dtbpf)Cl₂ (153.45 mg, 235.45 µmol) was added. Under a nitrogen atmosphere, the mixture was stirred at 95°Cto allow for reacting for 2.0 hours. The reaction mixture was cooled to room temperature, water (100 mL) was added, and the mixture was extracted with ethyl acetate (100 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **360b** (781 mg, 1.58 mmol, yield: 66.93%).
MS m/z (ESI): 496.6 [M+1]

### Step 3

### 2-[6-(azacyclobutan-3-yl)-3-methyl-[1,2,4]triazolo[4,3-a]pyridin-8-yl]-N-ethyl-5-fluoro-N-(isopropyl)benzamide 360c

Compound **360b** (781 mg, 1.58 mmol) were dissolved in methane dioxide (3.0 mL), followed by the addition of trifluoroacetic acid (1.48 g, 12.98 mmol, 1 mL) at 0°C. The mixture was stirred at room temperature to allow for reacting for 1.0 hours. The reaction mixture was concentrated under a reduced pressure, followed by the adding of dichloromethane (30 mL), regulating to PH > 7 with a 10% aqueous sodium hydroxide solution (40 mL) and extracting for separation, and the aqueous phase was extracted with dichloromethane (10 mL x 2). The organic phases were combined, dried over anhydrous sodium sulfate, and concentrated under a reduced pressure, to obtain the crude product of title Compound **360c** (623 mg, 1.58 mmol, yield: 99.96%). The crude product was directly used in the next step of reaction without purification.
MS m/z (ESI): 396.1 [M+1]

### Step 4

### 2-(6-{1-[1-(1,3-dioxolan-2-yl)-4-methylpentan-3-yl]azacyclobutan-3-yl}-3-methyl-[1,2,4]triazolo[4,3-a]pyridin-8-yl)-N-ethyl-5-fluoro-N-(isopropyl)benzamide 360d

Compound **360c** (623 mg, 1.58 mmol) and 1-(1,3-dioxolan-2-yl)-4-methylpentan-3-one **181a** (408.16 mg, 2.37 mmol) were dissolved in methanol (20 mL), followed by the addition of ZnCl₂(430.70 mg, 3.16 mmol), and the mixture was stirred at 50°C for 1.0 hour. The mixture was cooled to room temperature, followed by adding sodium cyanoborohydride (297.87 mg, 4.74 mmol) and stirring at 50°C for 11 hour. A saturated aqueous ammonium chloride solution (50 mL) was added to the reaction mixture, which was then extracted with ethyl acetate (50 mL x 3). The organic phases were combined, dried over anhydrous sodium sulfate, and concentrated under a reduced pressure. The resulting residues were purified by the silica-gel column chromatography using dichloromethane/methanol as an elution system, to obtain the crude product of title Compound **360d** (450 mg).
MS m/z (ESI): 552.2 [M+1]

### Step 5

### 2-(6-{1-[(3R)-1-(1,3-dioxolan-2-yl)-4-methylpentan-3-yl]azacyclobutan-3-yl}-3-methyl-[1,2,4]triazolo[4,3-a]pyridin-8-yl)-N-ethyl-5-fluoro-N-(isopropyl)benzamide 360d-1

### 2-(6-{1-[(3S)-1-(1,3-dioxolan-2-yl)-4-methylpentan-3-yl]azetidin-3-yl}-3-methyl-[1,2,4]triazolo[4,3-a]pyridin-8-yl)-N-ethyl-5-fluoro-N-(propan-2-yl)benzamide

### 2-(6-{1-[(3S)-1-(1,3-dioxolan-2-yl)-4-methylpentan-3-yl]azacyclobutan-3-yl}-3-methyl-[1,2,4]triazolo[4,3-a]pyridin-8-yl)-N-ethyl-5-fluoro-N-(isopropyl)benzamide 360d-2

The crude product of Compound **360d** (450 mg) was separated and purified by the chiral preparative SFC (preparative column: DAICEL CHIRALCEL OD (250 mm * 30 mm,10 um), mobile phase: carbon dioxide-isopropanol (0.1% ammonia water); elution gradient: 45%), to obtain the crude products of title Compounds **360d-1** (134 mg, 242.89 µmol, yield: 15.37%) and **360d-2** (120 mg, 217.51 µmol, yield: 13.77%)

### Single-configuration Compound 360d-1:

MS m/z (ESI):552.3 [M+1]
Chiral HPLC analysis: retention time: 1.975 min; chromatographic column: Chiralcel OD-3 50 x 4.6 mm I.D., 3 um; mobile phase: carbon dioxide-isopropanol (0.05% diethylamide ): elution gradient: 5%-40%.

### Single-configuration Compound 360d-2:

MS m/z (ESI):552.3 [M+1]
Chiral HPLC analysis: retention time: 1.664 min; chromatographic column: Chiralcel OD-3 50 x 4.6 mm I.D., 3 um; mobile phase: carbon dioxide-isopropanol (0.05% diethylamide ): elution gradient: 5%-40%.

### Step 6

### N-ethyl-5-fluoro-2-(3-methyl-6-{1-[(3R)-2-methyl-6-oxohexan-3-yl]azacyclobutan-3-yl}-[1,2,4]triazolo[4,3-a]pyridin-8-yl)-N-(isopropyl)benzamide 360e-1

Compound **360d-1** (134 mg, 242.89 µmol) was dissolved in acetonitrile (2.0 mL), followed by the addition of a hydrochloric acid solution (1.0 M; 2.0 mL). The mixture was stirred at 50°C to allow for reacting for 2.0 hours. The reaction mixture was concentrated under a reduced pressure, followed by adding dichloromethane (5 mL) and water (5 mL), regulating to pH > 7 using a 10 % aqueous NaOH solution and extracting with dichloromethane (10 mL x 3). The organic phases were combined, dried over anhydrous sodium sulfate, and concentrated under a reduced pressure, to obtain the crude product of title Compound **360e-1** (123 mg, 242.30 µmol, yield: 99.76%). The crude product was directly used in the next step of reaction without purification.

### Single-configuration Compound 360e-1:

MS m/z (ESI): 508.2 [M+1]

### Step 7

### N-ethyl-5-fluoro-2-(3-methyl-6-{1-[(3R)-2-methyl-6-(4-methylpiperazin-1-yl)hexan-3-yl]azacyclobutan-3-yl}-[1,2,4]triazolo[4,3-a]pyridin-8-yl)-N-(isopropyl)benzamide 360-1

The crude product of Compound **360e-1** (123 mg, 242.30 µmol) and 1-methylpiperazine (29.12 mg, 290.76 µmol) were dissolved in methanol (10 mL). Acetic acid (1.46 mg, 24.23 µmol) was added, and the mixture was stirred at room temperature for 1.0 hour. Sodium cyanoborohydride (45.68 mg, 726.89 µmol) was added, allowing for reacting at room temperature for 11 hours. A saturated aqueous ammonium chloride solution (50 mL) was added to the reaction mixture, which was then extracted with ethyl acetate (50 mL x 3). The organic phases were combined, washed with saturated brine, dried over anhydrous sodium sulfate, and concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% ammonia water), elution gradient: 36%-66%). The title Compound **360-1** (10.2 mg, 17.24 µmol, yield: 7.11 %) was obtained as yellow solid.

### Single-configuration Compound 360-1:

MS m/z (ESI):592.4 [M+1]
Chiral SFC: retention time: 1.180 min; chromatographic column: Chiralcel OD-3 50×4.6 mm I.D., 3um,; mobile phase: CO₂/MeOH (0.05% DEA): elution gradient: 5%-40%.

¹H NMR (400 MHz, CDCl₃) δ 7.96 - 7.71 (m, 2H), 7.50 - 7.34 (m, 1H), 7.26 - 7.09 (m, 2H), 4.45 - 3.37 (m, 5H), 3.26 - 2.89 (m, 3H), 2.78 (s, 3H), 2.64 - 2.04 (m, 13H), 1.86 - 1.72 (m, 2H), 1.62 - 1.48 (m, 2H), 1.41 - 1.03 (m, 3H), 1.01 - 0.76 (m, 12H), 0.33 (br d, J = 6.4 Hz, 2H).

### Step 8

### N-ethyl-5-fluoro-2-(3-methyl-6-{1-[(3S)-2-methyl-6-oxohexan-3-yl]azacyclobutan-3-yl}-[1,2,4]triazolo[4,3-a]pyridin-8-yl)-N-(isopropyl)benzamide 360e-2

Compound **360d-2** (120 mg, 217.51 µmol) was dissolved in acetonitrile (2.0 mL), followed by the addition of a hydrochloric acid solution (1.0 M; 2.0 mL). The mixture was stirred at 50°C to allow for reacting for 2.0 hours. The reaction mixture was concentrated under a reduced pressure, followed by adding dichloromethane (5 mL) and water (5 mL), regulating to pH > 7 using a 10 % aqueous NaOH solution and extracting with dichloromethane (10 mL x 3). The organic phases were combined, dried over anhydrous sodium sulfate, and concentrated under a reduced pressure, to obtain the crude product of title Compound **360e-2** (110 mg, 216.69 µmol, yield: 99.62%). The crude product was directly used in the next step of reaction without purification.

### Single-configuration Compound 360e-2:

MS m/z (ESI): 508.3[M+1]

### Step 9

### N-ethyl-5-fluoro-2-(3-methyl-6-{1-[(3S)-2-methyl-6-(4-methylpiperazin-1-yl)hexan-3-yl]azacyclobutan-3-yl}-[1,2,4]triazolo[4,3-a]pyridin-8-yl)-N-(isopropyl)benzamide 360-2

The crude product of Compound **360e-2** (110 mg, 216.69 µmol) and 1-methylpiperazine (26.04 mg, 260.03 µmol) were dissolved in methanol (10 mL). Acetic acid (1.30 mg, 21.67 µmol) was added, and the mixture was stirred at room temperature for 1.0 hour. Sodium cyanoborohydride (40.85 mg, 650.07 µmol) was added, allowing for reacting at room temperature for 11 hours. A saturated aqueous ammonium chloride solution (50 mL) was added to the reaction mixture, which was then extracted with ethyl acetate (50 mL x 3). The organic phases were combined, washed with saturated brine, dried over anhydrous sodium sulfate, and concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% ammonia water), elution gradient: 36%-66%). The title Compound **360-2** (25.85 mg, 43.68 µmol, yield: 20.16%) was obtained as yellow solid.

### Single-configuration Compound 360-2:

MS m/z (ESI):592.4 [M+1]
Chiral SFC: retention time: 1.183 min; chromatographic column: Chiralcel OD-3 50×4.6 mm I.D., 3 um; mobile phase: CO₂/MeOH (0.05% DEA): elution gradient: 5%-40%.

¹H NMR (400 MHz, CDCl₃) δ 7.94 - 7.70 (m, 2H), 7.49 - 7.35 (m, 1H), 7.26 - 7.07 (m, 2H), 4.47 - 3.37 (m, 5H), 3.25 - 2.90 (m, 3H), 2.78 (s, 3H), 2.58 - 2.02 (m, 13H), 1.89 - 1.72 (m, 2H), 1.61 - 1.47 (m, 2H), 1.44 - 1.05 (m, 3H), 0.99 - 0.76 (m, 12H), 0.33 (br d, J = 6.4 Hz, 2H).

### Examples 368-1 and 368-2

### (3R)-4-[5-fluoro-2-(1-fluoro-3-methyl-6-{1-[(2R)-3-methyl-1-(4-methylpiperazin-1-yl)butan-2-yl]azacyclobutan-3-yl}imidazo[1,5-a]pyridin-8-yl)benzoyl]-3-methylmorpholine 368-1

### (3R)-4-[5-fluoro-2-(1-fluoro-3-methyl-6-{1-[(2S)-3-methyl-1-(4-methylpiperazin-1-yl)butan-2-yl]azacyclobutan-3-yl}imidazo[1,5-a]pyridin-8-yl)benzoyl]-3-methylmorpholine 368-2

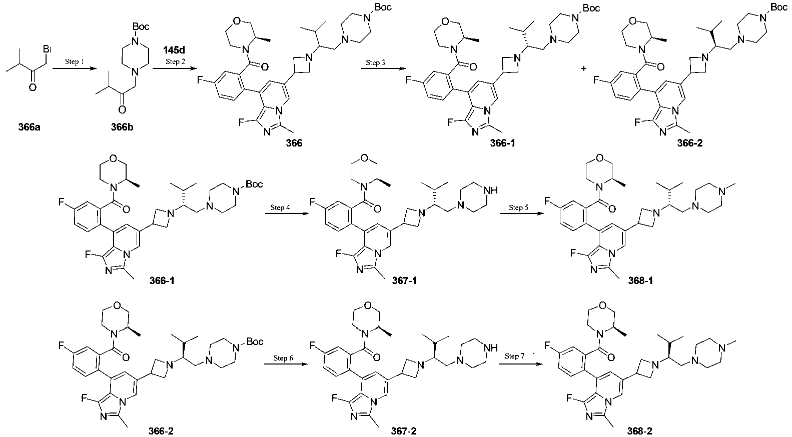

### Step 1

### 4-(3-methyl-2-oxobutyl)piperazin-1-tert-butyl carboxylate 366b

Piperazin-1-tert-butyl carboxylate (1.0 g, 5.37 mmol) was dissolved in DMF(15 mL), followed by the addition of 1-bromo-3-methyl-but-2-one **366a** (930.36 mg, 5.64 mmol) and potassium carbonate (2.23 g, 16.11 mmol L), and the mixture was stirred at 80°C for 12 hours. The reaction mixture was cooled to room temperature, followed by adding water (20 mL) and extracting with ethyl acetate (20 mL x 3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **366b** (1.2 g, 4.44 mmol, yield: 82.67%) as yellow oil.
MS m/z (ESI): 271.3 [M+1]
¹H NMR (400 MHz, CDCl₃) δ 3.53 - 3.44 (m, 4H), 3.28 (s, 2H), 2.76 - 2.65 (m, 1H), 2.49 - 2.40 (m, 4H), 1.46 (s, 9H), 1.10 (d, J = 7.0 Hz, 6H).

### Step 2

### 4-{2-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-3-methylbutyl}piperazin-1-tert-butyl carboxylate 366

Compound **145d** (830 mg, 1.95 mmol) and Compound **366b** (631.45 mg, 2.34 mmol) were dissolved in methanol (12 mL), followed by the addition of ZnCl₂ (530.54 mg, 3.89 mmol), and the mixture was stirred at 50°C for 1.0 hour. The mixture was cooled to room temperature, followed by adding sodium cyanoborohydride (366.92 mg, 5.84 mmol) and stirring at 50°C for 12 hour. A saturated aqueous sodium bicarbonate solution (15 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **366** (650 mg, 954.72 µmol, yield: 49.05%).
MS m/z (ESI): 681.5 [M+1]

### Step 3

### 4-[(2R)-2-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-3-methylbutyl]piperazin-1-tert-butyl carboxylate 366-1

### 4-[(2S)-2-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-3-methylbutyl]piperazin-1-tert-butyl carboxylate 366-2

Compound **366** (600 mg, 881.28 µmol) was separated and purified by the chiral preparative SFC (preparative column: DAICEL CHIRALPAK IK (250 mm * 30 mm,10 um), mobile phase: carbon dioxide-ethanol (0.1% ammonia water); elution gradient: 25%), to obtain the title Compounds **366-1** (300 mg, 440.64 µmol, yield: 50%) and **366-2** (200 mg, 293.76 µmol, yield: 33.3%)

### Single-configuration Compound 366-1:

MS m/z (ESI):681.8 [M+1]
Chiral HPLC analysis: retention time: 1.549 min; chromatographic column: Chiralpak IK-3 50 x 4.6 mm I.D., 3 um; mobile phase: carbon dioxide-ethanol (0.05% diethylamide): elution gradient: 5%-40%.

### Single-configuration Compound 366-2:

MS m/z (ESI):681.8 [M+1]
Chiral HPLC analysis: retention time: 1.673 min; chromatographic column: Chiralpak IK-3 50 x 4.6 mm I.D., 3 um; mobile phase: carbon dioxide-ethanol (0.05% diethylamide): elution gradient: 5%-40%.

### Step 4

### (3R)-4-[5-fluoro-2-(1-fluoro-3-methyl-6-{1-[(2R)-3-methyl-1-(piperazin-1-yl)butan-2-yl]azacyclobutan-3-yl}imidazo[1,5-a]pyridin-8-yl)benzoyl]-3-methylmorpholine 367-1

Compound **366-1** (150.00 mg, 220.32 µmol) was dissolved in dichloromethane (3.0 mL), followed by the addition of trifluoroacetic acid (1.48 g, 12.98 mmol, 1 mL). The mixture was stirred at room temperature to allow for reacting for 1.0 hour. The reaction mixture was concentrated under a reduced pressure, to obtain the crude product of title Compound **367-1** (130 mg), of which 100 mg of the crude product was directly used in the next step of reaction without purification. 30 mg of the crude product was purified by the HPLC (mobile phase: acetonitrile, water (0.1% ammonia water), elution gradient: 24%-54%). The title Compound **367-1** was obtained as yellow solid.

### Single-configuration Compound 367-1:

MS m/z (ESI): 581.4 [M+1]
Chiral HPLC analysis: retention time: 1.216 min; chromatographic column: Chiralcel OD-3 50 × 4.6 mm I.D., 3 um; mobile phase: carbon dioxide-methanol (0.05% diethylamide): elution gradient: 5%-40%.

¹H NMR (400 MHz, DMSO-*d₆*) δ 7.98 - 7.71 (m, 1H), 7.64 - 7.20 (m, 3H), 6.88 - 6.50 (m, 1H), 4.45 - 3.38 (m, 7H), 3.20 - 2.52 (m, 12H), 2.44 - 1.97 (m, 8H), 1.80 - 1.66 (m, 1H), 1.28 - 1.01 (m, 2H), 0.93 - 0.71 (m, 7H).

### Step 5

### (3R)-4-[5-fluoro-2-(1-fluoro-3-methyl-6-{1-[(2R)-3-methyl-1-(4-methylpiperazin-1-yl)butan-2-yl]azacyclobutan-3-yl}imidazo[1,5-a]pyridin-8-yl)benzoyl]-3-methylmorpholine 368-1

The crude product of Compound **367-1** (100.00 mg, 143.94 µmol, trifluoroacetate) was dissolved in methanol (2.0 mL). Triethylamine (29.13 mg, 287.88 µmol) and formaldehyde (116.81 mg, 1.44 mmol) were added, and the mixture was stirred at room temperature for 1.0 hour. Sodium cyanoborohydride (27.14 mg, 431.82 µmol) was added, allowing for reacting at room temperature for 8.0 hours. A saturated aqueous sodium bicarbonate solution (10 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 36%-66%). The title Compound **368-1** (48 mg, 80.71 µmol, yield: 56.07%) was obtained as yellow solid.

### Single-configuration Compound 368-1:

MS m/z (ESI):595.4 [M+1]
Chiral SFC: retention time: 1.047 min; chromatographic column: Chiralcel OD-3 50×4.6 mm I.D., 3um,; mobile phase: CO₂/MeOH (0.05% DEA): elution gradient: 5%-40%.

¹H NMR (400 MHz, DMSO-*d₆*) δ 7.99 - 7.73 (m, 1H), 7.66 - 7.22 (m, 3H), 6.89 - 6.53 (m, 1H), 4.43 - 3.44 (m, 6H), 3.30 - 2.52 (m, 10H), 2.43 - 2.04 (m, 13H), 1.79 - 1.67 (m, 1H), 1.23 - 0.67 (m, 9H).

### Step 6

### (3R)-4-[5-fluoro-2-(1-fluoro-3-methyl-6-{1-[(2S)-3-methyl-1-(piperazin-1-yl)butan-2-yl]azacyclobutan-3-yl}imidazo[1,5-a]pyridin-8-yl)benzoyl]-3-methylmorpholine 367-2

Compound **366-2** (150.00 mg, 220.32 µmol) was dissolved in dichloromethane (3.0 mL), followed by the addition of trifluoroacetic acid (2.22 g, 19.47 mmol, 1.50 mL). The mixture was stirred at room temperature to allow for reacting for 1.0 hour. The reaction mixture was concentrated under a reduced pressure, to obtain the crude product of title Compound **367-2** (130 mg), of which 100 mg of the crude product was directly used in the next step of reaction without purification. 30 mg of the crude product was purified by the HPLC (mobile phase: acetonitrile, water (0.1% ammonia water), elution gradient: 28%-58%). The title Compound **367-2** was obtained as yellow solid.

### Single-configuration Compound 367-2:

MS m/z (ESI): 581.4 [M+1]
Chiral HPLC analysis: retention time: 1.222 min; chromatographic column: Chiralcel OD-3 50 × 4.6 mm I.D., 3 um; mobile phase: carbon dioxide-methanol (0.05% diethylamide): elution gradient: 5%-40%.

¹H NMR (400 MHz, DMSO-*d₆*) δ 7.95 - 7.74 (m, 1H), 7.64 - 7.22 (m, 3H), 6.85 - 6.42 (m, 1H), 4.44 - 3.41 (m, 7H), 3.18 - 2.53 (m, 12H), 2.41 - 2.03 (m, 8H), 1.78 - 1.68 (m, 1H), 1.24 - 1.02 (m, 2H), 0.92 - 0.73 (m, 7H).

### Step 7

### (3R)-4-[5-fluoro-2-(1-fluoro-3-methyl-6-{1-[(2S)-3-methyl-1-(4-methylpiperazin-1-yl)butan-2-yl]azacyclobutan-3-yl}imidazo[1,5-a]pyridin-8-yl)benzoyl]-3-methylmorpholine 368-2

The crude product of Compound **367-2** (100.00 mg, 143.94 µmol, trifluoroacetate) was dissolved in methanol (2.0 mL). Triethylamine (29.13 mg, 287.88 µmol) and formaldehyde (116.81 mg, 1.44 mmol) were added, and the mixture was stirred at room temperature for 1.0 hour. Sodium cyanoborohydride (27.14 mg, 431.82 µmol) was added, allowing for reacting at room temperature for 8.0 hours. A saturated aqueous sodium bicarbonate solution (10 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 36%-66%). The title Compound **368-2** (33 mg, 55.49 µmol, yield: 38.55%) was obtained as yellow solid.

### Single-configuration Compound 368-2:

MS m/z (ESI):595.4 [M+1]
Chiral SFC: retention time: 1.05 min; chromatographic column: Chiralcel OD-3 50×4.6 mm I.D., 3um,; mobile phase: CO₂/MeOH (0.05% DEA): elution gradient: 5%-40%.

¹H NMR (400 MHz, DMSO-*d₆*) δ 7.98 - 7.71 (m, 1H), 7.64 - 7.23 (m, 3H), 6.88 - 6.44 (m, 1H), 4.38 - 3.44 (m, 6H), 3.25 (br s, 9H), 2.45 - 2.04 (m, 14H), 1.78 - 1.65 (m, 1H), 1.26 - 0.97 (m, 2H), 0.93 - 0.70 (m, 7H).

### Example 370

### 1-{6-[(4R)-4-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl] -5-methylhexyl]-2,6-diazaspiro[3.3]heptan-2-yl}ethan-1-one 370

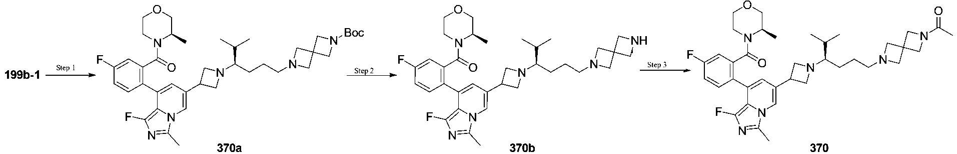

### Step 1

### 6-[(4R)-4-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-5-methylhexyl]-2,6-diazaspiro[3.3]heptan-2-tert-butyl carboxylate 370a

Compound **199b-1** (120 mg, 222.79 µmol) and 2,6-diazaspiro[3.3]heptan-2-tert-butyl carboxylate (128.46 mg, 445.58 µmol, oxalate) were dissolved in methanol (2.0 mL), followed by the addition of triethylamine (45.09 mg, 445.58 µmol), and the mixture was stirred at room temperature for 1.0 hour under the protection of nitrogen. Sodium cyanoborohydride (42.00 mg, 668.37 µmol) was added, allowing for reacting at room temperature for 8.0 hours under the protection of nitrogen. A saturated aqueous sodium bicarbonate solution (10 mL) was added to the reaction mixture, which was then extracted with dichloromethane (15 mL x 3). The organic phases were combined, dried over anhydrous sodium sulfate, and concentrated under a reduced pressure. The resulting residues were purified by the preparative thin-layer chromatography using dichloromethane/methanol as an elution system, to obtain the title product **370a** (130 mg, 180.33 µmol, yield: 80.94%) as yellow oil.
MS m/z (ESI):721.5 [M+1]

### Step 2

### 2-[(4R)-4-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-5-methylhexyl]-2,6-diazaspiro[3.3]heptane 370b

Compound **370a** (130 mg, 180.33 µmol) was dissolved in dichloromethane (3.0 mL), followed by the addition of trifluoroacetic acid (1.48 g, 12.98 mmol, 1 mL) at 25°C. The mixture was stirred at room temperature to allow for reacting for 0.5 hours. The reaction mixture was concentrated under a reduced pressure, and then dissolved by adding DCM (10 mL), followed by adding a 10% aqueous sodium hydroxide solution (15 mL), stirring for 0.5 hours and extracting with dichloromethane (10 mL x 3). The organic phases were combined, dried over anhydrous sodium sulfate and filtered, and the filtrate was concentrated under a reduced pressure, to obtain the crude product of title Compound **370b** (100 mg, 161.09 µmol, yield: 89.33%). The crude product was directly used in the next step of reaction without purification.
MS m/z (ESI): 621.5 [M+1]

### Step 3

### 1-{6-[(4R)-4-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-5-methylhexyl]-2,6-diazaspiro[3.3]heptan-2-yl}ethan-1-one 370

Triethylamine (48.90 mg, 483.27 µmol) and Compound **370b** (100 mg, 161.09 µmol) were dissolved in dichloromethane (3.0 mL), and cooled to 5°C, followed by the addition of acetic anhydride (32.89 mg, 322.18 µmol). The reaction mixture was stirred at room temperature for 12 hours. A saturated aqueous sodium bicarbonate solution (10 mL) was added to the reaction mixture, which was then extracted with dichloromethane (10 mL × 3). The organic phase was dried over anhydrous sodium sulfate, filtered and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient:30%-60%). The title Compound **370** (29.13 mg, 43.95 µmol, yield: 27.28%) was obtained as yellow solid.
MS m/z (ESI): 663.5 [M+1]
¹H NMR (400 MHz, DMSO-*d₆*) δ 7.98 - 7.76 (m, 1H), 7.64 - 7.23 (m, 3H), 6.87 - 6.36 (m, 1H), 4.41 - 3.39 (m, 11H), 3.23 - 2.52 (m, 12H), 2.31 - 2.19 (m, 2H), 2.04 - 1.94 (m, 1H), 1.77 - 1.63 (m, 4H), 1.33 - 1.03 (m, 6H), 0.89 - 0.70 (m, 7H).

### Examples 371 and 372

### (3R)-4-{5-fluoro-2-[1-fluoro-3-methyl-6-(1-{4-methyl-1-[(3R)-morpholin-3-yl]pentan-3-yl}azacyclobutan-3-yl)imidazo[1,5-a]pyridin-8-yl]benzoyl}-3-methylmorpholine 371

### (3R)-4-{5-fluoro-2-[1-fluoro-3-methyl-6-(1-{4-methyl-1-[(3R)-4-methylmorpholin-3-yl]pentan-3-yl}azacyclobutan-3-yl)imidazo[1,5-a]pyridin-8-yl]benzoyl}-3-methylmorpholine 372

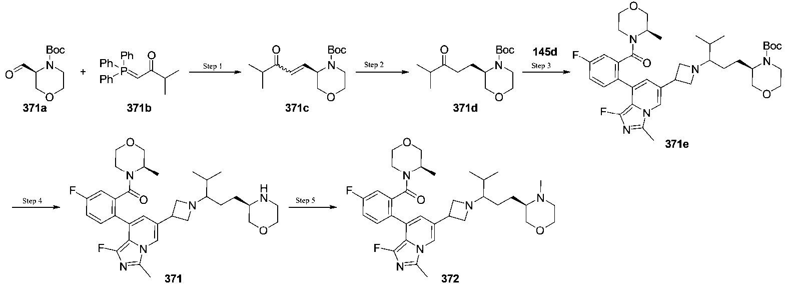

### Step 1

### (3R)-3-(4-methyl-3-oxo-1-ene-1-yl)morpholin-4-tert-butyl carboxylate 371c

3-Methyl-1-(triphenylphosphinidene)but-2-one **371b** (3.22 g, 9.29 mmol) was dissolved in dichloromethane (2.0 mL), followed by the addition of (3*S*)-3-formylmorpholin-4-tert-butyl carboxylate **371a**(1 g, 4.65 mmol), and the reaction mixture was stirred at 40°C for 48 hours. The reaction mixture was filtered and washed with, and the filtrate was concentrated under a reduced pressure. The resulting residues were purified by the silica-gel column chromatography using petroleum ether/ethyl acetate as an elution system, to obtain the title Compound **371c** (1 g, 3.53 mmol, yield: 75.96%).

¹H NMR (400 MHz, DMSO-*d₆*) δ 6.85 (dd, J = 4.9, 16.0 Hz, 1H), 6.14 (dd, J = 1.8, 16.0 Hz, 1H), 4.53 (s, 1H), 3.95 (d, J= 11.8 Hz, 1H), 3.80 (dd, J = 3.0, 11.4 Hz, 1H), 3.66 - 3.56 (m, 2H), 3.42 - 3.36 (m, 1H), 3.10 - 3.00 (m, 1H), 2.98 - 2.85 (m, 1H), 1.41 (s, 9H), 1.02 (dd, J = 2.0, 6.9 Hz, 6H).

### Step 2

### (3R)-3-(4-methyl-3-oxopentyl)morpholin-4-tert-butyl carboxylate 371d

Under the protection of nitrogen, Compound **371c** (1 g, 3.53 mmol) was dissolved in methanol (3.0 mL), followed by the addition of Pd/C(428.60 mg, 352.91 µmol, 10% content), and the reaction mixture was stirred at 30°C for 12 hours under a hydrogen atmosphere (20 PSI). The reaction mixture was filtered through diatomite, and the filtrate was concentrated under a reduced pressure, to obtain the title Compound **371d** (1 g, 3.50 mmol, yield: 99.39%).

¹H NMR (400 MHz, DMSO-*d₆*) δ 3.85 - 3.71 (m, 2H), 3.69 - 3.54 (m, 2H), 3.42 - 3.38 (m, 1H), 3.30 - 3.23 (m, 1H), 3.06 - 2.91 (m, 1H), 2.60 - 2.54 (m, 1H), 2.48 - 2.34 (m, 2H), 1.98 - 1.85 (m, 1H), 1.76 - 1.63 (m, 1H), 1.40 (s, 9H), 1.00 (dd, J = 2.4, 7.2 Hz, 6H).

### Step 3

### (3R)-3-{3-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-4-methylpentyl}morpholin-4-tert-butyl carboxylate 371e

Compound **145d** (809 mg, 1.90 mmol) and Compound **371d** (1.08 g, 3.79 mmol) were dissolved in methanol (10.0 mL), followed by the addition of ZnCl₂ (517.11 mg, 3.79 mmol), and the mixture was stirred at 25°C for 1.0 hour. The mixture was cooled to room temperature, followed by the addition of sodium cyanoborohydride (357.64 mg, 5.69 mmol), allowing for reacting at 25°C for 12 hours under the protection of nitrogen. Water (30 mL) was added to the reaction mixture, which was then extracted with ethyl acetate (30 mL x 3). The organic phases were combined, dried over anhydrous sodium sulfate, and concentrated under a reduced pressure. The resulting residues were purified by the silica-gel column chromatography using petroleum ether/ethyl acetate as an elution system, and the obtained crude product was purified by the HPLC (mobile phase: acetonitrile, water (0.1% ammonia water), elution gradient: 47%-77%), to obtain the title Compound **371e** (600 mg, 862.27 µmol, yield: 45.45%).
MS m/z (ESI): 696.0 [M+1]

### Step 4

### (3R)-4-{5-fluoro-2-[1-fluoro-3-methyl-6-(1-{4-methyl-1-[(3R)-morpholin-3-yl]pentan-3-yl}azacyclobutan-3-yl)imidazo[1,5-a]pyridin-8-yl]benzoyl}-3-methylmorpholine 371

Compound **371e** (123 mg, 176.77 µmol) was dissolved in dichloromethane (5.0 mL), followed by the addition of trifluoroacetic acid (1.48 g, 12.98 mmol, 1 mL) at 25°C. The mixture was stirred at room temperature to allow for reacting for 1.0 hours. The reaction mixture was concentrated under a reduced pressure. The crude product was dissolved in dichloromethane (5 mL), followed by adding water (5 mL) and regulating to PH > 7 with 10% aqueous NaOH solution. The mixture was extracted with dichloromethane (10 mL x 3), and the organic phases were combined and concentrated under a reduced pressure. The resulting crude product was purified by the HPLC (mobile phase: acetonitrile, water (0.1% ammonia water), elution gradient: 28%-58%), to obtain the title Compound 371 (75.19 mg, 126.22 µmol, yield: 71.4%) as yellow solid.
MS m/z (ESI):596.4 [M+1]
¹H NMR (400 MHz, CD₃OD) δ 7.82 (d, J = 12.4 Hz, 1H), 7.70 - 7.50 (m, 1H), 7.43 - 7.07 (m, 2H), 6.96 - 6.53 (m, 1H), 4.60 - 4.01 (m, 1H), 3.89 - 3.56 (m, 7H), 3.54 - 3.44 (m, 1H), 3.33 (s, 2H), 3.29 - 3.21 (m, 2H), 3.15 (t, J = 10.4 Hz, 1H), 3.02 - 2.80 (m, 3H), 2.70 (s, 1H), 2.62 (s, 3H), 2.21 (s, 2H), 1.92 - 1.80 (m, 1H), 1.61 - 1.46 (m, 1H), 1.39 (s, 3H), 1.34 - 1.26 (m, 1H), 1.24 - 1.15 (m, 1H), 1.00 - 0.88 (m, 6H), 0.83 (s, 1H).

### Step 5

### (3R)-4-{5-fluoro-2-[1-fluoro-3-methyl-6-(1-{4-methyl-1-[(3R)-4-methylmorpholin-3-yl]pentan-3-yl}azacyclobutan-3-yl)imidazo[1,5-a]pyridin-8-yl]benzoyl}-3-methylmorpholine 372

Compound **371** (128 mg, 214.87 µmol) was dissolved in methanol (5.0 mL), followed by the addition of acetic acid (38.71 mg, 644.60 µmol) and formaldehyde (26.15 mg, 322.30 µmol), and the mixture was stirred at 25°C for 1.0 hour. Sodium cyanoborohydride (27.00 mg, 429.73 µmol) was added, allowing for reacting at 25°C for 3.0 hours under the protection of nitrogen. Water (10 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 33%-63%), to obtain the title Compound **372** (76.95 mg, 126.20 µmol, yield: 58.73%).
MS m/z (ESI):610.4 [M+1]
¹H NMR (400 MHz, CD₃OD) δ 7.81 (d, J = 13.6 Hz, 1H), 7.64 - 7.50 (m, 1H), 7.38 - 7.16 (m, 2H), 6.87 - 6.60 (m, 1H), 3.83 - 3.53 (m, 8H), 3.33 - 3.18 (m, 8H), 2.70 (d, J = 12.0 Hz, 1H), 2.60 (s, 3H), 2.37 - 2.27 (m, 4H), 2.21 (s, 1H), 2.13 - 2.02 (m, 1H), 1.90 - 1.78 (m, 1H), 1.77 - 1.64 (m, 1H), 1.50 - 1.24 (m, 4H), 1.21 - 1.11 (m, 1H), 0.98 - 0.87 (m, 6H), 0.81 (d, J = 4.4 Hz, 1H).

### Examples 373 and 374

### (3R)-4-{5-fluoro-2-[1-fluoro-3-methyl-6-(1-{4-methyl-1-[(3S)-morpholin-3-yl]pentan-3-yl} azacyclobutan-3-yl)imidazo[1,5-a]pyridin-8-yl]benzoyl}-3-methylmorpholine 373

### (3R)-4-{5-fluoro-2-[1-fluoro-3-methyl-6-(1-{4-methyl-1-[(3S)-4-methylmorpholin-3-yl]pentan-3-yl}azacyclobutan-3-yl)imidazo[1,5-a]pyridin-8-yl]benzoyl}-3-methylmorpholine 374

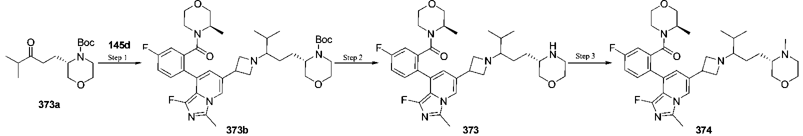

### Step 1

### (3S)-3-{3-[3-(1-fluoro-8-{4-fluoro-2-[(3R)-3-methylmorpholin-4-carbonyl]phenyl}-3-methylimidazo[1,5-a]pyridin-6-yl)azacyclobutan-1-yl]-4-methylpentyl}morpholin-4-tert-butyl carboxylate 373b

Compound **145d** (250 mg, 586.22 µmol) and Compound **373a**(167.30 mg, 586.22 µmol, synthesized according to the synthesis route of Compound **371d** by using (3*R*)-3-formylmorpholin-4-tert-butyl carboxylate as a starting material to replace (3*S*)-3-formylmorpholin-4-tert-butyl carboxylate **371a**) were dissolved in methanol (5.0 mL), followed by the addition of ZnCl₂ (159.80 mg, 1.17 mmol), and the mixture was stirred at 50°C for 1.0 hour. The mixture was cooled to room temperature, followed by the addition of sodium cyanoborohydride (110.52 mg, 1.76 mmol), allowing for reacting at 50°C for 11 hours under the protection of nitrogen. A saturated aqueous sodium carbonate solution (50 mL) was added to the reaction mixture, which was then extracted with ethyl acetate (50 mL x 3). The organic phases were combined, washed with saturated brine, dried over anhydrous sodium sulfate, and concentrated under a reduced pressure. The resulting residues were purified by the silica-gel column chromatography using petroleum ether/ethyl acetate as an elution system, to obtain the title Compound **373b** (370 mg, 531.73 µmol, yield: 90.70%).
MS m/z (ESI): 696.3 [M+1]

### Step 2

### (3R)-4-{5-fluoro-2-[1-fluoro-3-methyl-6-(1-{4-methyl-1-[(3S)-morpholin-3-yl}pentan-3-yl}azacyclobutan-3-yl)imidazo[1,5-a]pyridin-8-yl]benzoyl}-3-methylmorpholine 373

Compound **373b** (60 mg, 86.23 µmol) was dissolved in dichloromethane (6.0 mL), followed by the addition of trifluoroacetic acid (2.96 g, 25.96 mmol, 2 mL) at 25°C. The mixture was stirred at room temperature to allow for reacting for 1.0 hours. The reaction mixture was concentrated under a reduced pressure. The crude product was dissolved in dichloromethane (5 mL), followed by adding water (5 mL) and regulating to PH > 7 with 10% aqueous NaOH solution. The mixture was extracted with dichloromethane (10 mL x 3), and the organic phases were combined and concentrated under a reduced pressure. The resulting crude product was purified by the HPLC (mobile phase: acetonitrile, water (0.1% ammonia water), elution gradient: 28%-58%), to obtain the title Compound **373** (17.87 mg, 30.00 µmol, yield: 34.79%) as yellow solid.
MS m/z (ESI):596.2 [M+1]
¹H NMR (400 MHz, CD₃OD) δ 7.80 (d, J = 13.6 Hz, 1H), 7.66 - 7.49 (m, 1H), 7.40 - 7.16 (m, 2H), 6.89 - 6.59 (m, 1H), 4.64 - 4.01 (m, 2H), 3.83 - 3.54 (m, 7H), 3.48 (m, 1H), 3.28 - 3.10 (m, 4H), 3.03 - 2.79 (m, 3H), 2.74 - 2.65 (m, 1H), 2.60 (s, 3H), 2.20 (s, 1H), 1.90 - 1.76 (m, 1H), 1.52 - 1.15 (m, 6H), 1.04 - 0.50 (m, 8H).

### Step 3

### (3R)-4- {5-fluoro-2-[1-fluoro-3-methyl-6-(1-{4-methyl-1-[(3S)-4-methylmorpholin-3-yl]pentan-3-yl}azacyclobutan-3-yl)imidazo[1,5-a]pyridin-8-yl]benzoyl}-3-methylmorpholine 374

Compound **373** (54 mg, 90.65 µmol) was dissolved in methanol (5.0 mL), followed by the addition of formaldehyde (13.61 mg, 453.23 µmol), and the mixture was stirred at 25°C for 1.0 hour. Sodium cyanoborohydride (17.09 mg, 271.94 µmol) was added, allowing for reacting at 25°C for 11 hours under the protection of nitrogen. A saturated aqueous sodium bicarbonate solution (20 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 30%-630), to obtain the title Compound **374** (28.64 mg, 46.97 µmol, yield: 51.82%).
MS m/z (ESI):610.4 [M+1]
¹H NMR (400 MHz, CDCl₃) δ 7.56 - 7.39 (m, 2H), 7.27 - 7.08 (m, 2H), 6.83 - 6.58 (m, 1H), 4.64 - 4.13 (m, 1H), 3.84 - 3.51 (m, 8H), 3.40 - 3.23 (m, 2H), 3.20 - 2.93 (m, 4H), 2.84 - 2.65 (m, 2H), 2.62 (s, 3H), 2.38 - 2.28 (m, 4H), 2.11 - 2.02 (m, 2H), 1.88 - 1.73 (m, 2H), 1.46 - 1.13 (m, 5H), 0.96 - 0.88 (m, 6H), 0.85 - 0.59 (m, 1H).

### Example 375

### N-ethyl-5-fluoro-2-(3-methyl-6-{1-[(3R)-2-methyl-6-(morpholin-4-yl)hexan-3-yl]azacyclobutan-3-yl}imidazo[1,5-a]pyridin-8-yl)-N-(isopropyl)benzamide 375

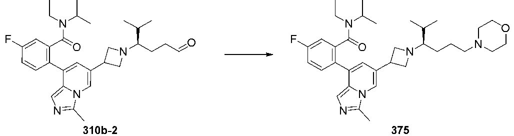

Compound **310b-2** (121 mg, 238.82 µmol) and morpholine (24.97 mg, 286.59 µmol) were dissolved in methanol (5.0 mL), followed by the addition of acetic acid (43.03 mg, 716.47 µmol), and the mixture was stirred at room temperature for 1.0 hour. Sodium cyanoborohydride (30.02 mg, 477.64 µmol) was added, allowing for reacting at 25°C for 13.0 hours under the protection of nitrogen. A saturated aqueous sodium carbonate solution (20 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 30%-60%), to obtain the title Compound **375** (37.24 mg, 64.45 µmol, yield: 26.99%) as faint yellow solid.
MS m/z (ESI):578.5[M+1]
Chiral SFC: retention time: 1.073 min; chromatographic column: Chiralcel OD-3 50 × 4.6 mm I.D., 3 um; mobile phase: CO₂/MeOH (0.05% DEA): elution gradient: 5%-40%.

¹H NMR (400 MHz, CD₃OD) δ 7.95 - 7.89 (m, 1H), 7.76 - 7.68 (m, 1H), 7.38 - 7.30 (m, 1H), 7.28 - 7.20 (m, 1H), 7.16 - 7.11 (m, 1H), 6.95 - 6.84 (m, 1H), 3.83 - 3.76 (m, 1H), 3.75 - 3.62 (m, 6H), 3.56 (td, J = 6.4, 12.8 Hz, 1H), 3.43 (qd, J = 6.4, 13.2 Hz, 1H), 3.28 - 3.22 (m, 1H), 3.09 - 2.95 (m, 1H), 2.66 (s, 3H), 2.52 - 2.42 (m, 4H), 2.36 (q, J = 7.6 Hz, 2H), 2.30 - 2.22 (m, 1H), 1.94 - 1.79 (m, 1H), 1.71 - 1.52 (m, 2H), 1.48 - 1.37 (m, 1H), 1.36 - 1.25 (m, 1H), 1.21 - 1.09 (m, 1H), 1.01 - 0.87 (m, 12H), 0.80 (t, J = 7.0 Hz, 1H), 0.30 (d, J = 6.4 Hz, 2H).

### Example 378

### 2-(6-{1-[(3R)-6-{3-acetyl-3,8-diazabicyclo[3.2.1]oct-8-yl}-2-methylhexan-3-yl]azacyclobutan-3-yl}-3-methylimidazo[1,5-a]pyridin-8-yl)-N-ethyl-5-fluoro-N-(isopropyl)benzamide 378

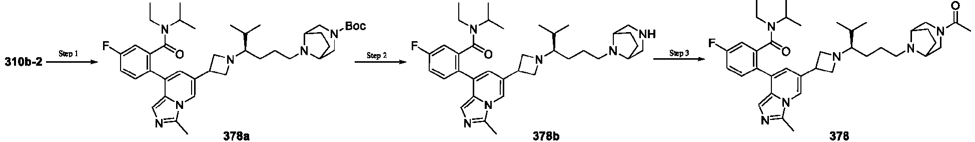

### Step 1

### 8-[(4R)-4-[3-(8-{2-[ethyl(isopropyl)aminoformyl]-4-fluorophenyl)-3-methylimidazo[1,5-a]pyridin-6-yl)azacyclobutan-1-yl]-5-methylhexyl]-3,8-diazabicyclo[3.2.1]octan-3-tert-butyl carboxylate 378a

Compound **310b-2** (180 mg, 355.27 µmol) and 3,8-diazabicyclo[3.2.1]octan-3-tert-butyl carboxylate (150.84 mg, 710.55 µmol) were dissolved in methanol (4.0 mL), followed by the addition of acetic acid (21.33 mg, 355.27 µmol), and the mixture was stirred at room temperature for 0.5 hours under the protection of nitrogen. Sodium cyanoborohydride (66.98 mg, 1.07 mmol) was added, allowing for reacting at room temperature for 12 hours under the protection of nitrogen. A saturated aqueous sodium bicarbonate solution (15 mL) was added to the reaction mixture, which was then extracted with dichloromethane (20 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title product **378a** (200 mg, 284.52 µmol, yield: 80.08%) as faint yellow solid.
MS m/z (ESI):703.8 [M+1]

### Step 2

### 2-(6-{1-[(3R)-6-{3,8-diazabicyclo[3.2.1]oct-8-yl}-2-methylhexan-3-yl]azacyclobutan-3-yl}-3-methylimidazo[1,5-a]pyridin-8-yl)-N-ethyl-5-fluoro-N-(isopropyl)benzamide 378b

Compound **378a** (200 mg, 284.52 µmol) was dissolved in dichloromethane (3.0 mL), followed by the addition of trifluoroacetic acid (1.48 g, 12.98 mmol, 1 mL) at 25°C. The mixture was stirred at room temperature to allow for reacting for 0.5 hours. The reaction mixture was concentrated under a reduced pressure, and then dissolved by adding DCM (10 mL), followed by adding a 10% aqueous sodium hydroxide solution (15 mL), stirring for 0.5 hours and extracting with dichloromethane (15 mL x 3). The organic phases were combined, dried over anhydrous sodium sulfate and filtered, and the filtrate was concentrated under a reduced pressure, to obtain the crude product of title Compound **378b** (170 mg, 282.01 µmol, yield: 99.12%). The crude product was directly used in the next step of reaction without purification.
MS m/z (ESI): 503.2 [M+1]

### Step 3

### 2-(6-{1-[(3R)-6-{3-acetyl-3,8-diazabicyclo[3.2.1]oct-8-yl}-2-methylhexan-3-yl]azacyclobutan-3-yl}-3-methylimidazo[1,5-a]pyridin-8-yl)-N-ethyl-5-fluoro-N-(isopropyl)benzamide 378

Triethylamine (85.61 mg, 846.02 µmol) and Compound **378b** (170 mg, 282.01 µmol) were dissolved in dichloromethane (4.0 mL), and cooled to 5°C, followed by the addition of acetic anhydride (57.58 mg, 564.01 µmol). The reaction mixture was stirred at room temperature for 1.0 hours. A saturated aqueous sodium bicarbonate solution (20 mL) was added to the reaction mixture, which was then extracted with dichloromethane (20 mL × 3). The organic phase was dried over anhydrous sodium sulfate, filtered and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient:30%-60%). The title Compound **378** (65.61 mg, 101.74 µmol, yield: 36.08%) was obtained as yellow solid.
MS m/z (ESI): 645.9 [M+1]
¹H NMR (400 MHz, DMSO-*d₆*) δ 8.01 - 7.81 (m, 1H), 7.78 - 7.51 (m, 1H), 7.50 - 7.13 (m, 2H), 7.13 - 6.93 (m, 1H), 6.90 - 6.65 (m, 1H), 4.36 - 3.83 (m, 1H), 3.65 - 3.34 (m, 5H), 3.29 - 2.62 (m, 8H), 2.62 - 2.57 (m, 3H), 2.35 - 2.13 (m, 2H), 2.10 - 1.99 (m, 1H), 1.99 - 1.86 (m, 3H), 1.83 - 1.63 (m, 3H), 1.53 - 1.19 (m, 6H), 1.09 - 0.69 (m, 13H), 0.30 - 0.23 (m, 2H).

### Example 379

### N-ethyl-5-fluoro-2-(3-methyl-6-{1-[(3R)-2-methyl-6-[(1R,4R)-2-oxa-5-azabicyclo[2.2.1]heptan-5-yl]hexan-3-yl]azacyclobutan-3-yl}imidazo[1,5-a]pyridin-8-yl)-N-(isopropyl)benzamide 379

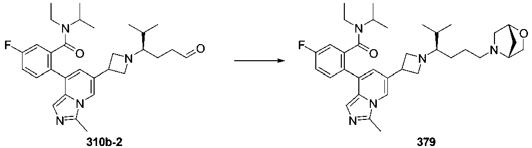

Compound **310b-2** (90 mg, 177.64 µmol) and (1R,4R)-2-oxa-5-azabicyclo[2.2.1]heptane (28.90 mg, 213.16 µmol, hydrochloride) were dissolved in methanol (2.0 mL), followed by the addition of triethylamine (53.92 mg, 532.91 µmol), and the mixture was stirred at room temperature for 1.0 hour. Sodium cyanoborohydride (33.49 mg, 532.91 µmol) was added, allowing for reacting at 25°C for 11.0 hours under the protection of nitrogen. A saturated aqueous sodium bicarbonate solution (15 mL) was added to the reaction mixture, which was then extracted with dichloromethane (20 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 35%-65%). The title Compound 379 (31.38 mg, 53.21 µmol, yield: 29.95%) was obtained as yellow solid.
MS m/z (ESI):590.7[M+1]
Chiral SFC: retention time: 1.160 min; chromatographic column: Chiralcel OD-3 50×4.6 mm I.D., 3 um; mobile phase: CO₂/MeOH (0.05% DEA): elution gradient: 5%-40%.

¹H NMR (400 MHz, DMSO-*d₆*) δ 7.93 (s, 1H), 7.73 - 7.63 (m, 1H), 7.42 - 7.26 (m, 2H), 7.08 - 6.99 (m, 1H), 6.87 - 6.70 (m, 1H), 4.27 (s, 1H), 3.78 (d, J = 7.2 Hz, 1H), 3.60 - 3.34 (m, 6H), 3.31 - 3.24 (m, 1H), 3.18 - 3.02 (m, 2H), 2.97 - 2.86 (m, 1H), 2.72 (d, J = 10.0 Hz, 1H), 2.59 (s, 3H), 2.49 - 2.29 (m, 3H), 2.03 (s, 1H), 1.77 - 1.63 (m, 2H), 1.55 - 1.46 (m, 1H), 1.45 - 1.01 (m, 5H), 0.92 - 0.71 (m, 12H), 0.27 (d, J = 6.5 Hz, 2H).

### Examples 381-1 and 381-2

### [(4R)-4-{3-[(4-{4-fluoro-2-[(3R)-3-methylmorpholin-4-carbonyl]phenyl}-1-methyl-1H-indazol-7-yl)methyl]azacyclobutan-1-yl}-5-methylhexyl](2-methoxyethyl)methylamine 381-1

### [(4S)-4-{3-[(4-{4-fluoro-2-[(3R)-3-methylmorpholin-4-carbonyl]phenyl}-1-methyl-1H-indazol-7-yl)methyl]azacyclobutan-1-yl}-5-methylhexyl](2-methoxyethyl)methylamine 381-2

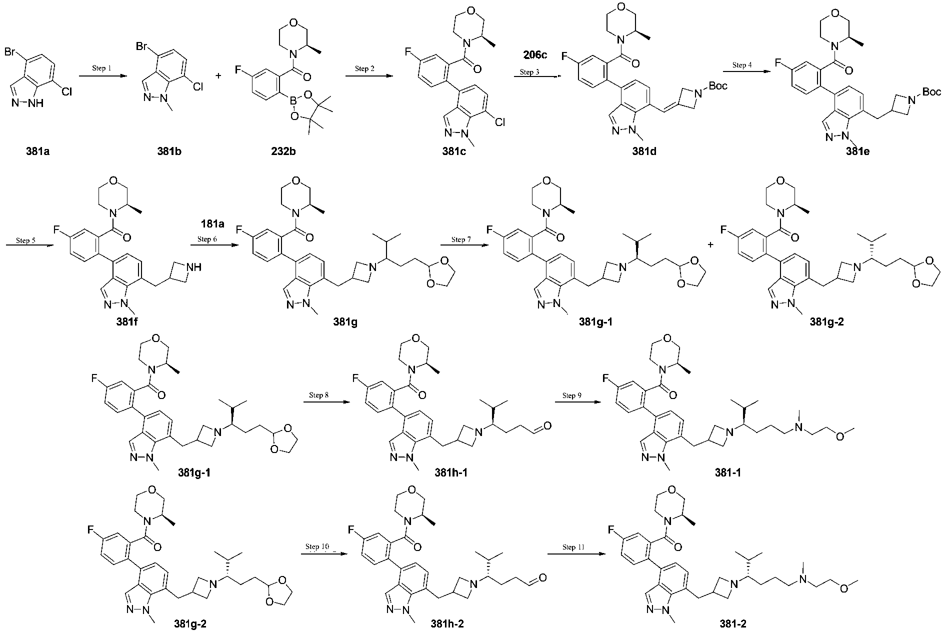

### Step 1

### 4-bromo-7-chloro-1-methyl-1H-indazole 381b

4-Bromo-7-chloro-1*H*-indazole **381a** (10 g, 43.20 mmol) was dissolved in N,N-dimethylformamide (100 mL), and cooled to 0°C. NaH (2.07 g, 51.84 mmol, 60% purity) was added, and the mixture was stirred at 0°C to allow for reacting for 0.5 hours. Iodomethane (7.36 g, 51.84 mmol) was added, and the mixture was stirred at room temperature to allow for reacting for 1.0 hour. A saturated aqueous ammonium chloride solution (30 mL) was slowly added at 0°C, and the mixture then extracted with ethyl acetate (60 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **381b** (7.47 g, 30.43 mmol, yield: 70.43%) as faint yellow solid.
MS m/z (ESI): 246.8 [M+1]
¹H NMR (400 MHz, DMSO-*d*₆) δ 8.07 (s, 1H), 7.42 - 7.27 (m, 2H), 4.31 (s, 3H).

### Step 2

### 7-chloro-4-{4-fluoro-2-[(3R)-3-methylmorpholin-4-carbonyl]phenyl}-1-methyl-1H-indazole 381c

Compound **381b** (4 g, 8.15 mmol), Compound **232b**(2.99 g, 8.15 mmol) and potassium phosphate (5.19 g, 24.44 mmol) were dissolved in dioxane (60 mL) and water (6.0 mL). Pd(dtbpf)Cl₂ (530.95 mg, 814.65 µmol) was added. Under a nitrogen atmosphere, the mixture was stirred at 90°Cto allow for reacting for 2.0 hours. The reaction mixture was cooled to room temperature, water (100 mL) was added, and the mixture was extracted with ethyl acetate (100 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **381c** (3 g, 7.74 mmol, yield: 94.95%).
MS m/z (ESI): 388.1 [M+1]
¹H NMR (400 MHz, CDCl₃) δ 7.48 (s, 1H), 7.40 (s, 1H), 7.24 - 7.06 (m, 2H), 6.68 (s, 1H), 4.63 - 4.44 (m, 1H), 4.35 - 4.26 (m, 2H), 3.95 (br d, J = 6.0 Hz, 2H), 3.67 (s, 3H), 3.31 - 2.99 (m, 2H), 2.61 (s, 3H), 1.66 (br s, 3H), 1.46 (s, 9H), 1.31 - 1.23 (m, 2H).

### Step 3

### 3-[(4-{4-fluoro-2-[(3R)-3-methylmorpholin-4-carbonyl]phenyl}-1-methyl-1H-indazol-7-yl)methylene]azacyclobutan-1-tert-butyl carboxylate 381d

Compound **381c** (500 mg, 1.29 mmol), 3-[(tetramethyl-1,3,2-dioxaboran-2-yl)methylene]azacyclobutan-1-tert-butyl carboxylate **206c** (380.55 mg, 1.29 mmol) and potassium phosphate (820.97 mg, 3.87 mmol) were dissolved in dioxane (5 mL) and water (1 mL). Pd(dtbpf)Cl₂ (84.02 mg, 128.92 µmol) was added. Under a nitrogen atmosphere, the mixture was stirred at 90°Cto allow for reacting for 2.0 hours. The reaction mixture was cooled to room temperature, water (100 mL) was added, and the mixture was extracted with ethyl acetate (100 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **381d** (518 mg, 995.02 µmol, yield: 77.18%).
MS m/z (ESI): 521.2 [M+1]
¹H NMR (400 MHz, CDCl₃) δ 7.92 - 7.80 (m, 1H), 7.62 - 7.49 (m, 1H), 7.27 - 7.15 (m, 3H), 7.14 - 7.05 (m, 1H), 7.04 - 6.92 (m, 1H), 4.85 - 4.64 (m, 4H), 4.38 - 4.30 (m, 3H), 4.18 - 4.02 (m, 1H), 3.68 - 3.34 (m, 2H), 3.24 - 3.11 (m, 1H), 3.10 - 2.93 (m, 1H), 2.89 - 2.77 (m, 1H), 2.58 - 2.27 (m, 1H), 1.61 (s, 3H), 1.49 (s, 9H).

### Step 4

### 3-[(4-{4-fluoro-2-[(3R)-3-methylmorpholin-4-carbonyl]phenyl}-1-methyl-1H-indazol-7-yl)methyl]azacyclobutan-1-tert-butyl carboxylate 381e

Compound **381d** (418 mg, 802.93 µmol) was dissolved in methanol (10 mL), followed by the addition of Rh/Al₂O₃ (32.90 mg, 160.59 µmol) under a nitrogen atmosphere. The reaction atmosphere was substituted with a nitrogen atmosphere (15 psi), and the mixture was stirred at room temperature for 3.0 hours. The reaction mixture was filtered, and the filtrate was concentrated under a reduced pressure, to obtain the crude product of title Compound **381e** (419 mg, 801.75 µmol, yield: 99.85%).
MS m/z (ESI): 523.2 [M+1]

### Step 5

### 7-[(azacyclobutan-3-yl)methyl]-4-{4-fluoro-2-[(3R)-3-methylmorpholin-4-carbonyl]phenyl}-1-methyl-1H-indazole 381f

Compound **381e** (300 mg, 574.04 µmol) was dissolved in methane dioxide (7.3 mL), followed by the addition of trifluoroacetic acid (2.57 g, 22.55 mmol, 1.74 mL) at 0°C. The mixture was stirred at room temperature to allow for reacting for 1.0 hours. The reaction mixture was concentrated under a reduced pressure, dissolved by adding DCM (5.0 mL) and water (5.0 mL), regulated to PH > 7 by adding a 10% aqueous sodium hydroxide solution, and extracted with dichloromethane (10 mL x 3). The organic phases were combined, dried over anhydrous sodium sulfate and filtered, and the filtrate was concentrated under a reduced pressure, to obtain the crude product of title Compound **381f** (242 mg, 572.79 µmol, yield: 99.78%). The crude product was directly used in the next step of reaction without purification.
MS m/z (ESI): 423.1 [M+1]

### Step 6

### 7-({1-[1-(1,3-dioxolan-2-yl)-4-methylpentan-3-yl]azacyclobutan-3-yl}methyl)-4-{4-ffuoro-2-[(3R)-3-methylmorpholin-4-carbonyl]phenyl}-1-methyl-1H-indazole 381g

Compound **381f** (242 mg, 572.79 µmol) and Compound **181a**(197.29 mg, 1.15 mmol) were dissolved in methanol (40 mL). Zinc chloride (156.14 mg, 1.15 mmol) was added, and the mixture was stirred at room temperature for 30 min. The mixture was cooled to room temperature, followed by adding sodium cyanoborohydride (107.99 mg, 1.72 mmol) and stirring at room temperature for 2.0 hours. Water (20 mL) was added, and the mixture was extracted with dichloromethane (10 mL×3). The organic phases were combined, dried over anhydrous sodium sulfate, and concentrated under a reduced pressure. The resulting residues were purified by the silica-gel column chromatography using dichloromethane/methanol as an elution system, to obtain the title Compound **381g** (291 mg, 502.84 µmol, yield: 87.79%) as yellow solid.
MS m/z (ESI): 579.4[M+1]

### Step 7

### 7-({1-[(3R)-1-(1,3-dioxolan-2-yl)-4-methylpentan-3-yl]azacyclobutan-3-yl}methyl)-4-{4-fluoro-2-[(3R)-3-methylmorpholin-4-carbonyl]phenyl}-1-methyl-1H-indazole 381g-1

### 7-({1-[(3S)-1-(1,3-dioxolan-2-yl)-4-methylpentan-3-yl]azacyclobutan-3-yl}methyl)-4-{4-fluoro-2-[(3R)-3-methylmorpholin-4-carbonyl]phenyl}-1-methyl-1H-indazole 381g-2

Compound **381g** (291 mg, 502.84 µmol) was separated and purified by the chiral preparative SFC (preparative column: DAICEL CHIRALCEL OD (250 mm * 30 mm,10 um), mobile phase: CO₂-MeOH (0.1% ammonia water); elution gradient: 32%), to obtain the title Compounds **381g-1** (97 mg, 167.61 µmol, yield: 33.3%) and **381g-2** (87 mg, 150.33 µmol, yield: 29.9%).

### Single-configuration Compound 381g-1:

MS m/z (ESI):579.4 [M+1]
Chiral HPLC analysis: retention time: 1.407 min; chromatographic column: Chiralcel OD-3 50 x 4.6 mm I.D., 3 um; mobile phase: CO₂-MeOH (0.05% diethylamide): elution gradient: 5%-40%.

### Single-configuration Compound 381g-2:

MS m/z (ESI):579.4 [M+1]
Chiral HPLC analysis: retention time: 1.544 min; chromatographic column: Chiralcel OD-3 50 x 4.6 mm I.D., 3 um; mobile phase: CO₂-MeOH (0.05% diethylamide): elution gradient: 5%-40%.

### Step 8

### (4R)-4-{3-[(4-{4-fluoro-2-[(3R)-3-methylmorpholin-4-carbonyl]phenyl}-1-methyl-1H-indazol-7-yl)methyl]azacyclobutan-1-yl}-5-methylhexanal 381h-1

Compound **381g-1** (97 mg, 167.61 µmol) was dissolved in acetonitrile (5.0 mL), followed by the addition of a hydrochloric acid solution (1.0 M; 5.0 mL). The mixture was stirred at 50°C to allow for reacting for 2.0 hours. The reaction mixture was concentrated under a reduced pressure, diluted with dichloromethane (5.0 mL) and water (5.0 mL), regulated to pH ≥ 7 with a 10 % aqueous NaOH solution, and extracted with dichloromethane (10 mL x 3). The organic phases were combined, dried over anhydrous sodium sulfate, and concentrated under a reduced pressure, to obtain the crude product of title Compound **381h-1** (89.62 mg, 167.62 µmol, yield: 100%). The crude product was directly used in the next step of reaction without purification.

### Single-configuration Compound 381h-1:

MS m/z (ESI): 535.3 [M+1]

### Step 9

### [(4R)-4-{3-[(4-{4-fluoro-2-[(3R)-3-methylmorpholin-4-carbonyl]phenyl}-1-methyl-1H-indazol-7-yl)methyl]azacyclobutan-1-yl}-5-methylhexyl](2-methoxyethyl)methylamine 381-1

The crude product of Compound **381h-1** (89.62 mg, 167.62 µmol) and 2-methoxy-N-methylethylamine (17.93 mg, 201.14 µmol) were dissolved in methanol (5.0 mL), followed by the addition of acetic acid (20.13 mg, 335.24 µmol), and the mixture was stirred at room temperature for 0.5 hours under the protection of nitrogen. Sodium cyanoborohydride (31.60 mg, 502.86 µmol) was added, allowing for reacting at 50°C for 2.0 hours under the protection of nitrogen. A saturated aqueous sodium bicarbonate solution (20 mL) was added to the reaction mixture, which was then extracted with dichloromethane (20 mL x 3). The organic phases were combined, dried over anhydrous sodium sulfate, and concentrated under a reduced pressure. The resulting residues were purified by the HPLC (mobile phase: acetonitrile, water (0.1% formic acid), elution gradient: 4%-34%). The title Compound **381-1** (11.86 mg, 19.51 µmol, yield: 11.64%) was obtained.

### Single-configuration Compound 381-1:

MS m/z (ESI):608.5 [M+1]
Chiral SFC: retention time: 0.827 min; chromatographic column: Chiralcel OJ-3 50×4.6 mm I.D., 3um,; mobile phase: CO₂/MeOH (0.05% DEA): elution gradient: 5%-40%.

¹H NMR (400 MHz, CD₃OD) δ 8.34 - 8.28 (m, 1H), 7.72 - 7.56 (m, 1H), 7.43 - 7.26 (m, 1H), 7.18 - 7.08 (m, 1H), 7.07 - 6.92 (m, 2H), 6.91 - 6.73 (m, 1H), 4.29 - 3.94 (m, 4H), 3.89 - 3.66 (m, 2H), 3.58 - 3.44 (m, 2H), 3.43 - 3.37 (m, 2H), 3.35 - 3.21 (m, 3H), 3.18 - 3.11 (m, 3H), 3.11 - 3.03 (m, 2H), 3.02 - 2.88 (m, 2H), 2.86 - 2.78 (m, 2H), 2.73 - 2.56 (m, 4H), 2.46 - 2.37 (m, 3H), 2.36 - 2.08 (m, 1H), 1.88 - 1.70 (m, 1H), 1.49 (s, 2H), 1.39 - 1.17 (m, 2H), 1.13 - 0.93 (m, 1H), 0.89 - 0.65 (m, 7H), 0.41 - -0.07 (m, 1H).

### Step 10

### (4S)-4-{3-[(4-{4-fluoro-2-[(3R)-3-methylmorpholin-4-carbonyl]phenyl}-1-methyl-1H-indazol-7-yl)methyl]azacyclobutan-1-yl}-5-methylhexanal 381h-2

Compound **381g-2** (87 mg, 150.33 µmol) was dissolved in acetonitrile (5.0 mL), followed by the addition of a hydrochloric acid solution (1.0 M; 5.0 mL). The mixture was stirred at 50°C to allow for reacting for 2.0 hours. The reaction mixture was concentrated under a reduced pressure, diluted with dichloromethane (5.0 mL) and water (5.0 mL), regulated to pH ≥ 7 with a 10 % aqueous NaOH solution, and extracted with dichloromethane (10 mL x 3). The organic phases were combined, dried over anhydrous sodium sulfate, and concentrated under a reduced pressure, to obtain the crude product of title Compound **381h-2** (80.38 mg, 150.34 µmol, yield: 100%). The crude product was directly used in the next step of reaction without purification.

### Single-configuration Compound 381h-2:

MS m/z (ESI): 535.2 [M+1]

### Step 11

### [(4S)-4-{3-[(4-{4-fluoro-2-[(3R)-3-methylmorpholin-4-carbonyl]phenyl}-1-methyl-1H-indazol-7-yl)methyl]azacyclobutan-1-yl}-5-methylhexyl](2-methoxyethyl)methylamine 381-2

The crude product of Compound **381h-2** (80.38 mg, 150.34 µmol) and 2-methoxy-*N*-methylethylamine (16.08 mg, 180.41 µmol) were dissolved in methanol (5 mL), followed by the addition of acetic acid (18.06 mg, 300.68 µmol), and the mixture was stirred at room temperature for 0.5 hours under the protection of nitrogen. Sodium cyanoborohydride (28.34 mg, 451.01 µmol) was added, allowing for reacting at 50°C for 2.0 hours under the protection of nitrogen. A saturated aqueous sodium bicarbonate solution (20 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% formic acid), elution gradient: 3%-33%). The title Compound **381-2** (10.08 mg, 16.58 µmol, yield: 11.03%) was obtained.

### Single-configuration Compound 381-2:

MS m/z (ESI):608.5 [M+1]
Chiral SFC: retention time: 1.250 min; chromatographic column: Chiralcel OD-3 50×4.6 mm I.D., 3um,; mobile phase: CO₂/MeOH (0.05% DEA): elution gradient: 5%-40%.

¹H NMR (400 MHz, CD₃OD) δ 8.27 (s, 1H), 7.64 - 7.53 (m, 1H), 7.36 - 7.22 (m, 1H), 7.11 - 7.01 (m, 1H), 7.01 - 6.87 (m, 2H), 6.84 - 6.69 (m, 1H), 4.05 (d, J = 4.0 Hz, 4H), 3.73 - 3.54 (m, 2H), 3.34 - 3.28 (m, 3H), 3.27 - 3.11 (m, 4H), 3.07 (s, 3H), 3.03 - 2.91 (m, 2H), 2.90 - 2.72 (m, 2H), 2.71 - 2.53 (m, 3H), 2.50 - 2.35 (m, 3H), 2.30 - 2.20 (m, 3H), 2.17 - 2.05 (m, 1H), 1.75 - 1.59 (m, 1H), 1.38 (s, 2H), 1.28 - 1.09 (m, 2H), 1.07 - 0.89 (m, 1H), 0.79 - 0.60 (m, 7H), 0.31 - -0.12 (m, 1H).

### Example 389

### N-ethyl-5-fluoro-2-(6-{1-[(3R)-6-(4-methoxypiperidin-1-yl)-2-methylhexan-3-yl]azacyclobutan-3-yl}-3-methylimidazo[1,5-a]pyridin-8-yl)-N-(isopropyl)benzamide 389

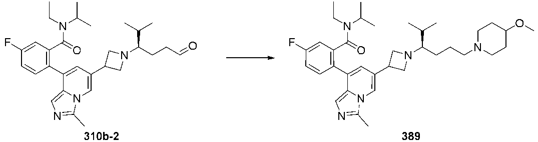

Compound **310b-2** (90 mg, 177.64 µmol) and 4-methoxypiperidine (40.92 mg, 355.27 µmol) were dissolved in methanol (2.0 mL), followed by the addition of acetic acid (10.67 mg, 177.64 µmol), and the mixture was stirred at room temperature for 1.0 hour. Sodium cyanoborohydride (33.49 mg, 532.91 µmol) was added, allowing for reacting at 25°C for 8.0 hours under the protection of nitrogen. A saturated aqueous sodium bicarbonate solution (10 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% formic acid), elution gradient: 0%-30%). The title Compound **389** (15.9 mg, 26.25 µmol, yield: 14.77%) was obtained.
MS m/z (ESI):606.9[M+1]
¹H NMR (400 MHz, DMSO-*d*₆) δ 8.20 (s, 1.7H), 8.01 - 7.87 (m, 1H), 7.76 - 7.62 (m, 1H), 7.45 - 7.27 (m, 2H), 7.08 - 6.99 (m, 1H), 6.87 - 6.69 (m, 1H), 3.63 - 3.57 (m, 2H), 3.55 - 3.50 (m, 1H), 3.48 - 3.41 (m, 1H), 3.35 - 3.26 (m, 1H), 3.23 - 3.11 (m, 6H), 2.98 - 2.86 (m, 1H), 2.78 - 2.69 (m, 2H), 2.59 (s, 3H), 2.35 (t, J = 7.2 Hz, 2H), 2.25 - 2.06 (m, 3H), 1.88 - 1.66 (m, 3H), 1.55 - 1.36 (m, 4H), 1.31 - 0.98 (m, 3H), 0.95 - 0.68 (m, 12H), 0.32 - 0.23 (m, 2H).

### Examples 394-1 and 394-2

### N-ethyl-2-(6-{1-[(3S)-6-(4-ethylpiperazin-1-yl)-2-methylhexan-3-yl]azacyclobutan-3-yl}-1-methyl-1H-indazol-4-yl)-5-fluoro-N-(isopropyl)benzamide 394-1

### N-ethyl-2-(6-{1-[(3R)-6-(4-ethylpiperazin-1-yl)-2-methylhexan-3-yl]azacyclobutan-3-yl}-1-methyl-1H-indazol-4-yl)-5-fluoro-N-(isopropyl)benzamide 394-2

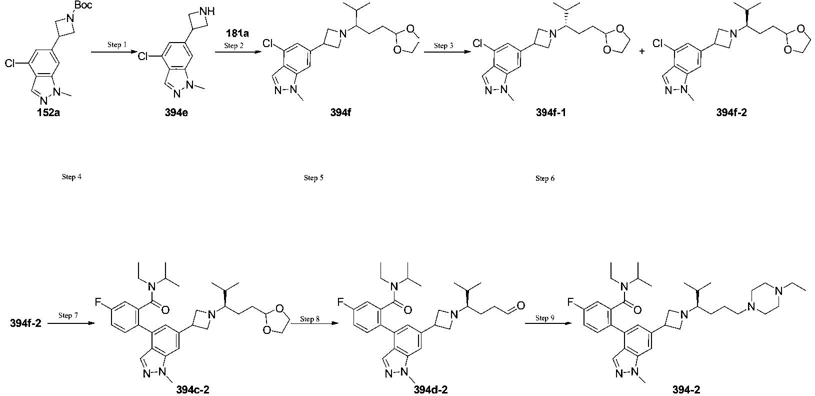

### Step 1

### 6-(azacyclobutan-3-yl)-4-chloro-1-methyl-1H-indazole 394e

Compound **152a** (500 mg, 1.55 mmol) was dissolved in dichloromethane (5.0 mL), followed by the addition of trifluoroacetic acid (2.96 g, 25.96 mmol, 2 mL) at 25°C. The mixture was stirred at room temperature to allow for reacting for 15 min. The reaction mixture was concentrated under a reduced pressure, followed by adding dichloromethane (20 mL), regulating to pH = 11 with a 10% aqueous sodium hydroxide solution, and extracting with dichloromethane (40 mL x 3). The organic phases were combined, dried over anhydrous sodium sulfate, and concentrated under a reduced pressure, to obtain the crude product of title Compound **394e** (350 mg, 1.58 mmol, yield: 101.6%) as yellow oil. The crude product was directly used in the next step of reaction without purification. MS m/z (ESI): 221.8 [M+1]

### Step 2

### 4-chloro-6-{1-[1-(1,3-dioxolan-2-yl)-4-methylpentan-3-yl]azacyclobutan-3-yl}-1-methyl-1H-indazole 394f

Compound **394e** (340 mg, 1.53 mmol) and 1-(1,3-dioxolan-2-yl)-4-methylpentan-3-one **181a** (343.38 mg, 1.99 mmol) were dissolved in methanol (5.0 mL), followed by the addition of ZnCl₂(418.08 mg, 3.07 mmol), and the mixture was stirred at 50°C for 1.0 hour. The mixture was cooled to room temperature, followed by adding sodium cyanoborohydride (289.14 mg, 4.60 mmol) and stirring at 50°C for 8.0 hour. A saturated aqueous sodium bicarbonate solution (15 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **394f** (350 mg, 926.15 µmol, yield: 60.39%) as yellow oil. MS m/z (ESI): 378.2 [M+1]

### Step 3

### 4-chloro-6-{1-[(3S)-1-(1,3-dioxolan-2-yl)-4-methylpentan-3-yl]azacyclobutan-3-yl}-1-methyl-1H-indazole 394f-1

### 4-chloro-6-{1-[(3R)-1-(1,3-dioxolan-2-yl)-4-methylpentan-3-yl]azacyclobutan-3-yl}-1-methyl-1H-indazole 394f-2

Compound **394f** (350 mg, 926.15 µmol) was separated and purified by the chiral preparative SFC (preparative column: DAICEL CHIRALCEL OD (250 mm * 30 mm, 10 um), mobile phase: carbon dioxide-isopropanol; elution gradient: 35%), to obtain the title Compounds **394f-1** (145 mg, 383.69 µmol, yield: 41.43%) and **394f-2** (145 mg, 383.69 µmol, yield: 41.43%).

### Single-configuration Compound 394f-1:

MS m/z (ESI): 378.0 [M+1]
Chiral HPLC analysis: retention time: 1.375 min; chromatographic column: Chiralcel OD-3 50 x 4.6 mm I.D., 3 um; mobile phase: carbon dioxide-isopropanol (0.05% diethylamide ): elution gradient: 5%-40%.

### Single-configuration Compound 394f-2:

MS m/z (ESI): 378.0 [M+1]
Chiral HPLC analysis: retention time: 1.534 min; chromatographic column: Chiralcel OD-3 50 x 4.6 mm I.D., 3 um; mobile phase: carbon dioxide-isopropanol (0.05% diethylamide ): elution gradient: 5%-40%.

### Step 4

### 2-(6-{1-[(3S)-1-(1,3-dioxolan-2-yl)-4-methylpentan-3-yl]azacyclobutan-3-yl}-1-methyl-1H-indazol-4-yl)-N-ethyl-5-fluoro-N-(isopropyl)benzamide 394c-1

Compound **394f-1** (145 mg, 383.69 µmol), Compound **4c**(257.24 mg, 767.38 µmol) and potassium phosphate (244.33 mg, 1.15 mmol) were dissolved in dioxane (2.0 mL) and water (0.4 mL). Pd(dtbpf)Cl₂ (25.01 mg, 38.37 µmol) was added. Under a nitrogen atmosphere, the mixture was stirred at 90°Cto allow for reacting for 1.0 hours. The reaction mixture was cooled to room temperature, water (10 mL) was added, and the mixture was extracted with ethyl acetate (15 mL x 3). The organic phases were combined, dried over anhydrous sodium sulfate, and concentrated under a reduced pressure. The resulting residues were purified by the preparative column chromatography using dichloromethane/methanol as an elution system, to obtain the title Compound **394c-1** (150 mg, 272.38 µmol, yield: 70.99%) as brown solid.
MS m/z (ESI): 551.3[M+1]
Chiral HPLC analysis: retention time: 1.122 min; chromatographic column: Chiralcel OD-3 50 x 4.6 mm I.D., 3 um; mobile phase: carbon dioxide-isopropanol (0.05% diethylamide ): elution gradient: 5%-40%.

### Step 5

### N-ethyl-5-fluoro-2-{1-methyl-6-{1-[(3S)-2-methyl-6-oxohexan-3-yl]azacyclobutan-3-yl]-1H-indazol-4-yl}-N-(isopropyl)benzamide 394d-1

Compound **394c-1** (150 mg, 272.38 µmol) was dissolved in acetonitrile (2.0 mL), followed by the addition of a hydrochloric acid solution (1.0 M; 2.0 mL). The mixture was stirred at 50°C to allow for reacting for 1.0 hours. The reaction mixture was concentrated under a reduced pressure, regulated to pH = 11 with a 10 % aqueous NaOH solution, and extracted with dichloromethane (20 mL x 3). The organic phases were combined, dried over anhydrous sodium sulfate, and concentrated under a reduced pressure, to obtain the crude product of title Compound **394d-1** (140 mg). The crude product was directly used in the next step of reaction without purification.

### Single-configuration Compound 394d-1:

MS m/z (ESI): 507.4 [M+1]

### Step 6

### N-ethyl-2-(6-{1-[(3S)-6-(4-ethylpiperazin-1-yl)-2-methylhexan-3-yl]azacyclobutan-3-yl}-1-methyl-1H-indazol-4-yl)-5-fluoro-N-(isopropyl)benzamide 394-1

The crude product of Compound **394d-1** (140 mg, 276.32 µmol) and 1-ethylpiperazine (63.11 mg, 552.65 µmol) were dissolved in methanol (2.0 mL), followed by the addition of acetic acid (24.89 mg, 414.48 µmol). Under the protection of nitrogen, the mixture was stirred at room temperature for 1.0 hour. Sodium cyanoborohydride (52.09 mg, 828.97 µmol) was added, allowing for reacting at room temperature for 8.0 hours under the protection of nitrogen. A saturated aqueous sodium bicarbonate solution (10 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system. The title Compound **394-1** (28.88 mg, 47.75 µmol, yield: 17.28%) was obtained.

### Single-configuration Compound 394-1:

MS m/z (ESI):605.3 [M+1]
Chiral SFC: retention time: 1.146 min; chromatographic column: Chiralpak AD-3 50×4.6 mm I.D., 3 um; mobile phase: CO₂/MeOH (0.05% DEA): elution gradient: 5%-40%.

¹H NMR (400 MHz, CDCl₃) δ 7.85 (s, 1H), 7.59 - 7.52 (m, 1H), 7.27 - 7.08 (m, 4H), 4.10 (s, 3H), 3.92 - 3.80 (m, 3H), 3.52 - 3.38 (m, 2H), 3.29 - 3.18 (m, 2H), 2.86 - 2.77 (m, 1H), 2.56 - 2.48 (m, 4H), 2.45 - 2.40 (m, 3H), 2.37 - 2.30 (m, 3H), 2.18 - 2.09 (m, 1H), 1.89 - 1.77 (m, 1H), 1.63 - 1.51 (m, 2H), 1.41 - 1.30 (m, 2H), 1.13 - 1.05 (m, 4H), 0.99 - 0.78 (m, 13H), 0.74 - 0.68 (m, 1H), 0.09 - 0.03 (m, 2H).

### Step 7

### 2-(6-{1-[(3R)-1-(1,3-dioxolan-2-yl)-4-methylpentan-3-yl]azacyclobutan-3-yl}-1-methyl-1H-indazol-4-yl)-N-ethyl-5-fluoro-N-(isopropyl)benzamide 394c-2

Compound **394f-2** (145 mg, 383.69 µmol), Compound **4c**(257.24 mg, 767.38 µmol) and potassium phosphate (244.33 mg, 1.15 mmol) were dissolved in dioxane (2.0 mL) and water (0.4 mL). Pd(dtbpf)Cl₂ (25.01 mg, 38.37 µmol) was added. Under a nitrogen atmosphere, the mixture was stirred at 95°Cto allow for reacting for 1.0 hours. The reaction mixture was cooled to room temperature, water (10 mL) was added, and the mixture was extracted with ethyl acetate (15 mL x 3). The organic phases were combined, dried over anhydrous sodium sulfate, and concentrated under a reduced pressure. The resulting residues were purified by the preparative column chromatography using dichloromethane/methanol as an elution system, to obtain the title Compound **394c-2** (150 mg, 272.38 µmol, yield: 70.99%) as brown solid.
MS m/z (ESI): 551.3[M+1]
Chiral HPLC analysis: retention time: 1.102 min; chromatographic column: Chiralcel OD-3 50 x 4.6 mm I.D., 3 um; mobile phase: carbon dioxide-isopropanol (0.05% diethylamide ): elution gradient: 5%-40%.

### Step 8

### N-ethyl-5-fluoro-2-{1-methyl-6-{1-[(3R)-2-methyl-6-oxohexan-3-yl]azacyclobutan-3-yl]-1H-indazol-4-yl}-N-(isopropyl)benzamide 394d-2

Compound **394c-2** (150 mg, 272.38 µmol) was dissolved in acetonitrile (1.5 mL), followed by the addition of a hydrochloric acid solution (1.0 M; 1.5 mL). The mixture was stirred at 50°C to allow for reacting for 1.0 hours. The reaction mixture was concentrated under a reduced pressure, regulated to pH = 11 with a 10 % aqueous NaOH solution, and extracted with dichloromethane (20 mL x 3). The organic phases were combined, dried over anhydrous sodium sulfate, and concentrated under a reduced pressure, to obtain the crude product of title Compound **394d-2** (140 mg). The crude product was directly used in the next step of reaction without purification.

### Single-configuration Compound 394d-2:

MS m/z (ESI): 507.3 [M+1]

### Step 9

### N-ethyl-2-(6-{1-[(3R)-6-(4-ethylpiperazin-l-yl)-2-methylhexan-3-yl]azacyclobutan-3-yl}-1-methyl-1H-indazol-4-yl)-5-fluoro-N-(isopropyl)benzamide 394-2

The crude product of Compound **394d-2** (140 mg, 276.32 µmol) and 1-ethylpiperazine (63.11 mg, 552.65 µmol) were dissolved in methanol (2.0 mL), followed by the addition of acetic acid (24.89 mg, 414.48 µmol). Under the protection of nitrogen, the mixture was stirred at room temperature for 1.0 hour. Sodium cyanoborohydride (17.36 mg, 276.32 µmol) was added, allowing for reacting at room temperature for 8.0 hours under the protection of nitrogen. A saturated aqueous sodium bicarbonate solution (10 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system. The title Compound **394-2** (44.93 mg, 74.28 µmol, yield: 26.88%) was obtained.

### Single-configuration Compound 394-2:

MS m/z (ESI):605.3 [M+1]
Chiral SFC: retention time: 1.033 min; chromatographic column: Chiralpak AD-3 50×4.6 mm I.D., 3 um; mobile phase: CO₂/MeOH (0.05% DEA): elution gradient: 5%-40%.

¹H NMR (400 MHz, CDCl₃) δ 7.85 (s, 1H), 7.60 - 7.49 (m, 1H), 7.26 - 7.02 (m, 4H), 4.10 (s, 3H), 3.94 - 3.78 (m, 3H), 3.51 - 3.36 (m, 2H), 3.31 - 3.17 (m, 2H), 2.86 - 2.76 (m, 1H), 2.58 - 2.49 (m, 4H), 2.46 - 2.41 (m, 3H), 2.37 - 2.28 (m, 3H), 2.18 - 2.10 (m, 1H), 1.89 - 1.76 (m, 1H), 1.63 - 1.49 (m, 2H), 1.42 - 1.30 (m, 2H), 1.14 - 1.02 (m, 4H), 0.96 - 0.77 (m, 13H), 0.75 - 0.68 (m, 1H), 0.08 - 0.03 (m, 2H).

### Example 396

### N-ethyl-5-fluoro-2-(6-{1-[(3R)-6-hydroxyl-2-methylhexan-3-yl]azacyclobutan-3-yl}-3-methylimidazo[1,5-a]pyridin-8-yl)-N-(isopropyl)benzamide 396

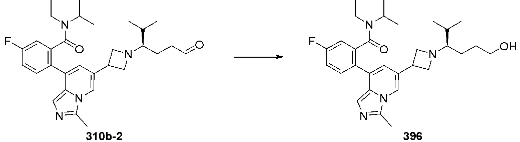

Compound **310b-2** (90 mg, 177.64 µmol) was dissolved in methanol (2.0 mL), followed by the addition of acetic acid (10.67 mg, 177.64 µmol), and the mixture was stirred at 50°C for 0.5 hours. Sodium cyanoborohydride (33.49 mg, 532.91 µmol) was added, allowing for reacting at 50°C for 12.0 hours under the protection of nitrogen. A saturated aqueous sodium carbonate solution (15 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 30%-60%), to obtain the title Compound **396** (22.76 mg, 44.74 µmol, yield: 25.19%) as yellow solid.
MS m/z (ESI):509.4[M+1]
¹H NMR (400 MHz, DMSO-*d*₆) δ 7.95 - 7.89 (m, 1H), 7.72 - 7.63 (m, 1H), 7.42 - 7.27 (m, 2H), 7.06 - 7.01 (m, 1H), 6.84 - 6.69 (m, 1H), 4.41 - 4.21 (m, 1H), 3.60 - 3.36 (m, 5H), 3.22 - 2.72 (m, 4H), 2.62 - 2.54 (m, 3H), 2.06 - 1.97 (m, 1H), 1.77 - 1.65 (m, 1H), 1.52 - 1.39 (m, 2H), 1.32 - 1.00 (m, 3H), 0.93 - 0.71 (m, 13H), 0.31 - 0.25 (m, 2H).

### Example 398

### N-ethyl-5-fluoro-2-(6-{1-[(3R)-6-[4-(2-hydroxyethyl)piperazin-1-yl]-2-methylhexan-3-yl]azacyclobutan-3-yl}-3-methylimidazo[1,5-a]pyridin-8-yl)-N-(isopropyl)benzamide 398

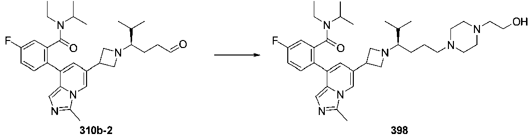

Compound **310b-2** (180 mg, 355.27 µmol) and 2-piperazin-1-ylethanol (92.50 mg, 710.55 µmol) were dissolved in methanol (4.0 mL), followed by the addition of acetic acid (21.33 mg, 355.27 µmol), and the mixture was stirred at room temperature for 0.5 hours. Sodium cyanoborohydride (66.98 mg, 1.07 mmol) was added, allowing for reacting 25°C for 15.0 hours under the protection of nitrogen. A saturated aqueous sodium carbonate solution (15 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 36%-66%), to obtain the title Compound **398** (85.12 mg, 137.10 µmol, yield: 38.59%) as yellow solid.
MS m/z (ESI):621.8[M+1]
¹H NMR (400 MHz, DMSO-*d*₆) δ 7.96 - 7.86 (m, 1H), 7.73 - 7.61 (m, 1H), 7.43 - 7.26 (m, 2H), 7.10 - 6.93 (m, 1H), 6.86 - 6.67 (m, 1H), 4.35 - 4.24 (m, 1H), 3.58 - 3.41 (m, 6H), 3.19 - 2.77 (m, 4H), 2.61 - 2.55 (m, 3H), 2.41 - 2.26 (m, 9H), 2.23 - 2.18 (m, 2H), 2.06 - 1.98 (m, 1H), 1.77 - 1.66 (m, 1H), 1.50 - 1.36 (m, 2H), 1.32 - 1.02 (m, 3H), 0.95 - 0.68 (m, 13H), 0.30 - 0.25 (m, 2H).

### Examples 399-1 and 399-2

### N-ethyl-5-fluoro-2-(6-{1-[(3S)-6-(4-methoxypiperidin-1-yl)-2-methylhexan-3-yl]azacyclobutan-3-yl}-1-methyl-1H-indazol-4-yl)-N-(isopropyl)benzamide 399-1

### N-ethyl-5-fluoro-2-(6-{1-[(3R)-6-(4-methoxypiperidin-1-yl)-2-methylhexan-3-yl]azacyclobutan-3-yl}-1-methyl-1H-indazol-4-yl)-N-(isopropyl)benzamide 399-2

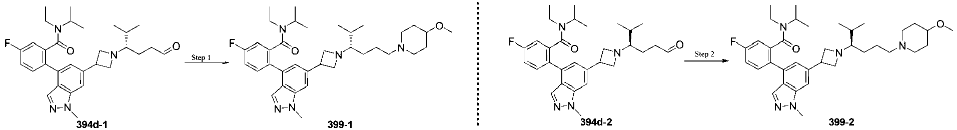

### Step 1

### N-ethyl-5-fluoro-2-(6-{1-[(3S)-6-(4-methoxypiperidin-1-yl)-2-methylhexan-3-yl]azacyclobutan-3-yl}-1-methyl-1H-indazol-4-yl)- N-(isopropyl)benzamide 399-1

Compound **394d-1** (200 mg, 276.32 µmol) and 4-methoxypiperidine (90.93 mg, 789.49 µmol) were dissolved in methanol (2.0 mL), followed by the addition of acetic acid (47.41 mg, 789.49 µmol). Under the protection of nitrogen, the mixture was stirred at room temperature for 1.0 hour. Sodium cyanoborohydride (74.42 mg, 1.18 mmol) was added, allowing for reacting at room temperature for 8.0 hours under the protection of nitrogen. A saturated aqueous sodium bicarbonate solution (10 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system. The obtained crude product was purified by the HPLC (mobile phase: acetonitrile, water (0.1% ammonia water), elution gradient: 50%-80%), to obtain the title Compound **399-1** (61.76 mg, 101.94 µmol, yield: 25.82%) as yellow solid.

### Single-configuration Compound 399-1:

MS m/z (ESI):606.3 [M+1]
Chiral SFC: retention time: 1.162 min; chromatographic column: Chiralcel OD-3 50×4.6 mm I.D., 3um,; mobile phase: CO₂/MeOH (0.05% DEA): elution gradient: 5%-40%.

¹H NMR (400 MHz, DMSO-*d*₆) δ 7.85 - 7.77 (m, 1H), 7.62 - 7.56 (m, 1H), 7.55 - 7.49 (m, 1H), 7.42 - 7.35 (m, 1H), 7.33 - 7.24 (m, 1H), 7.15 - 7.02 (m, 1H), 4.04 (s, 3H), 3.70 - 3.59 (m, 3H), 3.27 - 3.05 (m, 7H), 2.88 - 2.76 (m, 1H), 2.66 - 2.59 (m, 2H), 2.23 - 2.17 (m, 2H), 2.05 - 1.93 (m, 3H), 1.82 - 1.70 (m, 3H), 1.47 - 1.31 (m, 4H), 1.28 - 1.16 (m, 2H), 1.04 - 0.77 (m, 10H), 0.76 - 0.59 (m, 4H), 0.10 - 0.06 (m, 2H).

### Step 2

### N-ethyl-5-fluoro-2-(6-{1-[(3R)-6-(4-methoxypiperidin-1-yl)-2-methylhexan-3-yl]azacyclobutan-3-yl}-1-methyl-1H-indazol-4-yl)-N-(isopropyl)benzamide 399-2

Compound **394d-2** (180 mg, 355.27 µmol) and 4-methoxypiperidine (49.10 mg, 426.33 µmol) were dissolved in methanol (5.0 mL), followed by the addition of acetic acid (21.33 mg, 355.27 µmol). Under the protection of nitrogen, the mixture was stirred at room temperature for 1.0 hour. Sodium cyanoborohydride (66.98 mg, 1.07 mmol) was added, allowing for reacting at room temperature for 11.0 hours under the protection of nitrogen. A saturated aqueous sodium bicarbonate solution (15 mL) was added to the reaction mixture, which was then extracted with dichloromethane (25 mL x 3). The organic phases were combined, dried over anhydrous sodium sulfate, and concentrated under a reduced pressure. The resulting residues were purified by the silica-gel column chromatography using dichloromethane/methanol as an elution system. The obtained crude product was purified by the HPLC (mobile phase: acetonitrile, water (0.1% ammonia water), elution gradient: 44%-74%), to obtain the title Compound **399-2** (65.83 mg, 108.66 µmol, yield: 30.59%) as yellow solid.

### Single-configuration Compound 399-2:

MS m/z (ESI):606.8 [M+1]
Chiral SFC: retention time: 1.145 min; chromatographic column: Chiralcel OD-3 50×4.6 mm I.D., 3um,; mobile phase: CO₂/MeOH (0.05% DEA): elution gradient: 5%-40%.

¹H NMR (400 MHz, DMSO-*d*₆) δ 7.86 - 7.75 (m, 1H), 7.63 - 7.46 (m, 2H), 7.42 - 7.22 (m, 2H), 7.14 - 6.99 (m, 1H), 4.04 (s, 3H), 3.64 (s, 3H), 3.32 - 3.06 (m, 7H), 2.87 - 2.73 (m, 1H), 2.67 - 2.56 (m, 2H), 2.23 - 2.15 (m, 2H), 2.08 - 1.90 (m, 3H), 1.84 - 1.66 (m, 3H), 1.50 - 0.78 (m, 16H), 0.76 - 0.58 (m, 4H), 0.12 - 0.03 (m, 2H).

### Examples 402-1 and 402-2

### N-ethyl-5-fluoro-2-(1-methyl-6-{1-[(2R)-3-methyl-1-(4-methylpiperazin-1-yl)butan-2-yl]azacyclobutan-3-yl}-1H-indazol-4-yl)-N-(isopropyl)benzamide 402-1

### N-ethyl-5-fluoro-2-(1-methyl-6-{1-[(2S)-3-methyl-1-(4-methylpiperazin-1-yl)butan-2-yl]azacyclobutan-3-yl}-1H-indazol-4-yl)-N-(isopropyl)benzamide 402-2

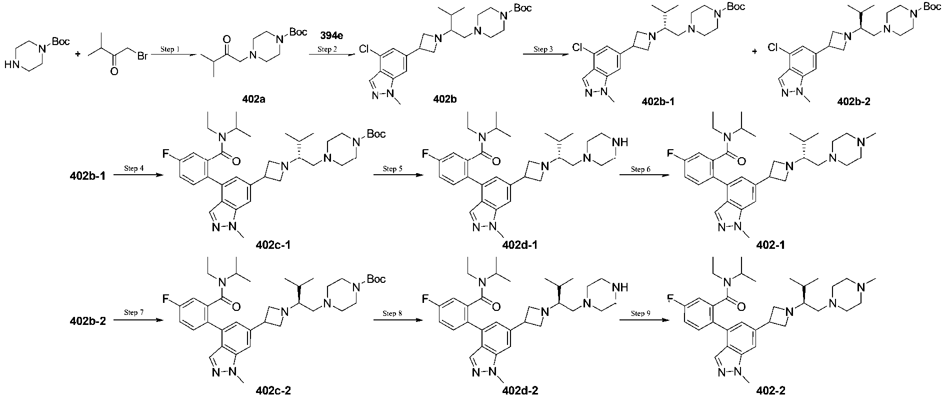

### Step 1

### 4-(3-methyl-2-oxobutyl)piperazin-1-tert-butyl carboxylate 402a

Piperazin-1-tert-butyl carboxylate (2.2 g, 11.81 mmol) was dissolved in DMF(30 mL), followed by the addition of 1-bromo-3-methyl-but-2-one (2.05 g, 12.40 mmol) and potassium carbonate (4.90 g, 35.44 mmol) at 25°C. The reaction mixture was heated to 80°C and stirred for 12 hours. The reaction mixture was cooled to room temperature, followed by adding water (20 mL) and extracting with ethyl acetate (20 mL x 3). The organic phases were combined, washed with water (20 mL x 3), dried over anhydrous sodium sulfate, and concentrated under a reduced pressure. The resulting residues were purified by the silica-gel column chromatography using petroleum ether/ethyl acetate as an elution system, to obtain the title Compound **402a** (2.29 g, 8.47 mmol, yield: 71.71%) as yellow oil.
MS m/z (ESI): 271.1 [M+1]
¹H NMR (400 MHz, CDCl₃) δ 3.45 - 3.38 (m, 4H), 3.23 (s, 2H), 2.72 - 2.56 (m, 1H), 2.43 - 2.33 (m, 4H), 1.39 (s, 9H), 1.03 (d, J = 7.2 Hz, 6H).

### Step 2

### 4-{2-[3-(4-chloro-1-methyl-1H-indazol-6-yl)azacyclobutan-1-yl]-3-methylbutyl}piperazin-1-tert-butyl carboxylate 402b

Compound **394e** (400 mg, 1.80 mmol) and Compound **402a** (634.19 mg, 2.35 mmol) were dissolved in methanol (5.0 mL), followed by the addition of ZnCl₂ (491.85 mg, 3.61 mmol), and the mixture was stirred at 50°C for 1.0 hour. The mixture was cooled to room temperature, followed by adding sodium cyanoborohydride (340.17 mg, 5.41 mmol) and stirring at 50°C for 11 hour. A saturated aqueous sodium bicarbonate solution (10 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **402b** (720 mg, 1.51 mmol, yield: 83.82%) as yellow oil.
MS m/z (ESI): 476.4 [M+1]

### Step 3

### 4-[(2R)-2-[3-(4-chloro-1-methyl-1H-indazol-6-yl)azacyclobutan-1-yl]-3-methylbutyl]piperazin-1-tert-butyl carboxylate 402b-1

### 4-[(2S)-2-[3-(4-chloro-1-methyl-1H-indazol-6-yl)azacyclobutan-1-yl]-3-methylbutyl]piperazin-1-tert-butyl carboxylate 402b-2

Compound **402b** (320 mg, 672.19 µmol) was separated and purified by the chiral preparative HPLC (preparative column: DAICEL CHIRALCEL OD (250 mm * 30 mm,10 um), mobile phase: carbon dioxide-methanol (0.1 ammonia water); elution gradient: 15%), to obtain the title Compounds **402b-1** (135 mg, 283.58 µmol, yield: 42.19%) and **402b-2** (120 mg, 252.07 µmol, yield: 37.50%).

### Single-configuration Compound 402b-1:

MS m/z (ESI): 476.4 [M+1]
Chiral HPLC analysis: retention time: 1.094 min; chromatographic column: Chiralcel OD-3 50 x 4.6 mm I.D., 3 um; mobile phase: carbon dioxide-isopropanol (0.05% diethylamide ): elution gradient: 5%-40%.

### Single-configuration Compound 402b-2:

MS m/z (ESI): 476.4 [M+1]
Chiral HPLC analysis: retention time: 1.296 min; chromatographic column: Chiralcel OD-3 50 x 4.6 mm I.D., 3 um; mobile phase: carbon dioxide-isopropanol (0.05% diethylamide ): elution gradient: 5%-40%.

### Step 4

### 4-[(2R)-2-[3-(4-{2-[ethyl(isopropyl)aminoformyl]-4-fluorophenyl}-1-methyl-1H-indazol-6-yl)azacyclobutan-1-yl)-3-methylbutyl)piperazin-1-tert-butyl carboxylate 402c-1

Compound **402b-1** (130.00 mg, 273.08 µmol), Compound **4c**(228.85 mg, 409.62 µmol) and potassium phosphate (173.90 mg, 819.24 µmol) were dissolved in dioxane (3.0 mL) and water (0.5 mL). Pd(dtbpf)Cl₂ (17.80 mg, 27.31 µmol) was added. Under a nitrogen atmosphere, the mixture was stirred at 95°Cto allow for reacting for 3.0 hours. The reaction mixture was cooled to room temperature, water (15 mL) was added, and the mixture was extracted with ethyl acetate (15 mL x 3). The organic phases were combined, dried over anhydrous sodium sulfate, and concentrated under a reduced pressure. The resulting residues were purified by the preparative column chromatography using dichloromethane/methanol as an elution system, to obtain the title Compound **402c-1** (110 mg, 169.53 µmol, yield: 62.08%) as oil.
MS m/z (ESI): 649.5

### Step 5

### N-ethyl-5-fluoro-2-(1-methyl-6-{1-[(2R)-3-methyl-1-(piperazin-1-yl)butan-2-yl]azacyclobutan-3-yl}-1H-indazol-4-yl)-N-(isopropyl)benzamide 402d-1

Compound **402c-1** (110 mg, 169.53 µmol) was dissolved in dichloromethane (3.0 mL), followed by the addition of trifluoroacetic acid (1.48 g, 12.98 mmol, 1 mL). The mixture was stirred at 25°C to allow for reacting for 1.0 hours. The reaction mixture was concentrated under a reduced pressure to obtain the crude product of title Compound **402d-1** (90 mg). The crude product was directly used in the next step of reaction without purification.
MS m/z (ESI): 549.4 [M+1]

### Step 6

### N-ethyl-5-fluoro-2-(1-methyl-6-{1-[(2R)-3-methyl-1-(4-methylpiperazin-1-yl)butan-2-yl]azacyclobutan-3-yl}-1H-indazol-4-yl)-N-(isopropyl)benzamide 402-1

The crude product of Compound **402d-1** (90 mg) and formaldehyde (24.62 mg, 246.02 µmol) were dissolved in methanol (3.0 mL), followed by the addition of triethylamine (49.79 mg, 492.04 µmol). Under the protection of nitrogen, the mixture was stirred at room temperature for 1.0 hour. Sodium cyanoborohydride (30.92 mg, 492.04 µmol) was added, allowing for reacting at room temperature for 11 hours under the protection of nitrogen. A saturated aqueous sodium bicarbonate solution (15 mL) was added to the reaction mixture, which was then extracted with dichloromethane (25 mL x 3). The organic phases were combined, dried over anhydrous sodium sulfate, and concentrated under a reduced pressure. The resulting residues were purified by the silica-gel column chromatography using dichloromethane/methanol as an elution system. The obtained crude product was purified by the HPLC (mobile phase: acetonitrile, water (0.1% ammonia water), elution gradient: 49%-79%), to obtain the title Compound **402-1** (71.05 mg, 126.25 µmol, yield: 76.98%).

### Single-configuration Compound 402-1:

MS m/z (ESI):563.4 [M+1]
Chiral SFC: retention time: 0.656 min; chromatographic column: Chiralcel OJ-3 50×4.6 mm I.D., 3um,; mobile phase: CO₂/MeOH (0.05% DEA): elution gradient: 5%-40%.

¹H NMR (400 MHz, DMSO-*d₆*) δ 7.89 - 7.76 (m, 1H), 7.64 - 7.55 (m, 1H), 7.54 - 7.46 (m, 1H), 7.43 - 7.25 (m, 2H), 7.18 - 7.02 (m, 1H), 4.04 (s, 3H), 3.74 - 3.60 (m, 3H), 3.43 - 3.35 (m, 1H), 3.31 - 3.09 (m, 3H), 2.89 - 2.76 (m, 1H), 2.46 - 2.14 (m, 10H), 2.10 (s, 3H), 1.83 - 1.69 (m, 1H), 1.08 - 0.77 (m, 10H), 0.67 (t, J = 6.8 Hz, 4H), 0.08 (d, J = 6.0 Hz, 2H).

### Step 7

### 4-[(2S)-2-[3-(4-{2-[ethyl(isopropyl)aminoformyl]-4-fluorophenyl}-1-methyl-1H-indazol-6-yl)azacyclobutan-1-yl)-3-methylbutyl)piperazin-1-tert-butyl carboxylate 402c-2

Compound **402b-2** (120 mg, 252.07 µmol), Compound **4c**(228.85 mg, 409.62 µmol) and potassium phosphate (160.52 mg, 756.22 µmol) were dissolved in dioxane (5.0 mL) and water (1.0 mL). Pd(dtbpf)Cl₂ (16.43 mg, 25.21 µmol) was added. Under a nitrogen atmosphere, the mixture was stirred at 100°Cto allow for reacting for 2.0 hours. The reaction mixture was cooled to room temperature, water (100 mL) was added, and the mixture was extracted with ethyl acetate (100 mL x 3). The organic phases were combined, dried over anhydrous sodium sulfate, and concentrated under a reduced pressure. The resulting residues were purified by the preparative column chromatography using dichloromethane/methanol as an elution system, to obtain the crude product of title Compound **402-2** (185 mg, 285.12 µmol, yield: 113%) as oil.
MS m/z (ESI): 649.3[M+1]

### Step 8

### N-ethyl-5-fluoro-2-(1-methyl-6-{1-[(2S)-3-methyl-1-(piperazin-1-yl)butan-2-yl]azacyclobutan-3-yl}-1H-indazol-4-yl)-N-(isopropyl)benzamide 402d-2

Compound **402c-2** (500 mg, 1.94 mmol) was dissolved in dichloromethane (3.0 mL), followed by the addition of trifluoroacetic acid (1.40 g, 12.28 mmol, 945.67 µL). The mixture was stirred at 25°C to allow for reacting for 1.0 hours. The reaction mixture was concentrated under a reduced pressure, dissolved by adding dichloromethane (30 mL), regulated to alkaline pH with a 10 % aqueous NaOH solution (40 mL), and let stand for separation, and the aqueous phase was extracted with dichloromethane (10 mL x 2). The organic phases were combined, dried over anhydrous sodium sulfate, and concentrated under a reduced pressure, to obtain the crude product of title Compound **402d-2** (156 mg, 284.29 µmol, yield: 99.71%). The crude product was directly used in the next step of reaction without purification.
MS m/z (ESI): 549.4 [M+1]

### Step 9

### N-ethyl-5-fluoro-2-(1-methyl-6-{1-[(2S)-3-methyl-1-(4-methylpiperazin-1-yl)butan-2-yl]azacyclobutan-3-yl}-1H-indazol-4-yl)-N-(isopropyl)benzamide 402-2

The crude product of Compound **402d-2** (156 mg, 284.29 µmol) and formaldehyde (85.36 mg, 2.84 mmol) were dissolved in methanol (5.0 mL). Under the protection of nitrogen, the mixture was stirred at room temperature for 1.0 hour. Sodium cyanoborohydride (53.60 mg, 852.87 µmol) was added, allowing for reacting at room temperature for 11.0 hours under the protection of nitrogen. A saturated aqueous sodium bicarbonate solution (20 mL) was added to the reaction mixture, which was then extracted with dichloromethane (10 mL x 3). The organic phases were combined, dried over anhydrous sodium sulfate, and concentrated under a reduced pressure. The resulting residues were purified by the preparative thin-layer chromatography using dichloromethane/methanol as an elution system. The obtained crude product was purified by the HPLC (mobile phase: acetonitrile, water (0.1% ammonia water), elution gradient: 48%-78%), to obtain the title Compound **402-2** (62.82 mg, 111.63 µmol, yield: 39.27%) as white solid.

### Single-configuration Compound 402-2:

MS m/z (ESI):563.3 [M+1]
Chiral SFC: retention time: 0.891 min; chromatographic column: Chiralpak AD-3 50×4.6 mm I.D., 3 um; mobile phase: CO₂/MeOH (0.05% DEA): elution gradient: 5%-40%.

¹H NMR (400 MHz, CDCl₃) δ 7.83 - 7.75 (m, 1H), 7.56 - 7.45 (m, 1H), 7.23 (br s, 1H), 7.19 - 7.06 (m, 3H), 4.04 (s, 3H), 4.00 - 3.73 (m, 2H), 3.52 - 3.21 (m, 3H), 2.86 - 2.68 (m, 1H), 2.65 - 2.14 (m, 13H), 1.89 - 1.37 (m, 4H), 1.24 - 0.55 (m, 13H), -0.01 (d, J = 6.6 Hz, 2H).

### Examples 403-1 and 403-2

### N-ethyl-5-fluoro-2-(3-methyl-6-{1-[(2S)-3-methyl-1-(4-methylpiperazin-1-yl)butan-2-yl]azacyclobutan-3-yl}imidazo[1,5-a]pyridin-8-yl)-N-(isopropyl)benzamide 403-1

### N-ethyl-5-fluoro-2-(3-methyl-6-{1-[(2R)-3-methyl-1-(4-methylpiperazin-1-yl)butan-2-yl]azacyclobutan-3-yl}imidazo[1,5-a]pyridin-8-yl)-N-(isopropyl)benzamide 403-2

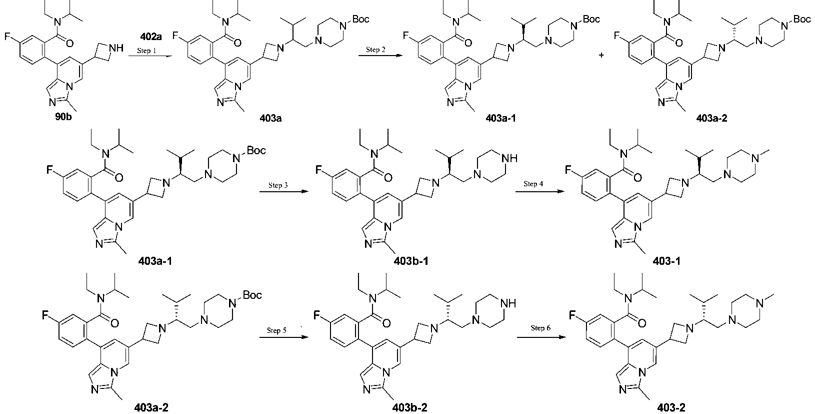

### Step 1

### 4-{2-[3-(8-{2-[ethyl(isopropyl)aminoformyl]-4-fluorophenyl}-3-methylimidazo[1,5-a]pyridin-6-yl)azacyclobutan-1-yl]-3-methylbutyl}piperazin-1-tert-butyl carboxylate 403a

Compound **90b** (500 mg, 1.27 mmol) and Compound **402a** (411.22 mg, 1.52 mmol) were dissolved in methanol (5.0 mL), followed by the addition of ZnCl₂ (345.50 mg, 2.53 mmol), and the mixture was stirred at 50°C for 1.0 hour. The mixture was cooled to room temperature, followed by adding sodium cyanoborohydride (238.95 mg, 3.80 mmol) and stirring at 50°C for 8.0 hour. A saturated aqueous sodium bicarbonate solution (10 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **403a** (500 mg, 770.59 µmol, yield: 60.80%) as yellow solid.
MS m/z (ESI): 649.7 [M+1]

### Step 2

### 4-[(2S)-2-[3-(8-{2-[ethyl(isopropyl)aminoformyl]-4-fluorophenyl}-3-methylimidazo[1,5-a]pyridin-6-yl)azacyclobutan-1-yl]-3-methylbutyl]piperazin-1-tert-butyl carboxylate 403a-1

### tert-butyl 4-[(2R)-2-[3-(8-{2-[ethyl(propan-2-yl)carbamoyl]-4-fluorophenyl}-3-methylimidazo[1,5-a]pyridin-6-yl)azetidin-1-yl]-3-methylbutyl]piperazine-1-carboxylate

### 4-[(2R)-2-[3-(8-{2-[ethyl(isopropyl)aminoformyl]-4-fluorophenyl}-3-methylimidazo[1,5-a]pyridin-6-yl)azacyclobutan-1-yl]-3-methylbutyl]piperazin-1-tert-butyl carboxylate 403a-2

Compound **403a** (350 mg, 926.15 µmol) was separated and purified by the chiral preparative SFC (preparative column: DAICEL CHIRALCEL OX (250 mm * 30 mm,10 um), mobile phase: carbon dioxide-methanol; elution gradient: 40%), to obtain the title Compounds **403a-1** (140 mg, 215.77 µmol, yield: 35.0%) and **403a-2** (140 mg, 215.77 µmol, yield: 35.0%).

### Single-configuration Compound 403a-1:

MS m/z (ESI): 649.5 [M+1]
Chiral HPLC analysis: retention time: 2.078 min; chromatographic column: Chiralcel OX-3 50 x 4.6 mm I.D., 3 um; mobile phase: carbon dioxide-isopropanol (0.05% diethylamide ): elution gradient: 5%-40%.

### Single-configuration Compound 403a-2:

MS m/z (ESI): 649.6 [M+1]
Chiral HPLC analysis: retention time: 2.267 min; chromatographic column: Chiralcel OX-3 50 x 4.6 mm I.D., 3 um; mobile phase: carbon dioxide-isopropanol (0.05% diethylamide ): elution gradient: 5%-40%.

### Step 3

### N-ethyl-5-fluoro-2-(3-methyl-6-{1-[(2S)-3-methyl-1-(piperazin-1-yl)butan-2-yl]azacyclobutan-3-yl}imidazo[1,5-a]pyridin-8-yl)-N-(isopropyl)benzamide 403b-1

Compound **403a-1** (100.00 mg, 154.12 µmol) was dissolved in dichloromethane (3.0 mL), followed by the addition of trifluoroacetic acid (1.48 g, 12.98 mmol, 1 mL). The mixture was stirred at 25°C to allow for reacting for 1.0 hours. The reaction mixture was concentrated under a reduced pressure to obtain the crude product of title Compound **403b-**1 (80 mg). The crude product was directly used in the next step of reaction without purification.
MS m/z (ESI): 549.4 [M+1]

### Step 4

### N-ethyl-5-fluoro-2-(3-methyl-6-{1-[(2S)-3-methyl-l-(4-methylpiperazin-1-yl)butan-2-yl]azacyclobutan-3-yl}imidazo[1,5-a]pyridin-8-yl)-N-(isopropyl)benzamide 403-1

The crude product of Compound **403b-1** (80 mg) and formaldehyde (54.91 mg, 603.54 µmol) were dissolved in methanol (3.0 mL), followed by the addition of triethylamine (24.43 mg, 241.41 µmol). Under the protection of nitrogen, the mixture was stirred at room temperature for 1.0 hour. Sodium cyanoborohydride (22.76 mg, 362.12 µmol) was added, allowing for reacting at room temperature for 11 hours under the protection of nitrogen. A saturated aqueous sodium bicarbonate solution (15 mL) was added to the reaction mixture, which was then extracted with dichloromethane (25 mL x 3). The organic phases were combined, dried over anhydrous sodium sulfate, and concentrated under a reduced pressure. The resulting residues were purified by the silica-gel column chromatography using dichloromethane/methanol as an elution system. The obtained crude product was purified by the HPLC (mobile phase: acetonitrile, water (0.1% ammonia water), elution gradient: 38%-68%), to obtain the title Compound **403-1** (44.46 mg, 79.00 µmol, yield: 65.45%).

### Single-configuration Compound 403-1:

MS m/z (ESI):563.6 [M+1]
Chiral SFC: retention time: 1.031 min; chromatographic column: Chiralcel OD-3 50×4.6 mm I.D., 3um,; mobile phase: CO₂/MeOH (0.05% DEA): elution gradient: 5%-40%.

¹H NMR (400 MHz, DMSO-*d*₆) δ 7.96 - 7.84 (m, 1H), 7.75 - 7.58 (m, 1H), 7.44 - 7.16 (m, 2H), 7.08 - 6.97 (m, 1H), 6.92 - 6.75 (m, 1H), 3.61 - 3.40 (m, 4H), 3.28 - 3.08 (m, 3H), 2.99 - 2.84 (m, 1H), 2.58 (s, 3H), 2.54 (s, 1H), 2.43 - 2.06 (m, 13H), 1.79 - 1.66 (m, 1H), 1.11 - 0.69 (m, 13H), 0.26 (d, J = 6.8 Hz, 2H).

### Step 5

### N-ethyl-5-fluoro-2-(3-methyl-6-{1-[(2R)-3-methyl-1-(piperazin-1-yl)butan-2-yl]azacyclobutan-3-yl}imidazo[1,5-a]pyridin-8-yl)-N-(isopropyl)benzamide 403b-2

Compound **402a-2** (120.00 mg, 184.94 µmol) was dissolved in dichloromethane (3.0 mL), followed by the addition of trifluoroacetic acid (1.48 g, 12.98 mmol, 1 mL). The mixture was stirred at 25°C to allow for reacting for 0.25 hours. The reaction mixture was concentrated under a reduced pressure to obtain the crude product of title Compound **403b-2** (100 mg). The crude product was directly used in the next step of reaction without purification.
MS m/z (ESI): 549.4 [M+1]

### Step 6

### N-ethyl-5-fluoro-2-(3-methyl-6-{1-[(2R)-3-methyl-1-(4-methylpiperazin-1-yl)butan-2-yl]azacyclobutan-3-yl}imidazo[1,5-a]pyridin-8-yl)-N-(isopropyl)benzamide 403-2

The crude product of Compound **403b-2** (100 mg) and formaldehyde (93.43 mg, 905.31 µmol) were dissolved in methanol (2.0 mL), followed by the addition of triethylamine (36.64 mg, 362.12 µmol). Under the protection of nitrogen, the mixture was stirred at room temperature for 0.5 hour. Sodium cyanoborohydride (34.13 mg, 543.18 µmol) was added, allowing for reacting at room temperature for 2.0 hours under the protection of nitrogen. A saturated aqueous sodium bicarbonate solution (10 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system. The obtained crude product was purified by the HPLC (mobile phase: acetonitrile, water (0.1% ammonia water), elution gradient: 38%-68%), to obtain the title Compound **403-2** (26.96 mg, 47.91 µmol, yield: 26.46%) as white solid.

### Single-configuration Compound 403-2:

MS m/z (ESI):563.8 [M+1]
Chiral SFC: retention time: 1.523 min; chromatographic column: Chiralcel OX-3 50×4.6 mm I.D., 3um,; mobile phase: CO₂/MeOH (0.05% DEA): elution gradient: 5%-40%.

¹H NMR (400 MHz, DMSO-*d*₆) δ 7.97 - 7.82 (m, 1H), 7.76 - 7.61 (m, 1H), 7.46 - 7.27 (m, 2H), 7.09 - 6.97 (m, 1H), 6.93 - 6.75 (m, 1H), 4.43 - 3.33 (m, 5H), 3.31 - 3.26 (m, 1H), 3.22 - 2.72 (m, 3H), 2.58 (s, 3H), 2.43 - 2.05 (m, 13H), 1.80 - 1.67 (m, 1H), 1.11 - 0.70 (m, 13H), 0.31 - 0.23 (m, 2H).

### Example 408

### 2-(6-{1-[(3R)-6-(4-ethanesulfonamidopiperidin-1-yl)-2-methylhexan-3-yl]azacyclobutan-3-yl}-3-methylimidazo[1,5-a]pyridin-8-yl)-N-ethyl-5-fluoro-N-(isopropyl)benzamide 408

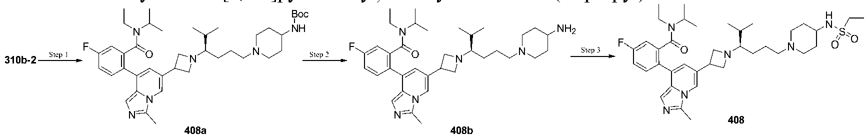

### Step 1

### N-{1-(4R)-4-[3-(8-{2-[ethyl(isopropyl)aminoformyl]-4-fluorophenyl}-3-methylimidazo[1,5-a]pyridin-6-yl)azacyclobutan-1-yl]-5-methylhexyl}piperidin-4-yl}tert-butyl carbamate 408a

Compound **310b-2** (180 mg, 355.27 µmol) and N-(piperidin-4-yl)tert-butyl carbamate (142.31 mg, 710.55 µmol) were dissolved in methanol (4.0 mL), followed by the addition of acetic acid (21.33 mg, 355.27 µmol), and under the protection of nitrogen, the mixture was stirred at 50°C for 0.5 hours. Sodium cyanoborohydride (66.98 mg, 1.07 mmol) was added, and the mixture was stirred at 50°C to allow for reacting for 12 hours under the protection of nitrogen. A saturated aqueous sodium bicarbonate solution (15 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title product **408a** (180 mg, 260.52 µmol, yield: 73.33%) as yellow solid.
MS m/z (ESI):691.5 [M+1]

### Step 2

### 2-(6-{1-[(3R)-6-(4-aminopiperidin-1-yl)-2-methylhexan-3-yl]azacyclobutan-3-yl}-3-methylimidazo[1,5-a]pyridin-8-yl)-N-ethyl-5-fluoro-N-(isopropyl)benzamide 408b

Compound **408a** (180 mg, 260.52 µmol) was dissolved in dichloromethane (3.0 mL), followed by the addition of trifluoroacetic acid (1.48 g, 12.98 mmol, 1 mL) at 25°C. The mixture was stirred at room temperature to allow for reacting for 0.5 hours. The reaction mixture was concentrated under a reduced pressure, dissolved by adding DCM (10 mL), regulated to PH = 10 by adding a 10% aqueous sodium hydroxide solution, and extracted with dichloromethane (15 mL x 3). The organic phases were combined, dried over anhydrous sodium sulfate and filtered, and the filtrate was concentrated under a reduced pressure, to obtain the crude product of title Compound **408b** (150 mg, 253.89 µmol, yield: 97.45%) as yellow solid. The crude product was directly used in the next step of reaction without purification.
MS m/z (ESI):591.5 [M+1]

### Step 3

### 2-(6-{1-[(3R)-6-(4-ethanesulfonamidopiperidin-1-yl)-2-methylhexan-3-yl]azacyclobutan-3-yl}-3-methylimidazo[1,5-a]pyridin-8-yl)-N-ethyl-5-fluoro-N-(isopropyl)benzamide 408

**408b** (150.00 mg, 253.89 µmol) was dissolved in dichloromethane (3.0 mL), followed by the addition of triethylamine (77.07 mg, 761.66 µmol), and the mixture was cooled in an ice bath while ethanesulfonyl chloride (39.17 mg, 304.66 µmol) was added, allowing for a reaction at room temperature for 12 hours under the protection of nitrogen. A saturated aqueous sodium bicarbonate solution (15 mL) was added to the reaction mixture, which was then extracted with dichloromethane (20 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient:36%-66%). The title Compound **408** (29.44 mg, 43.11 µmol, yield: 16.98%) was obtained as white solid.
MS m/z (ESI): 683.4 [M+1]
¹H NMR (400 MHz, DMSO-*d*₆) δ 7.95 - 7.89 (m, 1H), 7.81 - 7.56 (m, 1H), 7.48 - 7.21 (m, 2H), 7.18 - 6.91 (m, 2H), 6.87 - 6.58 (m, 1H), 3.60 - 3.48 (m, 3H), 3.04 - 2.90 (m, 4H), 2.77 - 2.67 (m, 2H), 2.61 - 2.56 (m, 3H), 2.55 - 2.53 (m, 4H), 2.25 - 2.13 (m, 2H), 2.05 - 1.98 (m, 1H), 1.93 - 1.80 (m, 2H), 1.79 - 1.66 (m, 3H), 1.48 - 1.35 (m, 4H), 1.27 - 1.06 (m, 6H), 0.93 - 0.71 (m, 12H), 0.30 - 0.24 (m, 2H).

### Example 414

### N-ethyl-5-fluoro-2-{6-[1-(1-methoxy-3-methylbutan-2-yl)azacyclobutan-3-yl]-3-methylimidazo[1,5-a]pyridin-8-yl}-N-(isopropyl)benzamide 414

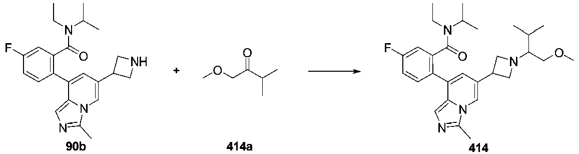

Compound **90b** (110 mg, 278.85 µmol) and 1-methoxy-3-methylbutan-2-one **414a** (64.78 mg, 557.69 µmol) were dissolved in methanol (3.0 mL), followed by the addition of ZnCl₂ (76.01 mg, 557.69 µmol), and the mixture was stirred at 50°C for 1.0 hour. The reaction mixture was cooled to room temperature, followed by adding sodium cyanoborohydride (43.81 mg, 697.11 µmol) and stirring at 50°C for 8.0 hours. The reaction mixture was filtered, and the filtrate was concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 38%-68%). The title Compound **414** (25 mg, 50.54 µmol, yield: 18.13%) was obtained.
MS m/z (ESI):495.2 [M+1]
¹H NMR (400 MHz, DMSO-*d*₆) δ 7.97 - 7.88 (m, 1H), 7.73 - 7.62 (m, 1H), 7.44 - 7.26 (m, 2H), 7.07 - 7.00 (m, 1H), 6.84 - 6.72 (m, 1H), 3.62 - 3.40 (m, 4H), 3.32 - 3.07 (m, 8H), 2.97 - 2.87 (m, 1H), 2.59 (s, 3H), 2.30 - 2.24 (m, 1H), 1.79 - 1.66 (m, 1H), 1.10 - 0.72 (m, 13H), 0.31 - 0.24 (m, 2H).

### Example 415

### N-ethyl-5-fluoro-2-{1-[(1-{6-[4-(2-hydroxyethyl)piperazin-1-yl]-2-methylhexan-3-yl}azacyclobutan-3-yl)methyl]-7-methyl-2-oxo-1H,2H,3H-imidazo[4,5-c]pyridin-3-yl} N-(isopropyl)benzamide 415

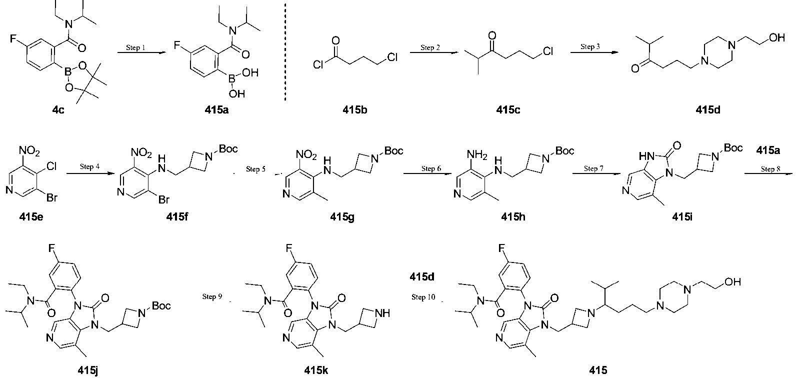

### Step 1

### {2-[ethyl(isopropyl)aminoformyl]-4-fluorophenyl}boric acid 415a

Compound **4c** (1 g, 1.49 mmol) was dissolved in DCM (5.0 mL), followed by the slow addition of trifluoroacetic acid (510.20 mg, 4.47 mmol) and methylboric acid (267.85 mg, 4.47 mmol), and the mixture was stirred at 25°C to allow for reacting for 2.0 hours. The reaction mixture was concentrated under a reduced pressure. The resulting residues were purified by the reverse phase column chromatography (column: Spherical C18, 20-35 µm, 100 A, 120 g; mobile phase: acetonitrile, water, elution gradient: 50%-60%), to obtain the title Compound **415a** (320 mg, 1.26 mmol, yield: 84.77%).
MS m/z (ESI): 236.2 [M-F+H]⁺

### Step 2

### 6-chloro-2-methylhexan-3-one 415c

4-Chlorobutyryl chloride **415b** (30 g, 212.77 mmol) was dissolved in anhydrous tetrahydrofuran (500 mL), followed by the addition of the tetrahydrofuran solution (100 mL) of iron (III) acetylacetonate (1.80 g, 5.11 mmol), and the mixture was stirred for 10 min. The mixture was cooled to 0°C, followed by the slow dropwise addition of isopropylmagnesium chloride (2 M, 107 mL). The reaction mixture was stirred at 0°C for 0.5 hours. A saturated aqueous ammonium chloride solution (100 mL) was then slowly added, and the mixture was fully stirred. The mixture was concentrated under a reduced pressure to remove tetrahydrofuran, and the remaining aqueous phase was extracted with ethyl acetate (100 mL x 3). The organic phases were combined, washed with saturated brine, dried over anhydrous sodium sulfate, and concentrated under a reduced pressure. The resulting residues were purified by the silica-gel column chromatography using petroleum ether/ethyl acetate as an elution system, to obtain the title Compound **415c** (21.4 g, 143.98 mmol, yield: 67.67%) as colorless oil.

¹H NMR (400 MHz, CDCl₃) δ 3.55 - 3.61 (m, 2 H), 2.53 - 2.71 (m, 3 H), 2.01 - 2.09 (m, 2 H), 1.10 (d, J=7.20 Hz, 6 H).

### Step 3

### 6-[4-(2-hydroxyethyl)piperazine -1-yl]-2-methylhexan-3-one 415d

Compound **415c** (11.4 g, 69.03 mmol) was dissolved in acetonitrile (120 mL), followed by the addition of 2-(piperazine -1-yl)ethanol (8.99 g, 69.03 mmol) and DIPEA(26.68 g, 206.44 mmol). Under a nitrogen atmosphere, the mixture was stirred at 80°C to allow for reacting for 16.0 hours. The reaction mixture was cooled to room temperature, and concentrated under a reduced pressure. The residues was added to a saturated aqueous potassium carbonate solution (200 mL), and the resulting mixture was extracted with dichloromethane (100 mL x 3). The organic phase was dried over anhydrous sodium sulfate, and concentrated under a reduced pressure. The resulting residues were purified by the silica-gel column chromatography using dichloromethane/methanol as an elution system, to obtain the title Compound **415d** (17.4 g, 71.80 mmol, yield: 104%) as brown oil.
MS m/z (ESI): 243.1 [M+1]
¹H NMR (400 MHz, DMSO-*d*₆) δ 4.36 (br s, 1 H), 3.46 (br s, 2 H), 2.57 - 2.65 (m, 1 H), 2.45 (br t, J=6.80 Hz, 2 H), 2.22 - 2.40 (m, 10 H), 2.18 (br t, J=7.20 Hz, 2 H), 1.59 (br t, J=6.80 Hz, 2 H), 0.99 (d, J=6.80 Hz, 6 H).

### Step 4

### 3-{[(3-bromo-5-nitropyridin-4-yl)amino]methyl}azacyclobutan-1-tert-butyl carboxylate 415f

3-Bromo-4-chloro-5-nitropyridine **415e** (7.0 g, 29.48 mmol) and 3-(aminomethyl)azacyclobutan-1-tert-butyl carboxylate (4.39 g, 23.59 mmol) were dissolved in tetrahydrofuran (50 mL), followed by the addition of triethylamine (5.97 g, 58.96 mmol). The mixture was stirred at room temperature to allow for reacting for 12 hours. The reaction mixture was diluted with ethyl acetate (150.0 mL), and washed with a saturated aqueous ammonium chloride solution (150 mL x 3). The organic phase was dried over anhydrous sodium sulfate and filtered, and the filtrate was concentrated under a reduced pressure. The resulting residues were purified by the silica-gel column chromatography using petroleum ether/ethyl acetate as an elution system, to obtain the title Compound **415f** (8.7 g, 22.47 mmol, yield: 76.21%) as yellow solid.

¹H NMR (400 MHz, CDCl₃) δ 8.98 (s, 1H), 8.52 (s, 1H), 4.10 (t, J = 8.5 Hz, 2H), 3.84 (dd, J = 5.4, 7.1 Hz, 2H), 3.68 (dd, J = 4.9, 8.9 Hz, 2H), 2.84 (tddd, J = 2.5, 5.1, 7.6, 12.7 Hz, 1H), 1.45 (s, 9H).

### Step 5

### 3-{[(3-methyl-5-nitropyridin-4-yl)amino]methyl}azacyclobutan-1-tert-butyl carboxylate 415g

Compound **415f** (4 g, 10.33 mmol), methylboric acid (1.24 g, 20.66 mmol) and potassium phosphate (6.58 g, 30.99 mmol) were dissolved in dioxane (40 mL). CataCXium A Pd-G3 (752.29 mg, 1.03 mmol) was added. Under a nitrogen atmosphere, the mixture was stirred at 100°Cto allow for reacting for 12.0 hours. The reaction mixture was cooled to room temperature, followed by adding ethyl acetate (150 mL) and washing with water (150 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **415g** (2.2 g, 6.82 mmol, yield: 66.07%) as yellow solid.
MS m/z (ESI): 323.1 [M+1]

### Step 6

### 3-{[(3-amino-5-methylpyridin-4-yl)amino]methyl}azacyclobutan-1-tert-butyl carboxylate 415h

Compound **415g** (1.9 g, 5.89 mmol) was dissolved in water (4.0 mL) and ethanol (20 mL). Ammonium chloride (1.58 g, 29.47 mmol) was added, and iron powder (3.29 g, 58.94 mmol) was added while stirring was performed. The reaction mixture was heated to 80°C and stirred for 1.0 hour. The mixture was diluted with ethyl acetate (250 mL), and washed with water (250 mL x 3). The organic phase was dried over anhydrous sodium sulfate, and concentrated under a reduced pressure. The resulting residues were purified by the HPLC (mobile phase: acetonitrile, water (0.1% ammonia water), elution gradient: 20%-50%), to obtain the title Compound **415h** (550 mg, 1.71 mmol, yield: 28.95%) as white solid.
MS m/z (ESI): 293.2[M+1]

### Step 7

### 3-({7-methyl-2-oxo-1H,2H,3H-imidazo[4,5-c]pyridin-1-yl}methyl)azacyclobutan-1-tert-butyl carboxylate 415i

Compound **415h** (500 mg, 1.71 mmol) was dissolved in acetonitrile (10 mL), followed by the addition of 1,1-carbonyldiimidazole (831.89 mg, 2.57 mmol, 50%purity) and DIPEA(265.23 mg, 2.05 mmol). The mixture was stirred at 25°C to allow for reacting for 12.0 hours. The reaction mixture was diluted with ethyl acetate (50 mL), and extracted with water (50 mL x 3). The organic phase was dried over anhydrous sodium sulfate, and concentrated under a reduced pressure. The resulting residues were purified by the silica-gel column chromatography using petroleum ether/ethyl acetate as an elution system, to obtain the title Compound **415i** (470 mg, 1.48 mmol, yield: 86.33%) as white solid.
MS m/z (ESI): 319.1[M+1]

### Step 8

### tert-butyl3-[(3-{2-[ethyl(isopropyl)aminoformyl]-4-fluorophenyl}7-methyl-2-oxo-1H,2H,3H-imidazol[4,5-c]pyridin-1-yl)methyl]azacyclobuten-1-tert-butyl carboxylate 415j

Compound **415i** (100 mg, 314.10 µmol) was dissolved in dichloromethane (4.0 mL). Compound **415a** (1.24 g, 20.66 mmol), copper acetate (28.53 mg, 157.05 µmol), 2,2'-bipyridine (24.53 mg, 157.05 µmol) and cesium carbonate (204.68 mg, 628.20 µmol) were added. 4Å molecular sieve (100 mg) was added, and the mixture was stirred at 25°C to allow for reacting for 3.0 hours under an oxygen atmosphere. Ethyl acetate (30 mL) was added to the reaction mixture, which was then washed with water (30 mL x 3). The organic phase was dried over anhydrous sodium sulfate, and concentrated under a reduced pressure. The resulting residues were purified by the silica-gel column chromatography using petroleum ether/ethyl acetate as an elution system, to obtain the title Compound **415j** (80 mg, 152.20 µmol, yield: 48.46%) as oil.
MS m/z (ESI): 526.3[M+1]

### Step 9

### 2-{1-[(azacyclobutan-3-yl)methyl]-7-methyl-2-oxo-1H,2H,3H-imidazo[4,5-c]pyridin-3-yl}-N-ethyl-5-fluoro-N-(isopropyl)benzamide 415k

Compound **415j** (70 mg, 133.18 µmol) was dissolved in dichloromethane (2.0 mL), followed by the addition of trifluoroacetic acid (2.07 g, 18.17 mmol, 1.40 mL) at 25°C. The mixture was stirred at room temperature to allow for reacting for 1.0 hours. The reaction mixture was concentrated under a reduced pressure, then dissolved by adding (3.0 mL), followed by adding a 10% aqueous sodium hydroxide solution (10 mL) and extracting with dichloromethane (10 mL x 2). The organic phases were combined, dried over anhydrous sodium sulfate and filtered, and the filtrate was concentrated under a reduced pressure, to obtain the crude product of title Compound **415k** (56 mg, 131.61 µmol, yield: 98.82%) as brown oil. The crude product was directly used in the next step of reaction without purification.
MS m/z (ESI):426.4 [M+1]

### Step 10

### N-ethyl-5-fluoro-2-{1-[(1-{6-[4-(2-hydroxyethyl)piperazin-1-yl]-2-methylhexan-3-yl}azacyclobutan-3-yl)methyl]-7-methyl-2-oxo-1H,2H,3H-imidazo[4,5-c]pyridin-3-yl} N-(isopropyl)benzamide 415

The crude product of Compound **415k** (56 mg, 131.61 µmol) and Compound **415d** (31.90 mg, 131.61 µmol) were dissolved in methanol (2.0 mL), followed by the addition of acetic acid (7.90 mg, 131.61 µmol), and the mixture was stirred at 50°C for 0.5 hours under the protection of nitrogen.Sodium cyanoborohydride (24.81 mg, 394.83 µmol) was added, allowing for reacting at 50°C for 12.0 hours under the protection of nitrogen. Ethyl acetate (20 mL) was added to the reaction mixture, which was then washed with water (20 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 22%-52%), to obtain the title Compound **415** (23.76 mg, 36.45 µmol, yield: 27.7%).
MS m/z (ESI):652.4 [M+1]
¹H NMR (400 MHz, DMSO-*d*₆) δ 8.04 (s, 1H), 7.81 - 7.76 (m, 1H), 7.60 (dd, J = 4.8, 9.2 Hz, 1H), 7.52 - 7.41 (m, 2H), 4.40 - 4.15 (m, 3H), 3.78 - 3.68 (m, 1H), 3.46 (t, J = 6.4 Hz, 3H), 3.17 - 3.11 (m, 2H), 3.03 - 2.89 (m, 3H), 2.69 - 2.65 (m, 1H), 2.58 - 2.54 (m, 4H), 2.41 - 2.24 (m, 9H), 2.20 - 2.14 (m, 2H), 1.94 - 1.88 (m, 1H), 1.70 - 1.58 (m, 1H), 1.43 - 1.31 (m, 2H), 1.21 - 0.96 (m, 8H), 0.87 - 0.74 (m, 7H), 0.72 - 0.63 (m, 2H).

### Examples 416-1 and 416-2

### N-ethyl-5-fluoro-2-(6-{1-[(2R)-1-[4-(2-hydroxyethyl)piperazin-1-yl]-3-methylbutan-2-yl]azacyclobutan-3-yl}-1-methyl-1H-indazol-4-yl)-N-(isopropyl)benzamide 416-1

### N-ethyl-5-fluoro-2-(6-{1-[(2S)-1-[4-(2-hydroxyethyl)piperazin-1-yl]-3-methylbutan-2-yl]azacyclobutan-3-yl}-1-methyl-1H-indazol-4-yl)-N-(isopropyl)benzamide 416-2

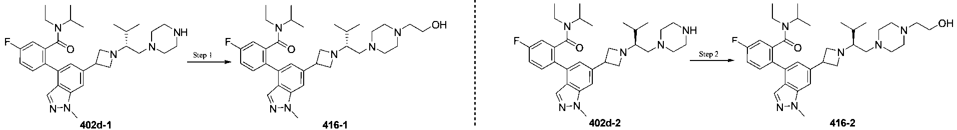

### Step 1

### N-ethyl-5-fluoro-2-(6-{1-[(2R)-[-[4-(2-hydroxyethyl)piperazin-1-yl]-3-methylbutan-2-yl]azacyclobutan-3-yl}-1-methyl-1H-indazol-4-yl)-N-(isopropyl)benzamide 416-1

Compound **402d-1** (150 mg, 273.36 µmol), 2-bromoethanol (40.99 mg, 328.03 µmol), potassium carbonate (113.34 mg, 820.07 µmol) and sodium iodide (4.10 mg, 27.34 µmol) were dissolved in acetonitrile (3.0 mL). Under the protection of nitrogen, the mixture was stirred at 85°C for 2.0 hours. A saturated aqueous sodium bicarbonate solution (15 mL) was added to the reaction mixture, which was then extracted with dichloromethane (25 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 37%-67%), to obtain the title Compound **416-1** (29.73 mg, 50.15 µmol, yield: 18.35%) as white solid.

### Single-configuration Compound 416-1:

MS m/z (ESI):593.9 [M+1]
Chiral SFC: retention time: 1.177 min; chromatographic column: Chiralcel OD-3 50×4.6 mm I.D., 3 um; mobile phase: CO₂/MeOH (0.05% DEA): elution gradient: 5%-40%.

¹H NMR (400 MHz, DMSO-*d*₆) δ 7.84 - 7.75 (m, 1H), 7.63 - 7.47 (m, 2H), 7.42 - 7.24 (m, 2H), 7.18 - 7.03 (m, 1H), 4.35 - 4.28 (m, 1H), 4.04 (s, 3H), 3.73 - 3.61 (m, 3H), 3.49 - 3.34 (m, 3H), 3.29 - 3.10 (m, 3H), 2.91 - 2.54 (m, 2H), 2.45 - 2.24 (m, 10H), 2.18 - 2.12 (m, 2H), 1.82 - 1.67 (m, 1H), 1.02 - 0.65 (m, 13H), 0.08 (d, J = 5.2 Hz, 2H).

### Step 2

### N-ethyl-5-fluoro-2-(6-{1-[(2S)-1-[4-(2-hydroxyethyl)piperazin-1-yl]-3-methylbutan-2-yl]azacyclobutan-3-yl}-1-methyl-1H-indazol-4-yl)-N-(isopropyl)benzamide 416-2

Compound **402d-2** (100 mg, 182.24 µmol), 2-bromoethanol (27.33 mg, 218.68 µmol) and potassium carbonate (113.34 mg, 820.07 µmol) were dissolved in acetonitrile (2.0 mL). Under the protection of nitrogen, the mixture was stirred at 85°C for 2.0 hours. A saturated aqueous sodium bicarbonate solution (20 mL) was added to the reaction mixture, which was then extracted with dichloromethane (20 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 37%-67%), to obtain the title Compound **416-2** (20.98 mg, 35.39 µmol, yield: 19.42%) as yellow solid.

### Single-configuration Compound 416-2:

MS m/z (ESI):593.5 [M+1]
Chiral SFC: retention time: 1.157 min; chromatographic column: Chiralpak OD-3 50×4.6 mm I.D., 3um,; mobile phase: CO₂/MeOH (0.05% DEA): elution gradient: 5%-40%.

¹H NMR (400 MHz, DMSO-*d*₆) δ 7.92 - 7.68 (m, 1H), 7.65 - 7.45 (m, 2H), 7.44 - 7.23 (m, 2H), 7.18 - 7.04 (m, 1H), 4.35 - 4.21 (m, 1H), 4.12 - 3.93 (m, 3H), 3.72 - 3.59 (m, 3H), 3.48 - 3.33 (m, 3H), 3.31 - 3.07 (m, 4H), 2.89 - 2.59 (m, 2H), 2.45 - 2.20 (m, 10H), 2.18 - 2.12 (m, 2H), 1.83 - 1.71 (m, 1H), 1.26 - 0.96 (m, 1H), 0.92 - 0.80 (m, 8H), 0.71 - 0.64 (m, 3H), 0.11 - 0.04 (m, 2H).

### Example 421

### N-ethyl-5-fluoro-2-{6-[1-(1-hydroxyl-3-methylbutan-2-yl)azacyclobutan-3-yl]-3-methylimidazo[1,5-a]pyridin-8-yl}-N-(isopropyl)benzamide 421

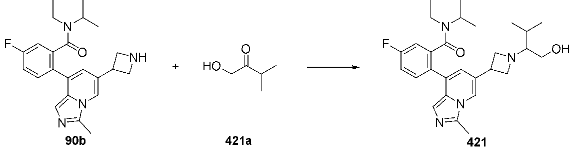

Compound **90b** (100 mg, 253.50 µmol) and 1-hydroxyl-3-methylbutan-2-one **421a** (51.78 mg, 506.99 µmol) were dissolved in methanol (3.0 mL), followed by the addition of ZnCl₂ (69.10 mg, 506.99 µmol), and the mixture was stirred at 55°C for 1.0 hour. The reaction mixture was cooled to room temperature, followed by adding sodium cyanoborohydride (47.79 mg, 760.49 µmol) and stirring at 55°C for 11 hours. A saturated aqueous sodium bicarbonate solution (15 mL) was added to the reaction mixture, which was then extracted with dichloromethane (25 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 30%-60%). The title Compound 421 (1.16 mg, 2.41 µmol) was obtained.
MS m/z (ESI):481.2 [M+1]
¹H NMR (400 MHz, CD₃OD) δ 7.93 - 7.81 (m, 1H), 7.75 - 7.64 (m, 1H), 7.37 - 7.26 (m, 1H), 7.24 - 7.17 (m, 1H), 7.12 (s, 1H), 6.94 - 6.84 (m, 1H), 3.89 - 3.33 (m, 9H), 3.13 - 2.96 (m, 1H), 2.65 (s, 3H), 2.39 - 2.26 (m, 1H), 1.97 - 1.77 (m, 1H), 1.14 - 0.78 (m, 13H), 0.33 (s, 2H).

### Example 424

### N-ethyl-5-fluoro-2-(6-{1-[(2S)-1-[4-(2-fluoroethyl)piperazin-1-yl]-3-methylbutan-2-yl]azacyclobutan-3-yl}-1-methyl-1H-indazol-4-yl)-N-(isopropyl)benzamide 424

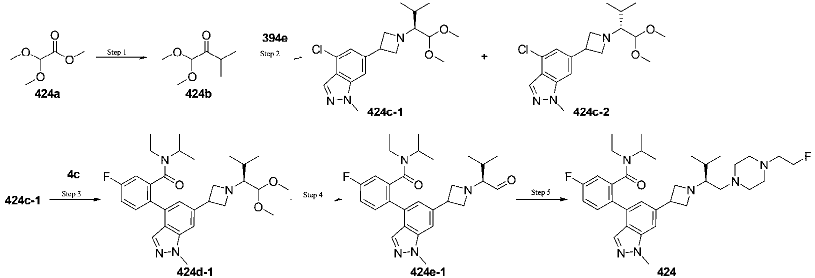

### Step 1

### 1,1-dimethoxy-3-methylbutan-2-one 424b

Under the protection of nitrogen, 2,2-dimethoxymethyl acetate (2 g, 14.91 mmol) and *N*-methoxymethylamine (1.82 g, 18.64 mmol) were dissolved in tetrahydrofuran (20 mL), and cooled to -40°C under the protection of nitrogen, followed by the slow dropwise addition of isopropylmagnesium bromide solution (3.0 M, 12.43 mL). After the completion of dropwise addition, the mixture was further stirred at -40°C for 2.0 hours. The reaction mixture was quenched by adding water (1.0 mL) and a saturated aqueous ammonium chloride solution (1.0 mL), then diluted by adding water (50 mL), and extracted with ethyl acetate (50 mL×3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **424b** (800 mg, 5.47 mmol, yield: 36.70%) as colorless oil.

¹H NMR (400 MHz, CDCl₃) δ 4.55 (s, 1H), 3.33 (s, 6H), 2.99 - 2.89 (m, 1H), 1.03 (d, J = 6.9 Hz, 6H).

### Step 2

### 4-chloro-6-{1-[(2S)-1,1-dimethoxy-3-methylbutan-2-yl]azacyclobutan-3-yl}-1-methyl-1H-indazole 424c-1

### 4-chloro-6-{1-[(2R)-1,1-dimethoxy-3-methylbutan-2-yl]azacyclobutan-3-yl}-1-methyl-1H-indazole 424c-2

Compound **394e** (344 mg, 1.55 mmol) and Compound **424b**(415.87 mg, 1.71 mmol) were dissolved in methanol (20 mL), and stirred at 50°C for 1.0 hour. The mixture was cooled to room temperature, followed by adding sodium cyanoborohydride (292.54 mg, 4.66 mmol) and stirring at 50°C for 11 hour. A saturated aqueous sodium bicarbonate solution (20 mL) was added to the reaction mixture, which was then extracted with dichloromethane (10 mL x 3). The organic phases were combined, dried over anhydrous sodium sulfate, and concentrated under a reduced pressure. The residues were purified by the silica-gel column chromatography using dichloromethane/methanol as an elution system, and the obtained crude product was separated and purified by the chiral preparative HPLC (preparative column: Phenomenex-Cellulose-2 (250 mm * 30 mm,5 um), mobile phase: carbon dioxide-methanol (0.1 ammonia water); elution gradient: 17%), to obtain the title Compounds **424c-1** (208 mg, 591.13 µmol, yield: 38.09%) and **424c-2** (247 mg, 701.96 µmol, yield: 45.24%).

### Single-configuration Compound 424c-1:

MS m/z (ESI): 352.0 [M+1]
Chiral HPLC analysis: retention time: 1.587 min; chromatographic column: Cellulose-2 50 x 4.6 mm I.D., 3 um; mobile phase: carbon dioxide-methanol (0.05% diethylamide): elution gradient: 5%-40%.

### Single-configuration Compound 424c-2:

MS m/z (ESI): 352.0 [M+1]
Chiral HPLC analysis: retention time: 1.474 min; chromatographic column: Cellulose-2 50 x 4.6 mm I.D., 3 um; mobile phase: carbon dioxide-methanol (0.05% diethylamide): elution gradient: 5%-40%.

### Step 3

### 2-(6-{1-[(2S)-1,1-dimethoxy-3-methylbutan-2-yl]azacyclobutan-3-yl}-1-methyl-1H-indazol-4-yl)-N-ethyl-5-fluoro-N-(isopropyl)benzamide 424d-1

Compound **424c-1** (208 mg, 591.13 µmol), Compound **4c**(495.39 mg, 886.69 µmol) and potassium phosphate (376.43 mg, 1.77 mmol) were dissolved in dioxane (10 mL) and water (2.0 mL). Pd(dtbpf)Cl₂ (38.53 mg, 59.11 µmol) was added. Under a nitrogen atmosphere, the mixture was stirred at 100°Cto allow for reacting for 2.0 hours. The reaction mixture was cooled to room temperature, followed by adding water (50 mL) and extracting with ethyl acetate (50 mL x 3). The organic phases were combined, dried over anhydrous sodium sulfate, and concentrated under a reduced pressure. The resulting residues were purified by the preparative column chromatography using dichloromethane/methanol as an elution system, to obtain the title Compound **424d-1** (310 mg, 590.85 µmol, yield: 99.95%) as oil.
MS m/z (ESI): 525.3

### Step 4

### N-ethyl-5-fluoro-2-(1-methyl-6-{1-[(2S)-3-methyl-1-oxabutan-2-yl]azacyclobutan-3-yl}-1H-indazol-4-yl)-N-(isopropyl)benzamide 424e-1

Compound **424d-1** (330 mg, 628.97 µmol) was dissolved in dichloromethane (3.0 mL), followed by the addition of trifluoroacetic acid (8.88 g, 77.88 mmol, 6 mL). The mixture was stirred at 30°C to allow for reacting for 24 hours. The reaction mixture was concentrated under a reduced pressure, regulated to PH = 10 with a 10% aqueous sodium hydroxide solution, and extracted with dichloromethane (15 mL x 3). The organic phases were combined, dried over anhydrous sodium sulfate, and concentrated under a reduced pressure, to obtain the crude product of title Compound **424e-1** (311 mg, 626.24 µmol, yield: 99.57%). The crude product was directly used in the next step of reaction without purification.
MS m/z (ESI): 497.4 [M+18+1]

### Step 5

### N-ethyl-5-fluoro-2-(6-{1-[(2S)-1-[4-(2-fluoroethyl)piperazin-1-yl]-3-methylbutan-2-yl]azacyclobutan-3-yl}-1-methyl-1H-indazol-4-yl)-N-(isopropyl)benzamide 424

Compound **424e-1** (240 mg, 483.28µmol) and 1-(2-fluoroethyl)piperazine (99.42 mg, 752.20 µmol) were dissolved in methanol (3.0 mL), followed by the addition of acetic acid (30.12 mg, 501.46 µmol). Under the protection of nitrogen, the mixture was stirred at 35°C for 1.0 hours. Sodium cyanoborohydride (94.54 mg, 1.50 mmol) was added, allowing for reacting at room temperature for 12 hours under the protection of nitrogen. A saturated aqueous sodium bicarbonate solution (30 mL) was added to the test reaction mixture, which was then extracted with dichloromethane (50 mL x 3). The organic phases were combined, dried over anhydrous sodium sulfate, and concentrated under a reduced pressure. The resulting residues were purified by the silica-gel column chromatography using dichloromethane/methanol as an elution system. The obtained crude product was purified by the HPLC (mobile phase: acetonitrile, water (0.1% ammonia water), elution gradient: 40%-70%), to obtain the title Compound **424** (3.1 mg, 5.21 µmol, yield: 2.08%) as yellow solid.

### Single-configuration Compound 424

MS m/z (ESI):595.4 [M+1]
Chiral SFC: retention time: 1.032 min; chromatographic column: Chiralcel OD-3 50×4.6 mm I.D., 3um,; mobile phase: CO₂/MeOH (0.05% DEA): elution gradient: 5%-40%.

¹H NMR (400 MHz, DMSO-*d*₆) δ 7.80 (s, 1H), 7.65 - 7.47 (m, 2H), 7.44 - 7.25 (m, 2H), 7.18 - 7.04 (m, 1H), 4.59 - 4.50 (m, 1H), 4.46 - 4.39 (m, 1H), 4.04 (s, 3H), 3.74 - 3.59 (m, 3H), 3.27 - 3.15 (m, 2H), 2.89 - 2.54 (m, 3H), 2.44 - 2.15 (m, 9H), 1.83 - 1.70 (m, 1H), 1.33 - 0.94 (m, 4H), 0.94 - 0.78 (m, 9H), 0.74 - 0.57 (m, 4H), 0.11 - 0.05 (m, 2H).

### Example 427

### 4-[3-(8-{2-[ethyl(isopropyl)aminoformyl]-4-fluorophenyl}-3-methylimidazo[1,5-a]pyridin-6-yl)azacyclobutan-1-yl]-5-methylhexanoic acid 427

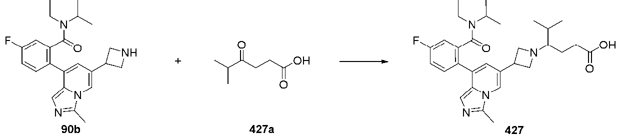

Compound **90b** (119 mg, 301.66 µmol) and 5-methyl-4-oxohexanoic acid **427a** (52.19 mg, 361.99 µmol) were dissolved in methanol (10 mL), followed by the addition of ZnCl₂ (82.23 mg, 603.32 µmol), and the mixture was stirred at 50°C for 1.0 hour. The reaction mixture was cooled to room temperature, followed by adding sodium cyanoborohydride (56.87 mg, 904.98 µmol) and stirring at 50°C for 11 hours. A saturated aqueous ammonium chloride solution (50 mL) was added to the reaction mixture, which was then extracted with ethyl acetate (100 mL x 3). The organic phases were combined, dried over anhydrous sodium sulfate, and concentrated under a reduced pressure. The resulting residues were purified by the HPLC (mobile phase: acetonitrile, water (0.1% hydrochloric acid), elution gradient:2%-32%)the residues were purified . The title Compound **427** (14.74 mg, 28.20 µmol, yield: 9.35%) was obtained as white solid.
MS m/z (ESI):523.3 [M+1]
¹H NMR (400 MHz, CDCl₃) δ 8.81 - 8.41 (m, 1H), 7.95 - 7.81 (m, 1H), 7.77 - 7.66 (m, 1H), 7.47 - 7.15 (m, 3H), 4.76 - 4.11 (m, 6H), 3.72 - 3.35 (m, 3H), 3.13 - 2.96 (m, 4H), 2.55 (br t, J = 7.2 Hz, 2H), 2.27 - 2.12 (m, 1H), 2.07 - 1.76 (m, 2H), 1.15 - 0.86 (m, 13H), 0.68 - 0.50 (m, 2H).

### Example 431

### N-ethyl-5-fluoro-2-(6-{1-[(2S)-1-[4-(2-hydroxylethyl)piperazin-1-yl]-3-methylbutan-2-yl]azacyclobutan-3-yl}-3methylimidazo[1,5-a]pyridin-8-yl)-N-(isopropyl)benzamide 431

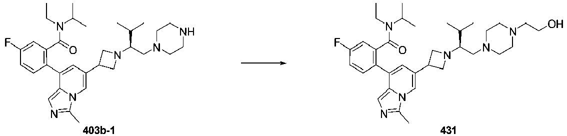

Compound **403b-1** (101 mg, 184.06 µmol) was dissolved in acetonitrile (5.0 mL), followed by the addition of potassium carbonate (97.90 mg, 552.18 µmol) and 2-bromoethanol (27.60 mg, 220.87 µmol). Under the protection of nitrogen, the mixture was stirred at 80°C to allow for reacting for 12 hours. Water (50 mL) was added to the reaction mixture, which was then extracted with ethyl acetate (100 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 28%-58%), to obtain the title Compound **431** (34.21 mg, 57.71 µmol, yield: 31.35%) as yellow solid.
MS m/z (ESI):593.3 [M+1]
Chiral SFC: retention time: 1.129 min; chromatographic column: Chiralcel OD-3 50×4.6 mm I.D., 3um,; mobile phase: CO₂/MeOH (0.05% DEA): elution gradient: 5%-40%.

¹H NMR (400 MHz, CDCl₃) δ 7.92 (d, J = 4.0 Hz, 1H), 7.72 - 7.64 (m, 1H), 7.43 - 7.27 (m, 2H), 7.07 - 7.00 (m, 1H), 6.91 - 6.74 (m, 1H), 4.39 - 4.27 (m, 1H), 3.64 - 3.39 (m, 6H), 3.32 - 2.89 (m, 4H), 2.59 (s, 3H), 2.47 - 2.09 (m, 13H), 1.80 - 1.67 (m, 1H), 1.07 - 0.72 (m, 13H), 0.27 (br d, J = 6.4 Hz, 2H).

### Example 433

### 2-(6-{1-[(3R)-6-[4-(1,3-dihydroxylpropan-2-yl)piperazin-1-yl]-2-methylhexan-3-yl]azacyclobutan-3-yl}-1-methyl-1H-indazol-4-yl)N-ethyl-5-fluoro-N-(isopropyl)benzamide 433

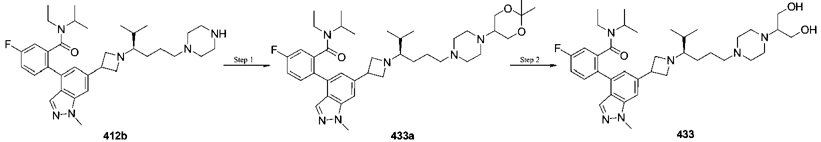

### Step 1

### 2-(6-{1-[(3R)-6-[4-(2,2-dimethyl-1,3-dioxacyclohexan-5-yl)piperazin-1-yl]-2-methylhexan-3-yl]azacyclobutan-3-yl} -1-methyl-1H-indazol-4-yl)-N-ethyl-5-fluoro-N-(isopropyl)benzamide 433a

Compound **412b** (130 mg, 225.39 µmol) and 2,2-dimethyl-1,3-dioxacyclohexan-5-one (44.00 mg, 338.08 µmol) were dissolved in methanol (3.0 mL). ZnCl₂ (61.44 mg, 450.77 µmol) was added, and the mixture was stirred at 50°C for 1.0 hour under the protection of nitrogen. Sodium cyanoborohydride (42.49 mg, 676.16 µmol) was added, allowing for reacting at 50°C for 8.0 hours under the protection of nitrogen. A saturated aqueous sodium bicarbonate solution (10 mL) was added to the reaction mixture, which was then extracted with dichloromethane (15 mL x 3). The organic phases were combined, dried over anhydrous sodium sulfate, and concentrated under a reduced pressure, to obtain the crude product of title Compound **433a** (150 mg, 217.10 µmol, yield: 96.34%) as yellow solid. The crude product was directly used in the next step of reaction without purification.
MS m/z (ESI):691.6[M+1]

### Step 2

### 2-(6-{1-[(3R)-6-[4-(1,3-dihydroxylpropan-2-yl)piperazin-1-yl]-2-methylhexan-3-yl]azacyclobutan-3-yl}-1-methyl-1H-indazol-4-yl)N-ethyl-5-fluoro-N-(isopropyl)benzamide 433

Compound **433a** (150 mg, 217.10 µmol) was dissolved in tetrahydrofuran (1.0 mL), followed by the addition of HCl (1 M, 1 mL), and the mixture was stirred at 25°C for 1.0 hour. A saturated aqueous sodium bicarbonate solution (10 mL) was added to the reaction mixture, which was then extracted with dichloromethane (15 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 33%-63%), to obtain the title Compound **433** (23.64 mg, 36.32 µmol, yield: 16.73%) as yellow solid.
MS m/z (ESI):651.4 [M+1]
Chiral SFC: retention time: 1.305 min; chromatographic column: Chiralpak OD-3 50×4.6 mm I.D., 3um,; mobile phase: CO₂/MeOH (0.05% DEA): elution gradient: 5%-40%.

¹H NMR (400 MHz, DMSO-*d*₆) δ 7.84 - 7.78 (m, 1H), 7.63 - 7.50 (m, 2H), 7.42 - 7.35 (m, 1H), 7.33 - 7.26 (m, 1H), 7.14 - 7.01 (m, 1H), 4.22 - 4.16 (m, 2H), 4.07 - 4.02 (m, 3H), 3.70 - 3.59 (m, 3H), 3.50 - 3.36 (m, 5H), 3.31 - 3.23 (m, 1H), 3.20 - 3.06 (m, 2H), 2.87 - 2.78 (m, 1H), 2.62 - 2.55 (m, 4H), 2.44 - 2.38 (m, 1H), 2.33 - 2.25 (m, 3H), 2.22 - 2.17 (m, 2H), 2.06 - 2.00 (m, 1H), 1.78 - 1.68 (m, 1H), 1.51 - 1.39 (m, 2H), 1.31 - 1.20 (m, 2H), 1.04 - 0.63 (m, 14H), 0.10 - 0.06 (m, 2H).

### Example 436

### 5-fluoro-N-methyl-2-(3-methyl-6-{1-[2-methyl-6-(4-methylpiperazin-1-yl)hexan-3-yl]azacyclobutan-3-yl}imidazo[1,5-a]pyridin-8-yl)-N-(isopropyl)benzamide 436

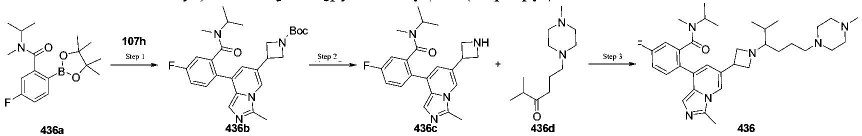

### Step 1

### 3-(8-{4-fluoro-2-[methyl(isopropyl)aminoformyl]phenyl}-3-methylimidazo[1,5-a]pyridin-6-yl)azacyclobutan-1-tert-butyl carboxylate 436b

Compound **107h** (1.0 g, 3.11 mmol), *N*-methyl-5-fluoro-*N*-isopropyl-2-(tetramethyl-1,3,2-dioxaboran-2-yl)benzamide **436a**(synthesized with reference to the synthesis method of Compound **4c**, 2.50 g, 4.66 mmol) and potassium phosphate (1.66 g, 7.82 mmol) were dissolved in dioxane (20 mL) and water (4.0 mL). Pd(dtbpf)Cl₂ (202.53 mg, 310.75 µmol) was added. Under a nitrogen atmosphere, the mixture was stirred at 95°Cto allow for reacting for 12.0 hours. The reaction mixture was cooled to room temperature, followed by adding water (30 mL) and extracting with ethyl acetate (30 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **436b** (650 mg, 1.35 mmol, yield: 43.53%) as brown solid.
MS m/z (ESI): 481.3 [M+1]

### Step 2

### 2-[6-(azacyclobutan-3-yl)-3-methylimidazo[1,5-a]pyridin-8-yl]-5-fluoro-N-methyl-N-(isopropyl)benzamide 436c

Compound **436b** (150 mg, 312.13 µmol) was dissolved in methane dioxide (3.0 mL), followed by the addition of trifluoroacetic acid (1.48 g, 12.98 mmol, 1 mL) at 0°C. The mixture was stirred at room temperature to allow for reacting for 0.5 hours. The reaction mixture was concentrated under a reduced pressure, dissolved by adding 20 mL of dichloromethane, regulated to PH = 10 with a 10% aqueous sodium hydroxide solution, and extracted with dichloromethane (20 mL x 3). The organic phases were combined, dried over anhydrous sodium sulfate, and concentrated under a reduced pressure, to obtain the crude product of title Compound **436c** (110 mg, 289.13 µmol, 92.63%). The crude product was directly used in the next step of reaction without purification.
MS m/z (ESI): 381.0 [M+1]

### Step 3

### 5-fluoro-N-methyl-2-(3-methyl-6-{1-[2-methyl-6-(4-methylpiperazin-1-yl)hexan-3-yl]azacyclobutan-3-yl}imidazo[1,5-a]pyridin-8-yl)-N-(isopropyl)benzamide 436

Compound **436c** (120 mg, 315.41 µmol) and 2-methyl-6-(4-methylpiperazin-1-yl)hexan-3-one **436d** (100 mg, 470.96 µmol) were dissolved in methanol (3.0 mL). ZnCl₂ (85.98 mg, 630.82 µmol)was added, and the mixture was stirred at 50°C for 0.5 hours. Sodium cyanoborohydride (59.46 mg, 946.23 µmol) was added, and the mixture was stirred at 50°C for 11.0 hours. A saturated aqueous sodium bicarbonate solution (10 mL) was added, and the mixture was then extracted with dichloromethane (15 mL × 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 30%-60%), to obtain the title Compound **436** (47.6 mg, 82.53 µmol, yield: 26.16 %) as yellow solid.
MS m/z (ESI): 577.4[M+1]
¹H NMR (400 MHz, DMSO-*d*₆) δ 7.92 (s, 1H), 7.78 - 7.66 (m, 1H), 7.44 - 7.26 (m, 2H), 7.11 - 7.01 (m, 1H), 6.89 - 6.69 (m, 1H), 4.61 - 3.44 (m, 4H), 3.20 - 2.97 (m, 2H), 2.69 - 2.56 (m, 5H), 2.38 - 1.98 (m, 15H), 1.79 - 1.65 (m, 1H), 1.52 - 1.37 (m, 2H), 1.30 - 1.14 (m, 2H), 1.06 - 0.58 (m, 11H), 0.27 - 0.22 (m, 1H).

### Example 437

### 2-(6-{1-[(5S)-5,6-dihydroxyl-2-methylhexan-3-yl]azacyclobutan-3-yl}-3-methylimidazo[1,5-a]pyridin-8-yl)-N-ethyl-5-fluoro-N-(isopropyl)benzamide 437

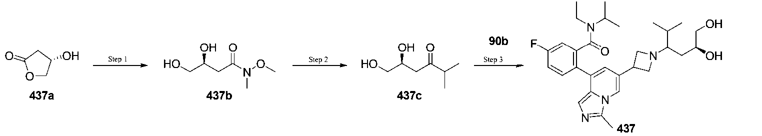

### Step 1

### (3S)-3,4-dihydroxyl-N-methoxy-N-methylbutyramide 437b

N-methoxymethylamine (8.60 g, 88.16 mmol, hydrochloride) was dissolved in dichloromethane (30 mL), and cooled to 0°C under the protection of nitrogen, followed by the slow dropwise addition of AlMe₃ (2.0 M, 44.08 mL). After the completion of dropwise addition, the mixture was further stirred at 0°C for 0.5 hours. Under the protection of nitrogen, the mixture was cooled to -10°C, followed by the slow dropwise addition of the solution of (4S)-4-hydroxyltetrahydrofuran-2-one **437a** (3 g, 29.39 mmol) in dichloromethane (20 mL). The reaction mixture was stirred at room temperature for 3.0 hours. The reaction mixture was quenched by adding sodium sulfate decahydrate while being cooled in an ice-water bath, and stirred for 1.0 hour. The mixture was filtered, and the filtrate was dried over anhydrous sodium sulfate and concentrated under a reduced pressure. The resulting residues were purified by the silica-gel column chromatography using petroleum ether/ethyl acetate as an elution system, to obtain the title Compound **437b** (2.2 g, 13.48 mmol, yield: 45.88%) as colorless oil.

¹H NMR (400 MHz, DMSO-*d*₆) δ 4.66 - 4.54 (m, 2H), 3.91 - 3.83 (m, 1H), 3.65 (s, 3H), 3.31 - 3.17 (m, 2H), 3.08 (s, 3H), 2.48 - 2.39 (m, 2H).

### Step 2

### (5S)-5,6-dihydroxyl-2-methylhexan-3-one 437c

Under the protection of nitrogen, an isopropyllithium solution (1.0 M, 9.19 mL) was added to a reaction flask, and cooled to -78°C under the protection of nitrogen, followed by the slow dropwise addition of the solution of Compound **437b** (500 mg, 3.06 mmol) in tetrahydrofuran (10 mL). After the completion of dropwise addition, the mixture was further stirred at -78°C for 8.0 hours. The reaction mixture was quenched by adding water (1.0 mL) and a saturated aqueous ammonium chloride solution (1.0 mL), then diluted by adding water (50 mL), and extracted with ethyl acetate (50 mL×3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **437c** (60 mg, 410.44 µmol, yield: 13.39%) as colorless oil.

¹H NMR (400 MHz, DMSO-*d*₆) δ 4.04 - 3.94 (m, 1H), 3.49 - 3.29 (m, 3H), 3.14 - 3.03 (m, 1H), 2.63 (td, J = 7.2, 14.0 Hz, 1H), 2.58 (d, J = 6.4 Hz, 2H), 1.04 (dd, J = 0.4, 6.8 Hz, 6H).

### Step 3

### 2-(6-{1-[(5S)-5,6-dihydroxyl-2-methylhexan-3-yl]azacyclobutan-3-yl}-3-methylimidazo[1,5-a]pyridin-8-yl)-N-ethyl-5-fluoro-N-(isopropyl)benzamide 437

Compound **90b** (160 mg, 405.59 µmol) and Compound **437c**(59.29 mg, 405.59 µmol) were dissolved in methanol (10.0 mL), followed by the addition of ZnCl₂ (110.56 mg, 811.19 µmol), and the mixture was stirred at 50°C for 1.0 hour. The mixture was cooled to room temperature, followed by adding sodium cyanoborohydride (76.46 mg, 1.22 mmol) and stirring at 50°C for 11 hour. A saturated aqueous ammonium chloride solution (50 mL) was added to the reaction mixture, which was then extracted with ethyl acetate (100 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 20%-50%), to obtain the title Compound **437** (24.57 mg, 46.83 µmol, yield: 11.55%) as white solid.
MS m/z (ESI): 525.3 [M+1]
¹H NMR (400 MHz, DMSO-*d*₆) δ 7.99 - 7.86 (m, 1H), 7.72 - 7.64 (m, 1H), 7.42 - 7.28 (m, 2H), 7.05 - 7.00 (m, 1H), 6.82 - 6.69 (m, 1H), 4.59 - 4.21 (m, 2H), 3.64 - 3.38 (m, 5H), 3.30 - 2.67 (m, 6H), 2.59 (s, 3H), 2.30 (br s, 1H), 1.92 - 1.69 (m, 1H), 1.48 - 0.95 (m, 3H), 0.93 - 0.72 (m, 12H), 0.29 (dd, J = 6.8, 13.2 Hz, 2H).

### Example 438

### 2-(6-{1-[(5R)-5,6-dihydroxyl-2-methylhexan-3-yl]azacyclobutan-3-yl}-3-methylimidazo[1,5-a]pyridin-8-yl)-N-ethyl-5-fluoro-N-(isopropyl)benzamide 438

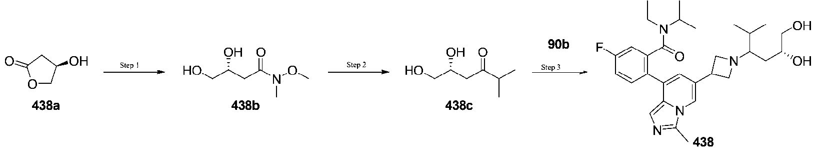

### Step 1

### (3R)-3,4-dihydroxyl-N-methoxy-N-methylbutyramide 438b

*N*-methoxymethylamine (3.73 g, 38.20 mmol, hydrochloride) was dissolved in dichloromethane (45 mL), and cooled to 0°C under the protection of nitrogen, followed by the slow dropwise addition of AlMe₃ (2.0 M, 19.10 mL). After the completion of dropwise addition, the mixture was further stirred at 0°C for 0.5 hours. Under the protection of nitrogen, the mixture was cooled to -10°C, followed by the slow dropwise addition of the solution of (4R)-4-hydroxyltetrahydrofuran-2-one **438a** (1.3 g, 12.73 mmol) in dichloromethane (10 mL). The reaction mixture was stirred at room temperature for 3.0 hours. The reaction mixture was quenched by adding sodium sulfate decahydrate while being cooled in an ice-water bath, and stirred for 1.0 hour. The mixture was filtered, and the filtrate was dried over anhydrous sodium sulfate and concentrated under a reduced pressure. The resulting residues were purified by the silica-gel column chromatography using petroleum ether/ethyl acetate as an elution system, to obtain the title Compound **438b** (970 mg, 5.94 mmol, yield: 46.68%) as colorless oil.

¹H NMR (400 MHz, DMSO-*d*₆) δ 4.62 (d, J = 5.2 Hz, 1H), 4.57 (t, J = 5.6 Hz, 1H), 3.92 - 3.82 (m, 1H), 3.65 (s, 3H), 3.33 - 3.22 (m, 2H), 3.08 (s, 3H), 2.48 - 2.38 (m, 2H).

### Step 2

### (5R)-5,6-dihydroxyl-2-methylhexan-3-one 438c

Under the protection of nitrogen, Compound **438b**(410 mg, 2.51 mmol) was dissolved in tetrahydrofuran (10 mL), and cooled to -78°C under the protection of nitrogen, followed by the slow dropwise addition of an isopropyllithium solution (1.0 M, 7.54 mL). After the completion of dropwise addition, the mixture was further stirred at -78°C for 8.0 hours. The reaction mixture was quenched by adding water (1.0 mL) and a saturated aqueous ammonium chloride solution (1.0 mL), then diluted by adding water (50 mL), and extracted with ethyl acetate (50 mL×3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **438c** (35 mg, 239.42 µmol, yield: 9.53%) as colorless oil.

¹H NMR (400 MHz, DMSO-*d*₆) δ 4.04 - 3.94 (m, 1H), 3.46 - 3.32 (m, 2H), 3.26 - 3.04 (m, 1H), 2.63 (td, J = 6.9, 13.9 Hz, 1H), 2.58 (d, J = 6.0 Hz, 2H), 1.04 (dd, J = 0.8, 7.2 Hz, 6H).

### Step 3

### 2-(6-{1-[(5R)-5,6-dihydroxyl-2-methylhexan-3-yl]azacyclobutan-3-yl}-3-methylimidazo[1,5-a]pyridin-8-yl)-N-ethyl-5-fluoro-N-(isopropyl)benzamide 438

Compound **90b** (119 mg, 301.66 µmol) and Compound **438c**(35 mg, 239.42 µmol) were dissolved in methanol (10.0 mL), followed by the addition of ZnCl₂ (82.23 mg, 603.32 µmol), and the mixture was stirred at 50°C for 1.0 hour. The reaction mixture was cooled to room temperature, followed by adding sodium cyanoborohydride (56.87 mg, 904.98 µmol) and stirring at 50°C for 11 hours. A saturated aqueous ammonium chloride solution (50 mL) was added to the reaction mixture, which was then extracted with ethyl acetate (100 mL x 3). The organic phases were combined, dried over anhydrous sodium sulfate, and concentrated under a reduced pressure. The resulting residues were purified by the preparative thin-layer chromatography using dichloromethane/methanol as an elution system, to obtain the title Compound **438** (35.13 mg, 66.96 µmol, yield: 22.2%) as white solid.
MS m/z (ESI): 525.4 [M+1]
¹H NMR (400 MHz, DMSO-*d*₆) δ 7.98 - 7.89 (m, 1H), 7.72 - 7.63 (m, 1H), 7.42 - 7.28 (m, 2H), 7.18 - 7.00 (m, 1H), 6.81 - 6.70 (m, 1H), 4.55 - 4.21 (m, 2H), 3.65 - 3.39 (m, 5H), 3.28 - 3.03 (m, 4H), 2.96 - 2.82 (m, 1H), 2.60 (s, 3H), 2.36 - 2.25 (m, 1H), 1.85 - 1.69 (m, 1H), 1.47 - 1.17 (m, 3H), 1.13 - 1.03 (m, 1H), 0.92 - 0.75 (m, 12H), 0.33 - 0.26 (m, 2H).

### Example 439

### N-ethyl-5-fluoro-2-(3-methyl-6-{1-[3-methyl-1-(morpholin-4-yl)butan-2-yl]azacyclobutan-3-yl}imidazo[1,5-a]pyridin-8-yl)-N-(isopropyl)benzamide 439

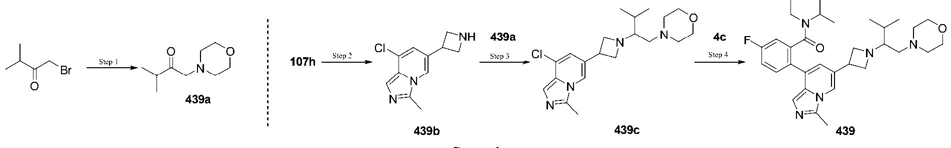

### Step 1

### 3-methyl-1-(morpholin-4-yl)butan-2-one 439a

Morpholine (500 mg, 5.74 mmol) was dissolved in acetonitrile (10 mL), followed by the addition of 1-bromo-3-methyl-but-2-one (947.13 mg, 5.74 mmol) and potassium carbonate (2.38 g, 17.22 mmol) at 25°C. The reaction mixture was heated to 80°C and stirred for 12 hours. The reaction mixture was cooled to room temperature, followed by adding water (20 mL) and extracting with ethyl acetate (20 mL x 3). The organic phases were combined, washed with water (20 mL x 3), dried over anhydrous sodium sulfate, and concentrated under a reduced pressure. The resulting residues were purified by the silica-gel column chromatography using petroleum ether/ethyl acetate as an elution system, to obtain the title Compound **439a** (930 mg, 5.46 mmol, yield: 95.18%) as yellow oil.
MS m/z (ESI): 282.1 [M+1]
¹H NMR (400 MHz, DMSO-*d*₆) δ 3.59 - 3.56 (m, 4H), 3.27 (s, 2H), 2.73 (td, J = 6.9, 13.8 Hz, 1H), 2.41 - 2.36 (m, 4H), 0.99 (d, J = 6.9 Hz, 6H).

### Step 2

### 3-{8-chloro-3-methylimidazo[1,5-a]pyridin-6-yl}azacyclobutane 439b

Compound **107h** (330 mg, 1.03 mmol) was dissolved in dichloromethane (5.0 mL), followed by the addition of trifluoroacetic acid (1.48 g, 12.98 mmol, 1 mL) at 25°C. The mixture was stirred at room temperature to allow for reacting for 1.0 hours. The reaction mixture was concentrated under a reduced pressure, followed by adding dichloromethane (20 mL), regulating to pH = 11 with a 10% aqueous sodium hydroxide solution, and extracting with dichloromethane (20 mL x 3). The organic phases were combined, dried over anhydrous sodium sulfate, and concentrated under a reduced pressure, to obtain the crude product of title Compound **439b** (200 mg, 902.18 µmol, yield: 87.98%) as yellow solid. The crude product was directly used in the next step of reaction without purification.

### Step 3

### 4-[2-(3-{8-chloro-3-methylimidazo[1,5-a]pyridin-6-yl}azacyclobutan-1-yl)-3-methylbutyl]morpholine 439c

Compound **439b** (220 mg, 992.40 µmol) and Compound **439a**(186.93 mg, 1.09 mmol) were dissolved in methanol (3.0 mL), followed by the addition of ZnCl₂ (270.53 mg, 1.98 mmol), and the mixture was stirred at 50°C for 0.5 hours. The mixture was cooled to room temperature, followed by adding sodium cyanoborohydride (187.09 mg, 2.98 mmol) and stirring at 50°C for 12 hour. A saturated aqueous sodium bicarbonate solution (50 mL) was added to the reaction mixture, which was then extracted with ethyl acetate (50 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **439c** (90 mg, 238.78 µmol, yield: 24.06%).
MS m/z (ESI): 377.3 [M+1]

### Step 4

### N-ethyl-5-fluoro-2-(3-methyl-6-{1-[3-methyl-1-(morpholin-4-yl)butan-2-yl]azacyclobutan-3-yl}imidazo[1,5-a]pyridin-8-yl)-N-(isopropyl)benzamide 439

Compound **439c** (130 mg, 344.90 µmol), Compound **4c**(138.74 mg, 413.88 µmol) and potassium phosphate (219.63 mg, 1.03 mmol) were dissolved in dioxane (2.0 mL) and water (0.5 mL). Pd(dtbpf)Cl₂ (22.48 mg, 34.49 µmol) was added. Under a nitrogen atmosphere, the mixture was stirred at 100°Cto allow for reacting for 12 hours. The reaction mixture was cooled to room temperature, followed by adding water (50 mL) and extracting with ethyl acetate (50 mL x 3). The organic phases were combined, dried over anhydrous sodium sulfate, and concentrated under a reduced pressure. The resulting residues were purified by the HPLC (mobile phase: acetonitrile, water (0.1% formic acid), elution gradient: 4%-34%), to obtain the title Compound **439** (13.14 mg, 23.90 µmol, yield: 6.93%) as white solid. MS m/z (ESI): 550.3

¹H NMR (400 MHz, DMSO-*d*₆) δ 7.95 (s, 1H), 7.69 (d, J = 6.4 Hz, 1H), 7.47 - 7.41 (m, 1H), 7.18 - 7.04 (m, 2H), 6.82 - 6.77 (m, 1H), 6.60 - 6.53 (m, 1H), 3.42 - 3.35 (m, 3H), 3.20 - 3.04 (m, 6H), 3.01 - 2.97 (m, 1H), 2.93 - 2.88 (m, 1H), 2.74 - 2.64 (m, 1H), 2.38 - 2.31 (m, 3H), 2.21 - 2.13 (m, 2H), 2.11 - 2.02 (m, 3H), 1.96 - 1.90 (m, 2H), 1.58 - 1.46 (m, 1H), 0.70 - 0.60 (m, 6H), 0.60 - 0.51 (m, 6H), 0.50 - 0.45 (m, 1H), 0.04 - -0.01 (m, 2H).

### Examples 441-1 and 441-2

### 2-(6-{1-[(2S)-1-(1,1-dioxo-thiomorpholin-4-yl)-3-methylbutan-2-yl]azacyclobutan-3-yl}-3-methylimidazo[1,5-a]pyridin-8-yl)-N-ethyl-5-fluoro- N-(isopropyl)benzamide 441-1

### 2-(6-{1-[(2R)-1-(1,1-dioxo-thiomorpholin-4-yl)-3-methylbutan-2-yl]azacyclobutan-3-yl}-3-methylimidazo[1,5-a]pyridin-8-yl)-N-ethyl-5-fluoro- N-(isopropyl)benzamide 441-2

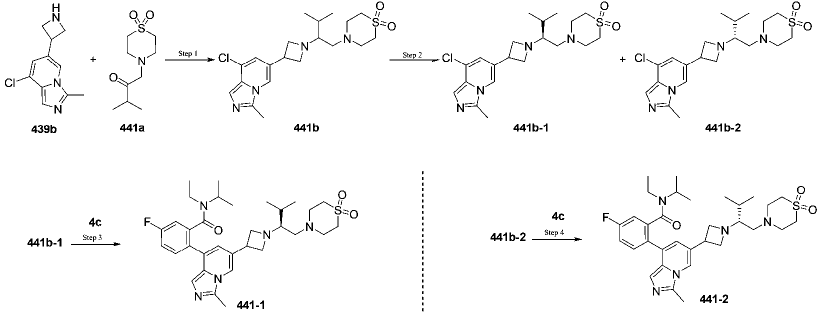

### Step 1

### 4-[2-(3-{8-chloro-3-methylimidazo[1,5-a]pyridin-6-yl}azacyclobutan-1-yl)-3-methylbutyl]-thiomorpholin-1,1-dione 441b

Compound **439b** (230 mg, 1.04 mmol) and 4-(3-methyl-2-oxobutyl)thiomorpholin-1,1-dione **441a** (250.28 mg, 1.14 mmol, synthesized with reference to the synthesis route of Compound **439a** by taking thiomorpholin-1,1-dione as a starting material) were dissolved in methanol (3.0 mL), followed by the addition of ZnCl₂ (282.82 mg, 2.08 mmol), and the mixture was stirred at 50°C for 1.0 hour. The mixture was cooled to room temperature, followed by adding sodium cyanoborohydride (195.60 mg, 3.11 mmol) and stirring at 50°C for 8.0 hour. A saturated aqueous sodium bicarbonate solution (10 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **441b** (280 mg, 658.84 µmol, yield: 63.50%).
MS m/z (ESI): 425.2 [M+1]

### Step 2

### 4-[(2S)-2-(3-{8-chloro-3-methylimidazo[1,5-a]pyridin-6-yl}azacyclobutan-1-yl)-3-methylbutyl]-thiomorpholin-1,1-dione 441b-1

### 4-[(2R)-2-(3-{8-chloro-3-methylimidazo[1,5-a]pyridin-6-yl}azacyclobutan-1-yl)-3-methylbutyl]-thiomorpholin-1,1-dione 441b-2

Compound **441b**(280 mg, 658.84 µmol) was separated and purified by the chiral preparative SFC (preparative column: DAICEL CHIRALPAK AS(250 mm * 30 mm, 10 um), mobile phase: carbon dioxide-isopropanol (0.1% ammonia water); elution gradient: 50%). The title Compound **441b-1** (120 mg, 282.36 µmol, yield: 42.86%) and the title Compound **441b-2** (120 mg, 282.36 µmol, yield: 42.86%) were obtained.

### Single-configuration Compound 441b-1:

MS m/z (ESI): 425.0 [M+1]
Chiral HPLC analysis: retention time: 1.882 min; chromatographic column: Chiralcel AS-3 50 x 4.6 mm I.D., 3 um; mobile phase: carbon dioxide-isopropanol (0.05% diethylamide ): elution gradient: 5%-40%.

### Single-configuration Compound 441b-2:

MS m/z (ESI): 425.0[M+1]
Chiral HPLC analysis: retention time: 2.128 min; chromatographic column: Chiralcel AS-3 50 x 4.6 mm I.D., 3 um; mobile phase: carbon dioxide-isopropanol (0.05% diethylamide ): elution gradient: 5%-40%.

### Step 3

### 2-(6-{1-[(2S)-1-(1,1-dioxo-thiomorpholin-4-yl)-3-methylbutan-2-yl]azacyclobutan-3-yl}-3-methylimidazo[1,5-a]pyridin-8-yl)-N-ethyl-5-fluoro- N-(isopropyl)benzamide 441-1

Compound **441b-1** (120 mg, 282.36 µmol), Compound **4c**(236.63 mg, 423.54 µmol) and potassium phosphate (179.81 mg, 847.09 µmol) were dissolved in dioxane (3.0 mL) and water (0.5 mL). Pd(dtbpf)Cl₂ (18.40 mg, 28.24 µmol) was added. Under a nitrogen atmosphere, the mixture was stirred at 100°Cto allow for reacting for 2 hours. The reaction mixture was cooled to room temperature, followed by adding a saturated aqueous sodium bicarbonate solution (10 mL) and extracting with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 33%-63%), to obtain the title Compound **441-1** (82.47 mg, 137.96 µmol, yield: 48.86%).
MS m/z (ESI): 598.3 [M+1]
Chiral HPLC analysis: retention time: 1.205 min; chromatographic column: Chiralcel OX-3 50 x 4.6mm I.D., 3 um; mobile phase: carbon dioxide-ethanol (0.05% diethylamide): elution gradient: 40%.

¹H NMR (400 MHz, DMSO-*d*₆) δ 8.00 - 7.85 (m, 1H), 7.74 - 7.63 (m, 1H), 7.43 - 7.27 (m, 2H), 7.09 - 6.99 (m, 1H), 6.94 - 6.77 (m, 1H), 3.65 - 3.35 (m, 5H), 3.25 - 3.16 (m, 2H), 3.12 - 2.74 (m, 9H), 2.61 - 2.55 (m, 3H), 2.42 - 2.19 (m, 3H), 1.80 - 1.66 (m, 1H), 1.09 - 0.67 (m, 13H), 0.28 - 0.19 (m, 2H).

### Step 4

### 2-(6-{1-[(2R)-1-(1,1-dioxo-thiomorpholin-4-yl)-3-methylbutan-2-yl]azacyclobutan-3-yl}-3-methylimidazo[1,5-a]pyridin-8-yl)-N-ethyl-5-fluoro- N-(isopropyl)benzamide 441-2

Compound **441b-2** (120 mg, 282.36 µmol), Compound **4c**(189.31 mg, 338.83 µmol) and potassium phosphate (179.81 mg, 847.09 µmol) were dissolved in dioxane (3.0 mL) and water (0.5 mL). Pd(dtbpf)Cl₂ (18.40 mg, 28.24 µmol) was added. Under a nitrogen atmosphere, the mixture was stirred at 100°Cto allow for reacting for 2 hours. The reaction mixture was cooled to room temperature, followed by adding a saturated aqueous sodium bicarbonate solution (10 mL) and extracting with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient: 30%-60%), to obtain the title Compound **441-2** (83.34 mg, 139.41 µmol, yield: 49.37%).
MS m/z (ESI): 598.3 [M+1]
Chiral HPLC analysis: retention time: 2.020 min; chromatographic column: Chiralcel OX-3 50 x 4.6mm I.D., 3 um; mobile phase: carbon dioxide-ethanol (0.05% diethylamide): elution gradient: 40%.

¹H NMR (400 MHz, DMSO-*d*₆) δ 7.98 - 7.89 (m, 1H), 7.73 - 7.64 (m, 1H), 7.42 - 7.27 (m, 2H), 7.08 - 7.00 (m, 1H), 6.94 - 6.79 (m, 1H), 3.66 - 3.36 (m, 5H), 3.26 - 3.14 (m, 2H), 3.10 - 2.72 (m, 9H), 2.63 - 2.55 (m, 3H), 2.43 - 2.18 (m, 3H), 1.81 - 1.66 (m, 1H), 1.10 - 0.68 (m, 13H), 0.28 - 0.18 (m, 2H).

### Example 445

### 2-(6-{1-[1-(1,4-dioxane -2-yl)-3-methylbutan-2-yl]azacyclobutan-3-yl}-3-methylimidazo[1,5-a]pyridin-8-yl)-N-ethyl-5-fluoro-N-(isopropyl)benzamide 445

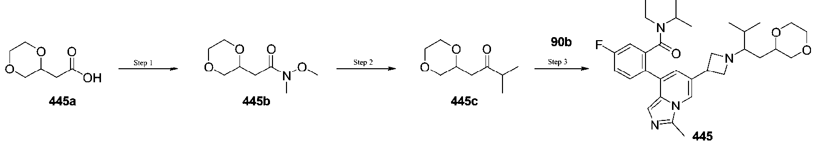

### Step 1

### 2-(1,4-dioxane -2-yl)-N-methoxy-N-methylacetamide 445b

2-(1,4-Dioxane -2-yl)acetic acid (500 mg, 3.42 mmol) was dissolved in dichloromethane (10 mL), followed by the addition of triethylamine (1.04 g, 10.26 mmol), EDCI(787.05 mg, 4.11 mmol) and HOBT (554.77 mg, 4.11 mmol). After the reaction mixture was stirred, *N*-methoxymethylamine (367.11 mg, 3.76 mmol, hydrochloride) was added. The reaction mixture was stirred at room temperature for 3.0 hours. The reaction mixture was quenched by adding a saturated aqueous sodium bicarbonate solution (20 mL), and extracted with dichloromethane (20 mL × 3). The organic phases were combined, dried over anhydrous sodium sulfate, and concentrated under a reduced pressure. The resulting residues were purified by the preparative thin-layer chromatography using petroleum ether/ethyl acetate as an elution system, to obtain the title Compound **445b** (200 mg, 1.06 mmol, yield: 31%).

¹H NMR (400 MHz, DMSO-*d*₆) δ 3.90 - 3.82 (m, 1H), 3.75 - 3.39 (m, 9H), 3.12 - 3.02 (m, 3H), 2.65 - 2.53 (m, 1H), 2.40 - 2.28 (m, 1H).

### Step 2

### 1-(1,4-dioxane -2-yl)-3-methylbutan-2-one 445c

Under the protection of nitrogen, Compound **445b** (200 mg, 1.06 mmol) was dissolved in tetrahydrofuran (4.0 mL), and cooled to -78°C under the protection of nitrogen, followed by the slow dropwise addition of an isopropyllithium solution (1.0 M, 3.17 mL). After the completion of dropwise addition, the mixture was further stirred at -78°C for 2.0 hours. The reaction mixture was quenched by adding a saturated aqueous ammonium chloride solution (20 mL), and extracted with ethyl acetate (20 mL×3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **445c** (20 mg, 116.13 µmol, yield: 10.99%) as colorless oil.

¹H NMR (400 MHz, DMSO-*d*₆) δ 3.87 (s, 1H), 3.69 - 3.49 (m, 4H), 3.46 - 3.38 (m, 1H), 3.21 - 3.12 (m, 1H), 2.64 - 2.53 (m, 2H), 2.49 - 2.44 (m, 1H), 1.02 - 0.93 (m, 6H).

### Step 3

### 2-(6-{1-[1-(1,4-dioxane -2-yl)-3-methylbutan-2-yl]azacyclobutan-3-yl}-3-methylimidazo[1,5-a]pyridin-8-yl)-N-ethyl-5-fluoro-N-(isopropyl)benzamide 445

Compound **90b** (80 mg, 202.80 µmol) and Compound **445c**(45.40 mg, 263.64 µmol) were dissolved in methanol (2.0 mL), followed by the addition of ZnCl₂ (82.92 mg, 608.39 µmol), and the mixture was stirred at 50°C for 1.0 hour. The reaction mixture was cooled to room temperature, followed by adding sodium cyanoborohydride (38.23 mg, 608.39 µmol) and stirring at 50°C for 12 hours. A saturated aqueous sodium bicarbonate solution (20 mL) was added to the reaction mixture, which was then extracted with dichloromethane (20 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonium bicarbonate), elution gradient: 32%-62%), to obtain the title Compound **445** (6.9 mg, 12.53 µmol, yield: 6.18%) as white solid.
MS m/z (ESI): 551.4 [M+1]
¹H NMR (400 MHz, CD₃OD) δ 8.00 - 7.80 (m, 1H), 7.79 - 7.55 (m, 1H), 7.50 - 6.97 (m, 3H), 6.96 - 6.75 (m, 1H), 3.83 - 3.40 (m, 11H), 3.24 - 2.95 (m, 3H), 2.73 - 2.56 (m, 3H), 2.55 - 2.39 (m, 1H), 1.99 - 1.79 (m, 1H), 1.50 - 0.76 (m, 16H), 0.34 - 0.28 (m, 2H).

### Examples 446-1 and 446-2

### 2-(6-{1-[(2S)-1-[(3R,4R)-3,4-dihydroxylpyrrolidin-1-yl]-3-methylbutan-2-yl]azacyclobutan-3-yl}-3-methylimidazo[1,5-a]pyridin-8-yl)-N-ethyl-5-fluoro-N-(isopropyl)benzamide 446-1

### 2-(6-{1-[(2R)-1-[(3R,4R)-3,4-dihydroxylpyrrolidin-1-yl]-3-methylbutan-2-yl]azacyclobutan-3-yl}-3-methylimidazo[1,5-a]pyridin-8-yl)-N-ethyl-5-fluoro-N-(isopropyl)benzamide 446-2

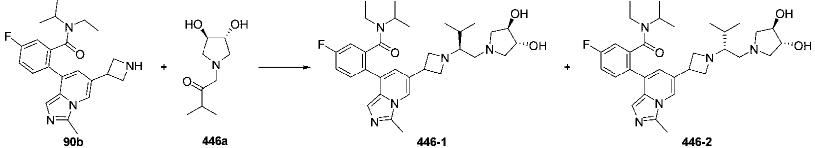

Compound **90b** (100 mg, 253.50 µmol) and 1-[(3*R*,4*R*)-3,4-dihydroxylpyrrolidin-l*-*yl]-3-methylbutan-2-one **446a** (71.20 mg, 380.24 µmol, synthesized with reference to the synthesis route of Compound **439a** by taking (3*R*,4*R*)-pyrrolidin-3,4-diol as a starting material) were dissolved in methanol (3.0 mL), followed by the addition of ZnCl₂ (69.10 mg, 506.99 µmol), and the mixture was stirred at 50°C for 1.0 hour. Sodium cyanoborohydride (47.79 mg, 760.49 µmol) was added, and the mixture was stirred at 50°C for 48 hours. A saturated aqueous potassium carbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonia water), elution gradient:24%-54%), to obtain the title Compounds **446-1** (4.67 mg, 8.25 µmol, yield: 3.26%) and **446-2** (4.52 mg, 7.99 µmol, yield: 3.15%).

### Single-configuration Compound 446-1:

MS m/z (ESI): 566.4 [M+1]
Chiral HPLC analysis: retention time: 2.250 min; chromatographic column: Chiralcel OX-3 50 x 4.6 mm I.D., 3 um; mobile phase: carbon dioxide-methanol (0.05% diethylamide): elution gradient: 5%-40%.

¹H NMR (400 MHz, DMSO-*d*₆) δ 7.91 (s, 1 H), 7.63 - 7.76 (m, 1 H), 7.27 - 7.44 (m, 2 H), 6.94 - 7.14 (m, 1 H), 6.64 - 6.90 (m, 1 H), 4.62 - 4.82 (m, 2 H), 3.79 (br d, J=4.00 Hz, 2 H), 3.58 (dt, J=13.20, 6.80 Hz, 2 H), 3.46 (br dd, J=14.40, 7.20 Hz, 2 H), 3.07 - 3.22 (m, 2 H), 2.94 (td, J=13.20, 6.40 Hz, 1 H), 2.67 - 2.86 (m, 3 H), 2.59 (s, 3 H), 2.24 - 2.33 (m, 3 H), 2.19 (br s, 2 H), 1.70 - 1.84 (m, 1 H), 0.90 (br d, J=6.40 Hz, 6 H), 0.71 - 0.86 (m, 7 H), 0.28 (br dd, J=6.00, 4.00 Hz, 2 H)

### Single-configuration Compound 446-2:

MS m/z (ESI): 566.4 [M+1]
Chiral HPLC analysis: retention time: 2.665 min; chromatographic column: Chiralcel OX-3 50 x 4.6 mm I.D., 3 um; mobile phase: carbon dioxide-methanol (0.05% diethylamide): elution gradient: 5%-40%.

¹H NMR (400 MHz, DMSO-*d*₆) δ 7.91 (s, 1 H), 7.60 - 7.75 (m, 1 H), 7.24 - 7.47 (m, 2 H), 6.97 - 7.08 (m, 1 H), 6.76 (d, J=5.60 Hz, 1 H), 4.60 - 4.87 (m, 2 H), 3.79 (br d, J=4.40 Hz, 2 H), 3.57 (br d, J=5.60 Hz, 2 H), 3.46 (br dd, J=12.40, 6.00 Hz, 2 H), 3.25 (br s, 1 H), 3.06 - 3.21 (m, 2 H), 2.87 - 2.99 (m, 1 H), 2.65 - 2.83 (m, 3 H), 2.58 (s, 3 H), 2.30 - 2.39 (m, 2 H), 2.17 - 2.29 (m, 3 H), 1.75 (br d, J=6.80 Hz, 1 H), 0.89 (br d, J=6.80 Hz, 6 H), 0.72 - 0.86 (m, 7 H), 0.28 (br dd, J=8.40, 7.20 Hz, 2 H)

### Example 447

### N-ethyl-5-fluoro-2-(6-{1-[1-(3-hydroxyloxacyclobutan-3-yl)-4-methylpentan-3-yl]azacyclobut-3-yl}-3-methylimidazo[1,5-a]pyridin-8-yl)-N-(isopropyl)benzamide 447

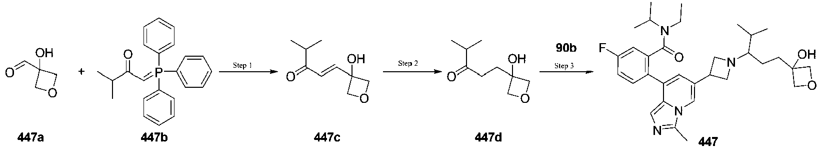

### Step 1

### 1-(3-hydroxyloxacyclobutan-3-yl)-4-methyl-1-ene-3-one 447c

3-Methyl-1-(triphenylphosphinidene)but-2-one **447b** (2.59 g, 7.48 mmol) was dissolved in dichloromethane (20 mL), followed by the addition of 3-hydroxyloxacyclobutan-3-formaldehyde **447a** (509 mg, 4.99 mmol). The reaction mixture was stirred at room temperature for 24 hours. The reaction mixture was concentrated under a reduced pressure. The resulting residues were purified by the preparative thin-layer chromatography using petroleum ether/ethyl acetate as an elution system, to obtain the title Compound **447c** (371 mg, 2.18 mmol, yield: 43.72%) as colorless oil.

¹H NMR (400 MHz, DMSO-*d*₆) δ 7.18 (d, J = 16.4 Hz, 1H), 6.44 (d, J = 15.6 Hz, 1H), 6.29 (s, 1H), 4.58 - 4.54 (m, 4H), 2.95 (td, J = 6.8, 13.6 Hz, 1H), 1.03 (d, J = 6.8 Hz, 6H).

### Step 2

### 1-(3-hydroxyloxacyclobutan-3-yl)-4-methylpentan-3-one 447d

Compound **447c** (371 mg, 2.18 mmol) was dissolved in ethanol (10.0 mL), followed by the addition of Pd/C (264.74 mg, 217.97 µmol, 10% purity) under the protection of argon. The reaction system was subjected to hydrogen substitution (25PSI), and stirred at 30°C to allow for reacting for 12 hours. The mixture was filtered through diatomite, and the filtrate was concentrated under a reduced pressure. The resulting residues were purified by the silica-gel column chromatography using petroleum ether/ethyl acetate as an elution system, to obtain the title Compound **447d** (84 mg, 487.75 µmol, yield: 22.38%) as colorless oil.

¹H NMR (400 MHz, DMSO-*d*₆) δ 5.59 (s, 1H), 4.40 - 4.28 (m, 4H), 2.70 - 2.59 (m, 1H), 2.57 - 2.53 (m, 2H), 1.92 - 1.85 (m, 2H), 1.01 (d, J = 6.9 Hz, 6H).

### Step 3

### N-ethyl-5-fluoro-2-(6-{1-[1-(3-hydroxyloxacyclobutan-3-yl)-4-methylpentan-3-yl]azacyclobut-3-yl}-3-methylimidazo[1,5-a]pyridin-8-yl)-N-(isopropyl)benzamide 447

Compound **90b** (192.41 mg, 487.75 µmol) and Compound **447d**(84 mg, 487.75 µmol) were dissolved in methanol (10 mL), followed by the addition of ZnCl₂ (132.96 mg, 975.49 µmol), and the mixture was stirred at 50°C for 1.0 hour. The mixture was cooled to room temperature, followed by adding sodium cyanoborohydride (91.95 mg, 1.46 mmol) and stirring at 50°C for 11 hour. A saturated aqueous sodium bicarbonate solution (20 mL) was added to the reaction mixture, which was then extracted with dichloromethane (20 mL x 3). The organic phases were combined, dried over anhydrous sodium sulfate, and concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% formic acid), elution gradient: 1%-30%), to obtain the title Compound **447** (20.89 mg, 37.93 µmol, yield: 7.78%) as yellow solid.
MS m/z (ESI): 551.2 [M+1]
¹H NMR (400 MHz, DMSO-*d*₆) δ 7.93 (s, 1H), 7.73 - 7.64 (m, 1H), 7.42 - 7.28 (m, 2H), 7.07 - 7.01 (m, 1H), 6.87 - 6.73 (m, 1H), 5.52 (br s, 1H), 4.38 (br d, J = 5.6 Hz, 2H), 4.33 - 4.24 (m, 2H), 3.64 - 3.39 (m, 4H), 3.22 - 3.04 (m, 2H), 3.00 - 2.87 (m, 1H), 2.59 (s, 3H), 2.05 (br s, 1H), 1.85 - 1.67 (m, 3H), 1.37 - 1.20 (m, 2H), 1.09 - 0.69 (m, 14H), 0.28 (dd, J = 2.4, 6.4 Hz, 2H).

### Examples 448-1 and 448-2

### 5-fluoro-2-(6-{1-[(3S)-6-[4-(2-hydroxylethyl)piperazin-1-yl]-2-methylhexan-3-yl]azacyclobutan-3-yl}3-methyl-imidazo[1,5-a]pyridin-8-yl)-N-ethyl-N-(isopropyl)benzamide 448-1

### 5-fluoro-2-(6-{1-[(3R)-6-[4-(2-hydroxylethyl)piperazin-1-yl]-2-methylhexan-3-yl]azacyclobutan-3-yl}3-methyl-imidazo[1,5-a]pyridin-8-yl)-N-ethyl-N-(isopropyl)benzamide 448-2

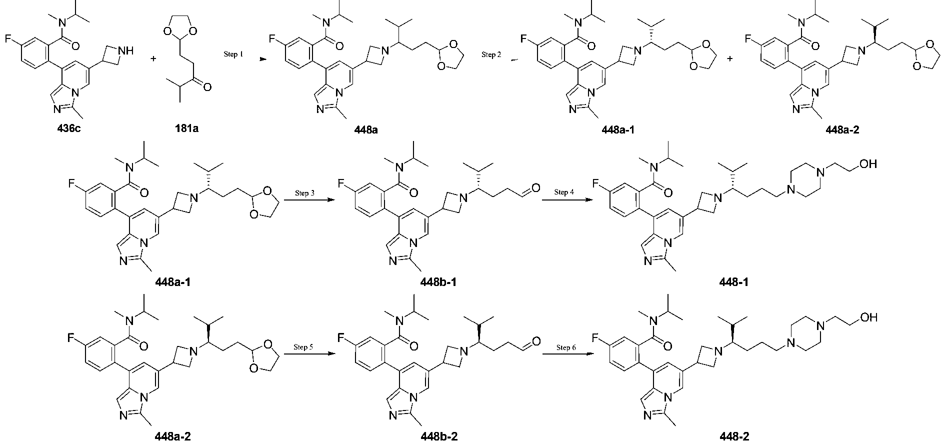

### Step 1

### 2-(6-{1-[1-(1,3-dioxolan-2-yl)-4-methylpentan-3-yl]azacyclobutan-3-yl}-3-methylimidazo[1,5-a]pyridin-8-yl)-N-methyl-5-fluoro-N-(isopropyl)benzamide 448a

Compound **436c** (340 mg, 893.66 µmol) and 1-(1,3-dioxolan-2-yl)-4-methylpentan-3-one **181a** (230.86 mg, 1.34 mmol) were dissolved in methanol (5.0 mL), followed by the addition of ZnCl₂ (243.60 mg, 1.79 mmol), and the mixture was stirred at 55°C for 1.0 hour. The mixture was cooled to room temperature, followed by adding sodium cyanoborohydride (168.48 mg, 2.68 mmol) and stirring at 55°C for 11 hour. A saturated aqueous sodium carbonate solution (20 mL) was added to the reaction mixture, which was then extracted with dichloromethane (20 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **448a** (280 mg, 521.73 µmol, yield: 58.38%) as yellow solid.
MS m/z (ESI): 551.3 [M+1]

### Step 2

### 2-(6-{1-[(3S)-1-(1,3-dioxolan-2-yl)-4-methylpentan-3-yl]azacyclobutan-3-yl}-3-methylimidazo[1,5-a]pyridin-8-yl) -5-fluoro-N-methyl-N-(isopropyl)benzamide 448a-1

### 2-(6-{1-[(3R)-1-(1,3-dioxolan-2-yl)-4-methylpentan-3-yl]azacyclobutan-3-yl}-3-methylimidazo[1,5-a]pyridin-8-yl) -5-fluoro-N-methyl-N-(isopropyl)benzamide 448a-2

Compound **448a** (280 mg, 521.73 µmol) was separated and purified by the chiral preparative HPLC (preparative column: DAICEL CHIRALCEL OD (250 mm * 30 mm,10 um), mobile phase: carbon dioxide-ethanol (0.1% ammonia water); elution gradient: 20%), to obtain the title Compounds **448a-1** (100 mg, 186.33 µmol, yield: 35.71%) and **448a-2** (90 mg, 167.70 µmol, yield: 32.14%).

### Single-configuration Compound 448a-1:

MS m/z (ESI):537.3 [M+1]
Chiral HPLC analysis: retention time: 1.137 min; chromatographic column: Chiralcel OD-3 50 x 4.6 mm I.D., 3 um; mobile phase: carbon dioxide-ethanol (0.05% diethylamide): elution gradient: 5%-40%.

### Single-configuration Compound 448a-2:

MS m/z (ESI): 537.3 [M+1]
Chiral HPLC analysis: retention time: 1.355 min; chromatographic column: Chiralcel OD-3 50 x 4.6 mm I.D., 3 um; mobile phase: carbon dioxide-ethanol (0.05% diethylamide): elution gradient: 5%-40%.

### Step 3

### 5-fluoro-N-methyl-2-(3-methyl-6-{1-[(3S)-2-methyl-6-oxohexan-3-yl]azacyclobutan-3-yl}imidazo[1,5-a]pyridin-8-yl)-N-(isopropyl)benzamide 448b-1

Compound **448a-1** (100 mg, 186.33 µmol) was dissolved in acetonitrile (2.0 mL), followed by the addition of a hydrochloric acid solution (1.0 M; 2.0 mL). The mixture was stirred at 55°C to allow for reacting for 1.0 hours. The reaction mixture was concentrated under a reduced pressure, regulated to pH = 10 with a 10 % aqueous NaOH solution, and extracted with dichloromethane (15 mL x 3). The organic phases were combined, dried over anhydrous sodium sulfate, and concentrated under a reduced pressure, to obtain the crude product of title Compound **448b-1** (80 mg, 162.39 µmol,yield: 87.15%). The crude product was directly used in the next step of reaction without purification.

### Single-configuration Compound 448b-1:

MS m/z (ESI): 493.3 [M+1]

### Step 4

### 5-fluoro-2-(6-{1-[(3S)-6-[4-(2-hydroxylethyl)piperazin-l-yl]-2-methylhexan-3-yl]azacyclobutan-3-yl}3-methyl-imidazo[1,5-a]pyridin-8-yl)-N-ethyl-N(isopropyl)benzamide 448-1

The crude product of Compound **448b-1** (80 mg, 162.39 µmol) and 2-piperazin-1-ylethanol (31.71 mg, 243.59 µmol) were dissolved in methanol (2.0 mL), followed by the addition of acetic acid (9.75 mg, 162.39 µmol), and the mixture was stirred at 35°C for 1.0 hour. Sodium cyanoborohydride (30.62 mg, 487.18 µmol) was added, allowing for reacting at 35°C for 11.0 hours under the protection of nitrogen. A saturated aqueous sodium bicarbonate solution (20 mL) was added to the reaction mixture, which was then extracted with dichloromethane (35 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 28%-48%). The title Compound **448-1** (41.74 mg, 68.79 µmol, yield: 42.36%) was obtained.

### Single-configuration Compound 448-1:

MS m/z (ESI):607.3 [M+1]
Chiral SFC: retention time: 1.185 min; chromatographic column: Chiralcel AD-3 50×4.6 mm I.D., 3 um; mobile phase: CO₂/MeOH (0.05% DEA): elution gradient: 5%-40%.

¹H NMR (400 MHz, DMSO-*d*₆) δ 7.92 (s, 1H), 7.77 - 7.66 (m, 1H), 7.44 - 7.27 (m, 2H), 7.11 - 7.02 (m, 1H), 6.87 - 6.72 (m, 1H), 4.60 (s, 7H), 3.18 - 2.97 (m, 2H), 2.79 - 2.54 (m, 5H), 2.46 - 2.16 (m, 13H), 2.09 - 1.99 (m, 1H), 1.79 - 1.63 (m, 1H), 1.56 - 1.34 (m, 2H), 1.31 - 0.56 (m, 13H), 0.24 (d, J = 6.4 Hz, 1H).

### Step 5

### 5-fluoro-N-methyl-2-(3-methyl-6-{1-[(3R)-2-methyl-6-oxohexan-3-yl]azacyclobutan-3-yl}imidazo[1,5-a]pyridin-8-yl)-N-(isopropyl)benzamide 448b-2

Compound **448a-2** (90 mg, 167.70 µmol) was dissolved in acetonitrile (2.0 mL), followed by the addition of a hydrochloric acid solution (1.0 M; 2.0 mL). The mixture was stirred at 50°C to allow for reacting for 1.0 hours. The reaction mixture was concentrated under a reduced pressure, regulated to pH = 10 with a 10 % aqueous NaOH solution, and extracted with dichloromethane (15 mL x 3). The organic phases were combined, dried over anhydrous sodium sulfate, and concentrated under a reduced pressure, to obtain the crude product of title Compound **448b-2** (70 mg, 142.10 µmol,yield: 84.73%) as yellow solid. The crude product was directly used in the next step of reaction without purification.

### Single-configuration Compound 448b-2:

MS m/z (ESI): 493.3 [M+1]

### Step 6

### 5-fluoro-2-(6-{1-[(3R)-6-[4-(2-hydroxylethyl)piperazin-1-yl]-2-methylhexan-3-yl]azacyclobutan-3-yl}3-methyl-imidazo[1,5-a]pyridin-8-yl)-N-ethyl-N-(isopropyl)benzamide 448-2

The crude product of Compound **448b-2** (70 mg, 142.10 µmol) and 2-piperazin-1-ylethanol (27.75 mg, 213.14 µmol) were dissolved in methanol (2.0 mL), followed by the addition of acetic acid (8.53 mg, 142.10 µmol), and the mixture was stirred at 35°C for 1.0 hour. Sodium cyanoborohydride (26.79 mg, 426.29 µmol) was added, allowing for reacting at 35°C for 11.0 hours under the protection of nitrogen. A saturated aqueous sodium bicarbonate solution (20 mL) was added to the reaction mixture, which was then extracted with dichloromethane (35 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 28%-48%). The title Compound **448-2** (35.20 mg, 58.01 µmol, yield: 40.82%) was obtained as yellow solid.

### Single-configuration Compound 448-2:

MS m/z (ESI):607.4 [M+1]
Chiral SFC: retention time: 1.117 min; chromatographic column: Chiralcel AD-3 50×4.6 mm I.D., 3um,; mobile phase: CO₂/MeOH (0.05% DEA): elution gradient: 5%-40%.

¹H NMR (400 MHz, DMSO-*d*₆) δ 7.92 (s, 1H), 7.77 - 7.66 (m, 1H), 7.45 - 7.27 (m, 2H), 7.12 - 7.02 (m, 1H), 6.89 - 6.71 (m, 1H), 4.62 - 3.41 (m, 7H), 3.19 - 2.99 (m, 2H), 2.77 - 2.54 (m, 5H), 2.47 - 2.14 (m, 13H), 2.08 - 1.99 (m, 1H), 1.77 - 1.63 (m, 1H), 1.53 - 1.35 (m, 2H), 1.31 - 0.55 (m, 13H), 0.24 (d, J = 6.4 Hz, 1H).

### Examples 449-1 and 449-2

### N-ethyl-5-fluoro-2-(3-methyl-6-{1-[(3R)-4-methyl-1-[(3R)-morpholin-3-yl]pentan-3-yl]azacyclobutan-3-yl}imidazo[1,5-a]pyridin-8-yl)-N-(isopropyl)benzamide 449-1

### N-ethyl-5-fluoro-2-(3-methyl-6-{1-[(3S)-4-methyl-1-[(3R)-morpholin-3-yl]pentan-3-yl]azacyclobutan-3-yl}imidazo[1,5-a]pyridin-8-yl)-N-(isopropyl)benzamide 449-2

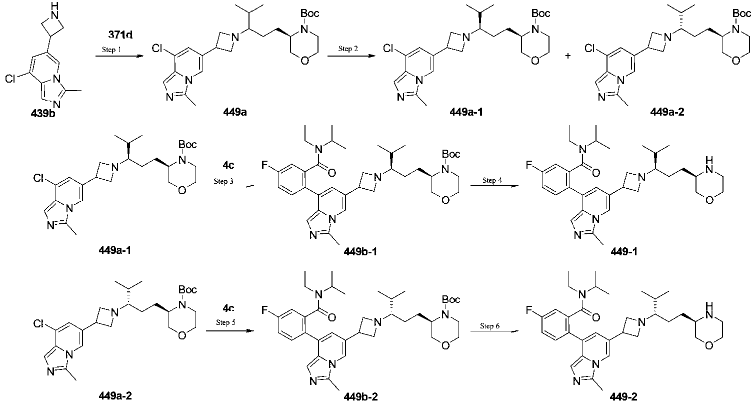

### Step 1

### (3R)-3-[3-(3-{8-chloro-3-methylimidazo[1,5-a]pyridin-6-yl}azacyclobutan-1-yl)-4-methylpentyl]morpholin-4-tert-butyl carboxylate 449a

Compound **439b** (344 mg, 1.55 mmol) and Compound **371d**(885.67 mg, 3.10 mmol) were dissolved in methanol (10.0 mL), followed by the addition of ZnCl₂ (423.01 mg, 3.10 mmol), and the mixture was stirred at 25°C for 1.0 hour. Sodium cyanoborohydride (292.54 mg, 4.66 mmol) was added, and the mixture was stirred at 50°C for 2.0 hours. Compound **439b** (344 mg, 1.55 mmol) was supplemented, and the mixture was further stirred at 50°C for 12.0 hours. A saturated aqueous sodium carbonate solution (20 mL) was added to the reaction mixture, which was then extracted with dichloromethane (20 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 52%-82%), to obtain the title Compound **449a** (320 mg, 651.65 µmol, yield: 21%) as yellow solid.
MS m/z (ESI): 491.4 [M+1]

### Step 2

### (3R)-3-[(3R)-3-(3-{8-chloro-3-methylimidazo[1,5-a]pyridin-6-yl}azacyclobutan-1-yl)-4-methylpentyl]morpholin-4-tert-butyl carboxylate 449a-1

### tert-butyl (3R)-3-[(3S)-3-(3-{8-chloro-3-methylimidazo[1,5-a]pyridin-6-yl}azetidin-1-yl)-4-methylpentyl]morpholine-4-carboxylate

### (3R)-3-[(3S)-3-(3-{8-chloro-3-methylimidazo[1,5-a]pyridin-6-yl}azacyclobutan-1-yl)-4-methylpentyl]morpholin-4-tert-butyl carboxylate 449a-2

Compound **449a** (320 mg, 651.65 µmol) was purified by the chiral preparative SFC (preparative column: Column: DAICEL CHIRALCEL OD(250 mm * 30 mm,10 um), mobile phase: carbon dioxide-methanol (0.1% ammonia water); elution gradient: 15%). The title Compound **449a-1** (216 mg, 439.86 µmol) and the title Compound **449a-2** (80 mg, 162.91 µmol) were obtained.

### Single-configuration Compound 449a-1:

MS m/z (ESI): 491.4 [M+1]
Chiral HPLC analysis: retention time: 1.146 min; chromatographic column: Chiralcel OD-3 50 x 4.6 mm I.D., 3 um; mobile phase: carbon dioxide-methanol (0.05% diethylamide): elution gradient: 5%-40%.

¹H NMR (400 MHz, CD₃OD) δ 7.85 (s, 1H), 7.34 (s, 1H), 7.06 (s, 1H), 3.92 - 3.69 (m, 6H), 3.67 - 3.57 (m, 1H), 3.53 (dd, J = 3.2, 11.6 Hz, 1H), 3.45 - 3.34 (m, 3H), 2.65 (s, 3H), 2.24 (s, 1H), 2.07 - 1.54 (m, 4H), 1.48 - 1.43 (m, 9H), 1.39 - 1.10 (m, 2H), 1.02 - 0.85 (m, 6H).

### Single-configuration Compound 449a-2:

MS m/z (ESI): 491.5 [M+1]
Chiral HPLC analysis: retention time: 1.203 min; chromatographic column: Chiralcel OD-3 50 x 4.6 mm I.D., 3 um; mobile phase: carbon dioxide-methanol (0.05% diethylamide): elution gradient: 5%-40%.

¹H NMR (400 MHz, CD₃OD) δ 7.86 (s, 1H), 7.35 (s, 1H), 7.06 (s, 1H), 3.83 - 3.73 (m, 6H), 3.68 - 3.61 (m, 1H), 3.54 (dd, J = 3.2, 11.8 Hz, 1H), 3.41 (dt, J = 2.8, 11.9 Hz, 3H), 2.65 (s, 3H), 2.03 (s, 1H), 1.91 - 1.72 (m, 4H), 1.47 - 1.45 (m, 9H), 1.35 (td, J = 2.6, 5.3 Hz, 2H), 0.95 (dd, J = 6.8, 15.4 Hz, 6H).

### Step 3

### (3R)-3-[(3R)-3-[3-(8-{2-[ethyl(isopropyl)aminoformyl]-4-fluorophenyl}-3-methylimidazo[1,5-a]pyridin-6-yl)azacyclobutan-1-yl]-4-methylpentyl]morpholin-4-tert-butyl carboxylate 449b-1

Compound **449a-1** (216 mg, 439.86 µmol), Compound **4c** (203.38 mg, 527.83 µmol) and potassium phosphate (280.1 mg, 1.32 mmol) were dissolved in dioxane (16 mL) and water (4 mL). Pd(dtbpf)Cl₂ (18.92 mg, 21.99 µmol) was added. Under a nitrogen atmosphere, the mixture was stirred at 95°Cto allow for reacting for 12 hours. The reaction mixture was cooled to room temperature, followed by adding water (50 mL) and extracting with ethyl acetate (50 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **449b-1** (158 mg, 238.00 µmol, yield: 54.11%) as yellow oil.
MS m/z (ESI): 664.5 [M+1]

### Step 4

### N-ethyl-5-fluoro-2-(3-methyl-6-{1-[(3R)-4-methyl-1-[(3R)-morpholin-3-yl]pentan-3-yl]azacyclobutan-3-yl}imidazo[1,5-a]pyridin-8-yl)-N-(isopropyl)benzamide 449-1

Compound **449b-1** (158 mg, 238.00 µmol) was dissolved in dichloromethane (15 mL), followed by the addition of trifluoroacetic acid (4.44 g, 38.94 mmol, 3 mL). The mixture was stirred at room temperature to allow for reacting for 1.0 hour. The reaction mixture was concentrated under a reduced pressure, followed by adding dichloromethane (5 mL) and water (5 mL), regulating to PH >7 with a 10% aqueous sodium hydroxide solution and extracting with dichloromethane (10 mL x 3). The organic phases were combined, dried over anhydrous sodium sulfate, and concentrated under a reduced pressure. The resulting residues were purified by the HPLC (mobile phase: acetonitrile, water (0.1%ammonium bicarbonate), elution gradient: 28%-58%), to obtain the title Compound **449-1** (81.07 mg, 143.81 µmol, yield: 60.42%) as yellow solid.
MS m/z (ESI): 564.4 [M+1]
Chiral HPLC analysis: retention time: 1.115 min; chromatographic column: Chiralcel OD-3 50 x 4.6 mm I.D., 3 um; mobile phase: carbon dioxide-methanol (0.05% diethylamide): elution gradient: 5%-40%.

¹H NMR (400 MHz, CD₃OD) δ 7.94 - 7.87 (m, 1H), 7.77 - 7.67 (m, 1H), 7.38 - 7.30 (m, 1H), 7.29 - 7.19 (m, 1H), 7.18 - 7.09 (m, 1H), 6.97 - 6.85 (m, 1H), 3.83 - 3.36 (m, 8H), 3.30 - 3.20 (m, 2H), 3.18 - 2.97 (m, 2H), 2.92 - 2.78 (m, 2H), 2.73 - 2.61 (m, 4H), 2.21 (s, 1H), 1.91 - 1.79 (m, 1H), 1.58 - 1.08 (m, 5H), 1.07 - 0.69 (m, 13H), 0.33 - 0.27 (m, 2H).

### Step 5

### (3R)-3-[(3S)-3-[3-(8-{2-[ethyl(isopropyl)aminofonnyl]-4-fluorophenyl}-3-methylimidazo[1,5-a]pyridin-6-yl)azacyclobutan-1-yl]-4-methylpentyl]morpholin-4-tert-butyl carboxylate 449b-2

Compound **449a-2** (80 mg, 162.91 µmol), Compound **4c**(75.33 mg, 195.49 µmol) and potassium phosphate (103.74 mg, 488.73 µmol) were dissolved in dioxane (16 mL) and water (4 mL). Pd(dtbpf)Cl₂ (7.01 mg, 8.15 µmol) was added. Under a nitrogen atmosphere, the mixture was stirred at 95°Cto allow for reacting for 16 hours. The reaction mixture was cooled to room temperature, followed by the addition of water (50 mL) and extraction with ethyl acetate (50 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **449b-2** (74 mg, 111.47 µmol, yield: 68.42%).
MS m/z (ESI): 664.6 [M+1]

### Step 6

### N-ethyl-5-fluoro-2-(3-methyl-6-{1-[(3S)-4-methyl-1-[(3R)-morpholin-3-yl]pentan-3-yl]azacyclobutan-3-yl}imidazo[1,5-a]pyridin-8-yl)-N-(isopropyl)benzamide 449-2

Compound **449b-2** (80 mg, 120.51 µmol) was dissolved in dichloromethane (1.0 mL), followed by the addition of trifluoroacetic acid (13.74 mg, 120.51 µmol, 9.28 µL). The mixture was stirred at room temperature to allow for reacting for 1.0 hour, and the reaction mixture was concentrated under a reduced pressure, followed by adding dichloromethane (5 mL) and water (5 mL), regulating to PH >7 with a 10% aqueous sodium hydroxide solution and extracting with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 28%-58%), to obtain the title Compound **449-2** (15.68 mg, 27.81 µmol, yield: 23.08%) as yellow solid.
MS m/z (ESI): 564.5 [M+1]
Chiral HPLC analysis: retention time: 1.113 min; chromatographic column: Chiralcel OD-3 50 x 4.6 mm I.D., 3 um; mobile phase: carbon dioxide-methanol (0.05% diethylamide): elution gradient: 5%-40%.

¹H NMR (400 MHz, CD₃OD) δ 7.93 - 7.89 (m, 1H), 7.76 - 7.68 (m, 1H), 7.39 - 7.30 (m, 1H), 7.28 - 7.20 (m, 1H), 7.17 - 7.10 (m, 1H), 6.97 - 6.84 (m, 1H), 3.81 - 3.64 (m, 5H), 3.38 (s, 3H), 3.29 - 3.21 (m, 2H), 3.19 - 2.98 (m, 2H), 2.89 - 2.79 (m, 2H), 2.72 - 2.62 (m, 4H), 2.25 - 2.16 (m, 1H), 1.93 - 1.78 (m, 1H), 1.58 - 1.45 (m, 1H), 1.44 - 1.11 (m, 5H), 0.97 - 0.89 (m, 11H), 0.80 (t, J = 7.0 Hz, 1H), 0.32 - 0.28 (m, 2H).

### Examples 454-1 and 454-2

### 4-[(4R)-4-[3-(8-{2-[ethyl(isopropyl)aminoformyl]-4-fluorophenyl}-3-methylimidazo[1,5-a]pyridin-6-yl)azacyclobutan-1-yl]-5-methylhexyl]piperazin-1-tert-butyl carboxylate 454-1

### 4-[(4S)-4-[3-(8-{2-[ethyl(isopropyl)aminoformyl]-4-fluorophenyl}-3-methylimidazo[1,5-a]pyridin-6-yl)azacyclobutan-1-yl]-5-methylhexyl]piperazin-1-tert-butyl carboxylate 454-2

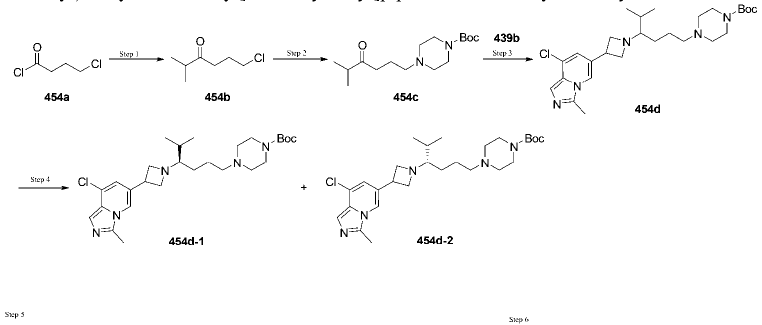

### Step 2

### 6-chloro-2-methylhexan-3-one 454b

4-Chlorobutyryl chloride **454a** (10 g, 70.92 mmol) was dissolved in tetrahydrofuran (100 mL), and cooled to 0°C under the protection of nitrogen, followed by the slow dropwise addition of an isopropylmagnesium chloride solution (2.0 M, 35.46 mL). After dropwise addition, the mixture was slowly warmed to 25°C and stirred for 12.0 hours. The reaction mixture was quenched by adding a saturated aqueous ammonium chloride solution (100 mL), and extracted with ethyl acetate (50 mL×3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **454b** (4.0 g, 26.91 mmol, yield: 37.95%) as colorless oil.

¹H NMR (400 MHz, CDCl₃) δ 3.61 - 3.56 (m, 2H), 2.70 - 2.58 (m, 3H), 2.09 - 2.01 (m, 2H), 1.12 (d, J = 7.0 Hz, 6H).

### Step 2

### 4-(5-methyl-4-oxohexyl)piperazin-1-tert-butyl carboxylate 454c

Compound **454b** (4.0 g, 26.91 mmol) was dissolved in acetonitrile (60 mL), followed by the addition of piperazin-1-tert-butyl carboxylate (6.01 g, 32.29 mmol), potassium carbonate (7.44 g, 53.82 mmol) and sodium iodide (403.40 mg, 2.69 mmol) at 25°C. The reaction mixture was heated to 85°C and stirred for 12 hours. The reaction mixture was cooled to room temperature, filtered, and washed with ethyl acetate (50 mL x 3). The organic phases were combined and concentrated under a reduced pressure. The resulting residues were purified by the silica-gel column chromatography using dichloromethane/methanol as an elution system, to obtain the title Compound **454c** (4 g, 13.40 mmol, yield: 49.81%) as colorless oil.

¹H NMR (400 MHz, CDCl₃) δ 3.43 - 3.39 (m, 4H), 2.61 (br d, J = 6.9 Hz, 1H), 2.48 (t, J = 7.1 Hz, 2H), 2.38 - 2.31 (m, 6H), 1.81 - 1.74 (m, 2H), 1.45 (s, 9H), 1.09 (d, J = 6.9 Hz, 6H).

### Step 3

### 4-[4-(3-{8-chloro-3-methylimidazo[1,5-a]pyridin-6-yl}azacyclobutan-1-yl)-5-methylhexyl]piperazin-1-tert-butyl carboxylate 454d

Compound **439b** (2 g, 9.02 mmol) and Compound **454c** (2.96 g, 9.92 mmol) were dissolved in methanol (20.0 mL), followed by the addition of ZnCl₂ (3.69 g, 27.07 mmol), and the mixture was stirred at 50°C for 1.0 hour. The mixture was cooled to room temperature, followed by adding sodium cyanoborohydride (1.70 g, 27.07 mmol) and stirring at 50°C for 12.0 hour. A saturated aqueous sodium bicarbonate solution (30 mL) was added to the reaction mixture, which was then extracted with dichloromethane (30 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **454d** (3.4 g, 6.74 mmol, yield: 74.76%) as yellow solid.
MS m/z (ESI): 504.3 [M+1]

### Step 4

### 4-[(4R)-4-(3-{8-chloro-3-methylimidazo[1,5-a]pyridin-6-yl}azacyclobutan-1-yl)-5-methylhexyl]piperazin-1-tert-butyl carboxylate 454d-1

### tert-butyl 4-[(4S)-4-(3-{ 8-chloro-3-methylimidazo[1,5-a]pyridin-6-yl}azetidin-1-yl)-5-methylhexyl]piperazine-1-carboxylate

### 4-[(4S)-4-(3-{8-chloro-3-methylimidazo[1,5-a]pyridin-6-yl}azacyclobutan-1-yl)-5-methylhexyl]piperazin-1-tert-butyl carboxylate 454d-2

Compound **454d**(3.4 g, 6.74 mmol) was separated and purified by the chiral preparative SFC (preparative column: DAICEL CHIRALCEL OD(250 mm * 30 mm,10 um), mobile phase: carbon dioxide-isopropanol (0.1% ammonia water); elution gradient: 30%). The title Compounds **454d-1** (1.65 g, 3.27 mmol, yield: 48.53%) and **454d-2** (1.6 g, 3.17 mmol, yield: 47.06%) were obtained as yellow solid.

### Single-configuration Compound 454d-1:

MS m/z (ESI): 504.2 [M+1]
Chiral HPLC analysis: retention time: 1.446 min; chromatographic column: Chiralcel AD-3 50 x 4.6 mm I.D., 3 um; mobile phase: carbon dioxide-isopropanol (0.05% diethylamide ): elution gradient: 5%-40%.

### Single-configuration Compound 454d-2:

MS m/z (ESI): 504.3 [M+1]
Chiral HPLC analysis: retention time: 1.645 min; chromatographic column: Chiralcel AD-3 50 x 4.6 mm I.D., 3 um; mobile phase: carbon dioxide-isopropanol (0.05% diethylamide ): elution gradient: 5%-40%.

### Step 5

### 4-[(4R)-4-[3-(8-{2-[ethyl(isopropyl)aminofonnyl]-4-fluorophenyl}-3-methylimidazo[1,5-a]pyridin-6-yl)azacyclobutan-1-yl]-5-methylhexyl]piperazin-1-tert-butyl carboxylate 454-1

Compound **454d-1** (1.65 g, 3.27 mmol), Compound **4c** (1.51 g, 3.93 mmol) and potassium phosphate (2.08 g, 9.82 mmol) were dissolved in dioxane (15 mL) and water (3 mL). Pd(dtbpf)Cl₂ (106.66 mg, 163.66 µmol) was added. Under a nitrogen atmosphere, the mixture was stirred at 95°Cto allow for reacting for 2.0 hours. The reaction mixture was cooled to room temperature, water (20 mL) was added, and the mixture was extracted with ethyl acetate (15 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **454-1** (1.8 g, 2.66 mmol, yield: 81.24%).
MS m/z (ESI): 677.5 [M+1]
¹H NMR (400 MHz, DMSO-*d₆*) δ 8.07 - 7.79 (m, 1H), 7.78 - 7.53 (m, 1H), 7.49 - 7.17 (m, 2H), 7.16 - 6.88 (m, 1H), 6.88 - 6.66 (m, 1H), 3.62 - 3.36 (m, 4H), 3.29 - 2.87 (m, 8H), 2.64 - 2.53 (m, 3H), 2.32 - 2.17 (m, 6H), 2.07 - 1.99 (m, 1H), 1.80 - 1.62 (m, 1H), 1.59 - 1.09 (m, 14H), 1.08 - 0.69 (m, 13H), 0.30 - 0.23 (m, 2H).

### Step 6

### 4-[(4S)-4-[3-(8-{2-[ethyl(isopropyl)aminoformyl]-4-fluorophenyl}-3-methylimidazo[1,5-a]pyridin-6-yl)azacyclobutan-1-yl]-5-methylhexyl]piperazin-1-tert-butyl carboxylate 454-2

Compound **454d-2** (1.60 g, 3.17 mmol), Compound **4c** (1.47 g, 3.81 mmol) and potassium phosphate (2.02 g, 9.52 mmol) were dissolved in dioxane (16 mL) and water (4 mL). Pd(dtbpf)Cl₂ (136.55 mg, 158.70 µmol) was added. Under a nitrogen atmosphere, the mixture was stirred at 95°Cto allow for reacting for 12 hours. The reaction mixture was cooled to room temperature, followed by adding water (30 mL) and extracting with ethyl acetate (30 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **454-2** (2.0 g, 2.96 mmol, yield: 93.23%).
MS m/z (ESI): 677.5 [M+1]
¹H NMR (400 MHz, DMSO-*d₆*) δ 8.03 - 7.79 (m, 1H), 7.75 - 7.56 (m, 1H), 7.46 - 7.21 (m, 2H), 7.14 - 6.87 (m, 1H), 6.87 - 6.63 (m, 1H), 3.70 - 3.36 (m, 4H), 3.29 - 2.87 (m, 8H), 2.65 - 2.54 (m, 3H), 2.30 - 2.17 (m, 6H), 2.06 - 1.99 (m, 1H), 1.77 - 1.62 (m, 1H), 1.52 - 1.13 (m, 14H), 1.09 - 0.70 (m, 13H), 0.29 - 0.24 (m, 2H).

### Examples 455 and 456

### N-ethyl-5-fluoro-2-{6-[1-(1-hydroxyl-4-methylpentan-3-yl)azacyclobutan-3-yl]-3-methylimidazo[1,5-a]pyridin-8-yl}-N-(isopropyl)benzamide 455

### N-ethyl-5-fluoro-2-[6-(1-{1-[4-(2-hydroxyethyl)piperazin-1-yl]-4-methylpentan-3-yl}azacyclobutan-3-yl)-3-methylimidazo[1,5-a]pyridin-8-yl]-N-(isopropyl)benzamide 456

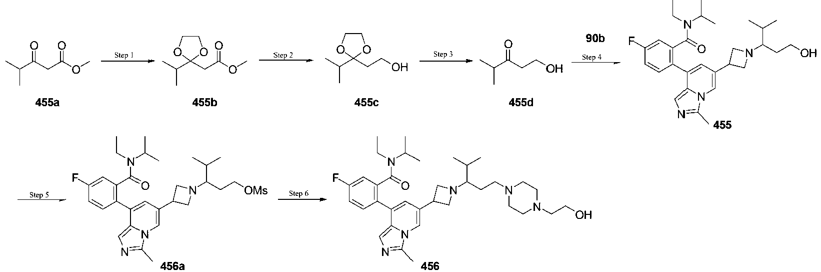

### Step 1

### 2-(1,4-dioxane -2-yl)-N-methoxy-N-methylacetamide 445b

2-(1,4-Dioxane -2-yl)acetic acid (500 mg, 3.42 mmol) was dissolved in dichloromethane (10 mL), followed by the addition of triethylamine (1.04 g, 10.26 mmol), EDCI(787.05 mg, 4.11 mmol) and HOBT (554.77 mg, 4.11 mmol). After the reaction mixture was stirred, *N*-methoxymethylamine (367.11 mg, 3.76 mmol, hydrochloride) was added. The reaction mixture was stirred at room temperature for 3.0 hours. The reaction mixture was quenched by adding a saturated aqueous sodium bicarbonate solution (20 mL), and extracted with dichloromethane (20 mL × 3). The organic phases were combined, dried over anhydrous sodium sulfate, and concentrated under a reduced pressure. The resulting residues were purified by the preparative thin-layer chromatography using petroleum ether/ethyl acetate as an elution system, to obtain the title Compound **445b** (200 mg, 1.06 mmol, yield: 31%).

¹H NMR (400 MHz, DMSO-*d₆*) δ 3.90 - 3.82 (m, 1H), 3.75 - 3.39 (m, 9H), 3.12 - 3.02 (m, 3H), 2.65 - 2.53 (m, 1H), 2.40 - 2.28 (m, 1H).

### Step 2

### 1-(1,4-dioxane-2-yl)-3-methylbutan-2-one 445c

Under the protection of nitrogen, Compound **445b** (200 mg, 1.06 mmol) was dissolved in tetrahydrofuran (4.0 mL), and cooled to -78°C under the protection of nitrogen, followed by the slow dropwise addition of an isopropyllithium solution (1.0 M, 3.17 mL). After the completion of dropwise addition, the mixture was further stirred at -78°C for 2.0 hours. The reaction mixture was quenched by adding a saturated aqueous ammonium chloride solution (20 mL), and extracted with ethyl acetate (20 mL×3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **445c** (20 mg, 116.13 µmol, yield: 10.99%) as colorless oil.

¹H NMR (400 MHz, DMSO-*d₆*) δ 3.87 (s, 1H), 3.69 - 3.49 (m, 4H), 3.46 - 3.38 (m, 1H), 3.21 - 3.12 (m, 1H), 2.64 - 2.53 (m, 2H), 2.49 - 2.44 (m, 1H), 1.02 - 0.93 (m, 6H).

### Step 3

### 2-(6-{1-[1-(1,4-dioxane -2-yl)-3-methylbutan-2-yl]azacyclobutan-3-yl}-3-methylimidazo[1,5-a]pyridin-8-yl)-N-ethyl-5-fluoro-N-(isopropyl)benzamide 445

Compound **90b** (80 mg, 202.80 µmol) and Compound **445c**(45.40 mg, 263.64 µmol) were dissolved in methanol (2.0 mL), followed by the addition of ZnCl₂ (82.92 mg, 608.39 µmol), and the mixture was stirred at 50°C for 1.0 hour. The reaction mixture was cooled to room temperature, followed by adding sodium cyanoborohydride (38.23 mg, 608.39 µmol) and stirring at 50°C for 12 hours. A saturated aqueous sodium bicarbonate solution (20 mL) was added to the reaction mixture, which was then extracted with dichloromethane (20 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% ammonium bicarbonate), elution gradient: 32%-62%), to obtain the title Compound **445** (6.9 mg, 12.53 µmol, yield: 6.18%) as white solid.
MS m/z (ESI): 551.4 [M+1]
¹H NMR (400 MHz, CD₃OD) δ 8.00 - 7.80 (m, 1H), 7.79 - 7.55 (m, 1H), 7.50 - 6.97 (m, 3H), 6.96 - 6.75 (m, 1H), 3.83 - 3.40 (m, 11H), 3.24 - 2.95 (m, 3H), 2.73 - 2.56 (m, 3H), 2.55 - 2.39 (m, 1H), 1.99 - 1.79 (m, 1H), 1.50 - 0.76 (m, 16H), 0.34 - 0.28 (m, 2H).

### Examples 457-1 and 457-2

### N-ethyl-5-fluoro-2-(3-methyl-6-{1-[(3S)-4-methyl-1-[(3S)-morpholin-3-yl]pentan-3-yl]azacyclobutan-3-yl}imidazo[1,5-a]pyridin-8-yl)-N-(isopropyl)benzamide 457-1

### N-ethyl-5-fluoro-2-(3-methyl-6-{1-[(3R)-4-methyl-1-[(3S)-morpholin-3-yl]pentan-3-yl]azacyclobutan-3-yl}imidazo[1,5-a]pyridin-8-yl)-N-(isopropyl)benzamide 457-2

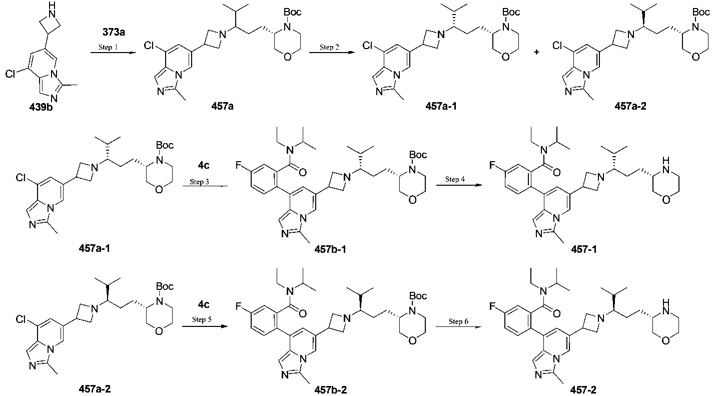

### Step 1

### (3S)-3-[3-(3-{8-chloro-3-methylimidazo[1,5-a]pyridin-6-yl}azacyclobutan-1-yl)-4-methylpentyl]morpholin-4-tert-butyl carboxylate 457a

Compound **439b** (380 mg, 1.71 mmol) and Compound **373a** (489.18 mg, 1.71 mmol) were dissolved in methanol (10.0 mL), followed by the addition of ZnCl₂ (467.27 mg, 3.43 mmol), and the mixture was stirred at 50°C for 1.0 hour. Sodium cyanoborohydride (323.16 mg, 5.14 mmol) was added, and the mixture was stirred at 50°C for 2.0 hours. A saturated aqueous potassium carbonate solution (30 mL) was added to the reaction mixture, which was then extracted with dichloromethane (30 mL x 3). The organic phases were combined, dried over anhydrous sodium sulfate, and concentrated under a reduced pressure. The resulting residues were purified by the silica-gel column chromatography using dichloromethane/methanol as an elution system, and the obtained crude product was purified by the HPLC (mobile phase: acetonitrile, water (0.1% formic acid), elution gradient: 10%-29%), to obtain the title Compound **457a** (210 mg, 101.82 µmol, yield: 5.94%) as white solid.
MS m/z (ESI): 491.1 [M+1]

### Step 2

### (3S)-3-[(3S)-3-(3-{8-chloro-3-methylimidazo[1,5-a]pyridin-6-yl}azacyclobutan-1-yl)-4-methylpentyl]morpholin-4-tert-butyl carboxylate 457a-1

### (3S)-3-[(3R)-3-(3-{8-chloro-3-methylimidazo[1,5-a]pyridin-6-yl}azacyclobutan-1-yl)-4-methylpentyl]morpholin-4-tert-butyl carboxylate 457a-2

Compound **457a** was separated and purified by the chiral preparative SFC (preparative column: DAICEL CHIRALPAK IG (250 mm * 30 mm,10 um), mobile phase: carbon dioxide-isopropanol (0.1% ammonia water); elution gradient: 40%). Mixtures **B** (MS m/z (ESI): 491.2 [M+1], Chiral HPLC analysis: retention time: 2.543 min; chromatographic column: Chiralpak AD-3 50 x 4.6 mm I.D., 3 um; mobile phase: carbon dioxide-isopropanol (0.05% diethylamide): elution gradient: 5%-40%) and **A** (MS m/z (ESI): 491.2 [M+1], Chiral HPLC analysis: retention time: 2.543 min; chromatographic column: (S,S)Whelk-Ol 50 x 4.6 mm I.D., 3.5 um; mobile phase: carbon dioxide-isopropanol (0.05% diethylamide): elution gradient: 5%-40%) were obtained. The mixture B was further separated and purified by the chiral preparative SFC (preparative column: DAICEL CHIRALPAK AD (250 mm * 30 mm, 10 um), mobile phase: carbon dioxide-isopropanol (0.1% ammonia water); elution gradient: 30%), to obtain the title Compound **457a-1** (110 mg); and the mixture A was further separated and purified by the chiral preparative HPLC (preparative column: DAICEL CHIRALPAK ID (250 mm * 30mm,10 um), mobile phase: n-hexane-isopropanol (0.1% ammonia water); elution gradient: 15%), to obtain the title Compound **457a-2** (100 mg).

### Single-configuration Compound 457a-1:

MS m/z (ESI): 491.2 [M+1]
Chiral HPLC analysis: retention time: 1.418 min; chromatographic column: DAICEL CHIRALPAK AD (250 mm * 30 mm,10 um), mobile phase: carbon dioxide-isopropanol (0.05% diethylamide); elution gradient: 5%-40%.

### Single-configuration Compound 457a-2:

MS m/z (ESI): 491.2 [M+1]
Chiral HPLC analysis: retention time: 2.784 min; chromatographic column: Chiralpak ID-3 50 x 4.6 mm I.D., 3 um; mobile phase: n-hexane-isopropanol (0.05% isopropylamine): elution gradient: 10%.

### Step 3

### (3S)-3-[(3S)-3-[3-(8-{2-[ethyl(isopropyl)aminofonnyl]-4-fluorophenyl}-3-methylimidazo[1,5-a]pyridin-6-yl)azacyclobutan-1-yl]-4-methylpentyl]morpholin-4-tert-butyl carboxylate 457b-1

Compound **457a-1** (110 mg, 224 µmol), Compound **4c**(225.27 mg, 672.01 µmol) and potassium phosphate (142.65 mg, 672.01 µmol) were dissolved in dioxane (2.0 mL) and water (0.5 mL). Pd(dtbpf)Cl₂ (14.60 mg, 22.40 µmol) was added. Under a nitrogen atmosphere, the mixture was stirred at 100°Cto allow for reacting for 16 hours. The reaction mixture was concentrated under a reduced pressure. The resulting residues were purified by the silica-gel column chromatography using dichloromethane/methanol as an elution system, to obtain the title Compound **457b-1** (160 mg, 241.01 µmol, yield: 107.59%) as brown oil.
MS m/z (ESI): 664.5 [M+1]

### Step 4

### N-ethyl-5-fluoro-2-(3-methyl-6-{1-[(3S)-4-methyl-1-[(3S)-morpholin-3-yl]pentan-3-yl]azacyclobutan-3-yl}imidazo[1,5-a]pyridin-8-yl)-N-(isopropyl)benzamide 457-1

Compound **457b-1** (160.00 mg, 241.01 µmol) was dissolved in dichloromethane (2.0 mL), followed by the addition of trifluoroacetic acid (1.48 g, 12.98 mmol, 1 mL). The mixture was stirred at room temperature to allow for reacting for 1.0 hour. The reaction mixture was concentrated under a reduced pressure, and purified by the HPLC (mobile phase: acetonitrile, water (0.1% formic acid), elution gradient: 0%-22%), to obtain the title Compound **457-1** (69.52 mg, 123.32 µmol, yield: 51.17%) as green solid.
MS m/z (ESI): 564.3 [M+1]
Chiral HPLC analysis: retention time: 1.113 min; chromatographic column: Chiralcel OD-3 50 x 4.6 mm I.D., 3 um; mobile phase: carbon dioxide-methanol (0.05% diethylamide): elution gradient: 5%-40%.

¹H NMR (400 MHz, DMSO-*d₆*) δ 8.76 - 10.14 (m, 2 H), 8.09 (br s, 1 H), 7.63 - 7.72 (m, 1 H), 7.40 (td, J=8.40, 2.40 Hz, 1 H), 7.34 (br d, J=8.40 Hz, 1 H), 7.05 - 7.11 (m, 1 H), 6.72 - 6.84 (m, 1 H), 4.05 - 4.27 (m, 2 H), 3.76 - 4.00 (m, 5 H), 3.61 (br t, J=11.20 Hz, 1 H), 3.41 - 3.48 (m, 2 H), 3.22 (br s, 3 H), 3.00 - 3.11 (m, 2 H), 2.88 (br dd, J=13.60, 6.80 Hz, 1 H), 2.61 (s, 3 H), 1.91 (br d, J=1.20 Hz, 1 H), 1.47 - 1.61 (m, 4 H), 0.85 - 0.98 (m, 10 H), 0.71 - 0.77 (m, 3 H), 0.26 (br d, J=6.00 Hz, 2 H).

### Step 5

### (3S)-3-[(3R)-3-[3-(8-{2-[ethyl(isopropyl)aminofonnyl]-4-fluorophenyl}-3-methylimidazo[1,5-a]pyridin-6-yl)azacyclobutan-1-yl]-4-methylpentyl]morpholin-4-tert-butyl carboxylate 457b-2

Compound **457a-2** (100 mg, 203.64 µmol), Compound **4c**(273.06 mg, 814.56 µmol) and potassium phosphate (129.68 mg, 610.92 µmol) were dissolved in dioxane (2.0 mL) and water (0.5 mL). Pd(dtbpf)Cl₂ (7.01 mg, 8.15 µmol) was added. Under a nitrogen atmosphere, the mixture was stirred at 90°Cto allow for reacting for 16 hours. The reaction mixture was concentrated under a reduced pressure. The resulting residues were purified by the silica-gel column chromatography using dichloromethane/methanol as an elution system, to obtain the title Compound **457b-2** (110 mg, 165.70 µmol, yield: 81.37%).
MS m/z (ESI): 664.6 [M+1]

### Step 6

### N-ethyl-5-fluoro-2-(3-methyl-6-{1-[(3R)-4-methyl-1-[(3S)-morpholin-3-yl]pentan-3-yl]azacyclobutan-3-yl}imidazo[1,5-a]pyridin-8-yl)-N-(isopropyl)benzamide 457-2

Compound **457b-2** (110 mg, 165.70 µmol) was dissolved in dichloromethane (2.0 mL), followed by the addition of trifluoroacetic acid (1.48 g, 12.98 mmol, 1 mL). The mixture was stirred at room temperature to allow for reacting for 1.0 hour. The reaction mixture was concentrated under a reduced pressure. The resulting residues were purified by the HPLC (mobile phase: acetonitrile, water (0.1% formic acid), elution gradient: 0%-27%), to obtain the title Compound **457-2** (64.25 mg, 113.97 µmol, yield: 68.78%) as green solid.
MS m/z (ESI): 564.3 [M+1]
Chiral HPLC analysis: retention time: 1.112 min; chromatographic column: Chiralcel OD-3 50 x 4.6 mm I.D., 3 um; mobile phase: carbon dioxide-methanol (0.05% diethylamide): elution gradient: 5%-40%.

¹H NMR (400 MHz, DMSO-*d₆*) δ 8.78 - 9.71 (m, 2 H), 8.05 - 8.27 (m, 1 H), 7.60 - 7.83 (m, 1 H), 7.26 - 7.51 (m, 2 H), 7.04 - 7.19 (m, 1 H), 6.80 (br d, J=11.20 Hz, 1 H), 4.07 - 4.55 (m, 4 H), 3.85 - 4.03 (m, 3 H), 3.63 (br t, J=11.60 Hz, 1 H), 3.41 - 3.49 (m, 2 H), 3.02 - 3.30 (m, 5 H), 2.89 (br dd, J=12.80, 5.60 Hz, 1 H), 2.63 (s, 3 H), 2.01 (br s, 1 H), 1.43 - 1.76 (m, 4 H), 0.80 - 1.15 (m, 10 H), 0.68 - 0.78 (m, 3 H), 0.28 (br d, J=6.00 Hz, 2 H).

### Example 458

### N-ethyl-5-fluoro-2-[1-methyl-6-(1-{4-methyl-1-[(3R)-morpholin-3-yl]pentan-3-yl}azacyclobutan-3-yl}-1H-indazol-4-yl)-N-(isopropyl)benzamide 458

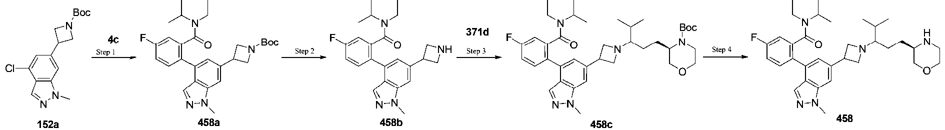

### Step 1

### 3-(4-{2-[ethyl(isopropyl)aminoformyl]-4-fluorophenyl}-1-methyl-1H-indazol-6-yl)azacyclobutan-1-tert-butyl carboxylate 458a

Compound **152a** (1 g, 3.11 mmol), Compound **4c**(1.04 g, 3.11 mmol) and potassium phosphate (1.65 g, 7.77 mmol) were dissolved in dioxane (10 mL) and water (2 mL). Xphos-Pd-G4 (267.39 mg, 310.75 µmol) was added. Under a nitrogen atmosphere, the mixture was stirred at 90°Cto allow for reacting for 1.0 hours. The reaction mixture was cooled to room temperature, water (10 mL) was added, and the mixture was extracted with ethyl acetate (15 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **458a** (800 mg, 1.62 mmol, yield: 52.05%) as brown solid.
MS m/z (ESI): 495.2 [M+1]

### Step 2

### 2-[6-(azacyclobutan-3-yl)-1-methyl-1H-indazol-4-yl]-N-ethyl-5-fluoro-N-(isopropyl)benzamide 458b

Compound **458a** (150 mg, 303.28 µmol) was dissolved in dichloromethane (6.0 mL), followed by the addition of trifluoroacetic acid (2.96 g, 25.96 mmol, 2 mL). The mixture was stirred at room temperature to allow for reacting for 1.0 hour. The reaction mixture was concentrated under a reduced pressure, followed by adding dichloromethane (5 mL) and water (5 mL), regulating to PH >7 with a 10% aqueous sodium hydroxide solution and extracting with dichloromethane (10 mL x 3). The organic phases were combined, dried over anhydrous sodium sulfate, and concentrated under a reduced pressure. The resulting residues were purified by the HPLC (mobile phase: acetonitrile, water (0.1%ammonium bicarbonate), elution gradient: 28%-58%), to obtain the title Compound **458b** (119 mg, 301.66 µmol, yield: 99.47%) as black solid. The crude product was directly used in the next step of reaction without purification.

### Step 3

### (3R)-3-{3-[3-(4-{2-[ethyl(isopropyl)aminoformyl]-4-fluorophenyl}-1-methyl-1H-indazol-6-yl)azacyclobutan-1-yl]-4-methylpentyl}morpholin-4-tert-butyl carboxylate 458c

Compound **458b** (119 mg, 301.66 µmol) and Compound **371d**(103.30 mg, 361.99 µmol) were dissolved in methanol (5.0 mL), followed by the addition of ZnCl₂ (82.23 mg, 603.32 µmol), and the mixture was stirred at 50°C for 1.0 hour. Sodium cyanoborohydride (37.91 mg, 603.32 µmol) was added, and the mixture was stirred at 50°C for 2.0 hours. Water (20 mL) was added to the reaction mixture, which was then extracted with dichloromethane (30 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **458c** (120 mg, 180.76 µmol, yield: 59.92%) as yellow oil.
MS m/z (ESI): 664.4 [M+1]

### Step 4

### N-ethyl-5-fluoro-2-[1-methyl-6-(1-{4-methyl-1-[(3R)-morpholin-3-yl]pentan-3-yl}azacyclobutan-3-yl}-1H-indazol-4-yl)-N-(isopropyl)benzamide 458

Compound **458c** (120.00 mg, 180.76 µmol) was dissolved in dichloromethane (6 mL), followed by the addition of trifluoroacetic acid (2.96 g, 25.96 mmol, 2 mL). The mixture was stirred at room temperature to allow for reacting for 1.0 hour. The reaction mixture was concentrated under a reduced pressure, followed by adding dichloromethane (5 mL) and water (5 mL), regulating to PH >7 with a 10% aqueous sodium hydroxide solution and extracting with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 35%-65%), to obtain the title Compound **458** (68.61 mg, 121.70 µmol, yield: 67.33%) as white solid.
MS m/z (ESI): 564.3 [M+1]
¹H NMR (400 MHz, CD₃OD) δ 7.88 (s, 1H), 7.67 - 7.61 (m, 1H), 7.52 - 7.46 (m, 1H), 7.35 (dt, J = 2.8, 8.4 Hz, 1H), 7.27 - 7.14 (m, 2H), 4.09 (s, 3H), 3.91 - 3.74 (m, 5H), 3.53 - 3.36 (m, 3H), 3.19 - 3.10 (m, 1H), 2.98 - 2.79 (m, 3H), 2.73 - 2.64 (m, 1H), 2.26 - 2.17 (m, 1H), 1.94 - 1.81 (m, 1H), 1.60 - 1.15 (m, 5H), 1.10 - 0.88 (m, 10H), 0.84 - 0.69 (m, 4H), 0.10 (d, J = 6.4 Hz, 2H).

### Examples 459-1 and 459-2

### N-(2,2-difluoroethyl)-5-fluoro-2-(6- {1-[(3R)-6-[4-(2-hydroxylethyl)piperazin-1-yl]-2-methylhexan-3-yl]azacyclobutan-3-yl}-3-methyl-imidazo[1,5-a]pyridin-8-yl)-N-(isopropyl)benzamide 459-1

### N-(2,2-difluoroethyl)-5-fluoro-2-(6-{1-[(3S)-6-[4-(2-hydroxylethyl)piperazin-1-yl]-2-methylhexan-3-yl]azacyclobutan-3-yl}-3-methyl-imidazo[1,5-a]pyridin-8-yl)-N-(isopropyl)benzamide 459-2

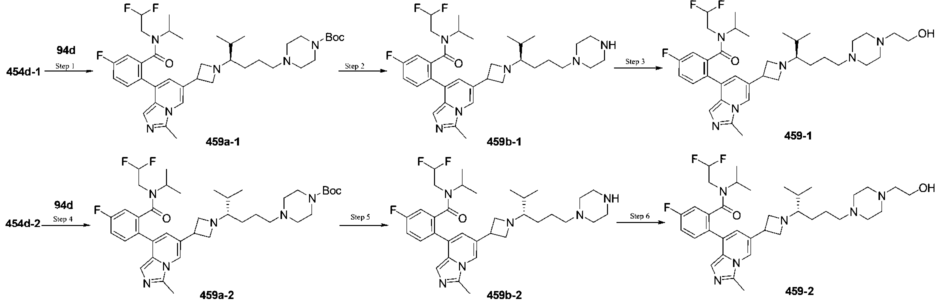

### Step 1

### 4-[(4R)-4-[3-(8-{2-[(2,2-difluoroethyl)(isopropyl)aminofonnyl]-4-fluorophenyl}-3-methylimidazo[1,5-a]pyridin-6-yl)azacyclobutan-1-yl]-5-methylhexyl]piperazin-1-tert-butyl carboxylate 459a-1

Compound **454d-1** (200.00 mg, 396.74 µmol), Compound **94d**(176.73 mg, 476.09 µmol) and potassium phosphate (252.64 mg, 1.19 mmol) were dissolved in dioxane (5 mL) and water (1 mL). Xphos-Pd-G4 (34.14 mg, 39.67 µmol) was added. Under a nitrogen atmosphere, the mixture was stirred at 90°Cto allow for reacting for 1.0 hours. The reaction mixture was cooled to room temperature, water (15 mL) was added, and the mixture was extracted with ethyl acetate (15 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **459a-1** (170 mg, 238.47 µmol, yield: 60.11%).
MS m/z (ESI): 713.4 [M+1]

### Step 2

### N-(2,2-difluoroethyl)-5-fluoro-2-(3-methyl-6-{1-[(3R)-2-methyl-6-(piperazin-1-yl)hexan-3-yl]azacyclobutan-3-yl}imidazo[1,5-a]pyridin-8-yl)-N-(isopropyl)benzamide 459b-1

Compound **459a-1**(170.00 mg, 238.47 µmol) was dissolved in dichloromethane (3.0 mL), followed by the addition of trifluoroacetic acid (1.48 g, 12.98 mmol, 1 mL). The mixture was stirred at 25°C to allow for reacting for 1.0 hours. The reaction mixture was concentrated under a reduced pressure, regulated to pH = 10 with a 10 % aqueous NaOH solution, and extracted with dichloromethane (15 mL x 3). The organic phases were combined, dried over anhydrous sodium sulfate, and concentrated under a reduced pressure, to obtain the crude product of title Compound **459b-1** (140 mg, 228.47 µmol, yield: 95.81%). The crude product was directly used in the next step of reaction without purification.
MS m/z (ESI): 613.4 [M+1]

### Step 3

### N-(2,2-difluoroethyl)-5-fluoro-2-(6-{1-[(3R)-6-[4-(2-hydroxylethyl)piperazin-1-yl]-2-methylhexan-3-yl]azacyclobutan-3-yl}-3-methyl-imidazo[1,5-a]pyridin-8-yl)-N-(isopropyl)benzamide 459-1

The crude product of Compound **459b-1** (140.00 mg, 228.47 µmol) was dissolved in acetonitrile (3.0 mL), followed by the addition of 2-bromoethanol (34.26 mg, 274.16 µmol) and potassium carbonate (94.73 mg, 685.41 µmol), and the mixture was stirred at 80°C for 12 hours. Water (10 mL) was added to the reaction mixture, which was then extracted with ethyl acetate (15 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 34%-54%). The title Compound **459-1** (25.25 mg, 38.44 µmol, yield: 16.83%) was obtained.

### Single-configuration Compound 459-1:

MS m/z (ESI):657.5 [M+1]
Chiral SFC: retention time: 1.115 min; chromatographic column: Chiralcel AD-3 50×4.6 mm I.D., 3 um; mobile phase: CO₂/MeOH (0.05% DEA): elution gradient: 5%-40%.

¹H NMR (400 MHz, DMSO-*d₆*) δ 7.93 (s, 1H), 7.73 - 7.64 (m, 1H), 7.48 - 7.26 (m, 2H), 7.07 - 6.96 (m, 1H), 6.82 - 6.67 (m, 1H), 5.98 - 5.57 (m, 1H), 4.37 - 4.28 (m, 1H), 3.69 - 3.33 (m, 8H), 3.16 - 3.00 (m, 2H), 2.62 - 2.55 (m, 3H), 2.41 - 1.66 (m, 14H), 1.52 - 1.32 (m, 2H), 1.31 - 0.80 (m, 12H), 0.30 (d, J = 6.4 Hz, 2H).

### Step 4

### 4-[(4S)-4-[3-(8-{2-[(2,2-difluoroethyl)(isopropyl)aminoformyl]-4-fluorophenyl}-3-methylimidazo[1,5-a]pyridin-6-yl)azacyclobutan-1-yl]-5-methylhexyl]piperazin-1-tert-butyl carboxylate 459a-2

Compound **454d-2** (200.00 mg, 396.74 µmol), Compound **94d**(176.73 mg, 476.09 µmol) and potassium phosphate (252.64 mg, 1.19 mmol) were dissolved in dioxane (5 mL) and water (1 mL). Xphos-Pd-G4 (34.14 mg, 39.67 µmol) was added. Under a nitrogen atmosphere, the mixture was stirred at 90°Cto allow for reacting for 1.0 hours. The reaction mixture was cooled to room temperature, water (15 mL) was added, and the mixture was extracted with ethyl acetate (15 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **459a-2** (260 mg, 364.72 µmol, yield: 91.93%).
MS m/z (ESI): 713.4 [M+1]

### Step 5

### N-(2,2-difluoroethyl)-5-fluoro-2-(3-methyl-6-{1-[(3S)-2-methyl-6-(piperazin-1-yl)hexan-3-yl]azacyclobutan-3-yl}imidazo[1,5-a]pyridin-8-yl)-N-(isopropyl)benzamide 459b-2

Compound **459a-2**(130.00 mg, 182.36 µmol) was dissolved in dichloromethane (3.0 mL), followed by the addition of trifluoroacetic acid (1.48 g, 12.98 mmol, 1 mL). The mixture was stirred at 25°C to allow for reacting for 1.0 hours. The reaction mixture was concentrated under a reduced pressure, regulated to pH = 10 with a 10 % aqueous NaOH solution, and extracted with dichloromethane (15 mL x 3). The organic phases were combined, dried over anhydrous sodium sulfate, and concentrated under a reduced pressure, to obtain the crude product of title Compound **459b-2** (110 mg, 179.51 µmol, yield: 98.44%). The crude product was directly used in the next step of reaction without purification.
MS m/z (ESI): 613.4 [M+1]

### Step 6

### N-(2,2-difluoroethyl)-5-fluoro-2-(6-{1-[(3S)-6-[4-(2-hydroxylethyl)piperazin-1-yl]-2-methylhexan-3-yl]azacyclobutan-3-yl}-3-methyl-imidazo[1,5-a]pyridin-8-yl)-N-(isopropyl)benzamide 459-2

The crude product of Compound **459b-2** (110.00 mg, 179.51 µmol) was dissolved in acetonitrile (3.0 mL), followed by the addition of 2-bromoethanol (26.92 mg, 215.42 µmol) and potassium carbonate (74.43 mg, 538.54 µmol), and the mixture was stirred at 80°C for 12 hours. Water (10 mL) was added to the reaction mixture, which was then extracted with ethyl acetate (15 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 34%-54%). The title Compound **459-2** (20.38 mg, 314.03 µmol, yield: 17.28%) was obtained.

### Single-configuration Compound 459-2:

MS m/z (ESI):657.5 [M+1]
Chiral SFC: retention time: 1.231 min; chromatographic column: Chiralcel OD-3 50×4.6 mm I.D., 3um,; mobile phase: CO₂/MeOH (0.05% DEA): elution gradient: 5%-40%.

¹H NMR (400 MHz, DMSO-*d₆*) δ 7.93 (s, 1H), 7.73 - 7.65 (m, 1H), 7.48 - 7.26 (m, 2H), 7.05 - 6.97 (m, 1H), 6.81 - 6.70 (m, 1H), 5.94 - 5.59 (m, 1H), 4.36 - 4.28 (m, 1H), 3.71 - 3.34 (m, 8H), 3.13 - 3.01 (m, 2H), 2.63 - 2.54 (m, 3H), 2.48 - 1.62 (m, 14H), 1.49 - 1.34 (m, 2H), 1.31 - 0.79 (m, 12H), 0.30 (d, J = 6.4 Hz, 2H).

### Example 461,462

### (2R)-2-{2-[3-(8-{2-[ethyl(isopropyl)aminoformyl]-4-fluorophenyl}-3-methylimidazo[1,5-a]pyridin-6-yl)azacyclobutan-1-yl]-3-methylbutyl}morpholin-4-tert-butyl carboxylate 461

### N-ethyl-5-fluoro-2-[3-methyl-6-(1-{3-methyl-1-[(2R)-morpholin-2-yl]butan-2-yl}azacyclobutan-3-yl)imidazo[1,5-a]pyridin-8-yl]- N-(isopropyl)benzamide 462

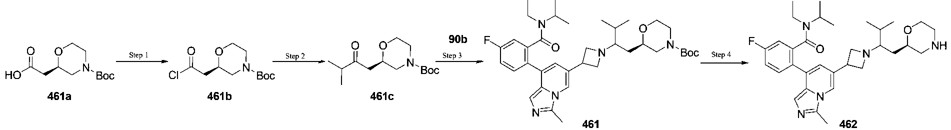

### Step 1

### (2R)-2-(2-chloro-2-oxyethyl)morpholin-4-tert-butyl carboxylate 461b

2-[(2*R*)-4-[(tert-butoxy)carbonyl]morpholin-2-yl]acetic acid (1 g, 4.08 mmol) was dissolved in dichloromethane (10 mL), followed by the addition of DMF (14.90 mg, 203.86 µmol). The mixture was cooled to 0°C, followed by the addition of SOCl₂ (727.58 mg, 6.12 mmol). The reaction mixture was stirred at room temperature for 1.0 hour, and concentrated under a reduced pressure, to obtain the crude product of title Compound **461b** (1 g, 3.79 mmol, yield: 93.01%). The crude product was directly used in the next step of reaction.

### Step 2

### (2R)-2-(3-methyl-2-oxobutyl)morpholin-4-tert-butyl carboxylate 461c

Under the protection of nitrogen, Compound **461b**(1.08 g, 4.10 mmol) was dissolved in tetrahydrofuran (15 mL), followed by the addition of ferric acetylacetonate (723.17 mg, 2.05 mmol). Under the protection of nitrogen, the mixture was cooled to 0°C, followed by the slow dropwise addition of an isopropylmagnesium chloride solution (2.0 M, 2.05 mL). After the completion of dropwise addition, the mixture was further stirred at 0°C for 1.0 hours. The reaction mixture was quenched by adding a saturated aqueous ammonium chloride solution (20 mL), and extracted with ethyl acetate (15 mL×3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **461c** (120 mg, 442.23 µmol, yield: 10.8%) as colorless oil.

¹H NMR (400 MHz, CDCl₃) δ 3.98 - 3.76 (m, 4H), 3.53 (dt, J = 2.5, 11.6 Hz, 1H), 2.98 - 2.82 (m, 1H), 2.78 - 2.55 (m, 3H), 2.44 (dd, J = 4.6, 16.4 Hz, 1H), 1.47 (s, 9H), 1.11 (d, J = 7.0 Hz, 6H).

### Step 3

### (2R)-2-12-[3-(8-{2-[ethyl(isopropyl)aminoformyl]-4-fluorophenyl}-3-methylimidazo[1,5-a]pyridin-6-yl)azacyclobutan-1-yl]-3-methylbutyl}morpholin-4-tert-butyl carboxylate 461

Compound **90b** (180 mg, 456.29 µmol) and Compound **461c**(123.82 mg, 456.29 µmol) were dissolved in methanol (2.0 mL), followed by the addition of ZnCl₂ (124.38 mg, 912.58 µmol), and the mixture was stirred at 50°C for 1.0 hour. The mixture was cooled to room temperature, followed by adding sodium cyanoborohydride (86.02 mg, 1.37 mmol) and stirring at 50°C for 10 hour. A saturated aqueous sodium bicarbonate solution (5.0 mL) was added to the reaction mixture, which was then extracted with dichloromethane (20 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **461** (150 mg, 230.83 µmol, yield: 50.59%) as white solid.
MS m/z (ESI): 650.4 [M+1]
¹H NMR (400 MHz, DMSO-*d₆*) δ 8.02 - 7.86 (m, 1H), 7.75 - 7.60 (m, 1H), 7.45 - 7.23 (m, 2H), 7.12 - 6.98 (m, 1H), 6.90 - 6.65 (m, 1H), 3.85 - 3.42 (m, 8H), 3.29 - 2.74 (m, 6H), 2.59 (s, 3H), 2.31 - 2.19 (m, 1H), 1.80 - 1.69 (m, 1H), 1.42 - 1.33 (m, 11H), 0.93 - 0.71 (m, 14H), 0.30 - 0.24 (m, 2H).

### Step 4

### N-ethyl-5-fluoro-2-[3-methyl-6-(1-{3-methyl-1-[(2R)-morpholin-2-yl]butan-2-yl}azacyclobutan-3-yl)imidazo[1,5-a]pyridin-8-yl]-N-(isopropyl)benzamide 462

Compound **461** (120 mg, 184.66 µmol) was dissolved in dichloromethane (1.5 mL), followed by the addition of trifluoroacetic acid (740.00 mg, 6.49 mmol, 0.5 mL), and the mixture was stirred at 25°C for 1.0 hour. The reaction mixture was concentrated under a reduced pressure, and purified by the HPLC (mobile phase: acetonitrile, water (0.1% ammonium bicarbonate), elution gradient: 29%-59%), to obtain the title Compound **462** (93 mg, 169.18 µmol, yield: 91.64%) as yellow solid.
MS m/z (ESI): 550.3 [M+1]
¹H NMR (400 MHz, DMSO-*d₆*) δ 7.99 - 7.84 (m, 1H), 7.75 - 7.62 (m, 1H), 7.45 - 7.26 (m, 2H), 7.09 - 6.98 (m, 1H), 6.84 - 6.66 (m, 1H), 4.35 - 3.38 (m, 6H), 3.28 - 2.54 (m, 11H), 2.48 - 2.13 (m, 3H), 1.79 - 1.66 (m, 1H), 1.35 - 0.72 (m, 15H), 0.32 - 0.25 (m, 2H).

### Example 463

### 5-fluoro-2-(6-{1-[(3R)-6-[4-(2-hydroxylethyl)piperazin-1-yl]-2-methylhexan-3-yl]azacyclobutan-3-yl}-3-methyl-imidazo[1,5-a]pyridin-8-yl)-N, N-di(isopropyl)benzamide 463

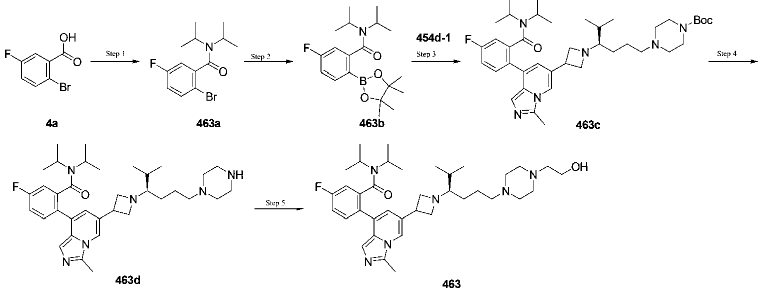

### Step 1

### 2-bromo-5-fluoro- N,N-di(isopropyl)benzamide 463a

N-isopropylprop-2-amine (508.24 mg, 5.02 mmol) and triethylamine (1.39 g, 13.70 mmol) were added to the solution of 2-bromo-5-fluoro-benzoic acid **4a**(1 g, 4.57 mmol) in dichloromethane (10 mL), followed by the slow addition of HATU (2.08 g, 5.48 mmol). The mixture was stirred at 25°C for 12 hours. Water (100 mL) was added to the reaction mixture, which was then extracted with dichloromethane (100 mL×3). The organic phases were combined, dried over anhydrous sodium sulfate, filtered and concentrated under a reduced pressure. The resulting residues were purified by the silica-gel column chromatography using petroleum ether/ethyl acetate as an elution system, to obtain the title Compound **463a** (0.46 g, 1.52 mmol, yield33.34%) as yellow solid.
MS m/z (ESI): 303.9 [M+1]

### Step 2

### 5-fluoro-N,N-di(isopropyl)-2-(4,4,5,5-tetramethyl-1,3,2-dioxaboran-2-yl)benzamide 463b

Compound **463a** (2 g, 6.62 mmol), potassium acetate (1.95 g, 19.86 mmol) and bis(pinacolato)diboron (3.36 g, 13.24 mmol) were dissolved in DMSO (10 mL). Under a nitrogen atmosphere, Pd(dppf)Cl₂ (242.14 mg, 330.93 µmol) was added, and the mixture was stirred at 110°C to allow for reacting for 12 hours. The mixture was cooled to room temperature, followed by adding 50 mL of ethyl acetate and washing with water (30ml×3). The organic layer was dried over anhydrous sodium sulfate, filtered and concentrated under a reduced pressure, and the resulting residues were purified by the silica-gel column chromatography using petroleum ether/ethyl acetate as an elution system, to obtain the title Compound **463b** (900 mg, 2.58 mmol, yield: 38.94%) as yellow solid.
MS m/z (ESI): 349.8 [M+1]

### Step 3

### 4-[(4R)-4-[3-(8-{2-[di(isopropyl)aminoformyl]-4-fluorophenyl}-3-methylimidazo[1,5-a]pyridin-6-yl)azacyclobutan-1-yl]-5-methylhexyl]piperazin-1-tert-butyl carboxylate 463c

Compound **454d-1** (220 mg, 436.42 µmol), Compound **463b**(179.31 mg, 349.13 µmol) and potassium phosphate (277.91 mg, 1.31 mmol) were dissolved in dioxane (20 mL) and water (5 mL). Pd(dtbpf)Cl₂ (28.44 mg, 43.64 µmol) was added. Under a nitrogen atmosphere, the mixture was stirred at 95°Cto allow for reacting for 2.0 hours. The reaction mixture was cooled to room temperature, followed by adding water (20 mL) and extracting with dichloromethane (20 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **463c** (141 mg, 204.07 µmol, yield: 46.76%) as yellow oil.
MS m/z (ESI): 691.5 [M+1]

### Step 4

### 5-fluoro-2-(3-methyl-6-{1-[(3R)-2-methyl-6-(piperazin-1-yl)hexan-3-yl]azacyclobutan-3-yl}imidazo[1,5-a]pyridin-8-yl)-N,N-di(isopropyl)benzamide 463d

Compound **463c** (200 mg, 289.46 µmol) was dissolved in dichloromethane (12 mL), followed by the addition of trifluoroacetic acid (4 mL). The mixture was stirred at 25°C to allow for reacting for 2.0 hours. The reaction mixture was concentrated under a reduced pressure, followed by adding dichloromethane (5 mL) and water (5 mL), regulating to pH >7 with a 10% aqueous NaOH solution and extracting with dichloromethane (10 mL x 3). The organic phases were combined, dried over anhydrous sodium sulfate, and concentrated under a reduced pressure, to obtain the crude product of title Compound **463d** (171 mg, 289.43 µmol, yield: 99.99%). The crude product was directly used in the next step of reaction without purification.
MS m/z (ESI): 591.4 [M+1]

### Step 5

### 5-fluoro-2-(6-{1-[(3R)-6-[4-(2-hydroxylethyl)piperazin-1-yl]-2-methylhexan-3-yl]azacyclobutan-3-yl}-3-methyl-imidazo[1,5-a]pyridin-8-yl)-N,N-di(isopropyl)benzamide 463

The crude product of Compound **463d** (170 mg, 287.74 µmol) was dissolved in acetonitrile (10.0 mL), followed by the addition of 2-bromoethanol (39.55 mg, 316.51 µmol) and potassium carbonate (119.30 mg, 863.21 µmol), and the mixture was stirred at 80°C for 2 hours. Water (20 mL) was added to the reaction mixture, which was then extracted with dichloromethane (20 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 32%-52%). The title Compound **463** (61.28 mg, 96.52 µmol, yield: 33.55%) was obtained as yellow solid.
MS m/z (ESI):635.5 [M+1]
Chiral SFC: retention time: 1.241 min; chromatographic column: Chiralcel OD-3 50×4.6 mm I.D., 3um,; mobile phase: CO₂/MeOH (0.05% DEA): elution gradient: 5%-40%.

¹H NMR (400 MHz, CD₃OD) δ 7.93 (s, 1H), 7.71 (dd, J = 5.6, 8.8 Hz, 1H), 7.32 (dt, J = 2.4, 8.0 Hz, 1H), 7.18 (dd, J = 2.8, 8.8 Hz, 1H), 7.13 (s, 1H), 6.92 (d, J = 7.0 Hz, 1H), 3.81 - 3.62 (m, 5H), 3.55 - 3.47 (m, 1H), 3.42 - 3.33 (m, 2H), 3.24 (t, J = 7.2 Hz, 1H), 2.66 (s, 3H), 2.63 - 2.42 (m, 8H), 2.36 (q, J = 7.6 Hz, 2H), 2.28 - 2.22 (m, 1H), 1.92 - 1.81 (m, 1H), 1.68 - 1.53 (m, 2H), 1.48 (d, J = 6.8 Hz, 3H), 1.45 - 1.20 (m, 3H), 1.19 - 1.14 (m, 3H), 1.08 - 0.85 (m, 10H), 0.24 (d, J = 6.4 Hz, 3H).

### Examples 464-1 and 464-2

### N-ethyl-5-fluoro-2-(6-{3-hydroxyl-1-[(3R)-6-[4-(2-hydroxylethyl)piperazin-1-yl]-2-methylhexan-3-yl]azacyclobutan-3-yl}-3-methylimidazo[1,5-a]pyridin-8-yl)-N-(isopropyl)benzamide 464-1

### N-ethyl-5-fluoro-2-(6-{3-hydroxyl-1-[(3S)-6-[4-(2-hydroxylethyl)piperazin-1-yl]-2-methylhexan-3-yl]azacyclobutan-3-yl}-3-methylimidazo[1,5-a]pyridin-8-yl)-N-(isopropyl)benzamide 464-2

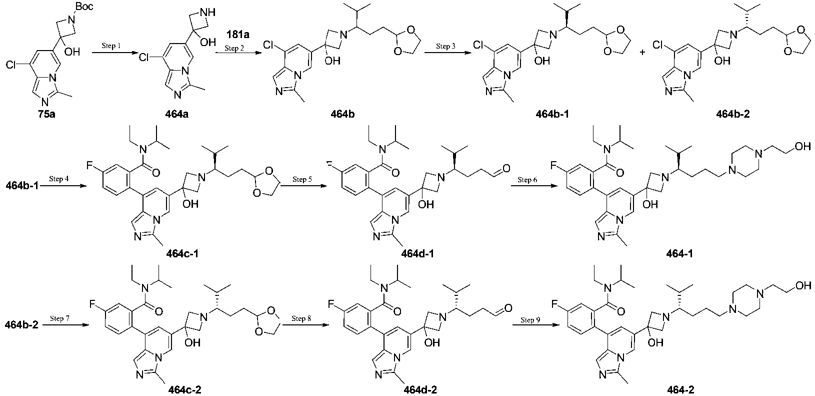

### Step 1

### 3-{8-chloro-3-methylimidazo[1,5-a]pyridin-6-yl}azacyclobutan-3-ol 464a

Compound **75a** (1.2 g, 3.55 mmol) was dissolved in dichloromethane (15.0 mL), followed by the addition of trifluoroacetic acid (7.40 g, 64.90 mmol, 5 mL) at 25°C. The mixture was stirred at room temperature to allow for reacting for 1.0 hours. The reaction mixture was concentrated under a reduced pressure, followed by adding dichloromethane (20 mL), regulating to pH > 7 with a 10% aqueous sodium hydroxide solution, and extracting with dichloromethane (40 mL x 3). The organic phases were combined, dried over anhydrous sodium sulfate, and concentrated under a reduced pressure, to obtain the crude product of title Compound **464a** (840 mg, 3.53 mmol, yield: 99.48%) as yellow solid. The crude product was directly used in the next step of reaction without purification.

### Step 2

### 3-{8-chloro-3-methylimidazo[1,5-a]pyridin-6-yl}-1-[1-(1,3-dioxolan-2-yl)-4-methylpentan-3-yl]azacyclobutan-3-ol 464b

Compound **464a** (840 mg, 3.53 mmol) and 1-(1,3-dioxolan-2-yl)-4-methylpentan-3-one **181a**(669.51 mg, 3.89 mmol) were dissolved in methanol (1.82 mL), followed by the addition of acetic acid (212.23 mg, 3.53 mmol), and the mixture was stirred at 40°C for 0.5 hours. The mixture was cooled to room temperature, followed by adding sodium cyanoborohydride (666.27 mg, 10.60 mmol) and stirring at 40°C for 15 hour. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **464b** (1.35 g, 3.43 mmol, yield: 96.98%) as yellow oil. MS m/z (ESI): 394.0 [M+1]

### Step 3

### 3-{8-chloro-3-methylimidazo[1,5-a]pyridin-6-yl}-1-[(3R)-1-(1,3-dioxolan-2-yl)-4-methylpentan-3-yl]azacyclobutan-3-ol 464b-1

### 3-{8-chloro-3-methylimidazo[1,5-a]pyridin-6-yl}-1-[(3S)-1-(1,3-dioxolan-2-yl)-4-methylpentan-3-yl]azacyclobutan-3-ol 464b-2

Compound **464b** (1.35 g, 3.43 mmol) was separated and purified by the chiral preparative SFC (preparative column: DAICEL CHIRALCEL OD (250 mm * 30 mm, 10 um), mobile phase: carbon dioxide-methanol (0.1% ammonia water); elution gradient: 15%), to obtain the title Compounds **464b-1** (600 mg, 1.52 mmol, yield: 44.44%) and **464b-2** (650 mg, 1.65 mmol, yield: 48.15%).

### Single-configuration Compound 464b-1:

MS m/z (ESI): 394.2 [M+1]
Chiral HPLC analysis: retention time: 1.166 min; chromatographic column: Chiralcel OD-3 50 x 4.6 mm I.D., 3 um; mobile phase: carbon dioxide-methanol (0.05% diethylamide): elution gradient: 5%-40%.

### Single-configuration Compound 464b-2:

MS m/z (ESI): 394.6 [M+1]
Chiral HPLC analysis: retention time: 1.300 min; chromatographic column: Chiralcel OD-3 50 x 4.6 mm I.D., 3 um; mobile phase: carbon dioxide-methanol (0.05% diethylamide): elution gradient: 5%-40%.

### Step 4

### 2-(6-{1-[(3R)-1-(1,3-dioxolan-2-yl)-4-methylpentan-3-yl]-3-hydroxylazacyclobutan-3-yl}-3-methylimidazo[1,5-a]pyridin-8-yl)-N-ethyl-5-fluoro-N-(isopropyl)benzamide 464c-1

Compound **464b-1** (460.00 mg, 1.17 mmol), Compound **4c**(587.20 mg, 1.75 mmol) and potassium phosphate (743.65 mg, 3.50 mmol) were dissolved in dioxane (5.0 mL) and water (1.0 mL). Pd(dtbpf)Cl₂ (76.11 mg, 116.78 µmol) was added. Under a nitrogen atmosphere, the mixture was stirred at 90°Cto allow for reacting for 12 hours. The reaction mixture was cooled to room temperature, water (10 mL) was added, and the mixture was extracted with ethyl acetate (15 mL x 3). The organic phases were combined, dried over anhydrous sodium sulfate, and concentrated under a reduced pressure. The resulting residues were purified by the preparative column chromatography using dichloromethane/methanol as an elution system, to obtain the title Compound **464c-1** (130 mg, 229.40 µmol, yield: 19.64%) as yellow solid.
MS m/z (ESI): 567.3[M+1]

### Step 5

### N-ethyl-5-fluoro-2-(6-{3-hydroxyl-1-[(3R)-2-methyl-6-oxohexan-3-yl]azacyclobutan-3-yl}-3-methylimidazo[1,5-a]pyridin-8-yl)-N-(isopropyl)benzamide 464d-1

Compound **464c-1** (130.00 mg, 229.40 µmol) was dissolved in acetonitrile (2.0 mL), followed by the addition of a hydrochloric acid solution (1.0 M; 2.0 mL). The mixture was stirred at 50°C to allow for reacting for 1.0 hours. The reaction mixture was concentrated under a reduced pressure, regulated to pH = 10 with a saturated aqueous potassium carbonate solution, and extracted with dichloromethane (15 mL x 3). The organic phases were combined, dried over anhydrous sodium sulfate, and concentrated under a reduced pressure, to obtain the crude product of title Compound **464d-1** (118 mg, 225.77 µmol, yield: 98.42%). The crude product was directly used in the next step of reaction without purification.

### Single-configuration Compound 464d-1:

MS m/z (ESI): 523.2 [M+1]

### Step 6

### N-ethyl-5-fluoro-2-(6-{3-hydroxyl-1-[(3R)-6-[4-(2-hydroxylethyl)piperazin-1-yl]-2-methylhexan-3-yl]azacyclobutan-3-yl}-3-methylimidazo[1,5-a]pyridin-8-yl)-N-(isopropyl)benzamide 464-1

The crude product of Compound **464d-1** (118.00 mg, 225.77 µmol) and 2-(piperazin-1-yl)ethanol (35.27 mg, 270.93 µmol) were dissolved in methanol (3.0 mL), followed by the addition of acetic acid (13.56 mg, 225.77 µmol). Under the protection of nitrogen, the mixture was stirred at room temperature for 1.0 hour. Sodium cyanoborohydride (42.56 mg, 677.31 µmol) was added, allowing for reacting at room temperature for 11 hours under the protection of nitrogen. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 20%-50%). The title Compound **464-1** (67.33 mg, 105.73 µmol, yield: 46.83%) was obtained as white solid.

### Single-configuration Compound 464-1:

MS m/z (ESI):637.3 [M+1]
Chiral SFC: retention time: 1.240 min; chromatographic column: Chiralpak OD-3 50×4.6 mm I.D., 3um,; mobile phase: CO₂/MeOH (0.05% DEA): elution gradient: 5%-40%.

¹H NMR (400 MHz, DMSO-*d₆*) δ 8.07 - 7.91 (m, 1H), 7.71 - 7.58 (m, 1H), 7.43 - 7.22 (m, 2H), 7.13 - 6.92 (m, 2H), 5.99 (s, 1H), 4.40 - 4.21 (m, 1H), 3.67 - 3.53 (m, 1H), 3.52 - 3.39 (m, 4H), 3.30 - 2.64 (m, 5H), 2.59 (s, 3H), 2.41 - 2.16 (m, 11H), 2.09 (d, J = 2.4 Hz, 1H), 1.78 - 1.64 (m, 1H), 1.51 - 1.37 (m, 2H), 1.34 - 1.14 (m, 2H), 1.08 - 0.72 (m, 13H), 0.31 (d, J = 6.4 Hz, 2H).

### Step 7

### 2-(6-{1-[(3S)-1-(1,3-dioxolan-2-yl)-4-methylpentan-3-yl]-3-hydroxylazacyclobutan-3-yl}-3-methylimidazo[1,5-a]pyridin-8-yl)-N-ethyl-5-fluoro-N-(isopropyl)benzamide 464c-2

Compound **464b-2** (550.00 mg, 1.40 mmol), Compound **4c**(936.12 mg, 2.09 mmol) and potassium phosphate (889.14 mg, 4.19 mmol) were dissolved in dioxane (8.0 mL) and water (2.0 mL). Xphos-Pd-G4 (60.07 mg, 69.81 µmol) was added. Under a nitrogen atmosphere, the mixture was stirred at 95°Cto allow for reacting for 2 hours. The reaction mixture was cooled to room temperature, followed by adding water (30 mL) and extracting with ethyl acetate (30 mL x 3). The organic phases were combined, dried over anhydrous sodium sulfate, and concentrated under a reduced pressure. The resulting residues were purified by the preparative column chromatography using dichloromethane/methanol as an elution system, to obtain the title Compound **464c-2** (600 mg, 1.06 mmol, yield: 75.83%) as yellow solid.
MS m/z (ESI): 567.3[M+1]

### Step 8

### N-ethyl-5-fluoro-2-(6-{3-hydroxyl-1-[(3S)-2-methyl-6-oxohexan-3-yl]azacyclobutan-3-yl}-3-methylimidazo[1,5-a]pyridin-8-yl)-N-(isopropyl)benzamide 464d-2

Compound **464c-2** (120.00 mg, 211.75 µmol) was dissolved in acetonitrile (2.0 mL), followed by the addition of a hydrochloric acid solution (1.0 M; 2.0 mL). The mixture was stirred at 50°C to allow for reacting for 0.5 hours. The reaction mixture was concentrated under a reduced pressure, regulated to pH = 10 with a 10% aqueous NaOH solution, and extracted with dichloromethane (10 mL x 3). The organic phases were combined, dried over anhydrous sodium sulfate, and concentrated under a reduced pressure, to obtain the crude product of title Compound **464d-2** (110 mg, 210.46 µmol, yield: 99.39%) as yellow solid. The crude product was directly used in the next step of reaction without purification.

### Single-configuration Compound 464d-2:

MS m/z (ESI): 523.2 [M+1]

### Step 9

### N-ethyl-5-fluoro-2-(6-{3-hydroxyl-1-[(3S)-6-[4-(2-hydroxylethyl)piperazin-1-yl]-2-methylhexan-3-yl]azacyclobutan-3-yl}-3-methylimidazo[1,5-a]pyridin-8-yl)-N-(isopropyl)benzamide 464-2

The crude product of Compound **464d-2** (110 mg, 210.46 µmol) and 2-(piperazin-1-yl)ethanol (32.88 mg, 252.56 µmol) were dissolved in methanol (2.0 mL), followed by the addition of acetic acid (12.64 mg, 210.46 µmol). Under the protection of nitrogen, the mixture was stirred at 50°C for 0.5 hours. Sodium cyanoborohydride (39.68 mg, 631.39 µmol) was added, allowing for reacting at 50°C for 1.5 hours under the protection of nitrogen. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 20%-50%). The title Compound **464-2** (33.30 mg, 52.29 µmol, yield: 24.84%) was obtained as white solid.

### Single-configuration Compound 464-2:

MS m/z (ESI):637.2 [M+1]
Chiral SFC: retention time: 1.255 min; chromatographic column: Chiralpak OD-3 50×4.6 mm I.D., 3um,; mobile phase: CO₂/MeOH (0.05% DEA): elution gradient: 5%-40%.

¹H NMR (400 MHz, DMSO-*d₆*) δ 8.04 - 7.95 (m, 1H), 7.75 - 7.56 (m, 1H), 7.43 - 7.21 (m, 2H), 7.13 - 6.94 (m, 2H), 6.20 - 5.57 (m, 1H), 4.37 - 4.22 (m, 1H), 3.66 - 3.56 (m, 1H), 3.52 - 3.40 (m, 4H), 3.31 - 2.73 (m, 5H), 2.61 - 2.57 (m, 3H), 2.40 - 2.16 (m, 11H), 2.12 - 2.05 (m, 1H), 1.77 - 1.66 (m, 1H), 1.48 - 1.37 (m, 2H), 1.31 - 1.17 (m, 2H), 1.04 - 0.73 (m, 13H), 0.33 - 0.29 (m, 2H).

### Example 465

### N-ethyl-5-fluoro-2-[1-methyl-7-(1-{2-methyl-6-[(1R,4R)-2-oxa-5-azabicyclo[2.2.1]heptan-5-yl]hexan-3-yl}azacyclobutan-3-yl)imidazo[1,5-a]pyridin-5-yl]-N-(isopropyl)benzamide 465

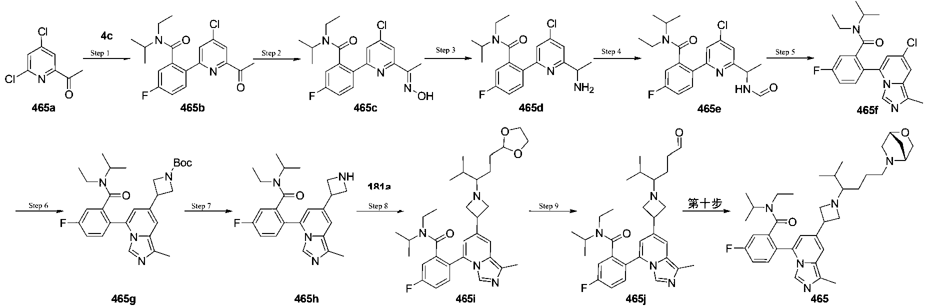

### Step 1

### 2-(6-acetyl-4-chloropyridin-2-yl)-N-ethyl-5-fluoro-N-(isopropyl)benzamide 465b

1-(4,6-Dichloropyridin-2-yl)ethan-1-one **465a** (1 g, 5.26 mmol), Compound 4c(1.76 g, 5.26 mmol) and sodium carbonate (1.67 g, 15.79 mmol) were dissolved in dioxane (10 mL) and water (2.0 mL). Under a nitrogen atmosphere, Pd(dppf)Cl₂ (385.05 mg, 526.24 µmol) was added, and the mixture was stirred at 70°C to allow for reacting for 1.0 hour. The reaction mixture was cooled to room temperature, followed by adding water (250 mL) and extracting with ethyl acetate (100 mL x 3). The organic phases were combined, washed with saturated brine, dried over anhydrous sodium sulfate and filtered, and the filtrate was concentrated under a reduced pressure. The resulting residues were purified by the silica-gel column chromatography using petroleum ether/ethyl acetate as an elution system, to obtain the title Compound **465b** (1.5 g, 4.13 mmol, yield: 78.56%).
MS m/z (ESI): 276.0 [M+1]
¹H NMR (400 MHz, CDCl₃) δ 8.03 - 7.96 (m, 1H), 7.86 - 7.78 (m, 2H), 7.27 - 7.19 (m, 1H), 7.16 - 7.08 (m, 1H), 3.55 (qd, J = 6.7, 13.4 Hz, 2H), 3.07 (dd, J = 6.9, 13.7 Hz, 1H), 2.77 - 2.73 (m, 3H), 1.13 (t, J = 7.1 Hz, 3H), 1.05 - 0.82 (m, 4H), 0.49 (d, J = 6.6 Hz, 2H).

### Step 2

### 2-{4-chloro-6-[1-(hydroxylimino)ethyl]pyridin-2-yl}-N-ethyl-5-fluoro-N-(isopropyl)benzamide 465c

Compound **465b** (500 mg, 1.38 mmol) and hydroxylamine (105.34 mg, 1.52 mmol, hydrochloride) were dissolved in methanol (10 mL). Under a nitrogen atmosphere, potassium carbonate (571.37 mg, 4.13 mmol) was added, and the mixture was stirred at 25°C to allow for reacting for 4.0 hours. The reaction mixture was filtered and washed with methanol, and the filtrate was concentrated under a reduced pressure, to obtain the crude product of title Compound **465c.** The crude product was directly used in the next step of reaction without purification.
MS m/z (ESI): 378.1 [M+1]

### Step 3

### 2-[6-(1-aminoethyl)-4-chloropyridin-2-yl]-N-ethyl-5-fluoro-N-(isopropyl)benzamide 465d

Compound **465c** (520 mg, 1.38 mmol) was dissolved in methanol (10 mL). Ammonium chloride (368.09 mg, 6.88 mmol) was added to the reaction mixture, and under a nitrogen atmosphere, zinc powder (449.96 mg, 6.88 mmol) was added. The mixture was stirred at room temperature to allow for reacting for 4 hour. The reaction mixture was filtered, and the filtrate was concentrated under a reduced pressure. The resulting residues were purified by the silica-gel column chromatography using petroleum ether/ethyl acetate as an elution system, to obtain the crude product of title Compound **465d** (677 mg, 1.86 mmol, yield: 135.2%). The crude product was directly used in the next step of reaction without further purification.
MS m/z (ESI): 363.9 [M+1]

### Step 4

### 2-[4-chloro-6-(1-formamide ethyl)pyridin-2-yl]-N-ethyl-5-fluoro-N-(isopropyl)benzamide 465e

Compound **465d** (677 mg, 1.86 mmol) was dissolved in ethyl formate (6.20 g, 83.73 mmol, 6.73 mL), followed by the addition of triethylamine (564.82 mg, 5.58 mmol) under a nitrogen atmosphere. The mixture was stirred at 60°C to allow for reacting for 12 hours, and the reaction mixture was concentrated under a reduced pressure. The resulting residues were purified by the silica-gel column chromatography using petroleum ether/ethyl acetate as an elution system, to obtain the title Compound **465e** (447 mg, 1.14 mmol, yield: 61.31%) as white solid.
MS m/z (ESI): 391.9 [M+1]

### Step 5

### 2-{7-chloro-1-methylimidazo[1,5-a]pyridin-5-yl}-N-ethyl-5-fluoro-N-(isopropyl)benzamide 465f

Compound **465e** (447 mg, 1.14 mmol) was dissolved in toluene (10 mL). POCl₃ (874.53 mg, 5.70 mmol) was added, and the mixture was stirred at 100°C to allow for reacting for 1.0 hour. The reaction mixture was cooled to room temperature, and concentrated under a reduced pressure. The residues were diluted with dichloromethane (20 mL), and slowly dropwise added to a saturated aqueous sodium bicarbonate solution (20 mL), and the mixture was extracted with dichloromethane (20 mL x 3). The organic phases were combined, dried over anhydrous sodium sulfate and filtered, and the filtrate was concentrated under a reduced pressure. The resulting residues were purified by the silica-gel column chromatography using petroleum ether/ethyl acetate as an elution system, to obtain the title Compound **465f** (360 mg, 962.95 µmol, yield: 84.42%) as yellow solid.
MS m/z (ESI): 374.0 [M+1]

### Step 6

### 3-(5-{2-[ethyl(isopropyl)aminoformyl]-4-fluorophenyl}-1-methylimidazo[1,5-a]pyridin-7-yl)azacyclobutan-1-tert-butyl carboxylate 465g

Compound **465f** (1 g, 2.67 mmol) was dissolved in DMF(20 mL), followed by the addition of Pd₂(dba)₃ (153.81 mg, 267.49 µmol) and RuPhos (124.82 mg, 267.49 µmol). Under a nitrogen atmosphere, {1-[(tert-butoxy)carbonyl]azacyclobutan-3-yl}zinc iodide (932.18 mg, 2.67 mmol) was added. The mixture was stirred at 100°C to allow for reacting for 12 hours. The reaction mixture was cooled to room temperature under a reduced pressure, added to 100 mL of water, and extracted with ethyl acetate (20 mL x 3), and the organic phases were combined, washed with a 5% aqueous lithium chloride solution (50 mL) and saturated brine (50 mL), dried over anhydrous sodium sulfate, and concentrated under a reduced pressure. The resulting residues were purified by the silica-gel column chromatography using petroleum ether/ethyl acetate as an elution system, to obtain the title Compound **465g** (560 mg, 1.13 mmol, yield: 42.33%).
MS m/z (ESI): 495.1 [M+1]
¹H NMR (400 MHz, DMSO-*d₆*) δ 7.95 - 7.71 (m, 2H), 7.50 - 7.39 (m, 3H), 6.48 - 6.38 (m, 1H), 4.25 - 4.12 (m, 2H), 3.85 (br s, 2H), 3.78 - 3.68 (m, 1H), 3.62 - 3.45 (m, 1H), 2.97 - 2.84 (m, 1H), 2.54 (s, 2H), 2.41 (s, 3H), 1.39 (s, 9H), 1.02 - 0.88 (m, 4H), 0.87 - 0.63 (m, 4H).

### Step 7

### 2-[7-(azacyclobutan-3-yl)-1-methylimidazo[1,5-a]pyridin-5-yl]-N-ethyl-5-fluoro-N-(isopropyl)benzamide 465h

Compound **465g** (500 mg, 1.01 mmol) was dissolved in dichloromethane (10 mL), followed by the addition of 2,6-dimethylpyridine (433.29 mg, 4.04 mmol), cooling to 0°C, and the slow dropwise addition of TMSOTf (674.05 mg, 3.03 mmol), and the mixture was stirred at 0°C to allow for reacting for 2 hours. At 0°C, a saturated aqueous ammonium chloride solution (20 mL) was added dropwise to the reaction mixture, which was then extracted with dichloromethane (20 mL x 3). The organic phases were combined, washed with saturated brine, dried over anhydrous sodium sulfate, and concentrated under a reduced pressure, to obtain the title Compound **465h** (398 mg, 1.01 mmol, yield: 99.8%). The crude product was directly used in the next step of reaction without purification.
MS m/z (ESI): 395.1 [M+1]

### Step 8

### 2-(7-{1-[1-(1,3-dioxolan-2-yl)-4-methylpentan-3-yl]azacyclobutan-3-yl}-1-methylimidazo[1,5-a]pyridin-5-yl)-N-ethyl-5-fluoro-N-(isopropyl)benzamide 465i

Compound **465h** (398 mg, 1.01 mmol) and 1-(1,3-dioxolan-2-yl)-4-methylpentan-3-one **181a** (173.76 mg, 1.01 mmol) were dissolved in methanol (10 mL), followed by the addition of ZnCl₂(275.02 mg, 2.02 mmol), and the mixture was stirred at 50°C for 1.0 hour. The mixture was cooled to room temperature, followed by adding sodium cyanoborohydride (190.21 mg, 3.03 mmol) and stirring at 50°C for 11 hour. A saturated aqueous sodium carbonate solution (20 mL) was added to the reaction mixture, which was then extracted with dichloromethane (10 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **465i** (44 mg, 79.90 µmol, yield: 7.92%).
MS m/z (ESI): 551.2 [M+1]

### Step 9

### N-ethyl-5-fluoro-2-{1-methyl-7-[1-(2-methyl-6-oxohexan-3-yl)azacyclobutan-3-yl]imidazo[1,5-a]pyridin-5-yl}-N-(isopropyl)benzamide 465j

Compound **465i** (30 mg, 54.48 µmol) was dissolved in acetonitrile (3.0 mL), followed by the addition of a hydrochloric acid solution (1.0 M; 1.0 mL). The mixture was stirred at 50°C to allow for reacting for 1.0 hours. The reaction mixture was concentrated under a reduced pressure, regulated to pH = 10 with a 10 % aqueous NaOH solution, and extracted with dichloromethane (15 mL x 3). The organic phases were combined, dried over anhydrous sodium sulfate, and concentrated under a reduced pressure, to obtain the crude product of title Compound **465j** (36 mg). The crude product was directly used in the next step of reaction without purification.
MS m/z (ESI): 507.2 [M+1]

### Step 10

### N-ethyl-5-fluoro-2-[1-methyl-7-(1-{2-methyl-6-[(IR,4R)-2-oxa-5-azabicyclo[2.2.1]heptan-5-yl]hexan-3-yl}azacyclobutan-3-yl)imidazo[1,5-a]pyridin-5-yl]-N-(isopropyl)benzamide 465

(1*R*,4*R*)-2-oxa-5-azabicyclo[2.2.1]heptane (49.46 mg, 364.75 µmol, hydrochloride) was dissolved in dichloromethane (5.0 mL), followed by the addition of triethylamine (100.66 mg, 994.76 µmol), and then the addition of Compound **465j** (110 mg, 204.22 µmol). Under the protection of nitrogen, the mixture was stirred at 35°C for 1.0 hours. Sodium cyanoborohydride (210.83 mg, 994.76 µmol) was added, allowing for reacting at 35°C for 11 hours under the protection of nitrogen. A saturated aqueous sodium bicarbonate solution (20 mL) was added to the reaction mixture, which was then extracted with dichloromethane (10 mL x 3). The organic phases were combined, dried over anhydrous sodium sulfate, and concentrated under a reduced pressure. The resulting residues were purified by the preparative thin-layer chromatography using dichloromethane/methanol as an elution system, to obtain the title Compound **465** (59.41 mg, 100.73 µmol, yield: 30.38%) as yellow solid.
MS m/z (ESI): 590.2 [M+1]
¹H NMR (400 MHz, CDCl₃) δ 7.82 (br s, 1H), 7.74 - 7.56 (m, 1H), 7.26 - 7.06 (m, 3H), 6.67 - 6.39 (m, 1H), 4.47 - 4.27 (m, 1H), 4.03 (br d, J = 7.8 Hz, 1H), 3.84 - 3.36 (m, 7H), 3.23 - 3.04 (m, 2H), 2.97 - 2.79 (m, 2H), 2.64 - 2.54 (m, 1H), 2.52 (s, 3H), 2.49 (br s, 1H), 2.14 - 2.02 (m, 1H), 1.98 - 1.69 (m, 5H), 1.51 (br s, 2H), 1.39 - 1.25 (m, 2H), 1.06 - 0.74 (m, 12H), 0.28 - 0.01 (m, 2H).

### Example 466

### N-ethyl-5-fluoro-2-[7-(1-{6-[4-(2-hydroxyethyl)piperazin-1-yl]-2-methylhexan-3-yl}azacyclobutan-3-yl)-1-methylimidazo[1,5-a]pyridin-5-yl]-N-(isopropyl)benzamide 466

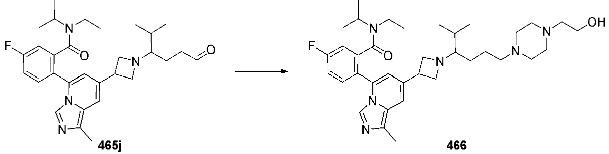

Compound **465j** (36 mg, 71.05 µmol) was dissolved in methanol (2.0 mL), followed by the addition of 2-(piperazin-1-yl)ethanol (10.18 mg, 78.16 µmol) and acetic acid (426.70 µg, 7.11 µmol). Under the protection of nitrogen, the mixture was stirred at 35°C for 1.0 hours. Sodium cyanoborohydride (13.40 mg, 213.16 µmol) was added, allowing for reacting at 35°C for 11 hours under the protection of nitrogen. A saturated aqueous sodium bicarbonate solution (20 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 23%-53%), to obtain the title Compound **466** (12.53 mg, 20.18 µmol, yield: 28.40%) as yellow solid.
MS m/z (ESI): 621.3 [M+1]
¹H NMR (400 MHz, CDCl₃) δ 7.83 (br s, 1H), 7.73 - 7.63 (m, 1H), 7.26 - 7.10 (m, 3H), 6.65 - 6.46 (m, 1H), 3.83 - 3.42 (m, 7H), 3.21 - 3.05 (m, 2H), 2.96 - 2.81 (m, 1H), 2.66 - 2.53 (m, 6H), 2.52 (s, 3H), 2.50 - 2.44 (m, 2H), 2.33 (br t, J = 7.6 Hz, 2H), 2.17 - 2.01 (m, 1H), 1.87 - 1.75 (m, 2H), 1.56 (br d, J = 4.0 Hz, 3H), 1.39 - 1.24 (m, 2H), 1.15 - 0.72 (m, 13H), 0.28 - 0.02 (m, 2H).

### Example 467

### 5-fluoro-N-(2-methoxyethyl)-2-[3-methyl-6-(1-{2-methyl-6-(1R,4R)-2-oxa-5-azabicyclo[2.2.1]heptan-5-yl}hexan-3-yl}azacyclobutan-3-yl)imidazo[1,5-a]pyridin-8-yl]-N-(isopropyl)benzamide 467

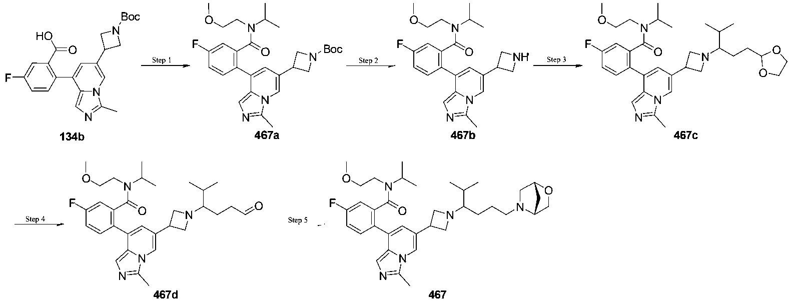

### Step 1

### 3-(8-{4-fluoro-2-[(2-methoxyethyl)(isopropyl)aminoformyl]phenyl}-3-methylimidazo[1,5-a]pyridin-6-yl)azacyclobutan-1-tert-butyl carboxylate 467a

Compound **134b** (250 mg, 587.61 µmol) was dissolved in dichloromethane (4 mL), followed by the addition of DIPEA (151.89 mg, 1.18 mmol) and HATU (268.11 mg, 705.13 µmol), respectively, and finally the addition of *N-*(2-methoxyethyl)prop-2-amine (103.29 mg, 881.42 µmol). Under a nitrogen atmosphere, the mixture was stirred at 25°C to allow for reacting for 2.0 hours. A saturated aqueous sodium bicarbonate solution (15 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **467a** (300 mg, 571.84 µmol, yield: 97.32%) as yellow solid.
MS m/z (ESI): 525.3 [M+1]

### Step 2

### 2-[6-(azacyclobutan-3-yl)-3-methylimidazo[1,5-a]pyridin-8-yl]-5-fluoro-N-(2-methoxyethyl)-N-(isopropyl)benzamide 467b

Compound **467a** (250 mg, 476.53 µmol) was dissolved in dichloromethane (3 mL), followed by the addition of trifluoroacetic acid (1.48 g, 12.98 mmol, 1 mL), and the mixture was stirred at 25°C to allow for reacting for 0.5 hours. The reaction mixture was concentrated under a reduced pressure, the resulting crude product was dissolved with dichloromethane (2 mL) and regulated to PH = 10 with a 1N aqueous NaOH solution, and the mixture was extracted with dichloromethane (20 mL x 3). The organic phases were combined, washed with saturated brine, dried over anhydrous sodium sulfate, and concentrated under a reduced pressure, to obtain the title Compound **467b** (200 mg, 471.13 µmol, yield: 98.87%) as yellow solid. The crude product was directly used in the next step of reaction without purification.
MS m/z (ESI): 425.0 [M+1]

### Step 3

### 2-(6-{1-[1-(1,3-dioxolan-2-yl)-4-methylpentan-3-yl]azacyclobutan-3-yl}-3-methylimidazo[1,5-a]pyridin-8-yl)-5-fluoro-N-(2-methoxyethyl)-N-(isopropyl)benzamide 467c

Compound **467b** (200 mg, 471.13 µmol) and 1-(1,3-dioxolan-2-yl)-4-methylpentan-3-one **181a** (97.37 mg, 565.36 µmol) were dissolved in methanol (2.0 mL), followed by the addition of acetic acid (28.29 mg, 471.13 µmol), and the mixture was stirred at 40°C for 0.5 hours. The mixture was cooled to room temperature, followed by adding sodium cyanoborohydride (88.82 mg, 1.41 mmol) and stirring at 40°C for 12 hour. A saturated aqueous sodium bicarbonate solution (15 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **467c** (150 mg, 258.29 µmol, yield: 54.82%).
MS m/z (ESI): 581.4 [M+1]

### Step 4

### 5-fluoro-N-(2-methoxyethyl)-2-{3-methyl-6-[1-(2-methyl-6-oxohexan-3-yl)azacyclobutan-3-yl]imidazo[1,5-a]pyridin-8-yl}-N-(isopropyl)benzamide 467d

Compound **467c** (100 mg, 172.20 µmol) was dissolved in acetonitrile (1.0 mL), followed by the addition of a hydrochloric acid solution (1.0 M; 1.0 mL). The mixture was stirred at 50°C to allow for reacting for 0.5 hours. The reaction mixture was concentrated under a reduced pressure, regulated to pH = 10 with a 10 % aqueous NaOH solution, and extracted with dichloromethane (10 mL x 3). The organic phases were combined, dried over anhydrous sodium sulfate, and concentrated under a reduced pressure, to obtain the crude product of title Compound **467d** (90 mg, 167.70 µmol, yield: 97.39%). The crude product was directly used in the next step of reaction without purification. MS m/z (ESI): 537.3 [M+1]

### Step 5

### 5-fluoro-N-(2-methoxyethyl)-2-[3-methyl-6-(1-{2-methyl-6-(1R,4R)-2-oxa-5-azabicyclo[2.2.1]heptan-5-yl}hexan-3-yl}azacyclobutan-3-yl)imidazo[1,5-a]pyridin-8-yl]-N-(isopropyl)benzamide 467

(1R,4R)-2-oxa-5-azabicyclo[2.2.1]heptane (27.29 mg, 201.24 µmol, hydrochloride) was dissolved in methanol (2.0 mL), followed by the addition of triethylamine (33.94 mg, 335.40 µmol) and then the addition of Compound **467d** (90 mg, 167.70 µmol). Under the protection of nitrogen, the mixture was stirred at 40°C for 0.5 hours. Sodium cyanoborohydride (31.62 mg, 503.09 µmol) was added, allowing for reacting at 40°C for 1.5 hours under the protection of nitrogen. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% formic acid), elution gradient: 10%), to obtain the title Compound 467 (5.15 mg, 8.31 µmol, yield: 4.95%) as yellow solid.
MS m/z (ESI): 620.4 [M+1]
¹H NMR (400 MHz,DMSO-*d₆*) δ 8.02 - 7.84 (m, 1H), 7.74 - 7.57 (m, 1H), 7.46 - 7.26 (m, 2H), 7.08 - 6.92 (m, 1H), 6.90 - 6.67 (m, 1H), 4.31 - 4.10 (m, 1H), 3.84 - 3.72 (m, 1H), 3.62 - 3.35 (m, 7H), 3.18 - 2.96 (m, 7H), 2.89 - 2.79 (m, 1H), 2.75 - 2.66 (m, 1H), 2.61 - 2.56 (m, 3H), 2.48 - 2.28 (m, 3H), 2.07 - 1.99 (m, 1H), 1.78 - 1.63 (m, 2H), 1.56 - 1.48 (m, 1H), 1.45 - 1.33 (m, 2H), 1.32 - 1.17 (m, 2H), 1.07 - 0.79 (m, 10H), 0.32 - 0.25 (m, 2H).

### Examples 467-1 and 467-2

### 5- fluoro-N-(2-methoxyethyl)-2-(3-methyl-6-{1-[(3R)-2-methyl-6-[(1R,4R)-2-oxa-5-azadicyclo[2.2.1]heptan-5-yl]hexan-3-yl]azacyclobutan-3-yl}imidazo[1,5-a]pyridin-8-yl)-N-(isopropyl)benzamide 467-1

### 5-fluoro-N-(2-methoxyethyl)-2-(3-methyl-6-{1-[(3S)-2-methyl-6-[(1R,4R)-2-oxa-5-azadicyclo[2.2.1]heptan-5-yl]hexan-3-yl]azacyclobutan-3-yl}imidazo[1,5-a]pyridin-8-yl)-N-(isopropyl)benzamide 467-2

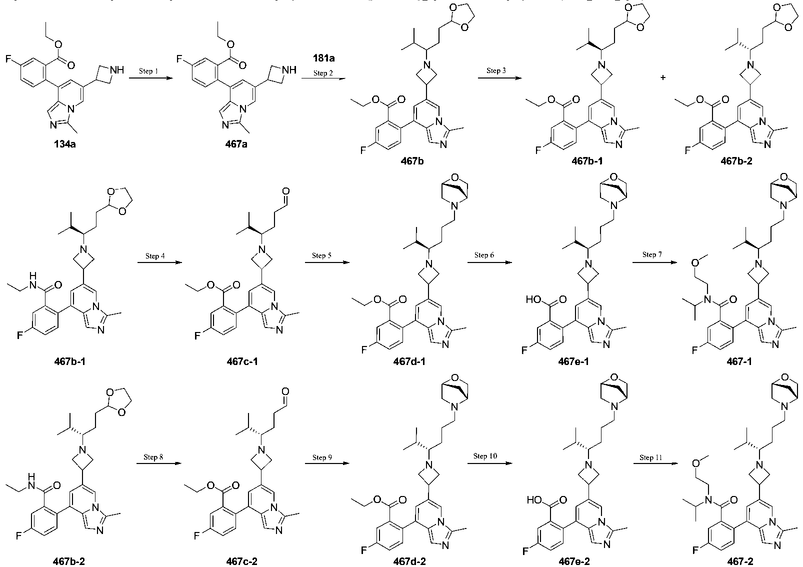

### Step 1

### 2-[6-(azacyclobutan-3-yl)-3-methylimidazo[1,5-a]pyridin-8-yl]-5-ethyl fluorobenzoate 467a

Compound **134a** (2.5 g, 5.51 mmol) was dissolved in methane dioxide (15.0 mL), followed by the addition of trifluoroacetic acid (7.40 g, 64.90 mmol, 5 mL). The mixture was stirred at room temperature to allow for reacting for 2.0 hours. The reaction mixture was concentrated under a reduced pressure, and the resulting crude product was regulated to PH = 10 with a 1M aqueous sodium hydroxide solution and extracted with dichloromethane (20 mL x 3). The organic phases were combined, dried over anhydrous sodium sulfate, and concentrated under a reduced pressure, to obtain the crude product of title Compound **467a** (1.9 g, 5.38 mmol, yield: 97.53%). The crude product was directly used in the next step of reaction without purification.
MS m/z (ESI): 354.2 [M+1]

### Step 2

### 2-(6-{1-[1-(1,3-dioxolan-2-yl)-4-methylpentan-3-yl]azacyclobutan-3-yl}-3-methylimidazo[1,5-a]pyridin-8-yl)-5-ethyl fluorobenzoate 467b

Compound **467a** (1.9 g, 5.38 mmol) and 1-(1,3-dioxolan-2-yl)-4-methylpentan-3-one **181a** (1.11 g, 6.45 mmol) were dissolved in methanol (20.0 mL), followed by the addition of acetic acid (322.87 mg, 5.38 mmol), and the mixture was stirred at 40°C for 0.5 hours. The mixture was cooled to room temperature, followed by adding sodium cyanoborohydride (1.01 g, 16.13 mmol) and stirring at 40°C for 12 hour. A saturated aqueous sodium bicarbonate solution (25 mL) was added to the reaction mixture, which was then extracted with dichloromethane (25 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **467b** (2 g, 3.92 mmol, yield: 72.99%)
MS m/z (ESI): 510.2 [M+1]

### Step 3

### 2-(6-{1-[(3R)-1-(1,3-dioxolan-2-yl)-4-methylpentan-3-yl]azacyclobutan-3-yl}-3-methylimidazo[1,5-a]pyridin-8-yl)-5-ethyl fluorobenzoate 467b-1

### 2-(6-{1-[(3S)-1-(1,3-dioxolan-2-yl)-4-methylpentan-3-yl]azacyclobutan-3-yl}-3-methylimidazo[1,5-a]pyridin-8-yl)-5-ethyl fluorobenzoate 467b-2

Compound **467b** (2 g, 3.92 mmol) was separated and purified by the chiral preparative SFC (preparative column: DAICEL CHIRALCEL OD (250 mm * 30 mm, 10 um), mobile phase: carbon dioxide-isopropanol (0.1% ammonia water); elution gradient: 30%), to obtain the title Compounds **467b-1** (800 mg, 1.57 mmol, yield: 40%) and **467b-2** (850 mg, 1.67 mmol, yield: 42.5%).

### Single-configuration Compound 467b-1:

MS m/z (ESI):510.2 [M+1]
Chiral HPLC analysis: retention time: 1.452 min; chromatographic column: Chiralcel OD-3 50 x 4.6 mm I.D., 3 um; mobile phase: carbon dioxide-isopropanol (0.05% diethylamide ): elution gradient: 5%-40%.

### Single-configuration Compound 467b-2:

MS m/z (ESI):510.3 [M+1]
Chiral HPLC analysis: retention time: 1.597 min; chromatographic column: Chiralcel OD-3 50 x 4.6 mm I.D., 3 um; mobile phase: carbon dioxide-isopropanol (0.05% diethylamide ): elution gradient: 5%-40%.

### Step 4

### 5-fluoro-2-(3-methyl-6-{1-[(3R)-2-methyl-6-oxohexan-3-yl]azacyclobutan-3-yl}imidazo[1,5-a]pyridin-8-yl)ethyl benzoate 467c-1

Compound **467b-1** (600.00 mg, 1.18 mmol) was dissolved in acetonitrile (6.0 mL), followed by the addition of a hydrochloric acid solution (1.0 M; 6.0 mL). The mixture was stirred at 50°C to allow for reacting for 1.0 hours. The reaction mixture was concentrated under a reduced pressure, followed by adding dichloromethane (5 mL) and water (5 mL), regulating to pH = 10 with a10 % aqueous NaOH solution, and extracting with dichloromethane (10 mL x 3). The organic phases were combined, dried over anhydrous sodium sulfate, and concentrated under a reduced pressure, to obtain the crude product of title Compound **467c-1** (540 mg, 1.16 mmol,yield: 98.52%). The crude product was directly used in the next step of reaction without purification.

### Single-configuration Compound 467c-1:

MS m/z (ESI): 466.3 [M+1]

### Step 5

### 5-fluoro-2-(3-methyl-6-{1-[(3R)-2-methyl-6-[(1R,4R)-2-oxa-5-azadicyclo[2.2.1]heptan-5-yl]hexan-3-yl]azacyclobutan-3-yl}imidazo[1,5-a]pyridin-8-yl)ethyl benzoate 467d-1

The crude product of Compound **467c-1** (540 mg, 1.16 mmol) and (1*R*,4*R*)-2-oxa-5-azadicyclo[2.2.1]heptane (188.73 mg, 1.39 mmol, hydrochloride) were dissolved in methanol (10.0 mL), followed by the addition of triethylamine (234.74 mg, 2.32 mmol), and the mixture was stirred at 40°C for 0.5 hours. Sodium cyanoborohydride (737.49 mg, 3.48 mmol) was added, allowing for reacting 40°C for 12.0 hours under the protection of nitrogen. A saturated aqueous sodium bicarbonate solution (20 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **467d-1** (500 mg, 911.26 µmol, yield: 78.56%) as yellow solid.

### Single-configuration Compound 467d-1:

MS m/z (ESI):549.3 [M+1]

### Step 6

### 5-fluoro-2-(3-methyl-6-{1-[(3R)-2-methyl-6-[(1R,4R)-2-oxa-5-azadicyclo[2.2.1]heptan-5-yl]hexan-3-yl]azacyclobutan-3-yl}imidazo[1,5-a]pyridin-8-yl)benzoic acid 467e-1

Compound **467d-1** (70 mg, 127.58 µmol) was dissolved in acetonitrile (1.0 mL), followed by the addition of potassium trimethylsilanolate (24.55 mg, 191.36 µmol). The mixture was stirred at 50°C to allow for reacting for 14 hours. The reaction mixture was concentrated under a reduced pressure, to obtain the crude product of title Compound **467e-1** (65 mg). The crude product was directly used in the next step of reaction without purification.

### Single-configuration Compound 467e-1:

MS m/z (ESI): 521.4 [M+1]

### Step 7

### 5-fluoro-N-(2-methoxyethyl)-2-(3-methyl-6-{1-[(3R)-2-methyl-6-[(1R,4R)-2-oxa-5-azadicyclo[2.2.1]heptan-5-yl]hexan-3-yl]azacyclobutan-3-yl}imidazo[1,5-a]pyridin-8-yl)-N-(isopropyl)benzamide 467-1

The crude product of Compound **467e-1** (65.00 mg, 124.85 µmol) was dissolved in dichloromethane (1.0 mL), followed by the addition of triethylamine (25.27 mg, 249.69 µmol) and HATU(56.96 mg, 149.82 µmol), and the mixture was stirred at room temperature for 5.0 min. *N*- (2-methoxyethyl)prop-2-amine (17.56 mg, 149.82 µmol) was added, allowing for reacting at 25°C for 12.0 hours under the protection of nitrogen. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The crude product was purified by the HPLC (mobile phase: acetonitrile, water (0.1% ammonia water), elution gradient: 29%-59%). The title Compound **467-1** (10.94 mg, 17.65 µmol, yield: 14.14%) was obtained as yellow solid.

### Single-configuration Compound 467-1:

MS m/z (ESI):620.2 [M+1]
Chiral SFC: retention time: 1.156 min; chromatographic column: Chiralcel OD 50×4.6 mm I.D., 3 um; mobile phase: CO₂/MeOH (0.05% DEA): elution gradient: 5%-40%.

¹H NMR (400 MHz, DMSO-*d₆*) δ 8.04 - 7.82 (m, 1H), 7.74 - 7.54 (m, 1H), 7.48 - 7.22 (m, 2H), 7.14 - 6.87 (m, 1H), 6.85 - 6.68 (m, 1H), 4.33 - 4.17 (m, 1H), 3.81 - 3.73 (m, 1H), 3.59 - 3.38 (m, 7H), 3.16 - 2.97 (m, 7H), 2.91 - 2.79 (m, 1H), 2.75 - 2.69 (m, 1H), 2.62 - 2.55 (m, 3H), 2.48 - 2.27 (m, 3H), 2.08 - 1.98 (m, 1H), 1.75 - 1.63 (m, 2H), 1.54 - 1.48 (m, 1H), 1.45 - 1.34 (m, 2H), 1.31 - 1.17 (m, 2H), 1.04 - 0.79 (m, 10H), 0.31 - 0.26 (m, 2H).

### Step 8

### 5-fluoro-2-(3-methyl-6-{1-[(3S)-2-methyl-6-oxohexan-3-yl]azacyclobutan-3-yl}imidazo[1,5-a]pyridin-8-yl)ethyl benzoate 467c-2

Compound **467b-2** (600.00 mg, 1.18 mmol) was dissolved in acetonitrile (6.0 mL), followed by the addition of a hydrochloric acid solution (1.0 M; 6.0 mL). The mixture was stirred at 50°C to allow for reacting for 1.0 hours. The reaction mixture was concentrated under a reduced pressure, followed by adding dichloromethane (5 mL) and water (5 mL), regulating to pH = 10 with a10 % aqueous NaOH solution, and extracting with dichloromethane (10 mL x 3). The organic phases were combined, dried over anhydrous sodium sulfate, and concentrated under a reduced pressure, to obtain the crude product of title Compound **467c-2** (540 mg, 1.16 mmol,yield: 98.52%). The crude product was directly used in the next step of reaction without purification.

### Single-configuration Compound 467c-2:

MS m/z (ESI): 466.2 [M+1]

### Step 9

### 5-fluoro-2-(3-methyl-6-{1-[(3S)-2-methyl-6-[(1R,4R)-2-oxa-5-azadicyclo[2.2.1]heptan-5-yl]hexan-3-yl]azacyclobutan-3-yl}imidazo[1,5-a]pyridin-8-yl)ethyl benzoate 467d-2

The crude product of Compound **467c-2** (540 mg, 1.16 mmol) and (1*R*,4*R*)-2-oxa-5-azadicyclo[2.2.1]heptane (188.73 mg, 1.39 mmol, hydrochloride) were dissolved in methanol (10.0 mL), followed by the addition of triethylamine (234.74 mg, 2.32 mmol), and the mixture was stirred at 30°C for 1.0 hours. Sodium cyanoborohydride (737.49 mg, 3.48 mmol) was added, allowing for reacting 30°C for 11.0 hours under the protection of nitrogen. A saturated aqueous sodium bicarbonate solution (20 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **467d-2** (500 mg, 911.26 µmol, yield: 78.56%) as yellow solid.

### Single-configuration Compound 467d-2:

MS m/z (ESI):549.4 [M+1]

### Step 10

### 5-fluoro-2-(3-methyl-6-{1-[(3S)-2-methyl-6-[(1R,4R)-2-oxa-5-azadicyclo[2.2.1]heptan-5-yl]hexan-3-yl]azacyclobutan-3-yl}imidazo[1,5-a]pyridin-8-yl)benzoic acid 467e-2

Compound **467d-2** (100.00 mg, 182.25 µmol) was dissolved in acetonitrile (2.0 mL), followed by the addition of potassium trimethylsilanolate (35.07 mg, 273.38 µmol). The mixture was stirred at 50°C to allow for reacting for 12 hours. The reaction mixture was concentrated under a reduced pressure, to obtain the crude product of title Compound **467e-2** (90 mg). The crude product was directly used in the next step of reaction without purification.

### Single-configuration Compound 467e-2:

MS m/z (ESI): 521.4 [M+1]

### Step 11

### 5-fluoro-N-(2-methoxyethyl)-2-(3-methyl-6-{1-[(3S)-2-methyl-6-[(1R,4R)-2-oxa-5-azadicyclo[2.2.1]heptan-5-yl]hexan-3-yl]azacyclobutan-3-yl}imidazo[1,5-a]pyridin-8-yl)-N-(isopropyl)benzamide 467-2

The crude product of Compound **467e-2** (90.00 mg, 172.87 µmol) was dissolved in dichloromethane (3.0 mL), followed by the addition of triethylamine (52.48 mg, 518.60 µmol) and HATU(78.87 mg, 207.44 µmol), and the mixture was stirred at room temperature for 5.0 min. *N*- (2-methoxyethyl)prop-2-amine (24.31 mg, 207.44 µmol) was added, allowing for reacting at 35°C for 12.0 hours under the protection of nitrogen. The reaction mixture was concentrated under a reduced pressure. The crude product was purified by the HPLC (mobile phase: acetonitrile, water (0.1% ammonia water), elution gradient: 28%-58%). The title Compound **467-2** (41.88 mg, 67.57 µmol, yield: 39.09%) was obtained as yellow solid.

### Single-configuration Compound 467-2:

MS m/z (ESI):620.3 [M+1]
Chiral SFC: retention time: 1.161 min; chromatographic column: Chiralcel OD 50×4.6 mm I.D., 3 um; mobile phase: CO₂/MeOH (0.05% DEA): elution gradient: 5%-40%.

¹H NMR (400 MHz, DMSO-*d₆*) δ 7.98 - 7.89 (m, 1H), 7.71 - 7.61 (m, 1H), 7.45 - 7.28 (m, 2H), 7.05 - 6.98 (m, 1H), 6.86 - 6.68 (m, 1H), 4.28 (s, 1H), 3.84 - 3.75 (m, 1H), 3.62 - 3.35 (m, 7H), 3.18 - 2.97 (m, 7H), 2.90 - 2.78 (m, 1H), 2.73 (d, J = 9.6 Hz, 1H), 2.59 (s, 3H), 2.49 - 2.30 (m, 3H), 2.03 (d, J = 2.0 Hz, 1H), 1.78 - 1.63 (m, 2H), 1.51 (d, J = 9.6 Hz, 1H), 1.45 - 1.33 (m, 2H), 1.32 - 1.17 (m, 2H), 1.05 - 0.81 (m, 10H), 0.29 (d, J = 4.8 Hz, 2H).

### Example 469

### N-ethyl-5-fluoro-2-[6-(1-{1-[(3R)-4-(2-hydroxyethyl)morpholin-3-yl]-4-methylpentan-3-yl}azacyclobutan-3-yl)-3-methylimidazo[1,5-a]pyridin-8-yl]-N-(isopropyl)benzamide 469

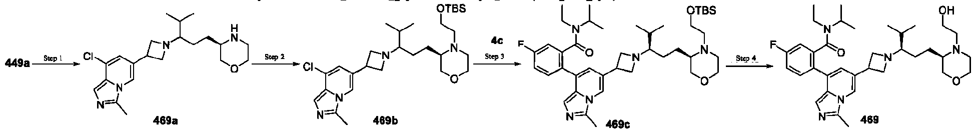

### Step 1

### (3R)-3-[3-(3-{8-chloro-3-methylimidazo[1,5-a]pyridin-6-yl}azacyclobutan-1-yl)-4-methylpentyl]morpholine 469a

Compound **449a** (350.00 mg, 712.74 µmol) was dissolved in dichloromethane (3.0 mL), followed by the addition of trifluoroacetic acid (1 mL). The mixture was stirred at room temperature to allow for reacting for 1.0 hour. The reaction mixture was concentrated under a reduced pressure, followed by the addition of dichloromethane (6 mL), and the reaction mixture was regulated to PH = 8 with triethylamine, and concentrated under a reduced pressure, to obtain the crude product of title Compound **469a** (300 mg, 767.36 µmol, yield: 107.66%). The crude product was directly used in the next step of reaction without purification.
MS m/z (ESI): 391.1 [M+1]

### Step 2

### (3R)-4-{2-[(tert-butyldimethylsilyl)oxy]ethyl}-3-[3-(3-{8-chloro-3-methylimidazo[1,5-a]pyridin-6-yl}azacyclobutan-1-yl)-4-methylpentyl]morpholine 469b

Compound **469a** (300.00 mg, 767.36 µmol) and 2-[(tert-butyldimethylsilicyl)oxy]acetaldehyde (267.52 mg, 1.53 mmol) were dissolved in methanol (5.0 mL), and the mixture was stirred at 25°C for 1.0 hour. Sodium cyanoborohydride (245.72 mg, 3.84 mmol) was added, and the mixture was stirred at 25°C for 2.0 hours. 2-[(Tert-butyldimethylsilicyl)oxy]acetaldehyde (267.52 mg, 1.53 mmol) was supplemented, and the mixture was further stirred at 25°C for 2.0 hours. A saturated aqueous potassium carbonate solution (15 mL) was added to the reaction mixture, which was then extracted with dichloromethane (10 mL x 3). The organic phases were combined, dried over anhydrous sodium sulfate, and concentrated under a reduced pressure. The resulting residues were purified by the preparative thin-layer chromatography using dichloromethane/methanol as an elution system, to obtain the title Compound **469b** (281 mg, 511.60 µmol, yield: 66.67%).
MS m/z (ESI): 549.3 [M+1]

### Step 3

### 2-[6-(1-{1-[(3R)-4-{2-[(tert-butyldimethylsilyl)oxy]ethyl}morpholin-3-yl]-4-methylpentan-3-yl}azacyclobutan-3-yl)-3-methylimidazo[1,5-a]pyridin-8yl]-N-ethyl-5-fluoro-N-(isopropyl)benzamide 469c

Compound **469b** (280 mg, 509.78 µmol), Compound **4c**(256.33 mg, 764.66 µmol) and potassium phosphate (324.62 mg, 1.53 mmol) were dissolved in dioxane (4 mL) and water (1 mL). Xphos Pd G4 (43.86 mg, 50.98 µmol) was added. Under a nitrogen atmosphere, the mixture was stirred at 95°Cto allow for reacting for 3.0 hours. The reaction mixture was cooled to room temperature, and concentrated under a reduced pressure. The resulting residues were purified by the preparative thin-layer chromatography using dichloromethane/methanol as an elution system, to obtain the title Compound **469c** (273 mg, 378.08 µmol, yield: 74.17%).
MS m/z (ESI): 722.5 [M+1]

### Step 4

### N-ethyl-5-fluoro-2-[6-(1-{1-[(3R)-4-(2-hydroxyethyl)morpholin-3-yl]-4-methylpentan-3-yl}azacyclobutan-3-yl)-3-methylimidazo[1,5-a]pyridin-8-yl]-N-(isopropyl)benzamide 469

Compound **469c** (260.00 mg, 360.08 µmol) was dissolved in dichloromethane (0.5 mL), followed by the addition of trifluoroacetic acid (4 mL). The mixture was stirred at room temperature to allow for reacting for 1.0 hour. The reaction mixture was concentrated under a reduced pressure, followed by adding dichloromethane (5 mL), regulating to PH =8 with triethylamine, and concentrating under a reduced pressure. The resulting residues were purified by the HPLC (mobile phase: acetonitrile, water (0.1%ammonium bicarbonate), elution gradient: 28%-58%) two times, to obtain the title Compound **469** (76.1 mg, 125.21 µmol) as yellow solid.
MS m/z (ESI): 608.3 [M+1]
¹H NMR (400 MHz, DMSO-*d₆*) δ 7.93 (s, 1 H), 7.64 - 7.73 (m, 1 H), 7.28 - 7.42 (m, 2 H), 7.00 - 7.07 (m, 1 H), 6.75 (br d, J=12.40 Hz, 1 H), 4.32 - 4.44 (m, 1 H), 3.48 - 3.65 (m, 5 H), 3.41 - 3.48 (m, 4 H), 3.21 - 3.32 (m, 2 H), 3.10 - 3.17 (m, 1 H), 3.06 (br t, J=6.00 Hz, 1 H), 2.86 - 2.99 (m, 1 H), 2.66 - 2.75 (m, 2 H), 2.59 (s, 3 H), 2.19 - 2.31 (m, 3 H), 1.99 (br s, 1 H), 1.66 - 1.77 (m, 1 H), 1.34 - 1.57 (m, 2 H), 1.33 (br s, 3 H), 0.86 - 0.93 (m, 4 H), 0.81 - 0.85 (m, 5 H), 0.76 - 0.80 (m, 2 H), 0.73 (br t, J=7.20 Hz, 1 H), 0.28 (br d, J=6.40 Hz, 2 H).

### Example 470

### N-ethyl-5-fluoro-2-[6-({1-[(3R)-6-[4-(2-hydroxylethyl)piperazin-1-yl]-2-methylhexan-3-yl]azacyclobutan-3-yl}methyl)-4-methylpyrrolo[1,2-a]pyrazin-8-yl]-N-(isopropyl)benzamide 470

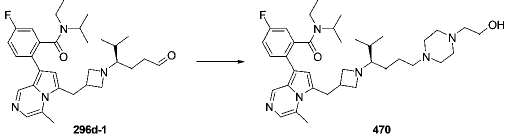

Compound **296d-1** (90 mg, 172.85 µmol) and 2-(piperazin-1-yl)ethanol (45.01 mg, 345.70 µmol) were dissolved in methanol (2 mL), followed by the addition of acetic acid (15.57 mg, 259.28 µmol), and the mixture was stirred at room temperature for 0.5 hours under the protection of nitrogen. Sodium cyanoborohydride (27.16 mg, 432.13 µmol) was added, allowing for reacting at room temperature for 12.0 hours under the protection of nitrogen. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the preparative thin-layer chromatography using dichloromethane/methanol as an elution system, and the obtained crude product was purified by the HPLC (mobile phase: acetonitrile, water (0.1% ammonium bicarbonate), elution gradient: 28%-58%). The title Compound **470** (46.51 mg, 73.26 µmol, yield: 42.38%) was obtained.
MS m/z (ESI):635.4 [M+1]
Chiral SFC: retention time: 1.374 min; chromatographic column: Chiralcel OD-3 50×4.6 mm I.D., 3um,; mobile phase: CO₂/MeOH (0.05% DEA): elution gradient: 5%-40%.

¹H NMR (400 MHz, DMSO-*d₆*) δ 8.54 (s, 1H), 7.57 - 7.49 (m, 1H), 7.36 - 7.17 (m, 3H), 6.65 - 6.56 (m, 1H), 4.36 - 4.28 (m, 1H), 3.55 - 3.34 (m, 7H), 3.26 - 2.95 (m, 2H), 2.83 - 2.74 (m, 5H), 2.69 - 2.60 (m, 1H), 2.41 - 2.24 (m, 9H), 2.22 - 2.14 (m, 2H), 1.97 - 1.91 (m, 1H), 1.74 - 1.61 (m, 1H), 1.44 - 1.32 (m, 2H), 1.28 - 1.04 (m, 3H), 0.95 - 0.68 (m, 13H), 0.31 - 0.20 (m, 2H).

### Example 474

### N-ethyl-5-fluoro-2-(6-{1-[(3R)-6-{4-[3-(hydroxymethyl)oxacyclobutan-3-yl]piperazin-1-yl}-2-methylhexan-3-yl]azacyclobutan-3yl}-3-methylimidazo[1,5-a]pyridin-8-yl)-N-(isopropyl)benzamide 474

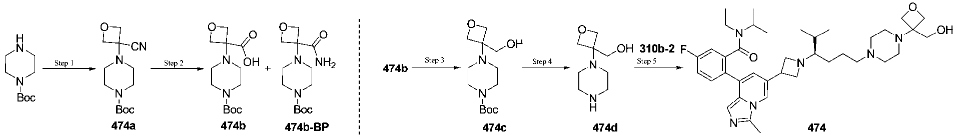

### Step 1

### 4-(3-cyanooxacyclobutan-3-yl)piperazin-1-tert-butyl carboxylate 474a

Piperazin-1-tert-butyl carboxylate (3 g, 16.11 mmol) was dissolved in dichloroethane (40 mL), followed by the addition of oxacyclobutan-3-one (1.74 g, 24.16 mmol) and acetic acid (1.16 g, 19.33 mmol). The reaction mixture was stirred at 50°C for 1.0 hour, followed by the slow dropwise addition of TMSCN (3.08 g, 31.05 mmol). The reaction mixture was stirred at 25°C for 12 hours. The reaction mixture was quenched by adding a saturated aqueous sodium bicarbonate solution (30 mL), and extracted with dichloromethane (20 mL × 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **474a** (2.4 g, 8.98 mmol, yield: 55.74%).
MS m/z (ESI): 212.3 [M-56]
¹H NMR (400 MHz, CDCl₃) δ 4.81 (d, J = 6.4 Hz, 2H), 4.67 (d, J = 6.4 Hz, 2H), 3.53 (br s, 4H), 2.38 (br t, J = 4.7 Hz, 4H), 1.47 (s, 9H).

### Step 2

### 3-{4-[(tert-butoxy)carbonyl]piperazin-1-yl}oxacyclobutan-3-carboxylic acid 474b

### 4-(3-aminoformyloxacyclobutan-3-yl)piperazin-1-tert-butyl carboxylate 474b-BP

Compound **474a** (1.5 g, 5.61 mmol) was dissolved in methanol (10.0 mL), followed by the dropwise addition of aqueous sodium hydroxide solution (2.0 M, 14.03 mL) under the protection of nitrogen. After the completion of dropwise addition, the mixture was stirred at 50°C for 12 hours. The reaction mixture was concentrated under a reduced pressure to remove methanol, diluted with water (10 mL) diluted, regulated to the PH value of 2-3 with 1M HCl, and then extracted with dichloromethane (15 mL×3), and the organic phases were combined, dried over anhydrous sodium sulfate, filtered and concentrated under a reduced pressure, to obtain the title Compound **474b-BP** (950 mg, 3.33 mmol, yield: 59.33%). After extraction, the aqueous phase was regulated to the PH value of 4-5, and further extracted with dichloromethane (15 mL×3), and the organic phases were combined, dried over anhydrous sodium sulfate, filtered and concentrated under a reduced pressure, to obtain the title Compound **474b** (210 mg, 733.44 µmol, yield: 13.07%).

### Compound 474b-BP:

MS m/z (ESI): 286.2 [M+1]

### Compound 474b:

MS m/z (ESI): 231.1 [M-56]
¹H NMR (400 MHz, DMSO-*d₆*) δ 13.94 - 12.04 (m, 1H), 4.59 (d, J = 6.4 Hz, 2H), 4.44 (d, J = 6.4 Hz, 2H), 3.36 - 3.34 (m, 5H), 2.38 (br t, J = 4.8 Hz, 4H), 1.39 (s, 9H).

### Step 3

### 4-[3-(hydroxymethyl)oxacyclobutan-3-yl]piperazin-1-tert-butyl carboxylate 474c

Compound **474b** (210 mg, 733.44 µmol) was dissolved in tetrahydrofuran (5.0 mL), and cooled to 0°C, followed by the slow dropwise addition of an LiAlH₄ solution (2.5 M, 440.06 µL), and the mixture was stirred at for 1.0 hour at 0°C. Under the protection of nitrogen, the reaction mixture was quenched by adding sodium sulfate decahydrate, diluted with ethyl acetate and then filtered. The filtrate was concentrated under a reduced pressure, to obtain the title Compound **474c** (100 mg, 367.19 µmol, yield: 50.06%) as colorless oil. The crude product was directly used in the next step of reaction without purification.
MS m/z (ESI): 273.2 [M+1]
¹H NMR (400 MHz, CDCl₃) δ 4.81 - 4.08 (m, 6H), 3.96 (br s, 1H), 3.51 - 3.39 (m, 4H), 2.51 (br dd, J = 3.8, 8.8 Hz, 4H), 1.47 (s, 9H).

### Step 4

### [3-(piperazin-1-yl)oxacyclobutan-3-yl]methanol 474d

Compound **474c** (100 mg, 367.19 µmol) was dissolved in dichloromethane (1.5 mL), followed by the addition of trifluoroacetic acid (0.5 mL). The mixture was stirred at 25°C to allow for reacting for 1.0 hours. The reaction mixture was concentrated under a reduced pressure, to obtain the crude product of title Compound **474d** (100 mg). The crude product was directly used in the next step of reaction without purification.
MS m/z (ESI): 173.2 [M+1]

### Step 5

### N-ethyl-5-fluoro-2-(6-{1-[(3R)-6-{4-[3-(hydroxymethyl)oxacyclobutan-3-yl]piperazin-1-yl}-2-methylhexan-3-yl]azacyclobutan-3yl}-3-methylimidazo[1,5-a]pyridin-8-yl)-N-(isopropyl)benzamide 474

Compound **310b-2** (120 mg, 236.85 µmol) was dissolved in dichloromethane (5.0 mL), followed by the addition of triethylamine (47.93 mg, 473.70 µmol) and the crude product of Compound **474d** (100 mg). Under the protection of nitrogen, the mixture was stirred at 25°C for 0.5 hours. Sodium cyanoborohydride (125.49 mg, 592.12 µmol) was added, allowing for reacting at 25°C for 12 hours under the protection of nitrogen. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 28%-48%), to obtain the title Compound **474** (55.49 mg, 83.71 µmol, yield: 35.34%) as white solid. MS m/z (ESI): 663.4 [M+1]
¹H NMR (400 MHz,DMSO-*d₆*) δ 7.92 (s, 1H), 7.73 - 7.63 (m, 1H), 7.43 - 7.27 (m, 2H), 7.07 - 6.99 (m, 1H), 6.88 - 6.71 (m, 1H), 4.84 - 4.79 (m, 1H), 4.31 - 4.24 (m, 4H), 3.72 - 3.65 (m, 2H), 3.60 - 3.40 (m, 4H), 3.31 - 2.87 (m, 4H), 2.59 (s, 3H), 2.49 - 2.18 (m, 10H), 2.05 - 1.99 (m, 1H), 1.77 - 1.65 (m, 1H), 1.51 - 1.37 (m, 2H), 1.30 - 0.96 (m, 3H), 0.91 - 0.71 (m, 12H), 0.29 - 0.25 (m, 2H).

### Example 475

### 3-{4-[(4R)-4-[3-(8-{2-[ethyl(isopropyl)aminoformyl]-4-fluorophenyl}-3-methylimidazo[1,5-a]pyridin-6-yl)azacyclobutan-1-yl]-5-methylhexyl]piperazin-1-yl}oxacyclobutan-3-formamide 475

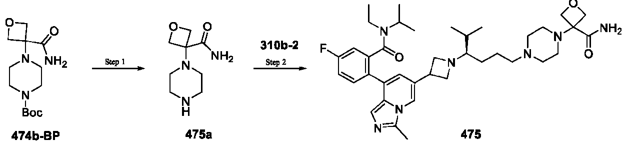

### Step 1

### 3-(piperazin-1-yl) oxacyclobutan-3-formamide 475a

Compound **474b-BP** (110 mg, 385.51 µmol) was dissolved in dichloromethane (1.5 mL), followed by the addition of trifluoroacetic acid (0.5 mL). The mixture was stirred at 25°C to allow for reacting for 1.0 hours. The reaction mixture was concentrated under a reduced pressure, to obtain the crude product of title Compound **475a** (100 mg). The crude product was directly used in the next step of reaction without purification.
MS m/z (ESI): 186.2 [M+1]

### Step 2

### 3-{4-[(4R)-4-[3-(8-{2-[ethyl(isopropyl)aminoformyl]-4-fluorophenyl}-3-methylimidazo[1,5-a]pyridin-6-yl)azacyclobutan-1-yl]-5-methylhexyl]piperazin-1-yl}oxacyclobutan-3-formamide 475

Compound **310b-2** (120 mg, 236.85 µmol) was dissolved in dichloromethane (5.0 mL), followed by the addition of triethylamine (47.93 mg, 473.70 µmol) and the crude product of Compound **475a** (100 mg). Under the protection of nitrogen, the mixture was stirred at 25°C for 0.5 hours. Sodium cyanoborohydride (125.49 mg, 592.12 µmol) was added, allowing for reacting at 25°C for 12 hours under the protection of nitrogen. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 28%-48%), to obtain the title Compound **475** (66.25 mg, 98.02 µmol, yield: 41.39%) as white solid. MS m/z (ESI): 676.4 [M+1]
¹H NMR (400 MHz,DMSO-*d₆*) δ 7.92 (s, 1H), 7.73 - 7.63 (m, 1H), 7.42 - 7.20 (m, 4H), 7.07 - 7.00 (m, 1H), 6.87 - 6.71 (m, 1H), 4.55 (d, J = 6.8 Hz, 2H), 4.42 (d, J = 6.8 Hz, 2H), 3.62 - 3.40 (m, 4H), 3.32 - 2.88 (m, 4H), 2.59 (s, 3H), 2.47 - 2.16 (m, 10H), 2.05 - 2.00 (m, 1H), 1.76 - 1.66 (m, 1H), 1.51 - 1.38 (m, 2H), 1.30 - 0.96 (m, 3H), 0.91 - 0.71 (m, 12H), 0.29 - 0.26 (m, 2H).

### Example 476

### 3-{4-[(4R)-4-[3-(8-{2-[ethyl(isopropyl)aminoformyl]-4-fluorophenyl}-3-methylimidazo[1,5-a]pyridin-6-yl)azacyclobutan-1-yl]-5-methylhexyl]piperazin-1-yl}-N,N-dimethyloxacyclobutan-3-formamide 476

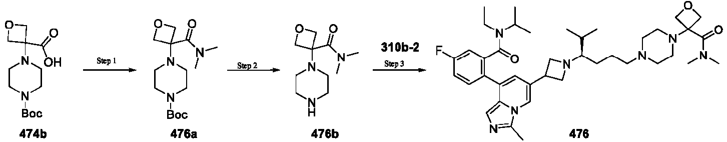

### Step 1

### 4-[3-(dimethylaminoformyl)oxacyclobutan-3-yl]piperazin-1-tert-butyl carboxylate 476a

Compound **474b** (160 mg, 558.81 µmol) was dissolved in dichloromethane (3.0 mL), followed by the addition of*N-*methylmethylamine (68.35 mg, 838.21 µmol, hydrochloride), triethylamine (169.64 mg, 1.68 mmol) and a T4P condensing agent (671.05 mg, 838.21 µmol, 45% purity), and the mixture was stirred at 25°C for 2.0 hours. The reaction mixture was quenched by adding water (10 mL) and extracted with dichloromethane (10 mL×3), and the organic phases were combined, dried over anhydrous sodium sulfate, filtered and concentrated under a reduced pressure. The resulting residues were purified by the silica-gel column chromatography using petroleum ether/ethyl acetate as an elution system, to obtain the title Compound **476a** (100 mg, 319.09 µmol, yield: 57.1%) as colorless oil. MS m/z (ESI): 314.2 [M+1]
¹H NMR (400 MHz, CDCl₃) δ 4.98 (d, J = 7.6 Hz, 2H), 4.77 - 4.72 (m, 2H), 3.49 - 3.45 (m, 4H), 2.96 (d, J = 15.8 Hz, 6H), 2.67 (br d, J = 4.3 Hz, 4H), 1.47 (s, 9H).

### Step 2

### N,N-dimethyl-3-(piperazin-1-yl)oxacyclobutan-3-formamide 476b

Compound **476a** (100 mg, 319.09 µmol) was dissolved in dichloromethane (1.5 mL), followed by the addition of trifluoroacetic acid (0.5 mL). The mixture was stirred at 25°C to allow for reacting for 1.0 hours. The reaction mixture was concentrated under a reduced pressure, to obtain the crude product of title Compound **476b** (70 mg). The crude product was directly used in the next step of reaction without purification.
MS m/z (ESI): 214.2 [M+1]

### Step 3

### 3-{4-[(4R)-4-[3-(8-{2-[ethyl(isopropyl)aminoformyl]-4-fluorophenyl}-3-methylimidazo[1,5-a]pyridin-6-yl)azacyclobutan-1-yl]-5-methylhexyl]piperazin-1-yl}-N,N-dimethyloxacyclobutan-3-formamide 476

Compound **310b-2** (100 mg, 197.37 µmol) was dissolved in dichloromethane (5.0 mL), followed by the addition of triethylamine (39.94 mg, 394.75 µmol) and the crude product of Compound **476b** (70 mg). Under the protection of nitrogen, the mixture was stirred at 25°C for 0.5 hours. Sodium cyanoborohydride (104.58 mg, 493.43 µmol) was added, allowing for reacting at 25°C for 12 hours under the protection of nitrogen. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0. 1% ammonia water), elution gradient: 36%-56%), to obtain the title Compound **476** (64.78 mg, 92.03 µmol, yield: 46.63%) as white solid. MS m/z (ESI): 704.3 [M+1]
¹H NMR (400 MHz,DMSO-*d₆*) δ 7.93 (s, 1H), 7.73 - 7.61 (m, 1H), 7.43 - 7.26 (m, 2H), 7.08 - 6.99 (m, 1H), 6.87 - 6.71 (m, 1H), 4.71 (s, 4H), 3.61 - 3.42 (m, 4H), 3.31 - 2.88 (m, 4H), 2.83 (s, 6H), 2.65 - 2.52 (m, 7H), 2.45 - 2.19 (m, 6H), 2.07 - 1.99 (m, 1H), 1.78 - 1.66 (m, 1H), 1.53 - 1.39 (m, 2H), 1.31 - 0.95 (m, 3H), 0.92 - 0.70 (m, 12H), 0.31 - 0.24 (m, 2H).

### Examples 477-1 and 477-2

### 5-fluoro-2-[6-({1-[(3R)-6-[4-(2-hydroxylethyl)piperazin-1-yl]-2-methylhexan-3-yl]azacyclobutan-3-yl}methyl)-4-methylpyrrolo[1,2-a]pyrazin-8-yl]-N-methyl-N-(isopropyl)benzamide 477-1

### 5-fluoro-2-[6-({1-[(3S)-6-[4-(2-hydroxylethyl)piperazin-1-yl]-2-methylhexan-3-yl]azacyclobutan-3-yl}methyl)-4-methylpyrrolo[1,2-a]pyrazin-8-yl]-N-methyl-N-(isopropyl)benzamide 477-2

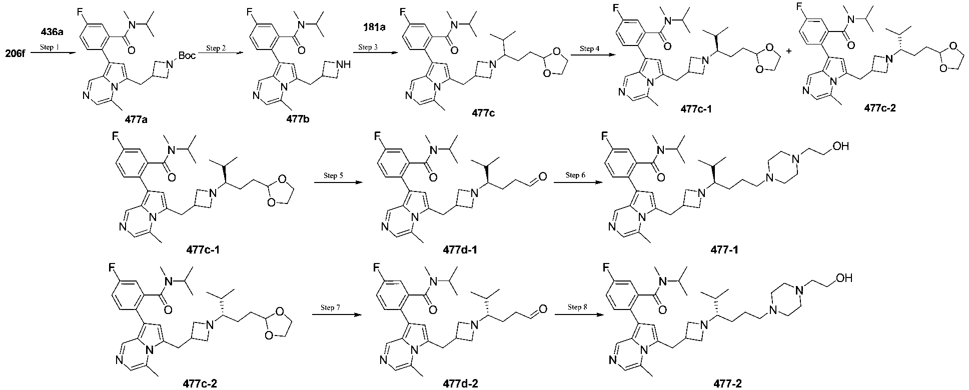

### Step 1

### 3-[(8-{4-fluoro-2-[methyl(isopropyl)aminoformyl]phenyl}-4-methylpyrrolo[1,2-a]pyrazin-6-yl)methyl)azacyclobutan-1-tert-butyl carboxylate 477a

Compound **206f** (1.2 g, 3.16 mmol), Compound **436a** (1.22 g, 3.79 mmol) and potassium phosphate (2.01 g, 9.47 mmol) were dissolved in dioxane (20 mL) and water (4.0 mL). Xphos Pd G4 (135.76 mg, 157.78 µmol) was added. Under a nitrogen atmosphere, the mixture was stirred at 95°Cto allow for reacting for 2.0 hours. The reaction mixture was cooled to room temperature, followed by adding water (30 mL) and extracting with ethyl acetate (30 mL x 3). The organic phases were combined, dried over anhydrous sodium sulfate, and concentrated under a reduced pressure.

The resulting residues were purified by the silica-gel column chromatography using dichloromethane/methanol as an elution system, to obtain the title Compound **477a** (1.5 g, 3.03 mmol, yield: 96.11%) as yellow solid. MS m/z (ESI): 495.5 [M+1]

### Step 2

### 2-{6-[(azacyclobutan-3-yl)methyl]-4-methylpyrrolo[1,2-a]pyrazin-8-yl} -5-fluoro-N-methyl-N-(isopropyl)benzamide 477b

Compound **477a** (1.5 g, 3.03 mmol) was dissolved in methane dioxide (15 mL), followed by the addition of trifluoroacetic acid (4.44 g, 38.94 mmol, 3 mL) at 0°C. The mixture was stirred at room temperature to allow for reacting for 2.0 hours. The reaction mixture was concentrated under a reduced pressure, dissolved by adding DCM (10.0 mL), regulated to PH = 10 by adding a 10% aqueous sodium hydroxide solution, and extracted with dichloromethane (30 mL x 3). The organic phases were combined, dried over anhydrous sodium sulfate and filtered, and the filtrate was concentrated under a reduced pressure, to obtain the crude product of title Compound **477b** (1.2 g, 3.04 mmol, yield: 100.3%). The crude product was directly used in the next step of reaction without purification. MS m/z (ESI): 395.2 [M+1]

### Step 3

### 2-[6-({1-[1-(1,3-dioxolan-2-yl)-4-methylpentan-3-yl]azacyclobutan-3-yl}methyl)-4-methylpyrrolo[1,2-a]pyrazin-8-yl] -5-fluoro-N-methyl-N-(isopropyl)benzamide 477c

Compound **477b** (1.2 g, 3.04 mmol) and Compound **181a** (628.66 mg, 3.65 mmol) were dissolved in methanol (20.0 mL). Acetic acid (182.68 mg, 3.04 mmol) was added, and the mixture was stirred at 40°C for 0.5 hours. The mixture was cooled to room temperature, followed by adding sodium cyanoborohydride (573.49 mg, 9.13 mmol) and stirring at 40°C for 12.0 hour. A saturated aqueous sodium bicarbonate solution (25 mL) was added, and the mixture was then extracted with dichloromethane (25 mL × 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **477c** (800 mg, 1.45 mmol, yield: 47.75%) as yellow oil.
MS m/z (ESI): 551.5[M+1]

### Step 4

### 2-[6-({1-[(3R)-1-(1,3-dioxolan-2-yl)-4-methylpentan-3-yl]azacyclobutan-3-yl}methyl)-4-methylpyrrolo[1,2-a]pyrazin-8-yl]-5-fluoro-N-methyl-N-(isopropyl)benzamide 477c-1

### 2-[6-({1-[(3S)-1-(1,3-dioxolan-2-yl)-4-methylpentan-3-yl]azacyclobutan-3-yl}methyl)-4-methylpyrrolo[1,2-a]pyrazin-8-yl]-5-fluoro-N-methyl-N-(isopropyl)benzamide 477c-2

Compound **477c** (180 mg, 326.85 µmol) was separated and purified by the chiral preparative SFC (preparative column: DAICEL CHIRALCEL OD (250 mm * 30 mm,10 um), mobile phase: CO₂-MeOH (0.1% ammonia water); elution gradient: 25%), to obtain the title Compounds **477c-1** (70 mg, 127.11 µmol, yield: 38.89%) and **477c-2** (80 mg, 145.27 µmol, yield: 44.44%).

### Single-configuration Compound 477c-1:

MS m/z (ESI):551.3 [M+1]
Chiral HPLC analysis: retention time: 1.166 min; chromatographic column: Chiralcel OD-3 50 x 4.6 mm I.D., 3 um; mobile phase: CO₂-MeOH (0.05% diethylamide): elution gradient: 5%-40%.

### Single-configuration Compound 477c-2:

MS m/z (ESI):551.3[M+1]
Chiral HPLC analysis: retention time: 1.281 min; chromatographic column: Chiralcel OD-3 50 x 4.6 mm I.D., 3 um; mobile phase: CO₂-MeOH (0.05% diethylamide): elution gradient: 5%-40%.

### Step 5

### 5-fluoro-N-methyl-2-[4-methyl-6-({1-[(3R)-2-methyl-6-oxohexan-3-yl]azacyclobutan-3-yl}methyl)pyrrolo[1,2-a]pyrazin-8-yl]-N-(isopropyl)benzamide 477d-1

Compound **477c-1** (70.00 mg, 127.11 µmol) was dissolved in acetonitrile (1.0 mL), followed by the addition of a hydrochloric acid solution (1.0 M; 1.0 mL). The mixture was stirred at 50°C to allow for reacting for 0.5 hours. The reaction mixture was concentrated under a reduced pressure, regulated to PH = 10 with a 10% aqueous sodium hydroxide solution, and extracted with dichloromethane (10 mL x 3). The organic phases were combined, dried over anhydrous sodium sulfate, and concentrated under a reduced pressure, to obtain the crude product of title Compound **477d-1** (60 mg, 118.42 µmol, yield: 93.17%). The crude product was directly used in the next step of reaction without purification.

### Single-configuration Compound 477d-1:

MS m/z (ESI): 507.3[M+1]

### Step 6

### 5-fluoro-2-[6-({1-[(3R)-6-[4-(2-hydroxylethyl)piperazin-1-yl]-2-methylhexan-3-yl]azacyclobutan-3-yl}methyl)-4-methylpyrrolo[1,2-a]pyrazin-8-yl]-N-methyl-N-(isopropyl)benzamide 477-1

The crude product of Compound **477d-1** (60.00 mg, 118.42 µmol) and 2-(piperazin-1-yl)ethanol (23.13 mg, 177.64 µmol) were dissolved in methanol (1 mL), followed by the addition of acetic acid (7.11 mg, 118.42 µmol), and the mixture was stirred at 40°C for 0.5 hours under the protection of nitrogen. Sodium cyanoborohydride (22.33 mg, 355.27 µmol) was added, allowing for reacting at 40°C for 12.0 hours under the protection of nitrogen. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 26%-56%), to obtain the title Compound **477-1** (25.31 mg, 40.77 µmol, yield: 34.42%).

### Single-configuration Compound 477-1:

MS m/z (ESI):621.3 [M+1]
Chiral SFC: retention time: 1.156 min; chromatographic column: Chiralcel AD-3 50×4.6 mm I.D., 3 um; mobile phase: CO₂/MeOH (0.05% DEA): elution gradient: 5%-40%.

¹H NMR (400 MHz, DMSO-*d₆*) δ 8.63 - 8.43 (m, 1H), 7.62 - 7.45 (m, 1H), 7.37 - 7.16 (m, 3H), 6.71 - 6.30 (m, 1H), 4.58 - 4.30 (m, 1H), 3.54 - 3.37 (m, 8H), 2.84 - 2.73 (m, 5H), 2.69 - 2.60 (m, 3H), 2.41 - 2.23 (m, 10H), 2.20 - 2.15 (m, 2H), 1.98 - 1.91 (m, 1H), 1.75 - 1.60 (m, 1H), 1.45 - 1.34 (m, 2H), 1.27 - 1.09 (m, 2H), 1.04 - 0.98 (m, 1H), 0.95 - 0.86 (m, 2H), 0.85 - 0.77 (m, 6H), 0.66 - 0.59 (m, 1H), 0.29 - -0.11 (m, 2H).

### Step 7

### 5-fluoro-N-methyl-2-[4-methyl-6-({1-[(3S)-2-methyl-6-oxohexan-3-yl]azacyclobutan-3-yl}methyl)pyrrolo[1,2-a]pyrazin-8yl]-N-(isopropyl)benzamide 477d-2

Compound **477c-2** (80.00 mg, 145.27 µmol) was dissolved in acetonitrile (1.0 mL), followed by the addition of a hydrochloric acid solution (1.0 M; 1.0 mL). The mixture was stirred at 50°C to allow for reacting for 0.5 hours. The reaction mixture was concentrated under a reduced pressure, regulated to PH = 10 with a 10% aqueous sodium hydroxide solution, and extracted with dichloromethane (10 mL x 3). The organic phases were combined, dried over anhydrous sodium sulfate, and concentrated under a reduced pressure, to obtain the crude product of title Compound **477d-2** (70 mg, 138.16 µmol, yield: 95.11%). The crude product was directly used in the next step of reaction without purification.

### Single-configuration Compound 477d-2:

MS m/z (ESI): 507.3 [M+1]

### Step 8

### 5-fluoro-2-[6-({1-[(3S)-6-[4-(2-hydroxylethyl)piperazin-1-yl]-2-methylhexan-3-yl]azacyclobutan-3-yl}methyl)-4-methylpyrrolo[1,2-a]pyrazin-8-yl]-N-methyl-N-(isopropyl)benzamide 477-2

The crude product of Compound **477d-2** (70.00 mg, 138.16 µmol) and 2-(piperazin-1-yl)ethanol (26.98 mg, 207.24 µmol) were dissolved in methanol (2 mL), followed by the addition of acetic acid (8.30 mg, 138.16 µmol), and the mixture was stirred at 40°C for 0.5 hours under the protection of nitrogen.Sodium cyanoborohydride (26.05 mg, 414.48 µmol) was added, allowing for reacting at 40°C for 12.0 hours under the protection of nitrogen. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 26%-56%), to obtain the title Compound **477-2** (42.19 mg, 67.96 µmol, yield: 49.19%).

### Single-configuration Compound 477-2:

MS m/z (ESI):621.3 [M+1]
Chiral SFC: retention time: 1.391 min; chromatographic column: Chiralcel OD-3 50×4.6 mm I.D., 3um,; mobile phase: CO₂/MeOH (0.05% DEA): elution gradient: 5%-40%.

¹H NMR (400 MHz, DMSO-*d₆*) δ 9.00 - 8.15 (m, 1H), 7.67 - 7.42 (m, 1H), 7.36 - 7.18 (m, 3H), 6.69 - 6.47 (m, 1H), 4.56 - 4.28 (m, 1H), 3.58 - 3.38 (m, 8H), 2.85 - 2.72 (m, 5H), 2.68 - 2.59 (m, 3H), 2.41 - 2.24 (m, 10H), 2.21 - 2.15 (m, 2H), 1.98 - 1.90 (m, 1H), 1.73 - 1.61 (m, 1H), 1.46 - 1.34 (m, 2H), 1.25 - 1.11 (m, 2H), 1.05 - 0.99 (m, 1H), 0.94 - 0.87 (m, 2H), 0.86 - 0.76 (m, 6H), 0.67 - 0.59 (m, 1H), 0.37 - -0.13 (m, 2H).

### Example 482

### N-ethyl-5-fluoro-2-(6-{1-[(3R)-6-[8-(2-hydroxyethyl)-2-oxa-5,8-diazaspiro[3.5]non-5-yl]-2-methylhexan-3-yl]azacyclobutan-3-yl}-3-methylimidazo[1,5-a]pyridin-8-yl)-N-(isopropyl)benzamide 482

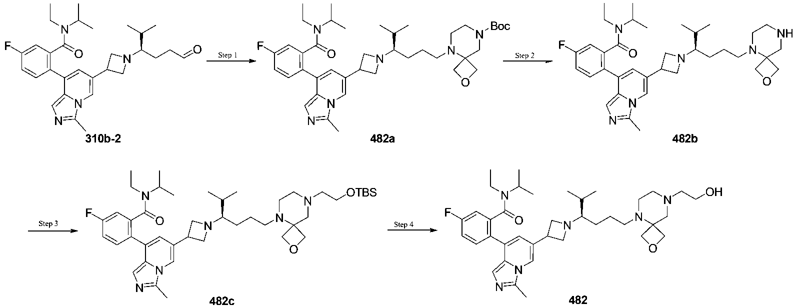

### Step 1

### 5-[(4R)-4-[3-(8-{2-[ethyl(isopropyl)aminoformyl]-4-fluorophenyl}-3-methylimidazo[1,5-a]pyridin-6-yl)azacyclobutan-1-yl]-5-methylhexyl]-2-oxa-5,8-diazaspiro[3.5]nonan-8-tert-butyl carboxylate 482a

Compound **310b-2** (200 mg, 394.75 µmol) and 2-oxa-5,8-diazaspiro[3.5]nonan-8-tert-butyl carboxylate (108.14 mg, 473.70 µmol) were dissolved in methanol (3.0 mL), followed by the addition of acetic acid (23.70 mg, 394.75 µmol), and the mixture was stirred at 35°C for 1.0 hour under the protection of nitrogen. Sodium cyanoborohydride (74.42 mg, 1.18 mmol) was added, and the mixture was stirred at 35°C to allow for reacting for 11.0 hours under the protection of nitrogen. A saturated aqueous sodium bicarbonate solution (30 mL) was added to the reaction mixture, which was then extracted with dichloromethane (65 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title product **482a** (120 mg, 166.91 µmol, yield: 42.28%) as yellow oil.
MS m/z (ESI):719.3 [M+1]

### Step 2

### N-ethyl-5-fluoro-2-(3-methyl-6-{1-[(3R)-2-methyl-6-{2-oxa-5,8-diazaspiro[3.5]non-5-yl}hexan-3-yl]azacyclobutan-3-yl}imidazo[1,5-a]pyridin-8-yl)-N-(isopropyl)benzamide 482b

Compound **482a** (100 mg, 139.09 µmol) was dissolved in dichloromethane (1.0 mL), followed by the addition of trifluoroacetic acid (740.00 mg, 6.49 mmol, 0.5 mL) at 25°C. The mixture was stirred at room temperature to allow for reacting for 0.5 hours. The reaction mixture was concentrated under a reduced pressure, regulated to PH = 14 by adding a 10%aqueous sodium hydroxide solution in an ice bath, and extracted with dichloromethane (50 mL x 3). The organic phases were combined, dried over anhydrous sodium sulfate and filtered, and the filtrate was concentrated under a reduced pressure, to obtain the crude product of title Compound **482b** (100 mg, 161.60 µmol, yield: 116.18%) as yellow solid. The crude product was directly used in the next step of reaction without purification. MS m/z (ESI):619.4 [M+1]

### Step 3

### 2-(6-{1-[(3R)-6-(9-{2-[(tert-butyldimethylsilyl)oxy]ethyl}-2-oxa-5,8-diazaspiro[3.5]non-5-yl)-2-methylhexan-3-yl]azacyclobutan-3-yl}-3-methylimidazo[1,5-a]pyridin-8-yl)-N-ethyl-5-fluoro-N-(isopropyl)benzamide 482c

Compound **482b** (100 mg, 161.60 µmol)) and 2-[tert-butyl(dimethyl)silicyl]oxyacetaldehyde (33.80 mg, 193.92 µmol) were dissolved in methanol (2.0 mL), followed by the addition of acetic acid (9.70 mg, 161.60 µmol), and the mixture was stirred at 35°C for 1.0 hour under the protection of nitrogen. Sodium cyanoborohydride (30.47 mg, 484.79 µmol) was added, and the mixture was stirred at 35°C to allow for reacting for 11 hours under the protection of nitrogen. A saturated aqueous sodium bicarbonate solution (30 mL) was added to the reaction mixture, which was then extracted with dichloromethane (65 mL x 3). The organic phases were combined, dried over anhydrous sodium sulfate, and concentrated under a reduced pressure, to obtain the crude product of title Compound **482c** (100 mg, 128.68 µmol, yield: 79.63%) as yellow solid. The crude product was directly used in the next step of reaction without purification.
MS m/z (ESI):777.4 [M+1]

### Step 4

### N-ethyl-5-fluoro-2-(6-{1-[(3R)-6-[8-(2-hydroxyethyl)-2-oxa-5,8-diazaspiro[3.5]non-5-yl]-2-methylhexan-3-yl]azacyclobutan-3-yl}-3-methylimidazo[1,5-a]pyridin-8-yl)-N-(isopropyl)benzamide 482

**482c** (100 mg, 128.68 µmol) was dissolved in trifluoroacetic acid (2.96 g, 25.96 mmol, 2 mL), and the mixture was stirred at 25°C for 0.5 hours.The reaction mixture was diluted with ethyl acetate (20 mL), and washed with a saturated aqueous sodium carbonate solution (20 mL x 3), and the organic phase was concentrated under a reduced pressure. The obtained crude product was purified by the HPLC (mobile phase: acetonitrile, water (0.1% ammonia water), elution gradient:28%-58%). The title Compound **482** (12.24 mg, 18.46 µmol, yield: 14.35%) was obtained as yellow solid.
MS m/z (ESI): 663.4 [M+1]
¹H NMR (400 MHz, DMSO-*d₆*) δ 7.93 (s, 1H), 7.70 - 7.64 (m, 1H), 7.42 - 7.33 (m, 1H), 7.33 - 7.28 (m, 1H), 7.06 - 7.01 (m, 1H), 6.75 (d, J = 15.6 Hz, 1H), 4.53 (d, J = 6.4 Hz, 2H), 4.38 (t, J = 5.2 Hz, 1H), 4.15 (d, J = 6.4 Hz, 2H), 3.61 - 3.41 (m, 6H), 3.30 - 3.27 (m, 1H), 3.19 - 3.03 (m, 2H), 2.97 - 2.89 (m, 1H), 2.64 - 2.53 (m, 7H), 2.45 - 2.39 (m, 2H), 2.38 - 2.30 (m, 4H), 2.08 - 2.00 (m, 1H), 1.77 - 1.66 (m, 1H), 1.52 - 1.39 (m, 2H), 1.34 - 1.21 (m, 2H), 0.94 - 0.70 (m, 13H), 0.30 - 0.24 (m, 2H).

### Examples 483 and 484

### N-ethyl-5- fluoro-2-(3-methyl-6- {1-[(3R)-2-methyl-6- {2-oxa-5,8-diazaspiro[3.5]non-8-yl} hexan-3-yl]azacyclobutan-3-yl}imidazo[1,5-a]pyridin-8-yl)-N-(isopropyl)benzamide 483

### 2-(6-{1-[(3R)-6-[8^{a}-(hydroxymethyl)-3-oxo-hexahydro-1H-[1,3]oxazolo[3,4-a]pyrazin-7-yl]-2-methylhexan-3-yl]azacyclobutan-3-yl}-3-methylimidazo[1,5-a]pyridin-8-yl)-N-ethyl-5-fluoro-N-(isopropyl)benzamide 484

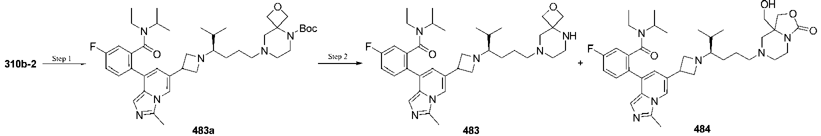

### Step 1

### 8-[(4R)-4-[3-(8-{2-[ethyl(isopropyl)aminoformyl]-4-fluorophenyl}-3-methylimidazo[1,5-a]pyridin-6-yl)azacyclobutan-1-yl]-5-methylhexyl]-2-oxa-5,8-diazaspiro[3.5]nonan-5-tert-butyl carboxylate 483a

Compound **310b-2** (200 mg, 394.75 µmol) and 2-oxa-5,8-diazaspiro[3.5]nonan-5-tert-butyl carboxylate (90.12 mg, 394.75 µmol) were dissolved in methanol (5.0 mL), followed by the addition of sodium cyanoborohydride (74.42 mg, 1.18 mmol), and the mixture was stirred at 50°C for 1.0 hour under the protection of nitrogen. Sodium cyanoborohydride (74.42 mg, 1.18 mmol) was further added, and the mixture was stirred at 50°C to allow for reacting for 2 hours under the protection of nitrogen. The reaction mixture was concentrated under a reduced pressure to remove the organic solvent, followed by adding a saturated aqueous potassium carbonate solution (10 mL) and extracting with dichloromethane (10 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title product **483a** (127 mg, 176.65 µmol, yield: 44.75%) as yellow solid.
MS m/z (ESI):719.6 [M+1]

### Step 2

### N-ethyl-5-fluoro-2-(3-methyl-6-{1-[(3R)-2-methyl-6-{2-oxa-5,8-diazaspiro[3.5]non-8-yl}hexan-3-yl]azacyclobutan-3-yl}imidazo[1,5-a]pyridin-8-yl)-N-(isopropyl)benzamide 483

### 2-(6-{1-[(3R)-6-[8^{a}-(hydroxymethyl)-3-oxo-hexahydro-1H-[1,3]oxazolo[3,4-a]pyrazin-7-yl]-2-methylhexan-3-yl]azacyclobutan-3-yl}-3-methylimidazo[1,5-a]pyridin-8-yl)-N-ethyl-5-fluoro-N-(isopropyl)benzamide 484

Compound **483a** (97 mg, 134.92 µmol) was dissolved in dichloromethane (3.0 mL), followed by the addition of trifluoroacetic acid (1.48 g, 12.98 mmol, 1 mL) at 25°C. The mixture was stirred at room temperature to allow for reacting for 1.0 hours. The reaction mixture was concentrated under a reduced pressure, dissolved by adding DCM (5 mL), regulated to PH > 7 by adding triethylamine, and concentrated under a reduced pressure. The crude product was purified by the HPLC (mobile phase: acetonitrile, water (0.1% ammonia water), elution gradient:28%-58%), to obtain the title Compounds **483** (14.27 mg, 23.06 µmol, yield: 17.09%) and **484** (18.72 mg, 28.24 µmol, yield: 20.93%).

### Compound 483:

MS m/z (ESI):619.4 [M+1]
¹H NMR (400 MHz, DMSO-*d₆*) δ 7.93 (s, 1 H), 7.61 - 7.74 (m, 1 H), 7.38 (td, J=8.40, 2.80 Hz, 1 H), 7.27 - 7.34 (m, 1 H), 6.99 - 7.07 (m, 1 H), 6.75 (d, J=15.60 Hz, 1 H), 4.35 - 4.51 (m, 2 H), 4.22 - 4.29 (m, 4 H), 3.45 - 3.53 (m, 2 H), 3.29 (br s, 1 H), 3.14 (br dd, J=14.01, 6.40 Hz, 1 H), 3.07 (br d, J=7.60 Hz, 1 H), 2.92 (dq, J=13.60, 6.80 Hz, 1 H), 2.58 (s, 5 H) ,2.45 (br s, 1 H), 2.17 - 2.29 (m, 4 H), 2.07 (s, 2 H), 2.02 - 2.06 (m, 1 H), 1.71 (br d, J=6.80 Hz, 1 H), 1.46 (br d, J=6.40 Hz, 2 H), 1.19 - 1.26 (m, 2 H), 0.71 - 0.91 (m, 13 H), 0.28 (br d, J=6.40 Hz, 2 H).

### Compound 484:

MS m/z (ESI):663.2 [M+1]
¹H NMR (400 MHz, DMSO-*d₆*) δ 7.93 (s, 1 H), 7.62 - 7.73 (m, 1 H), 7.27 - 7.44 (m, 2 H), 6.98 - 7.08 (m, 1 H), 6.76 (d, J=12.40 Hz, 1 H), 5.00 (t, J=5.60 Hz, 1 H), 4.14 - 4.24 (m, 1 H), 3.84 (br d, J=8.40 Hz, 1 H), 3.71 - 3.79 (m, 1 H), 3.40 - 3.61 (m, 6 H), 3.26 - 3.31 (m, 1 H), 3.10 - 3.18 (m, 1 H), 2.88 - 3.09 (m, 3 H), 2.85 (br d, J=11.20 Hz, 1 H), 2.70 - 2.77 (m, 1 H), 2.59 (s, 3 H), 2.19 - 2.33 (m, 2 H), 2.02 (br s, 1 H), 1.77 - 1.89 (m, 2 H), 1.65 - 1.76 (m, 1 H), 1.37 - 1.50 (m, 2 H), 1.14 - 1.31 (m, 2 H), 0.66 - 1.01 (m, 13 H), 0.27 (br d, J=5.20 Hz, 2 H).

### Example 485

### N-ethyl-5-fluoro-2-(3-methyl-6-{1-[1-(3-methyloxacyclobutan-3-yl)-4-[(1R,4R)-2-oxa-5-azabicyclo[2.2.1]heptan-5-yl]butyl]azacyclobut- 3-yl}imidazo[1,5-a]pyridin-8-yl)-N-(isopropyl)benzamide 485

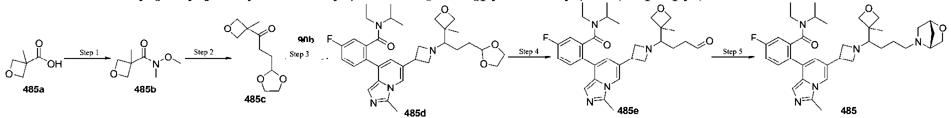

### Step 1

### N-methoxy-N,3-dimethyloxacyclobutan-3-formamide 485b

3-Methyloxacyclobutan-3-carboxylic acid **485a**(1 g, 8.61 mmol), *N*-methoxymethylamine (1.68 g, 17.22 mmol, hydrochloride), HATU (3.60 g, 9.47 mmol) and triethylamine (2.61 g, 25.84 mmol) were dissolved in DMF (10 mL). The reaction mixture was stirred at room temperature for 12 hours. The reaction mixture was diluted with water (20 mL) and extracted with ethyl acetate (20 mL × 2). The organic phases were combined, washed with saturated brine, dried over anhydrous sodium sulfate, and concentrated under a reduced pressure, to obtain the title Compound **485b** (790 mg, 4.96 mmol, yield: 57.63%).
¹H NMR (400 MHz, CDCl₃) δ 4.95 (d, J = 6.4 Hz, 2H), 4.28 (d, J = 6.4 Hz, 2H), 3.66 (s, 3H), 3.18 (s, 3H),1.66(s,3H).

### Step 2

### 3-(1,3-dioxolan-2-yl)-1-(3-methyloxacyclobutan-3-yl)propan-1-one 485c

Under the protection of nitrogen, Compound **485b** (790 mg, 4.96 mmol) was dissolved in tetrahydrofuran (10.0 mL), and cooled to -0°C under the protection of nitrogen, followed by the slow dropwise addition of magnesium 2-(1,3-dioxolan-2-yl)ethyl bromide solution (0.5 M, 17.41 mL). After the dropwise addition, the mixture was further stirred 0°C for 1.0 hour, then warmed to 25°C and further stirred for 12 hours. The reaction mixture was quenched with a saturated aqueous ammonium chloride solution (30 mL), and extracted with methyltert-butyl ether (20 mL×3). The organic phase was dried over anhydrous sodium sulfate, filtered and concentrated under a reduced pressure. The resulting residues were purified by the silica-gel column chromatography using petroleum ether/ethyl acetate as an elution system, to obtain the title Compound **485c** (410 mg, 2.05 mol, yield: 41%).
¹H NMR (400 MHz, CDCl₃) δ 4.97 - 4.91 (m, 1H), 4.89 (d, J = 6.0 Hz, 2H), 4.40 (d, J = 6.0 Hz, 2H), 3.98 - 3.93 (m, 2H), 3.88 - 3.83 (m, 2H), 2.66 - 2.58 (m, 2H), 2.03 (td, J = 3.6, 7.2 Hz, 2H), 1.56 (s, 3H).

### Step 3

### 2-(6-{1-[3-(1,3-dioxolan-2-yl)-1-(3-methyloxacyclobutan-3-yl)propyl]azacyclobut-3-yl}-3-methylimidazo[1,5-a]pyridin-8-yl)-N-ethyl-5-fluoro-N-(isopropyl)benzamide 485d

Compound **90b** (290 mg, 735.14 µmol) and Compound **485c** (273.37 mg, 955.68 µmol) were dissolved in methanol (2.0 mL), followed by the addition of ZnCl₂ (200.39 mg, 1.47 mmol), and the mixture was stirred at 50°C for 1.0 hour. The mixture was cooled to room temperature, followed by adding sodium cyanoborohydride (138.59 mg, 2.21 mmol) and stirring at 50°C for 12 hour. A saturated aqueous sodium bicarbonate solution (30 mL) was added to the reaction mixture, which was then extracted with dichloromethane (20 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **485d** (165 mg, 285.11 µmol, yield: 38.78%) as yellow solid.
MS m/z (ESI): 579.4 [M+1]
¹H NMR (400 MHz, CDCl₃) δ 7.71 - 7.63 (m, 1H), 7.58 - 7.50 (m, 1H), 7.23 - 7.10 (m, 3H), 6.88 - 6.76 (m, 1H), 4.89 - 4.72 (m, 2H), 4.68 - 4.59 (m, 1H), 4.16 (d, J = 5.2 Hz, 2H), 4.01 - 3.92 (m, 2H), 3.89 - 3.81 (m, 2H), 3.77 - 3.66 (m, 1H), 3.60 - 3.42 (m, 4H), 3.30 - 3.10 (m, 2H), 3.01 - 2.87 (m, 1H), 2.75 (d, J = 2.4 Hz, 1H), 2.68 (s, 3H), 1.67 (br dd, J = 4.4, 7.6 Hz, 1H), 1.56 - 1.42 (m, 5H), 1.29 - 1.10 (m, 2H), 1.00 - 0.89 (m, 5H), 0.78 (br t, J = 7.2 Hz, 1H), 0.26 (dd, J = 2.8, 6.4 Hz, 2H).

### Step 4

### N-ethyl-5-fluoro-2-(3-methyl-6-{1-[1-(3-methyloxacyclobutan-3-yl)-4-oxobutyl]azacyclobutan-3-alkyl}imidazo[1,5-a]pyridin-8-yl)-N-(isopropyl)benzamide 485e

Compound **485d** (50 mg, 86.40 µmol) was dissolved in dichloromethane (1.5 mL), followed by the addition of trifluoroacetic acid (0.5 mL). The mixture was stirred at 25°C to allow for reacting for 1.0 hours. The reaction mixture was regulated to pH = 8-9 with a saturated aqueous sodium bicarbonate solution and extracted with dichloromethane (8 mL x 3). The organic phases were combined, dried over anhydrous sodium sulfate, and concentrated under a reduced pressure, to obtain the crude product of title Compound **485e** (40 mg, 74.81 µmol, yield: 86.59%). The crude product was directly used in the next step of reaction without purification.
MS m/z (ESI): 535.4 [M+1]

### Step 5

### N-ethyl-5-fluoro-2-(3-methyl-6-{1-[1-(3-methyloxacyclobutan-3-yl)-4-[(1R,4R)-2-oxa-5-azabicyclo[2.2.1]heptan-5-yl]butyl]azacyclobut-3-yl}imidazo[1,5-a]pyridin-8-yl)-N-(isopropyl)benzamide 485

Compound **485e** (40 mg, 74.81 µmol) was dissolved in dichloromethane (2.0 mL), followed by the addition of triethylamine (15.14 mg, 149.63 µmol) and (1*R*,4*R*)-2-oxa-5-azabicyclo[2.2.1]heptane (12.17 mg, 89.78 µmol, hydrochloride). Under the protection of nitrogen, the mixture was stirred at 50°C for 1.0 hours. Sodium cyanoborohydride (47.57 mg, 224.44 µmol) was added, allowing for reacting at 50°C for 16 hours under the protection of nitrogen. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 21%-41%), to obtain the title Compound **485** (11.14 mg, 18.03 µmol, yield: 24.10%) as yellow solid.
MS m/z (ESI): 618.3 [M+1]
¹H NMR (400 MHz, CDCl₃) δ 7.71 - 7.63 (m, 1H), 7.55 - 7.50 (m, 1H), 7.22 - 7.12 (m, 3H), 6.93 - 6.80 (m, 1H), 4.88 - 4.71 (m, 1H), 4.60 (d, J = 5.2 Hz, 1H), 4.39 (s, 1H), 4.16 (d, J = 4.8 Hz, 2H), 4.08 - 3.99 (m, 1H), 3.74 - 3.61 (m, 2H), 3.60 - 3.39 (m, 5H), 3.27 - 3.11 (m, 2H), 2.99 - 2.86 (m, 2H), 2.75 - 2.70 (m, 1H), 2.68 (s, 3H), 2.61 - 2.41 (m, 3H), 1.87 - 1.73 (m, 2H), 1.45 (s, 3H), 1.39 - 1.28 (m, 3H), 1.20 - 1.09 (m, 2H), 1.00 - 0.84 (m, 6H), 0.27 - 0.24 (m, 2H).

### Example 486

### N-ethyl-5-fluoro-2-(6-{1-[(3R)-6-[4-(2-hydroxyethyl)-3-oxopiperazin-1-yl]-2-methylhexan-3-yl]azacyclobutan-3-yl}-3-methylimidazo[1,5-a]pyridin-8-yl)-N-(isopropyl)benzamide 486

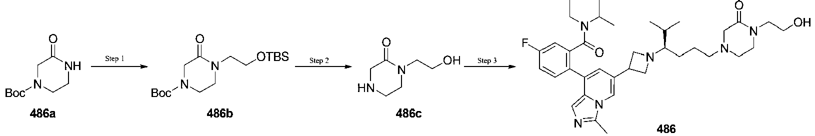

### Step 1

### 4-{2-[(tert-butyldimethylsilicyl)oxy]ethyl}-3-oxopiperazin-1-tert-butyl carboxylate 486b

3-Oxopiperazin-1-tert-butyl carboxylate **486a** (1 g, 4.99 mmol) and (2-bromoethoxy)(tert-butyl)dimethylsilane (1.19 g, 4.99 mmol) were dissolved in tetrahydrofuran (10 mL), followed by the addition of potassium hydroxide (336.24 mg, 5.99 mmol) and tetrabutylammonium iodide (368.93 mg, 998.83 µmol). The reaction mixture was stirred at 25°C for 12 hours, the reaction mixture was filtered, and the filtrate was concentrated under a reduced pressure. The resulting residues were purified by the silica-gel column chromatography using petroleum ether/ethyl acetate as an elution system, to obtain the title Compound **486b** (500 mg, 1.39 mmol, yield: 27.92%).

¹H NMR (400 MHz, DMSO-*d₆*) δ 3.89 (s, 2H), 3.71 - 3.66 (m, 2H), 3.55 - 3.49 (m, 2H), 3.43 - 3.37 (m, 4H), 1.42 - 1.38 (m, 9H), 0.87 - 0.83 (m, 9H), 0.05-0.02 (m, 6H).

### Step 2

### 1-(2-hydroxyethyl)piperazin-2-one 486c

Compound **486b** (150 mg, 418.36 µmol) was dissolved in dichloromethane (3.0 mL), followed by the addition of trifluoroacetic acid (1.48 g, 12.98 mmol, 1 mL). The mixture was stirred at 25°C for 4.0 hours. The reaction mixture was concentrated under a reduced pressure, to obtain the crude product of title Compound **486c** (60 mg). The crude product was directly used in the next step of reaction without purification.

¹H NMR (400 MHz, CD₃OD) δ 4.26 - 4.21 (m, 1H), 3.53 - 3.47 (m, 2H), 3.44 - 3.31 (m, 4H), 3.27 - 3.13 (m, 2H), 3.01 - 2.90 (m, 2H).

### Step 3

### N-ethyl-5-fluoro-2-(6-{1-[(3R)-6-[4-(2-hydroxyethyl)-3-oxopiperazin-1-yl]-2-methylhexan-3-yl]azacyclobutan-3-yl}-3-methylimidazo[1,5-a]pyridin-8-yl)-N-(isopropyl)benzamide 486

Compound **310b-2(135** mg, 266.45 µmol) was dissolved in methanol (5.0 mL), followed by the addition of triethylamine (53.92 mg, 532.91 µmol) and the crude product of Compound **486c** (60 mg). Under the protection of nitrogen, the mixture was stirred at 40°C for 0.5 hours. Sodium cyanoborohydride (50.23 mg, 799.36 µmol) was added, allowing for reacting at 40°C for 12 hours under the protection of nitrogen. A saturated aqueous sodium bicarbonate solution (15 mL) was added to the reaction mixture, which was then extracted with dichloromethane (15 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 25%-55%), to obtain the title Compound **486** (23.17 mg, 36.50 µmol, yield: 13.70%) as yellow solid.
MS m/z (ESI): 635.2 [M+1]
¹H NMR (400 MHz,DMSO-*d₆*) δ 7.93 (s, 1H), 7.73 - 7.63 (m, 1H), 7.45 - 7.23 (m, 2H), 7.10 - 6.96 (m, 1H), 6.89 - 6.71 (m, 1H), 4.76 - 4.61 (m, 1H), 3.63 - 3.38 (m, 7H), 3.31 - 3.03 (m, 6H), 2.98 - 2.84 (m, 3H), 2.63 - 2.54 (m, 5H), 2.34 - 2.24 (m, 2H), 2.12 - 1.96 (m, 1H), 1.79 - 1.65 (m, 1H), 1.55 - 1.39 (m, 2H), 1.32 - 1.02 (m, 3H), 0.93 - 0.70 (m, 12H), 0.30 - 0.26 (m, 2H).

### Example 487

### 2-(6-{1-[(3R)-6-[4-(3-cyanooxacyclobutan-3-yl)piperazin-1-yl]-2-methylhexan-3-yl]azacyclobutan-3-yl}-3-methylimidazo[1,5-a]pyridin-8-yl)-N-ethyl-5-fluoro-N-(isopropyl)benzamide 487

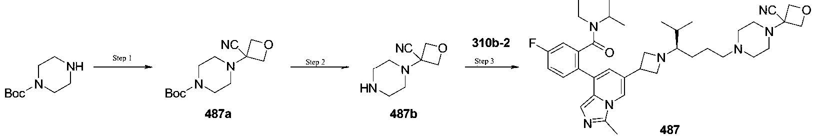

### Step 1

### 4-(3-cyanooxacyclobutan-3-yl)piperazin-1-tert-butyl carboxylate 487a

Piperazin-1-tert-butyl carboxylate (3 g, 16.11 mmol) and oxacyclobutan-3-one (1.74 g, 24.16 mmol) were dissolved in 1,2-dichloroethane (40 mL), followed by the addition of acetic acid (1.16 g, 19.33 mmol), and the reaction mixture was stirred at 50°C for 1.0 hour. The reaction mixture was cooled to room temperature, followed by the dropwise addition of TMSCN(3.49 g, 35.18 mmol), and the reaction mixture was stirred at 25°C for 16 hours. A saturated aqueous sodium bicarbonate solution (30 mL) was added to the reaction mixture, which was then extracted with dichloromethane (20 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **487a** (3.14 g, 11.75 mmol, yield: 72.92%) as white solid.
¹H NMR (400 MHz, CDCl₃) δ 4.79 (d, J=6.40 Hz, 2 H), 4.65 (d, J=6.40 Hz, 2 H), 3.43 - 3.57 (m, 4 H), 2.36 (br s, 4 H), 1.45 (s, 9 H).

### Step 2

### 3-(piperazin-1-yl)oxacyclobutan-3-nitrile 487b

Compound **487a** (200 mg, 748.16 µmol) was dissolved in dichloromethane (2.0 mL), followed by the addition of trifluoroacetic acid (1.48 g, 12.98 mmol, 1 mL). The mixture was stirred at 25°C for 1.0 hours. The reaction mixture was concentrated under a reduced pressure, and the residues were dissolved with dichloromethane (10 mL) and regulated to PH = 8 with triethylamine. The mixture was concentrated under a reduced pressure to remove the solvent, to obtain the crude product of title Compound **487b** (130 mg). The crude product was directly used in the next step of reaction without purification.
MS m/z (ESI): 168.3 [M+1]

### Step 3

### 2-(6-{1-[(3R)-6-[4-(3-cyanooxacyclobutan-3-yl)piperazin-1-yl]-2-methylhexan-3-yl]azacyclobutan-3-yl}-3-methylimidazo[1,5-a]pyridin-8-yl)-N-ethyl-5-fluoro-N-(isopropyl)benzamide 487

Compound **310b-2** (130 mg, 256.59 µmol) was dissolved in methanol (3.0 mL), followed by the addition of the crude product of Compound **487b** (130 mg). Under the protection of nitrogen, the mixture was stirred at 50°C for 1.0 hours. Sodium cyanoborohydride (80.62 mg, 1.28 mmol) was added, allowing for reacting 50°C for 16 hours under the protection of nitrogen. A saturated aqueous potassium carbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 0%-26%), to obtain the title Compound **487** (8.92 mg, 13.56 µmol, yield: 5.28%) as yellow solid.
MS m/z (ESI): 658.5 [M+1]
¹H NMR (400 MHz,DMSO-*d₆*) δ 8.20 (br s, 2 H), 7.96 (br s, 1 H), 7.61 - 7.74 (m, 1 H), 7.28 - 7.43 (m, 2 H), 6.99 - 7.08 (m, 1 H), 6.71 - 6.89 (m, 1 H), 4.71 (br d, J=6.80 Hz, 2 H), 4.55 (br d, J=6.40 Hz, 2 H), 3.58 (br d, J=6.80 Hz, 3 H), 3.41 - 3.47 (m, 1 H), 3.23 - 3.34 (m, 3 H), 2.85 - 2.95 (m, 1 H), 2.59 (s, 3 H), 2.44 (br s, 3 H), 2.21 - 2.37 (m, 7 H), 1.77 (br s, 1 H), 1.47 (br s, 2 H), 1.18 - 1.32 (m, 2 H), 1.06 (br s, 1 H), 0.68 - 0.91 (m, 13 H), 0.27 (br d, J=6.40 Hz, 2 H).

### Example 488

### N-ethyl-5-fluoro-2-{6-[(1-{6-[4-(2-hydroxyethyl)piperazin-1-yl]-2-methylhexan-3-yl}azacyclobutan-3-yl)oxy]-4-methylpyrrolo[1,2-a]pyrazin-8-yl}-N-(isopropyl)benzamide 488

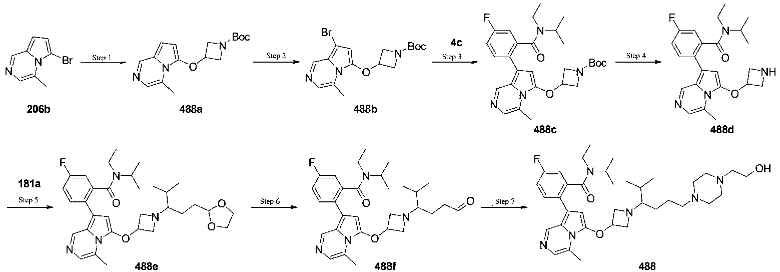

### Step 1

### 3-({4-methylpyrrolo[1,2-a]pyrazin-6-yl}oxy)azacyclobutan-1-tert-butyl carboxylate 488a

Compound 3-hydroxylazacyclobutan-1-tert-butyl formate (3.04 g, 17.53 mmol) was dissolved in DMSO(50 mL), and cooled to 0°C, followed by the slow addition of NaH (1.05 g, 26.30 mmol, 60%purity), and the mixture was stirred at 25°C to allow for reacting for 0.5 hours. Compound **206b** (3.7 g, 17.53 mmol) was added, and the mixture was stirred at 80°C under a nitrogen atmosphere to allow for reacting for 12.0 hours. The reaction mixture was cooled to room temperature, followed by the addition of a saturated aqueous ammonium chloride solution (50 mL), and the reaction mixture was extracted with ethyl acetate (50 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **488a** (160 mg, 527.43 µmol, yield: 3.01%) as yellow solid.
MS m/z (ESI): 304.2 [M+1]

### Step 2

### 3-({8-bromo-4-methylpyrrolo[1,2-a]pyrazin-6-yl}oxy)azacyclobutan-1-tert-butyl carboxylate 488b

Compound **488a** (200 mg, 659.29 µmol) was dissolved in dichloromethane (3.0 mL), and cooled to -10°C, followed by the addition of NBS(117.34 mg, 659.29 µmol), and the mixture was stirred at room temperature for 1.0 hour. A saturated aqueous sodium bicarbonate solution (30 mL) was added to the reaction mixture, which was then extracted with dichloromethane (20 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **488b** (160 mg, 418.57 µmol, yield: 63.49%) as yellow solid.
MS m/z (ESI): 384.0 [M+1]

### Step 3

### 3-[(8-{2-[ethyl(isopropyl)aminoformyl]-4-fluorophenyl}-4-methylpyrrolo[1,2-a]pyrazin-6-yl)oxy]azacyclobutan-1-tert-butyl carboxylate 488c

Compound **488b** (160 mg, 418.57 µmol), Compound **4c**(210.47 mg, 627.86 µmol) and potassium phosphate (266.55 mg, 1.26 mmol) were dissolved in dioxane (0.5 mL) and water (0.1 mL). Pd(dtbpf)Cl₂ (27.28 mg, 41.86 µmol) was added. Under a nitrogen atmosphere, the mixture was stirred at 90°Cto allow for reacting for 3.0 hours. The reaction mixture was cooled to room temperature, diluted by adding ethyl acetate (20 mL), and washed with water (20 mL x 3). The organic phase was dried over anhydrous sodium sulfate, and concentrated under a reduced pressure. The resulting residues were purified by the silica-gel column chromatography using petroleum ether/ethyl acetate as an elution system, to obtain the title Compound **488c** (170 mg, 332.94 µmol, yield: 79.54%) as yellow solid.
MS m/z (ESI): 511.2 [M+1]

### Step 4

### 2-[6-(azacyclobutan-3-yloxy)-4-methylpyrrolo[1,2-a]pyrazin-8-yl]-N-ethyl-5-fluoro-N-(isopropyl)benzamide 488d

Compound **488c** (170 mg, 332.94 µmol) was dissolved in methane dioxide (2.0 mL), followed by the addition of trifluoroacetic acid (740.00 mg, 6.49 mmol, 0.5 mL) at 0°C. The mixture was stirred at room temperature to allow for reacting for 0.5 hours. The reaction mixture was concentrated under a reduced pressure, followed by adding a 10% aqueous sodium hydroxide solution (10.0 mL) and extracting with dichloromethane (10 mL x 2). The organic phases were combined, dried over anhydrous sodium sulfate and filtered, and the filtrate was concentrated under a reduced pressure, to obtain the crude product of title Compound **488d** (140 mg, 341.06 µmol, yield: 102.44%). The crude product was directly used in the next step of reaction without purification.
MS m/z (ESI): 411.2 [M+1]

### Step 5

### 2-[6-({1-[1-(1,3-dioxolan-2-yl)-4-methylpentan-3-yl]azacyclobutan-3-yl}oxy)-4-methylpyrrolo[1,2-a]pyrazin-8-yl]-N-ethyl-5-fluoro-N-(isopropyl)benzamide 488e

Compound **488d** (120 mg, 292.34 µmol) and Compound **181a** (60.42 mg, 350.81 µmol) were dissolved in methanol (3.0 mL). Acetic acid (17.55 mg, 292.34 µmol) was added, and the mixture was stirred at 50°C for 1.0 hour. The reaction mixture was cooled to room temperature, followed by adding sodium cyanoborohydride (55.11 mg, 877.01 µmol) and stirring at 50°C for 11.0 hours. The mixture was diluted with ethyl acetate (20 mL), and washed with water (20 mL x 3). The organic phase was dried over anhydrous sodium sulfate, and concentrated under a reduced pressure. The resulting residues were purified by the preparative thin-layer chromatography using petroleum ether/ethyl acetate as an elution system, to obtain the title Compound **488e** (30 mg, 52.94 µmol, yield: 18.11%) as yellow oil.
MS m/z (ESI): 567.2[M+1]

### Step 6

### N-ethyl-5-fluoro-2-(4-methyl-6-{[1-(2-methyl-6-oxohexan-3-yl)azacyclobutan-3-yl]oxy}pyrrolo[1,2-a]pyrazin-8-yl)-N-(isopropyl)benzamide 488f

Compound **488e** (20 mg, 35.29 µmol) was dissolved in acetonitrile (0.5 mL), followed by the addition of a hydrochloric acid solution (1.0 M; 0.5 mL). The mixture was stirred at 50°C to allow for reacting for 1.0 hours. The reaction mixture was concentrated under a reduced pressure, followed by adding an aqueous saturated potassium carbonate solution (10 mL) and extracting with ethyl acetate (10 mL x 3). The organic phases were combined, dried over anhydrous sodium sulfate, and concentrated under a reduced pressure, to obtain the crude product of title Compound **488f** (18 mg, 34.44 µmol, yield: 97.59%). The crude product was directly used in the next step of reaction without purification.
MS m/z (ESI): 523.4[M+1]

### Step 7

### N-ethyl-5-fluoro-2-{6-[(1-{6-[4-(2-hydroxyethyl)piperazin-1-yl]-2-methylhexan-3-yl}azacyclobutan-3-yl)oxy]-4-methylpyrrolo[1,2-a]pyrazin-8-yl}-N-(isopropyl)benzamide 488

The crude product of Compound **488f** (18 mg, 34.44 µmol) and 2-(piperazin-1-yl)ethanol (5.38 mg, 41.33 µmol) were dissolved in methanol (2.0 mL), followed by the addition of acetic acid (2.07 mg, 34.44 µmol), under the protection of nitrogen, and the mixture was stirred at 50°C for 1.0 hour. Sodium cyanoborohydride (6.49 mg, 103.32 µmol) was added, allowing for reacting at 50°C for 11.0 hours under the protection of nitrogen. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (0.1% ammonia water), elution gradient: 38%-68%), to obtain the title Compound **488** (15.23 mg, 23.91 µmol, yield: 69.44%).
MS m/z (ESI):637.5 [M+1]
¹H NMR (400 MHz, DMSO-*d₆*) δ 8.44 (s, 1H), 7.56 - 7.50 (m, 1H), 7.35 - 7.28 (m, 1H), 7.27 - 7.20 (m, 1H), 7.07 (s, 1H), 6.27 - 6.20 (m, 1H), 4.82 - 4.73 (m, 1H), 4.41 - 4.23 (m, 1H), 3.73 - 3.60 (m, 2H), 3.49 - 3.43 (m, 2H), 3.42 - 3.37 (m, 1H), 3.30 - 3.22 (m, 1H), 3.12 - 2.93 (m, 3H), 2.66 (s, 3H), 2.42 - 2.25 (m, 9H), 2.22 - 2.17 (m, 2H), 2.07 - 2.00 (m, 1H), 1.75 - 1.64 (m, 1H), 1.47 - 1.34 (m, 2H), 1.28 - 1.07 (m, 3H), 0.93 - 0.76 (m, 12H), 0.74 - 0.68 (m, 1H), 0.35 - 0.30 (m, 2H).

### Example 490

### 2-(6-{1-[(3R)-6-{4-[3-(cyanomethyl)oxacyclobutan-3-y1]piperazin-1-yl}-2-methylhexan-3-yl]azacyclobutan-3-yl}-3-methylimidazo[1,5-a]pyridin-8-yl)-N-ethyl-5-fluoro-N-(isopropyl)benzamide 490

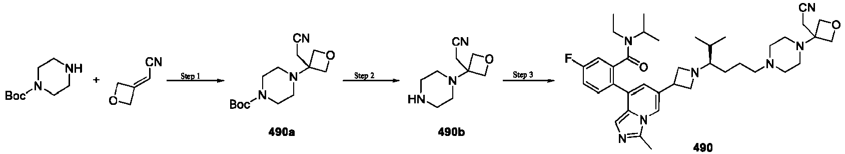

### Step 1

### 4-[3-(cyanomethyl)oxacyclobutan-3-yl]piperazin-1-tert-butyl carboxylate 490a

Piperazin-1-tert-butyl carboxylate (220.00 mg, 1.18 mmol) and 2-(oxacyclobutan-3-methylene)acetonitrile (123.56 mg, 1.30 mmol) were dissolved in acetonitrile (2.0 mL), followed by the addition of DBU(215.79 mg, 1.42 mmol), and the reaction mixture was stirred at 25°C for 12 hours. The reaction mixture was cooled to room temperature, followed by the dropwise addition of TMSCN(3.49 g, 35.18 mmol), and the reaction mixture was stirred at 25°C for 16 hours. Water (10 mL) was added to the reaction mixture, which was then extracted with ethyl acetate (10 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **490a** (100 mg, 355.43 µmol, yield: 30.09%) as white solid.

¹H NMR (400 MHz, DMSO-*d₆*) δ 4.46 (d, J = 6.4 Hz, 2H), 4.36 (d, J = 6.4 Hz, 2H), 3.34 - 3.32 (m, 2H), 3.32 - 3.29 (m, 2H), 2.96 (s, 2H), 2.36 - 2.29 (m, 4H), 1.39 (s, 9H).

### Step 2

### 2-[3-(piperazin-1-yl)oxacyclobutan-3-yl]acetonitrile 490b

Compound **490a** (45 mg, 159.94 µmol) was dissolved in dichloromethane (1.5 mL), followed by the addition of trifluoroacetic acid (740.00 mg, 6.49 mmol, 0.5 mL). The mixture was stirred at 25°C for 1.0 hours. The reaction mixture was concentrated under a reduced pressure, to obtain the crude product of title Compound **490b** (28 mg). The crude product was directly used in the next step of reaction without purification.

¹H NMR (400 MHz, CDCl₃) δ 4.70 (d, J = 6.8 Hz, 2H), 4.50 (d, J = 6.8 Hz, 2H), 3.40 (t, J = 4.8 Hz, 4H), 2.92 (s, 2H), 2.89 (t, J = 4.8 Hz, 4H).

### Step 3

### 2-(6-{1-[(3R)-6-{4-[3-(cyanomethyl)oxacyclobutan-3-yl]piperazin-1-yl}-2-methylhexan-3-yl]azacyclobutan-3-yl}-3-methylimidazo[1,5-a]pyridin-8-yl)-N-ethyl-5-fluoro-N-(isopropyl)benzamide 490

Compound **310b-2** (70 mg, 138.16 µmol) was dissolved in dichloromethane (2.0 mL), followed by the addition of the crude product of Compound **490b** (28 mg) and triethylamine (41.94 mg, 414.48 µmol). Under the protection of nitrogen, the mixture was stirred at 25°C for 1.0 hours. Sodium cyanoborohydride (87.85 mg, 414.48 µmol) was added, allowing for reacting at 25°C for 11 hours under the protection of nitrogen. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the HPLC (mobile phase: acetonitrile, water (10 mM ammonium acetate), elution gradient: 29%-59%), to obtain the title Compound **490** (29.49 mg, 43.89 µmol, yield: 31.77%) as yellow solid.
MS m/z (ESI): 672.5 [M+1]
¹H NMR (400 MHz,CD₃OD) δ 7.94 - 7.90 (m, 1H), 7.77 - 7.69 (m, 1H), 7.34 (dt, J = 2.8, 8.4 Hz, 1H), 7.28 - 7.21 (m, 1H), 7.17 - 7.12 (m, 1H), 6.95 - 6.87 (m, 1H), 4.61 (dd, J = 3.2, 6.4 Hz, 2H), 4.50 - 4.45 (m, 2H), 3.84 - 3.32 (m, 6H), 3.30 - 3.24 (m, 1H), 3.10 - 2.99 (m, 1H), 2.96 (d, J = 4.4 Hz, 2H), 2.66 (s, 3H), 2.62 - 2.32 (m, 10H), 2.31 - 2.24 (m, 1H), 1.92 - 1.82 (m, 1H), 1.68 - 1.53 (m, 2H), 1.47 - 1.09 (m, 3H), 0.98 - 0.77 (m, 12H), 0.30 (d, J = 6.4 Hz, 2H).

### Example 491

### N-ethyl-5-fluoro-2-[6-(1-{6-[4-(2-hydroxyethyl)piperazin-1-yl]-2-methylhexan-3-yl}azacyclobutan-3-yl)-3-methylimidazo[1,5-a]pyrazin-8-yl]-N-(isopropyl)benzamide 491

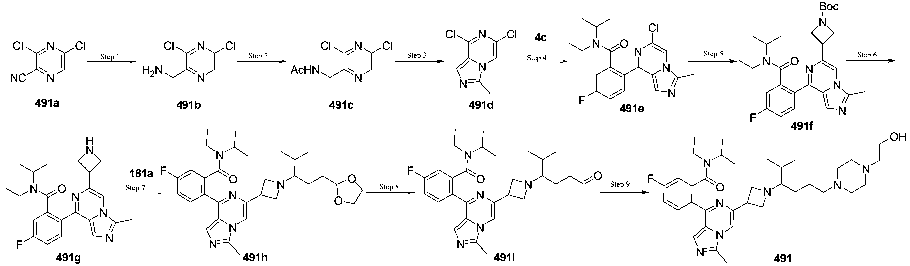

### Step 1

### 1-(3,5-dichloropyrazin-2-yl)methylamine 491b

Under an argon atmosphere, Raney Ni (0.5 g, 5.75 mmol) and 3,5-dichloropyrazin-2-nitrile **491a** (1 g, 5.75 mmol) were dissolved in acetic acid (20 mL), and the reaction mixture was added to a stainless steel hydrogenation flask. Hydrogen substitution was carried out three times, and under a hydrogen atmosphere (50 PSI), the reaction mixture was stirred at 50°C to allow for reacting for 12 hours. The reaction mixture was filtered through diatomite, and the filter cake was washed with methanol. The filtrate was concentrated under a reduced pressure, to obtain the crude product of title Compound **491b** (1.02 g, 5.73 mmol, yield: 99.7%). The crude product was directly used in the next step of reaction without purification.
MS m/z (ESI): 179.1 [M+1]

### Step 2

### N-[(3,5-dichloropyrazin-2-yl)methyl]acetamide 491c

Compound **491b** (1.95 g, 10.95 mmol) was dissolved in dichloromethane (60 mL), followed by the addition of triethylamine (4.43 g, 43.82 mmol). The reaction mixture was cooled to 0°C, followed by the slow dropwise addition of acetic anhydride (1.68 g, 16.43 mmol), and the reaction mixture was stirred at room temperature to allow for reacting for 12 hours. The reaction mixture was diluted by adding water (50 mL), the organic phase and the aqueous phase were separated, and the aqueous phase was extracted with ethyl acetate (40 mL x 3). The organic phases were combined, dried over anhydrous sodium sulfate, and concentrated under a reduced pressure. The resulting residues were purified by the silica-gel column chromatography using petroleum ether/ethyl acetate as an elution system, to obtain the title Compound **491c** (1.04 g, 4.73 mmol, yield: 43.15%) as yellow oil.
MS m/z (ESI): 220.1 [M+1]

### Step 3

### 6,8-dichloro-3-methylimidazol[1,5-a]pyrazine 491d

Compound **491c** (990 mg, 4.50 mmol) was dissolved in dichloromethane (10.0 mL), followed by the addition of a Burgess reagent (1.61 g, 6.75 mmol). The mixture was stirred at 40°C to allow for reacting for 4.0 hours. The reaction mixture was cooled to room temperature, followed by adding water (50 mL) and extracting with ethyl acetate (50 mL x 3). The organic phase was washed with saturated brine, dried over anhydrous sodium sulfate, and concentrated under a reduced pressure. The resulting residues were purified by the silica-gel column chromatography using petroleum ether/ethyl acetate as an elution system, to obtain the title Compound **491d** (680 mg, 3.37 mmol, yield: 74.81%) as yellow solid.
MS m/z (ESI): 204.1[M+1]

### Step 4

### 2-{6-chloro-3-methylimidazo[1,5-a]pyrazin-8-yl}-N-ethyl-5-fluoro-N-(isopropyl)benzamide 491e

Compound **491d** (250 mg, 1.24 mmol), Compound **4c**(373.31 mg, 1.11 mmol) and potassium phosphate (525.31 mg, 2.47 mmol) were dissolved in dioxane (10 mL) and water (2.0 mL). Pd (dppf)Cl₂ (90.54 mg, 123.74 µmol) was added. Under a nitrogen atmosphere, the mixture was stirred at 25°C to allow for reacting for 1.0 hours. The reaction mixture was concentrated under a reduced pressure. The resulting residues were purified by the silica-gel column chromatography using petroleum ether/ethyl acetate as an elution system, to obtain the title Compound **491e** (350 mg, 933.73 µmol, yield: 75.46%) as yellow solid.
MS m/z (ESI): 375.2 [M+1]
¹H NMR (400 MHz, CDCl₃) δ 7.89 - 7.80 (m, 1H), 7.75 (s, 1H), 7.61 (s, 1H), 7.26 - 7.08 (m, 2H), 4.45 - 3.94 (m, 1H), 3.48 - 3.20 (m, 2H), 2.69 (s, 3H), 1.34 - 1.14 (m, 9H).

### Step 5

### 3-(8-{2-[ethyl(isopropyl)aminoformyl]-4-fluorophenyl}-3-methylimidazo[1,5-a]pyrazin-6-yl)azacyclobutan-1-tert-butyl carboxylate 491f

Compound **491e** (347 mg, 925.73 µmol), nickel(II) chloride ethylene glycol dimethyl ether complex (30.51 mg, 138.86 µmol), pyridin-2,6-bis(carboximidamide) dihydrochloride (32.78 mg, 138.86 µmol) and sodium iodide (34.69 mg, 231.43 µmol) were dissolved in DMF(5.0 mL). Under a nitrogen atmosphere, 3-bromoazacyclobutan-1-tert-butyl formate (382.50 mg, 1.62 mmol), Mn(152.57 mg, 2.78 mmol) and trifluoroacetic acid (10.56 mg, 92.57 µmol) were added. The reaction mixture was heated to 60°C, and stirred under a nitrogen atmosphere to allow for reacting for 12.0 hours. The reaction mixture was quenched by adding ammonia water (10 mL), diluted with ethyl acetate (20 mL x) and then filtered, and the filter cake was washed with ethyl acetate. The organic phase of the filtrate was washed with water, dried over anhydrous sodium sulfate, and concentrated under a reduced pressure. The resulting residues were purified by the HPLC (mobile phase: acetonitrile, water (0.1% ammonia water), elution gradient: 31%-61%), to obtain the title Compound **491f** (60 mg, 121.07 µmol, yield: 13.08%). The crude product was directly used in the next step of reaction without purification.
MS m/z (ESI): 496.2 [M+1]
¹H NMR (400 MHz, CDCl₃) δ 7.81 (dd, J = 5.3, 8.6 Hz, 1H), 7.61 (s, 1H), 7.45 - 7.40 (m, 1H), 7.26 - 7.11 (m, 2H), 4.29 - 4.21 (m, 2H), 4.19 - 4.09 (m, 2H), 4.08 - 3.98 (m, 1H), 3.86 - 3.75 (m, 1H), 3.43 - 3.15 (m, 2H), 2.67 (s, 3H), 1.46 (s, 9H), 1.26 - 1.02 (m, 9H).

### Step 6

### 2-[6-(azacyclobutan-3-yl)-3-methylimidazo[1,5-a]pyrazin-8-yl]-N-ethyl-5-fluoro-N-(isopropyl)benzamide 491g

Compound **491f** (80 mg, 161.42 µmol) was dissolved in dichloromethane (3.0 mL), followed by the addition of trifluoroacetic acid (1.48 g, 12.98 mmol, 1 mL) at 0°C. The mixture was stirred at room temperature to allow for reacting for 1.0 hours. The reaction mixture was concentrated under a reduced pressure, dissolved by adding dichloromethane (10 mL), regulated to PH = 10 with 10% aqueous sodium hydroxide solution, and extracted with dichloromethane/isopropanol (10/1, 10 mL x 5), and the organic phases were combined, dried over anhydrous sodium sulfate, and concentrated under a reduced pressure, to obtain the crude product of title Compound **491g** (51 mg). The crude product was directly used in the next step of reaction without purification.
MS m/z (ESI): 396.6 [M+1]

### Step 7

### 2-(6-{1-[1-(1,3-dioxolan-2-yl)-4-methylpentan-3-yl]azacyclobutan-3-yl}-3-methylimidazo[1,5-a]pyrazin-8-yl)-N-ethyl-5-fluoro-N-(isopropyl)benzamide 491h

The crude product of Compound **491g** (51 mg) and 1-(1,3-dioxolan-2-yl)-4-methylpentan-3-one **181a** (28.74 mg, 141.86 µmol) were dissolved in methanol (5 mL), followed by the addition of ZnCl₂ (35.15 mg, 257.92 µmol), and the mixture was stirred at 50°C for 1.0 hour. The reaction mixture was cooled to room temperature, followed by adding sodium cyanoborohydride (24.31 mg, 386.88 µmol) and stirring at 50°C for 11 hours. A saturated aqueous sodium bicarbonate solution (10 mL) was added to the reaction mixture, which was then extracted with dichloromethane (10 mL x 3). The organic phases were combined, dried over anhydrous sodium sulfate, and concentrated under a reduced pressure. The resulting residues were purified by the silica-gel column chromatography using dichloromethane/methanol as an elution system, to obtain the title Compound **4191h** (44 mg, 79.75 µmol, yield: 61.84%).
MS m/z (ESI): 552.4 [M+1]

### Step 8

### N-ethyl-5-fluoro-2-{3-methyl-6-[1-(2-methyl-6-oxohexan-3-yl)azacyclobutan-3-yl]imidazo[1,5-a]pyrazin-8-yl}-N-(isopropyl)benzamide 491i

Compound **491h** (44 mg, 79.75 µmol) was dissolved in acetonitrile (3.0 mL), followed by the addition of a hydrochloric acid solution (1.0 M; 1.0 mL). The mixture was stirred at 50°C to allow for reacting for 1.0 hours. The reaction mixture was concentrated under a reduced pressure, followed by adding dichloromethane (5 mL) and water (5 mL), regulating to pH = 10 with a 10 % aqueous NaOH solution and extracting with dichloromethane (15 mL x 3). The organic phases were combined, dried over anhydrous sodium sulfate, and concentrated under a reduced pressure, to obtain the crude product of title Compound **491i** (40 mg, 78.80 µmol, yield: 98.8%). The crude product was directly used in the next step of reaction without purification.
MS m/z (ESI): 508.4 [M+1]

### Step 9

### N-ethyl-5-fluoro-2-[6-(1-{6-[4-(2-hydroxyethyl)piperazin-1-yl]-2-methylhexan-3-yl}azacyclobutan-3-yl)-3-methylimidazo[1,5-a]pyrazin-8-yl]-N-(isopropyl)benzamide 491

The crude product of Compound **491i** (40 mg, 78.80 µmol) and 2-(piperazin-1-yl)ethanol (15.39 mg, 118.19 µmol) were dissolved in methanol (2 mL). Acetic acid (4.73 mg, 78.80 µmol) was added, and the mixture was stirred at 35°C for 1.0 hour. Sodium cyanoborohydride (14.86 mg, 236.39 µmol) was added, allowing for reacting at 35°C for 11 hours. A saturated aqueous sodium carbonate solution (20 mL) was added to the reaction mixture, which was then extracted with dichloromethane (10 mL x 3). The organic phases were combined, dried over anhydrous sodium sulfate, and concentrated under a reduced pressure. The obtained crude product was purified by the reverse-phase C18 column chromatography (mobile phase: acetonitrile, water, elution gradient: 0%-100%). The title Compound **491** (9.33 mg, 15.00 µmol, yield: 19.04%) was obtained as yellow gel.
MS m/z (ESI):622.3 [M+1]
¹H NMR (400 MHz, CDCl₃) δ 7.83 (br s, 1H), 7.65 (br d, J = 4.3 Hz, 1H), 7.52 (s, 1H), 7.27 - 7.07 (m, 2H), 4.64 - 4.46 (m, 2H), 4.22 - 3.78 (m, 4H), 3.73 - 3.58 (m, 3H), 3.21 (m, 1H), 2.98 - 2.78 (m, 1H), 2.69 (s, 3H), 2.53 (m, 7H), 2.34 (m, 2H), 2.17 - 1.81 (m, 8H), 1.45 (m, 1H), 1.33 - 1.22 (m, 2H), 0.93 (m, 11H), 0.70 - 0.40 (m, 2H).

### Examples 504-1 and 504-2

### 5-fluoro-2-(6-{1-[(3S)-6-[(3R)-3-(methoxymethyl)piperazin-1-yl]-2-methylhexan-3-yl]azacyclobutan-3-yl}-3-methylimidazo[1,5-a]pyridin-8-yl)-N,N-di(isopropyl)benzamide 504-1

### 5-fluoro-2-(6-{1-[(3R)-6-[(3R)-3-(methoxymethyl)piperazin-1-yl]-2-methylhexan-3-yl]azacyclobutan-3-yl}-3-methylimidazo[1,5-a]pyridin-8-yl)-N,N-di(isopropyl)benzamide 504-2

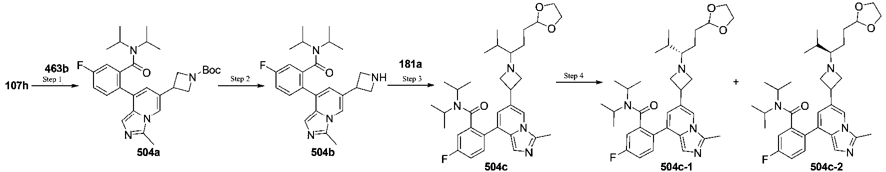

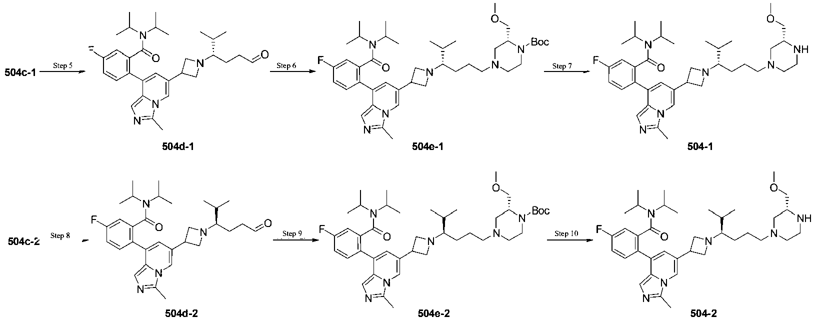

### Step 1

### 3-(8-{2-[di(isopropyl)aminoformyl]-4-fluorophenyl}-3-methylimidazo[1,5-a]pyridin-6-yl)azacyclobutan-1-tert-butyl carboxylate 504a

Compound **107h** (700 mg, 2.18 mmol), Compound **463b**(1.40 g, 2.61 mmol) and potassium phosphate (1.15 g, 5.44 mmol) were dissolved in dioxane (10.0 mL) and water (2.0 mL). Xphos Pd G4 (56.15 mg, 65.26 µmol) was added. Under a nitrogen atmosphere, the mixture was stirred at 90°Cto allow for reacting for 1.0 hours. The reaction mixture was cooled to room temperature, water (15 mL) was added, and the mixture was extracted with ethyl acetate (20 mL x 3). The organic phases were combined, dried over anhydrous sodium sulfate, and concentrated under a reduced pressure. The resulting residues were purified by the preparative column chromatography using dichloromethane/ethyl acetate as an elution system, to obtain the title Compound **504a** (950 mg, 1.87 mmol, yield: 85.86%).
MS m/z (ESI): 509.4[M+1]

### Step 2

### 2-[6-(azacyclobutan-3-yl)-3-methylimidazo[1,5-a]pyridin-8-yl]-5-fluoro-N,N-di(isopropyl)benzamide 504b

Compound **504a** (950 mg, 1.87 mmol) was dissolved in methane dioxide (10.0 mL), followed by the addition of trifluoroacetic acid (4.44 g, 38.94 mmol, 3 mL) at 0°C. The mixture was stirred at room temperature to allow for reacting for 1.0 hours. The mixture was concentrated under a reduced pressure, followed by the addition of dichloromethane (20 mL), and the mixture was regulated to pH > 7 with a saturated aqueous potassium carbonate solution and extracted with dichloromethane (20 mL x 3). The organic phases were combined, dried over anhydrous sodium sulfate, and concentrated under a reduced pressure, to obtain the crude product of title Compound **504b** (750 mg, 1.84 mmol, yield: 98.3%). The crude product was directly used in the next step of reaction without purification. MS m/z (ESI): 409.2 [M+1]

### Step 3

### 2-(6-{1-[1-(1,3-dioxolan-2-yl)-4-methylpentan-3-yl]azacyclobutan-3-yl}-3-methylimidazo[1,5-a]pyridin-8-yl)-5-fluoro-N,N-di(isopropyl)benzamide 504c

Compound **504b** (750 mg, 1.84 mmol) and 1-(1,3-dioxolan-2-yl)-4-methylpentan-3-one **181a** (474.28 mg, 2.75 mmol) were dissolved in methanol (10.0 mL), followed by the addition of acetic acid (220.50 mg, 3.67 mmol), and the mixture was stirred at 50°C for 0.5 hours. The mixture was cooled to room temperature, followed by adding sodium cyanoborohydride (346.12 mg, 5.51 mmol) and stirring at 50°C for 1.5 hour. A saturated aqueous sodium bicarbonate solution (10 mL) was added to the reaction mixture, which was then extracted with dichloromethane (15 mL x 3). The organic phases were combined, dried over anhydrous sodium sulfate, and concentrated under a reduced pressure. The resulting residues were purified by the silica-gel column chromatography using ethyl acetate/methanol as an elution system, to obtain the title Compound **504c** (900 mg, 1.59 mmol, yield: 86.80%)
MS m/z (ESI): 565.5 [M+1]

### Step 4

### 2-(6-{1-[(3S)-1-(1,3-dioxolan-2-yl)-4-methylpentan-3-yl]azacyclobutan-3-yl}-3-methylimidazo[1,5-a]pyridin-8-yl)-5-fluoro-N,N-di(isopropyl)benzamide 504c-1

### 2-(6-{1-[(3R)-1-(1,3-dioxolan-2-yl)-4-methylpentan-3-yl]azacyclobutan-3-yl}-3-methylimidazo[1,5-a]pyridin-8-yl)-5-fluoro-N,N-di(isopropyl)benzamide 504c-2

Compound **504c** (900 mg, 1.59 mmol) was separated and purified by the chiral preparative SFC (preparative column: DAICEL CHIRALCEL OD (250 mm * 30 mm, 10 um), mobile phase: carbon dioxide-isopropanol (0.1% ammonia water); elution gradient: 30%), to obtain the title Compounds **504c-1** (400 mg, 708.30 µmol, yield: 44.4%) and **504c-2** (400 mg, 708.30 µmol, yield: 44.4%).

### Single-configuration Compound 504c-1:

MS m/z (ESI):565.5 [M+1]
Chiral HPLC analysis: retention time: 1.383 min; chromatographic column: Chiralcel OD-3 50 x 4.6 mm I.D., 3 um; mobile phase: carbon dioxide-isopropanol (0.05% diethylamide ): elution gradient: 5%-40%.

### Single-configuration Compound 504c-2:

MS m/z (ESI):565.4 [M+1]
Chiral HPLC analysis: retention time: 1.664 min; chromatographic column: Chiralcel OD-3 50 x 4.6 mm I.D., 3 um; mobile phase: carbon dioxide-isopropanol (0.05% diethylamide ): elution gradient: 5%-40%.

### Step 5

### 5-fluoro-2-(3-methyl-6-{1-[(3S)-2-methyl-6-oxohexan-3-yl]azacyclobutan-3-yl}imidazo[1,5-a]pyridin-8-yl)-N,N-di(isopropyl)benzamide 504d-1

Compound **504c-1** (200 mg, 354.15 µmol) was dissolved in acetonitrile (6.0 mL), followed by the addition of a hydrochloric acid solution (1.0 M; 2.0 mL). The mixture was stirred at 50°C to allow for reacting for 1.0 hours. The reaction mixture was concentrated under a reduced pressure, regulated to pH = 10 with a 10 % aqueous NaOH solution, and extracted with dichloromethane (15 mL x 3). The organic phases were combined, dried over anhydrous sodium sulfate, and concentrated under a reduced pressure, to obtain the crude product of title Compound **504d-1** (184 mg, 353.38 µmol,yield: 99.78%). The crude product was directly used in the next step of reaction without purification.

### Single-configuration Compound 504d-1:

MS m/z (ESI): 521.4 [M+1]

### Step 6

### (2R)-4-[(4S)-4-[3-(8-{2-[di(isopropyl)aminoformyl]-4-fluorophenyl}-3-methylimidazo[1,5-a]pyridin-6-yl)azacyclobutan-1-yl]-5-methylhexyl]-2-(methoxymethyl)piperazin-1-tert-butyl carboxylate 504e-1

The crude product of Compound **504d-1** (184 mg, 353.38 µmol) and (2*R*)-2-(methoxymethyl)piperazin-1-tert-butyl carboxylate (81.39 mg, 353.38 µmol) were dissolved in methanol (5.0 mL), followed by the addition of acetic acid (2.12 mg, 35.34 µmol), and the mixture was stirred at 40°C for 1.0 hour. Sodium cyanoborohydride (66.62 mg, 1.06 mmol) was added, allowing for reacting 40°C for 12.0 hours under the protection of nitrogen. A saturated aqueous sodium bicarbonate solution (10 mL) was added to the reaction mixture, which was then extracted with dichloromethane (15 mL x 3). The organic phases were combined, dried over anhydrous sodium sulfate, and concentrated under a reduced pressure, to obtain the crude product of title Compound **504e-1** (259 mg, 352.39 µmol, yield: 99.72%). The crude product was directly used in the next step of reaction without purification.

### Single-configuration Compound 504e-1:

MS m/z (ESI):735.4 [M+1]

### Step 7

### 5-fluoro-2-(6-{1-[(3S)-6-[(3R)-3-(methoxymethyl)piperazin-1-yl]-2-methylhexan-3-yl]azacyclobutan-3-yl}-3-methylimidazo[1,5-a]pyridin-8-yl)-N,N-di(isopropyl)benzamide 504-1

Compound **504e-1** (259 mg, 352.39 µmol) was dissolved in dichloromethane (3.0 mL), followed by the addition of trifluoroacetic acid (1.48 g, 12.98 mmol, 1 mL) at 0°C. The mixture was stirred at room temperature to allow for reacting for 2.0 hours. The reaction mixture was concentrated under a reduced pressure, dissolved by adding dichloromethane (20 mL), regulated to PH = 10 with a 10% aqueous sodium hydroxide solution, and extracted with dichloromethane/isopropanol (10/1, 20 mL x 5), and the organic phases were combined, dried over anhydrous sodium sulfate, and concentrated under a reduced pressure. The resulting residues were purified by the preparative thin-layer chromatography using dichloromethane/methanol as an elution system, to obtain the title Compound **504-1** (46.44 mg, 73.15 µmol, yield: 20.76%).
MS m/z (ESI): 635.3 [M+1]
Chiral HPLC analysis: retention time: 1.128 min; chromatographic column: Chiralcel OD-3 50 x 4.6 mm I.D., 3 um; mobile phase: carbon dioxide-methanol (0.05% diethylamide): elution gradient: 5%-40%.

¹H NMR (400 MHz, DMSO-*d₆*) δ 7.92 (d, J = 4.4 Hz, 1H), 7.66 (dd, J = 5.6, 8.8 Hz, 1H), 7.35 (dt, J = 2.8, 8.8 Hz, 1H), 7.23 (dd, J = 2.8, 8.9 Hz, 1H), 7.01 (s, 1H), 6.84 - 6.71 (m, 1H), 4.49 - 3.35 (m, 6H), 3.30 - 2.96 (m, 9H), 2.80 - 2.61 (m, 4H), 2.58 (d, J = 1.2 Hz, 3H), 2.23 - 2.14 (m, 2H), 2.04 - 1.97 (m, 1H), 1.87 - 1.79 (m, 1H), 1.74 - 1.65 (m, 1H), 1.62 - 1.53 (m, 1H), 1.49 - 1.34 (m, 5H), 1.28 - 1.04 (m, 6H), 0.92 - 0.89 (m, 2H), 0.87 - 0.79 (m, 6H), 0.22 (br d, J = 6.4 Hz, 2H).

### Step 8

### 5-fluoro-2-(3-methyl-6-{1-[(3R)-2-methyl-6-oxohexan-3-yl]azacyclobutan-3-yl}imidazo[1,5-a]pyridin-8-yl)-N,N-di(isopropyl)benzamide 504d-2

Compound **504c-2** (200 mg, 354.15 µmol) was dissolved in acetonitrile (2.0 mL), followed by the addition of a hydrochloric acid solution (1.0 M; 2.0 mL). The mixture was stirred at 50°C to allow for reacting for 1.0 hours. The reaction mixture was concentrated under a reduced pressure, regulated to pH = 10 with a saturated aqueous potassium carbonate solution, and extracted with dichloromethane (15 mL x 3). The organic phases were combined, dried over anhydrous sodium sulfate, and concentrated under a reduced pressure, to obtain the crude product of title Compound **504d-2** (180 mg, 345.70 µmol,yield: 97.61%). The crude product was directly used in the next step of reaction without purification.

### Single-configuration Compound 504d-2:

MS m/z (ESI): 521.3 [M+1]

### Step 9

### (2R)-4-[(4R)-4-[3-(8-{2-[di(isopropyl)aminofonnyl]-4-fluorophenyl}-3-methylimidazo[1,5-a]pyridin-6-yl)azacyclobutan-1-yl]-5-methylhexyl]-2-(methoxymethyl)piperazin-1-tert-butyl carboxylate 504e-2

The crude product of Compound **504d-2** (200 mg, 384.11 µmol) and (2*R*)-2-(methoxymethyl)piperazin-1-tert-butyl carboxylate (97.31 mg, 422.52 µmol) were dissolved in methanol (3.0 mL), followed by the addition of acetic acid (34.60 mg, 576.17 µmol), and the mixture was stirred at 50°C for 0.5 hour. Sodium cyanoborohydride (72.42 mg, 1.15 mmol) was added, allowing for reacting 50°C for 12.0 hours under the protection of nitrogen. Ethyl acetate (20 mL) was added to the reaction mixture, which was then washed with water (20 mL x 3). The organic phases were combined, dried over anhydrous sodium sulfate, and concentrated under a reduced pressure, to obtain the crude product of title Compound **504e-2** (200 mg, 272.12 µmol, yield: 70.84%). The crude product was directly used in the next step of reaction without purification.

### Single-configuration Compound 504e-2:

MS m/z (ESI):735.5 [M+1]

### Step 10

### 5-fluoro-2-(6-{1-[(3R)-6-[(3R)-3-(methoxymethyl)piperazin-1-yl]-2-methylhexan-3-yl]azacyclobutan-3-yl}-3-methylimidazo[1,5-a]pyridin-8-yl)-N,N-di(isopropyl)benzamide 504-2

Compound **504e-2** (200 mg, 272.12 µmol) was dissolved in dichloromethane (2.0 mL), followed by the addition of trifluoroacetic acid (740.00 mg, 6.49 mmol, 0.5 mL) at 0°C. The mixture was stirred at room temperature to allow for reacting for 0.5 hours. The reaction mixture was concentrated under a reduced pressure, followed by adding a saturated aqueous sodium bicarbonate solution (30 mL) and extracting with dichloromethane (30 mL x 3), and the organic phases were combined, dried over anhydrous sodium sulfate, and concentrated under a reduced pressure. The resulting residues were purified by the preparative thin-layer chromatography using dichloromethane/methanol as an elution system, to obtain the title Compound **504-2** (58.29 mg, 91.81 µmol, yield: 33.74%) as yellow solid.
MS m/z (ESI): 635.5 [M+1]
Chiral HPLC analysis: retention time: 1.185 min; chromatographic column: Chiralcel OD-3 50 x 4.6 mm I.D., 3 um; mobile phase: carbon dioxide-methanol (0.05% diethylamide): elution gradient: 5%-40%.

¹H NMR (400 MHz, DMSO-*d₆*) δ 7.92 (d, J = 3.6 Hz, 1H), 7.66 (dd, J = 5.6, 8.4 Hz, 1H), 7.35 (dt, J = 2.4, 8.8 Hz, 1H), 7.23 (dd, J = 2.8, 8.8 Hz, 1H), 7.02 (s, 1H), 6.83 - 6.73 (m, 1H), 3.61 - 3.41 (m, 5H), 3.28 - 3.19 (m, 7H), 3.09 - 3.01 (m, 1H), 2.88 - 2.77 (m, 2H), 2.72 - 2.62 (m, 3H), 2.59 (s, 3H), 2.25 - 2.15 (m, 2H), 2.04 - 1.98 (m, 1H), 1.94 - 1.84 (m, 1H), 1.74 - 1.59 (m, 2H), 1.49 - 1.39 (m, 2H), 1.37 (d, J = 6.8 Hz, 3H), 1.23 - 1.13 (m, 2H), 1.07 (d, J = 6.4 Hz, 3H), 0.91 (d, J = 6.4 Hz, 3H), 0.87 - 0.80 (m, 6H), 0.22 (d, J = 6.4 Hz, 3H).

The following compounds were prepared and characterized according to the general synthetic scheme described herein and similar steps to those in the aforementioned embodiments.

| **Exa mple** | **Structure** | **MS m/z (ESI) [M+H]⁺** | **H NMR Spectroscopy** |
|---|---|---|---|
| **7** | 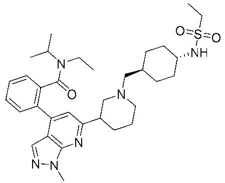 | 609.4 | ¹H NMR (400 MHz, DMSO-*d₆*) δ 8.75 (d, *J =* 134.4 Hz, 1H), 8.03 (s, 1H), 7.62 (m, 3H), 7.47 (m, 1H), 7.21 (m, 1H), 7.07 (dd, *J* = 10.8, 6.1 Hz, 1H), 4.10 (d, *J* = 25.6 Hz, 3H), 3.65 (dd, *J* = 55.2, 14.0 Hz, 2H), 3.01 (m, 9H), 1.93 (m, 10H), 0.98 (m, 15H), 0.20 (t, *J* = 6.8 Hz, 2H). |
| **10** | 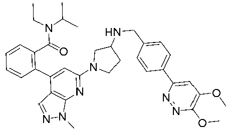 | 635.3 | ¹H NMR (400 MHz, DMSO-*d₆*) δ 8.10 (d, *J =* 7.6 Hz, 2H), 7.71 - 7.37 (m, 8H), 6.47 - 6.39 (m, 1H), 4.36 - 4.27 (m, 0.31H), 4.11 - 3.91 (m, 7.69H), 3.90 - 3.82 (m, 3H), 3.79 - 3.37 (m, 6H), 2.98 - 2.69 (m, 1H), 2.30 - 1.95 (m, 3H), 1.15 - 0.58 (m, 7H), 0.27 (d, *J* = 6.8 Hz, 2H). |
| **12** | 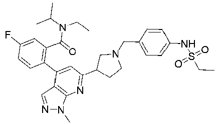 | 607.2 | ¹H NMR (400 MHz, DMSO-*d*₆-D₂O) δ 7.95 (d, *J* = 2.4 Hz, 1H), 7.73 - 7.61 (m, 1H), 7.45 (td, *J* = 8.4, 2.4 Hz, 1H), 7.41 - 7.25 (m, 3H), 7.21 - 7.07 (m, 3H), 4.21 - 4.14 (m, 0.24H), 4.04 (s, 3H), 3.63 (s, 3H), 3.40 - 3.19 (m, 2H), 3.05 (d, *J* = 7.2 Hz, 3H), 2.92 - 2.81 (m, 1H), 2.76 (dd, *J* = 15.2, 8.0 Hz, 1H), 2.69 (t, *J* = 9.6 Hz, 2H), 2.26 (s, 1H), 2.13 (dd, *J =* 12.4, 6.8 Hz, 1H), 1.18 (t, *J* = 7.2 Hz, 3H), 0.88 (t, *J* = 6.0 Hz, 3H), 0.79 - 0.59 (m, 3H), 0.16 (s, 2H). |
| **17** | 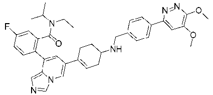 | 649.2 | ¹H NMR (400 MHz, DMSO-*d₆*) δ 8.37 (d, J = 7.2 Hz, 2H), 8.03 (d, J = 8.0 Hz, 2H), 7.75 - 7.67 (m, 1H), 7.60 - 7.56 (m, 1H), 7.53 - 7.48 (m, 2H), 7.42 -7.30 (m, 2H), 7.17 - 7.11 (m, 1H), 6.92 - 6.86 (m, 1H), 6.10 (s, 1H), 4.07 - 4.03 (m, 3H), 3.98 (s, 3H), 3.85 (s, 2H), 3.46 - 3.38 (m, 2H), 2.90 - 2.63 (m, 3H),2.48 - 2.43 (m, 2H), 2.33 (s, 1H), 2.09 - 1.93 (m, 2H), 1.55 - 1.42 (m, 1H), 1.06 - 0.78 (m, 4H), 0.76 - 0.67 (m, 3H), 0.24 (d, J = 6.4 Hz, 2H). |
| **20** | 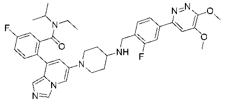 | 670.3 | ¹H NMR (400 MHz, DMSO-*d₆*) δ 8.24 (s, 1H), 7.98 - 7.87 (m, 2H), 7.78 (s, 1H), 7.71 - 7.61 (m, 3H), 7.41 - 7.28 (m, 2H), 7.07 - 7.02 (m, 1H), 6.68 - 6.61 (m, 1H), 4.06 (s, 3H), 3.99 (s, 3H), 3.86 (s, 2H), 3.49 - 3.39 (m, 3H), 2.99 - 2.86 (m, 1H), 2.84 - 2.55 (m, 4H), 2.01 - 1.90 (m, 2H), 1.52 - 1.38 (m, 2H), 0.93 - 0.71 (m, 7H), 0.33 - 0.29 (m, 2H). |
| **28** | 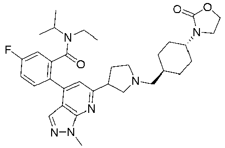 | 591.3 | ¹H NMR (400 MHz, DMSO-*d₆*) δ 7.95 (s, 1H), 7.73 - 7.61 (m, 1H), 7.49 - 7.34 (m, 2H), 7.13 (s, 1H), 4.27 - 4.19 (m, 2H), 4.04 (s, 3H), 3.59 - 3.53 (m, 1H), 3.47 (m, 2H), 3.40 - 3.35 (m, 2H), 3.07 - 2.57 (m, 6H), 2.41 - 1.13 (m, 12H), 1.05 - 0.59 (m, 8H), 0.21 (dd, J = 6.0, 3.6 Hz, 2H). |
| **32** | 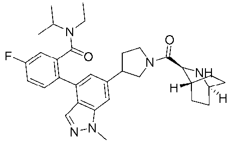 | 546.3 | ¹H NMR (400 MHz, DMSO-*d₆*) δ 8.36 (s, 1H), 7.90 - 7.74 (m, 1H), 7.64 - 7.50 (m, 2H), 7.38 (t, J = 8.4 Hz, 1H), 7.28 (t, J = 10.4 Hz, 1H), 7.11 - 6.95 (m, 1H), 4.30 - 3.94 (m, 5H), 3.94 - 3.74 (m, 1H), 3.71 - 3.57 (m, 2H), 3.43 - 3.31 (m, 2H), 3.23 (dd, J = 25.6, 11.2 Hz, 2H), 2.89 - 2.75 (m, 1H), 2.30 (d, J = 21.6 Hz, 1H), 2.08 (d, J = 18.8 Hz, 2H), 1.78 (d, J = 18.4 Hz, 3H), 1.63 (d, J = 7.2 Hz, 2H), 1.57 - 1.37 (m, 3H), 1.05 - 0.54 (m, 7H), 0.05 (d, J = 6.4 Hz, 2H). |
| **37** | 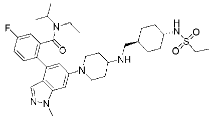 | 641.3 | ¹H NMR (400 MHz, DMSO-*d₆*) δ 8.24 (s, 1.73H), 7.70 (s, 1H), 7.62 - 7.56 (m, 1H), 7.38 (td, J = 8.8, 2.8 Hz, 1H), 7.33 - 7.26 (m, 1H), 7.03 (d, J = 7.6 Hz, 1H), 6.97 (s, 1H), 6.88 (d, J = 2.0 Hz, 1H), 3.97 (s, 3H), 3.87 (d, J = 12.0 Hz, 2H), 3.40 - 3.20 (m, 2H), 3.09 - 2.95 (m, 3H), 2.88 - 2.75 (m, 4H), 2.48 - 2.37 (m, 2H), 2.09 (d, J = 11.6 Hz, 2H), 1.91 (d, J = 10.4 Hz, 2H), 1.79 (d, J = 11.6 Hz, 2H), 1.67 - 1.48 (m, 3H), 1.28 -1.15 (s, 5H), 1.05 (q, J = 12.4 Hz, 2.7H), 0.87 (d, J = 6.8 Hz, 2.6H), 0.75 (t, J = 7.2 Hz, 2.8H), 0.65 (t, J = 7.2 Hz, 0.6H), 0.13 (t, J = 7.6 Hz, 2.2H). |
| **41** | 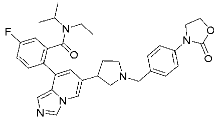 | 570.3 | ¹H NMR (400 MHz, DMSO-*d₆*) δ 8.36 (s, 1H), 8.26 - 8.12 (m, 2H), 7.71 - 7.62 (m, 1H), 7.54 - 7.46 (m, 2H), 7.42 - 7.29 (m, 4H), 7.11 (s, 1H), 6.75 - 6.65 (m, 1H), 4.48 - 4.39 (m, 3H), 4.07 - 4.00 (m, 3H), 3.67 - 3.58 (m, 2H), 3.49 - 3.37 (m, 1H), 3.29 - 3.23 (m, 1H), 2.96 - 2.83 (m, 2H), 2.74 - 2.64 (m, 2H), 2.28 - 2.12 (m, 1H), 1.90 - 1.71 (m, 1H), 1.07 - 0.68 (m, 7H), 0.31 - 0.19 (m, 2H). |
| **43** | 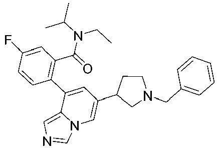 | 485.3 | ¹H NMR (400 MHz, CDCl₃) δ 8.10 (d, J = 3.6 Hz, 1H), 7.84 (m, J = 4.8 Hz, 1H), 7.68 - 7.62 (m, 1H), 7.43 (br s, 1H), 7.38 (br s, 1H), 7.34 (m, J = 8.8 Hz, 1H), 7.30 (m, J = 9.2 Hz, 1H), 7.25 (s, 1H), 7.24 - 7.16 (m, 2H), 7.14 (m, J = 11.2 Hz, 1H), 6.83 (m, J = 4.0 Hz, 1H), 3.77 (m, J = 2.96 Hz, 2H), 3.44 - 3.28 (m, 2H), 3.03 - 2.94 (m, 2H), 2.89 - 2.77 (m, 1H), 2.73 - 2.49 (m, 1H), 2.41 - 2.27 (m, 1H), 2.05 - 1.85 (m, 1H), 1.27 (br s, 1H), 0.98 - 0.91 (m, 5H), 0.78 (br t, J = 2.2 Hz, 2H), 0.24 (t, J = 3.6 Hz, 3H). |
| **44** | 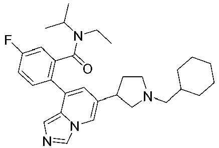 | 491.4 | ¹H NMR (400 MHz, CDCl₃) δ 8.06 (br s, 1H), 7.93 - 7.73 (m, 1H), 7.62 - 7.55 (m, 1H), 7.17 - 7.09 (m, 2H), 7.08 - 7.02 (m, 1H), 6.76 - 6.68 (m, 1H), 3.43 (m, J = 2.56 Hz, 2H), 3.36 - 3.26 (m, 1H), 3.00 - 2.86 (m, 2H), 2.84 - 2.76 (m, 1H), 2.68 - 2.59 (m, 1H), 2.46 - 2.35 (m, 1H), 1.96 - 1.79 (m, 3H), 1.73 - 1.65 (m, 3H), 1.64 - 1.58 (m, 3H), 1.22 - 1.17 (m, 2H), 1.16 - 1.13 (m, 1H), 1.09 - 1.05 (m, 1H), 0.93 - 0.84 (m, 7H), 0.73 - 0.68 (m, 1H), 0.19 (d, J = 6.8 Hz, 3H). |
| **45** | 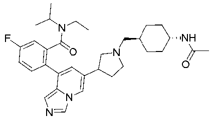 | 548.5 | ¹H NMR (400 MHz, DMSO-*d₆*) δ 8.35 (s, 1H), 8.26 - 8.20 (m, 1H), 7.73 - 7.62 (m, 2H), 7.39 (q, J = 8.4 Hz, 1H), 7.34 - 7.29 (m, 1H), 7.13 - 7.09 (m, 1H), 6.72 - 6.65 (m, 1H), 3.56 - 3.35 (m, 6H), 3.31 - 3.16 (m, 2H), 3.01 - 2.76 (m, 2H), 2.71 - 2.63 (m, 1H), 2.62 - 2.53 (m, 2H), 2.43 - 2.31 (m, 1H), 2.27 - 2.13 (m, 2H), 1.83 - 1.78 (m, J = 4.4 Hz, 2H), 1.57 - 1.43 (m, 1H), 1.40 - 1.30 (m, 1H), 1.27 - 1.02 (m, 3H), 0.90 (d, J = 5.6 Hz, 5H), 0.80 - 0.73 (m, 3H), 0.28 (br dd, J = 6.8, 7.2 Hz, 2H). |
| **46** | 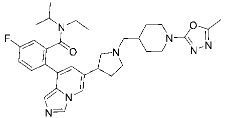 | 574.4 | ¹H NMR (400 MHz, DMSO-*d*₆) δ 8.36 (s, 1H), 8.27 - 8.20 (m, 1H), 8.16 (s, 0.3H), 7.72 - 7.62 (m, 1H), 7.43 - 7.27 (m, 2H), 7.15 - 7.07 (m, 1H), 6.75 - 6.66 (m, 1H), 3.81 - 3.74 (m, 2H), 3.47 - 3.40 (m, 1H), 3.33 - 3.21 (m, 2H), 2.99 - 2.81 (m, 4H), 2.76 - 2.54 (m, 3H), 2.41 - 2.29 (m, 5H), 2.23 - 2.13 (m, 1H), 1.93 - 1.66 (m, 4H), 1.20 - 1.08 (m, 2H), 0.96 - 0.68 (m, 7H), 0.30 - 0.23 (m, 2H). |
| **47** | 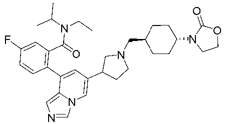 | 576.5 | ¹H NMR (400 MHz, DMSO-*d*₆) δ 8.36 (s, 1H), 8.28 - 8.22 (m, 1H), 8.19 (s, 1H), 7.71 - 7.62 (m, 1H), 7.43 - 7.27 (m, 2H), 7.12 (s, 1H), 6.74 - 6.66 (m, 1H), 4.27 - 4.18 (m, 2H), 3.56 - 3.20 (m, 6H), 3.11 - 2.70 (m, 4H), 2.67 - 2.54 (m, 1H), 2.45 - 2.38 (m, 1H), 2.26 - 2.13 (m, 1H), 1.98 - 0.68 (m, 18H), 0.34 - 0.21 (m, 2H). |
| **49** | 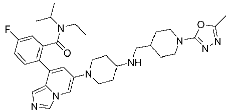 | 603.4 | ¹H NMR (400 MHz, CDCl₃) δ 8.06 (br s, 1H), 7.68 - 7.60 (m, 1H), 7.30 (s, 1H), 7.21 - 7.11 (m, 3H), 6.85 - 6.78 (m, 1H), 4.03 - 3.94 (m, 3H), 3.59 - 3.41 (m, 4H), 3.04 - 2.89 (m, 4H), 2.75 - 2.55 (m, 7H), 2.27 - 2.18 (m, 1H), 2.07 - 1.95 (m, 4H), 1.02 - 0.76 (m, 10H), 0.33 - 0.24 (m, 3H). |
| **63** | 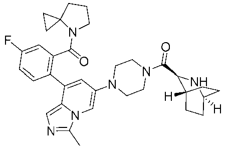 | 571.5 | ¹H NMR (400 MHz, METHANOL-d4) δ 7.74 - 7.69 (m, 1H), 7.38 - 7.33 (m, 3H), 7.15 (s, 1H), 6.80 - 6.76 (m, 1H), 4.56 - 4.54 (m, 1H), 3.74 - 3.66 (m, 3H), 3.61 - 3.46 (m, 3H), 3.24 - 3.21 (m, 4H), 2.71 - 2.67 (m, 4H), 2.23 - 2.16 (m, 3H), 1.88 - 1.84 (m, 2H), 1.63 - 1.59 (m, 3H), 1.34 - 1.32 (m, 6H), 0.92 - 0.89 (m, 3H). |
| **64** | 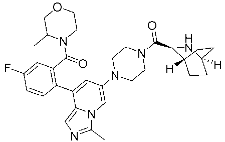 | 575.2 | ¹H NMR (400 MHz, DMSO-d6) δ = 7.86 - 7.58 (m, 1H), 7.51 - 7.26 (m, 3H), 7.09 - 6.88 (m, 1H), 6.71 - 6.44 (m, 1H), 4.63 - 4.00 (m, 1H), 3.99 - 3.86 (m, 1H), 3.80 - 3.42 (m, 6H), 3.25 - 2.72 (m, 9H), 2.62 - 2.54 (m, 3H), 1.93 - 1.42 (m, 9H), 1.21 - 0.99 (m, 2H), 0.76 - 0.37 (m, 1H). |
| **66** | 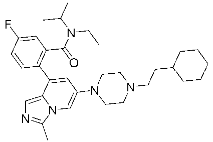 | 534.3 | ¹H NMR (400 MHz, DMSO-*d*₆) δ 7.71 - 7.63 (m, 1H), 7.42 - 7.23 (m, 3H), 7.01 - 6.94 (m, 1H), 6.64 - 6.58 (m, 1H), 3.48 - 3.39 (m, 2H), 3.06 - 2.86 (m, 5H), 2.57 - 2.52 (m, 3H), 2.49 (br s, 4H), 2.38 - 2.30 (m, 2H), 1.73 - 1.57 (m, 5H), 1.37 - 1.10 (m, 7H), 0.96 - 0.79 (m, 8H), 0.31 (br d, J = 6.5 Hz, 2H). |
| **74** | 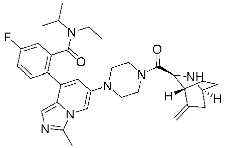 | 573.3 | ¹H NMR (400 MHz, DMSO-*d*₆) δ 7.70 - 7.61 (m, 1H), 7.40 - 7.29 (m, 2H), 7.00 - 6.96 (m, 1H), 6.62 (br d, J = 1.3 Hz, 1H), 6.44 - 6.32 (m, 1H), 4.97 - 4.93 (m, 1H), 4.76 - 4.69 (m, 1H), 4.36 (s, 1H), 3.94 (s, 1H), 3.77 - 3.57 (m, 2H), 3.47 - 3.40 (m, 1H), 3.19 - 3.12 (m, 2H), 3.02 (br s, 4H), 2.55 (s, 2H), 2.38 (br s, 1H), 2.34 - 2.19 (m, 2H), 2.15 (s, 1H), 2.08 (s, 1H), 1.72 - 1.51 (m, 3H), 1.49 - 1.39 (m, 2H), 1.35 - 1.27 (m, 1H), 1.09 (t, J = 7.2 Hz, 1H), 0.96 - 0.88 (m, 3H), 0.84 - 0.73 (m, 3H), 0.33 - 0.28 (m, 2H). |
| **78** | 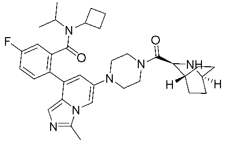 | 587.4 | ¹H NMR (400 MHz, DMSO-*d*₆) δ 7.71 - 7.64 (m, 1H), 7.41 - 7.13 (m, 3H), 7.03 - 6.97 (m, 1H), 6.70 - 6.49 (m, 1H), 3.96 (s, 1H), 3.86 - 3.74 (m, 1H), 3.69 - 3.50 (m, 5H), 3.07 - 2.93 (m, 4H), 2.86 (s, 1H), 2.55 (s, 3H), 2.06 - 1.32 (m, 17H), 1.14 - 1.08 (m, 2H), 0.94 - 0.85 (m, 1H), 0.34 - 0.24 (m, 1H). |
| **81** | 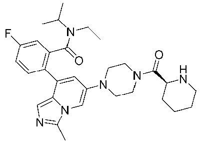 | 535.4 | ¹H NMR (400 MHz, CD₃OD) δ 8.54 (s, 1H), 7.75 - 7.68 (m, 1H), 7.38 - 7.30 (m, 2H), 7.28 - 7.21 (m, 1H), 7.09 (s, 1H), 6.81 - 6.76 (m, 1H), 4.21 - 4.09 (m, 1H), 3.87 - 3.66 (m, 4H), 3.61 - 3.41 (m, 2H), 3.22 - 2.87 (m, 7H), 2.62 (s, 3H), 1.97 - 1.52 (m, 6H), 1.10 - 0.76 (m, 7H), 0.35 - 0.31 (m, 2H). |
| **83** | 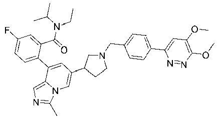 | 637.2 | ¹H NMR (400 MHz, DMSO-*d*₆) δ 7.86 - 7.65 (m, 3H), 7.47 - 7.03 (m, 6H), 6.80 - 6.73 (m, 1H), 6.50 - 6.42 (m, 1H), 3.89 - 3.77 (m, 3H), 3.74 (s, 3H), 3.53 - 3.35 (m, 2H), 3.27 - 3.14 (m, 1H), 3.06 - 2.94 (m, 2H), 2.78 - 2.59 (m, 2H), 2.53 - 2.41 (m, 2H), 2.34 (s, 3H), 2.04 - 1.90 (m, 1H), 1.69 - 1.52 (m, 1H), 1.06 - 0.41 (m, 8H), 0.10 - -0.05 (m, 2H). |
| **84** | 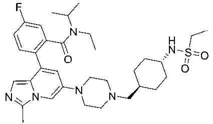 | 627.4 | ¹H NMR (400 MHz, DMSO-*d*₆) δ 7.72 - 7.62 (m, 1H), 7.40 - 7.22 (m, 3H), 7.03 - 6.95 (m, 2H), 6.65 - 6.57 (m, 1H), 3.48 - 3.38 (m, 1H), 3.24 - 3.15 (m, 1H), 3.04 - 2.90 (m, 8H), 2.58 - 2.52 (m, 3H), 2.45 (s, 3H), 2.15 - 2.07 (m, 2H), 1.92 - 1.72 (m, 5H), 1.49 - 1.34 (m, 1H), 1.22 - 1.15 (m, 5H), 0.98 - 0.71 (m, 9H), 0.33 - 0.29 (m, 2H). |
| **85** | 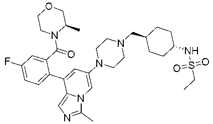 | 641.3 | ¹H NMR (400 MHz,CDCl₃) δ 7.72 - 7.61 (m, 1H), 7.24 - 7.19 (m, 1H), 7.19 - 7.12 (m, 1H), 7.12 - 7.09 (m, 1H), 7.02 - 6.93 (m, 1H), 6.76 (s, 1H), 6.66 - 6.63 (m, 1H), 4.69 - 4.43 (m, 1H), 4.21 - 3.72 (m, 1H), 3.63 - 3.51 (m, 1H), 3.37 - 3.31 (m, 2H), 3.27 - 3.21 (m, 1H), 3.17 - 3.13 (m, J = 4.8 Hz, 1H), 3.05 (q, J = 7.4 Hz, 3H), 2.91 - 2.76 (m, 1H), 2.64 (s, 3H), 2.56 - 2.54 (m, J = 8.4 Hz, 2H), 2.19 (d, J = 7.2 Hz, 2H), 2.13 - 2.07 (m, J = 10.0 Hz, 2H), 1.90 (d, J = 11.6 Hz, 2H), 1.71 - 1.63 (m, 2H), 1.50 - 1.43 (m, 3H), 1.38 (t, J = 7.2 Hz, 3H), 1.31 - 1.26 (m, 2H), 1.25 - 1.20 (m, 1H), 1.10 (d, J = 6.8 Hz, H), 1.02 (t, J = 7.2 Hz, 3H), 0.86 (d, J = 6.8 Hz, 1H). |
| **86** | 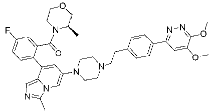 | 680.4 | ¹H NMR (400 MHz, CDCl₃) δ 7.96 (d, J = 7.6 Hz, 2H), 7.75 - 7.64 (m, 1H), 7.38 (d, J = 8.0 Hz, 2H), 7.25 - 7.17 (m, 2H), 7.15 (s, 2H), 7.05 - 6.98 (m, 1H), 6.82 - 6.63 (m, 1H), 4.25 (s, 3H), 4.04 (s, 3H), 3.82 - 3.72 (m, 1H), 3.68 - 3.51 (m, 2H), 3.35 - 3.05 (m, 6H), 3.03 - 2.92 (m, 3H), 2.91 - 2.73 (m, 6H), 2.67 (s, 3H), 1.41 - 1.25 (m, 2H), 1.12 (d, J = 6.4 Hz, 1H), 0.93 - 0.84 (m, 1H). |
| **88** | 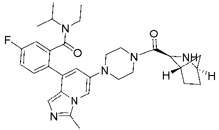 | 547.5 | ¹H NMR (400 MHz, DMSO-*d*₆) δ 7.72 (dd, J = 5.2, 8.4 Hz, 1H), 7.37 - 7.31 (m, 2H), 7.24 (dd, J = 2.8, 8.8 Hz, 1H), 7.09 (s, 1H), 6.81 - 6.78 (m, 1H), 3.92 - 3.81 (m, 3H), 3.78 - 3.67 (m, 3H), 3.56 (td, J = 6.8, 13.6 Hz, 1H), 3.45 (td, J = 6.4, 13.2 Hz, 1H), 3.28 - 3.09 (m, 4H), 3.08 - 2.98 (m, 1H), 2.70 (s, 1H), 2.62 (s, 3H), 1.82 - 1.72 (m, 2H), 1.71 - 1.62 (m, 2H), 1.61 - 1.54 (m, 1H), 1.52 - 1.46 (m, 1H), 1.21 - 1.06 (m, 1H), 1.03 - 0.90 (m, 6H), 0.81 (t, J = 7.0 Hz, 1H), 0.33 (d, J = 6.8 Hz, 2H). |
| **91** | 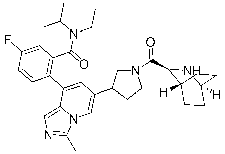 | 546.3 | ¹H NMR (400 MHz, DMSO-*d*₆) δ 8.06 - 7.90 (m, 1H), 7.72 - 7.61 (m, 1H), 7.42 - 7.28 (m, 2H), 7.04 (s, 1H), 6.70 - 6.53 (m, 1H), 4.00 - 3.54 (m, 4H), 3.53 - 3.38 (m, 3H), 2.99 - 2.86 (m, 1H), 2.79 (s, 1H), 2.59 (s, 3H), 2.34 - 1.22 (m, 12H), 1.08 - 0.70 (m, 7H), 0.32 - 0.23 (m, 2H). |
| **92** | 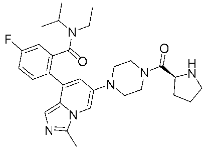 | 521.3 | ¹H NMR (400 MHz, DMSO-*d₆*) δ 7.70 - 7.63 (m, 1H), 7.41 - 7.28 (m, 3H), 7.00 - 6.95 (m, 1H), 6.65 - 6.60 (m, 1H), 3.92 - 3.82 (m, 1H), 3.72 - 3.39 (m, 6H), 3.13 - 2.76 (m, 7H), 2.69 - 2.60 (m, 1H), 2.55 (s, 3H), 2.05 - 1.95 (m, 1H), 1.71 - 1.51 (m, 3H), 1.08 - 0.73 (m, 7H), 0.31 (br d, J = 5.2 Hz, 2H). |
| **95** | 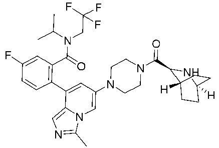 | 615.4 | ¹H NMR (400 MHz, CD₃OD) δ 7.76 - 7.65 (m, 1H), 7.43 - 7.35 (m, 1H), 7.34 (s, 1H), 7.29 (dd, J = 2.4, 2.8 Hz, 1H), 7.11 - 7.01 (m, 1H), 6.79 - 6.67 (m, 1H), 4.31 - 4.14 (m, 1H), 4.08 (s, 1H), 3.93 - 3.84 (m, 1H), 3.84 - 3.70 (m, 3H), 3.67 (s, 2H), 3.19 - 3.09 (m, 4H), 3.08 - 3.02 (m, 1H), 2.63 (s, 3H), 1.97 - 1.82 (m, 4H), 1.80 - 1.72 (m, 2H), 1.67 - 1.56 (m, 3H), 1.17 - 1.06 (m, 1H), 1.00 (d, J = 6.4 Hz, 3H), 0.45 (d, J = 6.4 Hz, 2H). |
| **96** | 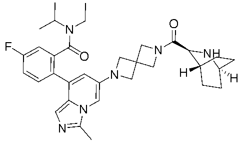 | 573.4 | ¹H NMR (400 MHz, DMSO-*d*₆) δ 7.70-7.62 (m, 1H), 7.40 - 7.28 (m, 2H), 7.05 - 6.94 (m, 2H), 6.27 - 6.18 (m, 1H), 4.46 - 4.26 (m, 2H), 4.15 - 4.02 (m, 2H), 3.95 - 3.88 (m, 4H), 3.55 (s, 1H), 3.47 - 3.39 (m, 1H), 3.31 - 3.29 (m, 1H), 2.99 - 2.88 (m, 1H), 2.78 - 2.74 (m, 1H), 2.52 - 2.51 (m, 3H), 1.76 - 1.66 (m, 3H), 1.60 - 1.37 (m, 6H), 0.94 - 0.72 (m, 7H), 0.33 - 0.27 (m, 2H). |
| **97** | 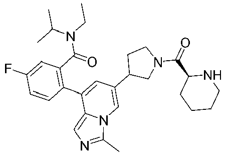 | 520.4 | ¹H NMR (400 MHz, CDCl₃) δ 7.71 - 7.61 (m, 1H), 7.60 - 7.45 (m, 1H), 7.23 - 7.16 (m, 2H), 7.15 - 7.09 (m, 1H), 6.81 - 6.73 (m, 1H), 4.11 - 3.78 (m, 2H), 3 .77 - 3.61 (m, 1H), 3.60 - 3.20 (m, 6H), 3.20 - 3.11 (m, 1H), 3.10 - 2.86 (m, 1H), 2.74 - 2.58 (m, 4H), 2.48 - 2.33 (m, 1H), 2.32 - 2.17 (m, 1H), 2.16 - 2.00 (m, 1H), 1.94 (s, 1H), 1.87 - 1.77 (m, 1H), 1.64 - 1.57 (m, 1H), 1.56 - 1.45 (m, 2H), 1.45 - 1.21 (m, 1H), 1.20 - 1.06 (m, 1H), 1.03 - 0.96 (m, 2H), 0.96 - 0.91 (m, 2H), 0.83 - 0.75 (m, 1H), 0.32 - 0.22 (m, 2H). |
| **99** | 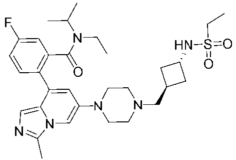 | 599.3 | ¹H NMR (400 MHz, DMSO-*d*₆) δ 7.64 - 7.54 (m, 1H), 7.36 (d, J = 8.4 Hz, 1H), 7.32 - 7.25 (m, 1H), 7.25 - 7.19 (m, 1H), 7.18 (s, 1H), 6.92 - 6.84 (m, 1H), 6.55 - 6.48 (m, 1H), 3.85 - 3.68 (m, 1H), 3.41 - 3.31 (m, 1H), 3.11 - 3.04 (m, 1H), 2.99 - 2.88 (m, 4H), 2.82 (q, J = 7.4 Hz, 3H), 2.46 (s, 4H), 2.36 - 2.32 (m, 2H), 2.02 - 1.93 (m, 4H), 1.59 - 1.40 (m, 1H), 1.29 - 1.14 (m, 1H), 1.09 (t, J = 7.4 Hz, 3H), 0.89 - 0.77 (m, 5H), 0.74 (t, J = 6.8 Hz, 3H), 0.70 - 0.62 (m, 1H), 0.22 (t, J = 8.6 Hz, 2H). |
| **100** | 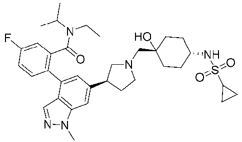 | 640.3 | ¹H NMR (400 MHz, DMSO-*d*₆) δ 8.24 (s, 0.86H), 7.79 (s, 1H), 7.62 - 7.51 (m, 2H), 7.38 (td, J = 8.8, 2.4 Hz, 1H), 7.33 - 7.24 (m, 1H), 7.07 (d, J = 15.6 Hz, 1H), 7.03 - 6.90 (m, 1H), 4.20 (dd, J = 13.2, 6.8 Hz, 0.41H), 4.02 (d, J = 3.2 Hz, 3H), 3.42 - 3.18 (m, 4H), 3.14 - 2.63 (m, 7H), 2.45 (t, J = 10.8 Hz, 1H), 2.22 (dd, J = 8.4, 4.0 Hz, 1H), 1.85 (dt, J = 20.0, 7.6 Hz, 1H), 1.70 - 1.19 (m, 8H), 1.06 - 0.53 (m, 11H), 0.08 (t, J = 6.4 Hz, 2H). |
| **102** | 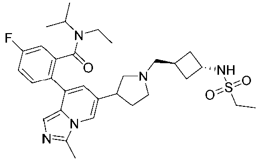 | 584.4 | ¹H NMR (400 MHz, CD₃OD) δ 7.96 - 7.86 (m, 1H), 7.76 - 7.64 (m, 1H), 7.40 - 7.30 (m, 1H), 7.29 - 7.19 (m, 1H), 7.13 (s, 1H), 6.86 - 6.78 (m, 1H), 3.67 - 3.48 (m, 1H), 3.45 - 3.35 (m, 2H), 3.29 - 3.23 (m, 2H), 3.13 - 3.03 (m, 2H), 3.03 - 2.95 (m, 2H), 2.94 - 2.85 (m, 1H), 2.77 - 2.69 (m, 2H), 2.67 (s, 3H), 2.64 - 2.55 (m, 1H), 2.54 - 2.40 (m, 2H), 2.38 - 2.28 (m, 1H), 2.26 - 2.16 (m, 4H), 2.03 - 1.84 (m, 1H), 1.74 - 1.62 (m, 2H), 1.44 (qd, J = 7.2, 14.8 Hz, 2H), 1.36 - 1.26 (m, 3H), 1.05 (t, J = 7.4 Hz, 3H), 0.99 (d, J = 6.8 Hz, 2H), 0.97 - 0.91 (m, 3H), 0.83 (t, J = 7.2 Hz, 1H), 0.37 - 0.31 (m, 2H). |
| **103** | 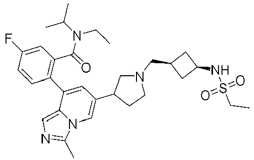 | 584.3 | ¹H NMR (400 MHz, CD₃OD) δ 7.96 - 7.86 (m, 1H), 7.76 - 7.64 (m, 1H), 7.40 - 7.30 (m, 1H), 7.29 - 7.19 (m, 1H), 7.13 (s, 1H), 6.86 - 6.78 (m, 1H), 3.67 - 3.48 (m, 1H), 3.45 - 3.35 (m, 2H), 3.29 - 3.23 (m, 2H), 3.13 - 3.03 (m, 2H), 3.03 - 2.95 (m, 2H), 2.94 - 2.85 (m, 1H), 2.77 - 2.69 (m, 2H), 2.67 (s, 3H), 2.64 - 2.55 (m, 1H), 2.54 - 2.40 (m, 2H), 2.38 - 2.28 (m, 1H), 2.26 - 2.16 (m, 4H), 2.03 - 1.84 (m, 1H), 1.74 - 1.62 (m, 2H), 1.44 (qd, J = 7.2, 14.8 Hz, 2H), 1.36 - 1.26 (m, 3H), 1.05 (t, J = 7.4 Hz, 3H), 0.99 (d, J = 6.8 Hz, 2H), 0.97 - 0.91 (m, 3H), 0.83 (t, J = 7.2 Hz, 1H), 0.37 - 0.31 (m, 2H). |
| **104** | 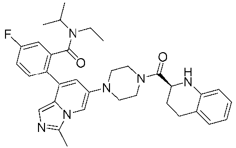 | 583.3 | ¹H NMR (400 MHz, DMSO-*d₆*) δ 7.74 - 7.62 (m, 1H), 7.43 - 7.27 (m, 3H), 7.03 - 6.95 (m, 1H), 6.92 - 6.79 (m, 2H), 6.70 - 6.40 (m, 3H), 5.56 (s, 1H), 4.41 - 4.24 (m, 1H), 3.80 - 3.57 (m, 4H), 3.51 - 3.38 (m, 1H), 3.15 - 2.87 (m, 5H), 2.78 - 2.54 (m, 5H), 2.04 - 1.91 (m, 1H), 1.77 - 1.62 (m, 1H), 1.11 - 0.71 (m, 7H), 0.37 - 0.24 (m, 2H). |
| **105** | 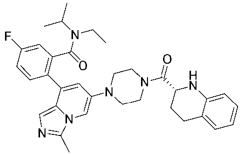 | 583.4 | ¹H NMR (400 MHz, DMSO-*d₆*) δ 7.71 - 7.63 (m, 1H), 7.42 - 7.29 (m, 3H), 7.01 - 6.96 (m, 1H), 6.90 - 6.81 (m, 2H), 6.67 - 6.62 (m, 1H), 6.59 - 6.54 (m, 1H), 6.47 - 6.41 (m, 1H), 5.59 - 5.53 (m, 1H), 4.42 - 4.34 (m, 1H), 3.80 - 3.56 (m, 4H), 3.50 - 3.40 (m, 1H), 3.31 - 3.26 (m, 1H), 3.15 - 2.98 (m, 4H), 2.97 - 2.88 (m, 1H), 2.78 - 2.68 (m, 1H), 2.60 (br s, 1H), 2.59 (br s, 2H), 2.07 (s, 1H), 1.98 (br dd, J = 6.2, 11.3 Hz, 1H), 1.76 - 1.63 (m, 1H), 1.09 - 0.72 (m, 7H), 0.35 - 0.27 (m, 2H). |
| **106** | 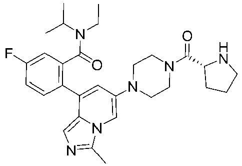 | 521.3 | ¹H NMR (400 MHz, DMSO-*d₆*) δ 8.27 (s, 1H), 7.83 (s, 1H), 7.72 - 7.63 (m, 1H), 7.44 - 7.28 (m, 2H), 7.11 - 7.02 (m, 1H), 6.71 - 6.62 (m, 1H), 3.76 - 3.38 (m, 8H), 3.07 - 2.86 (m, 5H), 2.42 (s, 1H), 1.54 - 1.30 (m, 4H), 1.26 - 0.98 (m, 3H), 0.98 - 0.67 (m, 7H), 0.34 - 0.26 (m, 2H). |
| **109** | 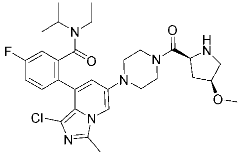 | 585.3 | ¹H NMR (400 MHz, DMSO-*d*₆) δ 7.54 - 7.17 (m, 4H), 6.61 (s, 1H), 3.90 - 3.79 (m, 2H), 3.75 - 3.47 (m, 5H), 3.20 - 2.81 (m, 10H), 2.61 - 2.52 (m, 4H), 2.35 - 2.24 (m, 1H), 1.62 - 1.48 (m, 1H), 1.18 - 0.66 (m, 7H), 0.35 - 0.25 (m, 2H). |
| **111** | 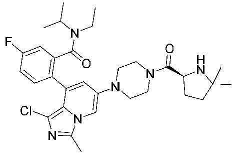 | 583.4 | ¹H NMR (400 MHz, DMSO-*d*₆) δ 7.48 - 7.38 (m, 1H), 7.38 - 7.26 (m, 3H), 6.61 (s, 1H), 4.04 - 3.95 (m, 1H), 3.78 - 3.47 (m, 5H), 3.18 - 2.82 (m, 6H), 2.55 (s, 3H), 2.24 - 2.10 (m, 1H), 1.75 - 1.54 (m, 2H), 1.40 - 1.28 (m, 1H), 1.20 - 1.03 (m, 6H), 1.02 - 0.73 (m, 7H), 0.29 (br d, J = 5.9 Hz, 2H). |
| **112** | 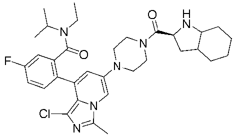 | 609.4 | ¹H NMR (400 MHz, DMSO-*d*₆) δ 7.47 -7.39 (m, 1H), 7.38 - 7.26 (m, 3H), 6.64 - 6.57 (m, 1H), 3.97 - 3.87 (m, 1H), 3.58 (br s, 4H), 3.08 - 2.86 (m, 6H), 2.55 (s, 3H), 2.23 - 2.09 (m, 1H), 1.96 - 1.86 (m, 1H), 1.75 - 1.57 (m, 2H), 1.51 - 1.27 (m, 6H), 1.24 - 1.00 (m, 4H), 0.99 - 0.73 (m, 7H), 0.36 - 0.24 (m, 2H). |
| **114** | 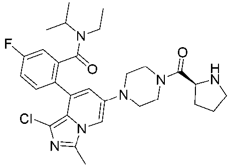 | 555.3 | ¹H NMR (400 MHz, DMSO-*d*₆) δ 7.48 - 7.39 (m, 1H), 7.38 - 7.26 (m, 3H), 6.61 (s, 1H), 3.93 - 3.86 (m, 1H), 3.73 - 3.53 (m, 5H), 3.10 (s, 1H), 3.05 - 2.81 (m, 6H), 2.69 - 2.60 (m, 1H), 2.55 (s, 3H), 2.08 - 1.96 (m, 1H), 1.73 - 1.53 (m, 3H), 1.24 - 0.74 (m, 8H), 0.34 - 0.26 (m, 2H). |
| **116** | 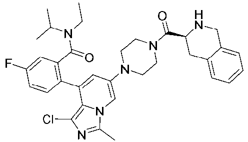 | 617.3 | ¹H NMR (400 MHz, DMSO-*d₆*) δ 7.48 -7.40 (m, 1H), 7.39 - 7.28 (m, 3H), 7.14 - 7.07 (m, 3H), 7.05 - 6.99 (m, 1H), 6.63 (s, 1H), 3.99 - 3.93 (m, 1H), 3.93 - 3.87 (m, 2H), 3.85 - 3.77 (m, 1H), 3.75 - 3.52 (m, 4H), 3.22 - 2.93 (m, 5H), 2.92 - 2.80 (m, 2H), 2.76 - 2.67 (m, 1H), 2.56 (s, 3H), 1.27 - 1.11 (m, 1H), 1.07 - 0.91 (m, 3H), 0.89 - 0.82 (m, 1H), 0.82 - 0.76 (m, 3H), 0.30 (d, J = 6.4 Hz, 2H). |
| **117** | 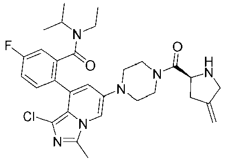 | 567.3 | ¹H NMR (400 MHz, CD₃OD) δ 7.59 - 7.43 (m, 1H), 7.37 - 7.26 (m, 2H), 7.26 - 7.18 (m, 1H), 6.79 (s, 1H), 5.07 - 5.00 (m, 2H), 4.34 - 4.17 (m, 1H), 3.91 - 3.66 (m, 6H), 3.57 - 3.41 (m, 2H), 3.25 - 2.89 (m, 6H), 2.62 (s, 3H), 2.50 - 2.37 (m, 1H), 1.26 - 1.10 (m, 1H), 1.07 - 0.88 (m, 6H), 0.44 - 0.35 (m, 2H). |
| **119** | 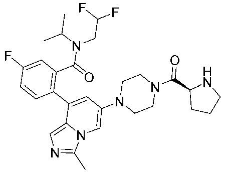 | 557.3 | ¹H NMR (400 MHz, DMSO-*d₆*) δ 7.73 - 7.63 (m, 1H), 7.48 - 7.26 (m, 3H), 6.95 (s, 1H), 6.60 (s, 1H), 5.94 - 5.54 (m, 1H), 3.90 - 3.82 (m, 1H), 3.63 (s, 5H), 3.55 - 3.46 (m, 1H), 3.10 - 2.93 (m, 5H), 2.68 - 2.59 (m, 1H), 2.55 (s, 3H), 2.06 - 1.92 (m, 1H), 1.73 - 1.51 (m, 3H), 1.26 - 0.84 (m, 4H), 0.43 - 0.22 (m, 3H). |
| **122** | 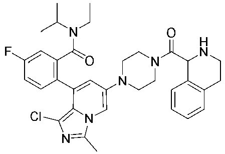 | 617.2 | ¹H NMR (400 MHz, DMSO-*d₆*) δ 7.47 -7.38 (m, 1H), 7.37 - 7.24 (m, 3H), 7.19 - 7.06 (m, 3H), 6.96 - 6.89 (m, 1H), 6.63 - 6.54 (m, 1H), 5.01 - 4.92 (m, 1H), 3.85 - 3.49 (m, 5H), 3.15 - 2.62 (m, 11H), 2.55 (s, 3H), 1.27 - 0.88 (m, 4H), 0.83 - 0.72 (m, 3H), 0.29 (s, 2H). |
| **123** | 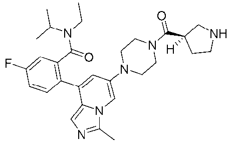 | 521.3 | ¹H NMR (400 MHz, CD₃OD) δ 7.52 - 7.39 (m, 1H), 7.15 - 7.04 (m, 2H), 7.03 - 6.95 (m, 1H), 6.85 (s, 1H), 6.55 (s, 1H), 3.59 - 3.48 (m, 4H), 3.32 (t, J = 13.0 Hz, 1H), 3.26 - 3.09 (m, 2H), 3.07 (s, 2H), 2.93 - 2.89 (m, 2H), 2.88 - 2.84 (m, 2H), 2.83 - 2.75 (m, 2H), 2.73 - 2.64 (m, 1H), 2.38 (s, 3H), 1.95 - 1.81 (m, 1H), 1.77 - 1.63 (m, 1H), 0.95 - 0.63 (m, 6H), 0.57 (t, J = 7.0 Hz, 1H), 0.10 (d, J = 6.4 Hz, 2H). |
| **124** | 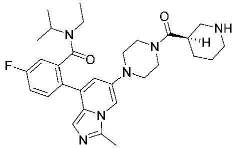 | 535.3 | ¹H NMR (400 MHz, CD₃OD) δ 7.59 - 7.43 (m, 1H), 7.37 - 7.26 (m, 2H), 7.26 - 7.18 (m, 1H), 6.79 (s, 1H), 5.07 - 5.00 (m, 2H), 4.34 - 4.17 (m, 1H), 3.91 - 3.66 (m, 6H), 3.57 - 3.41 (m, 2H), 3.25 - 2.89 (m, 6H), 2.62 (s, 3H), 2.50 - 2.37 (m, 1H), 1.26 - 1.10 (m, 1H), 1.07 - 0.88 (m, 6H), 0.44 - 0.35 (m, 2H). |
| **125** | 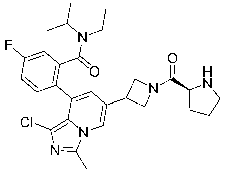 | 526.3 | ¹H NMR (400 MHz, CDCl₃) δ 11.77 - 10.48 (m, 1H), 7.65 - 7.43 (m, 1H), 7.40 - 7.26 (m, 1H), 7.13 - 6.69 (m, 3H), 4.78 - 4.23 (m, 4H), 4.21 - 4.03 (m, 1H), 3.94 - 3.73 (m, 1H), 3.69 - 3.54 (m, 1H), 3.33 (s, 3H), 3.08 - 2.75 (m, 1H), 2.57 (s, 3H), 2.42 - 2.13 (m, 1H), 2.09 - 1.81 (m, 3H), 1.43 - 1.10 (m, 1H), 1.08 - 0.97 (m, 1H), 0.95 - 0.86 (m, 2H), 0.83 - 0.67 (m, 3H), 0.40 - 0.22 (m, 2H). |
| **126** | 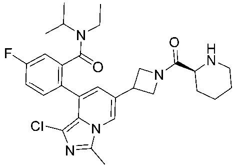 | 540.2 | ¹H NMR (400 MHz, CD₃OD) δ 8.04 (s, 1H), 7.62 - 7.49 (m, 1H), 7.40 - 7.16 (m, 2H), 7.03 - 6.87 (m, 1H), 4.83 - 4.63 (m, 2H), 4.55 - 4.32 (m, 2H), 4.18 - 3.94 (m, 3H), 3.88 - 3.69 (m, 1H), 3.55 - 3.37 (m, 2H), 3.22 - 2.94 (m, 2H), 2.67 (s, 3H), 2.28 - 2.09 (m, 1H), 2.03 - 1.84 (m, 2H), 1.82 - 1.61 (m, 3H), 1.45 - 1.28 (m, 1H), 1.20 - 1.12 (m, 1H), 1.08 - 1.02 (m, 2H), 0.97 - 0.87 (m, 3H), 0.48 - 0.38 (m, 2H). |
| **127** | 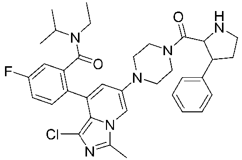 | 631.4 | ¹H NMR (400 MHz, DMSO-*d₆*) δ 7.48 - 7.02 (m, 9H), 6.57 - 6.37 (m, 1H), 4.29 - 3.90 (m, 1H), 3.70 - 3.43 (m, 3H), 3.38 - 3.19 (m, 5H), 3.14 - 2.58 (m, 7H), 2.53 (s, 3H), 2.35 - 2.06 (m, 1H), 1.89 - 1.68 (m, 1H), 1.25 - 0.67 (m, 7H), 0.38 - 0.17 (m, 2H). |
| **129** | 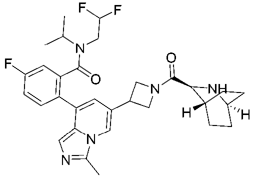 | 568.1 | ¹H NMR (400 MHz, DMSO-*d*₆) δ 8.10 - 8.01 (m, 1H), 7.69 (s, 1H), 7.49 - 7.28 (m, 2H), 7.07 - 6.97 (m, 1H), 6.71 - 6.54 (m, 1H), 5.96 - 5.46 (m, 1H), 4.72 - 4.04 (m, 4H), 4.02 - 3.81 (m, 2H), 3.69 - 3.47 (m, 3H), 2.80 - 2.67 (m, 1H), 2.59 (s, 3H), 2.04 - 1.22 (m, 10H), 1.00 - 0.81 (m, 3H), 0.33 (s, 3H). |
| **130** | 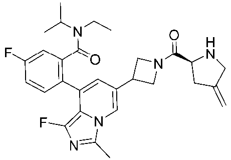 | 522.3 | ¹H NMR (400 MHz, DMSO-*d₆*) δ 7.98 (s, 1H), 7.60 - 7.48 (m, 1H), 7.42 - 7.28 (m, 2H), 6.65 - 6.51 (m, 1H), 5.00 - 4.80 (m, 2H), 4.68 - 4.43 (m, 1H), 4.33 - 4.11 (m, 2H), 4.01 - 3.77 (m, 2H), 3.75 - 3.63 (m, 1H), 3.59 - 3.41 (m, 2H), 3.09 - 2.75 (m, 2H), 2.72 - 2.53 (m, 5H), 2.44 - 2.28 (m, 1H), 1.17 - 0.73 (m, 7H), 0.53 - 0.33 (m, 2H). |
| **133** | 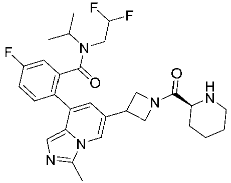 | 542.3 | ¹H NMR (400 MHz, CD₃OD) δ 8.01 (s, 1H), 7.80 - 7.61 (m, 1H), 7.38 (t, J = 8.2 Hz, 1H), 7.29 (d, J = 8.8 Hz, 1H), 7.15 (s, 1H), 6.89 - 6.79 (m, 1H), 6.06 - 5.52 (m, 1H), 4.78 - 4.56 (m, 1H), 4.48 - 4.23 (m, 2H), 4.16 - 3.88 (m, 2H), 3.81 - 3.58 (m, 2H), 3.46 - 3.31 (m, 2H), 3.15 - 3.00 (m, 1H), 2.67 (s, 4H), 1.97 - 1.74 (m, 2H), 1.65 - 1.38 (m, 4H), 1.02 - 0.90 (m, 3H), 0.60 - 0.17 (m, 3H). |
| **135** | 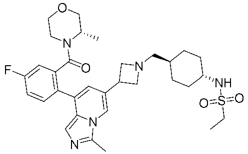 | 612.3 | ¹H NMR (400 MHz, CDCl₃) δ 7.80 - 7.51 (m, 2H), 7.26 - 7.13 (m, 3H), 7.00 (br s, 1H), 4.63 - 4.10 (m, 1H), 4.06 - 3.95 (m, 1H), 3.82 - 3.36 (m, 5H), 3.31 - 3.11 (m, 4H), 3.04 (q, J = 7.2 Hz, 2H), 2.99 - 2.84 (m, 1H), 2.69 (s, 3H), 2.45 - 2.33 (m, 2H), 2.13 - 2.03 (m, 2H), 1.94 - 1.72 (m, 4H), 1.37 (t, J = 7.6 Hz, 3H), 1.33 - 1.16 (m, 4H), 1.14 - 0.97 (m, 3H), 0.80 - 0.42 (m, 1H). |
| **137** | 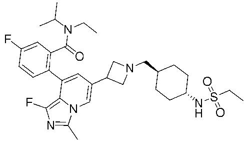 | 616.3 | ¹H NMR (400 MHz, DMSO-*d*₆) δ 7.83 (s, 1H), 7.56 - 7.47 (m, 1H), 7.39 - 7.25 (m, 2H), 7.00 - 6.94 (m, 1H), 6.72 - 6.60 (m, 1H), 3.46 (s, 4H), 3.13 - 2.90 (m, 6H), 2.53 (s, 3H), 2.28 - 2.18 (m, 2H), 2.08 - 1.63 (m, 5H), 1.21 - 1.11 (m, 6H), 0.97 - 0.75 (m, 9H), 0.39 (s, 2H). |
| **138** | 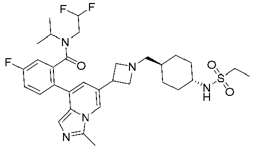 | 634.3 | ¹H NMR (400 MHz, DMSO-*d*₆) δ 7.93 (s, 1H), 7.72 - 7.64 (m, 1H), 7.48 - 7.23 (m, 2H), 7.04 - 6.91 (m, 2H), 6.75 (s, 1H), 5.91 - 5.58 (m, 1H), 3.68 - 3.46 (m, 5H), 3.01 (s, 5H), 2.62 - 2.54 (m, 3H), 2.34 - 2.19 (m, 2H), 1.89 - 1.67 (m, 4H), 1.26 - 1.11 (m, 6H), 1.01 - 0.84 (m, 5H), 0.37 - 0.23 (m, 3H). |
| **144** | 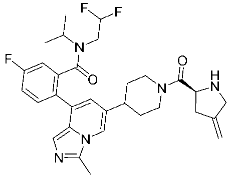 | 568.3 | ¹H NMR (400 MHz, CD₃OD) δ 7.66 (d, J = 11.6 Hz, 1H), 7.53 - 7.42 (m, 1H), 7.17 (dt, J = 8.4, 8.8 Hz, 1H), 7.11 - 7.05 (m, 1H), 6.93 - 6.88 (m, 1H), 6.53 - 6.47 (m, 1H), 5.81 - 5.34 (m, 1H), 4.87 - 4.74 (m, 2H), 4.54 - 4.43 (m, 1H), 4.02 - 3.88 (m, 2H), 3.59 - 3.36 (m, 3H), 3.29 - 3.18 (m, 1H), 3.07 - 2.99 (m, 1H), 2.76 - 2.56 (m, 3H), 2.49 - 2.42 (m, 3H), 2.33 - 2.13 (m, 1H), 1.85 - 1.71 (m, 2H), 1.61 - 1.36 (m, 2H), 1.27 - 1.06 (m, 1H), 1.01 - 0.51 (m, 4H), 0.33 - 0.01 (m, 3H). |
| **148** | 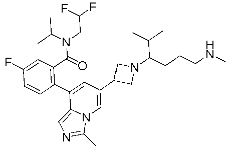 | 558.4 | ¹H NMR (400 MHz, CD₃OD) δ 7.92 (s, 1H), 7.75 - 7.68 (m, 1H), 7.43 - 7.34 (m, 1H), 7.33 - 7.28 (m, 1H), 7.16 - 7.11 (m, 1H), 6.92 - 6.77 (m, 1H), 5.99 - 5.64 (m, 1H), 3.83 - 3.77 (m, 1H), 3.77 - 3.69 (m, 2H), 3.69 - 3.62 (m, 2H), 3.29 - 3.24 (m, 2H), 2.75 - 2.68 (m, 2H), 2.66 (s, 3H), 2.50 (s, 3H), 2.27 - 2.23 (m, 1H), 2.20 - 2.03 (m, 1H), 1.92 - 1.84 (m, 1H), 1.67 - 1.59 (m, 2H), 1.46 - 1.37 (m, 2H), 1.36 - 1.32 (m, 2H), 1.29 (s, 3H), 0.98 - 0.95 (m, 3H), 0.94 - 0.89 (m, 3H), 0.36 - 0.32 (m, 2H). |
| **149** | 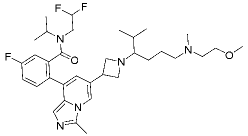 | 616.6 | ¹H NMR (400 MHz, CD₃OD) δ 7.93 (s, 1H), 7.75 - 7.68 (m, 1H), 7.42 - 7.35 (m, 1H), 7.30 (dd, J = 2.8, 8.8 Hz, 1H), 7.16 - 7.11 (m, 1H), 6.91 - 6.82 (m, J = 2.6 Hz, 1H), 6.01 - 5.63 (m, 1H), 3.85 - 3.79 (m, 1H), 3.79 - 3.61 (m, 4H), 3.57 - 3.51 (m, 2H), 3.34 (d, J = 3.2 Hz, 3H), 2.78 - 2.71 (m, 2H), 2.66 (s, 3H), 2.60 - 2.53 (m, 2H), 2.39 (d, J = 2.8 Hz, 3H), 2.31 - 2.26 (m, 1H), 1.94 - 1.83 (m, 1H), 1.67 - 1.58 (m, 2H), 1.45 - 1.27 (m, 6H), 0.98 - 0.91 (m, 9H), 0.35 (br d, J = 6.5 Hz, 2H). |
| **151** | 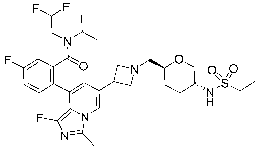 | 654.3 | ¹H NMR (400 MHz, DMSO-*d₆*) δ 7.77 (s, 1H), 7.64 - 7.49 (m, 1H), 7.40 - 7.22 (m, 2H), 6.73 - 6.56 (m, 1H), 6.14 - 5.72 (m, 1H), 4.08 - 3.93 (m, 1H), 3.84 - 3.59 (m, 5H), 3.40 - 3.32 (m, 2H), 3.30 - 3.20 (m, 3H), 3.17 - 2.97 (m, 3H), 2.67 - 2.53 (m, 5H), 2.17 - 2.03 (m, 1H), 1.77 - 1.63 (m, 1H), 1.56 - 1.39 (m, 2H), 1.31 (t, J = 7.2 Hz, 3H), 1.06 - 0.94 (m, 3H), 0.45 (s, 3H). |
| **155** | 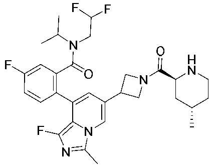 | 574.3 | ¹H NMR (400 MHz, DMSO-*d₆*) δ 7.96 (s, 1H), 7.64 - 7.50 (m, 1H), 7.47 - 7.26 (m, 2H), 6.59 - 6.41 (m, 1H), 6.15 - 5.70 (m, 1H), 4.72 - 4.40 (m, 1H), 4.33 - 4.04 (m, 2H), 3.98 - 3.39 (m, 6H), 2.85 - 2.63 (m, 2H), 2.55 (s, 3H), 1.92 - 1.62 (m, 2H), 1.57 - 1.41 (m, 1H), 1.30 - 1.14 (m, 1H), 1.06 - 0.86 (m, 7H), 0.43 (s, 3H). |
| **156** | 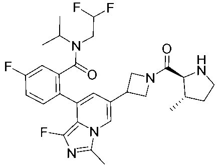 | 560.3 | ¹H NMR (400 MHz, DMSO-*d₆*) δ 7.98 (s, 1H), 7.64 - 7.24 (m, 3H), 6.64 - 6.35 (m, 1H), 6.17 - 5.65 (m, 1H), 4.70 - 4.44 (m, 1H), 4.40 - 3.77 (m, 5H), 3.73 - 3.46 (m, 3H), 3.12 (s, 1H), 3.00 - 2.70 (m, 2H), 2.55 (s, 3H), 2.13 (s, 1H), 1.82 (s, 1H), 1.51-1.19 (m, 1H), 1.09 - 0.89 (m, 6H), 0.43 (s, 3H). |
| **158** | 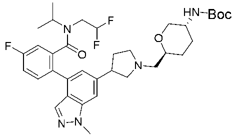 | 658.5 | ¹H NMR (400 MHz, CD₃OD) δ 7.71 (s, 1H), 7.53 - 7.47 (m, 1H), 7.40 (s, 1H), 7.28 - 7.19 (m, 1H), 7.14 (dd, J = 2.8, 8.8 Hz, 1H), 6.97 (s, 1H), 5.78 - 5.39 (m, 1H), 3.95 (s, 3H), 3.88 - 3.79 (m, 1H), 3.50 - 3.39 (m, 3H), 3.38 - 3.29 (m, 2H), 3.15 - 3.10 (m, 1H), 2.97 - 2.86 (m, 2H), 2.77 - 2.66 (m, 1H), 2.61 - 2.48 (m, 3H), 2.29 - 2.19 (m, 1H), 1.92 - 1.83 (m, 3H), 1.65 - 1.57 (m, 1H), 1.29 (s, 12H), 0.77 (d, J = 6.4 Hz, 3H), 0.01 (d, J = 6.4 Hz, 3H). |
| **160** | 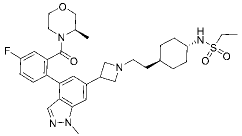 | 626.6 | ¹H NMR (400 MHz, DMSO-*d₆*) δ 7.92 - 7.73 (m, 1H), 7.72 - 7.51 (m, 2H), 7.50 - 7.27 (m, 2H), 7.21 - 6.92 (m, 2H), 4.34 - 3.38 (m, 9H), 3.23 - 2.66 (m, 8H), 2.43 (d, J = 6.8 Hz, 2H), 1.90 - 1.66 (m, 4H), 1.43 - 0.87 (m, 13H), 0.52 - 0.15 (m, 1H). |
| **162** | 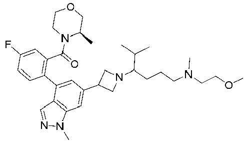 | 594.4 | ¹H NMR (400 MHz, CDCl₃) δ 8.52 (s, 1H), 7.90 - 7.76 (m, 1H), 7.66 - 7.45 (m, 1H), 7.29 - 7.05 (m, 4H), 4.54 - 4.20 (m, 1H), 4.16 - 3.93 (m, 6H), 3.63 - 2.90 (m, 11H), 2.85 - 2.47 (m, 6H), 2.41 - 2.32 (m, 4H), 1.95 - 1.77 (m, 1H), 1.69 - 1.54 (m, 2H), 1.46 - 1.34 (m, 2H), 1.24 - 1.16 (m, 1H), 1.04 - 0.87 (m, 7H), 0.53 - 0.08 (m, 1H). |
| **167** | 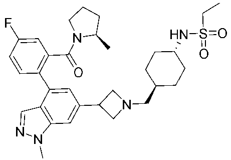 | 596.3 | ¹H NMR (400 MHz, DMSO-*d*₆) δ 7.91 - 7.74 (m, 1H), 7.70 - 7.58 (m, 1H), 7.55 - 7.49 (m, 1H), 7.45 - 7.25 (m, 2H), 7.14 - 6.92 (m, 2H), 4.12 - 4.03 (m, 3H), 3.94 - 3.82 (m, 1H), 3.77 - 3.67 (m, 1H), 3.67 - 3.59 (m, J = 6.0 Hz, 2H), 3.18 - 3.04 (m, 2H), 3.02 - 2.64 (m, 5H), 2.35 - 2.18 (m, 2H), 1.93 - 1.64 (m, 5H), 1.59 - 1.23 (m, 3H), 1.22 - 1.12 (m, 6H), 0.99 - 0.64 (m, 5H). |
| **168** | 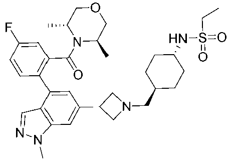 | 626.4 | ¹H NMR (400 MHz, DMSO-*d*₆) δ 7.74 - 7.33 (m, 5H), 7.07 - 6.86 (m, 2H), 4.06 (s, 3H), 3.80 - 3.48 (m, 5H), 3.19 - 2.91 (m, 7H), 2.42 - 2.07 (m, 4H), 1.90 - 1.71 (m, 4H), 1.22 - 1.12 (m, 6H), 1.04 - 0.80 (m, 5H), 0.62 (s, 3H). |
| **172** | 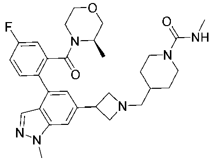 | 563.6 | ¹H NMR (400 MHz, DMSO-*d₆*) δ 7.81 (s, 1H), 7.73 - 7.52 (m, 2H), 7.51 - 7.20 (m, 2H), 7.19 - 6.87 (m, 1H), 6.55 - 6.26 (m, 1H), 4.34 - 3.37 (m, 11H), 3.25 - 2.69 (m, 5H), 2.69 - 2.52 (m, 5H), 2.38 - 2.21 (m, 2H), 1.85 - 1.21 (m, 4H), 1.21 - 0.85 (m, 4H), 0.56 - 0.13 (m, 1H). |
| **173** | 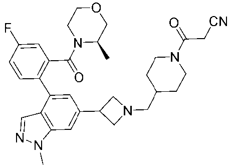 | 573.3 | ¹H NMR (400 MHz, DMSO-*d₆*) δ 7.81 (s, 1H), 7.70 - 7.55 (m, 2H), 7.49 - 7.25 (m, 2H), 7.20 - 6.93 (m, 1H), 4.34 - 3.45 (m, 13H), 3.17 - 2.54 (m, 7H), 2.45 - 2.17 (m, 3H), 1.81 - 1.44 (m, 3H), 1.19 - 0.86 (m, 4H), 0.52 - 0.06 (m, 1H). |
| **176** | 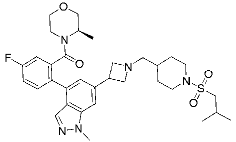 | 626.3 | ¹H NMR (400 MHz, DMSO-*d₆*) δ 7.82 (s, 1H), 7.71 - 7.54 (m, 1H), 7.49 - 7.25 (m, 2H), 7.17 - 6.69 (m, 2H), 4.32 - 3.91 (m, 5H), 3.82 - 3.02 (m, 12H), 2.93 - 2.85 (m, 2H), 2.80 - 2.67 (m, 2H), 2.32 - 2.26 (m, 1H), 2.19 - 2.03 (m, 1H), 1.88 - 1.68 (m, 2H), 1.39 - 1.17 (m, 2H), 1.14 - 0.77 (m, 10H), 0.50 - 0.11 (m, 1H). |
| **177** | 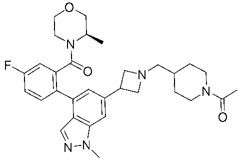 | 548.3 | ¹H NMR (400 MHz, DMSO-*d₆*) δ 7.65 (s, 1H), 7.53 - 7.38 (m, 2H), 7.31 - 7.10 (m, 2H), 7.02 - 6.77 (m, 1H), 4.18 - 3.86 (m, 5H), 3.71 - 3.46 (m, 5H), 3.44 - 3.18 (m, 2H), 3.02 - 2.50 (m, 6H), 2.19 - 2.14 (m, 2H), 1.83 - 1.77 (m, 3H), 1.63 - 1.49 (m, 2H), 1.42 - 1.06 (m, 2H), 1.00 - 0.86 (m, 2H), 0.83 - 0.73 (m, 2H), 0.33 - 0.00 (m, 1H). |
| **179** | 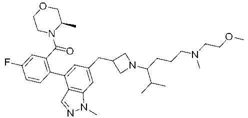 | 608.4 | ¹H NMR (400 MHz, CD₃OD) δ 7.73 - 7.62 (m, 1H), 7.49 - 7.35 (m, 1H), 7.28 - 7.01 (m, 3H), 6.88 - 6.74 (m, 1H), 4.26 - 3.95 (m, 1H), 3.92 - 3.76 (m, 3H), 3.46 - 3.20 (m, 6H), 3.13 (s, 4H), 3.07 - 2.91 (m, 1H), 2.85 - 2.77 (m, 4H), 2.75 - 2.54 (m, 2H), 2.34 (s, 2H), 2.25 - 2.15 (m, 2H), 2.10 - 2.04 (m, 3H), 1.95 (s, 1H), 1.72 - 1.59 (m, 1H), 1.43 - 1.12 (m, 4H), 1.09 - 1.00 (m, 2H), 0.91 - 0.81 (m, 1H), 0.77 - 0.73 (m, 3H), 0.71 - 0.67 (m, 3H), 0.30 - -0.03 (m, 1H). |
| **180** | 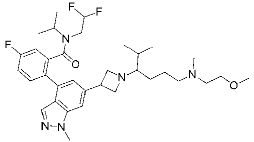 | 616.3 | ¹H NMR (400 MHz, CD₃OD) δ 7.85 (s, 1H), 7.71 - 7.59 (m, 1H), 7.50 (s, 1H), 7.44 - 7.34 (m, 1H), 7.32 - 7.23 (m, 1H), 7.18 - 7.06 (m, 1H), 5.91 - 5.58 (m, 2H), 4.08 (s, 3H), 3.94 - 3.78 (m, 4H), 3.60 - 3.47 (m, 4H), 2.68 - 2.60 (m, 2H), 2.48 - 2.41 (m, 2H), 2.30 (s, 3H), 2.28 - 2.21 (m, 1H), 1.97 - 1.81 (m, 2H), 1.67 - 1.54 (m, 3H), 1.44 - 1.25 (m, 6H), 0.97 - 0.88 (m, 8H), 0.14 - 0.10 (m, 2H). |
| **182-1** | 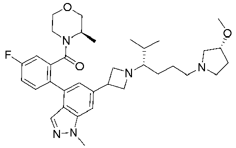 | 606.4 | ¹H NMR (400 MHz, CDCl₃) δ 8.12 - 7.81 (m, 1H), 7.71 - 7.52 (m, 1H), 7.41 - 7.27 (m, 2H), 7.26 - 7.08 (m, 2H), 4.63 - 4.27 (m, 1H), 4.14 (s, 3H), 4.07 - 3.97 (m, 3H), 3.70 - 3.51 (m, 2H), 3.51 - 3.37 (m, 3H), 3.33 - 3.27 (m, 3H), 3.24 - 3.12 (m, 1H), 3.04 - 2.93 (m, 2H), 2.90 - 2.81 (m, 2H), 2.80 - 2.70 (m, 2H), 2.61 - 2.34 (m, 2H), 2.27 - 2.07 (m, 1H), 2.04 - 1.84 (m, 2H), 1.78 - 1.67 (m, 2H), 1.54 - 1.37 (m, 2H), 1.32 - 1.21 (m, 2H), 1.10 - 1.03 (m, 1H), 1.02 - 0.87 (m, 6H), 0.59 - 0.06 (m, 2H). |
| **182-2** | 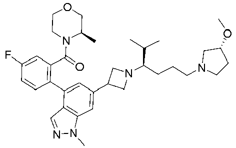 | 606.4 | ¹H NMR (400 MHz, CD₃OD) δ 7.98 - 7.80 (m, 1H), 7.77 - 7.57 (m, 1H), 7.57 - 7.45 (m, 1H), 7.42 - 7.06 (m, 3H), 4.52 - 4.02 (m, 4H), 4.01 - 3.34 (m, 7H), 3.28 - 2.64 (m, 9H), 2.59 - 2.23 (m, 5H), 2.16 - 2.02 (m, 1H), 1.96 - 1.76 (m, 2H), 1.70 - 1.19 (m, 6H), 1.11 - 0.86 (m, 7H), 0.56 - 0.14 (m, 1H). |
| **183-1** | 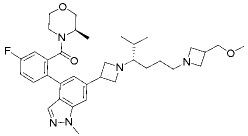 | 606.4 | ¹H NMR (400 MHz, CD₃OD) δ 7.91 - 7.84 (m, 1H), 7.72 - 7.50 (m, 2H), 7.42 - 7.34 (m, 1H), 7.34 - 7.20 (m, 2H), 4.12 - 4.08 (m, 3H), 3.99 - 3.78 (m, 4H), 3.49 - 3.45 (m, 2H), 3.41 (t, J = 7.8 Hz, 2H), 3.34 - 3.32 (m, 3H), 3.29 - 3.17 (m, 2H), 3.08 - 2.97 (m, 3H), 2.97 - 2.80 (m, 1H), 2.72 (td, J = 6.8, 13.2 Hz, 1H), 2.56 - 2.32 (m, 3H), 2.27 - 2.01 (m, 2H), 1.93 - 1.82 (m, 1H), 1.51 - 1.37 (m, 4H), 1.36 - 1.26 (m, 2H), 1.23 (d, J = 6.8 Hz, 1H), 1.06 (d, J = 6.8 Hz, 1H), 0.97 - 0.90 (m, 6H), 0.51 (d, J = 6.8 Hz, 1H). |
| **183-2** | 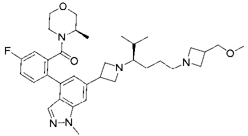 | 606.4 | ¹H NMR (400 MHz, CD3OD) δ 7.96 - 7.79 (m, 1H), 7.77 - 7.45 (m, 2H), 7.43 - 7.07 (m, 3H), 4.49 - 4.03 (m, 4H), 4.00 - 3.80 (m, 3H), 3.66 - 3.33 (m, 9H), 3.28 - 2.69 (m, 7H), 2.60 - 2.19 (m, 4H), 1.95 - 1.81 (m, 1H), 1.56 - 1.18 (m, 6H), 1.10 - 0.87 (m, 7H), 0.55 - 0.14 (m, 1H). |
| **184-1** | 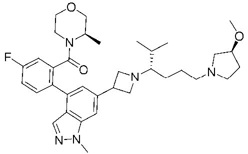 | 606.4 | ¹H NMR (400 MHz, CD3OD) δ 7.92 - 7.86 (m, 1H), 7.74 - 7.67 (m, 1H), 7.64 - 7.54 (m, 1H), 7.43 - 7.36 (m, 1H), 7.35 - 7.22 (m, 2H), 4.13 - 4.10 (m, 3H), 4.07 - 4.00 (m, 2H), 4.00 - 3.91 (m, 3H), 3.53 - 3.43 (m, 3H), 3.13 - 2.97 (m, 4H), 2.91 - 2.77 (m, 4H), 2.55 - 2.35 (m, 2H), 2.20 - 2.11 (m, 1H), 2.05 - 1.95 (m, 2H), 1.91 (s, 3H), 1.78 - 1.69 (m, 2H), 1.40 - 1.33 (m, 2H), 1.31 - 1.28 (m, 2H), 1.24 (d, J = 6.8 Hz, 1H), 1.07 (d, J = 6.8 Hz, 1H), 1.00 (t, J = 6.2 Hz, 3H), 0.97 - 0.93 (m, 3H), 0.50 (d, J = 6.8 Hz, 1H). |
| **184-2** | 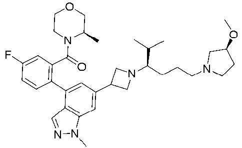 | 606.4 | ¹H NMR (400 MHz, CD3OD) δ 7.95 -7.83 (m, 1H), 7.72 - 7.57 (m, 1H), 7.56 - 7.45 (m, 1H), 7.42 - 7.31 (m, 1H), 7.31 - 7.07 (m, 2H), 4.47 - 4.26 (m, 1H), 4.13 - 4.07 (m, 3H), 4.00 - 3.80 (m, 5H), 3.65 - 3.40 (m, 2H), 3.27 - 3.23 (m, 3H), 3.13 (s, 2H), 2.74 - 2.73 (m, 1H), 2.78 - 2.61 (m, 3H), 2.56 - 2.37 (m, 4H), 2.25 (s, 1H), 2.13 - 2.00 (m, 1H), 1.93 - 1.84 (m, 1H), 1.83 - 1.74 (m, 1H), 1.67 - 1.55 (m, 2H), 1.52 - 1.27 (m, 3H), 1.25 - 1.21 (m, 1H), 1.08 - 1.04 (m, 1H), 0.99 - 0.90 (m, 6H), 0.54 - 0.13 (m, 1H). |
| **185** | 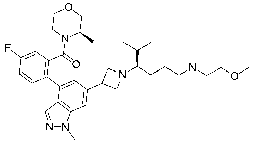 | 594.4 | ¹H NMR (400 MHz, DMSO-d6) δ 7.89 - 7.75 (m, 1H), 7.70 - 7.53 (m, 2H), 7.50 - 7.21 (m, 2H), 7.15 - 6.91 (m, 1H), 4.33 - 4.11 (m, 1H), 4.09 - 4.03 (m, 3H), 3.87 - 3.54 (m, 4H), 3.52 - 3.36 (m, 4H), 3.20 (s, 3H), 3.18 - 3.01 (m, 4H), 2.94 - 2.72 (m, 1H), 2.47 - 2.42 (m, 2H), 2.31 - 2.23 (m, 2H), 2.14 (s, 3H), 2.08 - 1.99 (m, 1H), 1.78 - 1.68 (m, 1H), 1.48 - 1.37 (m, 2H), 1.30 - 1.18 (m, 2H), 1.12 (d, J = 6.8 Hz, 1H), 0.98 (d, J = 6.8 Hz, 1H), 0.89 - 0.82 (m, 6H), 0.51 - 0.14 (m, 1H). |
| **188** | 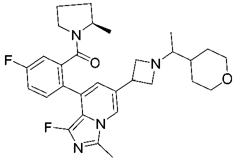 | 523.3 | ¹H NMR (400 MHz, DMSO-d6) δ 7.82 (s, 1H), 7.59 - 7.48 (m, 1H), 7.42 - 7.28 (m, 2H), 6.75 - 6.53 (m, 1H), 4.00 - 3.79 (m, 3H), 3.56 - 3.42 (m, 3H), 3.30 - 3.17 (m, 3H), 3.12 - 3.00 (m, 3H), 2.54 (s, 3H), 2.19 - 2.11 (m, 1H), 1.93 - 1.73 (m, 1H), 1.64 - 1.45 (m, 4H), 1.40 - 1.18 (m, 4H), 0.96 - 0.74 (m, 6H). |
| 189 | 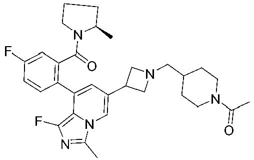 | 550.3 | ¹H NMR (400 MHz, DMSO-d6) δ 8.15 (s, 0.79H), 7.83 (s, 1H), 7.59 - 7.50 (m, 1H), 7.42 - 7.29 (m, 2H), 6.76 - 6.59 (m, 1H), 4.37 - 4.28 (m, 1H), 3.98 - 3.89 (m, 1H), 3.82 - 3.70 (m, 2H), 3.27 - 2.89 (m, 7H), 2.54 (s, 3H), 2.48 - 2.42 (m, 1H), 2.37 - 2.32 (m, 2H), 1.96 (s, 3H), 1.91 - 1.78 (m, 1H), 1.74 - 1.31 (m, 6H), 1.10 - 0.75 (m, 5H). |
| **190** | 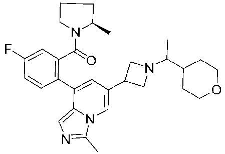 | 505.4 | ¹H NMR (400 MHz, DMSO-d6) δ 7.92 (s, 1H), 7.79 - 7.65 (m, 1H), 7.44 - 7.30 (m, 2H), 7.11 - 7.00 (m, 1H), 6.87 - 6.69 (m, 1H), 3.96 - 3.81 (m, 3H), 3.58 - 3.45 (m, 3H), 3.29 - 3.17 (m, 3H), 3.13 - 2.97 (m, 3H), 2.59 (s, 3H), 2.16 (s, 1H), 1.89 - 1.73 (m, 1H), 1.63 - 1.44 (m, 4H), 1.38 - 1.26 (m, 3H), 1.25 - 1.16 (m, 1H), 0.88 (s, 2H), 0.81 - 0.72 (m, 4H). |
| **192** | 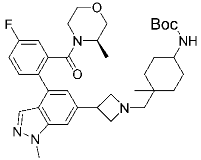 | 634.3 | ¹H NMR (400 MHz, CD3OD) δ 8.55 - 8.48 (m, 1H), 7.99 - 7.86 (m, 1H), 7.75 - 7.55 (m, 2H), 7.52 - 7.11 (m, 4H), 4.63 - 4.17 (m, 4H), 4.14 - 3.99 (m, 5H), 3.85 - 3.38 (m, 5H), 3.23 - 3.00 (m, 2H), 2.96 - 2.81 (m, 3H), 2.56 - 2.26 (m, 1H), 1.77 - 1.60 (m, 5H), 1.44 (s, 9H), 1.24 (d, J = 6.8 Hz, 2H), 1.00 (s, 3H), 0.51 - 0.16 (m, 1H). |
| **193** | 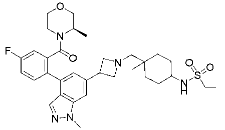 | 626.2 | ¹H NMR (400 MHz, CD3OD) δ 8.02 - 7.81 (m, 1H), 7.80 - 7.51 (m, 2H), 7.49 - 7.07 (m, 3H), 4.67 - 4.24 (m, 3H), 4.18 - 3.89 (m, 3H), 3.61 - 3.38 (m, 3H), 3.25 - 3.02 (m, 3H), 3.00 - 2.87 (m, 1H), 2.86 - 2.77 (m, 3H), 2.52 - 2.18 (m, 1H), 1.95 - 1.68 (m, 4H), 1.66 - 1.51 (m, 2H), 1.49 - 1.36 (m, 2H), 1.35 - 1.26 (m, 7H), 1.25 - 1.20 (m, 1H), 1.13 - 1.07 (m, 3H), 0.48 - 0.19 (m, 1H). |
| **200-1** | 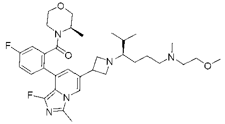 | 612.3 | ¹H NMR (400 MHz, DMSO-d6) δ 8.00 - 7.68 (m, 1H), 7.64 -7.20 (m, 3H), 6.70 (s, 1H), 4.40 - 3.34 (m, 9H), 3.20 (s, 3H), 3.17 - 2.51 (m, 8H), 2.47 - 2.38 (m, 2H), 2.31 - 2.23 (m, 2H), 2.13 (s, 3H), 2.06 - 1.98 (m, 1H), 1.78 - 1.62 (m, 1H), 1.51 - 1.01 (m, 6H), 0.90 - 0.65 (m, 7H). |
| **200-2** | 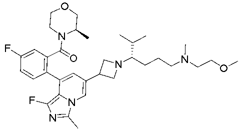 | 612.4 | ¹H NMR (400 MHz, CDCl₃) δ 7.61 - 7.35 (m, 2H), 7.25 - 7.03 (m, 2H), 6.84 - 6.55 (m, 1H), 5.40 - 4.10 (m, 1H), 3.76 - 3.65 (m, 2H), 3.62 - 3.46 (m, 4H), 3.35 (s, 3H), 3.22 - 3.11 (m, 2H), 3.07 - 2.71 (m, 2H), 2.61 (s, 3H), 2.47 - 2.36 (m, 2H), 2.30 (br s, 3H), 2.17 - 2.00 (m, 2H), 1.88 - 1.76 (m, 3H), 1.61 - 1.52 (m, 2H), 1.35 - 1.24 (m, 4H), 1.19 - 1.08 (m, 1H), 0.95 - 0.87 (m, 6H), 0.86 - 0.80 (m, 1H), 0.79 - 0.06 (m, 1H). |
| **201-1** | 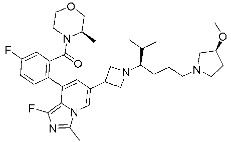 | 624.3 | ¹H NMR (400 MHz, CD3OD) δ 7.89 - 7.71 (m, 1H), 7.67 - 7.49 (m, 1H), 7.40 - 7.14 (m, 2H), 6.86 - 6.59 (m, 1H), 4.62 - 4.32 (m, 1H), 4.10 - 3.90 (m, 1H), 3.81 - 3.52 (m, 5H), 3.29 - 3.21 (m, 5H), 3.18 - 2.83 (m, 2H), 2.78 - 2.64 (m, 3H), 2.63 - 2.56 (m, 3H), 2.56 - 2.40 (m, 3H), 2.38 - 1.97 (m, 3H), 1.92 - 1.73 (m, 2H), 1.70 - 1.52 (m, 2H), 1.48 - 1.13 (m, 4H), 1.09 - 0.51 (m, 8H). |
| **201-2** | 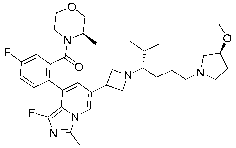 | 624.4 | ¹H NMR (400 MHz, CDCl₃) δ 7.54 - 7.37 (m, 2H), 7.24 - 7.05 (m, 2H), 6.78 - 6.55 (m, 1H), 5.38 - 4.10 (m, 1H), 3.97 - 3.88 (m, 1H), 3.76 - 3.65 (m, 2H), 3.62 - 3.33 (m, 3H), 3.28 (s, 3H), 3.19 - 3.09 (m, 2H), 3.06 - 2.91 (m, 1H),2.83 - 2.75 (m, 1H), 2.73 - 2.65 (m, 1H), 2.61 (s, 3H), 2.59 - 2.52 (m, 1H), 2.49 - 2.39 (m, 2H), 2.14 - 2.03 (m, 3H), 1.99 -1.91 (m, 1H), 1.88 - 1.72 (m, 3H), 1.67 - 1.54 (m, 2H), 1.35 - 1.25 (m, 3H), 1.19 - 1.07 (m, 1H), 0.89 (br t, J = 7.6 Hz, 6H),0.85 - 0.69 (m, 1H), 0.68 - 0.04 (m, 1H). |
| **202** | 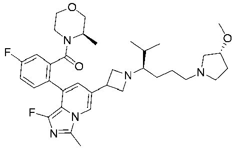 | 624.5 | ¹H NMR (400 MHz, CDCl₃) δ 7.50 - 7.27 (m, 2H), 7.12 (br s, 2H), 6.73 - 6.45 (m, 1H), 4.58 - 4.03 (m, 1H), 3.89-3.24 (m, 7H), 3.20 (s, 3H), 3.17 - 2.84 (m, 4H), 2.74 - 2.52 (m, 5H), 2.52 - 2.29 (m, 4H), 2.03 - 1.91 (m, 2H), 1.78 - 1.68 (m,2H), 1.54 - 1.41 (m, 2H), 1.34 - 1.01 (m, 4H), 0.99 - 0.49 (m, 8H). |
| **203** | 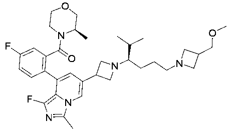 | 624.4 | ¹H NMR (400 MHz, CDCl₃) δ 7.86 - 7.51 (m, 2H), 7.36 - 7.13 (m, 2H), 6.87 - 6.59 (m, 1H), 4.74 - 3.98 (m, 2H),3.86 - 3.53 (m, 5H), 3.52 - 3.36 (m, 5H), 3.33 (s, 3H), 3.25 - 2.95 (m, 5H), 2.77 - 2.69 (m, 1H), 2.60 (s, 3H), 2.52 - 2.40 (m,2H), 2.27 - 2.13 (m, 1H), 1.91 - 1.15 (m, 8H), 0.97 - 0.86 (m, 6H), 0.83 - 0.59 (m, 1H). |
| **205** | 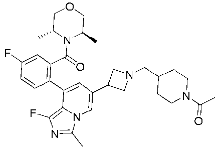 | 580.2 | ¹H NMR (400 MHz, DMSO-*d*₆) δ 7.89 (s, 1H), 7.55 - 7.47 (m, 1H), 7.46 - 7.34 (m, 2H), 6.61 (s, 1H), 4.32 (s, 1H), 3.80 - 3.47 (m, 6H), 3.28 - 2.88 (m, 5H), 2.80 - 2.52 (m, 4H), 2.49 - 2.41 (m, 2H), 2.30 (d, J = 6.8 Hz, 2H), 1.96 (s, 3H), 1.76 - 1.59 (m, 2H), 1.55 - 1.41 (m, 1H), 1.15 - 0.58 (m, 8H). |
| **208** | 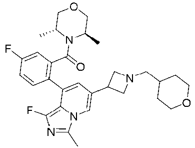 | 539.3 | ¹H NMR (400 MHz, CD₃OD) δ 7.86 (s, 1H), 7.58 - 7.46 (m, 1H), 7.38 - 7.27 (m, 2H), 6.67 (s, 1H), 3.96 - 3.88 (m, 4H), 3.86 - 3.75 (m, 2H), 3.55 - 3.46 (m, 2H), 3.41 (dt, J = 1.6, 12.0 Hz, 3H), 2.69 - 2.62 (m, 2H), 2.61 (s, 3H), 1.80 - 1.70 (m, 1H), 1.70 - 1.62 (m, 2H), 1.42 - 1.23 (m, 6H), 1.19 - 1.01 (m, 4H), 0.93 - 0.82 (m, 2H). |
| **210** | 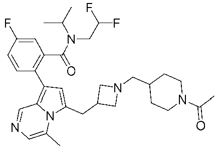 | 584.4 | ¹H NMR (400 MHz, CD₃OD) δ 8.51 (s, 1H), 7.59 - 7.50 (m, 1H), 7.33 (dt, J = 2.4, 8.4 Hz, 1H), 7.26 - 7.11 (m, 2H), 6.68 (s, 1H), 6.14 - 5.71 (m, 1H), 4.55 - 4.43 (m, 1H), 3.95 - 3.84 (m, 1H), 3.71 - 3.50 (m, 6H), 3.41 - 3.32 (m, 1H), 3.12 - 2.99 (m, 3H), 2.93 (td, J = 6.8, 14.0 Hz, 1H), 2.83 (s, 3H), 2.64 - 2.54 (m, 1H), 2.43 (d, J = 6.8 Hz, 2H), 2.07 (s, 3H), 1.83 - 1.59 (m, 3H), 1.33 - 0.99 (m, 3H), 0.95 (d, J = 6.4 Hz, 3H), 0.34 (d, J = 6.8 Hz, 2H). |
| **214** | 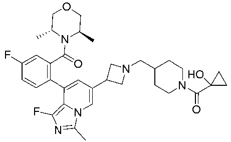 | 622.3 | ¹H NMR (400 MHz, CDCl₃) δ 7.48 - 7.37 (m, 2H), 7.24 - 7.16 (m, 2H), 6.66 (s, 1H), 4.52 (s, 2H), 3.98 - 3.57(m, 6H), 3.43 - 3.04 (m, 6H), 2.94 - 2.64 (m, 4H), 2.62 (s, 3H), 2.54 - 2.40 (m, 2H), 1.85 - 1.75 (m, 3H), 1.30 - 1.18 (m,3H), 1.14 - 1.08 (m, 4H), 1.05 - 1.02 (m, 1H), 0.98 - 0.94 (m, 2H). |
| **215** | 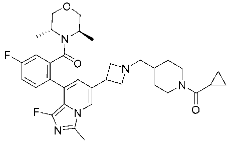 | 606.4 | ¹H NMR (400 MHz, CDCl₃) δ 7.46 - 7.38 (m, 2H), 7.25 - 7.15 (m, 2H), 6.61 (s, 1H), 4.64 - 4.50 (m, 1H), 4.21(d, J = 12.8 Hz, 1H), 3.92 - 3.56 (m, 5H), 3.46 - 2.74 (m, 7H), 2.62 (s, 3H), 2.60 - 2.53 (m, 1H), 2.46 - 2.36 (m, 2H), 1.91 -1.80 (m, 1H), 1.78 - 1.71 (m, 2H), 1.28 - 0.87 (m, 11H), 0.79 - 0.69 (m, 2H) |
| **216** | 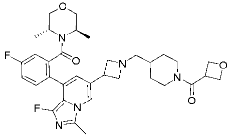 | 622.5 | ¹H NMR (400 MHz, CDCl₃) δ 7.47 - 7.35 (m, 2H), 7.24 - 7.14 (m, 2H), 6.67 - 6.55 (m, 1H), 4.95 - 4.87 (m,2H), 4.82 - 4.75 (m, 2H), 4.64 - 4.56 (m, 1H), 4.05 - 3.94 (m, 1H), 3.93 - 3.50 (m, 6H), 3.40 - 3.08 (m, 5H), 2.92 (br t, J =12.0 Hz, 2H), 2.61 (s, 3H), 2.59 - 2.54 (m, 1H), 2.45 - 2.33 (m, 2H), 1.86 - 1.73 (m, 2H), 1.58 - 1.49 (m, 1H), 1.21 - 0.93 (m,8H). |
| **217** | 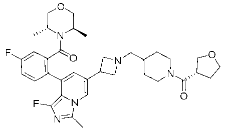 | 636.5 | ¹H NMR (400 MHz, CDCl₃) δ 7.47 - 7.38 (m, 2H), 7.24 - 7.17 (m, 2H), 6.67 - 6.54 (m, 1H), 4.67 - 4.57 (m,1H), 4.06 - 3.97 (m, 1H), 3.96 - 3.82 (m, 5H), 3.81 - 3.53 (m, 5H), 3.44 - 2.77 (m, 8H), 2.62 (s, 3H), 2.59 - 2.52 (m, 1H), 2.49-2.34 (m, 2H), 2.30 - 2.19 (m, 1H), 2.12 - 2.01 (m, 1H), 1.92 - 1.72 (m, 2H), 1.23 - 0.95 (m, 8H). |
| **218** | 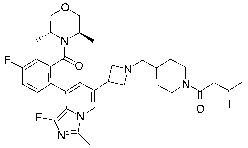 | 622.4 | ¹H NMR (400 MHz, CDCl₃) δ 7.46 - 7.37 (m, 2H), 7.24 - 7.16 (m, 2H), 6.60 (s, 1H), 4.63 (d, J = 13.2 Hz, 1H),3.96 - 3.54 (m, 7H), 3.41 - 2.78 (m, 7H), 2.62 (s, 3H), 2.57 - 2.49 (m, 1H), 2.46 - 2.35 (m, 2H), 2.23 - 2.19 (m, 2H), 2.16 -2.05 (m, 1H), 1.86 - 1.70 (m, 3H), 1.18 - 1.02 (m, 7H), 0.97 (d, J = 6.4 Hz, 6H). |
| **219** | 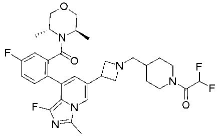 | 616.3 | ¹H NMR (400 MHz, CDCl₃) δ 7.53 - 7.35 (m, 2H), 7.25 - 7.15 (m, 2H), 6.69 - 6.55 (m, 1H), 6.26 - 5.94 (m,1H), 4.51 (d, J = 13.2 Hz, 1H), 4.09 (d, J = 13.2 Hz, 1H), 3.96 - 3.54 (m, 5H), 3.52 - 3.15 (m, 4H), 3.13 - 2.87 (m, 2H), 2.84-2.64 (m, 2H), 2.62 (s, 3H), 2.59 - 2.37 (m, 2H), 1.92 - 1.80 (m, 2H), 1.75 - 1.67 (m, 1H), 1.32 - 0.93 (m, 8H). |
| **221** | 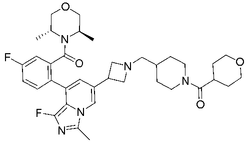 | 650.4 | ¹H NMR (400 MHz, CD₃OD) δ 7.85 (s, 1H), 7.60 - 7.50 (m, 1H), 7.40 - 7.29 (m, 2H), 6.70 (s, 1H), 4.60 (s, 4H), 4.53 (d, J = 13.2 Hz, 1H), 4.09 (d, J = 13.2 Hz, 1H), 3.97 (d, J = 11.2 Hz, 2H), 3.81 - 3.69 (m, 4H), 3.52 (t, J = 11.8 Hz, 3H), 3.11 (t, J = 12.2 Hz, 1H), 2.96 (ddd, J = 3.6, 8.0, 11.6 Hz, 2H), 2.63 (s, 3H), 2.50 (d, J = 6.8 Hz, 2H), 1.92 - 1.67 (m, 6H), 1.61 (d, J = 12.4 Hz, 2H), 1.39 - 1.26 (m, 1H), 1.23 - 0.92 (m, 8H). |
| **222** | 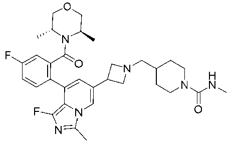 | 595.4 | ¹H NMR (400 MHz, CD₃OD) δ 7.85 (s, 1H), 7.59 - 7.50 (m, 1H), 7.39 - 7.29 (m, 2H), 6.69 (s, 1H), 4.60 (s, 2H), 3.99 (d, J = 13.6 Hz, 2H), 3.94 - 3.63 (m, 6H), 3.39 - 3.34 (m, 1H), 2.81 - 2.74 (m, 2H), 2.72 (s, 3H), 2.63 (s, 3H), 2.52 (d, J = 6.4 Hz, 2H), 2.06 (s, 2H), 1.75 (d, J = 12.8 Hz, 2H), 1.70 - 1.57 (m, 1H), 1.23 - 0.66 (m, 9H). |
| **223** | 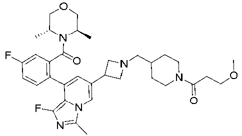 | 624.4 | ¹H NMR (400 MHz, CD₃OD) δ 7.83 (s, 1H), 7.56 - 7.48 (m, 1H), 7.38 - 7.29 (m, 2H), 6.67 (s, 1H), 4.79 - 4.39 (m, 4H), 4.00 (d, J = 12.4 Hz, 1H), 3.96 - 3.66 (m, 6H), 3.65 (t, J = 6.4 Hz, 2H), 3.32 (s, 3H), 3.12 - 3.02 (m, 1H), 2.66 - 2.62 (m, 2H), 2.61 (s, 3H), 2.50 (d, J = 6.8 Hz, 2H), 2.04 (s, 1H), 1.87 - 1.74 (m, 2H), 1.73 - 1.63 (m, 1H), 1.43 - 0.64 (m, 10H). |
| **224** | 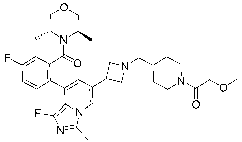 | 610.0 | ¹H NMR (400 MHz, CD₃OD) δ 7.98 - 7.81 (m, 1H), 7.57 - 7.47 (m, 1H), 7.38 - 7.26 (m, 2H), 6.79 - 6.45 (m, 1H), 4.58 (s, 2H), 4.48 (d, J = 12.8 Hz, 1H), 4.14 (q, J = 14.0 Hz, 3H), 3.92 - 3.72 (m, 7H), 3.41 - 3.36 (m, 4H), 3.08 - 2.92 (m, 2H), 2.66 - 2.59 (m, 3H), 2.57 (d, J = 6.4 Hz, 1H), 2.08 - 1.92 (m, 1H), 1.85 - 1.63 (m, 3H), 1.34 - 0.92 (m, 9H). |
| **225** | 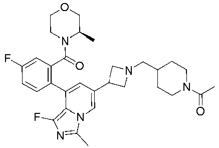 | 566.2 | ¹H NMR (400 MHz, DMSO-*d*₆) δ 7.86 (s, 1H), 7.65 - 7.47 (m, 1H), 7.45 - 7.24 (m, 2H), 6.86 - 6.51 (m, 1H), 4.47 - 3.39 (m, 9H), 3.16 - 2.80 (m, 5H), 2.58 - 2.52 (m, 3H), 2.49 - 2.42 (m, 2H), 2.34 - 2.26 (m, 2H), 2.00 - 1.91 (m, 3H), 1.78 - 1.58 (m, 2H), 1.56 - 1.44 (m, 1H), 1.24 - 0.62 (m, 5H). |
| **226** | 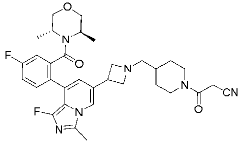 | 605.2 | ¹H NMR (400 MHz, CD₃OD) δ 7.83 (s, 1H), 7.57 - 7.49 (m, 1H), 7.38 - 7.29 (m, 2H), 6.74 - 6.64 (m, 1H), 4.52 - 4.41 (m, 1H), 3.97 - 3.59 (m, 7H), 3.30 - 3.22 (m, 5H), 3.15 - 3.07 (m, 1H), 2.95 - 2.79 (m, 1H), 2.72 - 2.65 (m, 1H), 2.63 - 2.58 (m, 3H), 2.51 - 2.43 (m, 2H), 1.87 - 1.75 (m, 2H), 1.73 - 1.62 (m, 1H), 1.33 - 0.74 (m, 9H). |
| **227** | 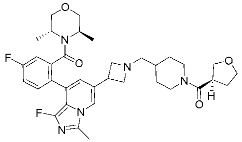 | 636.4 | ¹H NMR (400 MHz, DMSO-*d₆*) δ 7.90 (s, 1H), 7.54 - 7.48 (m, 1H), 7.46 - 7.36 (m, 2H), 6.68 - 6.53 (m, 1H), 4.37 - 4.30 (m, 1H), 3.98 - 3.78 (m, 3H), 3.70 - 3.62 (m, 5H), 3.36 - 3.25 (m, 3H), 3.19 - 3.11 (m, 3H), 2.99 - 2.93 (m, 1H), 2.56 - 2.53 (m, 3H), 2.35 - 2.31 (m, 2H), 2.08 - 1.93 (m, 4H), 1.76 - 1.46 (m, 4H), 1.12 - 0.74 (m, 9H). |
| **229** | 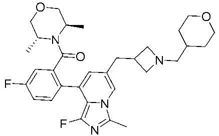 | 553.2 | ¹H NMR (400 MHz, DMSO-*d*₆) δ 7.85 (s, 1H), 7.52 - 7.45 (m, 1H), 7.43 - 7.36 (m, 2H), 6.35 - 6.22 (m, 1H), 3.83 - 3.75 (m, 3H), 3.40 - 3.33 (m, 3H), 3.28 - 3.19 (m, 4H), 2.96 - 2.89 (m, 2H), 2.73 - 2.66 (m, 3H), 2.56 - 2.52 (m, 3H), 2.34 - 2.29 (m, 2H), 2.10 - 2.03 (m, 1H), 1.57 - 1.47 (m, 3H), 1.18 - 0.72 (m, 9H). |
| **231** | 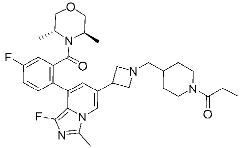 | 594.2 | ¹H NMR (400 MHz, CD₃OD) δ 8.63 - 8.38 (m, 1H), 8.20 - 7.78 (m, 1H), 7.68 - 7.16 (m, 3H), 6.92 - 6.40 (m, 1H), 4.70 - 3.39 (m, 11H), 2.96 (s, 11H), 2.12 - 1.67 (m, 3H), 1.10 (s, 11H). |
| **233** | 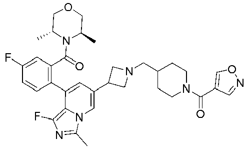 | 633.4 | ¹H NMR (400 MHz, DMSO-*d₆*) δ 9.00 (s, 1H), 8.05 (s, 1H), 7.56 - 7.35 (m, 4H), 6.79 - 6.52 (m, 1H), 4.26 - 4.17 (m, 1H), 4.12 (d, J = 13.2 Hz, 2H), 4.05 - 3.79 (m, 4H), 3.73 - 3.60 (m, 2H), 3.09 (q, J = 7.4 Hz, 2H), 3.01 - 2.90 (m, 2H), 2.65 (t, J = 12.6 Hz, 3H), 2.57 (s, 3H), 1.77 - 1.69 (m, 1H), 1.63 (d, J = 12.4 Hz, 2H), 1.25 - 1.15 (m, 3H), 1.14 - 0.96 (m, 6H). |
| **234** | 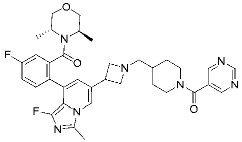 | 644.4 | ¹H NMR (400 MHz, DMSO-*d₆*) δ 9.25 (s, 1H), 8.85 (s, 2H), 7.88 (s, 1H), 7.55 - 7.47 (m, 1H), 7.43 - 7.37 (m, 2H), 6.76 - 6.47 (m, 1H), 4.44 (d, J = 12.0 Hz, 1H), 3.73 - 3.60 (m, 2H), 3.54 (s, 4H), 3.49 (s, 1H), 3.14 - 3.03 (m, 4H), 2.78 (t, J = 12.0 Hz, 1H), 2.55 (s, 3H), 2.33 (d, J = 6.4 Hz, 2H), 1.85 - 1.75 (m, 1H), 1.72 - 1.51 (m, 3H), 1.25 - 0.88 (m, 9H). |
| **235** | 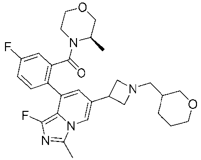 | 525.3 | ¹H NMR (400 MHz, CD₃OD) δ 7.80 (d, J = 10.4 Hz, 1H), 7.64 - 7.49 (m, 1H), 7.38 - 7.17 (m, 2H), 6.88 - 6.59 (m, 1H), 4.64 - 4.33 (m, 1H), 4.10 - 3.88 (m, 1H), 3.85 - 3.77 (m, 1H), 3.76 - 3.66 (m, 3H), 3.63 - 3.53 (m, 1H), 3.44 - 3.33 (m, 2H), 3.29 - 3.10 (m, 4H), 3.08 - 2.65 (m, 2H), 2.59 (s, 3H), 2.51 - 2.35 (m, 2H), 2.33 - 1.93 (m, 1H), 1.92 - 1.79 (m, 1H), 1.75 - 1.51 (m, 3H), 1.37 - 1.07 (m, 3H), 0.86 - 0.55 (m, 1H). |
| **236** | 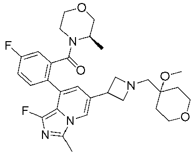 | 555.2 | ¹H NMR (400 MHz, DMSO-*d₆*) δ 7.95 - 7.78 (m, 1H), 7.65 - 7.47 (m, 1H), 7.44 - 7.25 (m, 2H), 6.82 - 6.49 (m, 1H), 4.38 - 3.44 (m, 10H), 3.40 - 2.64 (m, 11H), 2.55 (s, 3H), 1.64 - 1.43 (m, 4H), 1.27 - 0.49 (m, 3H). |
| **239** | 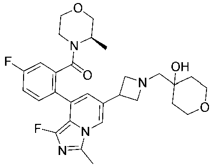 | 541.2 | ¹H NMR (400 MHz, CD₃OD) δ 7.77 (s, 1H), 7.67 - 7.49 (m, 1H), 7.41 - 7.16 (m, 2H), 7.10 - 6.73 (m, 1H), 4.63 - 4.06 (m, 2H), 3.83 - 3.53 (m, 9H), 3.38 - 3.33 (m, 2H), 3.19 - 2.87 (m, 2H), 2.64 - 2.51 (m, 5H), 2.28 - 1.86 (m, 1H), 1.71 - 1.59 (m, 2H), 1.56 - 1.46 (m, 2H), 1.35 - 1.25 (m, 1H), 1.24 - 1.09 (m, 1H), 0.85 - 0.56 (m, 1H). |
| **240** | 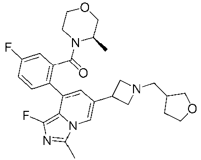 | 511.2 | ¹H NMR (400 MHz, DMSO-*d₆*) δ 7.97 -7.77 (m, 1H), 7.66 - 7.47 (m, 1H), 7.44 - 7.22 (m, 2H), 6.83 - 6.45 (m, 1H), 4.43 - 3.89 (m, 1H), 3.79 - 3.46 (m, 8H), 3.32 - 3.28 (m, 3H), 3.16 - 2.78 (m, 4H), 2.58 - 2.52 (m, 3H), 2.45 - 2.36 (m, 2H), 2.20 - 2.07 (m, 1H), 1.98 - 1.85 (m, 1H), 1.56 - 1.43 (m, 1H), 1.27 - 0.64 (m, 3H). |
| **242** | 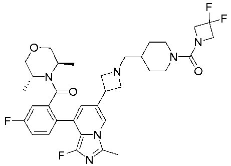 | 657.4 | ¹H NMR (400 MHz, DMSO-*d₆*) δ 7.89 (s, 1H), 7.55 - 7.36 (m, 3H), 6.60 (s, 1H), 4.28 (t, J = 12.8 Hz, 4H), 3.80 - 3.42 (m, 8H), 3.23 - 3.03 (m, 4H), 2.81 - 2.63 (m, 3H), 2.55 (s, 3H), 2.34 - 2.26 (m, 2H), 1.72 - 1.59 (m, 2H), 1.52 - 1.36 (m, 1H), 1.15 - 0.67 (m, 8H). |
| **246** | 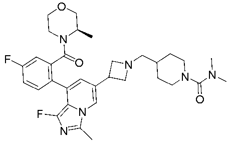 | 595.0 | ¹H NMR (400 MHz, CD₃OD) δ 7.71 (d, J = 9.2 Hz, 1H), 7.55 - 7.41 (m, 1H), 7.26 - 7.07 (m, 2H), 6.81 - 6.51 (m, 1H), 4.49 (s, 2H), 4.41 - 4.20 (m, 1H), 3.72 - 3.64 (m, 3H), 3.59 - 3.49 (m, 3H), 2.72 (s, 6H), 2.70 (d, J = 2.4 Hz, 1H), 2.68 - 2.61 (m, 2H), 2.50 (s, 3H), 2.44 (d, J = 6.8 Hz, 2H), 1.69 - 1.61 (m, 2H), 1.54 - 1.48 (m, 1H), 1.29 - 1.25 (m, 1H), 1.19 (s, 2H), 1.16 - 1.04 (m, 4H), 0.82 - 0.74 (m, 1H), 0.73 - 0.65 (m, 1H). |
| **247** | 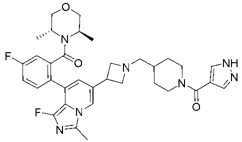 | 632.2 | ¹H NMR (400 MHz, CD₃OD) δ 7.89 (s, 1H), 7.58 - 7.48 (m, 1H), 7.39 - 7.27 (m, 2H), 6.92 - 6.60 (m, 1H), 4.06 - 3.97 (m, 2H), 3.91 - 3.80 (m, 2H), 3.68 - 3.59 (m, 2H), 2.77 (d, J = 5.6 Hz, 2H), 2.62 (s, 3H), 2.52 - 1.95 (m, 2H), 1.89 - 1.79 (m, 3H), 1.64 - 1.51 (m, 1H), 1.40 - 1.19 (m, 11H), 1.16 - 1.08 (m, 2H), 1.02 - 0.96 (m, 1H), 0.93 - 0.81 (m, 3H). |
| **248** | 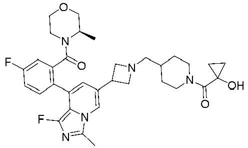 | 608.2 | ¹H NMR (400 MHz, DMSO-*d₆*) δ 7.85 (s, 1H), 7.64 - 7.48 (m, 1H), 7.44 - 7.25 (m, 2H), 6.83 - 6.52 (m, 1H), 6.26 - 6.17 (m, 1H), 4.47 - 3.87 (m, 3H), 3.79 - 3.48 (m, 5H), 3.28 - 2.64 (m, 7H), 2.56 - 2.52 (m, 3H), 2.34 - 2.27 (m, 2H), 1.78 - 1.61 (m, 2H), 1.58 - 1.47 (m, 1H), 1.26 - 0.51 (m, 10H). |
| **249** | 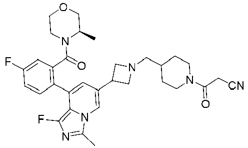 | 591.3 | ¹H NMR (400 MHz, DMSO-*d₆*) δ 7.92 - 7.79 (m, 1H), 7.62 - 7.47 (m, 1H), 7.44 - 7.25 (m, 2H), 6.89 - 6.50 (m, 1H), 4.32 - 3.92 (m, 4H), 3.77 - 3.48 (m, 6H), 3.15 - 3.05 (m, 2H), 3.03 - 2.89 (m, 2H), 2.69 - 2.53 (m, 6H), 2.34 - 2.28 (m, 2H), 1.79 - 1.62 (m, 2H), 1.58 - 1.44 (m, 1H), 1.28 - 0.87 (m, 5H), 0.81 - 0.53 (m, 1H). |
| **250** | 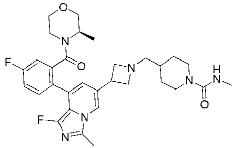 | 581.3 | ¹H NMR (400 MHz, DMSO-*d₆*) δ 7.85 (s, 1H), 7.65 - 7.45 (m, 1H), 7.44 - 7.25 (m, 2H), 6.83 - 6.49 (m, 1H), 6.35 - 6.21 (m, 1H), 4.45 - 3.38 (m, 8H), 3.22 - 2.75 (m, 5H), 2.62 - 2.51 (m, 9H), 2.32 - 2.25 (m, 2H), 1.72 - 1.52 (m, 2H), 1.47 - 1.33 (m, 1H), 1.26 - 0.85 (m, 4H), 0.82 - 0.56 (m, 1H). |
| **251** | 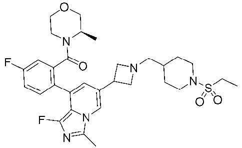 | 616.4 | ¹H NMR (400 MHz, DMSO-*d₆*) δ 7.86 (s, 1H), 7.63 - 7.48 (m, 1H), 7.45 - 7.27 (m, 2H), 6.83 - 6.49 (m, 1H), 4.36 - 3.88 (m, 1H), 3.72 - 3.51 (m, 6H), 3.32 - 3.20 (m, 5H), 3.15 - 3.05 (m, 2H), 3.03 - 2.97 (m, 2H), 2.79 - 2.70 (m, 2H), 2.57 - 2.52 (m, 3H), 2.36 - 2.29 (m, 2H), 1.86 - 1.68 (m, 2H), 1.45 - 1.34 (m, 1H), 1.23 - 1.04 (m, 7H), 0.81 - 0.60 (m, 1H). |
| **252** | 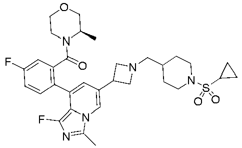 | 628.3 | ¹H NMR (400 MHz, DMSO-*d₆*) δ 7.86 (s, 1H), 7.63 - 7.47 (m, 1H), 7.46 - 7.19 (m, 2H), 6.84 - 6.47 (m, 1H), 4.41 - 3.88 (m, 1H), 3.75 - 3.49 (m, 6H), 3.33 - 3.30 (m, 6H), 3.16 - 3.04 (m, 2H), 2.82 - 2.72 (m, 2H), 2.56 - 2.53 (m, 3H), 2.38 - 2.28 (m, 2H), 1.89 - 1.68 (m, 2H), 1.49 - 1.33 (m, 1H), 1.28 - 0.84 (m, 8H), 0.78 - 0.51 (m, 1H). |
| **253** | 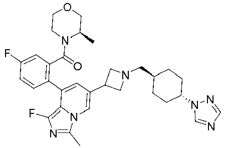 | 590.3 | ¹H NMR (400 MHz, DMSO-*d₆*) δ 8.52 (s, 1H), 8.03 - 7.73 (m, 2H), 7.65 - 7.23 (m, 3H), 6.91 - 6.46 (m, 1H), 4.47 - 3.39 (m, 8H), 3.28 - 2.76 (m, 5H), 2.56 (s, 3H), 2.32 (d, J = 6.8 Hz, 2H), 2.08 - 1.64 (m, 6H), 1.43 - 0.97 (m, 5H), 0.86 - 0.57 (m, 1H). |
| 254 | 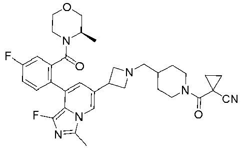 | 617.4 | ¹H NMR (400 MHz, DMSO-*d₆*) δ 7.97 -7.76 (m, 1H), 7.64 - 7.23 (m, 3H), 6.85 - 6.49 (m, 1H), 4.42 - 3.35 (m, 9H), 3.30 - 2.74 (m, 7H), 2.55 (s, 3H), 2.38 - 2.28 (m, 2H), 1.89 - 1.67 (m, 2H), 1.64 - 1.51 (m, 3H), 1.48 - 1.38 (m, 2H), 1.22 - 0.54 (m, 5H). |
| **256** | 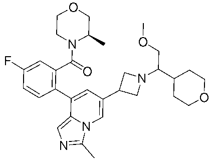 | 551.3 | ¹H NMR (400 MHz, CDCl₃) δ 7.88 - 7.46 (m, 2H), 7.26 - 7.10 (m, 3H), 7.01 - 6.70 (m, 1H), 4.63 - 4.09 (m,lH), 4.05 - 3.93 (m, 2H), 3.84 - 3.44 (m, 5H), 3.42 - 2.88 (m, 10H), 2.69 (s, 3H), 2.48 - 2.28 (m, 1H), 1.94 - 1.77 (m, 1H),1.67 - 1.41 (m, 7H), 1.33 - 1.26 (m, 1H), 1.17 - 1.03 (m, 1H), 0.81 - 0.40 (m, 1H). |
| **258** | 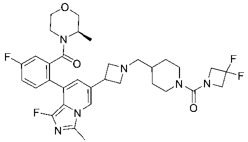 | 643.3 | ¹H NMR (400 MHz, DMSO-*d₆*) δ 7.93 - 7.79 (m, 1H), 7.63 - 7.24 (m, 3H), 6.83 - 6.49 (m, 1H), 4.28 (t, J = 13.2 Hz, 4H), 3.84 - 3.41 (m, 9H), 3.16 - 2.64 (m, 7H), 2.54 (s, 3H), 2.34 - 2.27 (m, 2H), 1.75 - 1.59 (m, 2H), 1.52 - 1.39 (m, 1H), 1.21 - 0.62 (m, 5H). |
| **259** | 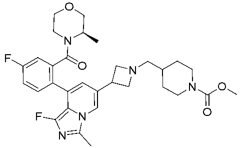 | 582.3 | ¹H NMR (400 MHz, DMSO-*d₆*) δ 7.92 - 7.77 (m, 1H), 7.62 - 7.47 (m, 1H), 7.43 - 7.25 (m, 2H), 6.84 - 6.50 (m, 1H), 4.37 - 3.69 (m, 4H), 3.61 - 3.46 (m, 8H), 3.16 - 3.02 (m, 4H), 2.81 - 2.63 (m, 3H), 2.56 - 2.53 (m, 3H), 2.32 - 2.28 (m, 2H), 1.78 - 1.58 (m, 2H), 1.51 - 1.39 (m, 1H), 1.25 - 1.04 (m, 2H), 1.03 - 0.91 (m, 2H), 0.80 - 0.55 (m, 1H). |
| **261** | 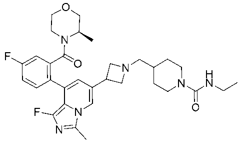 | 595.2 | ¹H NMR (400 MHz, CD₃OD) δ 7.80 (d, J = 10.8 Hz, 1H), 7.65 - 7.52 (m, 1H), 7.38 - 7.17 (m, 2H), 6.87 - 6.60 (m, 1H), 4.63 - 4.33 (m, 1H), 4.13 - 3.53 (m, 8H), 3.32 (s, 2H), 3.23 (s, 2H), 3.17 (q, J = 7.4 Hz, 2H), 3.05 - 2.86 (m, 1H), 2.78 - 2.71 (m, 2H), 2.60 (s, 3H), 2.47 (d, J = 6.4 Hz, 2H), 1.80 - 1.69 (m, 2H), 1.61 (tdd, J = 3.6, 7.2, 10.8 Hz, 1H), 1.30 (d, J = 6.4 Hz, 1H), 1.22 - 1.05 (m, 6H), 0.80 (d, J = 5.2 Hz, 1H). |
| **262** | 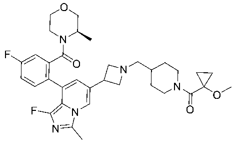 | 622.3 | ¹H NMR (400 MHz, DMSO-*d₆*) δ 7.86 (s, 1H), 7.67 - 7.46 (m, 1H), 7.44 - 7.16 (m, 2H), 6.90 - 6.43 (m, 1H), 4.57 - 4.05 (m, 3H), 4.03 - 3.62 (m, 1H), 3.36 (s, 5H), 3.18 (s, 3H), 3.15 - 2.61 (m, 6H), 2.55 (s, 3H), 2.39 - 2.25 (m, 2H), 1.85 - 1.65 (m, 2H), 1.63 - 1.46 (m, 1H), 1.35 - 0.48 (m, 10H). |
| **263-1** | 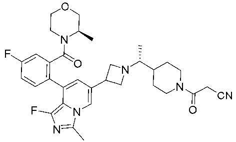 | 605.4 | ¹H NMR (400 MHz, CD₃OD) δ 7.83 (d, J = 14.0 Hz, 1H), 7.68 - 7.52 (m, 1H), 7.40 - 7.17 (m, 2H), 6.97 - 6.67 (m, 1H), 4.65 - 4.52 (m, 2H), 3.86 - 3.74 (m, 3H), 3.73 - 3.57 (m, 3H), 3.32 - 2.88 (m, 7H), 2.78 - 2.56 (m, 5H), 2.45 (s, 1H), 2.21- 1.93 (m, 1H), 1.77 (s, 2H), 1.67 - 1.55 (m, 1H), 1.38 - 1.16 (m, 4H), 0.92 (d, J = 5.6 Hz, 3H), 0.86 - 0.77 (m, 1H). |
| **263-2** | 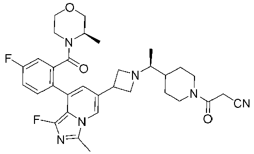 | 605.4 | ¹H NMR (400 MHz, CD₃OD) δ 7.86 - 7.74 (m, 1H), 7.65 - 7.50 (m, 1H), 7.39 - 7.16 (m, 2H), 6.93 - 6.58 (m, 1H), 4.63 - 4.32 (m, 2H), 3.86 - 3.54 (m, 6H), 3.31 (s, 2H), 3.27 - 2.88 (m, 5H), 2.81 - 2.63 (m, 1H), 2.60 (s, 4H), 2.39 (s, 1H), 2.27 - 1.94 (m, 1H), 1.74 (s, 2H), 1.67 - 1.51 (m, 1H), 1.47 - 1.11 (m, 4H), 0.88 (d, J = 6.0 Hz, 3H), 0.83 - 0.55 (m, 1H). |
| **264-1** | 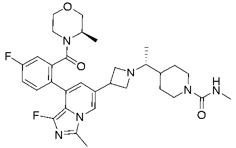 | 595.4 | ¹H NMR (400 MHz, CD₃OD) δ 7.81 (d, J = 12.2 Hz, 1H), 7.65 - 7.50 (m, 1H), 7.38 - 7.14 (m, 2H), 6.91 - 6.56 (m, 1H), 4.69 - 4.21 (m, 2H), 4.04 (t, J = 12.0 Hz, 2H), 3.85 - 3.52 (m, 5H), 3.40 - 3.31 (m, 2H), 3.27 - 3.17 (m, 2H), 3.05 - 2.86 (m, 1H), 2.79 - 2.72 (m, 1H), 2.70 (s, 3H), 2.69 - 2.64 (m, 1H), 2.60 (s, 3H), 2.44 - 2.31 (m, 1H), 1.74 - 1.60 (m, 2H), 1.59 - 1.44 (m, 1H), 1.34 - 1.12 (m, 4H), 0.88 (d, J = 6.0 Hz, 3H), 0.83 - 0.75 (m, 1H). |
| **264-2** | 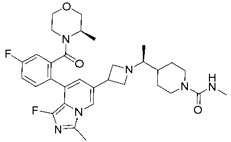 | 595.4 | ¹H NMR (400 MHz, CD₃OD) δ 7.80 (d, J = 11.6 Hz, 1H), 7.64 - 7.48 (m, 1H), 7.39 - 7.15 (m, 2H), 6.88 - 6.56 (m, 1H), 4.68 - 4.31 (m, 2H), 4.05 (t, J = 12.2 Hz, 2H), 3.81 - 3.53 (m, 5H), 3.40 - 3.31 (m, 2H), 3.27 - 3.21 (m, 1H), 3.20 - 3.14 (m, 1H), 3.06 - 2.88 (m, 1H), 2.80 - 2.73 (m, 1H), 2.71 (s, 3H), 2.69 - 2.64 (m, 1H), 2.60 (s, 3H), 2.37 (s, 1H), 1.70 - 1.60 (m, 2H), 1.58 - 1.45 (m, 1H), 1.33 - 1.13 (m, 4H), 0.87 (d, J = 6.4 Hz, 3H), 0.80 (d, J = 4.4 Hz, 1H). |
| **265** | 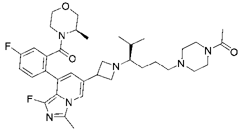 | 651.5 | ¹H NMR (400 MHz, CD₃OD) δ 7.87 - 7.72 (m, 1H), 7.66 - 7.51 (m, 1H), 7.50 - 7.14 (m, 3H), 6.89 - 6.56 (m, 1H), 4.58 (s, 3H), 3.81 - 3.52 (m, 9H), 3.29 - 3.22 (m, 2H), 2.60 (s, 3H), 2.52 - 2.35 (m, 6H), 2.33 - 2.24 (m, 1H), 2.08 (s, 3H), 1.87 (s, 1H), 1.60 (s, 2H), 1.47 - 1.37 (m, 1H), 1.36 - 1.24 (m, 3H), 1.21 - 1.12 (m, 1H), 0.98 - 0.89 (m, 6H), 0.86 - 0.75 (m, 1H). |
| **268** | 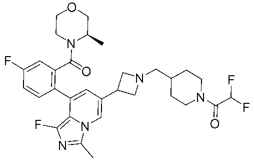 | 602.2 | ¹H NMR (400 MHz, DMSO-*d₆*) δ 7.96 - 7.77 (m, 1H), 7.65 - 7.24 (m, 3H), 6.92 - 6.50 (m, 2H), 4.43 - 3.34 (m, 9H), 3.27 - 2.64 (m, 7H), 2.54 (s, 3H), 2.36 - 2.27 (m, 2H), 1.89 - 1.50 (m, 3H), 1.22 - 0.52 (m, 5H). |
| **269** | 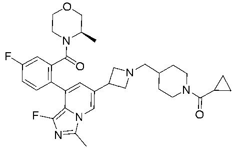 | 592.3 | ¹H NMR (400 MHz, DMSO-*d₆*) δ 8.03 - 7.72 (m, 1H), 7.66 - 7.18 (m, 3H), 6.93 - 6.44 (m, 1H), 4.48 - 3.35 (m, 9H), 3.29 - 2.73 (m, 7H), 2.55 (s, 3H), 2.37 - 2.26 (m, 2H), 1.99 - 1.47 (m, 4H), 1.22 - 0.52 (m, 9H). |
| **270** | 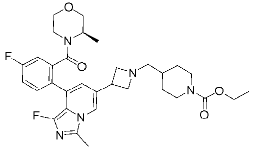 | 596.3 | ¹H NMR (400 MHz, CD₃OD) δ 7.92 - 7.75 (m, 1H), 7.68 - 7.49 (m, 1H), 7.44 - 7.14 (m, 2H), 6.92 - 6.59 (m, 1H), 4.72 - 4.34 (m, 3H), 4.14 - 4.06 (m, 4H), 3.84 - 3.48 (m, 5H), 3.28 - 2.97 (m, 3H), 2.91 - 2.66 (m, 3H), 2.60 (s, 3H), 2.54 - 2.42 (m, 2H), 1.81 - 1.55 (m, 3H), 1.37 - 1.03 (m, 7H), 0.85 - 0.56 (m, 1H). |
| **271** | 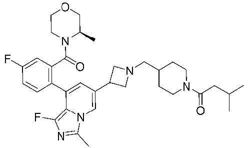 | 608.4 | ¹H NMR (400 MHz, DMSO-*d₆*) δ 7.85 (s, 1H), 7.63 - 7.48 (m, 1H), 7.45 - 7.21 (m, 2H), 6.84 - 6.50 (m, 1H), 4.45 - 3.36 (m, 9H), 3.18 - 2.75 (m, 5H), 2.54 (s, 3H), 2.48 - 2.43 (m, 1H), 2.30 (d, J = 6.8 Hz, 2H), 2.15 (d, J = 6.8 Hz, 2H), 2.02 - 1.86 (m, 1H), 1.81 - 1.60 (m, 2H), 1.58 - 1.42 (m, 1H), 1.26 - 0.50 (m, 12H). |
| **272** | 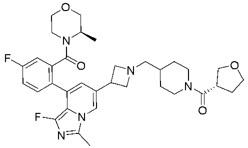 | 622.3 | ¹H NMR (400 MHz, DMSO-*d₆*) δ 7.93 - 7.79 (m, 1H), 7.62 - 7.49 (m, 1H), 7.45 - 7.25 (m, 2H), 6.81 - 6.49 (m, 1H), 4.40 - 3.45 (m, 14H), 3.17 - 2.74 (m, 6H), 2.56 - 2.53 (m, 3H), 2.34 - 2.28 (m, 2H), 2.02 - 1.92 (m, 2H), 1.80 - 1.63 (m, 2H), 1.59 - 1.44 (m, 1H), 1.27 - 0.53 (m, 6H). |
| **273** | 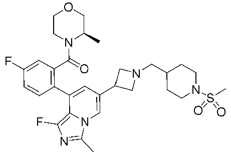 | 602.3 | ¹H NMR (400 MHz, DMSO-*d₆*) δ 7.94 - 7.75 (m, 1H), 7.63 - 7.49 (m, 1H), 7.46 - 7.25 (m, 2H), 6.83 - 6.50 (m, 1H), 4.45 - 3.37 (m, 9H), 3.31 - 3.24 (m, 2H), 3.16 - 2.96 (m, 3H), 2.84 - 2.80 (m, 3H), 2.69 - 2.61 (m, 2H), 2.57 - 2.52 (m, 3H), 2.37 - 2.30 (m, 2H), 1.89 - 1.69 (m, 2H), 1.45 - 1.30 (m, 1H), 1.26 - 1.00 (m, 4H), 0.83 - 0.55 (m, 1H). |
| **274** | 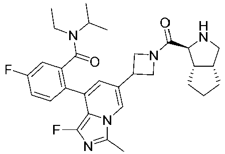 | 550.3 | ¹H NMR (400 MHz, DMSO-*d₆*) δ 8.00 - 7.94 (m, 1H), 7.58 - 7.49 (m, 1H), 7.41 - 7.28 (m, 2H), 6.66 - 6.48 (m, 1H), 4.70 - 4.46 (m, 1H), 4.34 - 4.12 (m, 2H), 4.02 - 3.76 (m, 2H), 3.52 - 3.36 (m, 1H), 3.29 - 2.65 (m, 5H), 2.56 - 2.53 (m, 3H), 2.37 - 2.26 (m, 1H), 1.82 - 1.19 (m, 7H), 1.04 - 0.72 (m, 7H), 0.47 - 0.31 (m, 2H). |
| **277** | 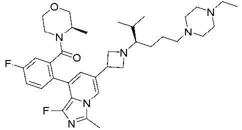 | 637.6 | ¹H NMR (400 MHz, CDCl₃) δ 7.57 - 7.31 (m, 2H), 7.25 - 7.04 (m, 2H), 6.81 - 6.49 (m, 1H), 4.68 - 4.10 (m,1H), 3.81 - 2.69 (m, 11H), 2.61 (s, 3H), 2.55 - 1.93 (m, 12H), 1.88 - 1.74 (m, 1H), 1.64 - 1.39 (m, 3H), 1.38 - 0.53 (m, 14H). |
| **278** | 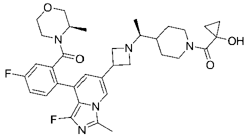 | 622.4 | ¹H NMR (400 MHz, DMSO-*d₆*) δ 7.94 - 7.78 (m, 1H), 7.66 - 7.21 (m, 3H), 6.90 - 6.43 (m, 1H), 6.29 - 6.16 (m, 1H), 4.66 - 3.37 (m, 9H), 3.25 - 2.71 (m, 6H), 2.57 - 2.52 (m, 3H), 2.24 - 2.15 (m, 1H), 1.76 - 1.39 (m, 3H), 1.28 - 0.98 (m, 4H), 0.92 - 0.46 (m, 9H). |
| **279** | 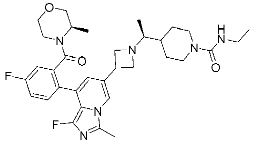 | 609.4 | ¹H NMR (400 MHz, DMSO-*d₆*) δ 7.92 - 7.79 (m, 1H), 7.61 - 7.26 (m, 3H), 6.87 - 6.42 (m, 1H), 6.38 - 6.30 (m, 1H), 4.41 - 3.63 (m, 4H), 3.62 - 3.38 (m, 5H), 3.31 - 2.74 (m, 9H), 2.57 - 2.53 (m, 3H), 2.22 - 2.13 (m, 1H), 1.64 - 1.31 (m, 3H), 1.25 - 1.04 (m, 3H), 1.02 - 0.92 (m, 4H), 0.77 - 0.54 (m, 4H). |
| **281** | 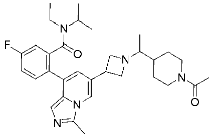 | 548.5 | ¹H NMR (400 MHz, DMSO-*d₆*) δ 7.96 - 7.89 (m, 1H), 7.73 - 7.63 (m, 1H), 7.43 - 7.26 (m, 2H), 7.08 - 6.99 (m, 1H), 6.87 - 6.66 (m, 1H), 4.50 - 4.38 (m, 1H), 3.83 (d, J = 12.4 Hz, 1H), 3.60 - 3.39 (m, 4H), 3.19 - 2.65 (m, 5H), 2.64 - 2.55 (m, 3H), 2.44 - 2.29 (m, 1H), 2.24 - 2.15 (m, 1H), 1.97 (s, 3H), 1.77 - 1.39 (m, 3H), 1.27 - 1.01 (m, 2H), 0.95 - 0.68 (m, 10H), 0.30 - 0.23 (m, 2H). |
| **281-1** | 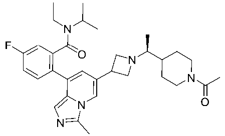 | 548.3 | ¹H NMR (400 MHz, DMSO-*d₆*) δ 7.94 (s, 1H), 7.75 - 7.61 (m, 1H), 7.47 - 7.23 (m, 2H), 7.04 (s, 1H), 6.93 - 6.65 (m, 1H), 4.51 - 4.18 (m, 1H), 3.94 - 3.75 (m, 1H), 3.61 - 3.42 (m, 4H), 3.21 - 2.74 (m, 5H), 2.60 (s, 3H), 2.45 - 2.30 (m, 1H), 2.21 (s, 1H), 1.98 (s, 3H), 1.79 - 1.39 (m, 3H), 1.29 - 0.70 (m, 12H), 0.27 (s, 2H). |
| **281-2** | 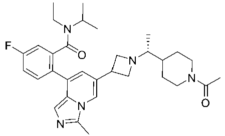 | 548.4 | ¹H NMR (400 MHz, DMSO-*d₆*) δ 7.93 (s, 1H), 7.68 (s, 1H), 7.51 - 7.18 (m, 2H), 7.03 (s, 1H), 6.94 - 6.62 (m, 1H), 4.54 - 4.21 (m, 1H), 3.93 - 3.75 (m, 1H), 3.50 (s, 4H), 3.22 - 2.77 (m, 5H), 2.66 - 2.54 (m, 3H), 2.45 - 2.32 (m, 1H), 2.20 (s, 1H), 1.97 (s, 3H), 1.74 - 1.38 (m, 3H), 1.23 - 1.03 (m, 2H), 0.99 - 0.63 (m, 10H), 0.27 (s, 2H). |
| **283-1** | 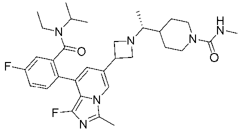 | 581.4 | ¹H NMR (400 MHz, CD₃OD) δ 7.81 - 7.74 (m, 1H), 7.59 - 7.52 (m, 1H), 7.30 (dt, J = 2.6, 8.4 Hz, 1H), 7.25 - 7.17 (m, 1H), 6.73 (d, J = 8.8 Hz, 1H), 4.59 (s, 1H), 4.05 (t, J = 11.8 Hz, 2H), 3.75 - 3.67 (m, 2H), 3.67 - 3.52 (m, 2H), 3.51 - 3.38 (m, 1H), 3.28 - 3.12 (m, 2H), 3.10 - 2.97 (m, 1H), 2.81 - 2.73 (m, 1H), 2.71 (s, 3H), 2.69 - 2.63 (m, 1H), 2.58 (s, 3H), 2.43 - 2.32 (m, 1H), 1.71 - 1.61 (m, 2H), 1.52 (t, J = 14.4 Hz, 1H), 1.34 - 1.09 (m, 3H), 1.00 (d, J = 6.4 Hz, 2H), 0.96 - 0.85 (m, 6H), 0.43 (d, J = 5.2 Hz, 2H). |
| **283-2** | 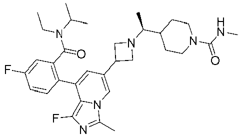 | 581.3 | ¹H NMR (400 MHz, DMSO-*d₆*) δ 7.87 - 7.80 (m, 1H), 7.56 - 7.48 (m, 1H), 7.39 - 7.25 (m, 2H), 6.73 - 6.55 (m, 1H), 6.33 -6.26 (m, 1H), 4.28 - 3.89 (m, 2H), 3.58 - 3.40 (m, 4H), 3.13 - 2.56 (m, 5H), 2.55 - 2.52 (m, 6H), 2.26 - 2.10 (m, 1H), 1.67 -1.30 (m, 3H), 1.16 - 0.68 (m, 13H), 0.39 (br s, 2H). |
| **284-1** | 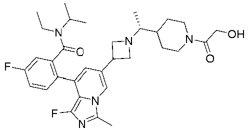 | 582.4 | ¹H NMR (400 MHz, CD₃OD) δ 7.83 - 7.74 (m, 1H), 7.62 - 7.49 (m, 1H), 7.31 (dt, J = 2.0, 8.4 Hz, 1H), 7.26 - 7.16 (m, 1H), 6.75 (d, J = 14.0 Hz, 1H), 4.57 (d, J = 11.6 Hz, 1H), 4.32 - 4.12 (m, 2H), 3.89 - 3.72 (m, 3H), 3.71 - 3.54 (m, 2H), 3.52 - 3.40 (m, 1H), 3.31 - 3.18 (m, 2H), 3.11 - 2.90 (m, 2H), 2.76 - 2.61 (m, 1H), 2.59 (s, 3H), 2.52 - 2.40 (m, 1H), 1.84 - 1.68 (m, 2H), 1.66 - 1.52 (m, 1H), 1.42 - 1.10 (m, 3H), 1.04 - 0.81 (m, 9H), 0.43 (d, J = 6.0 Hz, 2H). |
| **284-2** | 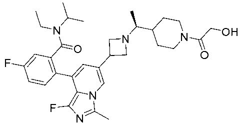 | 582.2 | ¹H NMR (400 MHz, DMSO-*d₆*) δ 7.90 - 7.77 (m, 1H), 7.58 - 7.47 (m, 1H), 7.40 - 7.23 (m, 2H), 6.78 - 6.53 (m, 1H), 4.46 -4.20 (m, 2H), 4.13 - 3.97 (m, 2H), 3.83 - 3.55 (m, 2H), 3.53 - 3.43 (m, 4H), 3.13 - 3.03 (m, 2H), 2.96 - 2.80 (m, 2H), 2.53 (s,3H), 2.47 - 2.34 (m, 1H), 2.25 - 2.15 (m, 1H), 1.77 - 1.39 (m, 3H), 1.26 - 0.72 (m, 12H), 0.46 - 0.31 (m, 2H). |
| **285** | 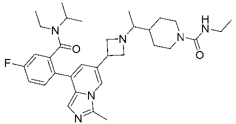 | 577.5 | ¹H NMR (400 MHz, DMSO-*d₆*) δ 7.96 - 7.91 (m, 1H), 7.72 - 7.63 (m, 1H), 7.42 - 7.27 (m, 2H), 7.06 - 7.00 (m, 1H), 6.83 - 6.70 (m, 1H), 6.37 - 6.30 (m, 1H), 4.00 (d, J = 11.2 Hz, 2H), 3.57 - 3.32 (m, 5H), 3.13 - 2.88 (m, 5H), 2.61 - 2.52 (m, 4H), 2.46 (s, 1H), 2.23 - 2.13 (m, 1H), 1.68 - 1.34 (m, 3H), 1.18 - 0.85 (m, 9H), 0.82 - 0.72 (m, 6H), 0.27 (dd, J = 2.8, 6.4 Hz, 2H). |
| **287** | 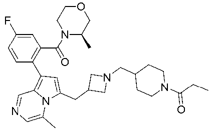 | 576.4 | ¹H NMR (400 MHz, DMSO-*d*₆) δ 8.62 - 8.38 (m, 1H), 7.64 - 7.44 (m, 1H), 7.39 - 7.17 (m, 3H), 6.62 - 6.51 (m, 1H), 4.37 - 3.88 (m, 2H), 3.83 - 3.35 (m, 8H), 3.13 - 2.66 (m, 10H), 2.31 - 2.20 (m, 4H), 1.80 - 1.37 (m, 4H), 1.26 - 1.13 (m, 1H), 1.06 - 0.85 (m, 6H), 0.63 - 0.29 (m, 1H). |
| **288** | 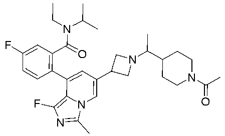 | 566.3 | ¹H NMR (400 MHz, DMSO-*d₆*) δ 7.84 (s, 1H), 7.57 - 7.47 (m, 1H), 7.39 - 7.26 (m, 2H), 6.75 - 6.54 (m, 1H), 4.51 - 4.18 (m, 1H), 3.88 - 3.76 (m, 1H), 3.58 - 3.38 (m, 4H), 3.26 - 2.57 (m, 5H), 2.53 (s, 3H), 2.45 - 2.29 (m, 1H), 2.18 (s, 1H), 1.96 (s, 3H), 1.77 - 1.34 (m, 3H), 1.27 - 1.01 (m, 2H), 0.99 - 0.65 (m, 10H), 0.39 (s, 2H). |
| **288-1** | 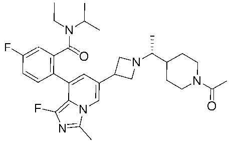 | 566.4 | ¹H NMR (400 MHz, CD₃OD) δ 7.82 - 7.74 (m, 1H), 7.60 - 7.50 (m, 1H), 7.30 (dt, J = 2.4, 8.4 Hz, 1H), 7.25 - 7.18 (m, 1H), 6.75 (br d, J = 14.4 Hz, 1H), 4.60 (d, J = 11.6 Hz, 2H), 3.97 (d, J = 13.2 Hz, 1H), 3.78 - 3.69 (m, 2H), 3.68 - 3.55 (m, 2H), 3.52 - 3.40 (m, 1H), 3.29 - 2.99 (m, 4H), 2.59 (s, 3H), 2.42 (s, 1H), 2.09 (s, 3H), 1.81 - 1.65 (m, 2H), 1.65 - 1.50 (m, 1H), 1.41 - 1.05 (m, 4H), 1.00 (d, J = 6.4 Hz, 2H), 0.96 - 0.81 (m, 6H), 0.43 (d, J = 5.6 Hz, 2H). |
| **288-2** | 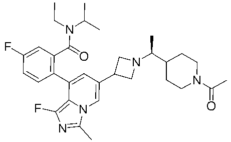 | 566.3 | ¹H NMR (400 MHz, DMSO-*d₆*) δ 7.92 - 7.75 (m, 1H), 7.58 - 7.25 (m, 3H), 6.79 - 6.54 (m, 1H), 4.51 - 4.18 (m, 1H), 3.89 -3.40 (m, 5H), 3.12 - 2.80 (m, 4H), 2.53 (s, 3H), 2.47 - 2.07 (m, 3H), 1.96 (s, 3H), 1.79 - 1.37 (m, 3H), 1.30 - 0.65 (m, 12H),0.50 - 0.29 (m, 2H). |
| **290** | 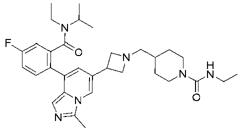 | 563.4 | ¹H NMR (400 MHz, CDCl₃) δ 7.72 - 7.60 (m, 1H), 7.57 - 7.47 (m, 1H), 7.23 - 7.07 (m, 3H), 6.95 - 6.81 (m, 1H),4.55 - 4.32 (m, 1H), 3.92 (br d, J = 12.0 Hz, 2H), 3.72 - 3.41 (m, 4H), 3.33 - 3.05 (m, 4H), 3.00 - 2.84 (m, 1H), 2.75 (br t, J =12.8 Hz, 2H), 2.67 (s, 3H), 2.46 - 2.33 (m, 2H), 1.81 - 1.70 (m, 2H), 1.62 - 1.19 (m, 3H), 1.14 (br t, J = 7.2 Hz, 5H), 1.00 -0.74 (m, 6H), 0.26 (m, 2H). |
| **292** | 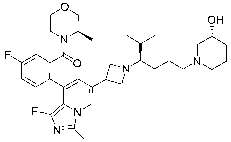 | 624.5 | ¹H NMR (400 MHz, CDCl₃) δ 7.58 - 7.34 (m, 2H), 7.25 - 7.06 (m, 2H), 6.84 - 6.49 (m, 1H), 4.70 - 4.07 (m,1H), 3.93 - 2.69 (m, 12H), 2.62 (s, 3H), 2.57 - 1.87 (m, 8H), 1.74 - 1.08 (m, 11H), 0.95 - 0.55 (m, 7H). |
| **293** | 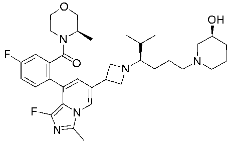 | 624.5 | ¹H NMR (400 MHz, DMSO-*d₆*) δ 7.97 -7.75 (m, 1H), 7.67 - 7.18 (m, 3H), 6.84 - 6.41 (m, 1H), 4.51 (d, J = 4.8 Hz, 1H), 4.41- 3.36 (m, 8H), 3.20 - 2.57 (m, 7H), 2.55 (s, 3H), 2.34 - 2.14 (m, 2H), 2.05 - 1.99 (m, 1H), 1.79 - 1.53 (m, 5H), 1.46 - 0.95 (m, 8H), 0.89 - 0.69 (m, 7H). |
| **294** | 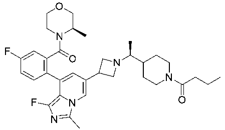 | 608.4 | ¹H NMR (400 MHz, DMSO-*d₆*) δ 8.01-7.75 (m, 1H), 7.67 - 7.21 (m, 3H), 6.93 - 6.39 (m, 1H), 4.60 - 3.36 (m, 9H), 3.20 - 2.79 (m, 5H), 2.55 (s, 3H), 2.49 - 2.14 (m, 5H), 1.75 - 1.42 (m, 5H), 1.23 - 0.67 (m, 11H). |
| **295** | 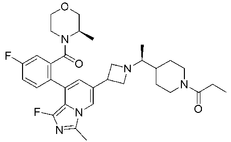 | 594.3 | ¹H NMR (400 MHz, DMSO-*d₆*) δ 7.96 - 7.75 (m, 1H), 7.61 - 7.25 (m, 3H), 6.92 - 6.37 (m, 1H), 4.52 - 3.44 (m, 8H), 3.13 - 2.68 (m, 5H), 2.58 - 2.51 (m, 3H), 2.47 - 2.13 (m, 6H), 1.74 - 1.38 (m, 3H), 1.26 - 0.89 (m, 7H), 0.79 - 0.56 (m, 4H). |
| **299** | 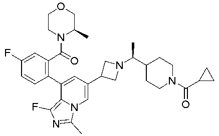 | 606.4 | ¹H NMR (400 MHz, DMSO-*d₆*) δ 8.03 -7.71 (m, 1H), 7.62 - 7.25 (m, 3H), 6.89 - 6.37 (m, 1H), 4.62 - 3.35 (m, 9H), 3.27 - 2.70 (m, 6H), 2.60 - 2.52 (m, 3H), 2.24 - 2.16 (m, 1H), 1.98 - 1.90 (m, 1H), 1.81 - 1.37 (m, 3H), 1.29 - 0.41 (m, 13H). |
| **300** | 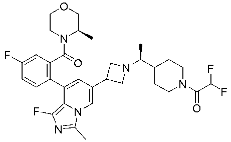 | 616.3 | ¹H NMR (400 MHz, DMSO-*d₆*) δ 7.95 -7.75 (m, 1H), 7.64 - 7.25 (m, 3H), 6.96 - 6.40 (m, 2H), 4.42 - 3.44 (m, 8H), 3.30 - 2.52 (m, 11H), 2.29 - 2.14 (m, 1H), 1.84 - 1.48 (m, 3H), 1.31 - 0.96 (m, 4H), 0.80 - 0.52 (m, 4H). |
| **301** | 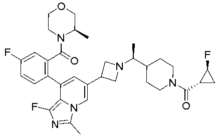 | 624.4 | ¹H NMR (400 MHz, DMSO-*d₆*) δ 8.01-7.71 (m, 1H), 7.67 - 7.05 (m, 3H), 6.99 - 6.39 (m, 1H), 4.92 - 4.61 (m, 1H), 4.48 - 3.37 (m, 8H), 3.31 - 2.52 (m, 11H), 2.43 - 2.17 (m, 2H), 1.70 - 0.52 (m, 13H). |
| **302** | 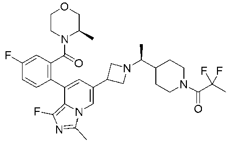 | 630.4 | ¹H NMR (400 MHz, DMSO-*d₆*) δ 7.97 -7.73 (m, 1H), 7.64 - 7.22 (m, 3H), 6.94 - 6.35 (m, 1H), 4.46 - 3.44 (m, 8H), 3.30 - 2.52 (m, 11H), 2.26 - 2.14 (m, 1H), 1.85 - 1.50 (m, 6H), 1.28 - 0.94 (m, 4H), 0.79 - 0.52 (m, 4H). |
| **303** | 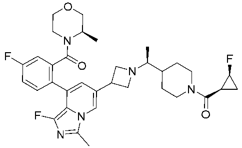 | 624.3 | ¹H NMR (400 MHz, DMSO-*d₆*) δ 7.95 - 7.79 (m, 1H), 7.67 - 7.21 (m, 3H), 6.94 - 6.38 (m, 1H), 5.01 - 4.76 (m, 1H), 4.49 - 3.46 (m, 8H), 3.23 - 2.66 (m, 6H), 2.55 (s, 3H), 2.46 - 2.03 (m, 3H), 1.83 - 0.89 (m, 10H), 0.81 - 0.52 (m, 4H). |
| **304** | 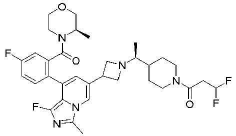 | 630.4 | ¹H NMR (400 MHz, DMSO-*d₆*) δ 8.01-7.77 (m, 1H), 7.68 - 7.23 (m, 3H), 6.89 - 6.44 (m, 1H), 6.44 - 6.08 (m, 1H), 4.54 - 3.36 (m, 9H), 3.23 - 2.63 (m, 8H), 2.55 (s, 3H), 2.46 - 2.31 (m, 1H), 2.21 (s, 1H), 1.75 - 1.40 (m, 3H), 1.28 - 0.90 (m, 4H), 0.80 - 0.52 (m, 4H). |
| **305** | 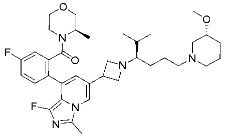 | 638.5 | ¹H NMR (400 MHz, DMSO-*d₆*) δ 7.96 - 7.70 (m, 1H), 7.64 - 7.24 (m, 3H), 6.87 - 6.45 (m, 1H), 4.41 - 3.36 (m, 7H), 3.22 (s,3H), 3.18 - 2.59 (m, 7H), 2.55 (s, 3H), 2.47 - 2.17 (m, 3H), 2.08 - 1.99 (m, 1H), 1.92 - 1.77 (m, 2H), 1.76 - 1.65 (m, 2H), 1.64- 1.51 (m, 1H), 1.50 - 0.96 (m, 8H), 0.89 - 0.55 (m, 7H). |
| **306** | 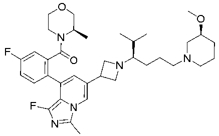 | 638.4 | ¹H NMR (400 MHz, DMSO-*d*₆) δ 7.86 (d, J = 10.4 Hz, 1H), 7.64 - 7.20 (m, 3H), 6.81 - 6.35 (m, 1H), 4.48 - 3.38 (m, 7H), 3.23 - 2.83 (m, 9H), 2.67 - 2.53 (m, 4H), 2.39 - 2.20 (m, 2H), 2.08 - 1.98 (m, 1H), 1.92 - 1.78 (m, 2H), 1.78 - 1.66 (m, 2H), 1.64 - 1.54 (m, 1H), 1.52 - 0.92 (m, 9H), 0.83 (dd, J = 6.8, 12.4 Hz, 7H). |
| **312-1** | 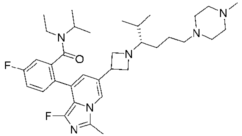 | 609.5 | ¹H NMR (400 MHz, CD₃OD) δ 7.84 - 7.75 (m, 1H), 7.62 - 7.50 (m, 1H), 7.32 (dt, J = 2.4, 8.4 Hz, 1H), 7.27 - 7.19 (m, 1H), 6.83 - 6.71 (m, 1H), 4.73 - 4.23 (m, 1H), 3.83 - 3.56 (m, 4H), 3.55 - 3.41 (m, 1H), 3.32 - 3.20 (m, 2H), 3.12 - 3.00 (m, 1H), 2.67 - 2.43 (m, 8H), 2.43 - 2.34 (m, 3H), 2.32 - 2.24 (m, 4H), 1.95 - 1.81 (m, 1H), 1.70 - 1.52 (m, 2H), 1.49 - 1.37 (m, 1H), 1.36 - 1.23 (m, 1H), 1.22 - 0.69 (m, 14H), 0.45 (d, J = 4.8 Hz, 2H). |
| **312-2** | 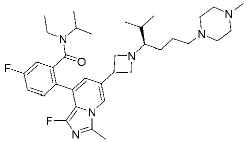 | 609.5 | ¹H NMR (400 MHz, DMSO-*d₆*) δ 7.88 - 7.79 (m, 1H), 7.57 - 7.47 (m, 1H), 7.40 - 7.24 (m, 2H), 6.77 - 6.54 (m, 1H), 3.56 - 3.40 (m, 4H), 3.18 - 2.87 (m, 4H), 2.56 - 2.52 (m, 3H), 2.40 - 2.14 (m, 10H), 2.13 - 2.09 (m, 3H), 2.03 - 1.99 (m, 1H), 1.75 - 1.64 (m, 1H), 1.49 - 1.36 (m, 2H), 1.31 - 1.07 (m, 3H), 0.99 - 0.89 (m, 3H), 0.86 - 0.78 (m, 9H), 0.46 - 0.32 (m, 2H). |
| **313** | 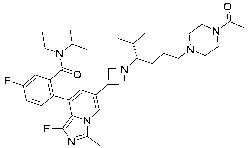 | 637.5 | ¹H NMR (400 MHz, CD₃OD) δ 7.81 - 7.73 (m, 1H), 7.60 - 7.48 (m, 1H), 7.30 (dt, J = 2.8, 8.4 Hz, 1H), 7.26 - 7.16 (m, 1H), 6.80 - 6.69 (m, 1H), 4.64 - 4.23 (m, 1H), 3.81 - 3.68 (m, 2H), 3.66 - 3.40 (m, 7H), 3.30 - 3.21 (m, 2H), 3.12 - 2.94 (m, 1H), 2.61 - 2.54 (m, 3H), 2.54 - 2.34 (m, 6H), 2.31 - 2.22 (m, 1H), 2.11 - 2.04 (m, 3H), 1.93 - 1.79 (m, 1H), 1.69 - 1.52 (m, 2H), 1.48 - 1.37 (m, 1H), 1.36 - 1.24 (m, 1H), 1.01 - 0.82 (m, 12H), 0.42 (d, J = 5.6 Hz, 2H). |
| **314** | 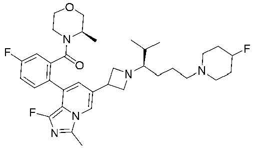 | 626.4 | ¹H NMR (400 MHz, DMSO-*d₆*) δ 7.93 - 7.79 (m, 1H), 7.61 - 7.47 (m, 1H), 7.44 - 7.24 (m, 2H), 6.84 - 6.43 (m, 1H), 4.74 - 4.51 (m, 1H), 4.44 - 3.37 (m, 7H), 3.16 - 2.77 (m, 4H), 2.57 - 2.52 (m, 3H), 2.48 - 2.41 (m, 2H), 2.26 - 2.16 (m, 4H), 2.07 - 2.00 (m, 1H), 1.85 - 1.59 (m, 5H), 1.51 - 1.36 (m, 2H), 1.30 - 1.05 (m, 4H), 0.98 - 0.38 (m, 8H). |
| **315** | 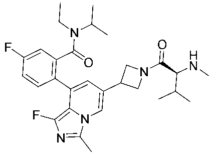 | 526.3 | ¹H NMR (400 MHz, CD₃OD) δ 7.87 - 7.80 (m, 1H), 7.61 - 7.50 (m, 1H), 7.30 (dt, J = 2.4, 8.4 Hz, 1H), 7.25 - 7.15 (m, 1H), 6.74 - 6.64 (m, 1H), 4.75 - 4.59 (m, 1H), 4.50 - 4.26 (m, 2H), 4.21 - 4.02 (m, 1H), 4.00 - 3.87 (m, 1H), 3.65 - 3.53 (m, 1H), 3.50 - 3.37 (m, 1H), 3.16 - 3.01 (m, 1H), 3.00 - 2.87 (m, 1H), 2.59 (s, 3H), 2.37 - 2.25 (m, 3H), 1.98 - 1.78 (m, 1H), 1.11 - 0.82 (m, 13H), 0.45 (d, J = 4.8 Hz, 2H). |
| **316** | 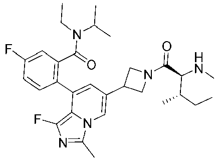 | 540.4 | ¹H NMR (400 MHz, CD₃OD) δ 7.88 - 7.79 (m, 1H), 7.61 - 7.50 (m, 1H), 7.30 (dt, J = 2.0, 8.4 Hz, 1H), 7.25 - 7.13 (m, 1H), 6.74 - 6.61 (m, 1H), 4.74 - 4.62 (m, 1H), 4.48 - 4.25 (m, 2H), 4.21 - 4.02 (m, 1H), 3.99 - 3.87 (m, 1H), 3.65 - 3.54 (m, 1H), 3.50 - 3.38 (m, 1H), 3.12 - 2.95 (m, 2H), 2.59 (s, 3H), 2.36 - 2.25 (m, 3H), 1.74 - 1.55 (m, 2H), 1.25 - 1.12 (m, 1H), 1.07 - 0.82 (m, 13H), 0.51 - 0.37 (m, 2H). |
| **317** | 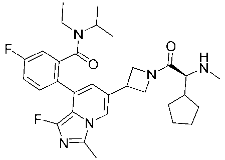 | 552.4 | ¹H NMR (400 MHz, CD₃OD) δ 7.88 - 7.80 (m, 1H), 7.64 - 7.49 (m, 1H), 7.36 - 7.26 (m, 1H), 7.24 - 7.14 (m, 1H), 6.74 - 6.60 (m, 1H), 4.75 - 4.63 (m, 1H), 4.51 - 4.26 (m, 2H), 4.22 - 4.02 (m, 1H), 3.99 - 3.81 (m, 1H), 3.65 - 3.54 (m, 1H), 3.49 - 3.38 (m, 1H), 3.12 - 2.97 (m, 2H), 2.59 (s, 3H), 2.37 - 2.27 (m, 3H), 2.11 - 1.97 (m, 1H), 1.94 - 1.80 (m, 1H), 1.76 - 1.48 (m, 5H), 1.44 - 1.23 (m, 2H), 1.16 - 0.82 (m, 7H), 0.55 - 0.39 (m, 2H). |
| **318** | 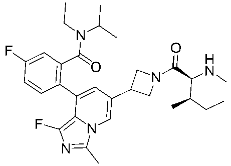 | 540.4 | ¹H NMR (400 MHz, CD₃OD) δ 7.67 - 7.57 (m, 1H), 7.42 - 7.28 (m, 1H), 7.09 (t, J = 8.4 Hz, 1H), 7.03 - 6.94 (m, 1H), 6.59 - 6.43 (m, 1H), 4.50 (t, J = 9.0 Hz, 1H), 4.45 - 4.35 (m, 1H), 4.31 - 4.13 (m, 2H), 4.12 - 4.01 (m, 1H), 4.00 - 3.81 (m, 1H), 3.79 - 3.65 (m, 1H), 3.44 - 3.32 (m, 1H), 3.29 - 3.16 (m, 1H), 2.94 - 2.77 (m, 2H), 2.39 (s, 3H), 2.13 - 2.04 (m, 3H), 1.53 - 1.37 (m, 1H), 1.36 - 1.23 (m, 1H), 1.13 - 0.94 (m, 1H), 0.80 (d, J = 6.0 Hz, 2H), 0.76 - 0.64 (m, 9H), 0.25 (d, J = 4.8 Hz, 2H). |
| **320** | 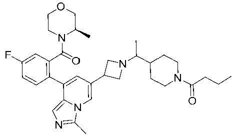 | 590.4 | ¹H NMR (400 MHz,CD₃OD) δ 8.00 - 7.87 (m, 1H), 7.85 - 7.63 (m, 1H), 7.46 - 7.04 (m, 3H), 7.03 - 6.70 (m, 1H), 4.67 - 4.30 (m, 2H), 4.08 - 3.65 (m, 5H), 3.61 - 3.50 (m, 1H), 3.27 - 2.92 (m, 5H), 2.83 - 2.48 (m, 5H), 2.42 - 2.33 (m, 3H), 1.98 - 1.67 (m, 3H), 1.67 - 1.54 (m, 3H), 1.34 - 1.09 (m, 4H), 1.00 - 0.93 (m, 3H), 0.91 - 0.84 (m, 3H), 0.75 - 0.40 (m, 1H). |
| **324-1** | 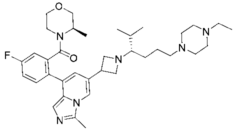 | 619.5 | ¹H NMR (400 MHz, DMSO-*d*₆) δ 7.99 (s, 1H), 7.78 - 7.60 (m, 1H), 7.47 - 7.27 (m, 2H), 7.13 - 6.96 (m, 1H), 6.92 - 6.68 (m, 1H), 4.38 - 3.84 (m, 1H), 3.72 - 3.41 (m, 5H), 3.25 - 3.03 (m, 4H), 3.01 - 2.91 (m, 1H), 2.87 - 2.69 (m, 1H), 2.62 - 2.57 (m, 3H), 2.38 - 2.17 (m, 11H), 2.07 - 2.00 (m, 1H), 1.92 - 1.63 (m, 2H), 1.49 - 1.38 (m, 2H), 1.28 - 1.12 (m, 3H), 1.06 - 1.00 (m, 1H), 0.99 - 0.92 (m, 3H), 0.88 - 0.80 (m, 6H), 0.69 - 0.40 (m, 1H). |
| **324-2** | 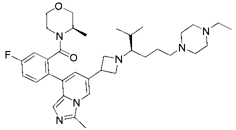 | 619.5 | ¹H NMR (400 MHz, DMSO-*d₆*) δ 8.07 -7.85 (m, 1H), 7.83 - 7.57 (m, 1H), 7.48 - 7.24 (m, 2H), 7.15 - 6.58 (m, 2H), 4.39 - 3.37 (m, 6H), 3.29 - 2.53 (m, 9H), 2.43 - 1.99 (m, 12H), 1.91 - 1.09 (m, 7H), 1.08 - 0.77 (m, 10H), 0.72 - 0.37 (m, 1H). |
| **325** | 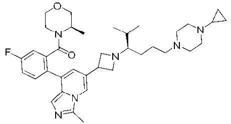 | 631.4 | ¹H NMR (400 MHz, DMSO-*d₆*) δ 8.01-7.90(m, 1H), 7.78 - 7.59 (m, 1H), 7.47 - 7.27 (m, 2H), 7.10 - 6.90 (m, 1H), 6.89 - 6.59 (m, 1H), 4.37 - 3.83 (m, 1H), 3.78 - 3.40 (m, 5H), 3.25 - 2.65 (m, 6H), 2.62 - 2.57 (m, 3H), 2.48 - 2.40 (m, 3H), 2.37 - 2.14 (m, 6H), 2.08 - 2.01 (m, 1H), 1.94 - 1.60 (m, 2H), 1.56 - 1.37 (m, 3H), 1.27 - 1.12 (m, 3H), 1.07 - 0.98 (m, 1H), 0.89 - 0.77 (m, 6H), 0.72 - 0.58 (m, 1H), 0.40 - 0.33 (m, 2H), 0.27 - 0.20 (m, 2H). |
| **326** | 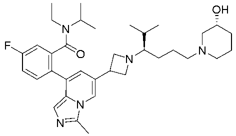 | 592.4 | ¹H NMR (400 MHz, DMSO-*d₆*) δ 7.97 -7.85 (m, 1H), 7.76 - 7.59 (m, 1H), 7.46 - 7.21 (m, 2H), 7.12 - 6.95 (m, 1H), 6.88 - 6.66 (m, 1H), 4.55 - 4.23 (m, 1H), 3.76 - 3.35 (m, 5H), 3.29 - 2.62 (m, 6H), 2.61 - 2.57 (m, 3H), 2.34 - 2.16 (m, 2H), 2.06 - 1.99 (m, 1H), 1.80 - 1.52 (m, 5H), 1.50 - 1.31 (m, 3H), 1.27 - 0.72 (m, 16H), 0.31 - 0.25 (m, 2H). |
| **327** | 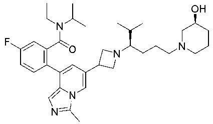 | 592.4 | ¹H NMR (400 MHz, DMSO-*d₆*) δ 7.96-7.86 (m, 1H), 7.74 - 7.61 (m, 1H), 7.44 - 7.24 (m, 2H), 7.13 - 6.92 (m, 1H), 6.89 - 6.65 (m, 1H), 4.56 - 4.22 (m, 1H), 3.60 - 3.36 (m, 5H), 3.25 - 2.61 (m, 6H), 2.60 - 2.57 (m, 3H), 2.33 - 2.14 (m, 2H), 2.06 - 1.97 (m, 1H), 1.79 - 1.53 (m, 5H), 1.48 - 1.30 (m, 3H), 1.27 - 0.96 (m, 4H), 0.92 - 0.71 (m, 12H), 0.31 - 0.25 (m, 2H). |
| **328** | 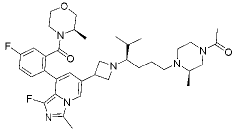 | 665.4 | ¹H NMR (400 MHz, CDCl₃) δ 7.61 - 7.45 (m, 2H), 7.27 - 7.01 (m, 2H), 6.93 - 6.69 (m, 1H), 5.13 - 4.77 (m, 4H), 4.60 - 4.27 (m, 3H), 4.19 - 3.99 (m, 2H), 3.97 - 3.67 (m, 3H), 3.66 - 3.06 (m, 6H), 3.02 - 2.68 (m, 5H), 2.61 (s, 3H), 2.53 - 2.31 (m, 2H), 2.15 - 1.97 (m, 4H), 1.57 - 1.23 (m, 2H), 1.19 - 0.86 (m, 10H), 0.78 - 0.57 (m, 1H). |
| **329** | 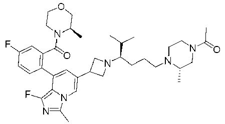 | 665.5 | ¹H NMR (400 MHz, DMSO-*d₆*) δ 7.98 - 7.75 (m, 1H), 7.63 - 7.23 (m, 3H), 6.85 - 6.38 (m, 1H), 4.40 - 3.44 (m, 8H), 3.25 - 2.52 (m, 12H), 2.45 - 1.79 (m, 8H), 1.77 - 1.63 (m, 1H), 1.48 - 1.35 (m, 2H), 1.28 - 0.98 (m, 4H), 0.96 - 0.68 (m, 10H). |
| **331** | 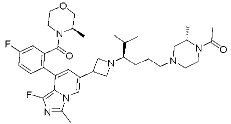 | 665.4 | ¹H NMR (400 MHz, DMSO-*d₆*) δ 7.98 - 7.75 (m, 1H), 7.64 - 7.23 (m, 3H), 6.85 - 6.40 (m, 1H), 4.52 - 3.44 (m, 8H), 3.25 - 2.52 (m, 11H), 2.29 - 1.63 (m, 10H), 1.52 - 1.02 (m, 9H), 0.90 - 0.69 (m, 7H). |
| **333** | 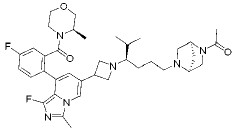 | 663.5 | ¹H NMR (400 MHz, CD₃OD) δ 7.81 (d, J = 14.0 Hz, 1H), 7.66 - 7.50 (m, 1H), 7.42 - 7.14 (m, 2H), 6.92 - 6.54 (m, 1H), 4.67 - 4.35 (m, 2H), 4.08 (d, J = 12.4 Hz, 1H), 3.84 - 3.53 (m, 7H), 3.38 (d, J = 10.0 Hz, 1H), 3.28 - 3.08 (m, 3H), 3.04 - 2.82 (m, 2H), 2.69 - 2.50 (m, 6H), 2.32 - 2.21 (m, 1H), 2.08 (s, 2H), 2.00 - 1.69 (m, 5H), 1.63 - 1.50 (m, 2H), 1.48 - 1.38 (m, 1H), 1.36 - 1.26 (m, 2H), 1.22 - 1.09 (m, 1H), 0.93 (dd, J = 6.4, 17.2 Hz, 6H), 0.85 - 0.75 (m, 1H). |
| **334** | 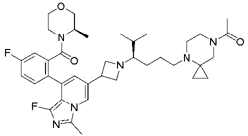 | 677.4 | ¹H NMR (400 MHz, DMSO-*d₆*) δ 7.97 -7.75 (m, 1H), 7.65 - 7.19 (m, 3H), 6.88 - 6.34 (m, 1H), 4.39 - 3.35 (m, 9H), 3.24 - 2.90 (m, 6H), 2.85 - 2.52 (m, 8H), 2.04 - 1.88 (m, 4H), 1.76 - 1.62 (m, 1H), 1.42 - 1.29 (m, 2H), 1.27 - 1.03 (m, 4H), 0.88 - 0.70 (m, 7H), 0.55 - 0.37 (m, 4H). |
| **335** | 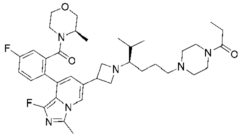 | 665.4 | ¹H NMR (400 MHz, DMSO-*d₆*) δ (400 MHz, DMSO-d6) δ = 7.96 - 7.76 (m, 1H), 7.63 - 7.22 (m, 3H), 6.85 - 6.42 (m, 1H), 4.45 - 3.33 (m, 11H), 3.26 - 2.70 (m, 5H), 2.55 (s, 3H), 2.35 - 2.18 (m, 8H), 2.04 (d, J = 2.4 Hz, 1H), 1.78 - 1.63 (m, 1H), 1.52 - 1.38 (m, 2H), 1.32 - 1.02 (m, 4H), 0.96 (t, J = 7.2 Hz, 3H), 0.89 - 0.58 (m, 7H). |
| **336** | 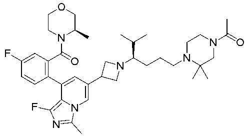 | 679.5 | ¹H NMR (400 MHz, DMSO-*d₆*) δ 7.91-7.79 (m, 1H), 7.63 - 7.23 (m, 3H), 6.80 - 6.46 (m, 1H), 4.36 - 3.34 (m, 9H), 3.21 - 2.63 (m, 7H), 2.54 (s, 3H), 2.44 (m, 1H), 2.40 - 2.31 (m, 1H), 2.27 - 2.18 (m, 2H), 2.06 - 1.90 (m, 4H), 1.77 - 1.65 (m, 1H), 1.37 (s, 2H), 1.29 - 1.04 (m, 4H), 0.92 (s, 3H), 0.90 - 0.72 (m, 10H). |
| **337** | 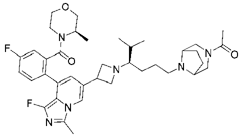 | 677.4 | ¹H NMR (400 MHz, CDCl₃) δ 7.55 - 7.34 (m, 2H), 7.25 - 7.05 (m, 2H), 6.82 - 6.56 (m, 1H), 4.66 - 4.06 (m, 2H), 3.82 - 3.29 (m, 7H), 3.25 - 2.54 (m, 10H), 2.30 (br t, J = 6.9 Hz, 2H), 2.09 (br s, 1H), 2.05 (s, 3H), 1.95 - 1.76 (m, 3H), 1.70 (br s, 3H), 1.62 - 1.45 (m, 4H), 1.43 - 1.04 (m, 4H), 0.94 - 0.87 (m, 5H), 0.83 - 0.57 (m, 1H). |
| **338** | 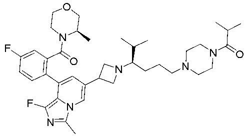 | 679.5 | ¹H NMR (400 MHz, CD₃OD) δ 7.81 (d, J = 14.0 Hz, 1H), 7.64 - 7.51 (m, 1H), 7.38 - 7.17 (m, 2H), 6.86 - 6.59 (m, 1H), 4.79 - 3.97 (m, 2H), 3.77 (t, J = 6.0 Hz, 1H), 3.74 - 3.68 (m, 1H), 3.67 - 3.43 (m, 7H), 3.28 - 3.21 (m, 2H), 3.19 - 3.06 (m, 1H), 3.04 - 2.86 (m, 2H), 2.60 (s, 3H), 2.52 - 2.34 (m, 6H), 2.33 - 1.95 (m, 2H), 1.92 - 1.78 (m, 1H), 1.69 - 1.51 (m, 2H), 1.48 - 1.37 (m, 1H), 1.30 (d, J = 5.2 Hz, 2H), 1.17 (d, J = 4.8 Hz, 1H), 1.08 (d, J = 6.6 Hz, 6H), 0.94 (dd, J = 6.2, 16.8 Hz, 6H), 0.81 (d, J = 5.2 Hz, 1H). |
| **339** | 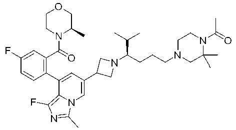 | 679.5 | ¹H NMR (400 MHz, DMSO-*d₆*) δ 7.94 - 7.78 (m, 1H), 7.61 - 7.24 (m, 3H), 6.78 - 6.45 (m, 1H), 4.39 - 3.38 (m, 7H), 3.19 - 2.65 (m, 5H), 2.57 - 2.53 (m, 3H), 2.39 - 2.30 (m, 2H), 2.26 - 2.16 (m, 2H), 2.12 - 2.01 (m, 3H), 1.93 (s, 3H), 1.78 - 1.64 (m, 1H), 1.51 - 1.04 (m, 13H), 0.97 - 0.50 (m, 8H). |
| **340** | 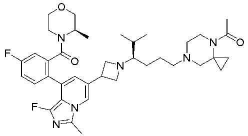 | 677.4 | ¹H NMR (400 MHz, DMSO-*d₆*) δ 7.99-7.75 (m, 1H), 7.66 - 7.23 (m, 3H), 6.85 - 6.39 (m, 1H), 4.40 - 3.37 (m, 8H), 3.15 - 2.53 (m, 9H), 2.41 - 1.88 (m, 10H), 1.75 - 1.65 (m, 1H), 1.48 - 1.01 (m, 7H), 0.89 - 0.52 (m, 10H). |
| **341** | 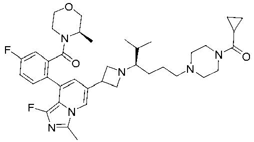 | 677.4 | ¹H NMR (400 MHz, DMSO-*d₆*) δ 7.95 -7.77 (m, 1H), 7.63 - 7.21 (m, 3H), 6.82 - 6.42 (m, 1H), 4.42 - 3.35 (m, 11H), 3.15 - 2.65 (m, 4H), 2.55 (s, 3H), 2.40 - 2.19 (m, 6H), 2.10 - 2.00 (m, 1H), 1.98 - 1.86 (m, 1H), 1.78 - 1.65 (m, 1H), 1.53 - 1.38 (m, 2H), 1.32 - 1.03 (m, 4H), 0.98 - 0.49 (m, 12H). |
| **342** | 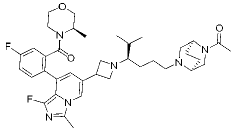 | 677.4 | ¹H NMR (400 MHz, DMSO-*d₆*) δ 7.98 - 7.71 (m, 1H), 7.70 - 7.16 (m, 3H), 7.01 - 6.16 (m, 1H), 4.39 - 4.17 (m, 1H), 4.00 - 3.65 (m, 2H), 3.62 - 3.42 (m, 6H), 3.20 - 2.63 (m, 9H), 2.58 - 2.52 (m, 3H), 2.48 - 2.32 (m, 3H), 2.07 - 1.99 (m, 1H), 1.95 - 1.85 (m, 3H), 1.80 - 1.58 (m, 3H), 1.57 - 1.32 (m, 3H), 1.31 - 1.02 (m, 4H), 0.98 - 0.58 (m, 7H). |
| **343** | 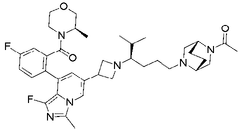 | 677.4 | ¹H NMR (400 MHz, DMSO-*d₆*) δ 7.93 - 7.76 (m, 1H), 7.64 - 7.23 (m, 3H), 6.87 - 6.44 (m, 1H), 4.43 - 4.16 (m, 1H), 4.09 - 3.62 (m, 2H), 3.60 - 3.36 (m, 6H), 3.20 - 2.63 (m, 9H), 2.61 - 2.52 (m, 3H), 2.49 - 2.37 (m, 3H), 2.09 - 1.98 (m, 1H), 1.94 - 1.88 (m, 3H), 1.79 - 1.60 (m, 3H), 1.56 - 1.34 (m, 3H), 1.31 - 1.05 (m, 4H), 0.89 - 0.69 (m, 7H). |
| **344** | 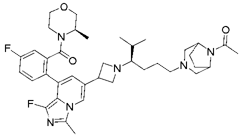 | 677.4 | ¹H NMR (400 MHz, CDCl₃) δ 7.57 - 7.30 (m, 2H), 7.25 - 7.01 (m, 2H), 6.80 - 6.58 (m, 1H), 4.67 - 3.93 (m, 3H), 3.75 - 2.92 (m, 9H), 2.78 - 2.44 (m, 5H), 2.36 - 2.09 (m, 4H), 2.05 (s, 3H), 1.98 - 1.71 (m, 5H), 1.70 - 1.05 (m, 9H), 0.98 - 0.52 (m, 7H). |
| **345** | 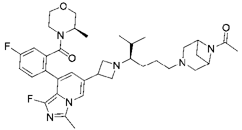 | 663.4 | ¹H NMR (400 MHz, DMSO-*d₆*) δ 7.99-7.76 (m, 1H), 7.66 - 7.21 (m, 3H), 6.92 - 6.40 (m, 1H), 4.39 - 3.35 (m, 9H), 3.23 - 2.52 (m, 12H), 2.42 (t, J = 7.2 Hz, 2H), 2.30 (q, J = 6.4 Hz, 1H), 2.07 - 1.99 (m, 1H), 1.79 - 1.65 (m, 5H), 1.53 - 1.37 (m, 2H), 1.31 - 1.02 (m, 4H), 0.89 - 0.67 (m, 7H). |
| **346** | 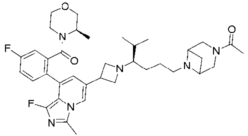 | 663.4 | ¹H NMR (400 MHz, DMSO-*d₆*) δ 7.95 -7.75 (m, 1H), 7.63 - 7.20 (m, 3H), 6.82 - 6.43 (m, 1H), 4.37 - 3.36 (m, 11H), 3.29 - 2.64 (m, 7H), 2.54 (s, 3H), 2.38 - 2.20 (m, 3H), 2.06 - 1.91 (m, 4H), 1.76 - 1.63 (m, 1H), 1.41 - 1.03 (m, 7H), 0.89 - 0.54 (m, 7H). |
| **347** | 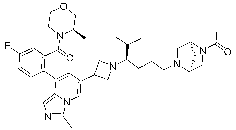 | 645.4 | ¹H NMR (400 MHz, DMSO-*d₆*) δ 8.02 - 7.88 (m, 1H), 7.81 - 7.59 (m, 1H), 7.48 - 7.26 (m, 2H), 7.12 - 6.91 (m, 1H), 6.88 - 6.62 (m, 1H), 4.49 - 3.34 (m, 9H), 3.30 - 2.65 (m, 8H), 2.60 (s, 3H), 2.48 - 2.30 (m, 3H), 2.08 - 2.00 (m, 1H), 1.93 (s, 1H), 1.89 - 1.80 (m, 2H), 1.78 - 1.50 (m, 3H), 1.46 - 1.13 (m, 5H), 1.03 (d, J = 6.8 Hz, 1H), 0.84 (dd, J = 7.2, 12.0 Hz, 6H), 0.70 - 0.37 (m, 1H). |
| **349** | 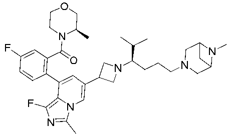 | 635.4 | ¹H NMR (400 MHz, CDCl₃) δ 7.71 - 7.29 (m, 2H), 7.26 - 6.97 (m, 2H), 6.93 - 6.61 (m, 1H), 4.64 - 4.04 (m, 6H), 4.00 - 3.48 (m, 12H), 3.36 - 3.05 (m, 5H), 2.99 - 2.85 (m, 2H), 2.77 - 2.64 (m, 3H), 2.61 (s, 3H), 2.47 - 1.94 (m, 3H), 1.77 - 1.57 (m, 2H), 1.31 - 1.15 (m, 1H), 1.05 - 0.92 (m, 5H), 0.81 - 0.56 (m, 1H). |
| **350** | 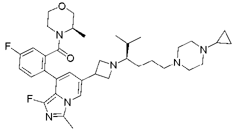 | 649.4 | ¹H NMR (400 MHz, DMSO-*d*₆) δ 7.91-7.78 (m, 1H), 7.61 - 7.23 (m, 3H), 6.85 - 6.42 (m, 1H), 4.43 - 3.34 (m, 7H), 3.23 - 2.64 (m, 5H), 2.57 - 2.52 (m, 3H), 2.49 - 2.42 (m, 4H), 2.36 - 2.10 (m, 6H), 2.07 - 1.98 (m, 1H), 1.75 - 1.63 (m, 1H), 1.58 - 1.34 (m, 3H), 1.31 - 1.03 (m, 4H), 0.89 - 0.72 (m, 7H), 0.40 - 0.32 (m, 2H), 0.28 - 0.19 (m, 2H). |
| **352** | 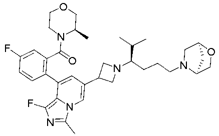 | 622.4 | ¹H NMR (400 MHz, DMSO-*d*₆) δ 7.96 - 7.75 (m, 1H), 7.63 - 7.24 (m, 3H), 6.83 - 6.40 (m, 1H), 4.40 - 3.35 (m, 11H), 3.24 - 2.52 (m, 9H), 2.49 - 2.29 (m, 3H), 2.06 - 1.99 (m, 1H), 1.76 - 1.64 (m, 2H), 1.56 - 1.47 (m, 1H), 1.44 - 1.33 (m, 2H), 1.32 - 1.05 (m, 4H), 0.90 - 0.69 (m, 7H). |
| **354** | 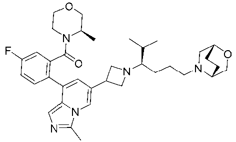 | 618.5 | ¹H NMR (400 MHz, CD₃OD) δ 8.00 - 7.89 (m, 1H), 7.85 - 7.63 (m, 1H), 7.42 - 7.21 (m, 2H), 7.21 - 7.08 (m, 1H), 7.05 - 6.76 (m, 1H), 4.51 - 4.01 (m, 2H), 3.84 - 3.76 (m, 2H), 3.75 - 3.66 (m, 3H), 3.62 - 3.51 (m, 1H), 3.34 - 3.29 (m, 4H), 3.28 - 3.16 (m, 2H), 3.14 - 2.96 (m, 2H), 2.94 - 2.86 (m, 1H), 2.75 - 2.65 (m, 4H), 2.64 - 2.55 (m, 2H), 2.34 - 2.23 (m, 1H), 2.12 - 1.96 (m, 2H), 1.91 - 1.82 (m, 1H), 1.79 - 1.67 (m, 2H), 1.64 - 1.52 (m, 2H), 1.49 - 1.40 (m, 1H), 1.39 - 1.31 (m, 1H), 1.27 (d, J = 7.2 Hz, 1H), 1.16 - 1.07 (m, 1H), 1.00 - 0.86 (m, 6H), 0.78 - 0.37 (m, 1H). |
| **355** | 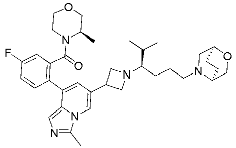 | 618.9 | ¹H NMR (400 MHz, DMSO-*d₆*) δ 8.03 -7.88 (m, 1H), 7.80 - 7.59 (m, 1H), 7.51 - 7.27 (m, 2H), 7.13 - 6.91 (m, 1H), 6.89 - 6.62 (m, 1H), 4.41 - 3.38 (m, 9H), 3.28 - 3.05 (m, 3H), 3.02 - 2.90 (m, 1H), 2.88 - 2.66 (m, 3H), 2.60 (s, 3H), 2.54 (s, 1H), 2.48 - 2.42 (m, J = 7.0, 7.0 Hz, 2H), 2.12 - 0.97 (m, 13H), 0.93 - 0.76 (m, 6H), 0.69 - 0.38 (m, 1H). |
| **356** | 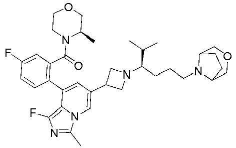 | 636.5 | ¹H NMR (400 MHz, CD₃OD) δ 7.80 (d, J = 13.6 Hz, 1H), 7.66 - 7.49 (m, 1H), 7.40 - 7.12 (m, 2H), 6.88 - 6.59 (m, 1H), 4.64 - 3.98 (m, 1H), 3.85 - 3.53 (m, 7H), 3.48 (d, J = 9.2 Hz, 2H), 3.35 - 3.20 (m, 4H), 3.11 (s, 2H), 3.07 - 2.65 (m, 2H), 2.60 (s, 3H), 2.37 - 2.20 (m, 3H), 2.03 - 1.76 (m, 5H), 1.57 (s, 2H), 1.48 - 1.36 (m, 1H), 1.35 - 1.23 (m, 2H), 1.17 (d, J = 4.8 Hz, 1H), 0.94 (dd, J = 6.4, 17.6 Hz, 6H), 0.85 - 0.59 (m, 1H). |
| **358** | 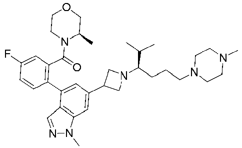 | 605.5 | ¹H NMR (400 MHz, CD₃OD) δ 8.44 (s, 1H), 8.02 - 7.85 (m, 1H), 7.78 - 7.54 (m, 2H), 7.46 - 7.12 (m, 3H), 4.57 - 4.37 (m, 2H), 4.29 - 4.21 (m, 2H), 4.17 - 4.03 (m, 4H), 3.70 - 3.39 (m, 2H), 3.27 - 3.14 (m, 2H), 3.08 - 2.90 (m, 5H), 2.82 - 2.68 (m, 3H), 2.65 (d, J = 6.4 Hz, 3H), 2.62 - 2.54 (m, 2H), 2.50 - 2.23 (m, 1H), 2.21 - 2.08 (m, 1H), 2.04 (s, 2H), 1.75 - 1.63 (m, 3H), 1.58 - 1.20 (m, 3H), 1.12 - 0.99 (m, 7H), 0.47 (d, J = 6.8 Hz, 1H). |
| **359** | 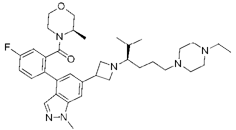 | 619.5 | ¹H NMR (400 MHz, CD₃OD) δ 7.96 - 7.83 (m, 1H), 7.75 - 7.50 (m, 2H), 7.44 - 7.05 (m, 3H), 4.58 (s, 3H), 4.15 - 4.07 (m, 3H), 3.96 - 3.86 (m, 3H), 3.66 - 3.41 (m, 2H), 2.56 - 2.31 (m, 10H), 2.19 (s, 1H), 2.10 - 1.99 (m, 1H), 1.96 - 1.84 (m, 1H), 1.73 - 1.53 (m, 3H), 1.52 - 1.36 (m, 2H), 1.33 (s, 1H), 1.29 (s, 2H), 1.23 (d, J = 7.0 Hz, 1H), 1.12 - 1.05 (m, 4H), 0.99 - 0.88 (m, 7H), 0.51 (d, J = 6.9 Hz, 1H). |
| **361** | 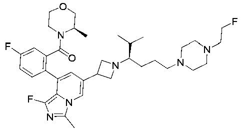 | 655.5 | ¹H NMR (400 MHz, DMSO-*d₆*) δ 7.93 - 7.70 (m, 1H), 7.63 - 7.47 (m, 1H), 7.45 - 7.22 (m, 2H), 6.94 - 6.31 (m, 1H), 4.59 - 4.39 (m, 2H), 4.38 - 3.34 (m, 7H), 3.28 - 2.57 (m, 7H), 2.57 - 2.53 (m, 3H), 2.46 - 2.16 (m, 10H), 2.07 - 1.99 (m, 1H), 1.77 - 1.62 (m, 1H), 1.52 - 1.34 (m, 2H), 1.32 - 1.02 (m, 4H), 0.91 - 0.65 (m, 7H). |
| **362** | 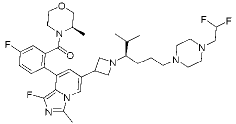 | 673.5 | ¹H NMR (400 MHz, DMSO-*d₆*) δ 7.97 - 7.74 (m, 1H), 7.65 - 7.17 (m, 3H), 6.88 - 6.44 (m, 1H), 6.35 - 5.88 (m, 1H), 4.45 - 3.32 (m, 7H), 3.23 - 2.59 (m, 7H), 2.55 (s, 3H), 2.48 - 2.16 (m, 10H), 2.08 - 1.96 (m, 1H), 1.78 - 1.60 (m, 1H), 1.51 - 1.33 (m, 2H), 1.31 - 1.02 (m, 4H), 0.89 - 0.62 (m, 7H). |
| **363** | 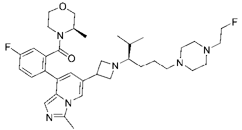 | 637.6 | ¹H NMR (400 MHz, CDCl₃) δ 7.77 - 7.51 (m, 2H), 7.25 - 7.13 (m, 3H), 6.92 - 6.70 (m, 1H), 4.79 - 4.50 (m, 3H), 3.80 - 3.47 (m, 5H), 3.42 - 2.86 (m, 6H), 2.76 - 2.71 (m, 1H), 2.70 (s, 3H), 2.68 - 2.65 (m, 1H), 2.62 - 2.47 (m, 6H), 2.35 - 2.29 (m, 2H), 2.15 - 2.04 (m, 1H), 1.90 - 1.75 (m, 2H), 1.59 - 1.49 (m, 3H), 1.35 - 1.23 (m, 3H), 1.14 - 1.08 (m, 1H), 0.95 - 0.88 (m, 6H), 0.81 - 0.76 (m, 1H). |
| **364** | 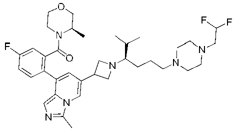 | 655.8 | ¹H NMR (400 MHz, CD₃OD) δ 8.04 - 7.87 (m, 1H), 7.85 - 7.64 (m, 1H), 7.42 - 7.22 (m, 2H), 7.21 - 7.06 (m, 1H), 7.03 - 6.77 (m, 1H), 6.13 - 5.77 (m, 1H), 4.57 - 4.00 (m, 1H), 3.86 - 3.33 (m, 6H), 3.29 - 3.22 (m, 2H), 3.22 - 2.91 (m, 2H), 2.85 - 2.72 (m, 2H), 2.71 - 2.65 (m, 4H), 2.63 (s, 3H), 2.52 (s, 4H), 2.40 - 2.31 (m, 2H), 2.29 - 2.23 (m, 1H), 2.01 - 1.76 (m, 2H), 1.66 - 1.52 (m, 2H), 1.47 - 1.35 (m, 1H), 1.34 - 1.24 (m, 2H), 1.12 (d, J = 6.8 Hz, 1H), 1.00 - 0.85 (m, 6H), 0.76 - 0.39 (m, 1H). |
| **365** | 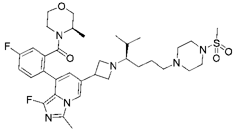 | 687.4 | ¹H NMR (400 MHz, DMSO-*d₆*) δ 7.94 - 7.78 (m, 1H), 7.63 - 7.24 (m, 3H), 6.84 - 6.46 (m, 1H), 4.39 - 3.34 (m, 7H), 3.28 - 2.94 (m, 8H), 2.85 (s, 4H), 2.55 (s, 3H), 2.41 (s, 4H), 2.35 - 2.22 (m, 2H), 2.08 - 1.99 (m, 1H), 1.77 - 1.65 (m, 1H), 1.56 - 1.34 (m, 2H), 1.32 - 1.03 (m, 4H), 0.96 - 0.73 (m, 7H). |
| **369** | 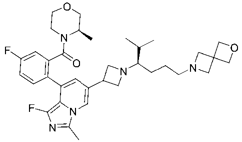 | 622.5 | ¹H NMR (400 MHz, DMSO-*d₆*) δ 7.95 -7.71 (m, 1H), 7.63 - 7.47 (m, 1H), 7.45 - 7.24 (m, 2H), 6.84 - 6.40 (m, 1H), 4.76 - 4.46 (m, 4H), 4.42 - 3.36 (m, 7H), 3.20 - 2.70 (m, 9H), 2.59 - 2.52 (m, 3H), 2.27 - 2.17 (m, 2H), 2.03 - 1.93 (m, 1H), 1.75 - 1.61 (m, 1H), 1.32 - 1.02 (m, 6H), 0.88 - 0.65 (m, 7H). |
| **376** | 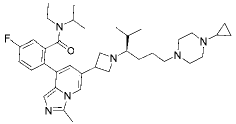 | 617.5 | ¹H NMR (400 MHz, DMSO-*d₆*) δ 7.72 - 7.64 (m, 1H), 7.50 - 7.37 (m, 1H), 7.21 - 7.01 (m, 2H), 6.90 - 6.72 (m, 1H), 6.61 - 6.44 (m, 1H), 4.11 - 3.09 (m, 5H), 3.04 - 2.41 (m, 5H), 2.37 - 2.32 (m, 3H), 2.11 - 1.90 (m, 6H), 1.83 - 1.73 (m, 1H), 1.53 - 1.41 (m, 1H), 1.33 - 1.13 (m, 3H), 1.06 - 0.78 (m, 3H), 0.70 - 0.46 (m, 13H), 0.20 - -0.10 (m, 7H). |
| **377** | 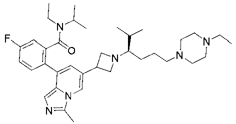 | 605.6 | ¹H NMR (400 MHz, CD₃OD) δ 7.95 - 7.83 (m, 1H), 7.77 - 7.66 (m, 1H), 7.38 - 7.04 (m, 3H), 6.97 - 6.84 (m, 1H), 4.40 - 3.36 (m, 6H), 3.29 - 3.22 (m, 1H), 3.10 - 2.94 (m, 1H), 2.74 - 2.33 (m, 14H), 2.30 - 2.21 (m, 1H), 1.92 - 1.81 (m, 1H), 1.69 - 1.04 (m, 9H), 1.01 - 0.76 (m, 12H), 0.30 (d, J = 6.4 Hz, 2H). |
| **380** | 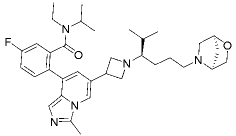 | 590.8 | ¹H NMR (400 MHz, CD₃OD) δ 7.95 - 7.89 (m, 1H), 7.76 - 7.68 (m, 1H), 7.34 (dt, J = 2.4, 8.4 Hz, 1H), 7.28 - 7.21 (m, 1H), 7.17 - 7.12 (m, 1H), 6.95 - 6.86 (m, 1H), 4.44 - 4.27 (m, 1H), 4.06 - 3.97 (m, 1H), 3.82 - 3.75 (m, 1H), 3.74 - 3.63 (m, 2H), 3.63 - 3.52 (m, 3H), 3.50 - 3.38 (m, 1H), 3.28 - 3.21 (m, 1H), 3.03 (td, J = 6.4, 12.4 Hz, 1H), 2.89 - 2.81 (m, 1H), 2.66 (s, 3H), 2.64 - 2.49 (m, 3H), 2.30 - 2.22 (m, 1H), 1.93 - 1.81 (m, 2H), 1.78 - 1.68 (m, 1H), 1.66 - 1.50 (m, 2H), 1.49 - 1.38 (m, 1H), 1.37 - 1.25 (m, 1H), 1.19 - 1.09 (m, 1H), 1.00 - 0.87 (m, 12H), 0.80 (t, J = 7.0 Hz, 1H), 0.30 (d, J = 6.8 Hz, 2H). |
| **382** | 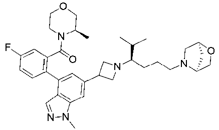 | 604.3 | ¹H NMR (400 MHz, CD₃OD) δ 7.98 - 6.81 (m, 6H), 4.32 - 4.11 (m, 1H), 3.98 - 3.89 (m, 3H), 3.88 - 3.78 (m, 1H), 3.77 - 3.62 (m, 3H), 3.48 - 3.38 (m, 2H), 3.36 - 3.21 (m, 1H), 3.20 - 3.01 (m, 5H), 3.00 - 2.80 (m, 1H), 2.79 - 2.60 (m, 2H), 2.58 - 2.15 (m, 4H), 2.14 - 2.01 (m, 1H), 1.83 - 1.63 (m, 2H), 1.60 - 1.52 (m, 1H), 1.51 - 1.34 (m, 2H), 1.34 - 1.22 (m, 1H), 1.21 - 1.11 (m, 1H), 1.06 (d, J = 7.2 Hz, 1H), 0.97 - 0.62 (m, 7H), 0.36 - -0.01 (m, 1H). |
| **383** | 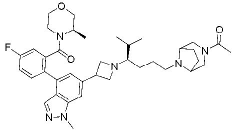 | 659.7 | ¹H NMR (400 MHz, DMSO-*d₆*) δ 7.89-7.74 (m, 1H), 7.72 - 7.50 (m, 2H), 7.48 - 7.23 (m, 2H), 7.17 - 6.91 (m, 1H), 4.34 - 3.79 (m, 5H), 3.75 - 3.35 (m, 6H), 3.27 - 2.54 (m, 10H), 2.28 - 2.21 (m, 2H), 2.10 - 2.02 (m, 1H), 1.96 - 1.88 (m, 3H), 1.83 - 1.70 (m, 3H), 1.52 - 1.39 (m, 3H), 1.36 - 1.23 (m, 3H), 1.16 - 1.08 (m, 1H), 1.01 - 0.95 (m, 1H), 0.90 - 0.82 (m, 6H), 0.53 - 0.16 (m, 1H). |
| **384** | 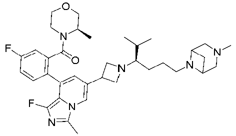 | 635.4 | ¹H NMR (400 MHz, DMSO-*d₆*) δ 7.97 -7.75 (m, 1H), 7.61 - 7.22 (m, 3H), 6.82 - 6.45 (m, 1H), 4.41 - 3.34 (m, 10H), 3.21 - 2.81 (m, 4H), 2.76 - 2.70 (m, 2H), 2.70 - 2.64 (m, 2H), 2.54 (s, 3H), 2.28 (s, 3H), 2.22 (s, 2H), 2.18 - 2.08 (m, 1H), 2.00 (s, 1H), 1.82 - 1.07 (m, 8H), 0.91 - 0.58 (m, 7H). |
| **385** | 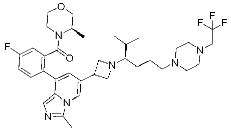 | 673.7 | ¹H NMR (400 MHz, CDCl₃) δ 7.75 - 7.54 (m, 2H), 7.26 - 7.12 (m, 3H), 6.99 - 6.68 (m, 1H), 4.62 - 4.09 (m, 1H), 3.78 - 3.67 (m, 2H), 3.63 - 3.50 (m, 2H), 3.42 - 3.11 (m, 4H), 3.00 - 2.89 (m, 3H), 2.70 (s, 6H), 2.57 - 2.44 (m, 4H), 2.37 - 2.27 (m, 2H), 2.17 - 2.08 (m, 1H), 1.92 - 1.76 (m, 2H), 1.60 - 1.47 (m, 3H), 1.42 - 1.23 (m, 4H), 1.16 - 1.06 (m, 1H), 0.95 - 0.87 (m, 6H), 0.79 - 0.42 (m, 1H). |
| **386** | 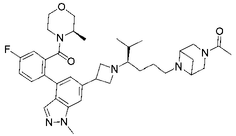 | 645.4 | ¹H NMR (400 MHz, CDCl₃) δ 7.72 - 7.62 (m, 1H), 7.45 - 7.32 (m, 1H), 7.13 - 7.09 (m, 1H), 7.06 - 6.84 (m, 3H),4.35 - 3.86 (m, 4H), 3.69 - 3.52 (m, 4H), 3.46 - 3.40 (m, 3H), 3.37 - 3.28 (m, 2H), 3.21 (br d, J = 11.4 Hz, 1H), 3.06 - 2.86 (m, 3H), 2.85 - 2.73 (m, 1H), 2.67 - 2.60 (m, 1H), 2.48 - 2.37 (m, 1H), 2.29 - 2.13 (m, 3H), 1.95 - 1.86 (m, 4H), 1.65 - 1.58(m, 1H), 1.29 - 1.03 (m, 7H), 0.86 (d, J = 6.9 Hz, 1H), 0.75 - 0.69 (m, 6H), 0.43 - -0.04 (m, 1H). |
| **387** | 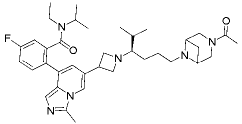 | 631.4 | ¹H NMR (400 MHz, CDCl₃) δ 7.70 - 7.62 (m, 1H), 7.56 - 7.50 (m, 1H), 7.22 - 7.10 (m, 3H), 6.89 - 6.78 (m, 1H), 3.81 - 3.36 (m, 11H), 3.24 - 3.06 (m, 2H), 2.99 - 2.85 (m, 1H), 2.68 (s, 3H), 2.62 - 2.55 (m, 1H), 2.42 - 2.31 (m, 2H), 2.16 - 2.04 (m, 4H), 1.50 - 1.12 (m, 6H), 1.01 - 0.74 (m, 13H), 0.25 (dd, J = 3.6, 6.8 Hz, 2H). |
| **388** | 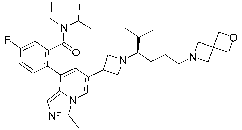 | 590.7 | ¹H NMR (400 MHz, DMSO-*d₆*) δ 7.97 -7.87 (m, 1H), 7.74 - 7.59 (m, 1H), 7.43 - 7.24 (m, 2H), 7.08 - 6.96 (m, 1H), 6.87 - 6.68 (m, 1H), 4.59 - 4.49 (m, 4H), 3.60 - 3.36 (m, 4H), 3.19 - 3.14 (m, 4H), 3.13 - 2.88 (m, 3H), 2.68 - 2.52 (m, 3H), 2.27 - 2.17 (m, 2H), 2.01 - 1.92 (m, 1H), 1.74 - 1.63 (m, 1H), 1.30 - 1.03 (m, 5H), 0.92 - 0.87 (m, 2H), 0.86 - 0.70 (m, 10H), 0.32 - -0.15 (m, 3H). |
| **390** | 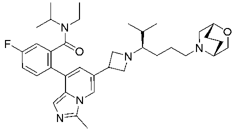 | 604.3 | ¹H NMR (400 MHz, CDCl₃) δ 8.47 - 8.31 (m, 2H), 7.77 - 7.55 (m, 2H), 7.26 - 6.89 (m, 4H), 4.64 - 3.65 (m, 8H), 3.55 - 3.32 (m, 4H), 3.05 - 2.82 (m, 5H), 2.70 (s, 3H), 2.49 - 2.29 (m, 1H), 2.23 - 1.55 (m, 8H), 1.38 - 1.14 (m, 1H), 1.13 - 0.76 (m, 12H), 0.31 - 0.22 (m, 2H). |
| **391** | 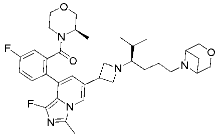 | 622.4 | ¹H NMR (400 MHz, CD₃OD) δ 7.80 (d, J = 14.8 Hz, 1H), 7.65 - 7.49 (m, 1H), 7.39 - 7.14 (m, 2H), 6.90 - 6.57 (m, 1H), 4.61 - 4.02 (m, 4H), 3.80 - 3.56 (m, 7H), 3.51 (d, J = 5.2 Hz, 2H), 3.27 - 3.20 (m, 2H), 3.18 - 2.88 (m, 2H), 2.68 (s, 2H), 2.60 (s, 3H), 2.25 (s, 1H), 1.87 (d, J = 8.4 Hz, 2H), 1.65 - 1.22 (m, 6H), 1.21 - 1.12 (m, 1H), 1.00 - 0.73 (m, 8H). |
| **392** | 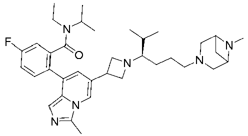 | 603.5 | ¹H NMR (400 MHz, CD₃OD) δ 8.53 (s, 2H), 8.10 (s, 1H), 7.78 - 7.66 (m, 1H), 7.46 - 7.16 (m, 3H), 7.01 - 6.90 (m, 1H), 4.65 - 4.02 (m, 8H), 3.60 - 3.34 (m, 3H), 3.29 - 2.96 (m, 7H), 2.75 (s, 2H), 2.69 (s, 3H), 2.39 - 2.27 (m, 1H), 2.14 (s, 1H), 1.80 - 1.58 (m, 4H), 1.19 - 0.78 (m, 14H), 0.35 - 0.28 (m, 2H). |
| **393** | 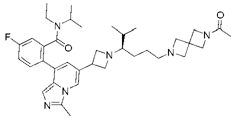 | 631.7 | ¹H NMR (400 MHz, CD₃OD) δ 8.07 - 7.95 (m, 1H), 7.80 - 7.64 (m, 1H), 7.46 - 7.09 (m, 3H), 7.03 - 6.91 (m, 1H), 4.37 - 4.33 (m, 2H), 4.29 - 4.04 (m, 5H), 4.01 - 3.93 (m, 4H), 3.93 - 3.80 (m, 3H), 3.61 - 3.36 (m, 2H), 3.11 - 2.85 (m, 5H), 2.09 - 1.95 (m, 1H), 1.88 - 1.79 (m, 3H), 1.64 - 1.53 (m, 3H), 1.50 - 1.42 (m, 1H), 1.35 - 1.28 (m, 1H), 1.07 - 0.78 (m, 13H), 0.32 - 0.26 (m, 2H). |
| **395** | 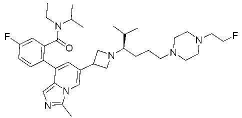 | 623.8 | ¹H NMR (400 MHz, CD₃OD) δ 7.95 - 7.86 (m, 1H), 7.79 - 7.65 (m, 1H), 7.42 - 7.04 (m, 3H), 6.98 - 6.79 (m, 1H), 4.67 - 4.58 (m, 1H), 4.53 - 4.26 (m, 1H), 3.84 - 3.38 (m, 5H), 3.28 - 3.22 (m, 1H), 3.10 - 2.97 (m, 1H), 2.87 - 2.41 (m, 13H), 2.40 - 2.33 (m, 2H), 2.30 - 2.22 (m, 1H), 1.97 - 1.79 (m, 1H), 1.70 - 0.71 (m, 18H), 0.33 - 0.28 (m, 2H). |
| **397** | 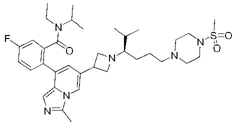 | 655.5 | ¹H NMR (400 MHz, DMSO-*d₆*) δ 7.93 (s, 1H), 7.74 - 7.62 (m, 1H), 7.44 - 7.25 (m, 2H), 7.08 - 6.98 (m, 1H), 6.86 - 6.69 (m, 1H), 4.32 - 3.38 (m, 4H), 3.28 (s, 1H), 3.19 - 3.01 (m, 6H), 2.98 - 2.87 (m, 1H), 2.84 (s, 3H), 2.58 (s, 3H), 2.42 (s, 4H), 2.29 (s, 2H), 2.03 (s, 1H), 1.71 (s, 1H), 1.45 (s, 2H), 1.33 - 0.98 (m, 3H), 0.92 - 0.69 (m, 12H), 0.26 (br d, J = 6.4 Hz, 2H). |
| **400-1** | 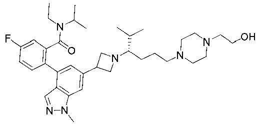 | 621.4 | ¹H NMR (400 MHz, DMSO-*d₆*) δ 7.84 - 7.77 (m, 1H), 7.63 - 7.49 (m, 2H), 7.42 - 7.26 (m, 2H), 7.13 - 7.02 (m, 1H), 4.33 (t, J = 5.6 Hz, 1H), 4.04 (s, 3H), 3.69 - 3.60 (m, 3H), 3.48 - 3.36 (m, 3H), 3.31 - 3.24 (m, 1H), 3.20 - 3.06 (m, 2H), 2.88 - 2.75 (m, 1H), 2.42 - 2.24 (m, 9H), 2.23 - 2.18 (m, 2H), 2.06 - 1.99 (m, 1H), 1.79 - 1.67 (m, 1H), 1.50 - 1.37 (m, 2H), 1.31 - 1.16 (m, 2H), 1.07 - 0.59 (m, 14H), 0.11 - 0.05 (m, 2H). |
| **400-2** | 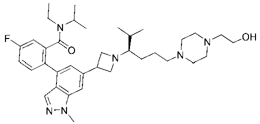 | 621.6 | ¹H NMR (400 MHz, CDCl₃) δ 7.85 (s, 1H), 7.59 - 7.52 (m, 1H), 7.25 - 7.12 (m, 3H), 4.10 (s, 3H), 3.93 - 3.76 (m, 3H), 3.61 (t, J = 5.2 Hz, 2H), 3.46 (td, J = 6.4, 13.2 Hz, 2H), 3.30 - 3.13 (m, 2H), 2.86 - 2.77 (m, 1H), 2.63 - 2.28 (m, 12H), 2.10 (br s, 1H), 1.97 - 1.76 (m, 3H), 1.62 - 1.49 (m, 2H), 1.44 - 1.23 (m, 2H), 1.13 - 0.67 (m, 13H), 0.06 (d, J = 6.8 Hz, 2H). |
| **401-1** | 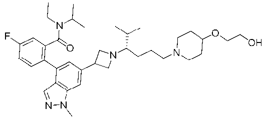 | 636.9 | ¹H NMR (400 MHz, DMSO-*d₆*) δ 7.86 - 7.71 (m, 1H), 7.65 - 7.45 (m, 2H), 7.43 - 7.21 (m, 2H), 7.16 - 6.96 (m, 1H), 4.60 - 4.38 (m, 1H), 4.26 - 3.99 (m, 3H), 3.74 - 3.55 (m, 3H), 3.51 - 3.33 (m, 5H), 3.32 - 3.02 (m, 5H), 2.87 - 2.59 (m, 3H), 2.27 - 2.13 (m, 2H), 2.08 - 1.86 (m, 3H), 1.84 - 1.65 (m, 3H), 1.50 - 0.95 (m, 7H), 0.90 - 0.77 (m, 8H), 0.71 - 0.63 (m, 3H), 0.11 - 0.03 (m, 2H). |
| **401-2** | 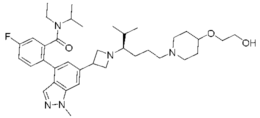 | 636.6 | ¹H NMR (400 MHz, DMSO-*d₆*) δ 7.86 - 7.76 (m, 1H), 7.63 - 7.49 (m, 2H), 7.44 - 7.24 (m, 2H), 7.13 - 7.00 (m, 1H), 4.64 - 4.33 (m, 1H), 4.26 - 3.96 (m, 3H), 3.76 - 3.53 (m, 3H), 3.49 - 3.33 (m, 5H), 3.31 - 3.04 (m, 5H), 2.87 - 2.61 (m, 3H), 2.34 - 2.12 (m, 2H), 2.11 - 1.87 (m, 3H), 1.86 - 1.65 (m, 3H), 1.49 - 0.97 (m, 7H), 0.89 - 0.79 (m, 8H), 0.70 - 0.64 (m, 3H), 0.12 - 0.03 (m, 2H). |
| **404** | 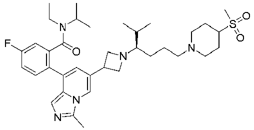 | 654.3 | ¹H NMR (400 MHz, DMSO-*d₆*) δ 7.93 (s, 1H), 7.75 - 7.62 (m, 1H), 7.44 - 7.24 (m, 2H), 7.11 - 6.98 (m, 1H), 6.88 - 6.68 (m, 1H), 3.60 - 3.34 (m, 4H), 3.32 - 3.25 (m, 1H), 3.20 - 2.91 (m, 6H), 2.91 - 2.88 (m, 3H), 2.59 (s, 3H), 2.29 - 2.19 (m, 2H), 2.05 - 1.66 (m, 6H), 1.62 - 1.40 (m, 4H), 1.34 - 0.97 (m, 3H), 0.92 - 0.70 (m, 12H), 0.27 (d, J = 6.4 Hz, 2H). |
| **405** | 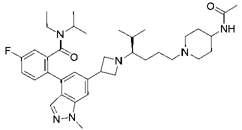 | 633.5 | ¹H NMR (400 MHz, CD₃OD) δ 7.77 (s, 1H), 7.56 - 7.50 (m, 1H), 7.42 - 7.35 (m, 1H), 7.24 (dt, J = 2.8, 8.4 Hz, 1H), 7.17 - 7.08 (m, 1H), 7.06 (s, 1H), 3.98 (s, 3H), 3.84 - 3.69 (m, 3H), 3.59 - 3.48 (m, 1H), 3.38 (td, J = 6.8, 13.2 Hz, 1H), 3.33 - 3.22 (m, 2H), 3.22 - 3.18 (m, 6H), 2.88 - 2.76 (m, 3H), 2.26 (dt, J = 3.2, 8.0 Hz, 2H), 2.20 - 2.13 (m, 1H), 2.01 (t, J = 11.0 Hz, 2H), 1.58 - 1.13 (m, 7H), 0.97 (d, J = 7.2 Hz, 1H), 0.89 - 0.78 (m, 9H), 0.75 - 0.58 (m, 4H), - 0.01 (d, J = 6.8 Hz, 2H). |
| **406** | 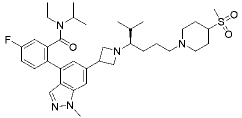 | 654.4 | ¹H NMR (400 MHz, DMSO7.85 - 7.76 (m, 1H), 7.65 - 7.47 (m, 2H), 7.43 - 7.22 (m, 2H), 7.17 - 6.98 (m, 1H), 4.27 - 4.00 (m, 3H), 3.70 - 3.58 (m, 3H), 3.43 - 3.32 (m, 1H), 3.29 - 3.24 (m, 1H), 3.20 - 3.06 (m, 2H), 3.04 - 2.91 (m, 3H), 2.89 (s, 3H), 2.86 - 2.77 (m, 1H), 2.25 (t, J = 7.2 Hz, 2H), 2.07 - 2.00 (m, 1H), 1.98 - 1.83 (m, 4H), 1.79 - 1.68 (m, 1H), 1.62 - 1.40 (m, 4H), 1.32 - 1.16 (m, 2H), 1.04 - 0.61 (m, 13H), 0.08 (d, J = 6.4 Hz, 2H). |
| **407** | 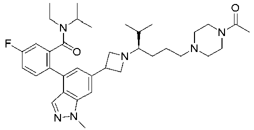 | 619.9 | ¹H NMR (400 MHz, DMSO-*d₆*) δ 7.81 (s, 1H), 7.65 - 7.48 (m, 2H), 7.43 - 7.23 (m, 2H), 7.17 - 7.00 (m, 1H), 4.04 (s, 3H), 3.65 (s, 3H), 3.38 (s, 5H), 3.29 - 3.07 (m, 3H), 2.87 - 2.58 (m, 1H), 2.38 - 2.18 (m, 6H), 2.04 (s, 1H), 1.96 (s, 3H), 1.80 - 1.68 (m, 1H), 1.47 (s, 2H), 1.34 - 1.16 (m, 2H), 1.07 - 0.60 (m, 13H), 0.08 (d, J = 6.4 Hz, 2H). |
| **409** | 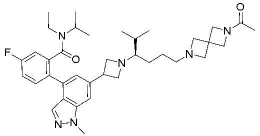 | 631.3 | ¹H NMR (400 MHz, DMSO-*d₆*) δ 7.85 - 7.79 (m, 1H), 7.65 - 7.50 (m, 2H), 7.44 - 7.26 (m, 2H), 7.14 - 7.02 (m, 1H), 4.11 (s, 2H), 4.07 - 4.02 (m, 3H), 3.83 (s, 2H), 3.69 - 3.59 (m, 3H), 3.41 - 3.35 (m, 1H), 3.30 - 3.22 (m, 1H), 3.19 - 3.08 (m, 6H), 2.88 - 2.76 (m, 1H), 2.31 - 2.21 (m, 2H), 2.03 - 1.96 (m, 1H), 1.76 - 1.68 (m, 4H), 1.26 - 1.14 (m, 3H), 1.04 - 0.64 (m, 14H), 0.10 - 0.04 (m, 2H). |
| **410** | 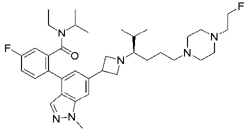 | 623.3 | ¹H NMR (400 MHz, CDCl₃) δ 7.85 - 7.76 (m, 1H), 7.54 - 7.44 (m, 1H), 7.28 - 7.21 (m, 1H), 7.19 - 7.02 (m, 3H), 4.56 (q, J = 4.4 Hz, 1H), 4.44 (q, J = 4.4 Hz, 1H), 4.04 (s, 3H), 3.94 - 3.13 (m, 6H), 2.86 - 2.22 (m, 13H), 1.91- 1.60 (m, 4H), 1.46 - 1.17 (m, 3H), 1.08 - 0.59 (m, 13H), 0.04 - 0.04 (m, 2H). |
| **411** | 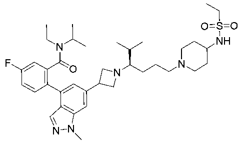 | 683.6 | ¹H NMR (400 MHz, DMSO-*d₆*) δ 7.88 - 7.71 (m, 1H), 7.68 - 7.44 (m, 2H), 7.43 - 7.19 (m, 2H), 7.15 - 6.90 (m, 2H), 4.23 - 3.98 (m, 3H), 3.72 - 3.54 (m, 3H), 3.42 - 3.33 (m, 1H), 3.27 - 2.91 (m, 7H), 2.86 - 2.65 (m, 3H), 2.24 - 2.12 (m, 2H), 2.08 - 1.97 (m, 1H), 1.92 - 1.80 (m, 2H), 1.78 - 1.67 (m, 3H), 1.49 - 1.34 (m, 4H), 1.28 - 0.98 (m, 6H), 0.89 - 0.80 (m, 8H), 0.71 - 0.64 (m, 3H), 0.11 - 0.04 (m, 2H). |
| **412** | 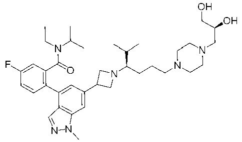 | 651.3 | ¹H NMR (400 MHz, DMSO-*d₆*) δ 7.84-7.79 (m, 1H), 7.62 - 7.50 (m, 2H), 7.42 - 7.26 (m, 2H), 7.13 - 7.01 (m, 1H), 4.56 - 4.46 (m, 1H), 4.34 - 4.30 (m, 1H), 4.08 - 4.02 (m, 3H), 3.68 - 3.54 (m, 4H), 3.41 - 3.35 (m, 1H), 3.31 - 3.27 (m, 2H), 3.18 - 3.07 (m, 2H), 2.87 - 2.78 (m, 1H), 2.44 - 2.15 (m, 12H), 2.06 - 2.00 (m, 1H), 1.77 - 1.69 (m, 1H), 1.48 - 1.40 (m, 2H), 1.30 - 1.24 (m, 1H), 1.22 - 1.16 (m, 1H), 1.03 - 0.63 (m, 14H), 0.11 - 0.05 (m, 2H). |
| **413** | 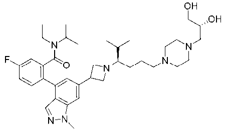 | 651.3 | ¹H NMR (400 MHz, DMSO-*d₆*) δ 7.85 -7.76 (m, 1H), 7.64 - 7.49 (m, 2H), 7.43 - 7.26 (m, 2H), 7.14 - 7.02 (m, 1H), 4.65 - 4.28 (m, 2H), 4.10 - 3.99 (m, 3H), 3.73 - 3.52 (m, 4H), 3.42 - 3.35 (m, 1H), 3.30 - 3.25 (m, 2H), 3.20 - 3.06 (m, 2H), 2.87 - 2.74 (m, 1H), 2.46 - 2.15 (m, 12H), 2.08 - 1.99 (m, 1H), 1.80 - 1.67 (m, 1H), 1.50 - 1.38 (m, 2H), 1.31 - 1.24 (m, 1H), 1.23 - 1.16 (m, 1H), 1.06 - 0.62 (m, 14H), 0.10 - 0.06 (m, 2H). |
| **417** | 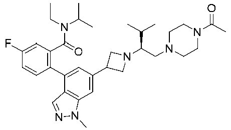 | 591.8 | ¹H NMR (400 MHz, DMSO-*d₆*) δ 7.85 -7.77 (m, 1H), 7.63 - 7.45 (m, 2H), 7.42 - 7.25 (m, 2H), 7.18 - 7.04 (m, 1H), 4.26 - 3.99 (m, 3H), 3.78 - 3.54 (m, 3H), 3.47 - 3.33 (m, 5H), 3.31 - 3.09 (m, 3H), 2.89 - 2.67 (m, 1H), 2.48 - 2.13 (m, 8H), 2.01 - 1.89 (m, 3H), 1.85 - 1.69 (m, 1H), 1.29 - 0.94 (m, 1H), 0.94 - 0.81 (m, 8H), 0.72 - 0.63 (m, 3H), 0.10 - 0.03 (m, 2H). |
| **418** | 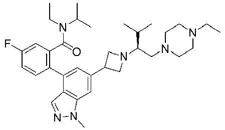 | 577.3 | ¹H NMR (400 MHz, DMSO-*d₆*) δ 7.86 - 7.78 (m, 1H), 7.63 - 7.49 (m, 2H), 7.42 - 7.26 (m, 2H), 7.18 - 7.05 (m, 1H), 4.04 (s, 3H), 3.71 - 3.62 (m, 3H), 3.42 - 3.38 (m, 1H), 3.30 - 3.10 (m, 4H), 2.89 - 2.77 (m, 1H), 2.37 - 2.14 (m, 11H), 1.82 - 1.71 (m, 1H), 1.04 - 0.80 (m, 14H), 0.70 - 0.64 (m, 3H), 0.13 - 0.04 (m, 2H). |
| **419** | 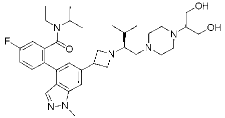 | 623.4 | ¹H NMR (400 MHz, DMSO-*d₆*) δ 7.85 -7.77 (m, 1H), 7.63 - 7.48 (m, 2H), 7.43 - 7.25 (m, 2H), 7.17 - 7.03 (m, 1H), 4.27 - 4.13 (m, 2H), 4.04 (s, 3H), 3.72 - 3.60 (m, 3H), 3.47 - 3.39 (m, 4H), 3.29 - 3.10 (m, 3H), 2.88 - 2.76 (m, 1H), 2.61 - 2.54 (m, 4H), 2.45 - 2.22 (m, 6H), 2.18 - 2.09 (m, 2H), 1.81 - 1.72 (m, 1H), 1.04 - 0.64 (m, 14H), 0.12 - 0.05 (m, 2H). |
| **420** | 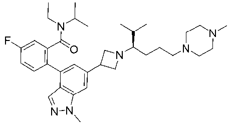 | 591.5 | ¹H NMR (400 MHz, CD₃OD) δ 7.87 (s, 1H), 7.67 - 7.59 (m, 1H), 7.54 - 7.46 (m, 1H), 7.38 - 7.30 (m, 1H), 7.27 - 7.14 (m, 2H), 4.09 (s, 3H), 3.93 - 3.76 (m, 3H), 3.55 - 3.34 (m, 2H), 2.98 - 2.21 (m, 16H), 1.95 - 1.82 (m, 1H), 1.67 - 1.53 (m, 2H), 1.49 - 1.26 (m, 2H), 1.13 - 0.69 (m, 14H), 0.10 (d, J = 6.8 Hz, 2H). |
| **422** | 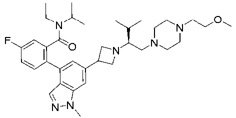 | 607.4 | ¹H NMR (400 MHz, DMSO-*d₆*) δ 7.85 - 7.74 (m, 1H), 7.64 - 7.46 (m, 2H), 7.44 - 7.23 (m, 2H), 7.18 - 7.01 (m, 1H), 4.10 - 3.98 (m, 3H), 3.71 - 3.60 (m, 3H), 3.42 - 3.35 (m, 3H), 3.32 - 3.14 (m, 8H), 2.88 - 2.78 (m, 1H), 2.45 - 2.20 (m, 10H), 2.18 - 2.11 (m, 2H), 1.82 - 1.69 (m, 1H), 1.05 - 0.96 (m, 1H), 0.92 - 0.80 (m, 8H), 0.71 - 0.64 (m, 3H), 0.10 - 0.04 (m, 2H). |
| **423** | 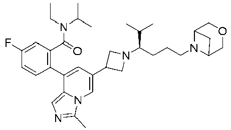 | 590.4 | ¹H NMR (400 MHz, CD₃OD) δ 7.99 - 7.82 (m, 1H), 7.79 - 7.61 (m, 1H), 7.45 - 7.04 (m, 3H), 6.98 - 6.83 (m, 1H), 4.32 - 4.17 (m, 2H), 3.90 - 3.36 (m, 9H), 3.30 - 3.23 (m, 2H), 3.13 - 2.92 (m, 1H), 2.79 - 2.51 (m, 6H), 2.33 - 2.19 (m, 1H), 1.94 - 1.79 (m, 2H), 1.58 - 1.09 (m, 5H), 1.02 - 0.77 (m, 12H), 0.33 - 0.28 (m, 2H). |
| **425** | 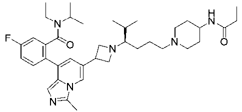 | 647.5 | ¹H NMR (400 MHz, CD₃OD) δ 8.39 (s, 2H), 8.06 - 8.00 (m, 1H), 7.77 - 7.70 (m, 1H), 7.40 - 7.24 (m, 2H), 7.22 - 7.15 (m, 1H), 7.01 - 6.89 (m, 1H), 4.37 - 4.17 (m, 2H), 4.02 - 3.81 (m, 4H), 3.63 - 3.50 (m, 1H), 3.48 - 3.36 (m, 3H), 3.11 - 2.83 (m, 6H), 2.68 (s, 3H), 2.25 - 2.14 (m, 2H), 2.12 - 2.00 (m, 3H), 1.89 - 1.26 (m, 7H), 1.14 - 0.78 (m, 16H), 0.30 (d, J = 6.4 Hz, 2H). |
| **426** | 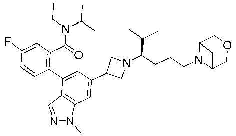 | 590.3 | ¹H NMR (400 MHz, DMSO-*d₆*) δ 7.83 - 7.79 (m, 1H), 7.63 - 7.56 (m, 1H), 7.54 - 7.49 (m, 1H), 7.42 - 7.35 (m, 1H), 7.33 - 7.26 (m, 1H), 7.13 - 7.02 (m, 1H), 4.08 - 4.01 (m, 5H), 3.69 - 3.60 (m, 3H), 3.53 (d, J = 10.8 Hz, 2H), 3.41 - 3.33 (m, 4H), 3.31 - 3.25 (m, 1H), 3.19 - 3.07 (m, 2H), 2.87 - 2.78 (m, 1H), 2.41 (q, J = 6.4 Hz, 1H), 2.05 - 1.99 (m, 1H), 1.77 - 1.64 (m, 2H), 1.40 - 1.22 (m, 4H), 1.05 - 0.80 (m, 10H), 0.72 - 0.62 (m, 4H), 0.07 (d, J = 5.6 Hz, 2H). |
| **428** | 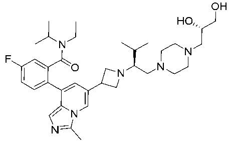 | 623.5 | ¹H NMR (400 MHz, DMSO-*d₆*) δ 7.91 (d, J = 4.3 Hz, 1H), 7.72 - 7.63 (m, 1H), 7.42 - 7.27 (m, 2H), 7.05 - 7.00 (m, 1H), 6.79 (d, J = 14.5 Hz, 1H), 4.50 (br s, 1H), 4.31 (dd, J = 2.3, 4.3 Hz, 1H), 3.67 - 3.37 (m, 5H), 3.31 - 2.88 (m, 6H), 2.58 (s, 3H), 2.47 - 2.07 (m, 13H), 1.72 (br d, J = 2.5 Hz, 1H), 1.07 - 0.71 (m, 13H), 0.26 (br d, J = 6.5 Hz, 2H). |
| **429** | 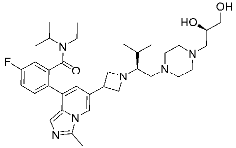 | 623.4 | ¹H NMR (400 MHz, DMSO-*d₆*) δ 7.91 (d, J = 4.0 Hz, 1H), 7.71 - 7.63 (m, 1H), 7.42 - 7.27 (m, 2H), 7.05 - 7.00 (m, 1H), 6.90 - 6.76 (m, 1H), 4.54 - 4.46 (m, 1H), 4.35 - 4.28 (m, 1H), 3.62 - 3.37 (m, 5H), 3.31 - 2.85 (m, 6H), 2.58 (s, 3H), 2.48 - 2.09 (m, 13H), 1.79 - 1.66 (m, 1H), 1.08 - 0.71 (m, 13H), 0.26 (br d, J = 6.4 Hz, 2H). |
| **430** | 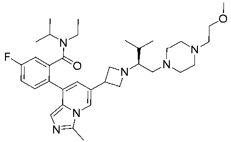 | 607.4 | ¹H NMR (400 MHz, CDCl₃) δ 7.92 (d, J = 3.6 Hz, 1H), 7.72 - 7.64 (m, 1H), 7.41 - 7.28 (m, 2H), 7.07 - 7.01 (m, 1H), 6.91 - 6.77 (m, 1H), 3.63 - 3.34 (m, 6H), 3.31 - 2.88 (m, 6H), 2.59 (s, 3H), 2.46 - 2.10 (m, 13H), 1.81 - 1.66 (m, 1H), 1.08 - 0.72 (m, 13H), 0.27 (d, J = 6.4 Hz, 2H). |
| **432** | 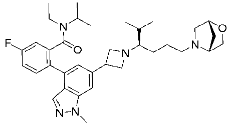 | 590.5 | ¹H NMR (400 MHz, DMSO-*d₆*) δ 7.83 - 7.79 (m, 1H), 7.63 - 7.56 (m, 1H), 7.55 - 7.49 (m, 1H), 7.38 (dt, J = 2.8, 8.8 Hz, 1H), 7.33 - 7.25 (m, 1H), 7.13 - 7.01 (m, 1H), 4.31 - 4.20 (m, 1H), 4.04 (s, 3H), 3.79 (d, J = 7.2 Hz, 1H), 3.68 - 3.60 (m, 3H), 3.48 - 3.43 (m, 1H), 3.39 (s, 1H), 3.20 - 3.06 (m, 2H), 2.88 - 2.77 (m, 1H), 2.72 (d, J = 9.6 Hz, 1H), 2.54 (s, 1H), 2.43 - 2.30 (m, 2H), 2.06 - 1.99 (m, 1H), 1.78 - 1.63 (m, 2H), 1.51 (d, J = 9.2 Hz, 1H), 1.45 - 1.17 (m, 5H), 1.05 - 0.96 (m, 1H), 0.88 - 0.81 (m, 9H), 0.71 - 0.62 (m, 4H), 0.07 (d, J = 6.4 Hz, 2H). |
| **434** | 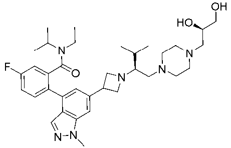 | 623.4 | ¹H NMR (400 MHz, DMSO-*d₆*) δ 7.83 - 7.79 (m, 1H), 7.62 - 7.56 (m, 1H), 7.53 - 7.48 (m, 1H), 7.42 - 7.35 (m, 1H), 7.32 - 7.26 (m, 1H), 7.11 - 7.05 (m, 1H), 4.54 - 4.46 (m, 1H), 4.35 - 4.28 (m, 1H), 4.07 - 4.00 (m, 3H), 3.72 - 3.61 (m, 3H), 3.58 - 3.52 (m, 1H), 3.42 - 3.35 (m, 1H), 3.30 - 3.23 (m, 4H), 3.23 - 3.17 (m, 1H), 2.89 - 2.77 (m, 1H), 2.70 - 2.55 (m, 1H), 2.44 - 2.24 (m, 9H), 2.20 - 2.14 (m, 3H), 1.82 - 1.70 (m, 1H), 0.92 - 0.88 (m, 3H), 0.84 (t, J = 6.8 Hz, 6H), 0.74 - 0.62 (m, 4H), 0.10 - 0.06 (m, 2H). |
| **435** | 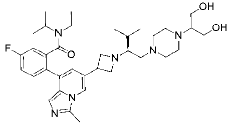 | 623.4 | ¹H NMR (400 MHz, DMSO-*d₆*) δ 8.00-7.81 (m, 1H), 7.75 - 7.58 (m, 1H), 7.47 - 7.20 (m, 2H), 7.13 - 6.91 (m, 1H), 6.90 - 6.75 (m, 1H), 4.38 - 4.10 (m, 2H), 3.63 - 3.34 (m, 8H), 3.30 - 2.65 (m, 5H), 2.60 - 2.54 (m, 6H), 2.48 - 2.20 (m, 6H), 2.18 - 2.07 (m, 2H), 1.81 - 1.67 (m, 1H), 1.25 - 1.05 (m, 1H), 1.02 - 0.66 (m, 12H), 0.31 - 0.24 (m, 2H). |
| **440** | 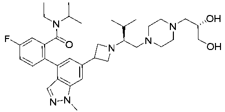 | 623.3 | ¹H NMR (400 MHz, DMSO-*d₆*) δ 7.78 - 7.83 (m, 1 H), 7.56 - 7.62 (m, 1 H), 7.48 -7.54 (m, 1 H), 7.39 (td, J=8.60, 2.80 Hz, 1 H), 7.26 - 7.34 (m, 1 H), 7.08 (d, J=13.20 Hz, 1 H), 4.51 (br s, 1 H), 4.32 (d, J=4.40 Hz, 1 H), 4.04 (s, 3 H), 3.62 - 3.70 (m, 3 H), 3.53 - 3.59 (m, 1 H), 3.37 (br d, J=6.80 Hz, 1 H), 3.26 - 3.31 (m, 3 H), 3.18 - 3.24 (m, 1 H), 2.83 (ddd, J=13.60, 6.80, 3.20 Hz, 1 H), 2.39 (br d, J=3.20 Hz, 5 H), 2.24 - 2.33 (m, 4 H), 2.10 - 2.23 (m, 4 H), 1.72 - 1.81 (m, 1 H), 1.01 (br d, J=6.00 Hz, 1 H), 0.88 - 0.92 (m, 3 H), 0.84 (t, J=6.80 Hz, 6 H), 0.68 (br t, J=6.80 Hz, 4 H), 0.08 (br d, J=6.40 Hz, 2 H). |
| **442** | 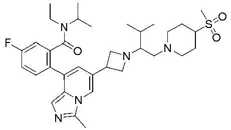 | 627.4 | ¹H NMR (400 MHz, CD₃OD) δ 7.92 (d, J = 8.4 Hz, 1H), 7.78 - 7.67 (m, 1H), 7.40 - 7.19 (m, 2H), 7.17 - 7.08 (m, 1H), 7.03 - 6.86 (m, 1H), 3.93 - 3.79 (m, 1H), 3.77 - 3.62 (m, 2H), 3.60 - 3.35 (m, 3H), 3.28 - 3.23 (m, 2H), 3.21 - 3.17 (m, 2H), 3.11 - 2.94 (m, 1H), 2.81 (d, J = 10.0 Hz, 3H), 2.66 (s, 4H), 2.58 - 2.42 (m, 3H), 2.38 - 2.29 (m, 2H), 1.95 - 1.79 (m, 1H), 1.00 - 0.78 (m, 12H), 0.31 - 0.22 (m, 2H). |
| **443** | 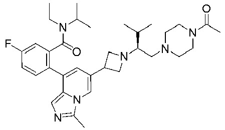 | 591.4 | ¹H NMR (400 MHz, DMSO-*d₆*) δ 7.99-7.83 (m, 1H), 7.77 - 7.58 (m, 1H), 7.47 - 7.21 (m, 2H), 7.12 - 6.93 (m, 1H), 6.93 - 6.74 (m, 1H), 4.34 - 3.36 (m, 7H), 3.26 - 2.71 (m, 4H), 2.66 - 2.52 (m, 3H), 2.43 - 2.09 (m, 8H), 2.01 - 1.90 (m, 3H), 1.81 - 1.69 (m, 1H), 1.24 - 1.03 (m, 1H), 0.91 - 0.70 (m, 12H), 0.27 - 0.20 (m, 2H). |
| **444** | 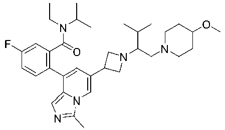 | 578.4 | ¹H NMR (400 MHz, DMSO-*d₆*) δ 7.91 (d, J=5.20 Hz, 1 H), 7.67 (dd, J=8.00, 5.60 Hz, 1 H), 7.27 - 7.42 (m, 2 H), 6.99 - 7.06 (m, 1 H), 6.79 (d, J=14.01 Hz, 1 H), 3.53 - 3.63 (m, 2 H), 3.46 (td, J=13.20, 6.80 Hz, 2 H), 3.25 - 3.29 (m, 1 H), 3.19 (s, 4 H), 3.02 - 3.15 (m, 2 H), 2.88 - 2.99 (m, 1 H), 2.73 (br d, J=2.00 Hz, 1 H), 2.55 - 2.67 (m, 4 H), 2.24 (br s, 1 H), 2.13 (br s, 2 H), 2.00 - 2.09 (m, 1 H), 1.95 (br d, J=10.00 Hz, 1 H), 1.68 - 1.85 (m, 3 H), 1.29 - 1.47 (m, 2 H), 0.96 - 1.13 (m, 1 H), 0.86 - 0.91 (m, 5 H), 0.75 - 0.84 (m, 6 H), 0.73 (br t, J=6.80 Hz, 1 H), 0.26 (br d, J=6.40 Hz, 2 H). |
| **450** | 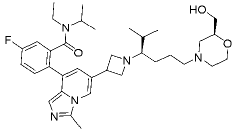 | 608.4 | ¹H NMR (400 MHz, DMSO-*d₆*) δ 7.92 (s, 1H), 7.73 - 7.63 (m, 1H), 7.42 - 7.27 (m, 2H), 7.07 - 7.00 (m, 1H), 6.86 - 6.71 (m, 1H), 4.61 (s, 1H), 4.35 - 3.68 (m, 1H), 3.60 - 3.34 (m, 7H), 3.31 - 2.74 (m, 6H), 2.59 (s, 4H), 2.30 - 2.16 (m, 2H), 2.08 - 2.00 (m, 1H), 1.98 - 1.87 (m, 1H), 1.77 - 1.61 (m, 2H), 1.53 - 1.38 (m, 2H), 1.34 - 0.97 (m, 3H), 0.92 - 0.71 (m, 12H), 0.28 (d, J = 6.4 Hz, 2H). |
| **451** | 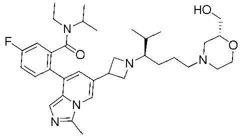 | 608.4 | ¹H NMR (400 MHz, CD₃OD) δ 8.19 - 7.97 (m, 1H), 7.87 - 7.58 (m, 1H), 7.47 - 7.11 (m, 3H), 7.05 - 6.81 (m, 1H), 4.61 - 4.37 (m, 2H), 4.29 - 3.91 (m, 4H), 3.79 - 3.37 (m, 6H), 3.30 - 3.25 (m, 1H), 3.20 - 2.97 (m, 3H), 2.80 - 2.63 (m, 5H), 2.58 - 2.46 (m, 1H), 2.41 - 2.24 (m, 1H), 2.20 - 2.02 (m, 1H), 1.85 - 1.52 (m, 4H), 1.30 - 0.79 (m, 13H), 0.34 - 0.29 (m, 2H). |
| **452** | 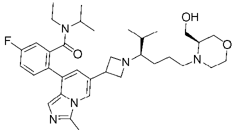 | 608.4 | ¹H NMR (400 MHz, CD₃OD) δ 8.11 - 8.02 (m, 1H), 7.86 - 7.55 (m, 1H), 7.41 - 7.16 (m, 3H), 7.03 - 6.87 (m, 1H), 4.55 - 4.33 (m, 2H), 4.29 - 4.03 (m, 3H), 3.92 - 3.37 (m, 8H), 3.25 - 2.99 (m, 4H), 2.94 - 2.86 (m, 1H), 2.85 - 2.75 (m, 2H), 2.73 - 2.65 (m, 3H), 2.20 - 2.05 (m, 1H), 1.86 - 1.55 (m, 4H), 1.15 - 0.78 (m, 13H), 0.34 - 0.28 (m, 2H). |
| **453** | 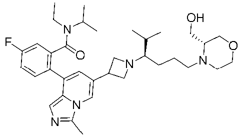 | 608.2 | ¹H NMR (400 MHz, CD₃OD) δ 7.95 - 7.88 (m, 1H), 7.77 - 7.68 (m, 1H), 7.38 - 7.31 (m, 1H), 7.29 - 7.21 (m, 1H), 7.17 - 7.12 (m, 1H), 6.96 - 6.85 (m, 1H), 3.87 - 3.35 (m, 12H), 3.28 - 3.22 (m, 1H), 3.11 - 2.97 (m, 1H), 2.85 - 2.71 (m, 2H), 2.69 - 2.64 (m, 3H), 2.50 - 2.32 (m, 3H), 2.26 (s, 1H), 1.94 - 1.80 (m, 1H), 1.71 - 1.38 (m, 3H), 1.34 - 1.06 (m, 2H), 0.99 - 0.77 (m, 12H), 0.30 (d, J = 6.4 Hz, 2H). |
| **460** | 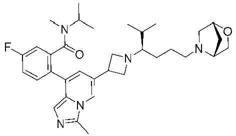 | 677.4 | ¹H NMR (400 MHz, DMSO-*d₆*) δ 8.00 - 7.82 (m, 1H), 7.82 - 7.58 (m, 1H), 7.52 - 7.18 (m, 2H), 7.18 - 6.91 (m, 1H), 6.88 - 6.69 (m, 1H), 4.55 - 4.13 (m, 1H), 3.86 - 3.70 (m, 1H), 3.60 - 3.33 (m, 6H), 3.18 - 3.00 (m, 2H), 2.76 - 2.69 (m, 1H), 2.62 - 2.57 (m, 4H), 2.48 - 2.28 (m, 4H), 2.08 - 1.99 (m, 1H), 1.79 - 0.19 (m, 20H). |
| **466** | 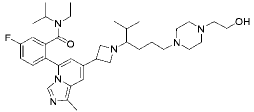 | 621.3 | ¹H NMR (400 MHz, CDCl₃) δ 7.83 (br s, 1H), 7.73 - 7.63 (m, 1H), 7.26 - 7.10 (m, 3H), 6.65 - 6.46 (m, 1H), 3.83 - 3.42 (m, 7H), 3.21 - 3.05 (m, 2H), 2.96 - 2.81 (m, 1H), 2.66 - 2.53 (m, 6H), 2.52 (s, 3H), 2.50 - 2.44 (m, 2H), 2.33 (br t, J = 7.6 Hz, 2H), 2.17 - 2.01 (m, 1H), 1.87 - 1.75 (m, 2H), 1.56 (br d, J = 4.0 Hz, 3H), 1.39 - 1.24 (m, 2H), 1.15 - 0.72 (m, 13H), 0.28 - 0.02 (m, 2H). |
| **468** | 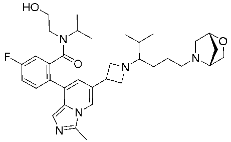 | 606.1 | ¹H NMR (400 MHz,CD₃OD) δ 7.96 - 7.86 (m, 1H), 7.79 - 7.65 (m, 1H), 7.41 - 7.22 (m, 2H), 7.21 - 7.10 (m, 1H), 6.97 - 6.82 (m, 1H), 4.58 (d, J = 5.2 Hz, 1H), 4.45 - 4.36 (m, 1H), 4.07 - 3.96 (m, 1H), 3.91 - 3.33 (m, 9H), 3.31 - 3.02 (m, 4H), 2.91 - 2.82 (m, 1H), 2.66 (s, 3H), 2.63 - 2.52 (m, 2H), 2.28 (d, J = 4.0 Hz, 1H), 1.98 - 1.81 (m, 2H), 1.79 - 1.69 (m, 1H), 1.68 - 1.51 (m, 2H), 1.47 - 1.10 (m, 3H), 1.06 - 0.87 (m, 9H), 0.35 - 0.25 (m, 2H). |
| **468-1** | 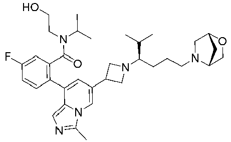 | 606.1 | ¹H NMR (400 MHz, DMSO-*d₆*) δ 8.02 - 7.80 (m, 1H), 7.77 - 7.52 (m, 1H), 7.46 - 7.21 (m, 2H), 7.12 - 6.87 (m, 1H), 6.83 - 6.62 (m, 1H), 4.75 - 4.54 (m, 1H), 4.31 - 4.16 (m, 1H), 3.84 - 3.73 (m, 1H), 3.61 - 3.41 (m, 7H), 3.19 - 3.03 (m, 3H), 3.00 - 2.83 (m, 2H), 2.76 - 2.69 (m, 1H), 2.62 - 2.56 (m, 3H), 2.45 - 2.38 (m, 1H), 2.37 - 2.31 (m, 1H), 2.08 - 2.00 (m, 1H), 1.76 - 1.64 (m, 2H), 1.55 - 1.48 (m, 1H), 1.46 - 1.34 (m, 2H), 1.33 - 1.16 (m, 3H), 1.05 - 0.78 (m, 10H), 0.29 - 0.21 (m, 2H). |
| **471** | 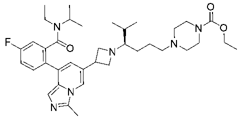 | 649.2 | ¹H NMR (400 MHz, DMSO-*d₆*) δ 7.93 (s, 1H), 7.73 - 7.64 (m, 1H), 7.42 - 7.27 (m, 2H), 7.07 - 7.00 (m, 1H), 6.75 (d, J = 15.6 Hz, 1H), 4.01 (q, J = 7.2 Hz, 2H), 3.58 - 3.40 (m, 4H), 3.32 - 3.26 (m, 5H), 3.17 - 3.03 (m, 2H), 2.97 - 2.87 (m, 1H), 2.58 (s, 3H), 2.32 - 2.21 (m, 6H), 2.08 - 1.99 (m, 1H), 1.76 - 1.66 (m, 1H), 1.52 - 1.39 (m, 2H), 1.30 - 1.19 (m, 2H), 1.16 (t, J = 7.2 Hz, 3H), 0.99 - 0.69 (m, 13H), 0.27 (d, J = 6.4 Hz, 2H). |
| **472** | 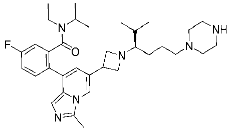 | 577.2 | ¹H NMR (400 MHz, DMSO-*d₆*) δ 7.93 (s, 1H), 7.67 (dd, J = 5.6, 8.4 Hz, 1H), 7.43 - 7.26 (m, 2H), 7.08 - 6.99 (m, 1H), 6.75 (d, J = 18.4 Hz, 1H), 3.62 - 3.39 (m, 6H), 3.34 - 3.26 (m, 1H), 3.16 - 3.01 (m, 2H), 2.92 (dd, J = 6.8, 13.6 Hz, 1H), 2.64 (s, 3H), 2.58 (s, 3H), 2.34 - 2.13 (m, 6H), 2.02 (s, 1H), 1.77 - 1.65 (m, 1H), 1.43 (s, 2H), 1.31 - 0.99 (m, 3H), 0.92 - 0.71 (m, 12H), 0.27 (d, J = 6.4 Hz, 2H). |
| **473** | 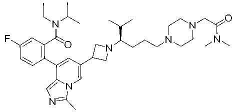 | 662.4 | ¹H NMR (400 MHz, CD₃OD) δ 7.95 - 7.88 (m, 1H), 7.71 - 7.62 (m, 1H), 7.41 - 7.26 (m, 2H), 7.06 - 6.99 (m, 1H), 6.87 - 6.70 (m, 1H), 3.59 - 3.40 (m, 4H), 3.34 - 3.25 (m, 2H), 3.19 - 3.02 (m, 4H), 2.99 (s, 3H), 2.95 - 2.86 (m, 1H), 2.78 (s, 3H), 2.58 (s, 3H), 2.43 - 2.16 (m, 9H), 2.08 - 1.99 (m, 1H), 1.78 - 1.64 (m, 1H), 1.52 - 1.35 (m, 2H), 1.29 - 0.70 (m, 15H), 0.27 (d, J = 6.4 Hz, 2H). |
| **478** | 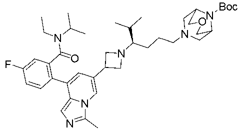 | 719.3 | ¹H NMR (400 MHz, DMSO-*d₆*) δ 7.98 - 7.79 (m, 1H), 7.74 - 7.55 (m, 1H), 7.47 - 7.21 (m, 2H), 7.16 - 6.93 (m, 1H), 6.89 - 6.60 (m, 1H), 3.87 - 3.76 (m, 2H), 3.65 (br s, 2H), 3.61 - 3.36 (m, 7H), 3.20 - 3.00 (m, 2H), 2.99 - 2.80 (m, 3H), 2.62 - 2.56 (m, 3H), 2.24 - 2.08 (m, 4H), 2.03 (br s, 1H), 1.77 - 1.65 (m, 1H), 1.54 - 1.34 (m, 11H), 1.32 - 1.01 (m, 3H), 0.97 - 0.69 (m, 12H), 0.32 - 0.24 (m, 2H). |
| **479** | 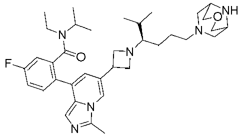 | 619.3 | ¹H NMR (400 MHz, DMSO-*d₆*) δ 7.98 - 7.84 (m, 1H), 7.75 - 7.57 (m, 1H), 7.46 - 7.16 (m, 2H), 7.12 - 6.94 (m, 1H), 6.89 - 6.53 (m, 1H), 3.70 - 3.45 (m, 9H), 3.20 - 3.00 (m, 3H), 2.96 - 2.79 (m, 3H), 2.71 - 2.65 (m, 2H), 2.61 - 2.56 (m, 3H), 2.26 - 2.09 (m, 4H), 2.07 - 1.99 (m, 1H), 1.78 - 1.65 (m, 1H), 1.51 - 1.38 (m, 2H), 1.31 - 1.06 (m, 3H), 0.95 - 0.71 (m, 12H), 0.31 - 0.25 (m, 2H) |
| **480** | 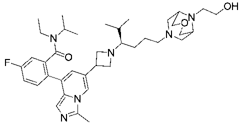 | 663.4 | ¹H NMR (400 MHz, DMSO-*d₆*) δ 7.92 (s, 1H), 7.78 - 7.55 (m, 1H), 7.47 - 7.18 (m, 2H), 7.15 - 6.92 (m, 1H), 6.87 - 6.65 (m, 1H), 4.36 - 4.24 (m, 1H), 3.84 - 3.71 (m, 2H), 3.62 - 3.39 (m, 9H), 3.19 - 3.02 (m, 2H), 2.98 - 2.85 (m, 1H), 2.75 - 2.69 (m, 2H), 2.66 - 2.55 (m, 7H), 2.46 - 2.35 (m, 2H), 2.18 - 2.07 (m, 2H), 2.06 - 1.99 (m, 1H), 1.80 - 1.62 (m, 1H), 1.52 - 1.37 (m, 2H), 1.33 - 0.99 (m, 3H), 0.93 - 0.70 (m, 12H), 0.30 - 0.25 (m, 2H). |
| **481** | 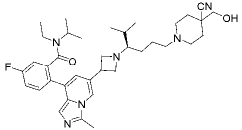 | 631.5 | ¹H NMR (400 MHz, DMSO-*d₆*) δ 7.93 (s, 1 H), 7.63 -7.72 (m, 1 H), 7.28 - 7.41 (m, 2 H), 7.00 - 7.07 (m, 1 H), 6.75 (d, J=17.60 Hz, 1 H), 5.38 (t, J=5.60 Hz, 1 H), 3.47 - 3.59 (m, 3 H), 3.43 (br d, J=4.00 Hz, 2 H), 3.26 - 3.31 (m, 1 H), 3.02 - 3.18 (m, 2 H), 2.92 (dq, J=13.60, 6.40 Hz, 1 H), 2.83 (br d, J=11.20 Hz, 2 H), 2.59 (s, 3 H), 2.23 - 2.31 (m, 2 H), 1.98 - 2.07 (m, 3 H), 1.67 - 1.81 (m, 3 H), 1.38 - 1.50 (m, 4 H), 1.01 - 1.32 (m, 3 H), 0.70 - 0.95 (m, 13 H), 0.27 (d, J=6.80 Hz, 2 H). |
| **482** | 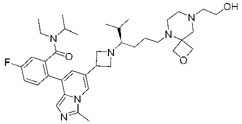 | 663.4 | ¹H NMR (400 MHz, DMSO-*d₆*) δ 7.93 (s, 1H), 7.70 - 7.64 (m, 1H), 7.42 - 7.33 (m, 1H), 7.33 - 7.28 (m, 1H), 7.06 - 7.01 (m, 1H), 6.75 (d, J = 15.6 Hz, 1H), 4.53 (d, J = 6.4 Hz, 2H), 4.38 (t, J = 5.2 Hz, 1H), 4.15 (d, J = 6.4 Hz, 2H), 3.61 - 3.41 (m, 6H), 3.30 - 3.27 (m, 1H), 3.19 - 3.03 (m, 2H), 2.97 - 2.89 (m, 1H), 2.64 - 2.53 (m, 7H), 2.45 - 2.39 (m, 2H), 2.38 - 2.30 (m, 4H), 2.08 - 2.00 (m, 1H), 1.77 - 1.66 (m, 1H), 1.52 - 1.39 (m, 2H), 1.34 - 1.21 (m, 2H), 0.94 - 0.70 (m, 13H), 0.30 - 0.24 (m, 2H). |
| **489** | 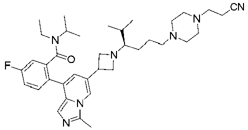 | 630.6 | ¹H NMR (400 MHz, DMSO-*d₆*) δ 7.92 (s, 1H), 7.78 - 7.58 (m, 1H), 7.46 - 7.24 (m, 2H), 7.07 - 6.98 (m, 1H), 6.88 - 6.65 (m, 1H), 3.60 - 3.40 (m, 4H), 3.30 - 2.84 (m, 4H), 2.67 - 2.52 (m, 7H), 2.45 - 2.28 (m, 7H), 2.26 - 2.19 (m, 2H), 2.07 - 1.99 (m, 1H), 1.76 - 1.66 (m, 1H), 1.52 - 1.36 (m, 2H), 1.29 - 1.03 (m, 3H), 0.99 - 0.67 (m, 13H), 0.31 - 0.24 (m, 2H) |
| **492** | 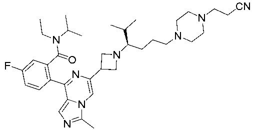 | 631.5 | ¹H NMR (400 MHz, CDCl₃) δ 7.82 - 7.73 (m, 1H), 7.63 - 7.57 (m, 1H), 7.47 (s, 1H), 7.26 - 7.11 (m, 2H), 4.49 - 3.83 (m, 1H), 3.75 - 3.61 (m, 3H), 3.36 - 3.14 (m, 4H), 2.75 - 2.69 (m, 2H), 2.68 (s, 3H), 2.55 - 2.43 (m, 8H), 2.31 (br t, J = 7.2 Hz, 2H), 2.14 - 2.09 (m, 1H), 2.06 - 1.92 (m, 3H), 1.85 - 1.75 (m, 1H), 1.61 - 1.47 (m, 2H), 1.37 - 1.22 (m, 2H), 1.13 - 1.05 (m, 3H), 1.04 - 0.99 (m, 3H), 0.94 - 0.82 (m, 8H). |
| **493** | 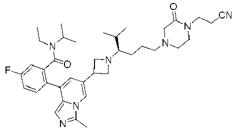 | 644.4 | ¹H NMR (400 MHz,CD₃OD) δ 8.36 (s, 1H), 8.11 - 7.99 (m, 1H), 7.82 - 7.59 (m, 1H), 7.41 - 7.18 (m, 3H), 7.05 - 6.76 (m, 1H), 4.57 - 4.42 (m, 2H), 4.36 - 4.17 (m, 2H), 4.16 - 3.99 (m, 1H), 3.67 - 3.32 (m, 7H), 3.23 - 2.97 (m, 3H), 2.89 - 2.64 (m, 7H), 2.60 - 2.42 (m, 2H), 2.21 - 2.07 (m, 1H), 1.78 - 1.54 (m, 4H), 1.14 - 0.79 (m, 13H), 0.35 - 0.28 (m, 2H). |
| **494** | 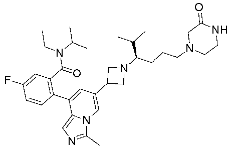 | 591.6 | ¹H NMR (400 MHz, DMSO-*d₆*) δ 7.93 (s, 1H), 7.74 - 7.62 (m, 2H), 7.43 - 7.25 (m, 2H), 7.06 - 7.00 (m, 1H), 6.84 - 6.71 (m, 1H), 3.60 - 3.42 (m, 6H), 3.17 - 3.03 (m, 4H), 2.95 - 2.85 (m, 3H), 2.60 - 2.57 (m, 3H), 2.34 - 2.27 (m, 2H), 2.06 - 2.01 (m, 1H), 1.76 - 1.66 (m, 1H), 1.52 - 1.40 (m, 2H), 1.31 - 1.04 (m, 3H), 0.96 - 0.70 (m, 13H), 0.30 - 0.26 (m, 2H). |
| **495** | 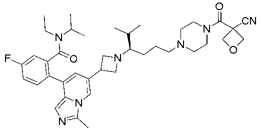 | 686.5 | ¹H NMR (400 MHz,DMSO-*d₆*) δ 7.93 (s, 1H), 7.75 - 7.65 (m, 1H), 7.44 - 7.28 (m, 2H), 7.09 - 6.99 (m, 1H), 6.87 - 6.73 (m, 1H), 5.01 - 4.92 (m, 4H), 3.60 - 3.43 (m, 6H), 3.21 - 2.89 (m, 5H), 2.59 (s, 3H), 2.43 - 2.26 (m, 6H), 2.09 - 2.00 (m, 1H), 1.77 - 1.67 (m, 1H), 1.56 - 1.40 (m, 2H), 1.33 - 1.16 (m, 2H), 1.16 - 0.66 (m, 14H), 0.27 (m, 2H). |
| **496** | 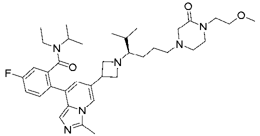 | 649.5 | ¹H NMR (400 MHz, DMSO-*d*6) δ = 7.93 (s, 1H), 7.73 - 7.64 (m, 1H), 7.41 - 7.27 (m, 2H), 7.06 - 7.01 (m, 1H), 6.85 - 6.72 (m, 1H), 3.59 - 3.40 (m, 8H), 3.31 - 3.28 (m, 2H), 3.22 (s, 3H), 3.17 - 3.04 (m, 2H), 2.97 - 2.88 (m, 3H), 2.58 (s, 5H), 2.34 - 2.25 (m, 2H), 2.07 - 2.01 (m, 1H), 1.77 - 1.68 (m, 1H), 1.51 - 1.41 (m, 2H), 1.33 - 0.68 (m, 16H), 0.29 - 0.25 (m, 2H) |
| **497** | 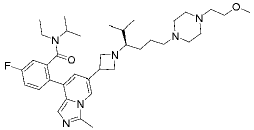 | 635.4 | ¹H NMR (400 MHz, DMSO-*d₆*) δ = 7.93 (s, 1H), 7.72 - 7.64 (m, 1H), 7.38 (dt, J = 2.8, 8.4 Hz, 1H), 7.34 - 7.27 (m, 1H), 7.07 - 7.01 (m, 1H), 6.76 (d, J = 17.2 Hz, 1H), 3.60 - 3.43 (m, 4H), 3.42 - 3.38 (m, 2H), 3.30 (br s, 1H), 3.21 (s, 3H), 3.18 - 3.04 (m, 2H), 2.99 - 2.88 (m, 1H), 2.59 (s, 3H), 2.44 - 2.26 (m, 9H), 2.21 (br t, J = 6.8 Hz, 2H), 2.08 - 2.00 (m, 1H), 1.77 - 1.68 (m, 1H), 1.52 - 1.37 (m, 2H), 1.30 - 1.16 (m, 2H), 1.14 - 1.00 (m, 1H), 0.97 - 0.68 (m, 13H), 0.29 (br d, J = 6.4 Hz, 2H) |
| **498** | 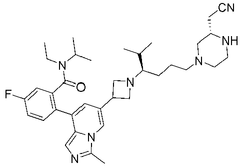 | 616.4 | ¹H NMR (400 MHz, DMSO-*d*₆) δ = 7.92 (s, 1H), 7.72 - 7.63 (m, 1H), 7.42 - 7.26 (m, 2H), 7.06 - 6.99 (m, 1H), 6.85 - 6.70 (m, 1H), 3.62 - 3.38 (m, 5H), 3.32 - 2.60 (m, 9H), 2.58 (s, 3H), 2.56 - 2.52 (m, 2H), 2.25 - 2.18 (m, 2H), 2.05 - 1.99 (m, 1H), 1.88 (t, J = 10.0 Hz, 1H), 1.71 (t, J = 7.2 Hz, 2H), 1.51- 1.36 (m, 2H), 1.30 - 1.14 (m, 2H), 1.10 - 0.70 (m, 13H), 0.28 (d, J = 6.0 Hz, 2H) |
| **499** | 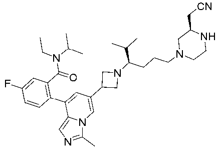 | 616.5 | ¹H NMR (400 MHz, DMSO-*d*₆) δ = 7.92 (s, 1H), 7.75 - 7.59 (m, 1H), 7.51 - 7.20 (m, 2H), 7.09 - 6.96 (m, 1H), 6.86 - 6.70 (m, 1H), 3.61 - 3.40 (m, 4H), 3.30 - 3.01 (m, 3H), 2.95 -2.52 (m, 12H), 2.25 - 2.19 (m, 2H), 2.06 - 2.00 (m, 1H), 1.93 - 1.83 (m, 1H), 1.79 - 1.63 (m, 2H), 1.50 - 1.37 (m, 2H), 1.34 - 0.99 (m, 3H), 0.92 - 0.71 (m, 12H), 0.31 - 0.25 (m, 2H) |
| **500** | 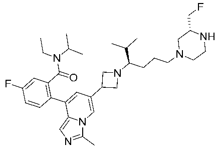 | 609.5 | ¹H NMR (400 MHz, DMSO-*d*₆) δ = 8.01 - 7.84 (m, 1H), 7.74 - 7.63 (m, 1H), 7.45 - 7.25 (m, 2H), 7.08 - 6.99 (m, 1H), 6.86 - 6.69 (m, 1H), 4.46 - 4.24 (m, 2H), 3.68 - 3.48 (m, 4H), 3.15 - 2.66 (m, 9H), 2.63 - 2.55 (m, 3H), 2.34 - 2.24 (m, 2H), 2.18 - 2.07 (m, 1H), 2.03 - 1.94 (m, 1H), 1.87 - 1.67 (m, 2H), 1.52 - 1.05 (m, 6H), 0.92 - 0.71 (m, 12H), 0.30 - 0.22 (m, 2H) |
| **501** | 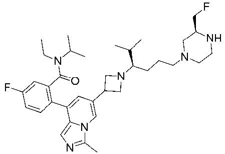 | 609.5 | ¹H NMR (400 MHz, DMSO-*d₆*) δ = 7.92 (s, 1H), 7.72 - 7.63 (m, 1H), 7.42 - 7.26 (m, 2H), 7.08 - 6.98 (m, 1H), 6.85 - 6.69 (m, 1H), 4.38 - 4.19 (m, 2H), 3.60 - 3.40 (m, 4H), 3.18 - 2.74 (m, 6H), 2.73 - 2.60 (m, 3H), 2.58 (s, 3H), 2.25 - 2.17 (m, 2H), 2.03 (s, 1H), 1.88 (t, J = 10.0 Hz, 1H), 1.76 - 1.62 (m, 2H), 1.52 - 0.93 (m, 6H), 0.92 - 0.71 (m, 12H), 0.28 (d, J = 6.4 Hz, 2H) |
| **502** | 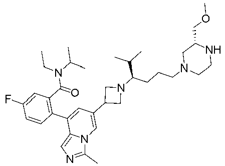 | 621.5 | ¹H NMR (400 MHz, DMSO-*d₆*) δ 7.92 (s, 1 H), 7.63 -7.74 (m, 1 H), 7.26 - 7.41 (m, 2 H), 6.99 - 7.07 (m, 1 H), 6.75 (d, *J*=17.20 Hz, 1 H), 3.42 - 3.62 (m, 5 H), 3.30 (br s, 1 H), 3.15 - 3.24 (m, 6 H), 3.03 - 3.13 (m, 2 H), 2.92 (td, *J=13.60,* 6.40 Hz, 1 H), 2.75 - 2.81 (m, 2 H), 2.57 - 2.70 (m, 6 H), 2.20 (br t, J=7.20 Hz, 2 H), 2.02 (br s, 1 H), 1.80 - 1.91 (m, 1 H), 1.68 - 1.76 (m, 1 H), 1.54 - 1.63 (m, 1 H), 1.35 - 1.50 (m, 2 H), 1.13 - 1.29 (m, 2 H), 0.90 (br d, *J=6.40* Hz, 2 H), 0.69 - 0.87 (m, 10 H), 0.28 (br d, J=6.40 Hz, 2 H) |
| **503** | 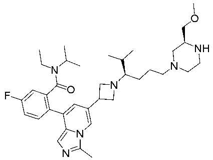 | 621.5 | ¹H NMR (400 MHz, DMSO-*d*₆) δ = 7.93 (s, 1H), 7.77 - 7.59 (m, 1H), 7.50 - 7.17 (m, 2H), 7.13 - 6.93 (m, 1H), 6.90 - 6.65 (m, 1H), 3.61 - 3.39 (m, 5H), 3.27 - 3.03 (m, 8H), 2.98 - 2.87 (m, 1H), 2.86 - 2.78 (m, 2H), 2.73 - 2.55 (m, 6H), 2.28 - 2.13 (m, 2H), 2.08 - 1.98 (m, 1H), 1.93 - 1.82 (m, 1H), 1.76 - 1.58 (m, 2H), 1.52 - 1.35 (m, 2H), 1.31 - 0.99 (m, 3H), 0.93 - 0.71 (m, 12H), 0.31 - 0.24 (m, 2H) |
| **505-1** | 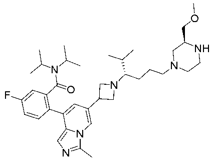 | 635.5 | ¹H NMR (400 MHz, DMSO-*d₆*) δ 7.95 -7.85 (m, 1H), 7.69 - 7.62 (m, 1H), 7.40 - 7.31 (m, 1H), 7.26 - 7.21 (m, 1H), 7.04 - 7.00 (m, 1H), 6.83 - 6.73 (m, 1H), 3.60 - 3.34 (m, 5H), 3.29 - 3.24 (m, 1H), 3.22 - 3.19 (m, 3H), 3.19 - 3.10 (m, 3H), 3.09 - 3.01 (m, 1H), 2.80 - 2.71 (m, 2H), 2.68 - 2.57 (m, 6H), 2.23 - 2.14 (m, 2H), 2.03 - 1.97 (m, 1H), 1.88 - 1.78 (m, 1H), 1.76 - 1.65 (m, 1H), 1.62 - 1.52 (m, 1H), 1.49 - 1.34 (m, 5H), 1.28 - 1.13 (m, 2H), 1.10 - 1.03 (m, 3H), 0.94 - 0.88 (m, 3H), 0.87 - 0.79 (m, 6H), 0.28 - 0.17 (m, 3H). |
| **505-2** | 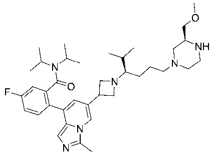 | 635.9 | ¹H NMR (400 MHz, DMSO-*d₆*) δ 7.97 -7.89 (m, 1H), 7.70 - 7.62 (m, 1H), 7.39 - 7.30 (m, 1H), 7.27 - 7.18 (m, 1H), 7.06 - 6.99 (m, 1H), 6.84 - 6.72 (m, 1H), 3.59 - 3.38 (m, 5H), 3.29 - 3.25 (m, 1H), 3.22 - 3.19 (m, 3H), 3.19 - 3.10 (m, 3H), 3.08 - 3.01 (m, 1H), 2.80 - 2.71 (m, 2H), 2.69 - 2.58 (m, 6H), 2.23 - 2.15 (m, 2H), 2.03 - 1.98 (m, 1H), 1.88 - 1.79 (m, 1H), 1.76 - 1.66 (m, 1H), 1.57 (dt, J = 4.4, 10.0 Hz, 1H), 1.49 - 1.35 (m, 5H), 1.29 - 1.05 (m, 5H), 0.91 (d, J = 6.8 Hz, 3H), 0.88 - 0.79 (m, 6H), 0.23 (d, J = 6.4 Hz, 3H). |
| **506** | 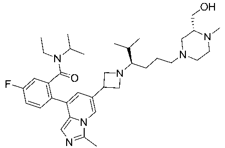 | 621.6 | ¹H NMR (400 MHz, CDCl₃) δ 7.66 (dd, J = 5.4, 8.4 Hz, 1H), 7.57 - 7.51 (m, 1H), 7.22 - 7.11 (m, 3H), 6.91 - 6.78 (m, 1H), 3.97 - 3.86 (m, 1H), 3.82 - 3.66 (m, 2H), 3.66 - 3.41 (m, 4H), 3.26 - 3.07 (m, 2H), 2.99 - 2.71 (m, 4H), 2.68 (s, 3H), 2.51 - 2.42 (m, 1H), 2.34 (s, 3H), 2.34 - 2.07 (m, 6H), 1.87 - 1.79 (m, 1H), 1.61 - 1.51 (m, 2H), 1.40 - 1.12 (m, 3H), 1.04 - 0.75 (m, 13H), 0.25 (d, J = 6.8 Hz, 2H) |
| **507** | 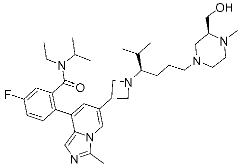 | 621.5 | ¹H NMR (400 MHz, DMSO-*d₆*) δ 7.95 -7.89 (m, 1H), 7.67 (dd, J = 5.6, 8.4 Hz, 1H), 7.42 - 7.35 (m, 1H), 7.30 (dd, J = 2.8, 9.2 Hz, 1H), 7.05 - 6.99 (m, 1H), 6.74 (d, J = 16.4 Hz, 1H), 4.51 - 4.21 (m, 1H), 3.59 - 3.44 (m, 5H), 3.25 - 3.03 (m, 4H), 2.93 (qd, J = 6.8, 13.6 Hz, 1H), 2.81 (br d, J = 10.4 Hz, 1H), 2.62 (br s, 1H), 2.59 (s, 4H), 2.22 - 2.17 (m, 2H), 2.15 (s, 3H), 2.11 (br dd, J = 8.8, 10.8 Hz, 1H), 2.04 - 2.00 (m, 1H), 1.99 - 1.92 (m, 2H), 1.76 - 1.67 (m, 2H), 1.50 - 1.38 (m, 2H), 1.31 - 1.08 (m, 3H), 0.93 - 0.76 (m, 12H), 0.31 - 0.27 (m, 2H) |
| **508** | 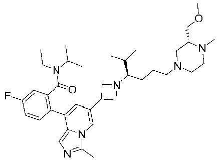 | 635.6 | ¹H NMR (400 MHz, DMSO-*d₆*) δ 7.92 (s, 1 H), 7.61 - 7.74 (m, 1 H), 7.25 - 7.44 (m, 2 H), 7.00 - 7.09 (m, 1 H), 6.75 (d, J=17.20 Hz, 1 H), 3.38 - 3.62 (m, 5 H), 3.25 - 3.32 (m, 2 H), 3.04 - 3.22 (m, 6 H), 2.86 - 2.99 (m, 1 H), 2.66 - 2.75 (m, 1 H), 2.55 - 2.65 (m, 5 H), 2.06 - 2.23 (m, 7 H), 1.93 - 2.05 (m, 2 H), 1.67 - 1.80 (m, 2 H), 1.35 - 1.48 (m, 2 H), 1.13 - 1.29 (m, 2 H), 0.90 (br d, J=6.40 Hz, 2 H), 0.69 - 0.87 (m, 10 H), 0.28 (br d, J=6.40 Hz, 2 H) |
| **509** | 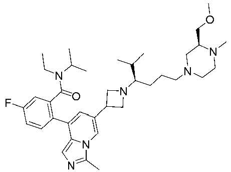 | 635.5 | ¹H NMR (400 MHz, CD₃OD) δ 7.97 - 7.86 (m, 1H), 7.78 - 7.67 (m, 1H), 7.38 - 7.29 (m, 1H), 7.27 - 7.21 (m, 1H), 7.16 - 7.12 (m, 1H), 6.94 - 6.86 (m, 1H), 3.82 - 3.32 (m, 8H), 3.30 - 3.22 (m, 4H), 3.14 - 2.97 (m, 1H), 2.95 - 2.75 (m, 3H), 2.73 - 2.59 (m, 3H), 2.42 - 2.21 (m, 8H), 2.20 - 2.11 (m, 1H), 2.05 - 1.94 (m, 1H), 1.92 - 1.81 (m, 1H), 1.69 - 1.51 (m, 2H), 1.47 - 1.10 (m, 3H), 1.02 - 0.75 (m, 12H), 0.33 - 0.27 (m, 2H) |
| **510** | 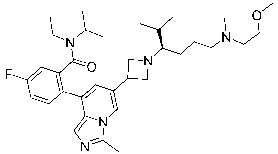 | 580.5 | ¹H NMR (400 MHz, CD₃OD) δ 7.95 - 7.86 (m, 1H), 7.75 - 7.65 (m, 1H), 7.37 - 7.27 (m, 1H), 7.26 - 7.17 (m, 1H), 7.16 - 7.07 (m, 1H), 6.94 - 6.84 (m, 1H), 3.82 - 3.35 (m, 7H), 3.31 - 3.20 (m, 4H), 3.18 - 2.69 (m, 2H), 2.65 (s, 3H), 2.62 - 2.56 (m, 2H), 2.41 (q, J = 7.2 Hz, 2H), 2.31 - 2.20 (m, 4H), 1.93 - 1.78 (m, 1H), 1.66 - 1.48 (m, 2H), 1.44 - 1.08 (m, 3H), 0.97 - 0.75 (m, 12H), 0.29 (d, J = 5.6 Hz, 2H) |

### Activity Assay Examples

### Experimental Example 1: Evaluation of Inhibitory Activity of Compounds Against Menin-MLL Binding

### Reagents and Instruments:

His-Menin (M1-L615 end), purchased from BIORTUS, Catalog Number: BP9210; and
FITC-MLL4-43, purchased from GenScript, Catalog Number: C491HFF190-3/PE1324.

### Experimental method:

In a 10 µL reaction solution (20 mM HEPES, pH 7.5, 50 mM NaCl, 1 mM DTT, 0.01% BSA) containing any compound from the examples, 150 nM His-Menin was added to a 384-well plate (Corning, Model: 4514). Each concentration was set up in duplicate wells. After centrifugation at 1000 RPM for 1 min, the plate was incubated at 25°C for 15 min. Then, 15 nM FITC-MLL1₄₋₄₃ (20 mM HEPES, pH 7.5, 50 mM NaCl, 1 mM DTT, 0.01% BSA) was added to a 384-well plate. After centrifugation at 1000 RPM for 1 min, the reaction proceeded at 25°C for 60 min. Changes in fluorescence polarization were measured using a plate reader (PerkinElmer, Envision 2104). Based on the measured signals, the binding inhibition rate of the compound at each concentration was calculated, and the IC50 (half-maximal inhibitory concentration) of the compound was determined using statistical analysis software GraphPad Prism (GraphPad Software, Inc.) by means of a nonlinear fitting formula: 4-Parameter Logistic Model or Sigmoidal Dose-Response Model: $Y = Bottom + \frac{Top - Bottom}{1 + {10}^{\left({LogIC}_{50}-X\right) \times Hill Slope}} ,$ in which X indicates the logarithm of compound concentration, Y indicates a fluorescence polarization mP value, and Top and Bottom indicate the maximum and minimum readouts of fluorescence polarization mP values. Experimental results are shown in Table 1.

**Table 1: Inhibitory Activity of Compounds Against Menin-MLL Binding**

| Example | Menin-MLL IC₅₀ (nM) | Example | Menin-MLL IC₅₀ (nM) | Example | Menin-MLL IC₅₀ (nM) | Example | Menin-MLL IC₅₀ (nM) |
|---|---|---|---|---|---|---|---|
| 4 | **17.5** | 163-2 | **54.0** | 281 | **7.0** | 394-2 | **3.2** |
| 11 | **155** | 164 | **6.0** | 281-1 | **9.0** | 395 | **2.0** |
| 11-1 | **744** | 165 | **177** | 281-2 | **79.0** | 396 | **3.0** |
| 11-2 | **65.5** | 165-1 | **122** | 282 | **4.0** | 397 | **4.2** |
| 15 | **11.5** | 165-2 | **262** | 282-1 | **9.0** | 398 | **1.9** |
| 16 | **14.5** | 166 | **271** | 282-2 | **1.0** | 399-1 | **110** |
| 17 | **9.5** | 166-1 | **231** | 283-1 | **20.0** | 399-2 | **3.3** |
| 18-1 | **6.0** | 166-2 | **248** | 283-2 | **2.0** | 400-1 | **220** |
| 18-2 | **5.5** | 167 | **57.0** | 284-1 | **39.0** | 400-2 | **3.7** |
| 19 | **43.5** | 168 | **32.5** | 284-2 | **2.0** | 401-1 | **130** |
| 20 | **45.0** | 170 | **60.0** | 285 | **4.0** | 401-2 | **3.7** |
| 21 | **18.5** | 171 | **86.0** | 286 | **3.5** | 402-1 | **110** |
| 22 | **25.0** | 172 | **98.0** | 287 | **6.0** | 402-2 | **10.0** |
| 22-1 | **23.0** | 173 | **118** | 288 | **6.0** | 403-1 | **3.9** |
| 22-2 | **14.0** | 174 | **125** | 288-1 | **11.0** | 403-2 | **10.0** |
| 23 | **102** | 175 | **138** | 288-2 | **2.0** | 404 | **2.3** |
| 24 | **23.0** | 176 | **424** | 289 | **10.0** | 405 | **1.6** |
| 25 | **19.0** | 177 | **217** | 290 | **4.0** | 406 | **7.0** |
| 25-1 | **5.7** | 178 | **115** | 291 | **3.0** | 407 | **33.0** |
| 25-2 | **21.0** | 179 | **89.0** | 291-1 | **6.0** | 408 | **1.7** |
| 26 | **44.5** | 180 | **22.0** | 292 | **4.5** | 409 | **4.0** |
| 28 | **118** | 181-1 | **621** | 293 | **3.1** | 410 | **5.8** |
| 29 | **10.5** | 181-2 | **13.5** | 294 | **9.5** | 411 | **2.7** |
| 30 | **6.0** | 182-1 | **824** | 295 | **5.0** | 412 | **6.8** |
| 31 | **275** | 182-2 | **13.0** | 296-1 | **1.0** | 413 | **6.9** |
| 34 | **168** | 183-1 | **507** | 296-2 | **3.0** | 414 | **11.8** |
| 35 | **39.5** | 183-2 | **11.7** | 297-1 | **10.8** | 415 | **160.0** |
| 36 | **156** | 184-1 | **805** | 297-2 | **13.5** | 416-1 | **150** |
| 38 | **128** | 184-2 | **13.0** | 298 | **14.5** | 416-2 | **10.6** |
| 39 | **137** | 185 | **14.5** | 299 | **15.0** | 417 | **62.0** |
| 40 | **83.0** | 186-1 | **262** | 300 | **24.0** | 418 | **6.3** |
| 41 | **92.0** | 186-2 | **5.2** | 301 | **9.0** | 419 | **8.1** |
| 42 | **3.5** | 187 | **16.5** | 302 | **22.0** | 420 | **3.8** |
| 44 | **29.5** | 188 | **12.0** | 303 | **5.0** | 421 | **14.0** |
| 45 | **95.5** | 189 | **33.0** | 304 | **8.0** | 422 | **29.0** |
| 46 | **62.5** | 190 | **29.5** | 305 | **8.0** | 423 | **1.7** |
| 47 | **25.0** | 195 | **41.0** | 306 | **6.5** | 424 | **78.0** |
| 50 | **14.0** | 196-1 | **50.0** | 307-1 | **14.5** | 425 | **2.7** |
| 51 | **64.0** | 196-2 | **17.0** | 307-2 | **6.0** | 426 | **3.2** |
| 52 | **28.5** | 197 | **6.5** | 308 | **49.0** | 427 | **120** |
| 54 | **105** | 198 | **4.0** | 309 | **60.0** | 428 | **3.7** |
| 55 | **21.0** | 199-1 | **26.0** | 310-1 | **31.0** | 429 | **2.6** |
| 55-1-1 | **14.0** | 199-2 | **205** | 310-2 | **3.6** | 430 | **8.4** |
| 55-1-2 | **120** | 200-1 | **4.3** | 311-1 | **42.0** | 431 | **3.9** |
| 55-2-1 | **343** | 200-2 | **75.0** | 311-2 | **8.5** | 432 | **4.5** |
| 57 | **107** | 201-1 | **2.6** | 312-1 | **32.0** | 433 | **3.3** |
| 58 | **124** | 201-2 | **56.0** | 312-2 | **2.8** | 434 | **8.8** |
| 59 | **23.0** | 202 | **4.3** | 313 | **48.0** | 435 | **4.9** |
| 61 | **58.0** | 203 | **3.3** | 314 | **2.5** | 436 | **8.3** |
| 64 | **144** | 204 | **4.8** | 315 | **39.0** | 437 | **12.0** |
| 65 | **136** | 205 | **10.3** | 316 | **99.0** | 438 | **14.0** |
| 68 | **298** | 206 | **3.5** | 317 | **92.0** | 439 | **21.0** |
| 69 | **222** | 207-1 | **111** | 318 | **39.0** | 440 | **8.9** |
| 70 | **93.0** | 207-2 | **269** | 319 | **19.0** | 441-1 | **11.0** |
| 71 | **15.0** | 208 | **9.0** | 319-1 | **39.0** | 441-2 | **110** |
| 72 | **54.0** | 209-1 | **29.5** | 319-2 | **328** | 442 | **21.0** |
| 73 | **28.0** | 209-2 | **4.5** | 320 | **12.0** | 443 | **19.5** |
| 74 | **120** | 210 | **3.0** | 321-1 | **10.7** | 444 | **45.0** |
| 75 | **17.5** | 211 | **3.0** | 321-2 | **3.8** | 445 | **20.0** |
| 76 | **40.0** | 212 | **12.5** | 322 | **2.0** | 446-1 | **7.3** |
| 77 | **100** | 213 | **11.0** | 323-1 | **179** | 446-2 | **44.0** |
| 78 | **55.0** | 214 | **5.0** | 323-2 | **2.7** | 447 | **25.0** |
| 79 | **279** | 215 | **11.0** | 324-1 | **126** | 448-1 | **120** |
| 80 | **122** | 216 | **23.0** | 324-2 | **2.5** | 448-2 | **2.2** |
| 81 | **188** | 217 | **9.5** | 325 | **3.2** | 449-1 | **2.6** |
| 83 | **6.0** | 218 | **13.0** | 326 | **2.0** | 449-2 | **34.0** |
| 84 | **165** | 219 | **12.0** | 327 | **1.8** | 450 | **5.2** |
| 86 | **152** | 220 | **167** | 328 | **16.0** | 451 | **4.7** |
| 88 | **52.0** | 221 | **27.0** | 329 | **11.0** | 452 | **5.3** |
| 89 | **173** | 222 | **7.0** | 330 | **16.0** | 453 | **5.3** |
| 90 | **11.0** | 223 | **15.0** | 331 | **14.0** | 454-1 | **7.7** |
| 91 | **57.0** | 224 | **26.0** | 332 | **9.3** | 454-2 | **80.0** |
| 92 | **46.5** | 225 | **28.0** | 333 | **5.0** | 455 | **9.4** |
| 93 | **21.0** | 226 | **7,5** | 334 | **30.0** | 456 | **14.0** |
| 94 | **44.5** | 227 | **29.0** | 335 | **8.0** | 457-1 | **40.0** |
| 95 | **148** | 228 | **130** | 336 | **9.0** | 457-2 | **2.6** |
| 97 | **210** | 229 | **57.0** | 337 | **4.0** | 458 | **6.4** |
| 100 | **12.0** | 230 | **2.0** | 338 | **9.0** | 459-1 | **1.9** |
| 101 | **45.5** | 231 | **22.5** | 339 | **13.0** | 459-2 | **40.0** |
| 102 | **70.5** | 232 | **17.5** | 340 | **33.0** | 460 | **2.8** |
| 103 | **70.5** | 233 | **42.0** | 341 | **10.0** | 461 | **43.0** |
| 106 | **153** | 234 | **11.0** | 342 | **6.2** | 462 | **11.0** |
| 107 | **4.5** | 235 | **49.5** | 343 | **8.3** | 463 | **2.3** |
| 108 | **7.2** | 236 | **180** | 344 | **16.0** | 464-1 | **15.0** |
| 109 | **96.0** | 237 | **34.0** | 345 | **18.0** | 464-2 | **190** |
| 110 | **25.0** | 238 | **10.0** | 346 | **3.8** | 465 | **3.1** |
| 111 | **38.0** | 238-1 | **80.0** | 347 | **9.0** | 466 | **2.9** |
| 112 | **24.0** | 238-2 | **18.0** | 348 | **7.3** | 467 | **5.0** |
| 113 | **201** | 239 | **199** | 349 | **4.0** | 467-1 | **1.5** |
| 114 | **19.0** | 240 | **53.5** | 350 | **3.3** | 467-2 | **54.0** |
| 115 | **152** | 241 | **31.5** | 351 | **12.5** | 468 | **4.0** |
| 116 | **52.0** | 242 | **9.5** | 352 | **2.9** | 468-1 | **1.2** |
| 117 | **21.0** | 243 | **31.5** | 353 | **2.6** | 469 | **11.0** |
| 119 | **41.0** | 244 | **17.0** | 354 | **5.3** | 470 | **0.9** |
| 120 | **8.5** | 244-1 | **9.5** | 355 | **4.5** | 471 | **4.8** |
| 121 | **23.5** | 244-2 | **34.0** | 356 | **4.4** | 472 | **1.5** |
| 122 | **217** | 245-1 | **8.5** | 357-1 | **15.5** | 473 | **1.1** |
| 123 | **174** | 245-2 | **58.0** | 357-2 | **22.0** | 474 | **0.9** |
| 124 | **80.5** | 246 | **73.0** | 358 | **13.5** | 475 | **1.0** |
| 125 | **26.5** | 247 | **9.5** | 359 | **6.0** | 476 | **1.9** |
| 126 | **19.0** | 248 | **30.5** | 360-1 | **83.0** | 477-1 | **1.1** |
| 127 | **73.5** | 249 | **24.0** | 360-2 | **2693** | 477-2 | **27.0** |
| 128 | **37.0** | 250 | **17.0** | 361 | **5.2** | 478 | **1.1** |
| 129 | **11.0** | 251 | **106** | 362 | **4.6** | 479 | **2.1** |
| 130 | **62.0** | 252 | **109** | 363 | **6.2** | 480 | **1.5** |
| 131 | **15.5** | 253 | **39.0** | 364 | **5.1** | 481 | **1.9** |
| 132 | **6.0** | 254 | **48.0** | 365 | **8.5** | 482 | **2.4** |
| 133 | **30.0** | 255 | **86.0** | 366-1 | **1148** | 483 | **19.0** |
| 134 | **17.5** | 256 | **189** | 366-2 | **38.5** | 484 | **4.2** |
| 135 | **102** | 257-1 | **131** | 367-1 | **3.9** | 485 | **42.0** |
| 136 | **129** | 257-2 | **13.8** | 367-2 | **4.0** | 486 | **4.2** |
| 137 | **6.5** | 258 | **50.0** | 368-1 | **23.0** | 487 | **2.2** |
| 138 | **6.0** | 259 | **59.0** | 368-2 | **4.3** | 488 | **3.4** |
| 139 | **42.0** | 260 | **3.0** | 369 | **3.8** | 489 | **2.0** |
| 140 | **161** | 261 | **8.5** | 370 | **3.8** | 490 | **2.9** |
| 141 | **5.5** | 262 | **257** | 371 | **4.8** | 491 | **130** |
| 142 | **10.0** | 263-1 | **74.0** | 372 | **32.0** | 492 | **270** |
| 143 | **2.7** | 263-2 | **7.0** | 373 | **6.4** | 493 | **5.0** |
| 144 | **51.0** | 264-1 | **116** | 374 | **26.0** | 494 | **4.1** |
| 145 | **18.0** | 264-2 | **6.0** | 375 | **3.9** | 495 | **2.4** |
| 146 | **46.0** | 265 | **12.8** | 376 | **3.1** | 496 | **3.8** |
| 147 | **140** | 266 | **13.0** | 377 | **2.0** | 497 | **0.9** |
| 148 | **9.0** | 267 | **12.0** | 378 | **2.8** | 498 | **2.8** |
| 149 | **8.5** | 268 | **32.5** | 379 | **1.8** | 499 | **2.1** |
| 150 | **24.5** | 269 | **33.5** | 380 | **2.2** | 500 | **2.2** |
| 151 | **15.5** | 270 | **24.0** | 382 | **9.2** | 501 | **1.6** |
| 152 | **74.5** | 271 | **27.0** | 383 | **17.0** | 502 | **0.9** |
| 153 | **31.0** | 272 | **23.5** | 384 | **2.7** | 503 | **1.0** |
| 154 | **166** | 273 | **44.0** | 385 | **4.7** | 504-1 | **17.0** |
| 155 | **23.0** | 274 | **19.5** | 386 | **20.0** | 504-2 | **1.2** |
| 156 | **30.0** | 275 | **3.2** | 387 | **2.5** | 505-1 | **22.0** |
| 158 | **165** | 276-1 | **15.0** | 388 | **2.1** | 505-2 | **1.0** |
| 159 | **69.0** | 276-2 | **7.0** | 389 | **1.9** | 506 | **0.9** |
| 160 | **62.0** | 277 | **3.3** | 390 | **2.6** | 507 | **1.0** |
| 161 | **33.5** | 278 | **11.0** | 391 | **2.2** | 508 | **1.1** |
| 162 | **24.0** | 279 | **6.0** | 392 | **2.0** | 509 | **1.1** |
| 163 | **38.0** | 280 | **19.5** | 393 | **2.2** | 510 | **0.9** |
| 163-1 | **47.0** | 280-1 | **15.0** | 394 | **6.1** | | |
| | | 280-2 | **160** | 394-1 | **188** | | |

### Experimental Example 2: Evaluation of Inhibitory Activity of Compounds Against Proliferation of Human Tumor Cells

This experiment assessed the inhibitory effect of the compounds against proliferation using in vitro cultured human leukemia cells MV-4-11 (carrying MLL-AF4), MOLM-13 (carrying MLL-AF9), and KG-1a (wild-type MLL, serving as a control cell line to exclude compounds exhibiting general cytotoxicity).

### Reagents and Instruments:

Human acute lymphoblastic leukemia cells MV-4-11, purchased from American Type Culture Collection, Catalog Number: CRL-9591;
Human acute myeloid leukemia cells MOLM-13, purchased from COBIOER BIOSCIENCES CO., LTD, Catalog Number: CBP60678;
Human acute myeloid leukemia cells KG-1a, purchased from American Type Culture Collection, Catalog Number: CCL-246.1;
Inverted Microscope, purchased from Olympus Tokyo;
Countess^{™} Automated Cell Counter, purchased from Invitrogen, Model: C10281; and
Multilabel Reader, purchased from Bio-Tek, Model: Synergy HT.

### Experimental method:

MV-4-11 cells were cultured in an IMDM medium containing 10% fetal bovine serum, KG-a cells were cultured in an IMDM medium containing 20% fetal bovine serum, or MOLM-13 cells were cultured in an RPMI-1640 medium containing 20% fetal bovine serum, each supplemented with 100 U·mL⁻¹ penicillin and 100 µg·mL⁻¹ streptomycin. These cells were cultured at 37°C with 5% CO₂. The suspensions of individual cell lines were prepared in their respective media and seeded into 96-well plates at a density of 4,000 cells/well for MV-4-11 and MOLM-13 cells, or 3,000 cells/well for KG-1a cells. On the day of the experiment, a compound stock solution was diluted in a 3-fold or 4-fold gradient using DMSO. Each gradient-diluted stock solution was then further diluted 250-fold with a complete medium to prepare a series of working solutions at different concentrations. Subsequently, 100 µL of working solution (containing a 2-fold concentration series of the test compound) was added to each well, with duplicate wells set up for each concentration.

Seven days after compound addition (on Day 7), an Alamar Blue assay reagent (MCE, HY-111391 / CS-0040317) was added to measure the fluorescence intensity in each well. Wells containing the cells in the growth medium supplemented with 0.1% DMSO (Day 7) served as high-read control wells. The readouts from the wells containing the cells in the growth medium supplemented with 0.1% DMSO on the day of compound addition (Day 0) served as low-reading control well. GraphPad Prism software was used to calculate the concentration required to inhibit 50% of cell proliferation (GI50), which serves as a cell proliferation inhibition activity index. The GI50 (half-maximal inhibitory concentration) of the compound was determined using the following nonlinear fitting formula: 4-Parameter Logistic Model or Sigmoidal Dose-Response Model: $Y = Bottom + \frac{Top - Bottom}{1 + {10}^{\left({LogGI}_{50}-X\right) \times Hill Slope}} ,$ in which X indicates the logarithm of compound concentration, Y indicates an inhibition rate (% inhibition), Top indicates a readout of high-reading control well, and Bottom indicates a readout of low-reading control well, with $Inhibition rate \left(% inhibition\right) = \frac{Readout of Compound well - Readout of low - reading control well}{Read of high - reading control well - Readout of low - reading control well} \times 100 % .$

Experimental results are shown in Table 2.

**Table 2: Inhibitory Activity of Compounds Against Proliferation of Human Tumor Cells**

| Example | **MV-4-11 IC₅₀ (nM)** | **MOLM-13 IC₅₀ (nM)** | **KG-1a IC₅₀ (nM)** | Example | **MV-4-11 IC₅₀ (nM)** | **MOLM-13 IC₅₀ (nM)** | **KG-1a IC₅₀ (nM)** |
|---|---|---|---|---|---|---|---|
| **15** | 23 | ND | 17000 | **293** | 2 | 24 | 20000 |
| **16** | 14 | ND | 20000 | **294** | 4.9 | 46 | ND |
| **17** | 17 | ND | 20000 | **295** | 19 | 47 | ND |
| **18-1** | 18 | 61 | 9700 | **296-1** | 0.75 | 3 | ND |
| **18-2** | 9.4 | 43 | 6500 | **297-1** | 12 | 36 | ND |
| **19** | 49 | ND | 20000 | **305** | 3.4 | 12 | 20000 |
| **20** | 54 | ND | 20000 | **306** | 2.8 | 7.7 | 20000 |
| **22-1** | 79 | ND | 20000 | **307-2** | 0.69 | 3.7 | ND |
| **22-2** | 22 | ND | 20000 | **310-2** | 0.97 | 6.2 | ND |
| **25-1** | 4.7 | ND | 12600 | **311-2** | 4.7 | 26 | ND |
| **42** | 3.3 | 26 | 3300 | **312-2** | 0.91 | 5.3 | ND |
| **282** | 5.4 | 25 | ND | **314** | 1.1 | 7.3 | ND |
| **55-1-1** | 18.5 | 44 | ND | **321-1** | 6.4 | 46 | ND |
| **72** | 57 | ND | 20000 | **321-2** | 2.6 | 17 | ND |
| **73** | 61 | ND | 20000 | **323-2** | 2.4 | 15 | ND |
| **75** | 25 | 78 | 20000 | **324-2** | 1.3 | 9.2 | ND |
| **83** | 5.5 | 33 | 7400 | **342** | ND | 5.3 | ND |
| **90** | 20 | 99 | 20000 | **343** | ND | 5.9 | ND |
| **91** | 68 | ND | 20000 | **346** | ND | 8.3 | ND |
| **100** | 17 | 51 | ND | **349** | ND | 16.5 | ND |
| **120** | 8.5 | 29.5 | ND | **350** | ND | 4.6 | 12000 |
| **121** | 42 | 128 | ND | **351** | ND | 44 | >20000 |
| **128** | 56 | 30 | ND | **352** | ND | 6 | ND |
| **129** | 16 | 12 | ND | **353** | 4.1 | 5.4 | >20000 |
| **131** | 50 | 40 | ND | **354** | ND | 5 | ND |
| **132** | 17 | 16 | ND | **355** | ND | 6.5 | ND |
| **133** | 49 | 31 | ND | **356** | ND | 4.1 | ND |
| **134** | 26 | 21 | 20000 | **358** | ND | 91 | >20000 |
| **137** | 4.7 | 10 | ND | **359** | ND | 66 | ND |
| **138** | 3 | 7.6 | 20000 | **361** | ND | 3.3 | ND |
| **139** | 44 | 96 | ND | **362** | ND | 2.8 | ND |
| **141** | 1.1 | 5.4 | ND | **363** | ND | 20 | ND |
| **142** | 2.2 | 10 | 3600 | **364** | ND | 3.9 | ND |
| **145** | 13 | 29.5 | 19000 | **365** | ND | 14 | ND |
| **146** | 89 | ND | ND | **368-2** | 5 | 8.6 | 13000 |
| **148** | 0.95 | 3.6 | ND | **369** | ND | 3 | ND |
| **149** | 2.1 | 8.3 | 5300 | **370** | ND | 4.2 | ND |
| **150** | 21 | 36 | ND | **371** | ND | 5.3 | >20000 |
| **151** | 40 | 61 | ND | **373** | ND | 6 | ND |
| **162** | 27 | 67 | 7000 | **377** | ND | 1.7 | ND |
| **164** | 2.2 | 6.1 | ND | **378** | ND | 5.8 | ND |
| **168** | 32 | 104 | 20000 | **379** | 0.4 | 2.5 | 11500 |
| **181-2** | 7.9 | 39 | ND | **380** | ND | 3.8 | ND |
| **182-2** | ND | 45 | 7500 | **382** | ND | 30 | ND |
| **183-2** | 7.9 | 39 | 11000 | **384** | ND | 4.1 | ND |
| **184-2** | 6.3 | 34 | ND | **385** | ND | 8.1 | ND |
| **185** | 26 | 70 | 20000 | **387** | ND | 8.9 | ND |
| **186-2** | 4.3 | 28 | 20000 | **389** | ND | 5.3 | 12000 |
| **187** | 15 | 36 | ND | **391** | 1.9 | 6 | ND |
| **188** | 9.1 | 27 | ND | **393** | ND | 16 | ND |
| **189** | 29 | 44 | ND | **395** | ND | 6.1 | ND |
| **190** | 25 | 40 | ND | **396** | ND | 22 | >20000 |
| **195** | 13 | 35 | ND | **398** | 1.1 | 4.3 | >20000 |
| **196-1** | 36 | 120 | ND | **399-2** | ND | 25 | ND |
| **196-2** | 9.6 | 28 | ND | **400-2** | ND | 12 | ND |
| **197** | 4 | 10.2 | 20000 | **401-2** | ND | 29 | ND |
| **200-1** | 3.2 | 10.2 | 20000 | **402-2** | ND | 65 | 11000 |
| **201-1** | 2.4 | 5.1 | 20000 | **403-1** | ND | 7.7 | 15000 |
| **202** | 1.8 | 11 | 20000 | **403-2** | ND | 38 | ND |
| **203** | 2.1 | 5.6 | 20000 | **404** | ND | 8.1 | ND |
| **204** | 2.8 | 11.2 | 18000 | **405** | ND | 7.2 | ND |
| **205** | 7.4 | 19 | 20000 | **406** | ND | 37 | ND |
| **206** | 3.5 | 15 | 20000 | **408** | ND | 4.2 | ND |
| **208** | 6.6 | 9.6 | 20000 | **409** | ND | 31 | ND |
| **209-1** | 12 | 21 | ND | **410** | ND | 37 | ND |
| **209-2** | 2 | 5 | ND | **411** | ND | 12 | ND |
| **210** | 7.1 | 41 | ND | **412** | ND | 39 | ND |
| **211** | 2.2 | 5.7 | ND | **413** | ND | 45 | ND |
| **212** | 16 | 31 | ND | **416-2** | ND | 69 | ND |
| **213** | 13 | 29 | ND | **419** | ND | 85 | ND |
| **214** | 7.5 | 12 | ND | **420** | 18 | 43 | ND |
| **215** | 6.7 | 13 | ND | **423** | 1.2 | 2.7 | ND |
| **216** | 22 | 32 | ND | **425** | ND | 9.9 | ND |
| **217** | 6.9 | 14 | ND | **426** | ND | 30 | ND |
| **218** | 6 | 11 | ND | **428** | ND | 52 | ND |
| **219** | 12 | 14 | ND | **429** | ND | 25 | ND |
| **221** | 12 | 27 | ND | **430** | ND | 46 | ND |
| **222** | 4.4 | 9 | ND | **431** | 4.7 | 15 | ND |
| **223** | 8.1 | 19 | ND | **432** | ND | 38 | ND |
| **224** | 14 | 36 | ND | **433** | ND | 18 | ND |
| **225** | 25 | 60 | ND | **434** | ND | 32 | ND |
| **226** | 2.1 | 5.3 | ND | **435** | ND | 26 | ND |
| **230** | 1 | 2.9 | ND | **436** | ND | 42 | ND |
| **231** | 6.1 | 16 | ND | **440** | ND | 56 | ND |
| **232** | 17 | 34 | ND | **441-1** | ND | 75 | ND |
| **234** | 5.8 | 14 | ND | **445** | ND | 68 | ND |
| **238-2** | 11 | 27 | ND | **448-2** | ND | 13 | ND |
| **242** | 5.9 | 28 | ND | **449-1** | ND | 6.1 | ND |
| **243** | 42 | 200 | ND | **450** | ND | 21 | ND |
| **244** | 13 | 64 | ND | **451** | ND | 25 | ND |
| **244-1** | 16 | 39 | 20000 | **452** | ND | 39 | ND |
| **244-2** | 54 | 130 | ND | **453** | ND | 22 | ND |
| **245-1** | 6.6 | 40 | 20000 | **455** | ND | 69 | ND |
| **247** | 9.2 | 22 | ND | **457-2** | ND | 5 | ND |
| **248** | 34 | 63 | ND | **458** | ND | 31 | ND |
| **249** | 50 | 44 | ND | **459-1** | ND | 4.7 | ND |
| **250** | 26 | 45 | ND | **460** | ND | 12 | ND |
| **257-2** | 17 | 31 | 20000 | **463** | ND | 4.3 | ND |
| **258** | 36 | 49 | ND | **467-1** | ND | 23 | ND |
| **259** | 55 | 110 | ND | **468-1** | ND | 19 | ND |
| **260** | 7.1 | 10 | ND | **470** | ND | 3.8 | ND |
| **261** | 9.6 | 23 | 20000 | **472** | ND | 24 | ND |
| **263-2** | 5.3 | 13 | 20000 | **473** | ND | 16 | ND |
| **264-2** | 4.7 | 12 | 20000 | **474** | ND | 11 | ND |
| **265** | 8.6 | 25 | 20000 | **475** | ND | 25 | ND |
| **266** | 13 | 33 | ND | **477-1** | ND | 10 | ND |
| **267** | 20 | 45 | ND | **480** | ND | 74 | ND |
| **268** | 36 | 96 | ND | **481** | ND | 5.5 | ND |
| **269** | 37 | 83 | ND | **482** | ND | 36 | ND |
| **271** | 12 | 33 | ND | **488** | ND | 19 | ND |
| **272** | 19 | 38 | ND | **489** | ND | 5.9 | ND |
| **275** | 2.6 | 9.4 | 20000 | **495** | ND | 27 | ND |
| **277** | 1.6 | 7.3 | 20000 | **497** | ND | 41 | ND |
| **278** | 8.3 | 24 | ND | **498** | ND | 23 | ND |
| **279** | 3.3 | 7.4 | ND | **499** | ND | 29 | ND |
| **280-1** | 6.9 | 27 | ND | **502** | ND | 9.8 | ND |
| **284-2** | 1.5 | 6.8 | ND | **503** | ND | 8.5 | ND |
| **285** | 5.4 | 23 | ND | **504-2** | ND | 13 | ND |
| **286** | 4.6 | 19 | 20000 | **505-2** | ND | 9.9 | ND |
| **287** | 9 | 48 | 20000 | **506** | ND | 13 | ND |
| **288** | 15 | 55 | ND | **507** | ND | 15 | ND |
| **289** | 11 | 45 | 20000 | **508** | ND | 11 | ND |
| **290** | 5.2 | 24 | ND | **509** | ND | 7.2 | ND |
| **291** | 1.6 | 7 | ND | **510** | ND | 9.9 | ND |
| **292** | 2.4 | 13 | 20000 | | | | |

| | | | | | | | |
|---|---|---|---|---|---|---|---|
| ND = Not detected A compound exhibiting >100-fold selectivity toward control organelles compared to leukemia cells indicates lower cytotoxicity for that compound. | | | | | | | |

### Experimental Example 3: Evaluation of Inhibitory Activity of Compounds Against MEISI Gene Expression in Human Tumor Cells

The effect of test samples on MEIS1 gene expression in vitro cultured human leukemia cells MV-4-11 (carrying MLL-AF4), MOLM-13 (carrying MLL-AF9), and OCI-AML3 (carrying NPM1c) was studied.

### Reagents and Instruments:

Human acute lymphoblastic leukemia cells MV-4-11, purchased from American Type Culture Collection, Catalog Number: CRL-9591;
Human acute myeloid leukemia cells MOLM-13, purchased from COBIOER BIOSCIENCES CO., LTD, Catalog Number: CBP60678;
Human acute myeloid leukemia cells OCI-AML3, purchased from COBIOER BIOSCIENCES CO., LTD, Catalog Number: CBP60817;
MEIS1 Primer/Probe, purchased from IDT, Catalog Number: 40831613; and
GAPDH Primer/Probe, purchased from IDT, Catalog Number: 22214836.

### Experimental method:

MV-4-11 cells were cultured in an IMDM medium containing 10% fetal bovine serum, or MOLM-13 and OCI-AML3 cells were cultured in an RPMI-1640 medium containing 20% fetal bovine serum, each supplemented with 100 U·mL⁻¹ penicillin and 100 µg·mL⁻¹ streptomycin. These cells were cultured at 37°C with 5% CO₂. The suspensions of individual cell lines were prepared in their respective media and seeded into 48-well plates at a density of 100,000 cells/well. On the day of the experiment, a compound stock solution was diluted in a 6-fold gradient using DMSO. Each gradient-diluted stock solution was then further diluted 250-fold with a complete medium to prepare a series of working solutions at different concentrations. Next, 250 µL of working solution (containing the complete medium with a 2-fold serial concentration of the test compound or a positive control compound, or containing a control complete medium) was added to each well. After 48 hours of incubation, cells from the 48-well plate were collected into 1 mL centrifuge tubes, centrifuged at 1000 rpm for 5 minutes, and the complete medium was removed. Cells were washed twice with 1 mL PBS and centrifuged to remove PBS. Total RNA was extracted using the RNeasy Mini Kit (250) (QIAGEN, 74106). The threshold cycle (Ct) value for gene expression was determined using the HiScript II U+ One Step qRT-PCR Probe Kit (Vazyme) (Vazyme, Q223-01), with 3 replicates per sample. The prepared reaction mixture was placed in an ABI 7500 real-time PCR instrument, and the reaction proceeded following the procedures below.

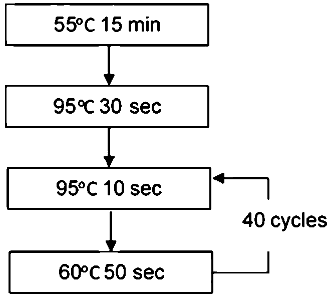

Next, for the relative MEIS1 gene expression, the concentration (EC50) that inhibits 50% of relative MEIS1 gene expression was calculated using GraphPad Prism 8 software. The EC₅₀ (half-maximal inhibitory concentration) of the compound was determined using the following nonlinear fitting formula: 4-Parameter Logistic Model or Sigmoidal Dose-Response Model: $Y = Bottom + \frac{Top - Bottom}{1 + {10}^{\left({LogEC}_{50}-X\right) \times Hill Slope}} ,$ in which X indicates the logarithm of compound concentration, Y indicates a relative MEIS 1 gene expression, Top and Bottom indicate highest and lowest readouts expressed as relative MEIS 1 gene expression; and relative MEIS 1 gene expression = 2^{-ΔΔCt}, with ΔΔCt = threshold cycle Ct of MEIS 1 gene - threshold cycle Ct of GAPDH.

Experimental results are shown in Table 3.

**Table 3: Inhibitory Activity of Compounds Against MEIS 1 Gene Expression in Human Tumor Cells**

| Example | **MV-4-11 MEISI EC₅₀ (nM)** | **MOLM-13 MEIS1 EC₅₀ (nM)** | **OCI-AML3 MEIS1 EC₅₀ (nM)** | Example | **MV-4-11 MEISI EC₅₀ (nM)** | **MOLM-13 MEISI EC₅₀ (nM)** | **OCI-AML3 MEIS1 EC₅₀ (nM)** |
|---|---|---|---|---|---|---|---|
| **30** | 20 | ND | ND | **305** | 7.6 | ND | ND |
| **75** | 82 | ND | ND | **306** | 4.6 | ND | ND |
| **83** | 24 | ND | ND | **310-2** | 5.0 | 6.5 | 12 |
| **134** | 55 | 53 | ND | **311-2** | 19 | 11 | 23 |
| **137** | 16 | 9.0 | ND | **312-2** | 5.7 | 2.5 | 3.9 |
| **138** | 15 | 8.0 | ND | **314** | 13 | 3.4 | 8.9 |
| **142** | 6 | 2.3 | ND | **321-1** | 53 | ND | ND |
| **185** | 55 | ND | ND | **321-2** | ND | 10 | 32 |
| **186-2** | 3.8 | ND | ND | **323-2** | 8.6 | 8.4 | 17 |
| **188** | 42 | ND | ND | **324-2** | 7.3 | 1.3 | 9.5 |
| **189** | 89 | ND | ND | **325** | 7.6 | 4.2 | ND |
| **197** | 14 | ND | ND | **326** | 4.6 | 5.5 | ND |
| **200-1** | 12 | 1.0 | 13 | **329** | 63 | 17.9 | ND |
| **201-1** | 3.4 | 1.0 | 2.4 | **331** | 63 | 20.3 | ND |
| **202** | 3.5 | ND | ND | **333** | 15 | 6.2 | ND |
| **204** | 15 | 4.3 | 13 | **336** | 19 | 9.3 | ND |
| **205** | 18 | ND | ND | **337** | 13 | 5.5 | ND |
| **208** | 23 | ND | ND | **338** | 27 | 1.8 | ND |
| **257-2** | 19 | 15 | 33 | **341** | 26 | 6.0 | ND |
| **260** | 7.6 | ND | ND | **346** | 10 | 5.0 | ND |
| **261** | 8.5 | ND | ND | **350** | 6.8 | 4.0 | ND |
| **264-2** | 18 | ND | ND | **352** | 7.5 | 2.8 | ND |
| **265** | 54 | 38 | 70 | **353** | 7.4 | 2.4 | 21 |
| **275** | 8.3 | 7.7 | 16 | **368-2** | 7.8 | 9.1 | 17 |
| **277** | 6.5 | 3.3 | 12 | **371** | 3.0 | 1.1 | ND |
| **280-1** | 61 | 23 | 34 | **379** | 2.4 | 2.0 | 7.5 |
| **284-2** | ND | 5.9 | 5.8 | **391** | 2.9 | 2.1 | 11 |
| **292** | 15 | ND | ND | **398** | 1.9 | 2.6 | 13 |
| **293** | 23 | 9.1 | 31 | **423** | 1.3 | 2.3 | 6.1 |
| **297-1** | 32 | 12 | 34 | **431** | 5.5 | 4.6 | 33 |

| | | | | | | | |
|---|---|---|---|---|---|---|---|
| ND = Not detected | | | | | | | |

Described above provides only preferred embodiments of the present invention, which are not intended to limit the present invention. Any modification, equivalent substitution or the like made under the spirit and principle of the present invention shall be construed as falling within the protection scope of the present invention.

The foregoing embodiments and methods described in the present invention may vary depending on the ability, experience and preferences of those skilled in the art.

The method steps are listed only in a specific order in the present invention, without imposing any limits on the order of the method steps.

## Claims

1. A compound or a pharmaceutically acceptable salt, stereoisomer, ester, prodrug, solvate and deuterated compound thereof, wherein said compound has a structure of: 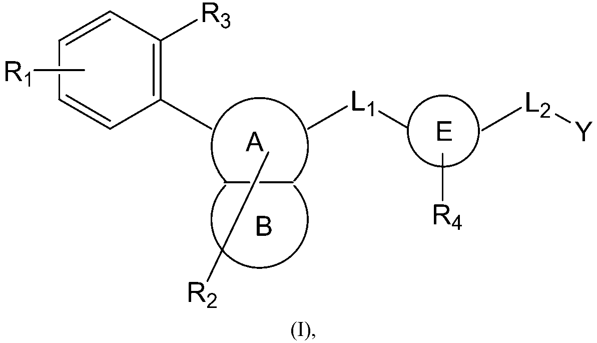 wherein
Ring A is 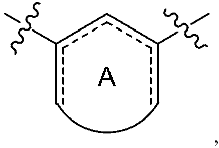 ------ represents a single or double bond, and said Ring A is a five- to seven-membered aromatic or heterocyclic ring;
Ring B is a five- to seven-membered nitrogenous heterocyclic ring;
Ring E is a three- to eight-membered saturated or partially saturated aliphatic ring, an aromatic ring, or a four- to twelve-membered saturated or partially saturated heterocyclic ring;
L₁ and L₂ are independently selected from the group consisting of: a single bond, C₁-C₁₀ alkylene, and -(C₀-C₁₀ alkylene)-Q-(C₀-C₁₀ alkylene)- , with Q selected from the group consisting of: -O-, -S-, -C(O)-, -C(O)O-, -OC(O)-, - C(O)N(R_{La})-, -N(R_{La})C(O)-, -N(R_{La})C(O)O-, -N(R_{La})C(O)N(R_{La})-, -N(R_{La})-, -S(O)₂-, -S(O)₂N(R_{La})-, -N(R_{La})S(O)₂-, -S(O)-, -S(O)N(R_{La})-, -N(R_{La})S(O)-, -C(=NR_{La})-, and -C(=NR_{La})-NR_{La}-; H atom(s) in said alkylene may be optionally substituted with a group selected from the group consisting of: H, C₁-C₁₀ alkyl, C₂-C₁₀ alkenyl, C₂-C₁₀ alkynyl, C₃-C₁₀ cycloalkyl, C₆-C₁₀ aryl, 4-10-membered heterocyclyl, halogen, cyano, nitro, azido, halogenated C₁-C₁₀ alkyl, hydroxyl, C₁-C₁₀ alkoxy, halogenated C₁-C₁₀ alkoxy, amino, and C₁-C₁₀ alkylamino; H in said cycloalkyl, aryl or heterocyclyl may be optionally substituted with a group selected from the group consisting of: H, halogen, C₁-C₁₀ alkyl, and halogenated C₁-C₁₀ alkyl; R_{La} is selected from the group consisting of: H, C₁-C₁₀ alkyl, C₂-C₁₀ alkenyl, C₂-C₁₀ alkynyl, C₃-C₁₀ cycloalkyl, C₃-C₁₀ cycloalkylalkyl, C₆-C₁₀ aryl, C₇-C₁₂ aralkyl, 4-10-membered heterocyclyl, and 4-10-membered heterocyclylalkyl;
R₁ is one or more independent substituents on the benzene ring, and each R₁ is independently selected from the group consisting of: H, halogen, C₁-C₁₀ alkyl, C₂-C₁₀ alkenyl, C₂-C₁₀ alkynyl, cyano, nitro, azido, halogenated C₁-C₁₀ alkyl, halogenated C₁-C₁₀ alkoxy, C₁-C₁₀ cyanoalkyl, -OR₁₀₁, -C(O)R₁₀₁, -C(O)OR₁₀₁, - NR₁₀₂C(O)OR₁₀₁, -OC(O)R₁₀₁, -NR₁₀₂SO₂R₁₀₁, -SO₂NR₁₀₁R₁₀₂, -NR₁₀₂C(O)R₁₀₁, -C(O)NR₁₀₁R₁₀₂, -NR₁₀₁R₁₀₂, -SR₁₀₁, -S(O)R₁₀₁, -S(O)₂R₁₀₁, -SO₃H, -NR₁₀₂(CR₁₀₃R₁₀₄)ₜOR₁₀₁, -(C₀-C₆ alkylene)-(C₆-C₁₀ aryl), -SO₂-(C₀-C₆ alkylene)-(C₆-C₁₀ aryl), -S-(C₀-C₆ alkylene)-(C₆-C₁₀ aryl), -O-(C₀-C₆ alkylene)-(C₆-C₁₀ aryl), -(C₀-C₆ alkylene)-(4-10-membered heterocyclyl), -SO₂-(C₀-C₆ alkylene)-(4-10-membered heterocyclyl), -S-(C₀-C₆ alkylene)-(4-10-membered heterocyclyl), -O-(C₀-C₆ alkylene)-(4-10-membered heterocyclyl), -(C₀-C₆ alkylene)-(C₃-C₁₀ cycloalkyl), -SO₂-(C₀-C₆ alkylene)-(C₃-C₁₀ cycloalkyl), -S-(C₀-C₆ alkylene)-(C₃-C₁₀ cycloalkyl), and -O-(C₀-C₆ alkylene)-(C₃-C₁₀ cycloalkyl), with t being an integer ranging from 0 to 5; wherein said C₁-C₁₀ alkyl, C₂-C₁₀ alkenyl, C₂-C₁₀ alkynyl, C₃-C₁₀ cycloalkyl, C₆-C₁₀ aryl, or 4-10-membered heterocyclyl may be optionally substituted with one or more groups selected from the group consisting of: halogen, cyano, nitro, azido, -OR', -C(O)R', -C(O)OR', -OC(O)R', -NR'C(O)OR", -NR'SO₂R", -SO₂NR'R", - NR'C(O)R", -C(O)NR'R", -NR'R", -SR', -SOR', -SO₂R', -SO₃H, C₁-C₁₀ alkyl, halogenated C₁-C₁₀ alkyl, halogenated C₁-C₁₀ alkoxy, C₁-C₁₀ cyanoalkyl, C₃-C₁₀ cycloalkyl, phenyl, and 4-10-membered heterocyclyl; R₁₀₁ to R₁₀₄ are each independently selected from the group consisting of: H, C₁-C₁₀ alkyl, C₂-C₁₀ alkenyl, C₂-C₁₀ alkynyl, C₃-C₁₀ cycloalkyl, C₃-C₁₀ cycloalkylalkyl, C₆-C₁₀ aryl, C₇-C₁₂ aralkyl, 4-10-membered heterocyclyl, and 4-10-membered heterocyclylalkyl; wherein said C₁-C₁₀ alkyl, C₂-C₁₀ alkenyl, C₂-C₁₀ alkynyl, C₃-C₁₀ cycloalkyl, C₆-C₁₀ aryl, or 4-10-membered heterocyclyl may be optionally substituted with one or more groups selected from the group consisting of: halogen, cyano, nitro, azido, -OR', -C(O)R', -C(O)OR', -OC(O)R', -NR'C(O)OR", -NR'SO₂R", - SO₂NR'R", -NR'C(O)R", -C(O)NR'R", -NR'R", -SR', -SOR', -SO₂R', -SO₃H, C₁-C₁₀ alkyl, halogenated C₁-C₁₀ alkyl, halogenated C₁-C₁₀ alkoxy, C₁-C₁₀ cyanoalkyl, C₃-C₁₀ cycloalkyl, phenyl, and 4-10-membered heterocyclyl;
R₂ is one or more independent substituents on the Ring 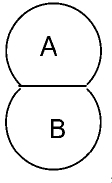 , and each R₂ is independently selected from the group consisting of: H, =O, C₁-C₁₀ alkyl, C₂-C₁₀ alkenyl, C₂-C₁₀ alkynyl, halogen, cyano, nitro, azido, halogenated C₁-C₁₀ alkyl, halogenated C₁-C₁₀ alkoxy, C₁-C₁₀ cyanoalkyl, -OR₂₀₁, -C(O)R₂₀₁, -C(O)OR₂₀₁, -NR₂₀₂C(O)OR₂₀₁, - OC(O)R₂₀₁, -NR₂₀₂SO₂R₂₀₁, -SO₂NR₂₀₁R₂₀₂, -NR₂₀₂C(O)R₂₀₁, -C(O)NR₂₀₁R₂₀₂, -NR₂₀₁R₂₀₂, -SR₂₀₁, -S(O)R₂₀₁, - S(O)₂R₂₀₁, -SO₃H, -NR₂₀₂(CR₂₀₃R₂₀₄)ⱼOR₂₀₁, -(C₀-C₆ alkylene)-(C₆-C₁₀ aryl), -SO₂-(C₀-C₆ alkylene)-(C₆-C₁₀ aryl), - S-(C₀-C₆ alkylene)-(C₆-C₁₀ aryl), -O-(C₀-C₆ alkylene)-(C₆-C₁₀ aryl), -(C₀-C₆ alkylene)-(4-10-membered heterocyclyl), -SO₂-(C₀-C₆ alkylene)-(4-10-membered heterocyclyl), -S-(C₀-C₆ alkylene)-(4-10-membered heterocyclyl), -O-(C₀-C₆ alkylene)-(4-10-membered heterocyclyl), -(C₀-C₆ alkylene)-(C₃-C₁₀ cycloalkyl), -SO₂-(C₀-C₆ alkylene)-(C₃-C₁₀ cycloalkyl), -S-(C₀-C₆ alkylene)-(C₃-C₁₀ cycloalkyl), and -O-(C₀-C₆ alkylene)-(C₃-C₁₀ cycloalkyl), with j being an integer ranging from 0 to 5; wherein said C₁-C₁₀ alkyl, C₂-C₁₀ alkenyl, C₂-C₁₀ alkynyl, C₃-C₁₀ cycloalkyl, C₆-C₁₀ aryl, or 4-10-membered heterocyclyl may be optionally substituted with one or more groups selected from the group consisting of: halogen, cyano, nitro, azido, -OR', -C(O)R', -C(O)OR', -OC(O)R', - NR'C(O)OR", -NR'SO₂R", -SO₂NR'R", -NR'C(O)R", -C(O)NR'R", -NR'R", -SR', -SOR', -SO₂R', -SO₃H, C₁-C₁₀ alkyl, halogenated C₁-C₁₀ alkyl, halogenated C₁-C₁₀ alkoxy, C₁-C₁₀ cyanoalkyl, C₃-C₁₀ cycloalkyl, phenyl, and 4-10-membered heterocyclyl;
R₂₀₁ to R₂₀₄ are each independently selected from the group consisting of: H, C₁-C₁₀ alkyl, C₂-C₁₀ alkenyl, C₂-C₁₀ alkynyl, C₃-C₁₀ cycloalkyl, C₃-C₁₀ cycloalkylalkyl, C₆-C₁₀ aryl, C₇-C₁₂ aralkyl, 4-10-membered heterocyclyl, and 4-10-membered heterocyclylalkyl; wherein said C₁-C₁₀ alkyl, C₂-C₁₀ alkenyl, C₂-C₁₀ alkynyl, C₃-C₁₀ cycloalkyl, C₆-C₁₀ aryl, or 4-10-membered heterocyclyl may be optionally substituted with one or more groups selected from the group consisting of: halogen, cyano, nitro, azido, -OR', -C(O)R', -C(O)OR', -OC(O)R', -NR'C(O)OR", -NR'SO₂R", - SO₂NR'R", -NR'C(O)R", -C(O)NR'R", -NR'R", -SR', -SOR', -SO₂R', -SO₃H, C₁-C₁₀ alkyl, halogenated C₁-C₁₀ alkyl, halogenated C₁-C₁₀ alkoxy, C₁-C₁₀ cyanoalkyl, C₃-C₁₀ cycloalkyl, phenyl, and 4-10-membered heterocyclyl;
R₃ is selected from the group consisting of: H, halogen, C₁-C₁₀ alkyl, C₂-C₁₀ alkenyl, C₂-C₁₀ alkynyl, cyano, nitro, azido, halogenated C₁-C₁₀ alkyl, halogenated C₁-C₁₀ alkoxy, C₁-C₁₀ cyanoalkyl, -OR₃₀₁, -C(O)R₃₀₁, -C(O)OR₃₀₁, - NR₃₀₂C(O)OR₃₀₁, -OC(O)R₃₀₁, -NR₃₀₂SO₂R₃₀₁, -SO₂NR₃₀₁R₃₀₂, -NR₃₀₂C(O)R₃₀₁, -C(O)NR₃₀₁R₃₀₂, -NR₃₀₁R₃₀₂, - SR₃₀₁, -S(O)R₃₀₁, -S(O)₂R₃₀₁, -SO₃H, -NR₃₀₂(CR₃₀₃R₃₀₄)ᵢOR₃₀₁, -(C₀-C₆ alkylene)-(C₆-C₁₀ aryl), -SO₂-(C₀-C₆ alkylene)-(C₆-C₁₀ aryl), -S-(C₀-C₆ alkylene)-(C₆-C₁₀ aryl), -O-(C₀-C₆ alkylene)-(C₆-C₁₀ aryl), -(C₀-C₆ alkylene)-(4-10-membered heterocyclyl), -SO₂-(C₀-C₆ alkylene)-(4-10-membered heterocyclyl), -S-(C₀-C₆ alkylene)-(4-10-membered heterocyclyl), -O-(C₀-C₆ alkylene)-(4-10-membered heterocyclyl), -(C₀-C₆ alkylene)-(C₃-C₁₀ cycloalkyl), -SO₂-(C₀-C₆ alkylene)-(C₃-C₁₀ cycloalkyl), -S-(C₀-C₆ alkylene)-(C₃-C₁₀ cycloalkyl), and -O-(C₀-C₆ alkylene)-(C₃-C₁₀ cycloalkyl), with i being an integer ranging from 0 to 5; wherein said C₁-C₁₀ alkyl, C₂-C₁₀ alkenyl, C₂-C₁₀ alkynyl, C₃-C₁₀ cycloalkyl, C₆-C₁₀ aryl, or 4-10-membered heterocyclyl may be optionally substituted with one or more groups selected from the group consisting of: halogen, cyano, nitro, azido, -OR', -C(O)R', -C(O)OR', -OC(O)R', - NR'C(O)OR", -NR'SO₂R", -SO₂NR'R", -NR'C(O)R", -C(O)NR'R", -NR'R", -SR', -SOR', -SO₂R', -SO₃H, C₁-C₁₀ alkyl, halogenated C₁-C₁₀ alkyl, halogenated C₁-C₁₀ alkoxy, C₁-C₁₀ cyanoalkyl, C₃-C₁₀ cycloalkyl, phenyl, and 4-10-membered heterocyclyl;
R₃₀₁ to R₃₀₄ are each independently selected from the group consisting of: H, C₁-C₁₀ alkyl, C₂-C₁₀ alkenyl, C₂-C₁₀ alkynyl, C₃-C₁₀ cycloalkyl, C₃-C₁₀ cycloalkylalkyl, C₆-C₁₀ aryl, C₇-C₁₂ aralkyl, 4-10-membered heterocyclyl, and 4-10-membered heterocyclylalkyl; wherein said C₁-C₁₀ alkyl, C₂-C₁₀ alkenyl, C₂-C₁₀ alkynyl, C₃-C₁₀ cycloalkyl, C₆-C₁₀ aryl, or 4-10-membered heterocyclyl may be optionally substituted with one or more groups selected from the group consisting of: halogen, cyano, nitro, azido, -OR', -C(O)R', -C(O)OR', -OC(O)R', -NR'C(O)OR", -NR'SO₂R", - SO₂NR'R", -NR'C(O)R", -C(O)NR'R", -NR'R", -SR', -SOR', -SO₂R', -SO₃H, C₁-C₁₀ alkyl, halogenated C₁-C₁₀ alkyl, halogenated C₁-C₁₀ alkoxy, C₁-C₁₀ cyanoalkyl, C₃-C₁₀ cycloalkyl, phenyl, and 4-10-membered heterocyclyl;
or, R₃₀₁ and R₃₀₂, together with the atom attached thereto, form heterocyclyl, and said heterocyclyl may be optionally substituted with one or more groups selected from the group consisting of: H, =O, C₁-C₁₀ alkyl, C₂-C₁₀ alkenyl, C₂-C₁₀ alkynyl, halogen, cyano, nitro, azido, halogenated C₁-C₁₀ alkyl, halogenated C₁-C₁₀ alkoxy, C₁-C₁₀ cyanoalkyl, - OR₅, -C(O)R₅, -C(O)OR₅, -NR₆C(O)OR₅, -OC(O)R₅, -NR₆SO₂R₅, -SO₂NR₅R₆, -NR₆C(O)R₅, -C(O)NR₅R₆, -NR₅R₆, -SR₅, -S(O)R₅, -S(O)₂R₅, -SO₃H, -(C₀-C₆ alkylene)-(C₆-C₁₀ aryl), -SO₂-(C₀-C₆ alkylene)-(C₆-C₁₀ aryl), -S-(C₀-C₆ alkylene)-(C₆-C₁₀ aryl), -O-(C₀-C₆ alkylene)-(C₆-C₁₀ aryl), -(C₀-C₆ alkylene)-(4-10-membered heterocyclyl), -SO₂-(C₀-C₆ alkylene)-(4-10-membered heterocyclyl), -S-(C₀-C₆ alkylene)-(4-10-membered heterocyclyl), -O-(C₀-C₆ alkylene)-(4-10-membered heterocyclyl), -(C₀-C₆ alkylene)-(C₃-C₁₀ cycloalkyl), -SO₂-(C₀-C₆ alkylene)-(C₃-C₁₀ cycloalkyl), -S-(C₀-C₆ alkylene)-(C₃-C₁₀ cycloalkyl), and -O-(C₀-C₆ alkylene)-(C₃-C₁₀ cycloalkyl); wherein said C₁-C₁₀ alkyl, C₂-C₁₀ alkenyl, C₂-C₁₀ alkynyl, C₃-C₁₀ cycloalkyl, C₆-C₁₀ aryl, or 4-10-membered heterocyclyl may be optionally substituted with one or more groups selected from the group consisting of: halogen, cyano, nitro, azido, - OR', -C(O)R', -C(O)OR', -OC(O)R', -NR'C(O)OR", -NR'SO₂R", -SO₂NR'R", -NR'C(O)R", -C(O)NR'R", -NR'R", - SR', -SOR', -SO₂R', -SO₃H, C₁-C₁₀ alkyl, halogenated C₁-C₁₀ alkyl, halogenated C₁-C₁₀ alkoxy, C₁-C₁₀ cyanoalkyl, C₃-C₁₀ cycloalkyl, phenyl, and 4-10-membered heterocyclyl;
R₅ and R₆ are each independently selected from the group consisting of: H, C₁-C₁₀ alkyl, C₂-C₁₀ alkenyl, C₂-C₁₀ alkynyl, C₃-C₁₀ cycloalkyl, C₃-C₁₀ cycloalkylalkyl, C₆-C₁₀ aryl, C₇-C₁₂ aralkyl, 4-10-membered heterocyclyl, and 4-10-membered heterocyclylalkyl; wherein said C₁-C₁₀ alkyl, C₂-C₁₀ alkenyl, C₂-C₁₀ alkynyl, C₃-C₁₀ cycloalkyl, C₆-C₁₀ aryl, or 4-10-membered heterocyclyl may be optionally substituted with one or more groups selected from the group consisting of: halogen, cyano, nitro, azido, -OR', -C(O)R', -C(O)OR', -OC(O)R', -NR'C(O)OR", -NR'SO₂R", - SO₂NR'R", -NR'C(O)R", -C(O)NR'R", -NR'R", -SR', -SOR', -SO₂R', -SO₃H, C₁-C₁₀ alkyl, halogenated C₁-C₁₀ alkyl, halogenated C₁-C₁₀ alkoxy, C₁-C₁₀ cyanoalkyl, C₃-C₁₀ cycloalkyl, phenyl, and 4-10-membered heterocyclyl;
R₄ is one or more independent substituents on the Ring E, and each R₄ is independently selected from the group consisting of: H, halogen, =O, C₁-C₁₀ alkyl, C₂-C₁₀ alkenyl, C₂-C₁₀ alkynyl, cyano, nitro, azido, halogenated C₁-C₁₀ alkyl, halogenated C₁-C₁₀ alkoxy, C₁-C₁₀ cyanoalkyl, -OR₄₀₁, -C(O)R₄₀₁, -C(O)OR₄₀₁, -NR₁₀₂C(O)OR₄₀₁, -OC(O)R₄₀₁, -NR₄₀₂SO₂R₄₀₁, -SO₂NR₄₀₁R₄₀₂, -NR₄₀₂C(O)R₄₀₁, -C(O)NR₄₀₁R₄₀₂, -NR₄₀₁R₄₀₂, -SR₄₀₁, -S(O)R₄₀₁, -S(O)₂R₄₀₁, -SO₃H, -NR₄₀₂(CR₄₀₃R₄₀₄)ₘOR₁₀₄, -(C₀-C₆ alkylene)-(C₆-C₁₀ aryl), -SO₂-(C₀-C₆ alkylene)-(C₆-C₁₀ aryl), -S-(C₀-C₆ alkylene)-(C₆-C₁₀ aryl), -O-(C₀-C₆ alkylene)-(C₆-C₁₀ aryl), -(C₀-C₆ alkylene)-(4-10-membered heterocyclyl), -SO₂-(C₀-C₆ alkylene)-(4-10-membered heterocyclyl), -S-(C₀-C₆ alkylene)-(4-10-membered heterocyclyl), -O-(C₀-C₆ alkylene)-(4-10-membered heterocyclyl), -(C₀-C₆ alkylene)-(C₃-C₁₀ cycloalkyl), -SO₂-(C₀-C₆ alkylene)-(C₃-C₁₀ cycloalkyl), - S-(C₀-C₆ alkylene)-(C₃-C₁₀ cycloalkyl), and -O-(C₀-C₆ alkylene)-(C₃-C₁₀ cycloalkyl), with m being an integer ranging from 0 to 5; wherein said C₁-C₁₀ alkyl, C₂-C₁₀ alkenyl, C₂-C₁₀ alkynyl, C₃-C₁₀ cycloalkyl, C₆-C₁₀ aryl, or 4-10-membered heterocyclyl may be optionally substituted with one or more groups selected from the group consisting of: halogen, cyano, nitro, azido, -OR', -C(O)R', -C(O)OR', -OC(O)R', -NR'C(O)OR", -NR'SO₂R", -SO₂NR'R", - NR'C(O)R", -C(O)NR'R", -NR'R", -SR', -SOR', -SO₂R', -SO₃H, C₁-C₁₀ alkyl, halogenated C₁-C₁₀ alkyl, halogenated C₁-C₁₀ alkoxy, C₁-C₁₀ cyanoalkyl, C₃-C₁₀ cycloalkyl, phenyl, and 4-10-membered heterocyclyl;
R₄₀₁ to R₄₀₄ are each independently selected from the group consisting of: H, C₁-C₁₀ alkyl, C₂-C₁₀ alkenyl, C₂-C₁₀ alkynyl, C₃-C₁₀ cycloalkyl, C₃-C₁₀ cycloalkylalkyl, C₆-C₁₀ aryl, C₇-C₁₂ aralkyl, 4-10-membered heterocyclyl, and 4-10-membered heterocyclylalkyl; wherein said C₁-C₁₀ alkyl, C₂-C₁₀ alkenyl, C₂-C₁₀ alkynyl, C₃-C₁₀ cycloalkyl, C₆-C₁₀ aryl, or 4-10-membered heterocyclyl may be optionally substituted with one or more groups selected from the group consisting of: halogen, cyano, nitro, azido, -OR', -C(O)R', -C(O)OR', -OC(O)R', -NR'C(O)OR", -NR'SO₂R", - SO₂NR'R", -NR'C(O)R", -C(O)NR'R", -NR'R", -SR', -SOR', -SO₂R', -SO₃H, C₁-C₁₀ alkyl, halogenated C₁-C₁₀ alkyl, halogenated C₁-C₁₀ alkoxy, C₁-C₁₀ cyanoalkyl, C₃-C₁₀ cycloalkyl, phenyl, and 4-10-membered heterocyclyl; Y is selected from the group consisting of: C₁-C₁₀ alkyl, C₂-C₁₀ alkenyl, C₂-C₁₀ alkynyl, three- to eight-membered saturated or partially saturated alcyl, aryl, four- to twelve-membered heterocyclyl, aryl-fused three- to eight-membered alcyl, aryl-fused three- to eight-membered heterocyclyl, three- to eight-membered aliphatic ring-fused aryl, three- to eight-membered aliphatic ring-fused heterocyclyl, three- to eight-membered heterocyclic ring-fused three- to eight-membered aliphatic ring, three- to eight-membered heterocyclic ring-fused aryl, and three- to eight-membered heterocyclic ring-fused five- to ten-membered heterocyclyl, and Y may be optionally substituted with one or more R^{Y} groups, with R^{Y} selected from the group consisting of: H, =O, 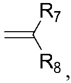 C₁-C₁₀ alkyl, C₂-C₁₀ alkenyl, C₂-C₁₀ alkynyl, halogen, cyano, nitro, azido, halogenated C₁-C₁₀ alkyl, halogenated C₁-C₁₀ alkoxy, C₁-C₁₀ cyanoalkyl, - OR₇, -C(O)R₇, -C(O)OR₇, -NR₈C(O)OR₇, -OC(O)R₇, -NR₈SO₂R₇, -SO₂NR₇R₈, -NR₇C(O)R₈, -C(O)NR₇R₈, -NR₇R₈, -SR₇, -S(O)R₇, -S(O)₂R₇, -SO₃H, -(C₀-C₆ alkylene)-(C₆-C₁₀ aryl), -SO₂-(C₀-C₆ alkylene)-(C₆-C₁₀ aryl), -S-(C₀-C₆ alkylene)-(C₆-C₁₀ aryl), -O-(C₀-C₆ alkylene)-(C₆-C₁₀ aryl), -(C₀-C₆ alkylene)-(4-10-membered heterocyclyl), -SO₂-(C₀-C₆ alkylene)-(4-10-membered heterocyclyl), -S-(C₀-C₆ alkylene)-(4-10-membered heterocyclyl), -O-(C₀-C₆ alkylene)-(4-10-membered heterocyclyl), -(C₀-C₆ alkylene)-(C₃-C₁₀ cycloalkyl), -SO₂-(C₀-C₆ alkylene)-(C₃-C₁₀ cycloalkyl), -S-(C₀-C₆ alkylene)-(C₃-C₁₀ cycloalkyl), and -O-(C₀-C₆ alkylene)-(C₃-C₁₀ cycloalkyl); wherein said C₁-C₁₀ alkyl, C₂-C₁₀ alkenyl, C₂-C₁₀ alkynyl, C₃-C₁₀ cycloalkyl, C₆-C₁₀ aryl, or 4-10-membered heterocyclyl may be optionally substituted with one or more groups selected from the group consisting of: halogen, cyano, nitro, azido, - OR', -C(O)R', -C(O)OR', -OC(O)R', -NR'C(O)OR", -NR'SO₂R", -SO₂NR'R", -NR'C(O)R", -C(O)NR'R", -NR'R", - SR', -SOR', -SO₂R', -SO₃H, C₁-C₁₀ alkyl, halogenated C₁-C₁₀ alkyl, halogenated C₁-C₁₀ alkoxy, C₁-C₁₀ cyanoalkyl, C₃-C₁₀ cycloalkyl, phenyl, and 4-10-membered heterocyclyl;
R₇ and R₈ are each independently selected from the group consisting of: H, C₁-C₁₀ alkyl, C₂-C₁₀ alkenyl, C₂-C₁₀ alkynyl, C₃-C₁₀ cycloalkyl, C₃-C₁₀ cycloalkylalkyl, C₆-C₁₀ aryl, C₇-C₁₂ aralkyl, 4-10-membered heterocyclyl, and 4-10-membered heterocyclylalkyl; wherein said C₁-C₁₀ alkyl, C₂-C₁₀ alkenyl, C₂-C₁₀ alkynyl, C₃-C₁₀ cycloalkyl, C₆-C₁₀ aryl, or 4-10-membered heterocyclyl may be optionally substituted with one or more groups selected from the group consisting of: halogen, cyano, nitro, azido, -OR', -C(O)R', -C(O)OR', -OC(O)R', -NR'C(O)OR", -NR'SO₂R", - SO₂NR'R", -NR'C(O)R", -C(O)NR'R", -NR'R", -SR', -SOR', -SO₂R', -SO₃H, C₁-C₁₀ alkyl, halogenated C₁-C₁₀ alkyl, halogenated C₁-C₁₀ alkoxy, C₁-C₁₀ cyanoalkyl, C₃-C₁₀ cycloalkyl, phenyl, and 4-10-membered heterocyclyl; and R' and R" are each independently selected from the group consisting of: H, C₁-C₁₀ alkyl, C₂-C₁₀ alkenyl, C₂-C₁₀ alkynyl, C₃-C₁₀ cycloalkyl, C₃-C₁₀ cycloalkylalkyl, C₆-C₁₀ aryl, C₇-C₁₂ aralkyl, 4-10-membered heterocyclyl, and 4-10-membered heterocyclylalkyl.

2. The compound according to claim 1, wherein said 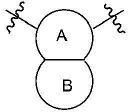 moiety is 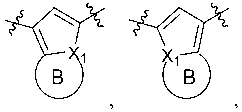 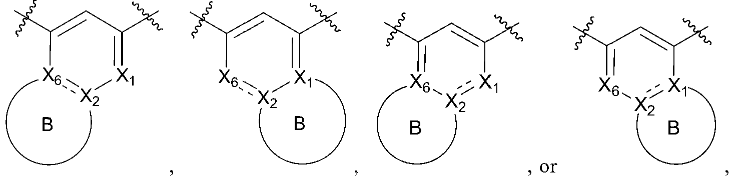 wherein X₁ is N or C, X₂ is N or C, X₆ is N or C, and -̅ -̅ -̅ -̅ -̅ -̅ represents a single or double bond;
preferably, said 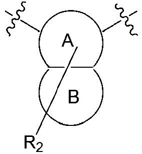 moiety is selected from the following structures: 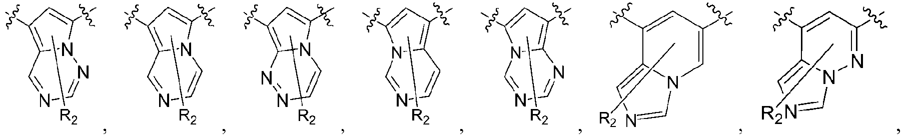 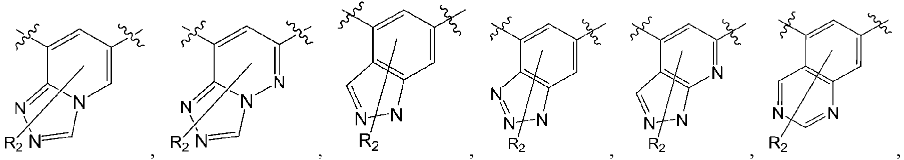 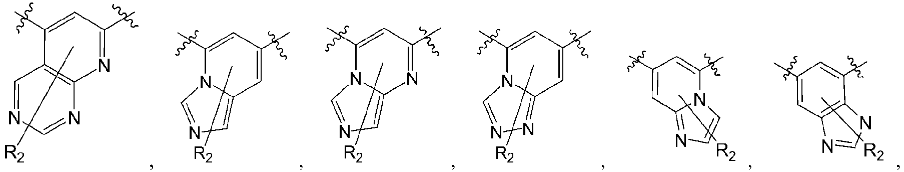 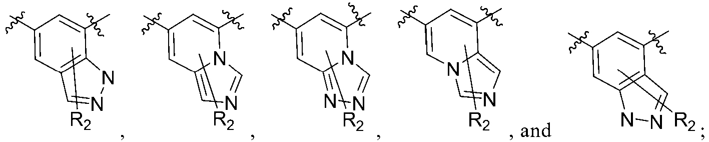
preferably, said 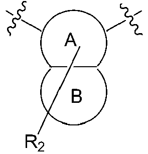 moiety is 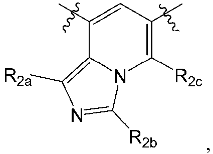 wherein R₂ₐ is selected from the group consisting of: H and halogen, R_{2b} is selected from the group consisting of: H, C₁-C₆ alkyl, halogenated C₁-C₆ alkyl, halogenated C₁-C₆ alkoxy, -(C₀-C₃ alkylene)-(C₃-C₆ cycloalkyl), and -C(O)(C₀-C₆ alkyl), and R_{2c} is selected from the group consisting of: H and halogen;
preferably, said 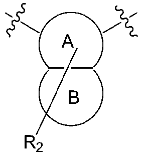 moiety is 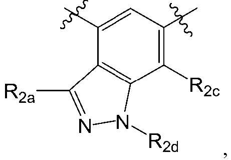 wherein R₂ₐ is selected from the group consisting of: H and halogen, R_{2d} is selected from the group consisting of: H, C₁-C₆ alkyl, halogenated C₁-C₆ alkyl, -(C₀-C₃ alkylene)-(C₃-C₆ cycloalkyl), and -C(O)(C₀-C₆ alkyl), and R_{2c} is selected from the group consisting of: H and halogen; and
more preferably, said 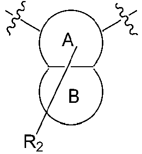 moiety is selected from the group consisting of: 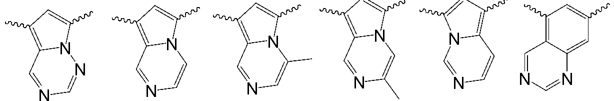 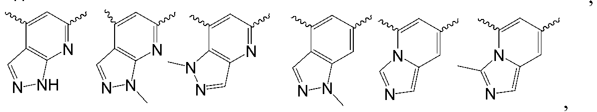 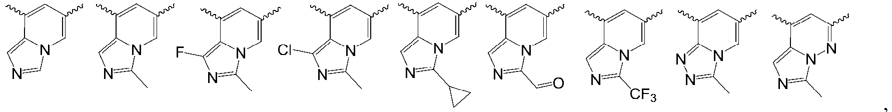 and

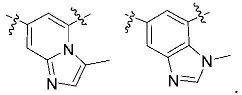

3. The compound according to claim 1 or 2, wherein said 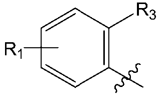 moiety is 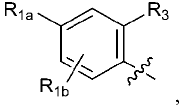 wherein R₁ₐ is H or halogen;
R_{1b} is one or more independent substituents on a benzene ring, each of which is independently selected from the group consisting of: H, halogen, C₁-C₆ alkyl, halogen, cyano, nitro, azido, halogenated C₁-C₆ alkyl, - N(C₀-C₆ alkyl)C(O)(C₀-C₆ alkyl), and -C(O)N(C₀-C₆ alkyl)(C₀-C₆ alkyl);
preferably, R₁ₐ is H, F, or Cl;
more preferably, said 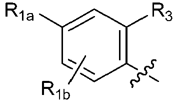 moiety is

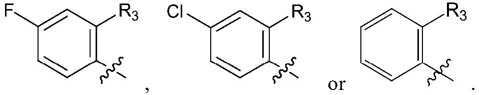

4. The compound according to any one of claims 1 to 3, wherein R₃ is -NR₃₀₂C(O)R₃₀₁ or -C(O)NR₃₀₁R₃₀₂, wherein R₃₀₁ or R₃₀₂ is independently selected from the group consisting of: H, optionally substituted C₁-C₆ alkyl, optionally substituted C₃-C₆ cycloalkyl, optionally substituted C₃-C₁₀ cycloalkylalkyl, optionally substituted 4-10-membered heterocyclyl, and optionally substituted 4-10-membered heterocyclylalkyl; or,
R₃ is 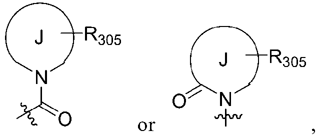 wherein Ring J is a 4-8-membered saturated heterocyclic ring, and R₃₀₅ is one or more independent substituents on Ring J, each of which is independently selected from the group consisting of: H, halogen, C₁-C₆ alkyl, C₂-C₆ alkenyl, C₂-C₆ alkynyl, cyano, hydroxyl, amino, halogenated C₁-C₆ alkyl, C₁-C₆ alkoxy, C₁-C₆ alkylamino, halogenated C₁-C₆ alkoxy, C₁-C₆ cyanoalkyl, C₃-C₆ cycloalkyl, and 4-10-membered heterocyclyl; or,
R₃ is -(C₀-C₆ alkylene)-(C₆-C₁₀ aryl), preferably 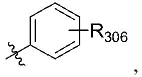 wherein R₃₀₆ is one or more independent substituents on a benzene ring, each of which is independently selected from the group consisting of: H, halogen, C₁-C₆ alkyl, C₂-C₆ alkenyl, C₂-C₆ alkynyl, cyano, hydroxyl, amino, halogenated C₁-C₆ alkyl, C₁-C₆ alkoxy, C₁-C₆ alkylamino, halogenated C₁-C₆ alkoxy, C₁-C₆ cyanoalkyl, C₃-C₆ cycloalkyl, and 4-10-membered heterocyclyl; or,
R₃ is -(C₀-C₆ alkylene)-(4-10-membered heterocyclyl), preferably selected from the group consisting of: 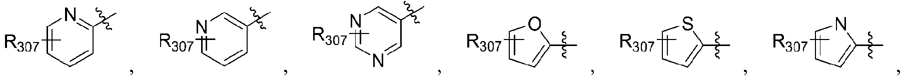 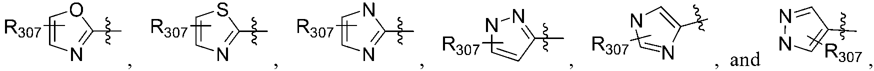 wherein R₃₀₇ is one or more independent substituents on a ring, each of which is independently selected from the group consisting of: H, halogen, C₁-C₆ alkyl, C₂-C₆ alkenyl, C₂-C₆ alkynyl, cyano, hydroxyl, amino, halogenated C₁-C₆ alkyl, C₁-C₆ alkoxy, C₁-C₆ alkylamino, halogenated C₁-C₆ alkoxy, C₁-C₆ cyanoalkyl, C₃-C₆ cycloalkyl, and 4-10-membered heterocyclyl.

5. The compound according to claim 4, wherein R₃ is 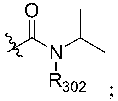
preferably, R₃₀₂ is selected from the group consisting of: H, C₁-C₆ alkyl, halogenated C₁-C₆ alkyl, and C₃-C₆ cycloalkyl; and
more preferably, R₃ is selected from the group consisting of:

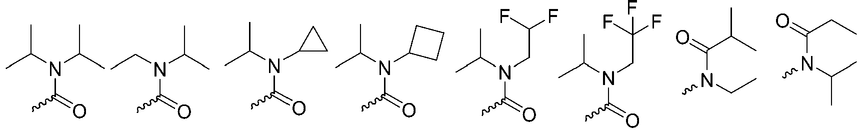

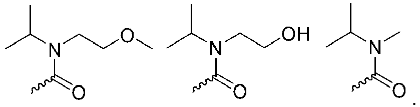

6. The compound according to claim 4, wherein R₃ is selected from the group consisting of: 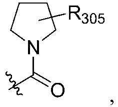 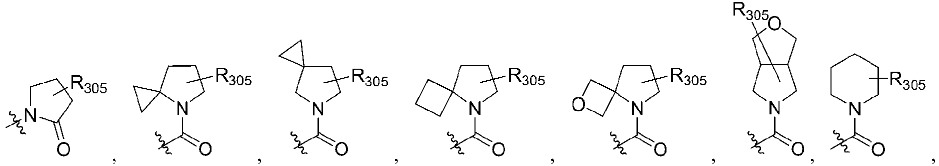 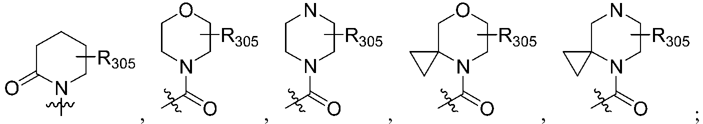
preferably, R₃₀₅ is selected from the group consisting of: H, halogen, C₁-C₆ alkyl, halogenated C₁-C₆ alkyl, C₃-C₆ cycloalkyl, and 4-6-membered heterocycloalkyl; and
more preferably, R₃ is selected from the group consisting of:

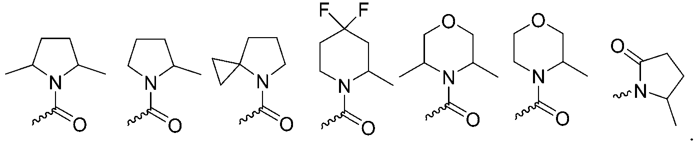

7. The compound according to claim 4, wherein R₃ is 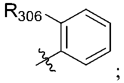
preferably, R₃₀₆ is selected from the group consisting of: H, halogen, C₁-C₆ alkyl, halogenated C₁-C₆ alkyl, and C₃-C₆ cycloalkyl; and
more preferably, R₃ is selected from the group consisting of:

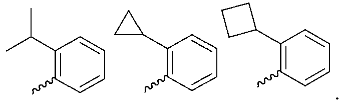

8. The compound according to claim 4, wherein R₃₀₇ is selected from the group consisting of: H, halogen, C₁-C₆ alkyl, halogenated C₁-C₆ alkyl, and C₃-C₆ cycloalkyl; and
preferably, R₃ is selected from the group consisting of:

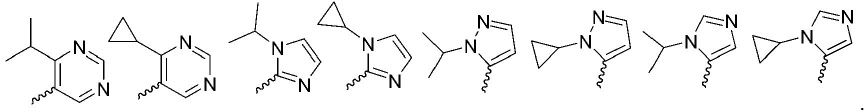

9. The compound according to any one of claims 1 to 8, wherein L₁ is a single bond, C₁-C₆ alkylene, -O-, -S- or -N(R_{La})-, and R_{La} is selected from the group consisting of: H and C₁-C₆ alkyl, preferably a single bond.

10. The compound according to any one of claims 1 to 9, wherein said 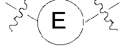 moiety is selected from the group consisting of: 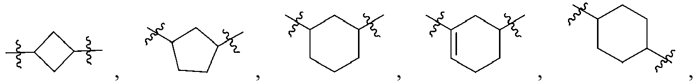 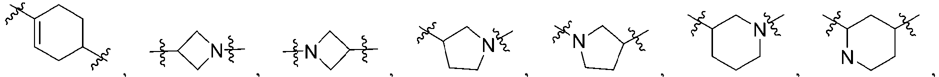 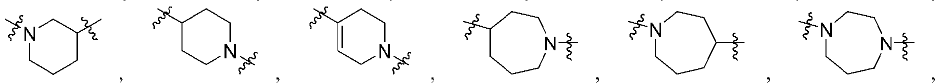 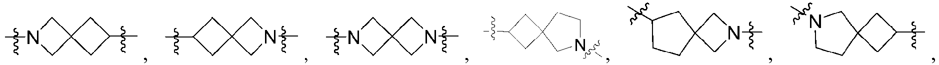 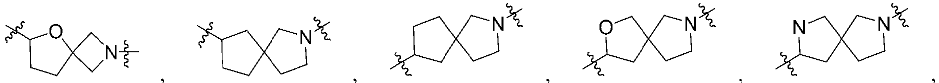 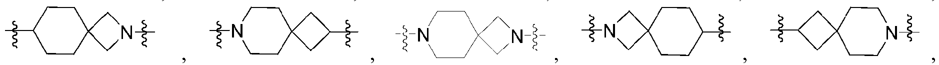 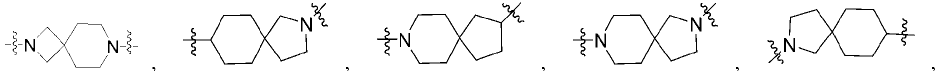 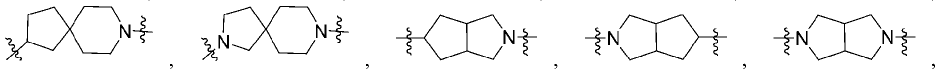 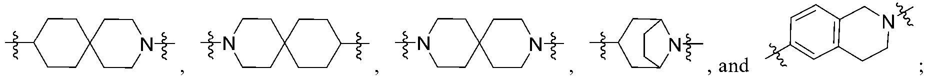
preferably, R₄ is selected from the group consisting of: H, halogen, =O, cyano, nitro, azido, hydroxyl, C₁-C₆ alkyl, halogenated C₁-C₆ alkyl, C₁-C₆ alkoxy, halogenated C₁-C₆ alkoxy, and C₁-C₆ cyanoalkyl, more preferably selected from the group consisting of: H, halogen, hydroxyl, C₁-C₃ alkyl, and C₁-C₃ alkoxy; and
more preferably, said 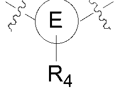 moiety is selected from the group consisting of:

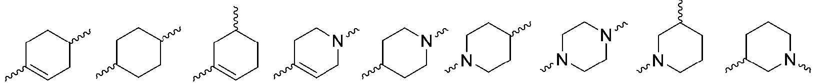

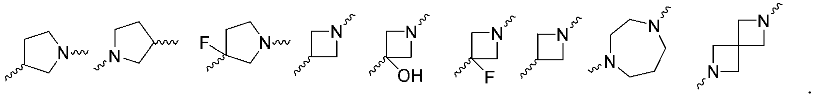

11. The compound according to any one of claims 1 to 10, wherein L₂ is a single bond, C₁-C₆ alkylene or - (C₀-C₆ alkylene)-Q-(C₀-C₆ alkylene)-, with Q selected from the group consisting of: -O-, -C(O)-, -N(C₀-C₃ alkyl)-, and -N(C₀-C₃ alkyl)C(O)-, wherein H atom(s) in said alkylene may be optionally substituted with a group selected from the group consisting of: H, C₁-C₃ alkyl, halogen, cyano, halogenated C₁-C₃ alkyl, hydroxyl, C₁-C₃ alkoxy, halogenated C₁-C₃ alkoxy, amino, and C₁-C₆ alkylamino; and
preferably, L₂ is selected from the group consisting of: methylene, 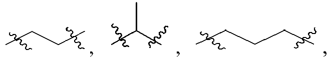 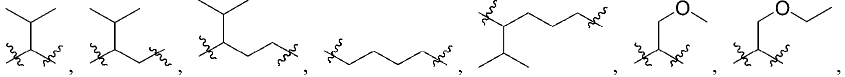 -O-, -C(O)-, -NHC(O)-,

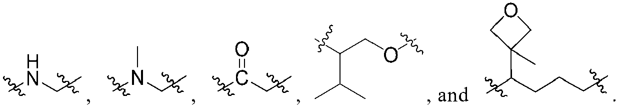

12. The compound according to any one of claims 1 to 11, wherein Y is selected from groups in sets Y1, Y2, Y3, Y4, and Y5;
Y1 is 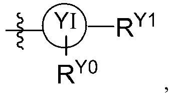 wherein Ring YI is a 3-8-membered saturated or partially saturated aliphatic ring, a benzene ring, or a 4-12-membered saturated or partially saturated heterocyclic ring;
preferably, Y1 is selected from the group consisting of: 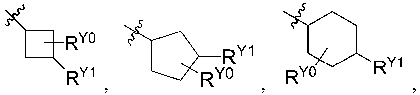 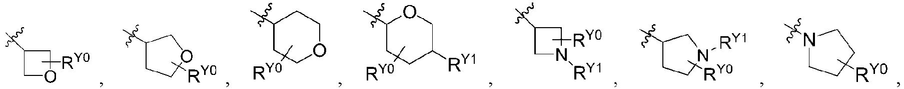 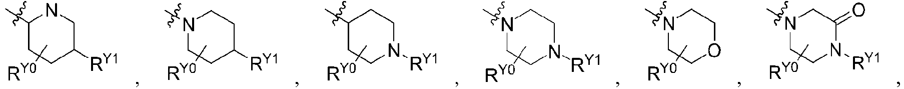 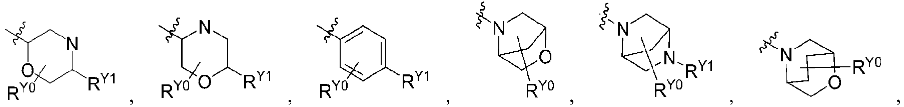 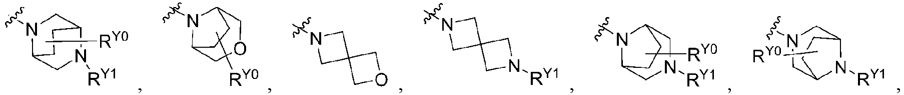 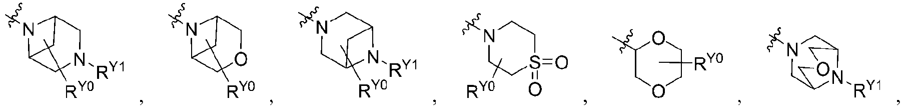 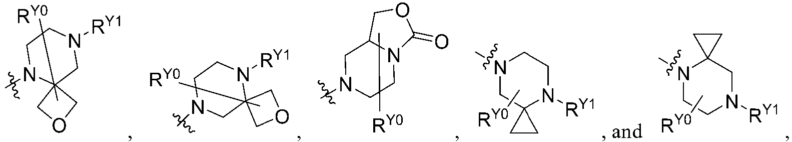 R^{Y0} is one or more independent substituents on a ring, R^{Y1} is selected from the group consisting of: H, -C(O)R₇, -C(O)OR₇, - NR₈SO₂R₇, -NR₈C(O)OR₇, -NR₈C(O)R₇, -C(O)NR₇R₈, -NR₇R₈, -S(O)₂R₇, C₁-C₆ alkyl, -(C₀-C₆ alkylene)-(phenyl), -(C₀-C₆ alkylene)-(4-10-membered heterocyclyl), and -(C₀-C₆ alkylene)-(C₃-C₆ cycloalkyl); wherein said C₁-C₆ alkyl, C₃-C₆ cycloalkyl, phenyl, or 4-10-membered heterocyclyl may be optionally substituted with one or more groups selected from the group consisting of: halogen, cyano, nitro, azido, -OR', -C(O)R', -C(O)OR', -NR'C(O)R", -C(O)NR'R", -NR'R", C₁-C₆ alkyl, halogenated C₁-C₆ alkyl, halogenated C₁-C₆ alkoxy, C₁-C₆ cyanoalkyl, C₁-C₆ hydroxylalkyl, C₁-C₆ alkoxyalkyl, C₃-C₆ cycloalkyl, phenyl, and 4-10-membered heterocyclyl; R₇ and R₈ are each independently selected from the group consisting of: H, C₁-C₆ alkyl, C₃-C₆ cycloalkyl, -(C₀-C₆ alkylene)-(C₃-C₆ cycloalkyl), -(C₀-C₆ alkylene)-(phenyl), and -(C₀-C₆ alkylene)-(4-10-membered heterocyclyl); wherein said C₁-C₆ alkyl, C₃-C₆ cycloalkyl, phenyl, or 4-10-membered heterocyclyl may be optionally substituted with one or more groups selected from the group consisting of: halogen, cyano, nitro, azido, -OR', -C(O)R', -C(O)OR', -NR'C(O)R", -C(O)NR'R", -NR'R", C₁-C₆ alkyl, halogenated C₁-C₆ alkyl, halogenated C₁-C₆ alkoxy, C₁-C₆ cyanoalkyl, C₃-C₆ cycloalkyl, phenyl, and 4-10-membered heterocyclyl;
Y2 is 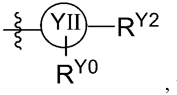 wherein Ring YII is a benzene ring-fused four- to eight-membered aliphatic ring, a benzene ring-fused four- to eight-membered heterocyclic ring, or a bicyclic heterocyclic ring;
preferably, Y2 is selected from the group consisting of: 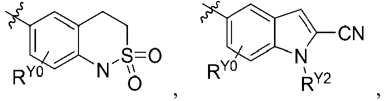 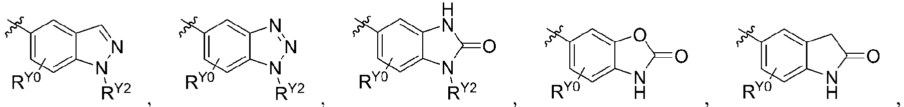 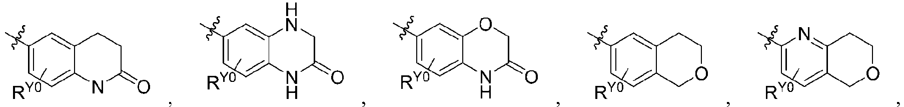 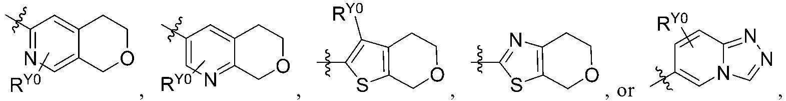 R^{Y0} is one or more independent substituents on a ring, R^{Y2} is selected from the group consisting of: H, cyano, C₁-C₆ alkyl, and 4-10-membered heterocyclyl, wherein said C₁-C₆ alkyl or 4-10-membered heterocyclyl may be optionally substituted with one or more groups selected from the group consisting of: =O, halogen, cyano, nitro, amino, 4-10-membered heterocyclyl, C₃-C₈ cycloalkyl, 4-10-membered heterocyclyl, -(C₀-C₄ alkylene)-OH, -(C₁-C₄ alkylene)-O-(C₁-C₄ alkyl), C₁-C₄ alkoxy, halogenated C₁-C₄ alkoxy, -C(O)O(C₀-C₆ alkyl), -C(O)N(C₀-C₆ alkyl)(C₀-C₆ alkyl), -N(C₀-C₆ alkyl)C(O) (C₀-C₆ alkyl), -SO₂(C₀-C₆ alkyl), -SO₂(phenyl), -SO₂(halogenated C₁-C₆ alkyl), -SO₂N(C₀-C₆ alkyl)(C₀-C₆ alkyl), -SO₂NH(phenyl), -NHSO₂(C₀-C₆ alkyl), -NHSO₂(phenyl) and -NHSO₂(halogenated C₁-C₆ alkyl);
Y3 is 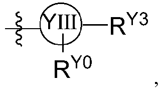 wherein Ring YIII is a four- to eight-membered monocyclic nitrogenous saturated heterocyclic ring, a four- to eight-membered monocyclic nitrogenous saturated heterocyclic ring-fused benzene ring, a four- to eight-membered monocyclic nitrogenous saturated heterocyclic ring-fused four- to eight-membered aliphatic ring, or a six- to ten-membered bridged nitrogenous saturated heterocyclic ring;
preferably, Y3 is selected from the group consisting of: 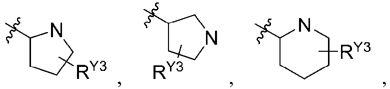 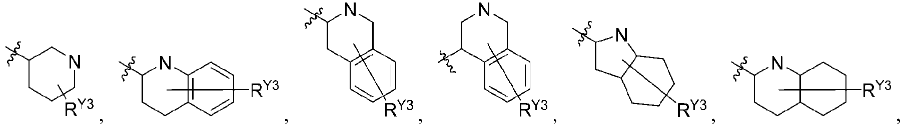 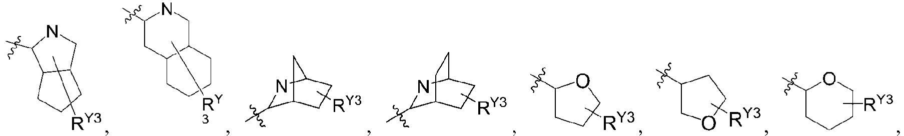 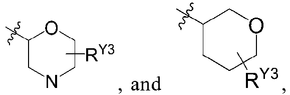 R^{Y3} is one or more independent substituents on a ring, and R^{Y3} is selected from the group consisting of: H, =O, 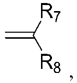 C₁-C₆ alkyl, halogenated C₁-C₆ alkyl, C₁-C₆ alkoxy, halogenated C₁-C₆ alkoxy, -(C₀-C₆ alkylene)-(C₃-C₆ cycloalkyl), -(C₀-C₆ alkylene)-(phenyl), -(C₀-C₆ alkylene)-(4-10-membered heterocyclyl); wherein said C₃-C₆ cycloalkyl, phenyl, or 4-10-membered heterocyclyl may be optionally substituted with one or more groups selected from the group consisting of: halogen, cyano, nitro, azido, -OR', -C(O)R', -C(O)OR', -NR'C(O)R", -C(O)NR'R", -NR'R", C₁-C₆ alkyl, halogenated C₁-C₆ alkyl, halogenated C₁-C₆ alkoxy, C₁-C₆ cyanoalkyl, C₃-C₆ cycloalkyl, phenyl, and 4-10-membered heterocyclyl;
R^{Y0} is selected from the group consisting of: H, =O, C₁-C₆ alkyl, C₂-C₆ alkenyl, C₂-C₆ alkynyl, halogen, cyano, nitro, azido, halogenated C₁-C₆ alkyl, halogenated C₁-C₆ alkoxy, C₁-C₆ cyanoalkyl, -OR₇, -C(O)R₇, - C(O)OR₇, -NR₈C(O)OR₇, -OC(O)R₇, -NR₈SO₂R₇, -SO₂NR₇R₈, -NR₇C(O)R₈, -C(O)NR₇R₈, -NR₇R₈, -SR₇, - S(O)R₇, -S(O)₂R₇, -SO₃H, -(C₀-C₆ alkylene)₋(phenyl), -SO₂-(C₀-C₆ alkylene)₋(phenyl), -S-(C₀-C₆ alkylene)₋(phenyl), -O-(C₀-C₆ alkylene)₋(phenyl), -(C₀-C₆ alkylene)₋(4-10-membered heterocyclyl), -SO₂-(C₀-C₆ alkylene)₋(4-10-membered heterocyclyl), -S-(C₀-C₆ alkylene)₋(4-10-membered heterocyclyl), -O-(C₀-C₆ alkylene)₋(4-10-membered heterocyclyl), -(C₀-C₆ alkylene)₋(C₃-C₆ cycloalkyl), -SO₂-(C₀-C₆ alkylene)₋(C₃-C₆ cycloalkyl), -S-(C₀-C₆ alkylene)₋(C₃-C₆ cycloalkyl), and -O-(C₀-C₆ alkylene)₋(C₃-C₆ cycloalkyl), and said C₁-C₆ alkyl, C₂-C₆ alkenyl, C₂-C₆ alkynyl, C₃-C₆ cycloalkyl, phenyl, or 4-10-membered heterocyclyl may be optionally substituted with one or more groups selected from the group consisting of: halogen, cyano, nitro, azido, -OR', -C(O)R', -C(O)OR', -NR'C(O)R", -C(O)NR'R", -NR'R", C₁-C₆ alkyl, halogenated C₁-C₆ alkyl, halogenated C₁-C₆ alkoxy, C₁-C₆ cyanoalkyl, C₃-C₆ cycloalkyl, phenyl, and 4-10-membered heterocyclyl;
Y4 is 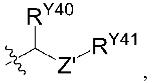
wherein,
R^{Y40} is selected from the group consisting of: C₁-C₆ alkyl, halogenated C₁-C₆ alkyl, C₃-C₆ cycloalkyl, 3-6-membered heterocyclyl, and C₁-C₃ alkyl-substituted 3-6-membered heterocyclyl;
Z' is selected from the group consisting of: C₀-C₆ alkylene, -(C₀-C₃ alkylene)-O-(C₀-C₃ alkylene)-, -(C₀-C₃ alkylene)-S-(C₀-C₃ alkylene)-, -(C₀-C₃ alkylene)-NH-(C₀-C₃ alkylene)-, -(C₀-C₃ alkylene)-C(O)NH-(C₀-C₃ alkylene)-, -(C₀-C₃ alkylene)-NHC(O)-(C₀-C₃ alkylene)-, and C₃-C₆ cycloalkylene; wherein H in said alkylene is optionally substituted with one or more groups selected from the group consisting of: halogen, cyano, hydroxyl, and amino;
R^{Y41} is selected from the group consisting of: H, halogen, cyano, C₁-C₆ alkyl, C₃-C₁₀ cycloalkyl, C₆-C₁₀ aryl, 4-12-membered heterocyclyl, -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -S(C₀₋₁₀ alkyl), -SO(C₀₋₁₀ alkyl), - SO₂(C₀₋₁₀ alkyl), -SO₂N(C₀₋₁₀ alkyl)(C₀₋₁₀ alkyl), -COO(C₀₋₁₀ alkyl), -OCO(C₀₋₁₀ alkyl), -CON(C₀₋₁₀ alkyl)(C₀₋₁₀ alkyl), and -CO(C₀₋₁₀ alkyl); wherein H in said alkyl, cycloalkyl, aryl, or heterocyclyl is optionally substituted with a group selected from the group consisting of: halogen, cyano, C₁-C₆ alkyl, C₃-C₁₀ cycloalkyl, 4-12-membered heterocyclyl, -N(C₀₋₁₀ alkyl)(C₀₋₁₀ alkyl), -N(C₀₋₁₀ alkyl)CO(C₀₋₁₀ alkyl), - N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -S(C₀₋₁₀ alkyl), -SO(C₀₋₁₀ alkyl), -SO₂(C₀₋₁₀ alkyl), -SO₂N(C₀₋₁₀ alkyl)(C₀₋₁₀ alkyl), -COO(C₀₋₁₀ alkyl), -OCO(C₀₋₁₀ alkyl), -CON(C₀₋₁₀ alkyl)(C₀₋₁₀ alkyl), -CO(C₀₋₁₀ alkyl), -N(C₀₋₁₀ alkyl)(4-10-membered heterocyclyl), -N(C₀₋₁₀ alkyl)CO(4-10-membered heterocyclyl), -N(C₀₋₁₀ alkyl)CON(4-10-membered heterocyclyl), -N(C₀₋₁₀ alkyl)SO₂(4-10-membered heterocyclyl), -O(4-10-membered heterocyclyl), -S(4-10-membered heterocyclyl), -SO(4-10-membered heterocyclyl), -SO₂(4-10-membered heterocyclyl), -SO₂N(C₀₋₁₀ alkyl)( 4-10-membered heterocyclyl), -COO(4-10-membered heterocyclyl), -OCO(4-10-membered heterocyclyl), -CON(C₀₋₁₀ alkyl)( 4-10-membered heterocyclyl), and -CO(4-10-membered heterocyclyl), wherein H in said alkyl or heterocyclyl is optionally substituted with one or more groups selected from the group consisting of: halogen, cyano, C₁-C₆ alkyl, halogenated C₁-C₆ alkyl, C₁-C₆hydroxylalkyl, C₁-C₆ alkoxyalkyl, -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), - O(C₀₋₁₀ alkyl), -S(C₀₋₁₀ alkyl), -SO(C₀₋₁₀ alkyl), -SO₂(C₀₋₁₀ alkyl), -SO₂N(C₀₋₁₀ alkyl)(C₀₋₁₀ alkyl), -COO(C₀₋₁₀ alkyl), -OCO(C₀₋₁₀ alkyl), -CON(C₀₋₁₀ alkyl)(C₀₋₁₀ alkyl), and -CO(C₀₋₁₀ alkyl);
Y5 is 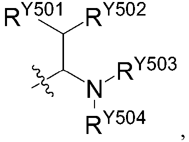 wherein R^{Y501} and R^{Y502} are independently selected from the group consisting of: H, halogen, hydroxyl, amino, C₁-C₃ alkyl, halogenated C₁-C₃ alkyl, and -(C₀-C₃ alkylene)-(C₃-C₆ cycloalkyl), wherein said alkyl or cycloalkyl may be optionally substituted with one or more groups selected from the group consisting of: halogen, cyano, hydroxyl, amino, C₁-C₆ alkoxy, and C₁-C₆ alkylamino; or, R^{Y501} and R^{Y502}, together with the carbon atom attached thereto, form an aliphatic ring or a heterocyclic ring, wherein said aliphatic ring or heterocyclic ring may be optionally substituted with one or more groups selected from the group consisting of: halogen, cyano, hydroxyl, amino, C₁-C₆ alkyl, and halogenated C₁-C₆ alkyl;
R^{Y503} and R^{Y504} are independently selected from the group consisting of: H and C₁-C₆ alkyl, wherein said alkyl may be optionally substituted with one or more groups selected from the group consisting of: halogen, cyano, hydroxyl, amino, C₁-C₆ alkoxy, and C₁-C₆ alkylamino; or, R^{Y503} and R^{Y504}, together with the nitrogen atom attached thereto, form a heterocyclic ring, wherein said heterocyclic ring may be optionally substituted with one or more groups selected from the group consisting of: halogen, cyano, nitro, azido, C₁-C₁₀ alkyl, C₂-C₁₀ alkenyl, C₂-C₁₀ alkynyl, -(C₀-C₆ alkylene)-(C₃-C₁₀ cycloalkyl), -(C₀-C₆ alkylene)-(phenyl), -(C₀-C₆ alkylene)-(4-10-membered heterocyclyl), halogenated C₁-C₁₀ alkyl, halogenated C₁-C₁₀ alkoxy, -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -S(C₀₋₁₀ alkyl), -SO(C₀₋₁₀ alkyl), -SO₂(C₀₋₁₀ alkyl), -SO₂N(C₀₋₁₀ alkyl)(C₀₋₁₀ alkyl), - COO(C₀₋₁₀ alkyl), -OCO(C₀₋₁₀ alkyl), -CON(C₀₋₁₀ alkyl)(C₀₋₁₀ alkyl), -CO(C₀₋₁₀ alkyl), and -CO(C₃-C₁₀ cycloalkyl); wherein H in said C₀₋₁₀ alkyl, C₁-C₁₀ alkyl, C₂-C₁₀ alkenyl, C₂-C₁₀ alkynyl, C₀-C₆ alkylene, C₃-C₁₀ cycloalkyl, phenyl, or 4-10-membered heterocyclyl is optionally substituted with one or more groups selected from the group consisting of: halogen, cyano, nitro, azido, C₁-C₁₀ alkyl, halogenated C₁-C₁₀ alkyl, halogenated C₁-C₁₀ alkoxy, -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -S(C₀₋₁₀ alkyl), -SO(C₀₋₁₀ alkyl), - SO₂(C₀₋₁₀ alkyl), -SO₂N(C₀₋₁₀ alkyl)(C₀₋₁₀ alkyl), -COO(C₀₋₁₀ alkyl), -OCO(C₀₋₁₀ alkyl), -CON(C₀₋₁₀ alkyl)(C₀₋₁₀ alkyl), and -CO(C₀₋₁₀ alkyl).

13. The compound according to claim 12, wherein R^{Y1} is selected from the group consisting of: -NR₈SO₂R₇, -NR₈C(O)OR₇, -C(O)R₇, -C(O)OR₇, -C(O)NR₇R₈, -S(O)₂R₇, and -(CH₂)ₙ(4-10-membered heterocyclyl),
R₇ is selected from the group consisting of: H, C₁-C₆ alkyl, halogenated C₁-C₆ alkyl, hydroxyl-substituted C₁-C₆ alkyl, -(C₀-C₃ alkylene)-(C₃-C₆ cycloalkyl), and -(C₀-C₃ alkylene)-(4-10-membered heterocyclyl), wherein said C₁-C₆ alkyl, C₃-C₆ cycloalkyl, or 4-10-membered heterocyclyl may be optionally substituted with one or more groups selected from the group consisting of: halogen, cyano, hydroxyl, C₁-C₆ alkyl, halogenated C₁-C₆ alkyl, C₁-C₆ alkoxy, halogenated C₁-C₆ alkoxy, hydroxyl-substituted C₁-C₆ alkyl, - C(O)N(C₀-C₆ alkyl)( C₀-C₆ alkyl), and -N(C₀-C₆ alkyl)C(O)(C₀-C₆ alkyl),
R₈ is selected from the group consisting of: H and C₁-C₆ alkyl;
more preferably, R^{Y1} is selected from the group consisting of:
H, C₁-C₆ alkyl, halogenated C₁-C₆ alkyl, hydroxyl-substituted C₁-C₆ alkyl, C₁-C₆ alkoxyalkyl, -O-(hydroxyl-substituted C₁-C₆ alkyl), -O-(C₁-C₆ alkoxyalkyl), C₁-C₆ cyanoalkyl, 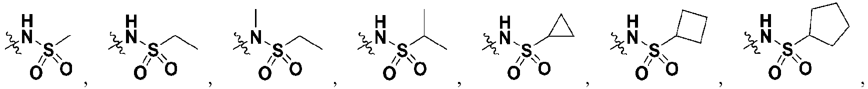 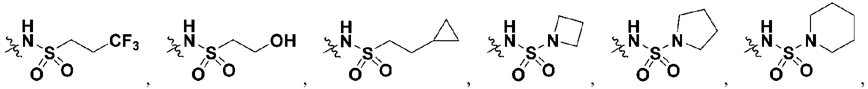 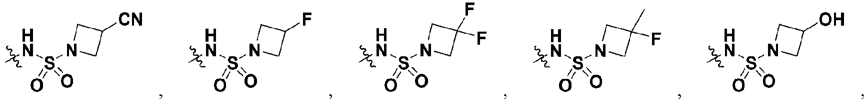 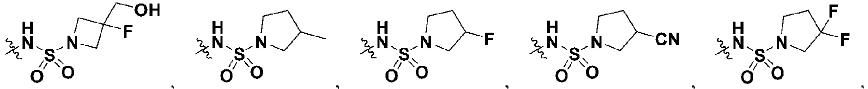 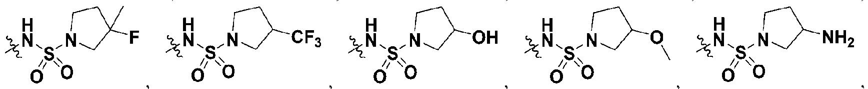 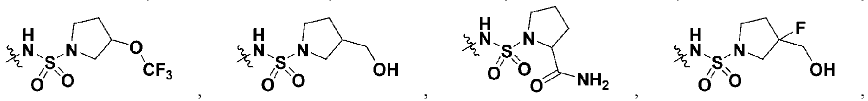 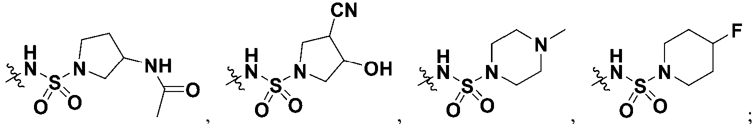 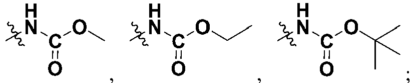 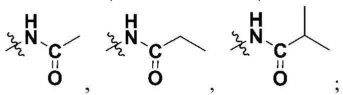 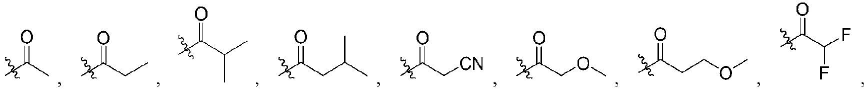 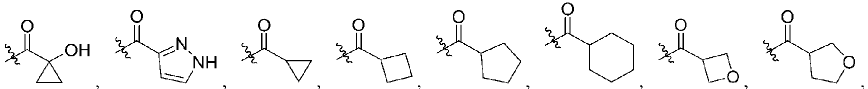 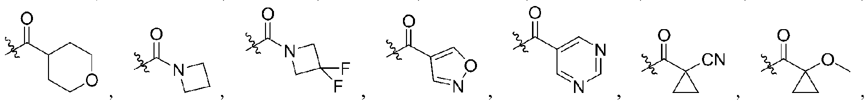 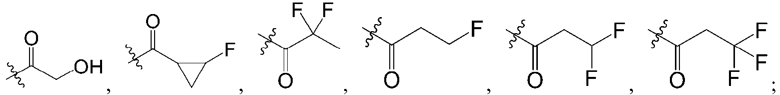 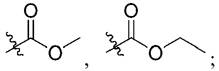 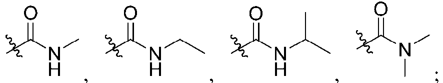 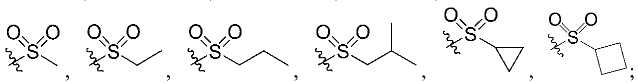 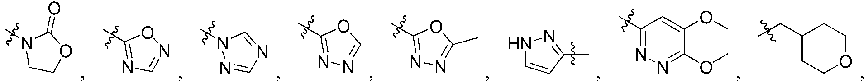 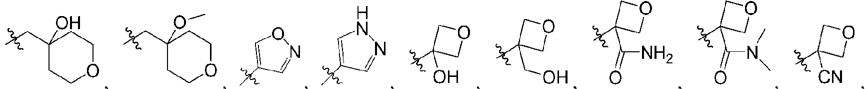 and 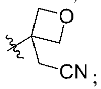
preferably, in Y1, R^{Y0} is selected from the group consisting of: H, halogen, C₁-C₃ alkyl, halogenated C₁-C₃ alkyl, hydroxyl, and C₁-C₃ alkoxy;
more preferably, Y1 is selected from the group consisting of: 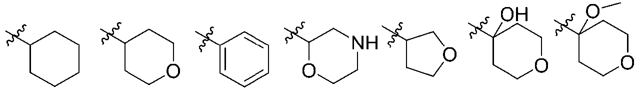 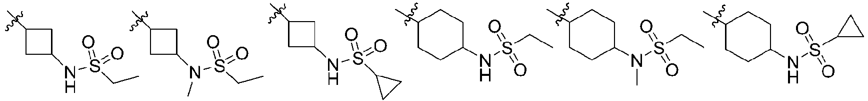 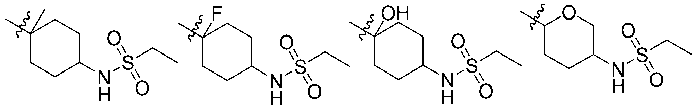 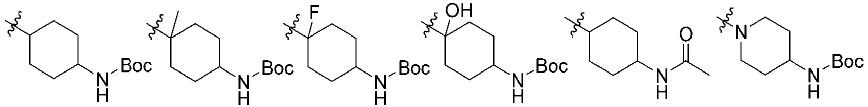 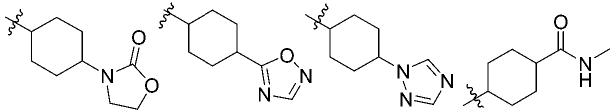 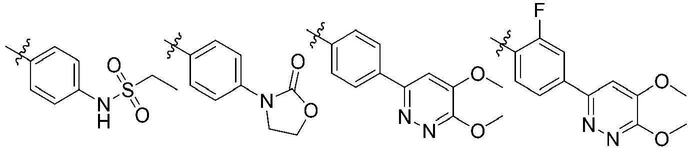 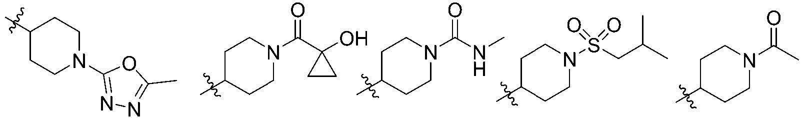 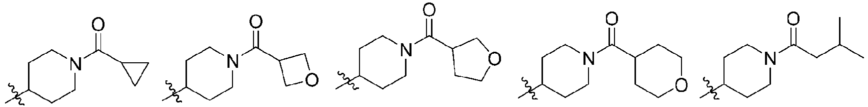 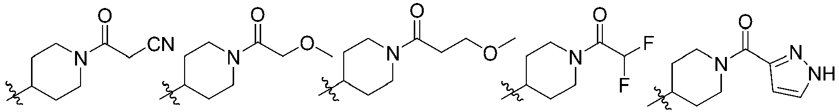 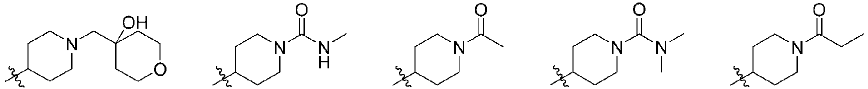 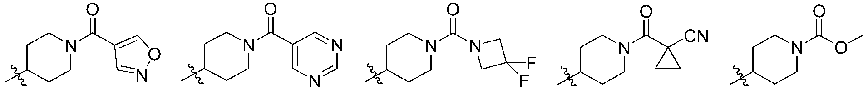 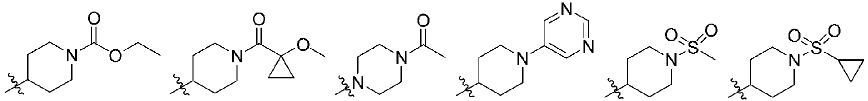 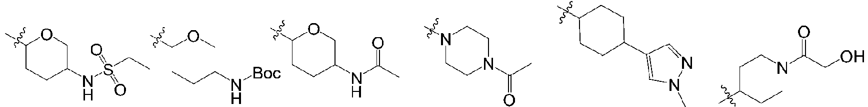 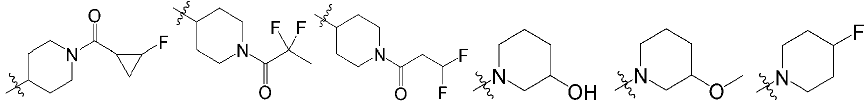 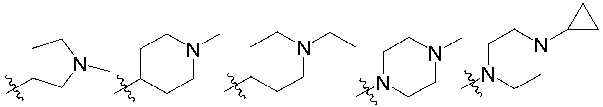 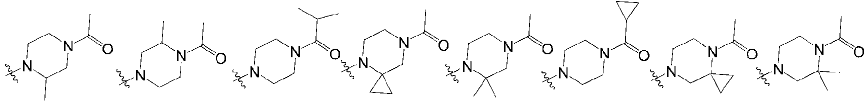 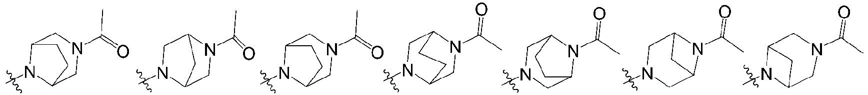 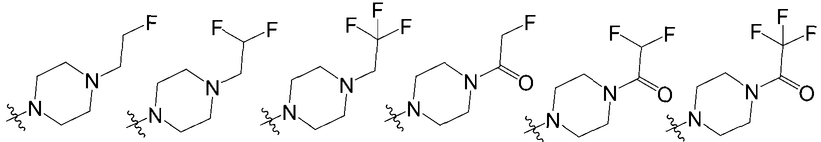 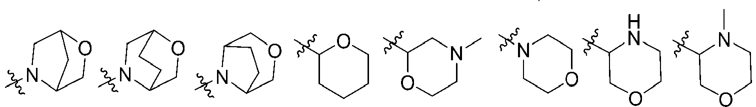 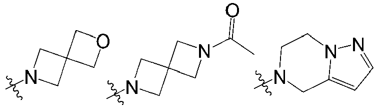 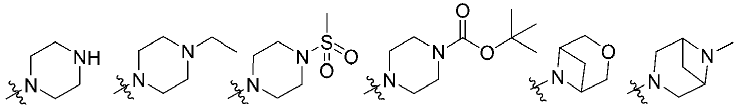 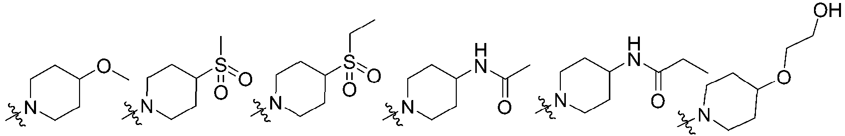 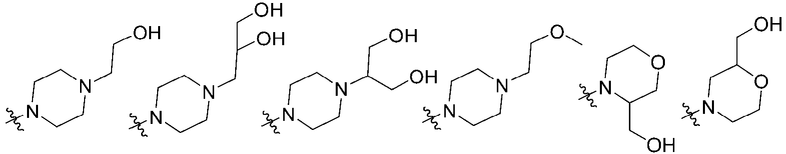 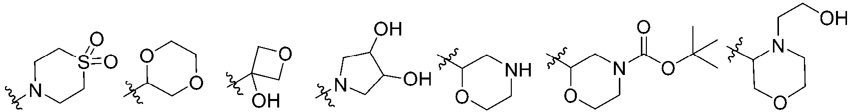 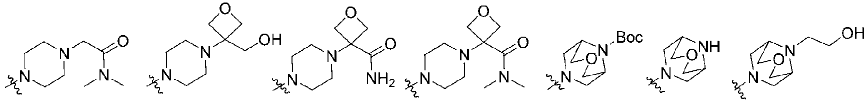 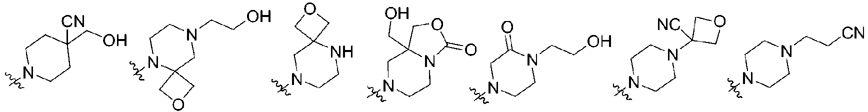 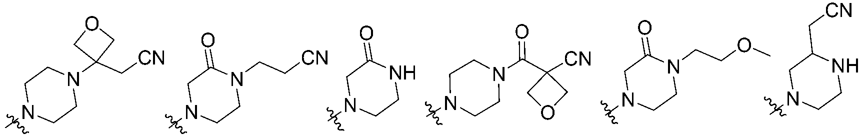 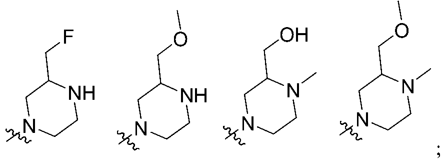 and
preferably, L₂ is C₁-C₆ alkylene, alkoxy-substituted C₁-C₆ alkylene (for example, 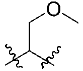 and 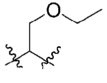 ), -N(C₀-C₃ alkyl)-(C₀-C₆ alkylene)-, or -(C₀-C₆ alkylene)-O- (for example, 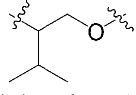 ).

14. The compound according to claim 12, wherein R^{Y2} is selected from the group consisting of: H, -(C₁-C₄ alkylene)-OH, -(C₁-C₄ alkylene)-O-(C₁-C₄ alkyl), -(C₀-C₄ alkylene)-(C₃-C₆ cycloalkyl), and -(C₀-C₄ alkylene)-(optionally substituted 4-10-membered heterocyclyl);
preferably, in Y2, R^{Y0} is selected from the group consisting of: H, halogen, C₁-C₃ alkyl, halogenated C₁-C₃ alkyl, hydroxyl, and C₁-C₃ alkoxy;
more preferably, Y2 is selected from the group consisting of: 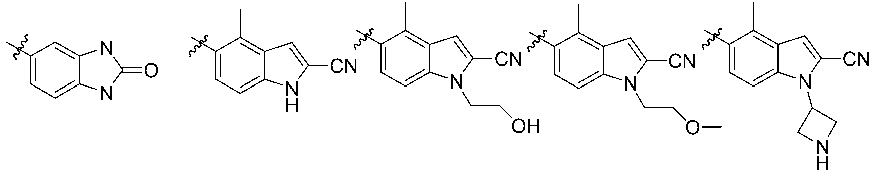 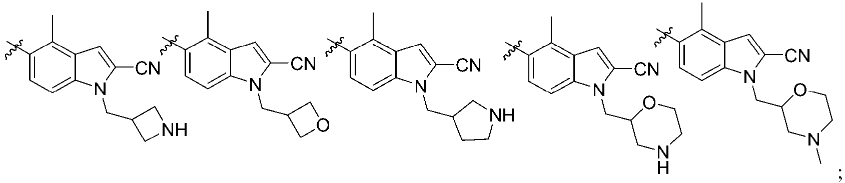 and
preferably, L₂ is C₁-C₆ alkylene, alkoxy-substituted C₁-C₆ alkylene (for example, 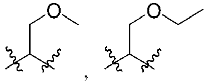 ) or -N(C₀-C₃ alkyl)-(C₀-C₆ alkylene)-.

15. The compound according to claim 12, wherein R^{Y3} is selected from the group consisting of: H, 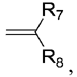 halogen, C₁-C₆ alkyl, C₁-C₆ alkoxy, phenyl, and halogenated phenyl;
preferably, in Y3, R^{Y0} is selected from the group consisting of: H, halogen, C₁-C₃ alkyl, halogenated C₁-C₃ alkyl, hydroxyl, and C₁-C₃ alkoxy;
more preferably, Y3 is selected from the group consisting of: 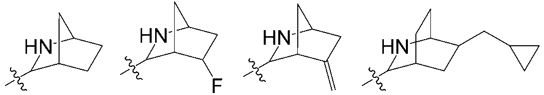 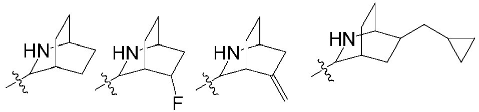 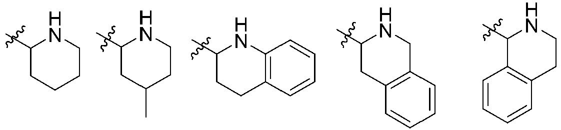 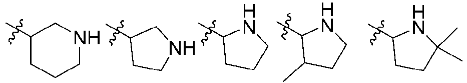 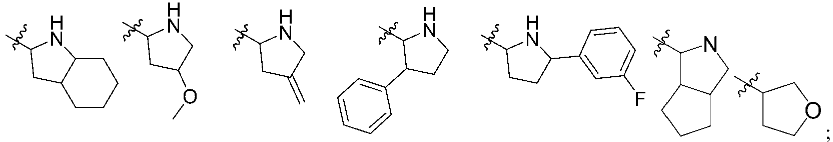 and
preferably, L₂ is C(O).

16. The compound according to claim 12, wherein R^{Y40} is C₁-C₄ alkyl, halogenated C₁-C₄ alkyl or 3-6-membered heterocyclyl optionally substituted with C₁-C₃ alkyl, preferably selected from the group consisting of: 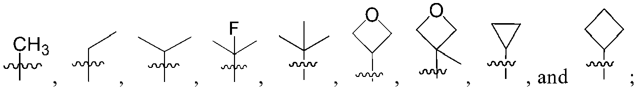
preferably, Z' is a single bond, C₁-C₆ alkylene or C₃-C₆ cycloalkylene, wherein H in said alkylene is optionally substituted with one or more groups selected from the group consisting of: halogen, cyano, and hydroxyl; preferably, R^{Y41} is selected from the group consisting of: -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -COO(C₀₋₁₀ alkyl), -OCO(C₀₋₁₀ alkyl), and -CON(C₀₋₁₀ alkyl)(C₀₋₁₀ alkyl); wherein H in said alkyl is optionally substituted with one or more groups selected from the group consisting of: halogen, cyano, hydroxyl, and C₁-C₆ alkoxy; or, R^{Y41} is 4-12-membered heterocyclyl, H in said heterocyclyl is optionally substituted with one or more R^{Y42}, each R^{Y42} is independently selected from the group consisting of: H, C₁-C₆ alkyl, halogenated C₁-C₆ alkyl, C₁-C₆ hydroxylalkyl, C₁-C₆ alkoxyalkyl, C₃-C₆ cycloalkyl, 4-6-membered saturated heterocyclyl, -N(C₀₋₆ alkyl)(C₀₋₆ alkyl), -N(C₀₋₆ alkyl)CO(C₀₋₆ alkyl), -N(C₀₋₆ alkyl)CON(C₀₋₆ alkyl), -N(C₀₋₆ alkyl)SO₂(C₀₋₆ alkyl), -O(C₀₋₆ alkyl), -S(C₀₋₆ alkyl), -SO(C₀₋₆ alkyl), -SO₂(C₀₋₆ alkyl), -SO₂N(C₀₋₆ alkyl)(C₀₋₆ alkyl), -COO(C₀₋₆ alkyl), -OCO(C₀₋₆ alkyl), -CON(C₀₋₆ alkyl)(C₀₋₆ alkyl), -CO(C₀₋₆ alkyl), -N(C₀₋₆ alkyl)(4-6-membered heterocyclyl), -SO₂(4-6-membered heterocyclyl), -COO(4-6-membered heterocyclyl), -OCO(4-6-membered heterocyclyl), -CON(C₀₋₆ alkyl)( 4-6-membered heterocyclyl), and -CO(4-6-membered heterocyclyl); wherein H in said alkyl or heterocyclyl is optionally substituted with one or more groups selected from the group consisting of: halogen, cyano, hydroxyl, C₁-C₆ alkyl, halogenated C₁-C₆ alkyl, C₁-C₆ hydroxylalkyl, C₁-C₆ alkoxyalkyl, C₁-C₆ cyanoalkyl, -N(C₀₋₆ alkyl)(C₀₋₆ alkyl), -N(C₀₋₆ alkyl)CO(C₀₋₆ alkyl), -N(C₀₋₆ alkyl)CON(C₀₋₆ alkyl), -N(C₀₋₆ alkyl)SO₂(C₀₋₆ alkyl), -O(C₀₋₆ alkyl), -S(C₀₋₆ alkyl), -SO(C₀₋₆ alkyl), -SO₂(C₀₋₆ alkyl), -SO₂N(C₀₋₆ alkyl)(C₀₋₆ alkyl), -COO(C₀₋₆ alkyl), -OCO(C₀₋₆ alkyl), -CON(C₀₋₆ alkyl)(C₀₋₆ alkyl), and -CO(C₀₋₆ alkyl);
more preferably, R^{Y41} is selected from the group consisting of: -N(C₀₋₁₀ alkyl)(C₀₋₁₀ alkyl), -O(C₀₋₁₀ alkyl), and -COO(C₀₋₁₀ alkyl); wherein H in said alkyl is optionally substituted with one or more groups selected from the group consisting of: hydroxyl and C₁-C₃ alkoxy; or, R^{Y41} is selected from the group consisting of: 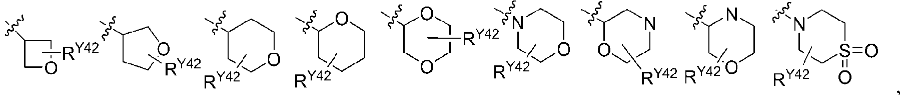 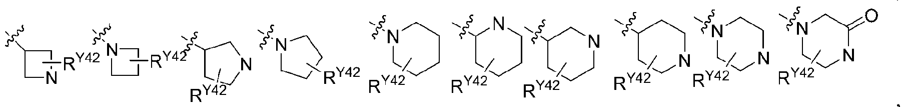 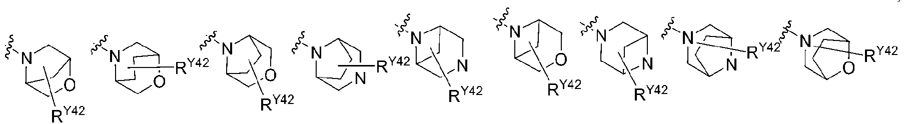 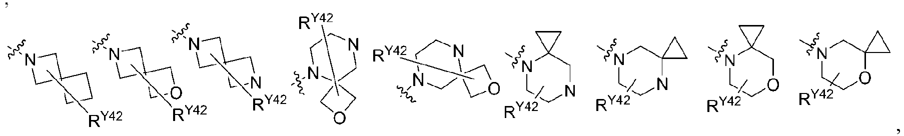 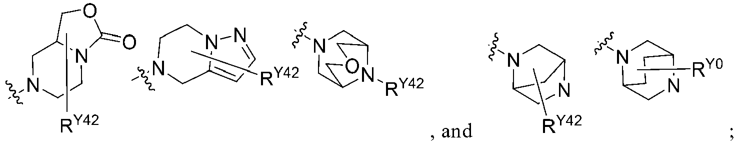
more preferably, each R^{Y42} is independently selected from the group consisting of: H, halogen, 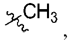 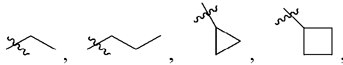 CF₃, CHF₂, CH₂F, 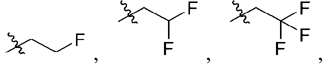 hydroxyl, 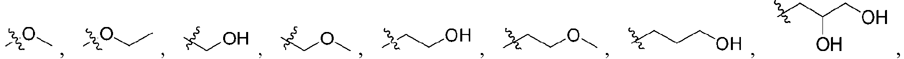 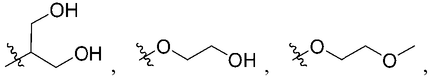 cyano, 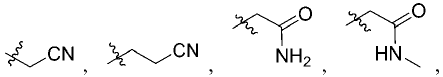 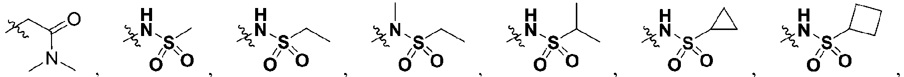 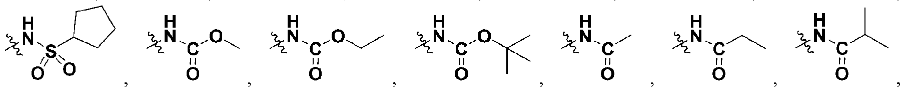 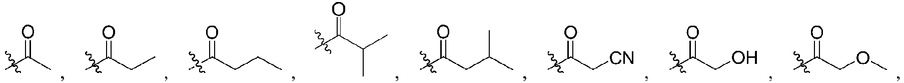 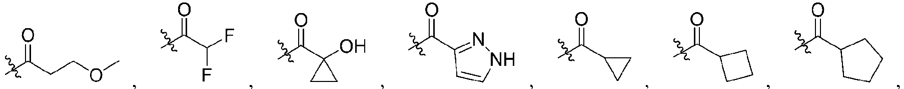 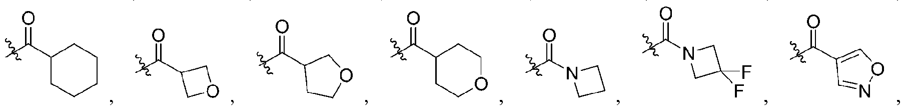 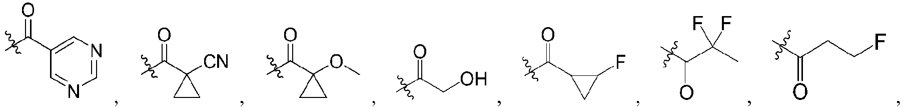 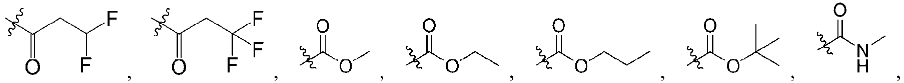 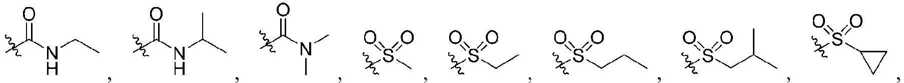 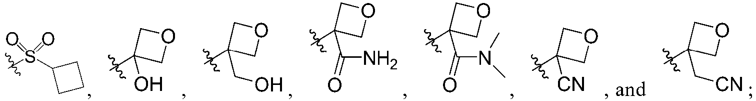
preferably, Y4 is selected from the group consisting of: 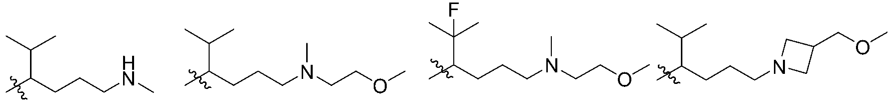 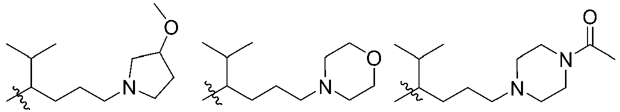 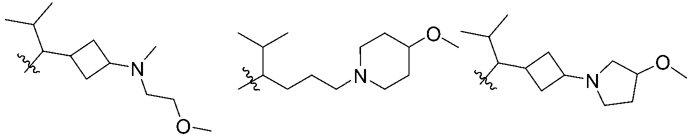 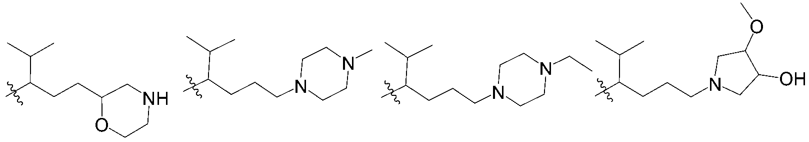 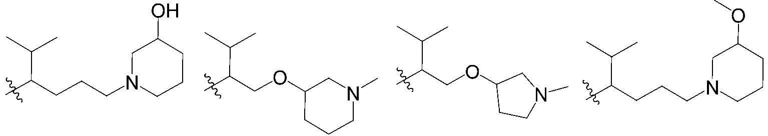 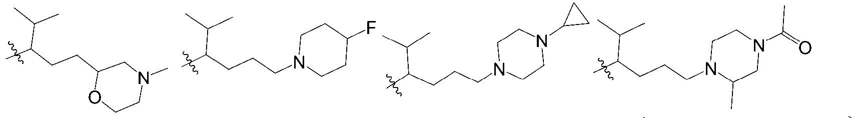 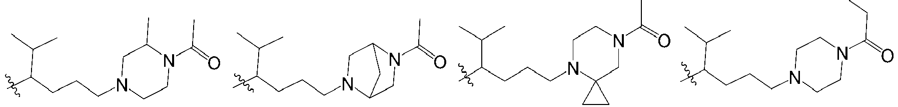 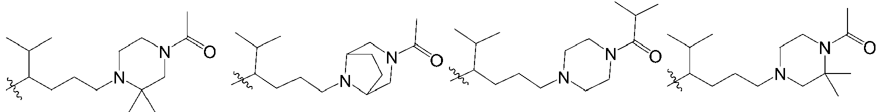 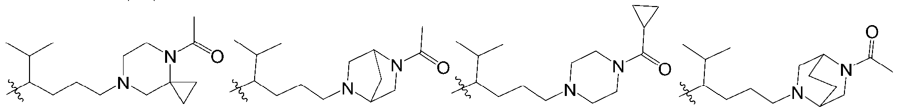 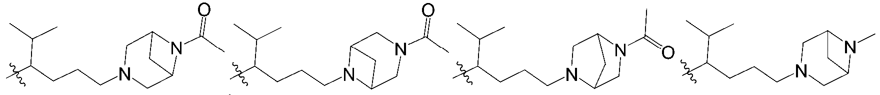 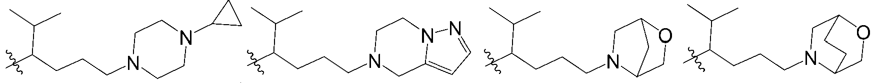 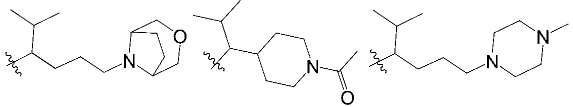 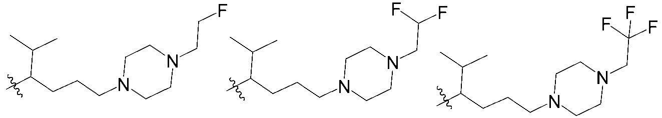 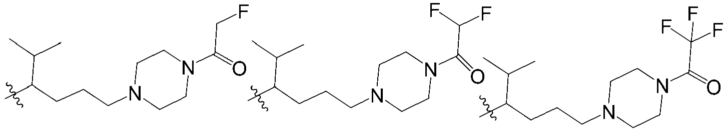 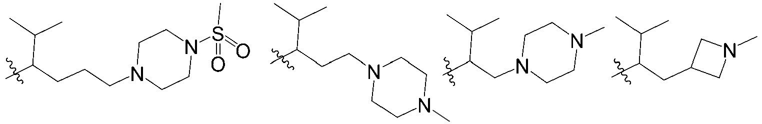 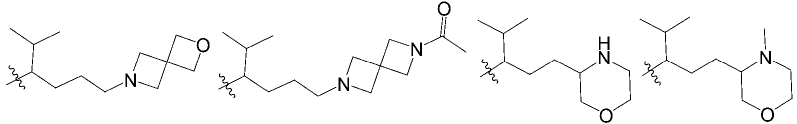 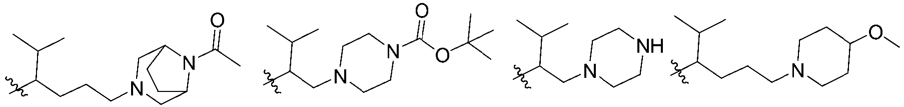 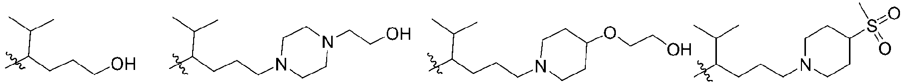 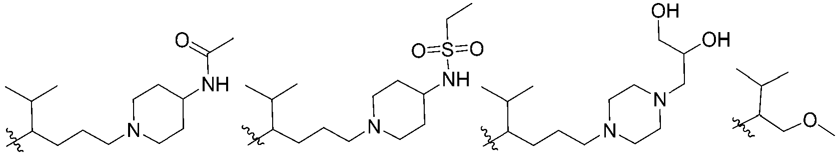 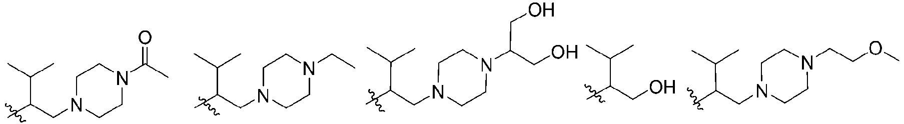 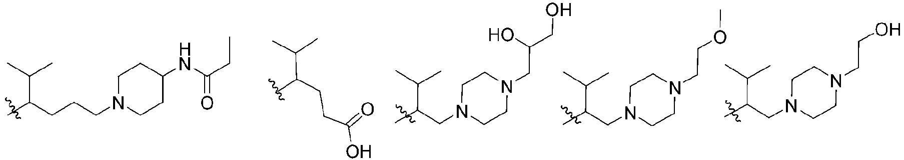 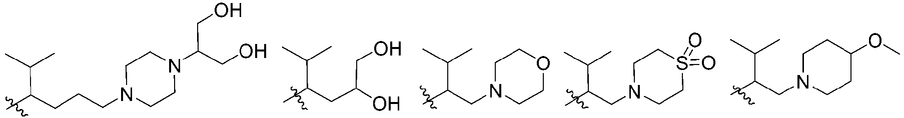 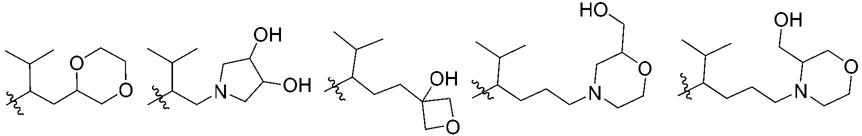 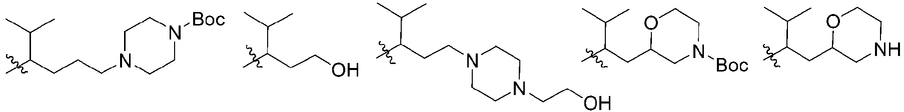 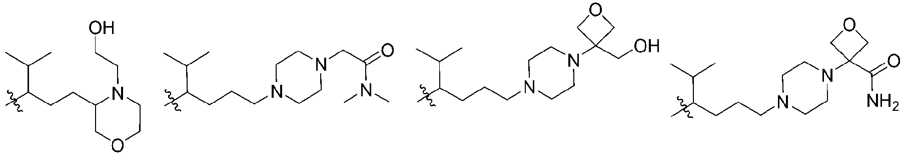 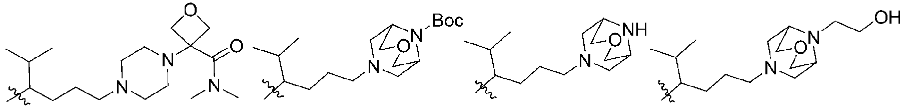 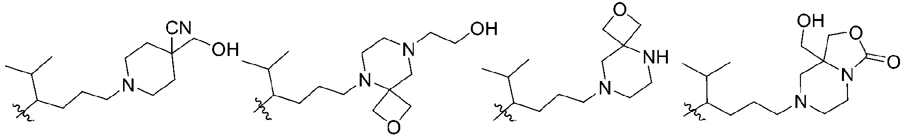 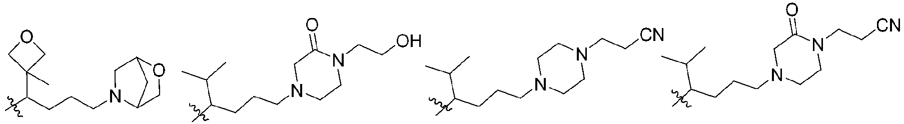 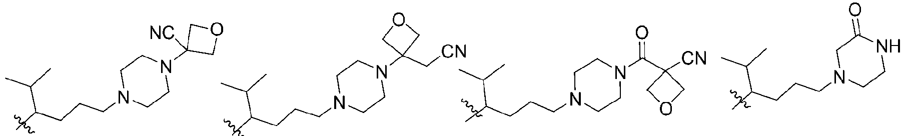 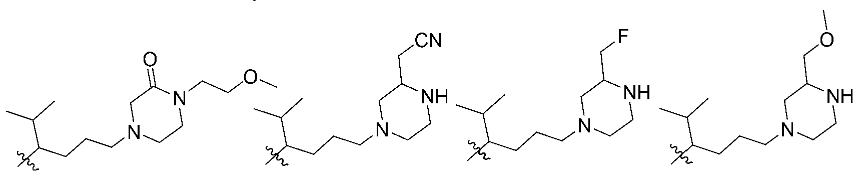 and 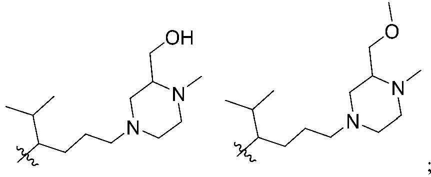 and
preferably, L₂ is a single bond.

17. The compound according to claim 12, wherein R^{Y501} and R^{Y502} are independently selected from the group consisting of: C₁-C₃ alkyl, and halogenated C₁-C₃ alkyl; or, R^{Y501} and R^{Y502}, together with the carbon atom attached thereto, form C₃-C₆ cycloalkyl, and said C₃-C₆ cycloalkyl may be optionally substituted with one or more groups selected from the group consisting of: halogen, cyano, hydroxyl, amino, C₁-C₃ alkoxy, and C₁-C₃ alkylamino;
preferably, R^{Y503} is H, and R^{Y503} is C₁-C₃ alkyl; or,
R^{Y303} and R^{Y504}, together with the nitrogen atom attached thereto, form a saturated heterocyclic ring, which is selected from the group consisting of: 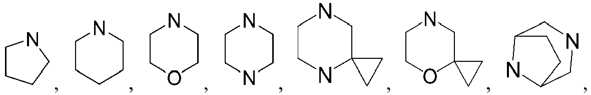 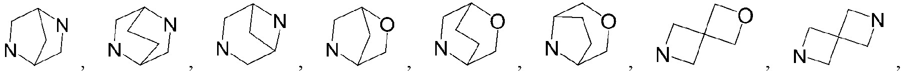 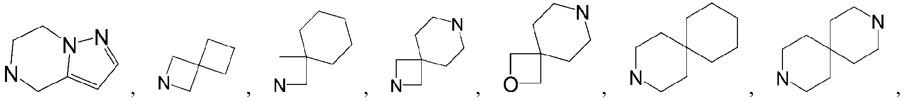 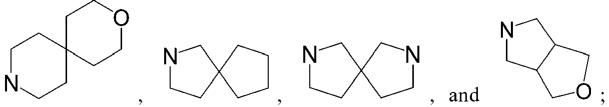 said heterocyclic ring is optionally substituted with one or more R^{Y505} groups;
R^{Y505} is selected from the group consisting of: halogen, cyano, nitro, azido, C₁-C₁₀ alkyl, C₂-C₁₀ alkenyl, C₂-C₁₀ alkynyl, -(C₀-C₆ alkylene)-(C₃-C₁₀ cycloalkyl), -(C₀-C₆ alkylene)-(phenyl), -(C₀-C₆ alkylene)-(4-10-membered heterocyclyl), halogenated C₁-C₁₀ alkyl, halogenated C₁-C₁₀ alkoxy, -N(C₀₋₁₀ alkyl)(C₀₋₁₀ alkyl), - N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -S(C₀₋₁₀ alkyl), -SO(C₀₋₁₀ alkyl), -SO₂(C₀₋₁₀ alkyl), -SO₂N(C₀₋₁₀ alkyl)(C₀₋₁₀ alkyl), -COO(C₀₋₁₀ alkyl), -OCO(C₀₋₁₀ alkyl), -CON(C₀₋₁₀ alkyl)(C₀₋₁₀ alkyl), -CO(C₀₋₁₀ alkyl), and -CO(C₃-C₁₀ cycloalkyl); wherein H in said C₀₋₁₀ alkyl, C₁-C₁₀ alkyl, C₂-C₁₀ alkenyl, C₂-C₁₀ alkynyl, C₀-C₆ alkylene, C₃-C₁₀ cycloalkyl, phenyl, or 4-10-membered heterocyclyl is optionally substituted with one or more groups selected from the group consisting of: halogen, cyano, nitro, azido, C₁-C₁₀ alkyl, halogenated C₁-C₁₀ alkyl, halogenated C₁-C₁₀ alkoxy, -N(C₀₋₁₀ alkyl)(C₀₋₁₀ alkyl), -N(C₀₋₁₀ alkyl)CO(C₀₋₁₀ alkyl), -N(C₀₋₁₀ alkyl)CON(C₀₋₁₀ alkyl), -N(C₀₋₁₀ alkyl)SO₂(C₀₋₁₀ alkyl), -O(C₀₋₁₀ alkyl), -S(C₀₋₁₀ alkyl), -SO(C₀₋₁₀ alkyl), -SO₂(C₀₋₁₀ alkyl), -SO₂N(C₀₋₁₀ alkyl)(C₀₋₁₀ alkyl), -COO(C₀₋₁₀ alkyl), -OCO(C₀₋₁₀ alkyl), -CON(C₀₋₁₀ alkyl)(C₀₋₁₀ alkyl), and -CO(C₀₋₁₀ alkyl); and
more preferably, Y5 is selected from the group consisting of: 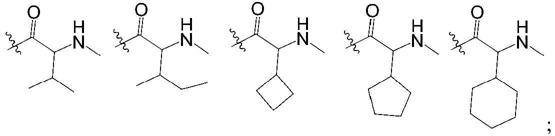
preferably, said L₂ is C(O).

18. The compound according to claim 1, wherein said compound is selected from the following structures: 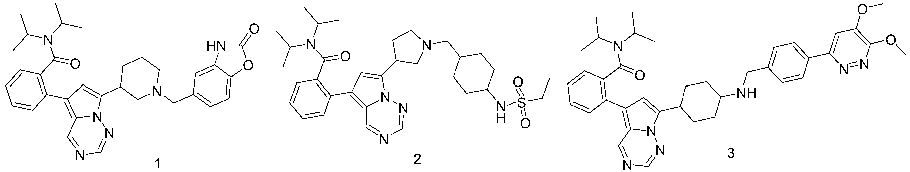 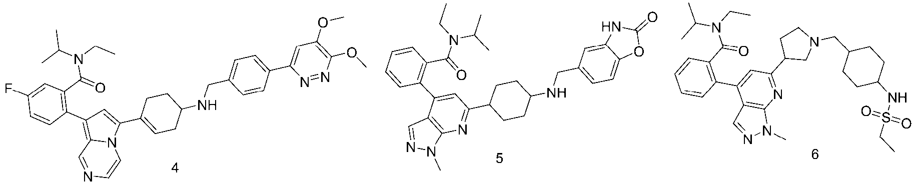 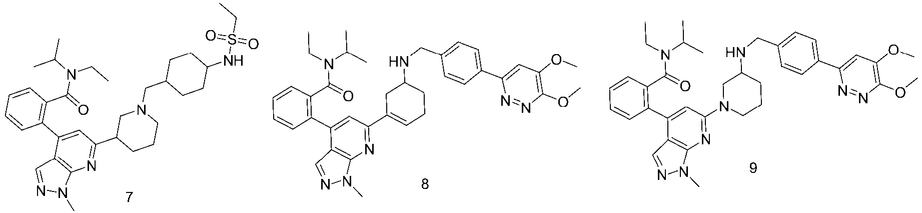 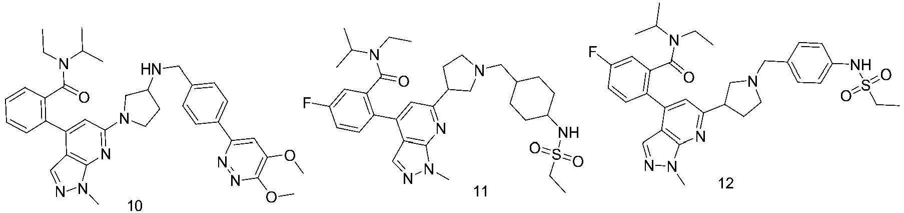 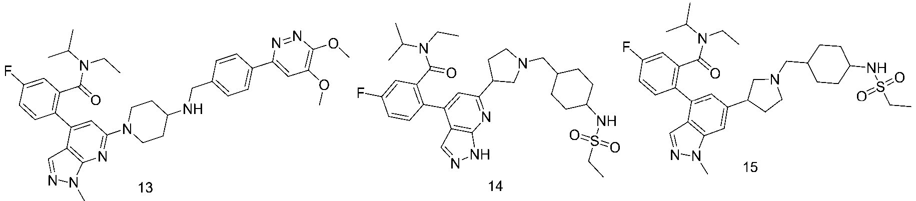 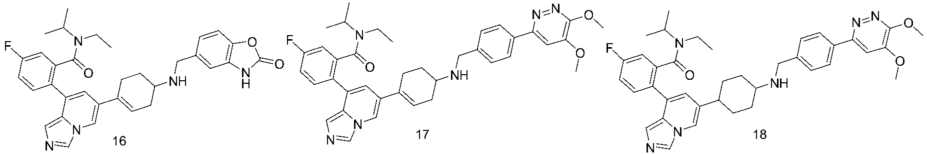 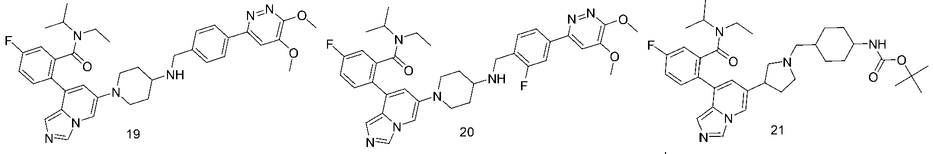 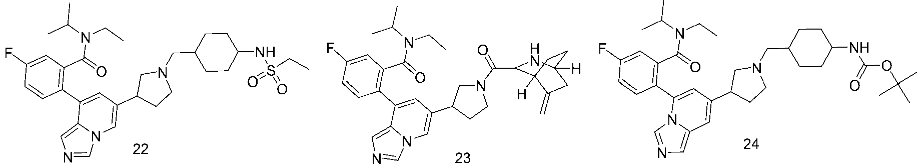 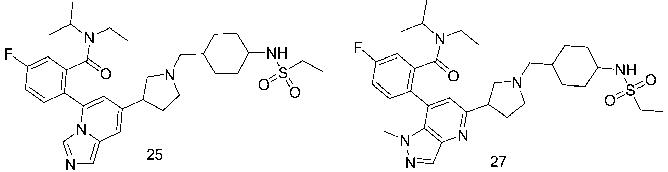 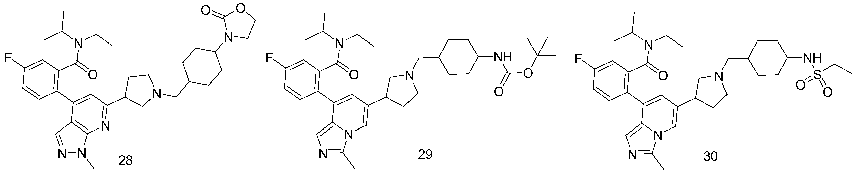 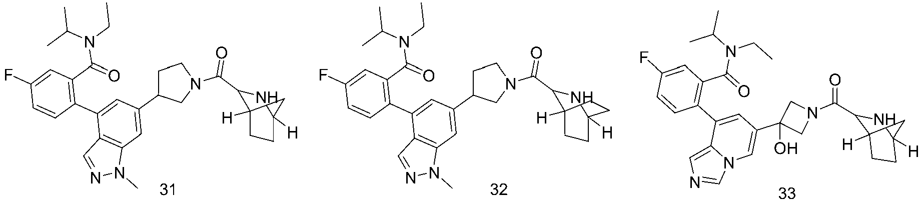 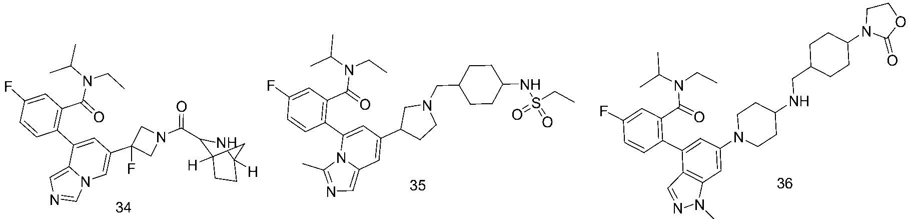 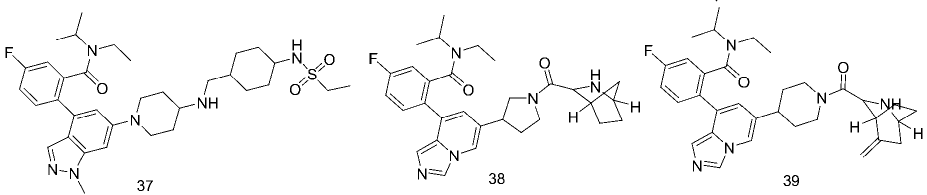 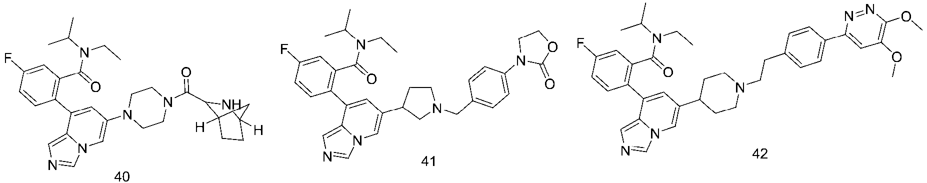 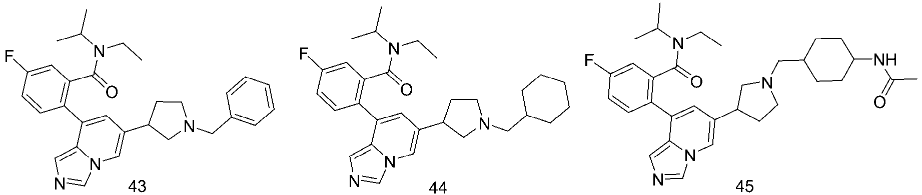 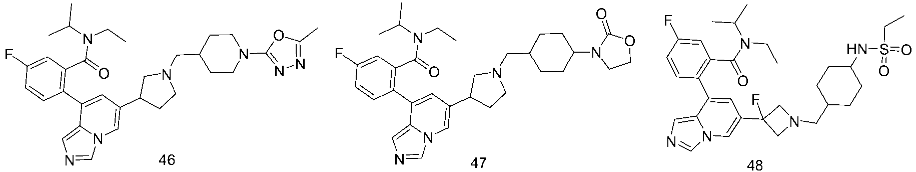 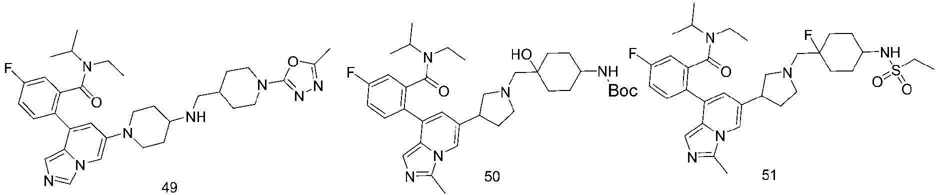 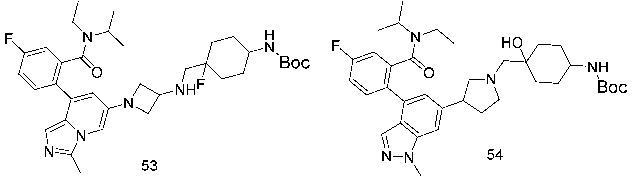 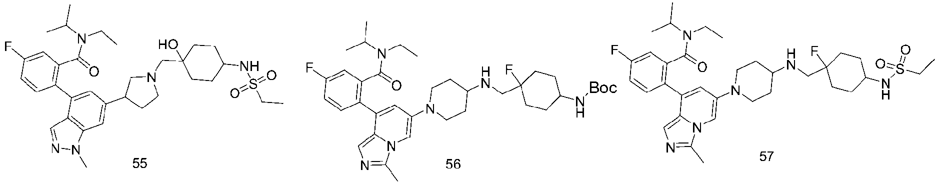 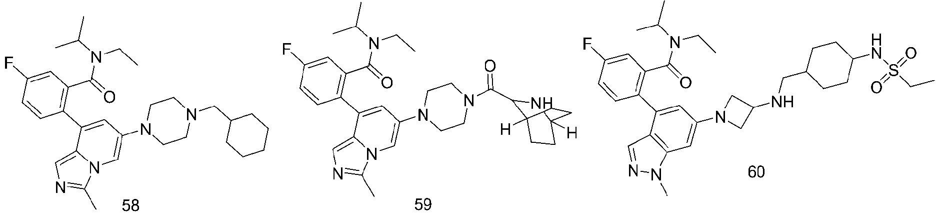 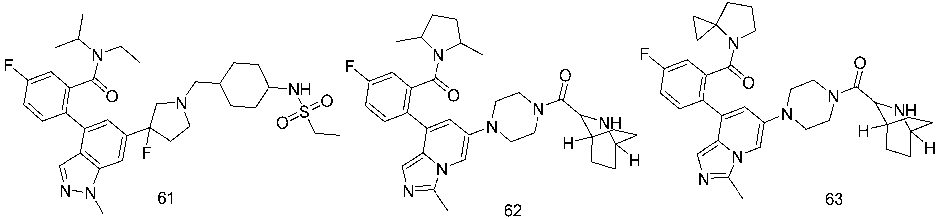 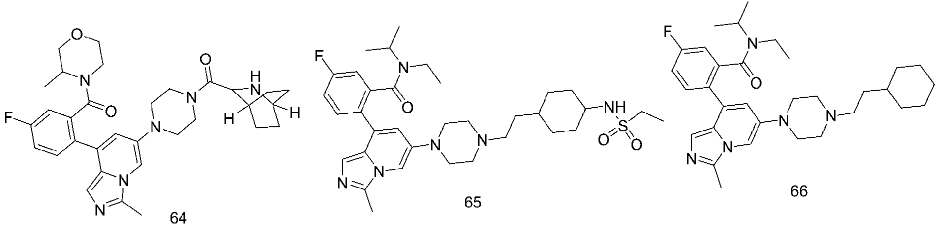 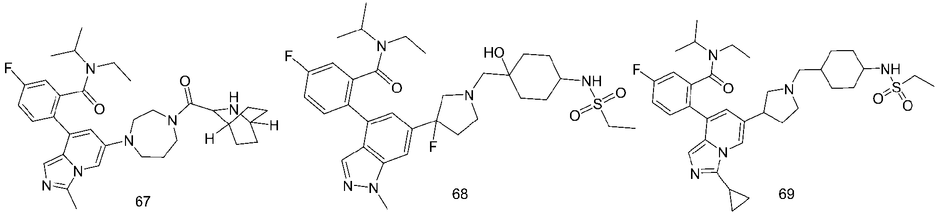 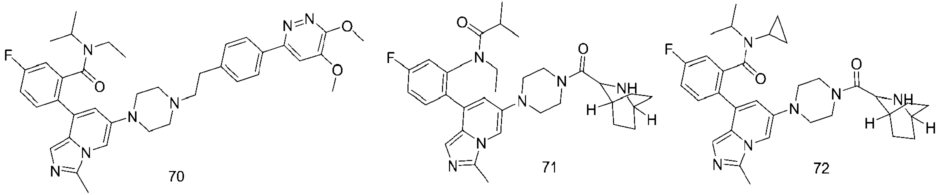 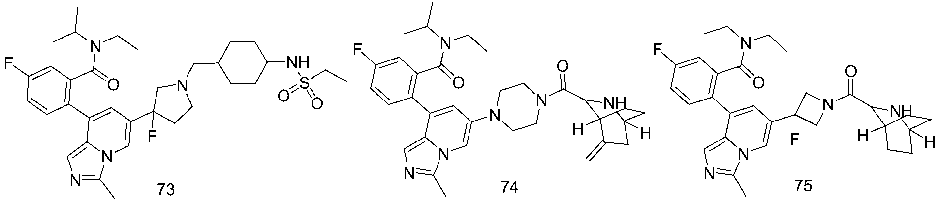 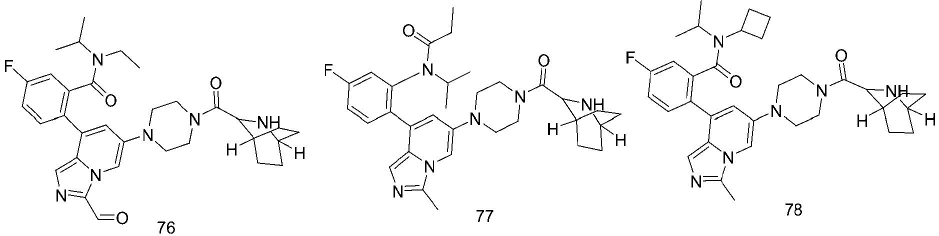 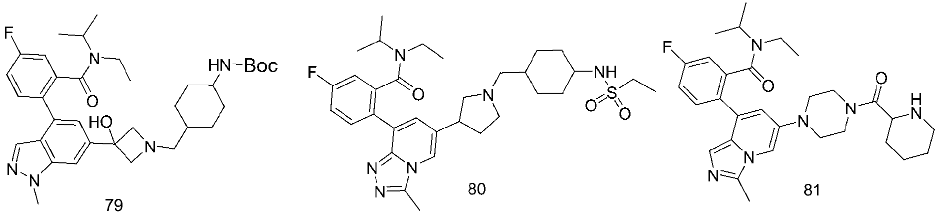 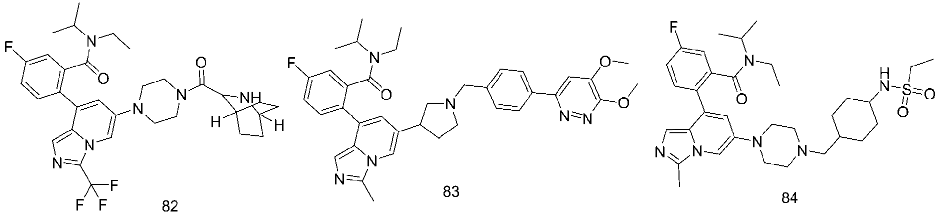 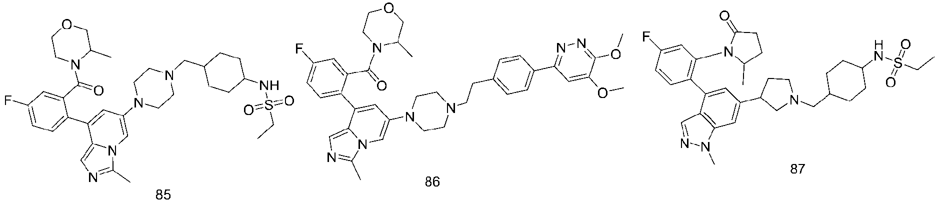 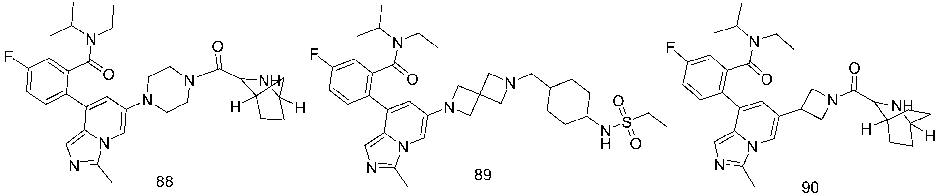 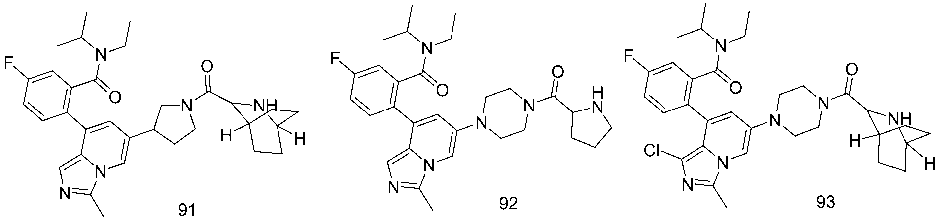 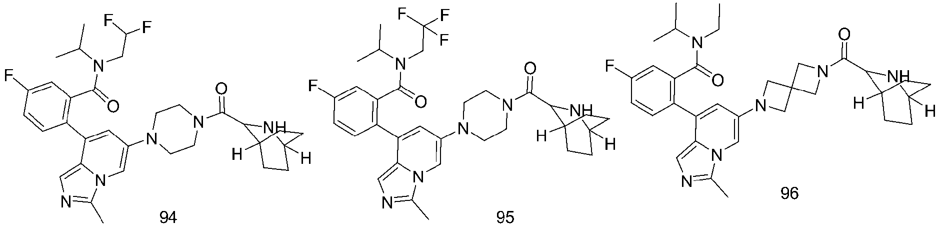 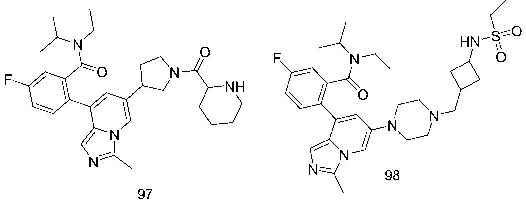 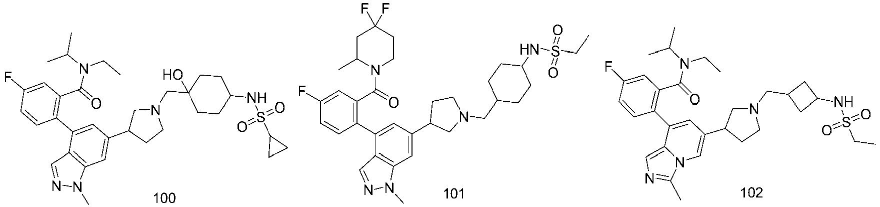 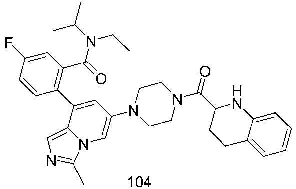 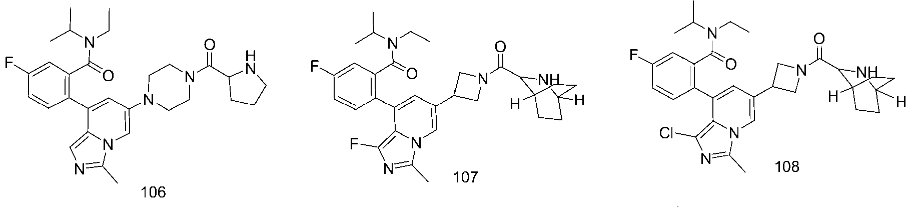 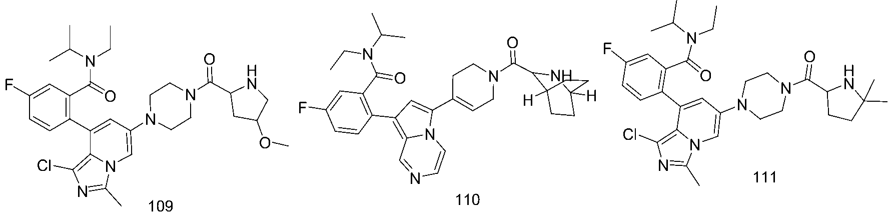 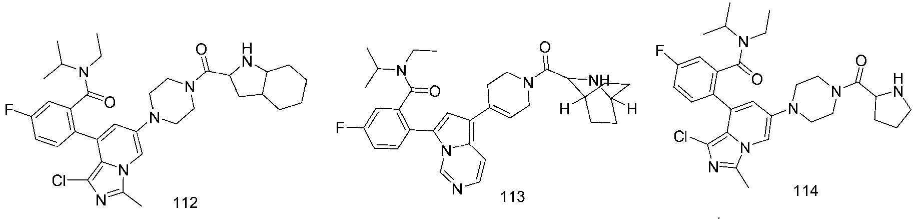 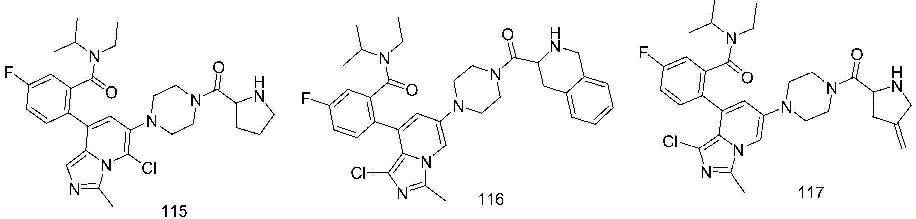 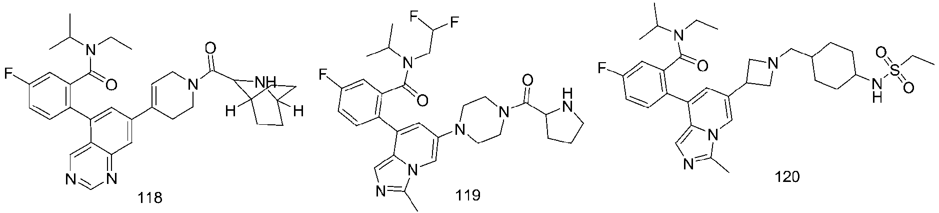 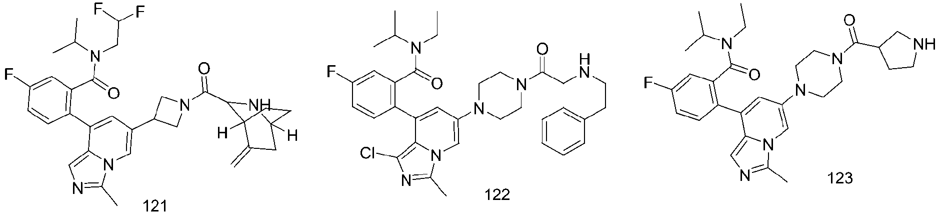 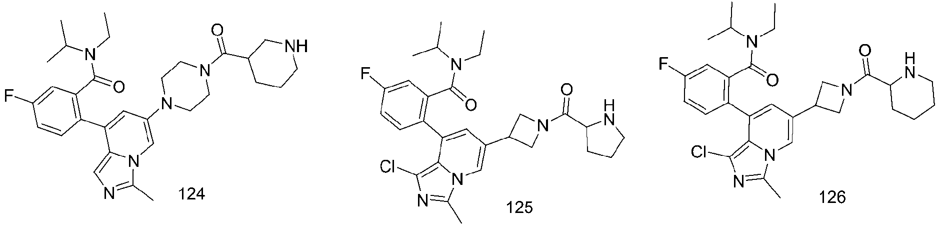 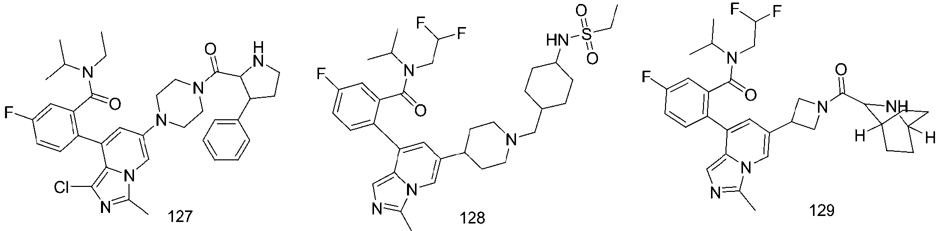 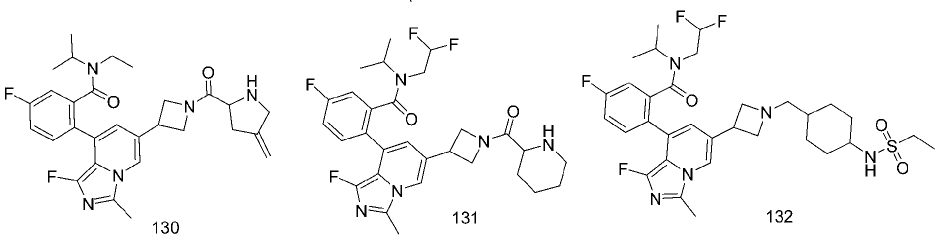 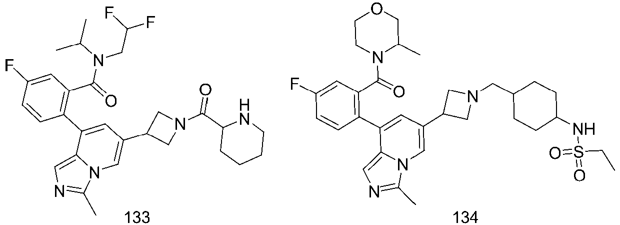 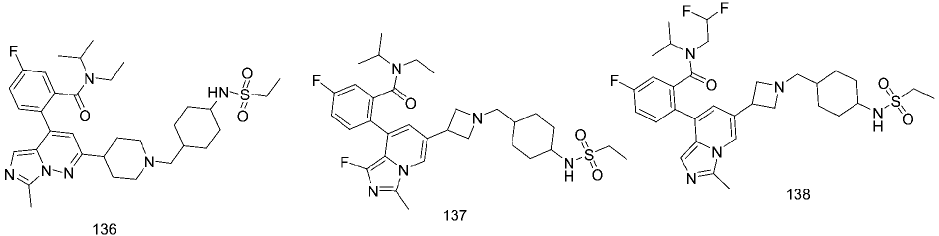 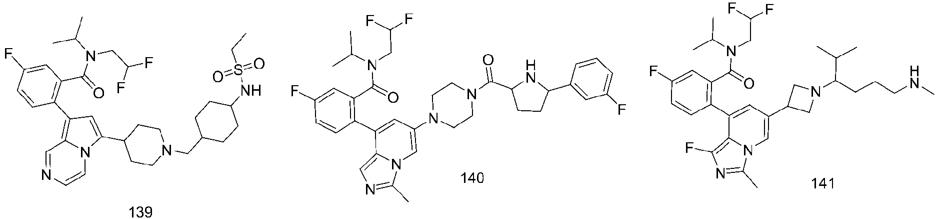 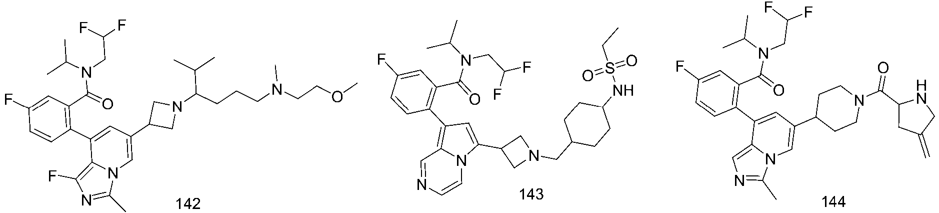 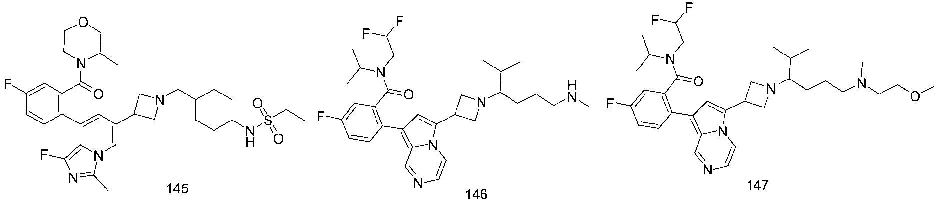 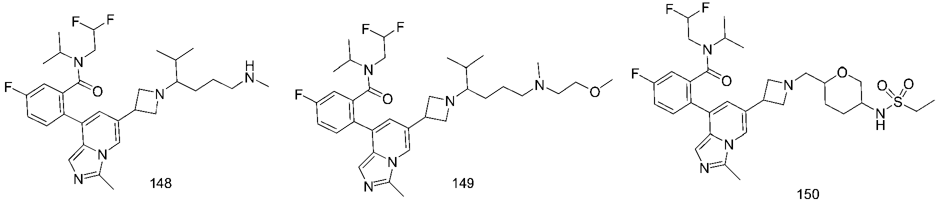 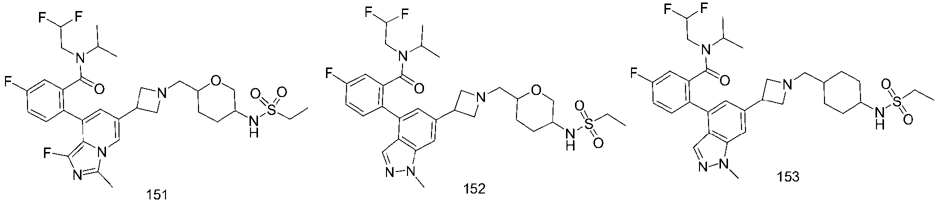 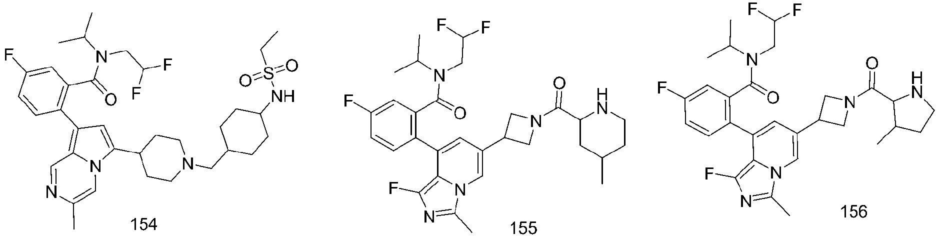 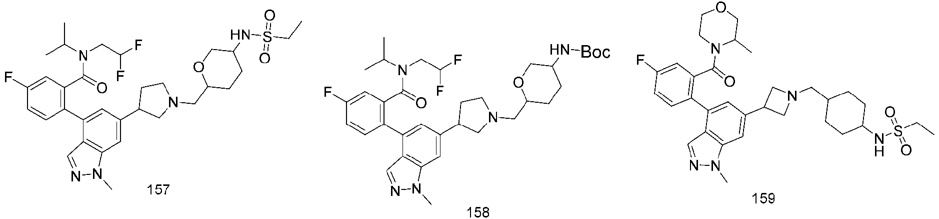 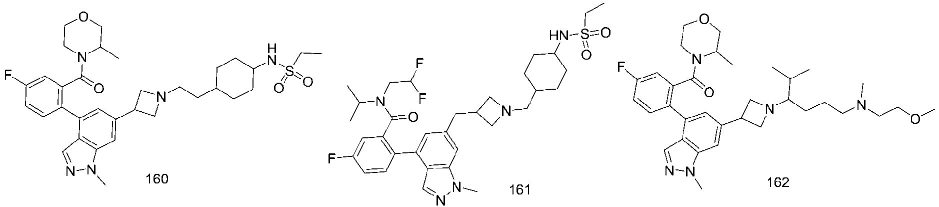 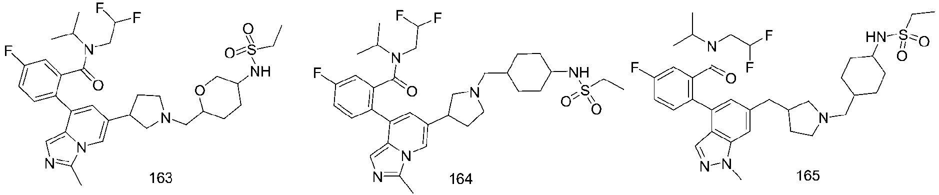 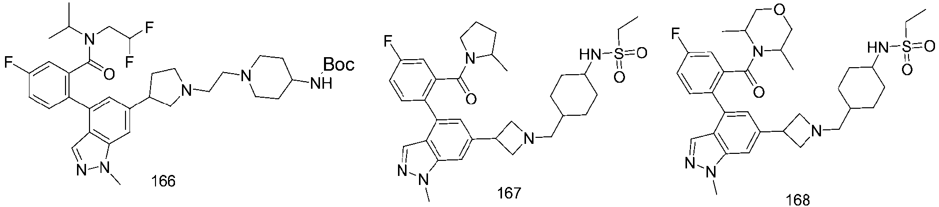 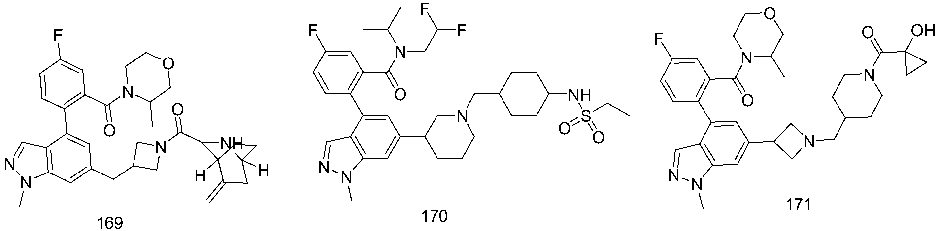 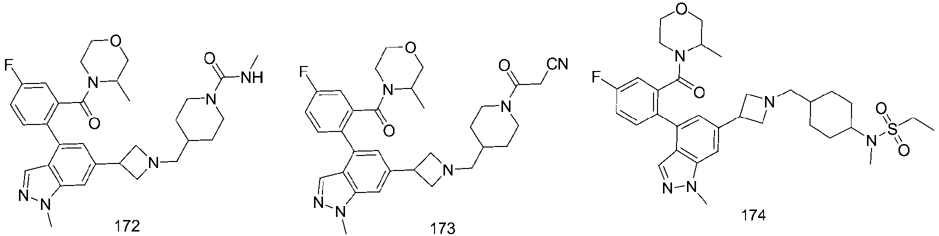 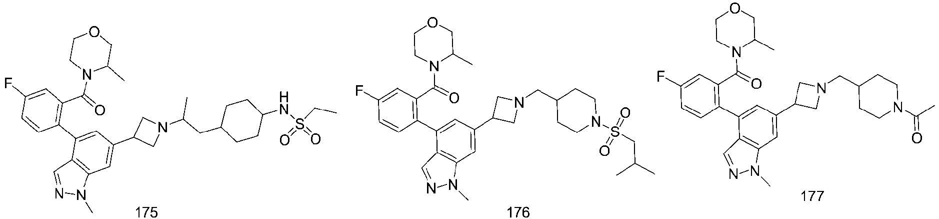 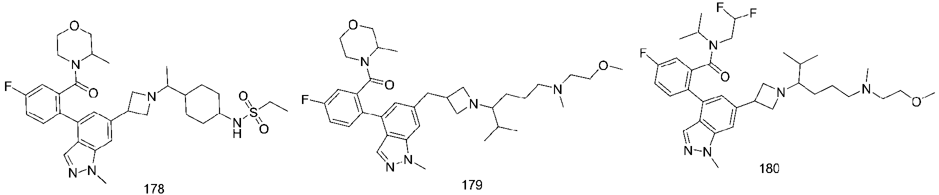 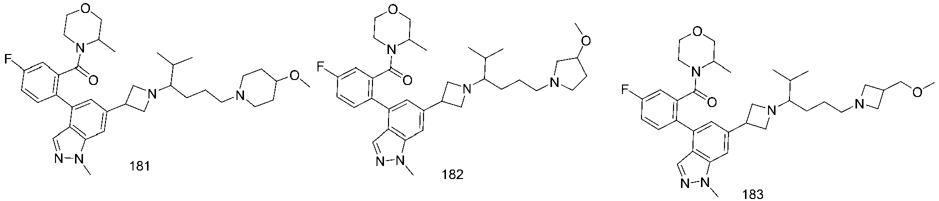 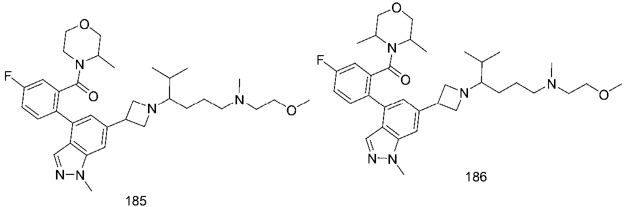 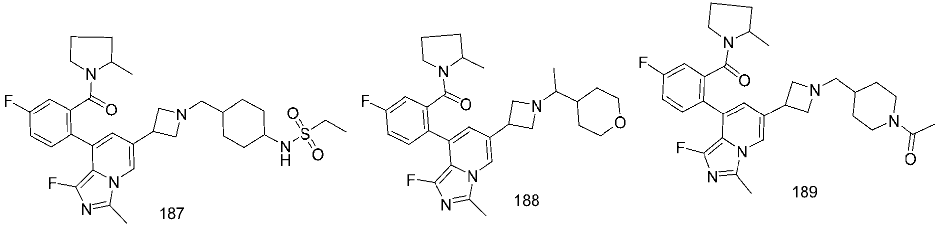 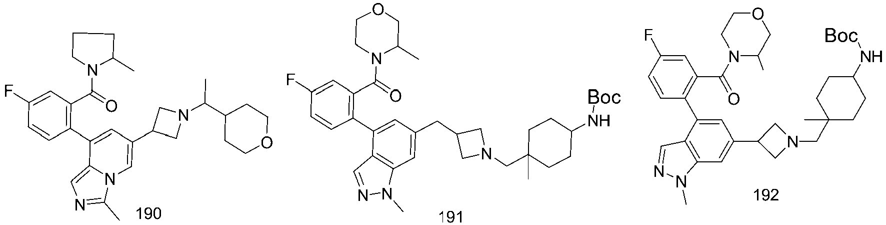 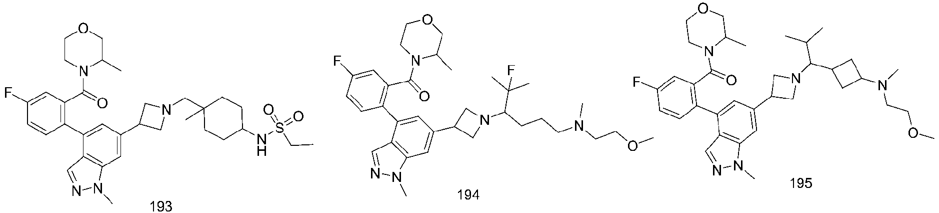 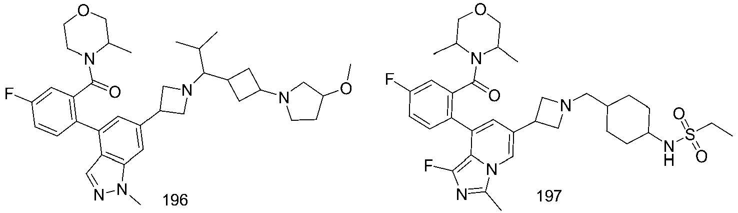 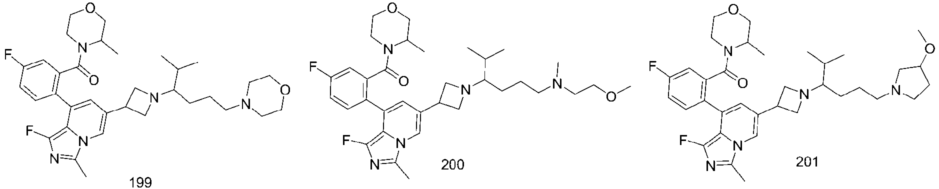 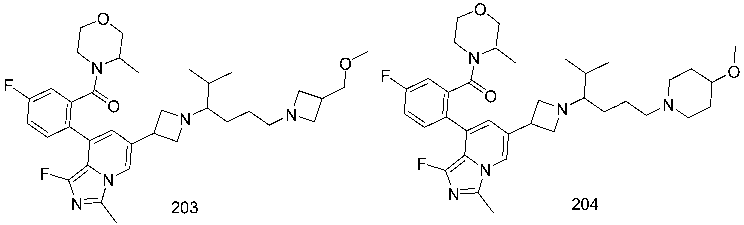 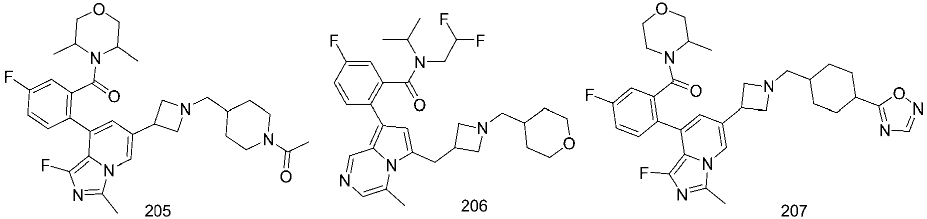 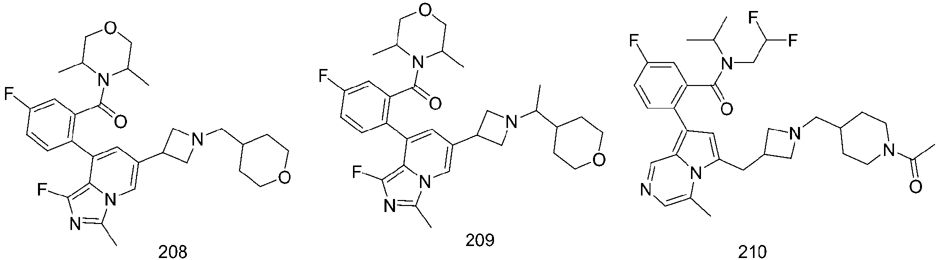 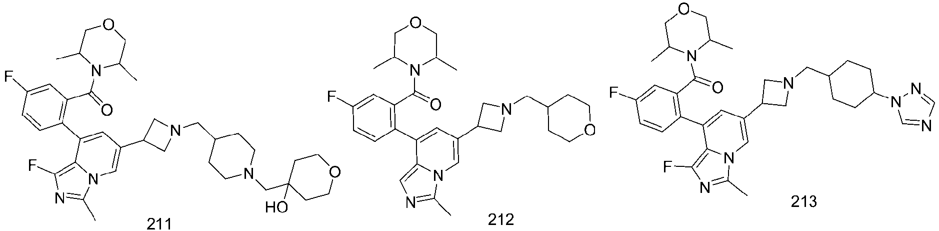 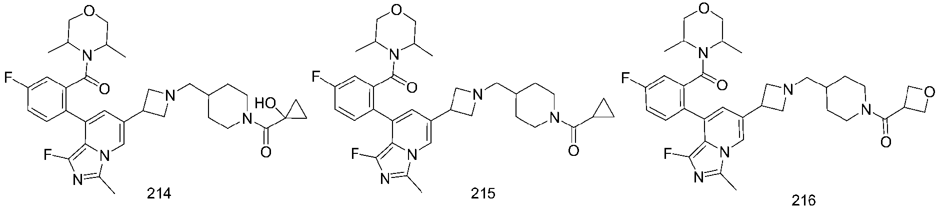 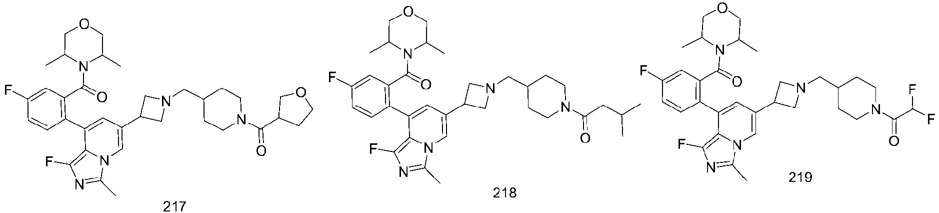 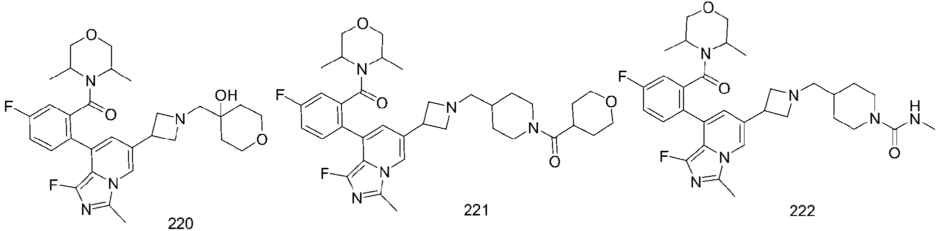 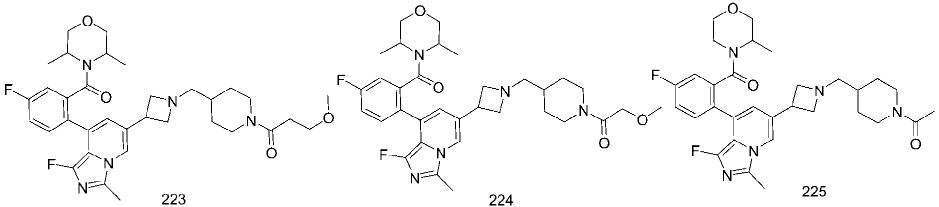 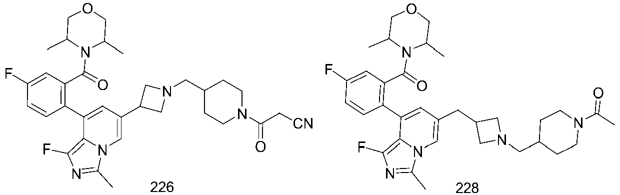 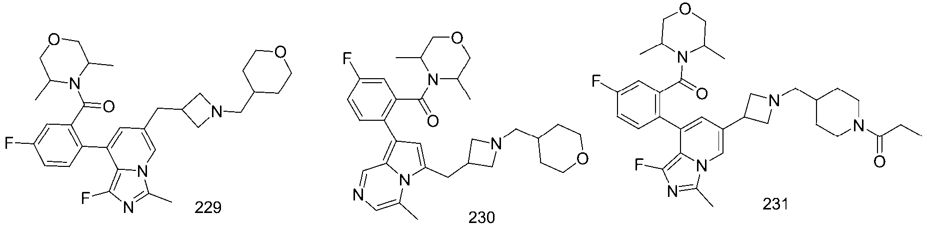 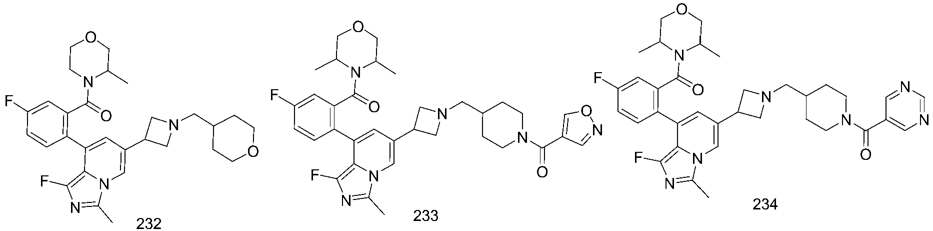 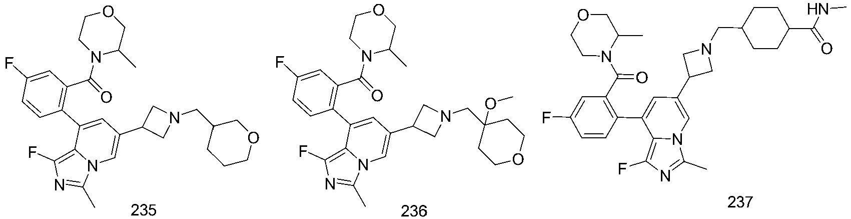 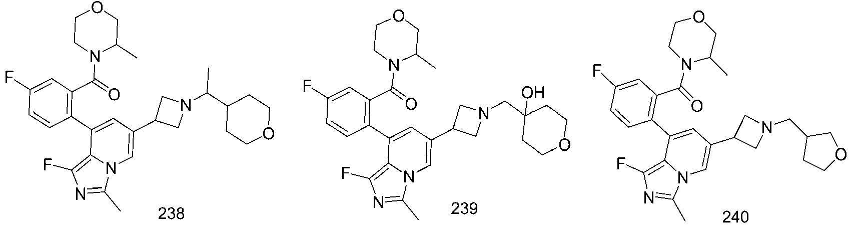 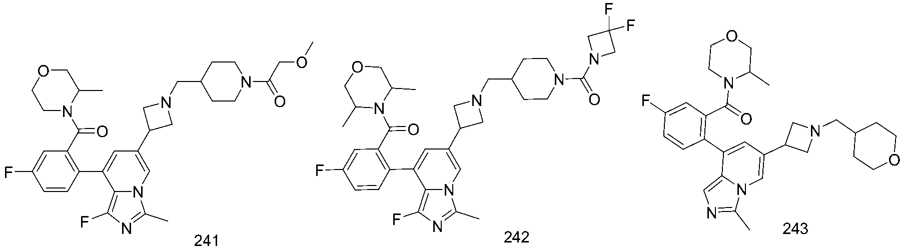 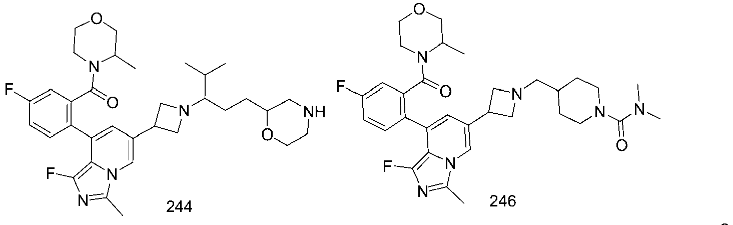 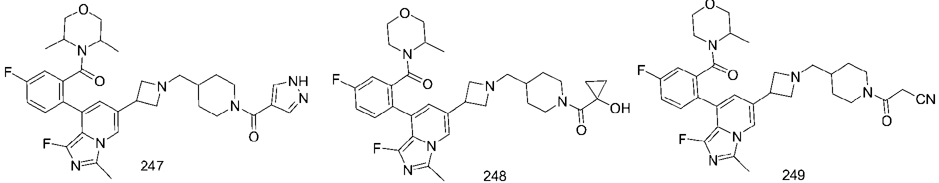 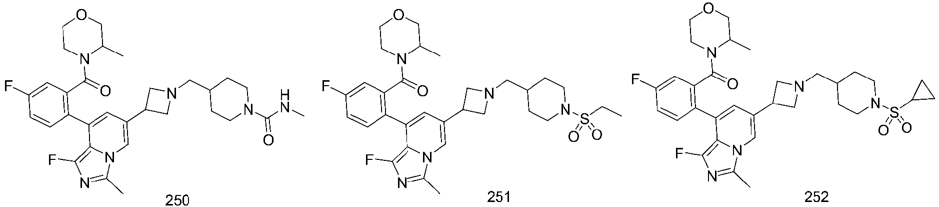 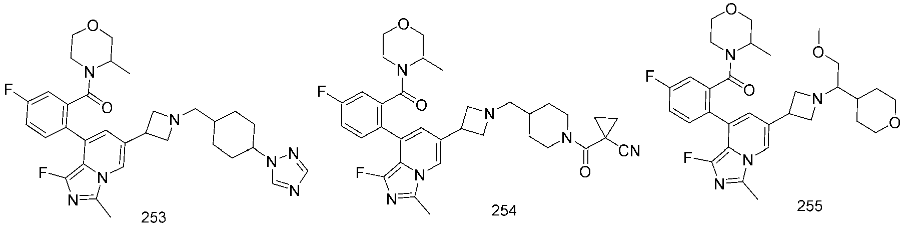 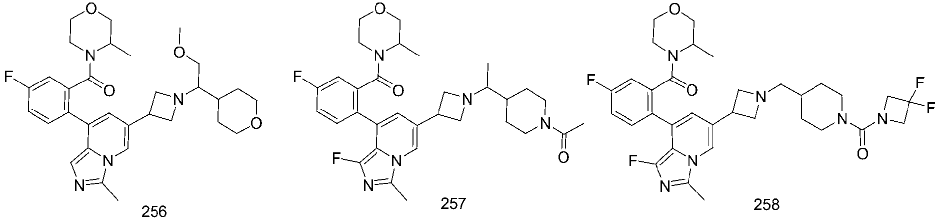 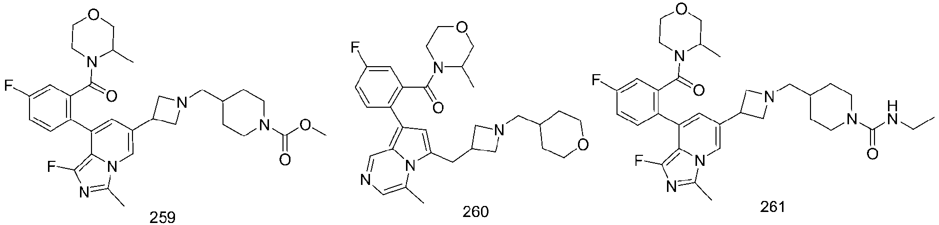 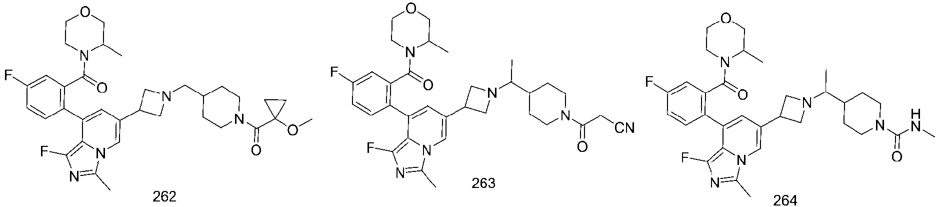 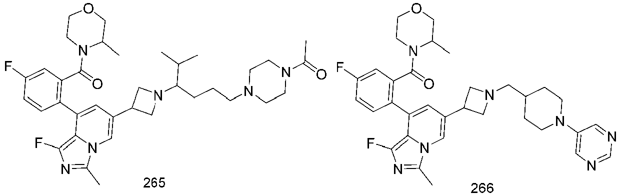 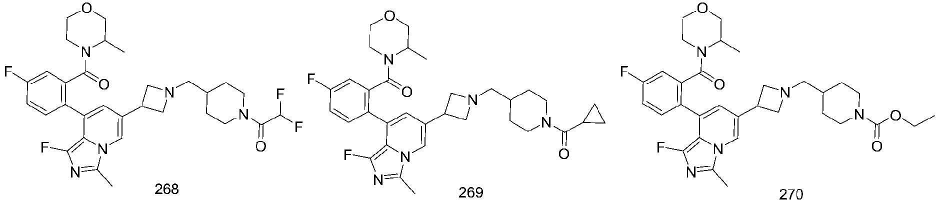 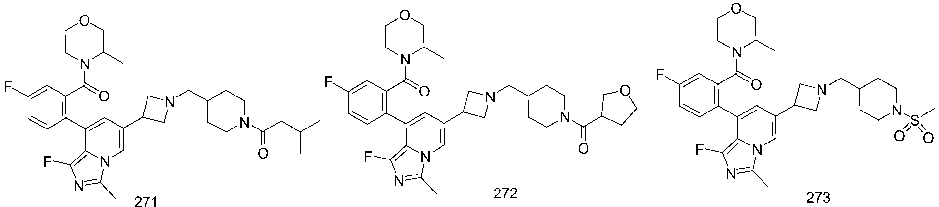 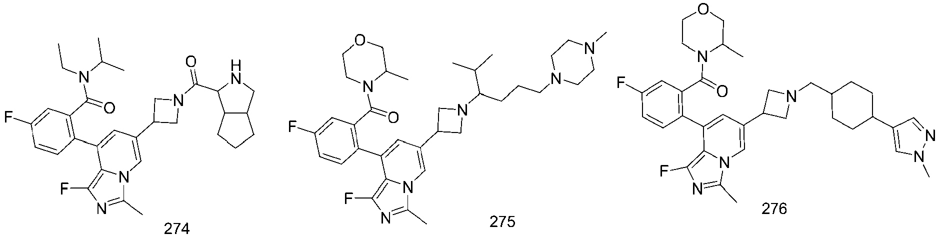 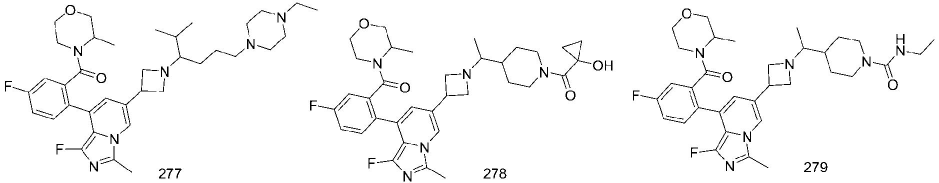 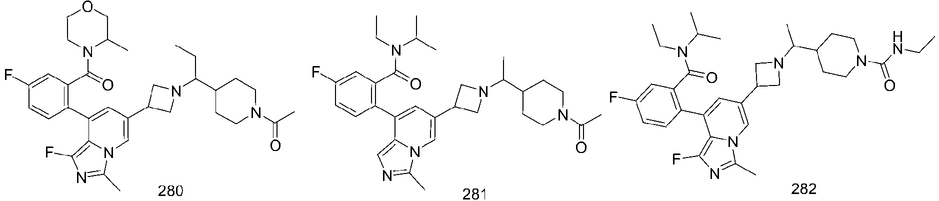 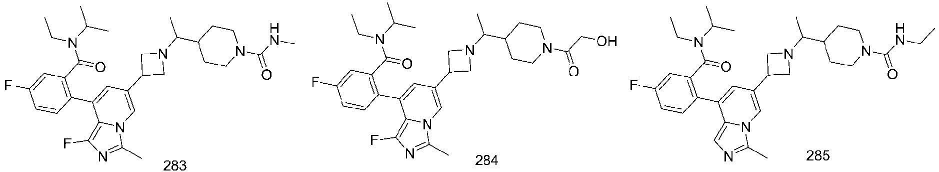 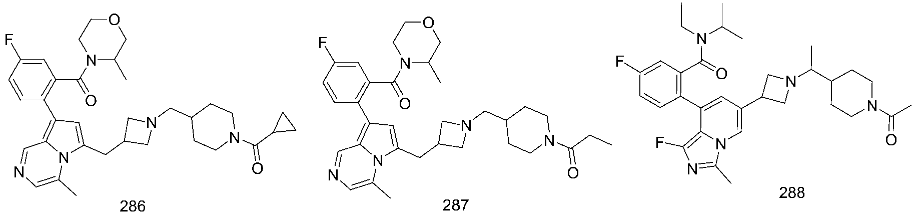 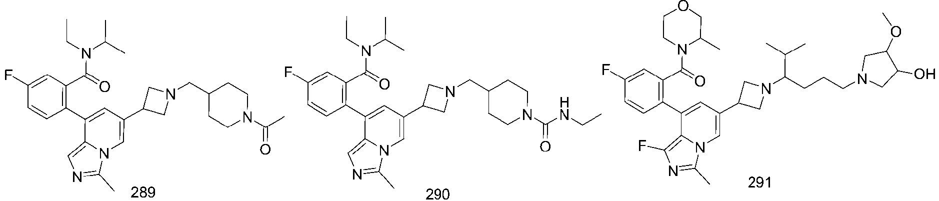 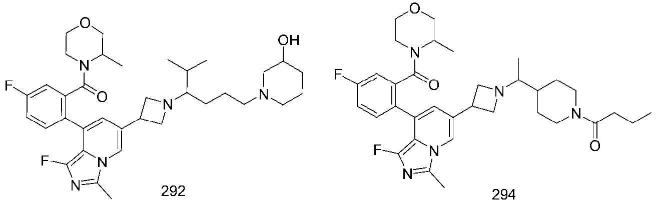 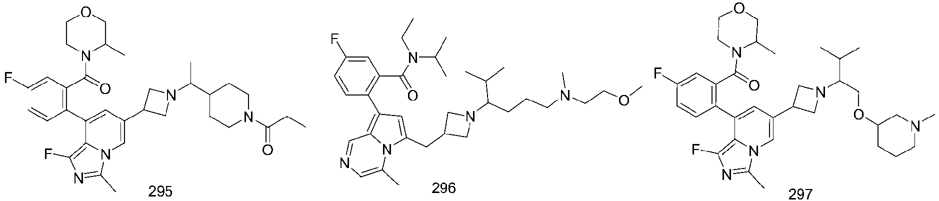 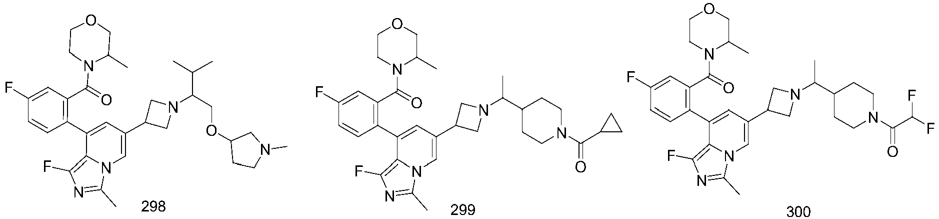 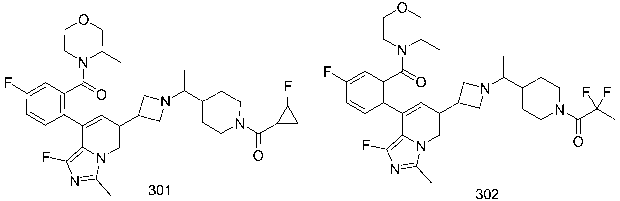 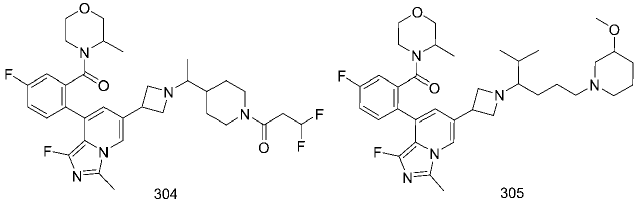 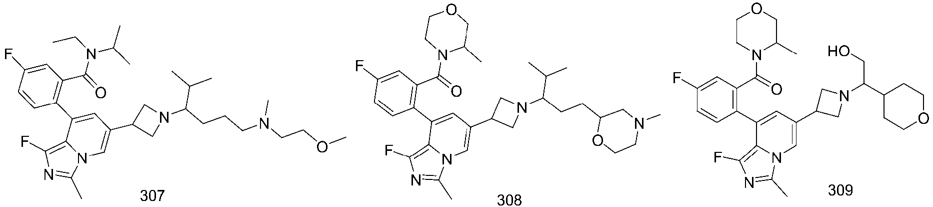 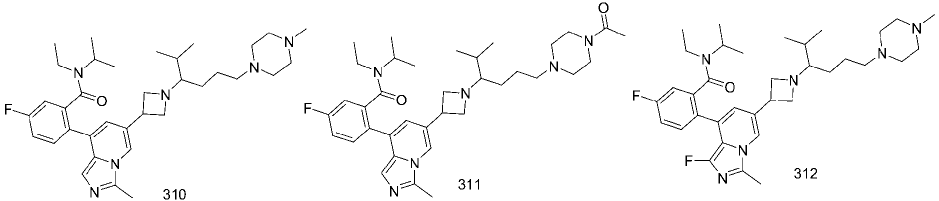 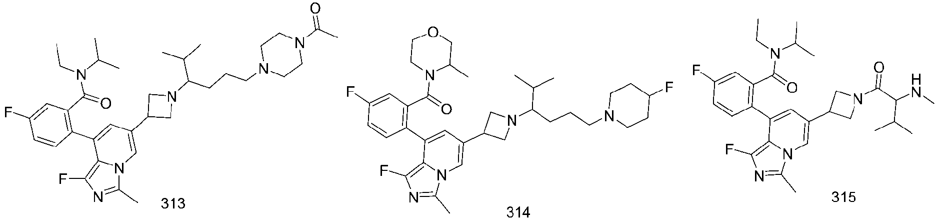 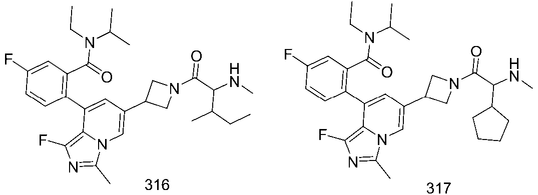 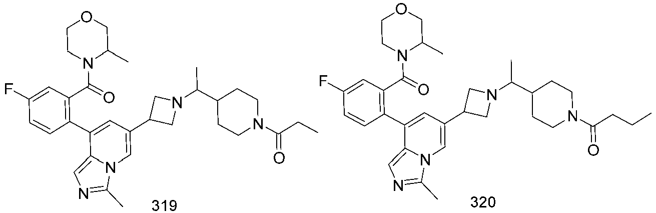 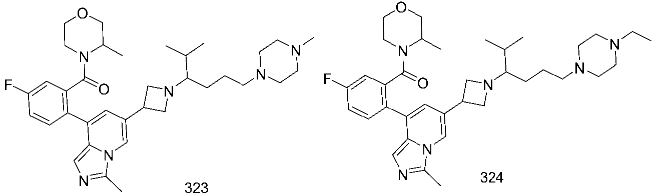 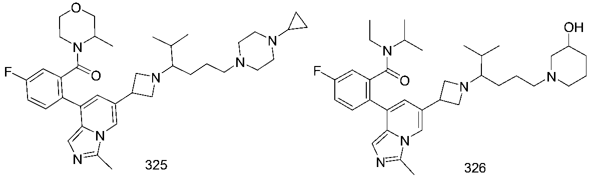 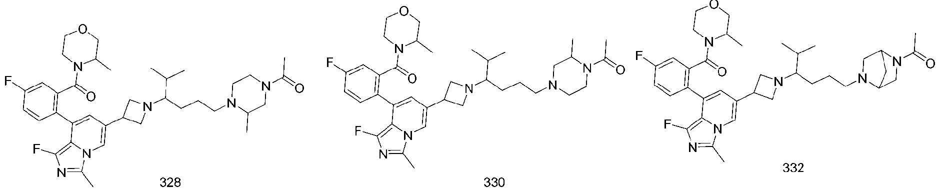 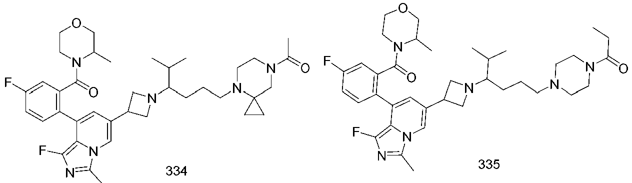 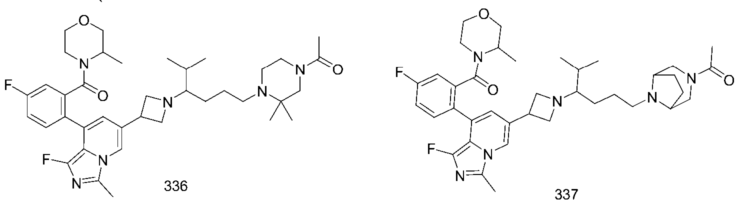 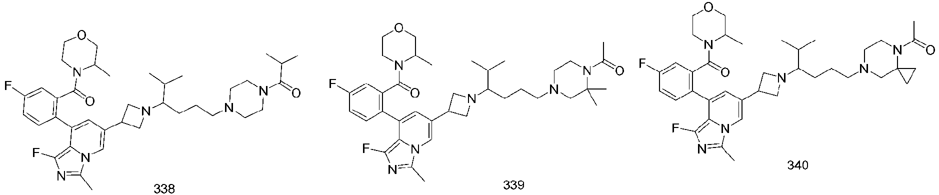 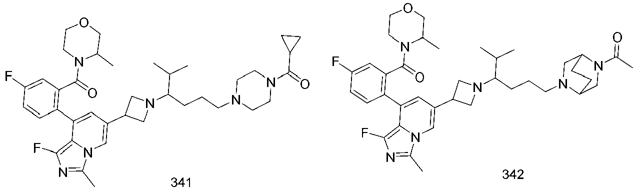 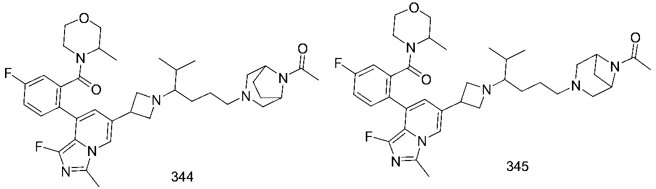 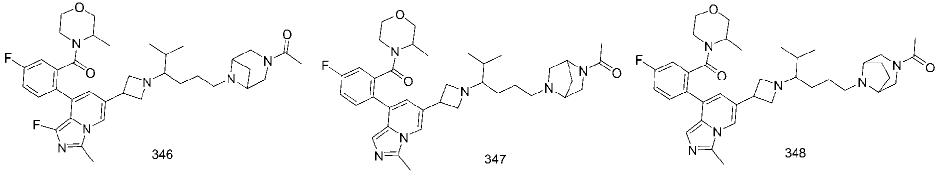 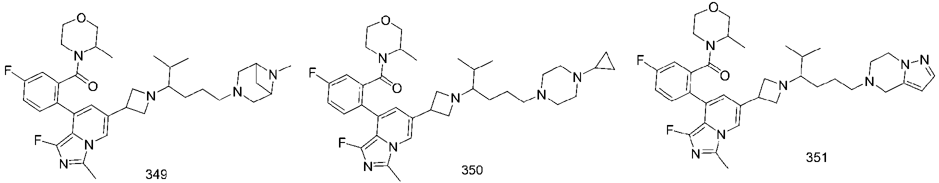 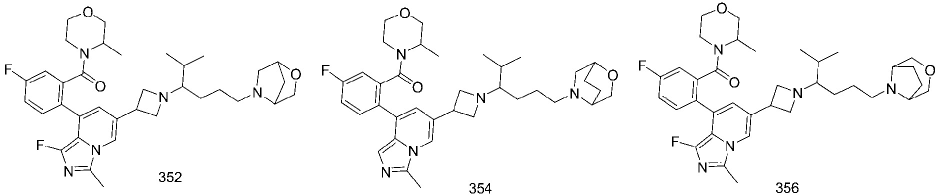 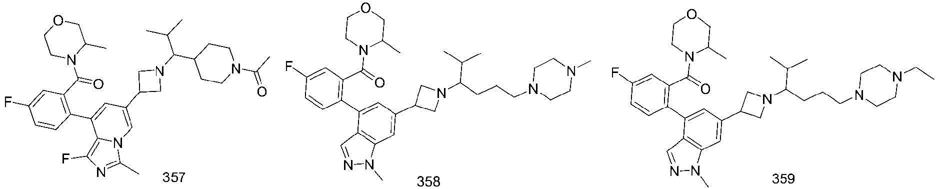 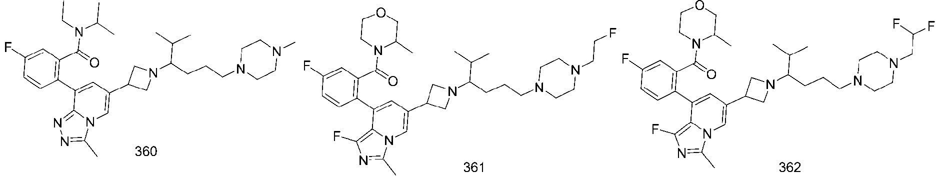 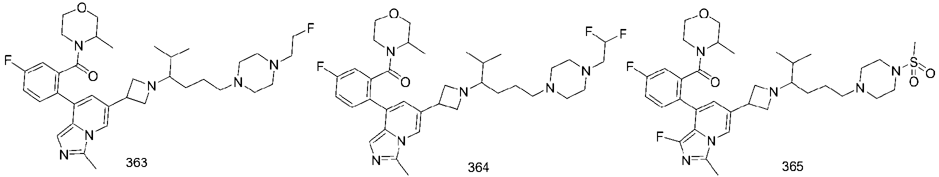 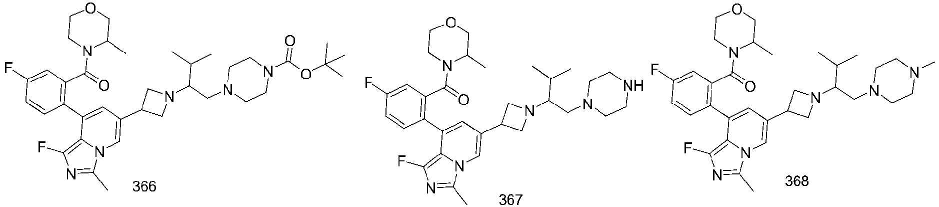 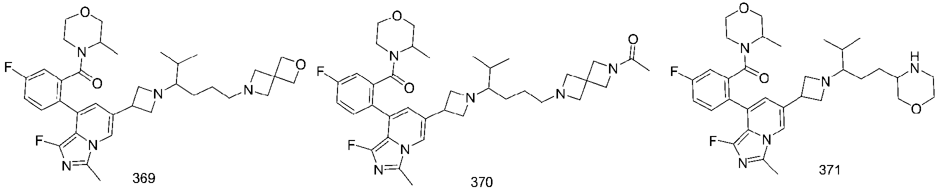 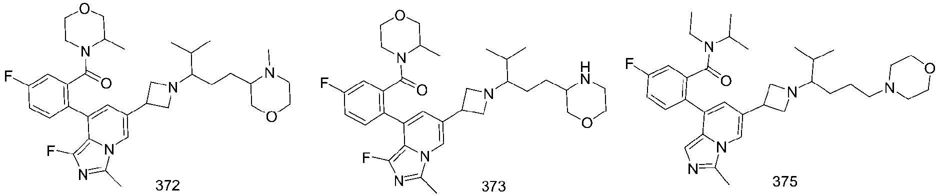 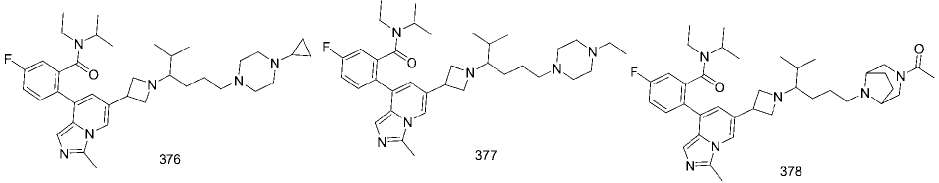 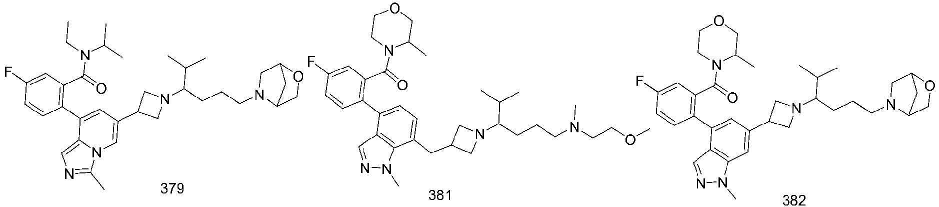 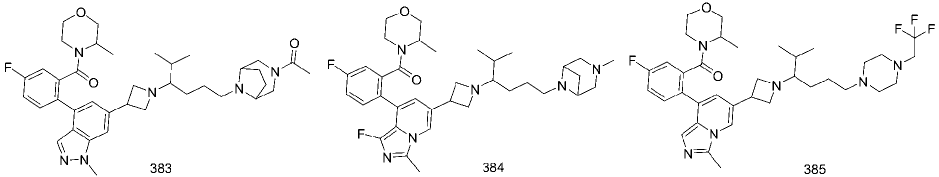 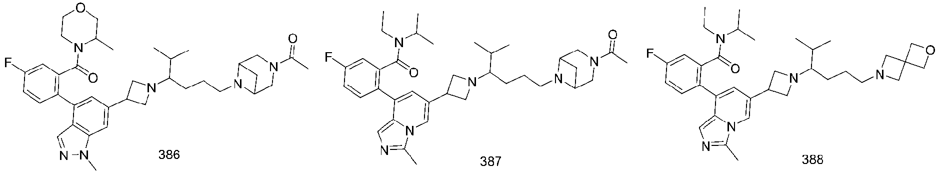 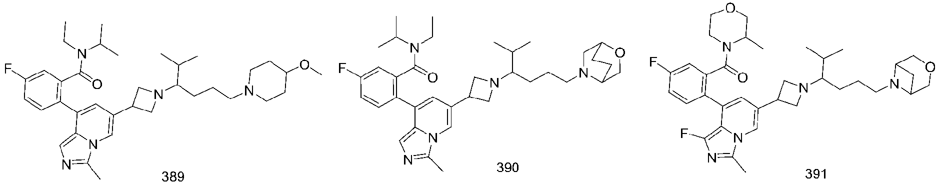 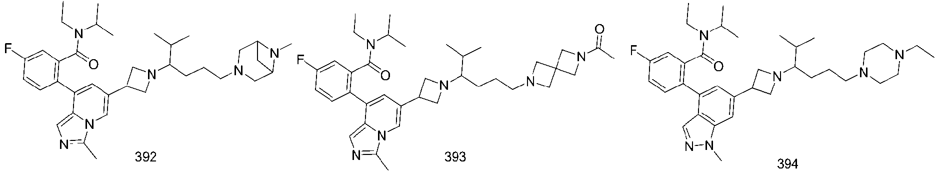 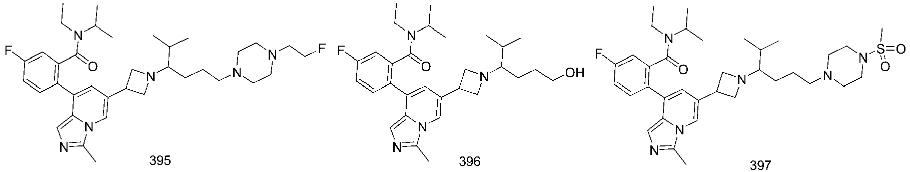 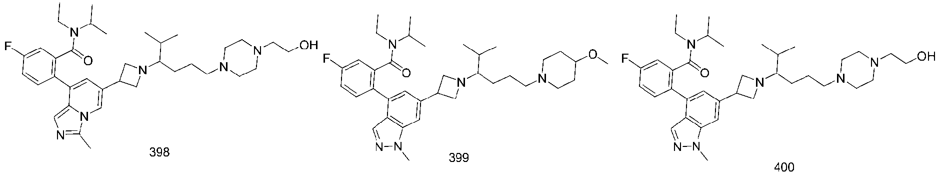 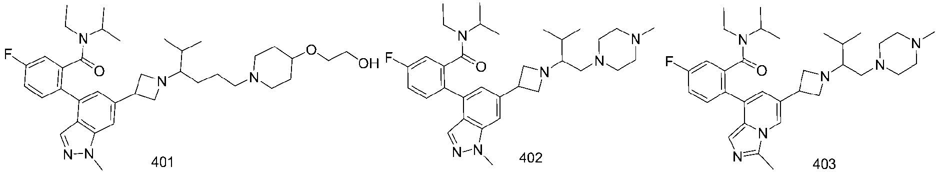 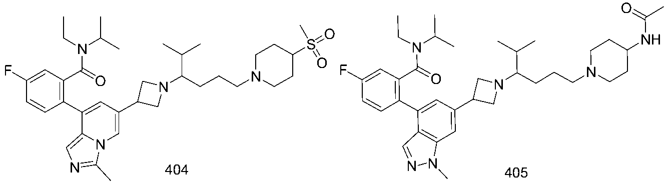 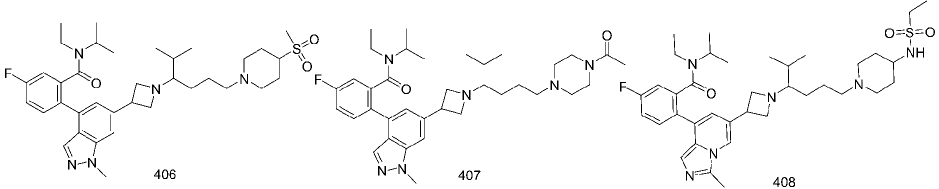 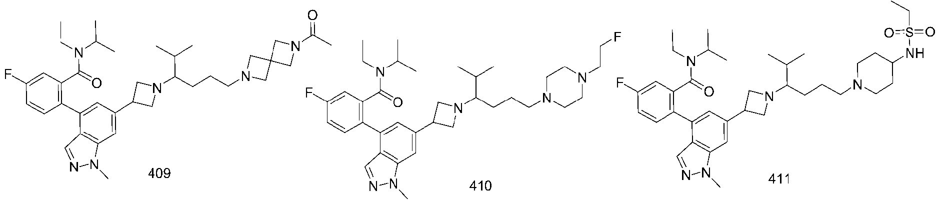 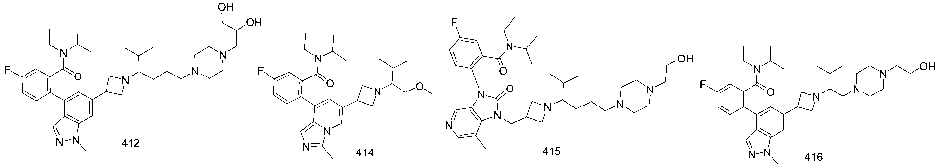 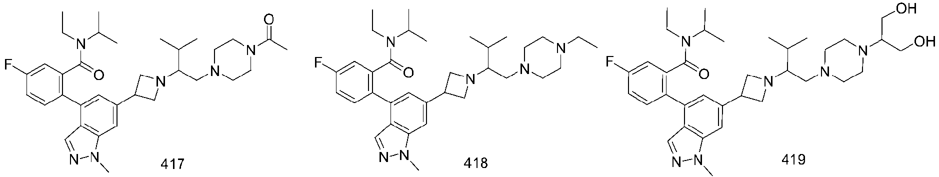 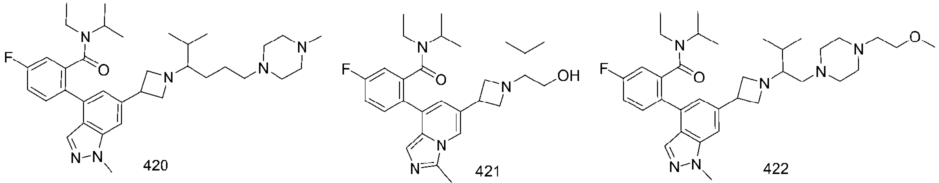 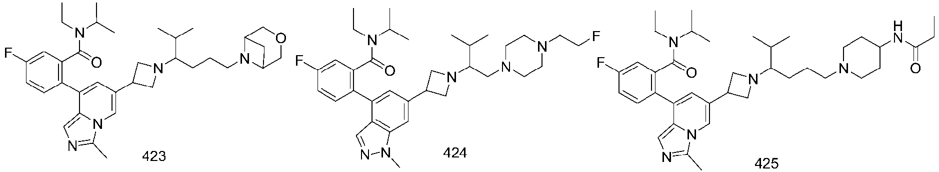 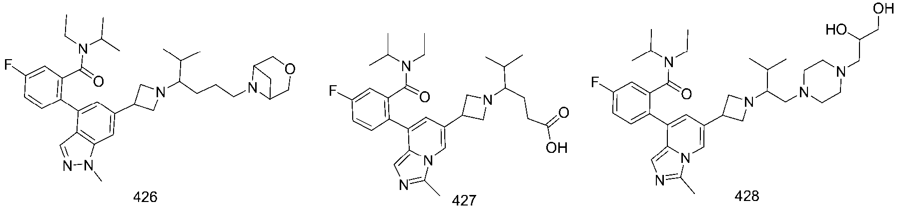 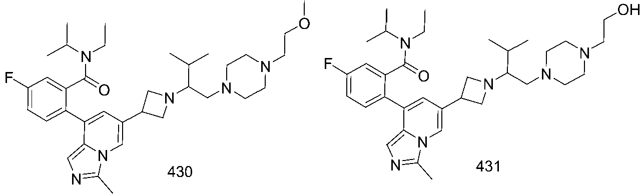 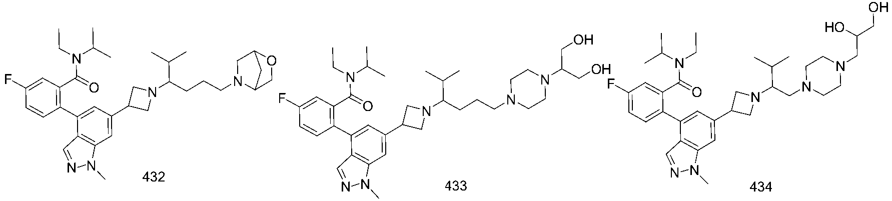 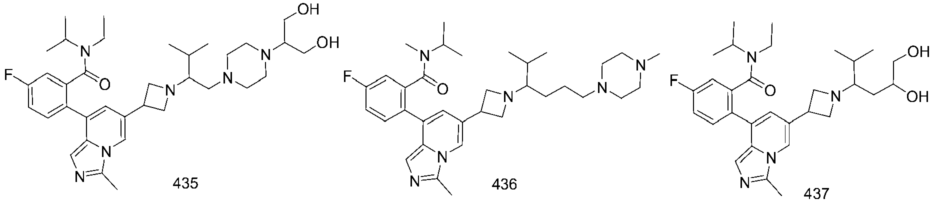 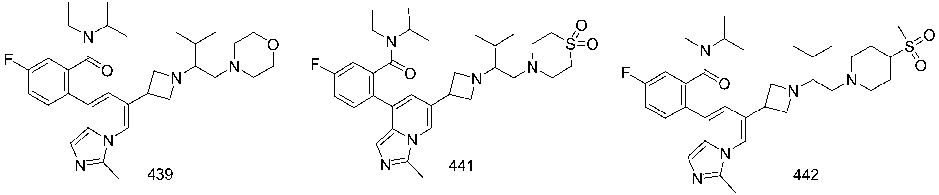 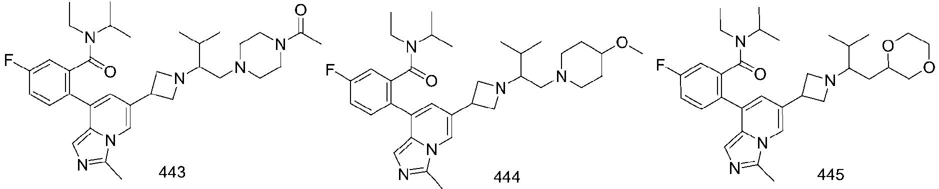 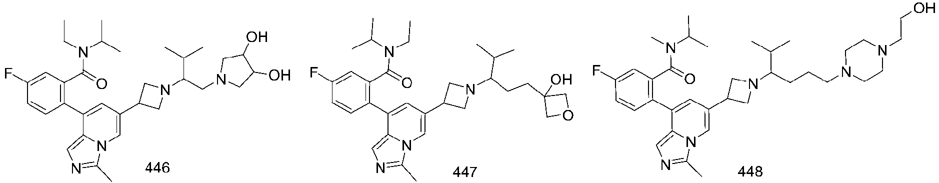 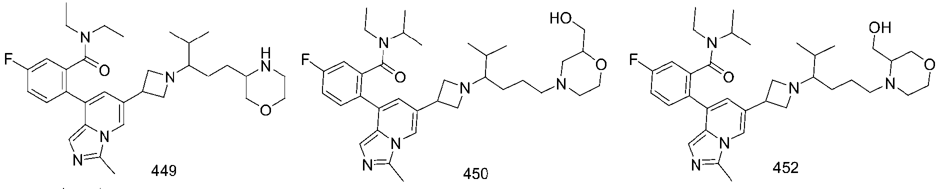 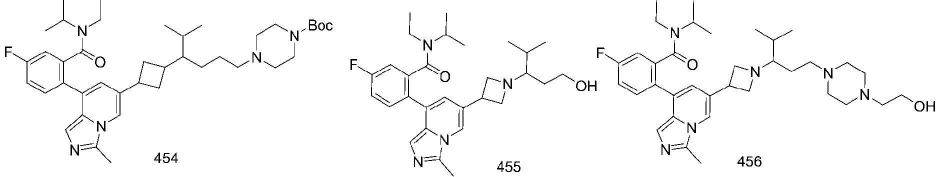 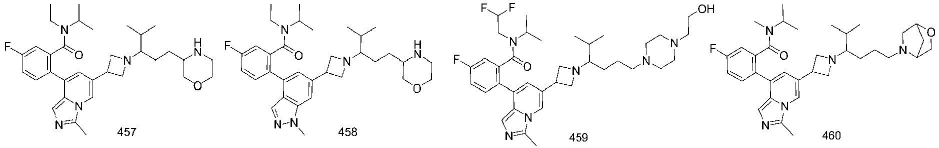 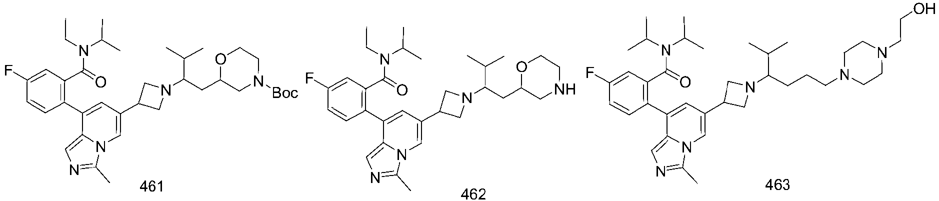 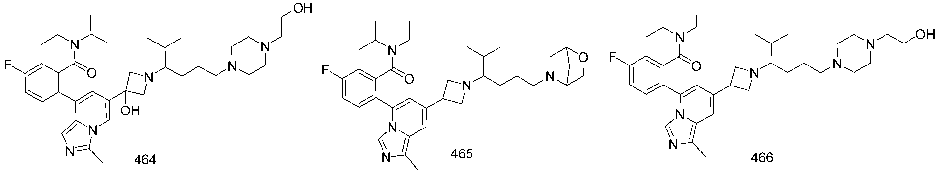 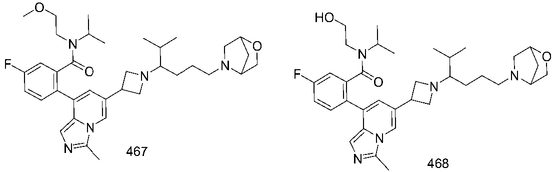 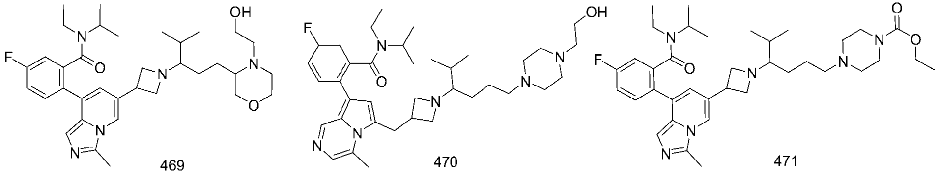 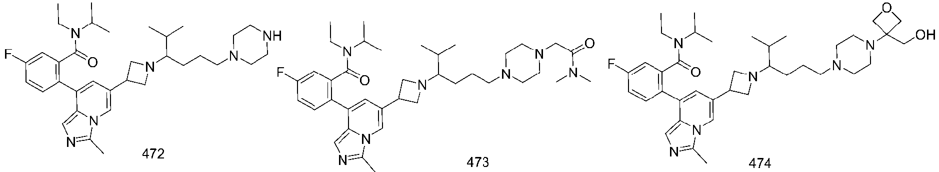 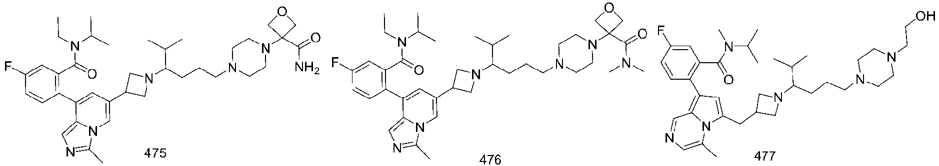 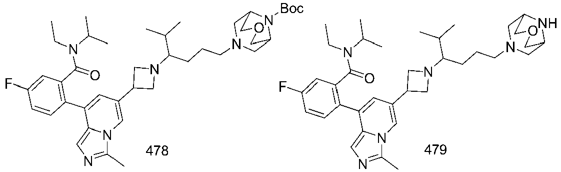 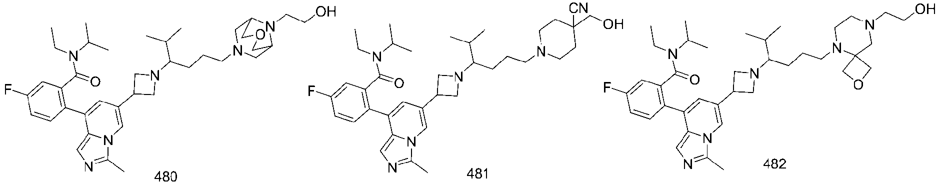 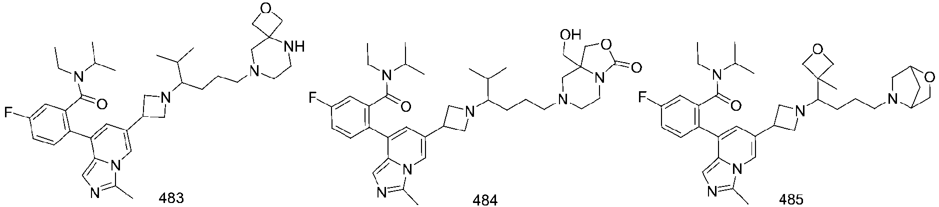 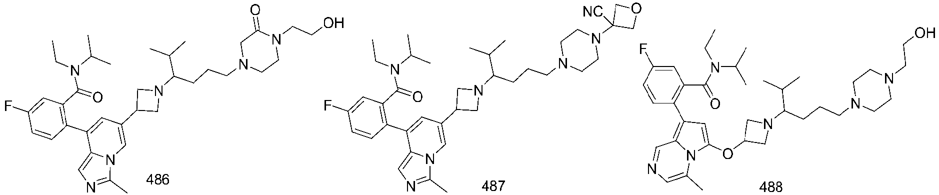 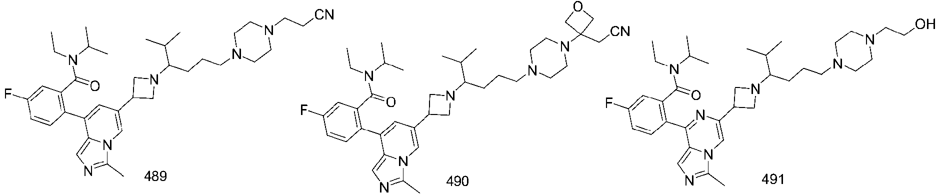 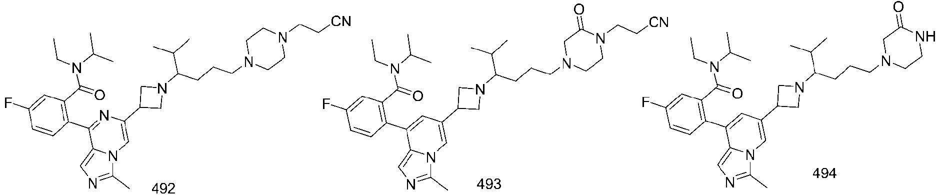 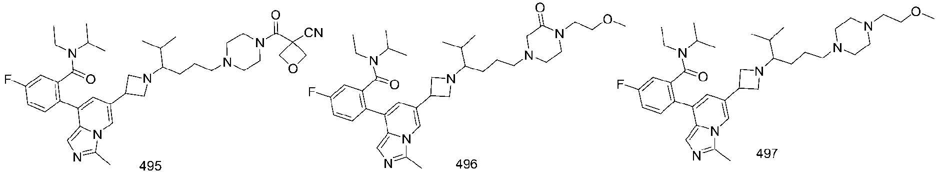 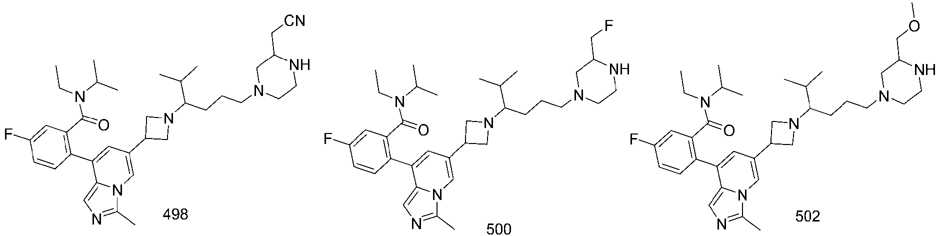 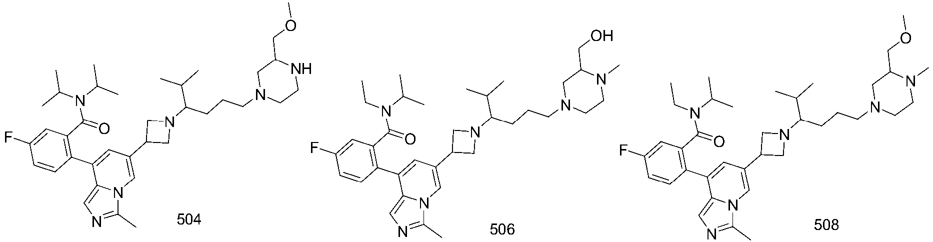 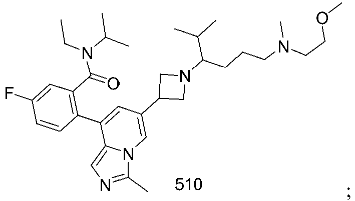 preferably, said stereoisomer is selected from the following structures:

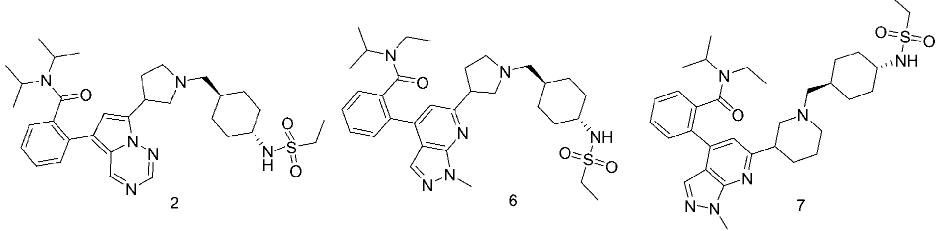

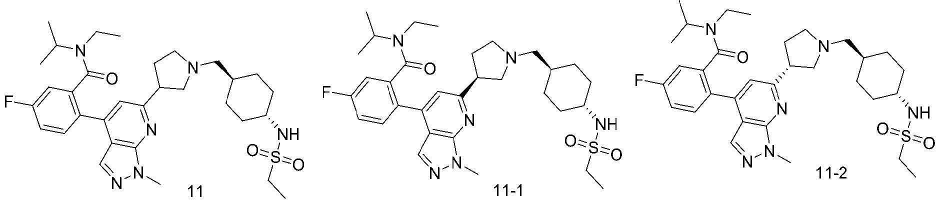

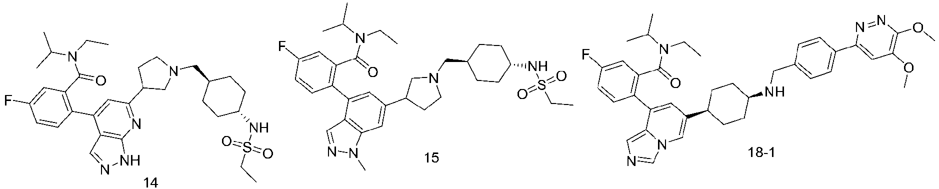

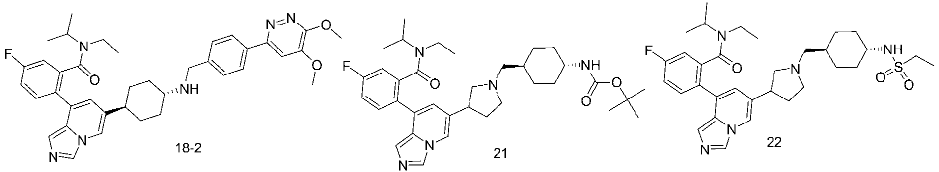

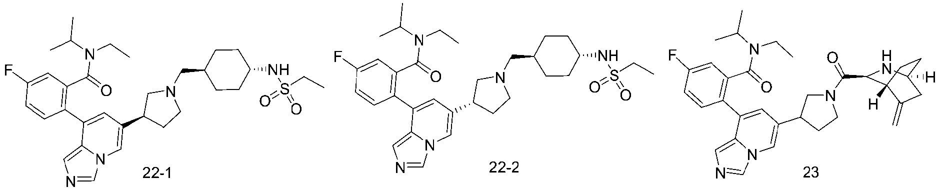

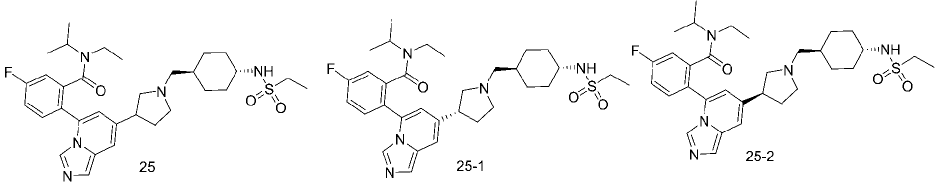

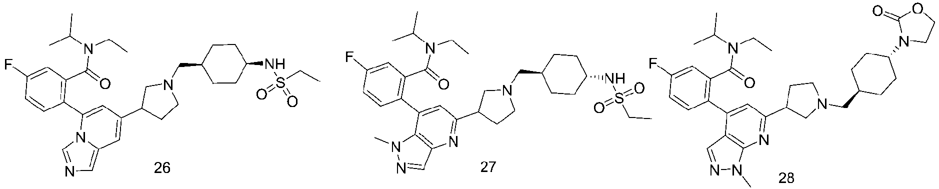

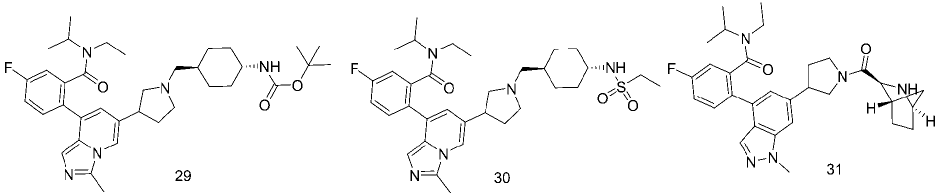

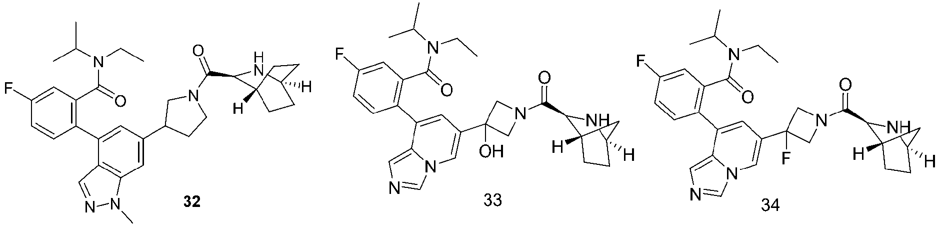

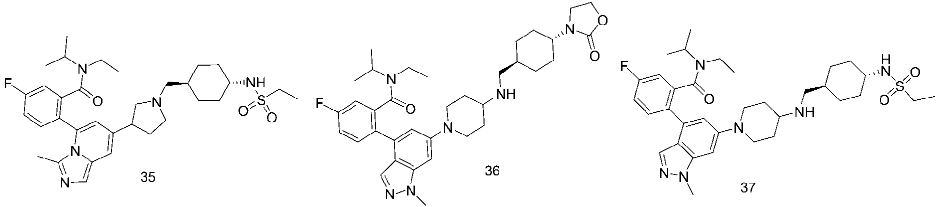

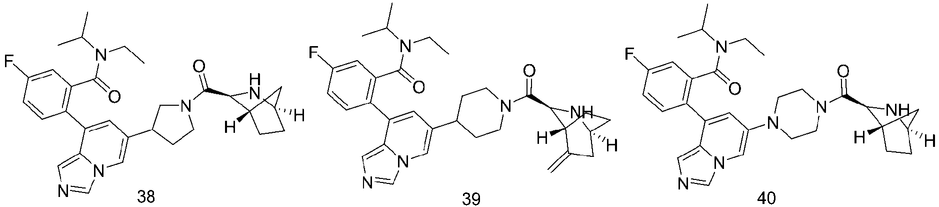

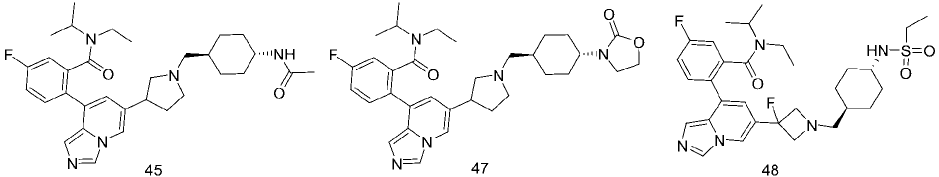

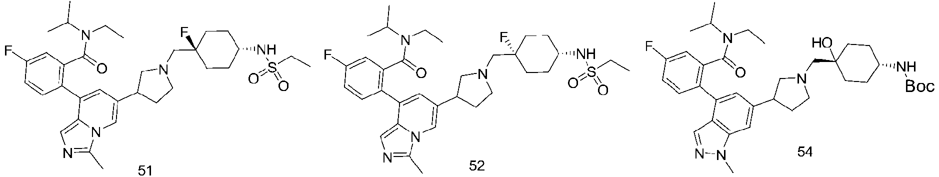

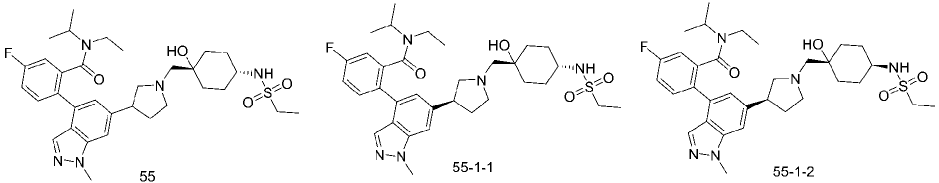

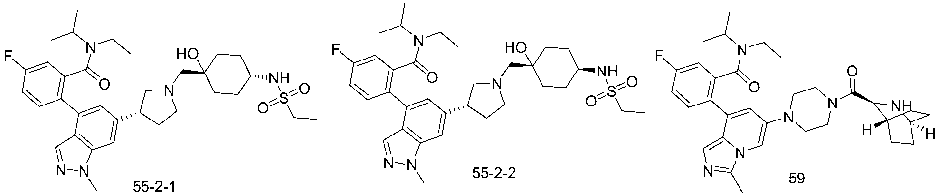

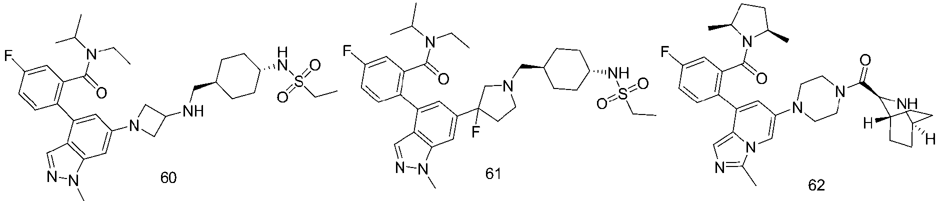

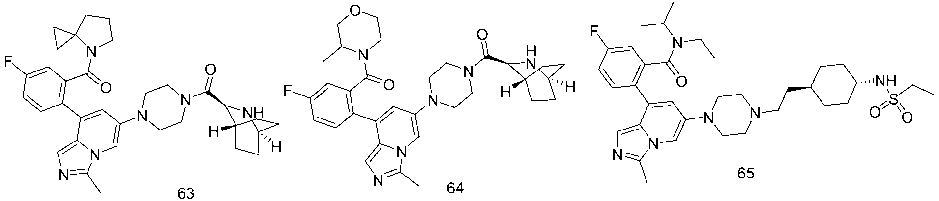

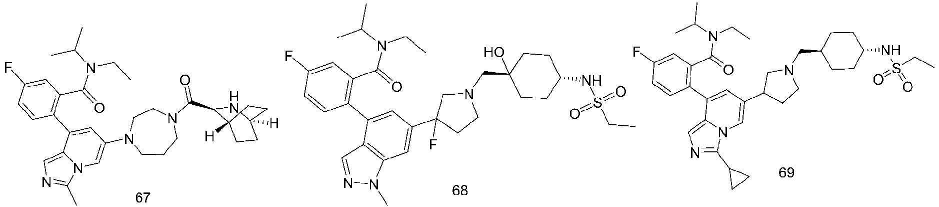

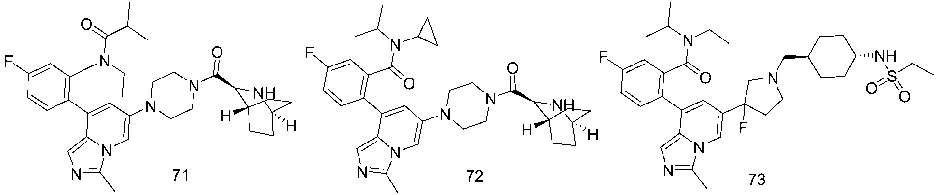

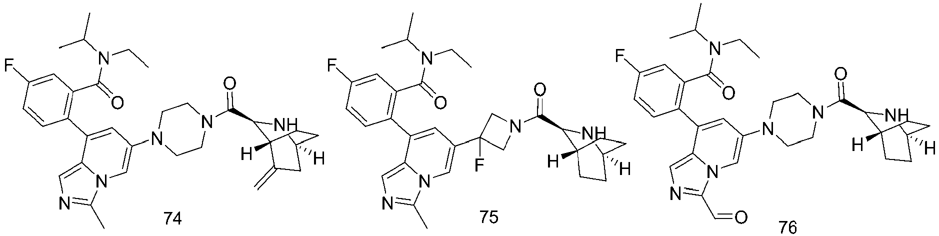

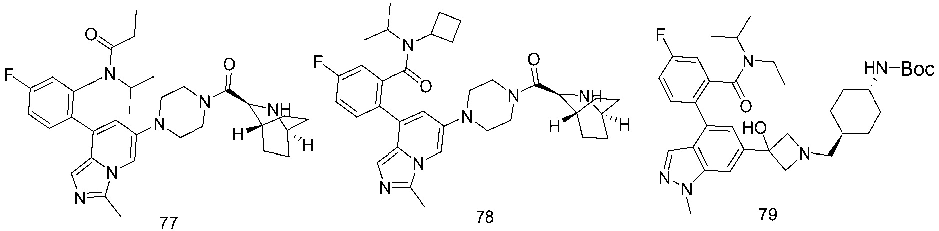

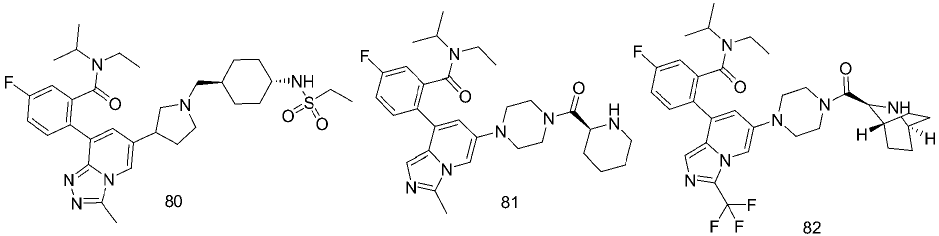

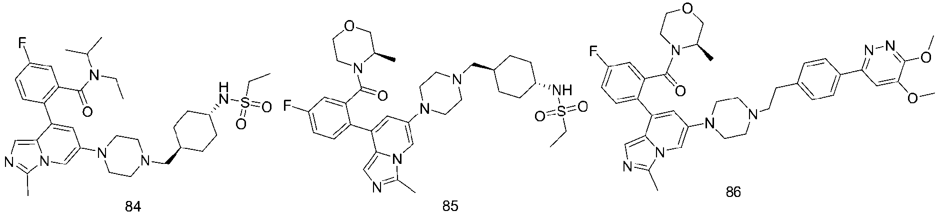

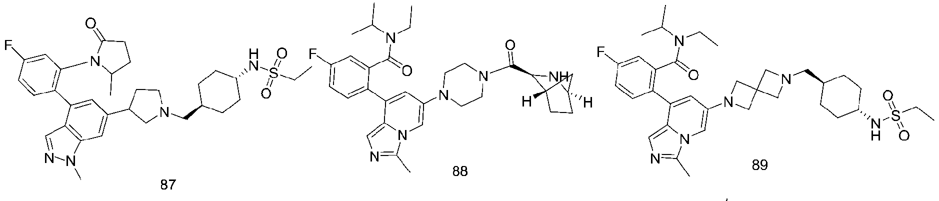

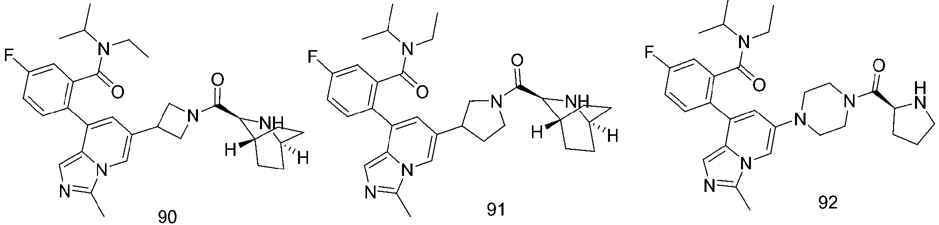

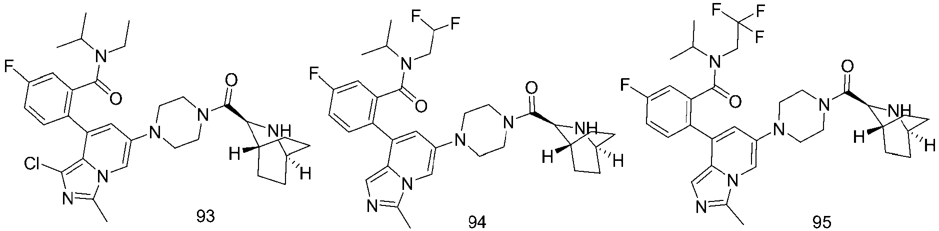

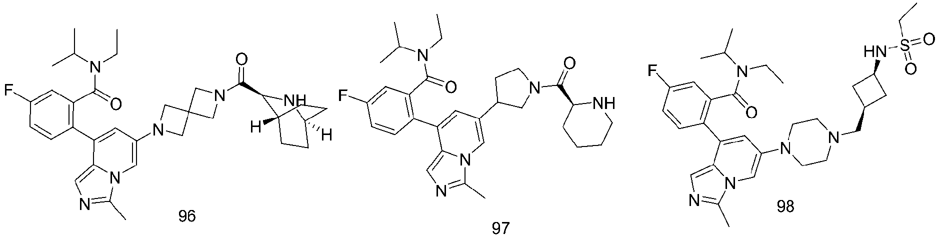

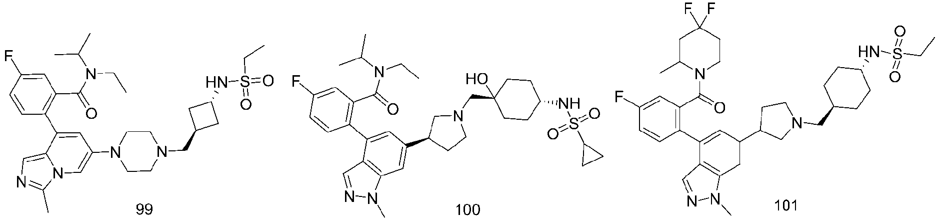

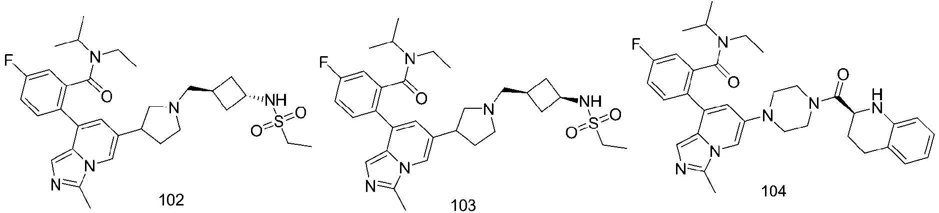

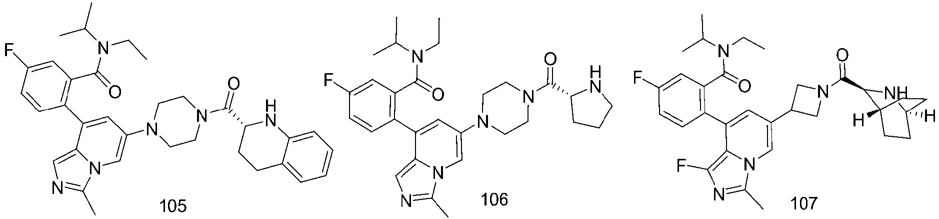

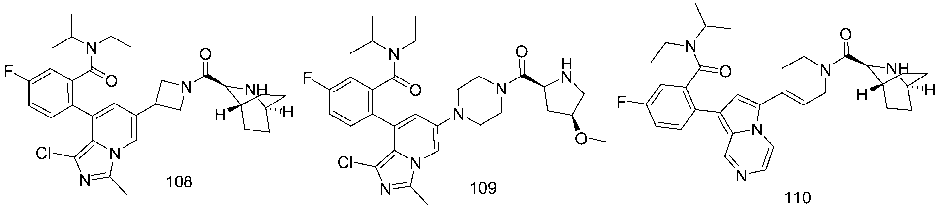

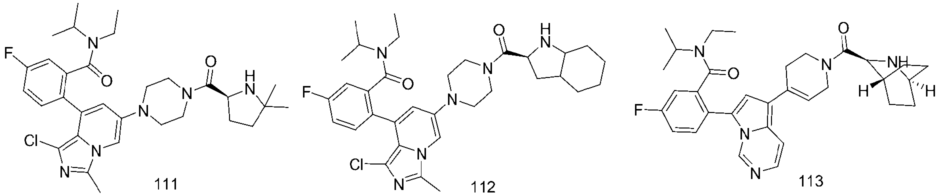

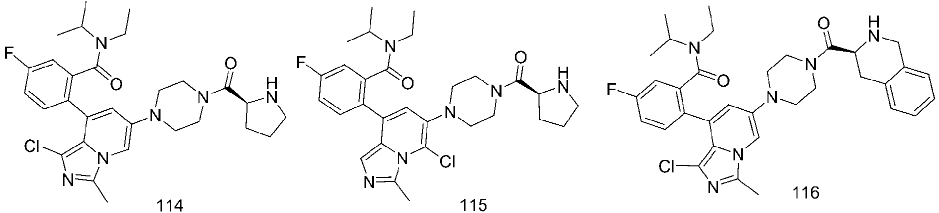

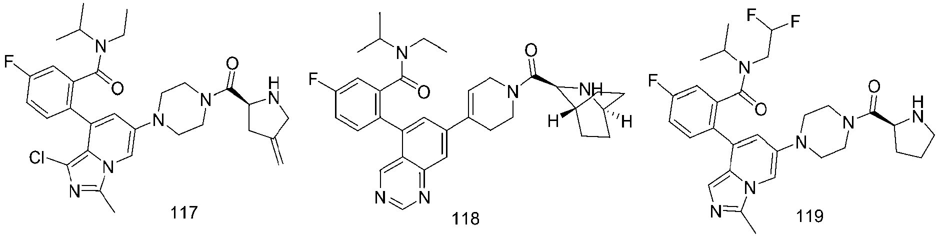

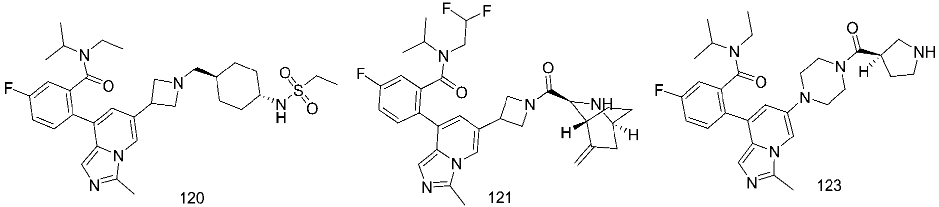

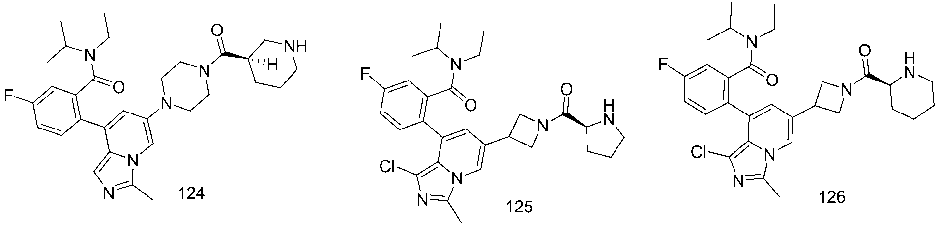

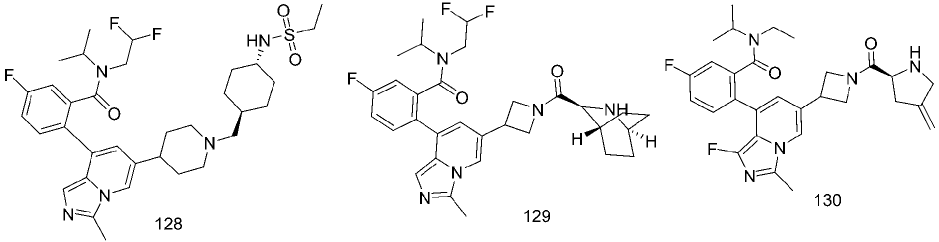

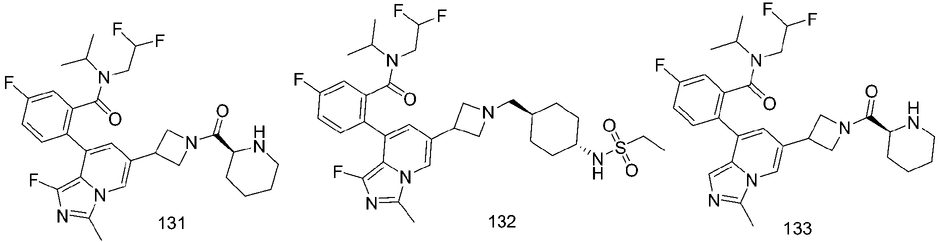

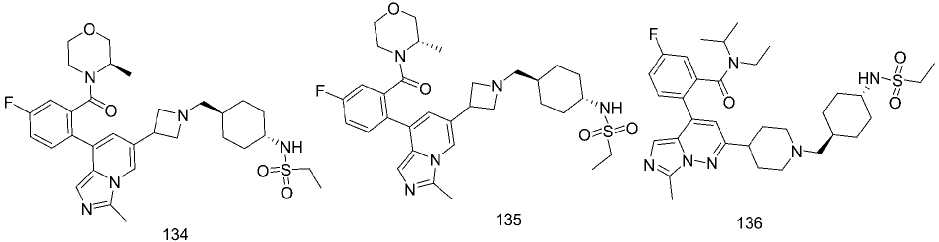

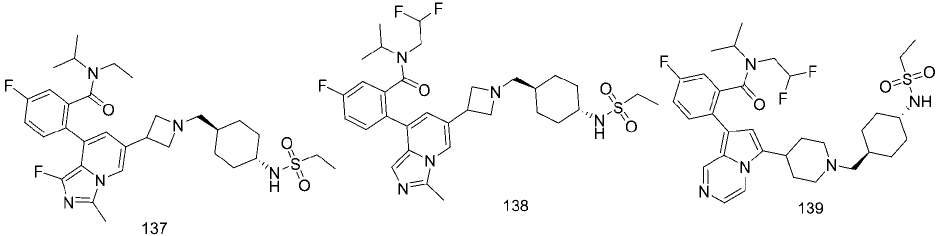

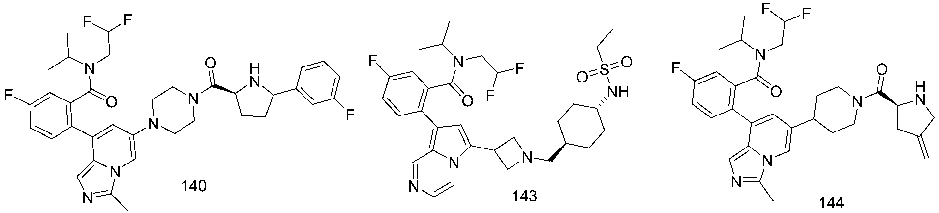

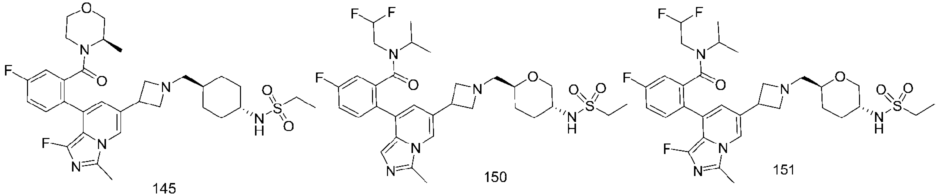

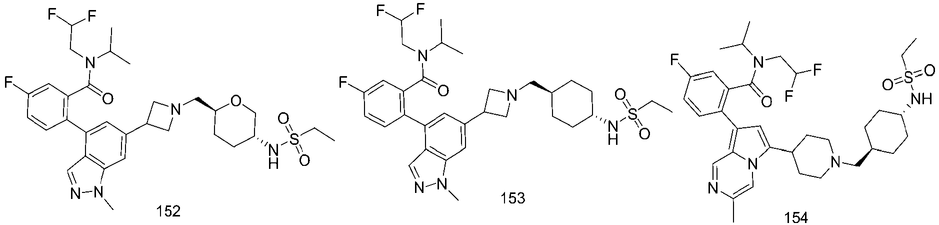

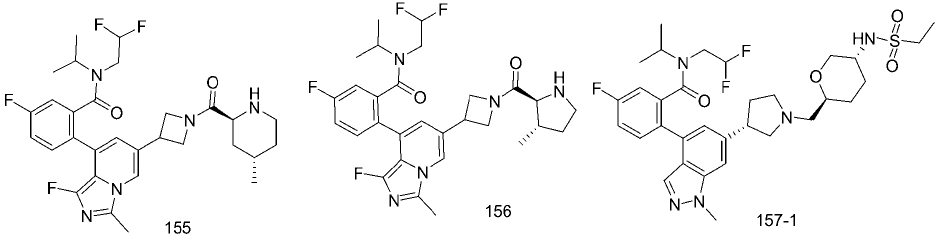

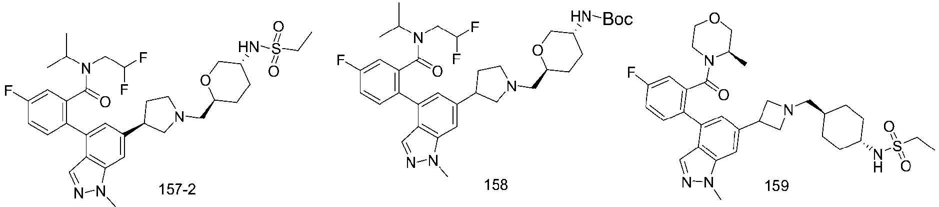

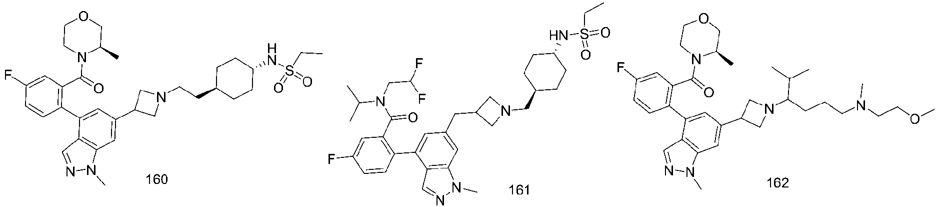

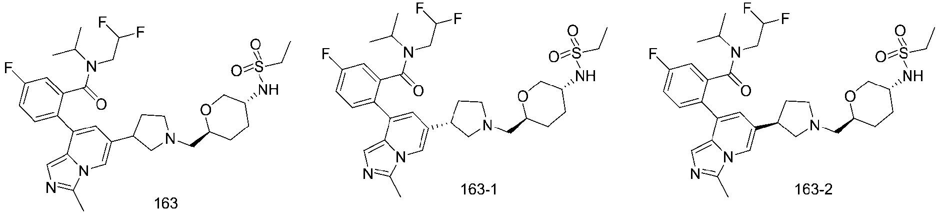

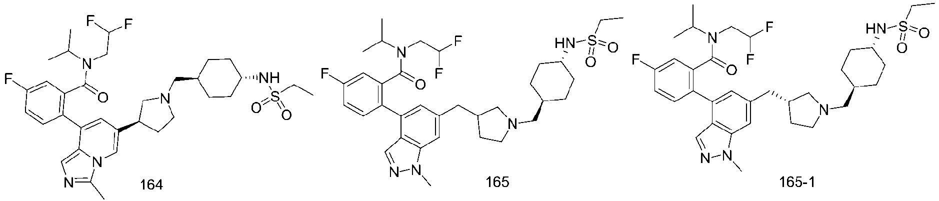

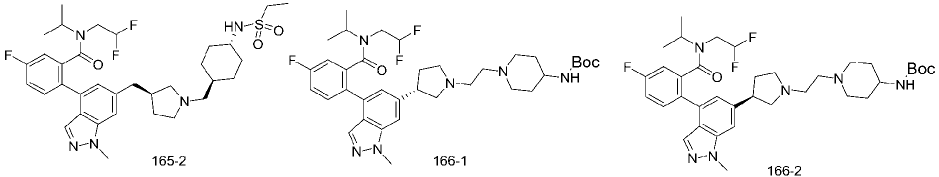

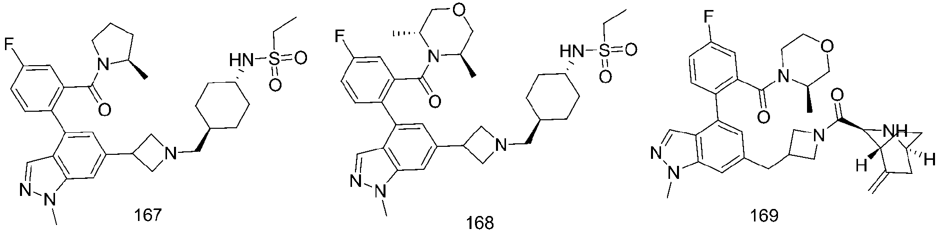

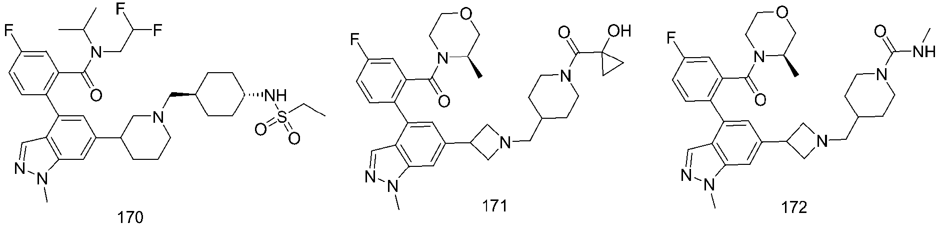

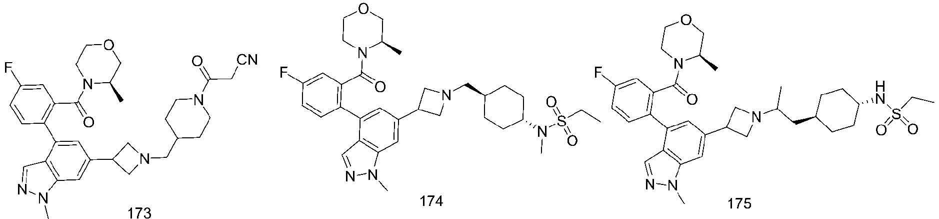

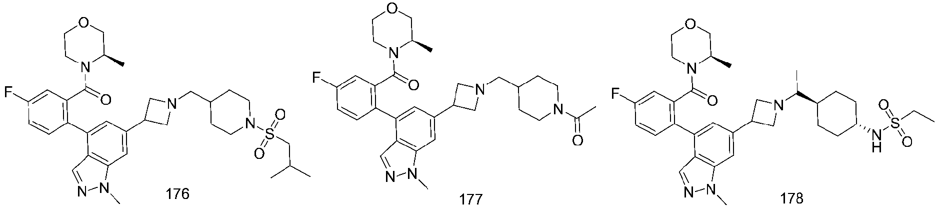

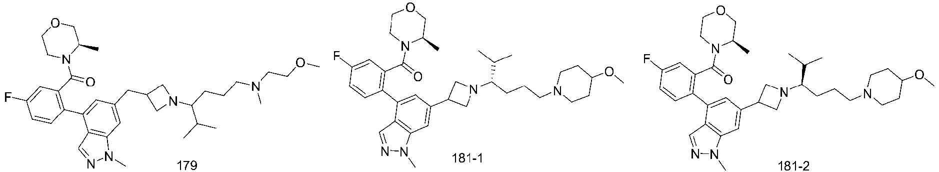

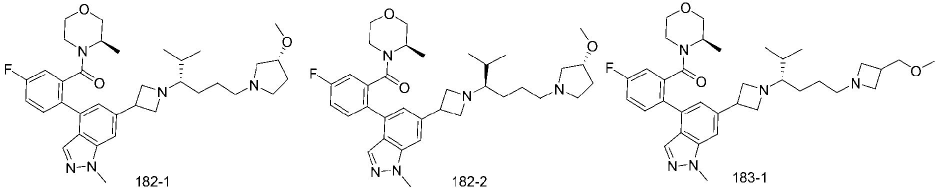

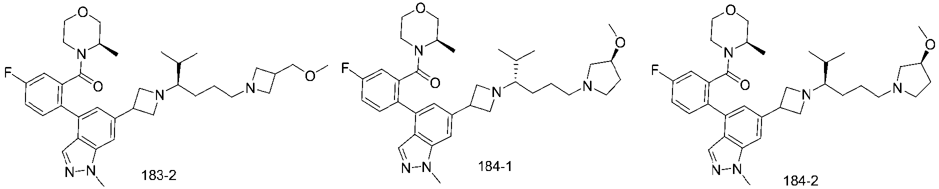

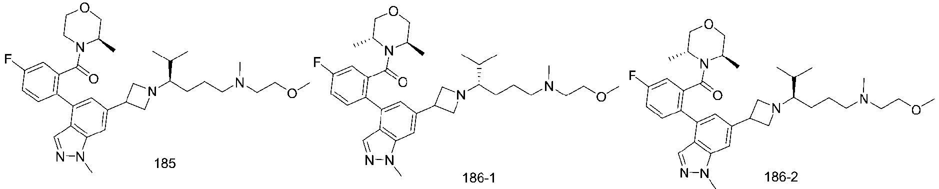

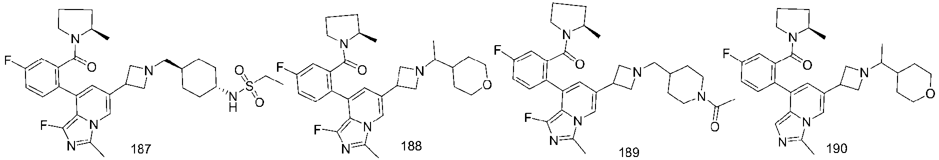

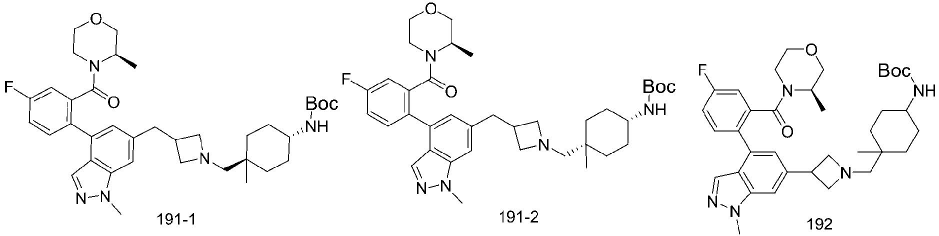

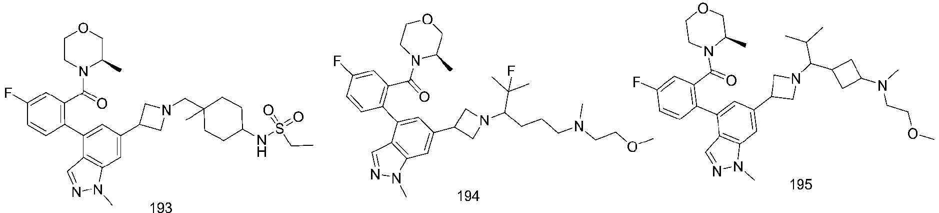

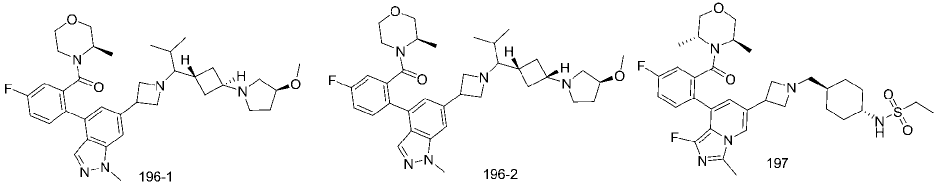

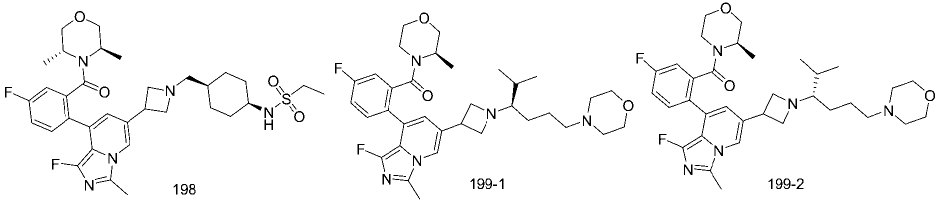

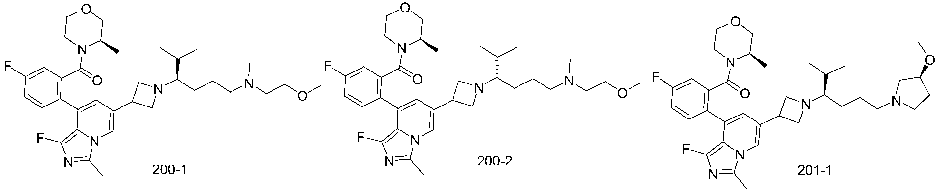

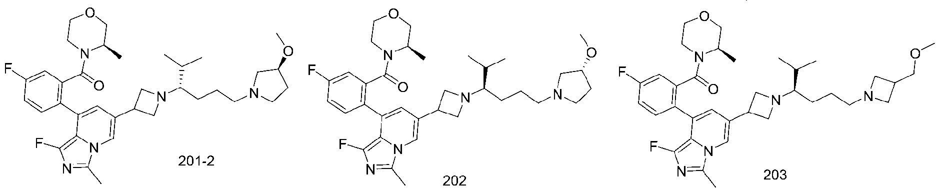

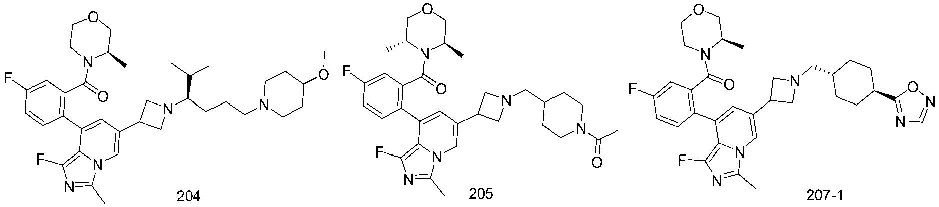

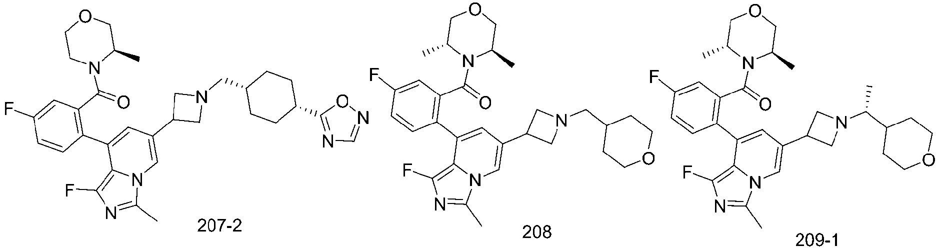

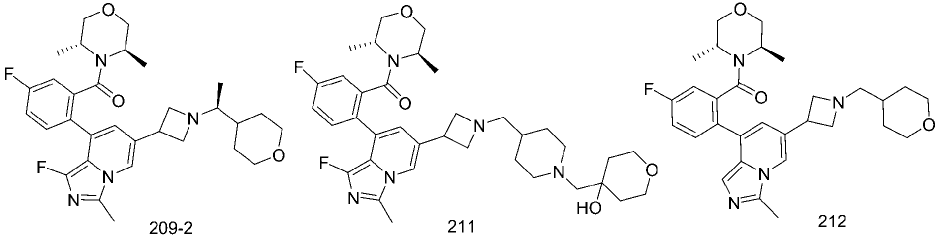

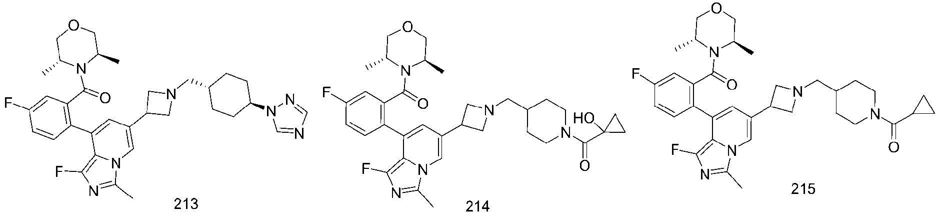

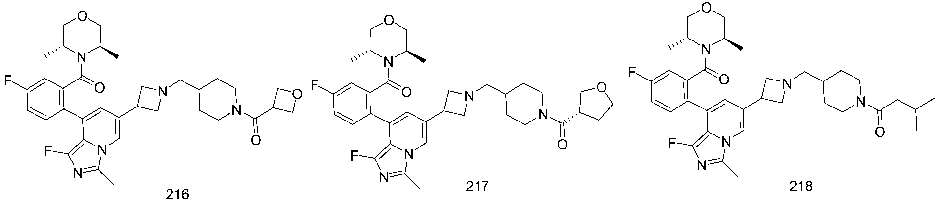

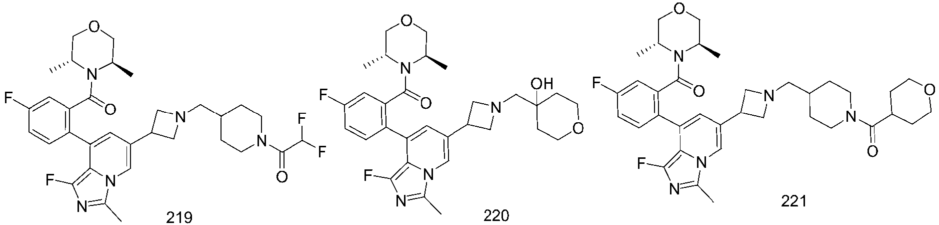

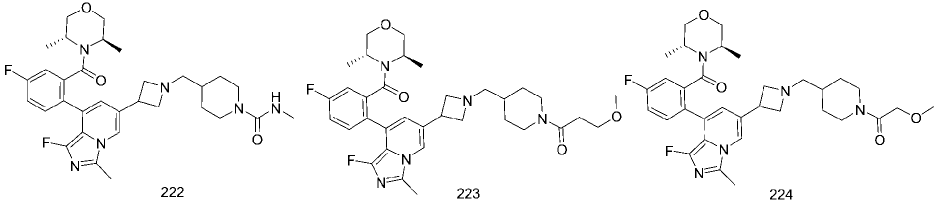

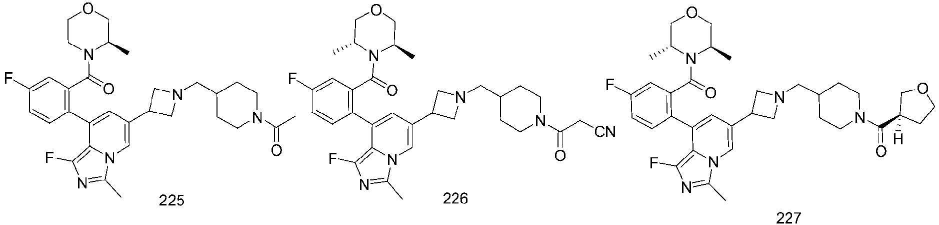

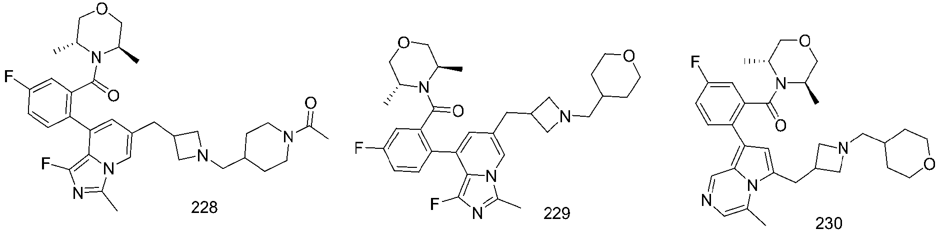

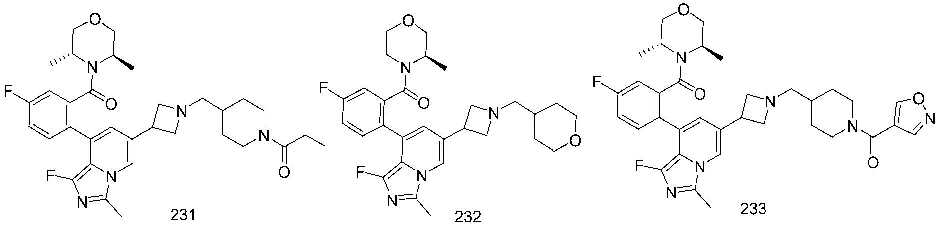

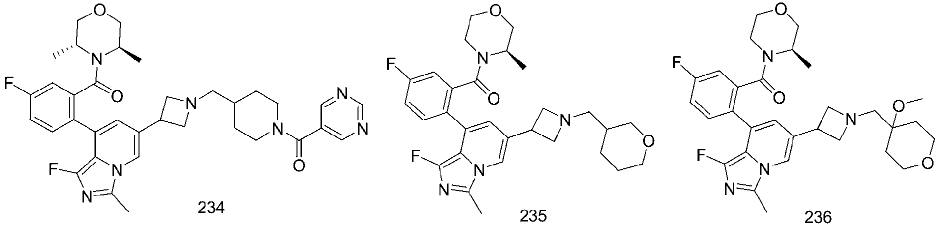

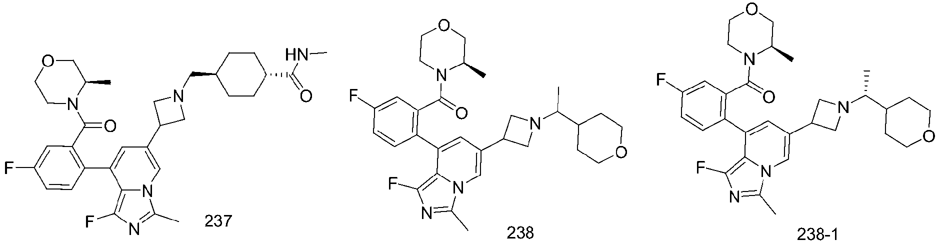

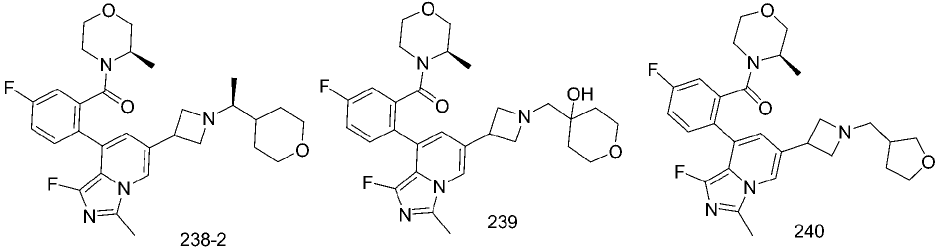

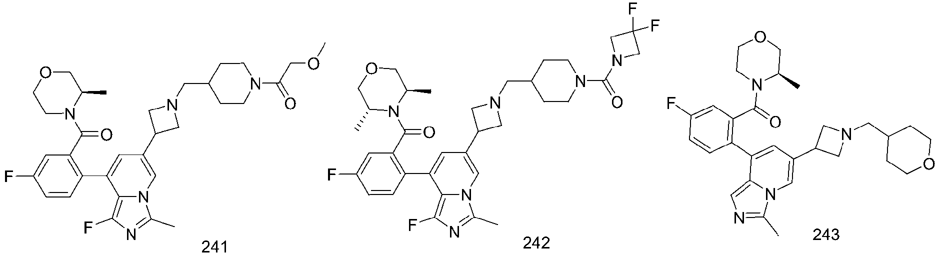

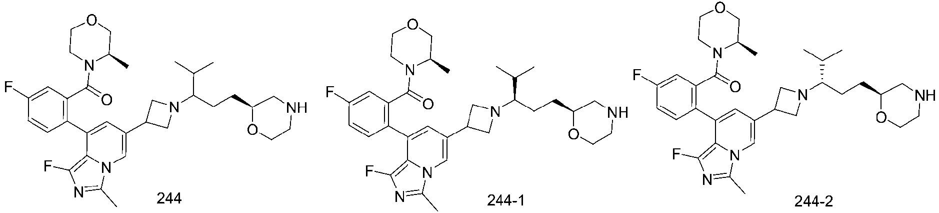

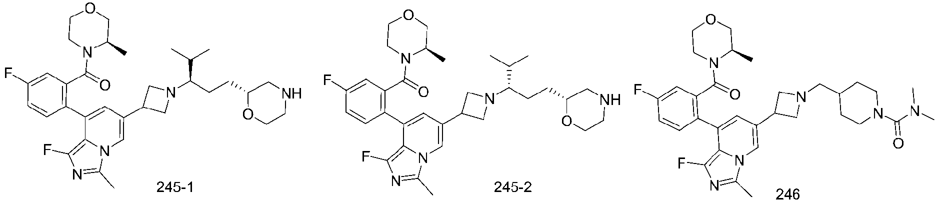

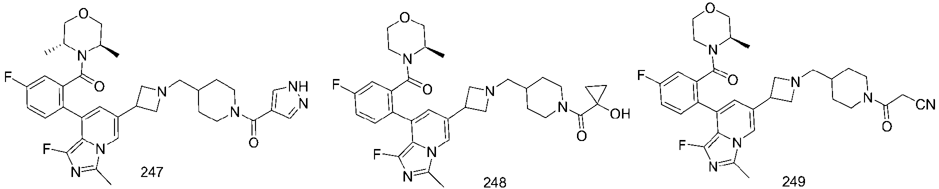

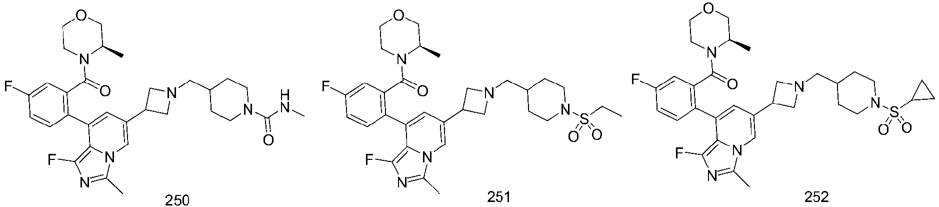

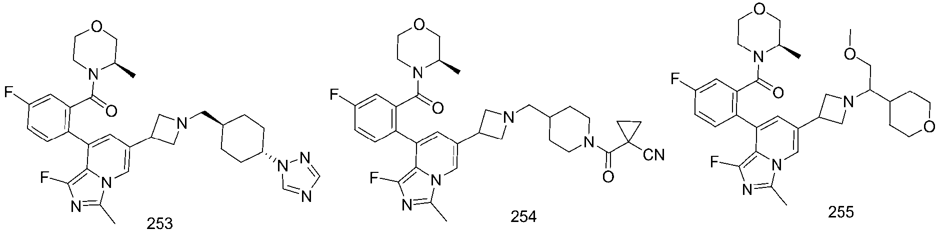

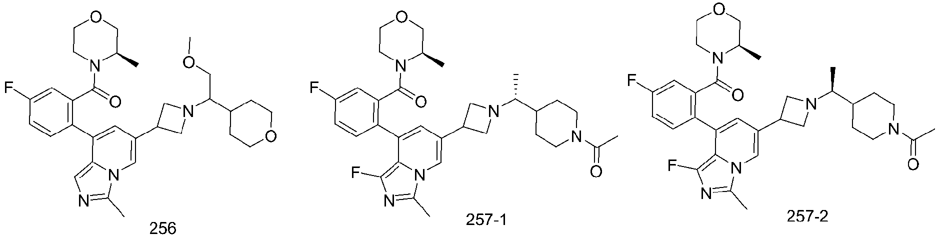

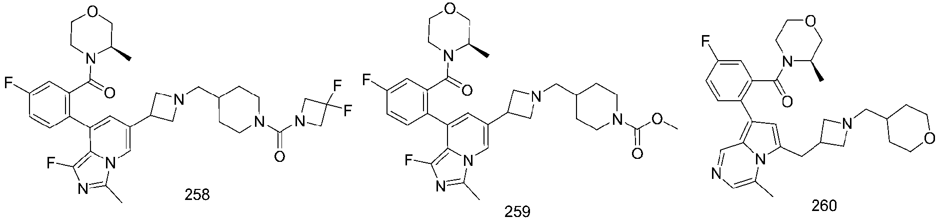

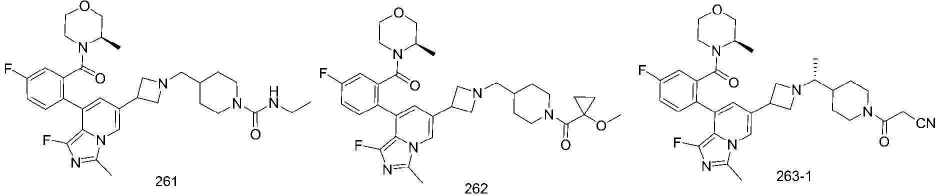

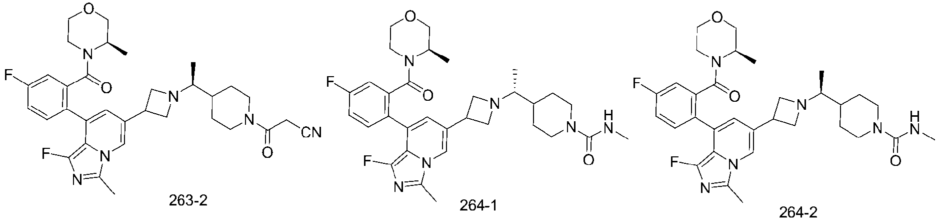

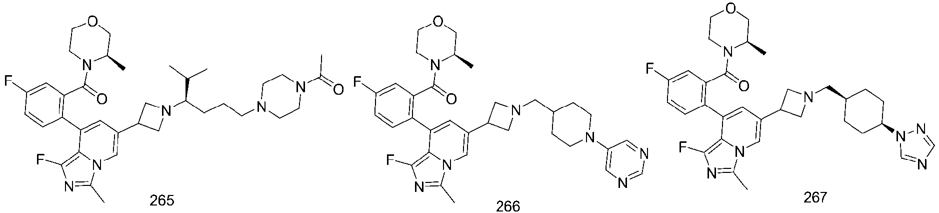

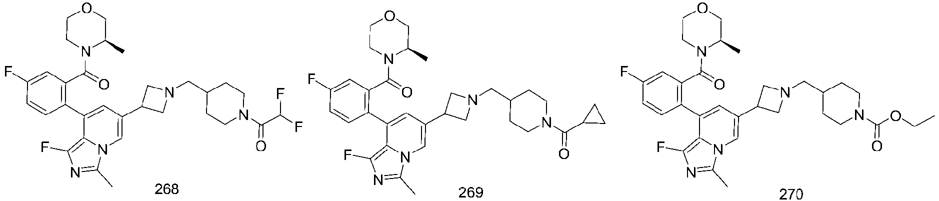

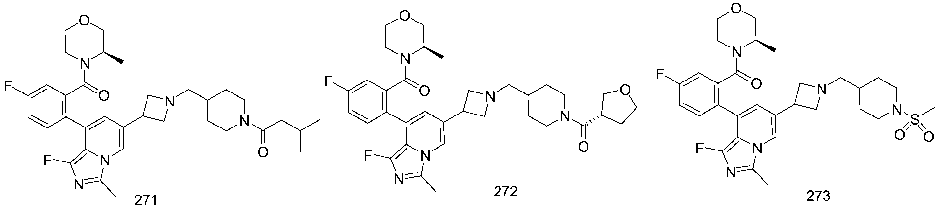

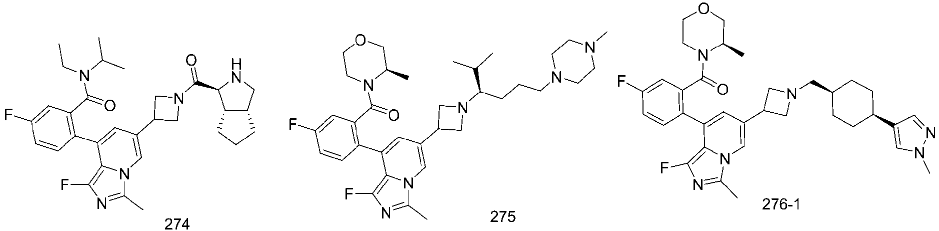

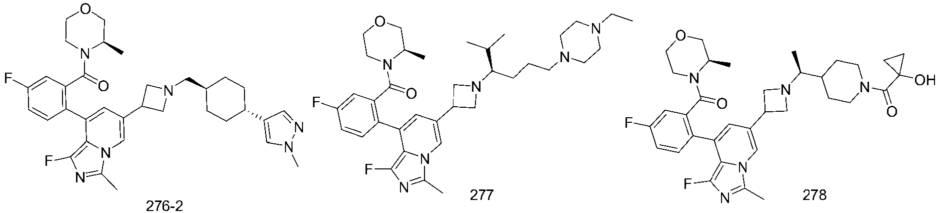

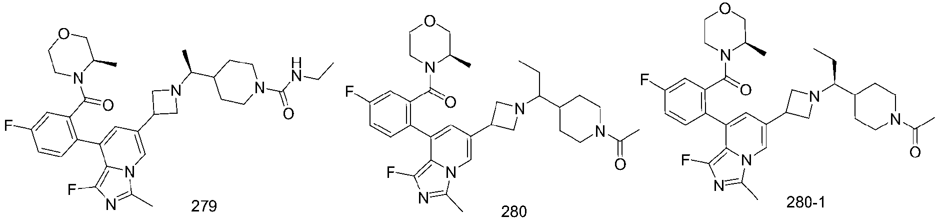

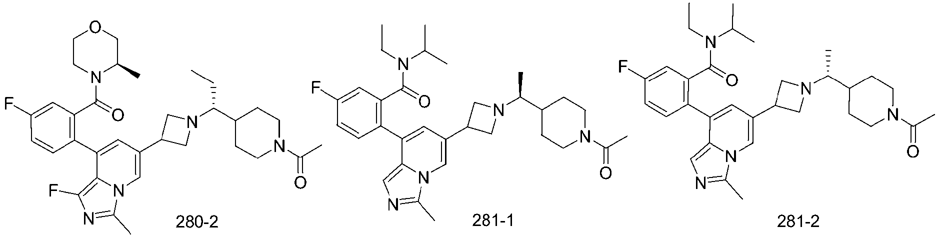

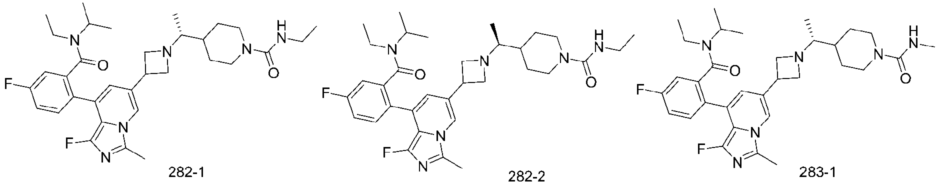

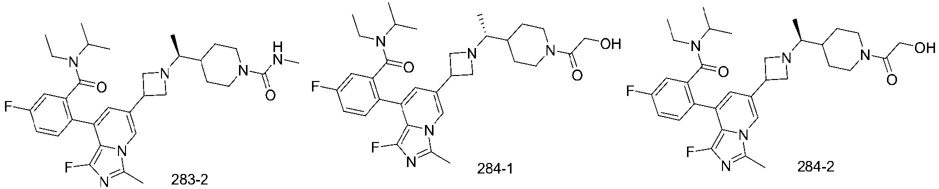

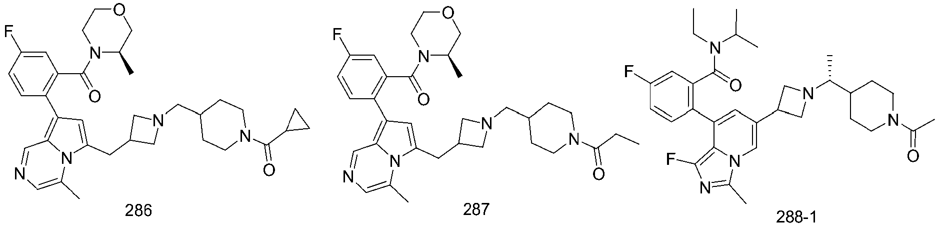

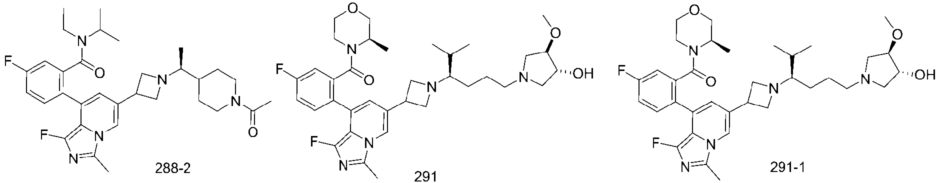

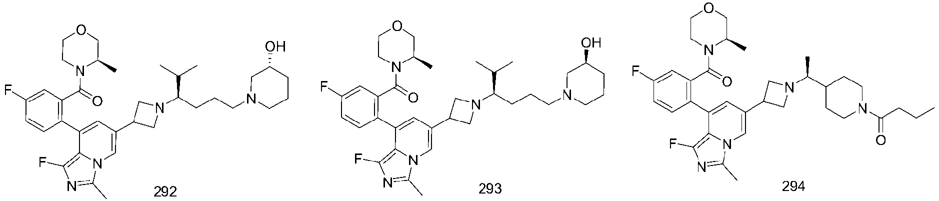

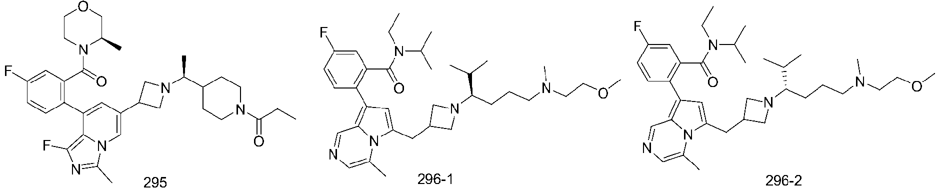

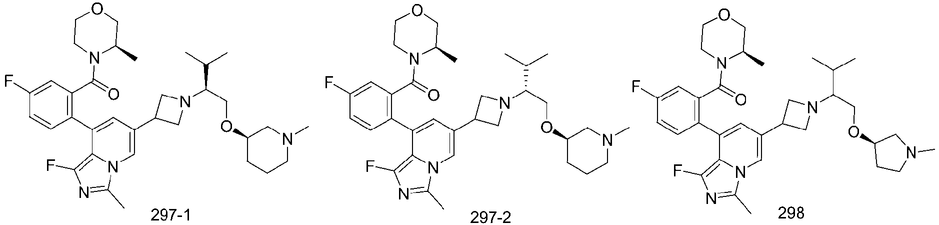

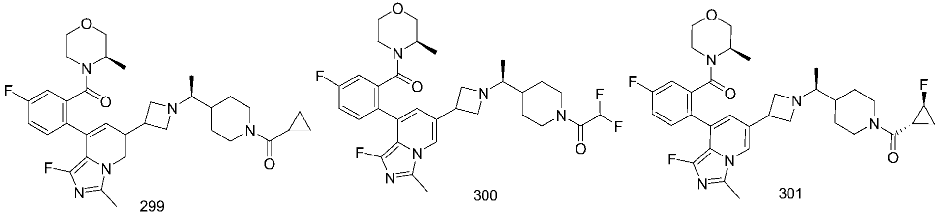

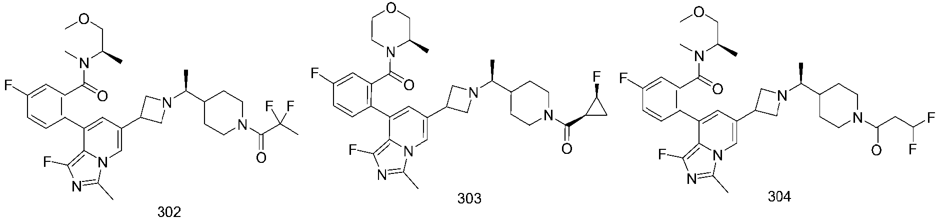

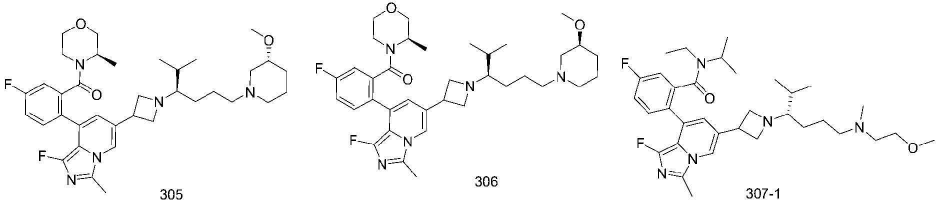

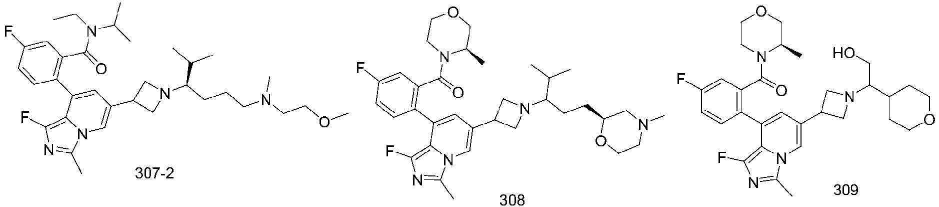

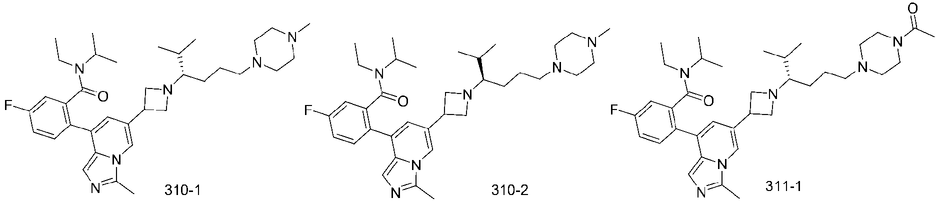

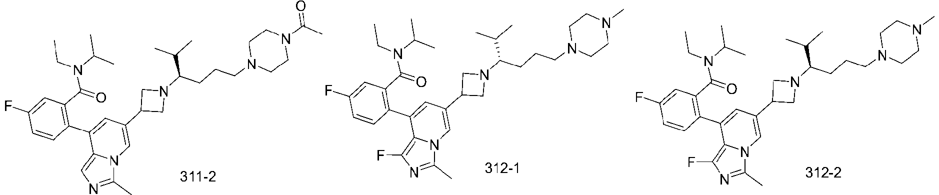

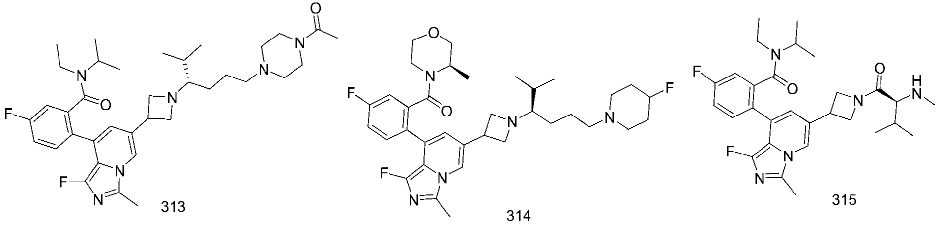

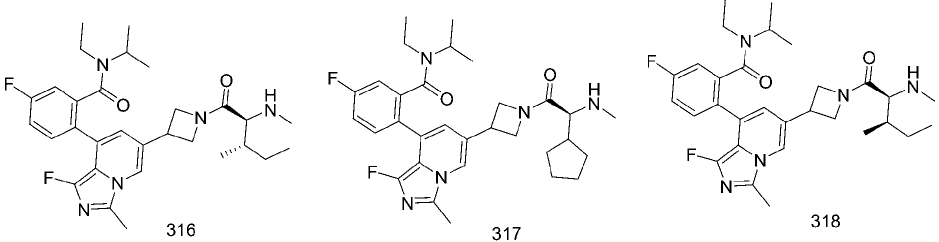

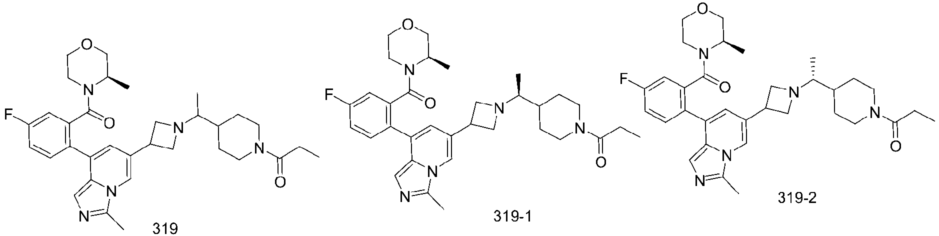

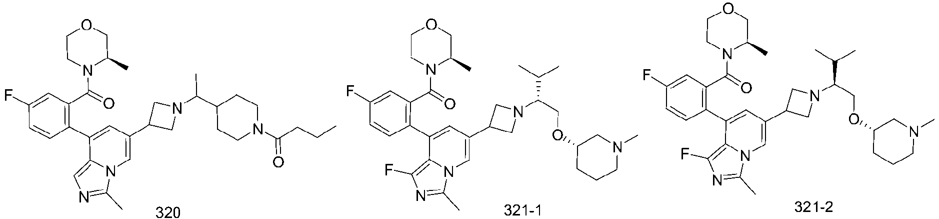

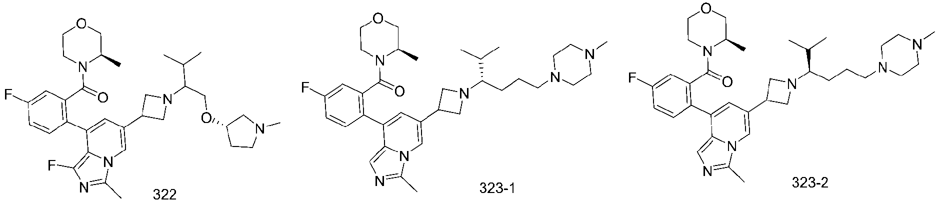

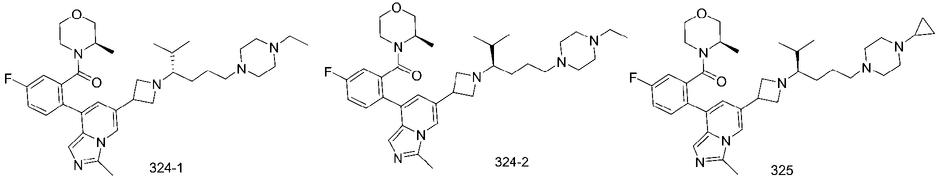

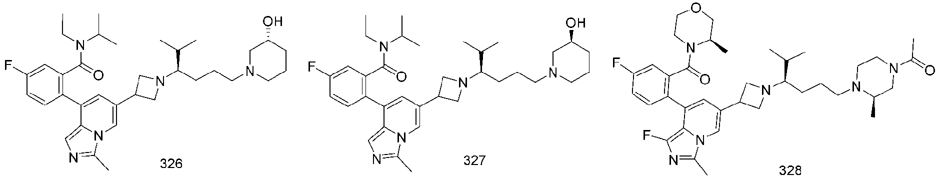

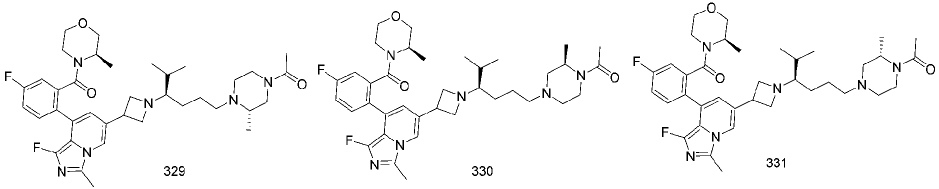

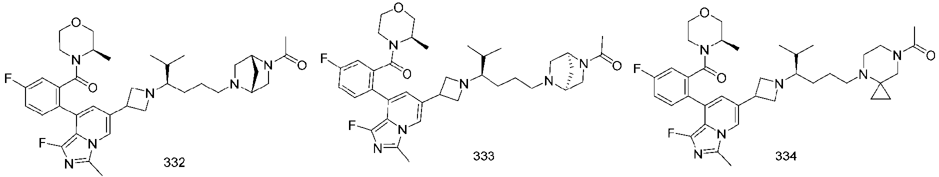

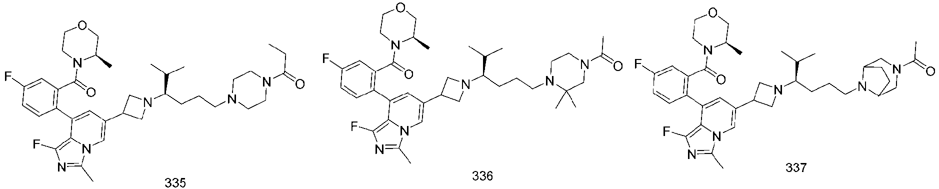

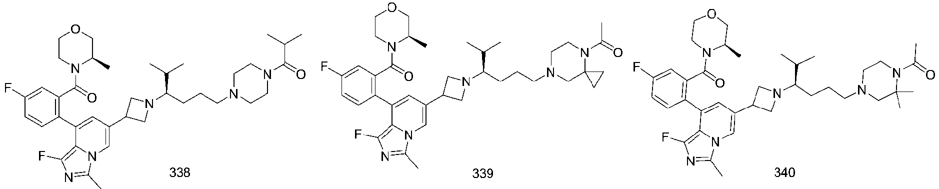

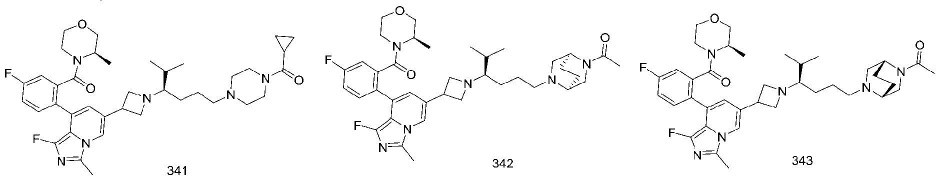

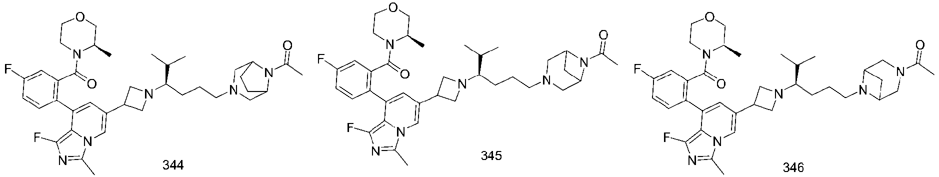

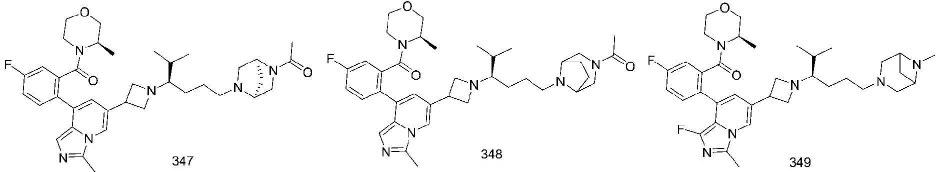

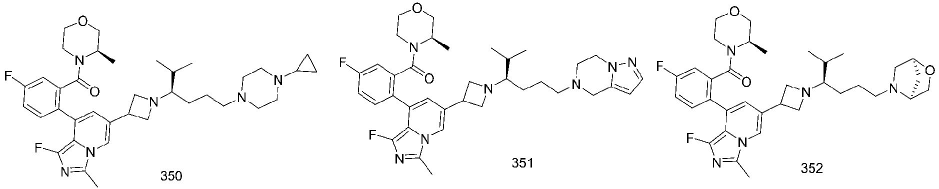

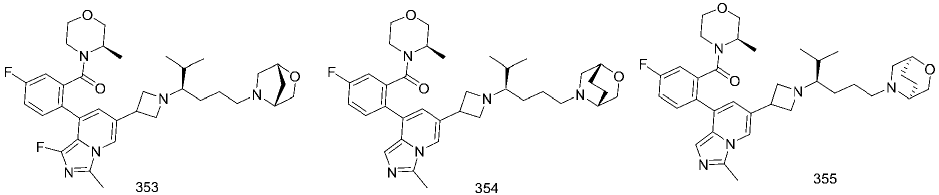

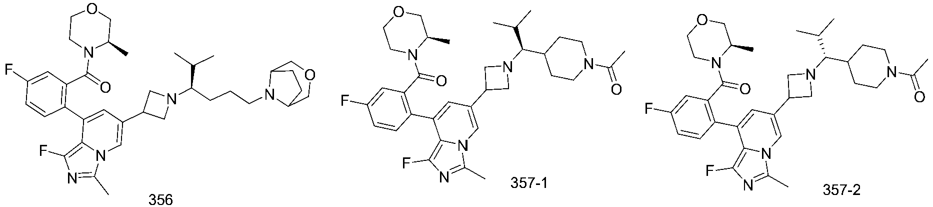

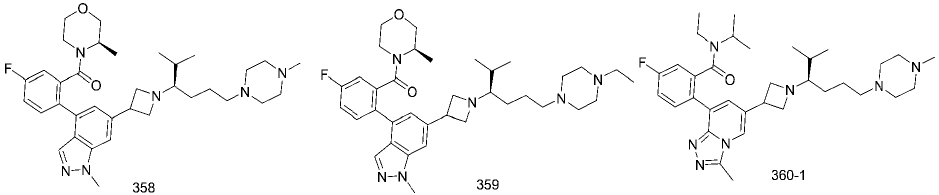

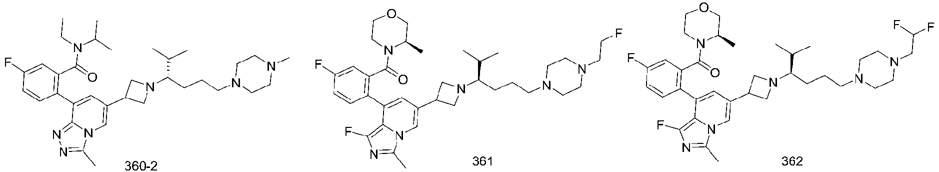

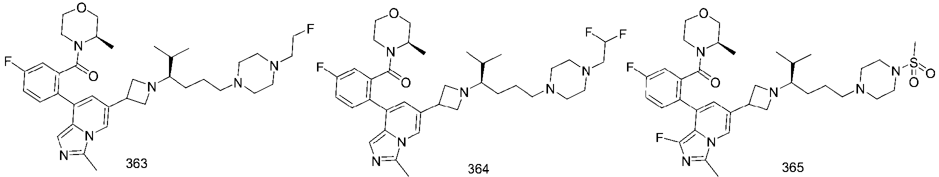

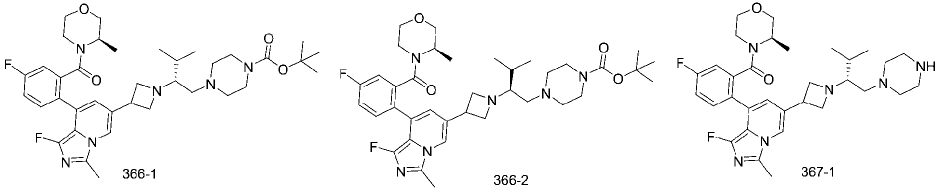

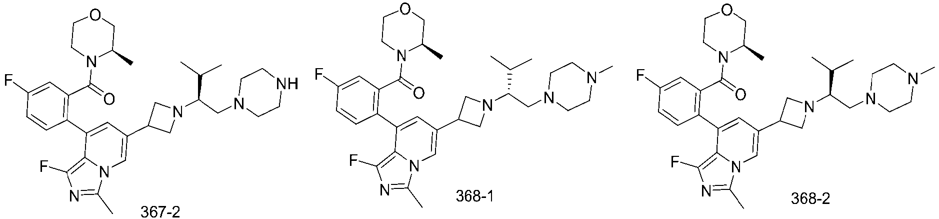

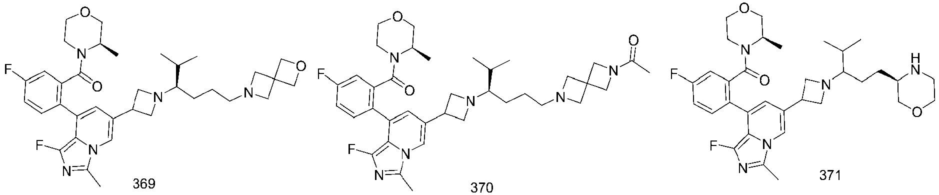

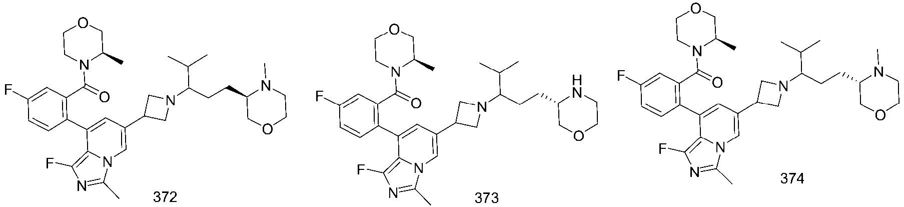

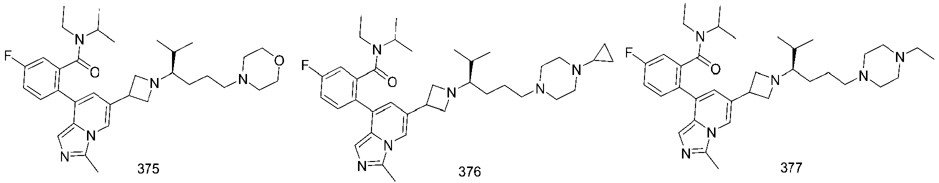

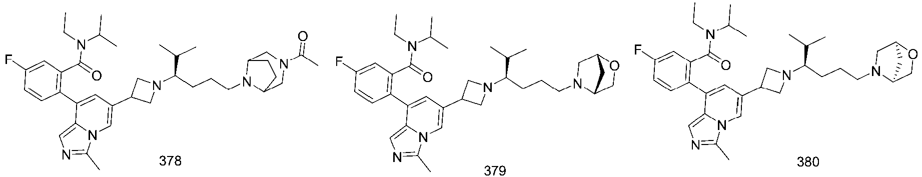

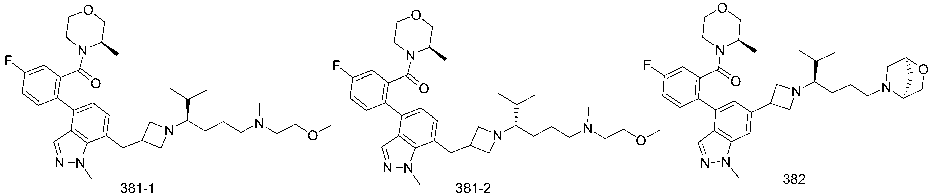

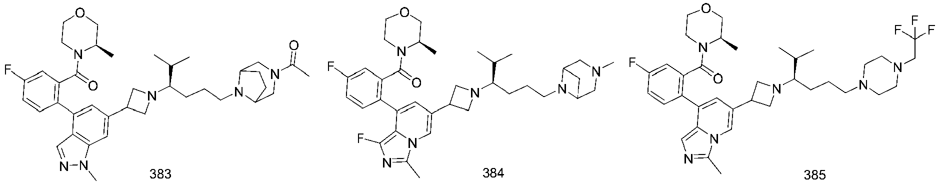

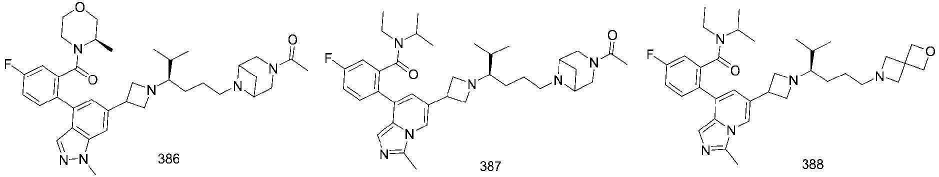

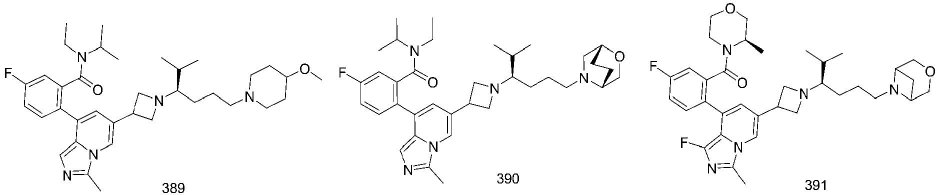

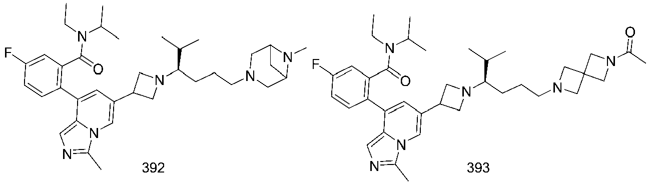

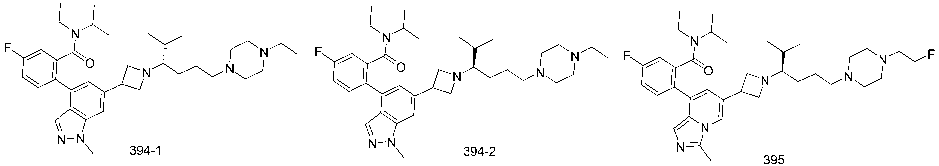

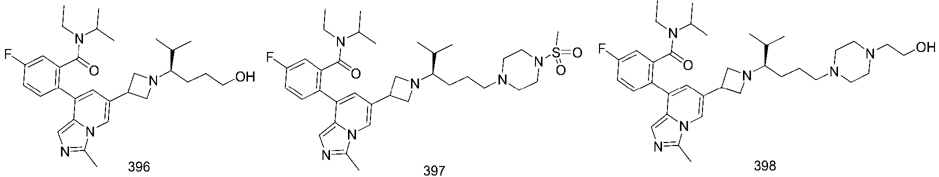

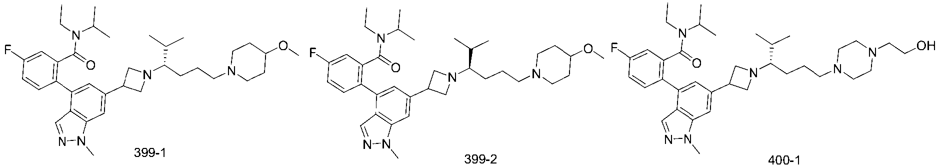

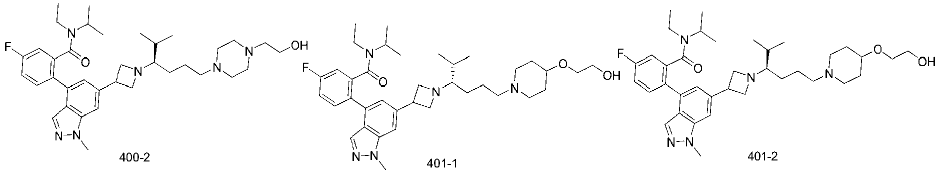

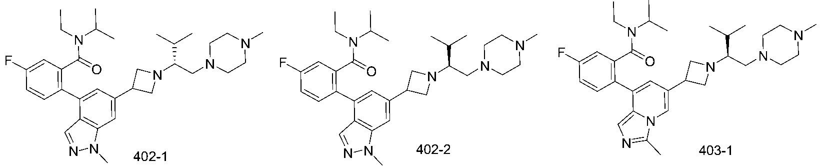

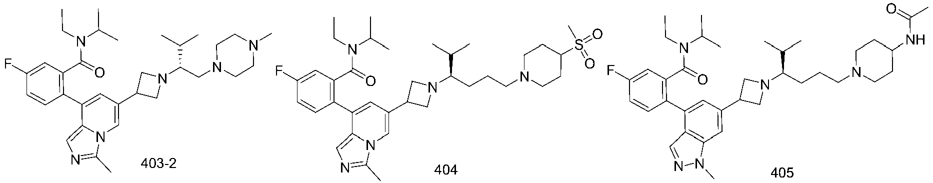

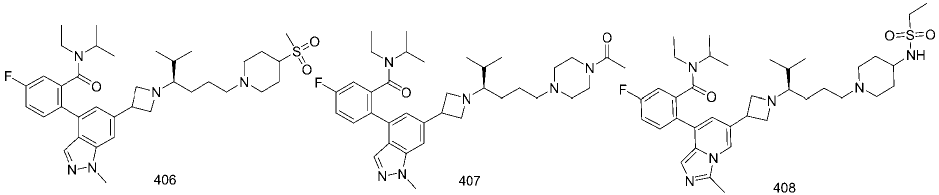

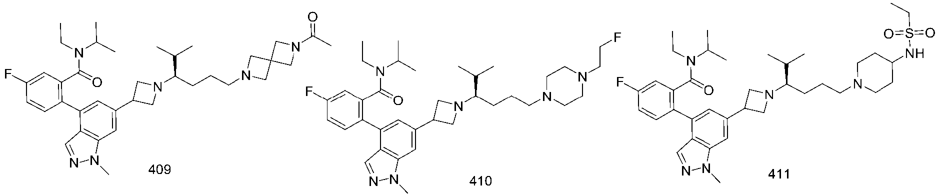

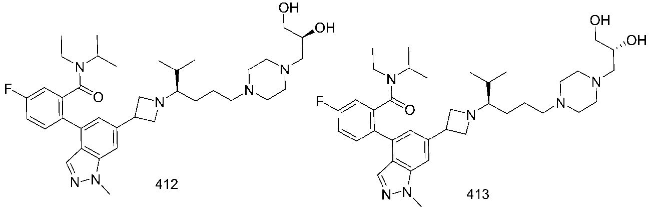

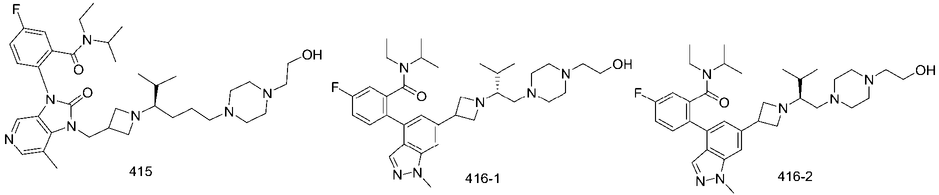

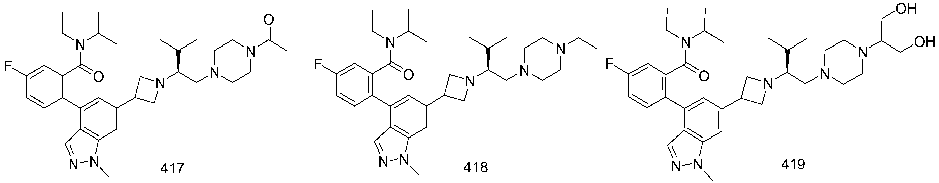

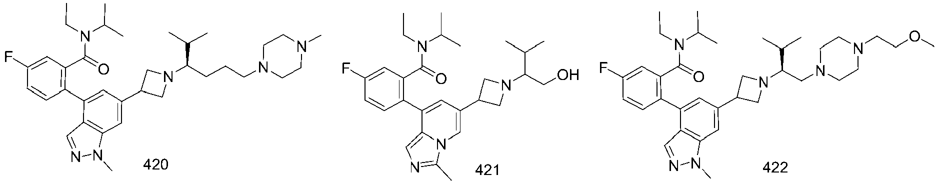

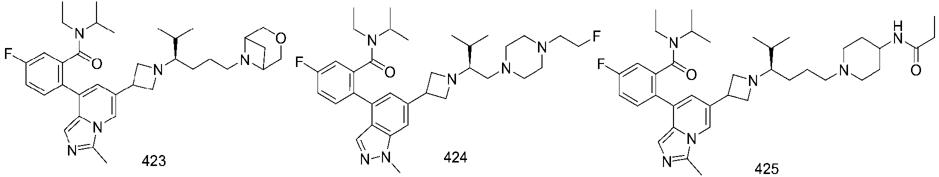

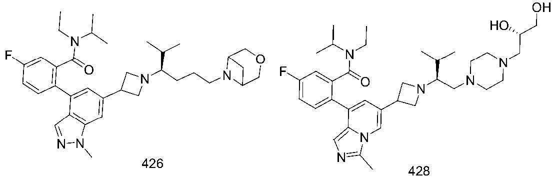

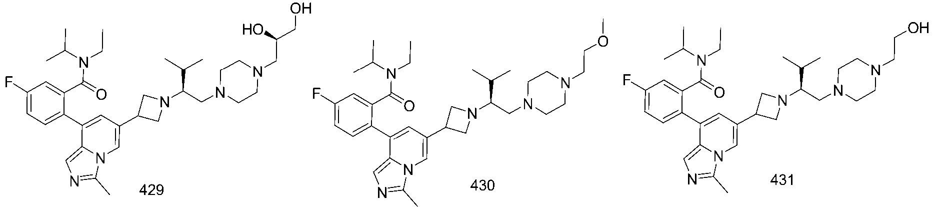

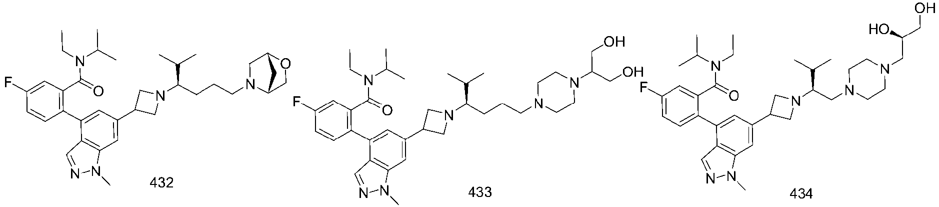

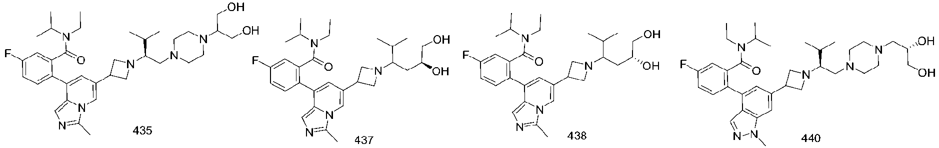

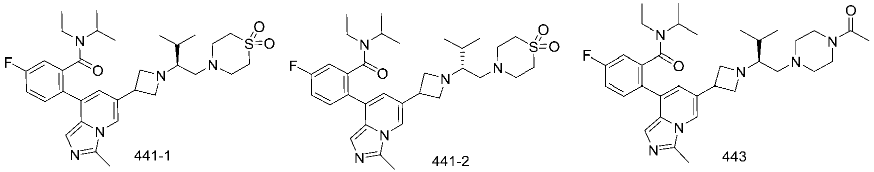

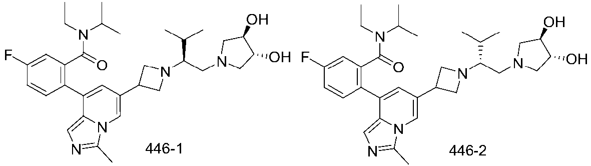

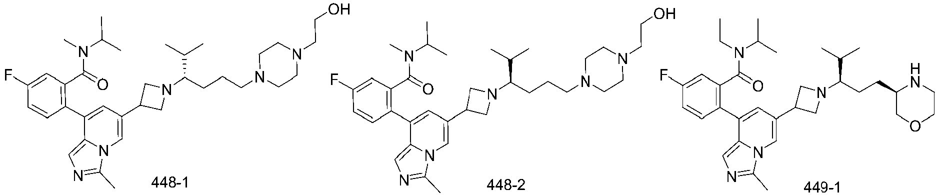

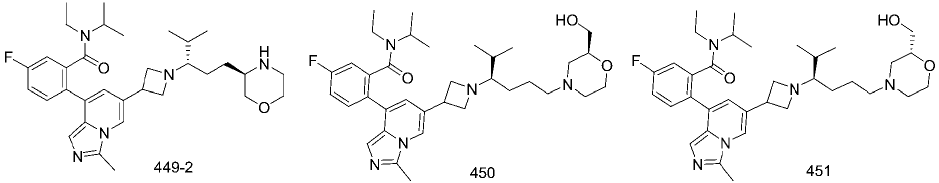

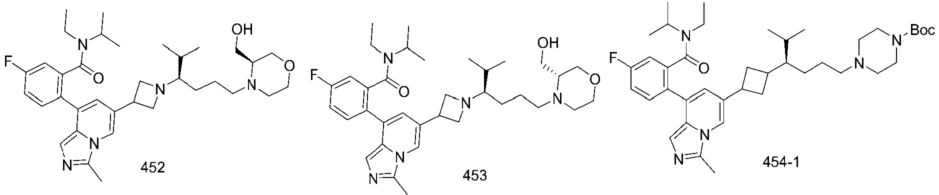

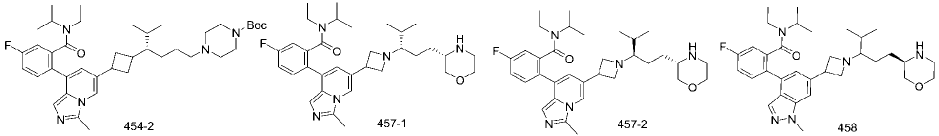

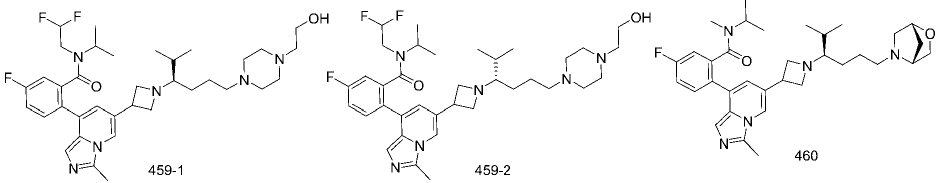

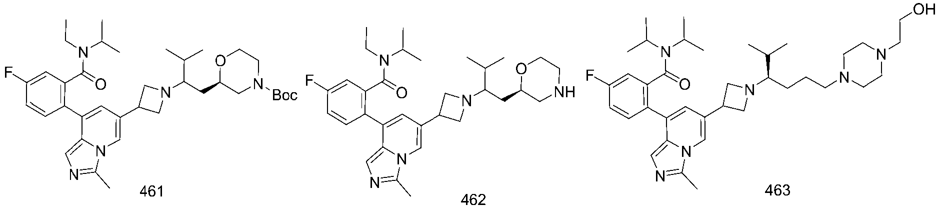

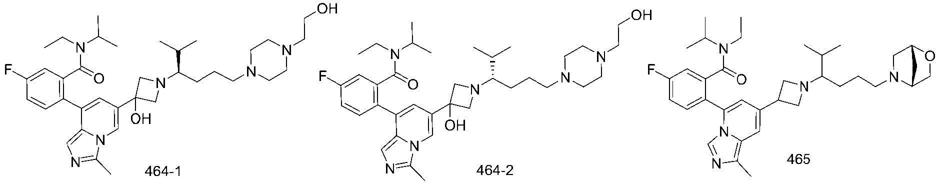

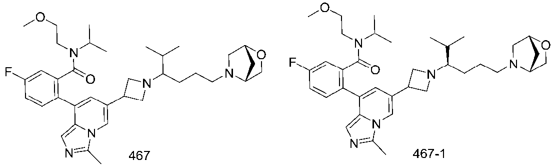

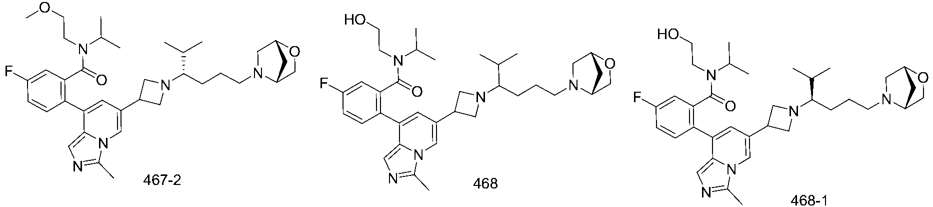

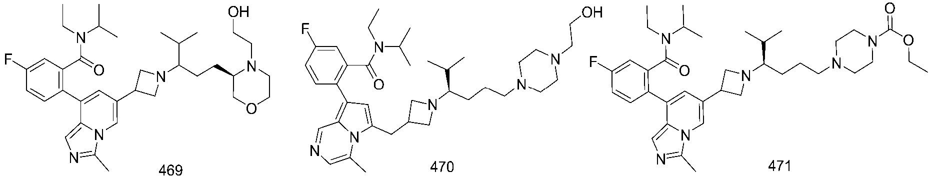

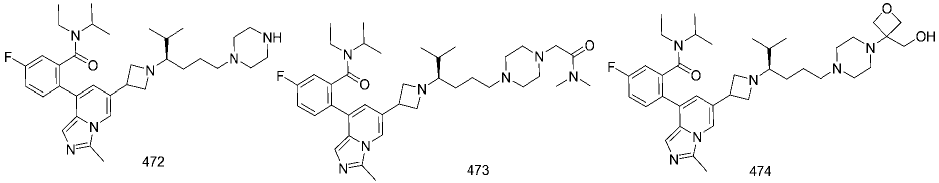

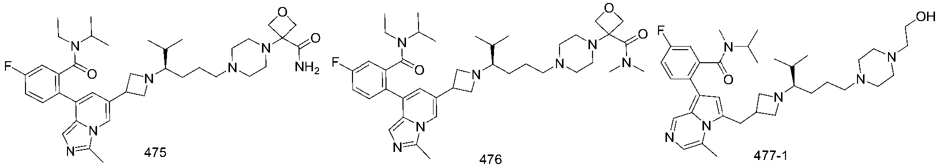

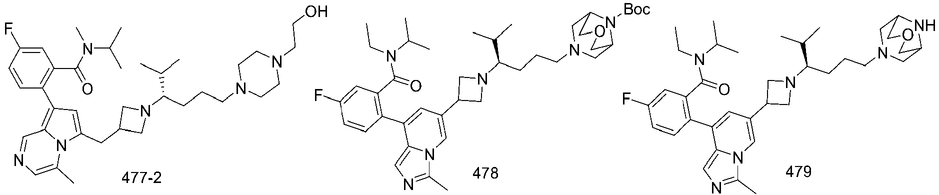

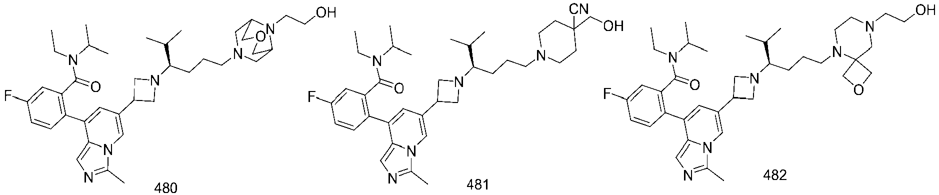

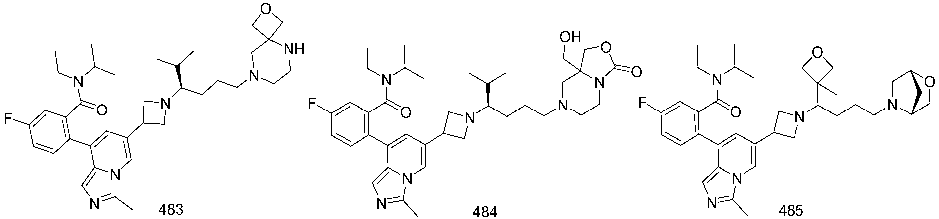

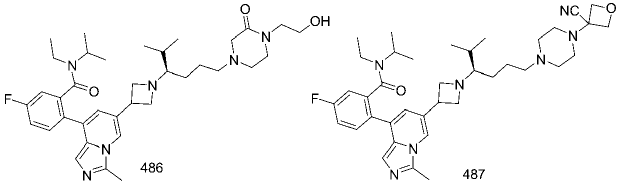

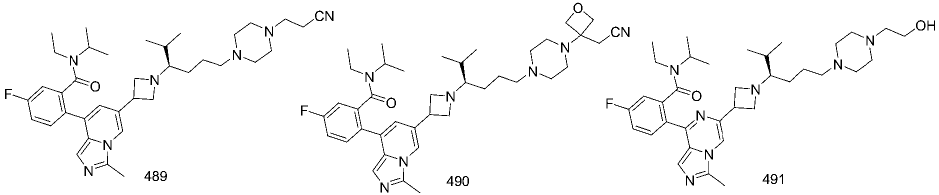

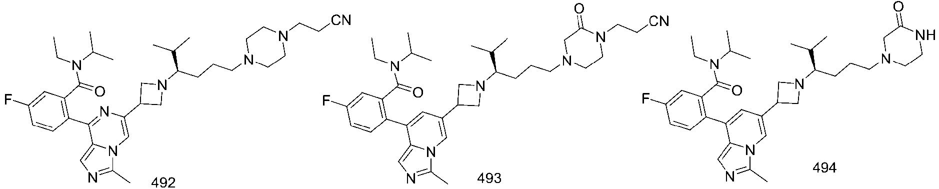

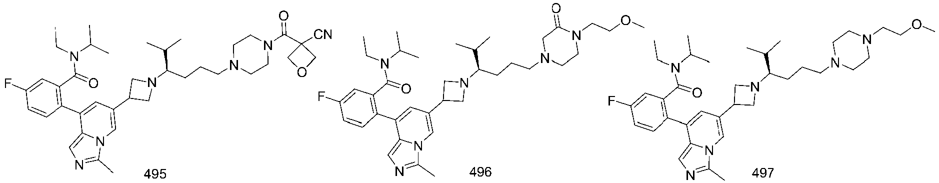

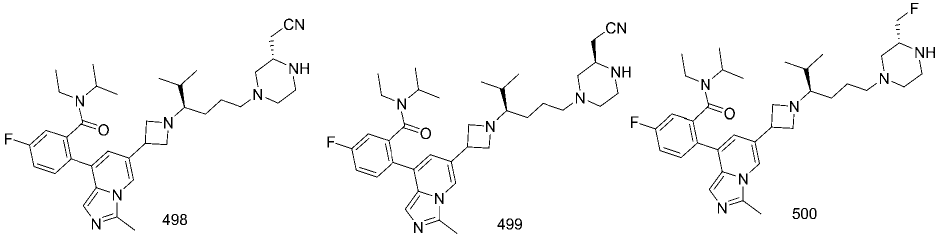

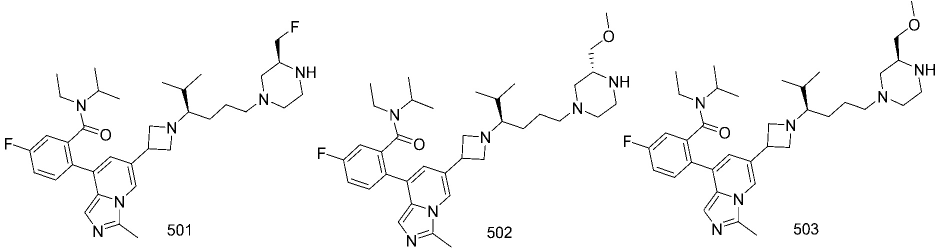

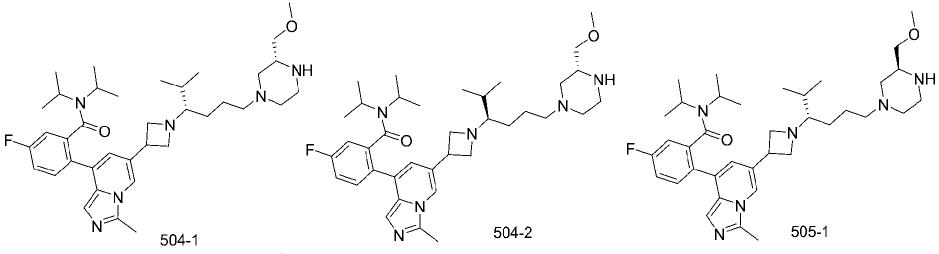

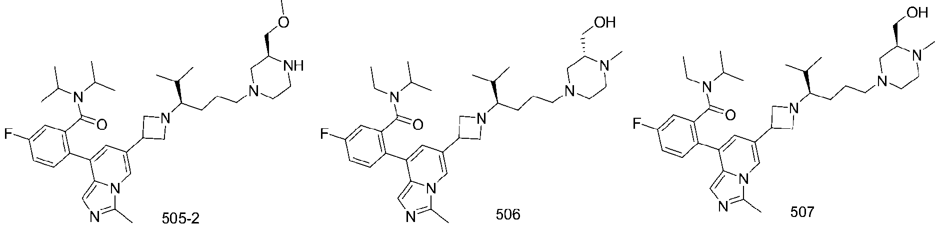

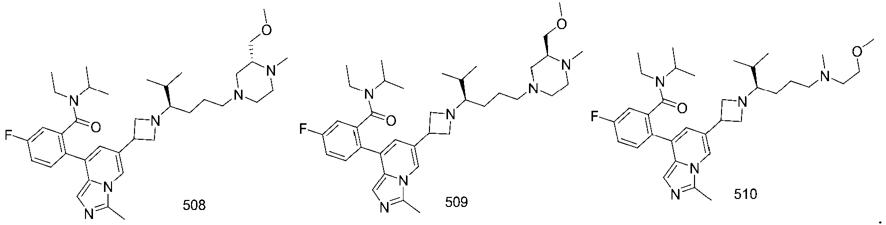

19. A pharmaceutical composition, comprising the compound according to any one of claims 1 to 18 or a pharmaceutically acceptable salt, stereoisomer, ester, prodrug, solvate and deuterated compound thereof, and a pharmaceutically acceptable excipient.

20. Use of the compound according to any one of claims 1 to 18 or a pharmaceutically acceptable salt, stereoisomer, ester, prodrug, solvate and deuterated compound thereof in the preparation of a medicament for inhibiting Menin-MLL interaction.

21. Use of the compound according to any one of claims 1 to 18 or a pharmaceutically acceptable salt, stereoisomer, ester, prodrug, solvate and deuterated compound thereof in the preparation of a medicament for preventing and/or treating a disease mediated by Menin-MLL interaction;
preferably, said diseases is selected from the group consisting of: a tumor, diabetes, insulin resistance, hyperglycemia, an autoimmune disease and non-alcoholic hepatitis; and
more preferably, said tumor is a hematological tumor.
